(19) Europäisches Patentamt
European Patent Office
Office européen des brevets

(11) EP 1 748 065 A2

(12) **EUROPEAN PATENT APPLICATION**

(43) Date of publication:
**31.01.2007 Bulletin 2007/05**

(51) Int Cl.:
***C12N 5/06*** *(2006.01)*

(21) Application number: **06015664.3**

(22) Date of filing: **31.12.2003**

(84) Designated Contracting States:
**AT BE BG CH CY CZ DE DK EE ES FI FR GB GR HU IE IT LI LU MC NL PT RO SE SI SK TR**

(30) Priority: **10.01.2003 US 439123 P**
**06.05.2003 US 468402 P**

(62) Document number(s) of the earlier application(s) in accordance with Art. 76 EPC:
**03782680.7 / 1 587 916**

(71) Applicant: **Cyclacel Limited**
**Dundee DD1 5JJ (GB)**

(72) Inventors:
- **Glover, David**
**Department of Genetics**
**Cambridge CB2 3EH (GB)**
- **Bell, Graham**
**Cyclacel Limited**
**Dundee DD1 5JJ (GB)**
- **Frenz, Lisa**
**Cyclacel Limited, Pologen Division**
**Babraham, Cambridgeshire CB2 4AT (GB)**
- **Midgley, Carol**
**Cyclacel Limited, Pologen Division**
**Babraham, Cambridgeshire CB2 4AT (GB)**

(74) Representative: **Furlong, Isla Jane et al**
**D Young & Co**
**120 Holborn**
**London EC1N 2DY (GB)**

Remarks:
•The complete document including Reference Tables and the Sequence Listing is available on CD-ROM from the European Patent Office, Vienna sub-office
•This application was filed on 27 - 07 - 2006 as a divisional application to the application mentioned under INID code 62.
•The sequence listing, which is published as annex to the application documents, was filed after the date of filing. The applicant has declared that it does not include matter which goes beyond the content of the application as filed.

(54) **Cell cycle progression proteins**

(57) The invention describes human genes involved in cell cycle progression, including mitosis and meiosis. The invention also relates to the use of these "cell cycle progression" genes and proteins in the modulation of cell cycle progression in cells and methods for identifying modulators of these genes or proteins and hence modulators of mitosis and meiosis.

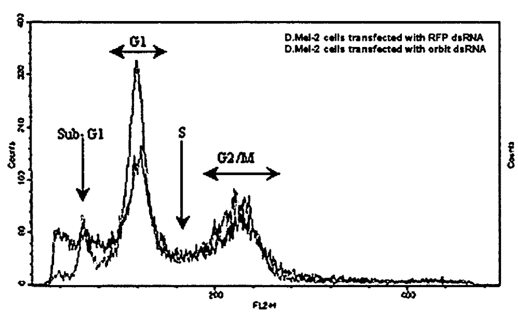


Figure 181

EP 1 748 065 A2

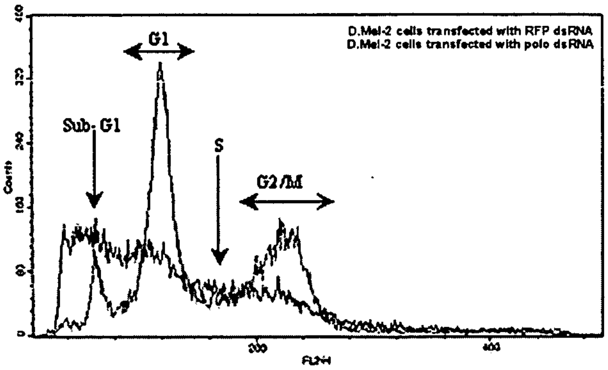


Figure 181 (cont'd)

## Description

The present invention relates to a number of human genes, and the proteins they encode, implicated in the processes of cell cycle progression, including mitosis and meiosis. The invention also relates to the use of these "cell cycle progression" genes and proteins in the modulation of cell cycle progression in cells and methods for identifying modulators of these genes or proteins and hence modulators of mitosis and meiosis.

## BACKGROUND

Proliferative growth of normal cells requires an orderly progression through a series of distinct steps, a process known as the cell cycle. Progression through the cell cycle is modulated by nutrient availability, cell size and growth factors through complex signalling pathways involving phosphorylation cascades and the strictly regulated expression and stability of specific proteins required at each phase of the cell cycle.

The phases of the cell cycle begin with the M phase, where cytoplasmic division (cytokinesis) occurs. The M phase is followed by the G1 phase, in which the cells resume a high rate of biosynthesis and growth. The S phase begins with DNA synthesis, and ends when the DNA content has doubled. The cell then enters the G2 phase, which ends when mitosis starts, signalled by the appearance of condensed chromosomes. Terminally differentiated cells are arrested in the G1 phase, and no longer undergo cell division.

The sequence of cell cycle events is rigorously controlled at specific checkpoints to ensure that each discrete stage in the cell cycle has been completed before the next is initiated. Human diseases associated with abnormal cell proliferation, including cancer, result when these rigorous controls on cell cycle progression are perturbed.

The elucidation of the genes and gene products involved in the cell cycle and its control will provide novel opportunities in the prophylactic, diagnostic and therapeutic management of cancer and other proliferation-related diseases (*e.g.*, atherosclerosis).

On the other hand, it is also sometimes desirable to enhance proliferation of cells in a controlled manner. For example, proliferation of cells is useful in wound healing and where growth of tissue is desirable. Thus, identifying genes, their gene products and modulators which promote, enhance or deter the inhibition of proliferation is desirable.

Despite the desirability of identifying cell cycle components and modulators, there is a deficit of such compounds in the field. Accordingly, it would be advantageous to identify genes and their corresponding protein products whose activity is associated with cell cycle progression.

## SUMMARY

We have now identified a number of human genes involved with cell cycle progression, for example the processes of mitosis and/or meiosis. Discovery of the role of these genes has been through assays configured to identify genes involved in cell cycle progression by knocking down gene expression using RNAi and assessing the resultant phenotype for abnormalities in cell cycle progression.

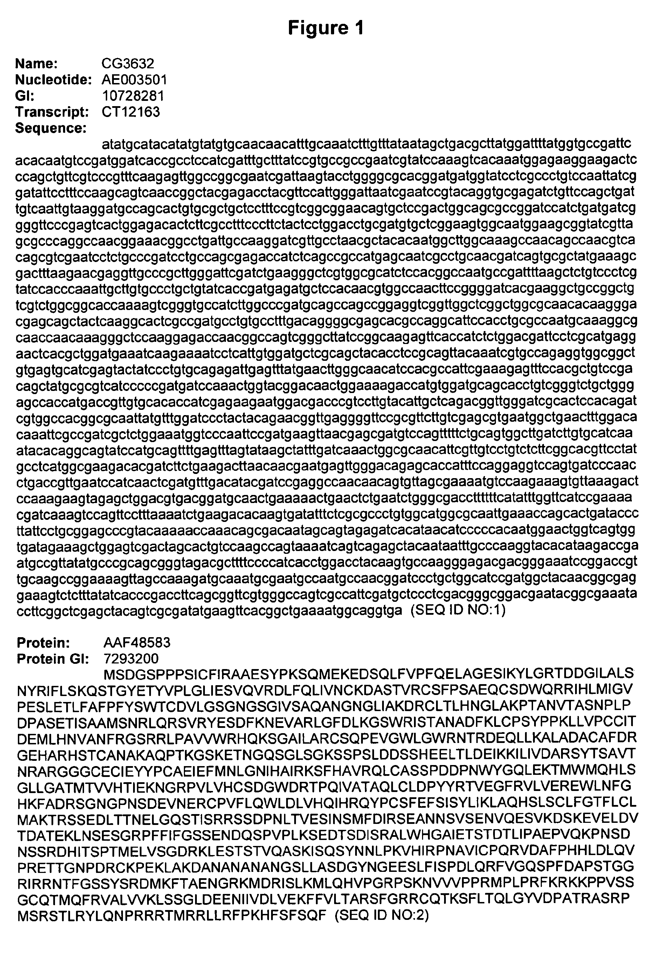

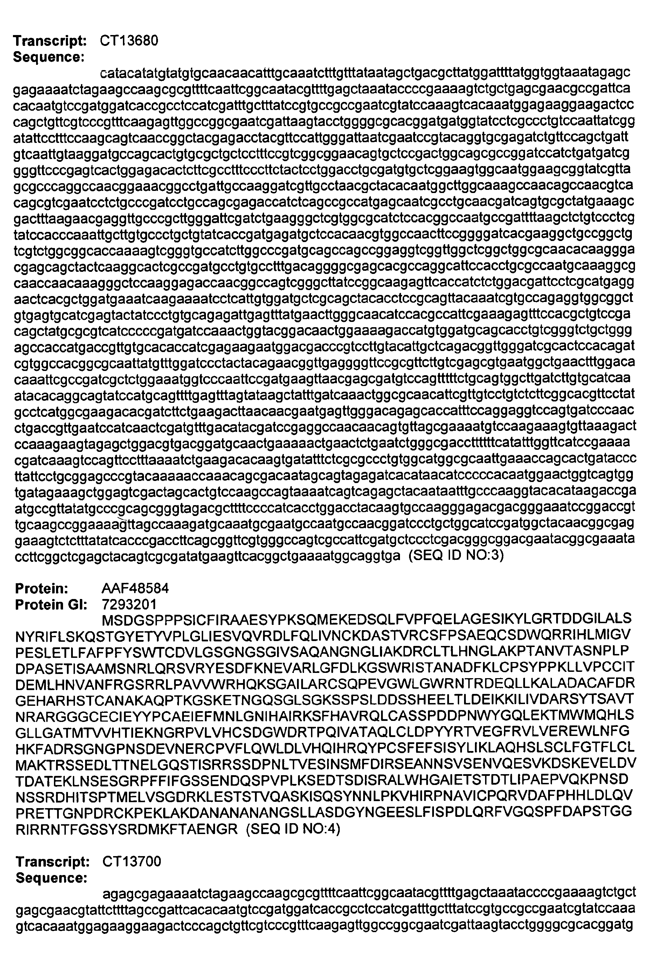

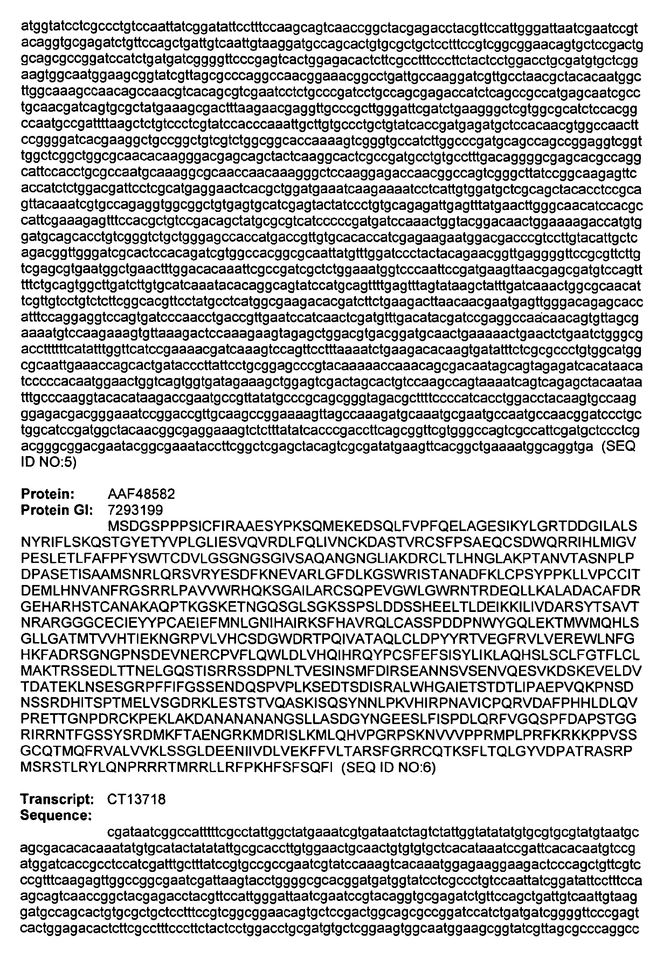

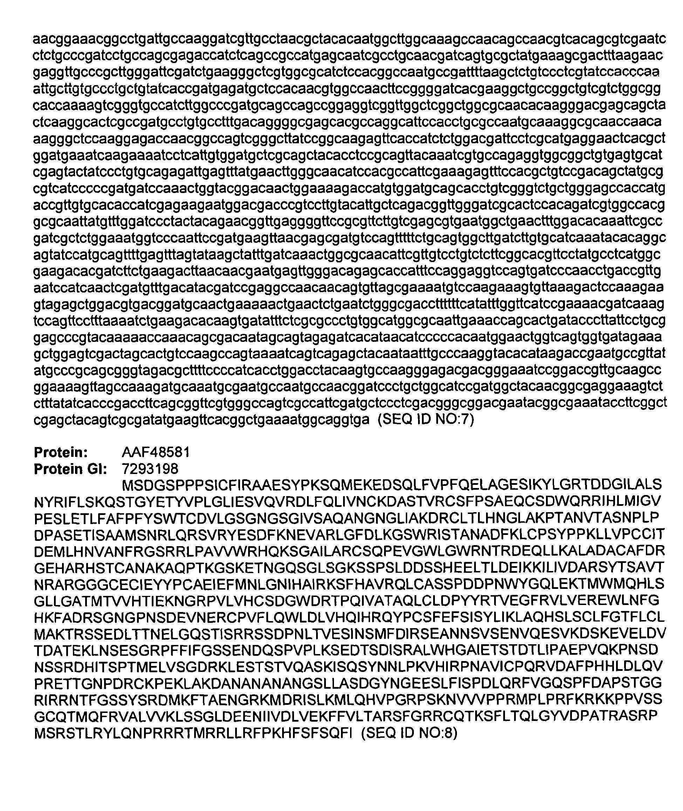

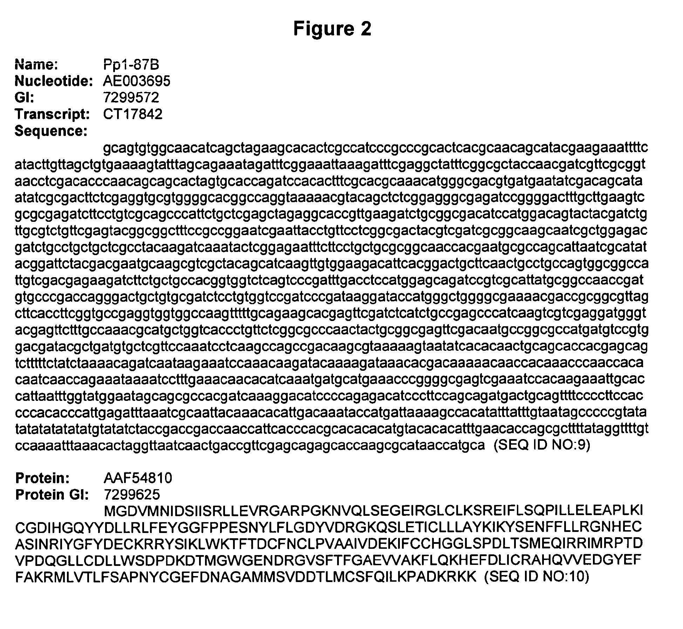

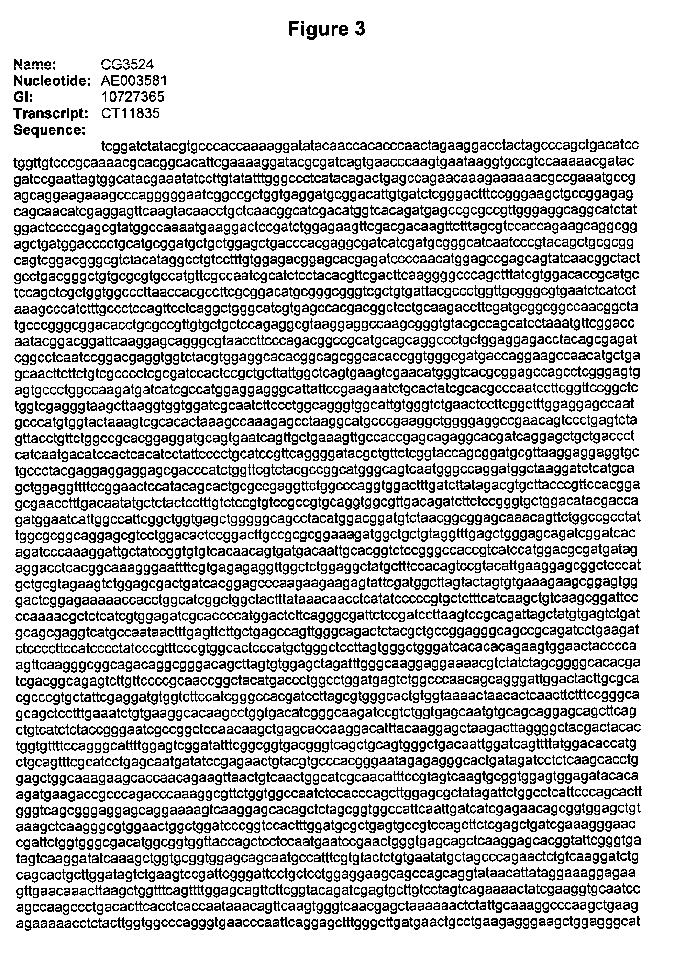

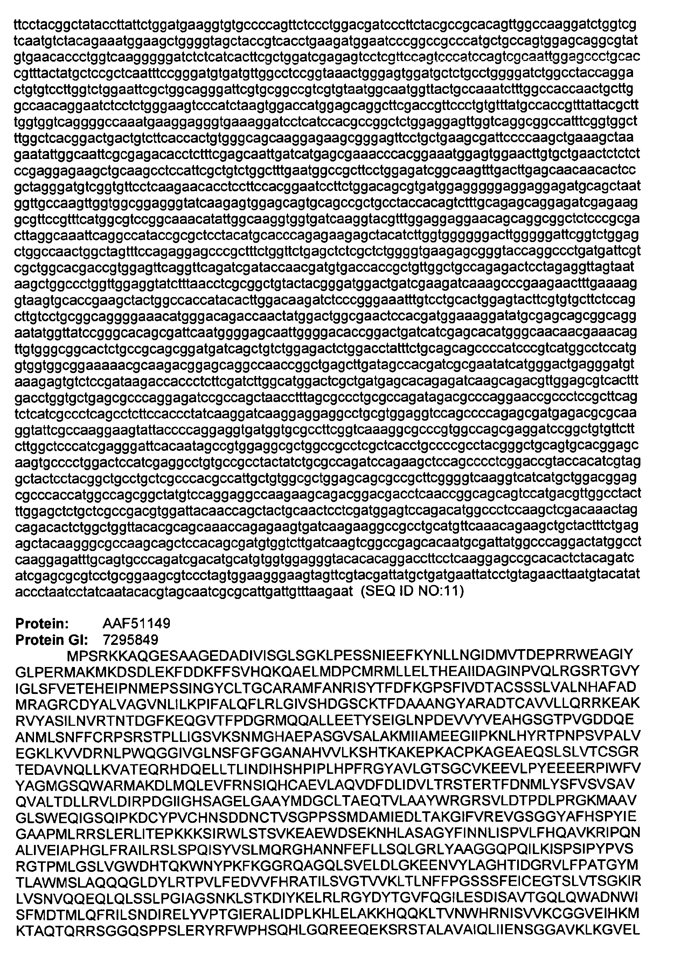

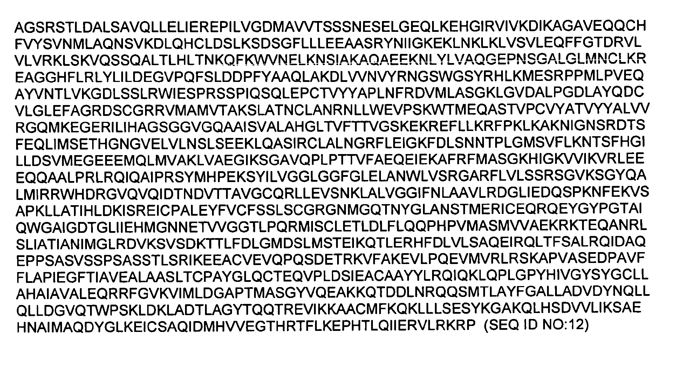

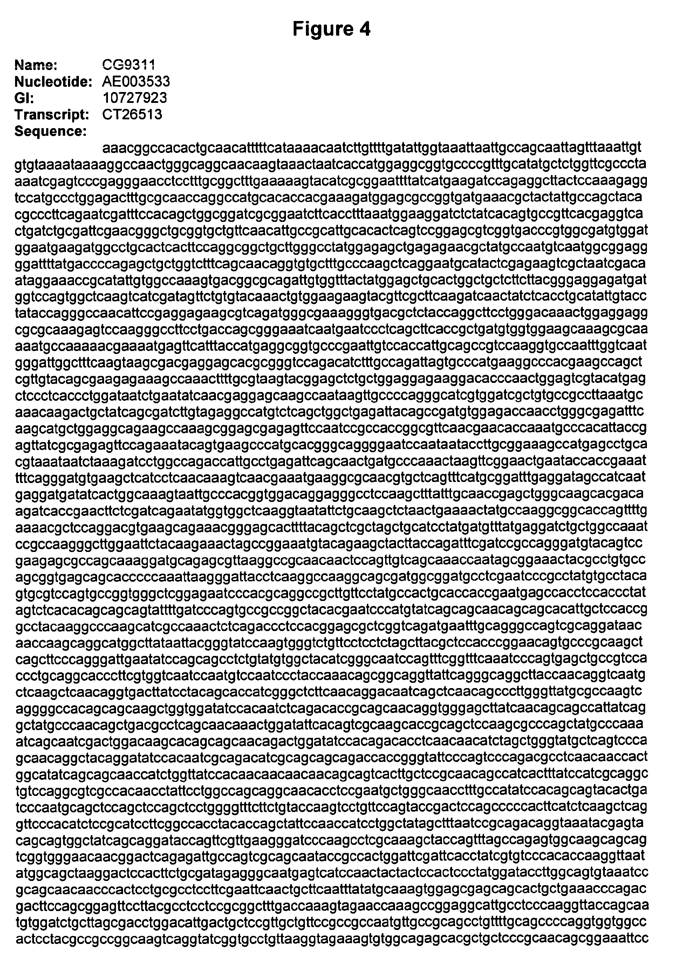

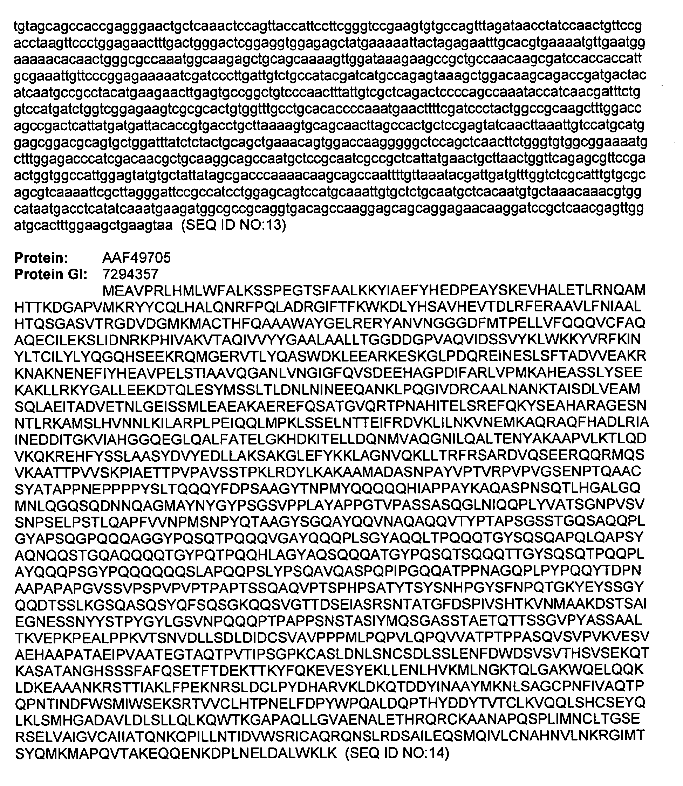

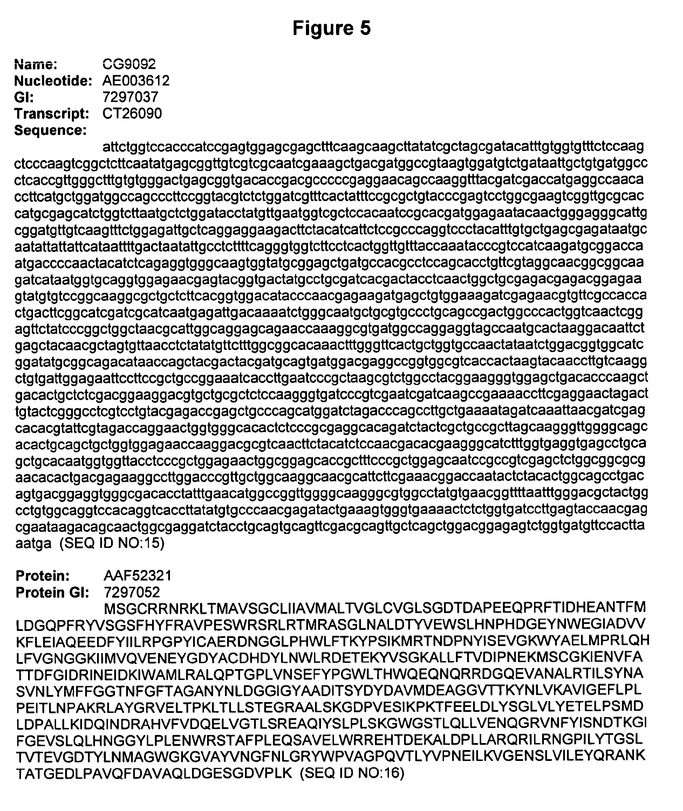

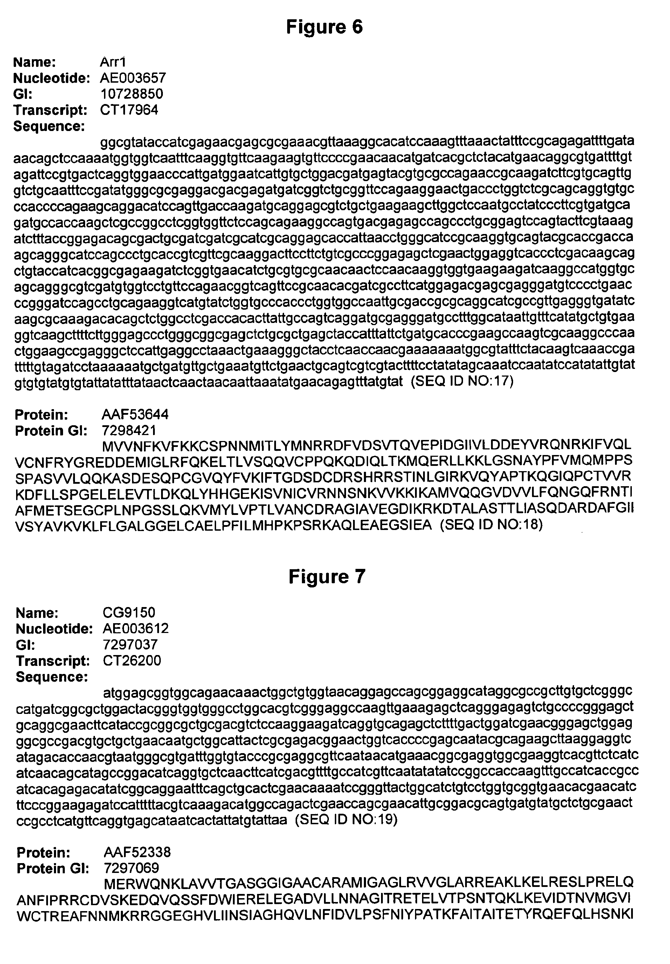

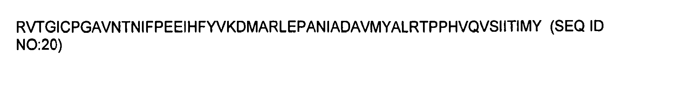

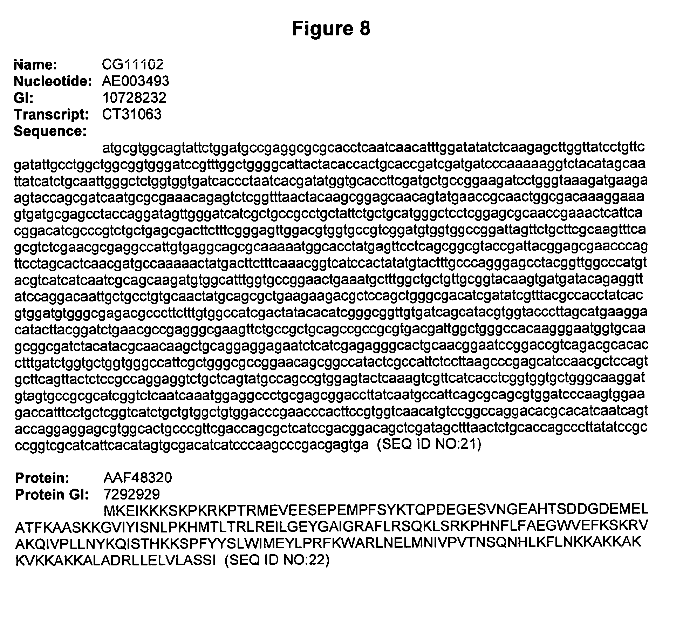

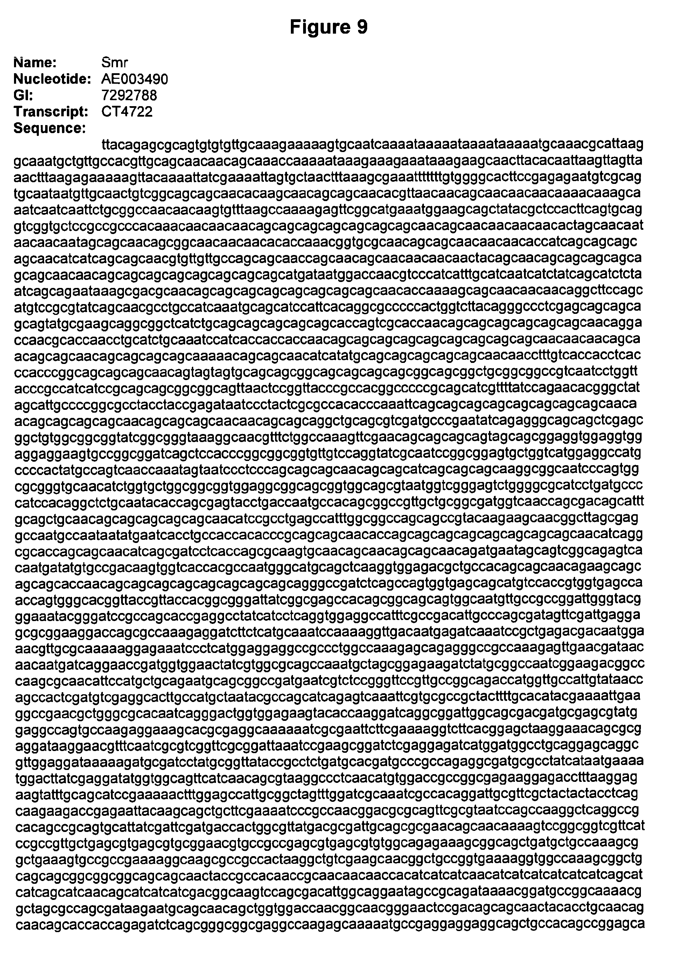

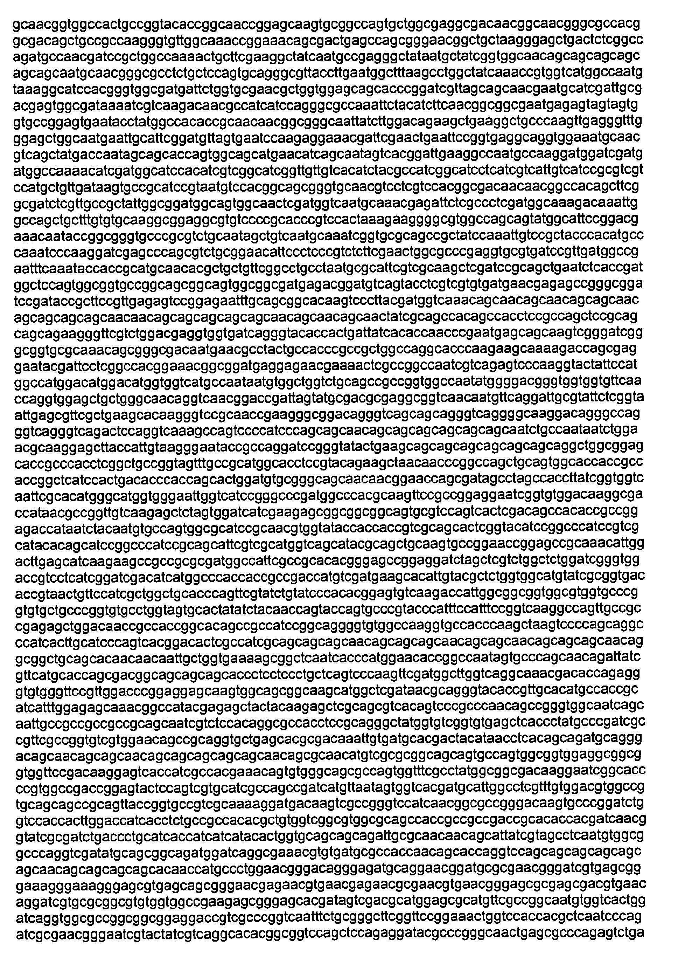

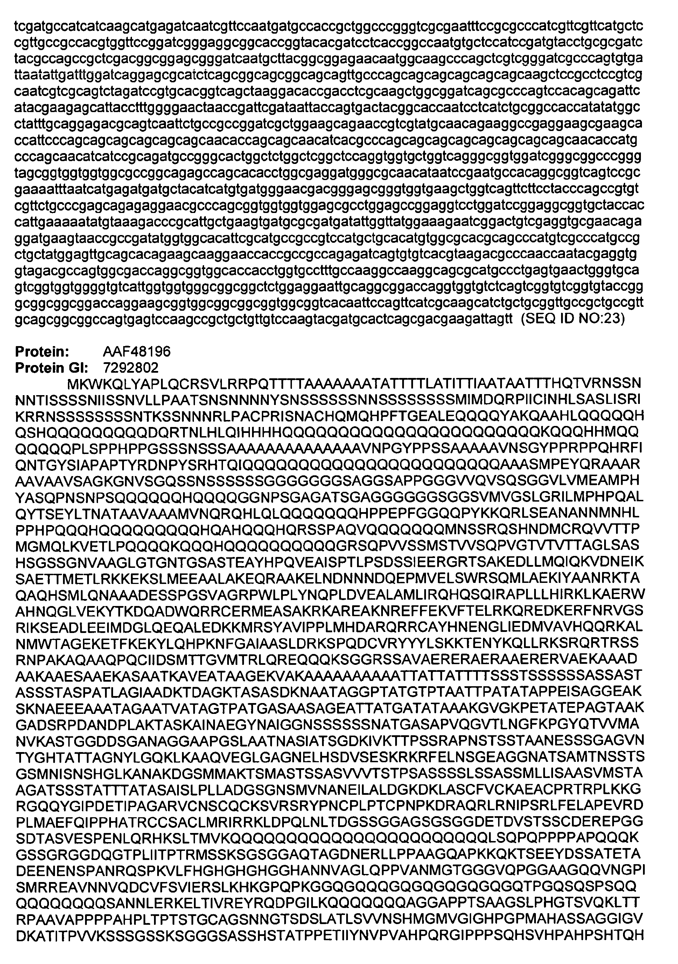

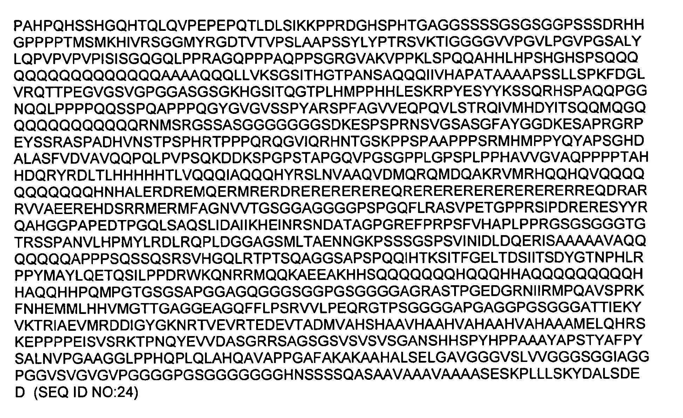

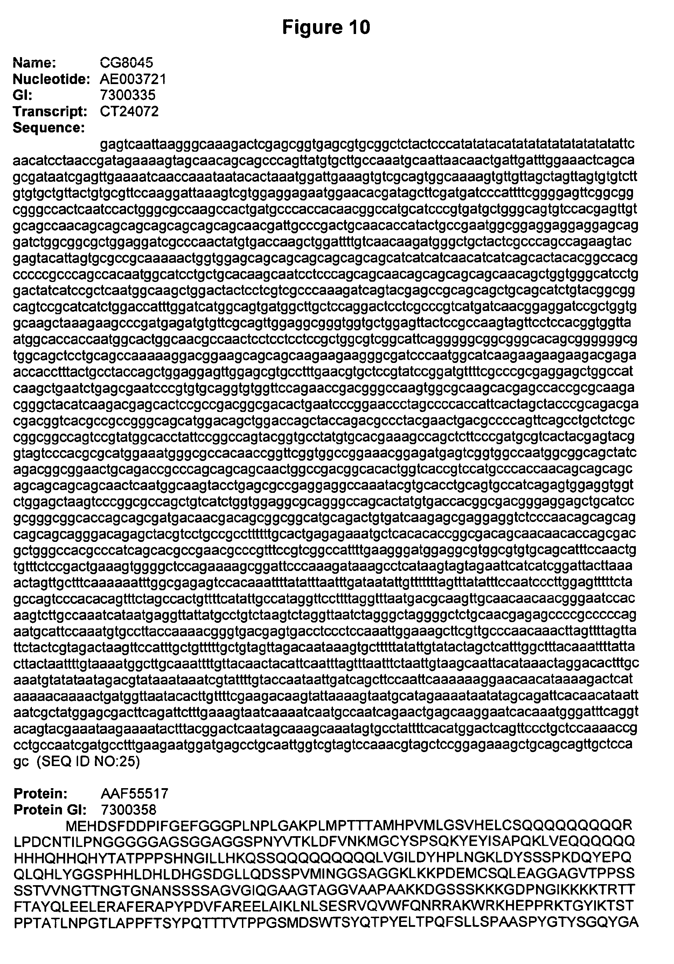

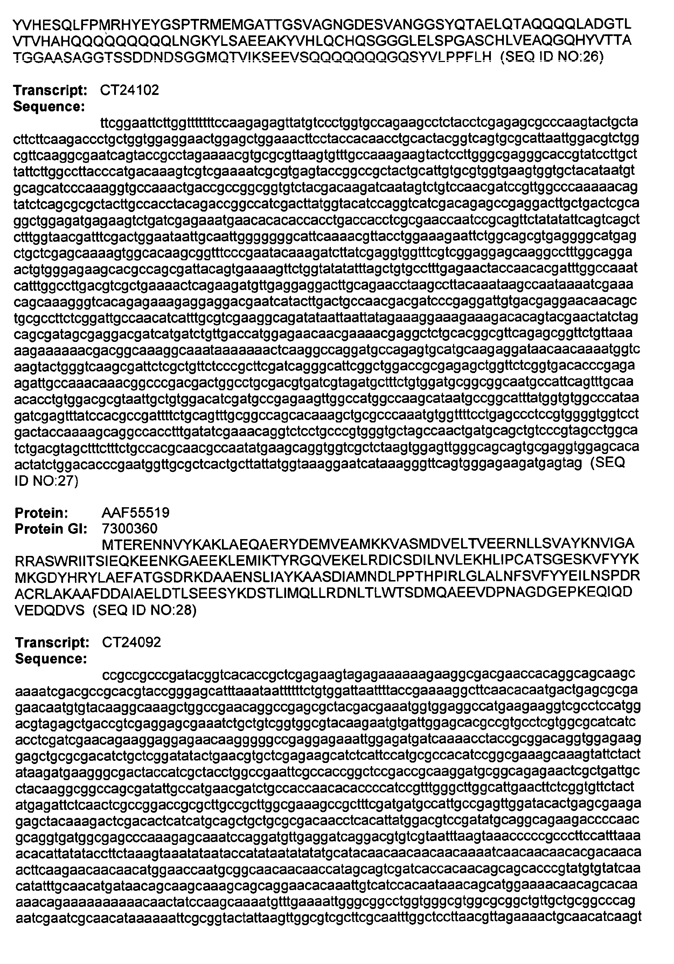

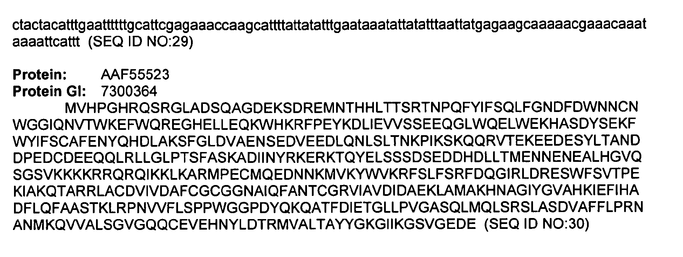

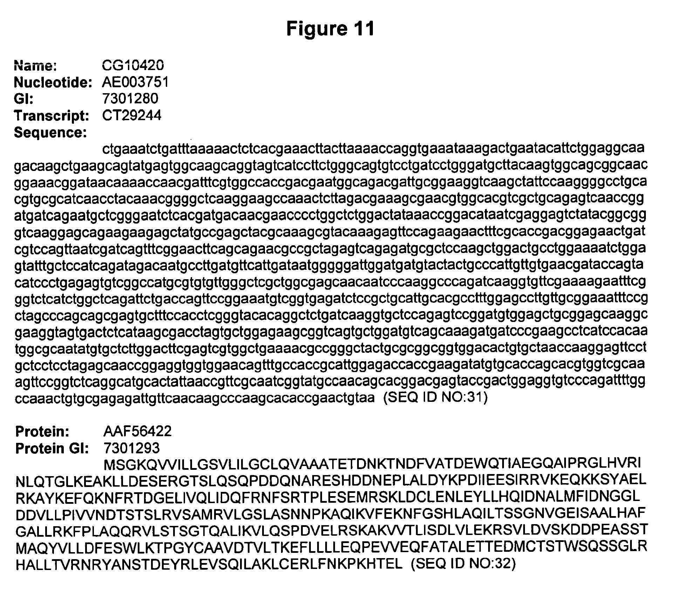

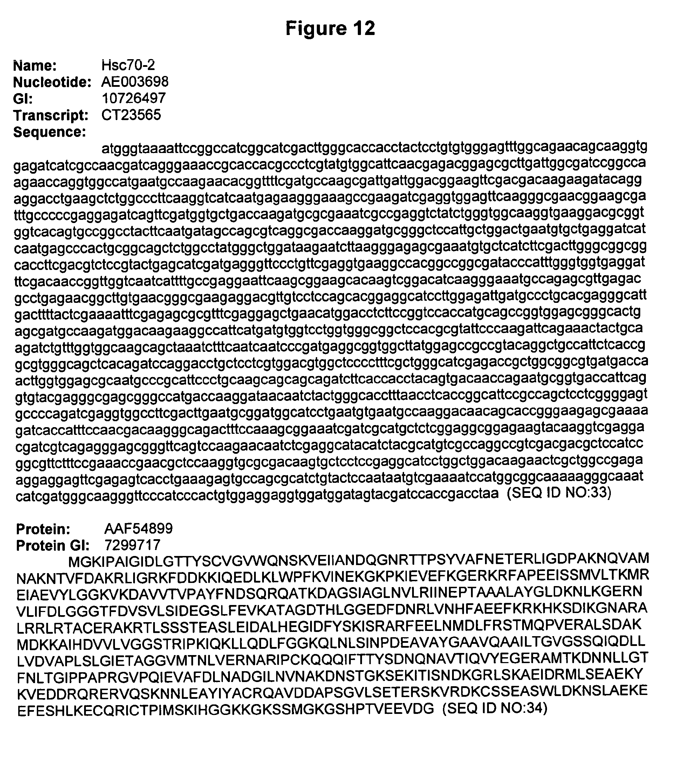

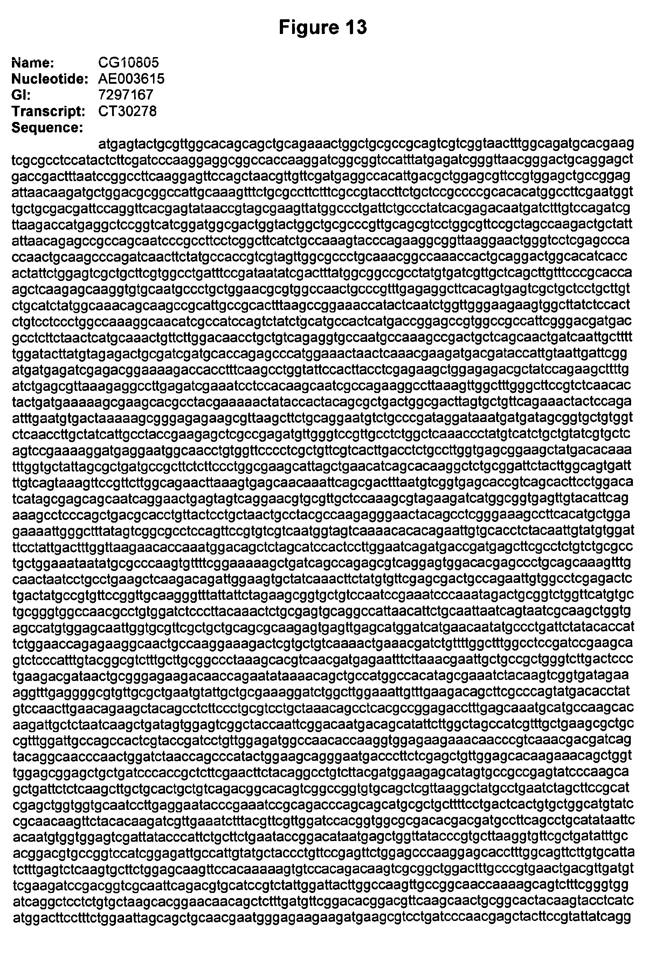

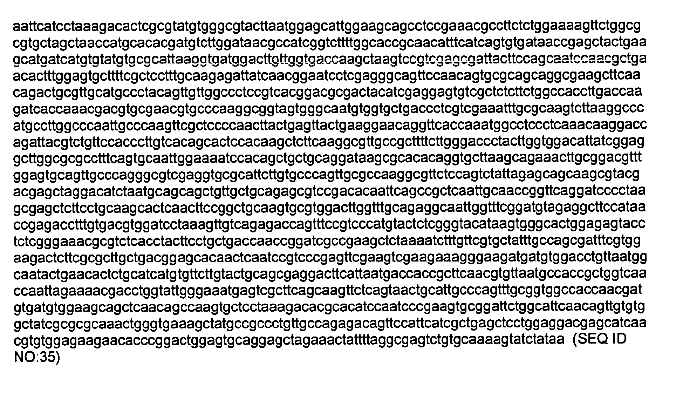

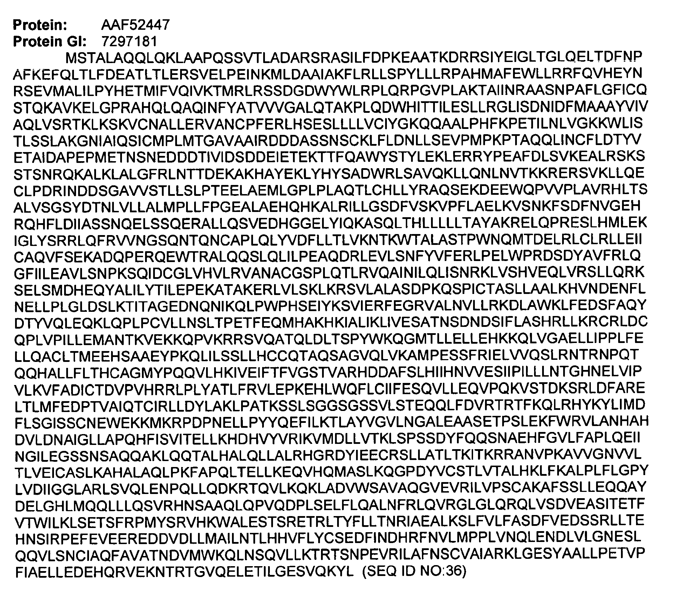

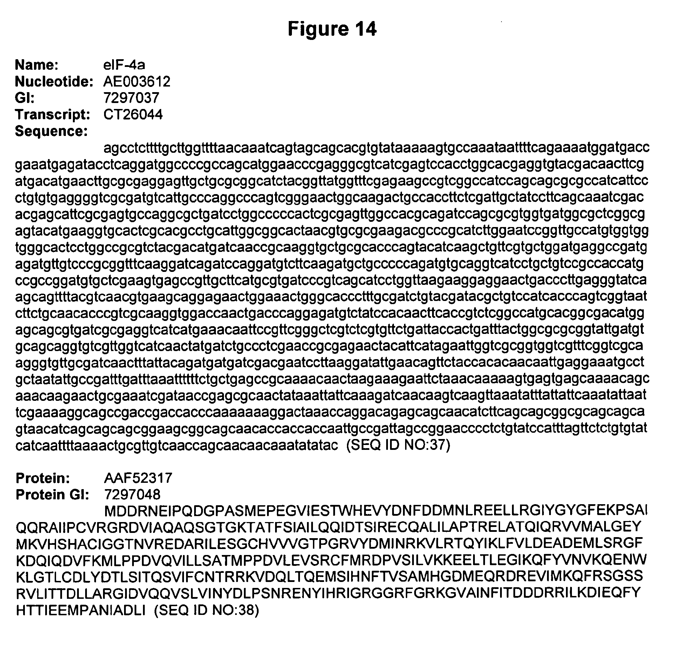

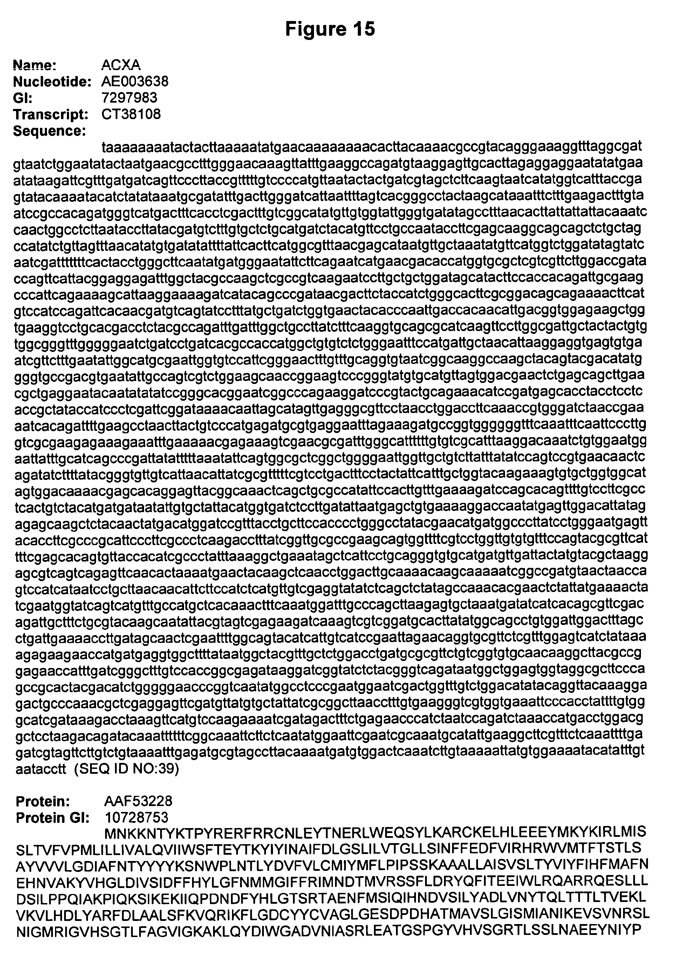

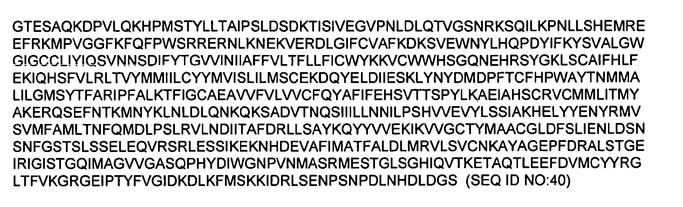

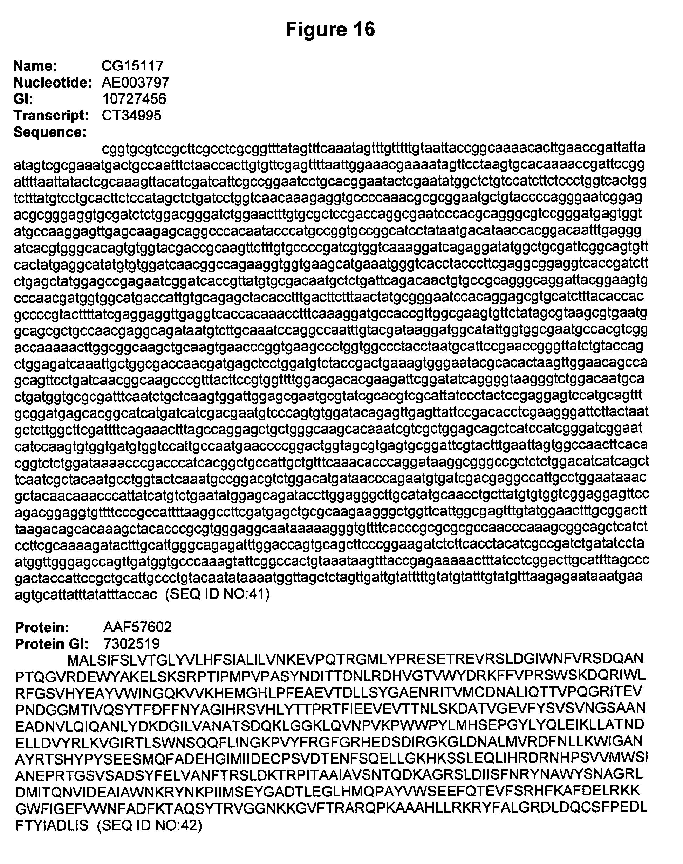

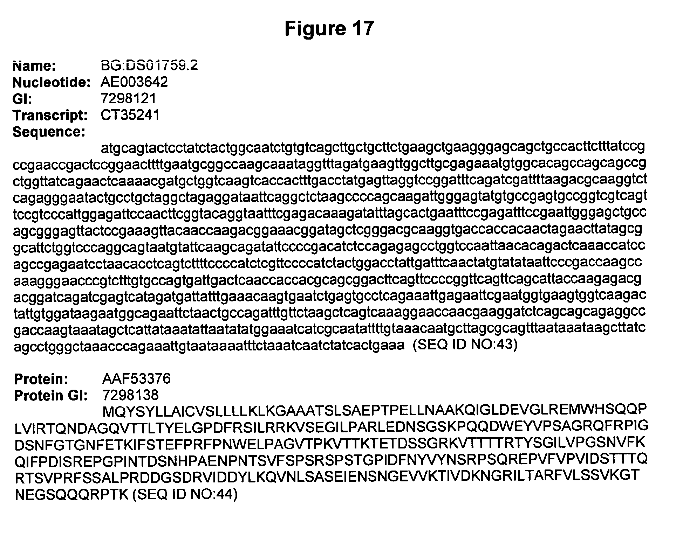

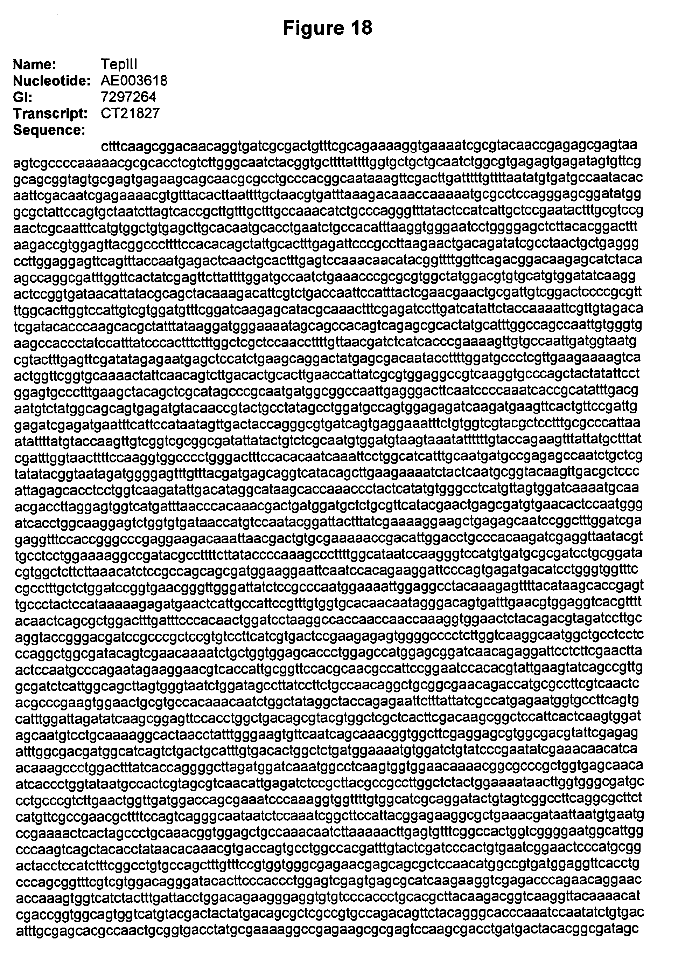

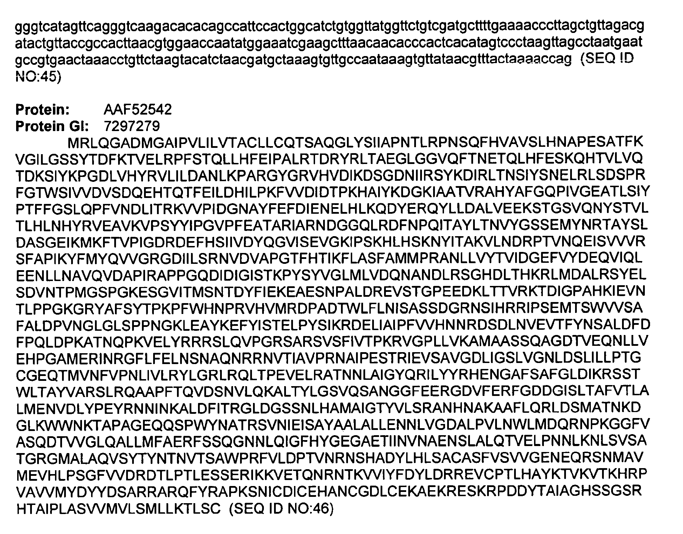

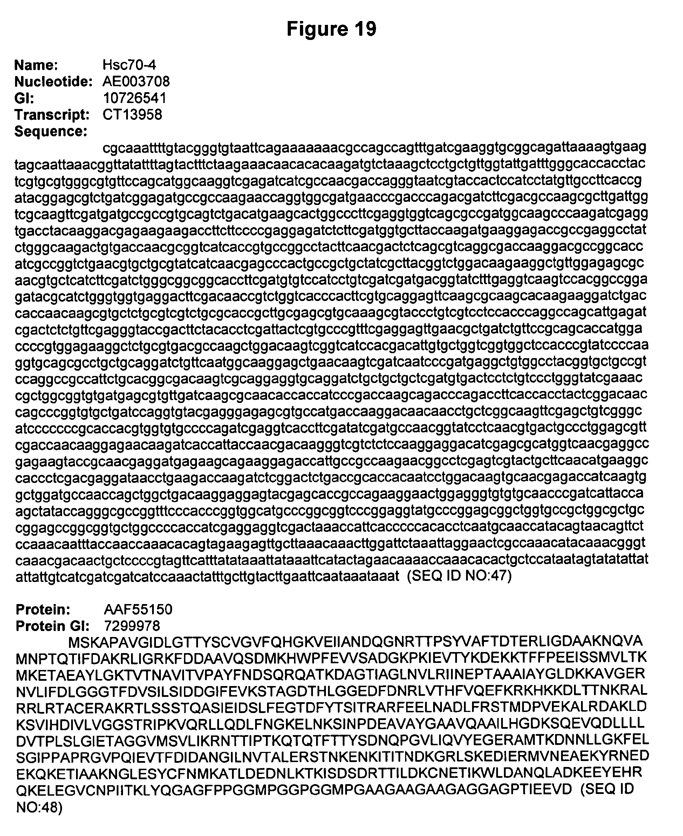

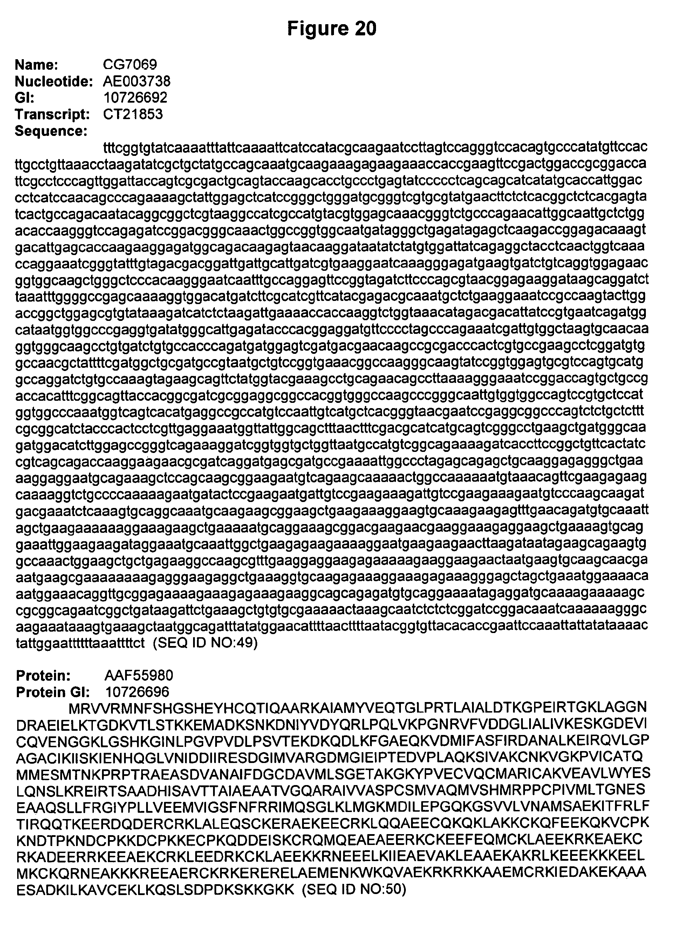

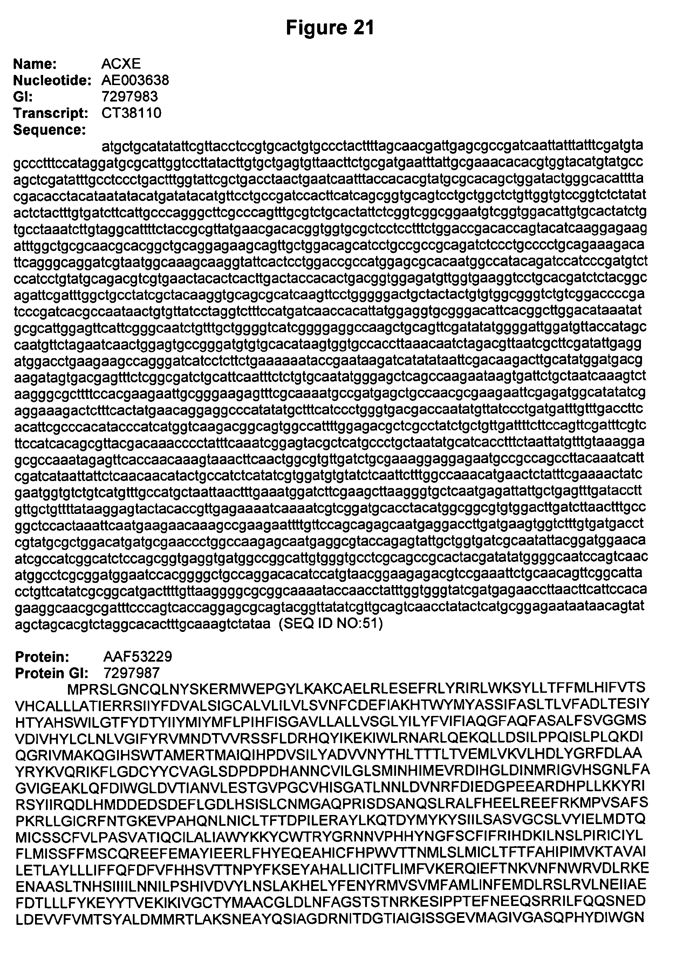

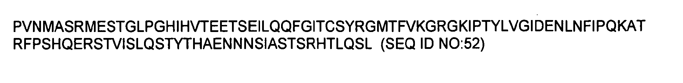

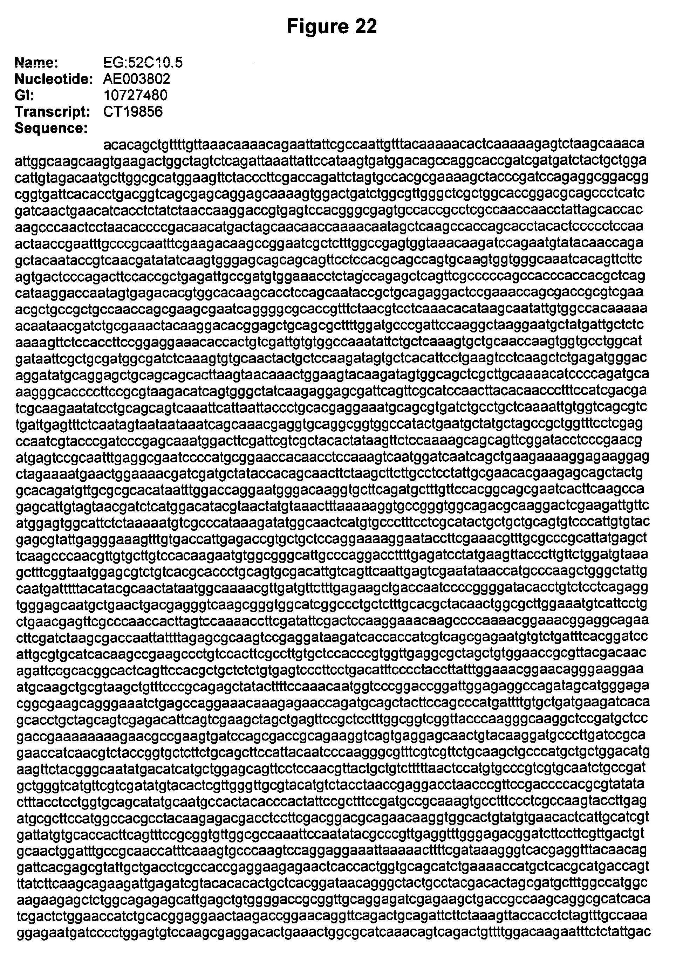

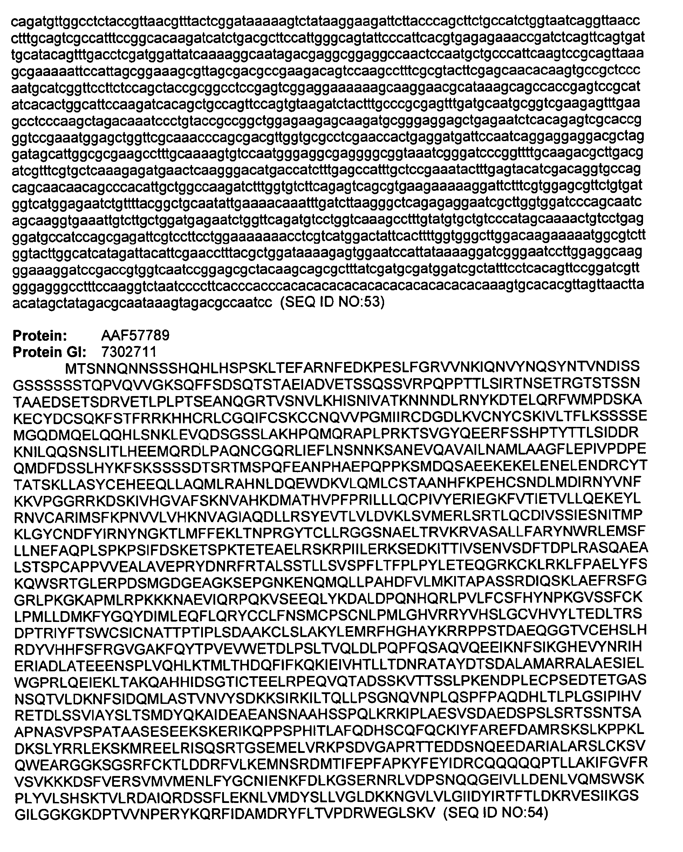

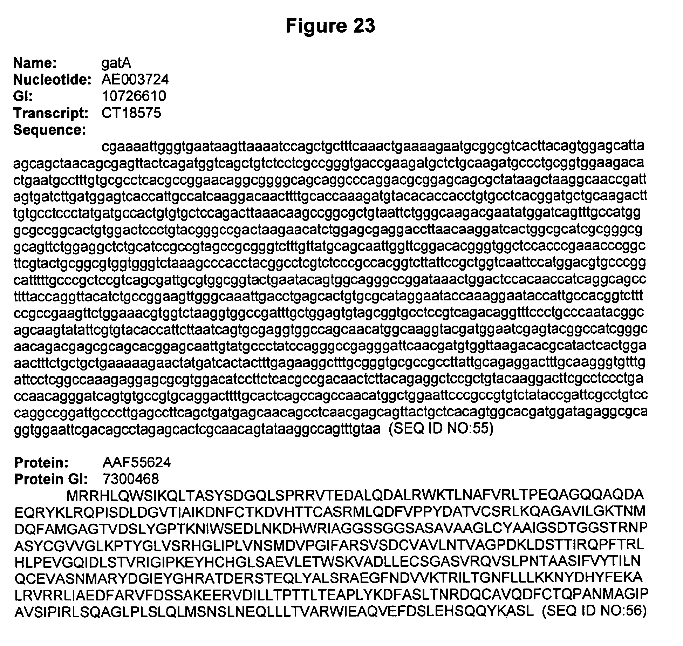

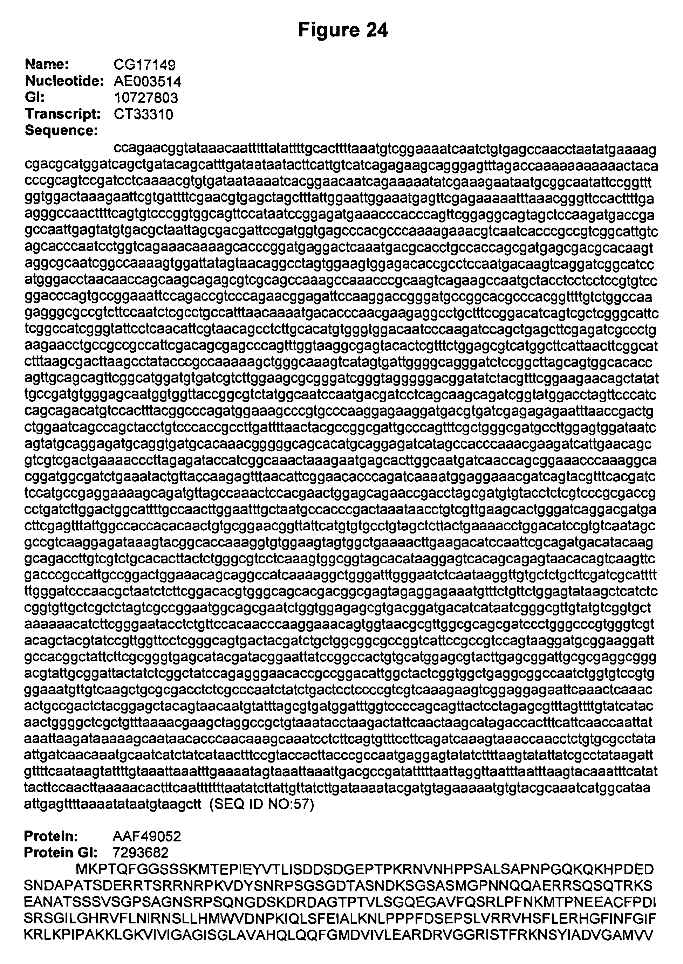

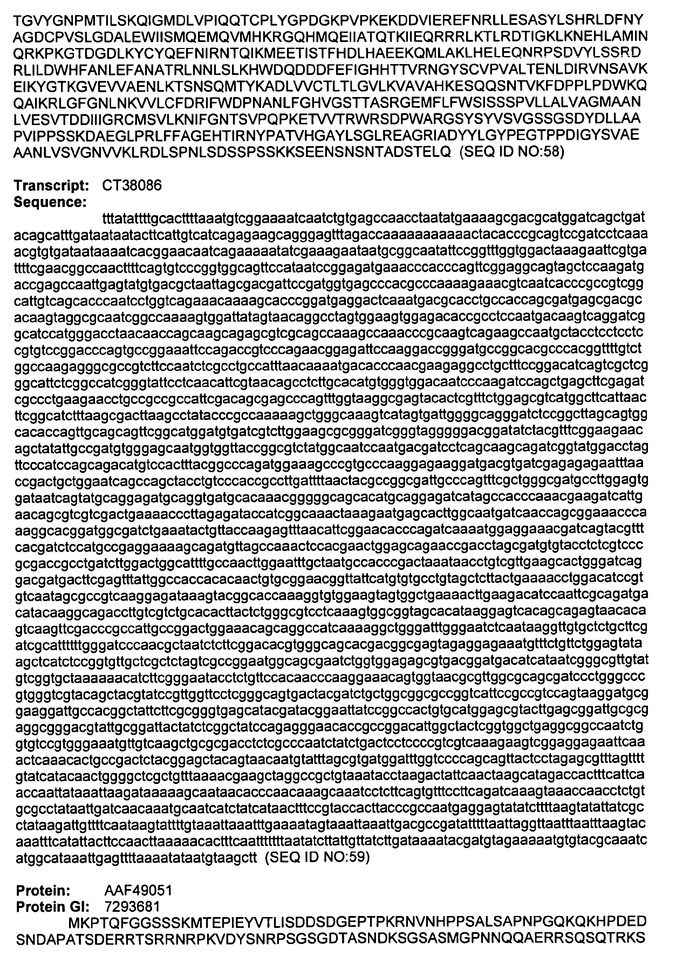

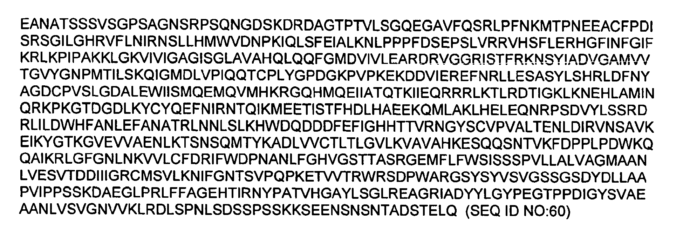

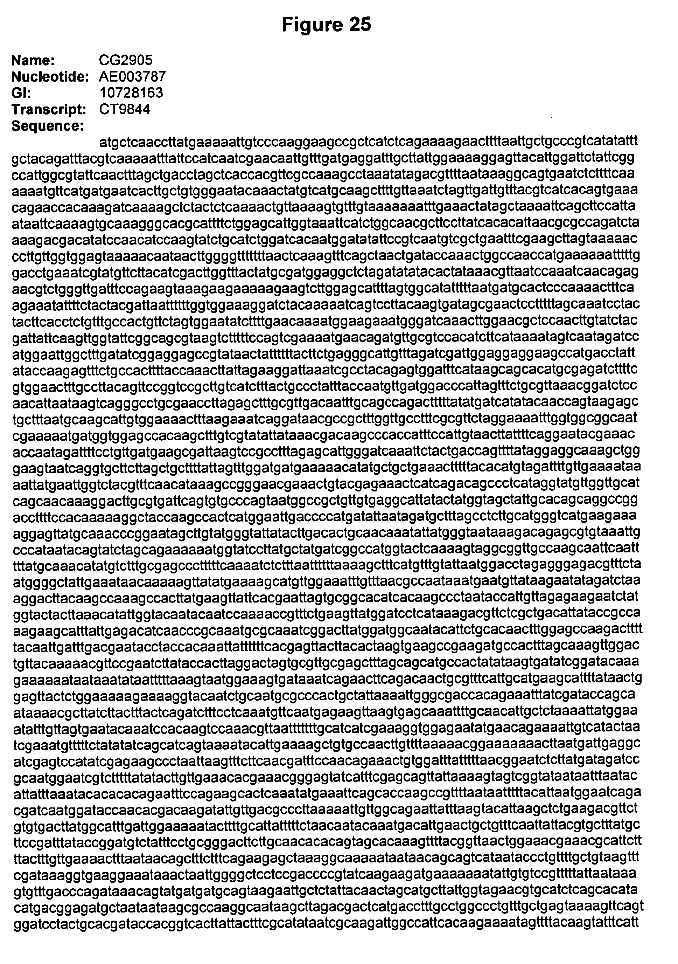

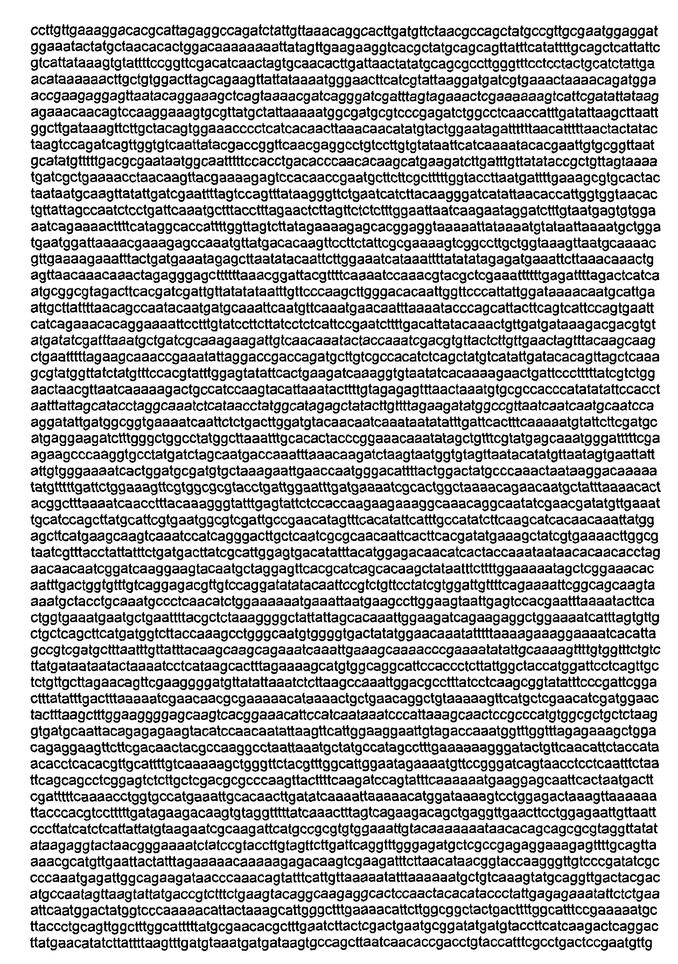

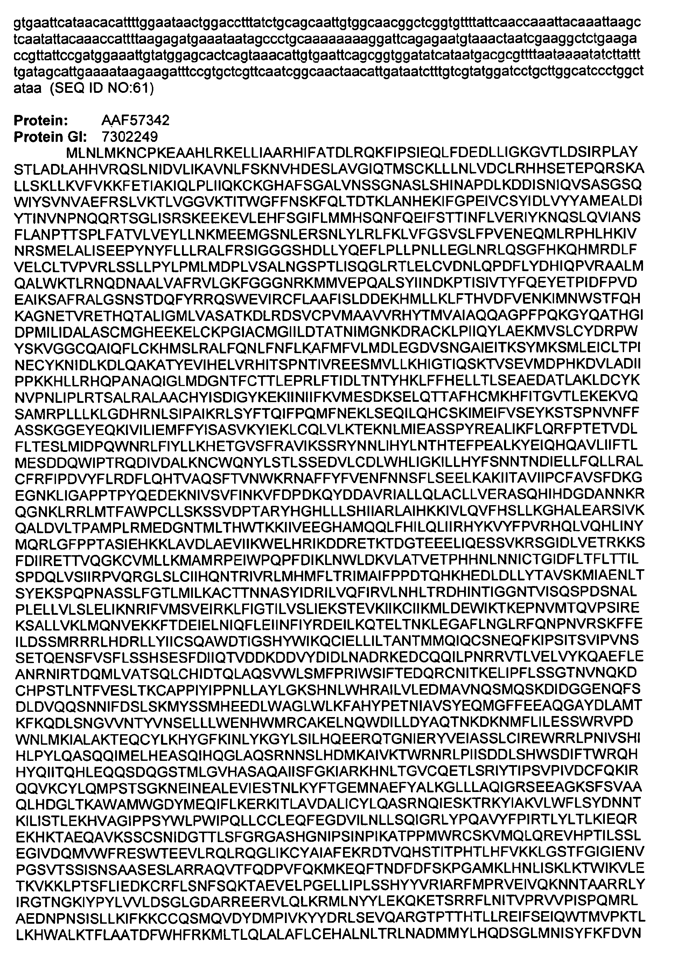

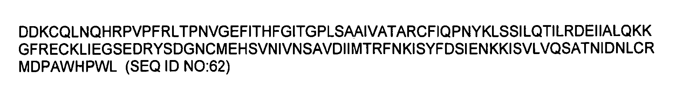

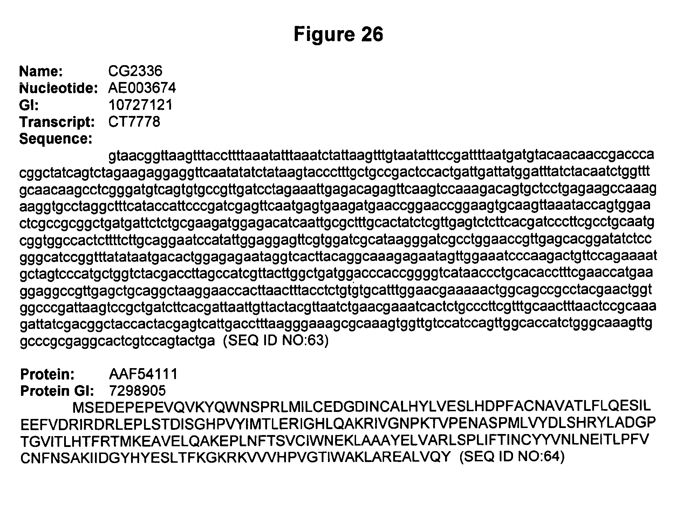

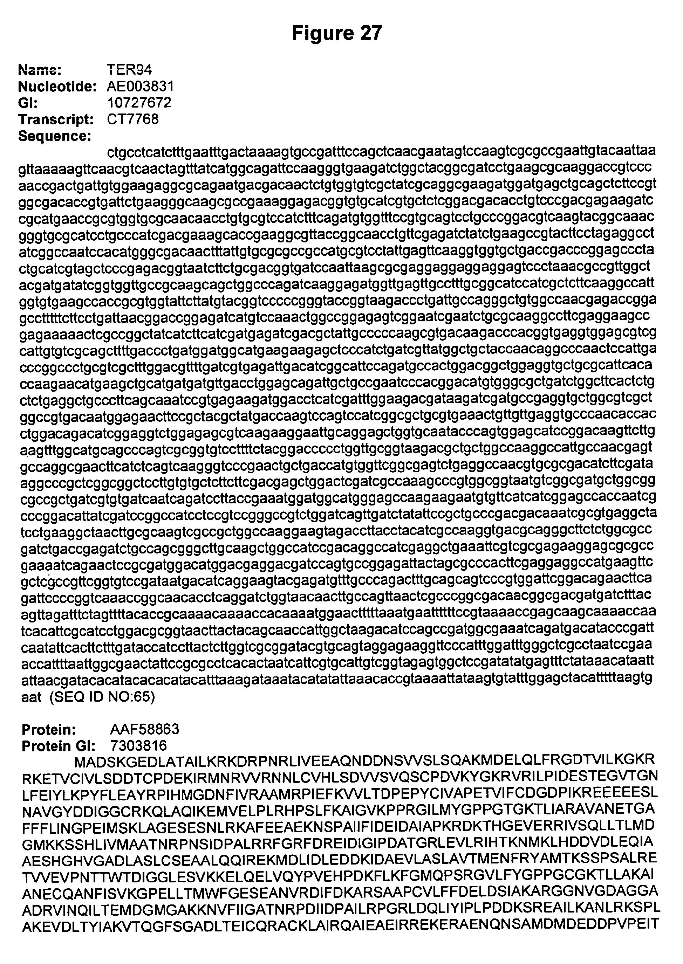

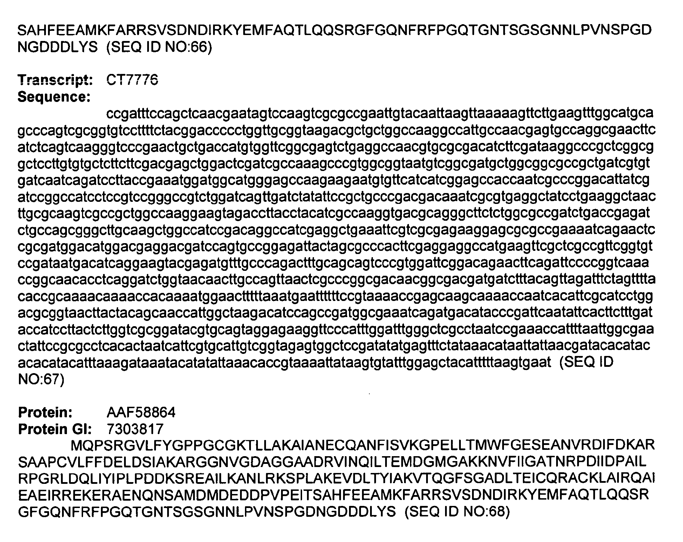

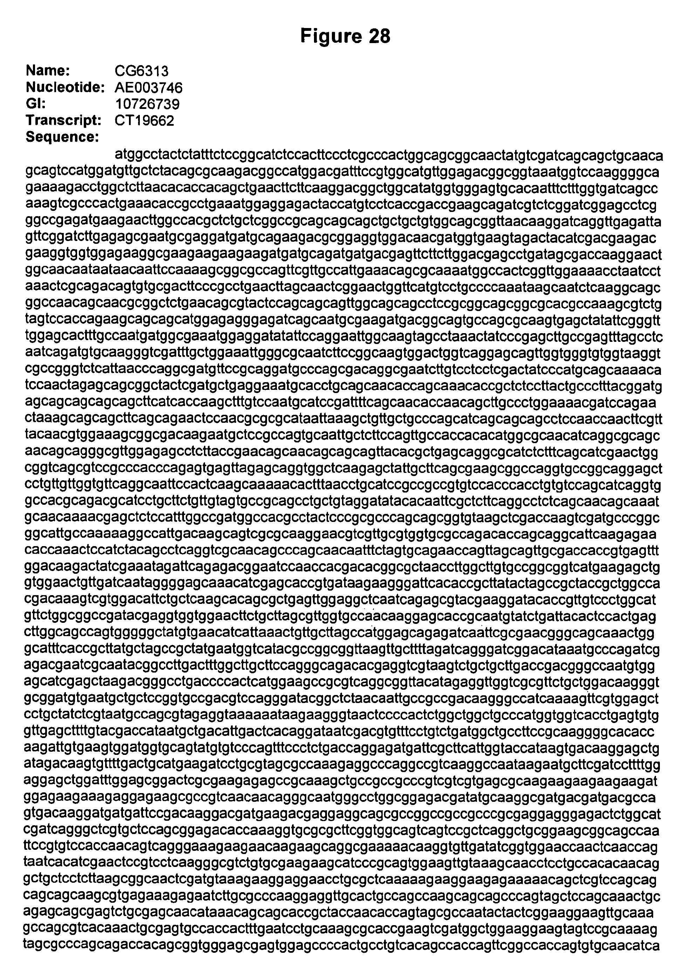

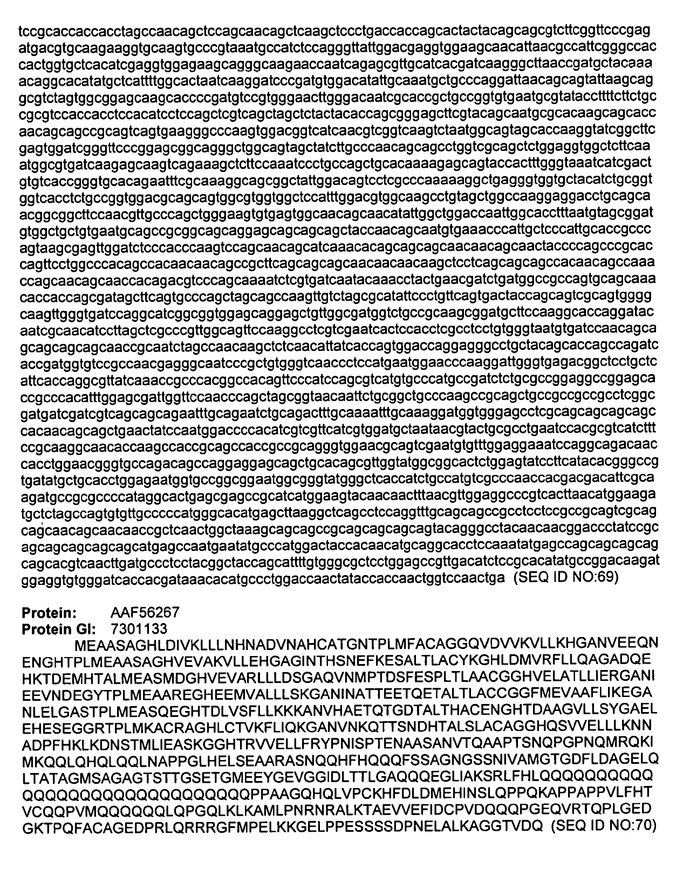

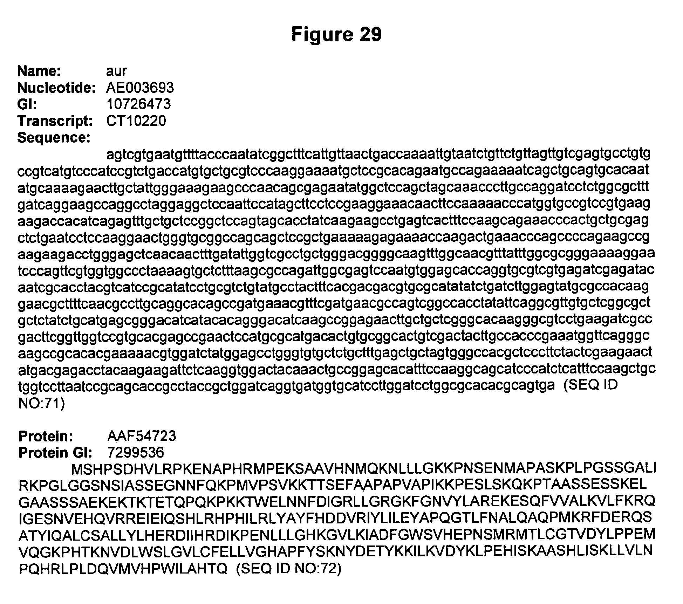

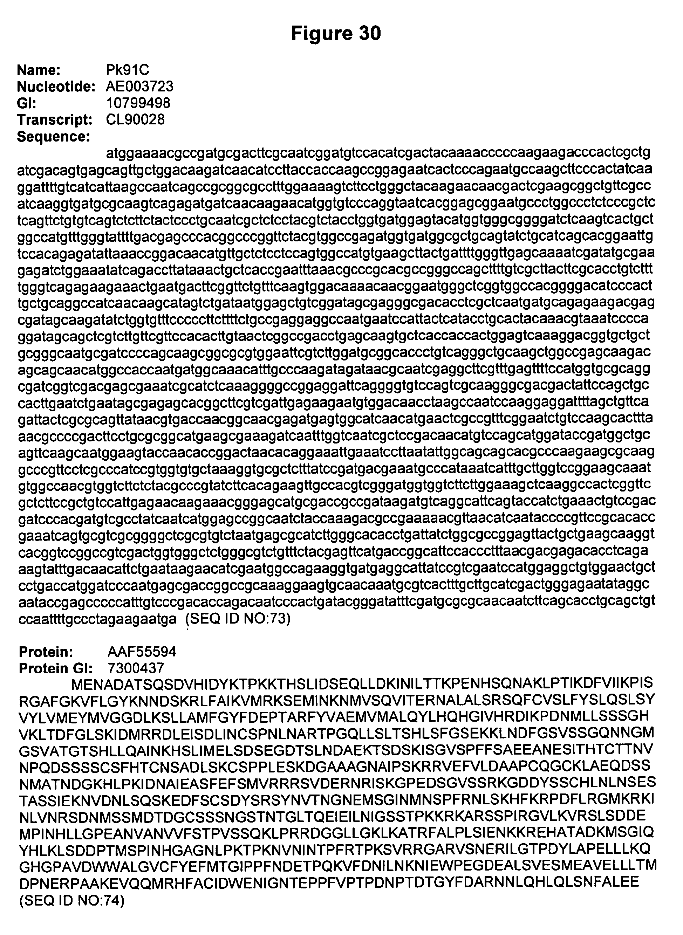

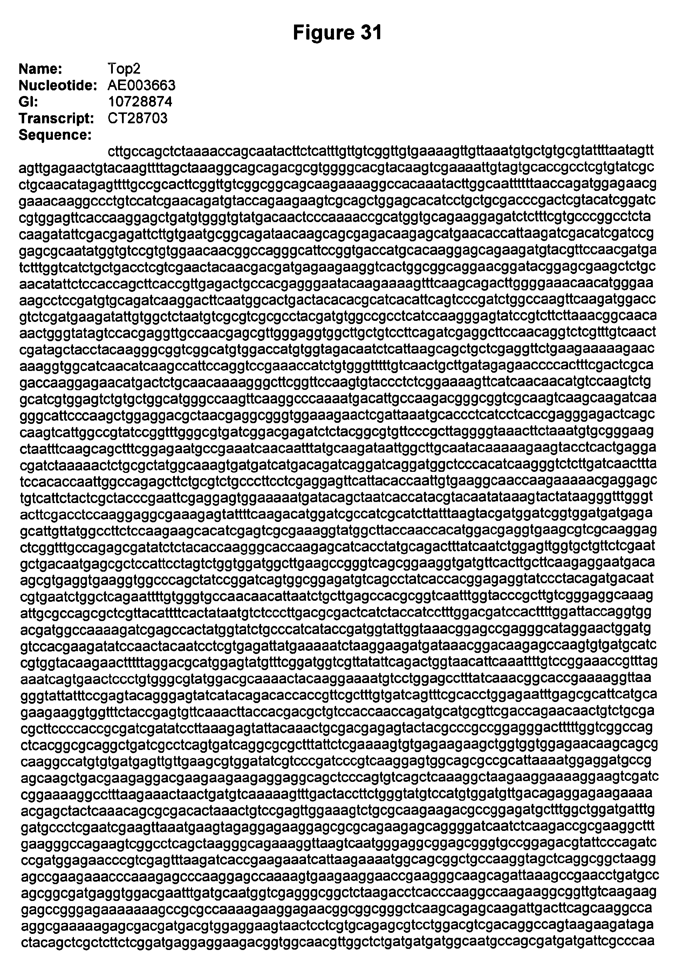

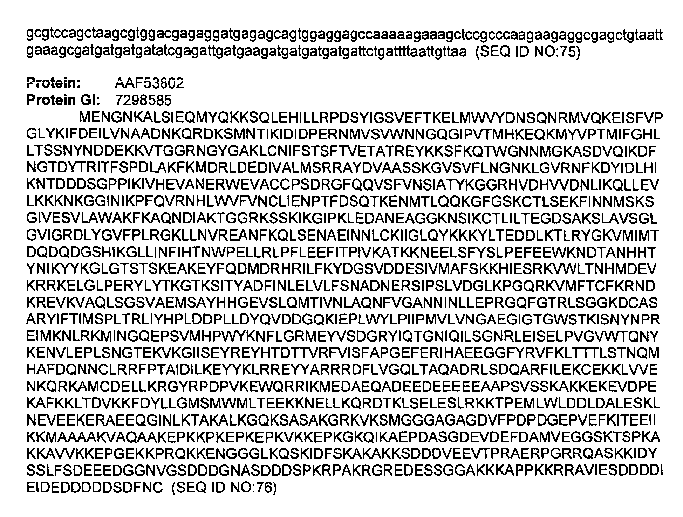

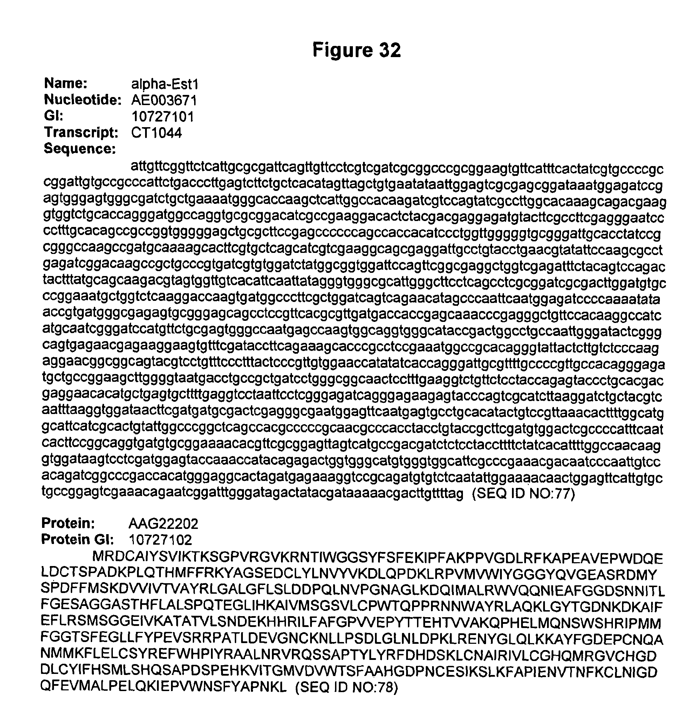

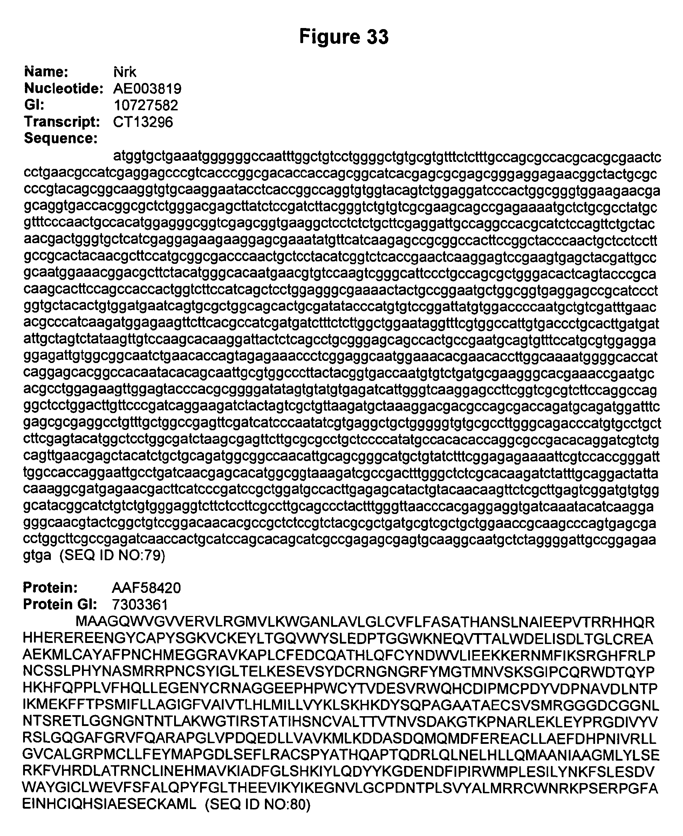

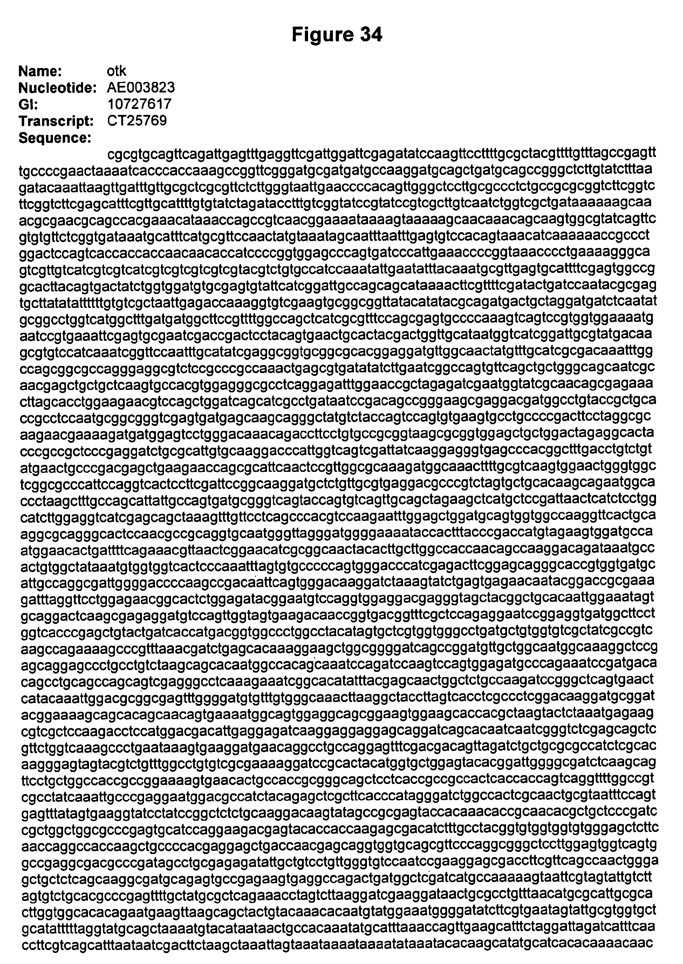

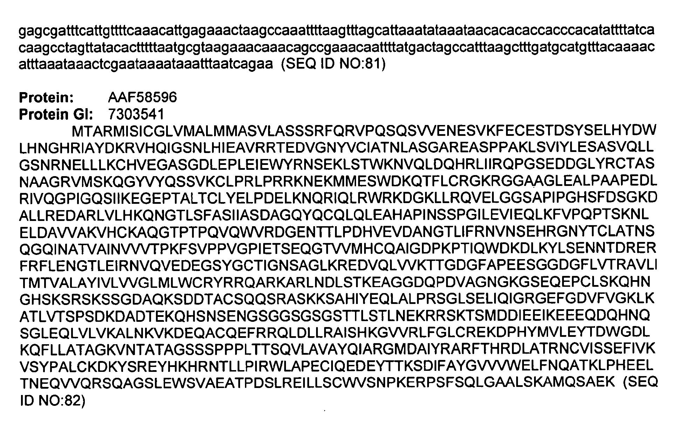

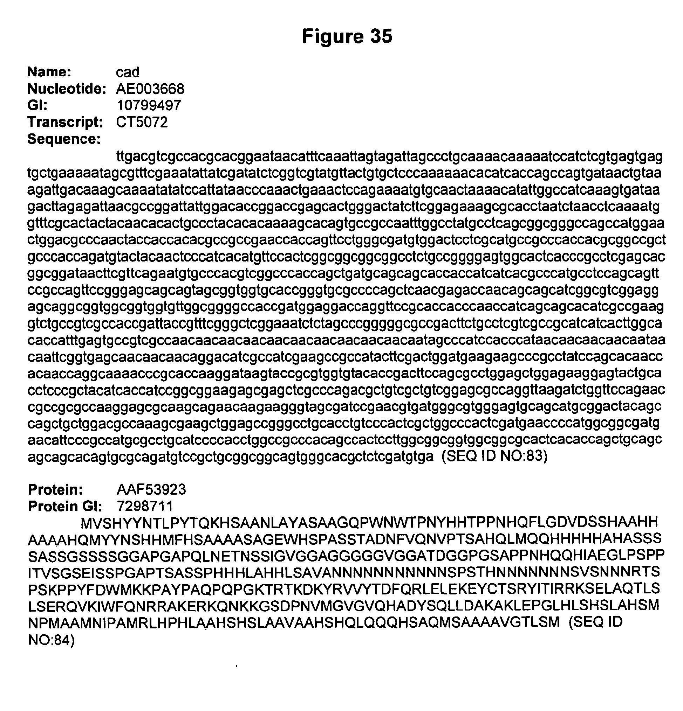

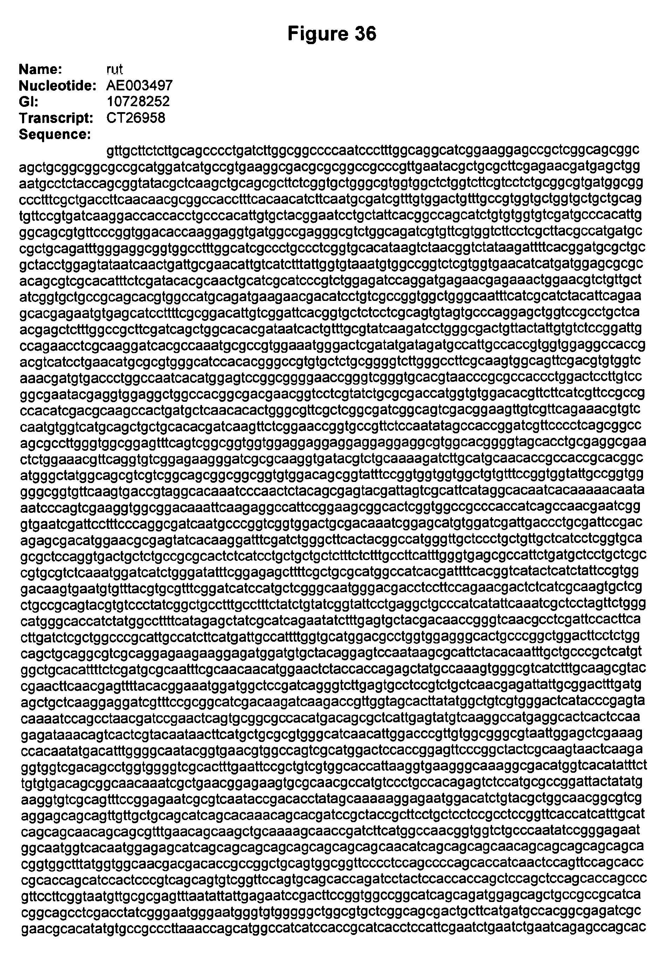

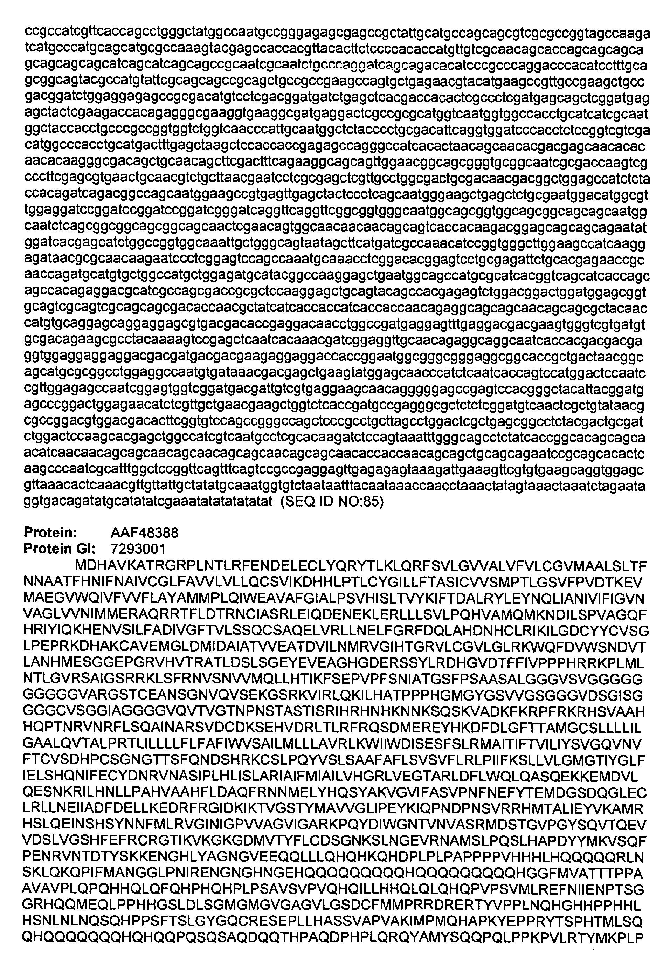

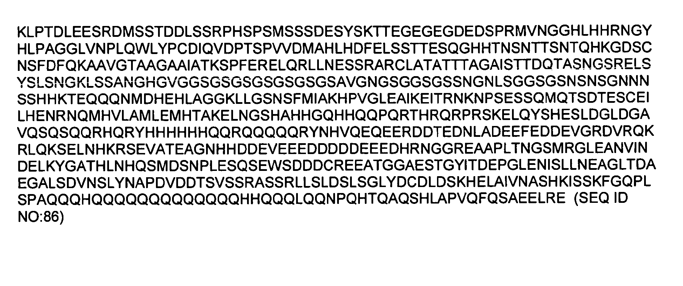

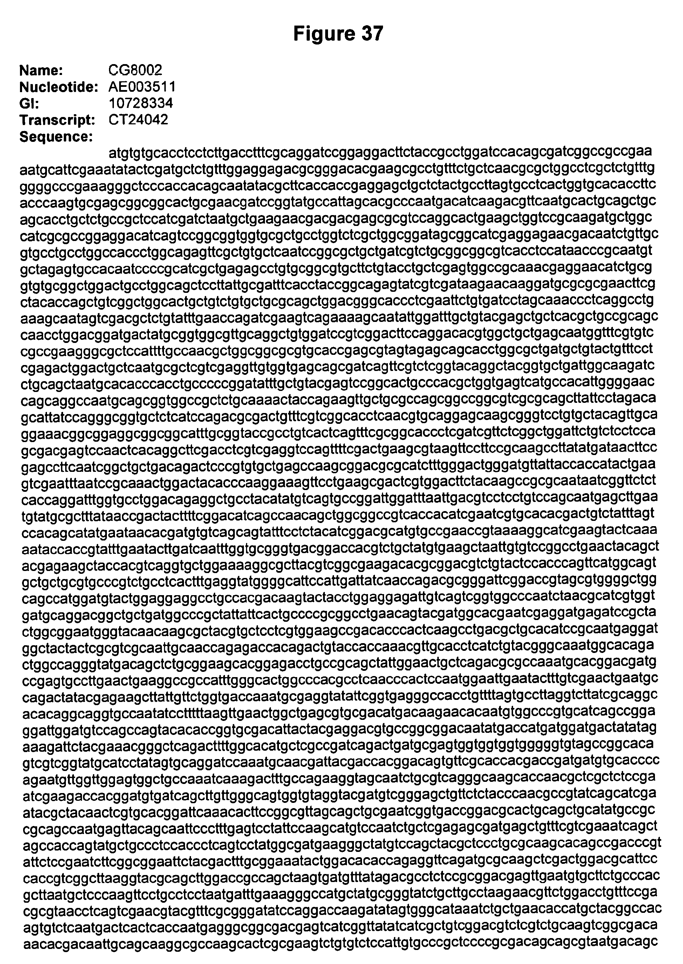

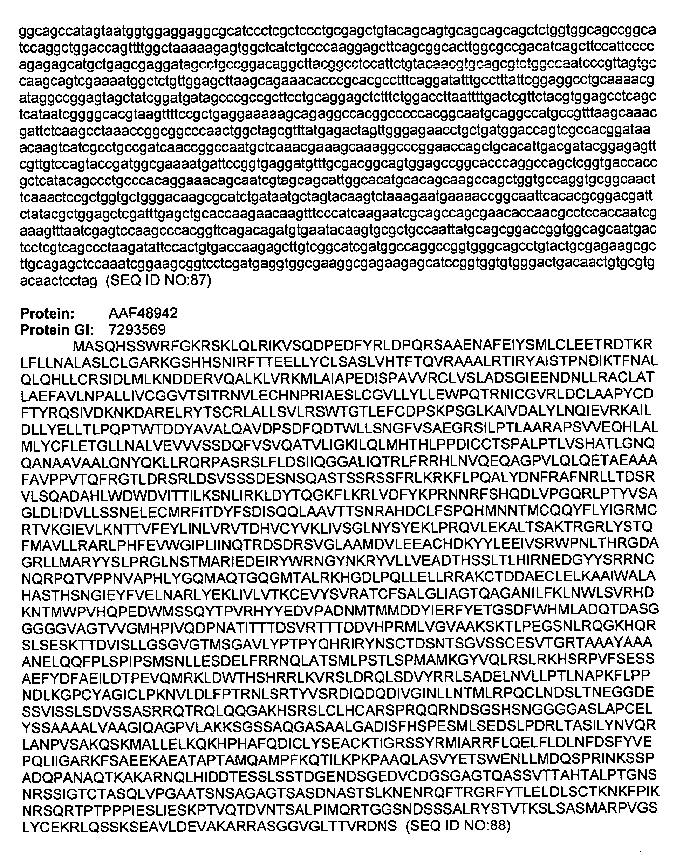

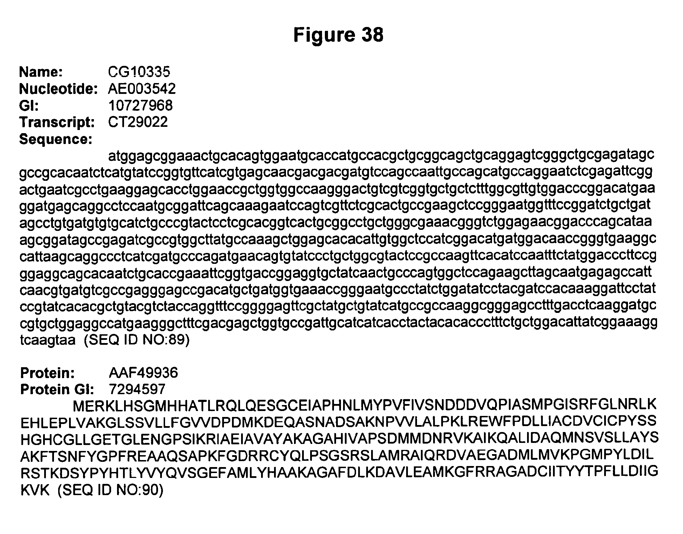

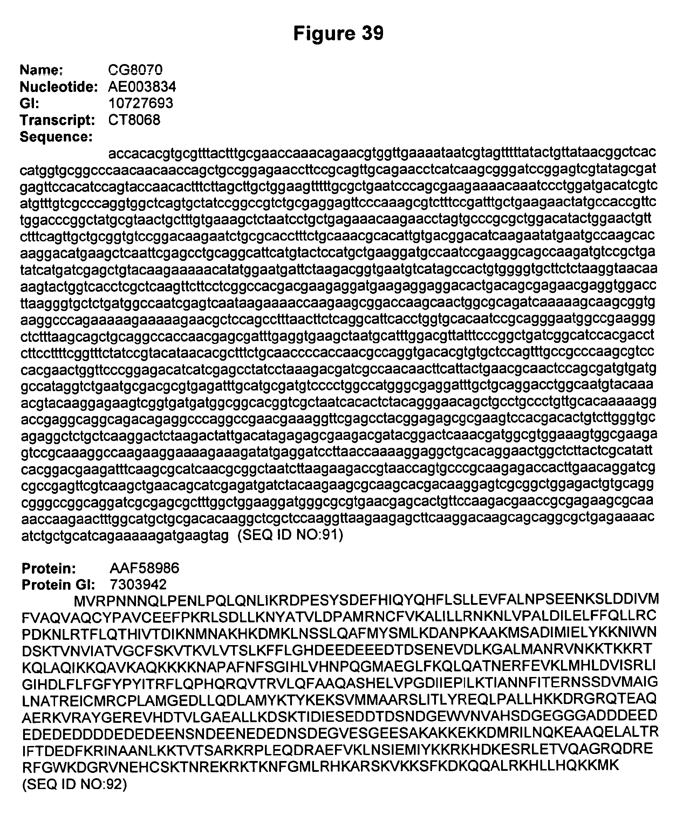

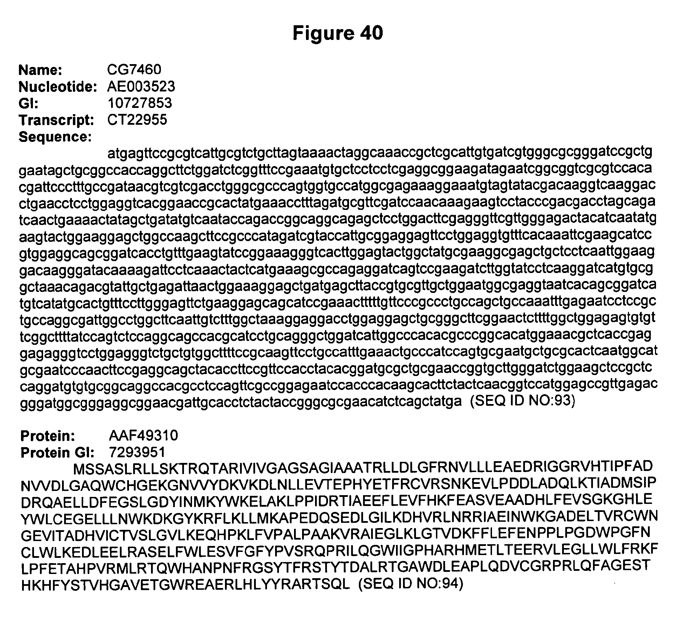

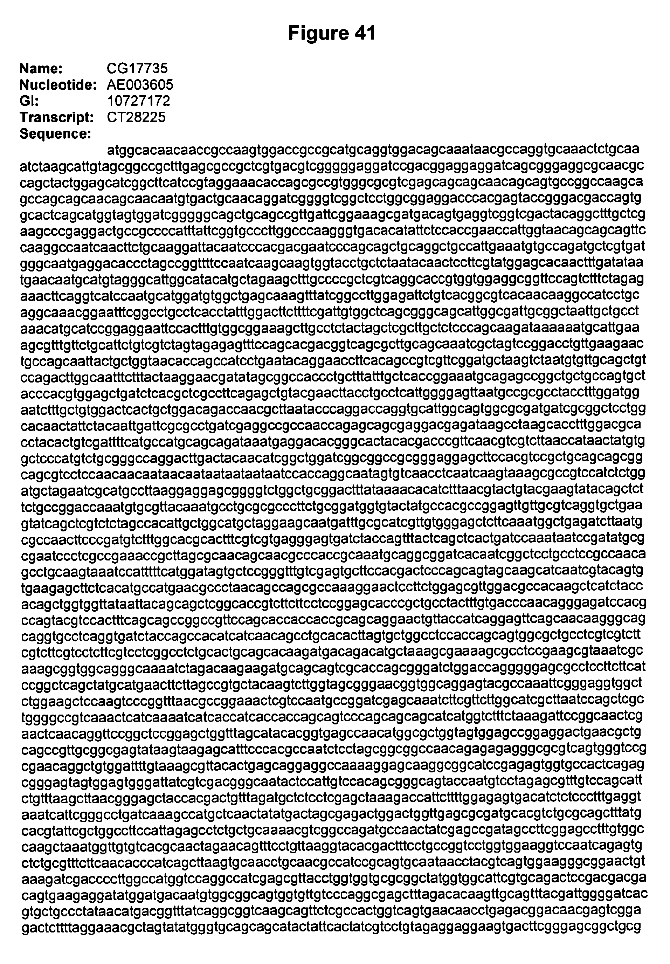

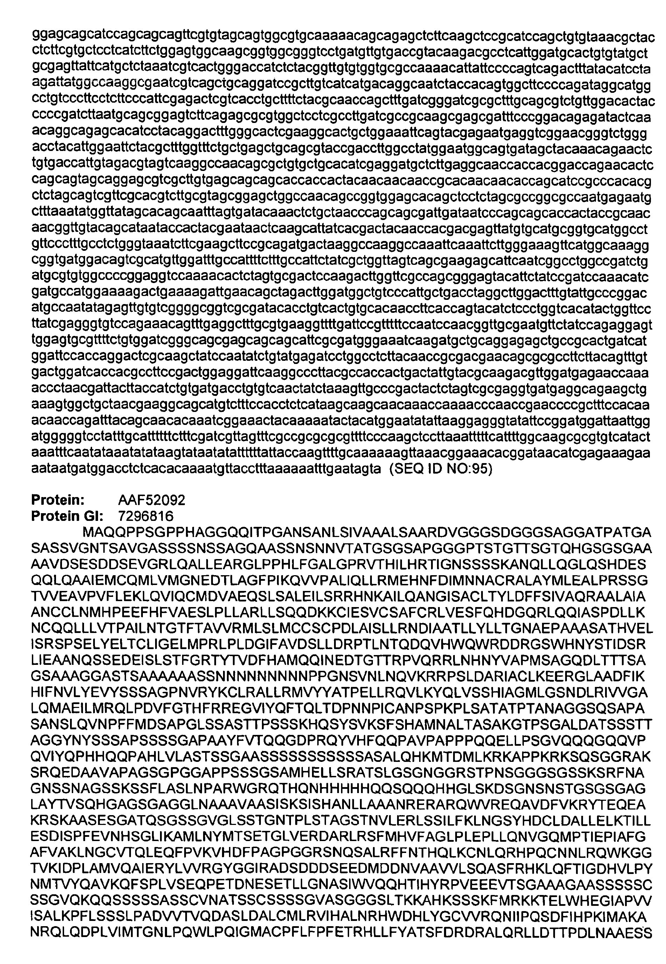

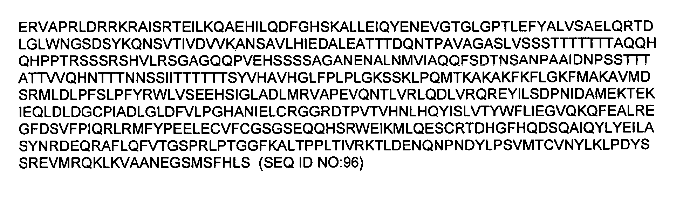

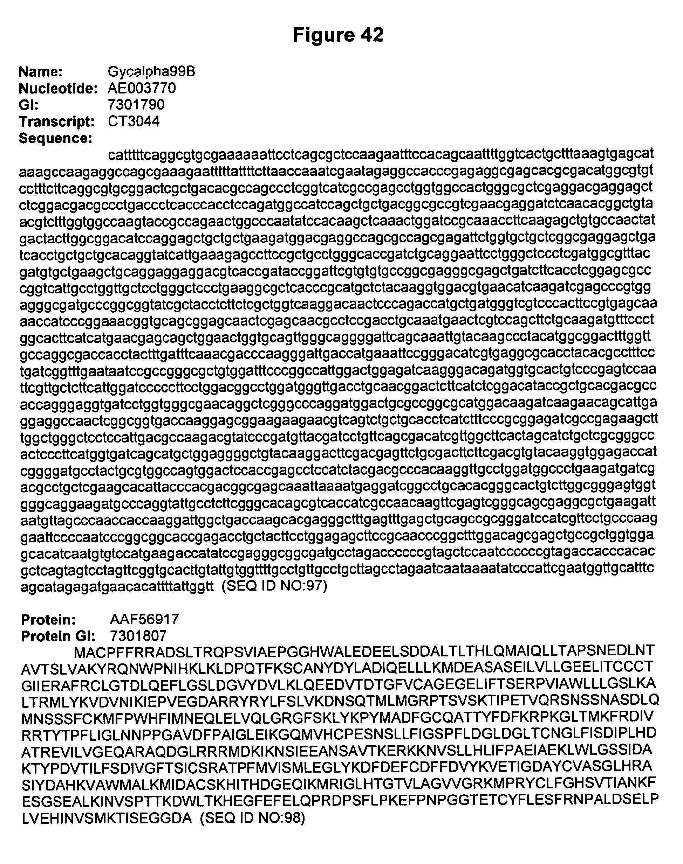

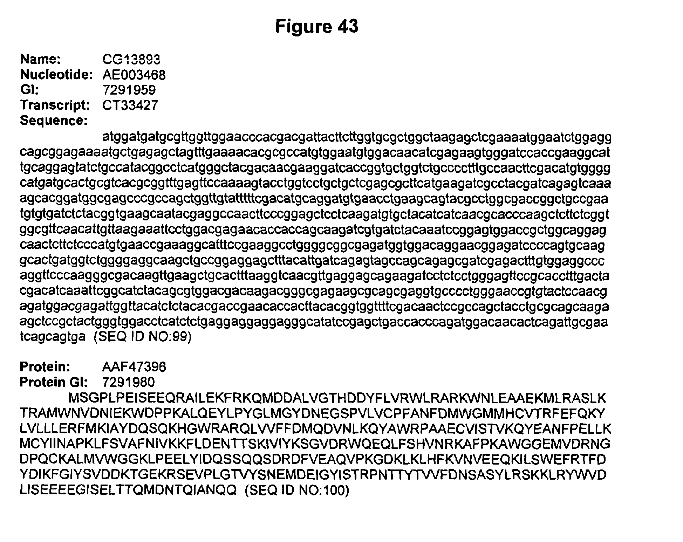

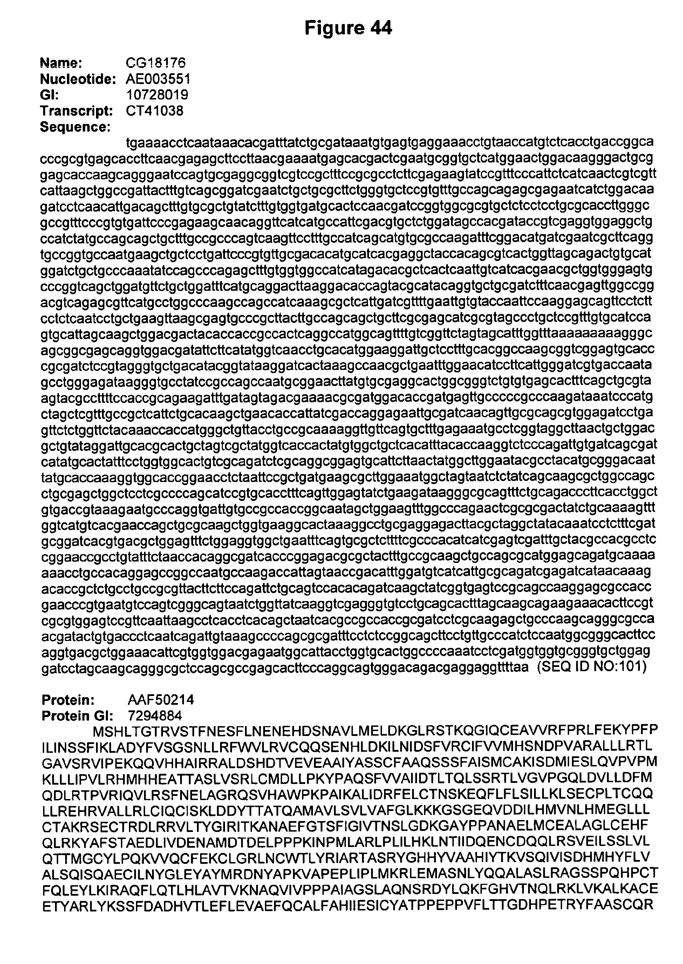

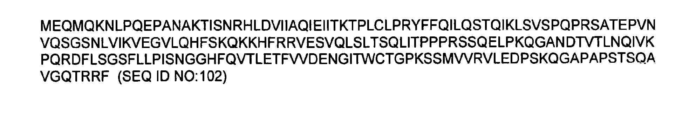

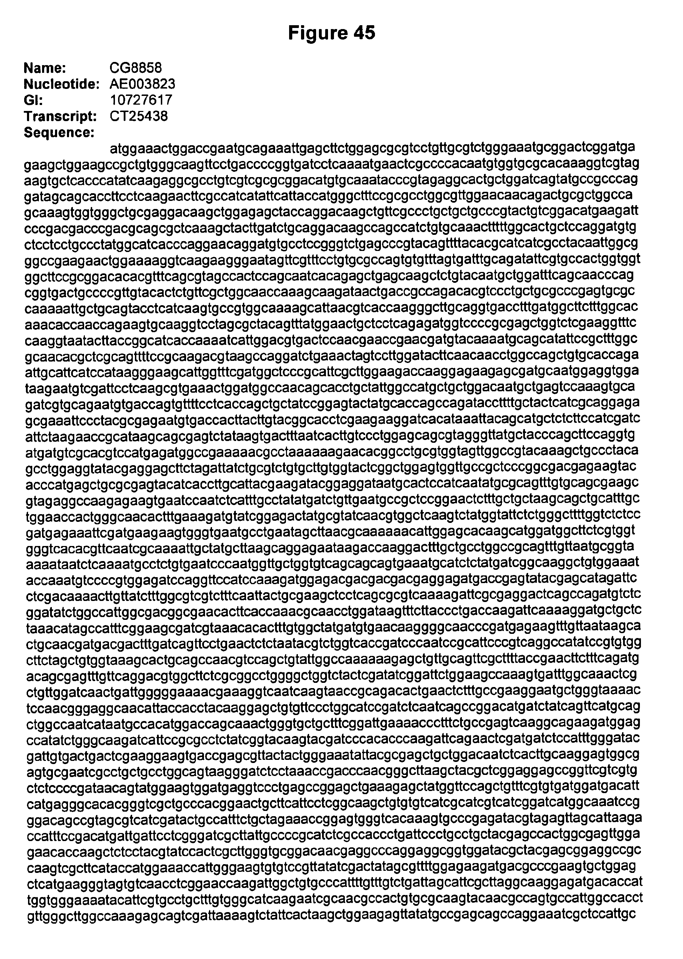

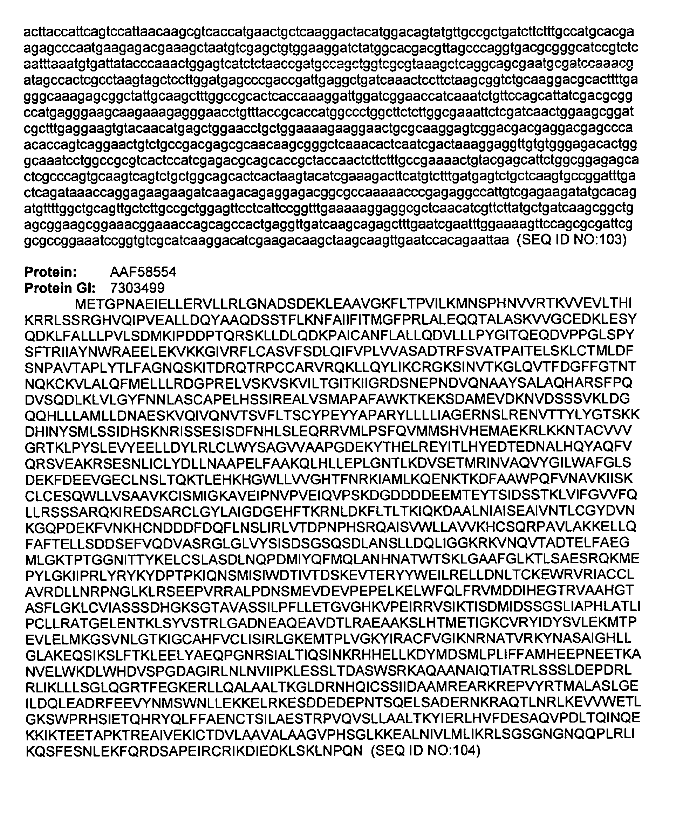

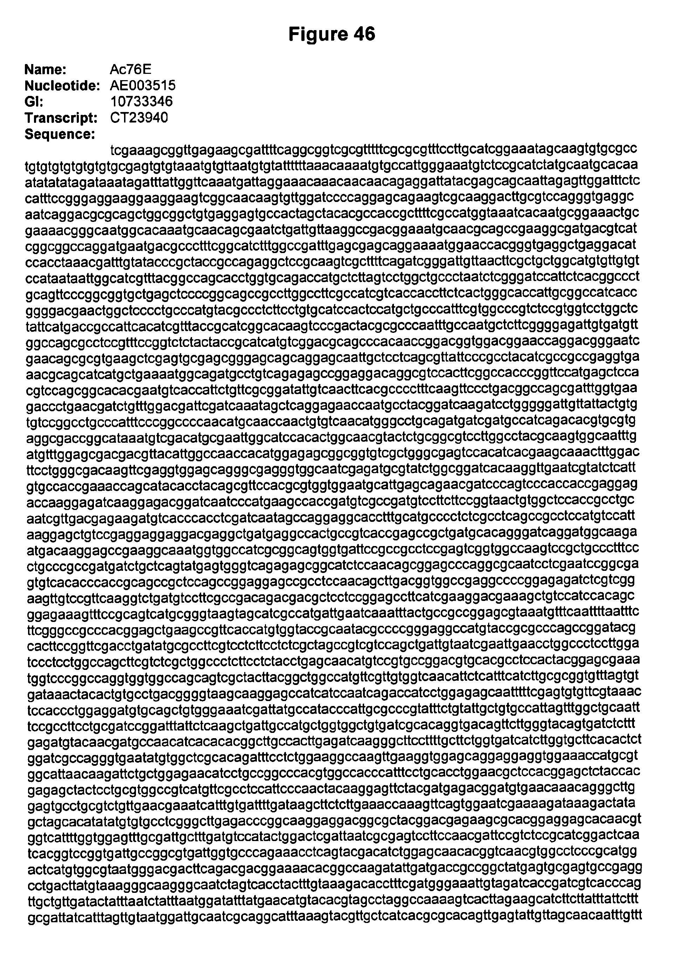

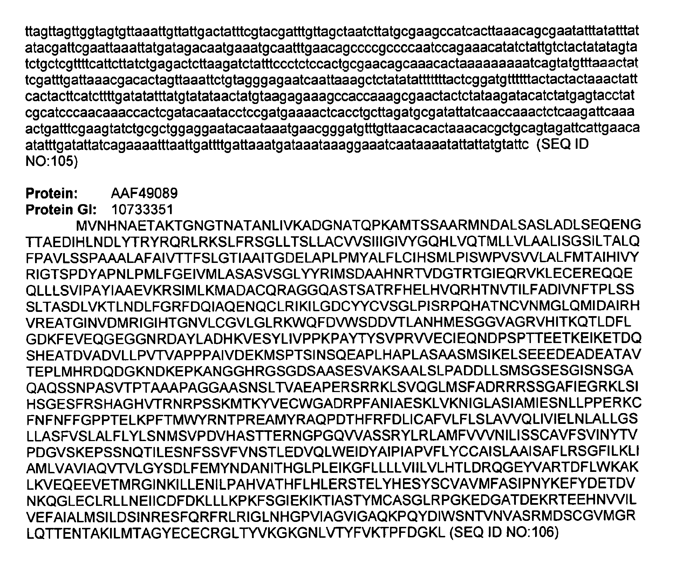

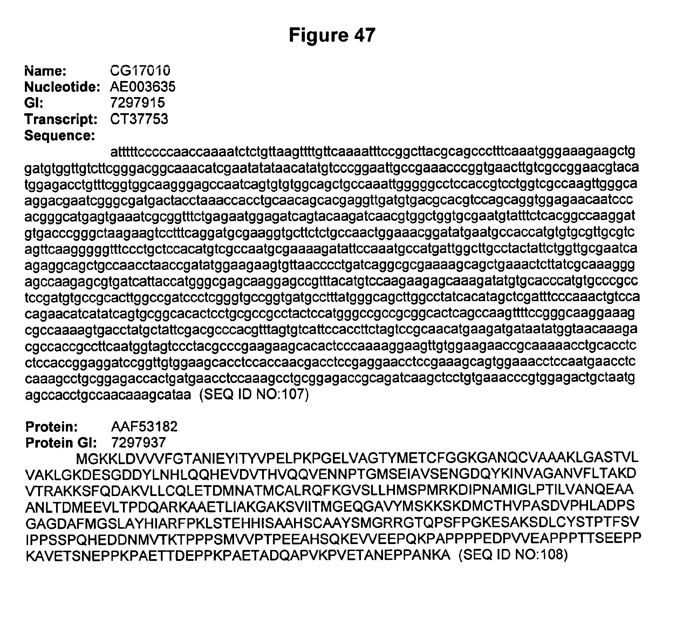

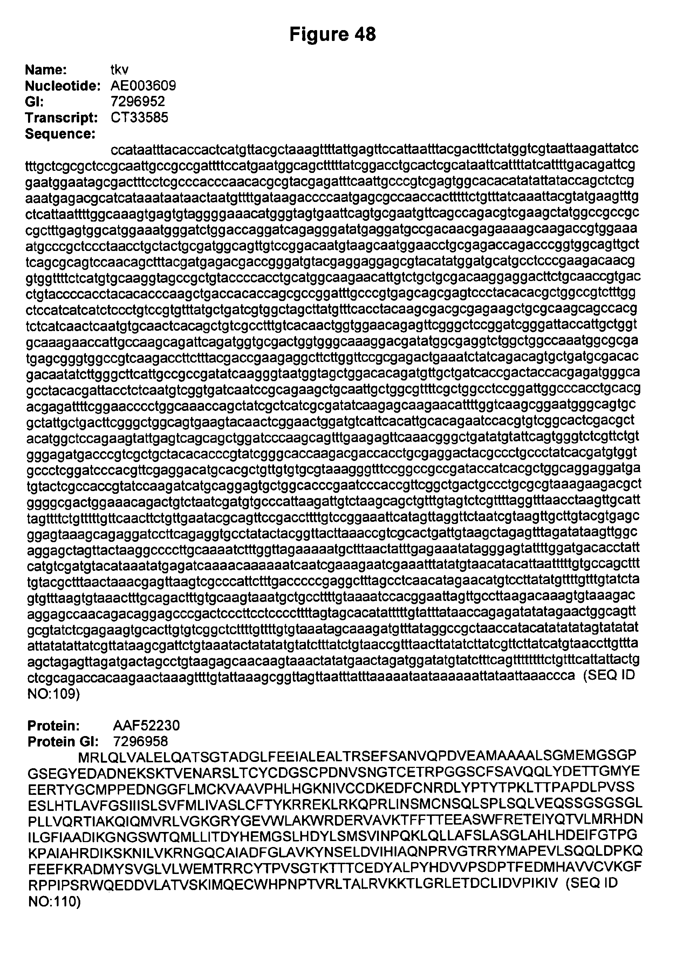

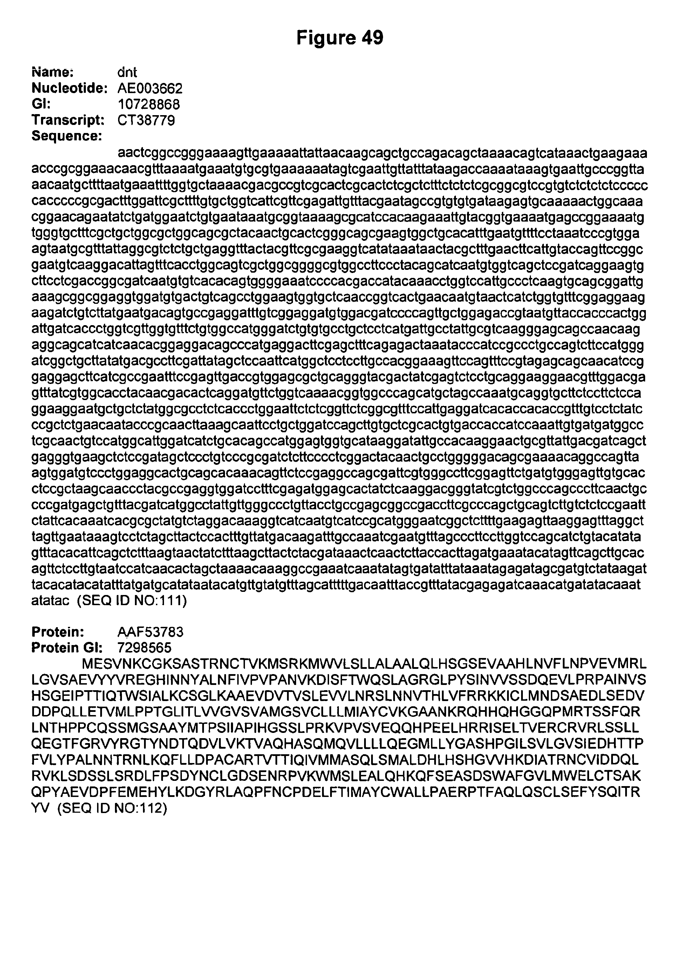

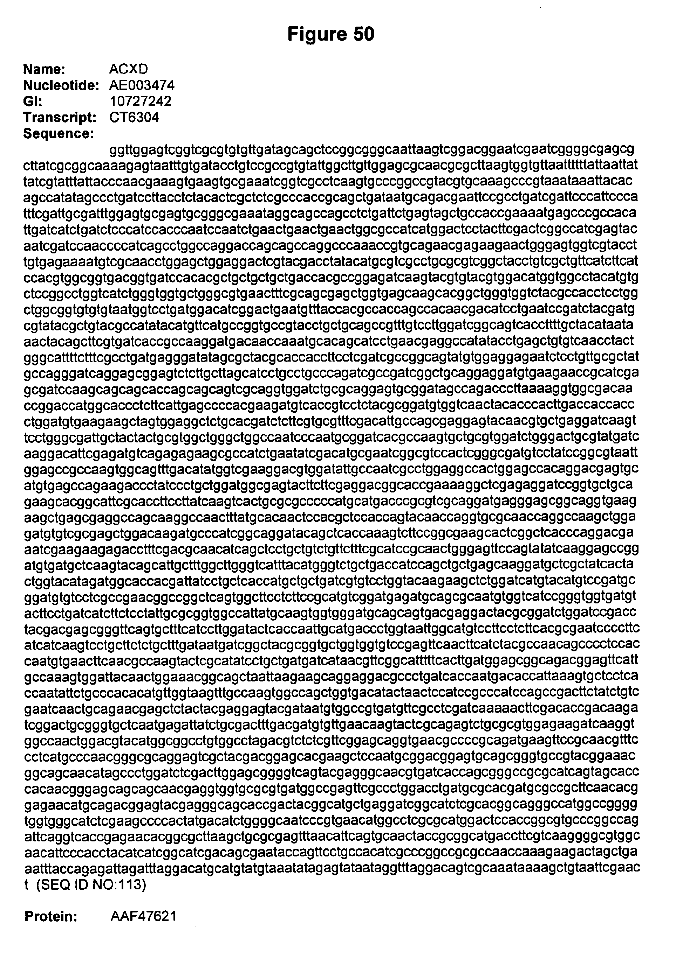

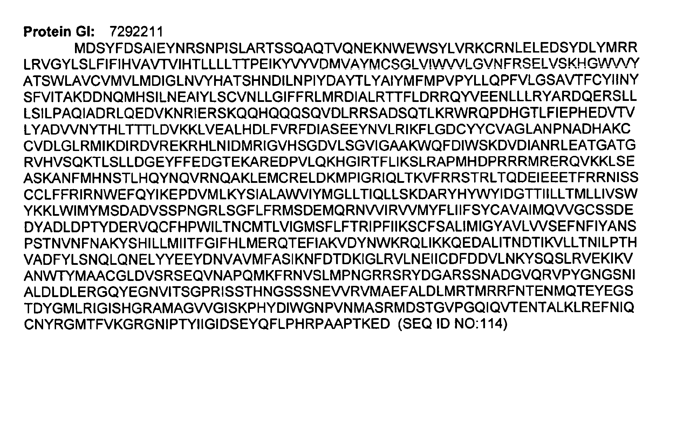

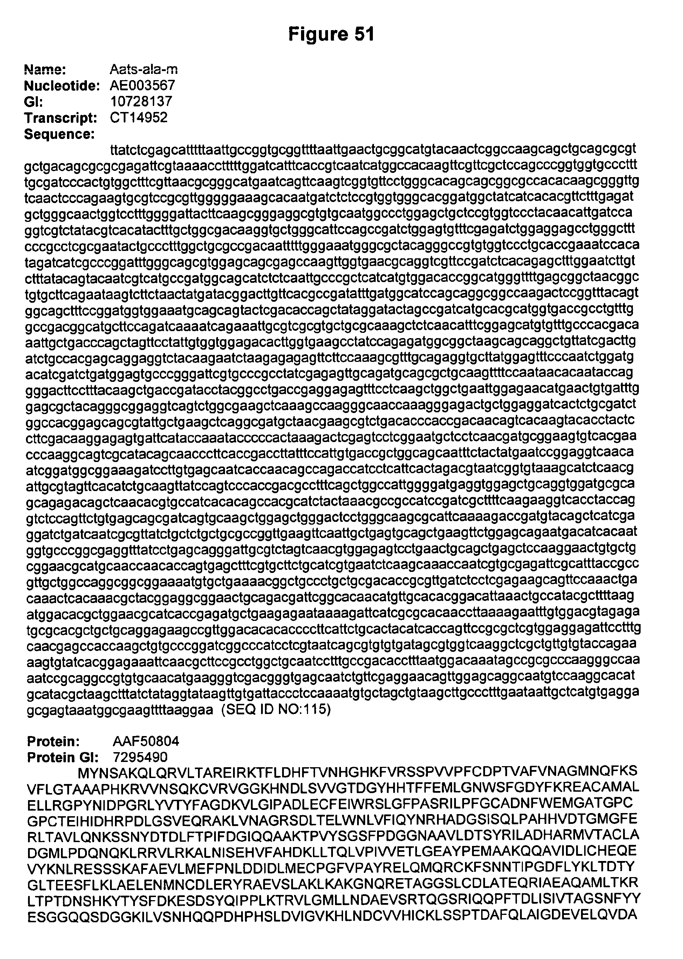

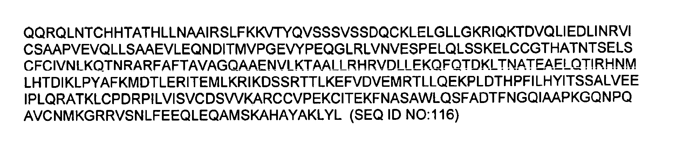

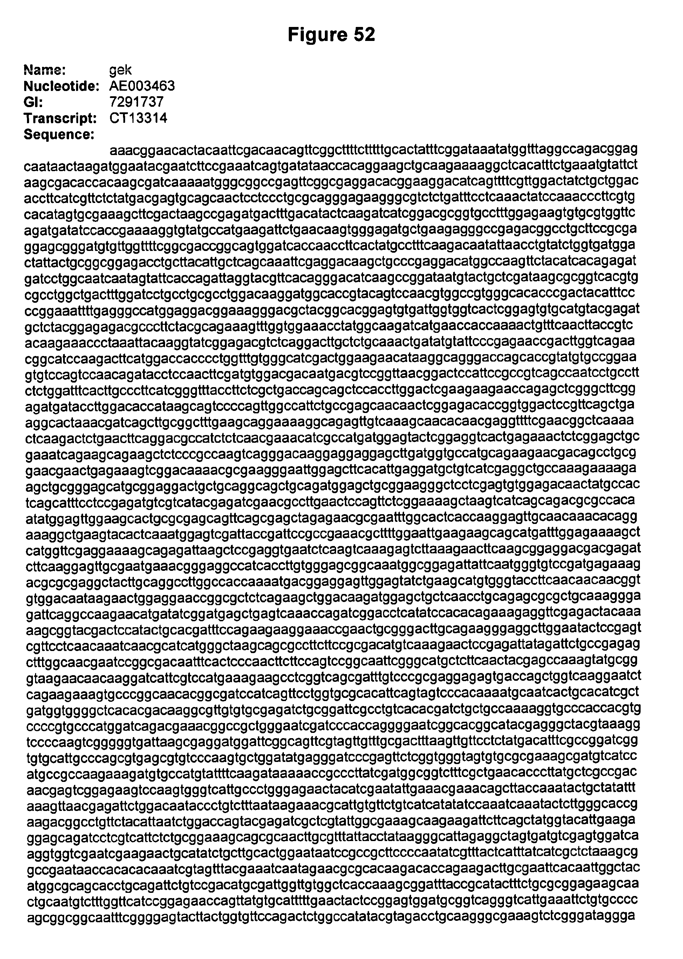

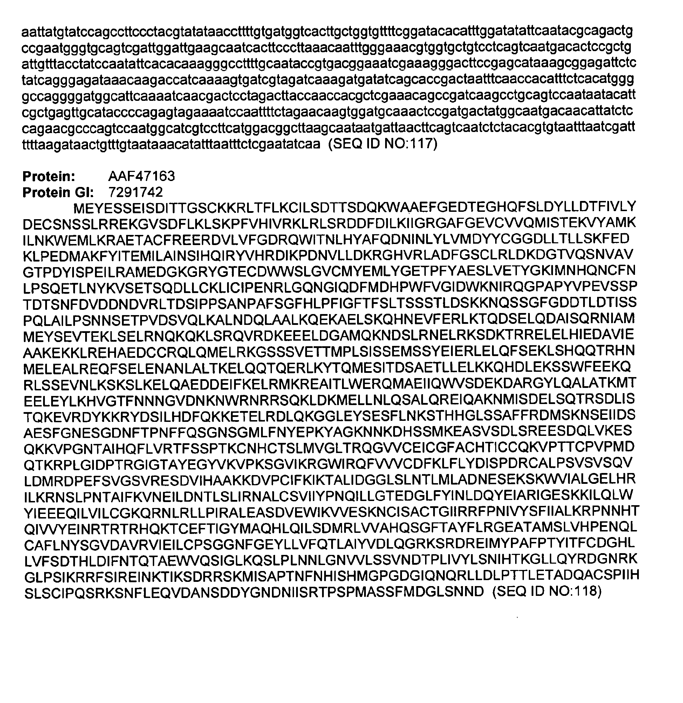

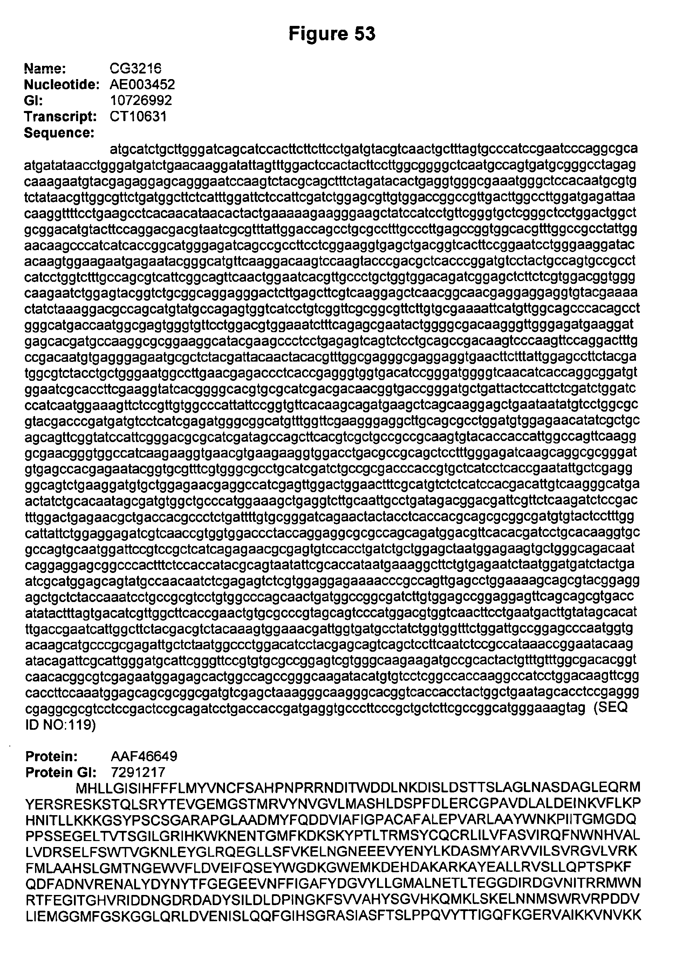

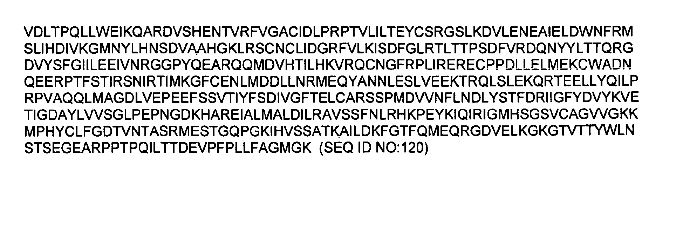

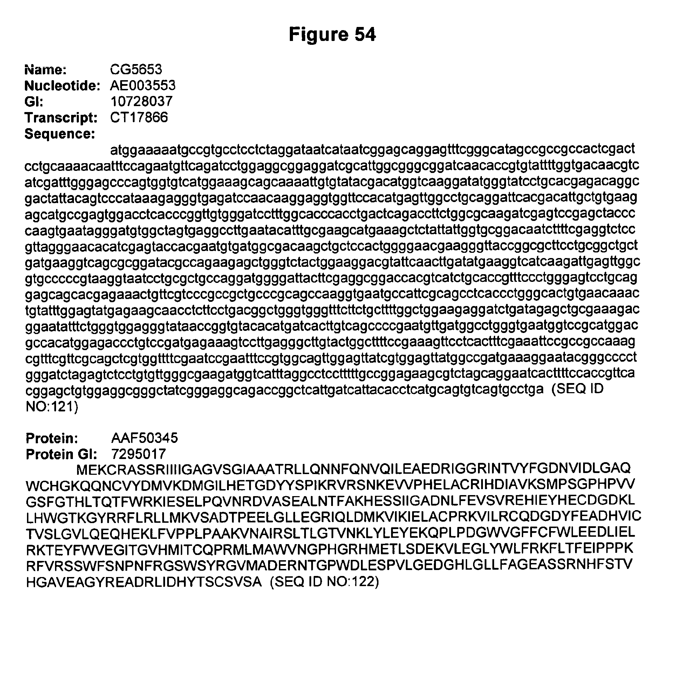

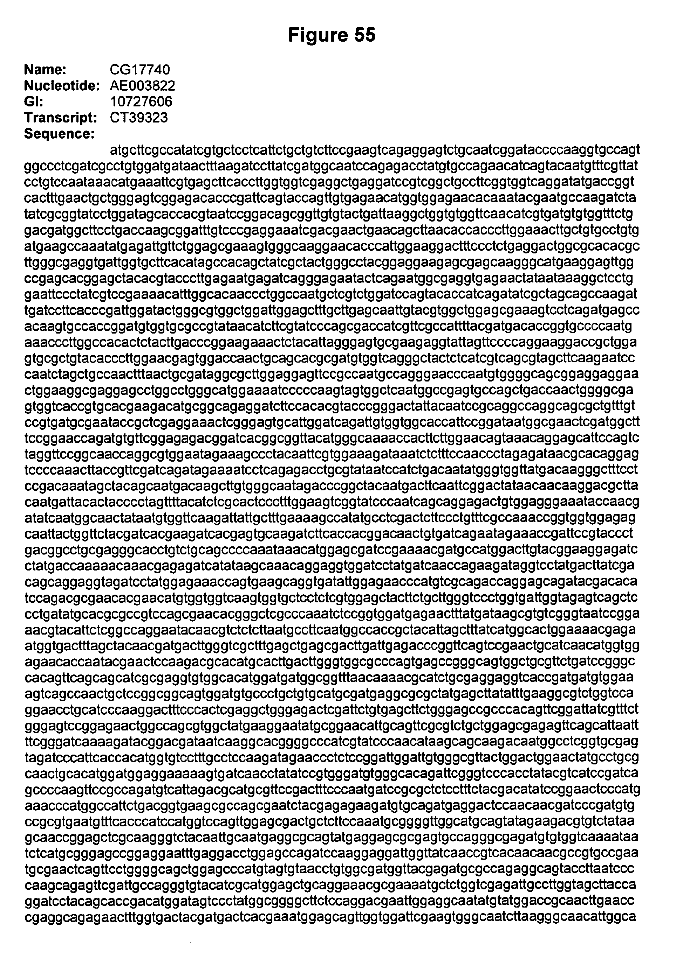

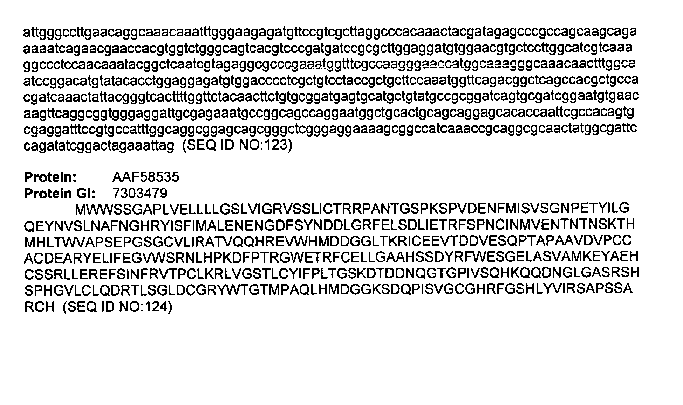

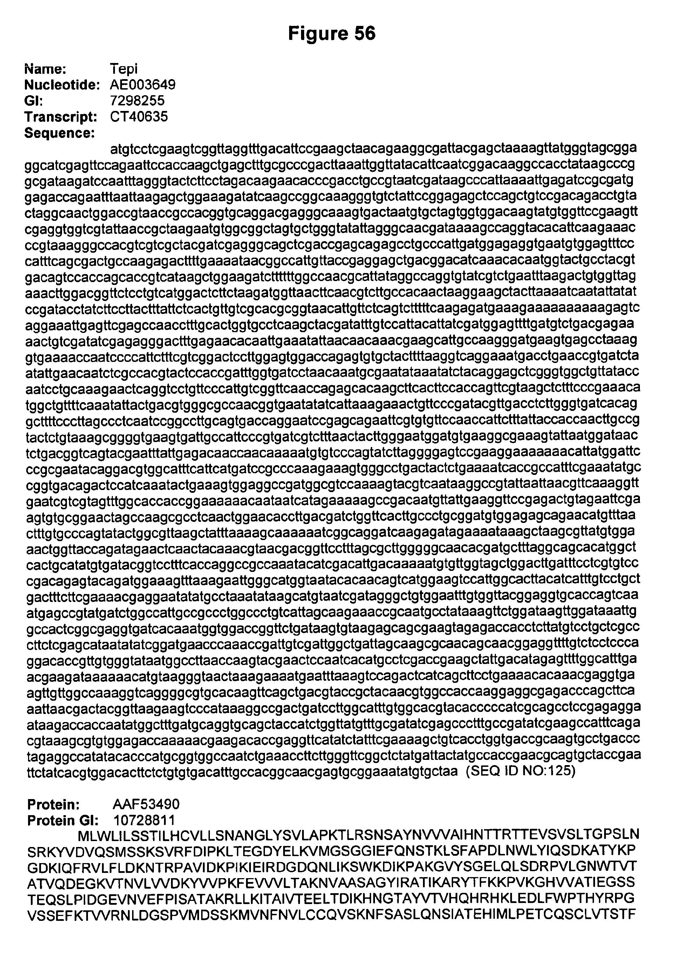

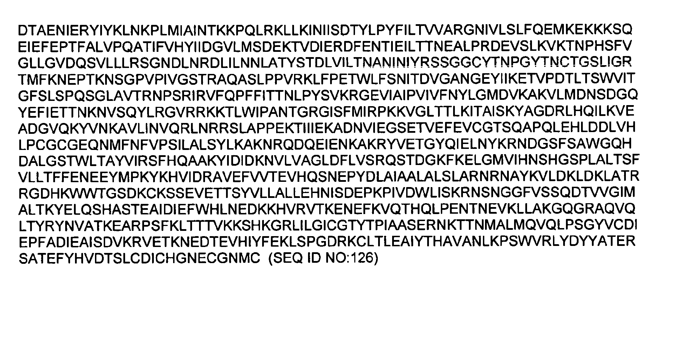

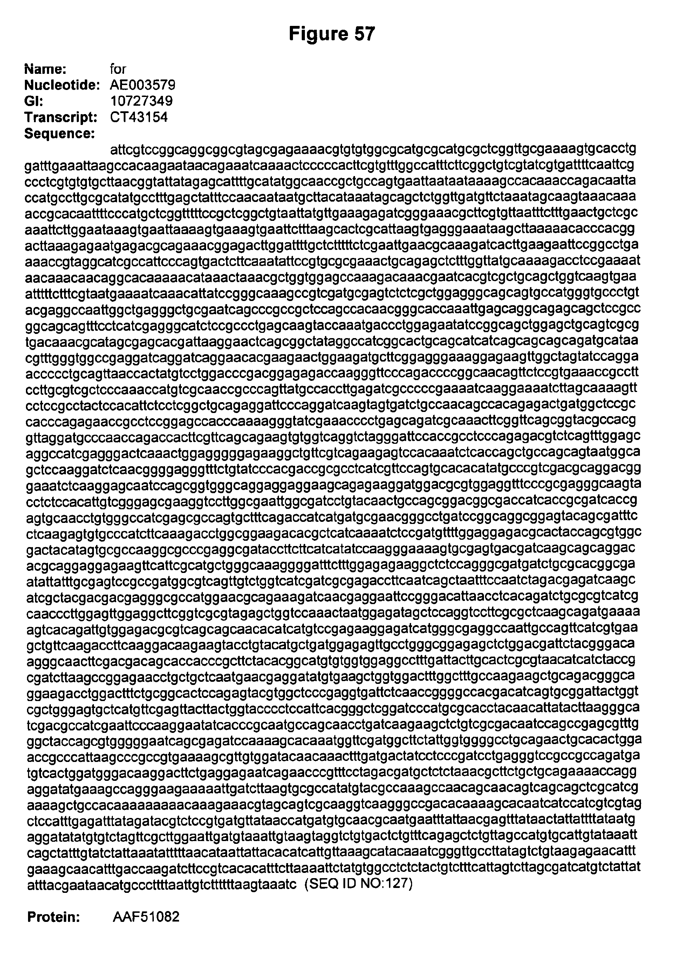

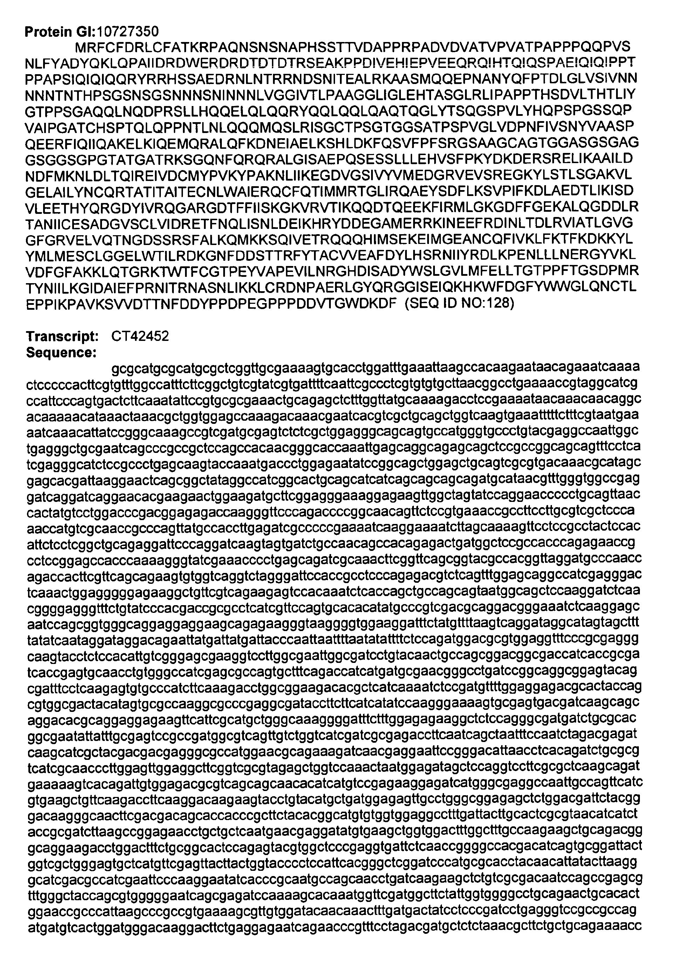

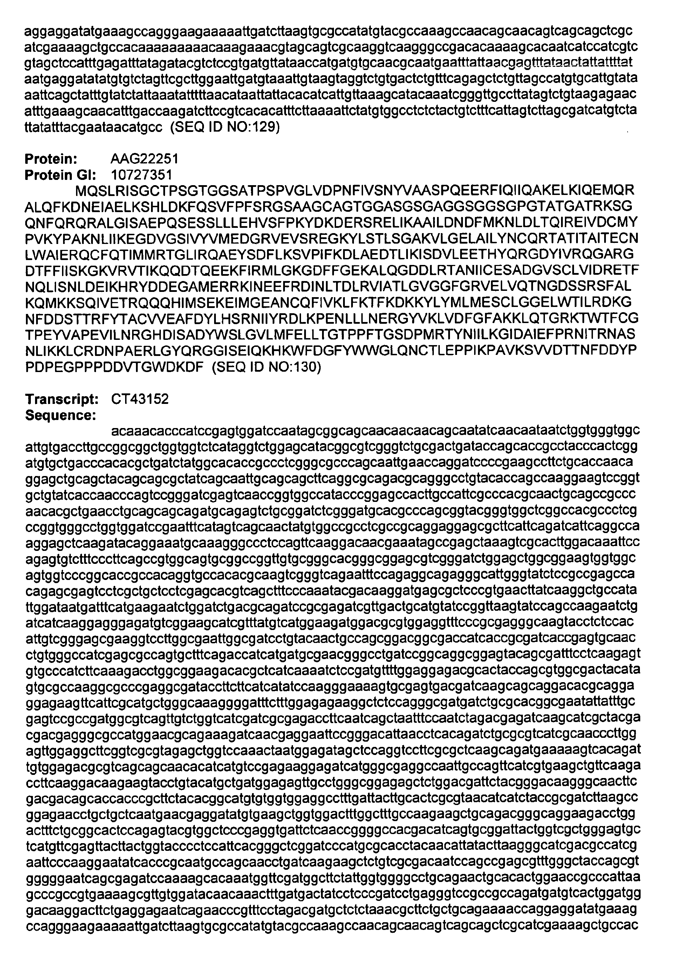

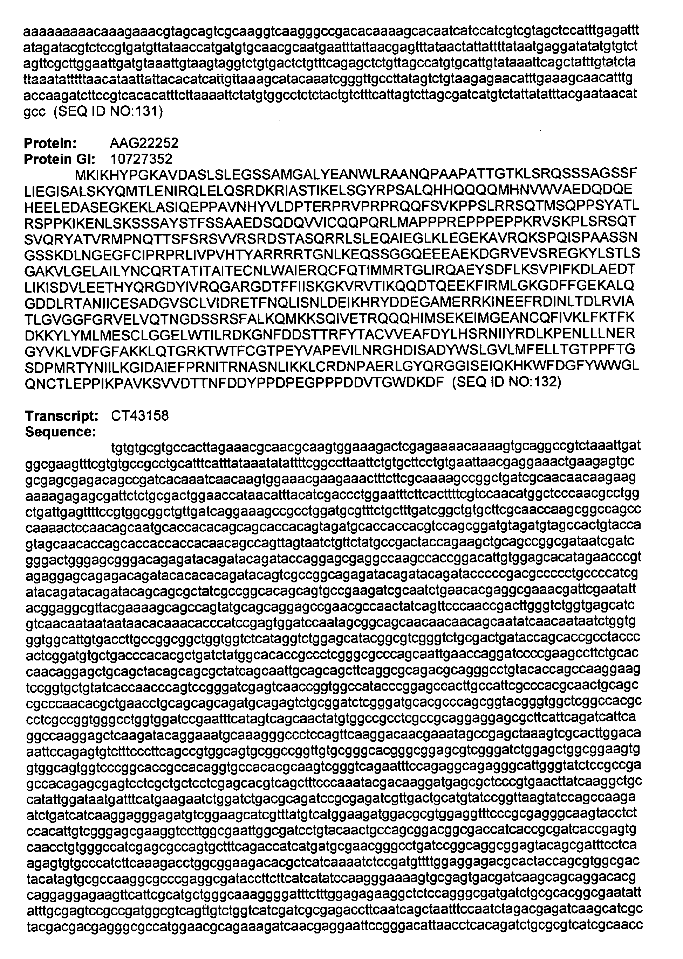

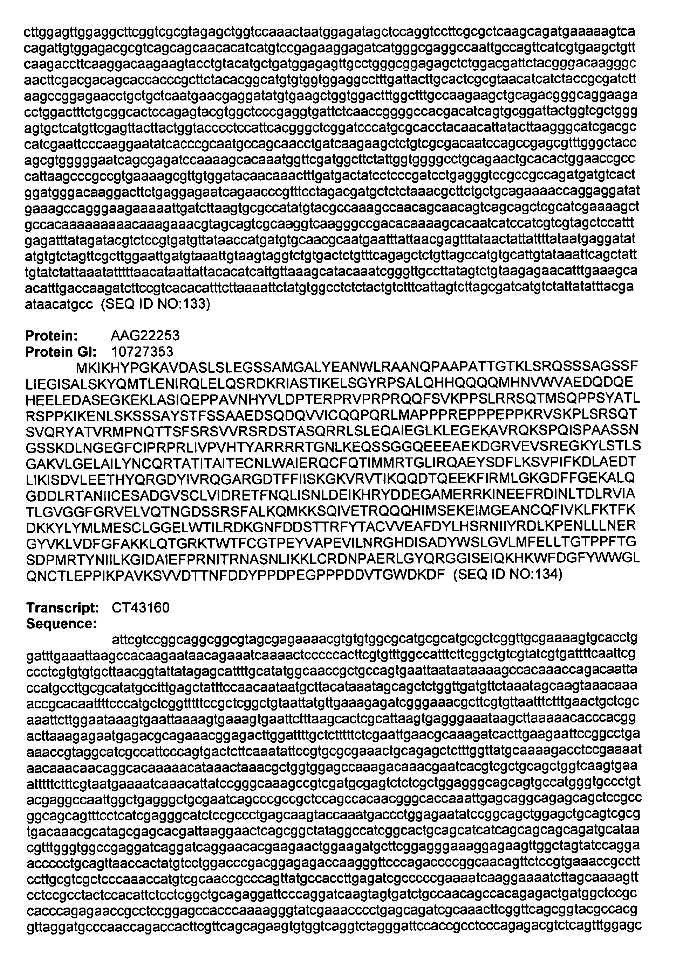

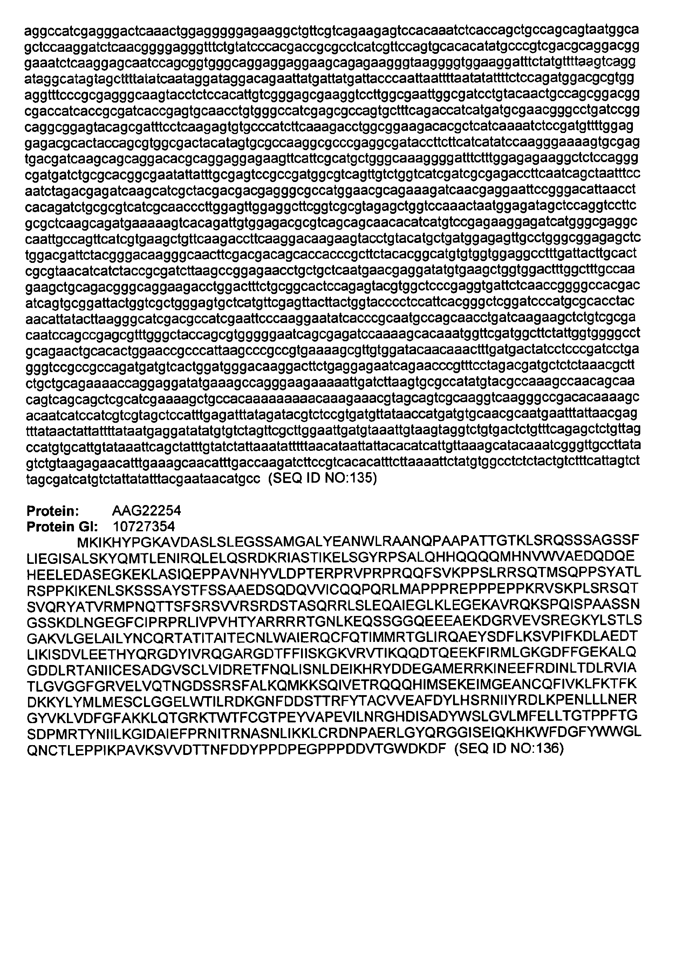

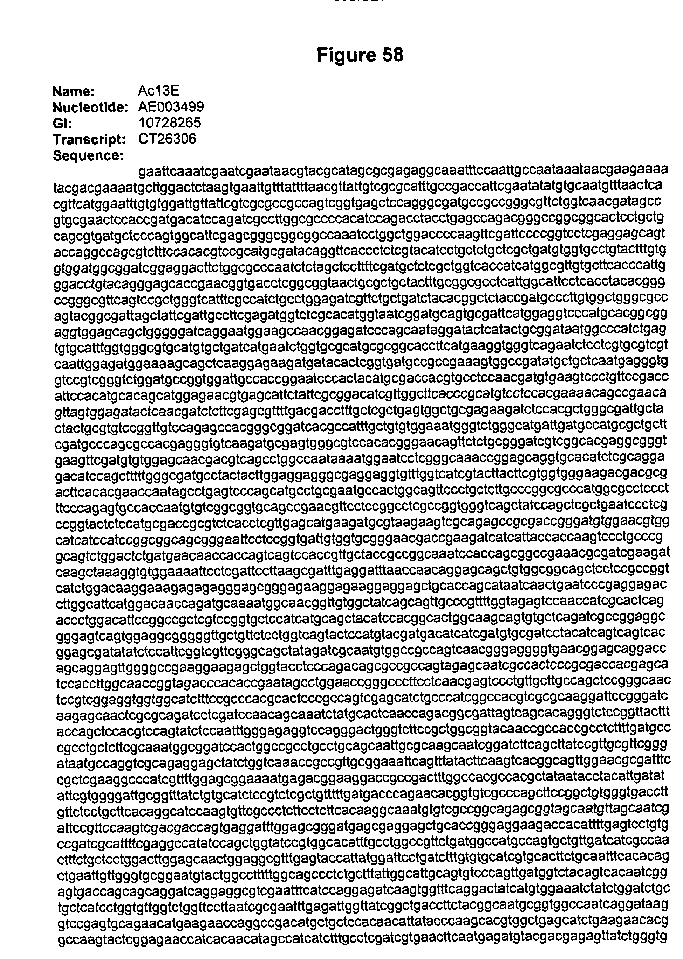

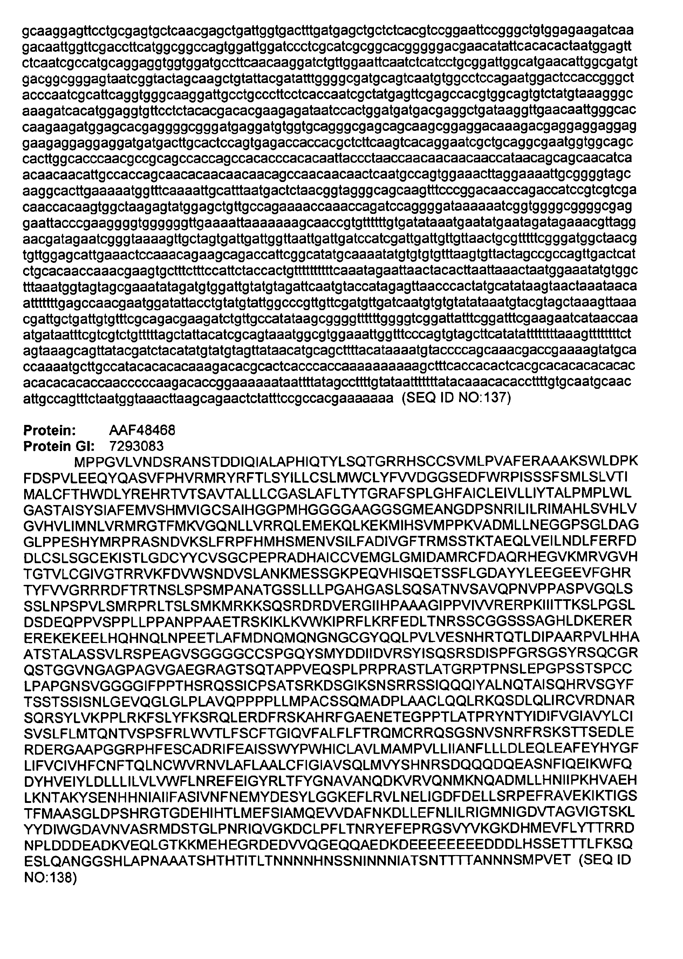

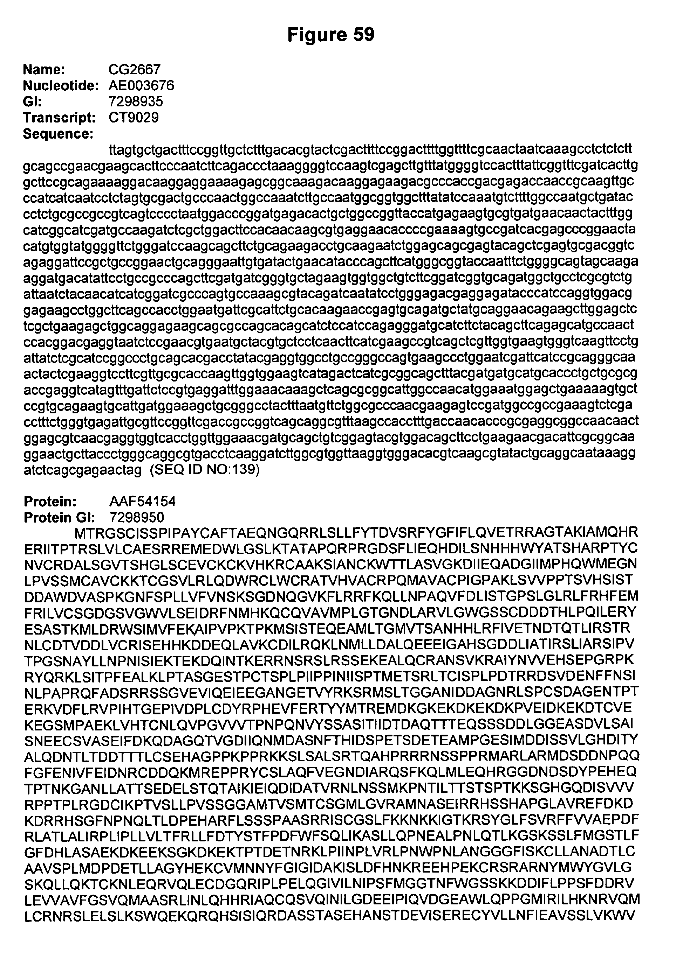

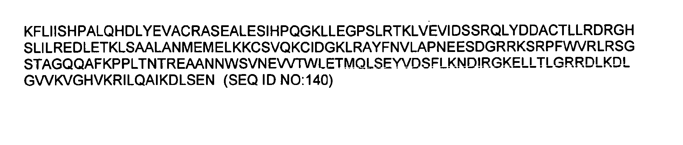

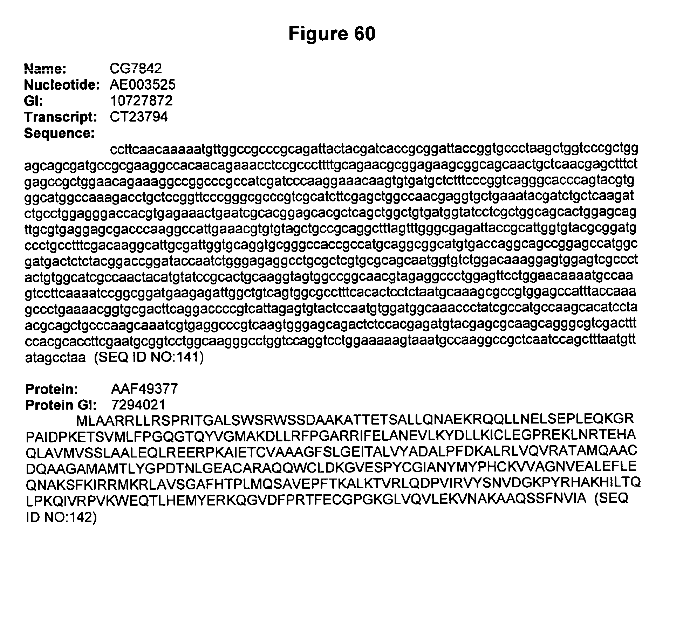

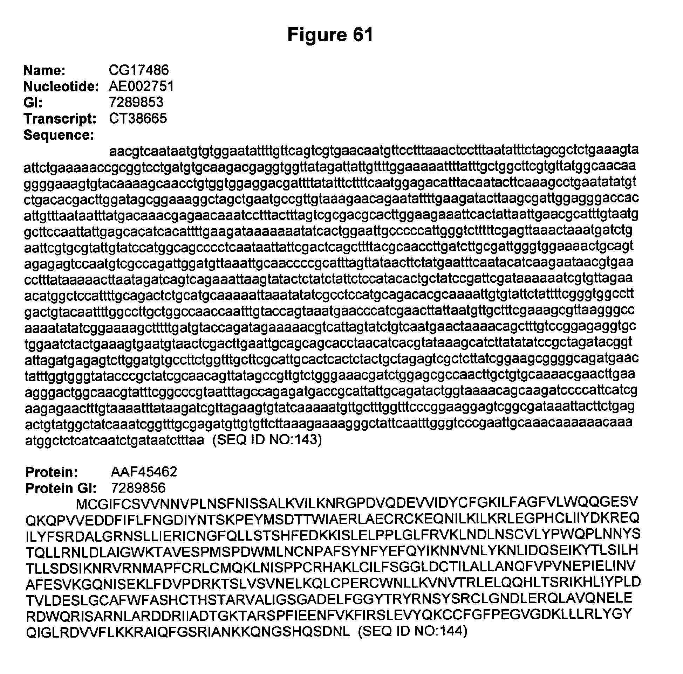

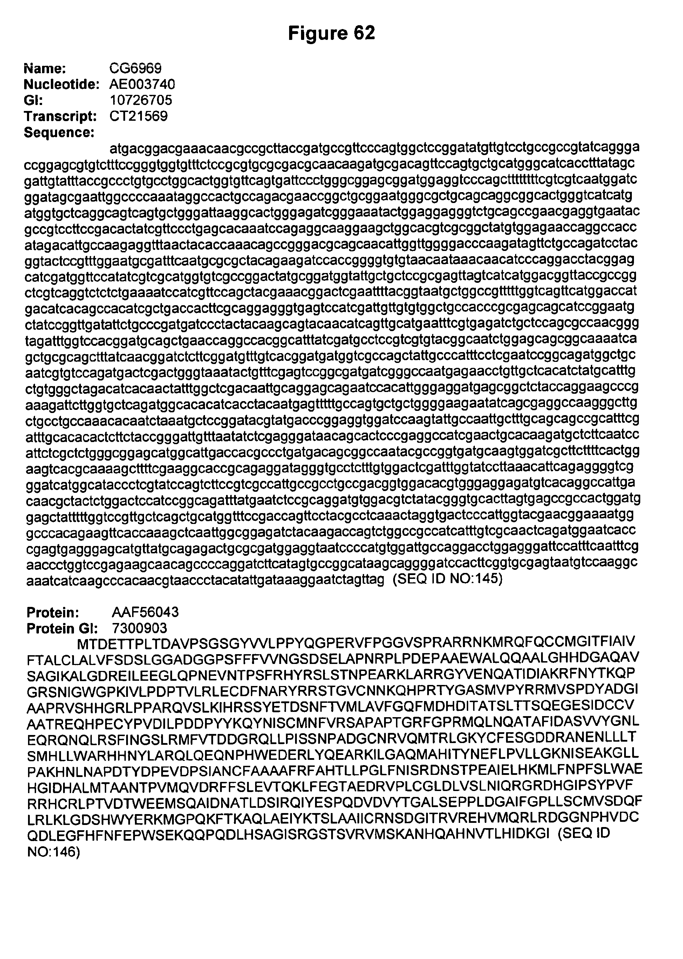

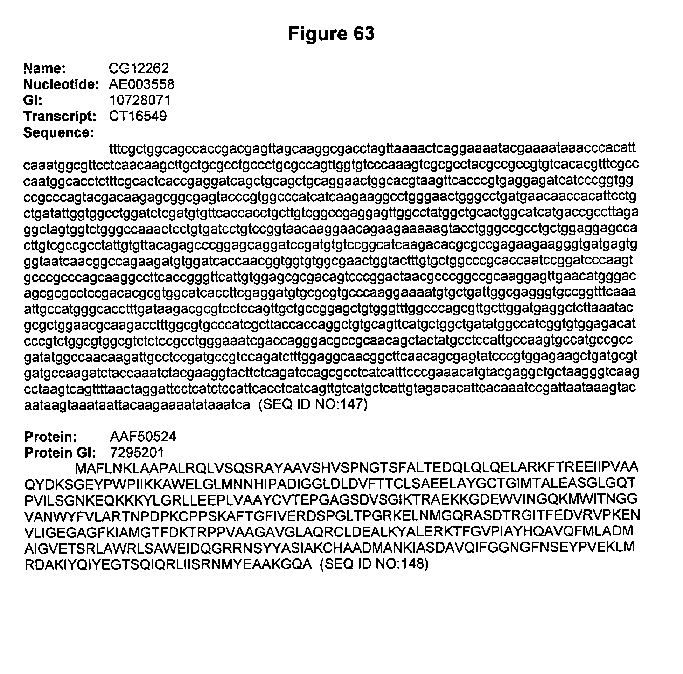

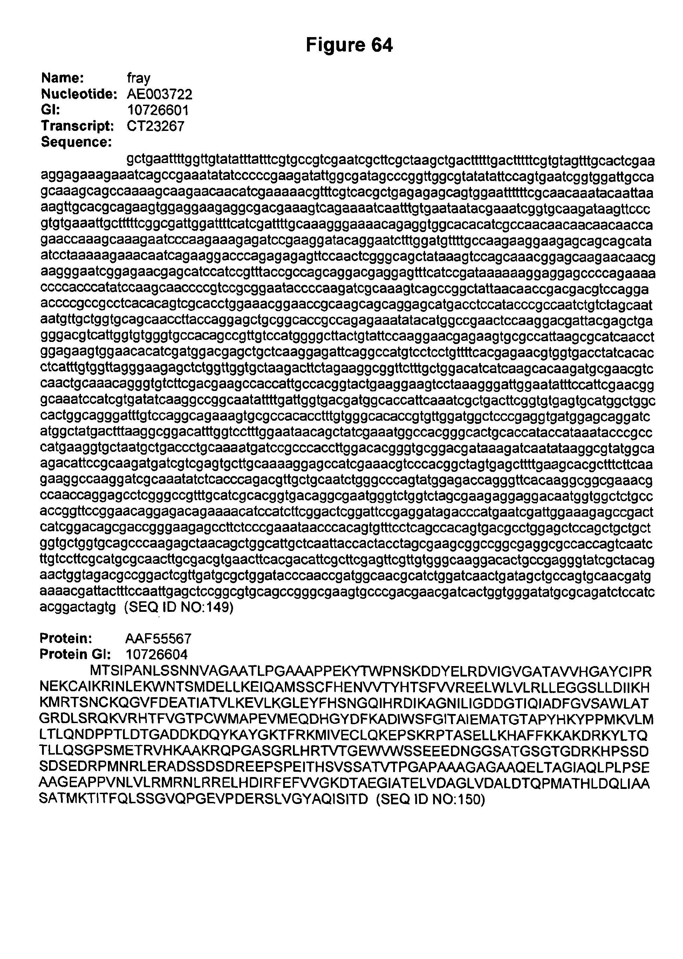

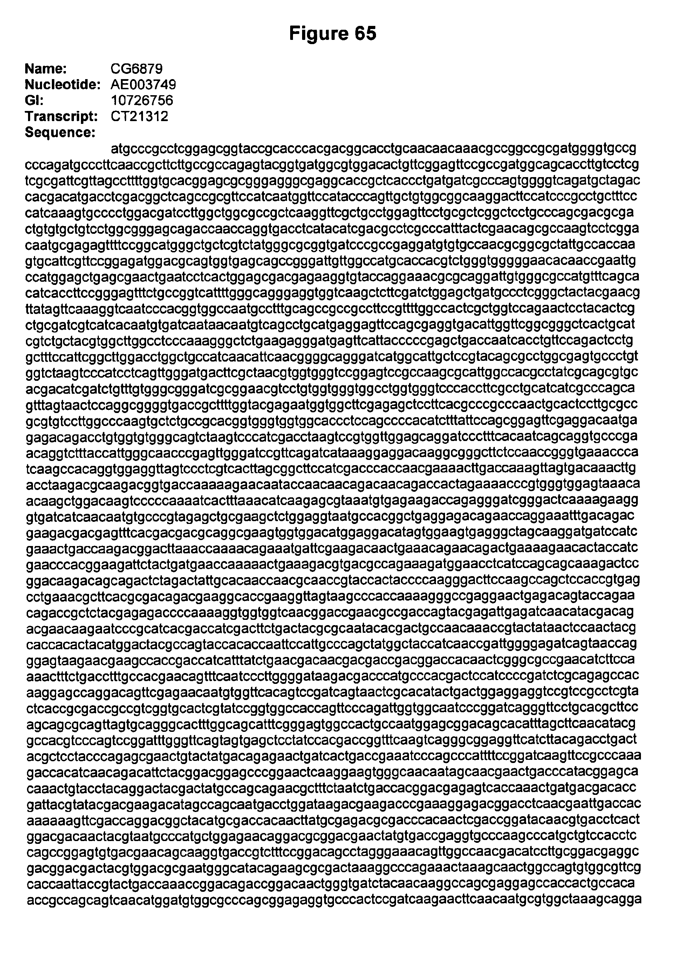

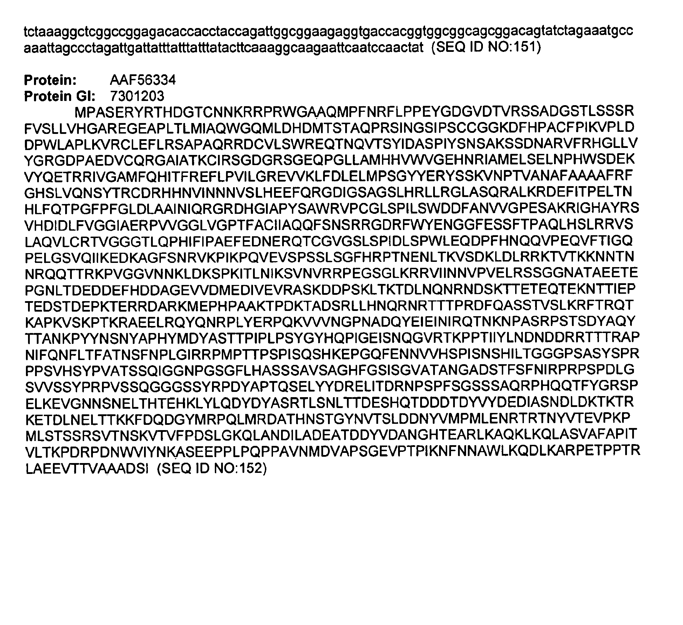

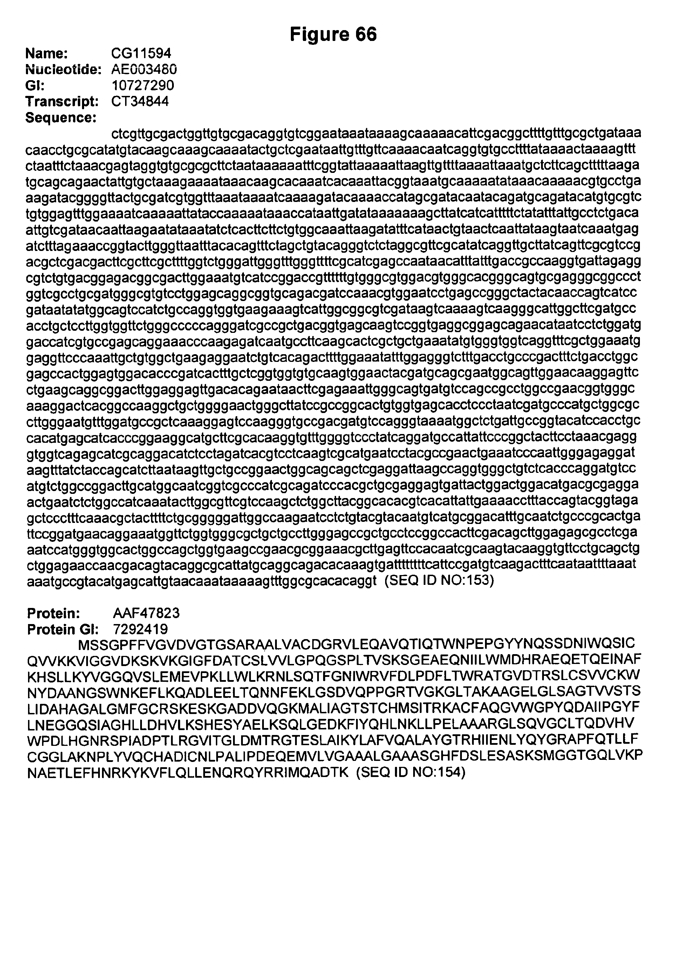

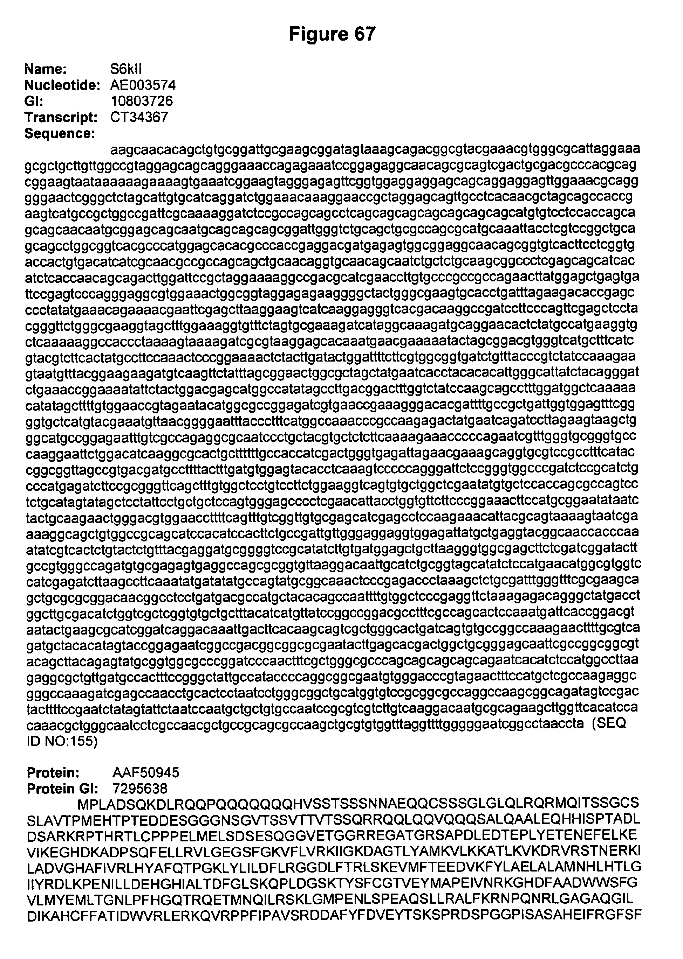

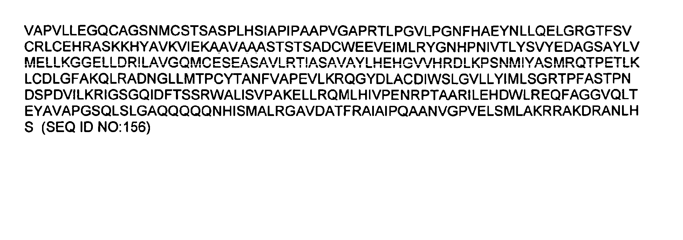

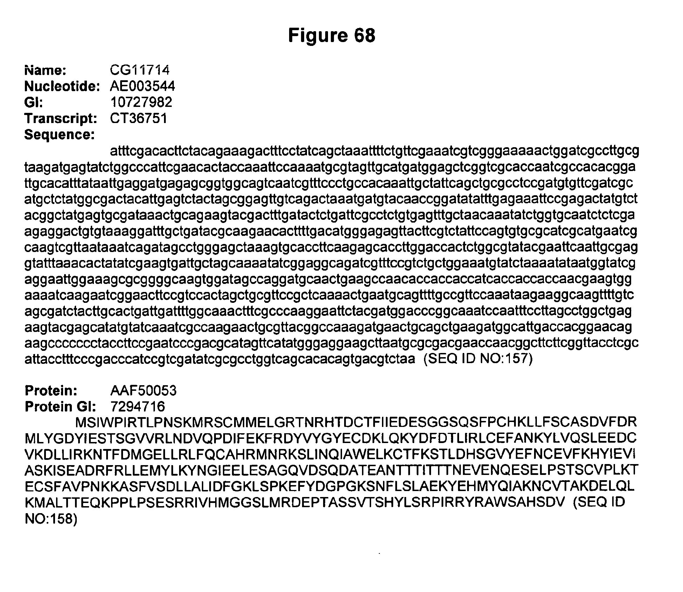

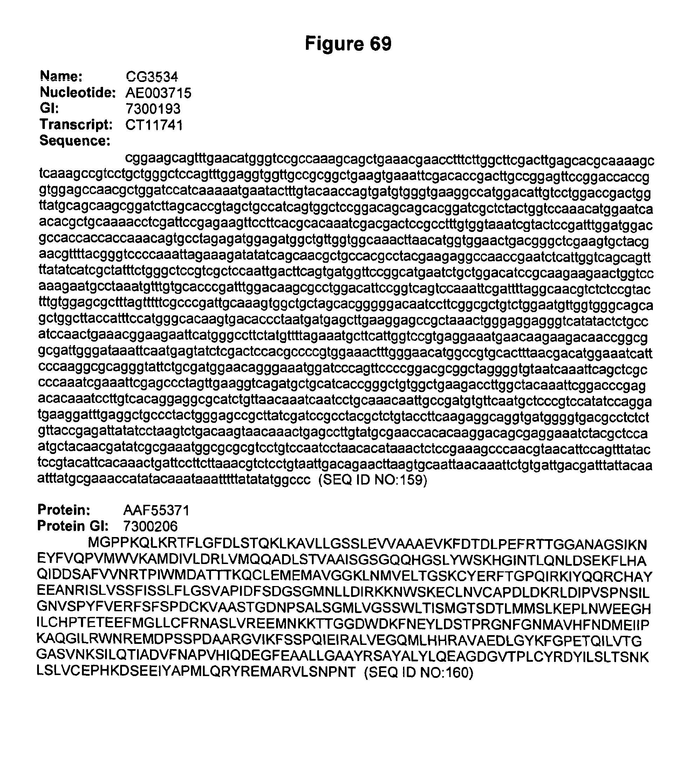

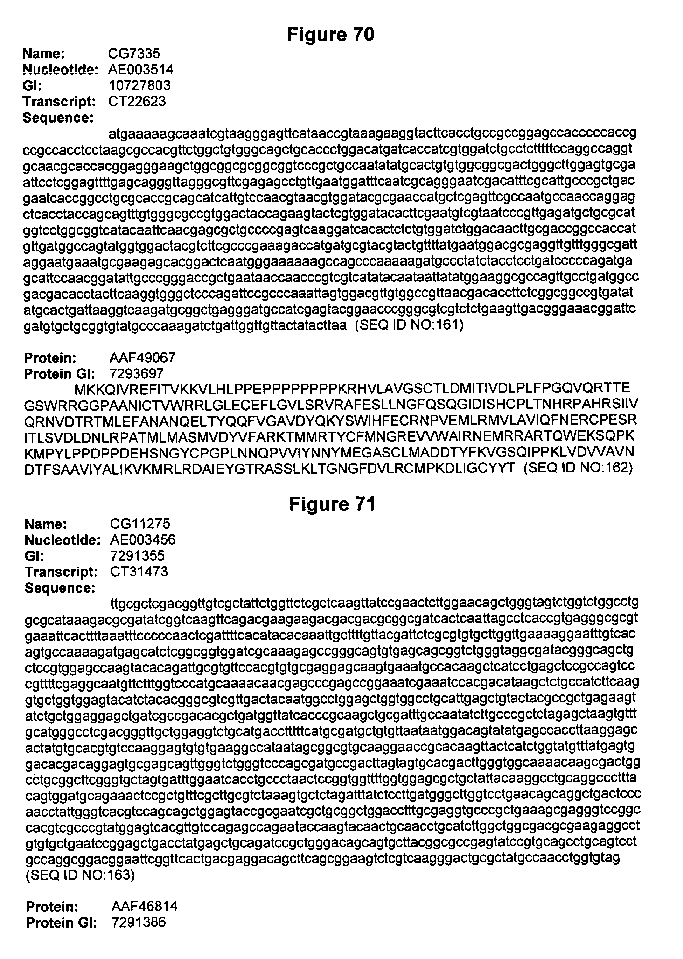

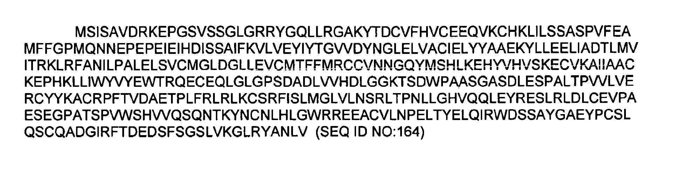

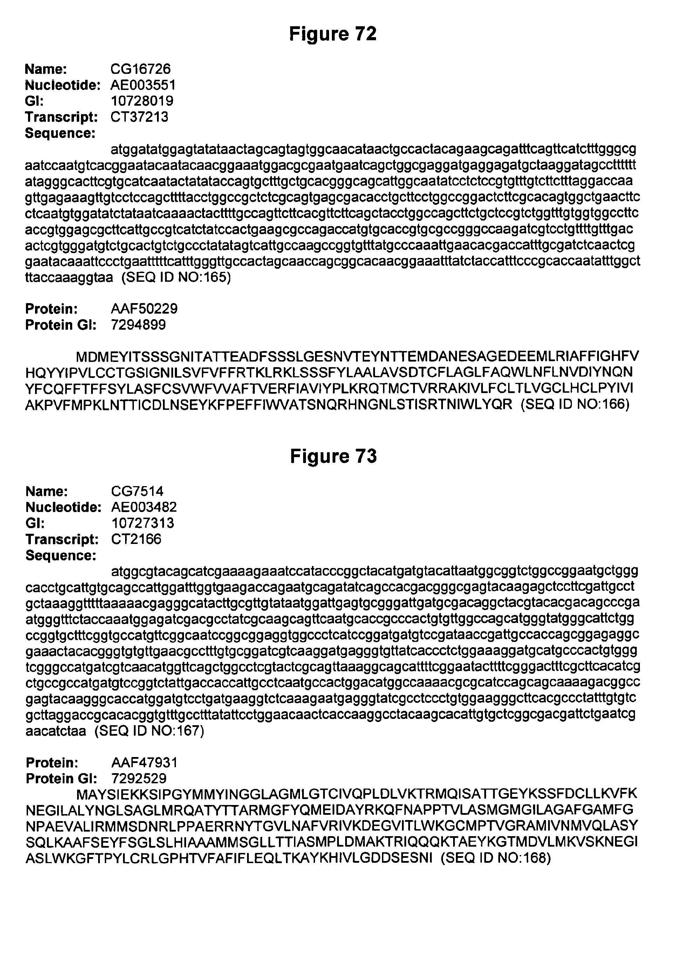

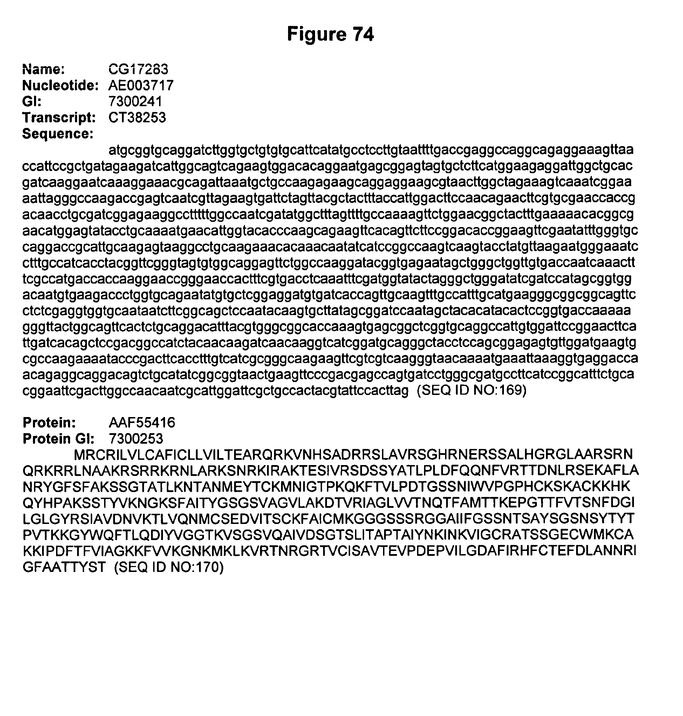

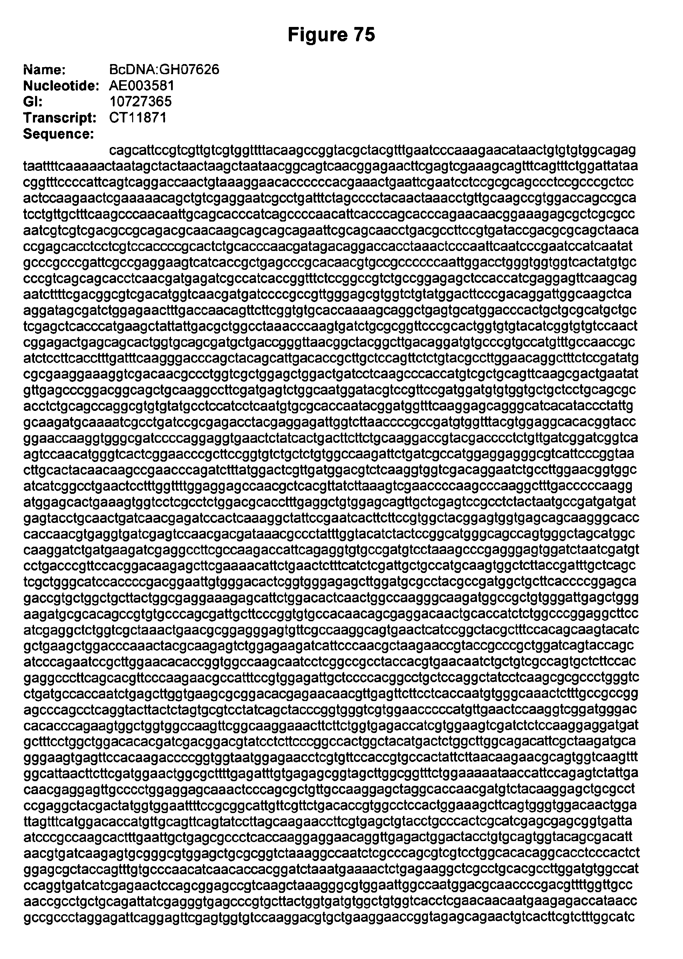

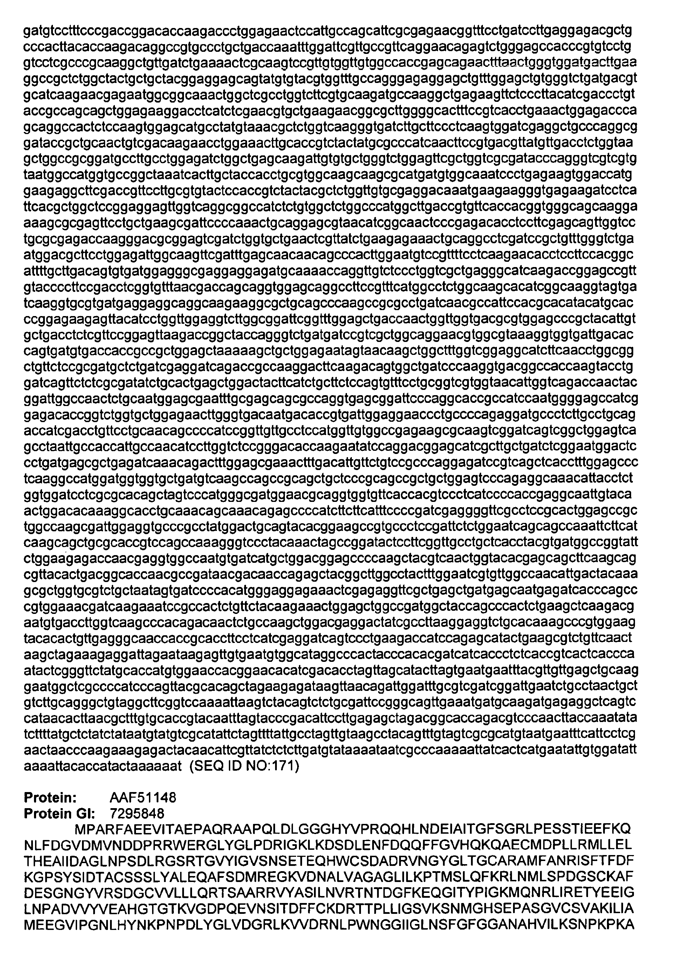

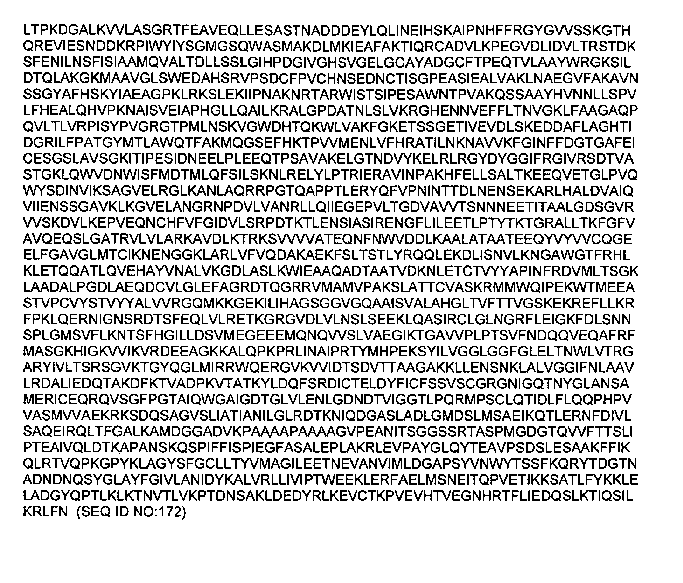

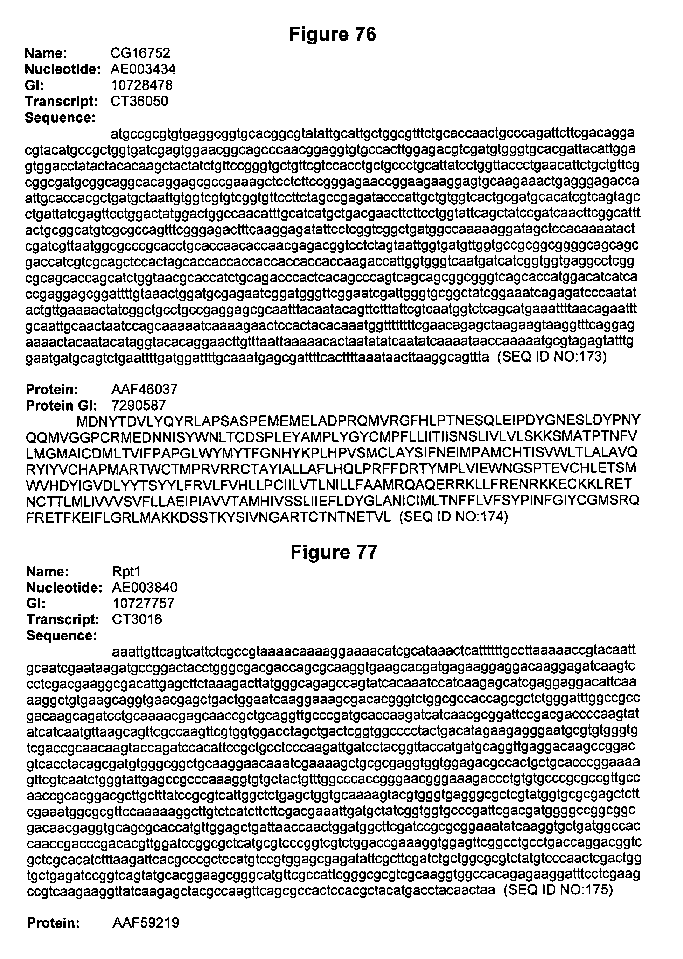

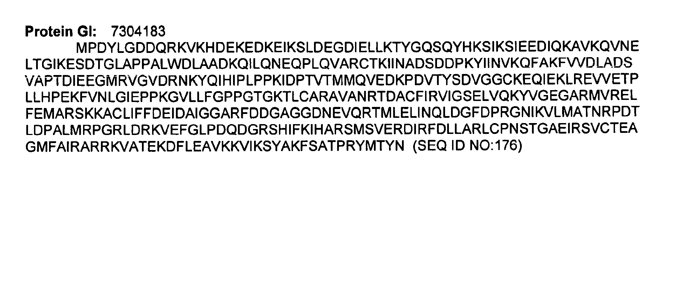

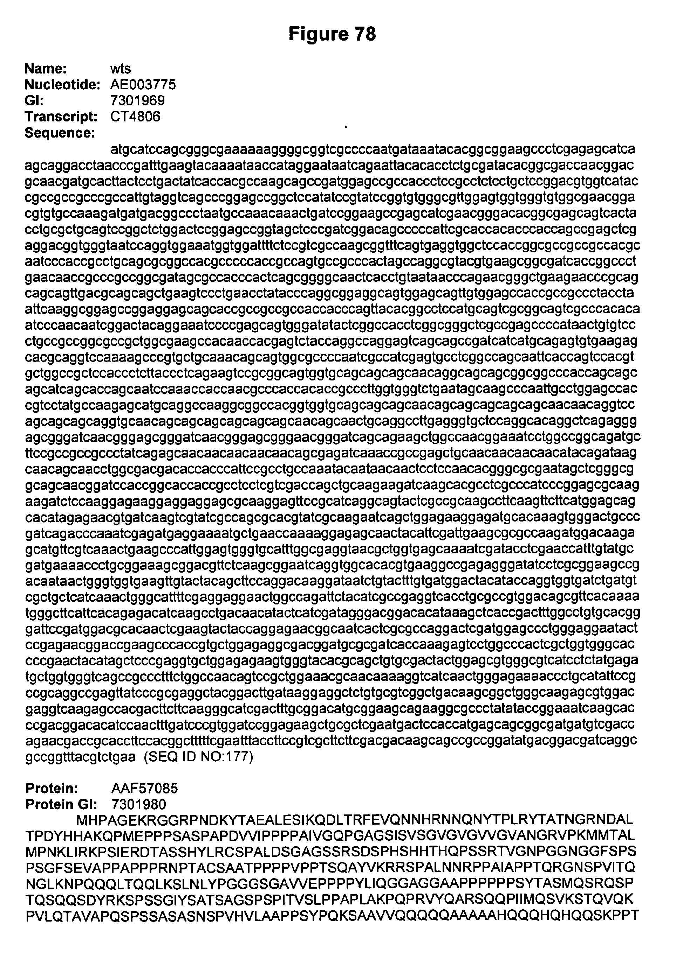

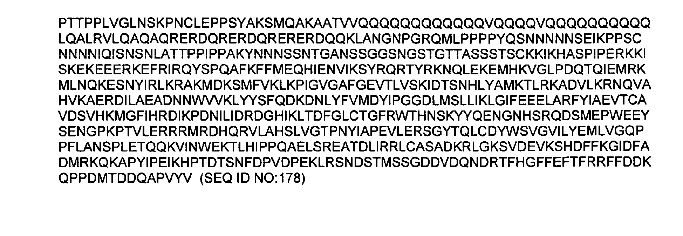

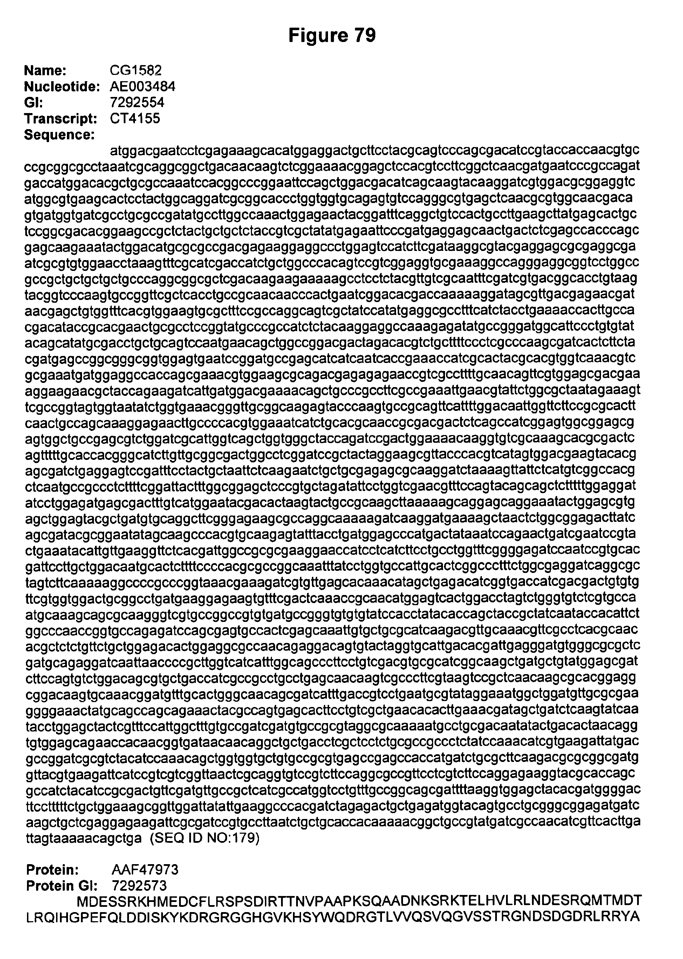

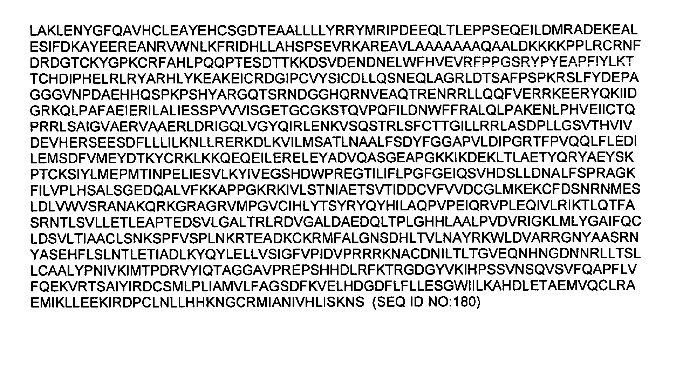

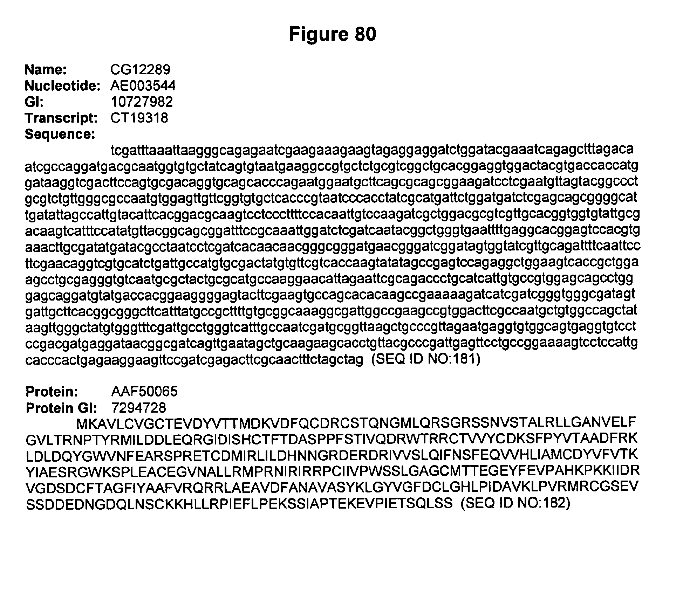

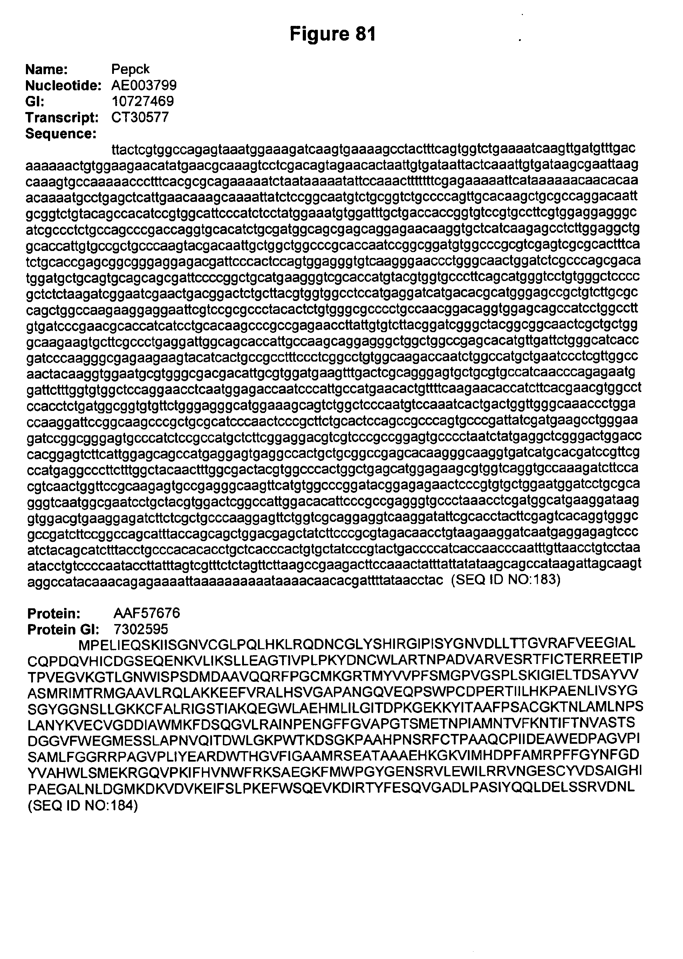

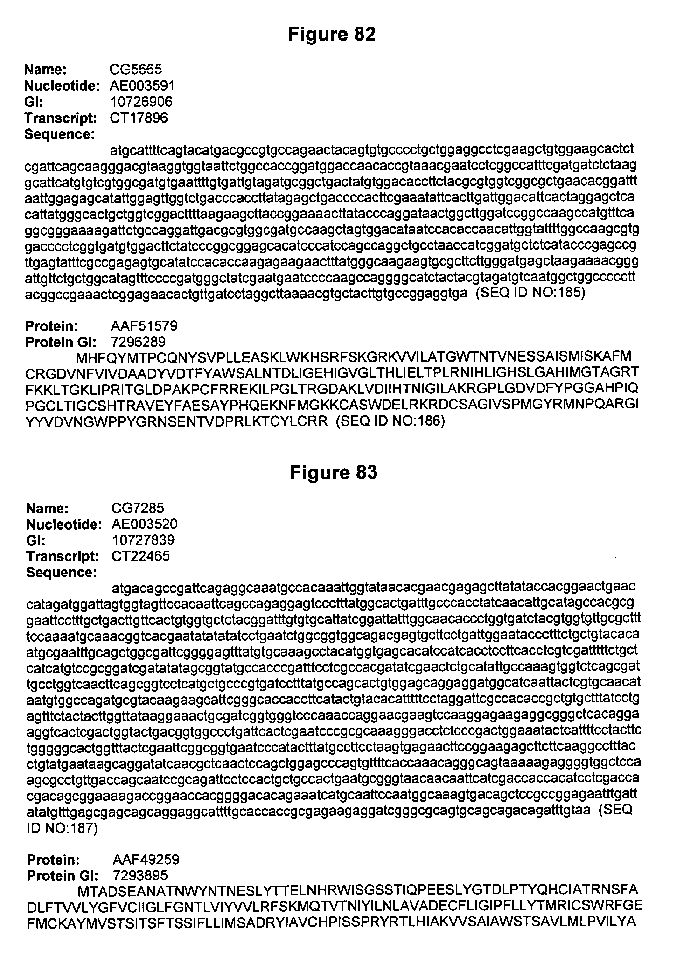

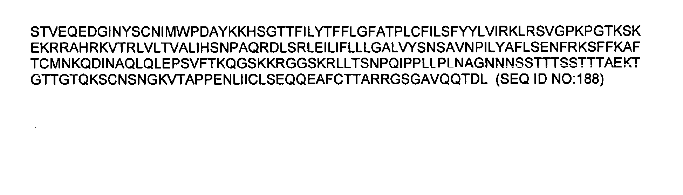

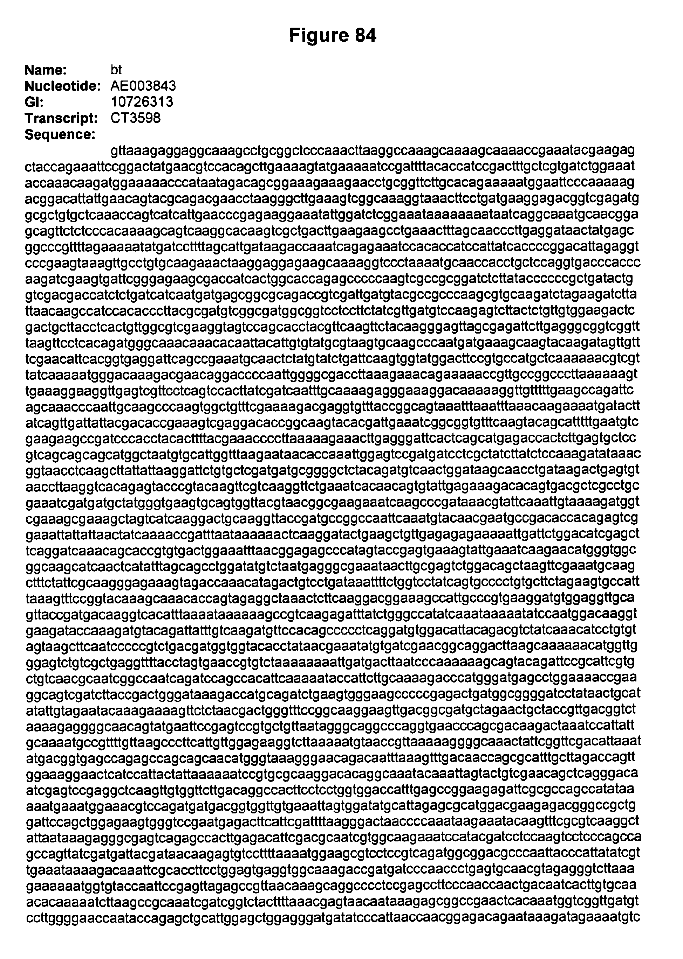

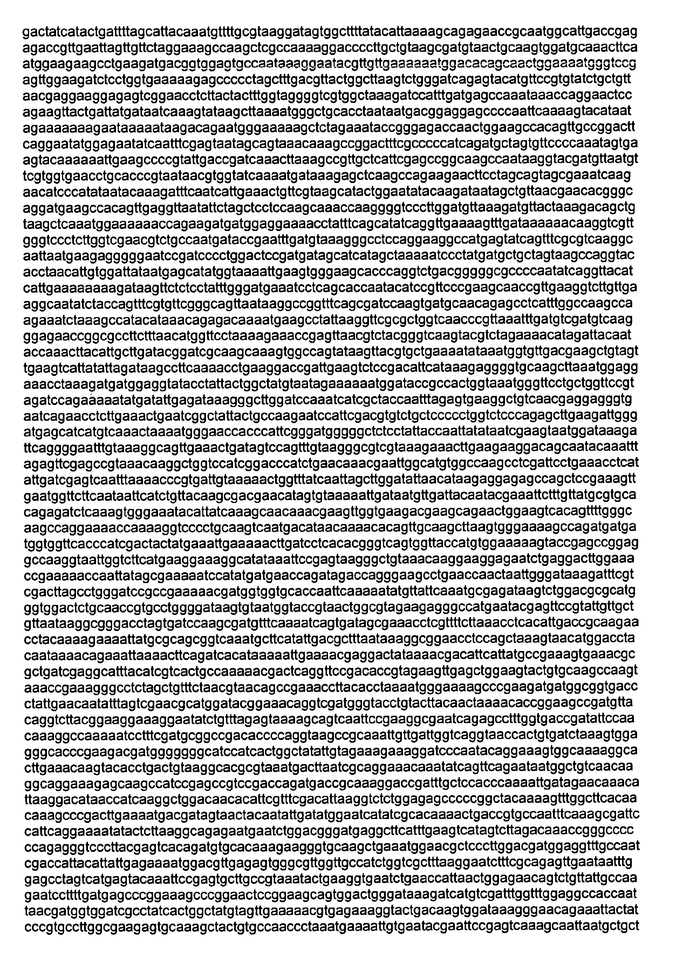

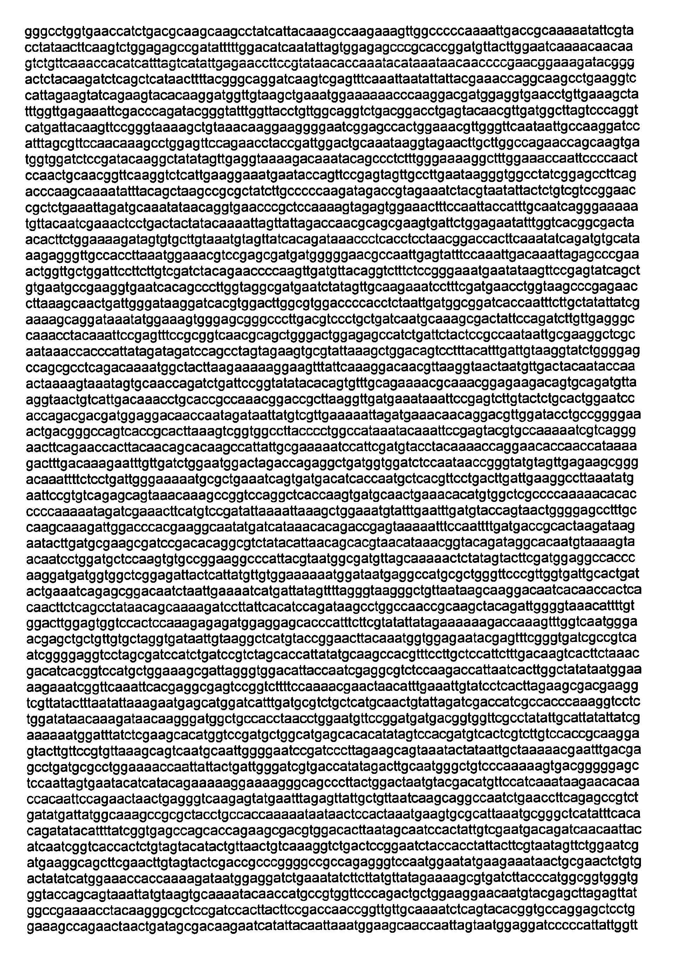

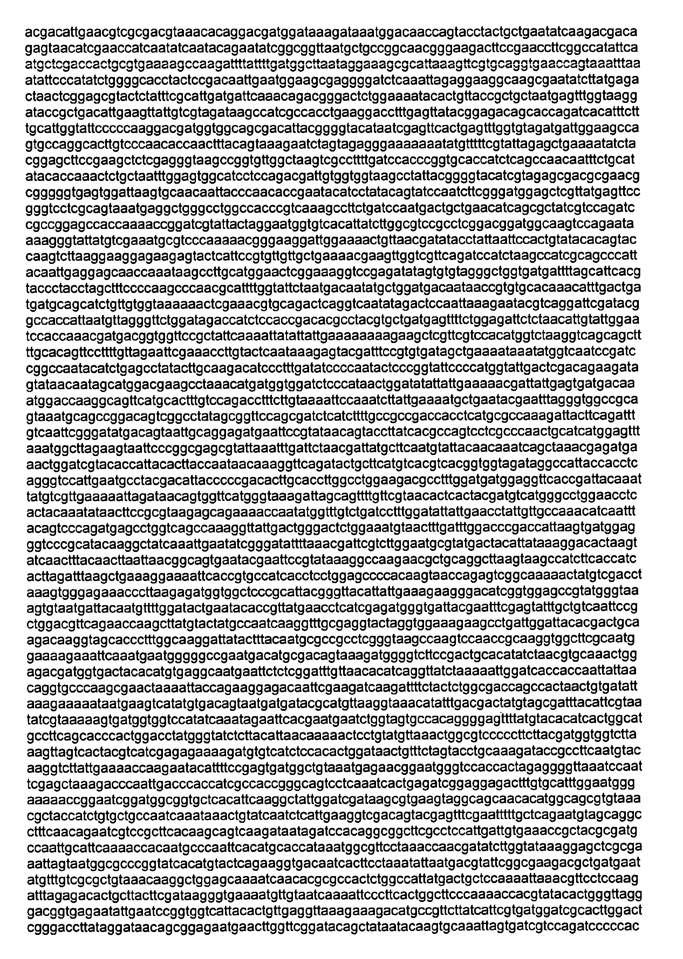

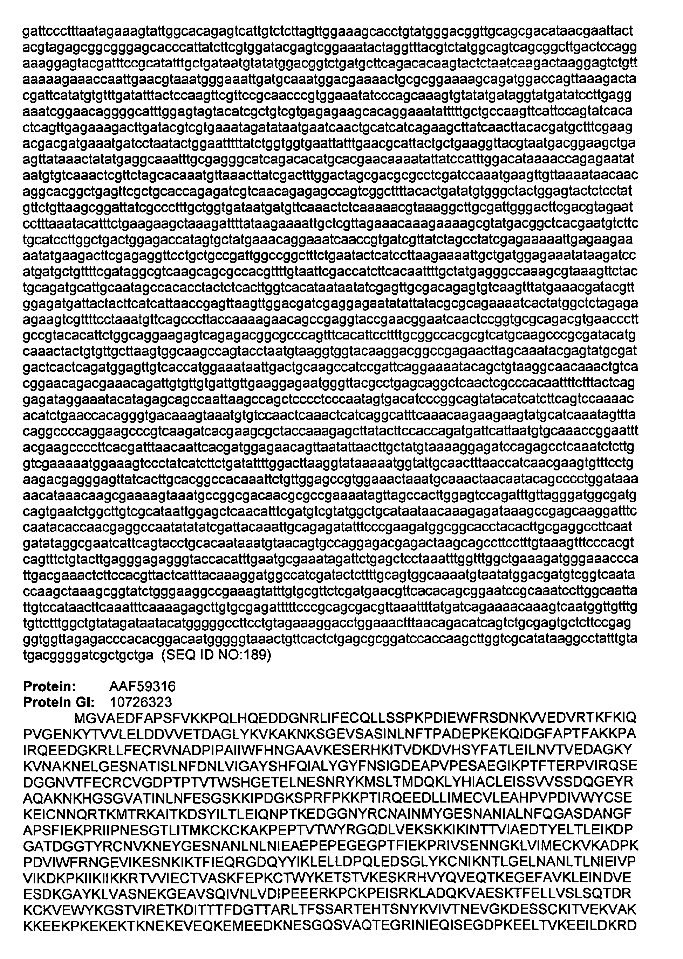

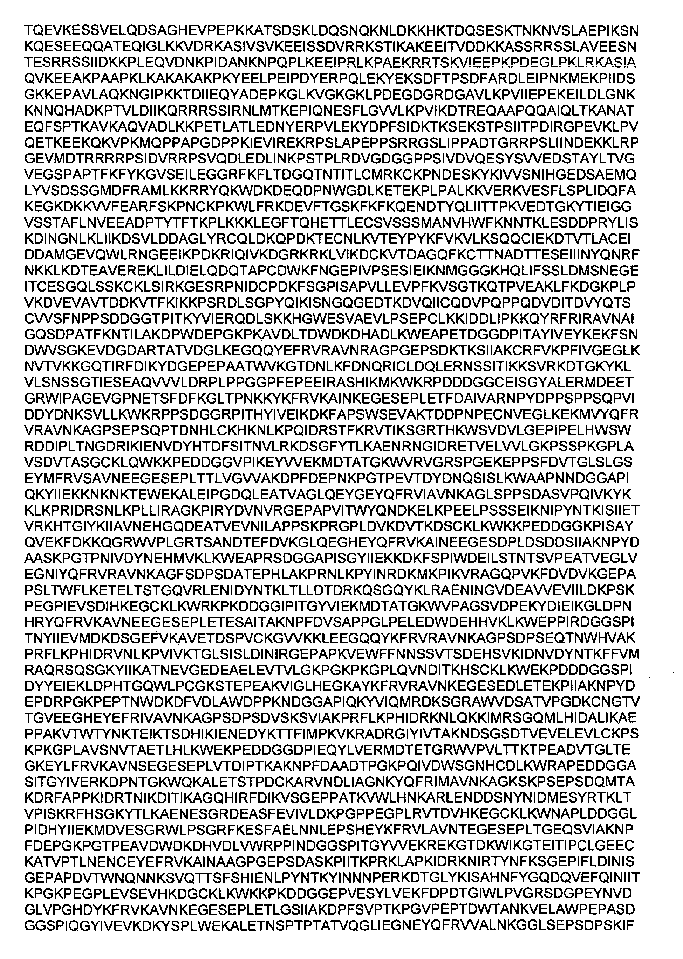

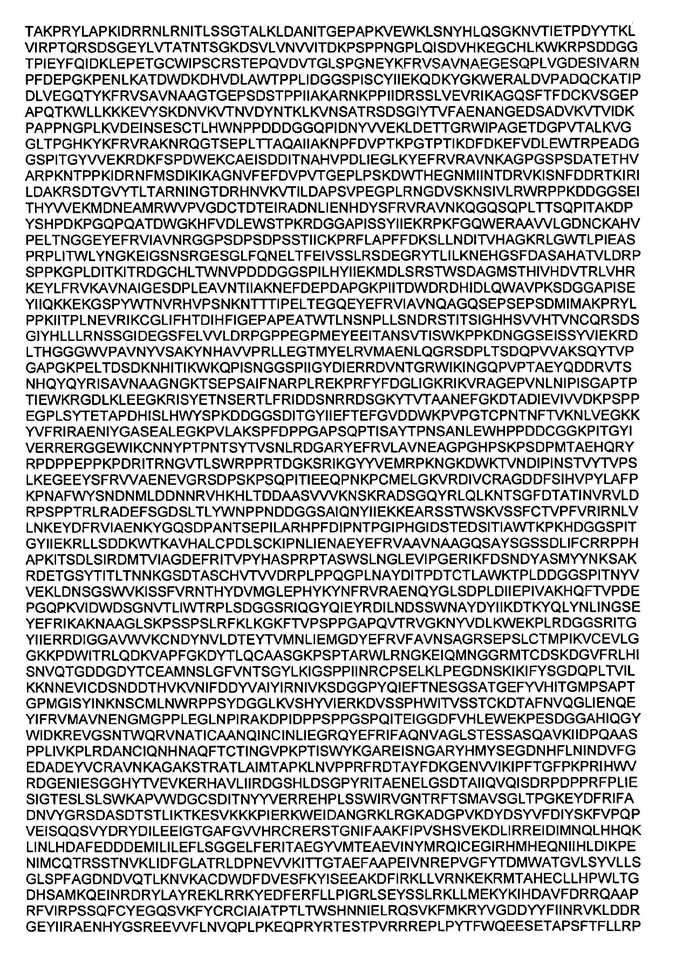

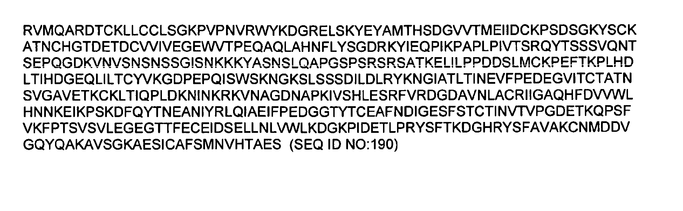

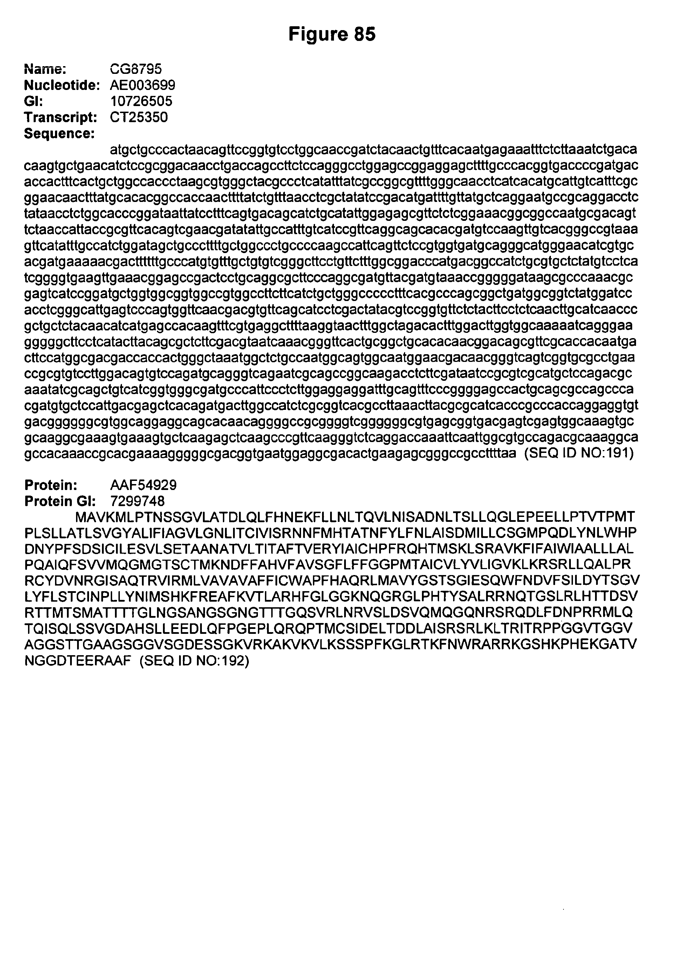

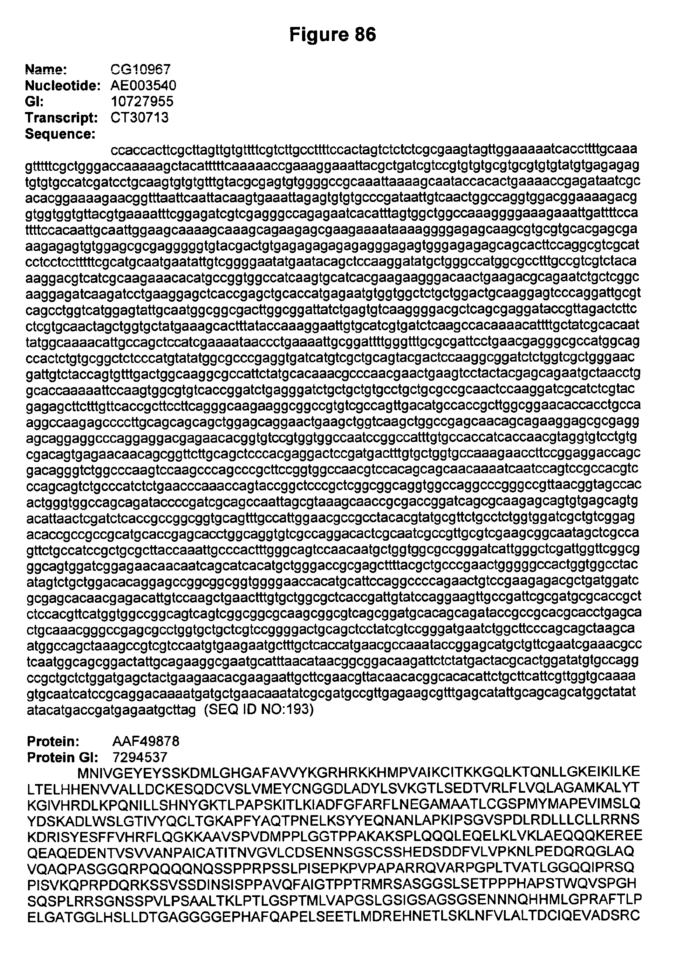

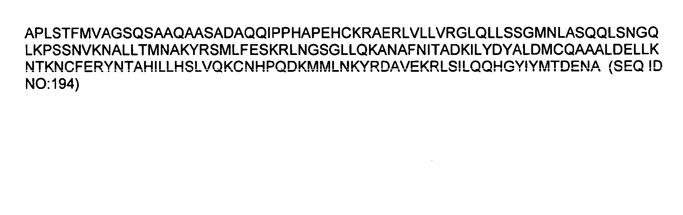

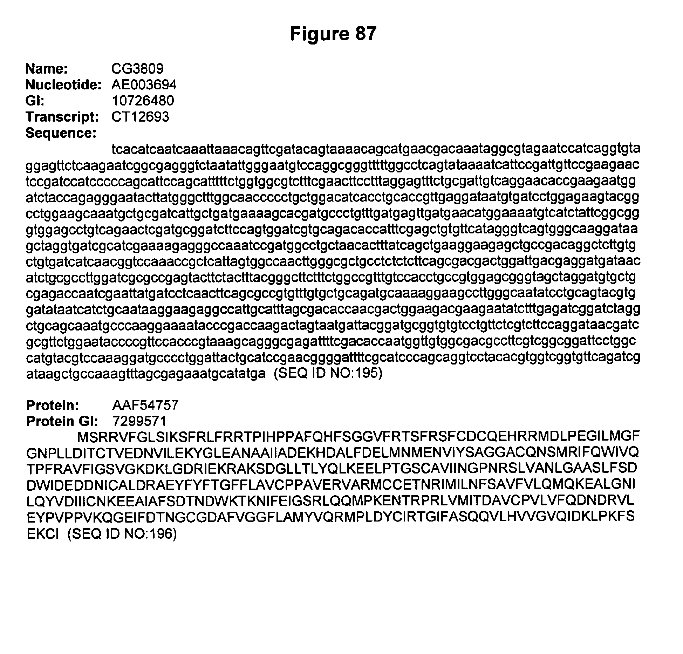

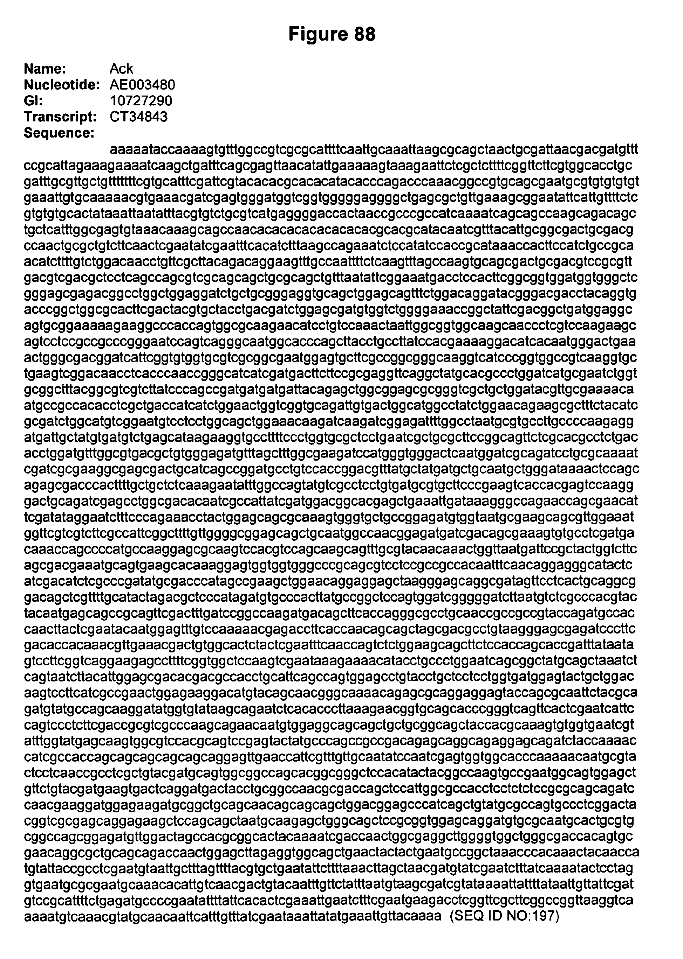

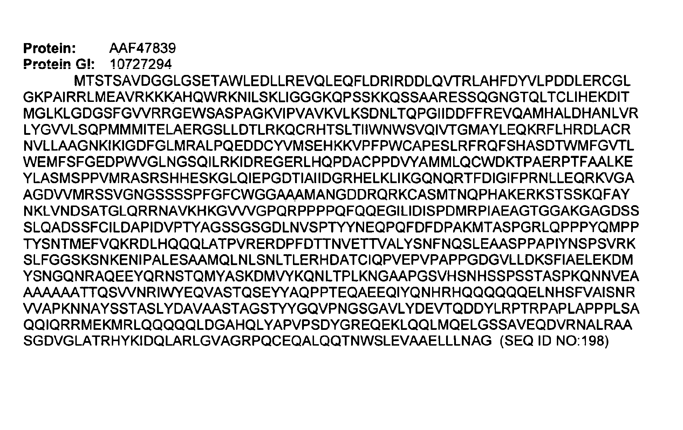

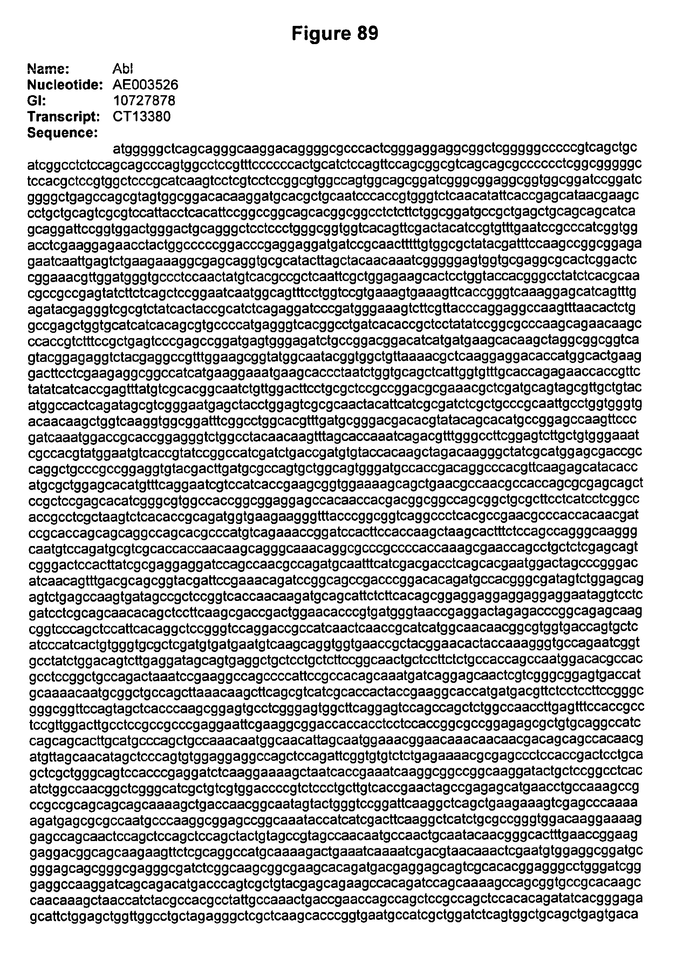

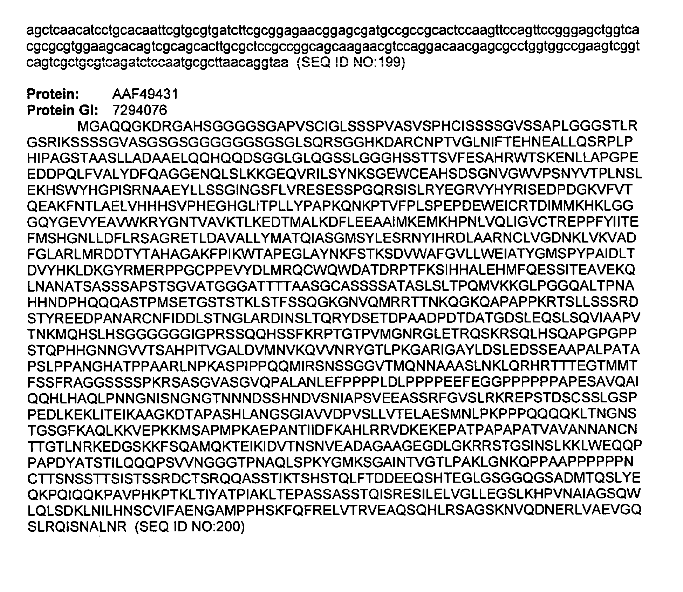

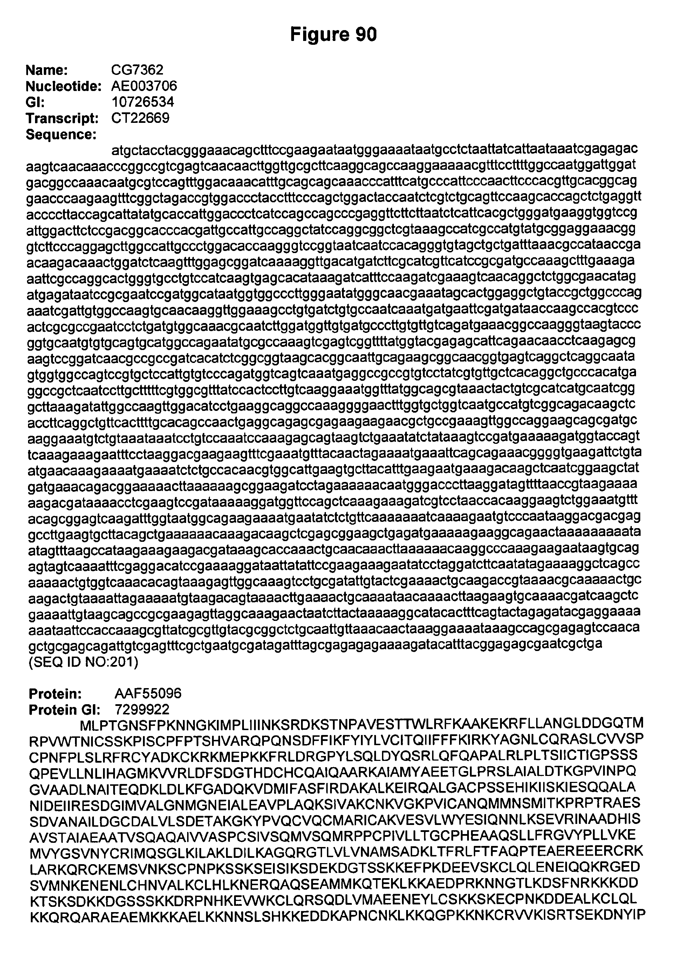

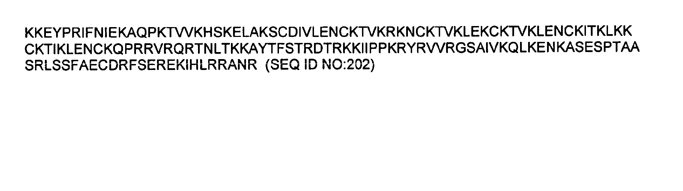

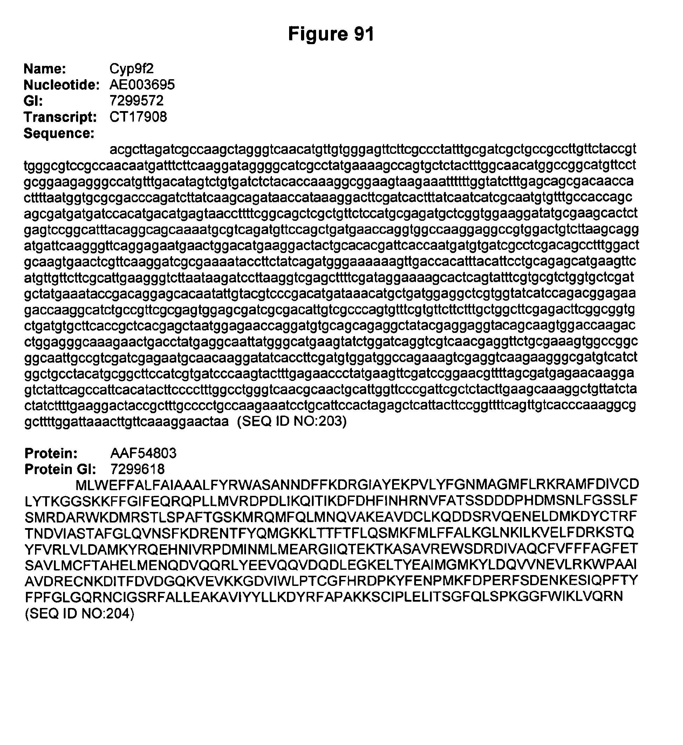

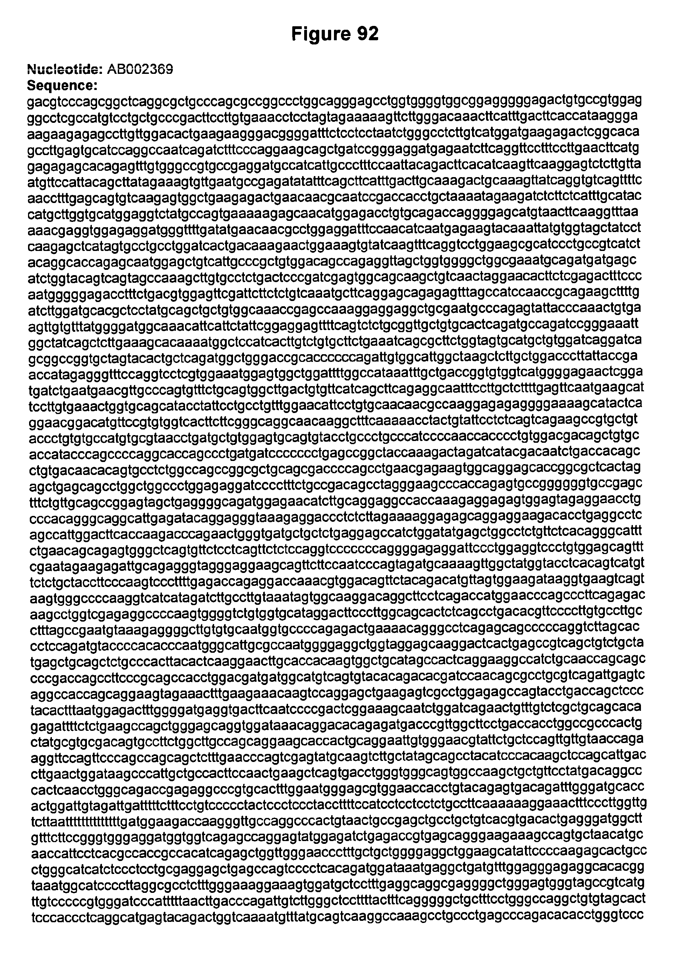

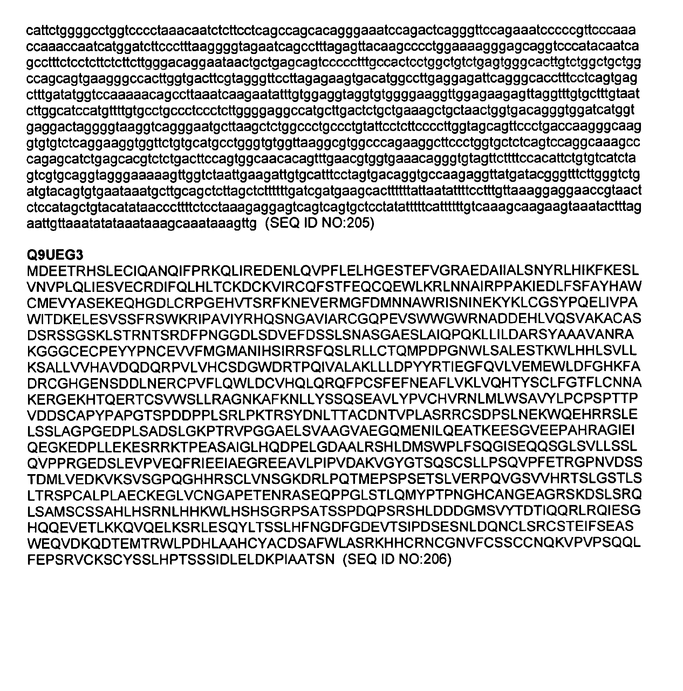

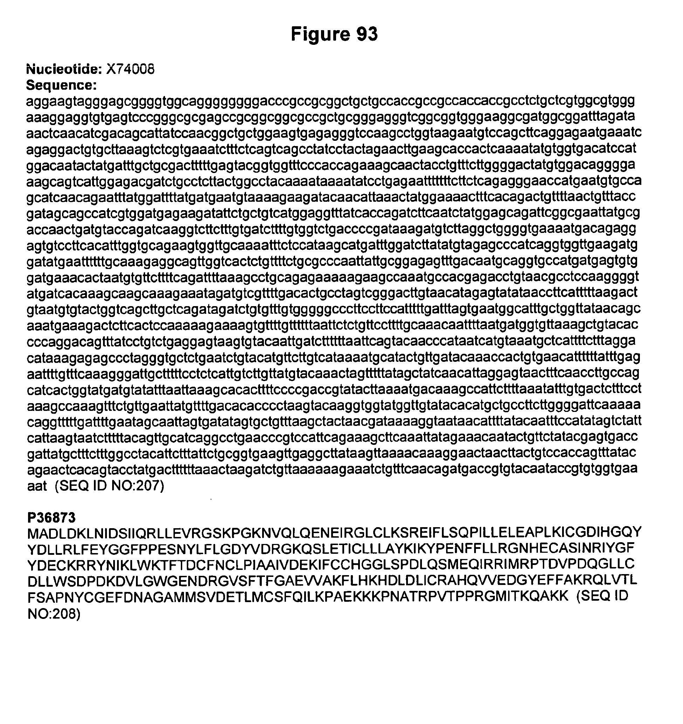

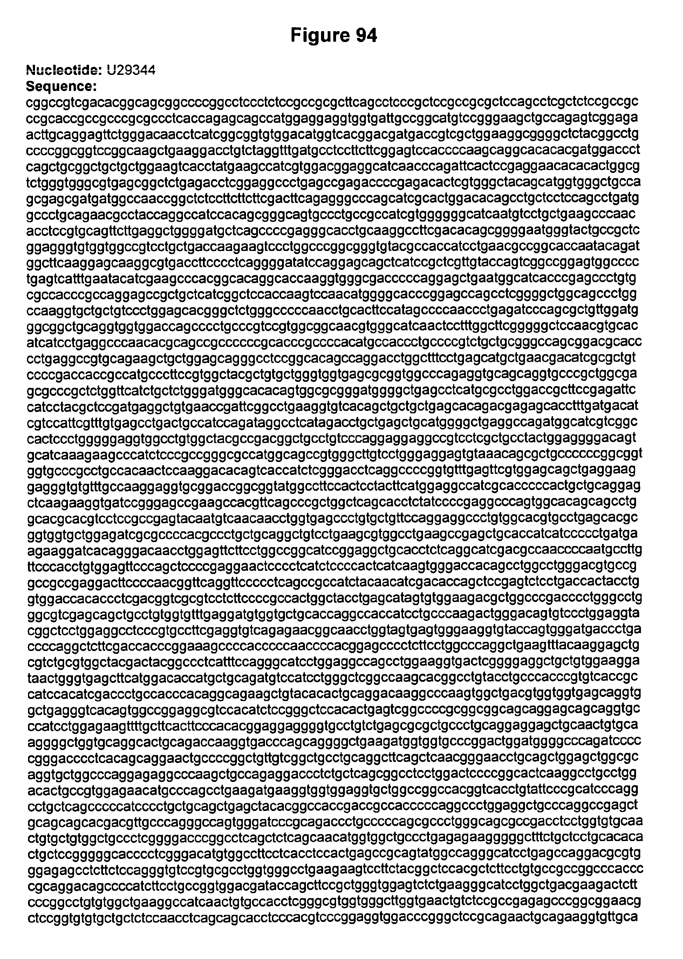

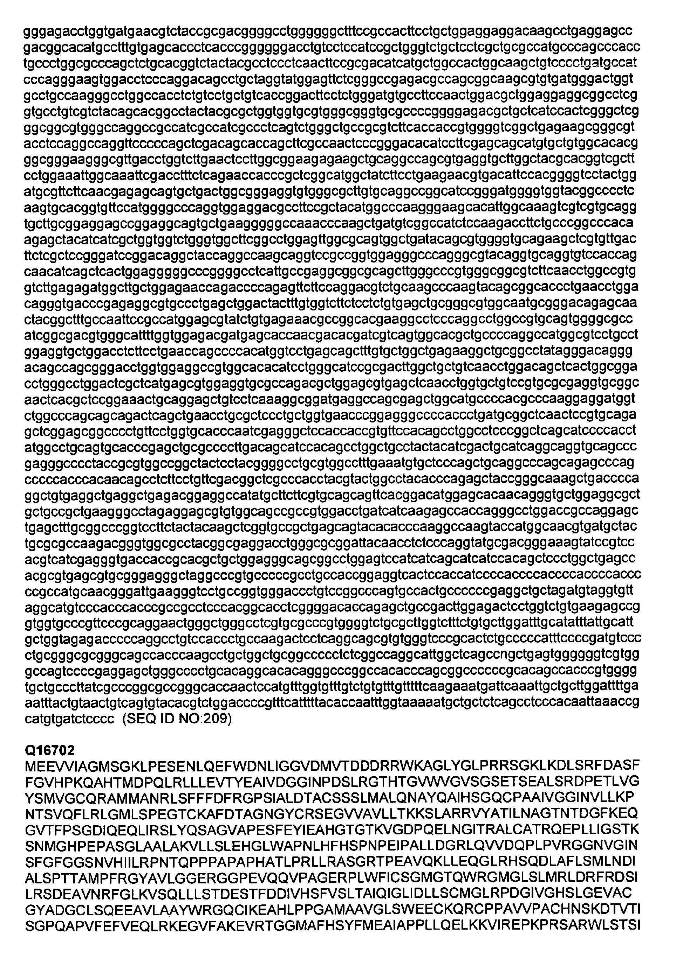

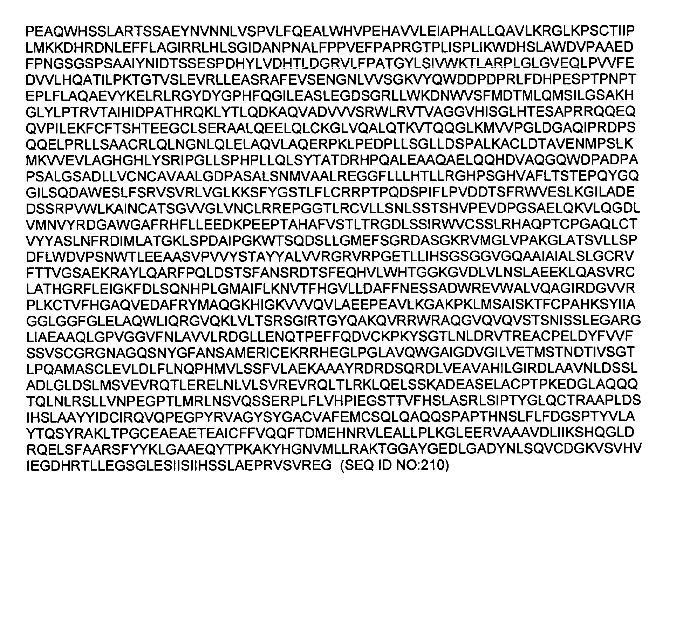

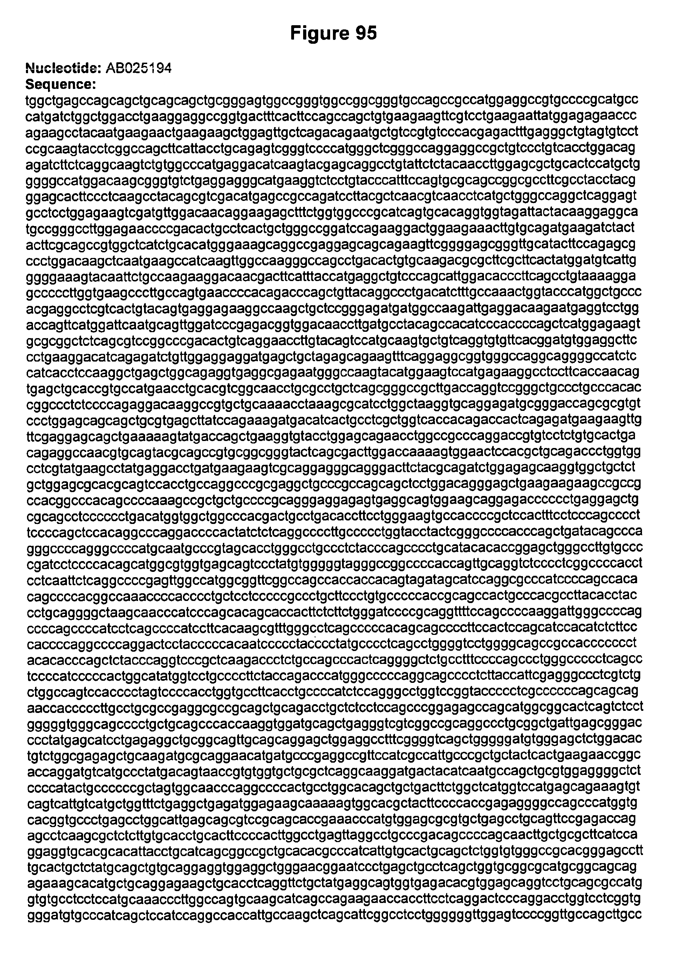

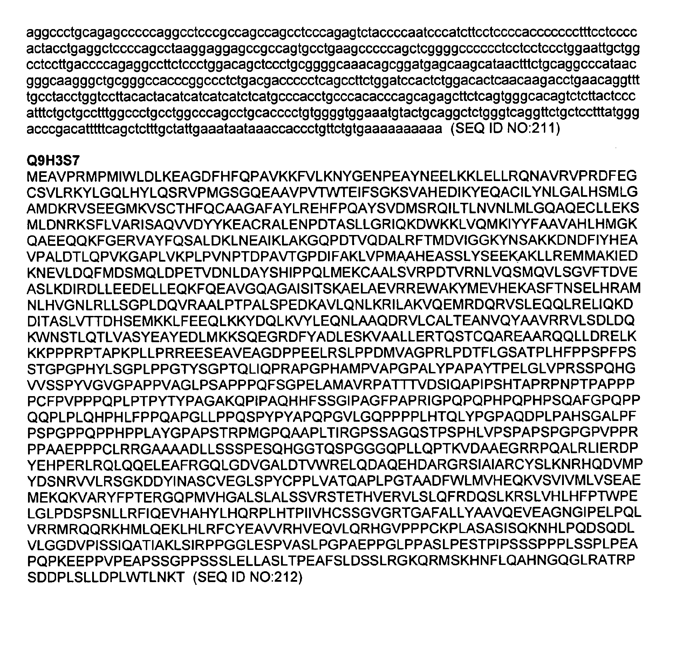

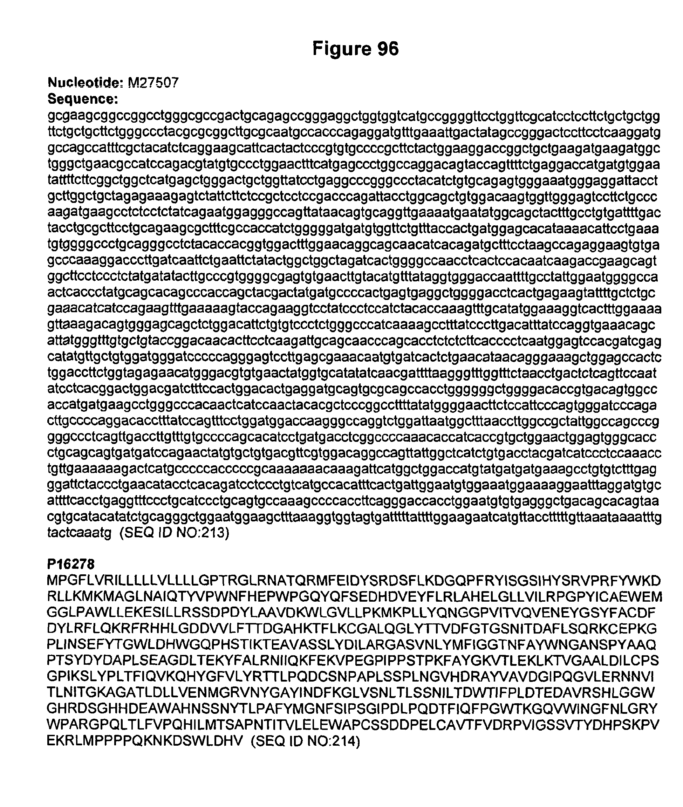

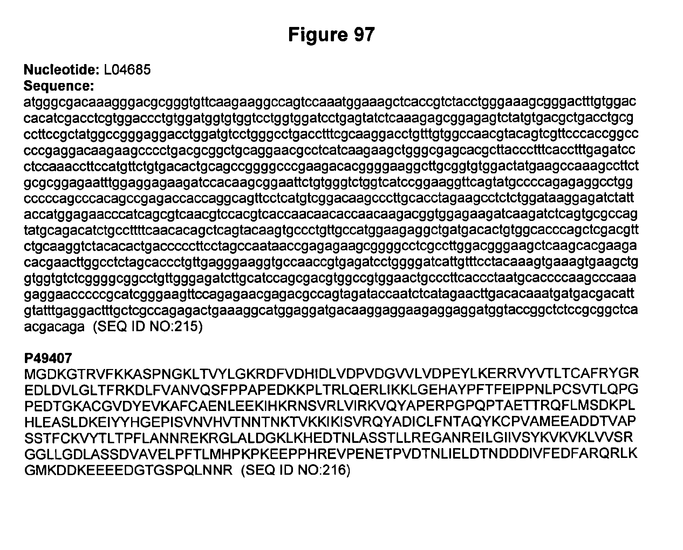

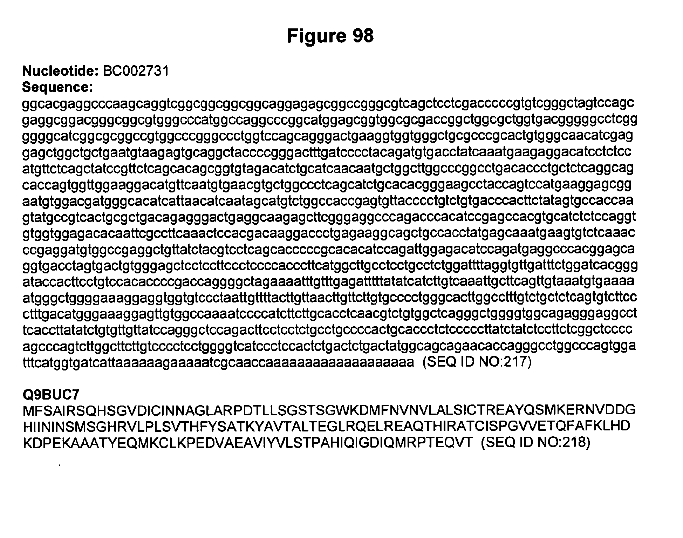

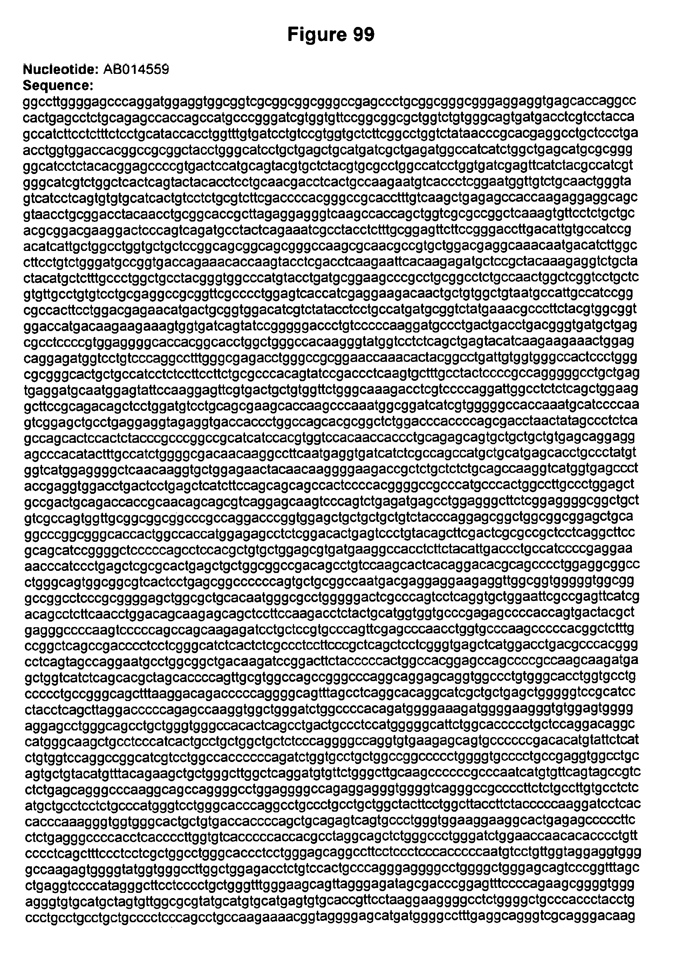

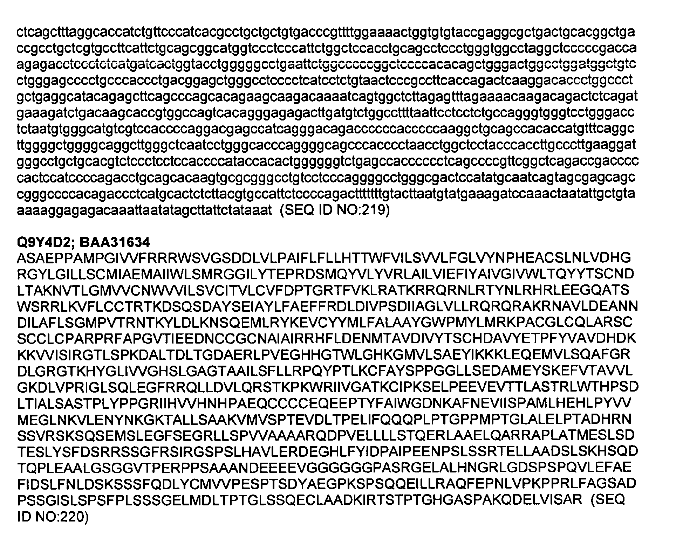

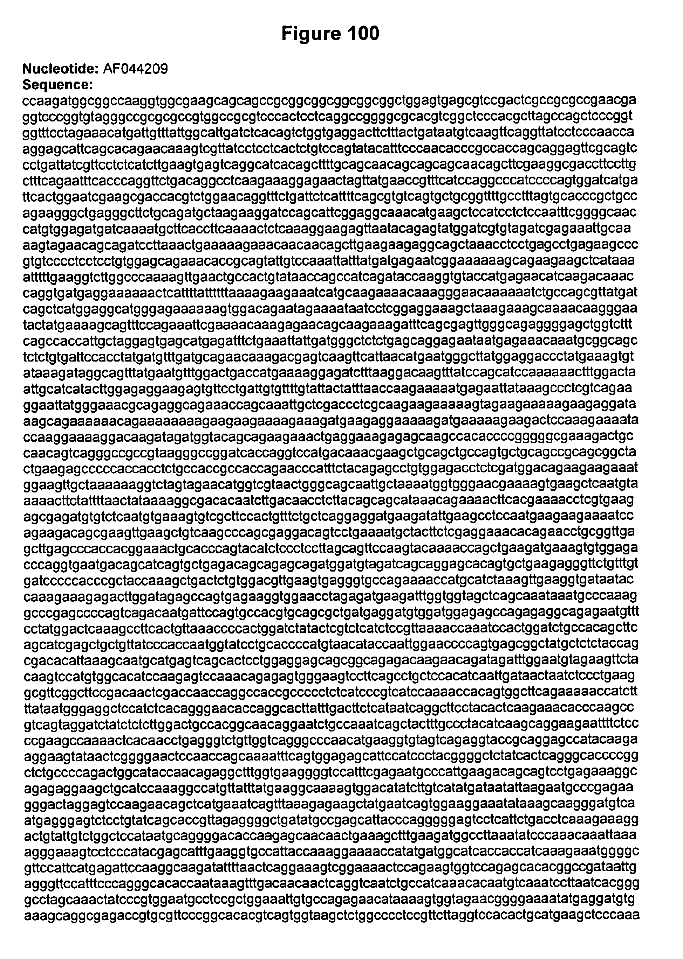

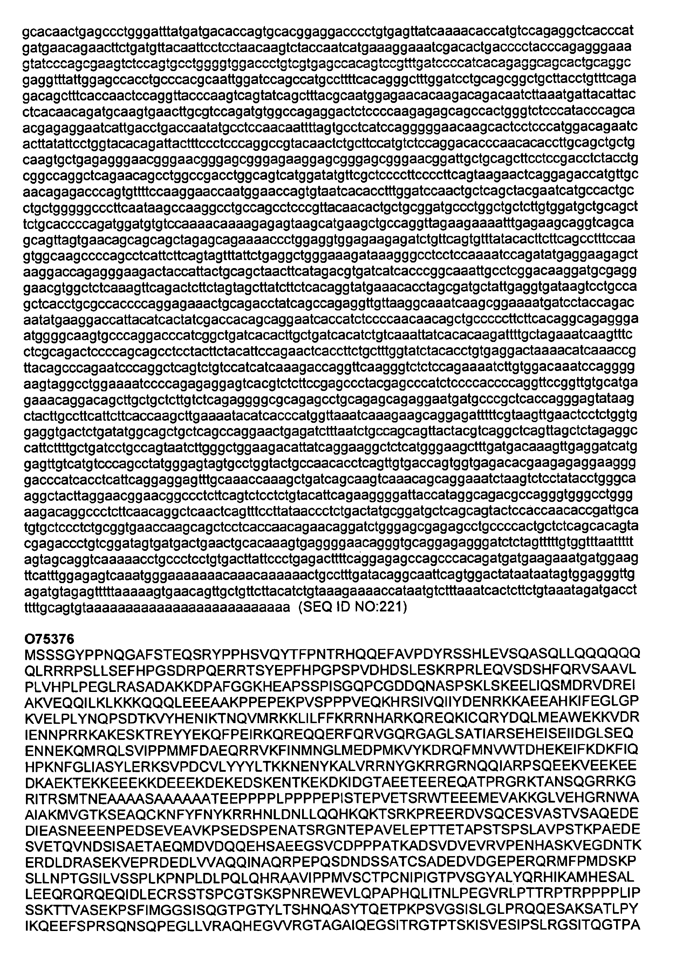

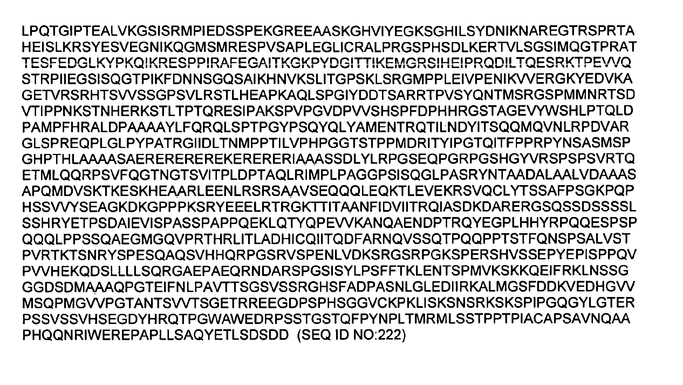

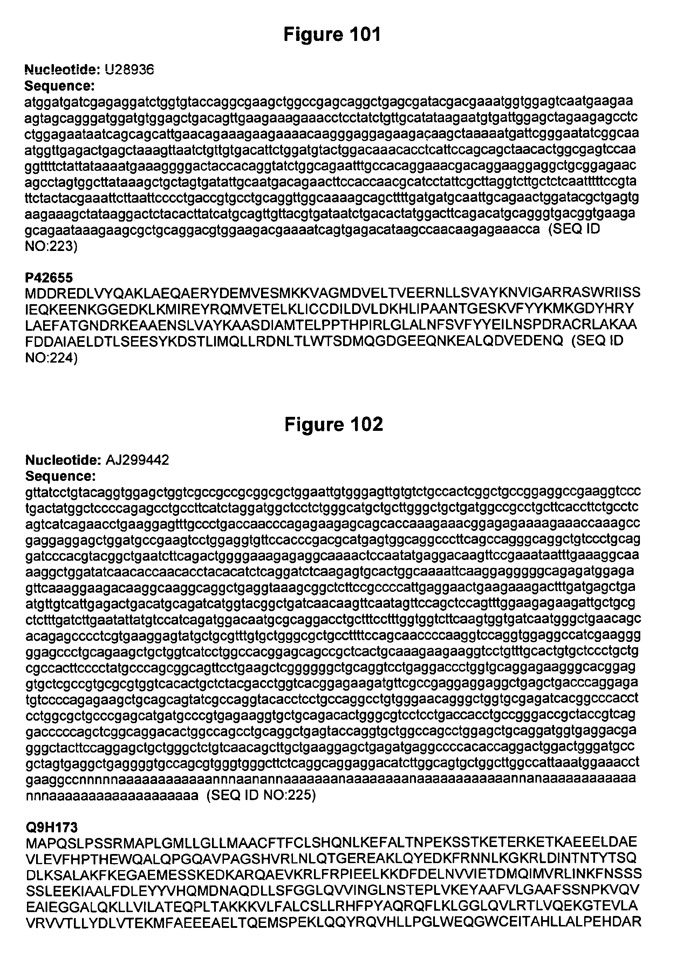

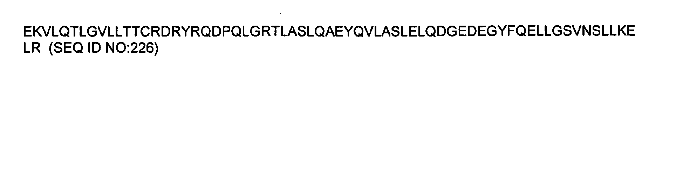

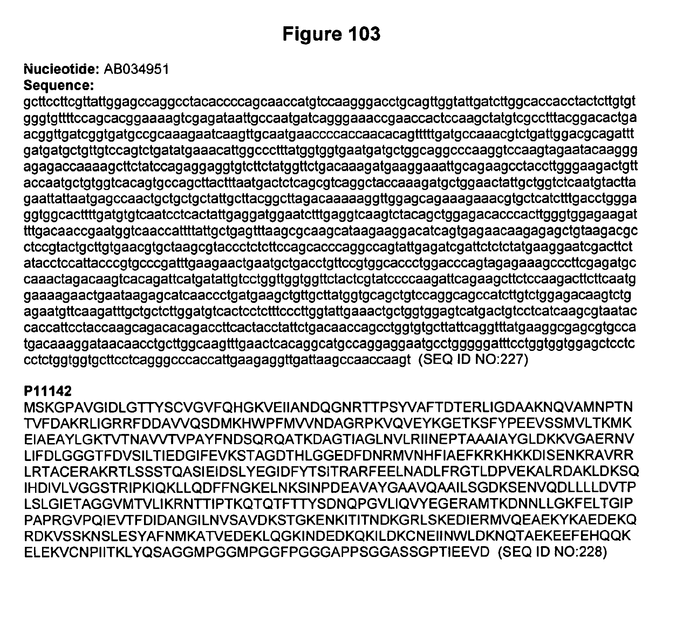

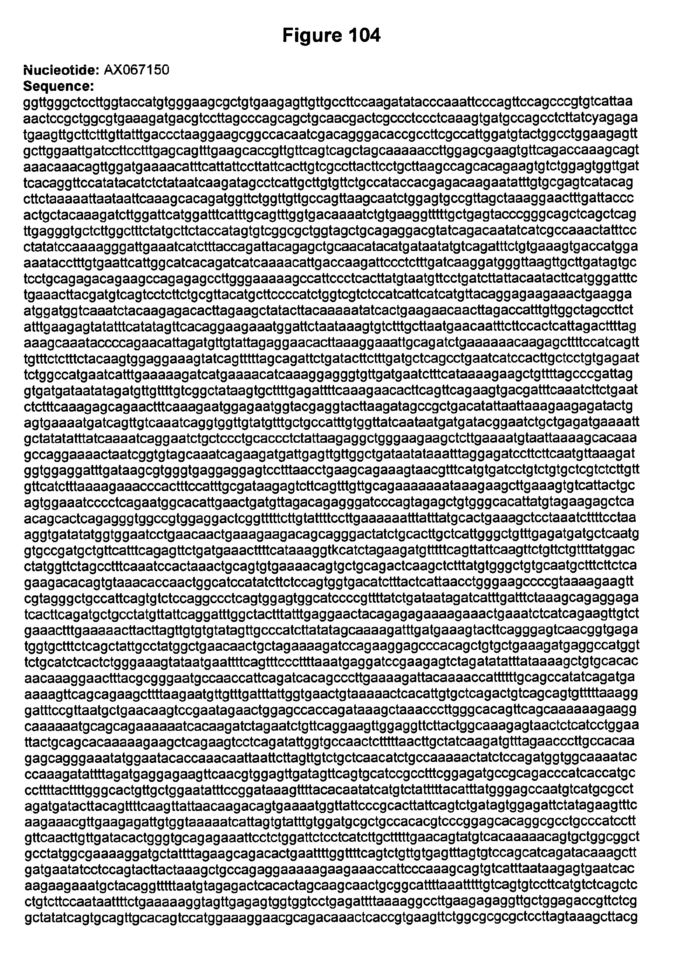

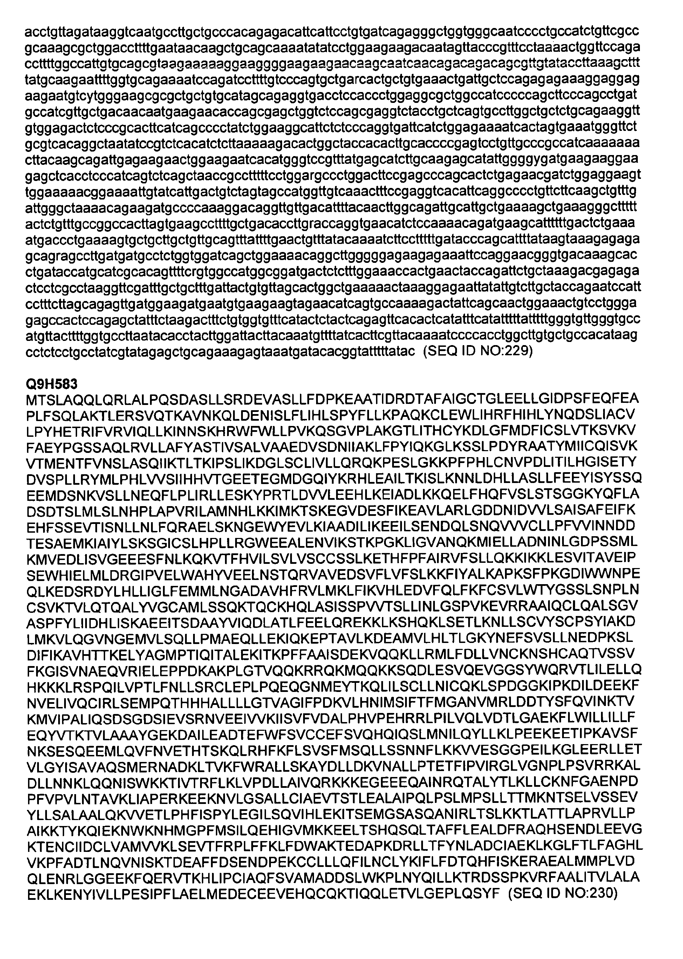

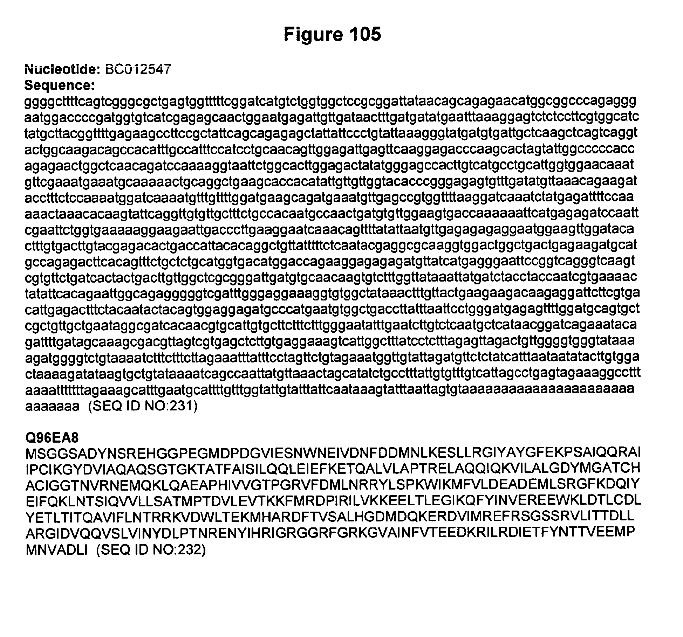

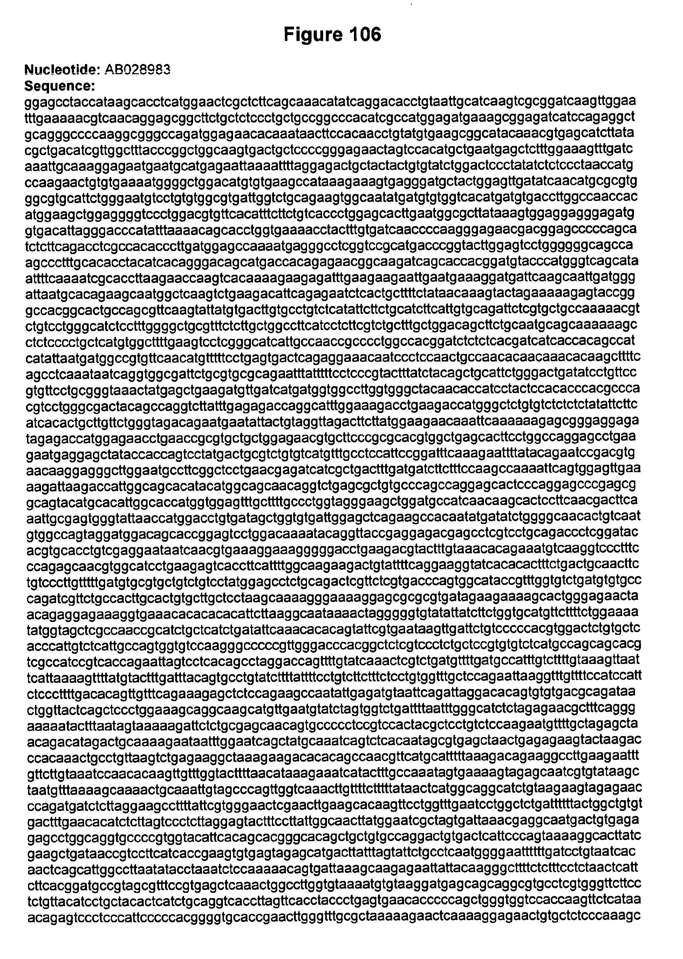

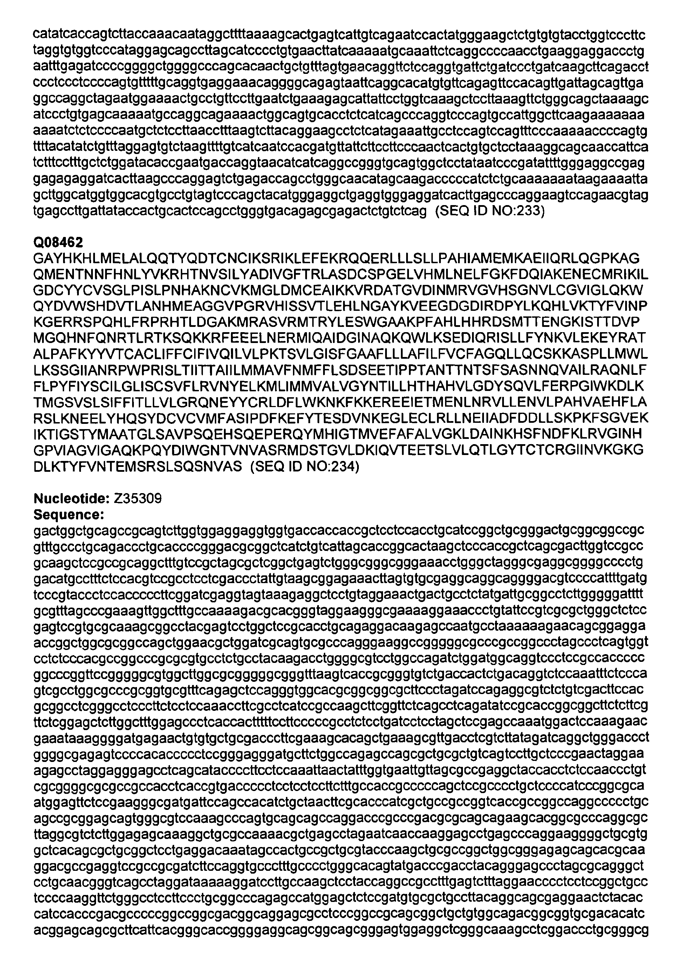

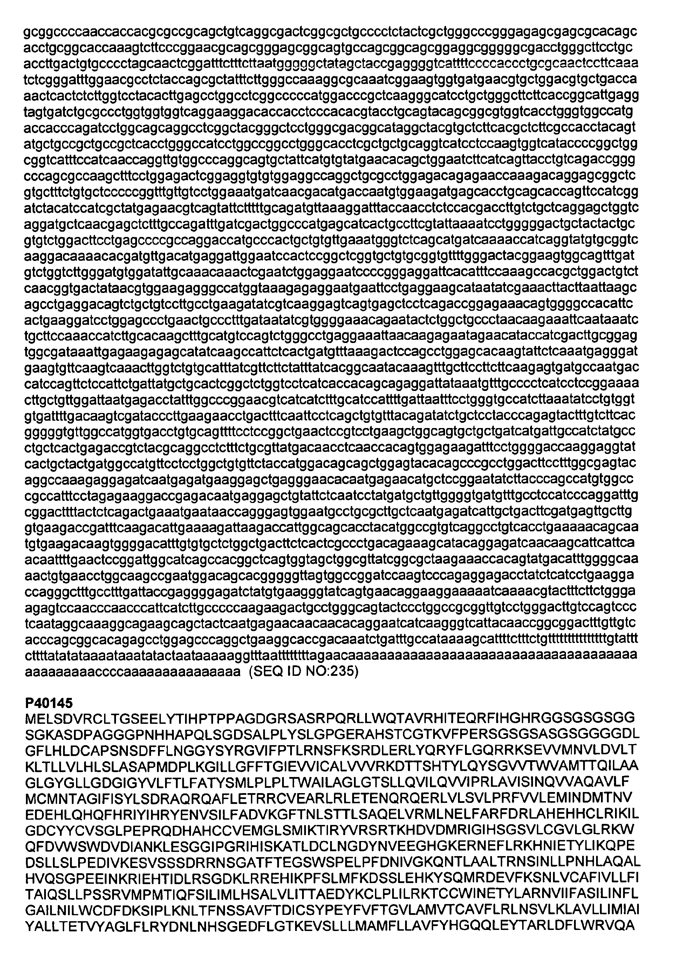

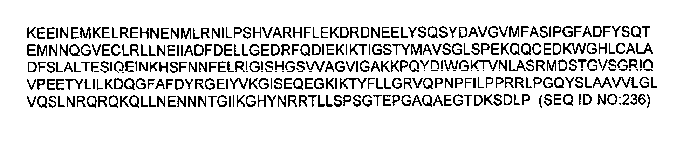

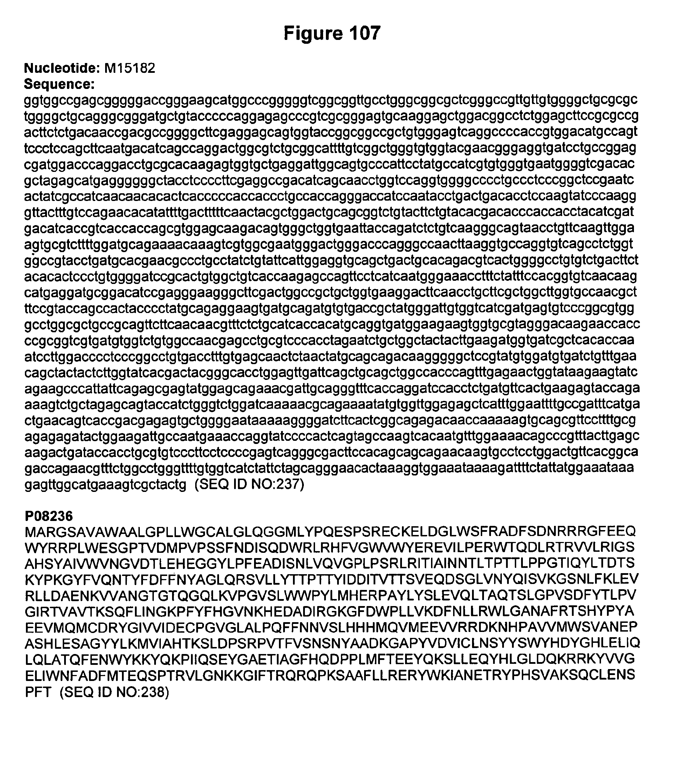

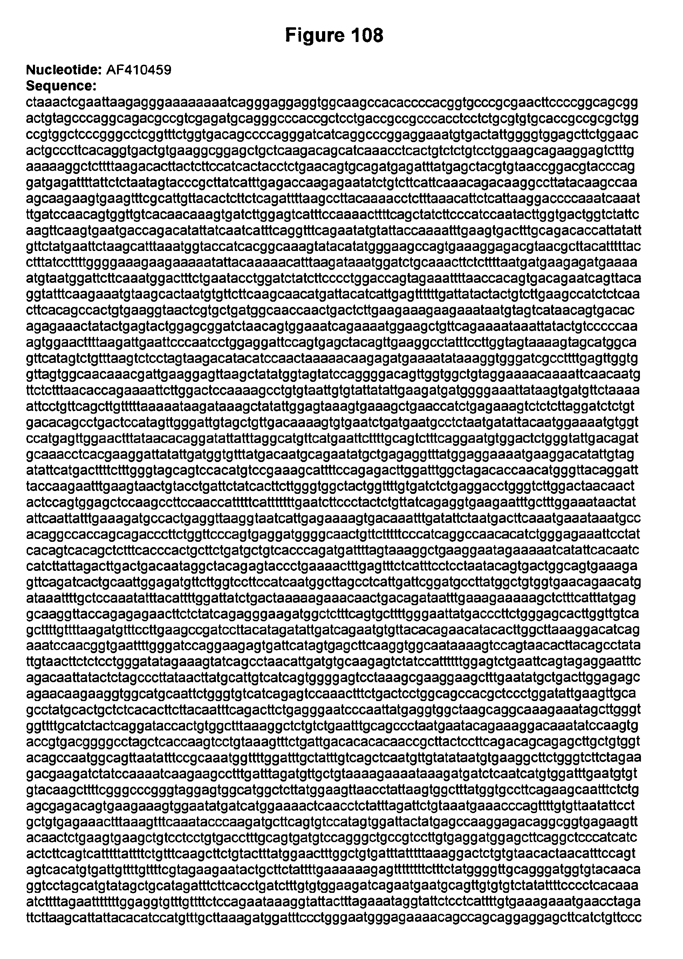

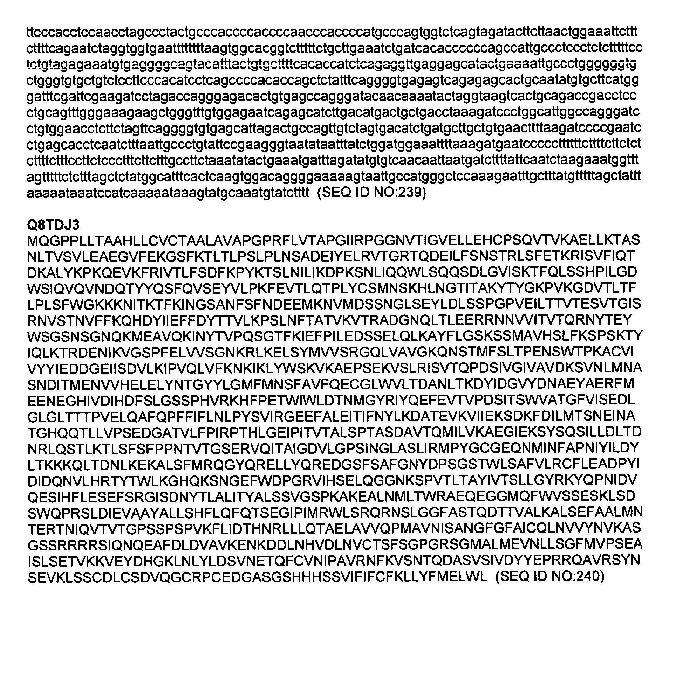

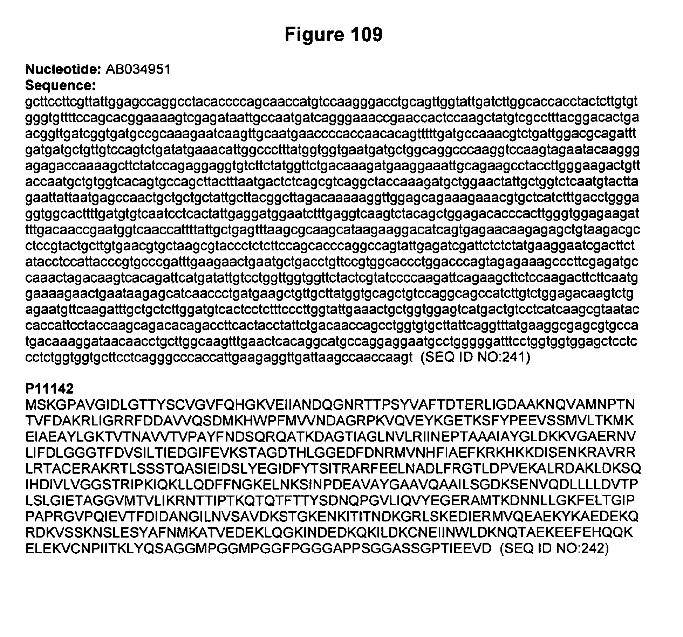

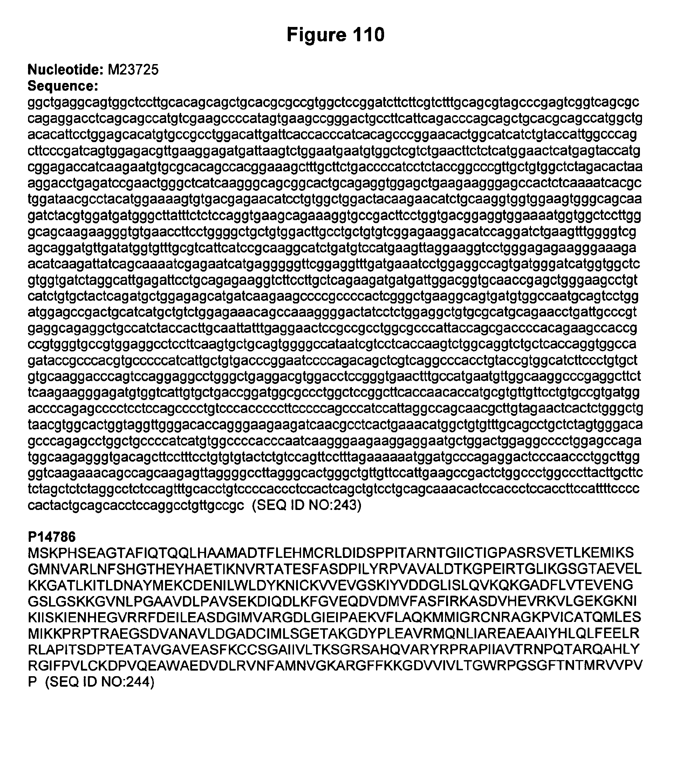

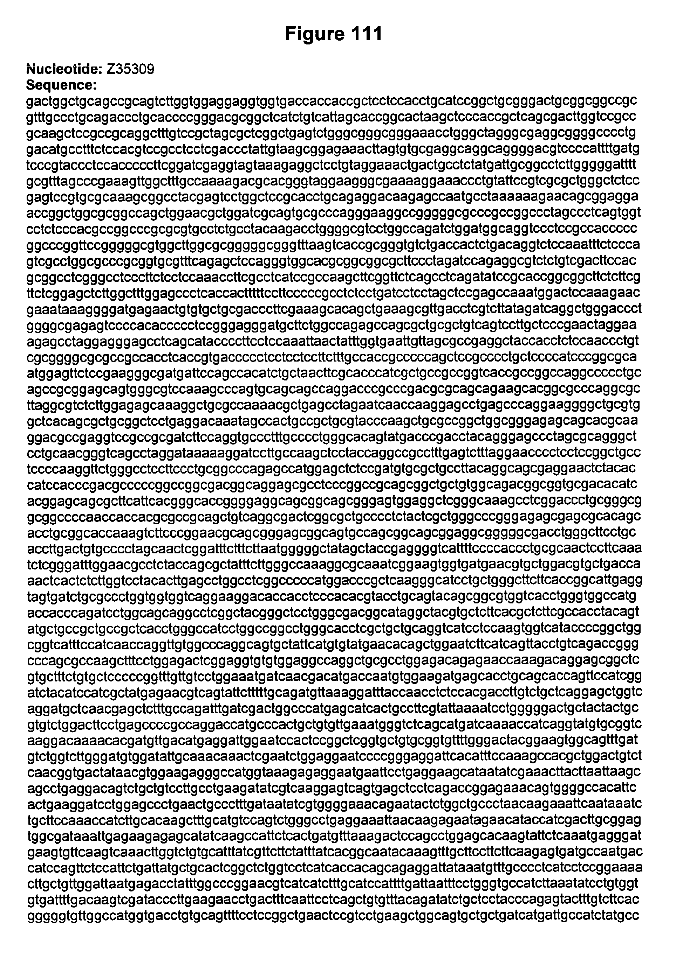

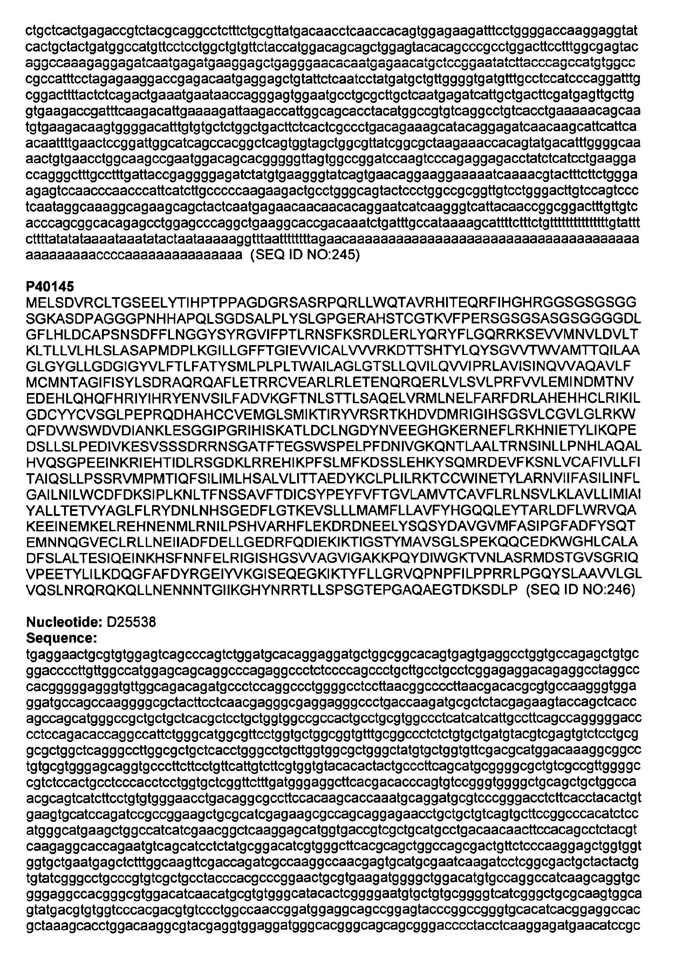

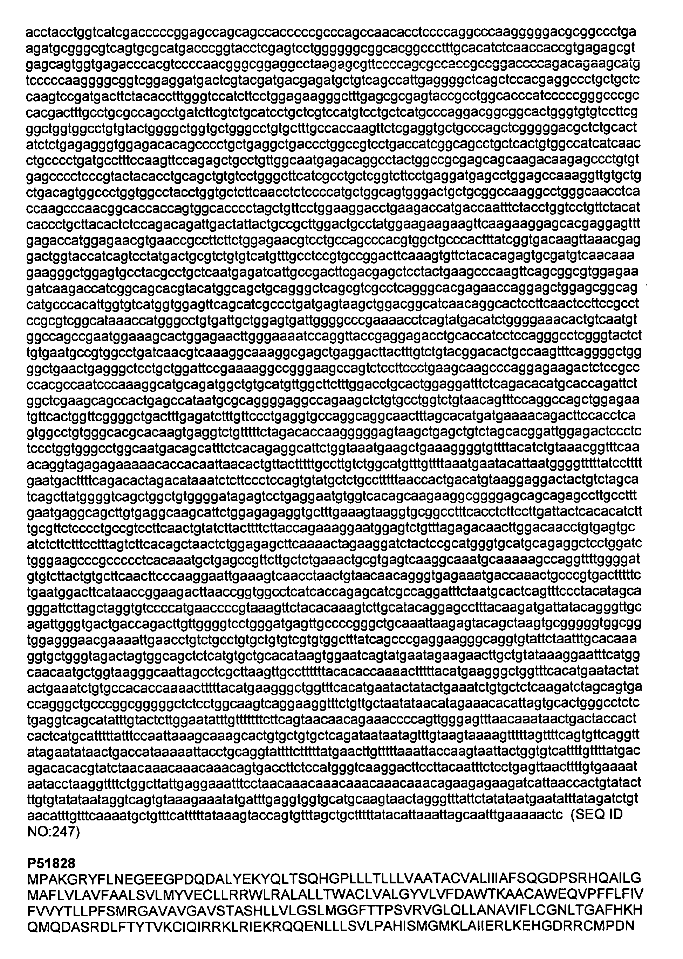

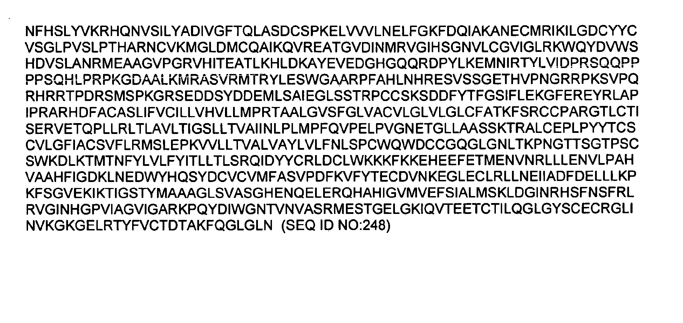

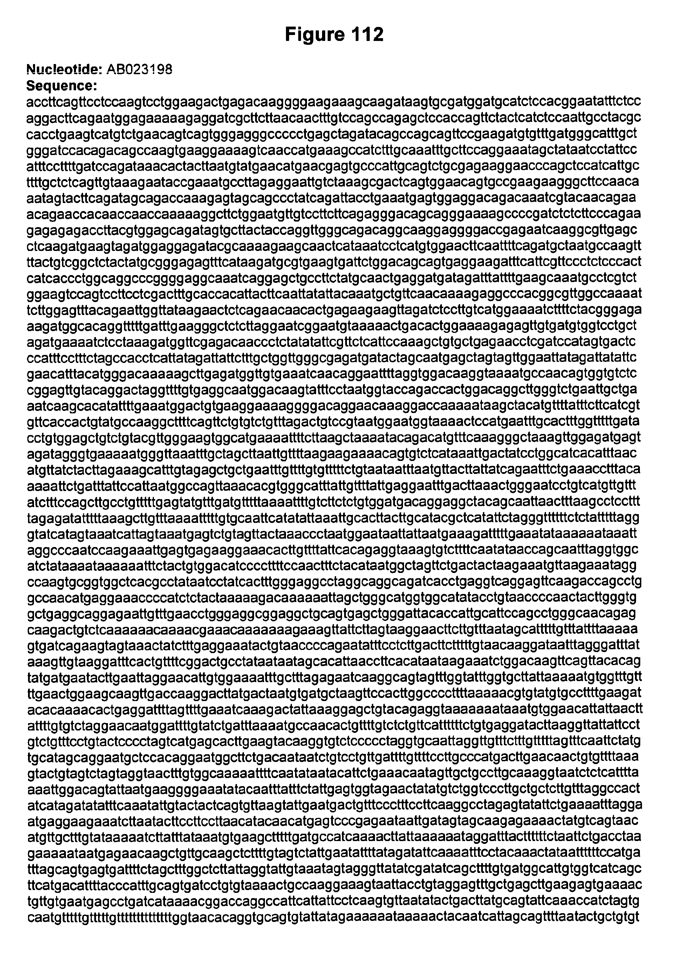

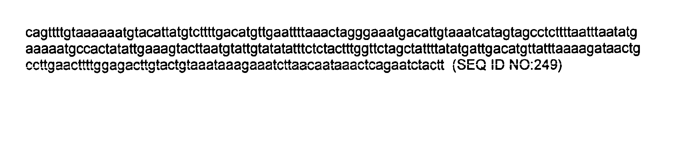

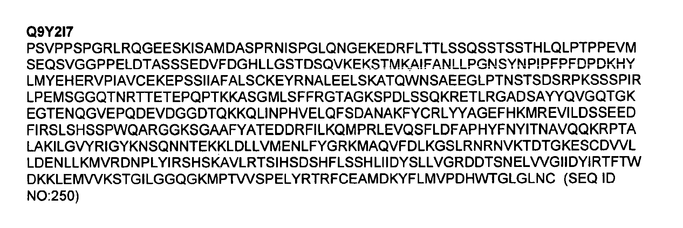

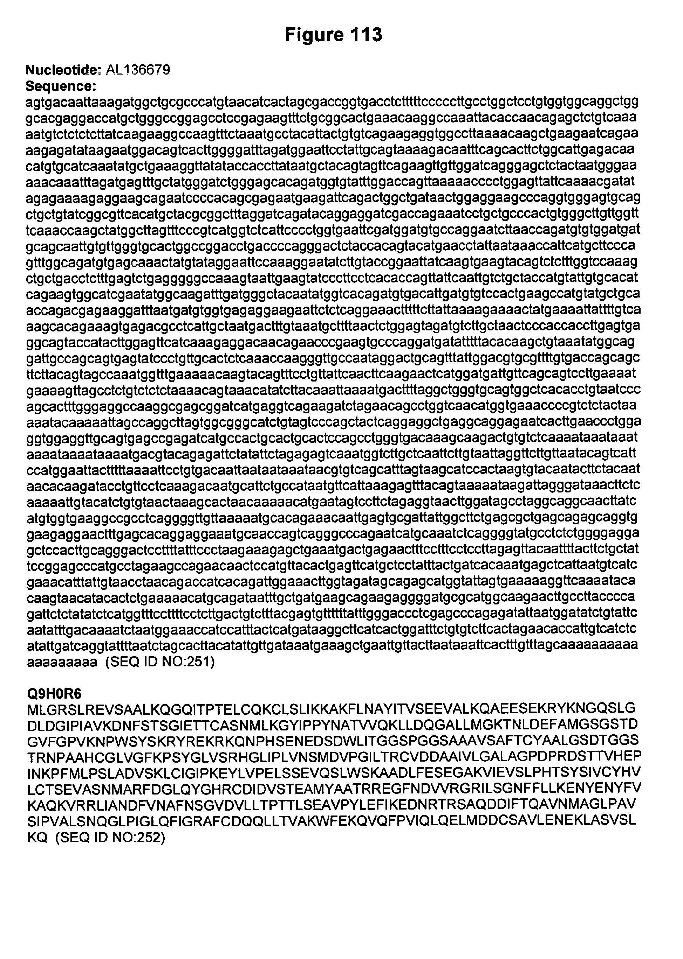

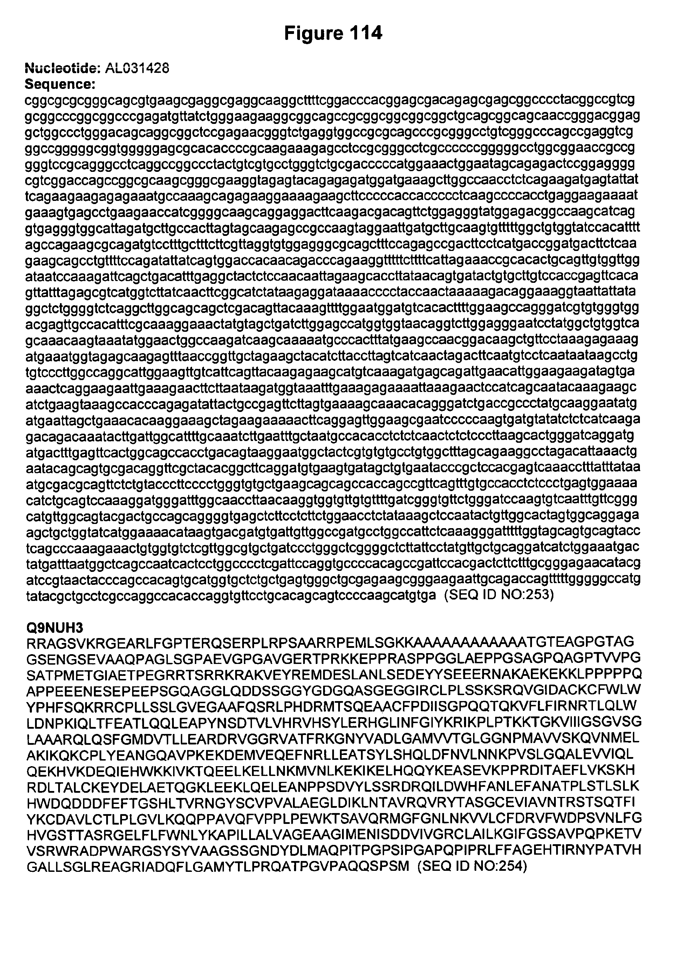

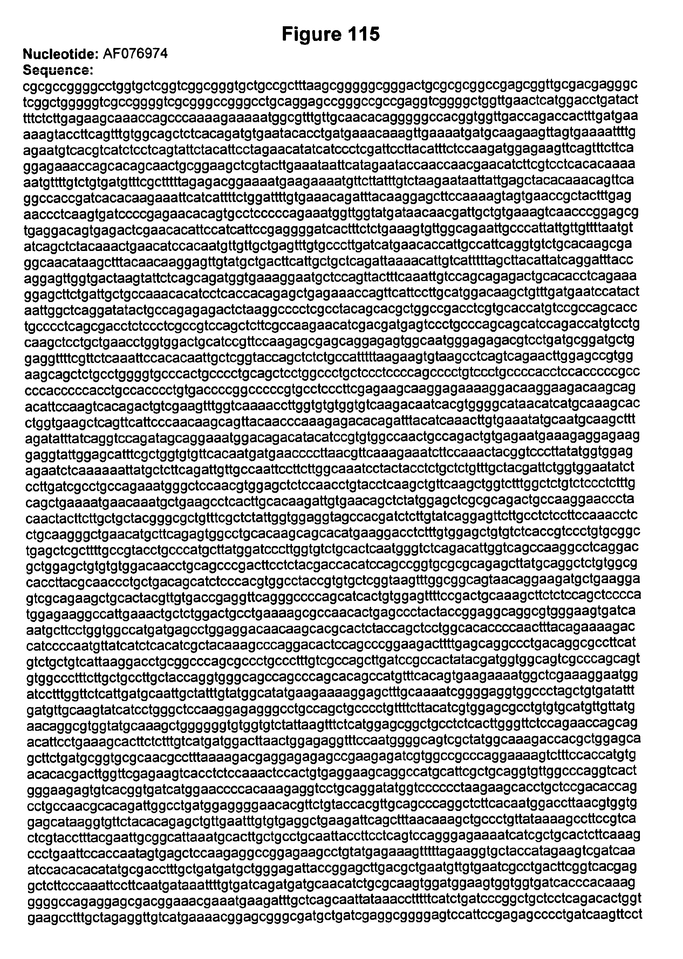

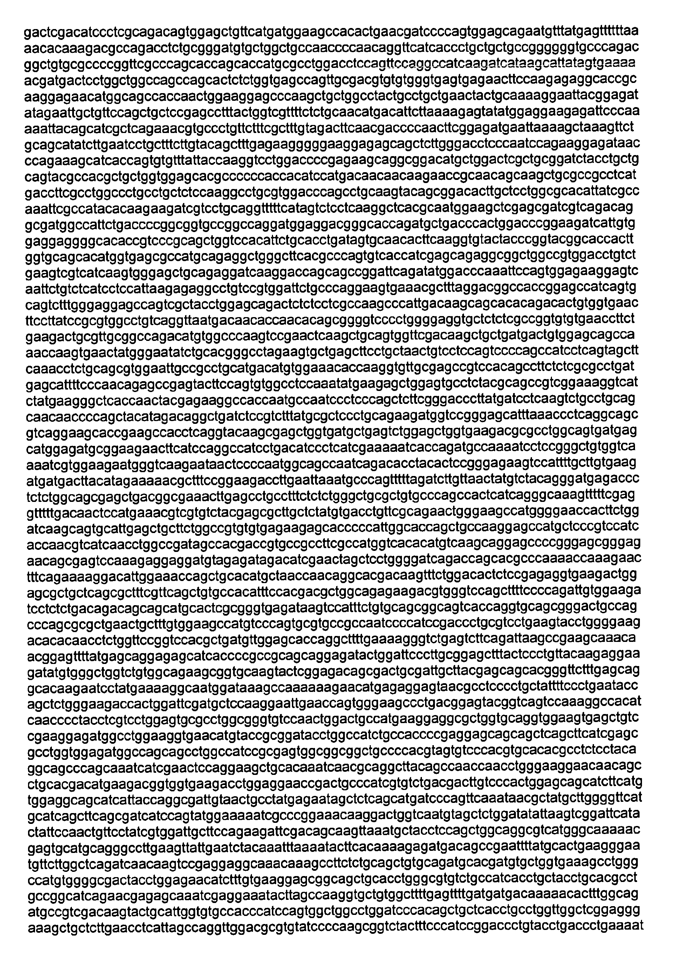

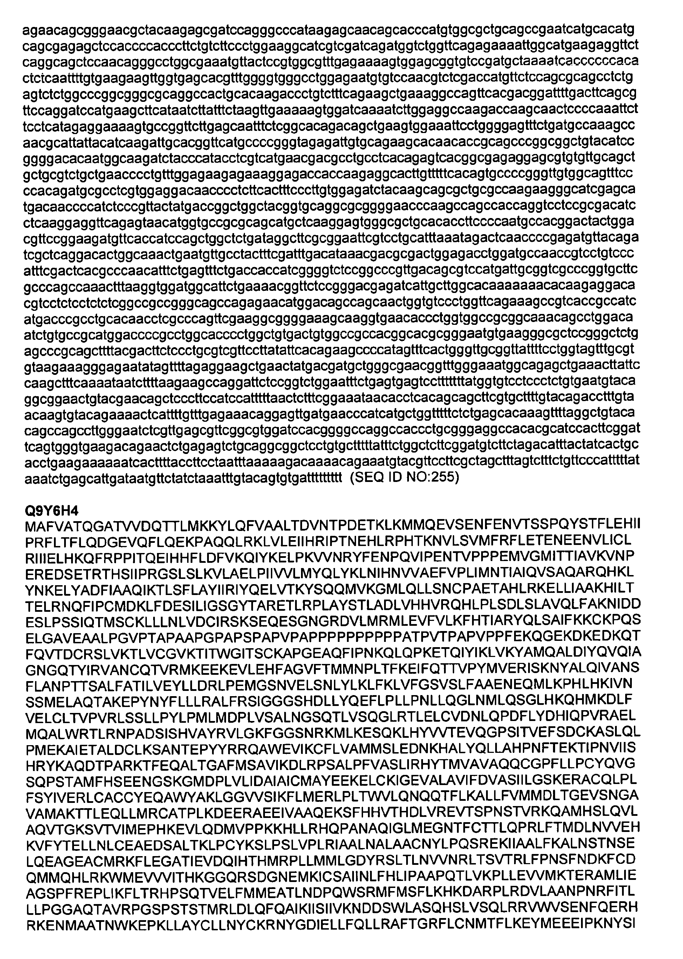

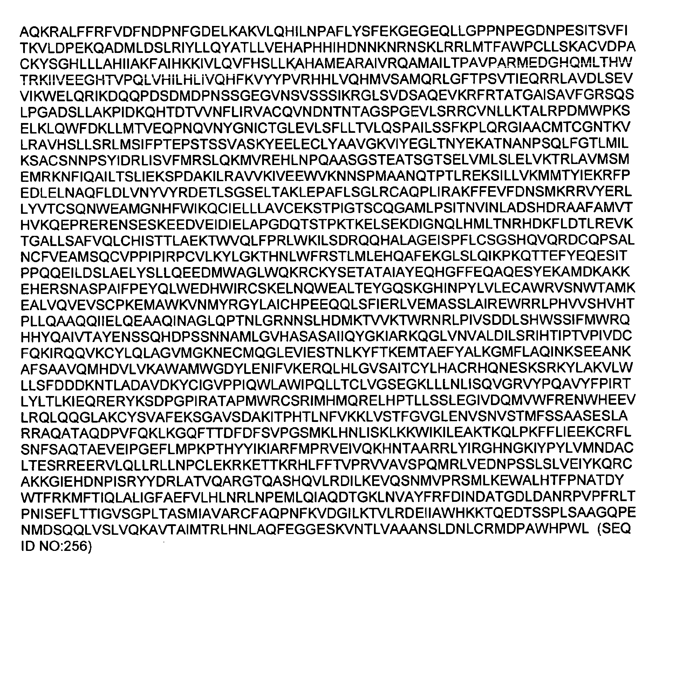

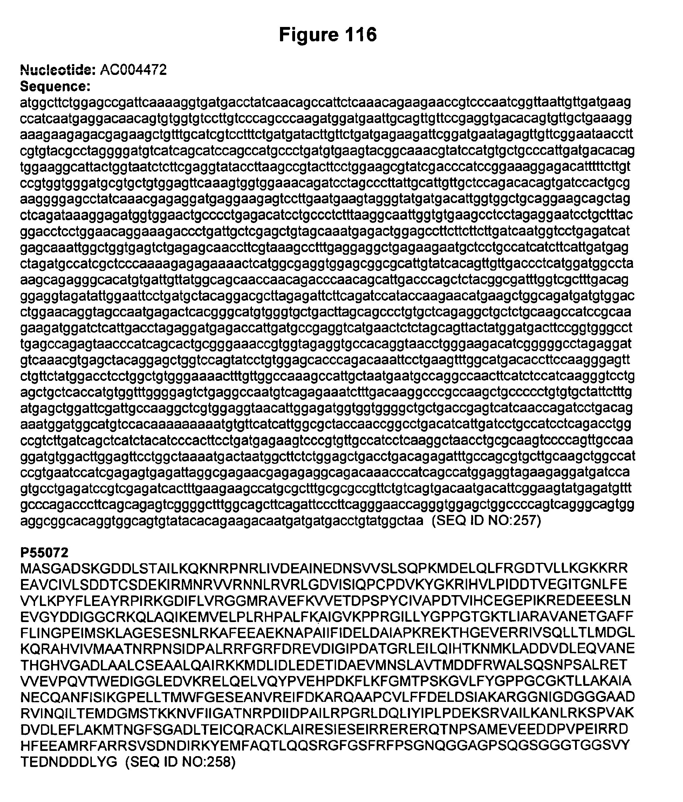

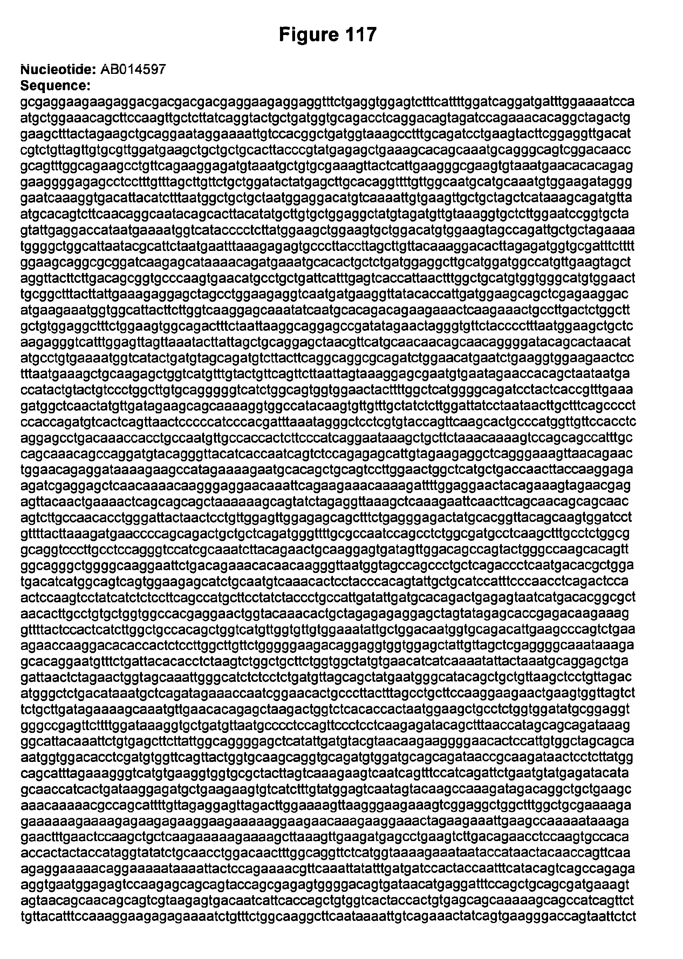

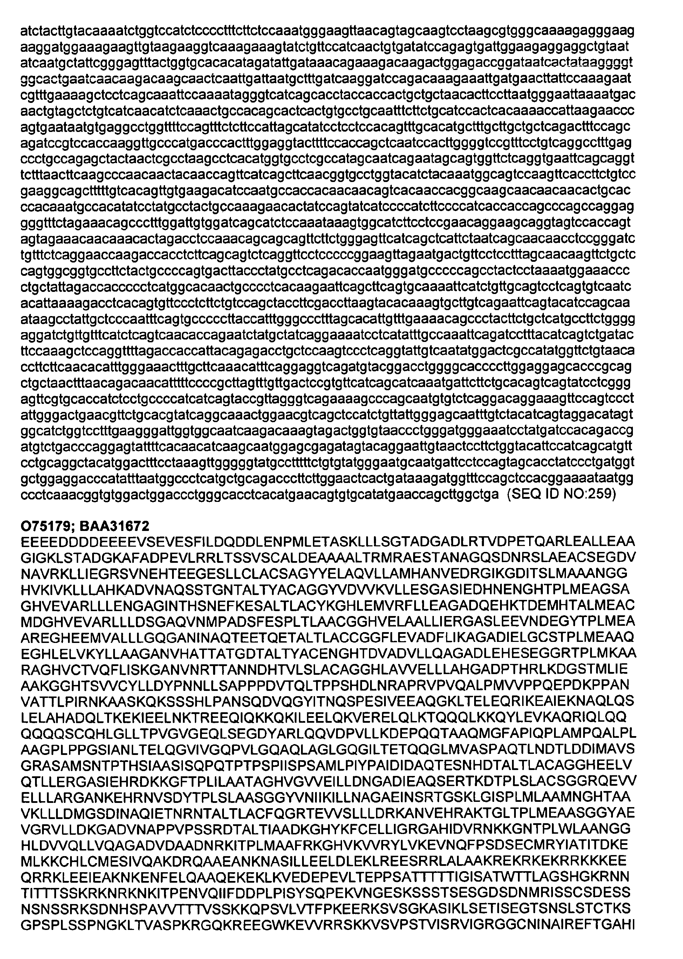

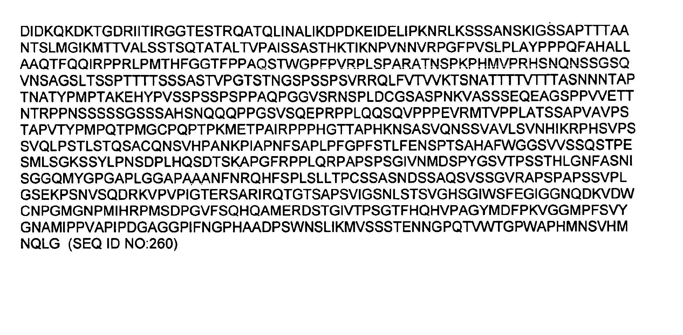

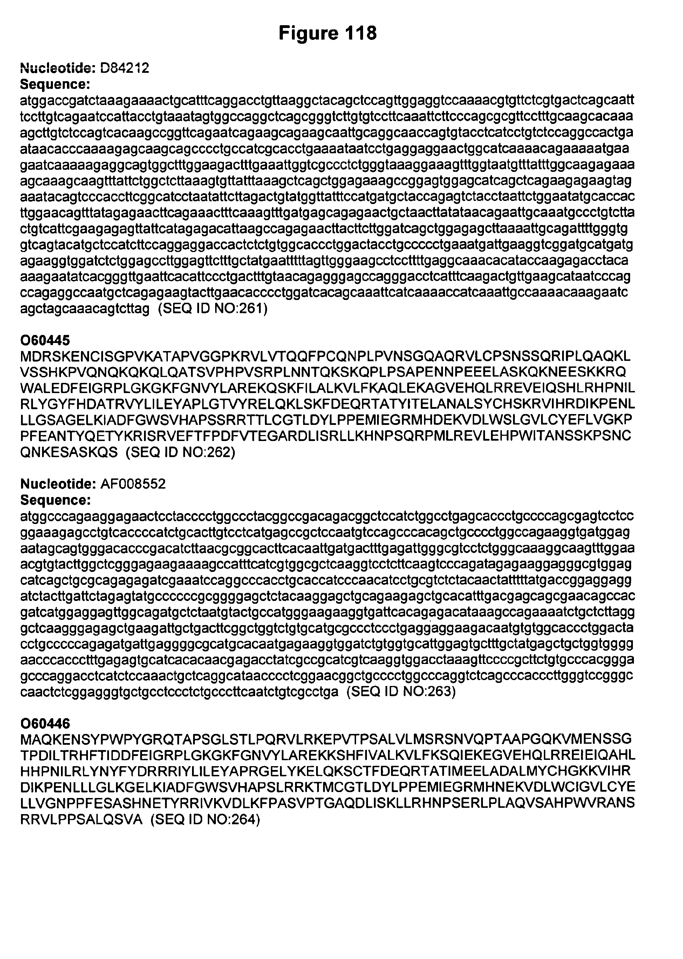

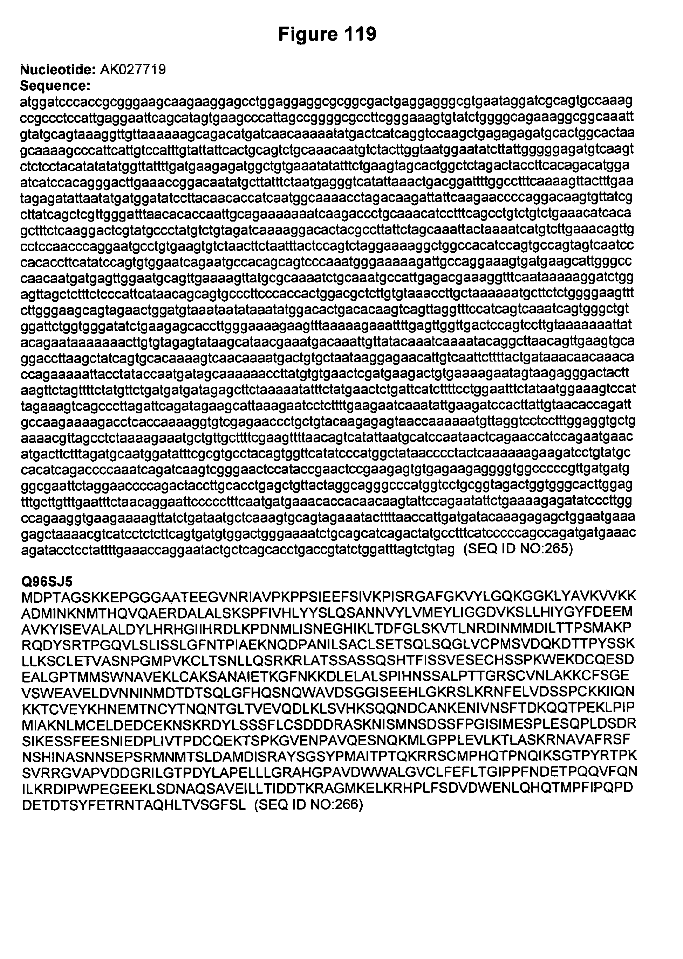

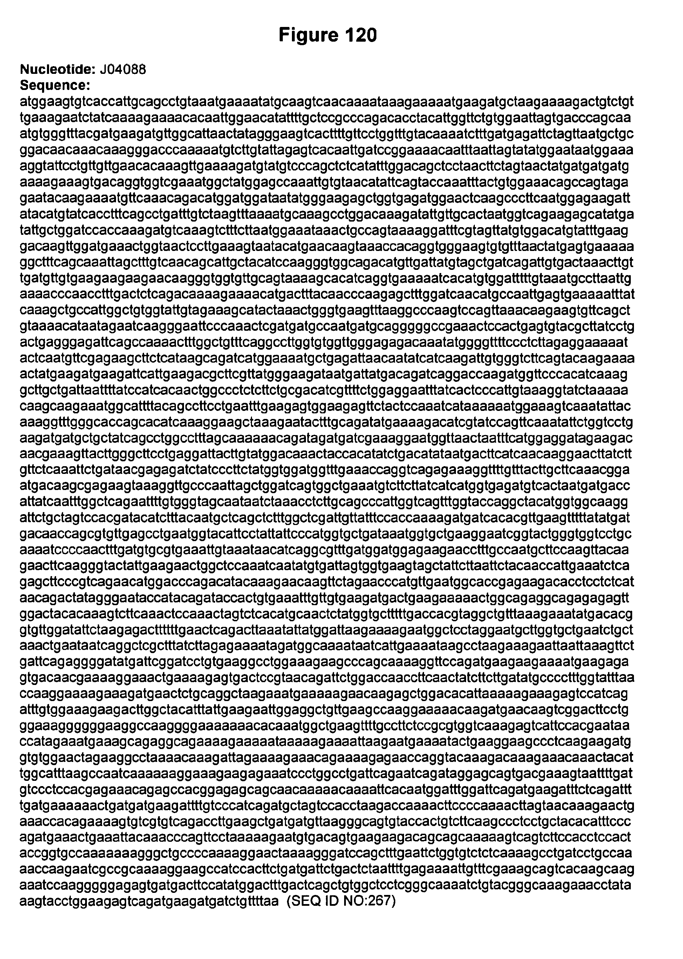

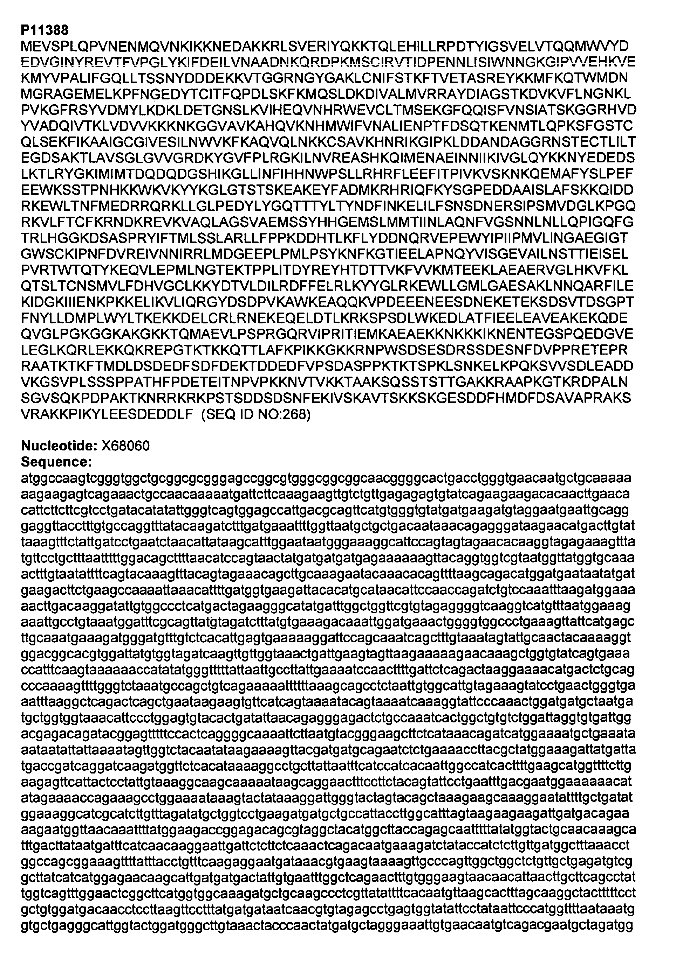

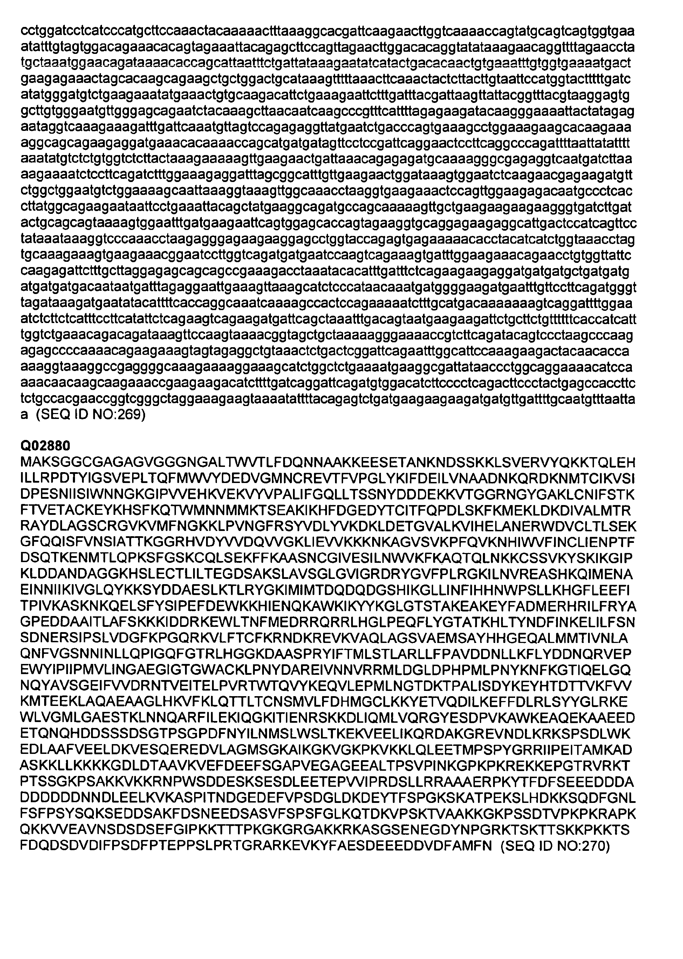

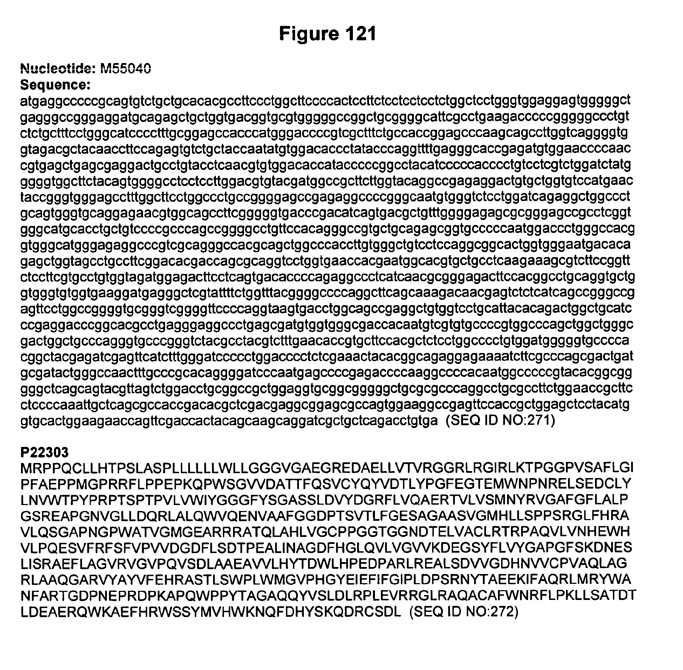

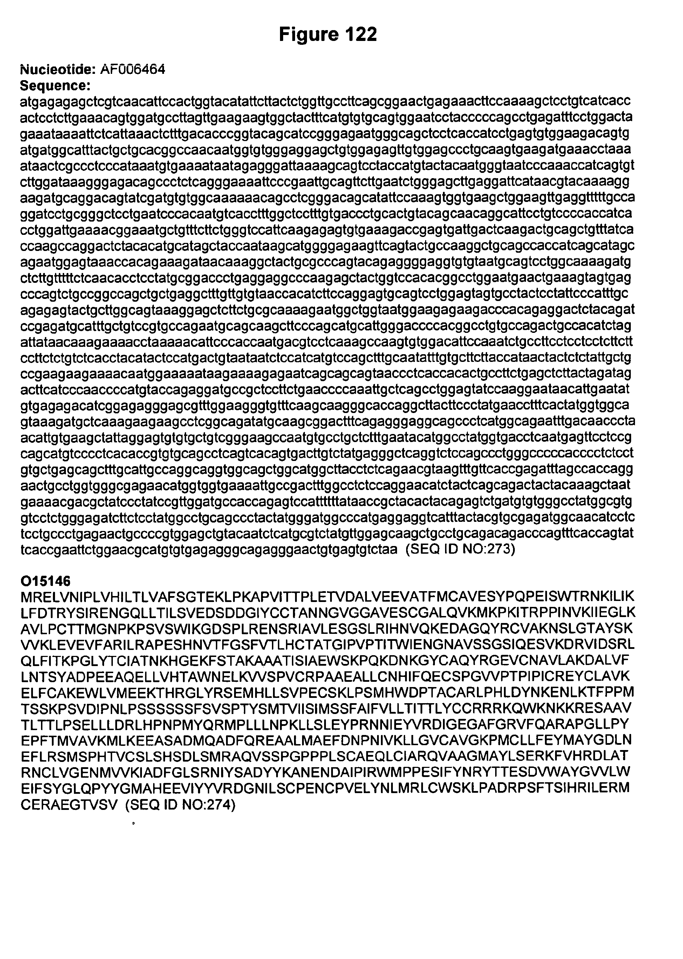

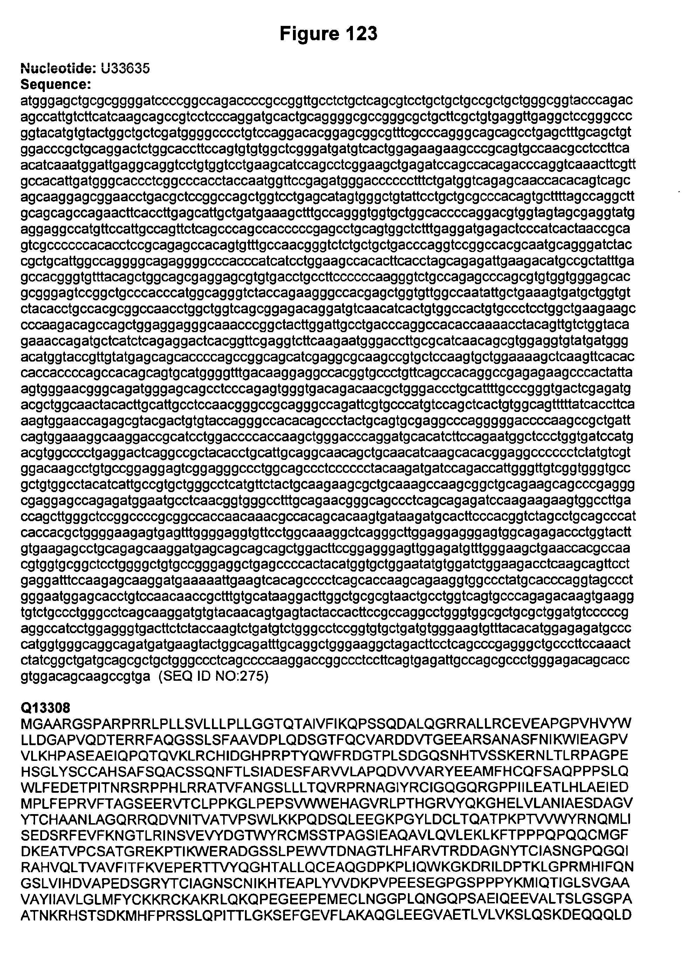

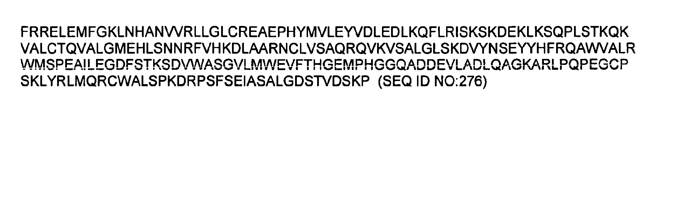

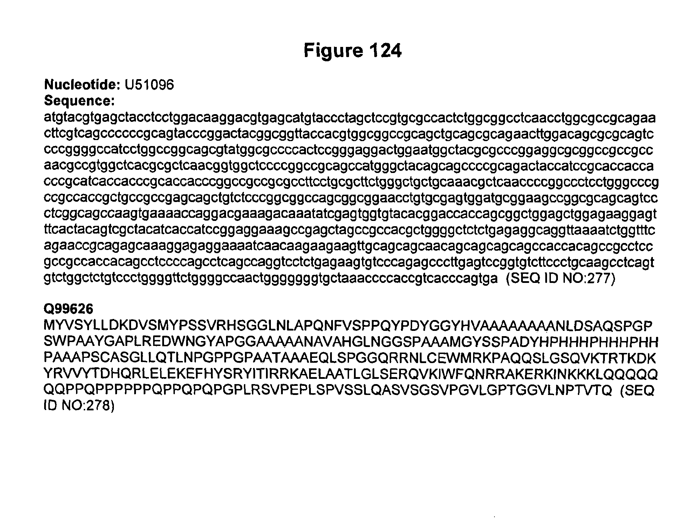

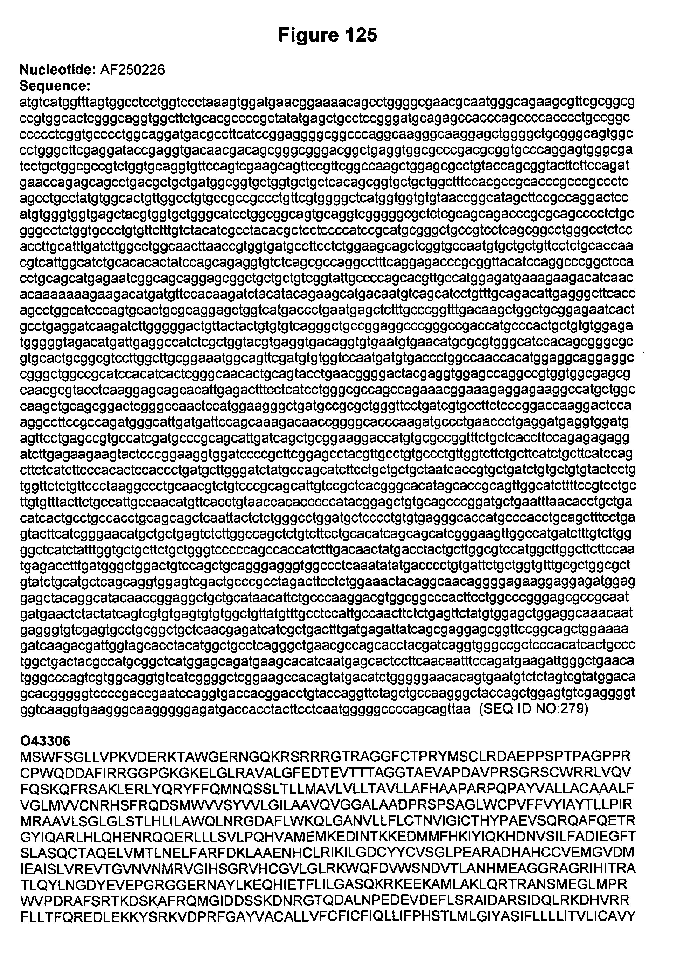

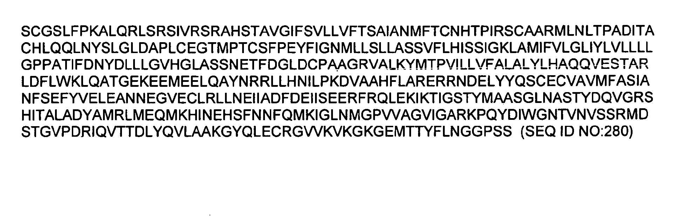

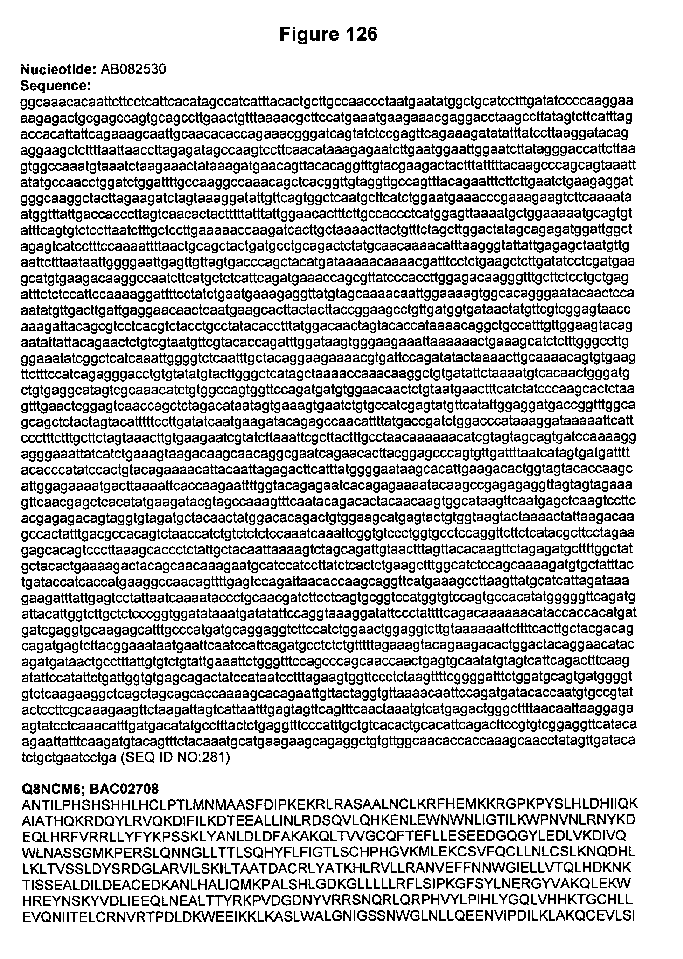

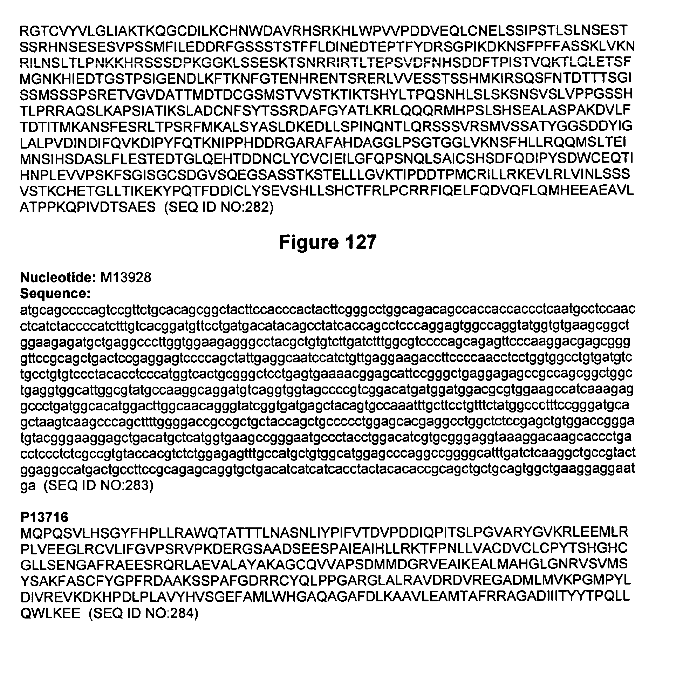

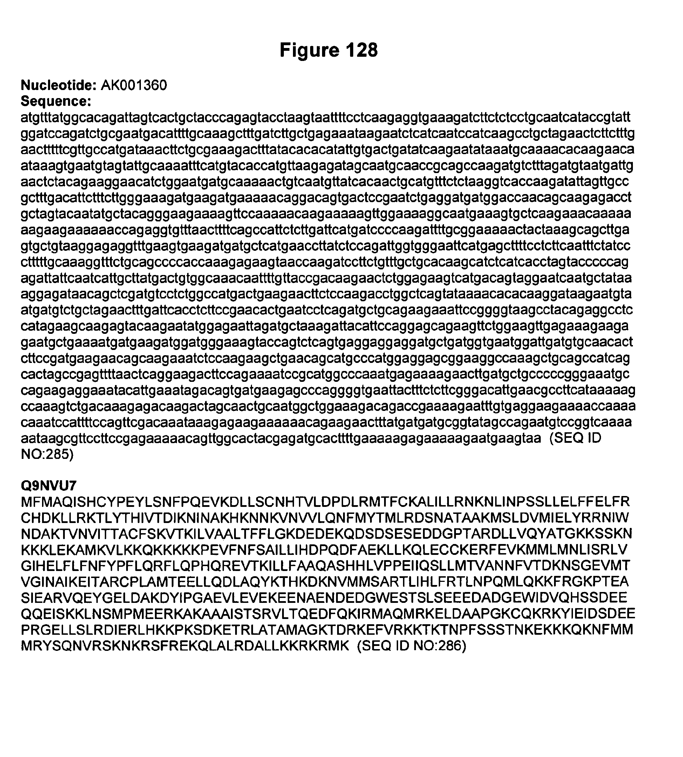

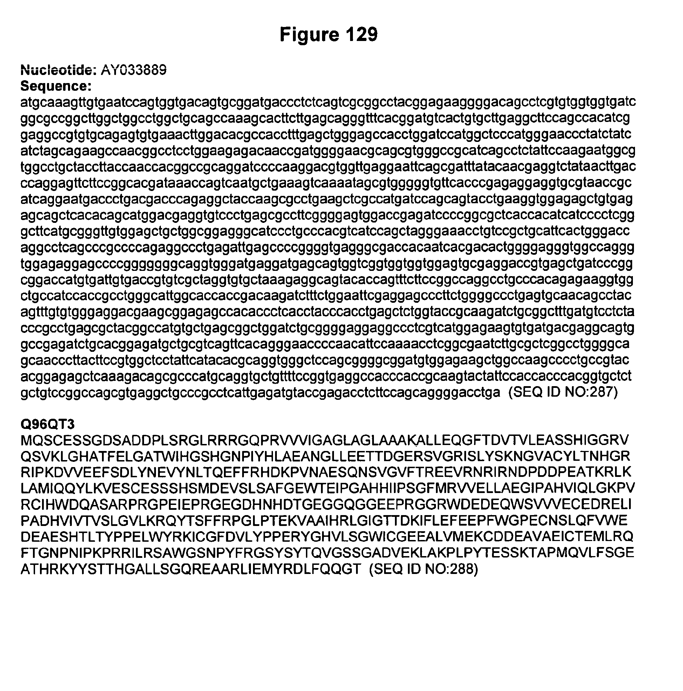

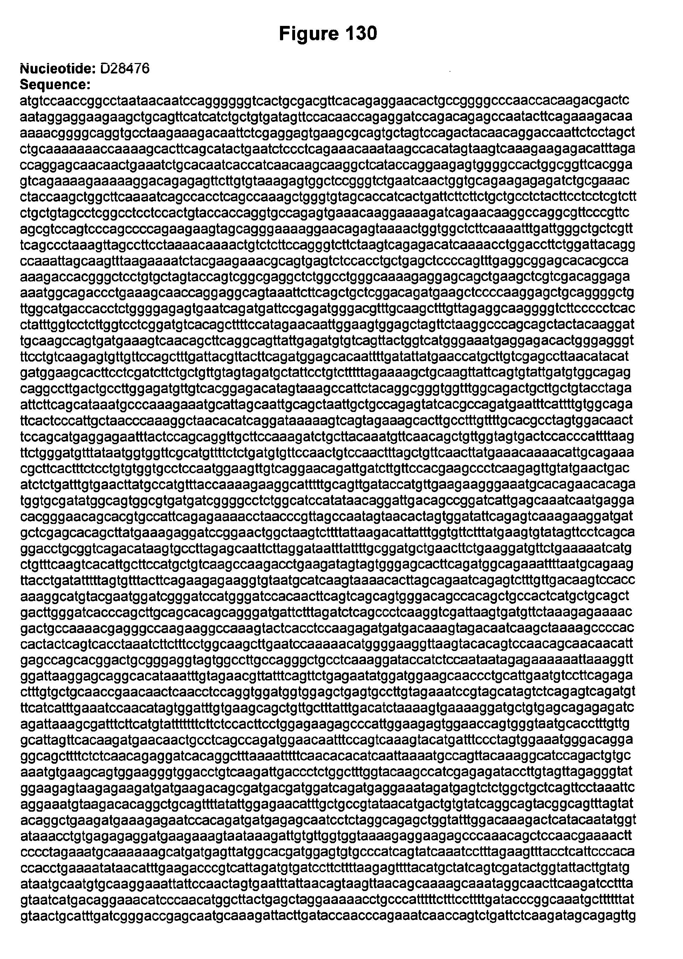

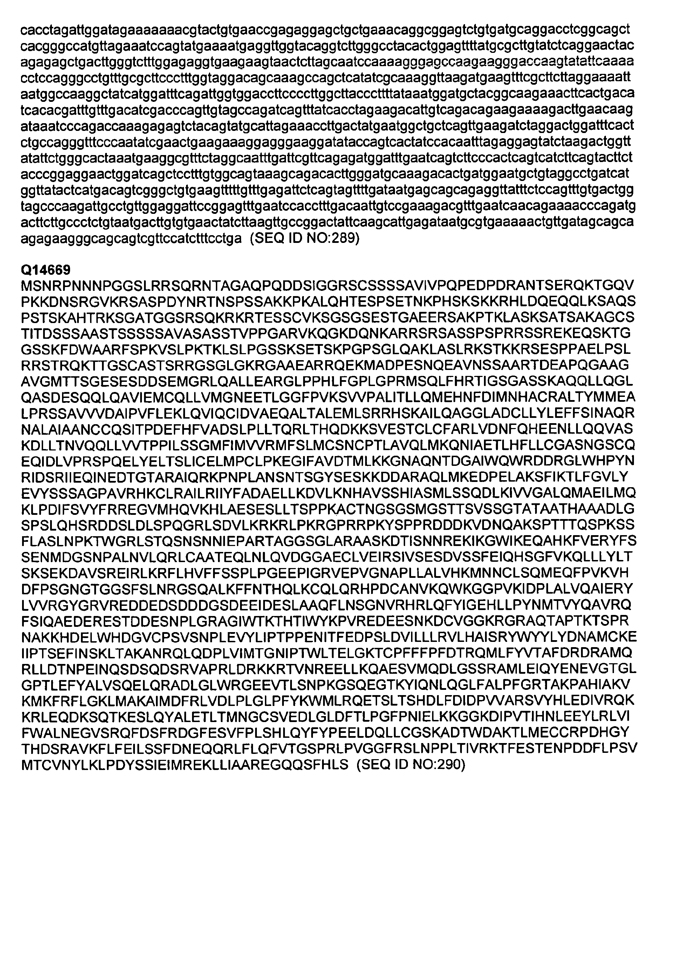

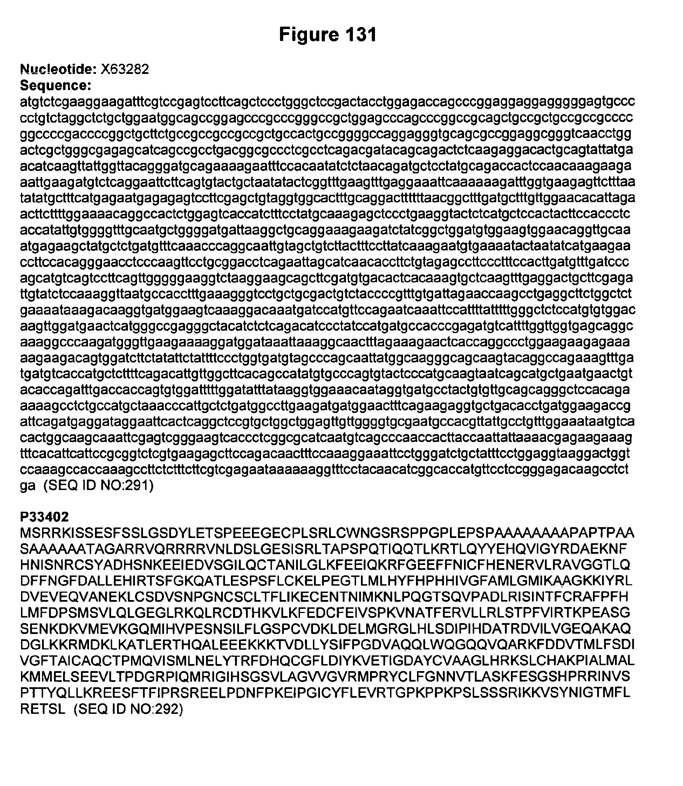

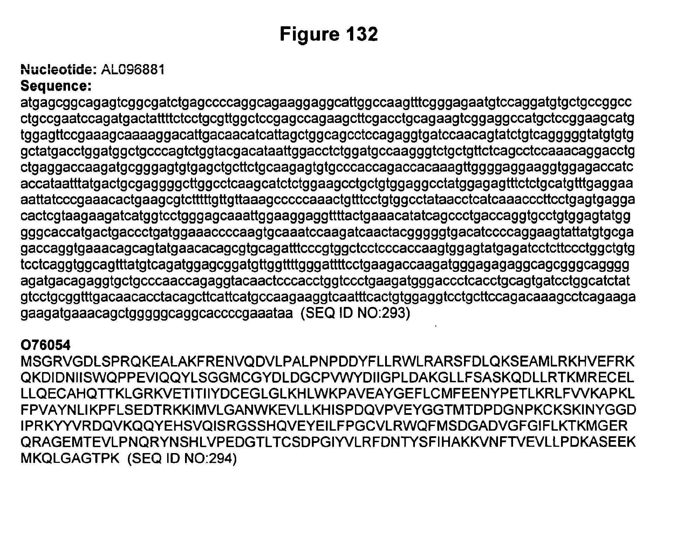

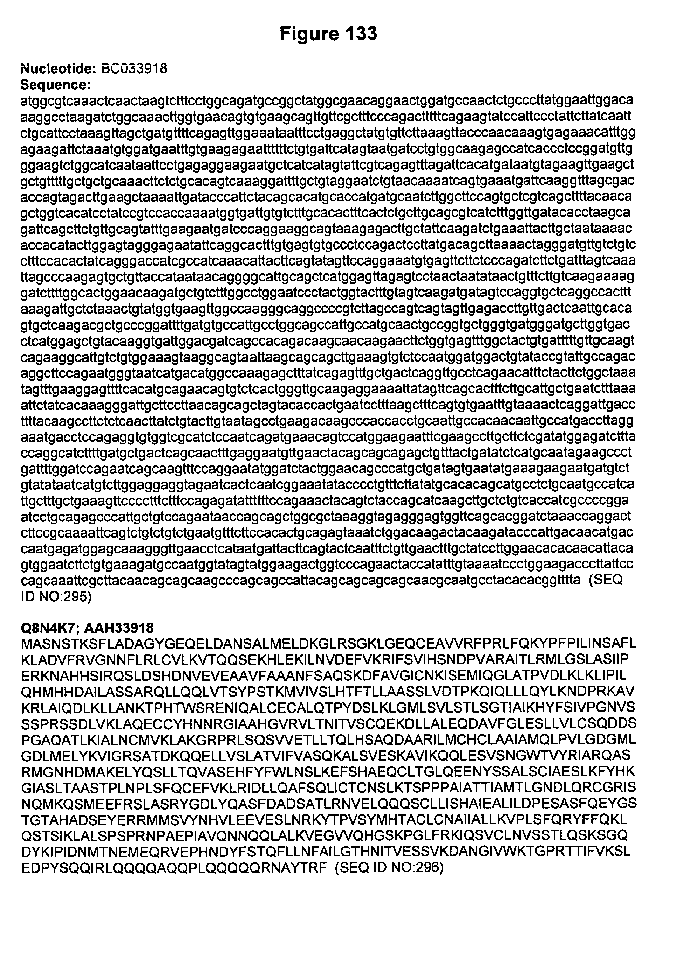

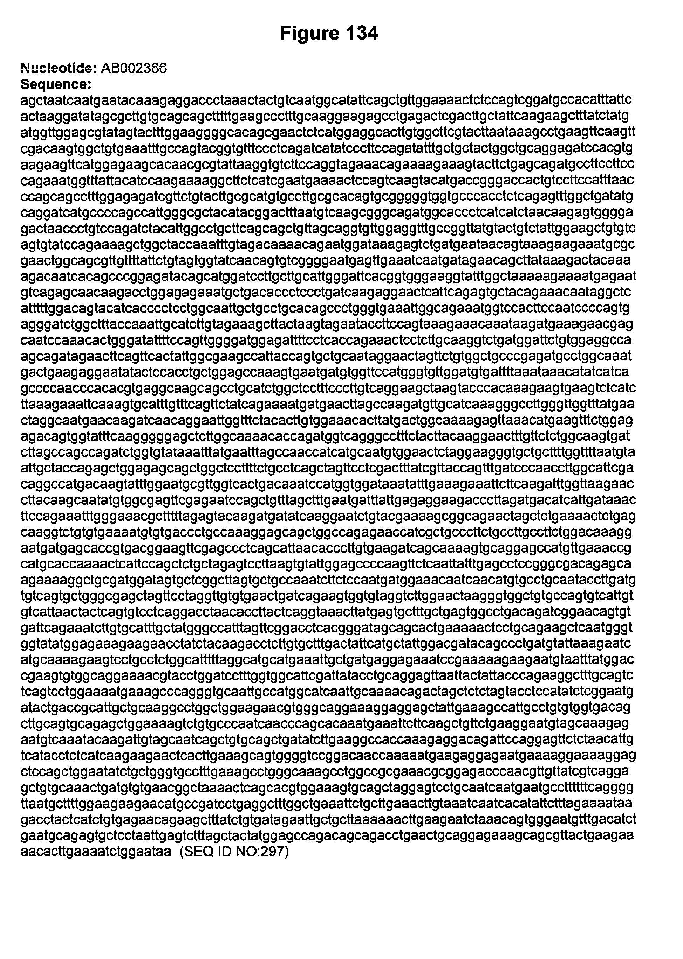

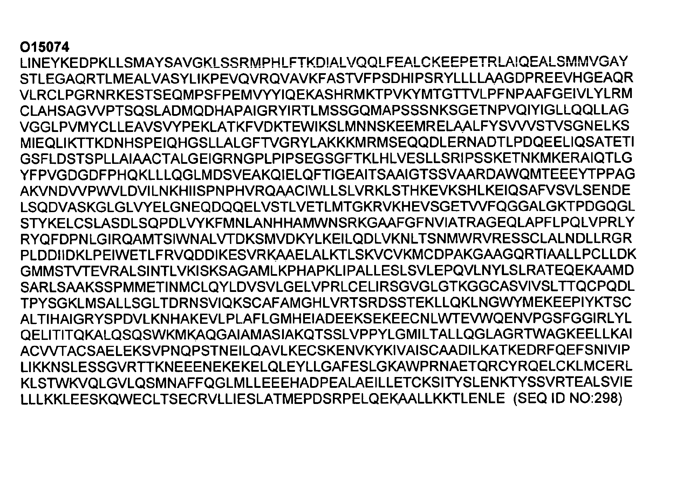

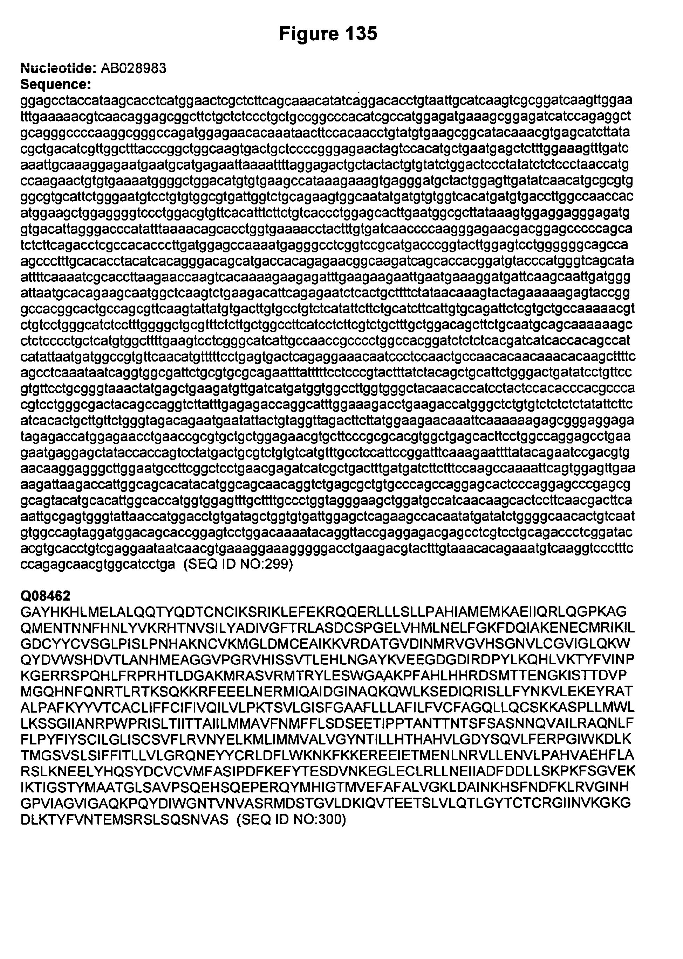

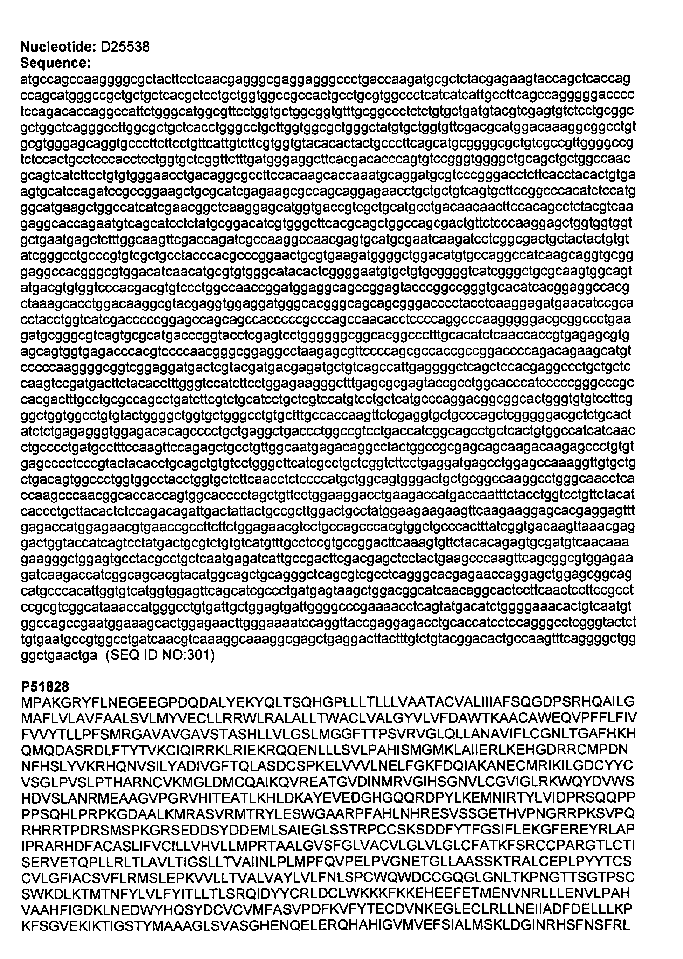

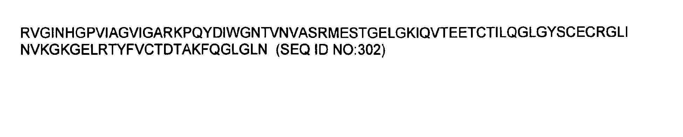

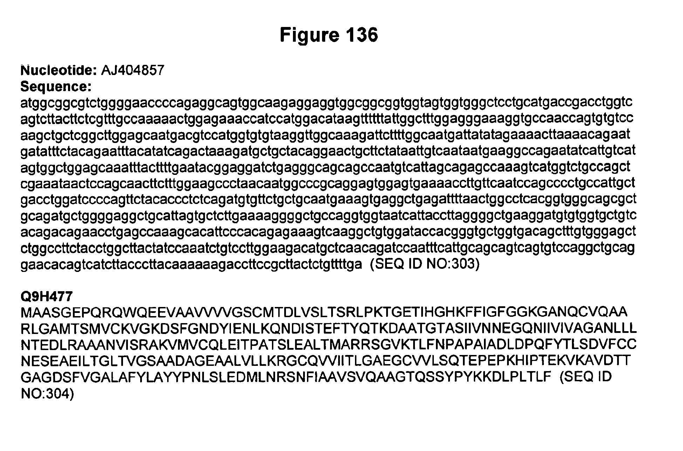

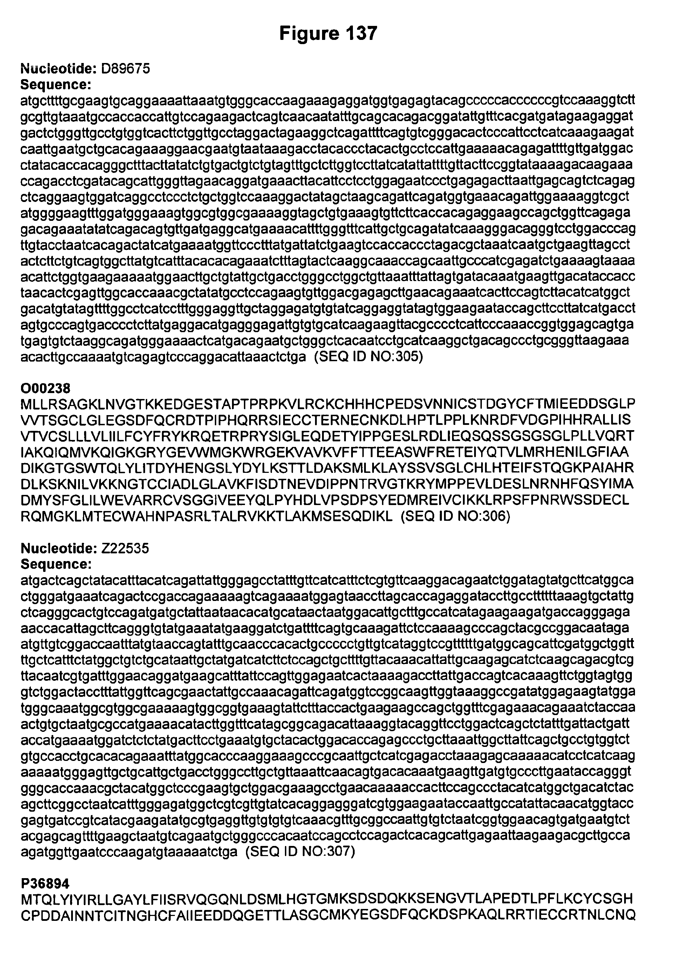

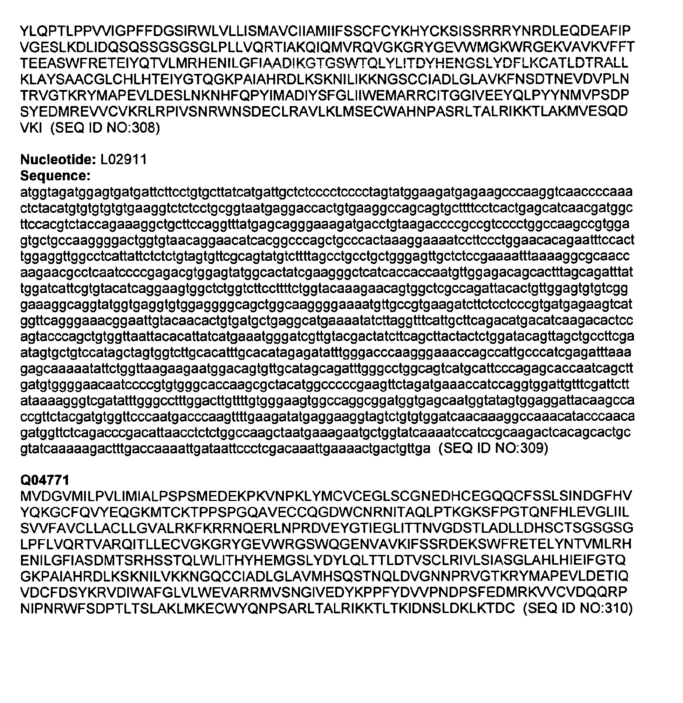

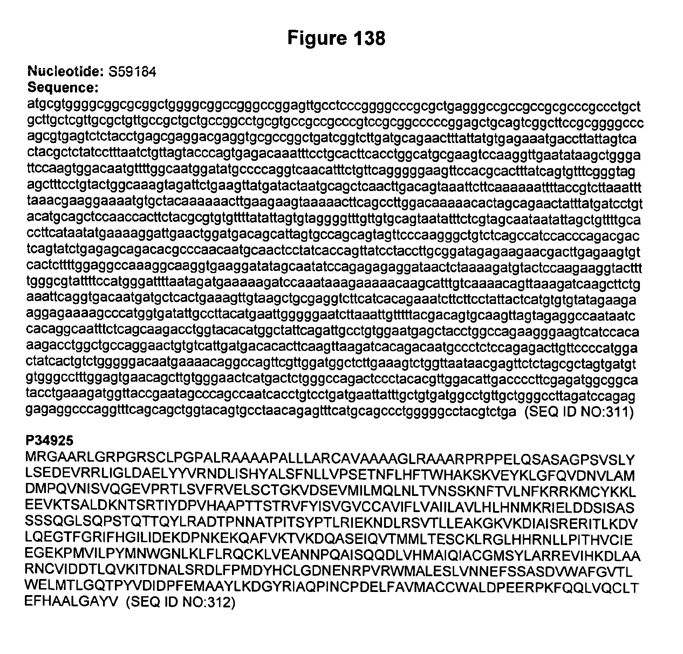

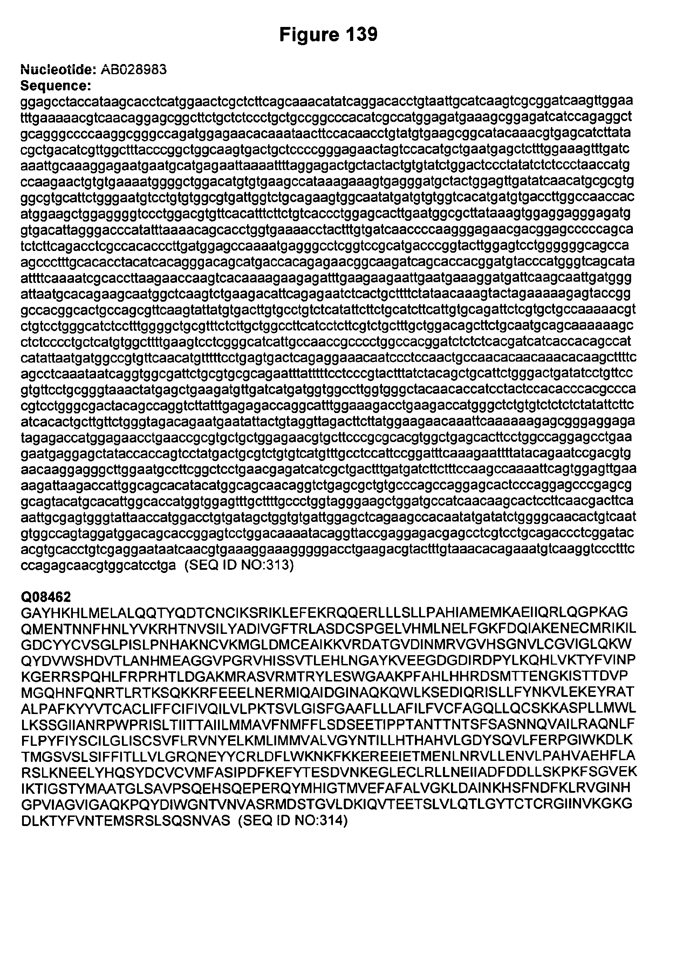

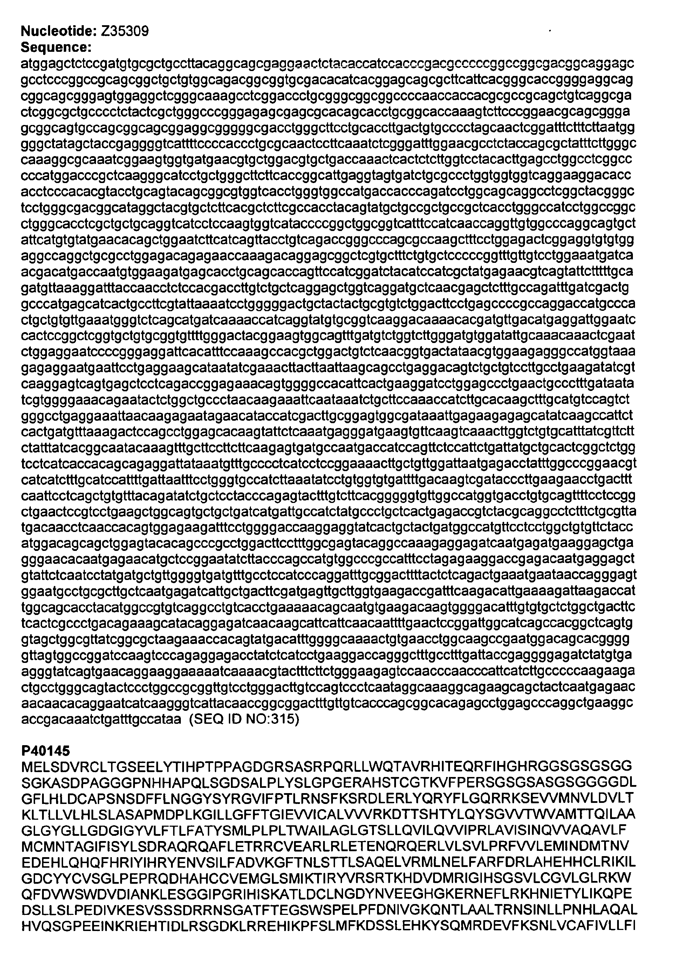

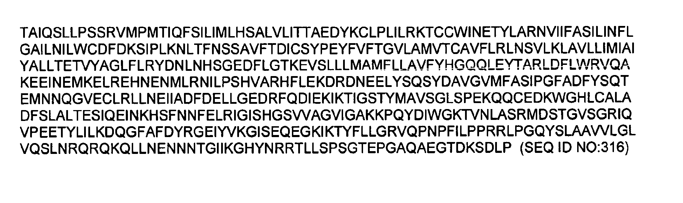

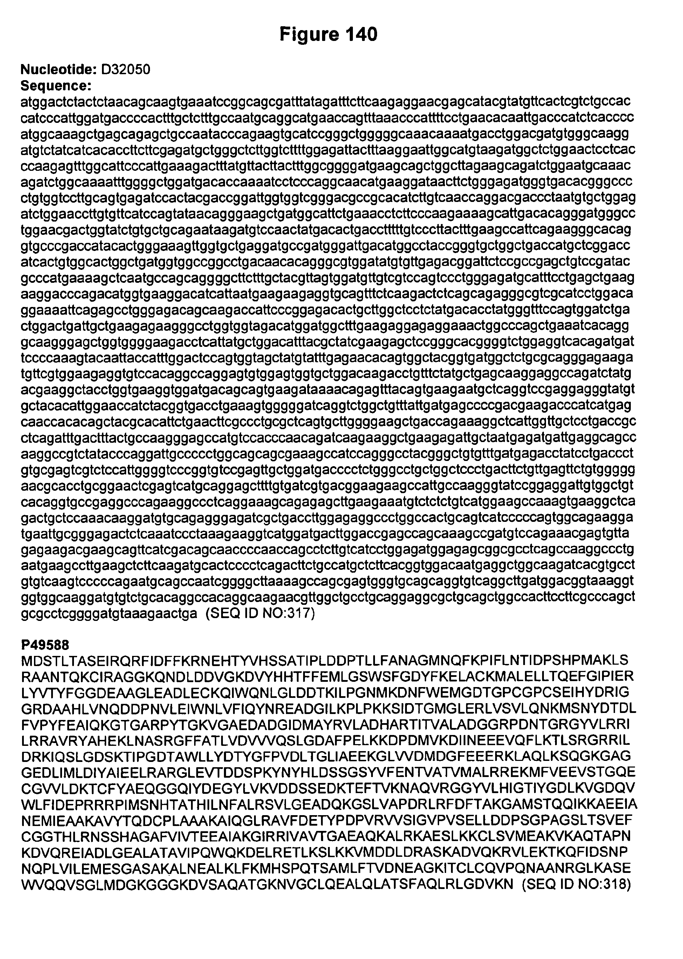

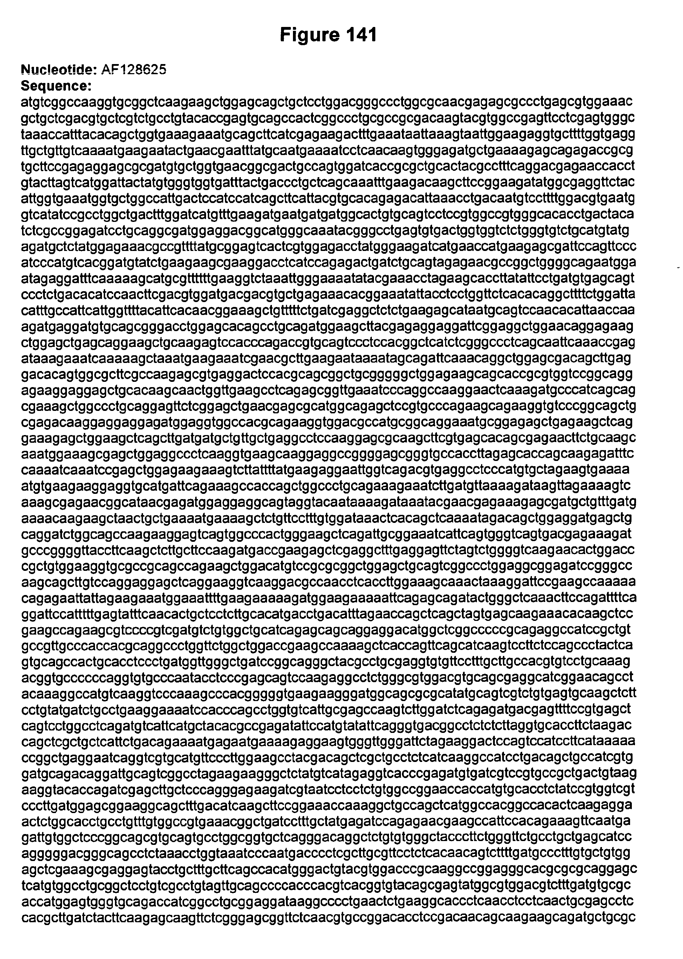

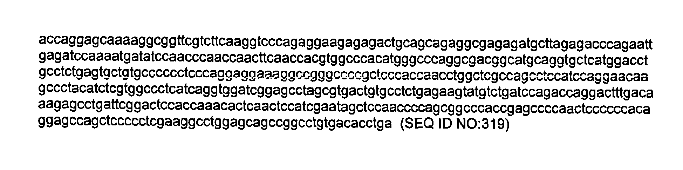

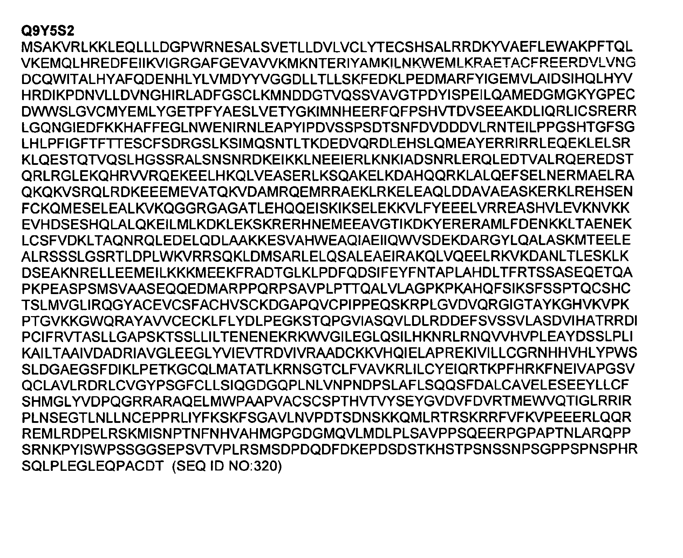

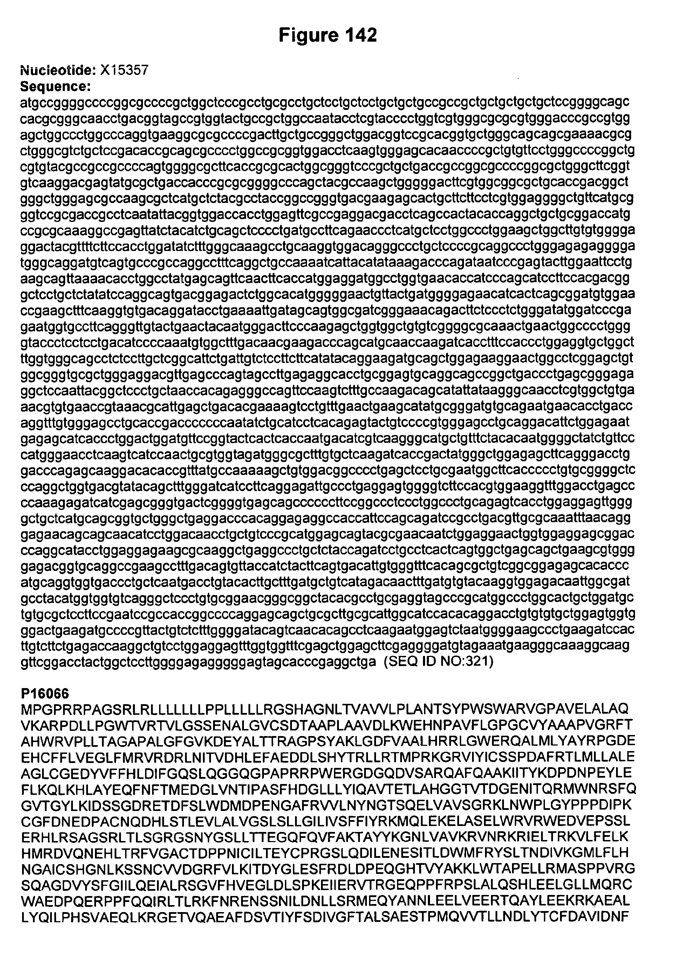

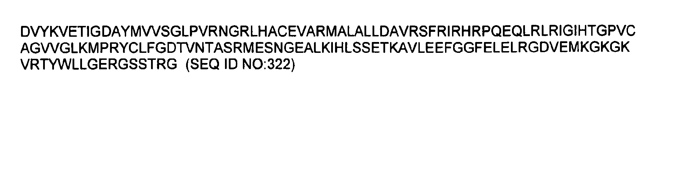

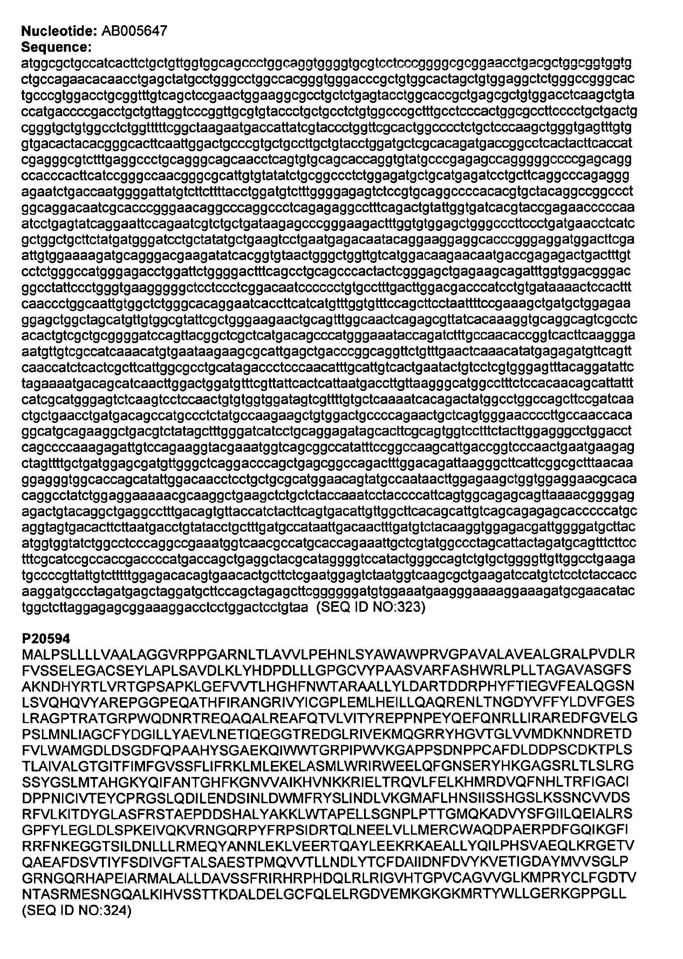

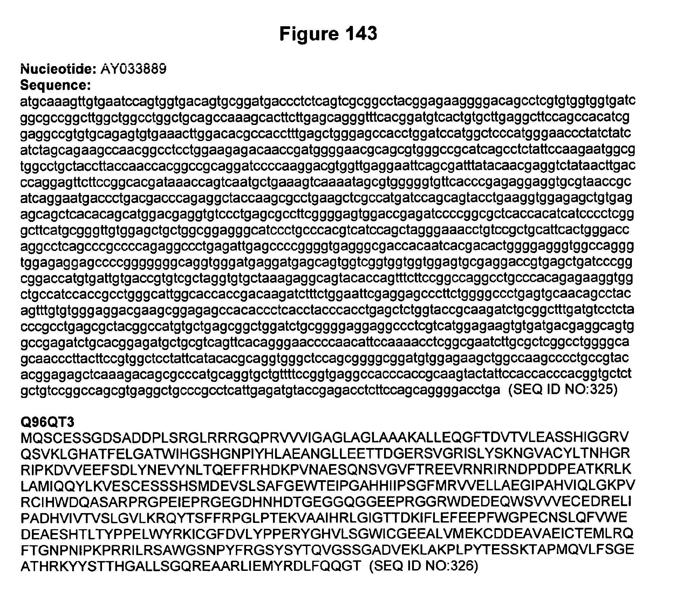

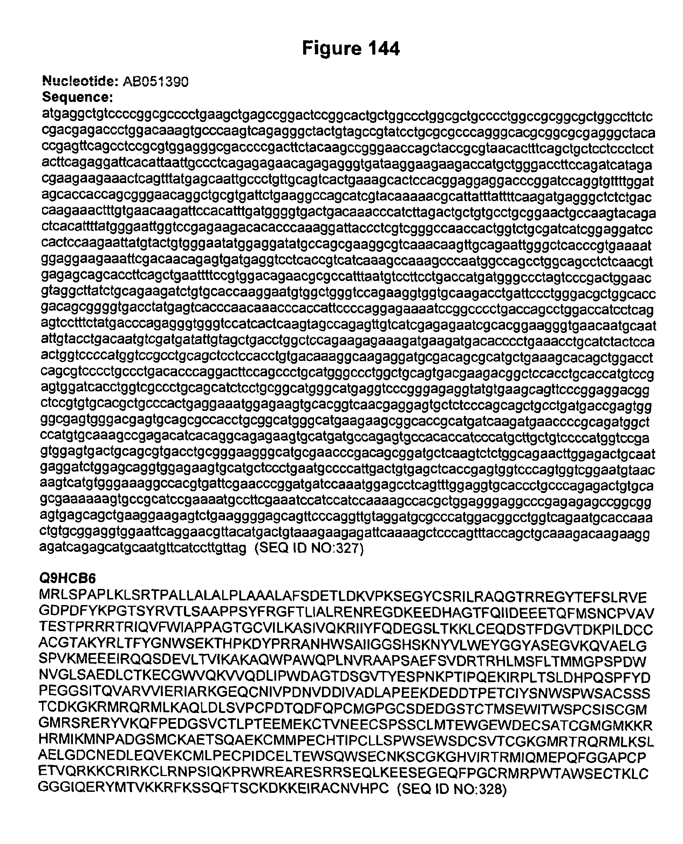

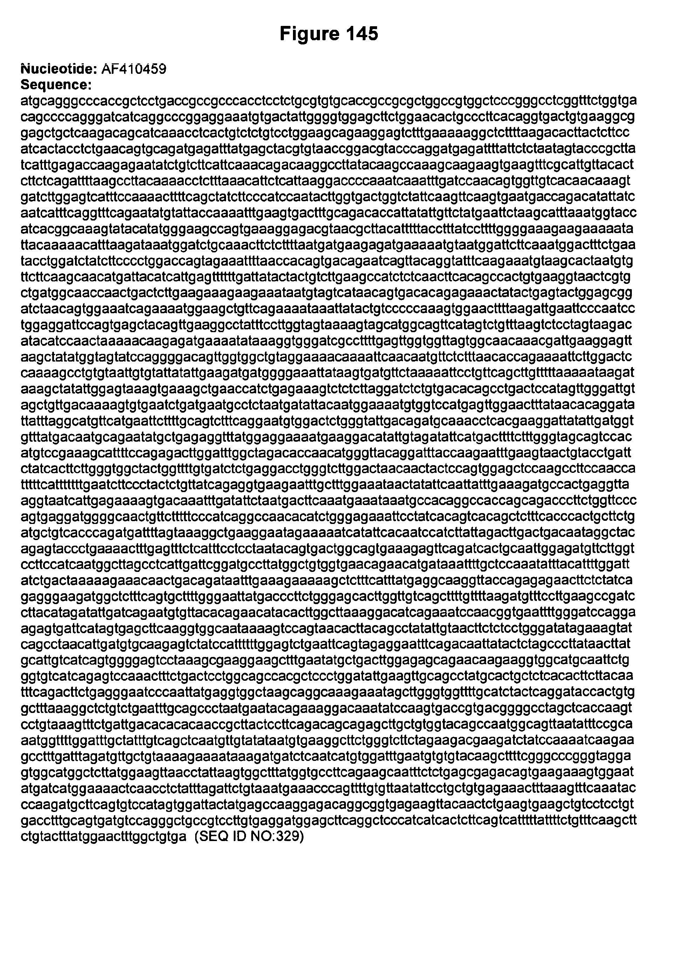

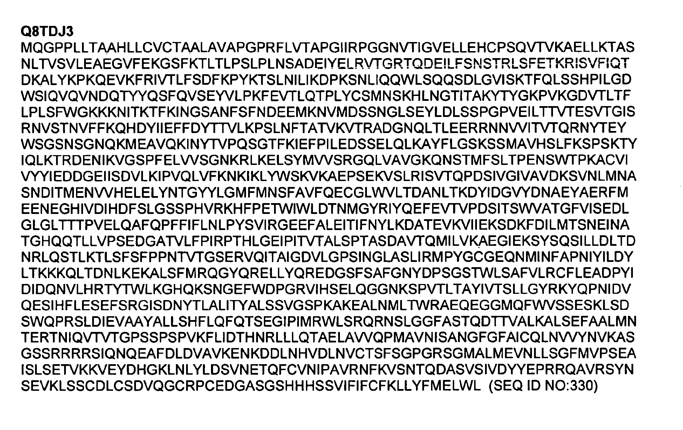

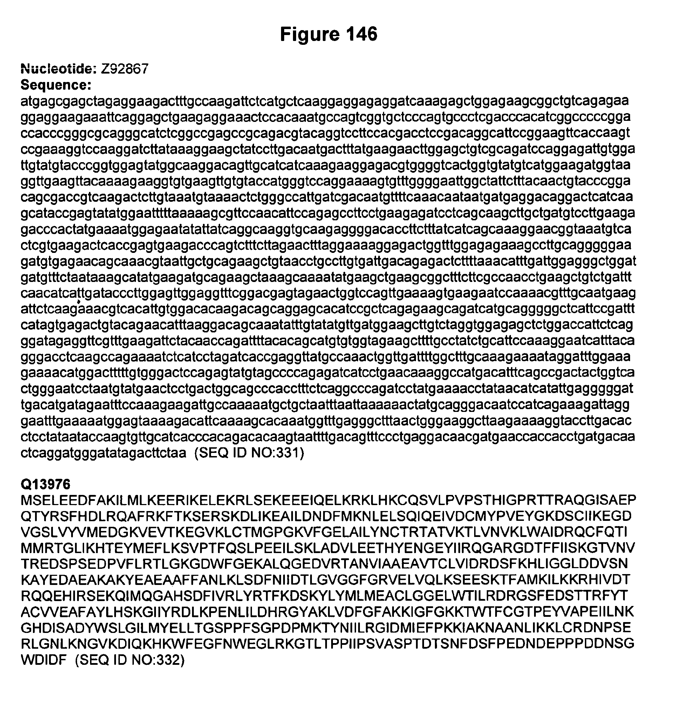

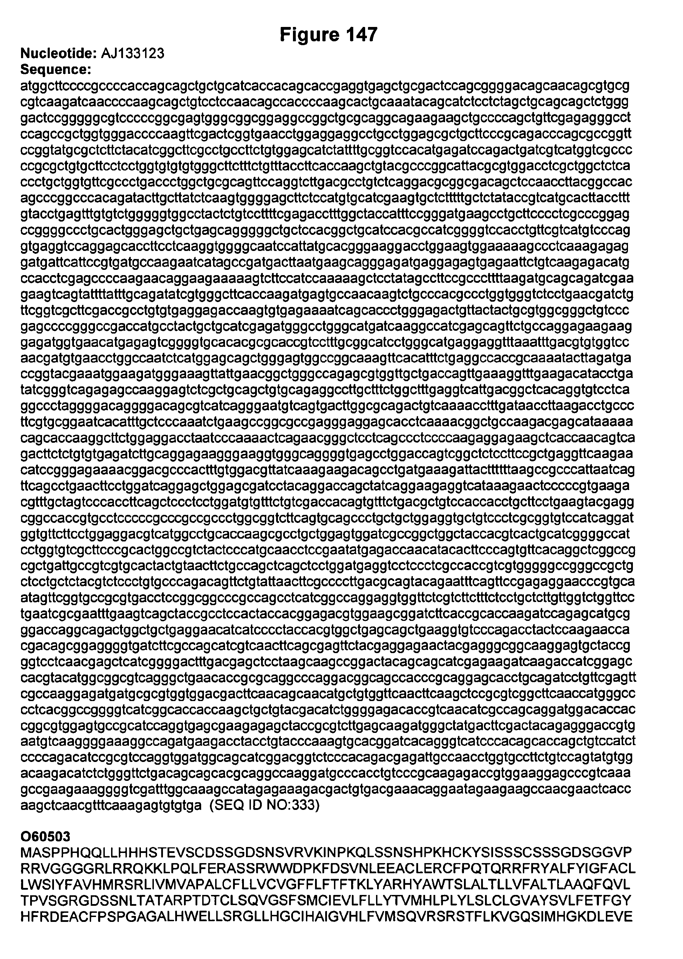

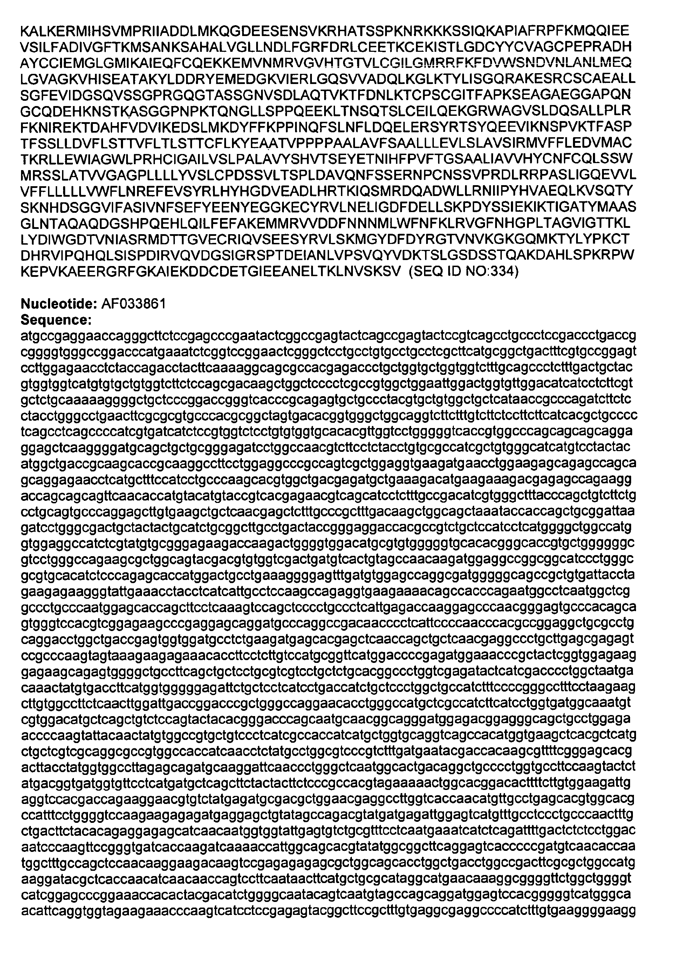

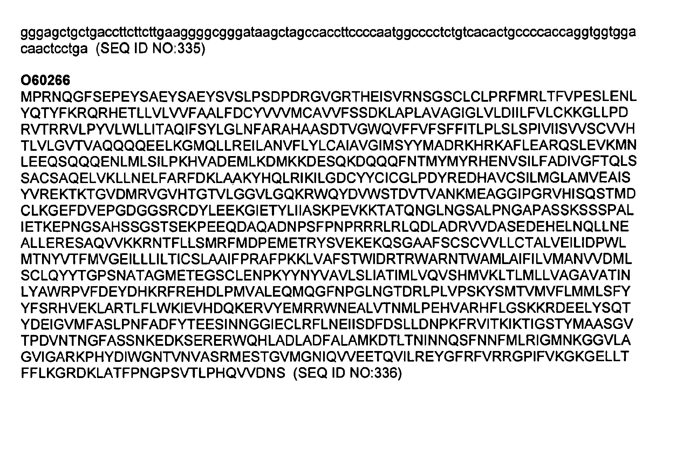

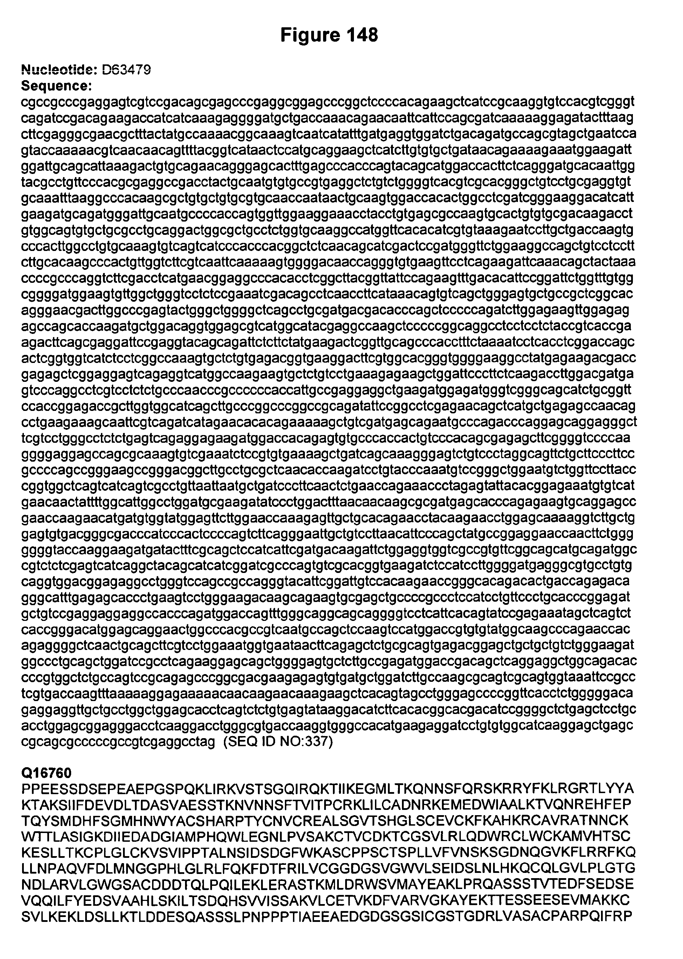

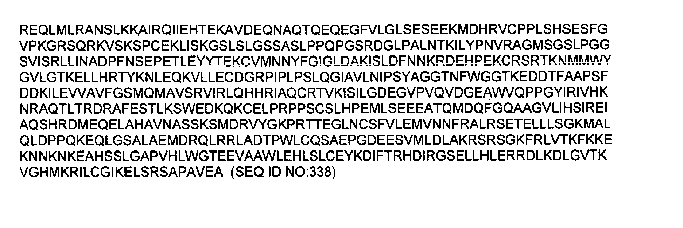

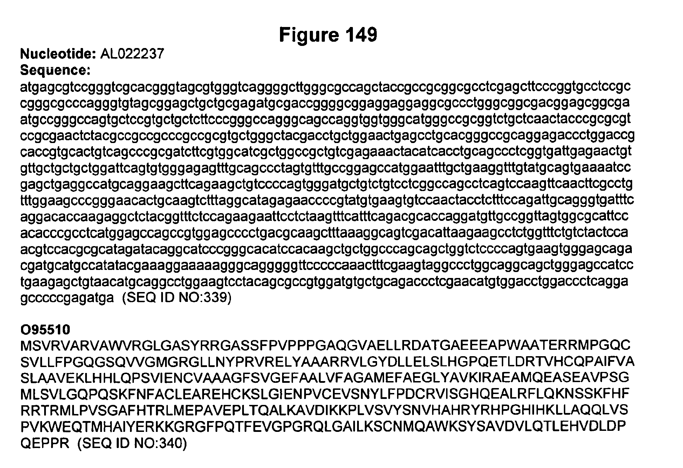

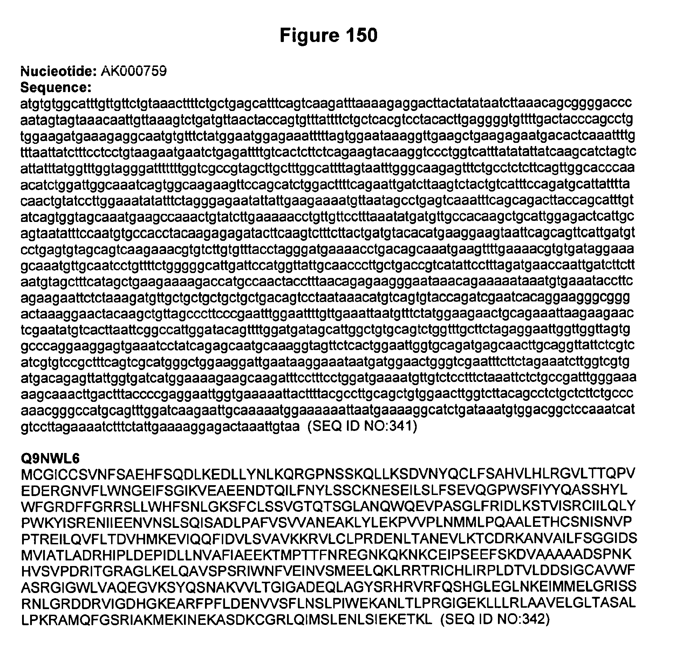

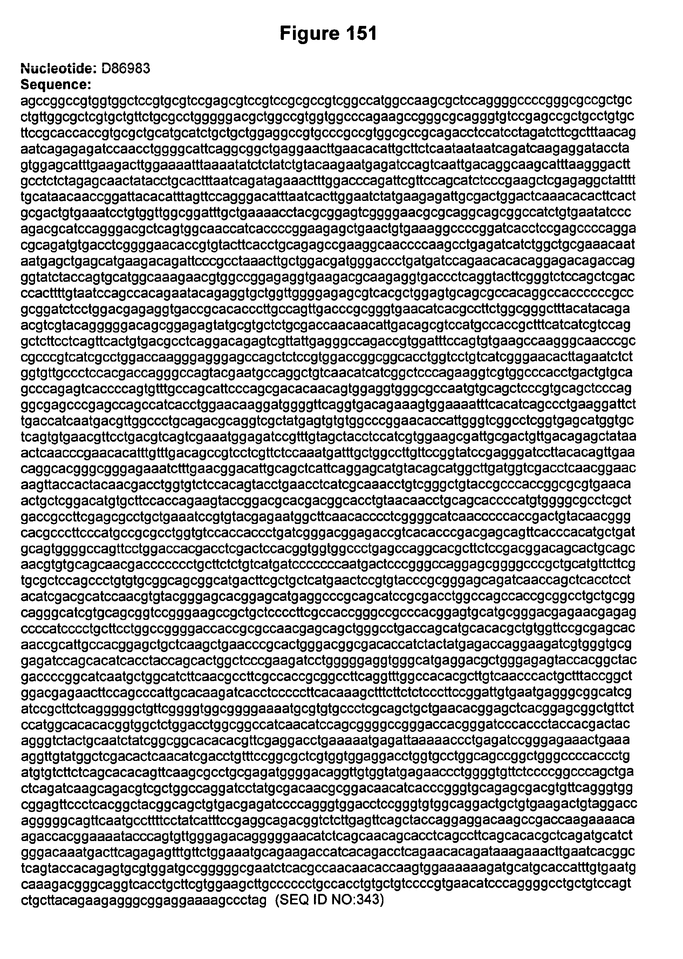

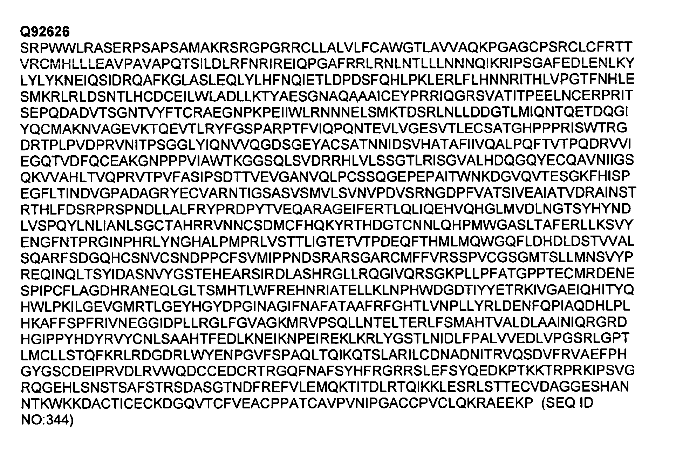

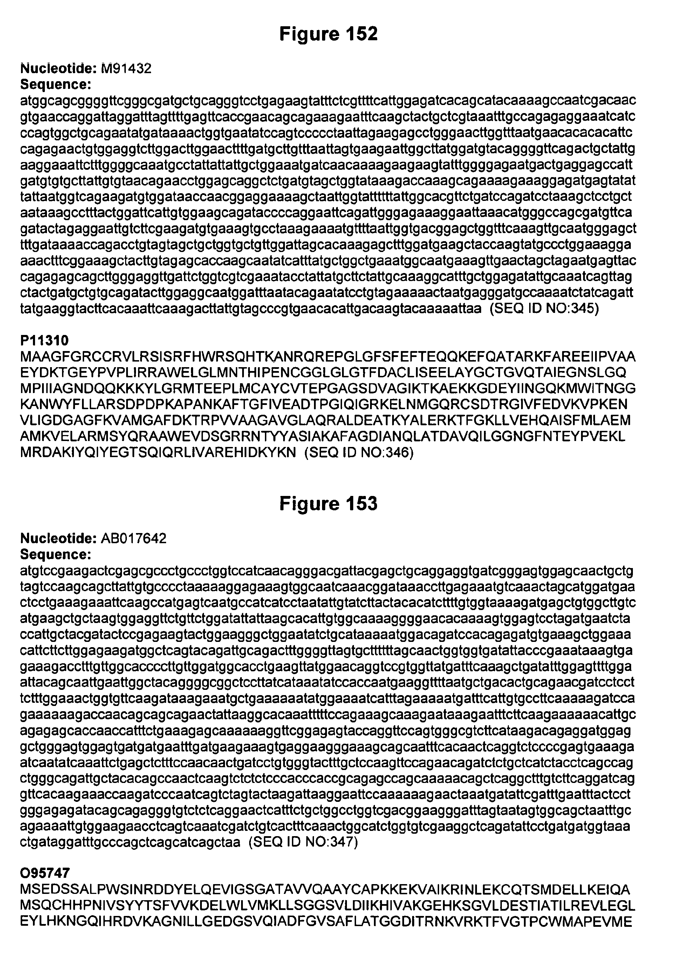

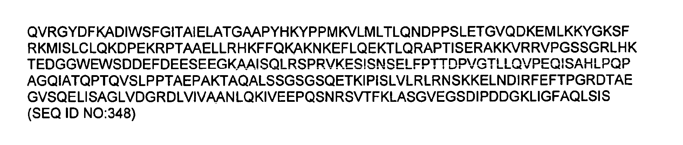

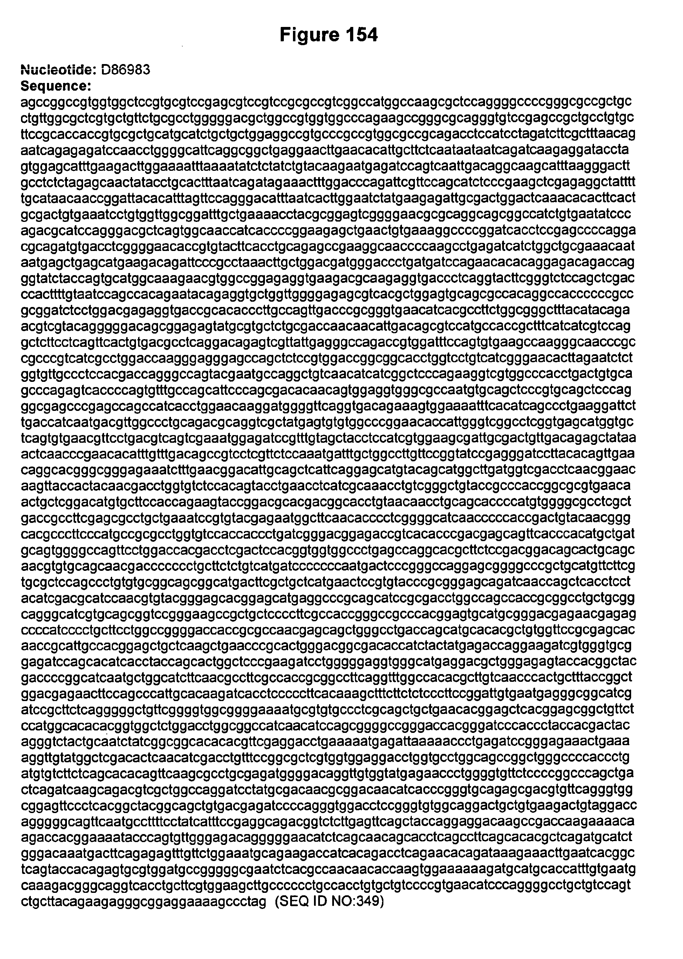

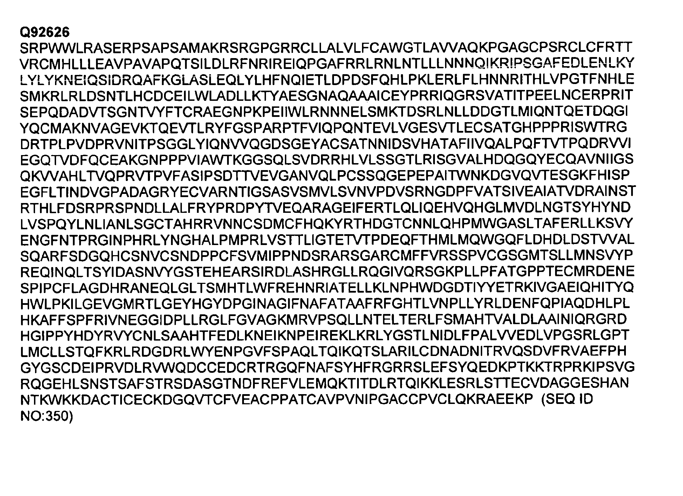

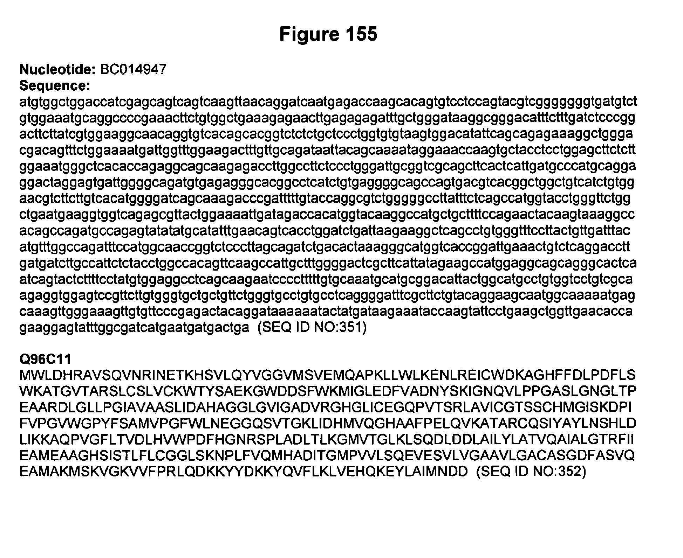

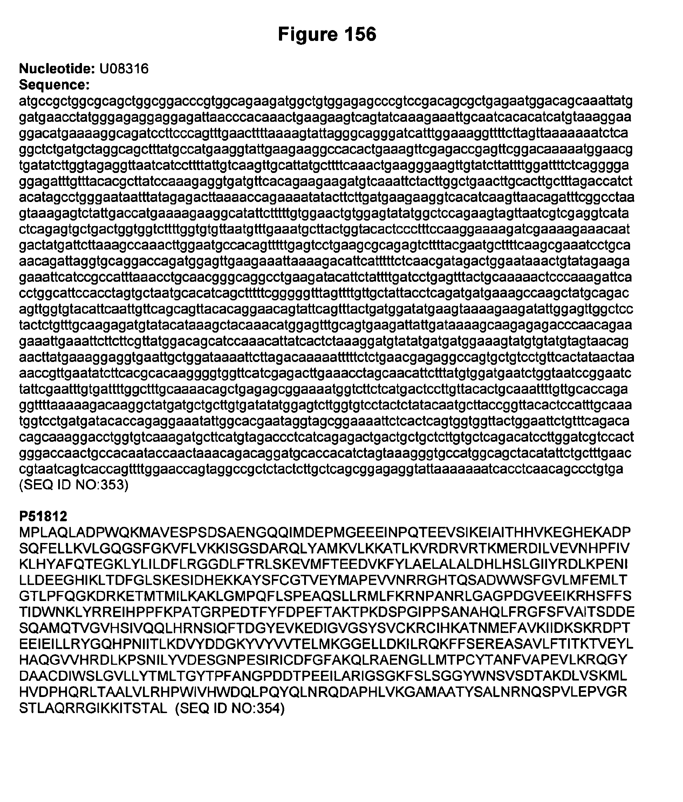

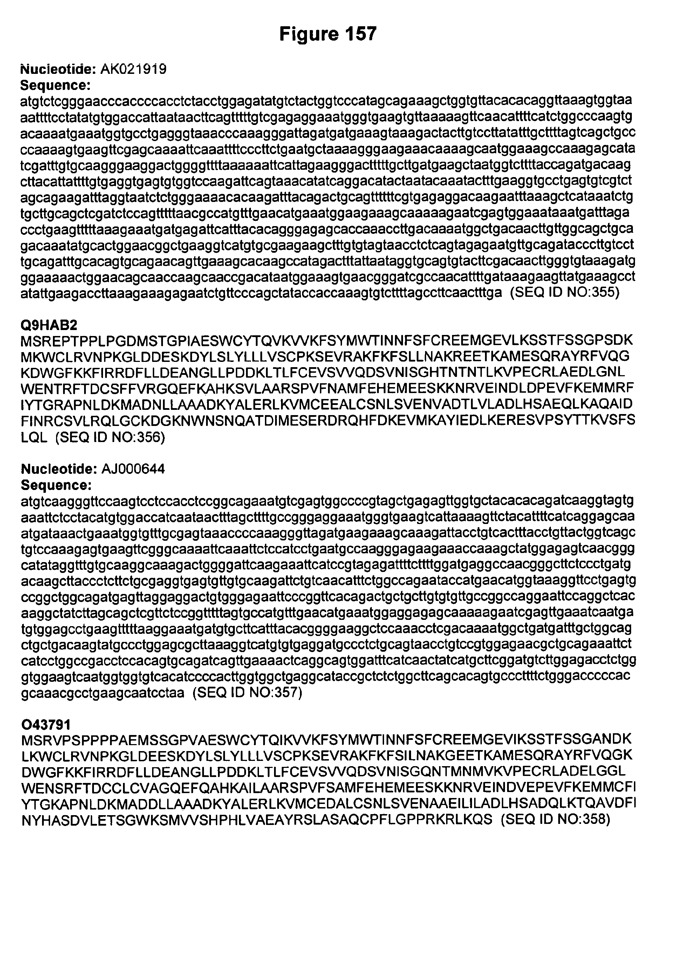

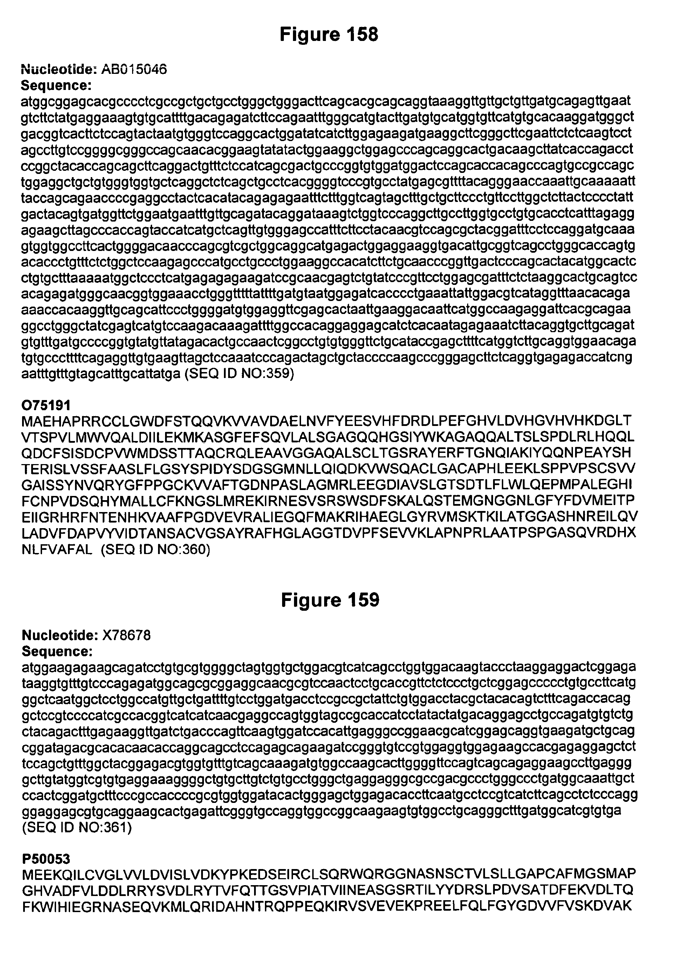

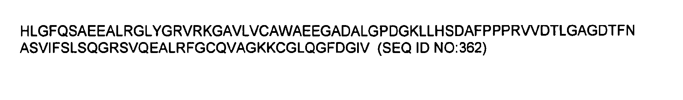

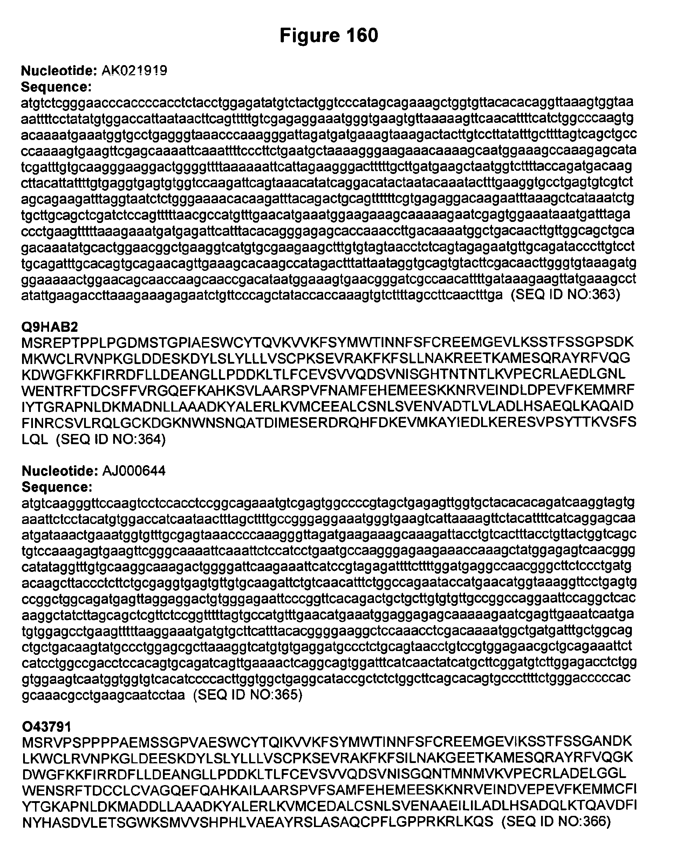

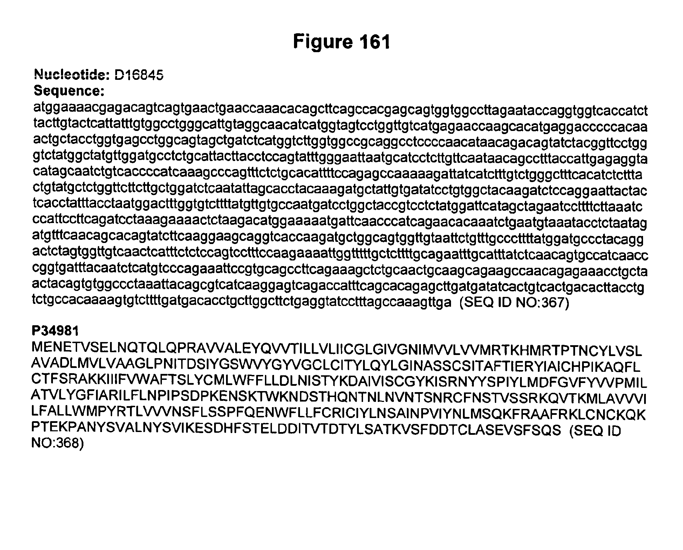

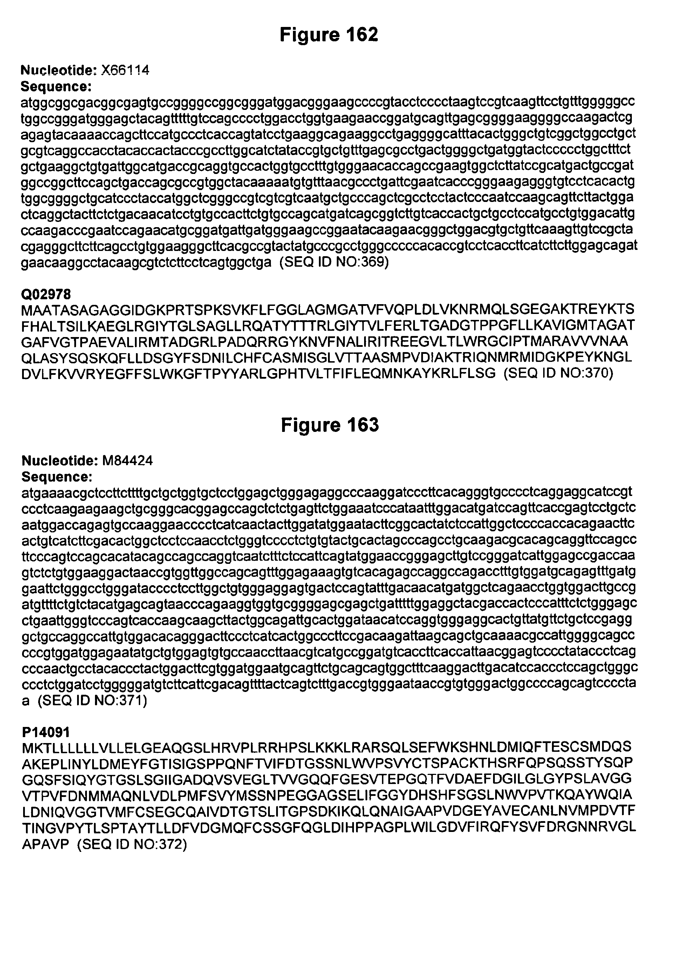

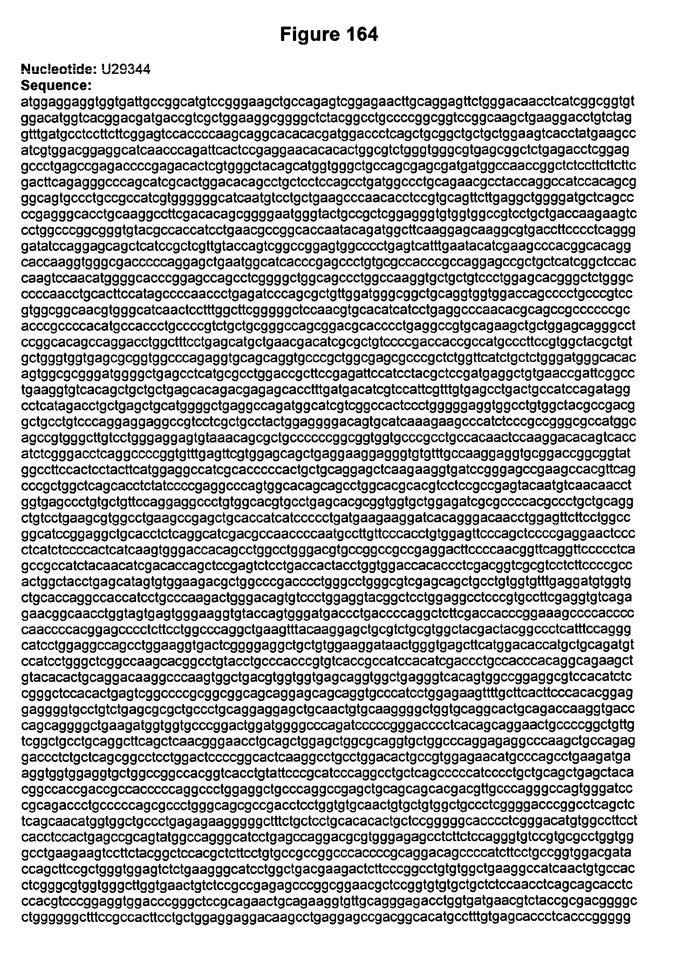

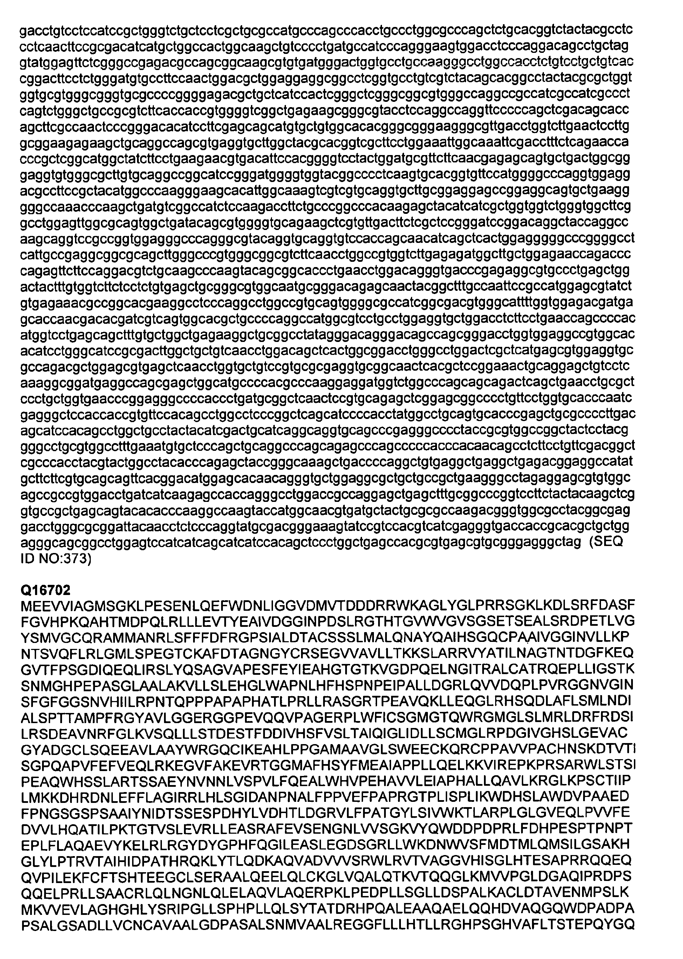

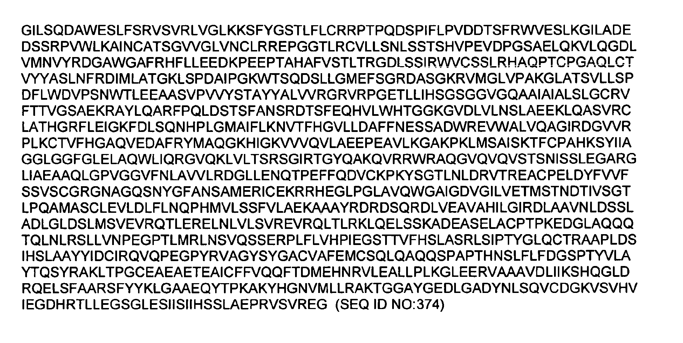

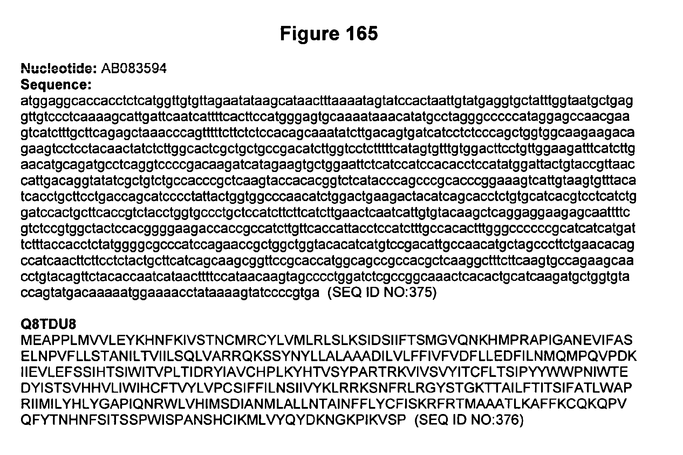

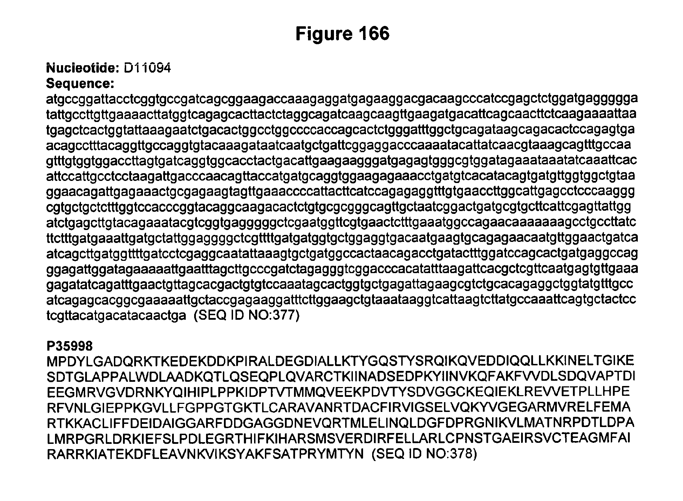

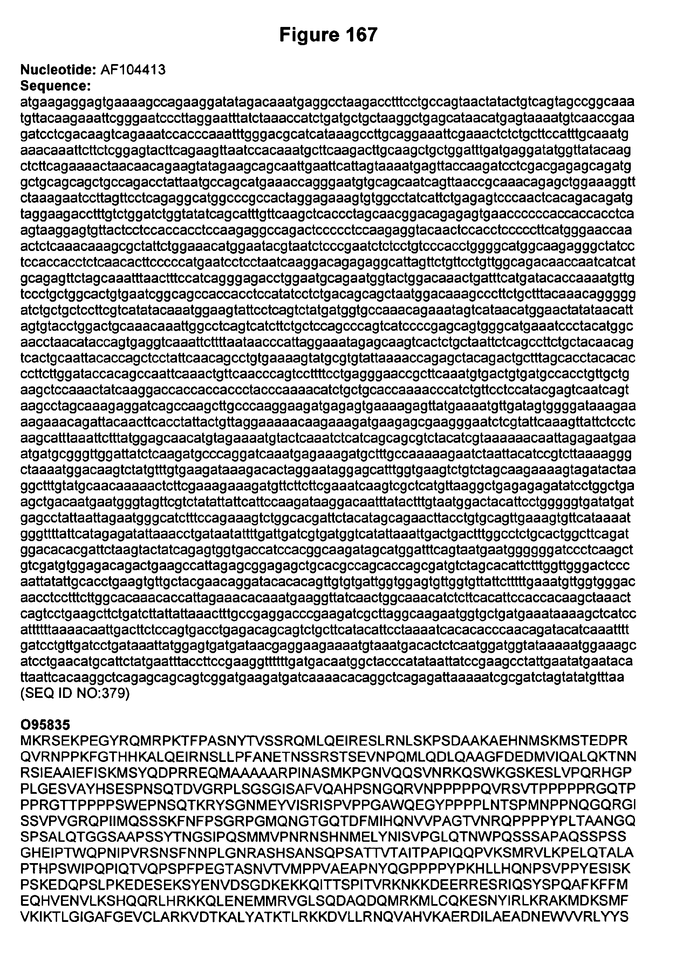

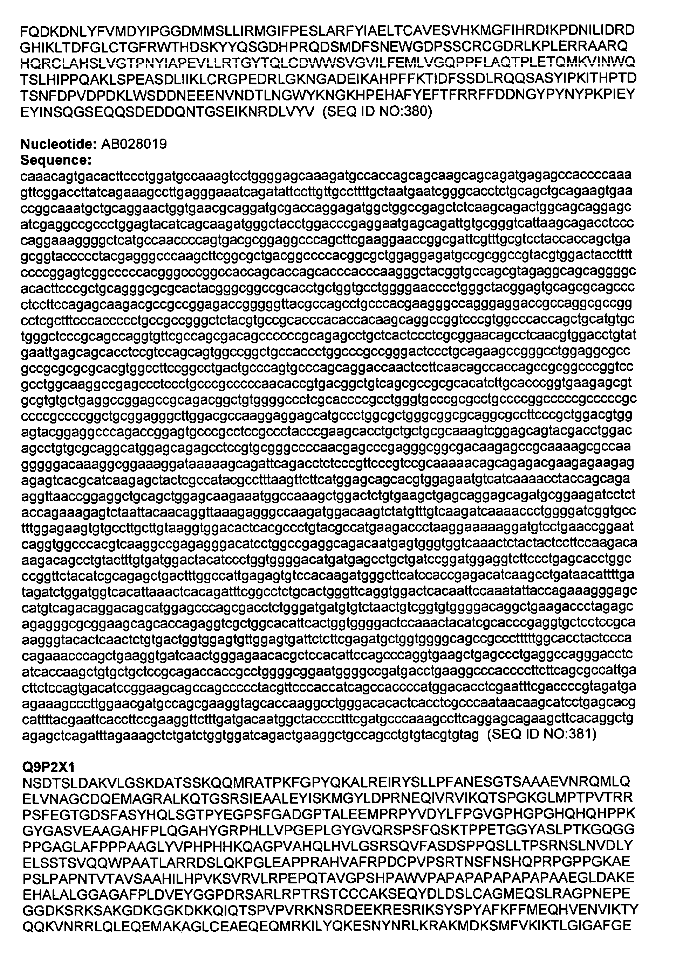

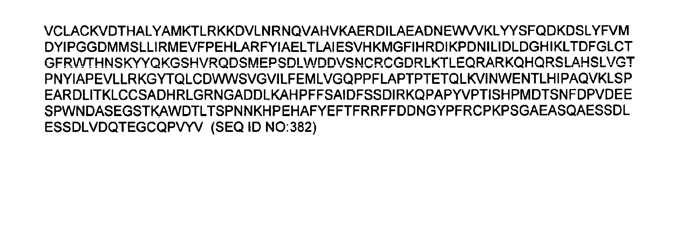

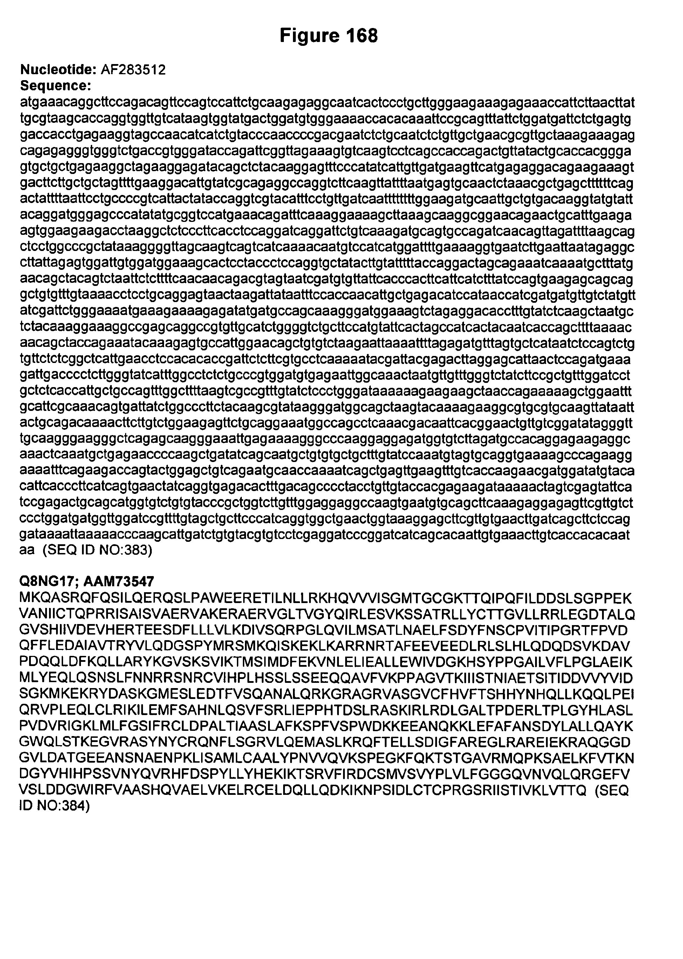

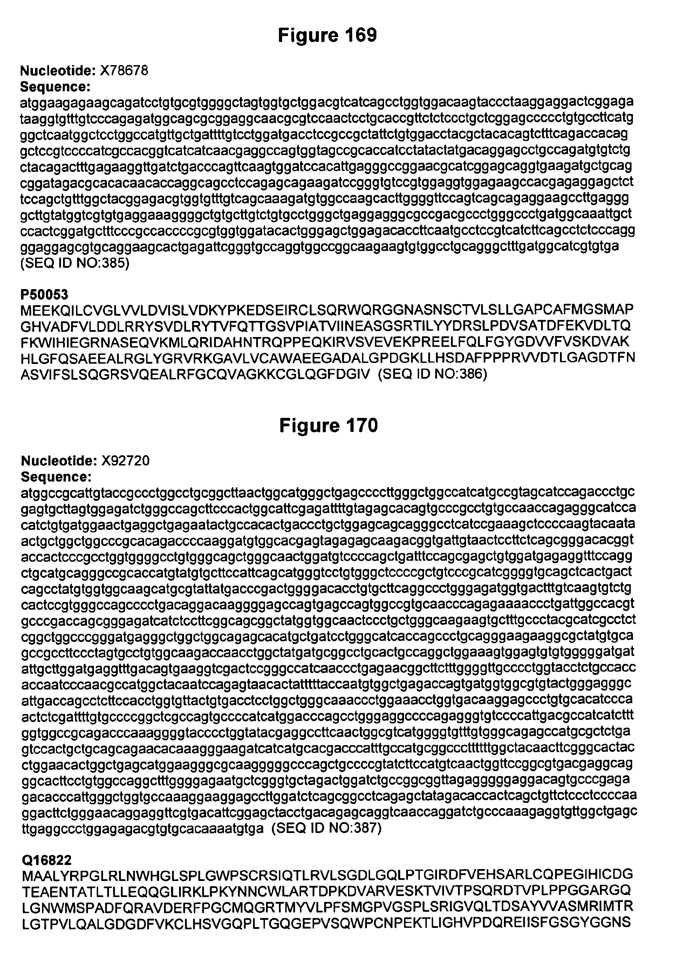

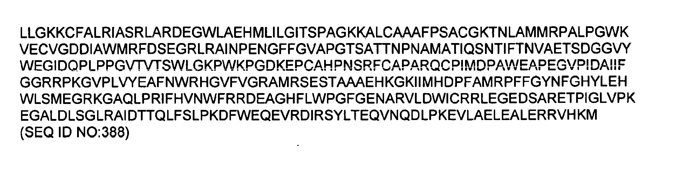

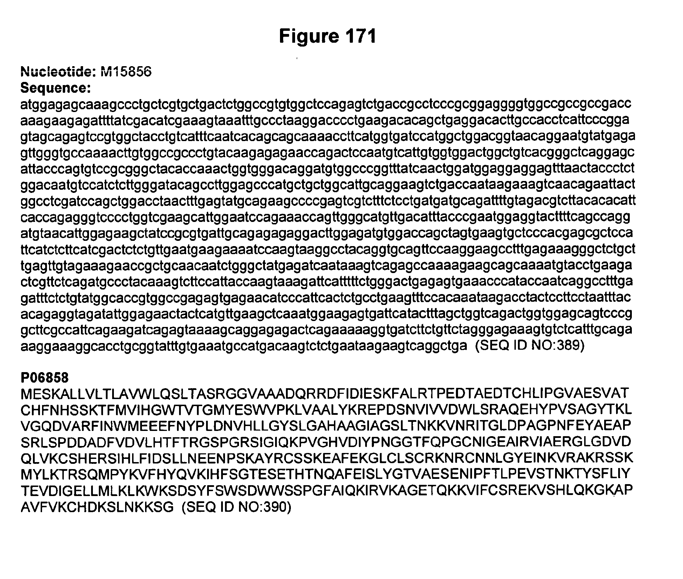

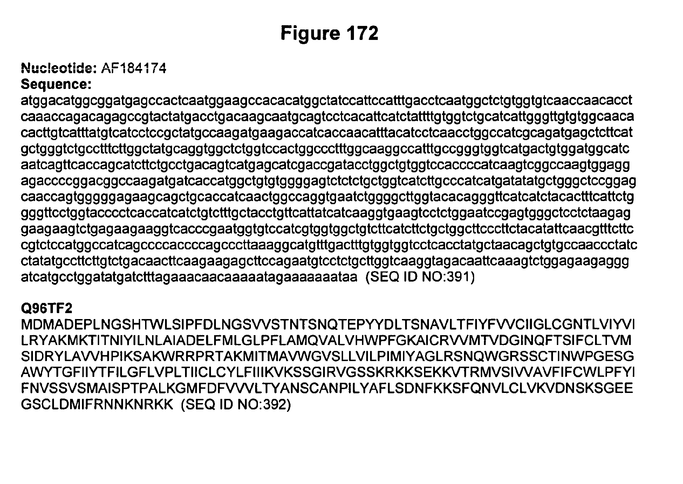

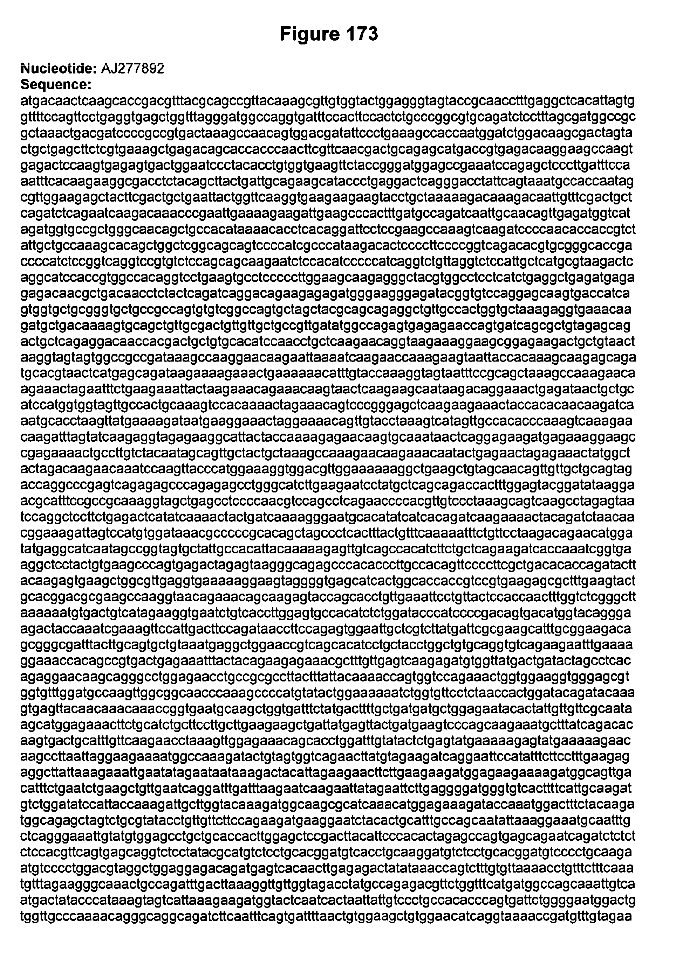

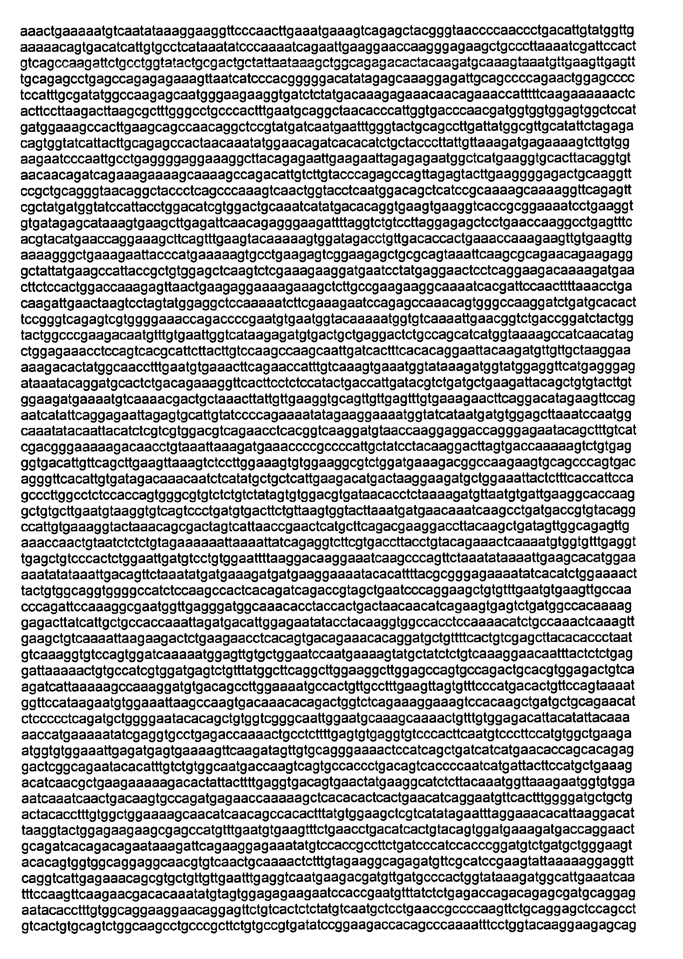

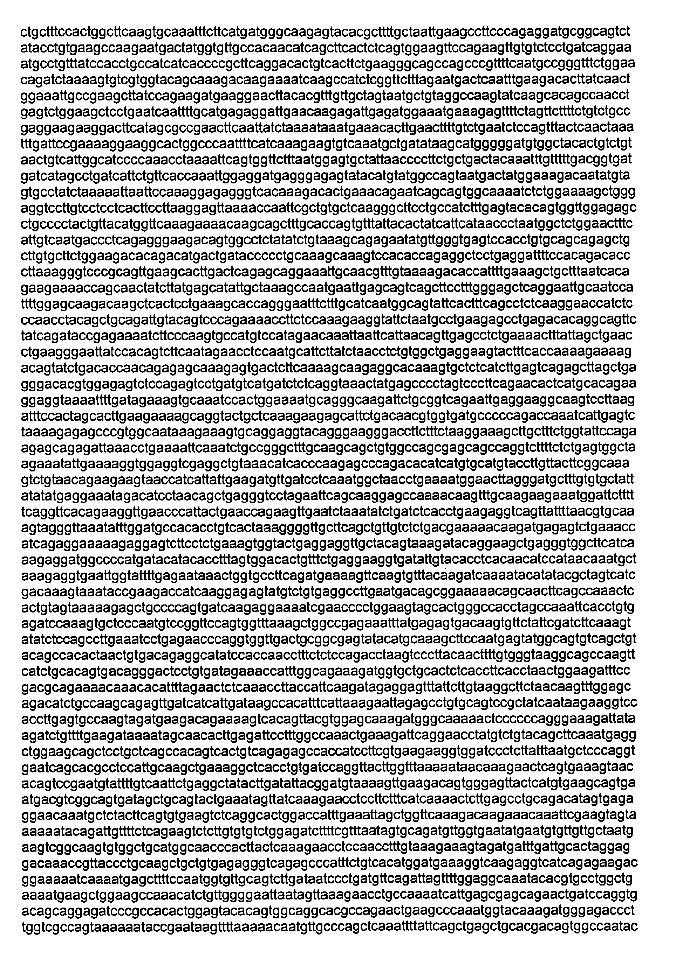

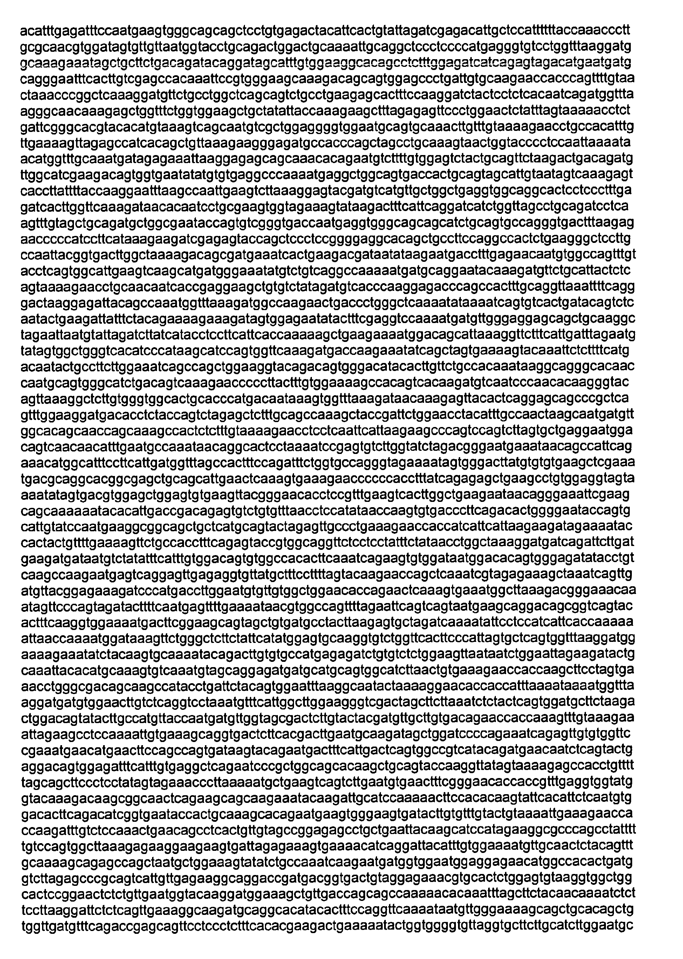

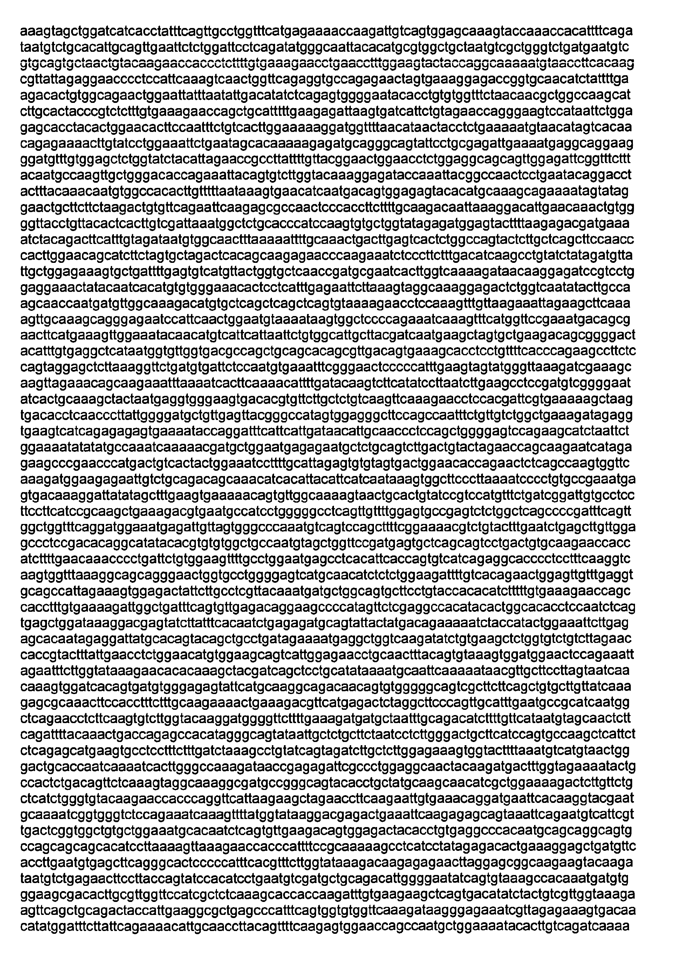

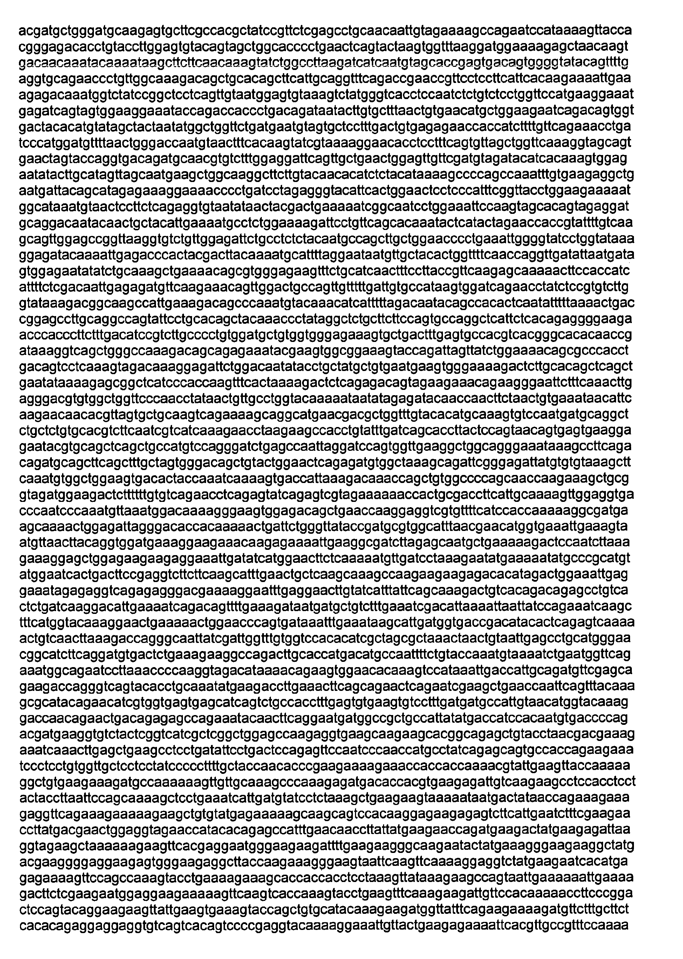

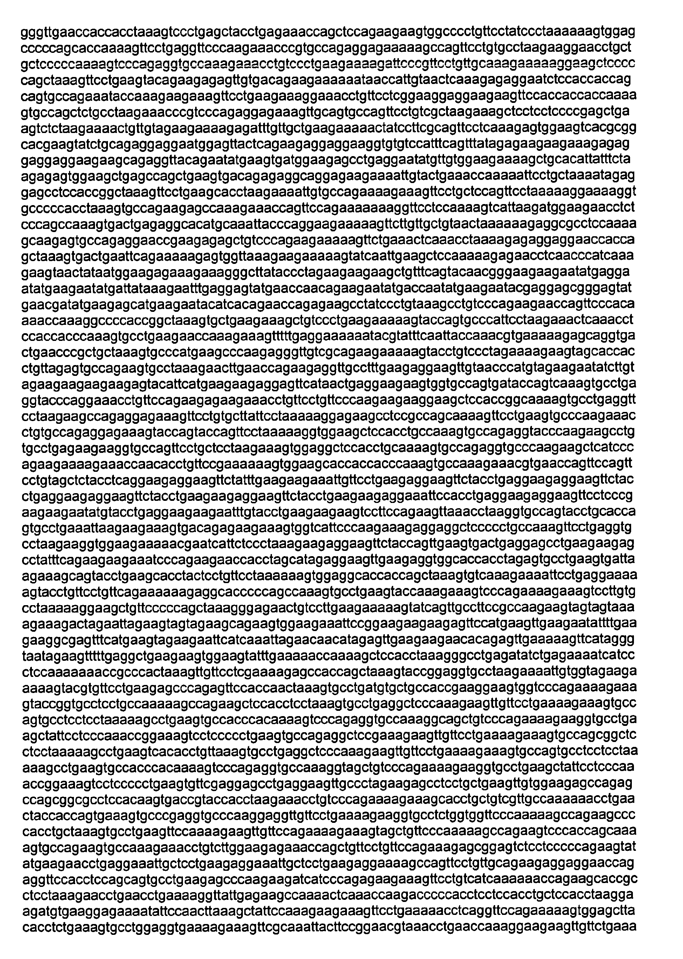

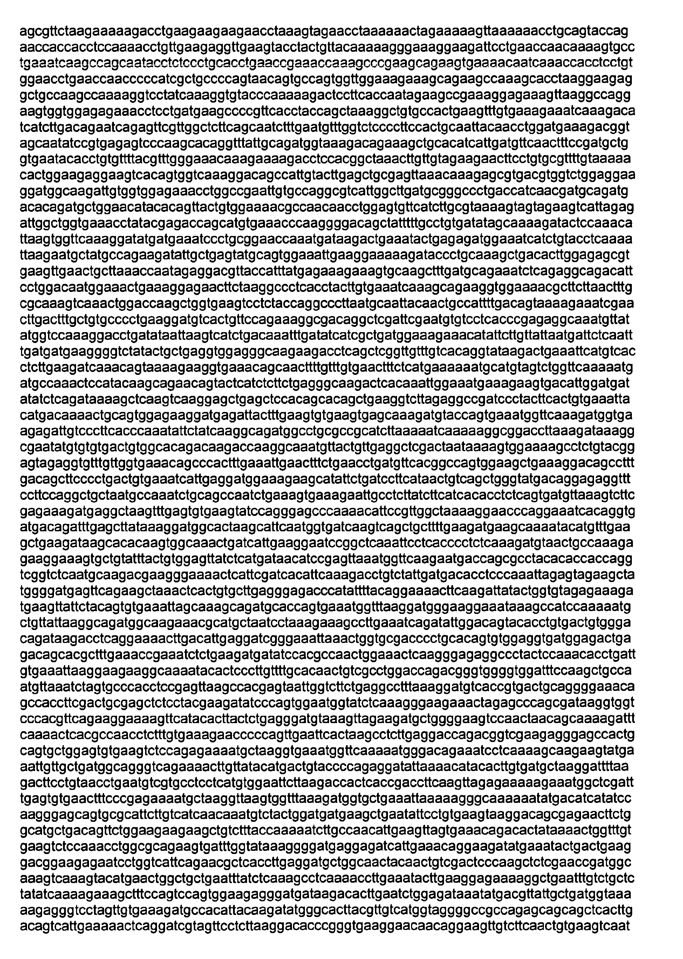

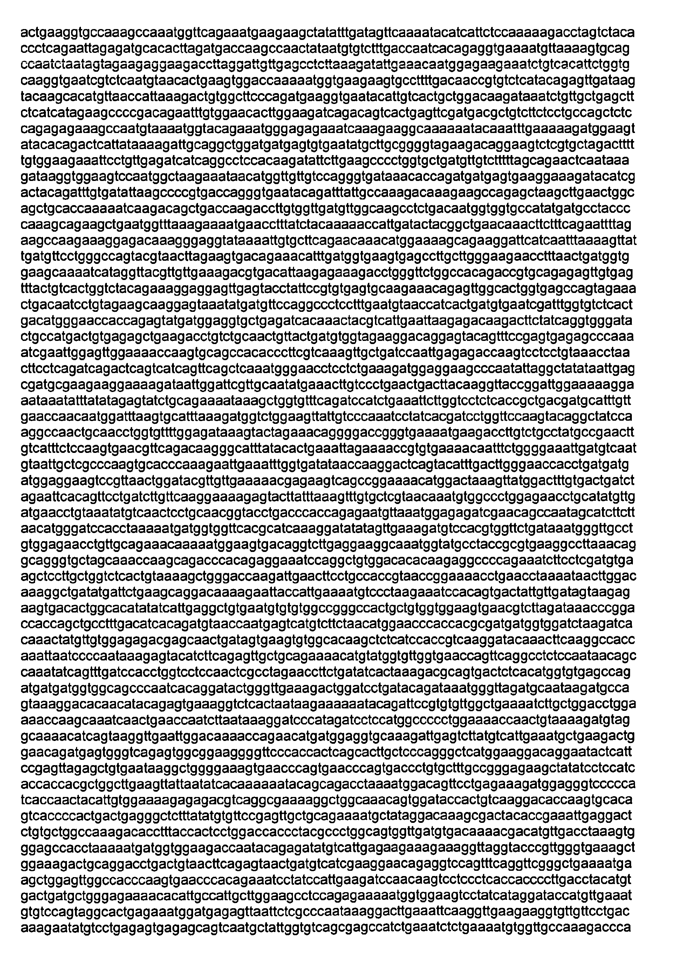

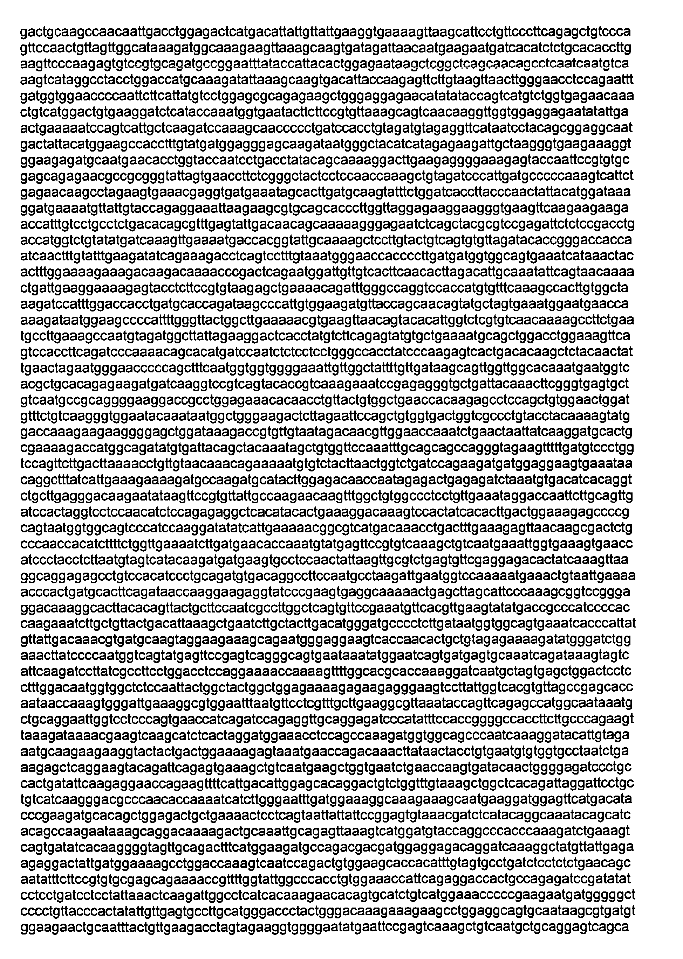

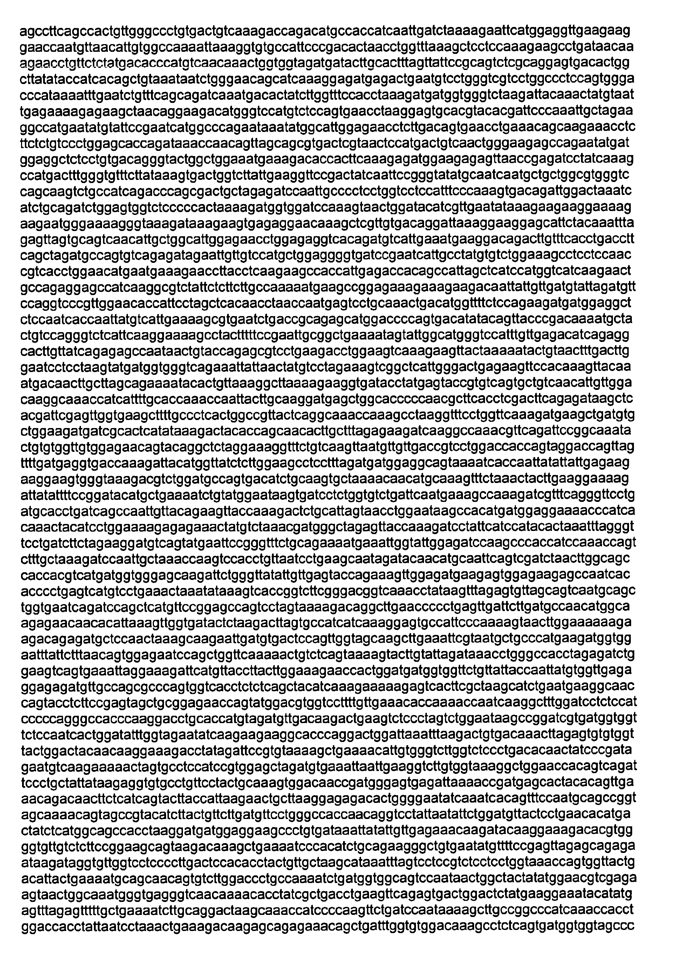

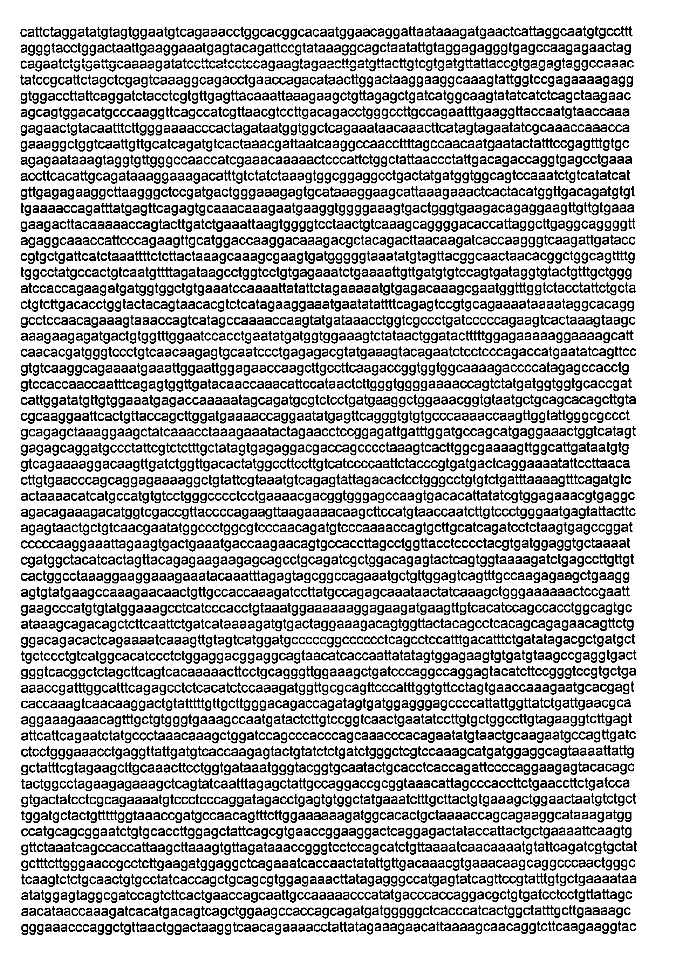

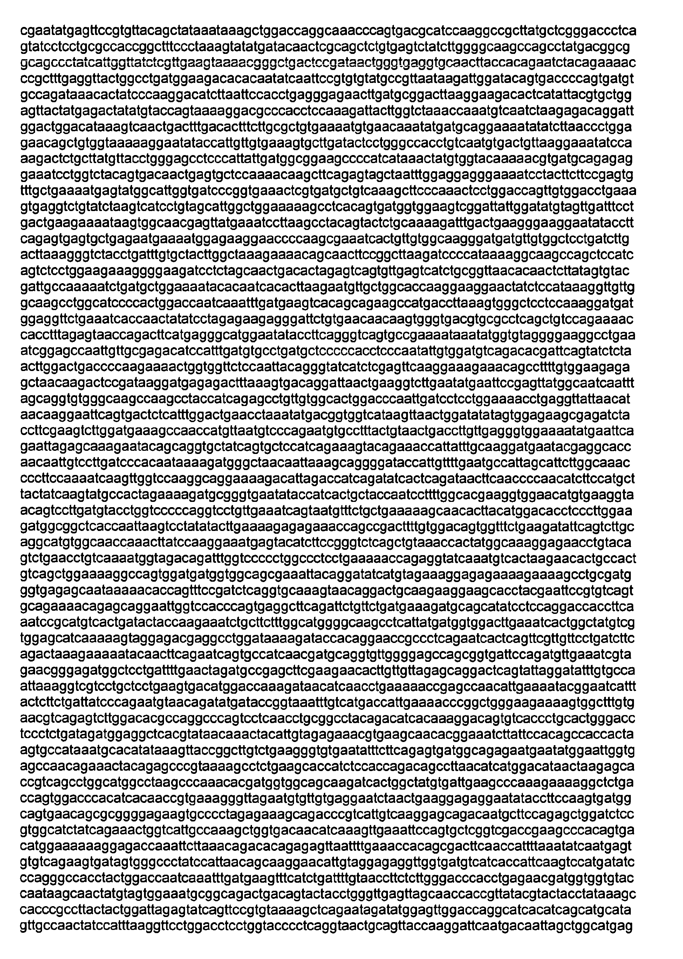

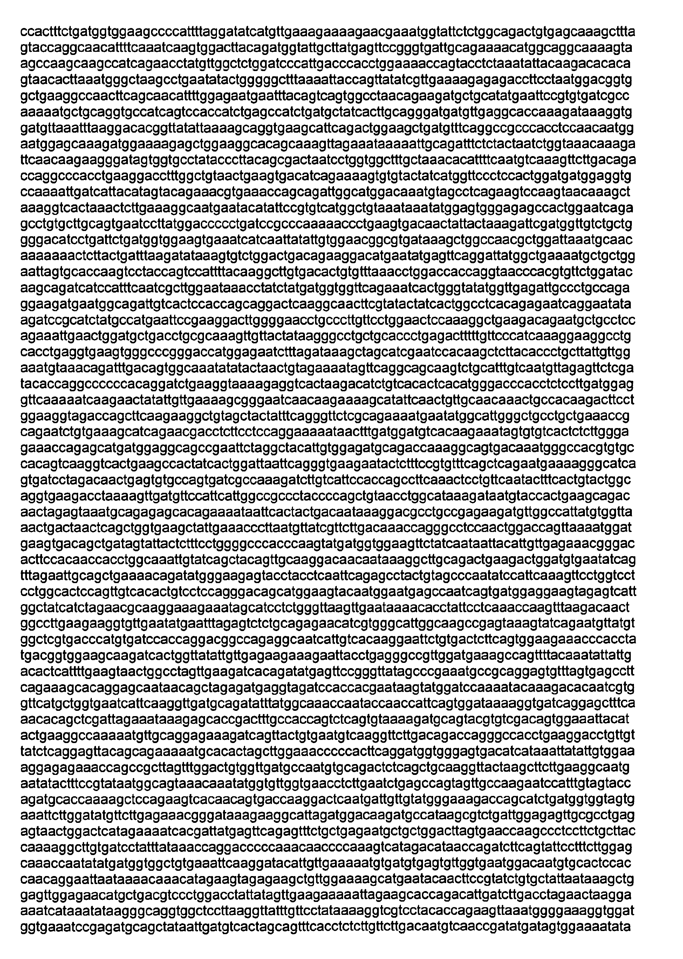

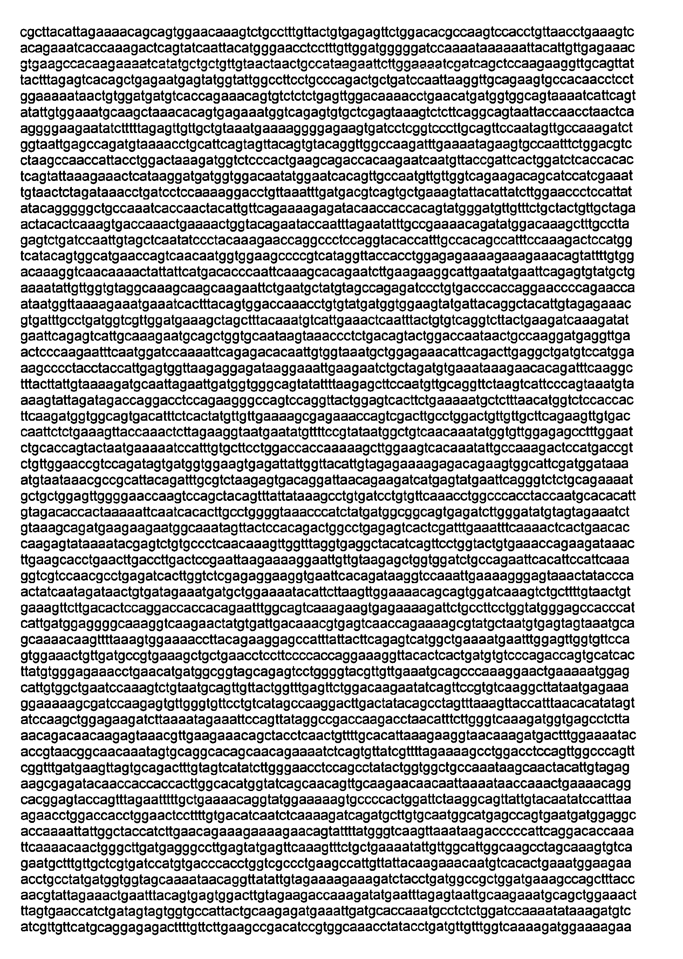

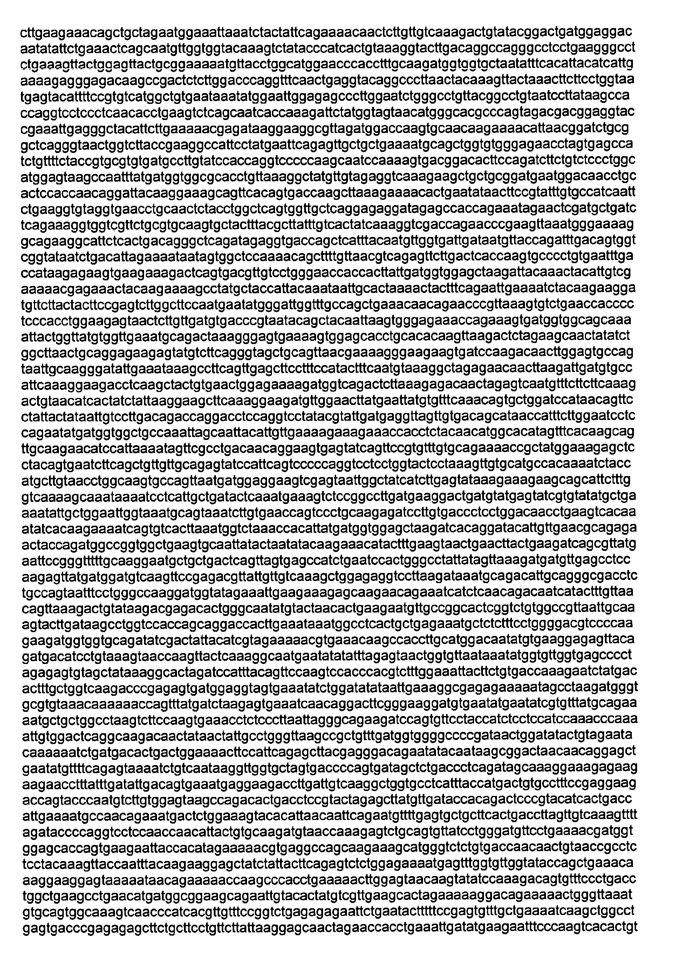

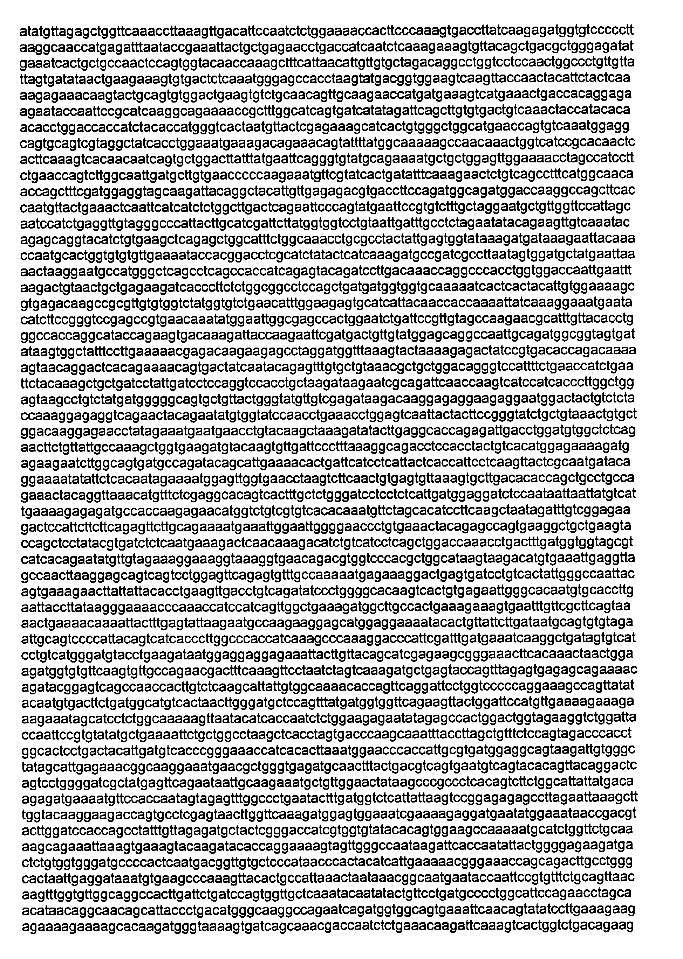

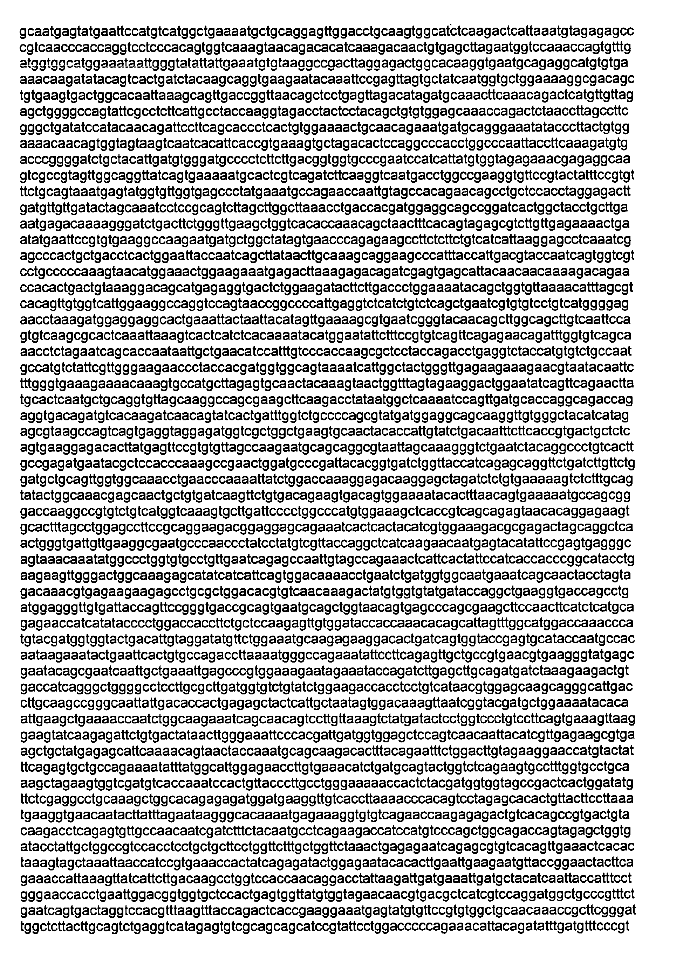

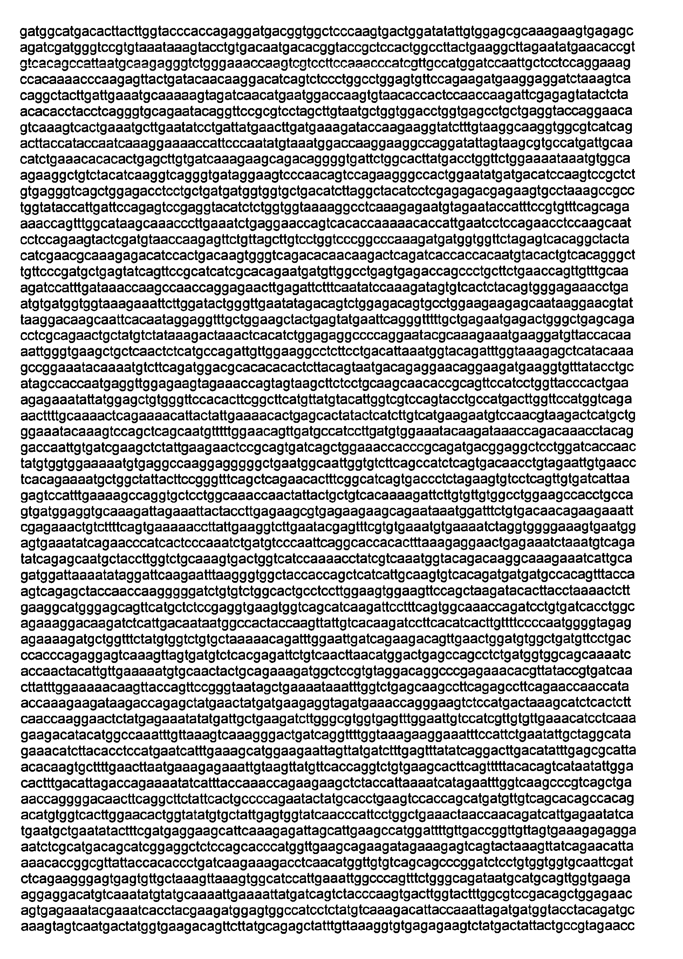

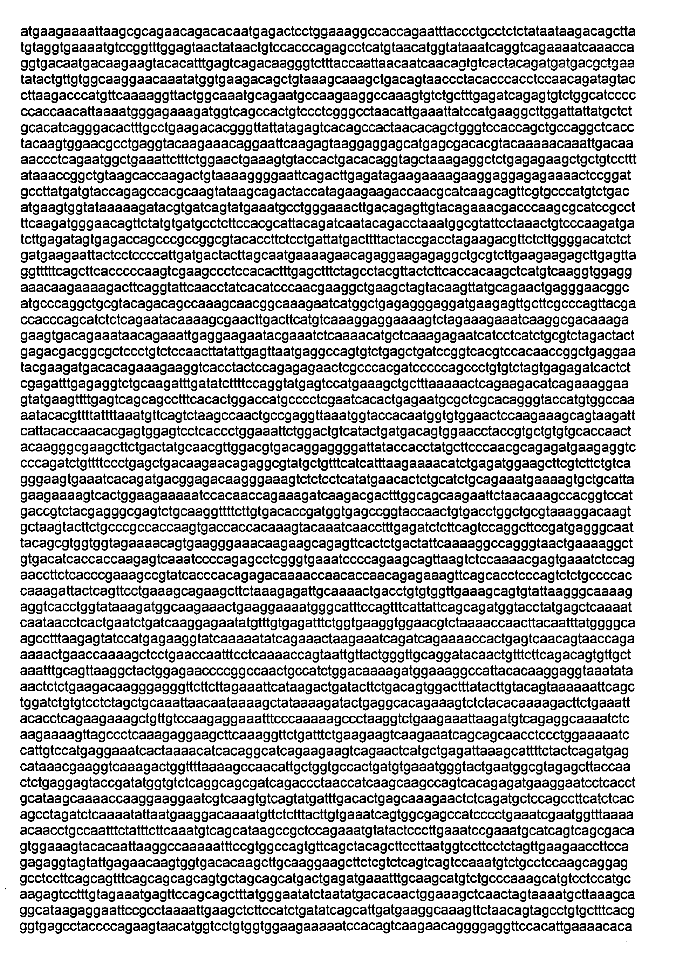

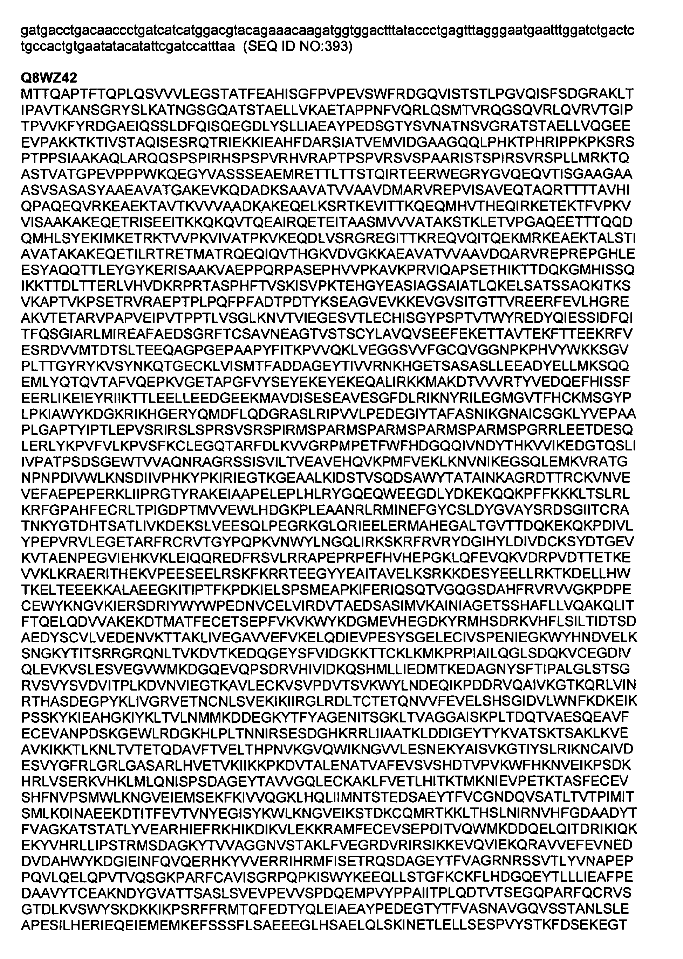

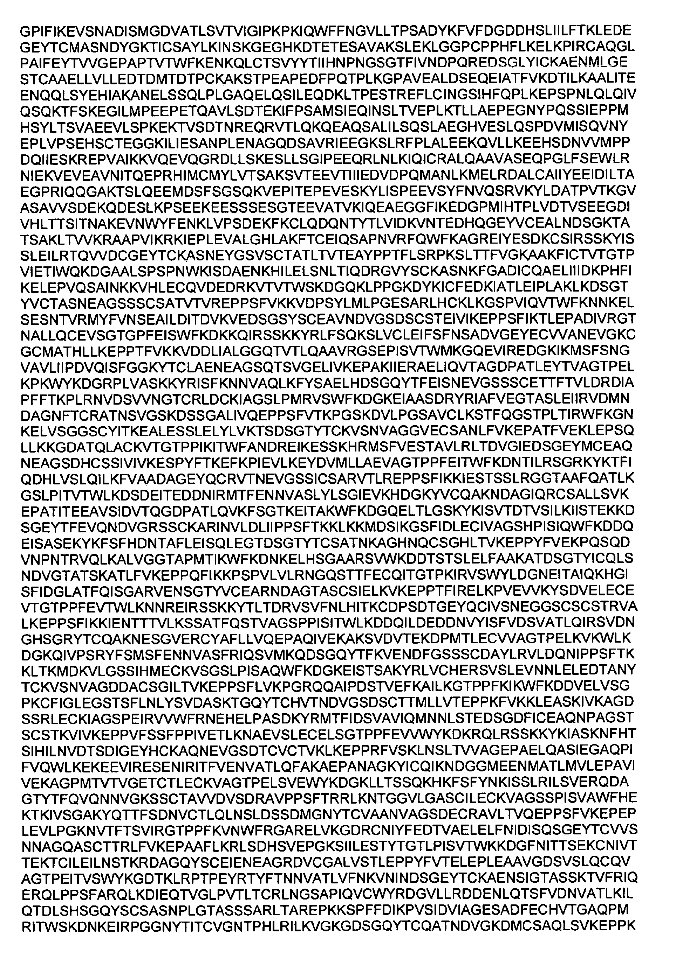

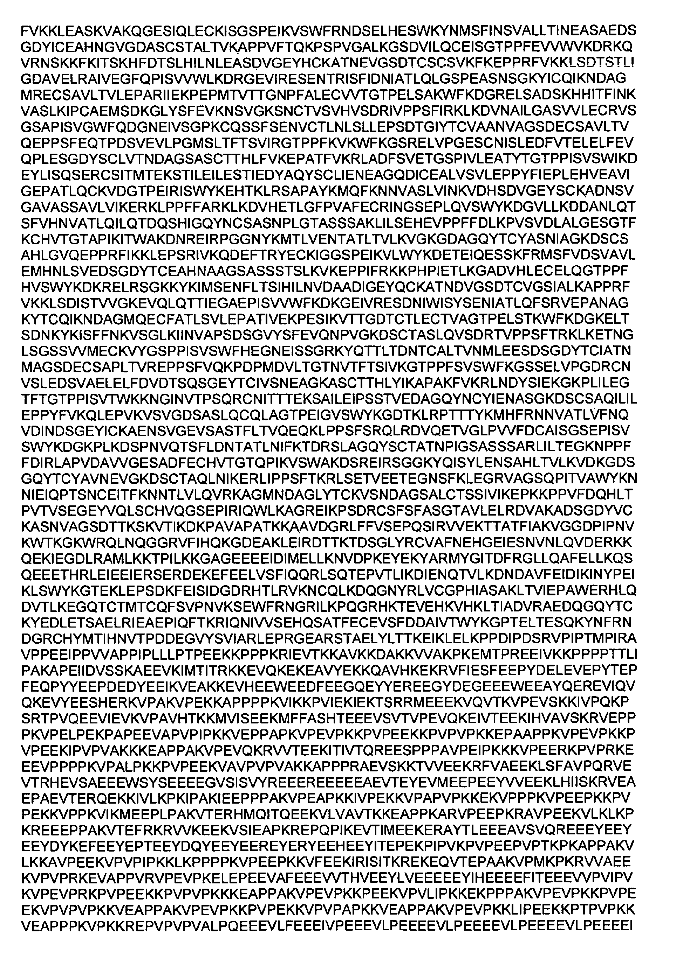

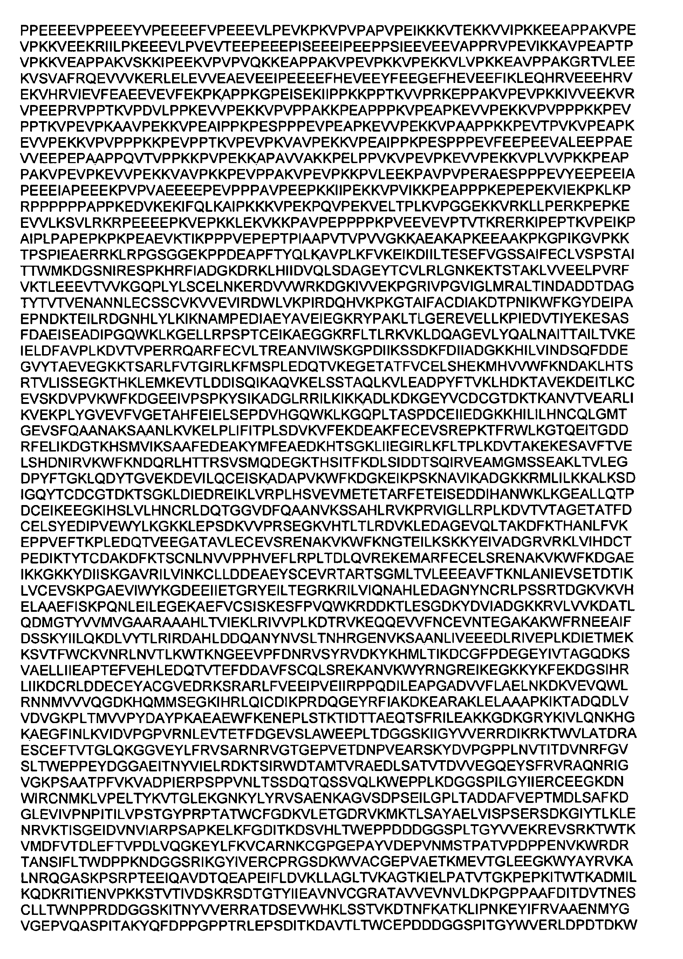

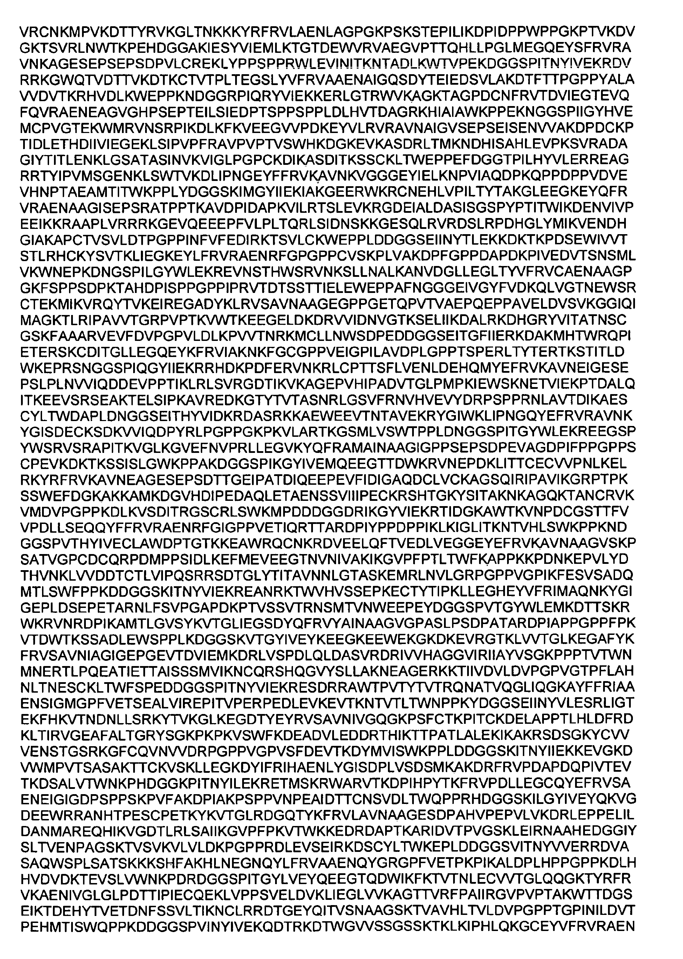

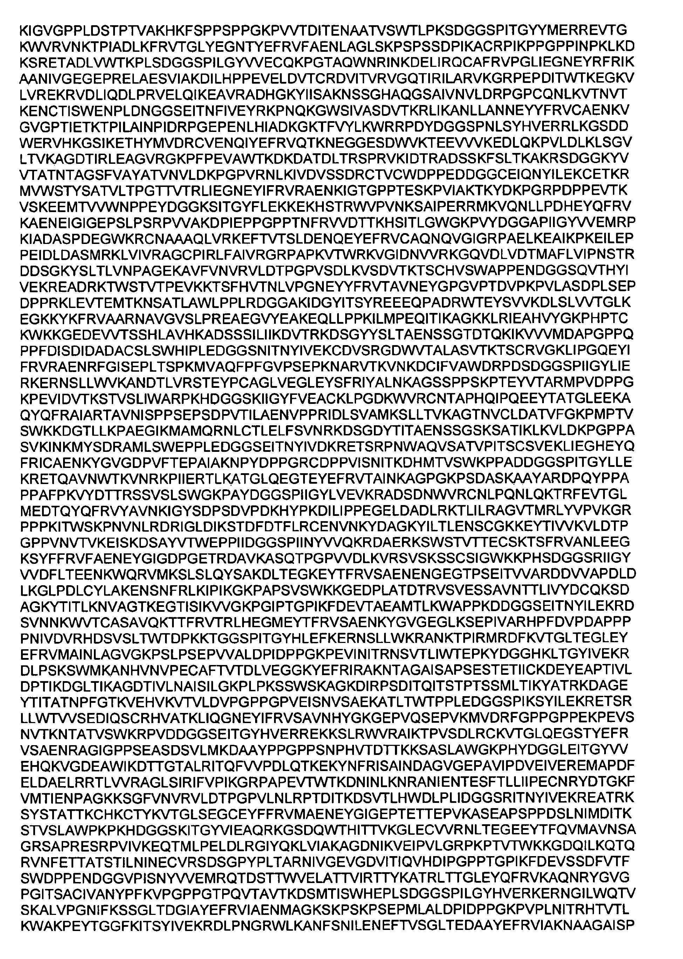

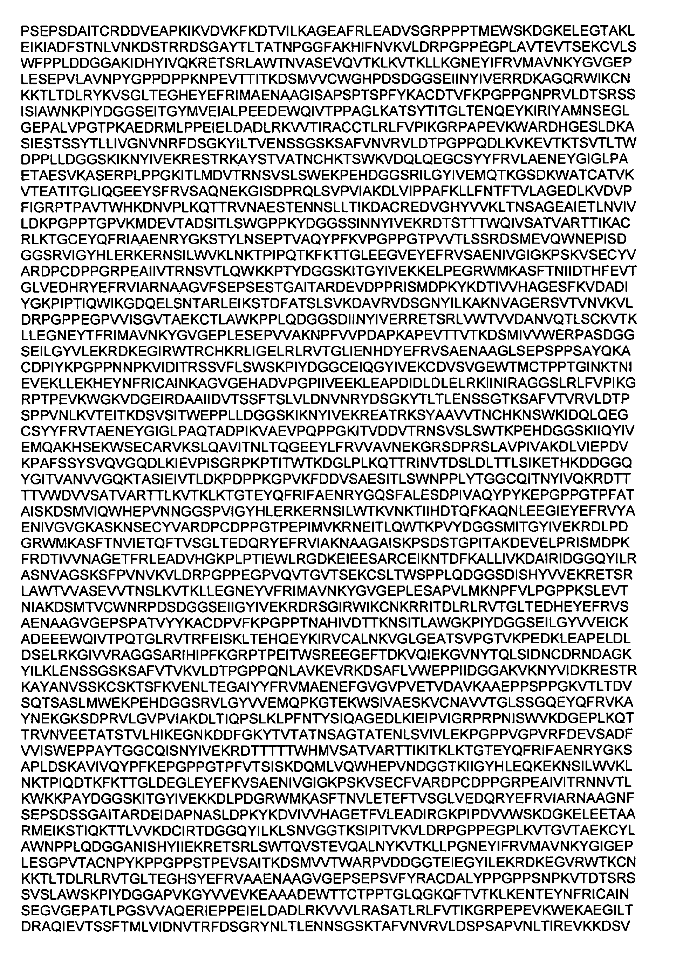

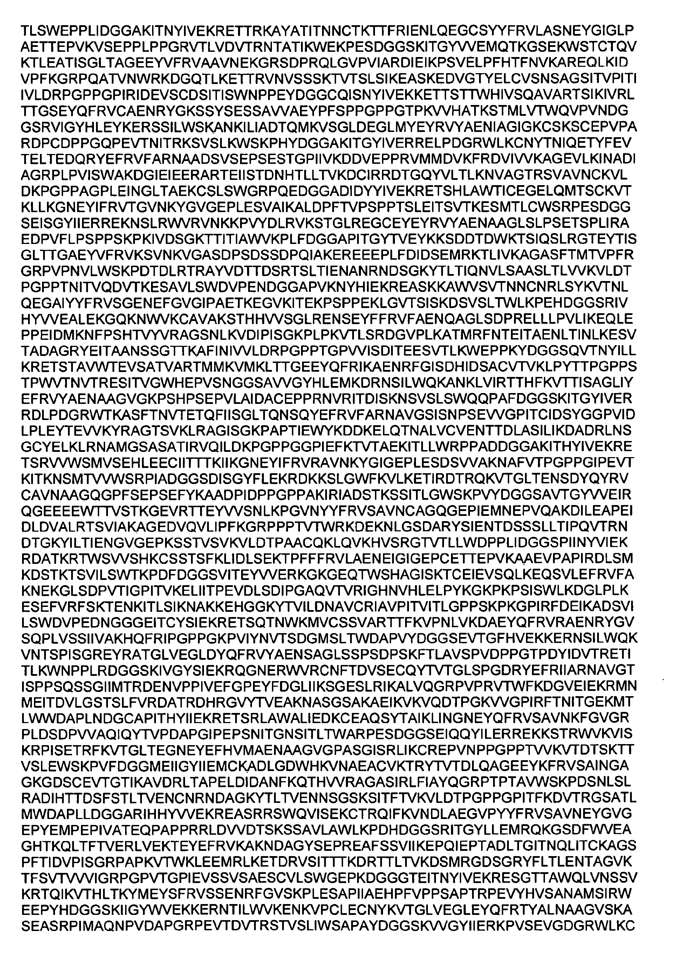

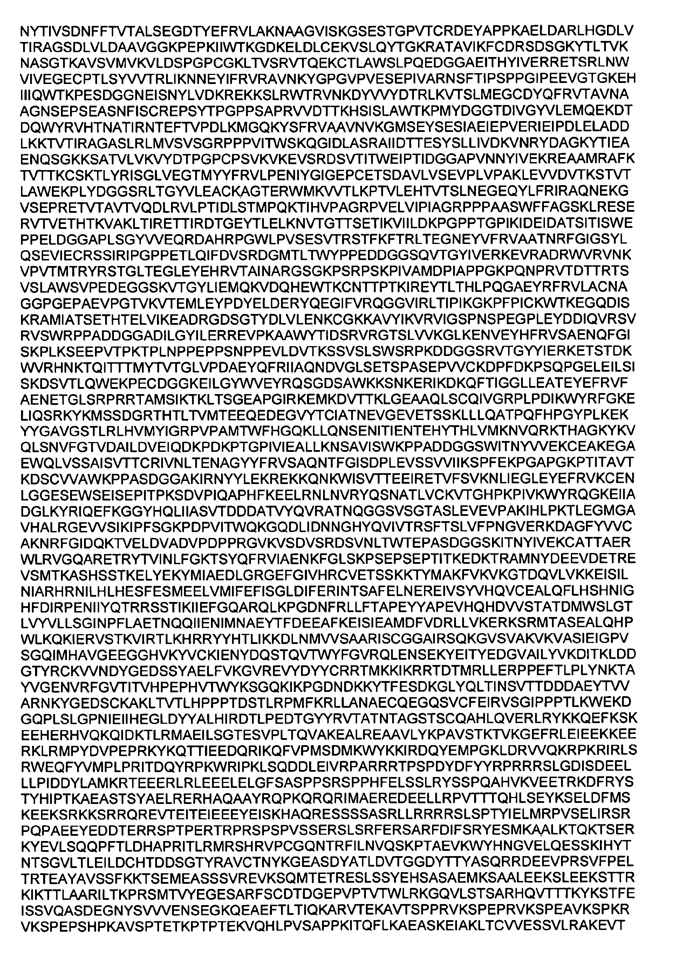

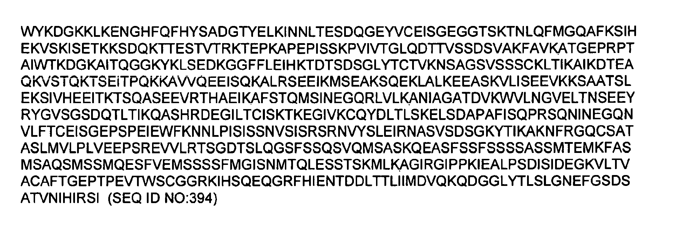

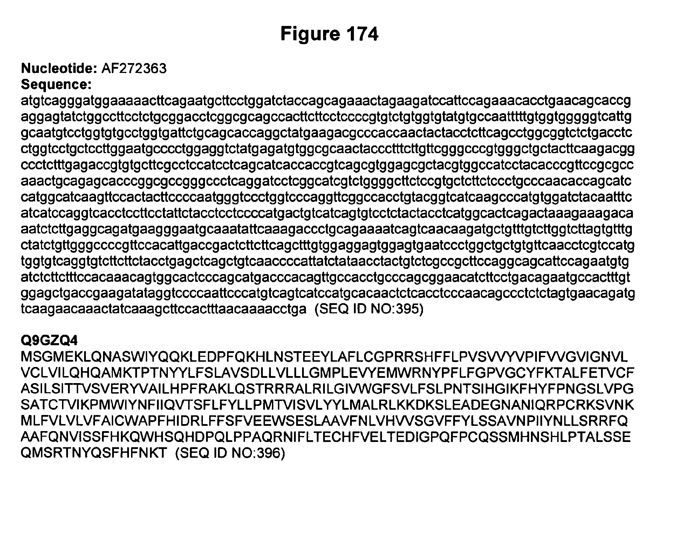

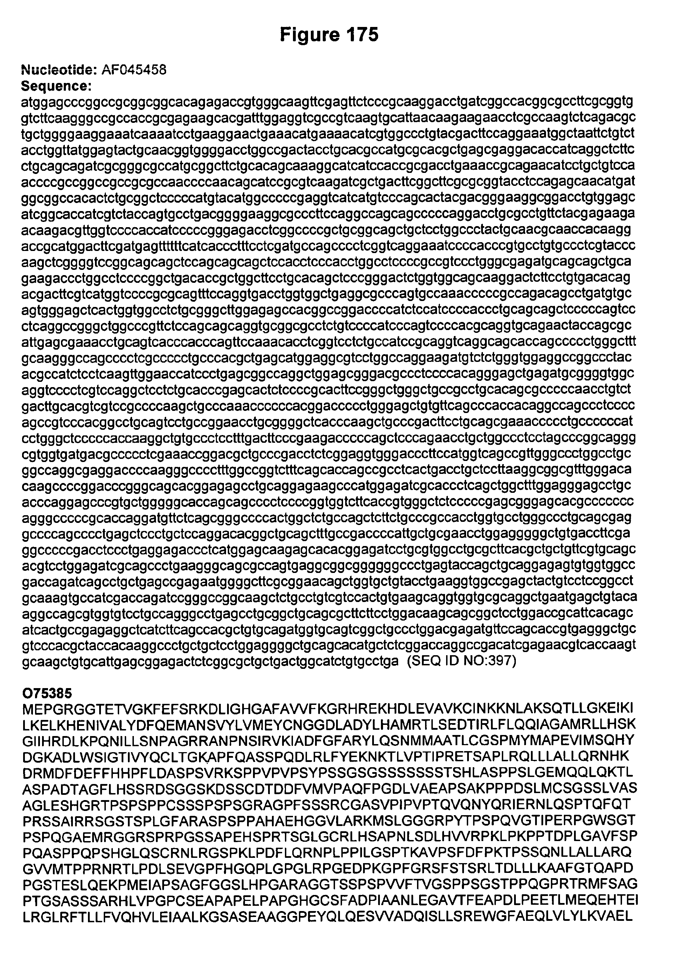

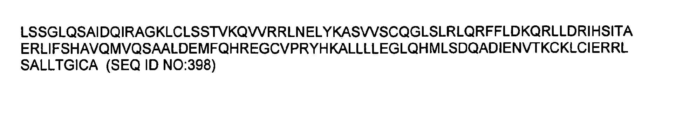

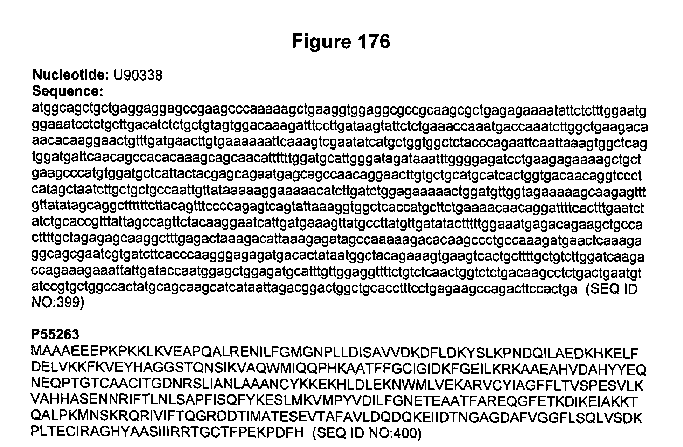

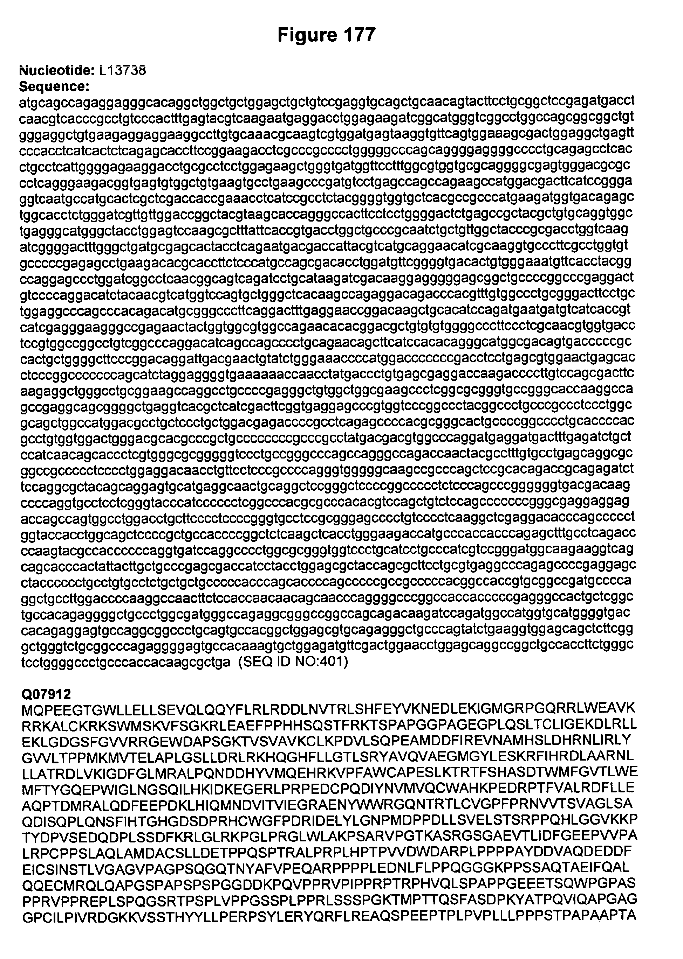

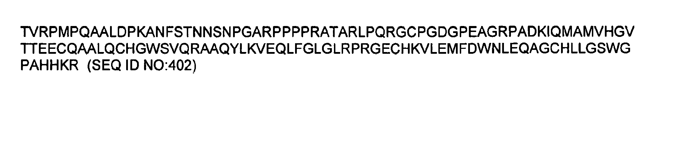

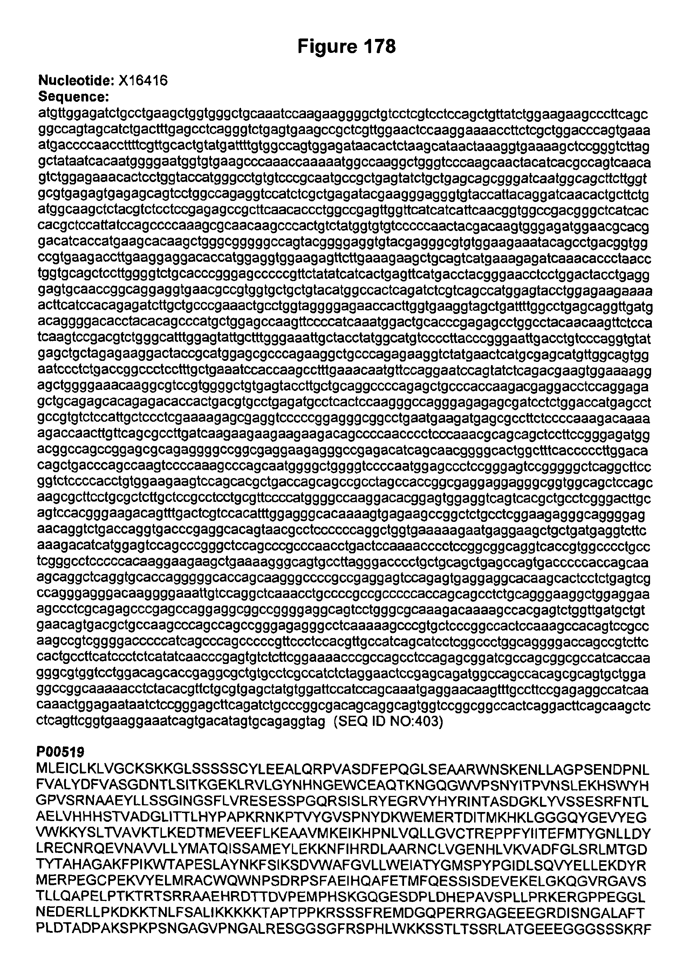

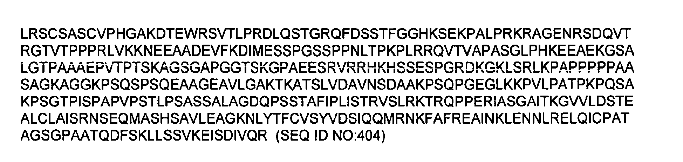

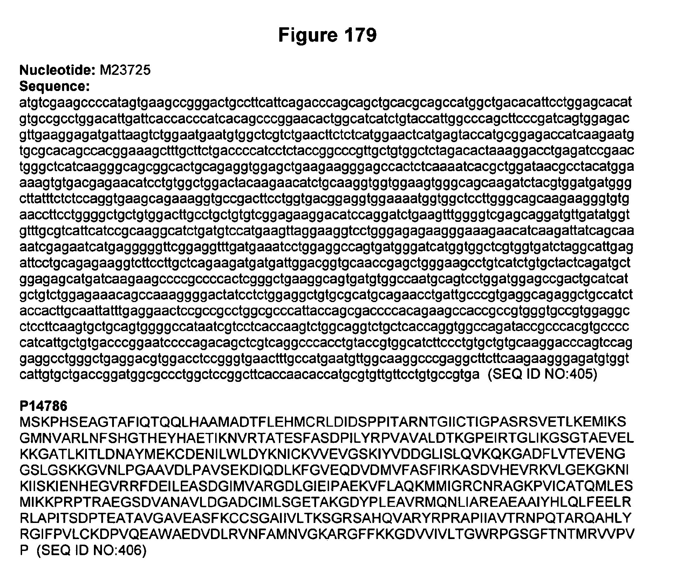

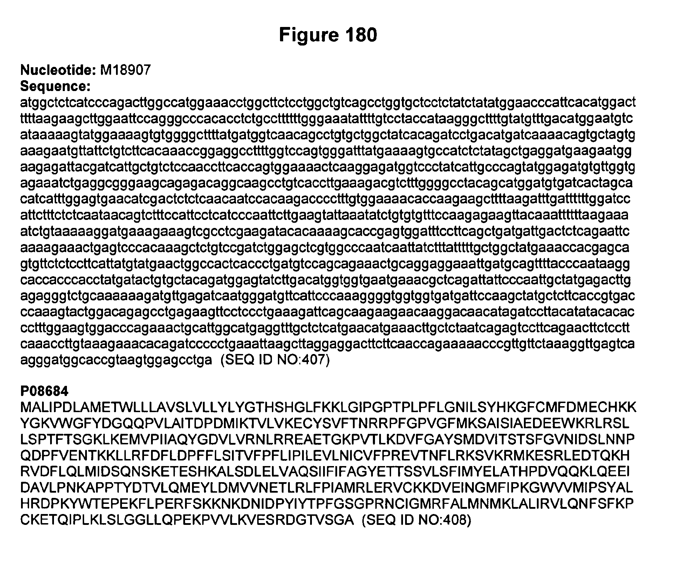

According to a first aspect of the present invention, we provide a polynucleotide selected from the group consisting of odd-numbered SEQ ID NOs in Figures 1 to 180, or a polypeptide encoded by the polynucleotide, for use in a method of prevention, treatment or diagnosis of a disease in an individual.

There is provided, according to a second aspect of the present invention, a polypeptide selected from the group consisting of even-numbered SEQ ID NOs in Figures 1 to 180 for use in a method of prevention, treatment or diagnosis of a disease in an individual.

Preferably, the polypeptide or polynucleotide is used to identify a substance capable of binding to the polypeptide, which method comprises incubating the polypeptide, or a polypeptide encoded by the polynucleotide, with a candidate substance under suitable conditions and determining whether the substance binds to the polypeptide.

Preferably, the polypeptide or polynucleotide is used to identify a substance capable of modulating the function of the polypeptide, the method comprising the steps of: incubating the polypeptide, or a polypeptide encoded by the polynucleotide, with a candidate substance and determining whether activity of the polypeptide is thereby modulated.

In preferred embodiments, the polynucleotide or polypeptide, preferably in an effective amount, is administered to an individual in need of such treatment. Alternatively, or in addition, the substance identified by the method is administered to an individual in need of such treatment.

Preferably, the polynucleotide is provided for a diagnostic use as specified therein, in which the presence or absence of a polynucleotide is detected in a biological sample in a method comprising: (a) bringing the biological sample containing nucleic acid such as DNA or RNA into contact with a probe comprising a fragment of at least 15 nucleotides of the polynucleotide under hybridising conditions; and (b) detecting any duplex formed between the probe and nucleic acid in the sample.

Preferably, the presence or absence of a polypeptide is detected in a biological sample in a method comprising:

(a) providing an antibody capable of binding to the polypeptide; (b) incubating a biological sample with said antibody under conditions which allow for the formation of an antibody-antigen complex; and (c) determining whether antibody-antigen complex comprising said antibody is formed.

We provide, according to a third aspect of the present invention, an antibody capable of binding to a polypeptide selected from the group consisting of even-numbered SEQ ID NOs in Figures 1 to 180 for use in a method of prevention, treatment or diagnosis of a disease in an individual.

Preferably, the antibody is provided for a diagnostic use as specified therein, in which the method comprises contacting a cell or tissue sample with the antibody and detecting an antibody/antigen complex, wherein said detection is indicative of said disease or condition.

Preferably, the disease comprises a proliferative disease, a disease or condition characterized by cell proliferation in mammalian tissue or a tumour, preferably a cancer.

As a fourth aspect of the present invention, there is provided a method for detecting the presence or absence of a polynucleotide in a biological sample which comprises: (a) bringing the biological sample containing DNA or RNA into contact with a probe comprising a fragment of at least 15 nucleotides of a nucleic acid selected from the group consisting of odd-numbered SEQ ID NOs in Figures 1 to 180 under hybridising conditions; and (b) detecting a duplex formed between the probe and nucleic acid in the sample.

We provide, according to a fifth aspect of the present invention, a method of modulating, preferably down-regulating, the expression of a polynucleotide selected from the group consisting of odd-numbered SEQ ID NOs in Figures 1 to 180 in a cell, preferably an isolated cell, the method comprising introducing a double stranded RNA (dsRNA) corresponding to the polynucleotide, or an antisense RNA corresponding to the polynucleotide, or a fragment thereof, into the cell.

The present invention, in a sixth aspect, provides use of a polypeptide in a method of identifying a substance capable of affecting the function of the corresponding gene, in which the polypeptide is selected from the group consisting of even-numbered SEQ ID NOs in Figures 1 to 180

In a seventh aspect of the present invention, there is provided use of a polypeptide in an assay for identifying a substance capable of inhibiting the cell division cycle, in which the polypeptide is selected from the group consisting of even-numbered SEQ ID NOs in Figures 1 to 180.

Preferably, the substance is capable of inhibiting cell cycle progression, preferably mitosis and/or meiosis.

According to an eighth aspect of the present invention, we provide a substance identified by a method or assay as set out above.

We provide, according to a ninth aspect of the invention, use of such a substance in a method of inhibiting the function of a polypeptide, or a method of regulating a cell division cycle function.

There is provided, in accordance with a tenth aspect of the present invention, a method of modulating cell cycle progression in a cell, the method comprising: (a) transforming into the cell a nucleic acid sequence selected from the group consisting of: (i) a nucleic acid sequence encoding a polypeptide of any of the even-numbered SEQ ID NOs in Figures 1 to 180; (ii) a nucleic acid sequence encoding a polypeptide having at least 80% sequence identity to a polypeptide of any of the even-numbered SEQ ID NOs in Figures 1 to 180; (iii) a nucleic acid having a sequence comprising any of the odd-numbered SEQ ID NOs in Figures 1 to 180; (iv) a nucleic acid having at least 80% sequence identity to a nucleic acid having a sequence comprising any of the odd-numbered SEQ ID NOs in Figures 1 to 180; and (v) a complement of any of (i) to (iv); wherein the nucleic acid sequence is operably linked to a regulatory sequence; and (b) culturing the cell under conditions whereby the nucleic acid sequence is expressed; thereby modulating cell cycle progression in the cell.

As an eleventh aspect of the invention, we provide a method of modulating cell cycle progression in a cell, the method comprising: (a) transforming into the cell an isolated nucleic acid sequence that encodes a ribonucleic acid (RNA) precursor, wherein the precursor comprises: (i) a first stem portion comprising a 15 to 40 nucleotide long sequence from any of the odd-numbered SEQ ID NOs in Figures 1 to 180; (ii) a second stem portion comprising a 15 to 40 nucleotide long sequence that is complementary to 15 to 40 consecutive nucleotides of a sequence of any of the odd-numbered SEQ ID NOs in Figures 1 to 180, and wherein the first and second stem portions can hybridise with each other to form a duplex stem; and (iii) a loop portion that connects the two stem portions; (b) culturing the cell under conditions whereby the nucleic acid sequence is expressed, thereby modulating cell cycle progression in the cell.

Preferably, the nucleic acid sequence encodes a polypeptide selected from the group consisting of the even-numbered SEQ ID NOs in Figures 1 to 180. Preferably, the nucleic acid sequence comprises a sequence selected from the group consisting of the odd-numbered SEQ ID NOs in Figures 1 to 180, or a complement thereof.

In some embodiments, cell cycle progression is decreased, preferably resulting from a decrease in proliferation of the cell. In other embodiments, cell cycle progression is increased, preferably resulting from a increase in proliferation of the cell.

We provide, according to a twelfth aspect of the invention, a host cell transformed by a method as described.

According to a thirteenth aspect of the present invention, we provide a method for providing a mammal with an anti-proliferative protein, the method comprising introducing into the mammal a mammalian cell transformed by a method as set out above.

There is provided, according to a fourteenth aspect of the present invention, a RNA precursor encoded by a nucleic acid sequence selected from the group consisting of the odd-numbered SEQ ID NOs in Figures 1 to 180. We provide, according to a fifteenth aspect of the present invention, a composition comprising, as a biologically active ingredient, such an RNA precursor.

Preferably, the RNA precursor or a composition is for use in a method of treatment of a disease or condition characterised by cell proliferation, preferably cancer.

We provide, according to a fifteenth aspect of the present invention, a pharmaceutical composition comprising, as an active ingredient: (a) a cell cycle progression nucleic acid sequence of the odd-numbered SEQ ID NOs in Figures 1 to 180; (b) a cell cycle progression polypeptide selected from the group consisting of the even-numbered SEQ ID NOs in Figures 1 to 180; or (c) an antibody to a cell cycle progression polypeptide selected from the group consisting of the even-numbered SEQ ID NOs in Figures 1 to 180; and a pharmaceutically-acceptable carrier.

According to a sixteenth aspect of the present invention, we provide an antagonist of a cell cycle progression polypeptide selected from the group consisting of the even-numbered SEQ ID NOs in Figures 1 to 180 for use in a method for treating a disease or condition characterized by cell proliferation in mammalian tissue, the method comprising contacting the tissue with the antagonist.

According to a seventeenth aspect of the present invention, we provide an agonist of a cell cycle progression polypeptide selected from the group consisting of the even-numbered SEQ ID NOs in Figures 1 to 180 for use in a method for treating a disease or condition characterized by cell proliferation in mammalian tissue, the method comprising contacting the tissue with the agonist.

We provide, according to an eighteenth aspect of the present invention, a kit for treating a disease or condition characterized by cell proliferation in mammalian tissue, the kit comprising: (a) a polypeptide encoded by a nucleic acid sequence of any of the odd-numbered SEQ ID NOs in Figures 1 to 180; (b) a nucleic acid having a nucleotide sequence of any of the odd-numbered SEQ ID NOs in Figures 1 to 180; or (c) an antibody recognising an epitope of a polypeptide of (a).

According to a nineteenth aspect of the present invention, we provide an array comprising at least two cell cycle progression genes having nucleic acid sequences of any of the odd-numbered SEQ ID NOs in Figures 1 to 180.

Preferably, the nucleic acid sequences are DNA sequences or RNA sequences.

As an twentieth aspect of the invention, we provide an array comprising at least two cell cycle progression proteins having polypeptide sequences of any of the even-numbered SEQ ID NOs in Figures 1 to 180.

By "cell cycle progression" is meant any of the steps or stages in the cell cycle, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, and cytokinesis functions. Functions of the polynucleotides and polypeptides disclosed herein also include functions such as chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways. By saying that the "functional activity" of the polynucleotides and polypeptides disclosed herein is increased or decreased, it is meant that cell cycle progression is increased or decreased as a result of a change in one of these functions. Change in cell cycle progression can be measured by any of a number of standard assays, *e.g.*, mitotic index.

## DESCRIPTION OF THE DRAWINGS

Figure 1 shows the nucleotide sequence for *Drosophila* gene CG3632 (GI 10728281), which has four transcripts, CT12163 (SEQ ID NO:1), CT13680 (SEQ ID NO:3), CT13700 (SEQ ID NO:5) and CT13718 (SEQ ID NO:7), which encode GI Acc. AAF48583 (SEQ ID NO:2), AAF48584 (SEQ ID NO:4), AAF48582 (SEQ ID NO:6) and AAF48581 (SEQ ID NO:8), respectively.

Figure 2 shows the nucleotide sequence for *Drosophila* gene Pp1-87B (GI 7299572) (SEQ ID NO:9), which encodes protein GI Acc. AAF54810 (SEQ ID NO:10).

Figure 3 shows the nucleotide sequence for *Drosophila* gene CG3524 (GI 10727365) (SEQ ID NO:11), which encodes protein GI Acc. AAF51149 (SEQ ID NO:12).

Figure 4 shows the nucleotide sequence for *Drosophila* gene CG9311 (GI 10727923) (SEQ ID NO:13), which encodes protein GI Acc. AAF49705 (SEQ ID NO:14).

Figure 5 shows the nucleotide sequence for *Drosophila* gene CG9092 (GI 7297037) (SEQ ID NO:15), which encodes protein GI Acc. AAF52321 (SEQ ID NO:16).

Figure 6 shows the nucleotide sequence for *Drosophila* gene Arr1 (GI 10728850) (SEQ ID NO:17), which encodes protein GI Acc. AAF53644 (SEQ ID NO:18).

Figure 7 shows the nucleotide sequence for *Drosophila* gene CG9150 (GI 7297037) (SEQ ID NO:19), which encodes protein GI Acc. AAF52338 (SEQ ID NO:20).

Figure 8 shows the nucleotide sequence for *Drosophila* gene CG11102 (GI 10728232) (SEQ ID NO:21), which encodes protein GI Acc. AAF48320 (SEQ ID NO:22).

Figure 9 shows the nucleotide sequence for *Drosophila* gene Smr (GI 7292788) (SEQ ID NO:23), which encodes protein GI Acc. AAF48196 (SEQ ID NO:24).

Figure 10 shows the nucleotide sequence for *Drosophila* gene CG8045 (GI 7300335), which has three transcripts, CT24072 (SEQ ID NO:25), CT24102 (SEQ ID NO:27) andCT24092 (SEQ ID NO:29), which encode GI Acc. AAF55517 (SEQ ID NO:26), GI Acc. AAF55519 (SEQ ID NO:28) and GI Acc. AAF55523 (SEQ ID NO:30), respectively.

Figure 11 shows the nucleotide sequence for *Drosophila* gene CG10420 (GI 7301280) (SEQ ID NO:31), which encodes protein GI Acc. AAF56422 (SEQ ID NO:32).

Figure 12 shows the nucleotide sequence for *Drosophila* gene Hsc70-2 (GI 10726497) (SEQ ID NO:33), which encodes protein GI Acc. AAF54899 (SEQ ID NO:34).

Figure 13 shows the nucleotide sequence for *Drosophila* gene CG10805 (GI 7297167) (SEQ ID NO:35), which encodes protein GI Acc. AAF52447 (SEQ ID NO:36).

Figure 14 shows the nucleotide sequence for *Drosophila* gene eIF-4a (GI 7297037) (SEQ ID NO:37), which encodes protein GI Acc. AAF52317 (SEQ ID NO:38).

Figure 15 shows the nucleotide sequence for *Drosophila* gene ACXA (GI 7297983) (SEQ ID NO:39), which encodes protein GI Acc. AAF53228 (SEQ ID NO:40).

Figure 16 shows the nucleotide sequence for *Drosophila* gene CG15117 (GI 10727456) (SEQ ID NO:41), which encodes protein GI Acc. AAF57602 (SEQ ID NO:42).

Figure 17 shows the nucleotide sequence for *Drosophila* gene BG:DS01759.2 (GI 7298121) (SEQ ID NO:43), which encodes protein GI Acc. AAF53376 (SEQ ID NO:44).

Figure 18 shows the nucleotide sequence for *Drosophila* gene TepIII (GI 7297264) (SEQ ID NO:45), which encodes protein GI Acc. AAF52542 (SEQ ID NO:46).

Figure 19 shows the nucleotide sequence for *Drosophila* gene Hsc70-4 (GI 10726541) (SEQ ID NO:47), which encodes protein GI Acc. AAF55150 (SEQ ID NO:48).

Figure 20 shows the nucleotide sequence for *Drosophila* gene CG7069 (GI 10726692) (SEQ ID NO:49), which encodes protein GI Acc. AAF55980 (SEQ ID NO:50).

Figure 21 shows the nucleotide sequence for *Drosophila* gene ACXE (GI 7297983) (SEQ ID NO:51), which encodes protein GI Acc. AAF53229 (SEQ ID NO:52).

Figure 22 shows the nucleotide sequence for *Drosophila* gene EG:52C10.5 (GI 10727480) (SEQ ID NO:53), which encodes protein GI Acc. AAF57789 (SEQ ID NO:54).

Figure 23 shows the nucleotide sequence for *Drosophila* gene gatA (GI 10726610) (SEQ ID NO:55), which encodes protein GI Acc. AAF55624 (SEQ ID NO:56).

Figure 24 shows the nucleotide sequence for *Drosophila* gene CG17149 (GI 10727803), which has two transcripts, CT33310 (SEQ ID NO:57) and CT38086 (SEQ ID NO:59), which encode GI Acc. AAF49052 (SEQ ID NO:58) and GI Acc. AAF49051 (SEQ ID NO:60), respectively.

Figure 25 shows the nucleotide sequence for *Drosophila* gene CG2905 (GI 10728163) (SEQ ID NO:61), which encodes protein GI Acc. AAF57342 (SEQ ID NO:62).

Figure 26 shows the nucleotide sequence for *Drosophila* gene CG2336 (GI 10727121) (SEQ ID NO:63), which encodes protein GI Acc. AAF54111 (SEQ ID NO:64).

Figure 27 shows the nucleotide sequence for *Drosophila* gene TER94 (GI 10727672), which has two transcripts, CT7768 (SEQ ID NO:65) and CT7776 (SEQ ID NO:67), which encode GI Acc. AAF58863 (SEQ ID NO:66) and GI Acc. AAF58864 (SEQ ID NO:68), respectively.

Figure 28 shows the nucleotide sequence for *Drosophila* gene CG6313 (GI 10726739) (SEQ ID NO:69), which encodes protein GI Acc. AAF56267 (SEQ ID NO:70).

Figure 29 shows the nucleotide sequence for *Drosophila* gene aur (GI 10726473) (SEQ ID NO:71), which encodes protein GI Acc. AAF54723 (SEQ ID NO:72).

Figure 30 shows the nucleotide sequence for *Drosophila* gene Pk91C (GI 10799498) (SEQ ID NO:73), which encodes protein GI Acc. AAF55594 (SEQ ID NO:74).

Figure 31 shows the nucleotide sequence for *Drosophila* gene Top2 (GI 10728874) (SEQ ID NO:75), which encodes protein GI Acc. AAF53802 (SEQ ID NO:76).

Figure 32 shows the nucleotide sequence for *Drosophila* gene alpha-Est1 (GI 10727101) (SEQ ID NO:77), which encodes protein GI Acc. AAG22202 (SEQ ID NO:78).

Figure 33 shows the nucleotide sequence for *Drosophila* gene Nrk (GI 10727582) (SEQ ID NO:79), which encodes protein GI Acc. AAF58420 (SEQ ID NO:80).

Figure 34 shows the nucleotide sequence for *Drosophila* gene otk (GI 10727617) (SEQ ID NO:81), which encodes protein GI Acc. AAF58596 (SEQ ID NO:82).

Figure 35 shows the nucleotide sequence for *Drosophila* gene cad (GI 10799497) (SEQ ID NO:83), which encodes protein GI Acc. AAF53923 (SEQ ID NO:84).

Figure 36 shows the nucleotide sequence for *Drosophila* gene Rut (GI 10728252) (SEQ ID NO:85), which encodes protein GI Acc. AAF48388 (SEQ ID NO:86).

Figure 37 shows the nucleotide sequence for *Drosophila* gene CG8002 (GI 10728334) (SEQ ID NO:87), which encodes protein GI Acc. AAF48942 (SEQ ID NO:88).

Figure 38 shows the nucleotide sequence for *Drosophila* gene CG10335 (GI 10727968) (SEQ ID NO:89), which encodes protein GI Acc. AAF49936 (SEQ ID NO:90).

Figure 39 shows the nucleotide sequence for *Drosophila* gene CG8070 (GI 10727693) (SEQ ID NO:91), which encodes protein GI Acc. AAF58986 (SEQ ID NO:92).

Figure 40 shows the nucleotide sequence for *Drosophila* gene CG7460 (GI 10727853) (SEQ ID NO:93), which encodes protein GI Acc. AAF49310 (SEQ ID NO:94).

Figure 41 shows the nucleotide sequence for *Drosophila* gene CG17735 (GI 10727172) (SEQ ID NO:95), which encodes protein GI Acc. AAF52092 (SEQ ID NO:96).

Figure 42 shows the nucleotide sequence for *Drosophila* gene Gycalpha99B (GI 7301790) (SEQ ID NO:97), which encodes protein GI Acc. AAF56917 (SEQ ID NO:98).

Figure 43 shows the nucleotide sequence for *Drosophila* gene CG13893 (GI 7291959) (SEQ ID NO:99), which encodes protein GI Acc. AAF47396 (SEQ ID NO:100).

Figure 44 shows the nucleotide sequence for *Drosophila* gene CG18176 (GI 10728019) (SEQ ID NO:101), which encodes protein GI Acc. AAF50214 (SEQ ID NO:102).

Figure 45 shows the nucleotide sequence for *Drosophila* gene CG8858 (GI 10727617) (SEQ ID NO:103), which encodes protein GI Acc. AAF58554 (SEQ ID NO:104).

Figure 46 shows the nucleotide sequence for *Drosophila* gene Ac76E (GI 10733346) (SEQ ID NO:105), which encodes protein GI Acc. AAF49089 (SEQ ID NO:106).

Figure 47 shows the nucleotide sequence for *Drosophila* gene CG17010 (GI 7297915) (SEQ ID NO:107), which encodes protein GI Acc. AAF53182 (SEQ ID NO:108).

Figure 48 shows the nucleotide sequence for *Drosophila* gene Tkv (GI 7296952) (SEQ ID NO:109), which encodes protein GI Acc. AAF52230 (SEQ ID NO:110).

Figure 49 shows the nucleotide sequence for *Drosophila* gene Dnt (GI 10728868) (SEQ ID NO:111), which encodes protein GI Acc. AAF53783 (SEQ ID NO:112).

Figure 50 shows the nucleotide sequence for *Drosophila* gene ACXD (GI 10727242) (SEQ ID NO:113), which encodes protein GI Acc. AAF47621 (SEQ ID NO:114).

Figure 51 shows the nucleotide sequence for *Drosophila* gene Aats-ala-m (GI 10728137) (SEQ ID NO:115), which encodes protein GI Acc. AAF50804 (SEQ ID NO:116).

Figure 52 shows the nucleotide sequence for *Drosophila* gene Gek (GI 7291737) (SEQ ID NO:117), which encodes protein GI Acc. AAF47163 (SEQ ID NO:118).

Figure 53 shows the nucleotide sequence for *Drosophila* gene CG3216 (GI 10726992) (SEQ ID NO:119), which encodes protein GI Acc. AAF46649 (SEQ ID NO:120).

Figure 54 shows the nucleotide sequence for *Drosophila* gene CG5653 (GI 10728037) (SEQ ID NO:121), which encodes protein GI Acc. AAF50345 (SEQ ID NO:122).

Figure 55 shows the nucleotide sequence for *Drosophila* gene CG17740 (GI 10727606) (SEQ ID NO:123), which encodes protein GI Acc. AAF58535 (SEQ ID NO:124).

Figure 56 shows the nucleotide sequence for *Drosophila* gene TepI (GI 7298255) (SEQ ID NO:125), which encodes protein GI Acc. AAF53490 (SEQ ID NO:126).

Figure 57 shows the nucleotide sequence for *Drosophila* gene *for* (GI 10727349), which has five transcripts, CT43154 (SEQ ID NO:127), CT42452 (SEQ ID NO:129), CT43152 (SEQ ID NO:131), CT43158 (SEQ ID NO:133) and CT43160 (SEQ ID NO:135), which encode GI Acc. AAF51082 (SEQ ID NO:128), GI Acc. AAG22251 (SEQ ID NO:130), GI Acc. AAG22252 (SEQ ID NO:132), GI Acc. AAG22253 (SEQ ID NO:134) and GI Acc. AAG22254 (SEQ ID NO:136), respectively.

Figure 58 shows the nucleotide sequence for *Drosophila* gene Ac13E (GI 10728265) (SEQ ID NO:137), which encodes protein GI Acc. AAF48468 (SEQ ID NO:138).

Figure 59 shows the nucleotide sequence for *Drosophila* gene CG2667 (GI 7298935) (SEQ ID NO:139), which encodes protein GI Acc. AAF54154 (SEQ ID NO:140).

Figure 60 shows the nucleotide sequence for *Drosophila* gene CG7842 (GI 10727872) (SEQ ID NO:141), which encodes protein GI Acc. AAF49377 (SEQ ID NO:142).

Figure 61 shows the nucleotide sequence for *Drosophila* gene CG 17486 (GI 7289853) (SEQ ID NO:143), which encodes protein GI Acc. AAF45462 (SEQ ID NO:144).

Figure 62 shows the nucleotide sequence for *Drosophila* gene CG6969 (GI 10726705) (SEQ ID NO:145), which encodes protein GI Acc. AAF56043 (SEQ ID NO:146).

Figure 63 shows the nucleotide sequence for *Drosophila* gene CG12262 (GI 10728071) (SEQ ID NO:147), which encodes protein GI Acc. AAF50524 (SEQ ID NO:148).

Figure 64 shows the nucleotide sequence for *Drosophila* gene Fray (GI 10726601) (SEQ ID NO:149), which encodes protein GI Acc. AAF55567 (SEQ ID NO:150).

Figure 65 shows the nucleotide sequence for *Drosophila* gene CG6879 (GI 10726756) (SEQ ID NO:151), which encodes protein GI Acc. AAF56334 (SEQ ID NO:152).

Figure 66 shows the nucleotide sequence for *Drosophila* gene CG11594 (GI 10727290) (SEQ ID NO:153), which encodes protein GI Acc. AAF47823 (SEQ ID NO:154).

Figure 67 shows the nucleotide sequence for *Drosophila* gene S6kII (GI 10803726) (SEQ ID NO:155), which encodes protein GI Acc. AAF50945 (SEQ ID NO:156).

Figure 68 shows the nucleotide sequence for *Drosophila* gene CG11714 (GI 10727982) (SEQ ID NO:157), which encodes protein GI Acc. AAF50053 (SEQ ID NO:158).

Figure 69 shows the nucleotide sequence for *Drosophila* gene CG3534 (GI 7300193) (SEQ ID NO:159), which encodes protein GI Acc. AAF55371 (SEQ ID NO:160).

Figure 70 shows the nucleotide sequence for *Drosophila* gene CG7335 (GI 10727803) (SEQ ID NO:161), which encodes protein GI Acc. AAF49067 (SEQ ID NO:162).

Figure 71 shows the nucleotide sequence for *Drosophila* gene CG11275 (GI 7291355) (SEQ ID NO:163), which encodes protein GI Acc. AAF46814 (SEQ ID NO:164).

Figure 72 shows the nucleotide sequence for *Drosophila* gene CG16726 (GI 10728019) (SEQ ID NO:165), which encodes protein GI Acc. AAF50229 (SEQ ID NO:166).

Figure 73 shows the nucleotide sequence for *Drosophila* gene CG7514 (GI 10727313) (SEQ ID NO:167), which encodes protein GI Acc. AAF47931 (SEQ ID NO:168).

Figure 74 shows the nucleotide sequence for *Drosophila* gene CG17283 (GI 7300241) (SEQ ID NO:169), which encodes protein GI Acc. AAF55416 (SEQ ID NO:170).

Figure 75 shows the nucleotide sequence for *Drosophila* gene BcDNA:GH07626 (GI 10727365) (SEQ ID NO:171), which encodes protein GI Acc. AAF51148 (SEQ ID NO:172).

Figure 76 shows the nucleotide sequence for *Drosophila* gene CG16752 (GI 10728478) (SEQ ID NO:173), which encodes protein GI Acc. AAF46037 (SEQ ID NO:174).

Figure 77 shows the nucleotide sequence for *Drosophila* gene Rpt1 (GI 10727757) (SEQ ID NO:175), which encodes protein GI Acc. AAF59219 (SEQ ID NO:176).

Figure 78 shows the nucleotide sequence for *Drosophila* gene Wts (GI 7301969) (SEQ ID NO:177), which encodes protein GI Acc. AAF57085 (SEQ ID NO:178).

Figure 79 shows the nucleotide sequence for *Drosophila* gene CG1582 (GI 7292554) (SEQ ID NO:179), which encodes protein GI Acc. AAF47973 (SEQ ID NO:180).

Figure 80 shows the nucleotide sequence for *Drosophila* gene CG12289 (GI 10727982) (SEQ ID NO:181), which encodes protein GI Acc. AAF50065 (SEQ ID NO:182).

Figure 81 shows the nucleotide sequence for *Drosophila* gene Pepck (GI 10727469) (SEQ ID NO:183), which encodes protein GI Acc. AAF57676 (SEQ ID NO:184).

Figure 82 shows the nucleotide sequence for *Drosophila* gene CG5665 (GI 10726906) (SEQ ID NO:185), which encodes protein GI Acc. AAF51579 (SEQ ID NO:186).

Figure 83 shows the nucleotide sequence for *Drosophila* gene CG7285 (GI 10727839) (SEQ ID NO:187), which encodes protein GI Acc. AAF49259 (SEQ ID NO:188).

Figure 84 shows the nucleotide sequence for *Drosophila* gene Bt (GI 10726313) (SEQ ID NO:189), which encodes protein GI Acc. AAF59316 (SEQ ID NO:190).

Figure 85 shows the nucleotide sequence for *Drosophila* gene CG8795 (GI 10726505) (SEQ ID NO:191), which encodes protein GI Acc. AAF54929 (SEQ ID NO:192).

Figure 86 shows the nucleotide sequence for *Drosophila* gene CG10967 (GI 10727955) (SEQ ID NO:193), which encodes protein GI Acc. AAF49878 (SEQ ID NO:194).

Figure 87 shows the nucleotide sequence for *Drosophila* gene CG3809 (GI 10726480) (SEQ ID NO:195), which encodes protein GI Acc. AAF54757 (SEQ ID NO:196).

Figure 88 shows the nucleotide sequence for *Drosophila* gene Ack (GI 10727290) (SEQ ID NO:197), which encodes protein GI Acc. AAF47839 (SEQ ID NO:198).

Figure 89 shows the nucleotide sequence for *Drosophila* gene Abl (GI 10727878) (SEQ ID NO:199), which encodes protein GI Acc. AAF49431 (SEQ ID NO:200).

Figure 90 shows the nucleotide sequence for *Drosophila* gene CG7362 (GI 10726534) (SEQ ID NO:201), which encodes protein GI Acc. AAF55096 (SEQ ID NO:202).

Figure 91 shows the nucleotide sequence for *Drosophila* gene Cyp9f2 (GI 7299572) (SEQ ID NO:203), which encodes protein GI Acc. AAF54803 (SEQ ID NO:204).

Figure 92 shows the nucleic acid (SEQ ID NO:205) and amino acid (SEQ ID N0:206) sequence for the human homolog to *Drosophila* gene CG3632 (SWISS-PROT Ref. No. Q9UEG3).

Figure 93 shows the nucleic acid (SEQ ID NO:207) and amino acid (SEQ ID NO:208) sequence for the human homolog to *Drosophila* gene Pp1-87B (SWISS-PROT Ref. No. P36873).

Figure 94 shows the nucleic acid (SEQ ID NO:209) and amino acid (SEQ ID NO:210) sequence for the human homolog to *Drosophila* gene CG3524 (SWISS-PROT Ref. No. Q 16702).

Figure 95 shows the nucleic acid (SEQ ID NO:211) and amino acid (SEQ ID NO:212) sequence for the human homolog to *Drosophila* gene CG9311 (SWISS-PROT Ref. No. Q9H3S7).

Figure 96 shows the nucleic acid (SEQ ID NO:213) and amino acid (SEQ ID NO:214) sequence for the human homolog to *Drosophila* gene CG9092 (SWISS-PROT Ref. No. P16278).

Figure 97 shows the nucleic acid (SEQ ID NO:215) and amino acid (SEQ ID NO:216) sequence for the human homolog to *Drosophila* gene Arr1 (SWISS-PROT Ref. No. P49407).

Figure 98 shows the nucleic acid (SEQ ID NO:217) and amino acid (SEQ ID NO:218) sequence for the human homolog to *Drosophila* gene CG9150 (SWISS-PROT Ref. No. Q9BUC7).

Figure 99 shows the nucleic acid (SEQ ID NO:219) and amino acid (SEQ ID NO:220) sequence for the human homolog to *Drosophila* gene CG11102 (SWISS-PROT Ref. No. BAA31634).

Figure 100 shows the nucleic acid (SEQ ID NO:221) and amino acid (SEQ ID NO:222) sequence for the human homolog to *Drosophila* gene Smr (SWISS-PROT Ref. No. O75376).

Figure 101 shows the nucleic acid (SEQ ID NO:223) and amino acid (SEQ ID NO:224) sequence for the human homolog to *Drosophila* gene CG8045 (SWISS-PROT Ref. No. P42655).

Figure 102 shows the nucleic acid (SEQ ID NO:225) and amino acid (SEQ ID NO:226) sequence for the human homolog to *Drosophila* gene CG10420 (SWISS-PROT Ref. No. Q9H173).

Figure 103 shows the nucleic acid (SEQ ID NO:227) and amino acid (SEQ ID NO:228) sequence for the human homolog to *Drosophila* gene Hsc70-2 (SWISS-PROT Ref. No. P11142).

Figure 104 shows the nucleic acid (SEQ ID NO:229) and amino acid (SEQ ID NO:230) sequence for the human homolog to *Drosophila* gene CG10805 (SWISS-PROT Ref. No. Q9H583).

Figure 105 shows the nucleic acid (SEQ ID NO:231) and amino acid (SEQ ID NO:232) sequence for the human homolog to *Drosophila* gene eIF-4a (SWISS-PROT Ref. No. Q96EA8).

Figure 106 shows the nucleic acid sequences (SEQ ID NO:233 and SEQ ID NO:235, respectively) and the amino acid sequences (SEQ ID NO:234 and SEQ ID NO:236, respectively) for the two human homologs to *Drosophila* gene ACXA (SWISS-PROT Ref. No. Q08462 and SWISS-PROT Ref. No. P40145).

Figure 107 shows the nucleic acid (SEQ ID NO:237) and amino acid (SEQ ID NO:238) sequence for the human homolog to *Drosophila* gene CG15117 (SWISS-PROT Ref. No. P08236).

Figure 108 shows the nucleic acid (SEQ ID NO:239) and amino acid (SEQ ID NO:240) sequence for the human homolog to *Drosophila* gene TepIII (SWISS-PROT Ref. No. Q8TDJ3).

Figure 109 shows the nucleic acid (SEQ ID NO:241) and amino acid (SEQ ID NO:242) sequence for the human homolog to *Drosophila* gene Hsc70-4 (SWISS-PROT Ref. No. P11142).

Figure 110 shows the nucleic acid (SEQ ID NO:243) and amino acid (SEQ ID NO:244) sequence for the human homolog to *Drosophila* gene CG7069 (SWISS-PROT Ref. No. P14786).

Figure 111 shows the nucleic acid sequences (SEQ ID NO:245 and SEQ ID NO:247, respectively) and amino acid sequences (SEQ ID NO:246 and SEQ ID NO:248, respectively) for the two human homologs to *Drosophila* gene ACXE (SWISS-PROT Ref. No. P40145 and SWISS-PROT Ref. No. P51828).

Figure 112 shows the nucleic acid (SEQ ID NO:249) and amino acid (SEQ ID NO:250) sequence for the human homolog to *Drosophila* gene EG:52C10.5 (SWISS-PROT Ref. No. Q9Y2I7).

Figure 113 shows the nucleic acid (SEQ ID NO:251) and amino acid (SEQ ID NO:252) sequence for the human homolog to *Drosophila* gene gatA (SWISS-PROT Ref. No. Q9H0R6).

Figure 114 shows the nucleic acid (SEQ ID NO:253) and amino acid (SEQ ID NO:254) sequence for the human homolog to *Drosophila* gene CG17149 (SWISS-PROT Ref. No. Q9NUH3).

Figure 115 shows the nucleic acid (SEQ ID NO:255) and amino acid (SEQ ID NO:256) sequence for the human homolog to *Drosophila* gene CG2905 (SWISS-PROT Ref. No. Q9Y6H4).

Figure 116 shows the nucleic acid (SEQ ID NO:257) and amino acid (SEQ ID NO:258) sequence for the human homolog to *Drosophila* gene TER94 (SWISS-PROT Ref. No. P55072).

Figure 117 shows the nucleic acid (SEQ ID NO:259) and amino acid (SEQ ID NO:260) sequence for the human homolog to *Drosophila* gene CG6313 (SWISS-PROT Ref. No. BAA31672).

Figure 118 shows the nucleic acid sequences (SEQ ID NO:261 and SEQ ID NO:263, respectively) and amino acid sequences (SEQ ID NO:262 and SEQ ID NO:264, respectively) for the two human homologs to *Drosophila* gene aur (SWISS-PROT Ref. No. 060445 and SWISS-PROT Ref. No. 060446).

Figure 119 shows the nucleic acid (SEQ ID NO:265) and amino acid (SEQ ID NO:266) sequence for the human homolog to *Drosophila* gene Pk91C (SWISS-PROT Ref. No. Q96SJ5).

Figure 120 shows the nucleic acid sequences (SEQ ID NO:267 and SEQ ID NO:269, respectively) and amino acid sequences (SEQ ID NO:268 and SEQ ID NO:270, respectively) for the two human homologs to *Drosophila* gene Top2 (SWISS-PROT Ref. No. P11388 and SWISS-PROT Ref. No. Q02880).

Figure 121 shows the nucleic acid (SEQ ID NO:271) and amino acid (SEQ ID NO:272) sequence for the human homolog to *Drosophila* gene alpha-Est1 (SWISS-PROT Ref. No. P22303).

Figure 122 shows the nucleic acid (SEQ ID NO:273) and amino acid (SEQ ID NO:274) sequence for the human homolog to *Drosophila* gene Nrk (SWISS-PROT Ref. No. 015146).

Figure 123 shows the nucleic acid (SEQ ID NO:275) and amino acid (SEQ ID NO:276) sequence for the human homolog to *Drosophila* gene otk (SWISS-PROT Ref. No. Q13308).

Figure 124 shows the nucleic acid (SEQ ID NO:277) and amino acid (SEQ ID NO:278) sequence for the human homolog to *Drosophila* gene cad (SWISS-PROT Ref. No. Q99626).

Figure 125 shows the nucleic acid (SEQ ID NO:279) and amino acid (SEQ ID NO:280) sequence for the human homolog to *Drosophila* gene Rut (SWISS-PROT Ref. No. 043306).

Figure 126 shows the nucleic acid (SEQ ID NO:281) and amino acid (SEQ ID NO:282) sequence for the human homolog to *Drosophila* gene CG8002 (SWISS-PROT Ref. No. BAC02708).

Figure 127 shows the nucleic acid (SEQ ID NO:283) and amino acid (SEQ ID NO:284) sequence for the human homolog to *Drosophila* gene CG10335 (SWISS-PROT Ref. No. P13716).

Figure 128 shows the nucleic acid (SEQ ID NO:285) and amino acid (SEQ ID NO:286) sequence for the human homolog to *Drosophila* gene CG8070 (SWISS-PROT Ref. No. Q9NVU7).

Figure 129 shows the nucleic acid (SEQ ID NO:287) and amino acid (SEQ ID NO:288) sequence for the human homolog to *Drosophila* gene CG7460 (SWISS-PROT Ref. No. Q96QT3).

Figure 130 shows the nucleic acid (SEQ ID NO:289) and amino acid (SEQ ID NO:290) sequence for the human homolog to *Drosophila* gene CG17735 (SWISS-PROT Ref. No. Q 14669).

Figure 131 shows the nucleic acid (SEQ ID NO:291) and amino acid (SEQ ID NO:292) sequence for the human homolog to *Drosophila* gene Gycalpha99B (SWISS-PROT Ref. No. P33402).

Figure 132 shows the nucleic acid (SEQ ID NO:293) and amino acid (SEQ ID NO:294) sequence for the human homolog to *Drosophila* gene CG13893 (SWISS-PROT Ref. No. 076054).

Figure 133 shows the nucleic acid (SEQ ID NO:295) and amino acid (SEQ ID NO:296) sequence for the human homolog to *Drosophila* gene CG18176 (SWISS-PROT Ref. No. AAH33918).

Figure 134 shows the nucleic acid (SEQ ID NO:297) and amino acid (SEQ ID NO:298) sequence for the human homolog to *Drosophila* gene CG8858 (SWISS-PROT Ref. No. 015074).

Figure 135 shows the nucleic acid sequences (SEQ ID NO:299 and SEQ ID NO:301, respectively) and amino acid sequences (SEQ ID NO:300 and SEQ ID NO:302, respectively) for the two human homologs to *Drosophila* gene Ac76E (SWISS-PROT Ref. No. Q08462 and SWISS-PROT Ref. No. P51828).

Figure 136 shows the nucleic acid (SEQ ID NO:303) and amino acid (SEQ ID NO:304) sequence for the human homolog to *Drosophila* gene CG17010 (SWISS-PROT Ref. No. Q9H477).

Figure 137 shows the nucleic acid sequences (SEQ ID NO:305, SEQ ID NO:307 and SEQ ID NO:309, respectively) and amino acid sequences (SEQ ID NO:306, SEQ ID NO:308 and SEQ ID NO:310, respectively) for the three human homologs to *Drosophila* gene Tkv (SWISS-PROT Ref. No. 000238, SWISS-PROT Ref. No. P36894 and SWISS-PROT Ref. No. Q04771).

Figure 138 shows the nucleic acid (SEQ ID NO:311) and amino acid (SEQ ID NO:312) sequence for the human homolog to *Drosophila* gene Dnt (SWISS-PROT Ref. No.P34925).

Figure 139 shows the nucleic acid sequences (SEQ ID NO:313 and SEQ ID NO:315, respectively) and amino acid sequences (SEQ ID NO:314 and SEQ ID NO:316, respectively) for the two human homologs to *Drosophila* gene ACXD (SWISS-PROT Ref. No. Q08462 and SWISS-PROT Ref. No. P40145).

Figure 140 shows the nucleic acid (SEQ ID NO:317) and amino acid (SEQ ID NO:318) sequence for the human homolog to *Drosophila* gene Aats-ala-m (SWISS-PROT Ref. No. P49588).

Figure 141 shows the nucleic acid (SEQ ID NO:319) and amino acid (SEQ ID NO:320) sequence for the human homolog to *Drosophila* gene Gek (SWISS-PROT Ref. No. Q9Y5S2).

Figure 142 shows the nucleic acid sequences (SEQ ID NO:321 and SEQ ID NO:323, respectively) and amino acid seqience (SEQ ID NO:322 and SEQ ID NO:324, respectively) for the two human homologs to *Drosophila* gene CG3216 (SWISS-PROT Ref. No. P16066 and SWISS-PROT Ref. No. P20594).

Figure 143 shows the nucleic acid (SEQ ID NO:325) and amino acid (SEQ ID NO:326) sequence for the human homolog to *Drosophila* gene CG5653 (SWISS-PROT Ref. No. Q96QT3).

Figure 144 shows the nucleic acid (SEQ ID NO:327) and amino acid (SEQ ID NO:328) sequence for the human homolog to *Drosophila* gene CG17740 (SWISS-PROT Ref. No. Q9HCB6).

Figure 145 shows the nucleic acid (SEQ ID NO:329) and amino acid (SEQ ID NO:330) sequence for the human homolog to *Drosophila* gene Tepl (SWISS-PROT Ref. No. Q8TDJ3).

Figure 146 shows the nucleic acid (SEQ ID NO:331) and amino acid (SEQ ID NO:332) sequence for the human homolog to *Drosophila* gene for (SWISS-PROT Ref. No. Q13976).

Figure 147 shows the nucleic acid sequences (SEQ ID NO:333 and SEQ ID NO:335, respectively) and amino acid sequences (SEQ ID NO:334 and SEQ ID NO:336, respectively) for the two human homologs to *Drosophila* gene Ac13E (SWISS-PROT Ref. No. 060503 and SWISS-PROT Ref. No. 060266).

Figure 148 shows the nucleic acid (SEQ ID NO:337) and amino acid (SEQ ID NO:338) sequence for the human homolog to *Drosophila* gene CG2667 (SWISS-PROT Ref. No. Q 16760).

Figure 149 shows the nucleic acid (SEQ ID NO:339) and amino acid (SEQ ID NO:340) sequence for the human homolog to *Drosophila* gene CG7842 (SWISS-PROT Ref. No. 095510).

Figure 150 shows the nucleic acid (SEQ ID NO:341) and amino acid (SEQ ID NO:342) sequence for the human homolog to *Drosophila* gene CG17486 (SWISS-PROT Ref. No. Q9NWL6).

Figure 151 shows the nucleic acid (SEQ ID NO:343) and amino acid (SEQ ID NO:344) sequence for the human homolog to *Drosophila* gene CG6969 (SWISS-PROT Ref. No. Q92626).

Figure 152 shows the nucleic acid (SEQ ID NO:345) and amino acid (SEQ ID NO:346) sequence for the human homolog to *Drosophila* gene CG12262 (SWISS-PROT Ref. No. P 11310).

Figure 153 shows the nucleic acid (SEQ ID NO:347) and amino acid (SEQ ID NO:348) sequence for the human homolog to *Drosophila* gene Fray (SWISS-PROT Ref. No. O95747).

Figure 154 shows the nucleic acid (SEQ ID NO:349) and amino acid (SEQ ID NO:350) sequence for the human homolog to *Drosophila* gene CG6879 (SWISS-PROT Ref. No. Q92626).

Figure 155 shows the nucleic acid (SEQ ID NO:351) and amino acid (SEQ ID NO:352) sequence for the human homolog to *Drosophila* gene CG11594 (SWISS-PROT Ref. No. Q96C11).

Figure 156 shows the nucleic acid (SEQ ID NO:353) and amino acid (SEQ ID NO:354) sequence for the human homolog to *Drosophila* gene S6kII (SWISS-PROT Ref. No. P51812).

Figure 157 shows the nucleic acid sequences (SEQ ID NO:355 and SEQ ID NO:357, respectively) and amino acid sequences (SEQ ID NO:356 and SEQ ID NO:358, respectively) for the two human homologs to *Drosophila* gene CG11714 (SWISS-PROT Ref. No. Q9HAB2 and SWISS-PROT Ref. No. 043791).

Figure 158 shows the nucleic acid (SEQ ID NO:359) and amino acid (SEQ ID NO:360) sequence for the human homolog to *Drosophila* gene CG3534 (SWISS-PROT Ref. No. O75191).

Figure 159 shows the nucleic acid (SEQ ID NO:361) and amino acid (SEQ ID NO:362) sequence for the human homolog to *Drosophila* gene CG7335 (SWISS-PROT Ref. No. P50053).

Figure 160 shows the nucleic acid sequences (SEQ ID NO:363 and SEQ ID NO:265, respectively) and amino acid sequences (SEQ ID NO:364 and SEQ ID NO:366, respectively) for the two human homologs to *Drosophila* gene CG11275 (SWISS-PROT Ref. No. Q9HAB2 and SWISS-PROT Ref. No. 043791).

Figure 161 shows the nucleic acid (SEQ ID NO:367) and amino acid (SEQ ID NO:368) sequence for the human homolog to *Drosophila* gene CG16726 (SWISS-PROT Ref. No. P34981).

Figure 162 shows the nucleic acid (SEQ ID NO:369) and amino acid (SEQ ID NO:370) sequence for the human homolog to *Drosophila* gene CG7514 (SWISS-PROT Ref. No. Q02978).

Figure 163 shows the nucleic acid (SEQ ID NO:371) and amino acid (SEQ ID NO:372) sequence for the human homolog to *Drosophila* gene CG17283 (SWISS-PROT Ref. No. P14091).

Figure 164 shows the nucleic acid (SEQ ID NO:373) and amino acid (SEQ ID NO:374) sequence for the human homolog to *Drosophila* gene BcDNA:GH07626 (SWISS-PROT Ref. No. Q16702).

Figure 165 shows the nucleic acid (SEQ ID NO:375) and amino acid (SEQ ID NO:376) sequence for the human homolog to *Drosophila* gene CG16752 (SWISS-PROT Ref. No. Q8TDU8).

Figure 166 shows the nucleic acid (SEQ ID NO:377) and amino acid (SEQ ID NO:378) sequence for the human homolog to *Drosophila* gene Rpt1 (SWISS-PROT Ref. No. P35998).

Figure 167 shows the nucleic acid sequences (SEQ ID NO:379 and SEQ ID NO:381, respectively) and amino acid sequences (SEQ ID NO:380 and SEQ ID NO:382, respectively) for the two human homologs to *Drosophila* gene Wts (SWISS-PROT Ref. No. 095835 and SWISS-PROT Ref. No. Q9P2X1).

Figure 168 shows the nucleic acid (SEQ ID NO:383) and amino acid (SEQ ID NO:384) sequence for the human homolog to *Drosophila* gene CG1582 (SWISS-PROT Ref. No. AAM73547).

Figure 169 shows the nucleic acid (SEQ ID NO:385) and amino acid (SEQ ID NO:386) sequence for the human homolog to *Drosophila* gene CG12289 (SWISS-PROT Ref. No. P50053).

Figure 170 shows the nucleic acid (SEQ ID NO:387) and amino acid (SEQ ID NO:388) sequence for the human homolog to *Drosophila* gene Pepck (SWISS-PROT Ref. No. Q 16822).

Figure 171 shows the nucleic acid (SEQ ID NO:389) and amino acid (SEQ ID NO:390) sequence for the human homolog to *Drosophila* gene CG5665 (SWISS-PROT Ref. No. P06858).

Figure 172 shows the nucleic acid (SEQ ID NO:391) and amino acid (SEQ ID NO:392) sequence for the human homolog to *Drosophila* gene CG7285 (SWISS-PROT Ref. No. Q96TF2).

Figure 173 shows the nucleic acid (SEQ ID NO:393) and amino acid (SEQ ID NO:394) sequence for the human homolog to *Drosophila* gene Bt (SWISS-PROT Ref. No. Q8WZ42).

Figure 174 shows the nucleic acid (SEQ ID NO:395) and amino acid (SEQ ID NO:396) sequence for the human homolog to *Drosophila* gene CG8795 (SWISS-PROT Ref. No. Q9GZQ4).

Figure 175 shows the nucleic acid (SEQ ID NO:397) and amino acid (SEQ ID NO:398) sequence for the human homolog to *Drosophila* gene CG10967 (SWISS-PROT Ref. No. 075385).

Figure 176 shows the nucleic acid (SEQ ID NO:399) and amino acid (SEQ ID NO:400) sequence for the human homolog to *Drosophila* gene CG3809 (SWISS-PROT Ref. No. P55263).

Figure 177 shows the nucleic acid (SEQ ID NO:401) and amino acid (SEQ ID NO:402) sequence for the human homolog to *Drosophila* gene Ack (SWISS-PROT Ref. No. Q07912).

Figure 178 shows the nucleic acid (SEQ ID NO:403) and amino acid (SEQ ID NO:404) sequence for the human homolog to *Drosophila* gene Abl (SWISS-PROT Ref. No. P00519).

Figure 179 shows the nucleic acid (SEQ ID NO:405) and amino acid (SEQ ID NO:406) sequence for the human homolog to *Drosophila* gene CG7362 (SWISS-PROT Ref. No. P14786).

Figure 180 shows the nucleic acid (SEQ ID NO:407) and amino acid (SEQ ID NO:408) sequence for the human homolog to *Drosophila* gene Cyp9f2 (SWISS-PROT Ref. No. P08684).

Figure 181 is a pair of graphs showing FACS profiles for D.Mel-2 cells.

Figure 182 shows cells transfected with Ect2 siRNA COD1513 (aaGUGGGCUUUGUAAAGAUGG) result in a block in mitosis.

Figure 183 shows preferred profile after adjustment of voltages on FSC and SSC channels.

Figure 184 shows the profile after the voltage on FL3 channel is also altered to enable analysis of cells in G1, S and G2/M, setting a gate (G2) to exclude doublets resulting from cell clumping.

Figure 185 is a histogram showing counts against FL3-H within the gated region (G2) and the associated histogram statistics is generated. Using the histogram statistics, events in G1, in S and in G2/M are calculated, where the number of G1 events was equal to M2 x 2, the number of G2/M events is equal to M3 x 2 and the number of S events equated to M1-[(M2 x 2)+(M3 x 2)].

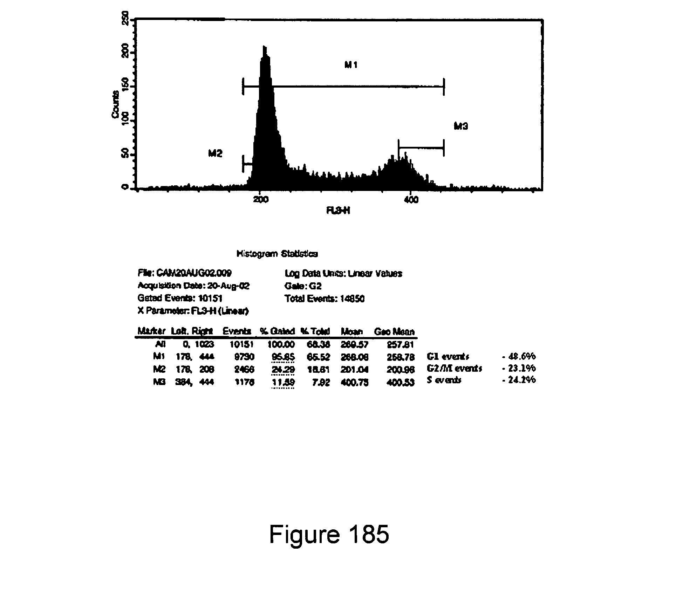

Figure 185 is a histogram showing counts against FL3-H within the gated region (G2) and the associated histogram statistics is generated. Using the histogram statistics, events in G1, in S and in G2/M are calculated, where the number of G1 events was equal to M2 x 2, the number of G2/M events is equal to M3 x 2 and the number of S events equated to M1-[(M2 x 2)+(M3 x 2)].

## DETAILED DESCRIPTION

This document describes human genes involved in cell cycle progression. Such genes can be used in assays described herein to determine if a candidate substance is an inhibitor of cell cycle progression. These same assays can be used to determine if the substance is an enhancer of cell cycle progression.

Such assays include binding asays, where the degree of binding of the substance to the polypeptides described herein is determined. Such assays also include determining if the substance prevents the binding between a polypeptide described herein and another substance known to bind that polypeptide.

The assays also include assays to determine the presence or absence of a polypeptide described herein in a sample, by adding to the sample a substance known to bind that polypeptide, and determining if binding takes place. Alternatively, a nucleic acid probe, the sequence of which is based on the nucleic acid that encodes the polypeptide, can be used to determine the presence or absence in the sample of the nucleic acid encoding the polypeptide. The probe can be added to the sample and incubated under hybridizing conditions to cause binding to the nucleic acid, if it is present in the sample. Such a probe can be labelled to more easily determine if binding has occurred. Alternatively, instead of a nucleic acid probe, an antibody specific for either the nucleic acid of the polypeptide can be used.

In situations where one wishes to know if a mutation has occurred in a nucleic acid encoding a polypeptide described herein, one can obtain a sample containing the nucleic acid, and use a nucleic acid probe specific for the region in which the mutation is believed to occur. Lack of binding, relative to a sample containing the equivalent nucleic acid without the mutation, indicates that the nucleic acid contains a mutation at that location. If it is not known where in the nucleic acid the mutation has occurred, then a sequential series of probes can be used which cover the entire length of the nucleic acid.

These assays can also be used to determine if a polypeptide described herein is being overexpressed in a tissue, *e.g.*, a tumor, or tissue suspected of harboring overproliferating cells. An amount of a known ligand can be added to a sample from the tissue, where the ligand binds the polypeptide. If the amount of bound ligand-polypeptide complex in the sample is greater than that found normally, then the polypeptide is overexpressed. "Normally" can mean the amount of polypeptide found in a sample of another tissue from the same organism, or the equivalent tissue from another organism, or relative to previously-determined baseline levels of expression.

Any of the assays described above can be incorporated into a kit for determining presence/absence of a nucleic acid or polypeptide described herein, or the level of expression of a polypeptide described herein.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, IRL Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

By "modulating cell cycle progression" is meant that for when a given cell is treated, its normal tendency to progress through the cell cycle is changed, either increased or decreased, compared to an untreated cell, where otherwise the environmental conditions are the same.

Mitosis can be measured by FACS analysis, microscopy or by assays based on the status of cell cycle proteins. Standard assays include, but are not limited to, those protocols used in the molecular biological arts to assess cell cycle arrest, cell cycle analysis, cell proliferation of various cell types, detection of apoptosis, *e.g.*, by apoptotic cell morphology or Annexin V-FITC assay, and inhibition of cancer cell growth or tumor growth in various animal models. Such assays are well-known to those of ordinary skill in those fields. Examples of assays are described herein.

The "functional activity" of a protein in the context of the methods and compositions described here describes the function the protein performs in its native environment. Altering the functional activity of a protein includes within its scope increasing, decreasing or otherwise altering the native activity of the protein itself. In addition, it also includes within its scope increasing or decreasing the level of expression and/or altering the intracellular distribution of the nucleic acid encoding the protein, and/or altering the intracellular distribution of the protein itself.

The term "expression" refers to the transcription of a gene's DNA template to produce the corresponding mRNA and translation of this mRNA to produce the corresponding gene product *(i.e.,* a peptide, polypeptide, or protein).

By "polynucleotide" or "polypeptide" is meant the DNA and protein sequences disclosed herein. The terms also include close variants of those sequences, where the variant possesses the same biological activity as the reference sequence. Such variant sequences include "alleles" (variant sequences found at the same genetic locus in the same or closely-related species), "homologs" (a gene related to a second gene by descent from a common ancestral DNA sequence, and separated by either speciation ("ortholog") or genetic duplication ("paralog")), so long as such variants retain the same biological activity as the reference sequence(s) disclosed herein.

It is also intended to include silent polymorphisms and conservative substitutions in the polynucleotides and polypeptides disclosed herein, so long as such variants retain the same biological activity as the reference sequence (s) as disclosed herein.

## POLYPEPTIDES

It will be understood that polypeptides identified herein are not limited to polypeptides identified in Table 1 or those polypeptides having the amino acid sequence encoded by the nucleic acid sequences identified in Table 1 or fragments thereof but also include homologous sequences obtained from any source, for example related viral/bacterial proteins, cellular homologues and synthetic peptides, as well as variants or derivatives thereof.

Thus, reference herein to "polypeptides" also includes those sequences encoding homologues from other species including animals such as mammals (*e.g.*, mice, rats or rabbits), especially primates. Particularly preferred polypeptides include homologous human sequences.

The term also covers variants, homologues or derivatives of the amino acid sequences encoded by the nucleic acids identified in Table 1, as well as variants, homologues or derivatives of the nucleotide sequences coding for the amino acid sequences.

In the context of the present document, a homologous sequence is taken to include an amino acid sequence which is at least 15, 20, 25, 30, 40, 50, 60, 70, 80 or 90% identical, preferably at least 95 or 98% identical at the amino acid level over at least 50 or 100, preferably 200, 300, 400 or 500 amino acids with any one of the polypeptide sequences disclosed herein. In particular, homology should typically be considered with respect to those regions of the sequence known to be essential for protein function rather than non-essential neighbouring sequences. This is especially important when considering homologous sequences from distantly related organisms.

Although homology can also be considered in terms of functional similarity *(i.e.,* amino acid residues having similar chemical properties/functions), in the context of the present document it is preferred to express homology in terms of sequence identity.

Homology comparisons can be conducted by eye, or more usually, with the aid of readily available sequence comparison programs. These publicly and commercially available computer programs can calculate percent homology between two or more sequences.

Percent homology may be calculated over contiguous sequences, i.e., one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 *ibid* — Chapter 18), FASTA (Altschul et al., 1990, J. Mol. Biol. 215:403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

The terms "variant" or "derivative" in relation to amino acid sequences includes any substitution of, variation of, modification of, replacement of, deletion of or addition of one (or more) amino acids from or to the sequence providing the resultant amino acid sequence retains substantially the same activity as the unmodified sequence, preferably having at least the same activity as the polypeptides identified in Table 1 or the polypeptides encoded by the nucleic acid sequences identified in Table 1.

Polypeptides having the amino acid sequence encoded by the nucleic acid sequences identified in Table 1, or fragments or homologues thereof may be modified for use here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Alternatively, modifications may be made to deliberately inactivate one or more functional domains of the polypeptides described here. Amino acid substitutions may include the use of non-naturally occurring analogues, for example to increase blood plasma half-life of a therapeutically administered polypeptide.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Polypeptides for use in the methods and compositions described here also include fragments of the full length sequences mentioned above. Preferably said fragments comprise at least one epitope. Methods of identifying epitopes are well known in the art. Fragments will typically comprise at least 6 amino acids, more preferably at least 10, 20, 30, 50 or 100 amino acids.

Proteins are typically made by recombinant means, for example as described below. However they may also be made by synthetic means using techniques well known to skilled persons such as solid phase synthesis. Proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the function of the protein of interest sequence. Proteins may also be obtained by purification of cell extracts from animal cells.

Multimeric proteins comprising the cell cycle progression proteins are also intended to be encompassed. By "multimer" is meant a protein comprising two or more copies of a subunit protein. The subunit protein may be one of the proteins described here, *e.g.*, a cell cycle progression protein as disclosed herein repeated two or more times. Such a multimer may also be a fusion or chimeric protein, *e.g.*, a repeated cell cycle progression protein may be combined with polylinker sequence, and/or one or more cell cycle progression peptides, which may be present in a single copy, or may also be tandemly repeated, *e.g.,* a protein may comprise two or more multimers within the overall protein.

Proteins may be in a substantially isolated form. It will be understood that the protein may be mixed with carriers or diluents which will not interfere with the intended purpose of the protein and still be regarded as substantially isolated. Such a protein may also be in a substantially purified form, in which case it will generally comprise the protein in a preparation in which more than 90%, *e.g.*, 95%, 98% or 99% of the protein in the preparation is a protein as identified herein.

A polypeptide may be labeled with a revealing label. The revealing label may be any suitable label which allows the polypeptide to be detected. Suitable labels include radioisotopes, *e.g.,* $^{125}I$, enzymes, antibodies, polynucleotides and linkers such as biotin. Labeled polypeptides may be used in diagnostic procedures such as immunoassays to determine the amount of the relevant polypeptide in a sample. Polypeptides or labeled polypeptides may also be used in serological or cell-mediated immune assays for the detection of immune reactivity to said polypeptides in animals and humans using standard protocols.

A polypeptide or labeled polypeptide or fragment thereof may also be fixed to a solid phase, for example the surface of a microarray, an immunoassay well or dipstick. Such labeled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

Immunoassays may be used for detecting polypeptides for examples in detecting a modulation of protein expression or function.

Polypeptides identified herein may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by *in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (*e.g.*, myristolation, glycosylation, truncation, lapidation and tyrosine, serine or threonine phosphorylation) as may be needed to confer optimal biological activity on recombinant expression products. Such cell culture systems in which polypeptides described here are expressed may be used in assay systems to identify candidate substances which interfere with or enhance the functions of the polypeptides in the cell.

In highly preferred embodiments, the cell cycle progression polypeptides are selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392, 394, 396, 398, 400, 402, 404, 406 and 408 ("the even-numbered SEQ ID NOs in Figures 1 to 180").

POLYNUCLEOTIDES

We demonstrate here that knockdown of genes as disclosed in the Examples causes a cell cycle defect, and that accordingly these genes and the proteins encoded by them are responsible for cell cycle function.

Polynucleotides or nucleic acids described here include polynucleotides identified in Table 1 or any one or more of the nucleic acid sequences encoding the polypeptides which are encoded by the nucleic acids identified in Table 1 and fragments thereof. Fragments will typically comprise at least 15 consecutive nucleotides of the full-length polynucleotide, more preferably at least 20, 30, 50 or 100 consecutive nucleotides of the full-length polynucleotide. It is straightforward to identify a nucleic acid sequence which encodes such a polypeptide, by reference to the genetic code. Furthermore, computer programs are available which translate a nucleic acid sequence to a polypeptide sequence, and/or *vice versa.* The disclosure of a nucleic acid and its corresponding polypeptide sequence includes a disclosure of all nucleic acids (and their sequences) which encode that polypeptide sequence.

It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides to reflect the codon usage of any particular host organism in which the polypeptides are to be expressed.

In preferred embodiments, nucleic acids comprise those polynucleotides, such as cDNA, mRNA, and genomic DNA. Such polynucleotides may typically comprise *Drosophila* cDNA, mRNA, and genomic DNA, *Homo sapiens* cDNA, mRNA, and genomic DNA, etc. Accession numbers are provided in the Examples for the nucleic acid sequences, and the polypeptides they encode can be derived by use of such accession numbers in a relevant database, such as a *Drosophila* sequence database, a human sequence database, including a Human Genome Sequence database, GadFly, FlyBase, etc. in particular, the annotated *Drosophila* sequence database of the Berkeley *Drosophila* Genome Project (GadFly: Genome Annotation Database of *Drosophila* at the world wide web site "fruitfly.org", in the directory "/annot/") may be used to identify such *Drosophila* and human polynucleotide or polypeptide sequences. Relevant sequences may also be obtained by searching sequence databases such as BLAST with the polypeptide sequences. In particular, a search using TBLASTN may be employed.

Nucleic acids for use in the methods and compositions described here may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of this document, it is to be understood that the polynucleotides described herein may be modified by any method available in the art. Such modifications may be carried out in order to enhance the *in vivo* activity or life span of polynucleotides.

The terms "variant", "homologue" or "derivative" in relation to the nucleotide sequence for use in the methods and compositions described here include any substitution of, variation of, modification of, replacement of, deletion of or addition of one (or more) nucleic acid from or to the sequence. Preferably said variant, homologues or derivatives code for a polypeptide having biological activity.

As indicated above, with respect to sequence homology, preferably there is at least 50 or 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

We also describe the use of nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides.

The term "selectively hybridizable" means that a nucleic acid is found to hybridize to the nucleic acid having a sequence identified in Table 1 at a level significantly above background. Background implies a level of signal generated by interaction between the test nucleic acid and a non-specific DNA member in a sample which is less than 10 fold, preferably less than 100 fold as intense as the specific interaction observed with the target DNA. The intensity of interaction may be measured, for example, by radiolabelling the probe, *e.g.*, with $^{32}P$.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below. Conditions for stringency are also described in U.S. Pat. No. 5,976,838, the teachings of which are incorporated herein by reference in its entirety. In particular, examples of highly stringent, stringent, reduced and least stringent conditions are provided in U.S. Pat. No. 5,976,838, in the Table on page 15. Examples of stringency conditions for solutions during and after hybridization are shown, and highly stringent conditions are those that are at least as stringent as, for example, conditions A-F; stringent conditions are at least as stringent as, for example, conditions G-L; and reduced stringency conditions are at least as stringent as, for example, conditions M-R of that table.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

Stringency conditions for hybridization refers to conditions of temperature and buffer composition which permit hybridization of a first nucleic acid sequence to a second nucleic acid sequence, wherein the conditions determine the degree of identity between those sequences which hybridize to each other. Therefore, "high stringency conditions" are those conditions wherein only nucleic acid sequences which are very similar to each other will hybridize. The sequences may be less similar to each other if they hybridize under moderate stringency conditions. Still less similarity is needed for two sequences to hybridize under low stringency conditions. By varying the hybridization conditions from a stringency level at which no hybridization occurs, to a level at which hybridization is first observed, conditions can be determined at which a given sequence will hybridize to those sequences that are most similar to it. The precise conditions determining the stringency of a particular hybridization include not only the ionic strength, temperature, and the concentration of destabilizing agents such as formamide, but also on factors such as the length of the nucleic acid sequences, their base composition, the percent of mismatched base pairs between the two sequences, and the frequency of occurrence of subsets of the sequences (*e.g.*, small stretches of repeats) within other non-identical sequences. Washing is the step in which conditions are set so as to determine a minimum level of similarity between the sequences hybridizing with each other. Generally, from the lowest temperature at which only homologous hybridization occurs, a 1% mismatch between two sequences results in a 1°C decrease in the melting temperature ($T_m$) for any chosen SSC concentration. Generally, a doubling of the concentration of SSC results in an increase in the $T_m$ of about 17°C. Using these guidelines, the washing temperature can be determined empirically, depending on the level of mismatch sought. Hybridization and wash conditions are explained in Current Protocols in Molecular Biology (Ausubel, F.M. et al., eds., John Wiley & Sons, Inc., 1995, with supplemental updates) on pages 2.10.1 to 2.10.16, and 6.3.1 to 6.3.6.

High stringency conditions can employ hybridization at either (1) 1X SSC (10X SSC = 3 M NaCl, 0.3 M $Na_3$-citrate·$2H_2O$ (88 g/liter), pH to 7.0 with 1 M HCl), 1% SDS (sodium dodecyl sulfate), 0.1-2 mg/ml denatured salmon sperm DNA at 65°C, (2) 1X SSC, 50% formamide, 1% SDS, 0.1 - 2 mg/ml denatured salmon sperm DNA at 42°C, (3) 1% bovine serum albumen (fraction V), 1 mM $Na_2$·EDTA, 0.5 M $NaHPO_4$ (pH 7.2) (1 M $NaHPO_4$ = 134 g $Na_2HPO_4$·$7H_2O$, 4 ml 85% $H_3PO_4$ per liter), 7% SDS, 0.1 - 2 mg/ml denatured salmon sperm DNA at 65°C, (4) 50% formamide, 5X SSC, 0.02 M Tris-HCl (pH 7.6), 1X Denhardt's solution (100X = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5X SSC, 5X Denhardt's solution, 1% SDS, 100 :g/ml denatured salmon sperm DNA at 65°C, or (6) 5X SSC, 5X Denhardt's solution, 50% formamide, 1% SDS, 100 :g/ml denatured salmon sperm DNA at 42°C, with high stringency washes of either (1) 0.3 - 0.1X SSC, 0.1% SDS at 65°C, or (2) 1 mM $Na_2EDTA$, 40 mM $NaHPO_4$ (pH 7.2), 1% SDS at 65°C. The above conditions are intended to be used for DNA-DNA hybrids of 50 base pairs or longer. Where the hybrid is believed to be less than 18 base pairs in length, the hybridization and wash temperatures should be 5-10°C below that of the calculated $T_m$ of the hybrid, where $T_m$ in °C = (2 x the number of A and T bases) + (4 x the number of G and C bases). For hybrids believed to be about 18 to about 49 base pairs in length, the $T_m$ in °C = (81.5°C + 16.6($log_{10}$M) + 0.41(% G + C) - 0.61 (% formamide) - 500/L), where "M" is the molarity of monovalent cations (*e.g.*, $Na^+$), and "L" is the length of the hybrid in base pairs.

Moderate stringency conditions can employ hybridization at either (1) 4X SSC, (10X SSC = 3 M NaCl, 0.3 M $Na_3$-citrate·$2H_2O$ (88 g/liter), pH to 7.0 with 1 M HCl), 1% SDS (sodium dodecyl sulfate), 0.1 - 2 mg/ml denatured salmon sperm DNA at 65°C, (2) 4X SSC, 50% formamide, 1% SDS, 0.1 - 2 mg/ml denatured salmon sperm DNA at 42°C, (3) 1% bovine serum albumen (fraction V), 1 mM $Na_2$·EDTA, 0.5 M $NaHPO_4$ (pH 7.2) (1 M $NaHPO_4$ = 134 g $Na_2HPO_4$·$7H_2O$, 4 ml 85% $H_3PO_4$ per liter), 7% SDS, 0.1 - 2 mg/ml denatured salmon sperm DNA at 65°C, (4) 50% formamide, 5X SSC, 0.02 M Tris-HCl (pH 7.6), 1X Denhardt's solution (100X = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5X SSC, 5X Denhardt's solution, 1% SDS, 100 □g/ml denatured salmon sperm DNA at 65°C, or (6) 5X SSC, 5X Denhardt's solution, 50% formamide, 1% SDS, 100 :g/ml denatured salmon sperm DNA at 42°C, with moderate stringency washes of 1X SSC, 0.1 % SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated $T_m$ of the hybrid, where $T_m$ in °C = (2 x the number of A and T bases) + (4 x the number of G and C bases). For hybrids believed to be about 18 to about 49 base pairs in length, the $T_m$ in °C = (81.5°C + 16.6($log_{10}$M) + 0.41(% G + C) - 0.61 (% formamide) - 500/L), where "M" is the molarity of monovalent cations (*e.g.*, $Na^+$), and "L" is the length of the hybrid in base pairs.

Low stringency conditions can employ hybridization at either (1) 4X SSC, (10X SSC = 3 M NaCl, 0.3 M $Na_3$-citrate·$2H_2O$ (88 g/liter), pH to 7.0 with 1 M HCl), 1% SDS (sodium dodecyl sulfate), 0.1 - 2 mg/ml denatured salmon sperm DNA at 50°C, (2) 6X SSC, 50% formamide, 1% SDS, 0.1 - 2 mg/ml denatured salmon sperm DNA at 40°C, (3) 1 % bovine serum albumen (fraction V), 1 mM $Na_2$·EDTA, 0.5 M $NaHPO_4$ (pH 7.2) (1 M $NaHPO_4$ = 134 g $Na_2HPO_4$·$7H_2O$, 4 ml 85% $H_3PO_4$ per liter), 7% SDS, 0.1 - 2 mg/ml denatured salmon sperm DNA at 50°C, (4) 50% formamide, 5X SSC, 0.02 M Tris-HCl (pH 7.6), 1X Denhardt's solution (100X = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 40°C, (5) 5X SSC, 5X Denhardt's solution, 1% SDS, 100 :g/ml denatured salmon sperm DNA at 50°C, or (6) 5X SSC, 5X Denhardt's solution, 50% formamide, 1% SDS, 100 :g/ml denatured salmon sperm DNA at 40°C, with low stringency washes of either 2X SSC, 0.1% SDS at 50°C, or (2) 0.5% bovine serum albumin (fraction V), 1 mM $Na_2EDTA$, 40 mM $NaHPO_4$ (pH 7.2), 5% SDS. The above conditions are intended to be used for DNA-DNA hybrids of 50 base pairs or longer. Where the hybrid is believed to be less than 18 base pairs in length, the hybridization and wash temperatures should be 5 -10°C below that of the calculated $T_m$ of the hybrid, where $T_m$ in °C = (2 x the number of A and T bases) + (4 x the number of G and C bases). For hybrids believed to be about 18 to about 49 base pairs in length, the $T_m$ in °C = (81.5°C + 16.6($log_{10}$M) + 0.41(% G + C) - 0.61 (% formamide) - 500/L), where "M" is the molarity of monovalent cations (*e.g.*, $Na^+$), and "L" is the length of the hybrid in base pairs.

In a preferred aspect, we disclose the use of nucleotide sequences that can hybridise to the nucleotide sequences described here under stringent conditions (*e.g.*, 65°C and 0.1xSSC (1xSSC = 0.15 M NaCl, 0.015 M $Na_3$ Citrate pH 7.0)).

Where the polynucleotide is double-stranded, both strands of the duplex, the use of either individually or in combination, is encompassed by the present document. Where the polynucleotide is single-stranded, it is to be understood that the use of the complementary sequence of that polynucleotide is also included within the scope of the present document.

Polynucleotides which are not 100% homologous to the sequences in Table 1 but which may be used in the methods and compositions described here can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (*e.g.*, rat, mouse, bovine and primate cells), may be obtained and such homologues and fragments thereof in general will be capable of selectively hybridising to sequences identified in Table 1. Such sequences may be obtained by probing cDNA libraries made from or genomic DNA libraries from other animal species, and probing such libraries with probes comprising all or part of any on of the sequences under conditions of medium to high stringency. The nucleotide sequences of or which encode the human homologues identified in column 3 of Table 1, may preferably be used to identify other primate/mammalian homologues or allelic variants.

Variants and strain/species homologues may also be obtained using degenerate PCR which will use primers designed to target sequences within the variants and homologues of the sequences of Table 1. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labeled fragments.

In addition, homologous sequences may be identified by searching nucleotide and/or protein databases using search algorithms such as the BLAST suite of programs. This approach is described below and in the Examples.

Alternatively, such polynucleotides may be obtained by site directed mutagenesis of characterised sequences. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides. For example, further changes may be desirable to represent particular coding changes found in nucleic acid sequences which give rise to mutant genes which have lost their regulatory function. Probes based on such changes can be used as diagnostic probes to detect such mutants.

Polynucleotides may be used to produce a primer, *e.g.*, a PCR primer, a primer for an alternative amplification reaction, a probe, *e.g.*, labeled with a revealing label by conventional means using radioactive or non-radioactive labels, or the polynucleotides may be cloned into vectors. Such primers, probes and other fragments will be at least 8, 9, 10, or 15, preferably at least 20, for example at least 25, 30 or 40 nucleotides in length, and are also encompassed by the term polynucleotides as used herein.

Polynucleotides such as a DNA polynucleotides and probes for use in the methods and compositions described here may be produced recombinantly, synthetically, or by any means available to those of skill in the art. They may also be cloned by standard techniques. We also describe a composition comprising one or more isolated polynucleotides encoding a cell cycle progression protein, *e.g.*, a vector containing a polynucleotide encoding a cell cycle progression protein, and also host cells containing such a vector. By "host cell" is meant a cell which has been or can be used as the recipient of transferred nucleic acid by means of a vector. Host cells can prokaryotic or eukaryotic, mammalian, plant, or insect, and can exist as single cells, or as a collection, *e.g.*, as a culture, or in a tissue culture, or in a tissue or an organism. Host cells can also be derived from normal or diseased tissue from a multicellular organism, *e.g.*, a mammal. Host cell, as used herein, is intended to include not only the original cell which was transformed with a nucleic acid, but also descendants of such a cell, which still contain the nucleic acid. The exogenous polynucleotide may be maintained as a non-integrated vector, for example, a plasmid, or alternatively, may be integrated into the host genome. The vector can also contain regulatory sequences, *e.g.*, sequences permitting expression of the polynucleotide.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (*e.g.*, of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (*e.g.*, by purifying the reaction mixture on an agarose gel) and recovering the amplified DNA. The primers may be designed to contain suitable restriction enzyme recognition sites so that the amplified DNA can be cloned into a suitable cloning vector.

Polynucleotides or primers for use in the methods and compositions described here may carry a revealing label. Suitable labels include radioisotopes such as $^{32}P$ or $^{35}S$, enzyme labels, or other protein labels such as biotin. Such labels may be added to polynucleotides or primers and may be detected by using techniques well known by those in the art.

Polynucleotides or primers for use in the methods and compositions described here or fragments thereof labeled or unlabeled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer as described here under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example WO 89/03891 and WO 90/13667.

Tests for sequencing nucleotides for use in the methods and compositions described here include bringing a biological sample containing target DNA or RNA into contact with a probe comprising a polynucleotide or primer of under hybridising conditions and determining the sequence by, for example the Sanger dideoxy chain termination method (see Sambrook *et al.*).

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

Tests for detecting or sequencing nucleotides identified in Table 1 in a biological sample may be used to determine particular sequences within cells in individuals who have, or are suspected to have, an altered gene sequence, for example within cancer cells including leukaemia cells and solid tumours such as breast, ovary, lung, colon, pancreas, testes, liver, brain, muscle and bone tumours. Cells from patients suffering from a proliferative disease may also be tested in the same way.

In addition, the identification of the genes described in the Examples will allow the role of these genes in hereditary diseases to be investigated. In general, this will involve establishing the status of the gene (*e.g.*, using PCR sequence analysis), in cells derived from animals or humans with, for example, neoplasms.

The probes for use in methods and compositions described here may conveniently be packaged in the form of a test kit in a suitable container. In such kits the probe may be bound to a solid support where the assay format for which the kit is designed requires such binding. The kit may also contain suitable reagents for treating the sample to be probed, hybridising the probe to nucleic acid in the sample, control reagents, instructions, and the like.

In highly preferred embodiments, the cell cycle progression polypeptides are selected from the group consisting of SEQ ID Nos: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391, 393, 395, 397, 399, 401, 403, 405 and 407 ("the odd-numbered SEQ ID NOs in Figures 1 to 180").

## HOMOLOGY SEARCHING

Sequence homology (or identity) may be determined using any suitable homology algorithm, using for example default parameters.

Advantageously, the BLAST algorithm is employed, with parameters set to default values. The BLAST algorithm is described in detail at the world wide web site ("www") of the National Center for Biotechnology Information (".ncbi") of the National Institutes of Health ("nih") of the U.S. government (".gov"), in the "/Blast/" directory, in the "blast_help.html" file. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8 (see the "blast_help.html" file, as described above) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul *et al.* (1994).

The five BLAST programs available at the National Center for Biotechnology Information web site perform the following tasks:

"blastp" compares an amino acid query sequence against a protein sequence database;

"blastn" compares a nucleotide query sequence against a nucleotide sequence database;

"blastx" compares the six-frame conceptual translation products of a nucleotide query sequence (both strands) against a protein sequence database;

"tblastn" compares a protein query sequence against a nucleotide sequence database dynamically translated in all six reading frames (both strands).

"tblastx" compares the six-frame translations of a nucleotide query sequence against the six-frame translations of a nucleotide sequence database.

BLAST uses the following search parameters:

HISTOGRAM Display a histogram of scores for each search; default is yes. (See parameter H in the BLAST Manual).

DESCRIPTIONS Restricts the number of short descriptions of matching sequences reported to the number specified; default limit is 100 descriptions. (See parameter V in the manual page). See also EXPECT and CUTOFF.

ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

MATRIX Specify an alternate scoring matrix for BLASTP, BLASTX, TBLASTN and TBLASTX. The default matrix is BLOSUM62 (Henikoff & Henikoff, 1992, Proc. Natl. Aacad Sci. USA 89(22):10915-9). The valid alternative choices include: PAM40, PAM120, PAM250 and IDENTITY. No alternate scoring matrices are available for BLASTN; specifying the MATRIX directive in BLASTN requests returns an error response.

STRAND Restrict a TBLASTN search to just the top or bottom strand of the database sequences; or restrict a BLASTN, BLASTX or TBLASTX search to just reading frames on the top or bottom strand of the query sequence.

FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States, 1993, Computers and Chemistry 17:191-201, or, for BLASTN, by the DUST program of Tatusov and Lipman (see the world wide web site of the NCBI). Filtering can eliminate statistically significant but biologically uninteresting reports from the blast output (*e.g.*, hits against common acidic-, basic- or proline-rich regions), leaving the more biologically interesting regions of the query sequence available for specific matching against database sequences.

Low complexity sequence found by a filter program is substituted using the letter "N" in nucleotide sequence (*e.g.*, "N" repeated 13 times) and the letter "X" in protein sequences (*e.g.*, "X" repeated 9 times).

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at the NCBI world wide web site described above, in the "/BLAST" directory.

**ANTIBODIES**

We also describe the use of monocional or polyclonal antibodies to polypeptides encoded by the nucleic acids identified in Table 1 or fragments thereof.

Methods for production of antibodies are known by those skilled in the art. If polyclonal antibodies are desired, a selected mammal (*e.g.*, mouse, rabbit, goat, horse, etc.) is immunised with an immunogenic polypeptide bearing an epitope(s) from a polypeptide. Serum from the immunised animal is collected and treated according to known procedures. If serum containing polyclonal antibodies to an epitope from a polypeptide contains antibodies to other antigens, the polyclonal antibodies can be purified by immunoaffinity chromatography. Techniques for producing and processing polyclonal antisera are known in the art. In order to generate a larger immunogenic response, polypeptides or fragments thereof maybe haptenised to another polypeptide for use as immunogens in animals or humans.

Monoclonal antibodies directed against epitopes in polypeptides can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against epitopes in the polypeptides described here can be screened for various properties; *i.e.,* for isotype and epitope affinity.

An alternative technique involves screening phage display libraries where, for example the phage express scFv fragments on the surface of their coat with a large variety of complementarity determining regions (CDRs). This technique is well known in the art.

Antibodies, both monoclonal and polyclonal, which are directed against epitopes from polypeptides encoded by the nucleic acids identified in Table 1 are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the agent against which protection is desired.

Techniques for raising anti-idiotype antibodies are known in the art. These anti-idiotype antibodies may also be useful in therapy.

For the purposes of this document, the term "antibody", unless specified to the contrary, includes fragments of whole antibodies which retain their binding activity for a target antigen. Such fragments include Fv, F(ab') and $F(ab')_2$ fragments, as well as single chain antibodies (scFv). Furthermore, the antibodies and fragments thereof may be humanised antibodies, for example as described in EP-A-239400.

Antibodies may be used in detecting cell cycle progression polypeptides identified herein in biological samples by a method which comprises: (a) providing an antibody as described; (b) incubating a biological sample with said antibody under conditions which allow for the formation of an antibody-antigen complex; and (c) determining whether antibody-antigen complex comprising said antibody is formed.

Suitable samples include extracts tissues such as brain, breast, ovary, lung, colon, pancreas, testes, liver, muscle and bone tissues or from neoplastic growths derived from such tissues.

Antibodies that specifically bind to the cell cycle progression proteins can be used in diagnostic methods and kits that are well known to those of ordinary skill in the art to detect or quantify the cell cycle progression proteins in a body fluid or tissue. Results from these tests can be used to diagnose or predict the occurrence or recurrence of a cancer and other cell cycle progression-mediated diseases.

We also disclose the use of the cell cycle progression proteins, antibodies to those proteins, and compositions comprising those proteins and/or their antibodies in diagnosis or prognosis of diseases characterized by proliferative activity. As used herein, the term "prognostic method" means a method that enables a prediction regarding the progression of a disease of a human or animal diagnosed with the disease, in particular, a cell cycle progression-dependent disease. The term "diagnostic method" as used herein means a method that enables a determination of the presence or type of cell cycle progression-dependent disease in or on a human or animal.

The cell cycle progression proteins can be used in a diagnostic method and kit to detect and quantify antibodies capable of binding the proteins. These kits would permit detection of circulating antibodies to the cell cycle progression proteins which indicates, *e.g.*, the proliferation of cancer cells. Patients that have such circulating anti-protein antibodies may be more likely to develop multiple tumors and cancers, and may be more likely to have recurrences of cancer after treatments or periods of remission.

Antibodies for use in the methods and compositions described here may be bound to a solid support and/or packaged into kits in a suitable container along with suitable reagents, controls, instructions and the like.

When used in a diagnostic composition, an antibody is preferably provided together with means for detecting the antibody, which can be enzymatic, fluorescent, radioisotopic or other means. The antibody and the detection means can be provided for simultaneous, simultaneous separate or sequential use, in a diagnostic kit intended for diagnosis.

## MEASURING EXPRESSION OF CELL CYCLE PROGRESSION GENES

Levels of gene expression may be determined using a number of different techniques.

*(a) at the RNA level*

Gene expression can be detected at the RNA level. RNA may be extracted from cells using RNA extraction techniques including, for example, using acid phenol/guanidine isothiocyanate extraction (RNAzol B; Biogenesis), or RNeasy RNA preparation kits (Qiagen).Typical assay formats utilising ribonucleic acid hybridisation include nuclear run-on assays, RT-PCR and RNase protection assays (Melton *et al., Nuc. Acids Res.* 12:7035. Methods for detection which can be employed include radioactive labels, enzyme labels, chemiluminescent labels, fluorescent labels and other suitable labels.

Typically, RT-PCR is used to amplify RNA targets. In this process, the reverse transcriptase enzyme is used to convert RNA to complementary DNA (cDNA) which can then be amplified to facilitate detection.

Many DNA amplification methods are known, most of which rely on an enzymatic chain reaction (such as a polymerase chain reaction, a ligase chain reaction, or a self-sustained sequence replication) or from the replication of all or part of the vector into which it has been cloned.

Many target and signal amplification methods have been described in the literature, for example, general reviews of these methods in Landegren, U. et al., Science 242:229-237 (1988) and Lewis, R., Genetic Engineering News 10:1, 54-55 (1990).

PCR is a nucleic acid amplification method described inter alia in U.S. Pat. Nos. 4,683,195 and 4,683,202. PCR can be used to amplify any known nucleic acid in a diagnostic context (Mok et al., 1994, Gynaecologic Oncology 52:247-252). Self-sustained sequence replication (3SR) is a variation of TAS, which involves the isothermal amplification of a nucleic acid template via sequential rounds of reverse transcriptase (RT), polymerase and nuclease activities that are mediated by an enzyme cocktail and appropriate oligonucleotide primers (Guatelli *et al.,* 1990, *Proc. Natl. Acad. Sci. USA* 87:1874). Ligation amplification reaction or ligation amplification system uses DNA ligase and four oligonucleotides, two per target strand. This technique is described by Wu, D. Y. and Wallace, R. B., 1989, *Genomics* 4:560. In the Qβ Replicase technique, RNA replicase for the bacteriophage Qβ, which replicates single-stranded RNA, is used to amplify the target DNA, as described by Lizardi *et al.,* 1988, *Bio/Technology* 6:1197.

Alternative amplification technology can also be exploited. For example, rolling circle amplification (Lizardi et al., 1998, Nat Genet 19:225) is an amplification technology available commercially (RCAT™) which is driven by DNA polymerase and can replicate circular oligonucleotide probes with either linear or geometric kinetics under isothermal conditions. A further technique, strand displacement amplification (SDA; Walker et al., 1992, Proc. Natl. Acad. Sci. USA 80:392) begins with a specifically defined sequence unique to a specific target.

*(b) at the polypeptide level*

Gene expression may also be detected by measuring the cell cycle progression polypeptides. This may be achieved by using molecules which bind to the cell cycle progression polypeptides. Suitable molecules/agents which bind either directly or indirectly to the cell cycle progression polypeptides in order to detect the presence of the protein include naturally occurring molecules such as peptides and proteins, for example antibodies, or they may be synthetic molecules.

Standard laboratory techniques such as immunoblotting as described above can be used to detect altered levels of cell cycle progression proteins, as compared with untreated cells in the same cell population.

Gene expression may also be determined by detecting changes in post-translational processing of polypeptides or post-transcriptional modification of nucleic acids. For example, differential phosphorylation of polypeptides, the cleavage of polypeptides or alternative splicing of RNA, and the like may be measured. Levels of expression of gene products such as polypeptides, as well as their post-translational modification, may be detected using proprietary protein assays or techniques such as 2D polyacrylamide gel electrophoresis.

Antibodies can be assayed for immunospecific binding by any method known in the art. The immunoassays which can be used include but are not limited to competitive and non-competitive assay systems using techniques such as western blots, radioimmunoassays, ELISA, sandwich immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays. Such assays are routine in the art (see, for example, Ausubel et al., eds, 1994, Current Protocols in Molecular Biology, Vol. 1, John Wiley & Sons, Inc., New York, which is incorporated by reference herein in its entirety).

## ARRAYS

Array technology and the various techniques and applications associated with it is described generally in numerous textbooks and documents. These include Lemieux et al., 1998, Molecular Breeding 4:277-289; Schena and Davis. Parallel Analysis with Biological Chips. in PCR Methods Manual (eds. M. Innis, D. Gelfand, J. Sninsky); Schena and Davis, 1999, Genes, Genomes and Chips. In DNA Microarrays: A Practical Approach (ed. M. Schena), Oxford University Press, Oxford, UK, 1999); *The Chipping Forecast (Nature Genetics* special issue; January 1999 Supplement); Mark Schena (Ed.), Microarray Biochip Technology, (Eaton Publishing Company); Cortes, 2000, The Scientist 14(17): 25; Gwynne and Page, Microarray analysis: the next revolution in molecular biology, Science, 1999, August 6; Eakins and Chu, 1999, Trends in Biotechnology, 17:217-218, and also at various world wide web sites.

Array technology overcomes the disadvantages with traditional methods in molecular biology, which generally work on a "one gene in one experiment" basis, resulting in low throughput and the inability to appreciate the "whole picture" of gene function. Currently, the major applications for array technology include the identification of sequence (gene / gene mutation) and the determination of expression level (abundance) of genes. Gene expression profiling may make use of array technology, optionally in combination with proteomics techniques (Celis et al., 2000, FEBS Lett, 480 (1):2-16; Lockhart and Winzeler, 2000, Nature 405(6788):827-836; Khan et al., 1999, 20(2):223-9). Other applications of array technology are also known in the art; for example, gene discovery, cancer research (Marx, 2000, Science 289: 1670-1672; Scherf et alet al., 2000, Nat Genet 24(3):236-44; Ross et al., 2000, Nat Genet 2000, 24(3):227-35), SNP analysis (Wang et al., 1998, Science 280(5366):1077-82), drug discovery, pharmacogenomics, disease diagnosis (for example, utilising microfluidics devices: Chemical & Engineering News, February 22, 1999, 77(8):27-36), toxicology (Rockett and Dix (2000), Xenobiotica 30(2):155-77; Afshari et al., 1999, Cancer Res 59(19):4759-60) and toxicogenomics (a hybrid of functional genomics and molecular toxicology). The goal of toxicogenomics is to find correlations between toxic responses to toxicants and changes in the genetic profiles of the objects exposed to such toxicants (Nuwaysir et al., 1999, Molecular Carcinogenesis 24:153-159).

In the context of the present document, array technology can be used, for example, in the analysis of the expression of one or more of the cell cycle progression proteins identified herein. In one embodiment, array technology may be used to assay the effect of a candidate compound on a number of the cell cycle progression proteins identified herein simultaneously. Accordingly, we further provide microarrays that include at least one, at least two or at least several of the nucleic acids identified in Table 1, or fragments thereof, or protein or antibody arrays.

In general, any library or group of samples may be arranged in an orderly manner into an array, by spatially separating the members of the library or group. Examples of suitable libraries for arraying include nucleic acid libraries (including DNA, cDNA, oligonucleotide, etc. libraries), peptide, polypeptide and protein libraries, as well as libraries comprising any molecules, such as ligand libraries, among others. Accordingly, where reference is made to a "library" in this document, unless the context dictates otherwise, such reference should be taken to include reference to a library in the form of an array. In the context of the present document, a "library" may include a sample of cell cycle progression proteins as identified herein.

The samples (*e.g.*, members of a library) are generally fixed or immobilised onto a solid phase, preferably a solid substrate, to limit diffusion and admixing of the samples. In a preferred embodiment, libraries of DNA binding ligands may be prepared. In particular, the libraries may be immobilised to a substantially planar solid phase, including membranes and non-porous substrates such as plastic and glass. Furthermore, the samples are preferably arranged in such a way that indexing (*i.e.,* reference or access to a particular sample) is facilitated. Typically the samples are applied as spots in a grid formation. Common assay systems may be adapted for this purpose. For example, an array may be immobilised on the surface of a microplate, either with multiple samples in a well, or with a single sample in each well. Furthermore, the solid substrate may be a membrane, such as a nitrocellulose or nylon membrane (for example, membranes used in blotting experiments). Alternative substrates include glass, or silica based substrates. Thus, the samples are immobilised by any suitable method known in the art, for example, by charge interactions, or by chemical coupling to the walls or bottom of the wells, or the surface of the membrane. Other means of arranging and fixing may be used, for example, pipetting, drop-touch, piezoelectric means, ink-jet and bubblejet technology, electrostatic application, etc. In the case of silicon-based chips, photolithography may be utilised to arrange and fix the samples on the chip.

The samples may be arranged by being "spotted" onto the solid substrate; this may be done by hand or by making use of robotics to deposit the sample. In general, arrays may be described as macroarrays or microarrays, the difference being the size of the sample spots. Macroarrays typically contain sample spot sizes of about 300 microns or larger and may be easily imaged by existing gel and blot scanners. The sample spot sizes in microarrays are typically less than 200 microns in diameter and these arrays usually contain thousands of spots. Thus, microarrays may require specialized robotics and imaging equipment, which may need to be custom made. Instrumentation is described generally in a review by Cortese, 2000, The Scientist 14(11):26.

Techniques for producing immobilised libraries of DNA molecules have been described in the art. Generally, most prior art methods described how to synthesise single-stranded nucleic acid molecule libraries, using for example masking techniques to build up various permutations of sequences at the various discrete positions on the solid substrate. U.S. Patent No. 5,837,832, the contents of which are incorporated herein by reference, describes an improved method for producing DNA arrays immobilised to silicon substrates based on very large scale integration technology. In particular, U.S. Patent No. 5,837,832 describes a strategy called "tiling" to synthesize specific sets of probes at spatially-defined locations on a substrate which may be used to produced the immobilised DNA libraries. U.S. Patent No. 5,837,832 also provides references for earlier techniques that may also be used.

Arrays of peptides (or peptidomimetics) may also be synthesised on a surface in a manner that places each distinct library member (*e.g.*, unique peptide sequence) at a discrete, predefined location in the array. The identity of each library member is determined by its spatial location in the array. The locations in the array where binding interactions between a predetermined molecule (*e.g.*, a target or probe) and reactive library members occur is determined, thereby identifying the sequences of the reactive library members on the basis of spatial location. These methods are described in U.S. Patent No. 5,143,854; WO 90/15070 and WO 92/10092; Fodor et al., 1991, Science 251:767; Dower and Fodor, 1991, Ann. Rep. Med. Chem. 26:271.

To aid detection, targets and probes may be labelled with any readily detectable reporter, for example, a fluorescent, bioluminescent, phosphorescent, radioactive, etc reporter. Such reporters, their detection, coupling to targets/probes, etc are discussed elsewhere in this document. Labelling of probes and targets is also disclosed in Shalon et al., 1996, Genome Res 6(7):639-45.

Specific examples of DNA arrays include the following:

> Format I: probe cDNA (~500 - ~5,000 bases long) is immobilized to a solid surface such as glass using robot spotting and exposed to a set of targets either separately or in a mixture. This method is widely considered as having been developed at Stanford University (Ekins and Chu, 1999, Trends in Biotechnology, 17:217-218).
>
> Format II: an array of oligonucleotide (~20 - ~25-mer oligos) or peptide nucleic acid (PNA) probes is synthesized either in situ (on-chip) or by conventional synthesis followed by on-chip immobilization. The array is exposed to labeled sample DNA, hybridized, and the identity/abundance of complementary sequences are determined. Such a DNA chip is sold by Affymetrix, Inc., under the GeneChip® trademark.

Examples of some commercially available microarray formats are set out, for example, in Marshall and Hodgson, 1998, Nature Biotechnology 16(1):27-31.

Data analysis is also an important part of an experiment involving arrays. The raw data from a microarray experiment typically are images, which need to be transformed into gene expression matrices - tables where rows represent for example genes, columns represent for example various samples such as tissues or experimental conditions, and numbers in each cell for example characterize the expression level of the particular gene in the particular sample. These matrices have to be analyzed further, if any knowledge about the underlying biological processes is to be extracted. Methods of data analysis (including supervised and unsupervised data analysis as well as bioinformatics approaches) are disclosed in Brazma and Vilo J, 2000, FEBS Lett 480(1):17-24.

As disclosed above, proteins, polypeptides, etc may also be immobilised in arrays. For example, antibodies have been used in microarray analysis of the proteome using protein chips (Borrebaeck CA, 2000, Immunol Today 21 (8):379-82). Polypeptide arrays are reviewed in, for example, MacBeath and Schreiber, 2000, Science, 289(5485): 1760-1763.

## MODIFYING THE FUNCTIONAL ACTIVITY OF A CELL CYCLE PROGRESSION PROTEIN

The functional activity of a cell cycle progression protein may be modified by suitable molecules/agents which bind either directly or indirectly to a cell cycle progression protein, or to the nucleic acid encoding it. Agents may be naturally occurring molecules such as peptides and proteins, for example antibodies, or they may be synthetic molecules. Methods of modulating the level of expression of a cell cycle progression protein include, for example, using antisense techniques.

Antisense constructs, i.e., nucleic acid, preferably RNA, constructs complementary to the sense nucleic acid or mRNA, are described in detail in US 6,100,090 (Monia *et al.*), and Neckers et al., 1992, Crit Rev Oncog 3(1-2): 175-231, the teachings of which document are specifically incorporated by reference. Other methods of modulating gene expression are known to those skilled in the art and include dominant negative approaches as well as introducing peptides or small molecules which inhibit gene expression or functional activity.

RNA interference (RNAi) is a method of post transcriptional gene silencing (PTGS) induced by the direct introduction of double-stranded RNA (dsRNA) and has emerged as a useful tool to knock out expression of specific genes in a variety of organisms. RNAi is described by Fire et al., Nature 391:806-811 (1998). Other methods of PTGS are known and include, for example, introduction of a transgene or virus. Generally, in PTGS, the transcript of the silenced gene is synthesised but does not accumulate because it is rapidly degraded. Methods for PTGS, including RNAi are described, for example, in the Ambion.com world wide web site, in the directory "/hottopics/", in the "rnai" file.

Suitable methods for RNAi *in vitro* are described herein. One such method involves the introduction of siRNA (small interfering RNA). Current models indicate that these 21-23 nucleotide dsRNAs can induce PTGS. Methods for designing effective siRNAs are described, for example, in the Ambion web site described above. RNA precursers can also be encoded by all or a part of one of the cell cycle progression nucleic acid sequences described herein.

## ASSAYS

We also describe assays that are suitable for identifying substances which bind to polypeptides described here and which affect, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, cytokinesis functions, chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

In general, a substance which inhibits one or more of these aspects of cell cycle progression either inhibits it completely, or leads to a significant (*i.e.*, greater than 50%) reduction in protein activity at concentrations of 500 mM or less, relative to controls. Preferably, the inhibition is by 75% relative to controls, more preferably by 90%, and most preferably by 95% or 100% relative to controls. A substance which enhances or increases one or more of these aspects of cell cycle progression leads to a significant *(i.e.,* greater than 50%) increase in protein activity at concentrations of 500mM or less, relative to controls. Preferably, the increase is by 75% relative to controls, more preferably by 90%, and most preferably by 95% or 100% relative to controls.

In addition, assays can be used to identify substances that interfere with binding of polypeptides as described herein, where appropriate, to components of cell division cycle machinery. Such assays are typically *in vitro.* Assays are also provided that test the effects of candidate substances identified in preliminary *in vitro* assays on intact cells in whole cell assays. The assays described below, or any suitable assay as known in the art, may be used to identify these substances.

We therefore describe one or more substances identified by any of the assays described below, viz, mitosis assays, meiotic assays, polypeptide binding assays, microtubule binding/polymerisation assays, microtubule purification and binding assays, microtubule organising centre (MTOC) nucleation activity assays, motor protein assay, assay for spindle assembly and function, assays for DNA replication, chromosome condensation assays, kinase assays, kinase inhibitor assays, and whole cell assays, each as described in further detail below.

### *Modulator Screening Assays*

Compounds having inhibitory, activating, or modulating activity can be identified using *in vitro* and *in vivo* assays for cell cycle progression protein activity and/or expression, *e.g.*, ligands, agonists, antagonists, and their homologs and mimetics. Modulator screening may be performed by adding a putative modulator test compound to a tissue or cell sample, and monitoring the effect of the test compound on the function and/or expression of a cell cycle progression protein. A parallel sample which does not receive the test compound is also monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds.

Methods for inducing cell cycle progression are well known in the art and include, without limitation, exposure to growth factors. Differences between treated and untreated cells indicates effects attributable to the test compound.

A substance that inhibits cell cycle progression as a result of an interaction with a polypeptide described here may do so in several ways. For example, if the substance inhibits cell division, mitosis and/or meiosis, it may directly disrupt the binding of a polypeptide to a component of the spindle apparatus by, for example, binding to the polypeptide and masking or altering the site of interaction with the other component. A substance which inhibits DNA replication may do so by inhibiting the phosphorylation or de-phosphorylation of proteins involved in replication. For example, it is known that the kinase inhibitor 6-DMAP (6-dimethylaminopurine) prevents the initiation of replication (Blow, J.J., 1993, J Cell Biol 122:993-1002). Candidate substances of this type may conveniently be preliminarily screened by *in vitro* binding assays as, for example, described below and then tested, for example in a whole cell assay as described below. Examples of candidate substances include antibodies which recognise a polypeptide as described here.

A substance which can bind directly to a polypeptide as described may also inhibit its function in cell cycle progression by altering its subcellular localisation and hence its ability to interact with its normal substrate. The substance may alter the subcellular localisation of the polypeptide by directly binding to it, or by indirectly disrupting the interaction of the polypeptide with another component. For example, it is known that interaction between the p68 and p180 subunits of DNA polymerase alpha-primase enzyme is necessary in order for p180 to translocate into the nucleus (Mizuno et al., 1998, Mol Cell Biol 18:3552-62), and accordingly, a substance which disrupts the interaction between p68 and p180 will affect nuclear translocation and hence activity of the primase. A substance which affects mitosis may do so by preventing the polypeptide and components of the mitotic apparatus from coming into contact within the cell.

These substances may be tested using, for example the whole cells assays described below. Non-functional homologues of relevant polypeptides may also be tested for inhibition of cell cycle progression since they may compete with the wild type protein for binding to components of the cell division cycle machinery whilst being incapable of the normal functions of the protein or block the function of the protein bound to the cell division cycle machinery. Such non-functional homologues may include naturally occurring mutants and modified sequences or fragments thereof.

Alternatively, instead of preventing the association of the components directly, the substance may suppress the biologically available amount of a polypeptide. This may be by inhibiting expression of the component, for example at the level of transcription, transcript stability, translation or post-translational stability. An example of such a substance would be antisense RNA or double-stranded interfering RNA sequences which suppresses the amount of mRNA biosynthesis.

Suitable candidate substances include peptides, especially of from about 5 to 30 or 10 to 25 amino acids in size, based on the sequence of the polypeptides described in the Examples, or variants of such peptides in which one or more residues have been substituted. Peptides from panels of peptides comprising random sequences or sequences which have been varied consistently to provide a maximally diverse panel of peptides may be used.

Suitable candidate substances also include antibody products (for example, monoclonal and polyclonal antibodies, single chain antibodies, chimeric antibodies and CDR-grafted antibodies) which are specific for a polypeptide as described here. Furthermore, combinatorial libraries, peptide and peptide mimetics, defined chemical entities, oligonucleotides, and natural product libraries may be screened for activity as inhibitors of binding of a polypeptide to the cell division cycle machinery, for example mitotic/meiotic apparatus (such as microtubules). The candidate substances may be used in an initial screen in batches of, for example 10 substances per reaction, and the substances of those batches which show inhibition tested individually. Candidate substances which show activity in *in vitro* screens such as those described below can then be tested in whole cell systems, such as mammalian cells which will be exposed to the inhibitor and tested for inhibition of any of the stages of the cell cycle.

A substance is identified as a modulator of cell cycle progression activity when it is found to inhibit, decrease, increase, enhance, or activate such activity. In general, a substance which inhibits one or more of these aspects of cell cycle progression either inhibits it completely, or leads to a significant (*i.e.*, greater than 50%) reduction in protein activity at concentrations of 500 mM or less, relative to controls (*i.e.*, substance known to not modulate one or more aspects of cell cycle progression). Preferably, the inhibition is by 75% relative to controls, more preferably by 90%, and most preferably by 95% or 100% relative to controls. The inhibition may prevent cell cycle progression, or may simply delay or prolong cell cycle progression. A substance which enhances or increases one or more of these aspects of cell cycle progression leads to a significant (*i.e.*, greater than 50%) increase in protein activity at concentrations of 500 mM or less, relative to controls. Preferably, the increase is by 75% relative to controls, more preferably by 90%, and most preferably by 95% or 100% relative to controls.

*Polypeptide Binding Assays*

One type of assay for identifying substances that bind to a polypeptide described here involves contacting the polypeptide, which is immobilised on a solid support, with a non-immobilised candidate substance determining whether and/or to what extent the polypeptide of interest and candidate substance bind to each other. Alternatively, the candidate substance may be immobilised and the polypeptide as set out in this document non-immobilised.

The binding of the substance to the cell cycle progression polypeptide can be transient, reversible or permanent. Preferably the substance binds to the polypeptide with a Kd value which is lower than the Kd value for binding to control polypeptides (*i.e.,* polypeptides known to not be cell cycle progression polypeptides). Preferably the Kd value of the substance is 2 fold less than the Kd value for binding to control polypeptides, more preferably with a Kd value 100 fold less, and most preferably with a Kd 1000 fold less than that for binding to the control polypeptide.

In a preferred assay method, the polypeptide is immobilised on beads such as agarose beads. Typically this is achieved by expressing the component as a GST-fusion protein in bacteria, yeast or higher eukaryotic cell lines and purifying the GST-fusion protein from crude cell extracts using glutathione-agarose beads (Smith and Johnson, 1988; Gene 67(10):31-40). As a control, binding of the candidate substance, which is not a GST-fusion protein, to the immobilised polypeptide is determined in the absence of the polypeptide. The binding of the candidate substance to the immobilised polypeptide is then determined. This type of assay is known in the art as a GST pulldown assay. Again, the candidate substance may be immobilised and the polypeptide non-immobilised.

It is also possible to perform this type of assay using different affinity purification systems for immobilising one of the components, for example Ni-NTA agarose and histidine-tagged components.

Binding of the polypeptide to the candidate substance may be determined by a variety of methods well-known in the art. For example, the non-immobilised component may be labeled (with for example, a radioactive label, an epitope tag or an enzyme-antibody conjugate). Alternatively, binding may be determined by immunological detection techniques. For example, the reaction mixture can be Western blotted and the blot probed with an antibody that detects the non-immobilised component. ELISA techniques may also be used.

Candidate substances are typically added to a final concentration of from 1 to 1000 nmol/ml, more preferably from 1 to 100 nmol/ml. In the case of antibodies, the final concentration used is typically from 100 to 500 μg/ml, more preferably from 200 to 300 μg/ml.

*Microtubule Binding/Polymerisation Assays*

In the case of cell cycle progression polypeptides that bind to microtubules, another type of *in vitro* assay involves determining whether a candidate substance modulates binding of the polypeptide to microtubules. Such an assay typically comprises contacting a polypeptide with microtubules in the presence or absence of the candidate substance and determining if the candidate substance has an affect on the binding of the polypeptide to the microtubules. This assay can also be used in the absence of candidate substances to confirm that a polypeptide does indeed bind to microtubules.

The binding of the substance to the cell cycle progression polypeptide can be transient, reversible or permanent. Preferably the substance binds to the polypeptide with a Kd value which is lower than the Kd value for binding to control polypeptides (*i.e.*, polypeptides known to not be cell cycle progression polypeptides). Preferably the Kd value of the substance is 2 fold less than the Kd value for binding to control polypeptides, more preferably with a Kd value 100 fold less, and most preferably with a Kd 1000 fold less than that for binding to the control polypeptide.

Microtubules may be prepared and assays conducted as follows.

*Microtubule Purification and Binding Assays*

Microtubules are purified from 0-3h-old *Drosophila* embryos essentially as described previously (Saunders et al., 1997, J. Cell Biol. 137(4):881-90). About 3 ml of embryos are homogenized with a Dounce homogenizer in 2 volumes of ice-cold lysis buffer (0.1 M Pipes/NaOH, pH6.6, 5 mM EGTA, 1 mM $MgSO_4$, 0.9 M glycerol, 1 mM DTT, 1 mM PMSF, 1 μg/ml aprotinin, 1 μg/ml leupeptin and 1 μg/ml pepstatin). The microtubules are depolymerized by incubation on ice for 15 min, and the extract is then centrifuged at 16,000 g for 30 minutes at 4°C. The supernatant is recentrifuged at 135,000 g for 90 minutes at 4°C. Microtubules in this later supernatant are polymerized by addition of GTP to 1 mM and taxol to 20 μM and incubation at room temperature for 30 minutes A 3 ml aliquot of the extract is layered on top of 3 ml 15% sucrose cushion prepared in lysis buffer. After centrifuging at 54,000g for 30 minutes at 20°C using a swing out rotor, the microtubule pellet is resuspended in lysis buffer.

Microtubule overlay assays are performed as previously described (Saunders *et al.,* 1997). 500 ng per lane of recombinant Asp, recombinant polypeptide, and bovine serum albumin (BSA, Sigma) are fractionated by 10% SDS-PAGE and blotted onto PVDF membranes (Millipore). The membranes are preincubated in TBST (50mM Tris pH 7.5, 150 mM NaCl, 0.05% Tween 20) containing 5% low fat powdered milk (LFPM) for 1 h and then washed 3 times for 15 minutes in lysis buffer. The filters are then incubated for 30 minutes in lysis buffer containing either 1 mM GDP, 1 mM GTP, or 1 mM GTP-$\gamma$-S. MAP-free bovine brain tubulin (Molecular Probes) is polymerised at a concentration of 2 $\mu$g/ml in lysis buffer by addition of GTP to a final concentration of 1 mM and incubated at 37°C for 30 minutes. The nucleotide solutions are removed and the buffer containing polymerised microtubules added to the membanes for incubation for 1h at 37°C with addition of taxol at a final concentration of 10 $\mu$M for the final 30 minutes. The blots are then washed 3 times with TBST and the bound tubulin detected using standard Western blot procedures using anti-$\beta$-tubulin antibodies (Boehringer Mannheim) at 2.5 $\mu$g/ml and the Super Signal detection system (Pierce).

It may be desirable in one embodiment of this type of assay to deplete the polypeptide of interest from cell extracts used to produce polymerise microtubules. This may, for example, be achieved by the use of suitable antibodies.

A simple extension to this type of assay would be to test the effects of a purified polypeptide upon the ability of tubulin to polymerise *in vitro* (for example, as used by Andersen and Karsenti, 1997, J. Cell. Biol. 139(4):975-83) in the presence or absence of a candidate substance (typically added at the concentrations described above). *Xenopus* cell-free extracts may conveniently be used, for example as a source of tubulin.

*Microtubule Organising Centre (MTOC) Nucleation Activity Assays*

Candidate substances, for example those identified using the binding assays described above, may be screening using a microtubule organising centre nucleation activity assay to determine if they are capable of disrupting MTOCs as measured by, for example, aster formation. This assay in its simplest form comprises adding the candidate substance to a cellular extract which in the absence of the candidate substance has microtubule organising centre nucleation activity resulting in formation of asters.

A substance is identified as inhibiting cell cycle progression activity when it is found to inhibit or decrease such activity. In general, a substance which inhibits one or more of these aspects of cell cycle progression either inhibits it completely, or leads to a significant (i. e., greater than 50%) reduction in protein activity at concentrations of 500 mM or less, relative to controls *(i.e.,* substance known to not modulate one or more aspects of cell cycle progression). Preferably, the inhibition is by 75% relative to controls, more preferably by 90%, and most preferably by 95% or 100% relative to controls. The inhibition may prevent cell cycle progression, or may simply delay or prolong cell cycle progression.

In a preferred embodiment, the assay system comprises (i) a polypeptide of interest and (ii) components required for microtubule organising centre nucleation activity except for functional polypeptide of intereset, which is typically removed by immunodepletion (or by the use of extracts from mutant cells). The components themselves are typically in two parts such that microtubule nucleation does not occur until the two parts are mixed. The polypeptide of interest may be present in one of the two parts initially or added subsequently prior to mixing of the two parts.

Subsequently, the polypeptide of interest and candidate substance are added to the component mix and microtubule nucleation from centrosomes measured, for example by immunostaining for the polypeptide of interest and visualising aster formation by immunofluorescence microscopy. The polypeptide of interest may be preincubated with the candidate substance before addition to the component mix. Alternatively, both the polypeptide of interest and the candidate substance may be added directly to the component mix, simultaneously or sequentially in either order.

The components required for microtubule organising centre formation typically include salt-stripped centrosomes prepared as described in Moritz et al., 1998, J. Cell Biol. 142(3):775-86). Stripping centrosome preparations with 2M KI removes the centrosome proteins CP60, CP 190, CNN and $\gamma$-tubulin. Of these, neither CP60 nor CP 190 appear to be required for microtubule nucleation. The other minimal components are typically provided as a depleted cellular extract, or conveniently, as a cellular extract from cells with a non-functional variant of a polypeptide of interest. Typically, labeled tubulin (usually $\beta$-tubulin) is also added to assist in visualising aster formation.

Alternatively, partially purified centrosomes that have not been salt-stripped may be used as part of the components. In this case, only tubulin, preferably labeled tubulin is required to complete the component mix.

Candidate substances are typically added to a final concentration of from 1 to 1000 nmol/ml, more preferably from 1 to 100 nmol/ml. In the case of antibodies, the final concentration used is typically from 100 to 500 $\mu$g/ml, more preferably from 200 to 300 $\mu$g/ml.

The degree of inhibition of aster formation by the candidate substance may be determined by measuring the number of normal asters per unit area for control untreated cell preparation and measuring the number of normal asters per unit area for cells treated with the candidate substance and comparing the result. Typically, a candidate substance is considered to be capable of disrupting MTOC integrity if the treated cell preparations have less than 50%, preferably less than 40, 30, 20 or 10% of the number of asters found in untreated cells preparations. It may also be desirable to stain cells for $\gamma$-tubulin to determine the maximum number of possible MTOCs present to allow normalisation between samples.

*Motor Protein Assay*

Polypeptides of interest may interact with motor proteins such as the Eg5-like motor protein *in vitro.* The effects of candidate substances on such a process may be determined using assays wherein the motor protein is immobilised on coverslips. Rhodamine labeled microtubules are then added and their translocation can be followed by fluorescent microscopy. The effect of candidate substances may thus be determined by comparing the extent and/or rate of translocation in the presence and absence of the candidate substance. Generally, candidate substances known to bind to a polypeptide of interest, would be tested in this assay. Alternatively, a high throughput assay may be used to identify modulators of motor proteins and the resulting identified substances tested for affects on a polypeptide of interest as described above.

Typically this assay uses microtubules stabilised by taxol (*e.g.*, Howard and Hyman 1993, Chandra and Endow, 1993 - both chapters in "Motility Assays for Motor Proteins" Ed. Jon Scholey, pub. Academic Press). If however, a polypeptide of interest were to promote stable polymerisation of microtubules (see above) then these microtubules could be used directly in motility assays.

Simple protein-protein binding assays as described above, using a motor protein and a polypeptide of interest may also be used to confirm that the polypeptide binds to the motor protein, typically prior to testing the effect of candidate substances on that interaction.

The binding of the substance to the cell cycle progression polypeptide can be transient, reversible or permanent. Preferably the substance binds to the polypeptide with a Kd value which is lower than the Kd value for binding to control polypeptides (*i.e.,* polypeptides known to not be cell cycle progression polypeptides). Preferably the Kd value of the substance is 2 fold less than the Kd value for binding to control polypeptides, more preferably with a Kd value 100 fold less, and most preferably with a Kd 1000 fold less than that for binding to the control polypeptide.

*Assay for Spindle Assembly and Function*

A further assay to investigate the function of a polypeptide of interest and the effect of candidate substances on those functions is an assay which measures spindle assembly and function. Typically, such assays are performed using *Xenopus* cell free systems, where two types of spindle assembly are possible. In the "half spindle" assembly pathway, a cytoplasmic extract of CSF arrested oocytes is mixed with sperm chromatin. The half spindles that form subsequently fuse together. A more physiological method is to induce CSF arrested extracts to enter interphase by addition of calcium, whereupon the DNA replicates and kinetochores form. Addition of fresh CSF arrested extract then induces mitosis with centrosome duplication and spindle formation (for discussion of these systems see Toumebize and Heald, 1996, Nature 382(6590):420-5).

Again, generally, candidate substances known to bind to a polypeptide described here, or non-functional polypeptide variants, would be tested in this assay. Alternatively, a high throughput assay may be used to identify modulators of spindle formation and function and the resulting identified substances tested for affects binding of the polypeptide of interest as described above.

The binding of the substance to the cell cycle progression polypeptide can be transient, reversible or permanent. Preferably the substance binds to the polypeptide with a Kd value which is lower than the Kd value for binding to control polypeptides *(i.e.,* polypeptides known to not be cell cycle progression polypeptides). Preferably the Kd value of the substance is 2 fold less than the Kd value for binding to control polypeptides, more preferably with a Kd value 100 fold less, and most preferably with a Kd 1000 fold less than that for binding to the control polypeptide.

*Assays for DNA Replication*

Another assay to investigate the function of a polypeptide of interest and the effect of candidate substances on those functions is as assay for replication of DNA. A number of cell free systems have been developed to assay DNA replication. These can be used to assay the ability of a substance to prevent or inhibit DNA replication, by conducting the assay in the presence of the substance. Suitable cell-free assay systems include, for example the SV-40 assay (Li and Kelly, 1984, Proc. Natl. Acad. Sci USA 81:6973-6977; Waga and Stillman, 1994, Nature 369:207-212). A *Drosophila* cell free replication system, for example as described by Crevel and Cotteril, 1991, EMBO J. 10:4361-4369, may also be used. A preferred assay is a cell free assay derived from *Xenopus* egg low speed supernatant extracts described in Blow and Laskey (1986, Cell 47:577-587) and Sheehan et al. (1988, J. Cell Biol. 106:1-12), which measures the incorporation of nucleotides into a substrate consisting of *Xenopus* sperm DNA or HeLa nuclei. The nucleotides may be radiolabelled and incorporation assayed by scintillation counting. Alternatively and preferably, bromo-deoxy-uridine (BrdU) is used as a nucleotide substitute and replication activity measured by density substitution. The latter assay is able to distinguish genuine replication initiation events from incorporation as a result of DNA repair. The human cell-free replication assay reported by Krude et al., 1997, Cell 88:109-19 may also be used to assay the effects of substances on the polypeptides of interest.

A substance is identified as inhibiting cell cycle progression activity when it is found to inhibit or decrease such activity. In general, a substance which inhibits one or more of these aspects of cell cycle progression either inhibits it completely, or leads to a significant (*i.e.,* greater than 50%) reduction in protein activity at concentrations of 50 mM or less, relative to controls *(i.e.,* substance known to not modulate one or more aspects of cell cycle progression). Preferably, the inhibition is by 75% relative to controls, more preferably by 90%, and most preferably by 95% or 100% relative to controls. The inhibition may prevent cell cycle progression, or may simply delay or prolong cell cycle progression.

*Other In vitro Assays*

Other assays for identifying substances that bind to a polypeptide of interest are also provided. For example, substances which affect chromosome condensation may be assayed using the *in vitro* cell free system derived from *Xenopus* eggs, as known in the art.

The binding of the substance to the cell cycle progression polypeptide can be transient, reversible or permanent. Preferably the substance binds to the polypeptide with a Kd value which is lower than the Kd value for binding to control polypeptides (*i.e*., polypeptides known to not be cell cycle progression polypeptides). Preferably the Kd value of the substance is 2 fold less than the Kd value for binding to control polypeptides, more preferably with a Kd value 100 fold less, and most preferably with a Kd 1000 fold less than that for binding to the control polypeptide.

Substances which affect kinase activity or proteolysis activity are of interest. It is known, for example, that temporal control of ubiquitin-proteasome mediated protein degradation is critical for normal G1 and S phase progression (reviewed in Krek, 1998, Curr Opin Genet Dev 8:36-42). A number of E3 ubiquitin protein ligases, designated SCFs (Skp1-cullin-F-box protein ligase complexes), confer substrate specificity on ubiquitination reactions, while protein kinases phosphorylate substrates destined for destruction and convert them into preferred targets for ubiquitin modification catalyzed by SCFs. Furthermore, ubiquitin-mediated proteolysis due to the anaphase-promoting complex/cyclosome (APC/C) is essential for separation of sister chromatids during mitosis, and exit from mitosis (Listovsky et al., 2000, Exp Cell Res 255:184-191).

Substances which inhibit or affect kinase activity may be identified by means of a kinase assay as known in the art, for example, by measuring incorporation of $^{32}P$ into a suitable peptide or other substrate in the presence of the candidate substance. Similarly, substances which inhibit or affect proteolytic activity may be assayed by detecting increased or decreased cleavage of suitable polypeptide substrates.

Assays for these and other protein or polypeptide activities are known to those skilled in the art, and may suitably be used to identify substances which bind to a polypeptide of interest and affect its activity.

*Whole Cell Assays*

Candidate substances may also be tested on whole cells for their effect on cell cycle progression, including mitosis and/or meiosis. Preferably the candidate substances have been identified by the above-described *in vitro* methods. Alternatively, rapid throughput screens for substances capable of inhibiting cell division, typically mitosis, may be used as a preliminary screen and then used in the *in vitro* assay described above to confirm that the affect is on a particular polypeptide of interest.

The candidate substance, *i.e.,* the test compound, may be administered to the cell in several ways. For example, it may be added directly to the cell culture medium or injected into the cell. Alternatively, in the case of polypeptide candidate substances, the cell may be transfected with a nucleic acid construct which directs expression of the polypeptide in the cell. Preferably, the expression of the polypeptide is under the control of a regulatable promoter.

Typically, an assay to determine the effect of a candidate substance identified by the method described above on a particular stage of the cell division cycle comprises administering the candidate substance to a cell and determining whether the substance inhibits that stage of the cell division cycle. Techniques for measuring progress through the cell cycle in a cell population are well known in the art. The extent of progress through the cell cycle in treated cells is compared with the extent of progress through the cell cycle in an untreated control cell population to determine the degree of inhibition, if any. For example, an inhibitor of mitosis or meiosis may be assayed by measuring the proportion of cells in a population which are unable to undergo mitosis/meiosis and comparing this to the proportion of cells in an untreated population.

The concentration of candidate substances used will typically be such that the final concentration in the cells is similar to that described above for the *in vitro* assays.

A candidate substance is typically considered to be an inhibitor of a particular stage in the cell division cycle (for example, mitosis) if the proportion of cells undergoing that particular stage (*i.e.,* mitosis) is reduced to below 50%, preferably below 40, 30, 20 or 10% of that observed in untreated control cell populations.

Suitably a polypeptide of interest in the context of the above assays is a polypeptide encoded by any nucleic acid sequence identified in Table 1.

## THERAPEUTIC USES

Many tumours are associated with defects in cell cycle progression, for example loss of normal cell cycle control. Tumour cells may therefore exhibit rapid and often aberrant mitosis. One therapeutic approach to treating cancer is therefore to inhibit mitosis in rapidly dividing cells. Such an approach may also be used for therapy of any proliferative disease in general. In general, a proliferative disease is defined as being "treated" if the cell proliferation associated with the disease or condition is significantly inhibited (*i.e.*, by 50% or more) relative to controls. Preferably, the inhibition is by 75% relative to controls, more preferably by 90%, and most preferably by 95% or 100% relative to controls. The inhibition may prevent cell proliferation, may simply delay or prolong proliferation.

Thus, since the polypeptides described appear to be required for normal cell cycle progression, they represent targets for inhibition of their functions, particularly in tumour cells and other proliferative cells. Another therapeutic approach to treating cancer may be an anti-angiogenic approach in which angiogenesis is targeted and thus growth of tumour blood vessels inhibited thereby depriving a tumour of blood supply.

The term proliferative disorder is used herein in a broad sense to include any disorder that requires control of the cell cycle, for example, cardiovascular disorders such as restenosis and cardiomyopathy, auto-immune disorders such as glomerulonephritis and rheumatoid arthritis, dermatological disorders such as psoriasis, anti-inflammatory, antifungal, antiparasitic disorders such as malaria, emphysema and alopecia.

Proliferative disorders also include malignant and pre-neoplastic disorders. The methods and compositions described here are especially useful in relation to treatment or diagnosis of adenocarcinomas such as: small cell lung cancer, and cancer of the kidney, uterus, prostrate, bladder, ovary, colon and breast. For example, malignancies which may be treatable include acute and chronic leukemias, lymphomas, myelomas, sarcomas such as Fibrosarcoma, myxosarcoma, liposarcoma, lymphangioendotheliosarcoma, angiosarcoma, endotheliosarcoma, chondrosarcoma, osteogenic sarcoma, chordoma, lymphangiosarcoma, synovioma, mesothelioma, leimyosarcoma, rhabdomyosarcoma, colon carcinoma, ovarian cancer, prostate cancer, pancreatic cancer, breast cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, choriocarcinoma, renal cell carcinoma, hepatoma, bile duct carcinoma seminoma, embryonal carcinoma, cervical cancer, testicular tumour, lung carcinoma, small cell lung carcinoma, bladder carcinoma, epithelial carcinoma, glioma, astrocytoma, ependymoma, pinealoma, hemangioblastoma, acoustic neuoma, medulloblastoma, craniopharyngioma, oligodendroglioma, menangioma, melanoma, neutroblastoma and retinoblastoma.

One possible approach is to express anti-sense constructs directed against polynucleotides as described here, preferably selectively in tumour cells, to inhibit gene function and prevent the tumour cell from progressing through the cell cycle. Anti-sense constructs may also be used to inhibit gene function to prevent cell cycle progression in a proliferative cell. Another approach is to use non-functional variants of polypeptides as described in this document that compete with the endogenous gene product for cellular components of cell cycle machinery, resulting in inhibition of function. Alternatively, compounds identified by the assays described above as binding to a polypeptide described herein may be administered to tumour or proliferative cells to prevent the function of that polypeptide. This may be performed, for example, by means of gene therapy or by direct administration of the compounds. Suitable antibodies as described herein may also be used as therapeutic agents.

Alternatively, double-stranded (ds) RNA is a powerful way of interfering with gene expression in a range of organisms that has recently been shown to be successful in mammals (Wianny and Zernicka-Goetz, 2000, Nat Cell Biol 2:70-75). Double stranded RNA corresponding to the sequence of a polynucleotide described herein can be introduced into or expressed in oocytes and cells of a candidate organism to interfere with cell division cycle progression.

In addition, a number of the mutations described herein exhibit aberrant meiotic phenotypes. Aberrant meiosis is an important factor in infertility since mutations that affect only meiosis and not mitosis will lead to a viable organism but one that is unable to produce viable gametes and hence reproduce. Consequently, the elucidation of genes involved in meiosis is an important step in diagnosing and preventing/treating fertility problems. Thus the polypeptides identified in mutant *Drosophila* having meiotic defects (as is clearly indicated in the Examples) may be used in methods of identifying substances that affect meiosis. In addition, these polypeptides, and corresponding polynucleotides, may be used to study meiosis and identify possible mutations that are indicative of infertility. This will be of use in diagnosing infertility problems.

## ADMINISTRATION

Substances identified or identifiable by the assay methods as described herein may preferably be combined with various components to produce useful compositions. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline. The compositions may be administered by direct injection. The composition may be formulated for parenteral, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to 1 mg/kg body weight.

Polynucleotides/vectors encoding polypeptide components (or antisense constructs) for use in inhibiting cell cycle progression, for example, inhibiting mitosis or meiosis, may be administered directly as a naked nucleic acid construct. They may further comprise flanking sequences homologous to the host cell genome. When the polynucleotides/vectors are administered as a naked nucleic acid, the amount of nucleic acid administered may typically be in the range of from 1 μg to 10 mg, preferably from 100 μg to 1 mg. It is particularly preferred to use polynucleotides/vectors that target specifically tumour or proliferative cells, for example by virtue of suitable regulatory constructs or by the use of targeted viral vectors.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam™ and transfectam™). Typically, nucleic acid constructs are mixed with the transfection agent to produce a composition.

Preferably the polynucleotide, polypeptide, compound or vector described here may be conjugated, joined, linked, fused, or otherwise associated with a membrane translocation sequence.

Preferably, the polynucleotide, polypeptide, compound or vector, etc described here may be delivered into cells by being conjugated with, joined to, linked to, fused to, or otherwise associated with a protein capable of crossing the plasma membrane and/or the nuclear membrane *(i.e.,* a membrane translocation sequence). Preferably, the substance of interest is fused or conjugated to a domain or sequence from such a protein responsible for the translocational activity. Translocation domains and sequences for example include domains and sequences from the HIV-1-trans-activating protein (Tat), *Drosophila* Antennapedia homeodomain protein and the herpes simplex-1 virus VP22 protein. In a highly preferred embodiment, the substance of interest is conjugated with penetratin protein or a fragment of this. Penetratin comprises the sequence RQIKIWFQNRRMKWKK and is described in Derossi et al., 1994, J. Biol. Chem. 269:10444-50; use of penetratin-drug conjugates for intracellular delivery is described in WO 00/01417. Truncated and modified forms of penetratin may also be used, as described in WO 00/2927.

Preferably the polynucleotide, polypeptide, compound or vector is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular or transdermal administration.

Use of timed release or sustained release delivery systems are also included in the present disclosure. Such systems are highly desirable in situations where surgery is difficult or impossible, *e.g.*, patients debilitated by age or the disease course itself, or where the risk-benefit analysis dictates control over cure.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials.

The routes of administration and dosages described are intended only as a guide since a skilled practitioner will be able to determine readily the optimum route of administration and dosage for any particular patient and condition.

## FURTHER ASPECTS

Further aspects and embodiments of the invention are now set out in the following paragraphs; it is to be understood that the invention encompasses these aspects. In these paragraphs, the term cell cycle progression polypeptide should preferably include the even numbered SEQ ID NO:s as set out in the Sequence Listings, and the term cell cycle progression nucleic acid should preferably include the even numbered SEQ ID NO:s as set out in the Sequence Listings.

We describe a method of identifying an agent that modulates the function of a cell cycle progression polypeptide, where the method includes: (a) providing a sample containing a cell cycle progression polypeptide and a candidate agent; (b) measuring the binding of the cell cycle progression polypeptide to the candidate agent in the sample; and (c) comparing the binding of the cell cycle progression polypeptide to the candidate agent in the sample with the binding of the cell cycle progression polypeptide to a control agent, where the control agent is known to not bind to the cell cycle progression polypeptide; where an increase in the binding of the cell cycle progression polypeptide to the candidate agent in the sample relative to the binding of the cell cycle progression polypeptide to the control agent indicates that the candidate agent modulates the function of the cell cycle progression polypeptide.

We also describe a method of detecting the presence in a sample of a cell cycle progression polypeptide, where the method includes: (a) bringing the biological sample containing DNA or RNA into contact with a probe comprising a fragment of at least 15 nucleotides of a cell cycle progression nucleic acid under hybridizing conditions; and (b) detecting a duplex formed between the probe and nucleic acid in the sample; where detection of a duplex indicates the presence in the sample of a cell cycle progression polypeptide.

We describe a method of detecting the presence in a sample of a cell cycle progression polypeptide, where the method includes: (a) providing an antibody capable of binding to the cell cycle progression polypeptide; (b) incubating a biological sample with said antibody under conditions which allow for the formation of an antibody-antigen complex; and (c) detecting an antibody-antigen complex comprising said antibody; where detection of an antibody-antigen complex indicates the presence in the sample of a cell cycle progression polypeptide.

We describe a method of modulating cell cycle progression in a cell, where the method includes: (a) transforming into the cell a double-stranded cell cycle progression nucleic acid sequence or a complement thereof, where the nucleic acid sequence is operably linked to a regulatory sequence; and (b) culturing the cell under conditions whereby the nucleic acid sequence is expressed; thereby modulating cell cycle progression in the cell.

We describe a method of modulating cell cycle progression in a cell, where the method includes: (a) transforming into the cell a double-stranded nucleic acid sequence encoding a cell cycle progression polypeptide, where the nucleic acid sequence is operably linked to a regulatory sequence; and (b) culturing the cell under conditions whereby the nucleic acid sequence is expressed; thereby modulating cell cycle progression in the cell.

We describe a method of modulating cell cycle progression in a cell, where the method includes: (a) transforming into the cell a double-stranded nucleic acid sequence encoding a polypeptide having at least 80% sequence identity with a cell cycle progression polypeptide, where the nucleic acid sequence is operably linked to a regulatory sequence; and (b) culturing the cell under conditions whereby the nucleic acid sequence is expressed; thereby modulating cell cycle progression in the cell.

We describe a method of modulating cell cycle progression in a cell, where the method includes: (a) transforming into the cell an isolated nucleic acid molecule comprising a regulatory sequence operably linked to a nucleic acid sequence that encodes a ribonucleic acid (RNA) precursor, where the precursor comprises: (i) a first stem portion comprising a 15 to 40 nucleotide long sequence that is identical to 15 to 40 consecutive nucleotides of a cell cycle progression nucleic acid sequence; (ii) a second stem portion comprising a 15 to 40 nucleotide long sequence that is complementary to 15 to 40 consecutive nucleotides of a cell cycle progression nucleic acid sequence, and where the first and second stem portions can hybridize with each other to form a duplex stem; and (iii) a loop portion that connects the two stem portions; (b) culturing the cell under conditions whereby the nucleic acid sequence is expressed; thereby modulating cell cycle progression in the cell.

In any of the methods described herein, the nucleic acid sequence is a cell cycle progression nucleic acid sequence, or a complement thereof. The nucleic acid sequence can encode cell cycle progression polypeptide. The nucleic acid sequence can encode a polypeptide having 80% sequence identity to a cell cycle progression polypeptide.

The methods described herein can be used to decrease cell cycle progression. The decrease can result in a decrease in proliferation of the cell.

The methods described herein can be used to increase cell cycle progression. The increase can result in a increase in proliferation of the cell.

We describe an RNA precursor encoded by a cell cycle progression nucleic acid sequence. Such an RNA precursor can be included in a composition as a biologically active ingredient. Such an RNA precursor or composition can be used for treating a disease or condition characterized by cell proliferation in mammalian tissue, by contacting the tissue with the RNA precursor or composition. The disease can be cancer.

We describe a host cell transformed by the methods described herein. Such a host cell can contain all or a part of the cell cycle progression nucleic acid sequences. Such a host cell can express all or a part of the cell cycle progression polypeptide sequences.

The methods described herein can be used to provide a mammal with an anti-proliferative protein, where the method includes introducing into the mammal a mammalian cell transformed by the methods described herein.

We describe a pharmaceutical composition comprising, as an active ingredient, a cell cycle progression nucleic acid sequence, and a pharmaceutically-acceptable carrier.

We describe a pharmaceutical composition comprising, as an active ingredient, a cell cycle progression polypeptide, and a pharmaceutically-acceptable carrier.

We describe a pharmaceutical composition comprising, as an active ingredient, an antibody to a cell cycle progression nucleic acid sequence, and a pharmaceutically-acceptable carrier. Such an antibody can be used in a method for diagnosing a disease or condition characterized by cell proliferation in mammalian tissue, the method comprising contacting the tissue with the antibody, and detecting an antibody/antigen complex, wherein said detection is indicative of said disease or condition.

We describe a pharmaceutical composition comprising, as an active ingredient, an antibody to a cell cycle progression polypeptide, and a pharmaceutically-acceptable carrier. Such an antibody can be used in a method for diagnosing a disease or condition characterized by cell proliferation in mammalian tissue, the method comprising contacting the tissue with the antibody, and detecting an antibody/antigen complex, wherein said detection is indicative of said disease or condition.

We describe a method for treating a disease or condition characterized by cell proliferation in mammalian tissue, the method comprising contacting the tissue with an antagonist of a cell cycle progression polypeptide.

We describe a method for treating a disease or condition characterized by cell proliferation in mammalian tissue, the method comprising contacting the tissue with an agonist of a cell cycle progression polypeptide.

We describe a kit for treating a disease or condition characterized by cell proliferation in mammalian tissue, the kit comprising: (a) a polypeptide encoded by a cell cycle progression nucleic acid sequence; (b) a nucleic acid having a cell cycle progression nucleotide sequence; or (c) an antibody recognising an epitope of a polypeptide of (a).

We describe an array comprising at least two cell cycle progression genes having cell cycle progression nucleic acid sequences. The nucleic acid sequences can be DNA sequences. The nucleic acid sequences can be RNA sequences.

We describe an array comprising at least two cell cycle progression proteins having polypeptide sequences as set out. Accordingly, in a first aspect, there is provided a method of modulating cell cycle progression in a cell comprising the step of increasing, decreasing or otherwise altering the functional activity of (i) a polypeptide having an amino acid sequence identified in Table 1; (ii) a polypeptide having an amino acid sequence encoded by a nucleic acid identified in Table 1; (iii) a polypeptide having at least 80% homology with i) or ii); (iv) a nucleic acid having a sequence identified in Table 1 or encoding a polypeptide having the sequence set out in any of i) to iii); (v) a nucleic acid which is capable of selectively hybridising to the sequence set out in iv); or (vi) the complement of iv) or v).

Suitably, the method comprises decreasing gene expression. In a preferred embodiment, the method comprises decreasing the nucleic acid functional activity by introducing a double stranded (dsRNA) corresponding to the nucleic acid, or an antisense RNA corresponding to the nucleic acid, or a fragment thereof, into the cell.

In another embodiment, the method comprises increasing the functional activity.

In a particularly preferred embodiment, the nucleic acid or polypeptide comprises a human nucleic acid or polypeptide as identified in Table 1.

In one embodiment, the method comprises: (a) providing an expression vector comprising a nucleic acid sequence; said nucleic acid sequence being selected from the group consisting of: (i) a nucleic acid having a sequence identified in Table 1; (ii) a nucleic acid which hybridises under stringent conditions to the sequence set out in i); or (iii) the complement of ii); and (b) introducing the expression vector into the cell and maintaining the cell under conditions permitting expression of the encoded polypeptide in the cell.

Knowledge of the genes involved in cell cycle progression allows the development of therapeutic agents for the treatment of medical conditions associated with aberrant cell cycle progression.

Accordingly, we describe a use of a nucleic acid identified in Table 1 or a polypeptide identified in Table 1 or a fragment thereof, in a method of prevention, treatment or diagnosis of a disease in an individual.

Suitably, the nucleic acid or polypeptide comprises a human nucleic acid or polypeptide identified in Table 1.

In one embodiment the nucleic acid or polypeptide is used to identify a substance capable of binding to the polypeptide, which method comprises incubating the polypeptide with a candidate substance under suitable conditions and determining whether the substance binds to the polypeptide.

In another embodiment, the nucleic acid or polypeptide is used to identify a substance capable of modulating the function of the polypeptide, the method comprising the steps of: incubating the polypeptide with a candidate substance and determining whether the activity of the polypeptide is thereby modulated.

Thus, we describe the use of a cell cycle progression polypeptide encoded by a nucleic acid identified in Table 1 in an assay for identifying a substance capable of inhibiting cell cycle progression.

The nucleic acid or polypeptide may be administered to an individual in need of such a treatment. Alternatively, or in addition, the substance identified by the method is administered to an individual in need of such treatment.

Also provided is a substance identified by the above uses. Such substances may be used in a method of therapy, such as in a method of affecting cell cycle progression, for example mitosis and/or meiosis.

We describe a process comprising the steps of: (a) performing one of the above methods; and (b) preparing a quantity of those one or more substances identified as being capable of binding to such a polypeptide.

Also provided is a process comprising the steps of: (a) performing one of the above methods; and (b) preparing a pharmaceutical composition comprising one or more substances identified as being capable of binding to a polypeptide.

The use may be for a method of diagnosis, in which the presence or absence of a nucleic acid is detected in a biological sample in a method comprising: (a) bringing the biological sample containing DNA or RNA into contact with a probe comprising a fragment of at least 15 nucleotides of the nucleic acid identified in Table 1 under hybridising conditions; and (b) detecting any duplex formed between the probe and nucleic acid in the sample.

Alternatively, or in addition, the absence or presence of a polypeptide is detected in a biological sample in a method comprising: (a) providing an antibody capable of binding to the polypeptide; (b) incubating a biological sample with said antibody under conditions which allow for the formation of an antibody-antigen complex; and (c) determining whether antibody-antigen complex comprising said antibody is formed.

In a particularly preferred embodiment, the disease comprises a disease associated with a defect in the cell cycle and, in particular, a proliferative disease such as cancer.

We describe a pharmaceutical composition comprising any one or more of the following: a polypeptide encoded by a nucleic acid identified in Table 1, or part thereof; a vector comprising a nucleic acid identified in Table 1; an antibody recognising an epitope of a polypeptide encoded by a nucleotide sequence identified in Table 1, together with a pharmaceutically acceptable carrier or diluent.

In one embodiment, the pharmaceutical composition is a vaccine composition.

In a further aspect, there is provided a nucleic acid identified in Table 1 for use in therapy.

In yet another aspect, there is provided a polypeptide encoded by a nucleic acid identified in Table 1 for use in therapy.

In a yet further aspect, there is provided an antibody capable of binding a polypeptide encoded by a nucleic acid identified in Table 1 for use in therapy.

Alternatively, we describe a method of treating a patient suffering from a disease associated with enhanced activity of a cell cycle progression protein encoded by a nucleic acid identified in Table 1, which method comprises administering to the patient an antagonist of said cell cycle progression protein.

In another aspect there is provided a method of treating a patient suffering from a disease associated with reduced activity of a cell cycle progression protein encoded by a nucleic acid identified in Table 1, which method comprises administering to the patient an agonist of said cell cycle progression protein.

In an additional aspect, we describe kits comprising polynucleotides, polypeptides or antibodies as described here and methods of using such kits in diagnosing the presence of absence of polynucleotides and polypeptides including deleterious mutant forms.

Accordingly, there is provided a diagnostic kit for a disease or susceptibility to a disease comprising any one or more of the following: a polypeptide encoded by a nucleic acid sequence identified in Table 1 or part thereof; a nucleic acid having a nucleotide sequence identified in Table 1; and an antibody recognising an epitope of a polypeptide encoded by a nucleic acid having a nucleotide sequence identified in Table 1.

In one embodiment, said diagnostic kit comprises an array, such as a nucleic acid or other microarray, comprising at least two cell cycle progression genes having nucleic acid sequences identified in Table 1, or fragments thereof. The fragments can be 15 nucleotides in length, or longer, up to the full length of the gene.

Suitably the disease or syndrome is one which is associated with abnormal cell cycle or proliferation such as cancer.

## EXAMPLES

The invention will now be further described by way of Examples, which are meant to serve to assist one of ordinary skill in the art in carrying out the invention and are not intended in any way to limit the scope of the invention.

*Introduction*

In order to identify new human cell cycle regulatory genes, candidate genes were identified by establishing the role of their *Drosophila* counterparts in cell cycle progression through an RNAi-based knockdown approach in cultured *Drosophila* cells followed by mitotic index evaluation (Cellomics Arrayscan) and confirming the role of the human gene through RNAi in human cells followed by FACS analysis and microscopy.

A list of Human cell cycle progression genes and their *Drosophila* counterparts for which data is described is presented in Table 1. Multiple protein sequence gi numbers are listed where alternative transcripts or splice variants for a *Drosophila* gene are listed in the data base, These Human and *Drosophila* genes and proteins are useful, for example, for screening for anti-proliferative molecules.

The homologies (*Drosophila* and human) are clustered homologies within a region of the gene and do not relate to comparison of the whole human gene with the whole *Drosophila* gene. The similarities quoted relate to the region of greatest homology for that gene. The score also indicates confidence in homology, with higher scores indicating higher confidence. The score relates not only to the percentage similarity, but also to the length of sequence over which the similarity extends.

The homologues are identified by performing a BLAST search and identifying the human protein with the best homology, i.e., the one nearest the top of the search results list, where genes are ranked in descending order according to the likelihood of a match not being due to chance. In some cases, there are other sections of the *Drosophila* and human genes with homology.

**Table 1**

| *Drosophila* Gene | | | Human homologue(s) | | BLAST results | |
|---|---|---|---|---|---|---|
| **Gene Name** | **Nucleotide sequence gi number** | **Protein sequence gi numbers** | **Gene Name** | **SWISS-PROT reference** | **Similarity** | **Score** |
| CG3632 | 10728281 | 7293200<br>7293201<br>7293199<br>7293198 | Similar to myotubularin related protein 3. Hypothetical FYVE domain-containing dual specificity protein phosphatase FYVE-DSP1C. | Q9UEG3 | 365/575 (62%) | 546 |
| Pp1-87B | 7299572 | 7299625 | Serine/threonine protein phosphatase PP1-gamma catalytic subunit (EC 3.1.3.16) (PP-1G). | P36873 | 289/299 (96%) | 590 |
| CG3524 | 10727365 | 7295849 | Fatty acid synthase (EC 2.3.1.85). | Q16702 | 748/1174 (62%) | 1011 |
| CG9311 | 10727923 | 7294357 | Protein tyrosine phosphatase HD-PTP (Protein tyrosine phosphatase TD14). | Q9H3S7 | 423/738 (56%) | 480 |
| CG9092 | 7297037 | 7297052 | Beta-galactosidase precursor (EC 3.2.1.23) (Lactase) (Acid beta-galactosidase). | P16278 | 364/672 (53%) | 402 |
| Arr1 | 10728850 | 7298421 | Beta-Arrestin 1. A regulator of GPCR activity | P49407 | 228/356 (63%) | 306 |
| CG9150 | 7297037 | 7297069 | Hypothetical protein with putative dehydrogenase/ reductase domains | Q9BUC7 | 101/176 (56%) | 115 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG11102 | 10728232 | 7292929 | KIAA0659 protein (Fragment). | BAA31634 | 330/562 (58%) | 422 |
| Smr | 7292788 | 7292802 | Nuclear receptor co-repressor 1 (NCR-1). | O75376 | 206/379 (53%) | 226 |
| CG8045 | 7300335 | 7300358<br>7300360<br>7300364 | 14-3-3 protein epsilon (Mitochondrial import stimulation factor L subunit) (Protein kinase C inhibitor protein-1) (KCIP-1) (14-3-3E). | P42655 | 237/258 (91%) | 424 |
| CG10420 | 7301280 | 7301293 | SIL1 protein precursor (Endoplasmic reticulum chaperone SIL1, homolog of yeast). | Q9H173 | 159/318 (49%) | 135 |
| Hsc70-2 | 10726497 | 7299717 | Heat shock cognate 71 kDa protein. | P11142 | 507/606 (82%) | 879 |
| CG10805 | 7297167 | 7297181 | Protein BAP28. | Q9H583 | 629/1324 (47%) | 465 |
| elF-4a | 7297037 | 7297048 | Similar to eukaryotic translation initiation factor 4A2 | Q96EA8 | 342/402 (84%) | 576 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| ACXA | 7297983 | 10728753 | CYA2 Adenylate cyclase, type II (EC 4.6.1.1) (ATP pyrophosphate-lyase) (Adenylyl cyclase) (Fragment). | Q08462 | 420/931 (44%) | 306 |
| | | | CYA8 Adenylate cyclase, type VIII (EC 4.6.1.1) (ATP pyrophosphate-lyase) (Ca(2+)/calmodulin activated adenylyl cyclase). | P40145 | 446/1037 (42%) | 296 |
| CG15117 | 10727456 | 7302519 | Beta-glucuronidase precursor (EC 3.2.1.31) (Beta-G1). | P08236 | 357/638 (55%) | 431 |
| BG:DS01759.2 | 7298121 | 7298138 | No human homologue | | | |
| TepIII | 7297264 | 7297279 | Cell surface antigen CD109. | Q8TDJ3 | 686/1478 (46%) | 500 |
| Hsc70-4 | 10726541 | 7299978 | HS7C Heat shock cognate 71 kDa protein. | P11142 | 539/612 (87%) | 994 |
| CG7069 | 10726692 | 10726696 | Pyruvate kinase, M2 isozyme (EC 2.7.1.40). | P14786 | 280/412 (67%) | 400 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| ACXE | 7297983 | 7297987 | CYA8 Adenylate cyclase, type VIII (EC 4.6.1.1) (ATP pyrophosphate-lyase) (Ca(2+)/calmodulin activated adenylyl cyclase). | P40145 | 454/968 (46%) | 331 |
| | | | CYA7 Adenylate cyclase, type VII (EC 4.6.1.1) (ATP pyrophosphate-lyase) (Adenylyl cyclase). | P51828 | 211/397 (52%) | 212 |
| EG:52C10.5 | 10727480 | 7302711 | FYVE finger-containing phosphoinositide kinase (EC 2.7.1.68) (1-phosphatidylinositol-4-phosphate kinase) (PIP5K) (Ptdlns(4)P-5-kinase) (p235) (Fragment). | Q9Y2I7 | 193/348 (54%) | 253 |
| gatA | 10726610 | 7300468 | Hypothetical protein | Q9H0R6 | 320/507 (62%) | 465 |
| CG17149 | 10727803 | 7293682<br>7293681 | DJ184J9.1 (Hypothetical protein KIAA0601) (Fragment) | Q9NUH3 | 552/856 (63%) | 793 |
| CG2905 | 10728163 | 7302249 | TRRAP protein/PI3 PI4 kinase, a novel ATM-related protein. | Q9Y6H4 | 2393/3582 (65%) | 3391 |
| CG2336 | 10727121 | 7298905 | No human homologue | | | |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| TER94 | 10727672 | 7303816<br>7303817 | Transitional endoplasmic reticulum ATPase (TER ATPase) (15S Mg(2+)- ATPase p97 subunit) (Valosin containing protein) (VCP) [Contains: Valosin]. | P55072 | 692/803 (86%) | 1248 |
| CG6313 | 10726739 | 7301133 | KIAA0697 protein (Fragment). | BAA31672 | 451/575 (78%) | 737 |
| aur | 10726473 | 7299536 | AURORA-related kinase 1 (DJ1167H4.2) (Serine/threonine kinase 15). | O60445 | 207/262 (78%) | 346 |
| | | | AURORA-related kinase 2 (Serine/ threonine kinase 12). | O60446 | 205/261 (78%) | 337 |
| Pk91C | 10799498 | 7300437 | hypothetical protein FLJ14813 | Q96SJ5 | | |
| Top2 | 10728874 | 7298585 | DNA topoisomerase II, alpha isozyme (EC 5.99.1.3). | P11388 | 651/898 (71%) | 1026 |
| | | | DNA topoisomerase II, beta isozyme (EC 5.99.1.3). | Q02880 | 903/1200 (74%) | 1470 |
| alpha-Est1 | 10727101 | 10727102 | Acetylcholinesterase precursor (EC 3.1.1.7) (AChE). | P22303 | 251/542 (45%) | 208 |

(continued)

| *Drosophila* Gene | | | Human homologue(s) | | BLAST results | |
|---|---|---|---|---|---|---|
| **Gene Name** | **Nucleotide sequence gi number** | **Protein sequence gi numbers** | **Gene Name** | **SWISS-PROT reference** | **Similarity** | **Score** |
| Nrk | 10727582 | 7303361 | Muscle specific tyrosine kinase receptor. | O15146 | 228/303 (74%) | 378 |
| otk | 10727617 | 7303541 | PTK7 Tyrosine-protein kinase-like 7 precursor (Colon carcinoma kinase-4) (CCK-4). | Q13308 | 156/245 (63%) | 208 |
| cad | 10799497 | 7298711 | Homeobox protein CDX-2 (Caudal-type homeobox protein 2) (CDX-3). | Q99626 | 67/81 (82%) | 123 |
| Rut | 10728252 | 7293001 | CYA6 Adenylate cyclase, type VI (EC 4.6.1.1) (ATP pyrophosphate-lyase) (Ca(2+)-inhibitable adenylyl cyclase). | O43306 | 293/441 (66%) | 405 |
| CG8002 | 10728334 | 7293569 | KIAA1999 protein (Fragment). | BAC02708 | 270/526 (51%) | 260 |
| CG10335 | 10727968 | 7294597 | HEM2 Delta-aminolevulinic acid dehydratase (EC 4.2.1.24) (Porphobilinogen synthase) (ALADH). | P13716 | 243/318 (75%) | 392 |
| CG8070 | 10727693 | 7303942 | Hypothetical protein FLJ10498 | Q9NVU7 | 301/458 (65%) | 447 |
| CG7460 | 10727853 | 7293951 | Polyamine oxidase isoform-1. | Q96QT3 | 209/521 (39%) | 137 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG17735 | 10727172 | 7296816 | Thyroid receptor interacting protein 12 (TRIP12). | Q14669 | 438/663 (65%) | 652 |
| Gycalpha99B | 7301790 | 7301807 | CYG4 Guanylate cyclase soluble, alpha-2 chain (EC 4.6.1.2) (GCS-alpha-2). | P33402 | 363/670 (54%) | 394 |
| CG13893 | 7291959 | 7291980 | SEC14-like protein 2 (Alpha-tocopherol associated protein) (TAP) (hTAP) (Supernatant protein factor) (SPF) (Squalene transfer protein). | O76054 | 179/316 (56%) | 194 |
| CG18176 | 10728019 | 7294884 | DKFZP434B168 protein. | AAH33918 | 517/994 (51%) | 502 |
| CG8858 | 10727617 | 7303499 | Hypothetical protein KIAA0368 (Fragment). | O15074 | 752/1552 (47%) | 607 |
| Ac76E | 10733346 | 10733351 | CYA2 Adenylate cyclase, type II (EC 4.6.1.1) (ATP pyrophosphate-lyase) (Adenylyl cyclase) (Fragment). | Q08462 | 194/267 (71%) | 294 |
| | | | Adenylate cyclase, type VII (EC 4.6.1.1) (ATP pyrophosphate-lyase) (Adenylyl cyclase). | P51828 | 257/463 (55%) | 291 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG17010 | 7297915 | 7297937 | Ribokinase (EC 2.7.1.15). | Q9H477 | 155/270 (57%) | 164 |
| Tkv | 7296952 | 22945650 | Bone morphogenetic protein receptor type IB precursor (EC 2.7.1.37). | O00238 | 313/489 (63%) | 460 |
| | | | Bone morphogenetic protein receptor type IA precursor (EC 2.7.1.37) (Serine/threonine-protein kinase receptor R5) (SKR5) (Activin receptor-like kinase 3) (ALK-3). | P36894 | 307/490 (62%) | 454 |
| | | | Activin receptor type I precursor (EC 2.7.1.37) (ACTR-I) (Serine/ threonine-protein kinase receptor R1) (SKR1) (Activin receptor-like kinase 2) (ALK-2) (TGF-B superfamily receptor type I) (TSR-I). | Q04771 | 309/521 (59%) | 394 |
| Dnt | 10728868 | 7298565 | Tyrosine-protein kinase RYK precursor (EC 2.7.1.112). | P34925 | 305/558 (54%) | 343 |

(continued)

| *Drosophila* Gene | | | Human homologue(s) | | BLAST results | |
|---|---|---|---|---|---|---|
| **Gene Name** | **Nucleotide sequence gi number** | **Protein sequence gi numbers** | **Gene Name** | **SWISS-PROT reference** | **Similarity** | **Score** |
| ACXD | 10727242 | 7292211 | CYA2 Adenylate cyclase, type II (EC 4.6.1.1) (ATP pyrophosphate-lyase) (Adenylyl cyclase) (Fragment). | Q08462 | 444/976 (45%) | 333 |
| | | | CYA8 Adenylate cyclase, type VIII (EC 4.6.1.1) (ATP pyrophosphate-lyase) (Ca(2+)/calmodulin activated adenylyl cyclase). | P40145 | 423/897 (46%) | 320 |
| Aats-ala-m | 10728137 | 7295490 | Alanyl-tRNA synthetase (EC 6.1.1.7) (Alanine--tRNA ligase) (AlaRS). | P49588 | 511/1047 (48%) | 489 |
| Gek | 7291737 | 7291742 | CDC42-binding protein kinase beta. | Q9Y5S2 | 977/1678 (57%) | 1155 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG3216 | 10726992 | 7291217 | Atrial natriuretic peptide receptor A precursor (ANP-A) (ANPRA) (GC-A) (Guanylate cyclase) (EC 4.6.1.2) (NPR-A) (Atrial natriuretic peptide A-type receptor). | P16066 | 588/1046 (55%) | 682 |
| | | | Atrial natriuretic peptide receptor B precursor (ANP-B) (ANPRB) (GC-B) (Guanylate cyclase) (EC 4.6.1.2) (NPR-B) (Atrial natriuretic peptide B-type receptor). | P20594 | 146/224 (64%) | 187 |
| CG5653 | 10728037 | 7295017 | Polyamine oxidase isoform-1. | Q96QT3 | 105/234 (44%) | 99 |
| CG17740 | 10727606 | 21627360 promoting | VSGP/F (vascular smooth muscle cell growth factor)-spondin. | Q9HCB6 | 261/527 (49%) | 259 |
| Tepl | 7298255 | 10728811 | Cell surface antigen CD109. | Q8TDJ3 | 644/1355 (46%) | 497 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| for | 10727349 | 10727350<br>10727351<br>10727352<br>10727353<br>10727354 | cGMP-dependent protein kinase 1, alpha isozyme (EC 2.7.1.37) (CGK 1 alpha) (cGKI-alpha). Exhibits a substrate specificity similar but not identical to CAK | Q13976 | 518/706 (73%) | 827 |
| Ac13E | 10728265 | 7293083 | Adenylate cyclase, type IX (EC 4.6.1.1) (ATP pyrophosphate-lyase) (Adenylyl cyclase). | O60503 | 286/521 (54%) | 351 |
| | | | Adenylate cyclase type III (EC 4.6.1.1) (Adenylate cyclase, olfactive type) (ATP pyrophosphate-lyase) (Adenylyl cyclase) (AC-III) (AC3). | O60266 | 174/307 (55%) | 208 |
| CG2667 | 7298935 | 7298950 | Diacylglycerol kinase, delta (EC 2.7.1.107) (Diglyceride kinase) (DGK-delta) (DAG kinase delta) (130 kDa diacylglycerol kinase) (Fragment). | Q16760 | 327/526 (61%) | 421 |
| CG7842 | 10727872 | 7294021 | BK1191B2.3.1 (Putative novel acyl transferase similar to C. ELEGANS C50D2.7) (Isoform 1) (Fragment). | O95510 | 203/310 (65%) | 299 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG17486 | 7289853 | 7289856 | Hypothetical protein FLJ20752 | Q9NWL6 | 310/632 (48%) | 273 |
| CG6969 | 10726705 | 7300903 | MYELOBLAST KIAA0230 [Fragment], a human melanoma associated gene | Q92626 | 295/574 (51%) | 317 |
| CG12262 | 10728071 | 7295201 | Acyl-CoA dehydrogenase, medium-chain specific, mitochondrial [Precursor] | P11310 | 337/411 (81%) | 587 |
| Fray | 10726601 | 10726604 | Oxidative-stress responsive 1, a Ser/Thr pkinase | O95747 | 338/524 (63%) | 513 |
| CG6879 | 10726756 | 7301203 | MYELOBLAST KIAA0230 [Fragment], a human melanoma associated gene | Q92626 | 298/594 (49%) | 318 |
| CG11594 | 10727290 | 7292419 | Hypothetical protein with FGGY carbohydrate kinase domain | Q96C11 | 286/442 (64%) | 402 |
| S6kII | 10803726 | 7295638 | Ribosomal protein S6 kinase alpha 3/Insulin-stimulated protein kinase 1 (ISPK-1). | P51812 | 520/744 (69%) | 803 |

(continued)

| *Drosophila* Gene | | | Human homologue(s) | | BLAST results | |
|---|---|---|---|---|---|---|
| **Gene Name** | **Nucleotide sequence gi number** | **Protein sequence gi numbers** | **Gene Name** | **SWISS-PROT reference** | **Similarity** | **Score** |
| CG11714 | 10727982 | 7294716 | Hypothetical protein | Q9HAB2 | 82/207 (39%) | 56 |
| | | | Speckle-type POZ protein [SPOP]/BTB domain protein (Fragment) [BDPL] | O43791 | 61/137 (44%) | 54 |
| CG3534 | 7300193 | 7300206 | Xylulokinase [XYLB] | 075191 | 299/489 (60%) | 395. |
| CG7335 | 10727803 | 7293697 | Ketohexokinase (EC 2.7.1.3) (Hepatic fructokinase) [KHK] | P50053 | 145/330 (43%) | 102 |
| CG11275 | 7291355 | 7291386 | Hypothetical protein | Q9HAB2 | 83/181 (45%) | 71 |
| | | | Speckle-type POZ protein [SPOP]/BTB domain protein (Fragment) [BDPL] | O43791 | 58/93 (61%) | 60 |
| CG16726 | 10728019 | 7294899 | Thyrotropin-releasing hormone receptor (TRH-R) (Thyroliberin receptor) [TRHR]. | P34981 | 81/166 (48%) | 75 |
| CG7514 | 10727313 | 7292529 | Mitochondrial 2-oxoglutarate/malate carrier protein | Q02978 | 199/296 (66%) | 301 |
| CG17283 | 7300241 | 7300253 | Cathepsin E [Precursor] | P14091 | 178/332 (53%) | 218 |
| BcDNA:GH07 626 | 10727365 | 7295848 | Fatty acid synthase (EC 2.3.1.85) | Q16702 | 743/1140 (65%) | 1077 |
| CG16752 | 10728478 | 7290587 | Putative G-protein coupled receptor [GPCR] | Q8TDU8 | 66/144 (45%) | 49 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| Rpt1 | 10727757 | 7304183 | MSS1 protein-26S protease regulatory subunit 7. | P35998 | 391/433 (89%) | 743 |
| Wts | 7301969 | 7301980 | Large tumor suppressor 1 [LATS1] | O95835 | 362/428 (83%) | 630 |
| | | | Large tumor suppressor 2 (Fragment) [HSLATS2] | Q9P2X1 | 346/414 (83%) | 604 |
| CG1582 | 7292554 | 7292573 | Putative DEAH-box RNA/DNA helicase | AAM7354 7 | 584/866 (66%) | 776 |
| CG12289 | 10727982 | 7294728 | Ketohexokinase (EC 2.7.1.3) (Hepatic fructokinase) [KHK] | P50053 | 141/300 (49%) | 147 |
| Pepck | 10727469 | 7302595 | Phosphoenolpyruvate (EC 4.1.1.32) carboxykinase, mitochondrial precursor [GTP] (Phosphoenolpyruvate carboxylase) (PEPCK-M) [PCK2] | Q16822 | 453/616 (73%) | 789 |
| CG5665 | 10726906 | 7296289 | Lipoprotein lipase precursor (EC 3.1.1.34) (LPL) [LPL] | P06858 | 119/259 (45%) | 105 |
| CG7285 | 10727839 | 7293895 | Somatostatin receptor 2B [SSTR2] | Q96TF2 | 174/335 (51%) | 201 |
| Bt | 10726313 | 10726323 | Titin | Q8WZ42 | 2478/577 2 (42%) | 1796 |

(continued)

| *Drosophila* Gene | | | Human homologue(s) | | BLAST results | |
|---|---|---|---|---|---|---|
| Gene Name | Nucleotide sequence gi number | Protein sequence gi numbers | Gene Name | SWISS-PROT reference | Similarity | Score |
| CG8795 | 10726505 | 7299748 | Neuromedin U receptor-type 2 G protein-coupled receptor TGR-1 | Q9GZQ4 | 167/302 (55%) | 177 |
| CG10967 | 10727955 | 7294537 | Serine/threonine-protein kinase ULK1 (EC 2.7.1.-) (Unc-51-like kinase 1) [ULK1] | O75385 | 255/504 (50%) | 327 |
| CG3809 | 10726480 | 7299571 | Adenosine kinase (EC 2.7.1.20) (AK) (Adenosine 5'-phosphotransferase) [ADK] | P55263 | 203/343 (58%) | 251 |
| Ack | 10727290 | 10727294 | Activated p21cdc42Hs kinase [ACK1] | Q07912 | 309/438 (69%) | 489 |
| Abl | 10727878 | 7294076 | Proto-oncogene tyrosine-protein kinase ABL1 (EC 2.7.1.112) (p150) (c-ABL) [ABL1] | P00519 | 407/470 (86%) | 742 |
| CG7362 | 10726534 | 7299922 | Pyruvate kinase, M2 isozyme (EC 2.7.1.40) [PKM2] | P14786 | 166/273 (59%) | 214 |
| Cyp9f2 | 7299572 | 7299618 | Cytochrome P450 3A4 (EC 1.14.14.1) (CYPIIIA4) (Nifedipine oxidase) (NF-25) (P450-PCN1) [CYP3A4] | P08684 | 282/505 (55%) | 270 |

**1) SYNTHESIS OF D.MEL-2 GENOMIC DNA AND cDNA**

i) Genomic DNA

D.Mel-2 cells from an established culture were grown in a 500 ml Erlenmeyer flask in *Drosophila*-SFM/ glutamine/Pen-Strep at 28°C until the culture reached $2x10^7$ cells/ml.

Cells were pelleted and washed twice with 50 ml PBS, then the pellet resuspended in 1 volume (10 ml) digestion buffer (100 mM NaCl, 10 mM Tris pH8, 25 mM EDTA pH8, 0.5% SDS, 0.1 mg/ml proteinase K) and incubated at 50°C for 15 hrs (in Hybaid rotisserie). DNA was extracted 3 times with an equal volume of Phenol/Chloroform and twice with an equal volume of Chloroform.

DNA was then precipitated by adding 1/2 volume of 7.5M ammonium acetate and 2 volumes of 97% ethanol, incubating at -20°C for 15 minutes, then centrifuging at 10,000 rpm, 4°C for 20 minutes. The pellet was washed with 70% ethanol, allowed to dry, then dissolved in 12 ml 10 mM Tris (pH 8.5).

For sufficient genomic DNA to amplify all the *Drosophila* genes requiring a genomic DNA template in the PCR reaction, this procedure was repeated a further 7 times, pooling the genomic DNA and the concentration assessed by measuring A260/A280 values.

ii) cDNA

D.Mel-2 total RNA was prepared as follows:

Cells were pelleted from 50 ml of a DMEL-2 culture at $5x10^6$ cells/ml and washed twice with 50 ml PBS, then resuspended in 4 ml denaturing solution (4 M guanidine thiocyanate, 25 mM sodium citrate, 0.5% N-laurylsarcosine (Sarkosyl), 0.1M 2-mercaptoethanol) in a corex tube.

400 μl 2 M sodium acetate pH 4.0 was added and mixed by inversion before adding 4 ml water-saturated phenol followed by 800 μl 49:1 chloroform/isoamylalcohol, then the suspension incubated on ice for 15 mins. The mixture was then spun in a centrifuge at 10,000 rpm for 30 mins and the aqueous phase transferred to a fresh tube. The RNA was precipitated by adding 4 ml isopropanol and incubating at 20°C for 20 mins. The RNA was centrifuged at 10,000 rpm for 30 minutes at 4°C to pellet the RNA and the pellet dissolved in 1200 μl denaturing solution. The RNA was precipitated a second time by adding 1200 μl isopropanol and incubating at -20°C for 20 mins. The mixture was then centrifuged at 10,000 rpm for 30 minutes at 4°C to pellet the RNA which was then washed with 1 ml 75% ethanol, incubating at room temperature for 10 mins, then centrifuging at 4°C for 5 mins. The supernatant was removed and the pellet allowed to dry before resuspending in 800 μl RNase- and DNase-free water. The concentration of the total RNA preparation was assessed from $A_{260}/A_{280}$ values.

D.Mel-2 cDNA was prepared using SuperScript™ First-strand synthesis system for RT-PCR (Invitrogen Ltd, 3 Fountain Drive, Inchinnan Business Park, Paisley PA4 9RF, UK).

To prepare sufficient cDNA to amplify all the *Drosophila* genes requiring a cDNA template in the PCR reaction, 96 50 μl cDNA preparations reactions in a 96-well plate were carried out as follows:

Reaction Mix (1) (800 μg D.Mel-2 RNA, 250 μl 10 mM dNTP mixture, 250 μl 500 μg/ml oligo dT primer, RNase- and DNase-free water to a final volume of 2.6 ml) was prepared and 26μl was aliquoted into each well of a 96-well plate, then incubated at 65°C for 5 minutes.

Reaction Mix (2) was prepared (500 μl 10x RT buffer, 1000 μl 25 mM $MgCl_2$, 500 μl 0.1 M DTT, 250 μl RNase OUT and 250 μl SuperScript™ II RT) was prewarmed to 42°C for 2 minutes, then 25 μl added to each well of the 96-well plate containing the 26 μl Reaction Mix (1).

The plate was heat sealed and incubated at 42°C for 1 hour, then at 70°C for 15 minutes on PTC-225 thermocycler (MJ Research, 590 Lincoln Street, Waltham, MA 02451-1003, USA). The reactions were placed on ice for 5 minutes, then 2.5 μl RNase H added to each well and incubated at 37°C for 20 minutes. The reactions were pooled, mixed and stored as 200 μl aliquots at -20°C.

2) **GENERATING *DROSOPHILA* GENE SPECIFIC dsRNA**

i) Designing and ordering RNAi primers.

Primers for the amplification of specific sequences of groups of *Drosophila* genes to be used as templates for the preparation of dsRNA were designed using PhilaSys primer design program (PhilaSysAmis Software Pvt. Ltd 120-1A Elephant Rock Road, 3 Brock Jaayenagar, Bangalore 560011, India). This program is designed to identify suitable primers for each of the *Drosophila* genes in the NCBI database, tagging a T7 RNA polymerase binding site sequence (TAATACGACTCACTATAGGGAGA) to the 5' end of each primer.
The following parameters were used in the primer design process:

Length of final PCR product = between 500 to 550 base pairs

Length of primer = 25-35 base pairs

Primer melting temperature = from 50.0 to 80.0°C

Minimum %GC = 35.0

Maximum permitted length of palindromes = 8 base pairs

Maximum permitted value of free energy of haripin loops = -1.0 kcal/mol

Minimum permitted value of primer-primer duplex free energy =-15.0 kcal/mol

Minimum permitted value for primer-primer 3' end duplex free energy = -3.0 kcal/mol

Maximum difference in the melting temperatures between primer pairs = 5°C

Maximum value for the difference in the %GC between the primers = 10.0

Primer concentrations = 250 pMol

Salt concentration = 50 mM

Primer pairs amplifying an intra-exon sequence were tagged as genomic (indicating that genomic DNA could be used in the PCR reaction) while sequences spanning 2 or more exons were tagged as cDNA (indicating a requirement for cDNA template for PCR amplification of the gene specific sequence).
Where no suitable primer pair for a gene was identified by the primer design program, the parameters were modified and the search process repeated. The minimum parameters settings were as follows:

Length of the final PCR product = between 150 to 200 base pairs

Length of the primer = 25-35 base pairs

Primer melting temperature = from 50.0 to 65.0°C

Minimum %GC of the primers = 30.0

Maximum permitted length of palindromes = 10 base pairs Maximum permitted value of free energy of hairpin loops = -2.5 kcal/mol

Minimum permitted value of primer-primer duplex free energy = -10.0 kcal/mol

Minimum permitted value for primer-primer 3' end duplex free energy = -5.0 kcal/mol

Maximum difference in the melting temperatures between primer pairs = 8°C

Maximum value for the difference in the %GC between the primers = 15.0

Primer concentrations = 250 pMol

Salt concentration = 50 mM

Primers (Table 2) were ordered from MWG (MWG Biotech (UK) Ltd, Mill Court, Featherstone Road, Wolverton Mill South, Milton Keynes, MK12 5RD, UK) pre-mixed at a concentration of 50 μM in 96 well Thermosprint plates.

ii) The primers were then used to synthesis *Drosophila* Gene Specific PCR products for dsRNA production.
The details of the primers for each plate were imported into the MWG AG Biotech RoboSeq 4204SE samples database.
The PCR reactions were aliquoted in a 96-well plate format using the RoboSeq 4204SE following the manufacturer's instructions. Each reaction comprised of 3 μl 17 μM gene specific primer pair, 2 μl D.Mel-2 genomic DNA or cDNA (2 μg/ml) as appropriate and 45 μl 1.1x thermo-start PCR master mix with 2.5 mM $MgCl_2$ (ABgene House, Blenheim Road, Epsom, Surrey KT19 9AP, UK) following the manufacturer's instructions. The RoboSeq 4204SE assigned barcode for each plate was recorded and the plates were stored at -80°C.
Once the RoboSeq 4204SE completed the PCR reaction set up, the plates were sealed and transferred to the PTC-225 thermocycler, running the programme THERMOPC, with steps 2 and 3 repeated 35 times:

1: 95°C 00:15

2: 95°C 00:30

3: 55°C 00:45

4: 72°C 01:00

5: 72°C 05:00

6: 4°C ∞

The PCR products were purified using the RoboSeq 4204SE together with Millipore's Montage™ $PCR_{96}$ Cleanup Kit (Millipore (U.K.) Ltd, Units 3&5, The Courtyards, Hatters Lane, Watford, WD18 8YH, UK).
The RoboSeq 4204SE assigned barcode for each plate was again recorded and the purified PCR product plates were stored at -20°C.
Each PCR product plate was thawed and sequences verified by running a sequencing reaction using ordered arrayed sequencing primers corresponding to the forward primers used in the original amplification reactions, but without the 5' T7 RNA polymerase binding site sequence. Sequencing reactions were performed by Lark Technologies, Inc. (Radwinter Road, Saffron Walden, Essex, CB11 3HY, UK).
The resulting sequences were verified to ensure that they corresponded to the sequences of the genes associated with each of the test wells, by BLAST searching the NCBI database.

iii) Gene specific PCR products were used as templates in *in vitro* transcription reactions to produce gene specific dsRNA.
Reactions were set up in a 96-well plate format using the RoboSeq 4204SE, with each reaction comprised of 8 μl gene specific PCR product, 6 μl 3.3x T7 RNA polymerase buffer (130.7 mM HEPES (pH 8.1), 16.3 mM DTT, 13.1% PEG 8000, 0.03% Triton X-100, 65.3 mM Mg(OAc)), 4 μl 25mM rNTP mix, 0.5 μl 50 U/μl yeast inorganic pyrophosphatase, 1 μl 20 U/μl Rnasin, 0.5 μl 80 U/μl T7 RNA polymerase. The reactions were incubated for 4 hours at 37°C on the PTC-225 thermocycler.
Transcribed RNA was treated with DNase I (2U/μl) at 37°C for 15 mins and diluted by the addition of 80 μl RNase-free Milli-Q water again with the aid of the RoboSeq 4204SE. To anneal the RNA, the samples were heated to 95°C for 10 mins and then cooled slowly to room temperature overnight.
The RoboSeq 4204SE assigned barcode for each plate was recorded and the dsRNA plates were stored at -20°C.

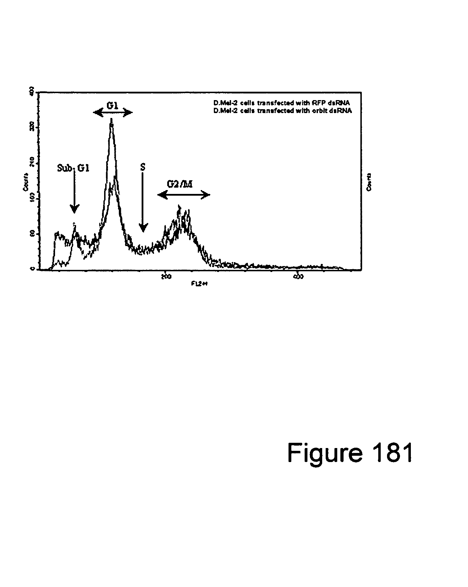

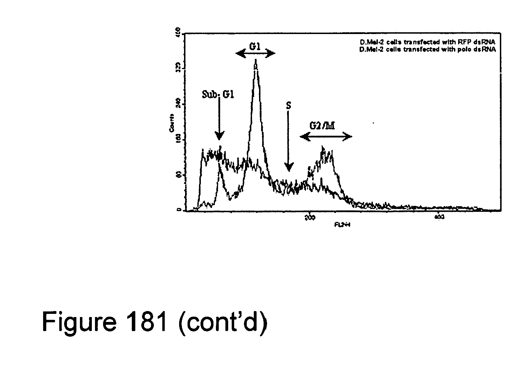

iv) Synthesis of control sequences from Red Fluorescent Protein (RFP) (Matz et al., 1999, Nat Biotechnol. 17: 969-973), *Drosophila* orbit and *Drosophila* polo dsRNA. RFP dsRNA was used as a negative control throughout, due to this gene being absent from D.Mel-2 cells. dsRNAs targeting 2 well established *Drosophila* cell cycle genes, orbit (Inoue et al., 2000, J Cell Biol. 149:153-166, Maiato et al., 2002, J Cell Biol. 157:749-760.) and polo (Sunkel and Glover, 1988, J Cell Sci. 89:25-38, Fenton and Glover, 1993, Nature 363:637-640, Carmena et al., 1998, J Cell Biol. 143:659-671), were used as positive controls throughout, as experiments had shown that RNAi of orbit or polo gene expression has a significant affect on cell cycle progression (See Figure 181 of FACS profiles for D.Mel-2 cells).

These control dsRNAs were prepared in 96 reactions in a 96-well plate format essentially as before, with some minor differences:

For PCR amplification of RFP gene specific sequence 0.1 μl 0.5 mg/ml 716 bp RFP sequence cloned into pGEM-T Easy vector (Invitrogen) together with primers RFP-1 and RFP-2 as follows:

RFP-1
TAATACGACTCACTATAGGGCGAATTGGGCCCGACGT

RFP-2
TAATACGACTCACTATAGGGCGATGCAGGCGGCCGCGAATTCACTAGT

polo specific sequence was amplified from D.Mel-2 cDNA using primers RNAi7 and RNAi8:

RNAi7
GAATTAATACGACTCACTATAGGGAGAGGCCGCGAAGCCCGAGGATAA GAGCA

RNAi8
GAATTAATACGACTCACTATAGGGAGAGATGACCATATCCGCCGCCGG TTTCCTT

orbit specific sequence was amplified from D.Mel-2 genomic DNA using primers RNAi192 and RNAi193:

RNAi192
TAATACGACTCACTATAGGGAGACCCGCATTGGCCGAACACCTGGAAC C)

RNAi193
TAATACGACTCACTATAGGGAGAACGTCGAGACCCCGCACCTGTAGAG T

The 96 dsRNA preparations for polo, orbit or RFP were pooled and the concentrations assessed from $A_{260}/A_{280}$ values. The samples were aliquoted and stored at - 20°C.

v) The quality and concentration of dsRNA in the samples was assessed by agarose gel electrophoresis and by fluorometry using PicoGreen reagent (Molecular Probes, PoortGebouw, Rijnsburgerweg 10, 2333 AA Leiden, The Netherlands) and a KC4 fluorometer (Bio-Tek® Instruments, Inc., Winooski, Vermont) linked to the RoboSeq 4204SE, following manufacturers' recommendations. The standard curve used to assess dsRNA concentrations was generated using orbit dsRNA prepared essentially as above, with the exception that the dsRNA (200 μg) was treated with 20 μg/ml RNaseA in the presence of 0.3 M NaCl for 30 minutes at 30°C, to remove ssRNA. The reaction was terminated with 0.1 mg/ml proteinase K and the dsRNA purified by extracting once with phenol/chloroform, once with chloroform and precipitating with 0.1 volume NaOAc (pH5.2) and 2.5 volumes absolute ethanol (-20°C). Following centrifuging at 13000rpm for 30minutes at 4°C, the dsRNA pellet was washed with 70% EtOH (-20°C), and resuspended in 200 μl RNase-free, DNase-free Milli-Q $H_2O$. The concentration of this standard dsRNA was assessed using $A_{260}/A_{280}$ readings.

vi) Each *Drosophila* gene specific dsRNA was diluted and arrayed into 3 wells of a 96-well Packard Viewplate, using 1 μg dsRNA per well, ready for transfection.

**3) RNA INTERFERENCE IN *DROSOPHILA* D.MEL-2 CELLS**

i) D.Mel-2 cells were cultured as follows ready for transfection with dsRNA.
A cryovial containing 1 ml cryopreserved D.Mel-2 cells (Passage #8) in 10% DMSO, *Drosophila*-SFM (Gibco #15240), 1% 200 mM L-glutamine (Gibco #25030) and 1% Penicllin/Streptomycin Solution (Gibco #15140) was rapidly thawed and the entire contents transferred into a 25 $cm^2$ tissue culture flask containing 5 ml *Drosophila*-SFM/glutamine/Pen-Strep. The cells were grown at 28°C for 4-5 days until they approached confluency, when they were transferred to a 75 $cm^2$ tissue culture flask containing 10 ml *Drosophila*-SFM/glutamine/Pen-Strep. The cells were again grown at 28°C for 3-4 days, until they approached confluency. Cells were split 1:10 and into fresh medium every 3-4 days with the date and passage number recorded on the flask. After thawing, the D.Mel-2 cells were ready for transfection following 3 passages.
ii) For each new batch of D.Mel-2 cells, the efficiency of dsRNA transfection of the cells was assessed, using fluorescently labelled dsRNA as follows.

Using 8 μl RFP, *Drosophila* polo or *Drosophila* orbit specific PCR products, including a 5' T7 RNA polymerase binding site on each strand, dsRNA was synthesised using amino-allyl substituted UTP, setting up 3 reactions with each template. Each reaction comprised of 6.12 μl 3.27x T7 RNA polymerase buffer (130.7 mM HEPES (PH 8.1), 16.3 mM DTT, 13.1% PEG 8000, 0.03% Triton X-100, 65.3 mM Mg(OAc)), 1 μl 100mM ATP, 1 μl 100mM CTP, 1 μl 100mM GTP, 1 μl 100mM amino-allyl-UTP (Sigma A5660), 0.5 μl 50 U/μl yeast inorganic pyrophosphatase, 1 μl 20 U/μl Rnasin and 0.5 μl 80 U/μl T7 RNA polymerase.

Unlabelled RFP, *Drosophila* polo or *Drosophila* orbit dsRNA was also synthesised in the same way, only using 1 μl 100mM UTP in each reaction instead of the amino-allyl substituted UTP.

The reactions were incubated at 37°C for 4 hours in the PTC-225 thermocycler, and then treated with DNase I (2 U/μl) at 37°C for 15 mins and annealed by heating to 95°C for 10 minutes and then cooling to 4°C over a 15 hour period. The 3 equivalent reactions containing labelled or unlabelled RFP, *Drosophila* polo or *Drosophila* orbit dsRNA were pooled and 140 μl RNase- and DNase-free water added to each.

18 BioRad P30 MicroBiospin columns were buffer exchanged to 100mM $NaHCO_3$ (pH7.5) by washing the columns through 3 times with 0.5 ml buffer per wash. Each of the pooled dsRNA preparations were divided into 3 and passed through a column (~65μl per column) following manufacturer's recommendations, then re-pooled. To each of the purified dsRNA preparations, 33 μl AlexaFluor 594-succinimidyl ester (Molecular Probes A-20004) resuspended at 10mg/ml in DMSO, and 8 μl 500mM $NaHCO_3$ (pH7.5) was added. The dsRNA and label were incubated at room temperature in the dark overnight.

Once again, each of the dsRNA preparations was purifed by splitting them in three and passing each through a BioRad P30 MicroBiospin column (10mM Tris pH7.4). The 3 purified dsRNA preparations were re-pooled and precipitated by the addition of 0.1 volumes NaOAc (pH5.2) and 2.5 volumes absolute ethanol (-20°C). The dsRNA was centrifuged at 13000rpm for 30minutes at 4°C and the supernatant removed. The pellet was washed twice with 70% EtOH (-20°C), then allowed to air dry, and resuspended in 200μl 10mM Tris (pH8.0).

The concentration and quality of dsRNA in the samples was assessed from $A_{260}/A_{280}$ readings and by agarose gel electrophoresis. The concentrations of the dsRNA preparations were adjusted to 67 ng/μl and 15 μl of each aliquoted into 16 wells of a Packard viewplate:

- Labeled polo dsRNA in columns 1 and 2
- Labeled orbit dsRNA in columns 3 and 4
- Labeled RFP dsRNA in columns 5 and 6
- Unlabeled polo dsRNA in columns 7 and 8
- Unlabeled orbit dsRNA in columns 9 and 10
- Unlabeled RFP dsRNA in columns 11 and 12

To each well 35 μl of logarithmically growing D.Mel-2 cells diluted to $2.3x10^5$ cells/ml in fresh *Drosophila*-SFM/glutamine/Pen-Strep pre-warmed to 28°C was added. The cells were incubated with the dsRNA (60nM) in a humid chamber at 28°C for 1 hr, then 100 μl *Drosophila*-SFM/glutamine/Pen-Strep pre-warmed to 28°C was added and the cells containing the dsRNA returned to the humid chamber at 28°C for 72 hrs. The medium was removed and the cells incubated with 100 μl Fixation Solution (3.7% formaldehyde, 1.33mM $CaCl_2$, 2.69mM KCl, 1.47mM $KH_2PO_4$, 0.52mM $MgCl_2$-$6H_20$, 137mM NaCl, 8.50mM $Na_2HPO_4$-$7H_2O$) pre-warmed to 28°C 15 minutes. The Fixation Solution was removed and the cells washed with 100 μl Wash Buffer (1.33mM $CaCl_2$, 2.69mM KCl, 1.47mM $KH_2PO_4$, 0.52mM $MgCl_2$-$6H_20$, 137mM NaCl, 8.50mM $Na_2HPO_4$-$7H_2O$). The cells were treated with 100 μl Permeabilisation Buffer (30.8mM NaCl, 0.31mM $KH_2PO_4$, 0.57mM $Na_2HPO_4$-$7H_2O$, 0.02% Triton X-100) for 15 minutes and once more with 100 μl Wash Buffer, prior to the addition of 50 μl Staining Solution (1 μg/ml Hoechst 33258, 1.33mM $CaCl_2$, 2.69mM KCl, 1.47mM $KH_2PO_4$, 0.52mM $MgCl_2$-$6H_20$, 137mM NaCl, 8.50mM $Na_2HPO_4$-$7H_2O$) per well. The cells were incubated with the Staining solution for 1 hour protected from the light. The Staining Solution was removed and the cells washed twice with 100 μl Wash Buffer. 200 μL Wash Buffer containing 0.02% sodium azide was finally added to the cells, the plates were sealed and the transfection efficiency analysed using the Cellomics ArrayScan HCS System (Cellomics Europe, St. Mary's Court, The Broadway, Old Amersham, Bucks, HP7 OUT, UK), with the 10x objective and the QuadBGRFR filter set following manufacturer's recommendations.

iii) Transfection of D.Mel-2 cells with gene specific dsRNA was carried out as for fluorescently labelled control dsRNA detailed above.

Following incubation of D.Mel-2 cells with the dsRNA for 72hrs at 28°C, the cells were fixed and stained using the Cellomics Mitotic Index Hitkit, essentially following the manufacturer's recommendations, with the exception that the Fixation solution was pre-warmed to 28°C and only half the specified volumes of primary and secondary antibody were added to the Primary Antibody Solution and to the Staining Solution, respectively. The kit provides a fixed endpoint assay based on immunofluorescence detection and localisation of a phosphorylated core histone protein that is abundant in the nuclei of dividing cells, to enable a determination of the Mitotic Index. The Mitotic Index (MI) represents the fraction of cells within a population under-going cell division and is a valuable means of characterising cell proliferation. The calculated value for mitotic index is often higher in cancerous cells due to uncontrolled cell proliferation.
The Mitotic Indices were analysed following manufacturer's recommendations, using the Cellomics ArrayScan HCS System, with the 10x objective and the DualBGIp filter set.
2,500 cells were assessed for each well as confirmed, by viewing cell counts for the wells of each plate within Data Viewer. The average MI value, the standard deviation and Ttest scores (assuming a two-tailed distribution with two samples of equal variance) for each gene, compared to the RFP control on the plate containing the gene was calculated from Mitotic Index data generated by the Arrayscan. The Ttest scores for each gene, compared to the water control on the plate containing the gene was also calculated, again assuming a two-tailed distribution with two samples of equal variance and the two Ttest scores for each gene combined to give an aggregate Ttest score. The Mitotic Index for D.Mel-2 cells transfected with each gene specific dsRNA was also expressed as a percentage of the mitotic index for cells transfected with the RFP control dsRNA for that plate. The percentage change in MI for each gene, relative to the RFP control was also calculated where:

$$\text{change in \%MI of gene X} = \left(\left(\frac{\text{MI for cells transfected with gene X specific dsRNA}}{\text{MI for cells transfected with RFP specific dsRNA}}\right) \times 100\right) - 100$$

and from this the absolute change in MI was identified.
Genes with an aggregate Ttest score less than 0.1 were viewed as significant and were ranked according to their absolute change in MI relative to the RFP control for further analysis (See Table 2 below).

**Table 2**

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| CG3632 | TAATACGACTCACTATAGGGAGAAGGTGCGAGATCTGTTCCAGCTGATT<br>TAATACGACTCACTATAGGGAGAAGGGCACAAGCAATTTGGGTGGATAC | 0.0740 | 126.6 |
| Pp1-87B | TAATACGACTCACTATAGGGAGATCCGCCGGAATCGAATTACCTGTTC<br>TAATACGACTCACTATAGGGAGAACTTGATGGGCTCGGCAGATGAGAT | 0.0048 | 107.8 |
| CG3524 | TAATACGACTCACTATAGGGAGAGGCAAAGAAGCACCAACAGAAGTTA<br>TAATACGACTCACTATAGGGAGATCTGGGCTAGCATATTCACAGAGTA | 0.0914 | 82.5 |
| CG9311 | TAATACGACTCACTATAGGGAGACTACCAAACAGCGGCAGGTTATTCAG<br>TAATACGACTCACTATAGGGAGAGTTGCTGCTGATATGCCAGTGGTTGT | 0.0980 | 74.6 |
| CG9092 | TAATACGACTCACTATAGGGAGATTAGCAAGGGTTGGGGCAGCACACT<br>TAATACGACTCACTATAGGGAGACACTGCAGGTAGATCCTCGCCAGTT | 0.0212 | 61.4 |
| Arr1 | TAATACGACTCACTATAGGGAGACAATTTCCGATATGGGCGCGAGGAC<br>TAATACGACTCACTATAGGGAGACTTCACCACCTTGTTGGAGTTGTTG | 0.0402 | -54.4 |
| CG9150 | TAATACGACTCACTATAGGGAGAGGTGGCAGAACAAACTGGCTGTGGT<br>TAATACGACTCACTATAGGGAGAATGGCGGTGATGGCAAACTTGGTGG | 0.0843 | -53.0 |
| CG11102 | TAATACGACTCACTATAGGGAGAATGCTTTGGCTGCTGTTGCGGTACAAGT<br>TAATACGACTCACTATAGGGAGAGCGGAGAGTAACTGAAGCACTGGAG | 0.0049 | 51.0 |
| Smr | TAATACGACTCACTATAGGGAGACCGCCGGAGACCATAATCTACAATG<br>TAATACGACTCACTATAGGGAGACGGGCACTGGTACTGGTTGTAGATA | 0.0606 | 50.3 |
| CG8045 | TAATACGACTCACTATAGGGAGACTGCTGTCGGTGGCGTACAAGAATG<br>TAATACGACTCACTATAGGGAGACTCAGTGTATCCAACTCGGCAATGG | 0.0065 | -49.5 |
| CG10420 | TAATACGACTCACTATAGGGAGAGGAGTCTATACGGCGGGTCAAGGAG<br>TAATACGACTCACTATAGGGAGAGAGCCTGTGTACCCGAGGTGGAAAG | 0.0509 | 49.4 |
| Hsc70-2 | TAATACGACTCACTATAGGGAGAGAAGTTCGACGACAAGAAGATACAG<br>TAATACGACTCACTATAGGGAGACGCTTGAATTCCTCGGCAAAATGAT | 0.0116 | -47.4 |
| CG10805 | TAATACGACTCACTATAGGGAGAATTAATCAGTAATCGCAAGCTGGTG<br>TAATACGACTCACTATAGGGAGATAGCTTCTGTTCAAGTTGGACATAG | 0.0746 | 46.1 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| elF-4a | TAATACGACTCACTATAGGGAGACTGCCACCTTCTCGATTGCTATCCT<br>TAATACGACTCACTATAGGGAGACTCCTGCTTCACGTTGACGTAAAAC | 0.0263 | -45.7 |
| ACXA | TAATACGACTCACTATAGGGAGATCCAACTGGCCTCTTAATACCTTAT<br>TAATACGACTCACTATAGGGAGACATAAAGGATACTGACATCGTTGTG | 0.0166 | 44.8 |
| CG15117 | TAATACGACTCACTATAGGGAGATGTACGATAAGGATGGCATATTGGT<br>TAATACGACTCACTATAGGGAGAGAAAATCGAAGCCAAGAGCATTAGT | 0.0690 | 44.1 |
| BG:DS01759.2 | TAATACGACTCACTATAGGGAGAGAACTCAAAACGATGCTGGTCAAGT<br>TAATACGACTCACTATAGGGAGAAAATCAATAGGTCCAGTAGATGGGG | 0.0521 | 43.6 |
| TepIII | TAATACGACTCACTATAGGGAGAGCGTTCATACGAACTGAGCGATGTG<br>TAATACGACTCACTATAGGGAGAGAGTAGGGCAACTCGGTGCTTATGT | 0.0165 | -41.4 |
| Hsc70-4 | TAATACGACTCACTATAGGGAGACTATCTGGGCAAGACTGTGACCAAC<br>TAATACGACTCACTATAGGGAGAGGCACGAGTAATCGAGGTGTAGAAG | 0.0711 | -40.9 |
| CG7069 | TAATACGACTCACTATAGGGAGACCAAGAAGGAGATGGCAGACAAGAG<br>TAATACGACTCACTATAGGGAGACAATCGATTTCTGGGCTAGGGGAAC | 0.0292 | -40.3 |
| ACXE | TAATACGACTCACTATAGGGAGATCCACTTCATCAGCGGTGCAGTCCT<br>TAATACGACTCACTATAGGGAGAGAGATCGTGCAGGACCTTCACCAAC | 0.0433 | 39.2 |
| EG:52C10.5 | TAATACGACTCACTATAGGGAGAGCAGAACTTCGATCTAAGCGACCAA<br>TAATACGACTCACTATAGGGAGATCAGCTAGCTTCGACTGAATGTCTC | 0.0813 | -37.9 |
| gatA | TAATACGACTCACTATAGGGAGAAAGGTGGCCGATTTGCTGGAGTGTA<br>TAATACGACTCACTATAGGGAGAGAATCGGTATAGACACGGCGGGAAT | 0.0851 | 37.1 |
| CG17149 | TAATACGACTCACTATAGGGAGAAGAGGCCTGCTTTCCGGACATCAGT<br>TAATACGACTCACTATAGGGAGAGTGGACATGTCTGCTGGATGGGAAC | 0.0283 | 36.6 |
| CG2905 | TAATACGACTCACTATAGGGAGAGATAAAGTTCTTGCTACAGTGGAAA<br>TAATACGACTCACTATAGGGAGAAAAGGTAAAGCATTTGAATCAGGAG | 0.0314 | -35.8 |
| CG2336 | TAATACGACTCACTATAGGGAGATCAGTCTAGAAGAGGAGGTTCAATA<br>TAATACGACTCACTATAGGGAGACAACTATTCTCTTTGCCTGTAAGTG | 0.0419 | 35.7 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| TER94 | TAATACGACTCACTATAGGGAGAGGTCTGGAGAGCGTCAAGAAGGAAT<br>TAATACGACTCACTATAGGGAGACGGGCAGCGGAATATAGATCAACTG | 0.0623 | -35.6 |
| CG6313 | TAATACGACTCACTATAGGGAGACCGCAATCTAGCCAACAAGCTCTCA<br>TAATACGACTCACTATAGGGAGAGGCGCAGTACGTTATTAGCATCCAC | 0.0752 | -35.2 |
| aur | TAATACGACTCACTATAGGGAGAACGTGCGCATATATCTGATCTTGGA<br>TAATACGACTCACTATAGGGAGAATTAAGGACCAGCAGCTTGGAAATG | 0.0147 | 35.1 |
| Pk91C | TAATACGACTCACTATAGGGAGAGCGATAGCAAGATATCTGGTGTTTC<br>TAATACGACTCACTATAGGGAGAGCTTAGGTTGTCCACATTCTTCTCA | 0.0560 | 34.9 |
| Top2 | TAATACGACTCACTATAGGGAGAAGGTGGTTTCTACCGAGTGTTCAAA<br>TAATACGACTCACTATAGGGAGATCTGTCAACATCCACATGGACATAC | 0.0071 | 34.3 |
| alpha-Est1 | TAATACGACTCACTATAGGGAGATCCGAAATGGCCGCACAGGGTATTA<br>TAATACGACTCACTATAGGGAGAGATTGAAATGGGGCGAGTCCACATC | 0.0185 | 33.4 |
| Nrk | TAATACGACTCACTATAGGGAGAAGATCTACTAGTCGCTGTTAAGATG<br>TAATACGACTCACTATAGGGAGAAAGCGAGAACTTGTTGTACAGTATG | 0.0453 | 33.1 |
| otk | TAATACGACTCACTATAGGGAGACAAGCCGACAATTCAGTGGGACAAG<br>TAATACGACTCACTATAGGGAGACTGCAGGCTGTGTCATCGGATTTCT | 0.0506 | 33.1 |
| cad | TAATACGACTCACTATAGGGAGAGGCGGATAACTTCGTTCAGAATGTG<br>TAATACGACTCACTATAGGGAGAGATAGGCGGGCTTCTTCATCCAGTC | 0.0587 | 32.4 |
| rut | TAATACGACTCACTATAGGGAGAGCGCCAAAGTACGAGCCACCACGTTACA<br>TAATACGACTCACTATAGGGAGACATGTCGACGACCGGAGAGGTGGGA | 0.0343 | 30.7 |
| CG8002 | TAATACGACTCACTATAGGGAGAAGCTGCACATTGACGATACGGAGAG<br>TAATACGACTCACTATAGGGAGAATGCCGACAAGCTCTTGGTCACAGT | 0.0560 | 30.4 |
| CG10335 | TAATACGACTCACTATAGGGAGACACAATCTCATGTATCCGGTGTTCA<br>TAATACGACTCACTATAGGGAGAAATTGGATGTGAACTTGGCGGAGTA | 0.0678 | 30.3 |
| CG8070 | TAATACGACTCACTATAGGGAGAGGGTGCTTCTCTAAGGTAACAAAAG<br>TAATACGACTCACTATAGGGAGATTAGGATAGGCTCGATGATGTCTCC | 0.0493 | 30.2 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| CG7460 | TAATACGACTCACTATAGGGAGACCTTTAGATGCGTTCGATCCAACAA<br>TAATACGACTCACTATAGGGAGAATGACATGATCCGCTGTGATTACCT | 0.0568 | 29.3 |
| CG17735 | TAATACGACTCACTATAGGGAGACCTTCAGAGCTGTACGAACTTACCT<br>TAATACGACTCACTATAGGGAGAAGATGGACGGCGCTTTACTTGATTG | 0.0658 | -29.1 |
| Gycalpha99B | TAATACGACTCACTATAGGGAGAGCTCTACAAGGTGGACGTGAACATC<br>TAATACGACTCACTATAGGGAGAAGAGCAACGAATTGGACTCGGGACA | 0.0720 | 28.4 |
| CG13893 | TAATACGACTCACTATAGGGAGAACGATCAGAGTCAAAAGCACGGATGG<br>TAATACGACTCACTATAGGGAGATGACCTTAAAGTGCAGCTTCAACTT | 0.0943 | 28.3 |
| CG18176 | TAATACGACTCACTATAGGGAGAAAAAGGTTGTTCAGTGCTTTGAGAA<br>TAATACGACTCACTATAGGGAGAACATGACCAAACTTTTGCAGATAGT | 0.0751 | -28.3 |
| CG8858 | TAATACGACTCACTATAGGGAGAACATGAGCTGGAACCTGCTGGAAAA<br>TAATACGACTCACTATAGGGAGACTCAGCCGCTTGATCAGCATAAGAA | 0.0617 | 27.8 |
| Ac76E | TAATACGACTCACTATAGGGAGAGTGCCACCGAAACCAGCATACACCT<br>TAATACGACTCACTATAGGGAGATCCGCTGTTGGAGATGCCGCTCTCT | 0.0907 | 27.0 |
| CG17010 | TAATACGACTCACTATAGGGAGAACGTGGCTGGTGCGAATGTATTTCT<br>TAATACGACTCACTATAGGGAGATGCCGCACTGATATGATGTTCTGTG | 0.0034 | -26.0 |
| tkv | TAATACGACTCACTATAGGGAGAGAACCATTGCCAAGCAGATTCAGAT<br>TAATACGACTCACTATAGGGAGATGAATGACATCCAGTTCCGAGTTGT | 0.0863 | 25.8 |
| dnt | TAATACGACTCACTATAGGGAGAGACCGGCGATCAATGTGTCACACAG<br>TAATACGACTCACTATAGGGAGAACTGGAACTTTCCGTGGCAAGGAGG | 0.0886 | 25.6 |
| ACXD | TAATACGACTCACTATAGGGAGAATGTTTACCACGCCACCAGCCACAA<br>TAATACGACTCACTATAGGGAGAATGGTCCGGTTGTCGCCACCTTTTA | 0.0673 | 25.1 |
| Aats-ala-m | TAATACGACTCACTATAGGGAGACTATTGTGGTGGAGACACTTGGTGA<br>TAATACGACTCACTATAGGGAGAGGAGTAGGTGTACTTGTGACTGTTG | 0.0676 | 25.0 |
| gek | TAATACGACTCACTATAGGGAGAGCAACAAACACAGGAAAGGCTGAAG<br>TAATACGACTCACTATAGGGAGAGGATATGAGGTCCGATCTGGTTTGA | 0.0959 | -24.9 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| CG3216 | TAATACGACTCACTATAGGGAGATCTACCAAATCCTGCCGCGTCCTGT<br>TAATACGACTCACTATAGGGAGAGGTGGCCGAGGACACATGTATCTTG | 0.0999 | 24.7 |
| CG5653 | TAATACGACTCACTATAGGGAGAGTACCACGAATGTGATGGCGACAAG<br>TAATACGACTCACTATAGGGAGACCATCAACATTCGGGGCTGACAAGT | 0.0703 | 24.1 |
| CG17740 | TAATACGACTCACTATAGGGAGACGAGGCAGAGAACTTTGGTGACTAC<br>TAATACGACTCACTATAGGGAGAAGCATGCACTCATCCGCACAGAAGT | 0.0969 | -23.6 |
| Tepl | TAATACGACTCACTATAGGGAGAAATGGATGTGAAGGCGAAAGTATTA<br>TAATACGACTCACTATAGGGAGAGTATACTGGGCACAAAGTTAAACAT | 0.0572 | 23.4 |
| for (CT43154 & CT43158) | TAATACGACTCACTATAGGGAGACGTTCAGCAGAAGTGTGGTCAGGTC<br>TAATACGACTCACTATAGGGAGACCGTCCGCTGGCAGTTGTACAGGAT | 0.0213 | 22.5 |
| Ac13E | TAATACGACTCACTATAGGGAGAACTCAACCAGACGGCGATTAGTCAG<br>TAATACGACTCACTATAGGGAGAAAGCAGGAGAACAAGGTCACCCACA | 0.0992 | 21.3 |
| CG2667 | TAATACGACTCACTATAGGGAGATGCCGGAACTGCAGGGAATTGTGAT<br>TAATACGACTCACTATAGGGAGAGAGCTGACGGCTTCGATGAAGTTGA | 0.0180 | 21.2 |
| CG7842 | TAATACGACTCACTATAGGGAGAGGAGGGACCACGTGAGAAACTGAAT<br>TAATACGACTCACTATAGGGAGAACGGCGCTTTGCATTAGAGGAGTGT | 0.0397 | 21.2 |
| CG17486 | TAATACGACTCACTATAGGGAGAACGCACTTGGAAGAAATTCACTATT<br>TAATACGACTCACTATAGGGAGATAGAATACACAATTTTGCGTGTCTG | 0.0501 | 20.3 |
| CG6969 | TAATACGACTCACTATAGGGAGAACAGAAGATCCACCGGGGTGTGTAA<br>TAATACGACTCACTATAGGGAGACTGATTTTGCCGCTGCTCCAGATTG | 0.0226 | 20.2 |
| CG12262 | TAATACGACTCACTATAGGGAGAGGTTCATTGTGGAGCGCGACAGTCC<br>TAATACGACTCACTATAGGGAGACTCCACGGGATACTCGCTGTTGAAG | 0.0966 | 19.6 |
| fray | TAATACGACTCACTATAGGGAGAATTAAGCGCATCAACCTGGAGAAGT<br>TAATACGACTCACTATAGGGAGACCAAATGTCCGCCTTAAAGTCATAG | 0.0749 | 19.5 |
| CG6879 | TAATACGACTCACTATAGGGAGACCGGATACAACGTGACCTCACTGGA<br>TAATACGACTCACTATAGGGAGACACCTCTTCCGCCAATCTGGTAGGT | 0.0645 | 19.5 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| CG11594 | TAATACGACTCACTATAGGGAGAGAGGAATCTGTCACAGACTTTTGGA<br>TAATACGACTCACTATAGGGAGACGTTTAGGAAGTAGCCGGGAATAAT | 0.0076 | 19.0 |
| S6kII | TAATACGACTCACTATAGGGAGAATTTTGCCGCTGATTGGTGGAGTTT<br>TAATACGACTCACTATAGGGAGACAGCAGGAATAGGAGCTATACTATG | 0.0168 | 18.9 |
| CG11714 | TAATACGACTCACTATAGGGAGAGAGTTACTTCGTCTATTCCAGTGTG<br>TAATACGACTCACTATAGGGAGATACTTCTCAGCCAGGCTAAGGAAAT | 0.0346 | 18.4 |
| CG3534 | TAATACGACTCACTATAGGGAGACCACCACCAAACAGTGCCTAGAGAT<br>TAATACGACTCACTATAGGGAGACCTTCAAGCTCATCATTAGGGTGTC | 0.0877 | 17.9 |
| CG7335 | TAATACGACTCACTATAGGGAGAAGGAGCTCACCTACCAGCAGTTTGT<br>TAATACGACTCACTATAGGGAGACCACTAATTTGGGCGGAATCTGGGA | 0.0094 | 17.8 |
| CG11275 | TAATACGACTCACTATAGGGAGAAGCTGTACTACGCCGCTGAGAAGTA<br>TAATACGACTCACTATAGGGAGAACAGCGGAGTTTCTGCATCCACTGT | 0.0040 | 17.7 |
| CG16726 | TAATACGACTCACTATAGGGAGAGCGAATCCAATGTCACGGAATACAA<br>TAATACGACTCACTATAGGGAGATGGCAATGACTATATAGGGCAGACA | 0.0359 | 17.3 |
| CG7514 | TAATACGACTCACTATAGGGAGACCACTGTGTTGGCCAGCATGGGTAT<br>TAATACGACTCACTATAGGGAGAGTGTGCGGTCCTAAGCGACACAAAT | 0.0939 | 17.0 |
| CG17283 | TAATACGACTCACTATAGGGAGAAAATTAGGGCCAAGACCGAGTCAAT<br>TAATACGACTCACTATAGGGAGACGAAATTTGAGGTCACGAAAGTGGT | 0.0921 | 16.8 |
| BcDNA:GH07 626 | TAATACGACTCACTATAGGGAGAGGCCACCGAGCAGAACTTTAACTGG<br>TAATACGACTCACTATAGGGAGACGGCCAGCTTACCAGAGGTCAACAT | 0.0885 | 16.7 |
| CG16752 | TAATACGACTCACTATAGGGAGAGTATATTGCATTGCTGGCGTTTCTG<br>TAATACGACTCACTATAGGGAGATGCCGCAGTAAATGCCGAAGTTGAT | 0.0333 | 16.3 |
| Rpt1 | TAATACGACTCACTATAGGGAGATGAGCTGGTGCAAAAGTACGTGGGT<br>TAATACGACTCACTATAGGGAGAGGCTTCGAGGAAATCCTTCTCTGTG | 0.0418 | -15.8 |
| wts | TAATACGACTCACTATAGGGAGAAACAGCAACTGCAGGCCTTGAGGGT<br>TAATACGACTCACTATAGGGAGAATACGTGCGCTGGCGATACGACTTG | 0.0630 | 15.7 |

(continued)

| *Drosophila* Gene Name | Froward and reverse primer sequences (including T7 RNA polyerase binding site) | Mitotic Index Data | |
|---|---|---|---|
| | | Aggregate T Test Score | MI as % Change Relative to RFP |
| CG1582 | TAATACGACTCACTATAGGGAGATCAACTGCCAGCAAAGGAGAACTTG<br>TAATACGACTCACTATAGGGAGATAAGCTTGCGGCAGTACTTAGTGTC | 0.0531 | -15.7 |
| CG12289 | TAATACGACTCACTATAGGGAGACACCAAGTATATAGCCGAGTCCAGA<br>TAATACGACTCACTATAGGGAGATCAGTGGGTGCAATGGAGGACTTTT | 0.0349 | -14.6 |
| Pepck | TAATACGACTCACTATAGGGAGAGTTGCACAAGCTGCGCCAGGACAAT<br>TAATACGACTCACTATAGGGAGACACCACGTAAGCAGAGTCCGTCAGT | 0.0124 | -14.2 |
| CG5665 | TAATACGACTCACTATAGGGAGAATTCACTAGGAGCTCACATTATGGG<br>TAATACGACTCACTATAGGGAGACACGTTTTAAGCCTAGGATCAACAG | 0.0576 | 14.0 |
| CG7285 | TAATACGACTCACTATAGGGAGAACACGAACGAGAGCTTATATACCAC<br>TAATACGACTCACTATAGGGAGAAGACCACTTTGGCAATATGCAGAGT | 0.0930 | 13.6 |
| bt | TAATACGACTCACTATAGGGAGACGGACCACTTCAAATATCAGATGTG<br>TAATACGACTCACTATAGGGAGAGGAAACTCGGAATTTGTAGGTTTGG | 0.0177 | 13.4 |
| CG8795 | TAATACGACTCACTATAGGGAGAATGGCAGTGGCAATGGAACGACAAC<br>TAATACGACTCACTATAGGGAGAGGTTTGTGGCTGCCTTTGCGTCTGG | 0.0865 | 13.3 |
| CG10967 | TAATACGACTCACTATAGGGAGAAGCGCAAGAGCAGTGTGAGCAGTGA<br>TAATACGACTCACTATAGGGAGACGCCAGCACAAAGTTCAGCTTGGAC | 0.0498 | 12.4 |
| CG3809 | TAATACGACTCACTATAGGGAGACTTCTTTCTGGCCGTTTGTCCACCT<br>TAATACGACTCACTATAGGGAGAGCTTATCGATCTGAACACCGACCAC | 0.0959 | 11.8 |
| Ack | TAATACGACTCACTATAGGGAGATCTACTCGAATTTCAACCAGTCTCT<br>TAATACGACTCACTATAGGGAGATCATACCAAATACGATTCACCACAC | 0.0319 | 10.0 |
| Abl | TAATACGACTCACTATAGGGAGACACGGGCGATAGTCTGGAGCAGAGT<br>TAATACGACTCACTATAGGGAGACGGAATGGGGCTGGCCTTCGGATTT | 0.0449 | 9.2 |
| CG7362 | TAATACGACTCACTATAGGGAGACCGCGAATCCGATGGCATAATGGTG<br>TAATACGACTCACTATAGGGAGATAGGACACGGCGGCCTCATTTGACT | 0.0886 | -9.0 |
| Cyp9f2 | TAATACGACTCACTATAGGGAGAATGAAATACCGACAGGAGCACAATA<br>TAATACGACTCACTATAGGGAGACGAACTTCATAGGGTTCTCAAAGTA | 0.0438 | 6.4 |

**4) ANALYSIS OF THE CONCENTRATION-DEPENDENT EFFECT OF TRANSFECTED dsRNA UPON *DROSOPHILA* D.MEL-2 CELLS MITOTIC INDEX.**

To confirm the initial effect of gene specific dsRNA on the mitotic index of transfected D.Mel-2 cells and also to demonstrate a dosage dependant effect on MI the concentration dependent effect of dsRNA is tested.
*Drosophila* gene specific dsRNA ranging in concentration from 10 ng per well to 4 μg per well is transfected into D.Mel-2 cells and the Mitotic Index of the transfected cells assayed using the Cellomics Mitotic Index HitKit as described previously. Each Cellomics assay plate includes the range of gene specific dsRNA samples and also an equivalent range of RFP control dsRNA samples for comparison.
The resulting data is analysed essentially as before, except that for each gene a bar graph showing the Mitotic Index value for D.Mel-2 cells transfected with the gene specific dsRNA at each concentration and for cells transfected with the control dsRNA at each concentration is constructed. Error bars corresponding to +/-1 standard deviation are also included.

**5) HUMAN HOMOLOGUES**

An automated web query system was used to identify matching BLAST hits from SWISS-PROT for *Drosophila* genes where RNAi resulted in a significant change in mitotic index in D.Mel-2 cells.
The *Drosophila* gene name was used to find matching entries in SWISS-PROT, using the get-entries service ("/cgi-bin/get-entries") at the EXpert Protein Analysis SYstem world wide web site ("expasy.org"). The search used an exact string match in the gene name field and *"Drosophila"* in the organism field. One or more accession numbers from the SWISS-PROT and trEMBL datasets were returned by this search.
Each accession number was used to identify the corresponding full SWISS-PROT entry, which was returned by requesting the accession number from "/cgi-bin/hub" at expasy.org. Each entry was used as a blastp query against the SWISS-PROT database via the BLAST service at expasy.org ("/cgi-bin/blast.pl"). All blast hits were stored, but only those matching rat, mouse or human were included in the output file. The SWISS-PROT files for hits from the above organisms were requested from expasy.org ("/cgi-bin/hub").
Human homologues with best homology (>25% similarity and BLAST score>10) over the majority of the *Drosophila* protein were earmarked for further validation. In some cases, several human proteins were identified as homologues of the *Drosophila* gene.
The sequence references for the human homologues are set out in Table 1.

**6) VALIDATING HUMAN MITOTIC GENE FUNCTION BY RNAi**

The identified human gene homologues are initially validated by RNA interference of gene expression in U2OS cells, using FACS analysis to identify human genes required for normal cell cycle progression.

i) Designing and ordering Human gene specific siRNA
siRNA sequences for gene specific RNAi are identified using the siRNA FINDER at the Ambion.com site, in the "/techlib/misc/" directory, in the file "siRNA_finder.html". siRNAs are identified at the middle and 3' end of the open reading frame and with a GC content of between 42.9% and 52.4%. BLAST searches are performed with prospective siRNAs to verify that they are unique to the selected human target gene. Where an siRNA shares more than 81% identity with another human gene, the next adjacent siRNA is selected. For each selected sequence, 0.2 μmole of purified, annealed, duplex siRNA is ordered from Dharmacon (Dharmacon Research, Inc., 1376 Miners Drive, #101, Lafayette, CO 80026, USA). siRNAs are resuspended in RNase- and DNase-free water and used to prepare 20 mM stock solutions.
ii) Culturing Human cell lines for RNAi analysis
Human Hela and U2OS cell lines are cultured for transfection with siRNA in the following media: 500ml DMEM with L-glutamine, sodium pyruvate and pyridoxine (Gibco: #41966-029 (InVitrogen Corporation, Carlsbad, California, USA)), 50ml FBS (E.U. approved, Gibco; #10106-169), 5ml Pen/Strep 5000 IU (Gibco: #15070-063).
hTERT-BJ1 cell line is cultured in: 400ml DMEM with L-glutamine, sodium pyruvate and pyridoxine (Gibco: #41966-029), 100ml M199 with Earle's salts, L-glutamine, $NaHCO_3$ (Sigma; #M4530), 50ml FBS (E.U. approved, Gibco; #10106-169).
To passsage the cells, the culture medium from a 75 $cm^2$ flask of cells is removed and the cells washed with pre-warmed (37°C) PBS (Dulbecco, W/O Calcium and Magnesium, W/O Sodium Bicarbonate; Gibco #14190-094). 0.5 ml pre-warmed (37°C) Trypsin/EDTA (Gibco #25300-054) is applied and spread evenly on the surface of the cells and incubated for 5 minutes at 37°C. The trypsin is neutralised with 4.5 ml of the pre-warmed (37°C) appropriate culture medium and the cells washed from the surface of the flask by pipetting.

Cell densities are measured using a haemocytometer.

iii)FACS analysis of U2OS cells transfected with gene specific siRNA is carried out as follows.

U2OS cells are transfected with 240 nM of each gene specific siRNA, using 2 ml 1x$10^5$ cells/ml cell suspension per well of the 6-well plates for each transfections and leaving the cells to grow overnight at 37°C/ 5%$CO_2$. siRNAs are thawed, the Oligofectamine Reagent is pre-warmed to room temperature and all culture media is pre-warmed to 37°C. In RNase-free, sterile Eppendorf tubes 12μl of each 20 mM siRNA solution is added to 200 μl of OptiMEM Serum-free medium (Gibco #31985-039). For each transfection reaction, 8μl of Oligofectamine™ Reagent (Invitrogen #12252-011) is mixed with 52μl of OptiMEM and incubated for 10 minutes at room temperature. This is prepared as a batch for all the transfections.

60 μl Oligofectamine/OptiMEM is mixed with each siRNA and incubate for 15-20 minutes at room temperature. 128 μl of OptiMEM is then added to each siRNA/Oligofectamine mix. After removing the culture medium from the cells, washing briefly with PBS and re-feeding with 600 μl of the appropriate cell culture medium without antibiotics and without FBS, 400 μl siRNA/Oligofectamine mix is added to the cells in the 6-well dish and left to incubate for 4 hours at 37°C/5% $CO_2$. The culture is subsequently supplemented with 1 ml of the appropriate cell culture medium containing 20% FBS and antibiotics and the cells incubated at 37°C/ 5% $CO_2$ for 48 hours.

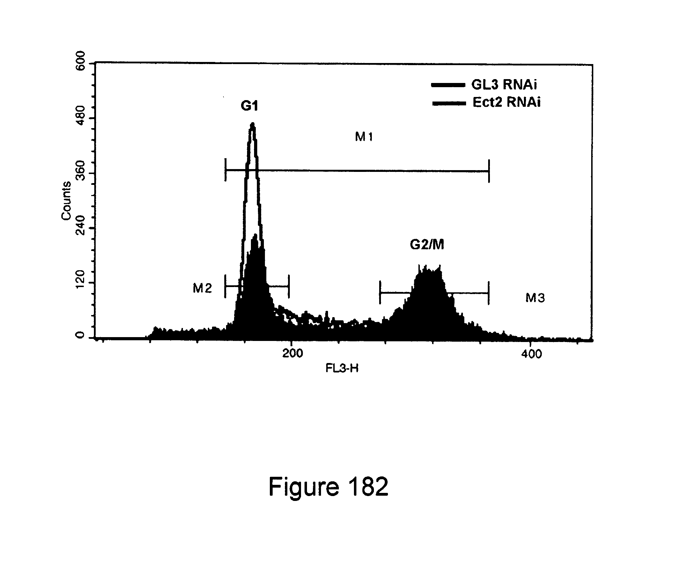

For each experiment cells transfected with siRNA (aaCUUACGCUGAGUACUUCGA) targeting the *Photinus pyralis* (GL3) luciferase gene (Accession no. U47296) (Harborth et al., 2001, J Cell Sci. 114:4557-4565) and cells transfected with no siRNA are included as negative controls. Cells transfected with Ect2 siRNA COD1513 (aaGUGGGCUUUGUAAAGAUGG) shown previously by us to result in a block in mitosis are included as the positive control (See FACS profiles in Figure 182).

Following incubation, the supernatant from each well is transferred to a 15 ml centrifuge tube. The well is rinsed with 0.5 ml pre-warmed (37°C) Trypsin/EDTA and this is combined with the 2 ml of supernatant from the same well. A further 0.5 ml pre-warmed (37°C) Trypsin/EDTA is added to the remaining cells, spread evenly over the surface of the cells and incubated for 5 minutes at 37°C. The trypsinised cells are added to the 2.5 ml supernatant from the same well and the cells pelleted by centrifuging at 2000 rpm for 5 minutes. The supernatant is removed and the cells resuspended in 1 ml PBS, transferring the resuspended cells to an Eppendorf tube. The cells are pelleted at 2000 rpm for 1 minute in a micofuge, most of the PBS removed and the cells resuspended in the remaining PBS by flicking the tube. 1 ml 70% ethanol is added drop-wise to the cells while vortexing the tube and the fixed cells stored at -20°C overnight.

The cells are pelleted by centrifuging at 1000rpm for 1 minute most of the ethanol removed and the cells resuspended in the remaining ethanol by flicking the tube as before. 0.5 ml PBS is added drop-wise to the cells while vortexing the tube. The cells are incubated with 3 μl 6 mg/ml RNase A and the DNA stained with 12.5 μl mg/ml propidium iodide at 37°C for 30 minutes, then stored on ice until ready for analysis.

The DNA content of the cells is analysed with BD FACSCalibur™ System (BD Biosciences, European Office, Denderstraat 24, 9320 Erembodegem, Belgium) using the FL3 channel and CellQuest software.

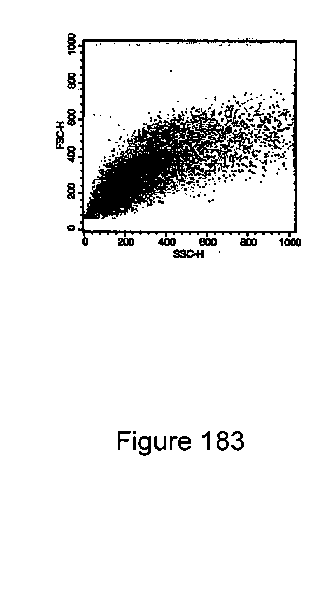

Voltages on FSC and SSC channels are altered until the profile appears as shown in Figure 183.

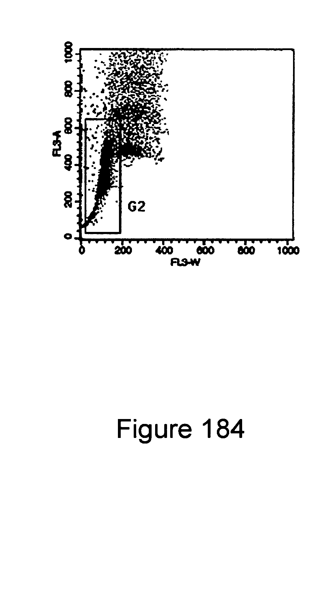

The voltage on FL3 channel is also altered to enable analysis of cells in G1, S and G2/M, setting a gate (G2) to exclude doublets resulting from cell clumping, as shown in Figure 184.

10,000 gated events are collected for analysis and dot plots of FSC-H against SSC-H and of FL3-A against FL3-W are generated, as demonstrated above.

A histogram showing counts against FL3-H within the gated region (G2) and the associated histogram statistics is also generated. Using the histogram statistics, events in G1, in S and in G2/M are calculated, where the number of G1 events was equal to M2 x 2, the number of G2/M events is equal to M3 x 2 and the number of S events equated to M1-[(M2 x 2)+(M3 x 2)]. See Figure 185 for example.

The cell cycle profile for cells transfected with the gene specific siRNA are compared with control cells transfected with GL3 siRNA, by comparison of the statistics and also by overlaying the histogram profiles, to identify human genes required for normal cell cycle progression.

Changes in cell cycle profile are deemed significant where the FACS profile is noticeably different when compared, by eye, to the negative control profile.

**7) ABNORMAL HUMAN CELL PHENOTYPES RESULTING FROM siRNA TRANSFECTION ARE ASSESSED BY IMMUNOSTAINING siRNA-TREATED CELLS AND MICROSCOPIC ANALYSIS.**

U2OS and HeLa human cell lines cultured as before are transfected with 240 nM gene specific siRNA, selecting siRNAs previously shown to induce in an abnormal FACS profile in transfected cells. The cells are transfected essentially as detailed above, except that a clean, sterile 22x22mm coverslip is first placed in each well of the 6-well plates used for transfections.

i) Immunostaining of siRNA-treated human cells for microscopic analysis is carried out as follows:

The medium is gently removed and the cells incubated with 1 ml Fixation Solution (60 mM PIPES, 25 mM Hepes, 10 mM EGTA, 4 mM $MgSO_4$, pH to 6.8 with KOH, 3.7% formaldehyde) pre-warmed to 37°C for 10 minutes at room temperature. The fixation solution is removed and the cells permeablised with 1 ml PBT (PBS +0.1% Triton X-100) for 2 minutes at room temperature. The permeablisation solution is removed and the cells incubated with 1 ml blocking solution (1% BSA / PBS / 0.1% Triton X-100), for 1 hour at room temperature. The blocking solution is replaced with 0.5 ml primary antibody solution (1% BSA / PBS / 0.1% Triton X-100 / 1:300 dilution rat YL1/2 anti-$\alpha$-tubulin antibody (SeroTec #MCA77G) / 1:750 dilution rabbit anti-$\gamma$-tubulin antibody (Sigma #DQ-19) and the cells incubated with the primary antibody solution either at room temperature for 2 hours or at 4°C overnight. The cells are washed 3 times for 5 minutes with 1 ml PBT, then incubated with 0.5 ml secondary antibody solution (1% BSA / PBS / 0.1% Triton X-100 / 1:300 dilution TRITC-donkey anti-rat IgG (Jackson Immunoresearch # 712-026-150) / 1:300 dilution AlexaFluor 488-goat anti-rabbit (Molecular Probes #A-11008) at room temperature for 45-60 minutes. The cells are once more washed 3 times for 5 minutes with 1 ml PBT and once for 5 minutes with 1 ml PBS. Finally, the coverslips are mounted, cells-side down on clean microscope slides using mounting medium containing DAPI (1.25% (w/v) n-propylgallate in 75% glycerol / 0.5 $\mu$g/ml DAPI, stored at 4°C), sealed with nail enamel and stored, protected from light at 4°C.

ii) Analysis of the mitotic phenotype of the immunostained, siRNA-treated cells.

Staining of DNA (Zeiss filter set 01), $\alpha$-tubulin (Zeiss filter set 15) and $\gamma$-tubulin (Zeiss filter set 10) is observed using Plan-neofluar 40x/1.30 oil Ph3 and Plan-Apochromat 63x/1.40 oil Ph3 objectives on the Zeiss Axioskop 2 plus (Carl Zeiss Ltd., Woodfield Road, Welwyn Garden City, Herts. AL7 1 LU, UK) using Axiocam HR CCD camera and AxioVision 3.0 software to acquire images.

For each experiment, the number of normal looking mitotic cells in prophase/prometaphase, metaphase, anaphase and telophase is quantified as well as the abnormal cells in each stage of the cell cycle. For each experiment, 200-250 mitotic cells are assessed. Of the abnormal cells, the percentage with misaligned chromosomes and lagging chromosomes and the number showing abnormal spindle morphology is quantitated. The number of centrosomes associated with each nucleus is also noted. For a more complete characterisation of the phenotype the ploidy and cell viability (cell confluency and number of apoptotic cells), the number of multinucleated interphase cells and the nuclear and overall cell morphology is assessed. To determine if a particular gene specific siRNA caused an abnormal cell cycle phenotype, comparisons are made with the phenotype of cells transfected with the GL3 siRNA control.

Positive results are judged as those where the overall mitotic defects were increased from the negative control by about 10% or more. There are also instances, however, where the number of overall mitotic defects does not increase significantly, yet there is a significant increase in the percentage of cells with one particular defect and this would also be viewed as significant.

## 8) DISEASE ASSOCIATION

The level of expression of a gene(s) in tumour and normal cells is determined by assessing the levels of hybridisation of a gene specific radioactive probe to a cancer profile array. The array consists of 240 total cDNAs from tumour cells and from equivalent normal cells from 13 tissue types (Cat no. 7841-1 - Clontech Laboratories, Inc., 1020 East Meadow Circle, Palo Alto, CA 94303-4230, USA). A gene specific DNA sequence of approximately 500bp is amplified from a plasmid containing the gene or a part of the gene and the PCR product purified following a standard PCR purification protocol (QIAquick PCR purification - QIAGEN GmbH, Max-Volmer-Strasse 4, 40724 Hilden, Germany). The purified PCR fragment is then radioactively labelling using 6000 Ci/mmol $\alpha^{32}$P [dCTP] (Amersham Biosciences UK Ltd., Amersham Place, Little Chalfont, Buckinghamshire HP7 9NA) following a standard random hexamer labelling protocol (High prime - Roche Diagnostics Ltd., Bell Lane, Lewes, East Sussex BN7 1LG, UK).

Changes in gene expression level between normal and cancer cells are detected by probing the cancer profile array with 100ng of radiolabelled probe, following the standard manufacturer's protocol (Clontech). Briefly, the cancer profile array is pre-hybridised for 90 minutes at 65°C in 10ml ExpressHyb (Clonetech) containing 1.5mg of denatured sheared salmon testis DNA. The pre-hybridisation solution discarded and the cancer profile array hybridised overnight at 65°C in 5ml ExpressHyb containing the labelled DNA probe, 30$\mu$g $C_ot$-1 DNA, 150$\mu$g sheared salmon testis DNA and 1% 20x SSC. After hybridisation, the cancer profile array is washed four times in 40ml 2xSSC, 1% SDS for 30 minutes at 65°C before 1 wash in 0.1xSSC, 0.5% SDS for 30 minutes at 65°C. A final wash is performed in 5xSSC for 5 minutes at room temperature.

The gene specific radiolabelled DNA bound to the cancer profile array is detected by exposing the array to X-ray film (Kodak Biomax MR) for 6-14 days at -70°C using intensifying screens.

The expression pattern for the gene in the cancer profile array is assessed, by noting the number of cases where gene expression is increased and the number where there is a decrease. Changes in gene expression in tumour cells are deemed significant where more than 50% of the samples in a tissue sample result in a decrease or an increase in gene expression in 4 or more tissue types.

Each of the applications and patents mentioned in this document, and each document cited or referenced in each of the above applications and patents, including during the prosecution of each of the applications and patents ("application cited documents") and any manufacturer's instructions or catalogues for any products cited or mentioned in each of the applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the claims.

## Claims

1. A polynucleotide selected from the group consisting of odd-numbered SEQ ID NOs in Figures 1 to 180, or a polypeptide encoded by the polynucleotide, for use in a method of prevention, treatment or diagnosis of a disease in an individual.

2. A polypeptide selected from the group consisting of even-numbered SEQ ID NOs in Figures 1 to 180 for use in a method of prevention, treatment or diagnosis of a disease in an individual.

3. A polynucleotide according to Claim 1 or a polypeptide according to Claim 2 for a use as specified therein, in which the polypeptide or polynucleotide is used to identify a substance capable of binding to the polypeptide, which method comprises incubating the polypeptide, or a polypeptide encoded by the polynucleotide, with a candidate substance under suitable conditions and determining whether the substance binds to the polypeptide.

4. A polynucleotide according to Claim 1 or 3 or a polypeptide according to Claim 2 or 3 for a use as specified therein, in which the polypeptide or polynucleotide is used to identify a substance capable of modulating the function of the polypeptide, the method comprising the steps of: incubating the polypeptide, or a polypeptide encoded by the polynucleotide, with a candidate substance and determining whether activity of the polypeptide is thereby modulated.

5. A polynucleotide or a polypeptide according to any preceding claim for a use as specified therein, in which the polynucleotide or polypeptide, preferably in an effective amount, is administered to an individual in need of such treatment.

6. A polynucleotide or a polypeptide according to Claim 3 or 4 for a use as specified therein, in which the substance identified by the method is administered to an individual in need of such treatment.

7. A polynucleotide according to any preceding claim for a diagnostic use as specified therein, in which the presence or absence of a polynucleotide is detected in a biological sample in a method comprising: (a) bringing the biological sample containing nucleic acid such as DNA or RNA into contact with a probe comprising a fragment of at least 15 nucleotides of the polynucleotide under hybridising conditions; and (b) detecting any duplex formed between the probe and nucleic acid in the sample.

8. A polypeptide according to any preceding claim for a diagnostic use as specified therein, in which the presence or absence of a polypeptide is detected in a biological sample in a method comprising: (a) providing an antibody capable of binding to the polypeptide; (b) incubating a biological sample with said antibody under conditions which allow for the formation of an antibody-antigen complex; and (c) determining whether antibody-antigen complex comprising said antibody is formed.

9. An antibody capable of binding to a polypeptide selected from the group consisting of even-numbered SEQ ID NOs in Figures 1 to 180 for use in a method of prevention, treatment or diagnosis of a disease in an individual.

10. An antibody according to Claim 9 for a diagnostic use as specified therein, in which the method comprises contacting a cell or tissue sample with the antibody and detecting an antibody/antigen complex, wherein said detection is indicative of said disease or condition.

**11.** A polynucleotide, polypeptide or an antibody according to any preceding claim for a use as specified therein, in which the disease comprises a proliferative disease, a disease or condition **characterized by** cell proliferation in mammalian tissue or a tumour, preferably a cancer.

**12.** A method for detecting the presence or absence of a polynucleotide in a biological sample which comprises:

(a) bringing the biological sample containing DNA or RNA into contact with a probe comprising a fragment of at least 15 nucleotides of a nucleic acid selected from the group consisting of odd-numbered SEQ ID NOs in Figures 1 to 180 under hybridising conditions; and
(b) detecting a duplex formed between the probe and nucleic acid in the sample.

**13.** A method of modulating, preferably down-regulating, the expression of a polynucleotide selected from the group consisting of odd-numbered SEQ ID NOs in Figures 1 to 180 in a cell, preferably an isolated cell, the method comprising introducing a double stranded RNA (dsRNA) corresponding to the polynucleotide, or an antisense RNA corresponding to the polynucleotide, or a fragment thereof, into the cell.

**14.** Use of a polypeptide in a method of identifying a substance capable of affecting the function of the corresponding gene, in which the polypeptide is selected from the group consisting of even-numbered SEQ ID NOs in Figures 1 to 180

**15.** Use of a polypeptide in an assay for identifying a substance capable of inhibiting the cell division cycle, in which the polypeptide is selected from the group consisting of even-numbered SEQ ID NOs in Figures 1 to 180.

**16.** Use as claimed in Claim 14 or 15, in which the substance is capable of inhibiting cell cycle progression, preferably mitosis and/or meiosis.

**17.** A substance identified by a method or assay according to any of Claims 3, 4, 14 or 15.

**18.** Use of a substance according to Claim 17 in a method of inhibiting the function of a polypeptide, or a method of regulating a cell division cycle function.

**19.** A method of modulating cell cycle progression in a cell, the method comprising:

(a) transforming into the cell a nucleic acid sequence selected from the group consisting of:

(i) a nucleic acid sequence encoding a polypeptide of any of the even-numbered SEQ ID NOs in Figures 1 to 180;
(ii) a nucleic acid sequence encoding a polypeptide having at least 80% sequence identity to a polypeptide of any of the even-numbered SEQ ID NOs in Figures 1 to 180;
(iii) a nucleic acid having a sequence comprising any of the odd-numbered SEQ ID NOs in Figures 1 to 180;
(iv) a nucleic acid having at least 80% sequence identity to a nucleic acid having a sequence comprising any of the odd-numbered SEQ ID NOs in Figures 1 to 180; and
(v) a complement of any of (i) to (iv);

wherein the nucleic acid sequence is operably linked to a regulatory sequence; and
(b) culturing the cell under conditions whereby the nucleic acid sequence is expressed; thereby modulating cell cycle progression in the cell.

**20.** A method of modulating cell cycle progression in a cell, the method comprising:

(a) transforming into the cell an isolated nucleic acid sequence that encodes a ribonucleic acid (RNA) precursor, wherein the precursor comprises:

(i) a first stem portion comprising a 15 to 40 nucleotide long sequence from any of the odd-numbered SEQ ID NOs in Figures 1 to 180;
(ii) a second stem portion comprising a 15 to 40 nucleotide long sequence that is complementary to 15 to 40 consecutive nucleotides of a sequence of any of the odd-numbered SEQ ID NOs in Figures 1 to 180, and wherein the first and second stem portions can hybridise with each other to form a duplex stem; and (iii) a loop portion that connects the two stem portions;

(b) culturing the cell under conditions whereby the nucleic acid sequence is expressed, thereby modulating cell cycle progression in the cell.

**21.** A method according to Claim 19 or 20, in which the nucleic acid sequence encodes a polypeptide selected from the group consisting of the even-numbered SEQ ID NOs in Figures 1 to 180.

**22.** A method according to Claim 19, 20 or 21, in which the nucleic acid sequence comprises a sequence selected from the group consisting of the odd-numbered SEQ ID NOs in Figures 1 to 180, or a complement thereof.

**23.** A method according to any of Claims 19 to 22, in which cell cycle progression is decreased, preferably resulting from a decrease in proliferation of the cell.

**24.** A method according to any of Claims 19 to 22, in which cell cycle progression is increased, preferably resulting from a increase in proliferation of the cell.

**25.** A host cell transformed by a method according to any of Claims 19 to 24.

**26.** A method for providing a mammal with an anti-proliferative protein, the method comprising introducing into the mammal a mammalian cell transformed by a method according to any of Claims 19 to 24.

**27.** A RNA precursor encoded by a nucleic acid sequence selected from the group consisting of the odd-numbered SEQ ID NOs in Figures 1 to 180.

**28.** A composition comprising, as a biologically active ingredient, an RNA precursor according to Claim 27.

**29.** A RNA precursor according to Claim 27, or a composition according to Claim 28, for use in a method of treatment of a disease or condition **characterised by** cell proliferation, preferably cancer.

**30.** A pharmaceutical composition comprising, as an active ingredient:

(a) a cell cycle progression nucleic acid sequence of the odd-numbered SEQ ID NOs in Figures 1 to 180;
(b) a cell cycle progression polypeptide selected from the group consisting of the even-numbered SEQ ID NOs in Figures 1 to 180; or
(c) an antibody to a cell cycle progression polypeptide selected from the group consisting of the even-numbered SEQ ID NOs in Figures 1 to 180;

and a pharmaceutically-acceptable carrier.

**31.** An antagonist of a cell cycle progression polypeptide selected from the group consisting of the even-numbered SEQ ID NOs in Figures 1 to 180 for use in a method for treating a disease or condition **characterized by** cell proliferation in mammalian tissue, the method comprising contacting the tissue with the antagonist.

**32.** An agonist of a cell cycle progression polypeptide selected from the group consisting of the even-numbered SEQ ID NOs in Figures 1 to 180 for use in a method for treating a disease or condition **characterized by** cell proliferation in mammalian tissue, the method comprising contacting the tissue with the agonist.

**33.** A kit for treating a disease or condition **characterized by** cell proliferation in mammalian tissue, the kit comprising:

(a) a polypeptide encoded by a nucleic acid sequence of any of the odd-numbered SEQ ID NOs in Figures 1 to 180;
(b) a nucleic acid having a nucleotide sequence of any of the odd-numbered SEQ ID NOs in Figures 1 to 180; or
(c) an antibody recognising an epitope of a polypeptide of (a).

**34.** An array comprising at least two cell cycle progression genes having nucleic acid sequences of any of the odd-numbered SEQ ID NOs in Figures 1 to 180.

**35.** An array according to Claim 34, wherein the nucleic acid sequences are DNA sequences or RNA sequences.

**36.** An array comprising at least two cell cycle progression proteins having polypeptide sequences of any of the even-numbered SEQ ID NOs in Figures 1 to 180.

# Figure 1

**Name:** CG3632
**Nucleotide:** AE003501
**GI:** 10728281
**Transcript:** CT12163
**Sequence:**

atatgcatacatatgtatgtgcaacaacatttgcaaatctttgtttataatagctgacgcttatggattttatggtgccgattc
acacaatgtccgatggatcaccgcctccatcgatttgctttatccgtgccgccgaatcgtatccaaagtcacaaatggagaaggaagactc
ccagctgttcgtcccgtttcaagagttggccggcgaatcgattaagtacctggggcgcacggatgatggtatcctcgccctgtccaattatcg
gatattcctttccaagcagtcaaccggctacgagacctacgttccattgggattaatcgaatccgtacaggtgcgagatctgttccagctgat
tgtcaattgtaaggatgccagcactgtgcgctgctcctttccgtcggcggaacagtgctccgactggcagcgccggatccatctgatgatcg
gggttcccgagtcactggagacactcttcgcctttcccttctactcctggacctgcgatgtgctcggaagtggcaatggaagcggtatcgtta
gcgcccaggccaacggaaacggcctgattgccaaggatcgttgcctaacgctacacaatggcttggcaaagccaacagccaacgtca
cagcgtcgaatcctctgcccgatcctgccagcgagaccatctcagccgccatgagcaatcgcctgcaacgatcagtgcgctatgaaagc
gactttaagaacgaggttgcccgcttgggattcgatctgaagggctcgtggcgcatctccacggccaatgccgattttaagctctgtccctcg
tatccacccaaattgcttgtgccctgctgtatcaccgatgagatgctccacaacgtggccaacttccggggatcacgaaggctgccggctg
tcgtctggcggcaccaaaagtcgggtgccatcttggcccgatgcagccagccggaggtcggttggctcggctggcgcaacacaaggga
cgagcagctactcaaggcactcgccgatgcctgtgcctttgacaggggcgagcacgccaggcattccacctgcgccaatgcaaaggcg
caaccaacaaagggctccaaggagaccaacggccagtcgggcttatccggcaagagttcaccatctctggacgattcctcgcatgagg
aactcacgctggatgaaatcaagaaaatcctcattgtggatgctcgcagctacacctccgcagttacaaatcgtgccagaggtggcggct
gtgagtgcatcgagtactatccctgtgcagagattgagtttatgaacttgggcaacatccacgccattcgaaagagtttccacgctgtccga
cagctatgcgcgtcatcccccgatgatccaaactggtacggacaactggaaaagaccatgtggatgcagcacctgtcgggtctgctggg
agccaccatgaccgttgtgcacaccatcgagaagaatggacgacccgtccttgtacattgctcagacggttgggatcgcactccacagat
cgtggccacggcgcaattatgtttggatccctactacagaacggttgaggggttccgcgttcttgtcgagcgtgaatggctgaactttggaca
caaattcgccgatcgctctggaaatggtcccaattccgatgaagttaacgagcgatgtccagtttttctgcagtggcttgatcttgtgcatcaa
atacacaggcagtatccatgcagttttgagtttagtataagctatttgatcaaactggcgcaacattcgttgtcctgtctcttcggcacgttcctat
gcctcatggcgaagacacgatcttctgaagacttaacaacgaatgagttgggacagagcaccatttccaggaggtccagtgatcccaac
ctgaccgttgaatccatcaactcgatgtttgacatacgatccgaggccaacaacagtgttagcgaaaatgtccaagaaagtgttaaagact
ccaaagaagtagagctggacgtgacggatgcaactgaaaaactgaactctgaatctgggcgacctttttcatatttggttcatccgaaaa
cgatcaaagtccagttcctttaaaatctgaagacacaagtgatatttctcgcgccctgtggcatggcgcaattgaaaccagcactgataccc
ttattcctgcggagcccgtacaaaaaccaaacagcgacaatagcagtagagatcacataacatcccccacaatggaactggtcagtgg
tgatagaaagctggagtcgactagcactgtccaagccagtaaaatcagtcagagctacaataatttgcccaaggtacacataagaccga
atgccgttatatgcccgcagcgggtagacgcttttccccatcacctggacctacaagtgccaagggagacgacgggaaatccggaccgt
tgcaagccggaaaagttagccaaagatgcaaatgcgaatgccaatgccaacggatccctgctggcatccgatggctacaacggcgag
gaaagtctctttatatcacccgaccttcagcggttcgtgggccagtcgccattcgatgctccctcgacgggcggacgaatacggcgaaata
ccttcggctcgagctacagtcgcgatatgaagttcacggctgaaaatggcaggtga (SEQ ID NO:1)

**Protein:** AAF48583
**Protein GI:** 7293200

MSDGSPPPSICFIRAAESYPKSQMEKEDSQLFVPFQELAGESIKYLGRTDDGILALS
NYRIFLSKQSTGYETYVPLGLIESVQVRDLFQLIVNCKDASTVRCSFPSAEQCSDWQRRIHLMIGV
PESLETLFAFPFYSWTCDVLGSGNGSGIVSAQANGNGLIAKDRCLTLHNGLAKPTANVTASNPLP
DPASETISAAMSNRLQRSVRYESDFKNEVARLGFDLKGSWRISTANADFKLCPSYPPKLLVPCCIT
DEMLHNVANFRGSRRLPAVVWRHQKSGAILARCSQPEVGWLGWRNTRDEQLLKALADACAFDR
GEHARHSTCANAKAQPTKGSKETNGQSGLSGKSSPSLDDSSHEELTLDEIKKILIVDARSYTSAVT
NRARGGGCECIEYYPCAEIEFMNLGNIHAIRKSFHAVRQLCASSPDDPNWYGQLEKTMWMQHLS
GLLGATMTVVHTIEKNGRPVLVHCSDGWDRTPQIVATAQLCLDPYYRTVEGFRVLVEREWLNFG
HKFADRSGNGPNSDEVNERCPVFLQWLDLVHQIHRQYPCSFEFSISYLIKLAQHSLSCLFGTFLCL
MAKTRSSEDLTTNELGQSTISRRSSDPNLTVESINSMFDIRSEANNSVSENVQESVKDSKEVELDV
TDATEKLNSESGRPFFIFGSSENDQSPVPLKSEDTSDISRALWHGAIETSTDTLIPAEPVQKPNSD
NSSRDHITSPTMELVSGDRKLESTSTVQASKISQSYNNLPKVHIRPNAVICPQRVDAFPHHLDLQV
PRETTGNPDRCKPEKLAKDANANANANGSLLASDGYNGEESLFISPDLQRFVGQSPFDAPSTGG
RIRRNTFGSSYSRDMKFTAENGRKMDRISLKMLQHVPGRPSKNVVVPPRMPLPRFKRKKPPVSS
GCQTMQFRVALVVKLSSGLDEENIIVDLVEKFFVLTARSFGRRCQTKSFLTQLGYVDPATRASRP
MSRSTLRYLQNPRRRTMRRLLRFPKHFSFSQF (SEQ ID NO:2)

**Transcript:** CT13680
**Sequence:**

catacatatgtatgtgcaacaacatttgcaaatctttgtttataatagctgacgcttatggattttatggtggtaaatagagc
gagaaaatctagaagccaagcgcgttttcaattcggcaatacgttttgagctaaataccccgaaaagtctgctgagcgaacgccgattca
cacaatgtccgatggatcaccgcctccatcgatttgctttatccgtgccgccgaatcgtatccaaagtcacaaatggagaaggaagactcc
cagctgttcgtcccgtttcaagagttggccggcgaatcgattaagtacctggggcgcacggatgatggtatcctcgccctgtccaattatcgg
atattcctttccaagcagtcaaccggctacgagacctacgttccattgggattaatcgaatccgtacaggtgcgagatctgttccagctgatt
gtcaattgtaaggatgccagcactgtgcgctgctcctttccgtcggcggaacagtgctccgactggcagcgccggatccatctgatgatcg
gggttcccgagtcactggagacactcttcgcctttcccttctactcctggacctgcgatgtgctcggaagtggcaatggaagcggtatcgtta
gcgcccaggccaacggaaacggcctgattgccaaggatcgttgcctaacgctacacaatggcttggcaaagccaacagccaacgtca
cagcgtcgaatcctctgcccgatcctgccagcgagaccatctcagccgccatgagcaatcgcctgcaacgatcagtgcgctatgaaagc
gactttaagaacgaggttgcccgcttgggattcgatctgaagggctcgtggcgcatctccacggccaatgccgatttaagctctgtccctcg
tatccacccaaattgcttgtgccctgctgtatcaccgatgagatgctccacaacgtggccaacttccggggatcacgaaggctgccggctg
tcgtctggcggcaccaaaagtcgggtgccatcttggcccgatgcagccagccggaggtcggttggctcggctggcgcaacacaaggga
cgagcagctactcaaggcactcgccgatgcctgtgcctttgacaggggcgagcacgccaggcattccacctgcgccaatgcaaaggcg
caaccaacaaagggctccaaggagaccaacggccagtcgggcttatccggcaagagttcaccatctctggacgattcctcgcatgagg
aactcacgctggatgaaatcaagaaaatcctcattgtggatgctcgcagctacacctccgcagttacaaatcgtgccagaggtggcggct
gtgagtgcatcgagtactatccctgtgcagagattgagtttatgaacttgggcaacatccacgccattcgaaagagtttccacgctgtccga
cagctatgcgcgtcatcccccgatgatccaaactggtacggacaactggaaaagaccatgtggatgcagcacctgtcgggtctgctggg
agccaccatgaccgttgtgcacaccatcgagaagaatggacgacccgtccttgtacattgctcagacggttgggatcgcactccacagat
cgtggccacggcgcaattatgtttggatccctactacagaacggttgaggggttccgcgttcttgtcgagcgtgaatggctgaactttggaca
caaattcgccgatcgctctggaaatggtcccaattccgatgaagttaacgagcgatgtccagtttttctgcagtggcttgatcttgtgcatcaa
atacacaggcagtatccatgcagttttgagtttagtataagctattgatcaaactggcgcaacattcgttgtcctgtctcttcggcacgttcctat
gcctcatggcgaagacacgatcttctgaagacttaacaacgaatgagttgggacagagcaccatttccaggaggtccagtgatcccaac
ctgaccgttgaatccatcaactcgatgtttgacatacgatccgaggccaacaacagtgttagcgaaaatgtccaagaaagtgttaaagact
ccaaagaagtagagctggacgtgacggatgcaactgaaaaactgaactctgaatctgggcgaccttttttcatatttggttcatccgaaaa
cgatcaaagtccagttcctttaaaatctgaagacacaagtgatatttctcgcgccctgtggcatggcgcaattgaaaccagcactgataccc
ttattcctgcggagcccgtacaaaaaccaaacagcgacaatagcagtagagatcacataacatcccccacaatggaactggtcagtgg
tgatagaaagctggagtcgactagcactgtccaagccagtaaaatcagtcagagctacaataatttgcccaaggtacacataagaccga
atgccgttatatgcccgcagcgggtagacgcttttccccatcacctggacctacaagtgccaagggagacgacgggaaatccggaccgt
tgcaagccggaaaagttagccaaagatgcaaatgcgaatgccaatgccaacggatccctgctggcatccgatggctacaacggcgag
gaaagtctctttatatcacccgaccttcagcggttcgtgggccagtcgccattcgatgctccctcgacgggcggacgaatacggcgaaata
ccttcggctcgagctacagtcgcgatatgaagttcacggctgaaaatggcaggtga (SEQ ID NO:3)

**Protein:** AAF48584
**Protein GI:** 7293201

MSDGSPPPSICFIRAAESYPKSQMEKEDSQLFVPFQELAGESIKYLGRTDDGILALS
NYRIFLSKQSTGYETYVPLGLIESVQVRDLFQLIVNCKDASTVRCSFPSAEQCSDWQRRIHLMIGV
PESLETLFAFPFYSWTCDVLGSGNGSGIVSAQANGNGLIAKDRCLTLHNGLAKPTANVTASNPLP
DPASETISAAMSNRLQRSVRYESDFKNEVARLGFDLKGSWRISTANADFKLCPSYPPKLLVPCCIT
DEMLHNVANFRGSRRLPAVVWRHQKSGAILARCSQPEVGWLGWRNTRDEQLLKALADACAFDR
GEHARHSTCANAKAQPTKGSKETNGQSGLSGKSSPSLDDSSHEELTLDEIKKILIVDARSYTSAVT
NRARGGGCECIEYYPCAEIEFMNLGNIHAIRKSFHAVRQLCASSPDDPNWYGQLEKTMWMQHLS
GLLGATMTVVHTIEKNGRPVLVHCSDGWDRTPQIVATAQLCLDPYYRTVEGFRVLVEREWLNFG
HKFADRSGNGPNSDEVNERCPVFLQWLDLVHQIHRQYPCSFEFSISYLIKLAQHSLSCLFGTFLCL
MAKTRSSEDLTTNELGQSTISRRSSDPNLTVESINSMFDIRSEANNSVSENVQESVKDSKEVELDV
TDATEKLNSESGRPFFIFGSSENDQSPVPLKSEDTSDISRALWHGAIETSTDTLIPAEPVQKPNSD
NSSRDHITSPTMELVSGDRKLESTSTVQASKISQSYNNLPKVHIRPNAVICPQRVDAFPHHLDLQV
PRETTGNPDRCKPEKLAKDANANANANGSLLASDGYNGEESLFISPDLQRFVGQSPFDAPSTGG
RIRRNTFGSSYSRDMKFTAENGR (SEQ ID NO:4)

**Transcript:** CT13700
**Sequence:**

agagcgagaaaatctagaagccaagcgcgttttcaattcggcaatacgttttgagctaaataccccgaaaagtctgct
gagcgaacgtattcttttagccgattcacacaatgtccgatggatcaccgcctccatcgatttgctttatccgtgccgccgaatcgtatccaaa
gtcacaaatggagaaggaagactcccagctgttcgtcccgtttcaagagttggccggcgaatcgattaagtacctggggcgcacggatg

atggtatcctcgccctgtccaattatcggatattcctttccaagcagtcaaccggctacgagacctacgttccattgggattaatcgaatccgt acaggtgcgagatctgttccagctgattgtcaattgtaaggatgccagcactgtgcgctgctcctttccgtcggcggaacagtgctccgactg gcagcgccggatccatctgatgatcggggttcccgagtcactggagacactcttcgcctttcccttctactcctggacctgcgatgtgctcgg aagtggcaatggaagcggtatcgttagcgcccaggccaacggaaacggcctgattgccaaggatcgttgcctaacgctacacaatggc ttggcaaagccaacagccaacgtcacagcgtcgaatcctctgcccgatcctgccagcgagaccatctcagccgccatgagcaatcgcc tgcaacgatcagtgcgctatgaaagcgactttaagaacgaggttgcccgcttgggattcgatctgaagggctcgtggcgcatctccacgg ccaatgccgattttaagctctgtccctcgtatccacccaaattgcttgtgccctgctgtatcaccgatgagatgctccacaacgtggccaactt ccggggatcacgaaggctgccggctgtcgtctggcggcaccaaaagtcgggtgccatcttggcccgatgcagccagccggaggtcggt tggctcggctggcgcaacacaagggacgagcagctactcaaggcactcgccgatgcctgtgcctttgacaggggcgagcacgccagg cattccacctgcgccaatgcaaaggcgcaaccaacaaagggctccaaggagaccaacggccagtcgggcttatccggcaagagttc accatctctggacgattcctcgcatgaggaactcacgctggatgaaatcaagaaaatcctcattgtggatgctcgcagctacacctccgca gttacaaatcgtgccagaggtggcggctgtgagtgcatcgagtactatccctgtgcagagattgagtttatgaacttgggcaacatccacgc cattcgaaagagtttccacgctgtccgacagctatgcgcgtcatcccccgatgatccaaactggtacggacaactggaaaagaccatgtg gatgcagcacctgtcgggtctgctgggagccaccatgaccgttgtgcacaccatcgagaagaatggacgacccgtccttgtacattgctc agacggttgggatcgcactccacagatcgtggccacggcgcaattatgtttggatccctactacagaacggttgaggggttccgcgttcttg tcgagcgtgaatggctgaactttggacacaaattcgccgatcgctctggaaatggtcccaattccgatgaagttaacgagcgatgtccagtt tttctgcagtggcttgatcttgtgcatcaaatacacaggcagtatccatgcagttttgagtttagtataagctatttgatcaaactggcgcaacat tcgttgtcctgtctcttcggcacgttcctatgcctcatggcgaagacacgatcttctgaagacttaacaacgaatgagttgggacagagcacc atttccaggaggtccagtgatcccaacctgaccgttgaatccatcaactcgatgtttgacatacgatccgaggccaacaacagtgttagcg aaaatgtccaagaaagtgttaaagactccaaagaagtagagctggacgtgacggatgcaactgaaaaactgaactctgaatctgggcg accttttttcatatttggttcatccgaaaacgatcaaagtccagttcctttaaaatctgaagacacaagtgatatttctcgcgccctgtggcatgg cgcaattgaaaccagcactgataccccttattcctgcggagcccgtacaaaaaccaaacagcgacaatagcagtagagatcacataaca tcccccacaatggaactggtcagtggtgatagaaagctggagtcgactagcactgtccaagccagtaaaatcagtcagagctacaataa tttgcccaaggtacacataagaccgaatgccgttatatgcccgcagcgggtagacgcttttccccatcacctggacctacaagtgccaag ggagacgacgggaaatccggaccgttgcaagccggaaaagttagccaaagatgcaaatgcgaatgccaatgccaacggatccctgc tggcatccgatggctacaacggcgaggaaagtctctttatatcacccgaccttcagcggttcgtgggccagtcgccattcgatgctccctcg acgggcggacgaatacggcgaaataccttcggctcgagctacagtcgcgatatgaagttcacggctgaaaatggcaggtga (SEQ ID NO:5)

**Protein:** AAF48582
**Protein GI:** 7293199

MSDGSPPPSICFIRAAESYPKSQMEKEDSQLFVPFQELAGESIKYLGRTDDGILALS NYRIFLSKQSTGYETYVPLGLIESVQVRDLFQLIVNCKDASTVRCSFPSAEQCSDWQRRIHLMIGV PESLETLFAFPFYSWTCDVLGSGNGSGIVSAQANGNGLIAKDRCLTLHNGLAKPTANVTASNPLP DPASETISAAMSNRLQRSVRYESDFKNEVARLGFDLKGSWRISTANADFKLCPSYPPKLLVPCCIT DEMLHNVANFRGSRRLPAVVWRHQKSGAILARCSQPEVGWLGWRNTRDEQLLKALADACAFDR GEHARHSTCANAKAQPTKGSKETNGQSGLSGKSSPSLDDSSHEELTLDEIKKILIVDARSYTSAVT NRARGGGCECIEYYPCAEIEFMNLGNIHAIRKSFHAVRQLCASSPDDPNWYGQLEKTMWMQHLS GLLGATMTVVHTIEKNGRPVLVHCSDGWDRTPQIVATAQLCLDPYYRTVEGFRVLVEREWLNFG HKFADRSGNGPNSDEVNERCPVFLQWLDLVHQIHRQYPCSFEFSISYLIKLAQHSLSCLFGTFLCL MAKTRSSEDLTTNELGQSTISRRSSDPNLTVESINSMFDIRSEANNSVSENVQESVKDSKEVELDV TDATEKLNSESGRPFFIFGSSENDQSPVPLKSEDTSDISRALWHGAIETSTDTLIPAEPVQKPNSD NSSRDHITSPTMELVSGDRKLESTSTVQASKISQSYNNLPKVHIRPNAVICPQRVDAFPHHLDLQV PRETTGNPDRCKPEKLAKDANANANANGSLLASDGYNGEESLFISPDLQRFVGQSPFDAPSTGG RIRRNTFGSSYSRDMKFTAENGRKMDRISLKMLQHVPGRPSKNVVVPPRMPLPRFKRKKPPVSS GCQTMQFRVALVVKLSSGLDEENIIVDLVEKFFVLTARSFGRRCQTKSFLTQLGYVDPATRASRP MSRSTLRYLQNPRRRTMRRLLRFPKHFSFSQFI (SEQ ID NO:6)

**Transcript:** CT13718
**Sequence:**

cgataatcggccatttttcgcctattggctatgaaatcgtgataatctagtctattggtatatatgtgcgtgcgtatgtaatgc agcgacacacaaatatgtgcatactatatattgcgcaccttgtggaactgcaactgtgtgtgctcacataaatccgattcacacaatgtccg atggatcaccgcctccatcgatttgctttatccgtgccgccgaatcgtatccaaagtcacaaatggagaaggaagactcccagctgttcgtc ccgtttcaagagttggccggcgaatcgattaagtacctggggcgcacggatgatggtatcctcgccctgtccaattatcggatattcctttcca agcagtcaaccggctacgagacctacgttccattgggattaatcgaatccgtacaggtgcgagatctgttccagctgattgtcaattgtaag gatgccagcactgtgcgctgctcctttccgtcggcggaacagtgctccgactggcagcgccggatccatctgatgatcggggttcccgagt cactggagacactcttcgcctttcccttctactcctggacctgcgatgtgctcggaagtggcaatggaagcggtatcgttagcgcccaggcc

aacggaaacggcctgattgccaaggatcgttgcctaacgctacacaatggcttggcaaagccaacagccaacgtcacagcgtcgaatc
ctctgcccgatcctgccagcgagaccatctcagccgccatgagcaatcgcctgcaacgatcagtgcgctatgaaagcgactttaagaac
gaggttgcccgcttgggattcgatctgaagggctcgtggcgcatctccacggccaatgccgattttaagctctgtccctcgtatccacccaa
attgcttgtgccctgctgtatcaccgatgagatgctccacaacgtggccaacttccggggatcacgaaggctgccggctgtcgtctggcgg
caccaaaagtcgggtgccatcttggcccgatgcagccagccggaggtcggttggctcggctggcgcaacacaagggacgagcagcta
ctcaaggcactcgccgatgcctgtgcctttgacaggggcgagcacgccaggcattccacctgcgccaatgcaaaggcgcaaccaaca
aagggctccaaggagaccaacggccagtcgggcttatccggcaagagttcaccatctctggacgattcctcgcatgaggaactcacgct
ggatgaaatcaagaaaatcctcattgtggatgctcgcagctacacctccgcagttacaaatcgtgccagaggtggcggctgtgagtgcat
cgagtactatccctgtgcagagattgagtttatgaacttgggcaacatccacgccattcgaaagagtttccacgctgtccgacagctatgcg
cgtcatcccccgatgatccaaactggtacggacaactggaaaagaccatgtggatgcagcacctgtcgggtctgctgggagccaccatg
accgttgtgcacaccatcgagaagaatggacgacccgtccttgtacattgctcagacggttgggatcgcactccacagatcgtggccacg
gcgcaattatgtttggatccctactacagaacggttgaggggttccgcgttcttgtcgagcgtgaatggctgaactttggacacaaattcgcc
gatcgctctggaaatggtcccaattccgatgaagttaacgagcgatgtccagtttttctgcagtggcttgatcttgtgcatcaaatacacaggc
agtatccatgcagttttgagtttagtataagctatttgatcaaactggcgcaacattcgttgtcctgtctcttcggcacgttcctatgcctcatggc
gaagacacgatcttctgaagacttaacaacgaatgagttgggacagagcaccatttccaggaggtccagtgatcccaacctgaccgttg
aatccatcaactcgatgtttgacatacgatccgaggccaacaacagtgttagcgaaaatgtccaagaaagtgttaaagactccaaagaa
gtagagctggacgtgacggatgcaactgaaaaactgaactctgaatctgggcgaccttttttcatatttggttcatccgaaaacgatcaaag
tccagttcctttaaaatctgaagacacaagtgatatttctcgcgccctgtggcatggcgcaattgaaaccagcactgataccctta ttcctgcg
gagcccgtacaaaaaccaaacagcgacaatagcagtagagatcacataacatcccccacaatggaactggtcagtggtgatagaaa
gctggagtcgactagcactgtccaagccagtaaaatcagtcagagctacaataatttgcccaaggtacacataagaccgaatgccgttat
atgcccgcagcgggtagacgcttttccccatcacctggacctacaagtgccaagggagacgacgggaaatccggaccgttgcaagcc
ggaaaagttagccaaagatgcaaatgcgaatgccaatgccaacggatccctgctggcatccgatggctacaacggcgaggaaagtct
ctttatatcacccgaccttcagcggttcgtgggccagtcgccattcgatgctccctcgacgggcggacgaatacggcgaaataccttcggct
cgagctacagtcgcgatatgaagttcacggctgaaaatggcaggtga (SEQ ID NO:7)

**Protein:** AAF48581
**Protein GI:** 7293198
MSDGSPPPSICFIRAAESYPKSQMEKEDSQLFVPFQELAGESIKYLGRTDDGILALS
NYRIFLSKQSTGYETYVPLGLIESVQVRDLFQLIVNCKDASTVRCSFPSAEQCSDWQRRIHLMIGV
PESLETLFAFPFYSWTCDVLGSGNGSGIVSAQANGNGLIAKDRCLTLHNGLAKPTANVTASNPLP
DPASETISAAMSNRLQRSVRYESDFKNEVARLGFDLKGSWRISTANADFKLCPSYPPKLLVPCCIT
DEMLHNVANFRGSRRLPAVVWRHQKSGAILARCSQPEVGWLGWRNTRDEQLLKALADACAFDR
GEHARHSTCANAKAQPTKGSKETNGQSGLSGKSSPSLDDSSHEELTLDEIKKILIVDARSYTSAVT
NRARGGGCECIEYYPCAEIEFMNLGNIHAIRKSFHAVRQLCASSPDDPNWYGQLEKTMWMQHLS
GLLGATMTVVHTIEKNGRPVLVHCSDGWDRTPQIVATAQLCLDPYYRTVEGFRVLVEREWLNFG
HKFADRSGNGPNSDEVNERCPVFLQWLDLVHQIHRQYPCSFEFSISYLIKLAQHSLSCLFGTFLCL
MAKTRSSEDLTTNELGQSTISRRSSDPNLTVESINSMFDIRSEANNSVSENVQESVKDSKEVELDV
TDATEKLNSESGRPFFIFGSSENDQSPVPLKSEDTSDISRALWHGAIETSTDTLIPAEPVQKPNSD
NSSRDHITSPTMELVSGDRKLESTSTVQASKISQSYNNLPKVHIRPNAVICPQRVDAFPHHLDLQV
PRETTGNPDRCKPEKLAKDANANANANGSLLASDGYNGEESLFISPDLQRFVGQSPFDAPSTGG
RIRRNTFGSSYSRDMKFTAENGRKMDRISLKMLQHVPGRPSKNVVVPPRMPLPRFKRKKPPVSS
GCQTMQFRVALVVKLSSGLDEENIIVDLVEKFFVLTARSFGRRCQTKSFLTQLGYVDPATRASRP
MSRSTLRYLQNPRRRTMRRLLRFPKHFSFSQFI (SEQ ID NO:8)

# Figure 2

**Name:** Pp1-87B
**Nucleotide:** AE003695
**GI:** 7299572
**Transcript:** CT17842
**Sequence:**

gcagtgtggcaacatcagctagaagcacactcgccatcccgcccgcactcacgcaacagcatacgaagaaattttc
atacttgttagctgtgaaaagtatttagcagaaatagatttcggaaattaaagatttcgaggctatttcggcgctaccaacgatcgttcgcggt
aacctcgacacccaacagcagcactagtgcaccagatccacactttcgcacgcaaacatgggcgacgtgatgaatatcgacagcata
atatcgcgacttctcgaggtgcgtggggcacggccaggtaaaaacgtacagctctcggagggcgagatccggggactttgcttgaagtc
gcgcgagatcttcctgtcgcagcccattctgctcgagctagaggcaccgttgaagatctgcggcgacatccatggacagtactacgatctg
ttgcgtctgttcgagtacggcggctttccgccggaatcgaattacctgttcctcggcgactacgtcgatcgcggcaagcaatcgctggagac
gatctgcctgctgctcgcctacaagatcaaatactcggagaatttcttcctgctgcgcggcaaccacgaatgcgccagcattaatcgcatat
acggattctacgacgaatgcaagcgtcgctacagcatcaagttgtggaagacattcacggactgcttcaactgcctgccagtggcggcca
ttgtcgacgagaagatcttctgctgccacggtggtctcagtcccgatttgacctccatggagcagatccgtcgcattatgcggccaaccgat
gtgcccgaccagggactgctgtgcgatctcctgtggtccgatcccgataaggataccatgggctggggcgaaaacgaccgcggcgttag
cttcaccttcggtgccgaggtggtggccaagtttttgcagaagcacgagttcgatctcatctgccgagcccatcaagtcgtcgaggatgggt
acgagttctttgccaaacgcatgctggtcaccctgttctcggcgcccaactactgcggcgagttcgacaatgccggcgccatgatgtccgtg
gacgatacgctgatgtgctcgttccaaatcctcaagccagccgacaagcgtaaaaagtaatatcacacaactgcagcaccacgagcag
tctttttctatctaaaacagatcaataagaaatccaaacaagatacaaaagataaacacgacaaaaacaaccacaaacccaaccaca
caatcaaccagaaataaaatcctttgaaacaacacatcaaatgatgcatgaaacccggggcgagtcgaaatccacaagaaattgcac
cattaatttggtatggaatagcagcgccacgatcaaaggacatccccagagacatcccttccagcagatgactgcagttttccccttccac
cccacacccattgagatttaaatcgcaattacaaacacattgacaaataccatgattaaaagccacatatttatttgtaatagcccccgtata
tatatatatatatgtatatctaccgaccgaccaaccattcacccacgcacacacatgtacacacatttgaacaccagcgcttttataggttttgt
ccaaaatttaaacactaggttaatcaactgaccgttcgagcagagcaccaagcgcataaccatgca (SEQ ID NO:9)

**Protein:** AAF54810
**Protein GI:** 7299625

MGDVMNIDSIISRLLEVRGARPGKNVQLSEGEIRGLCLKSREIFLSQPILLELEAPLKI
CGDIHGQYYDLLRLFEYGGFPPESNYLFLGDYVDRGKQSLETICLLLAYKIKYSENFFLLRGNHEC
ASINRIYGFYDECKRRYSIKLWKTFTDCFNCLPVAAIVDEKIFCCHGGLSPDLTSMEQIRRIMRPTD
VPDQGLLCDLLWSDPDKDTMGWGENDRGVSFTFGAEVVAKFLQKHEFDLICRAHQVVEDGYEF
FAKRMLVTLFSAPNYCGEFDNAGAMMSVDDTLMCSFQILKPADKRKK (SEQ ID NO:10)

# Figure 3

**Name:** CG3524
**Nucleotide:** AE003581
**GI:** 10727365
**Transcript:** CT11835
**Sequence:**

tcggatctatacgtgcccaccaaaaggatatacaaccacacccaactagaaggacctactagcccagctgacatcc
tggttgtcccgcaaaacgcacggcacattcgaaaaggatacgcgatcagtgaacccaagtgaataaggtgccgtccaaaaacgatac
gatccgaattagtggcatacgaaatatccttgtatatttgggccctcatacagactgagccagaacaaagaaaaaacgccgaaatgccg
agcaggaagaaagcccagggggaatcggccgctggtgaggatgcggacattgtgatctcgggactttccgggaagctgccggagag
cagcaacatcgaggagttcaagtacaacctgctcaacggcatcgacatggtcacagatgagccgcgccgttgggaggcaggcatctat
ggactccccgagcgtatggccaaaatgaaggactccgatctggagaagttcgacgacaagttctttagcgtccaccagaagcaggcgg
agctgatggacccctgcatgcggatgctgctggagctgacccacgaggcgatcatcgatgcgggcatcaatcccgtacagctgcgcgg
cagtcggacgggcgtctacataggcctgtcctttgtggagacggagcacgagatccccaacatggagccgagcagtatcaacggctact
gcctgacgggctgtgcgcgtgccatgttcgccaatcgcatctcctacacgttcgacttcaaggggcccagctttatcgtggacaccgcatgc
tccagctcgctggtggcccttaaccacgccttcgcggacatgcgggcgggtcgctgtgattacgccctggttgcgggcgtgaatctcatcct
aaagcccatctttgccctccagttcctcaggctgggcatcgtgagccacgacggctcctgcaagaccttcgatgcggcggccaacggcta
tgcccgggcggacacctgcgccgttgtgctgctccagaggcgtaaggaggccaagcgggtgtacgccagcatcctaaatgttcggacc
aatacggacggattcaaggagcagggcgtaaccttcccagacggccgcatgcagcaggccctgctggaggagacctacagcgagat
cggcctcaatccggacgaggtggtctacgtggaggcacacggcagcggcacaccggtgggcgatgaccaggaagccaacatgctga
gcaacttcttctgtcgcccctcgcgatccactccgctgcttattggctcagtgaagtcgaacatgggtcacgcggagccagcctcgggagtg
agtgccctggccaagatgatcatcgccatggaggagggcattattccgaagaatctgcactatcgcacgcccaatccttcggttccggctc
tggtcgagggtaagcttaaggtggtggatcgcaatcttccctggcagggtggcattgtgggtctgaactccttcggctttggaggagccaat
gcccatgtggtactaaagtcgcacactaaagccaaagagcctaaggcatgcccgaaggctggggaggccgaacagtccctgagtcta
gttacctgttctggccgcacggaggatgcagtgaatcagttgctgaaagttgccaccgagcagaggcacgatcaggagctgctgaccct
catcaatgacatccactcacatcctattcccctgcatccgttcaggggatacgctgttctcggtaccagcggatgcgttaaggaggaggtgc
tgccctacgaggaggaggagcgacccatctggttcgtctacgccggcatgggcagtcaatgggccaggatggctaaggatctcatgca
gctggaggttttccggaactccatacagcactgcgccgaggttctggcccaggtggactttgatcttatagacgtgcttacccgttccacgga
gcgaacctttgacaatatgctctactcctttgtctccgtgtccgccgtgcaggtggcgttgacagatcttctccgggtgctggacatacgacca
gatggaatcattggccattcggctggtgagctgggggcagcctacatggacggatgtctaacggcggagcaaacagttctggccgcctat
tggcgcggcaggagcgtcctggacactccggacttgccgcgcggaaagatggctgctgtaggtttgagctgggagcagatcggatcac
agatcccaaaggattgctatccggtgtgtcacaacagtgatgacaattgcacggtctccgggccaccgtcatccatggacgcgatgatag
aggacctcacggcaaagggaattttcgtgagagaggttggctctggaggctatgctttccacagtccgtacattgaaggagcggctcccat
gctgcgtagaagtctggagcgactgatcacggagcccaagaagaagagtattcgatggcttagtactagtgtgaaagaagcggagtgg
gactcggagaaaaaccacctggcatcggctggctactttataaacaacctcatatcccccgtgctctttcatcaagctgtcaagcggattcc
ccaaaacgctctcatcgtggagatcgcaccccatggactcttcagggcgattctccgatccttaagtccgcagattagctatgtgagtctgat
gcagcgaggtcatgccaataactttgagttcttgctgagccagttgggcagactctacgctgccggagggcagccgcagatcctgaagat
ctccccttccatcccctatcccgtttcccgtggcactcccatgctgggctccttagtgggctgggatcacacacagaagtggaactacccca
agttcaagggcggcagacaggcgggacagcttagtgtggagctagatttgggcaaggaggaaaacgtctatctagcggggcacacga
tcgacggcagagtcttgttccccgcaaccggctacatgaccctggcctggatgagtctggcccaacagcagggattggactacttgcgca
cgcccgtgctattcgaggatgtggtcttccatcgggccacgatccttagcgtgggcactgtggtaaaactaacactcaacttctttccgggca
gcagctcctttgaaatctgtgaaggcacaagcctggtgacatcgggcaagatccgtctggtgagcaatgtgcagcaggagcagcttcag
ctgtcatctctaccgggaatcgccggctccaacaagctgagcaccaaggacatttacaaggagctaagacttaggggctacgactacac
tggtgttttccagggcattttggagtcggatatttcggcggtgacgggtcagctgcagtgggctgacaattggatcagttttatggacaccatg
ctgcagtttcgcatcctgagcaatgatatccgagaactgtacgtgcccacgggaatagagagggcactgatagatcctctcaagcacctg
gagctggcaaagaagcaccaacagaagttaactgtcaactggcatcgcaacatttccgtagtcaagtgcggtggagtggagatacaca
agatgaagaccgcccagacccaaaggcgttctggtggccaatctccacccagcttggagcgctatagattctggcctcattcccagcactt
gggtcagcgggaggagcaggaaaagtcaaggagcacagctctagcggtggccattcaattgatcatcgagaacagcggtggagctgt
aaagctcaagggcgtggaactggctggatcccggtccactttggatgcgctgagtgccgtccagcttctcgagctgatcgaaagggaac
cgattctggtgggcgacatggcggtggttaccagctcctccaatgaatccgaactgggtgagcagctcaaggagcacggtattcgggtga
tagtcaaggatatcaaagctggtgcggtggagcagcaatgccatttcgtgtactctgtgaatatgctagcccagaactctgtcaaggatctg
cagcactgcttggatagtctgaagtccgattcgggattcctgctcctggaggaagcagccagcaggtataacattataggaaaggagaa
gttgaacaaacttaagctggtttcagttttggagcagttcttcggtacagatcgagtgcttgtcctagtcagaaaactatcgaaggtgcaatcc
agccaagccctgacacttcacctcaccaataaacagttcaagtgggtcaacgagctaaaaaactctattgcaaaggcccaagctgaag
agaaaaacctctacttggtggcccagggtgaacccaattcaggagctttgggcttgatgaactgcctgaagagggaagctggagggcat

ttcctacggctataccttattctggatgaaggtgtgccccagttctccctggacgatcccttctacgccgcacagttggccaaggatctggtcg
tcaatgtctacagaaatggaagctggggtagctaccgtcacctgaagatggaatcccggccgcccatgctgccagtggagcaggcgtat
gtgaacaccctggtcaaggggggatctctcatcacttcgctggatcgagagtcctcgttccagtcccatccagtcgcaattggagccctgcac
cgtttactatgctccgctcaatttccgggatgtgatgttggcctccggtaaactgggagtggatgctctgcctggggatctggcctaccagga
ctgtgtccttggtctggaattcgctggcagggattcgtgcggccgtcgtgtaatggcaatggttactgccaaatctttggccaccaactgcttg
gccaacaggaatctcctctgggaagtcccatctaagtggaccatggagcaggcttcgaccgttccctgtgtttatgccaccgtttattacgctt
tggtggtcaggggccaaatgaaggagggtgaaaggatcctcatccacgccggctctggaggagttggtcaggcggccatttcggtggct
ttggctcacggactgactgtcttcaccactgtgggcagcaaggagaagcgggagttcctgctgaagcgattccccaagctgaaagctaa
gaatattggcaattcgcgagacacctctttcgagcaattgatcatgagcgaaacccacggaaatggagtggaacttgtgctgaactctctct
ccgaggagaagctgcaagcctccattcgctgtctggctttgaatggccgcttcctggagatcggcaagtttgacttgagcaacaacactcc
gctagggatgtcggtgttcctcaagaacacctccttccacggaatccttctggacagcgtgatggagggggaggaggagatgcagctaat
ggttgccaagttggtggcggagggtatcaagagtggagcagtgcagccgctgcctaccacagtctttgcagagcaggagatcgagaag
gcgttccgtttcatggcgtccggcaaacatattggcaaggtggtgatcaaggtacgtttggaggaggaacagcaggcggctctcccgcga
cttaggcaaattcaggccataccgcgctcctacatgcacccagagaagagctacatcttggtggggggacttggggggattcggtctggag
ctggccaactggctagtttccagaggagcccgctttctggttctgagctctcgctctggggtgaagagcgggtaccaggccctgatgattcgt
cgctggcacgaccgtggagttcaggttcagatcgataccaacgatgtgaccaccgctgttggctgccagagactcctagaggttagtaat
aagctggccctggttggaggtatctttaacctcgcggctgtactacgggatggactgatcgaagatcaaagcccgaagaactttgaaaag
gtaagtgcaccgaagctactggccaccatacacttggacaagatctcccgggaaatttgtcctgcactggagtacttcgtgtgcttctccag
cttgtcctgcggcaggggaaacatgggacagaccaactatggactggcgaactccacgatggaaaggatatgcgagcagcggcagg
aatatggttatccgggcacagcgattcaatggggagcaattggggacaccggactgatcatcgagcacatgggcaacaacgaaacag
ttgtgggcggcactctgccgcagcggatgatcagctgtctggagactctggacctatttctgcagcagccccatcccgtcatggcctccatg
gtggtggcggaaaaacgcaagacggagcaggccaaccggctgagcttgatagccacgatcgcgaatatcatgggactgagggatgt
aaagagtgtctccgataagaccaccctcttcgatcttggcatggactcgctgatgagcacagagatcaagcagacgttggagcgtcacttt
gacctggtgctgagcgcccaggagatccgccagctaacctttagcgccctgcgccagatagacgcccaggaaccgccctccgcttcag
tctcatcgccctcagcctcttccaccctatcaaggatcaaggaggaggcctgcgtggaggtccagccccagagcgatgagacgcgcaa
ggtattcgccaaggaagtattaccccaggaggtgatggtgcgccttcggtcaaaggcgcccgtggccagcgaggatccggctgtgttctt
cttggctcccatcgagggattcacaatagccgtggaggcgctggccgcctcgctcacctgccccgcctacgggctgcagtgcacggagc
aagtgcccctggactccatcgaggcctgtgccgcctactatctgcgccagatccagaagctccagcccctcggaccgtaccacatcgtag
gctactcctacggctgcctgctcgcccacgccattgctgtggcgctggagcagcgccgcttcggggtcaaggtcatcatgctggacggag
cgcccaccatggccagcggctatgtccaggaggccaagaagcagacggacgacctcaaccggcagcagtccatgacgttggcctact
ttggagctctgctcgccgacgtggattacaaccagctactgcaactcctcgatggagtccagacatggccctccaagctcgacaaactag
cagacactctggctggttacacgcagcaaaccagagaagtgatcaagaaggccgcctgcatgttcaaacagaagctgctactttctgag
agctacaagggcgccaagcagctccacagcgatgtggtcttgatcaagtcggccgagcacaatgcgattatggcccaggactatggcct
caaggagatttgcagtgcccagatcgacatgcatgtggtggagggtacacacaggaccttcctcaaggagccgcacactctacagatc
atcgagcgcgtcctgcggaagcgtccctagtggaagggaagtagttcgtacgattatgctgatgaattatcctgtagaacttaatgtacatat
accctaatcctatcaatacacgtagcaatcgcgcattgattgtttaagaat (SEQ ID NO:11)

**Protein:** AAF51149
**Protein GI:** 7295849

MPSRKKAQGESAAGEDADIVISGLSGKLPESSNIEEFKYNLLNGIDMVTDEPRRWEAGIY
GLPERMAKMKDSDLEKFDDKFFSVHQKQAELMDPCMRMLLELTHEAIIDAGINPVQLRGSRTGVY
IGLSFVETEHEIPNMEPSSINGYCLTGCARAMFANRISYTFDFKGPSFIVDTACSSSLVALNHAFAD
MRAGRCDYALVAGVNLILKPIFALQFLRLGIVSHDGSCKTFDAAANGYARADTCAVVLLQRRKEAK
RVYASILNVRTNTDGFKEQGVTFPDGRMQQALLEETYSEIGLNPDEVVYVEAHGSGTPVGDDQE
ANMLSNFFCRPSRSTPLLIGSVKSNMGHAEPASGVSALAKMIIAMEEGIIPKNLHYRTPNPSVPALV
EGKLKVVDRNLPWQGGIVGLNSFGFGGANAHVVLKSHTKAKEPKACPKAGEAEQSLSLVTCSGR
TEDAVNQLLKVATEQRHDQELLTLINDIHSHPIPLHPFRGYAVLGTSGCVKEEVLPYEEEERPIWFV
YAGMGSQWARMAKDLMQLEVFRNSIQHCAEVLAQVDFDLIDVLTRSTERTFDNMLYSFVSVSAV
QVALTDLLRVLDIRPDGIIGHSAGELGAAYMDGCLTAEQTVLAAYWRGRSVLDTPDLPRGKMAAV
GLSWEQIGSQIPKDCYPVCHNSDDNCTVSGPPSSMDAMIEDLTAKGIFVREVGSGGYAFHSPYIE
GAAPMLRRSLERLITEPKKKSIRWLSTSVKEAEWDSEKNHLASAGYFINNLISPVLFHQAVKRIPQN
ALIVEIAPHGLFRAILRSLSPQISYVSLMQRGHANNFEFLLSQLGRLYAAGGQPQILKISPSIPYPVS
RGTPMLGSLVGWDHTQKWNYPKFKGGRQAGQLSVELDLGKEENVYLAGHTIDGRVLFPATGYM
TLAWMSLAQQQGLDYLRTPVLFEDVVFHRATILSVGTVVKLTLNFFPGSSSFEICEGTSLVTSGKIR
LVSNVQQEQLQLSSLPGIAGSNKLSTKDIYKELRLRGYDYTGVFQGILESDISAVTGQLQWADNWI
SFMDTMLQFRILSNDIRELYVPTGIERALIDPLKHLELAKKHQQKLTVNWHRNISVVKCGGVEIHKM
KTAQTQRRSGGQSPPSLERYRFWPHSQHLGQREEQEKSRSTALAVAIQLIIENSGGAVKLKGVEL

AGSRSTLDALSAVQLLELIEREPILVGDMAVVTSSSNESELGEQLKEHGIRVIVKDIKAGAVEQQCH
FVYSVNMLAQNSVKDLQHCLDSLKSDSGFLLLEEAASRYNIIGKEKLNKLKLVSVLEQFFGTDRVL
VLVRKLSKVQSSQALTLHLTNKQFKWVNELKNSIAKAQAEEKNLYLVAQGEPNSGALGLMNCLKR
EAGGHFLRLYLILDEGVPQFSLDDPFYAAQLAKDLVVNVYRNGSWGSYRHLKMESRPPMLPVEQ
AYVNTLVKGDLSSLRWIESPRSSPIQSQLEPCTVYYAPLNFRDVMLASGKLGVDALPGDLAYQDC
VLGLEFAGRDSCGRRVMAMVTAKSLATNCLANRNLLWEVPSKWTMEQASTVPCVYATVYYALVV
RGQMKEGERILIHAGSGGVGQAAISVALAHGLTVFTTVGSKEKREFLLKRFPKLKAKNIGNSRDTS
FEQLIMSETHGNGVELVLNSLSEEKLQASIRCLALNGRFLEIGKFDLSNNTPLGMSVFLKNTSFHGI
LLDSVMEGEEEMQLMVAKLVAEGIKSGAVQPLPTTVFAEQEIEKAFRFMASGKHIGKVVIKVRLEE
EQQAALPRLRQIQAIPRSYMHPEKSYILVGGLGGFGLELANWLVSRGARFLVLSSRSGVKSGYQA
LMIRRWHDRGVQVQIDTNDVTTAVGCQRLLEVSNKLALVGGIFNLAAVLRDGLIEDQSPKNFEKVS
APKLLATIHLDKISREICPALEYFVCFSSLSCGRGNMGQTNYGLANSTMERICEQRQEYGYPGTAI
QWGAIGDTGLIIEHMGNNETVVGGTLPQRMISCLETLDLFLQQPHPVMASMVVAEKRKTEQANRL
SLIATIANIMGLRDVKSVSDKTTLFDLGMDSLMSTEIKQTLERHFDLVLSAQEIRQLTFSALRQIDAQ
EPPSASVSSPSASSTLSRIKEEACVEVQPQSDETRKVFAKEVLPQEVMVRLRSKAPVASEDPAVF
FLAPIEGFTIAVEALAASLTCPAYGLQCTEQVPLDSIEACAAYYLRQIQKLQPLGPYHIVGYSYGCLL
AHAIAVALEQRRFGVKVIMLDGAPTMASGYVQEAKKQTDDLNRQQSMTLAYFGALLADVDYNQLL
QLLDGVQTWPSKLDKLADTLAGYTQQTREVIKKAACMFKQKLLLSESYKGAKQLHSDVVLIKSAE
HNAIMAQDYGLKEICSAQIDMHVVEGTHRTFLKEPHTLQIIERVLRKRP (SEQ ID NO:12)

# Figure 4

**Name:** CG9311
**Nucleotide:** AE003533
**GI:** 10727923
**Transcript:** CT26513
**Sequence:**

aaacggccacactgcaacatttttcataaaacaatcttgttttgatattggtaaattaattgccagcaattagtttaaattgt
gtgtaaaataaaaggccaactgggcaggcaacaagtaaactaatcaccatggaggcggtgccccgtttgcatatgctctggttcgcccta
aaatcgagtcccgagggaacctcctttgcggctttgaaaaagtacatcgcggaattttatcatgaagatccagaggcttactccaaagagg
tccatgccctggagactttgcgcaaccaggccatgcacaccacgaaagatggagcgccggtgatgaaacgctactattgccagctaca
cgcccttcagaatcgatttccacagctggcggatcgcggaatcttcacctttaaatggaaggatctctatcacagtgccgttcacgaggtca
ctgatctgcgattcgaacgggctgcggtgctgttcaacattgccgcattgcacactcagtccggagcgtcggtgacccgtggcgatgtggat
ggaatgaagatggcctgcactcacttccaggcggctgcttgggcctatggagagctgagagaacgctatgccaatgtcaatggcggagg
ggattttatgaccccagagctgctggtctttcagcaacaggtgtgctttgcccaagctcaggaatgcatactcgagaagtcgctaatcgaca
ataggaaaccgcatattgtggccaaagtgacggcgcagattgtggtttactatggagctgcactggctgctcttcttacgggaggagatgat
ggtccagtggctcaagtcatcgatagttctgtgtacaaactgtggaagaagtacgttcgcttcaagatcaactatctcacctgcatattgtacc
tataccagggccaacattccgaggagaagcgtcagatgggcgaaagggtgacgctctaccaggcttcctgggacaaactggaggagg
cgcgcaaagagtccaagggccttcctgaccagcgggaaatcaatgaatccctcagcttcaccgctgatgtggtggaagcaaagcgcaa
aaatgccaaaaacgaaaatgagttcatttaccatgaggcggtgcccgaattgtccaccattgcagccgtccaaggtgccaatttggtcaat
gggattggctttcaagtaagcgacgaggagcacgcgggtccagacatctttgccagattagtgcccatgaaggcccacgaagccagct
cgttgtacagcgaagagaaagccaaacttttgcgtaagtacggagctctgctggaggagaaggacacccaactggagtcgtacatgag
ctccctcaccctggataatctgaatatcaacgaggagcaagccaataagttgccccagggcatcgtggatcgctgtgccgccttaaatgc
aaacaagactgctatcagcgatcttgtagaggccatgtctcagctggctgagattacagccgatgtggagaccaacctgggcgagatttc
aagcatgctggaggcagaagccaaagcggagcgagagttccaatccgccaccggcgttcaacgaacaccaaatgcccacattaccg
agttatcgcgagagttccagaaatacagtgaagcccatgcacgggcaggggaatccaataataccttgcggaaagccatgagcctgca
cgtaaataatctaaagatcctggccagaccattgcctgagattcagcaactgatgcccaaactaagttcggaactgaataccaccgaaat
tttcagggatgtgaagctcatcctcaacaaagtcaacgaaatgaaggcgcaacgtgctcagtttcatgcggatttgaggatagccatcaat
gaggatgatatcactggcaaagtaattgcccacggtggacaggagggcctccaagctttatttgcaaccgagctgggcaagcacgaca
agatcaccgaacttctcgatcagaatatggtggctcaaggtaatattctgcaagctctaactgaaaactatgccaaggcggcaccagttttg
aaaacgctccaggacgtgaagcagaaacgggagcacttttacagctcgctagctgcatcctatgatgtttatgaggatctgctggccaaat
ccgccaagggcttggaattctacaagaaactagccggaaatgtacagaagctacttaccagatttcgatccgccagggatgtacagtcc
gaagagcgccagcaaaggatgcagagcgttaaggccgcaacaactccagttgtcagcaaaccaatagcggaaactacgcctgtgcc
agcggtgagcagcacccccaaattaagggattacctcaaggccaaggcagcgatggcggatgcctcgaatcccgcctatgtgcctaca
gtgcgtccagtgccggtgggctcggagaatcccacgcaggccgcttgttcctatgccactgcaccaccgaatgagccacctccaccctat
agtctcacacagcagcagtatttgatcccagtgccgccggctacacgaatcccatgtatcagcagcaacagcagcacattgctccaccg
gcctacaaggcccaagcatcgccaaactctcagaccctccacggagcgctcggtcagatgaatttgcagggccagtcgcaggataac
aaccaagcaggcatggcttataattacgggtatccaagtgggtctgttcctcctctagcttacgctccacccggaacagtgcccgcaagct
cagcttcccagggattgaatatccagcagcctctgtatgtggctacatcgggcaatccagtttcggtttcaaatcccagtgagctgccgtcca
ccctgcaggcacccttcgtggtcaatccaatgtccaatccctaccaaacagcggcaggttattcagggcaggcttaccaacaggtcaatg
ctcaagctcaacaggtgacttatcctacagcaccatcgggctcttcaacaggacaatcagctcaacagcccttgggttatgcgccaagtc
aggggccacagcagcaagctggtggatatccacaatctcagacaccgcagcaacaggtgggagcttatcaacagcagccattatcag
gctatgcccaacagctgacgcctcagcaacaaactggatattcacagtcgcaagcaccgcagctccaagcgcccagctatgcccaaa
atcagcaatcgactggacaagcacagcagcaacagactggatatccacagacacctcaacaacatctagctgggtatgctcagtccca
gcaacaggctacaggatatccacaatcgcagacatcgcagcagcagaccaccgggtattcccagtcccagacgcctcaacaaccact
ggcatatcagcagcaaccatctggttatccacaacaacaacaacagcagtcacttgctccgcaacagccatcactttatccatcgcaggc
tgtccaggcgtcgccacaacctattcctggccagcaggcaacacctccgaatgctgggcaacctttgccatatccacagcagtacactga
tcccaatgcagctccagctccagctcctggggtttcttctgtaccaagtcctgttccagtaccgactccagcccccacttcatctcaagctcag
gttcccacatctccgcatccttcggccacctacaccagctattccaaccatcctggctatagctttaatccgcagacaggtaaatacgagta
cagcagtggctatcagcaggataccagttcgttgaagggatcccaagcctcgcaaagctaccagtttagccagagtggcaagcagcag
tcggtgggaacaacggactcagagattgccagtcgcagcaataccgccactggattcgattcacctatcgtgtcccacaccaaggttaat
atggcagctaaggactccacttctgcgatagagggcaatgagtcatccaactactactccactccctatggataccttggcagtgtaaatcc
gcagcaacaacccactcctgcgcctccttcgaattcaactgcttcaatttatatgcaaagtggagcgagcagcactgctgaaacccagac
gacttccagcggagttccttacgcctcctccgcggctttgaccaaagtagaaccaaagccggaggcattgcctcccaaggttaccagcaa
tgtggatctgcttagcgacctggacattgactgctccgttgctgttccgccgccaatgttgccgcagcctgttttgcagccccaggtggtggcc
actcctacgccgccggcaagtcaggtatcggtgcctgttaaggtagaaagtgtggcagagcacgctgctcccgcaacagcggaaattcc

tgtagcagccaccgagggaactgctcaaactccagttaccattccttcgggtccgaagtgtgccagtttagataacctatccaactgttccg
acctaagttccctggagaactttgactgggactcggaggtggagagctatgaaaaattactagagaatttgcacgtgaaaatgttgaatgg
aaaaacacaactgggcgccaaatggcaagagctgcagcaaaagttggataaagaagccgctgccaacaagcgatccaccaccatt
gcgaaattgttcccggagaaaaatcgatcccttgattgtctgccatacgatcatgccagagtaaagctggacaagcagaccgatgactac
atcaatgccgcctacatgaagaacttgagtgccggctgtcccaactttattgtcgctcagactccccagccaaataccatcaacgatttctg
gtccatgatctggtcggagaagtcgcgcactgtggtttgcctgcacaccccaaatgaacttttcgatccctactggccgcaagctttggacc
agccgactcattatgatgattacaccgtgacctgcttaaaagtgcagcaacttagccactgctccgagtatcaacttaaattgtccatgcatg
gagcggacgcagtgctggatttatctctactgcagctgaaacagtggaccaaggggggctccagctcaacttctgggtgtggcggaaaatg
ctttggagacccatcgacaacgctgcaaggcagccaatgctccgcaatcgccgctcattatgaactgcttaactggttcagagcgttccga
actggtggccattggagtatgtgctattatagcgacccaaaacaagcagccaattttgttaaatacgattgatgtttggtctcgcatttgtgcgc
agcgtcaaaattcgcttagggattccgccatcctggagcagtccatgcaaattgtgctctgcaatgctcacaatgtgctaaacaaacgtgg
cataatgacctcatatcaaatgaagatggcgccgcaggtgacagccaaggagcagcaggagaacaaggatccgctcaacgagttgg
atgcactttggaagctgaagtaa (SEQ ID NO:13)

**Protein:** AAF49705
**Protein GI:** 7294357
MEAVPRLHMLWFALKSSPEGTSFAALKKYIAEFYHEDPEAYSKEVHALETLRNQAM
HTTKDGAPVMKRYYCQLHALQNRFPQLADRGIFTFKWKDLYHSAVHEVTDLRFERAAVLFNIAAL
HTQSGASVTRGDVDGMKMACTHFQAAAWAYGELRERYANVNGGGDFMTPELLVFQQQVCFAQ
AQECILEKSLIDNRKPHIVAKVTAQIVVYYGAALAALLTGGDDGPVAQVIDSSVYKLWKKYVRFKIN
YLTCILYLYQGQHSEEKRQMGERVTLYQASWDKLEEARKESKGLPDQREINESLSFTADVVEAKR
KNAKNENEFIYHEAVPELSTIAAVQGANLVNGIGFQVSDEEHAGPDIFARLVPMKAHEASSLYSEE
KAKLLRKYGALLEEKDTQLESYMSSLTLDNLNINEEQANKLPQGIVDRCAALNANKTAISDLVEAM
SQLAEITADVETNLGEISSMLEAEAKAEREFQSATGVQRTPNAHITELSREFQKYSEAHARAGESN
NTLRKAMSLHVNNLKILARPLPEIQQLMPKLSSELNTTEIFRDVKLILNKVNEMKAQRAQFHADLRIA
INEDDITGKVIAHGGQEGLQALFATELGKHDKITELLDQNMVAQGNILQALTENYAKAAPVLKTLQD
VKQKREHFYSSLAASYDVYEDLLAKSAKGLEFYKKLAGNVQKLLTRFRSARDVQSEERQQRMQS
VKAATTPVVSKPIAETTPVPAVSSTPKLRDYLKAKAAMADASNPAYVPTVRPVPVGSENPTQAAC
SYATAPPNEPPPPYSLTQQQYFDPSAAGYTNPMYQQQQQHIAPPAYKAQASPNSQTLHGALGQ
MNLQGQSQDNNQAGMAYNYGYPSGSVPPLAYAPPGTVPASSASQGLNIQQPLYVATSGNPVSV
SNPSELPSTLQAPFVVNPMSNPYQTAAGYSGQAYQQVNAQAQQVTYPTAPSGSSTGQSAQQPL
GYAPSQGPQQQAGGYPQSQTPQQQVGAYQQQPLSGYAQQLTPQQQTGYSQSQAPQLQAPSY
AQNQQSTGQAQQQQTGYPQTPQQHLAGYAQSQQQATGYPQSQTSQQQTTGYSQSQTPQQPL
AYQQQPSGYPQQQQQQSLAPQQPSLYPSQAVQASPQPIPGQQATPPNAGQPLPYPQQYTDPN
AAPAPAPGVSSVPSPVPVPTPAPTSSQAQVPTSPHPSATYTSYSNHPGYSFNPQTGKYEYSSGY
QQDTSSLKGSQASQSYQFSQSGKQQSVGTTDSEIASRSNTATGFDSPIVSHTKVNMAAKDSTSAI
EGNESSNYYSTPYGYLGSVNPQQQPTPAPPSNSTASIYMQSGASSTAETQTTSSGVPYASSAAL
TKVEPKPEALPPKVTSNVDLLSDLDIDCSVAVPPPMLPQPVLQPQVVATPTPPASQVSVPVKVESV
AEHAAPATAEIPVAATEGTAQTPVTIPSGPKCASLDNLSNCSDLSSLENFDWDSVSVTHSVSEKQT
KASATANGHSSSFAFQSETFTDEKTTKYFQKEVESYEKLLENLHVKMLNGKTQLGAKWQELQQK
LDKEAAANKRSTTIAKLFPEKNRSLDCLPYDHARVKLDKQTDDYINAAYMKNLSAGCPNFIVAQTP
QPNTINDFWSMIWSEKSRTVVCLHTPNELFDPYWPQALDQPTHYDDYTVTCLKVQQLSHCSEYQ
LKLSMHGADAVLDLSLLQLKQWTKGAPAQLLGVAENALETHRQRCKAANAPQSPLIMNCLTGSE
RSELVAIGVCAIIATQNKQPILLNTIDVWSRICAQRQNSLRDSAILEQSMQIVLCNAHNVLNKRGIMT
SYQMKMAPQVTAKEQQENKDPLNELDALWKLK (SEQ ID NO:14)

# Figure 5

**Name:** CG9092
**Nucleotide:** AE003612
**GI:** 7297037
**Transcript:** CT26090
**Sequence:**

attctggtccacccatccgagtggagcgagctttcaagcaagcttatatcgctagcgatacatttgtggtgtttctccaag
ctcccaagtcggctcttcaatatgagcggttgtcgtcgcaatcgaaagctgacgatggccgtaagtggatgtctgataattgctgtgatggcc
ctcaccgttgggctttgtgtgggactgagcggtgacaccgacgcccccgaggaacagccaaggtttacgatcgaccatgaggccaaca
ccttcatgctggatggccagcccttccggtacgtctctggatcgtttcactatttccgcgctgtacccgagtcctggcgaagtcggttgcgcac
catgcgagcatctggtcttaatgctctggatacctatgttgaatggtcgctccacaatccgcacgatggagaatacaactgggagggcattg
cggatgttgtcaagtttctggagattgctcaggaggaagacttctacatcattctccgcccaggtccctacatttgtgctgagcgagataatgc
aatattattattcataattttgactaatattgcctcttttcagggtggtcttcctcactggttgtttaccaaatacccgtccatcaagatgcggacca
atgaccccaactacatctcagaggtgggcaagtggtatgcggagctgatgccacgcctccagcacctgttcgtaggcaacggcggcaa
gatcataatggtgcaggtggagaacgagtacggtgactatgcctgcgatcacgactacctcaactggctgcgagacgagacggagaa
gtatgtgtccggcaaggcgctgctcttcacggtggacatacccaacgagaagatgagctgtggaaagatcgagaacgtgttcgccacca
ctgacttcggcatcgatcgcatcaatgagattgacaaaatctgggcaatgctgcgtgccctgcagccgactggcccactggtcaactcgg
agttctatcccggctggctaacgcattggcaggagcagaaccaaaggcgtgatggccaggaggtagccaatgcactaaggacaattct
gagctacaacgctagtgttaacctctatatgttctttggcggcacaaactttgggttcactgctggtgccaactataatctggacggtggcatc
ggatatgcggcagacataaccagctacgactacgatgcagtgatggacgaggccggtggcgtcaccactaagtacaaccttgtcaagg
ctgtgattggagaattccttccgctgccggaaatcaccttgaatcccgctaagcgtctggcctacggaagggtggagctgacacccaagct
gacactgctctcgacggaaggacgtgctgcgctctccaagggtgatcccgtcgaatcgatcaagccgaaaaccttcgaggaactagact
tgtactcgggcctcgtcctgtacgagaccgagctgcccagcatggatctagacccagccttgctgaaaatagatcaaattaacgatcgag
cacacgtattcgtagaccaggaactggtgggcacactctcccgcgaggcacagatctactcgctgccgcttagcaagggttggggcagc
acactgcagctgctggtggagaaccaaggacgcgtcaacttctacatctccaacgacacgaagggcatctttggtgaggtgagcctgca
gctgcacaatggtggttacctcccgctggagaactggcggagcaccgctttcccgctggagcaatccgccgtcgagctctggcggcgcg
aacacactgacgagaaggccttggacccgttgctggcaaggcaacgcattcttcgaaacggaccaatactctacactggcagcctgac
agtgacggaggtgggcgacacctatttgaacatggccggttggggcaagggcgtggcctatgtgaacggttttaatttgggacgctactgg
cctgtggcaggtccacaggtcaccttatatgtgcccaacgagatactgaaagtgggtgaaaactctctggtgatccttgagtaccaacgag
cgaataagacagcaactggcgaggatctacctgcagtgcagttcgacgcagttgctcagctggacggagagtctggtgatgttccactta
aatga (SEQ ID NO:15)

**Protein:** AAF52321
**Protein GI:** 7297052

MSGCRRNRKLTMAVSGCLIIAVMALTVGLCVGLSGDTDAPEEQPRFTIDHEANTFM
LDGQPFRYVSGSFHYFRAVPESWRSRLRTMRASGLNALDTYVEWSLHNPHDGEYNWEGIADVV
KFLEIAQEEDFYIILRPGPYICAERDNGGLPHWLFTKYPSIKMRTNDPNYISEVGKWYAELMPRLQH
LFVGNGGKIIMVQVENEYGDYACDHDYLNWLRDETEKYVSGKALLFTVDIPNEKMSCGKIENVFA
TTDFGIDRINEIDKIWAMLRALQPTGPLVNSEFYPGWLTHWQEQNQRRDGQEVANALRTILSYNA
SVNLYMFFGGTNFGFTAGANYNLDGGIGYAADITSYDYDAVMDEAGGVTTKYNLVKAVIGEFLPL
PEITLNPAKRLAYGRVELTPKLTLLSTEGRAALSKGDPVESIKPKTFEELDLYSGLVLYETELPSMD
LDPALLKIDQINDRAHVFVDQELVGTLSREAQIYSLPLSKGWGSTLQLLVENQGRVNFYISNDTKGI
FGEVSLQLHNGGYLPLENWRSTAFPLEQSAVELWRREHTDEKALDPLLARQRILRNGPILYTGSL
TVTEVGDTYLNMAGWGKGVAYVNGFNLGRYWPVAGPQVTLYVPNEILKVGENSLVILEYQRANK
TATGEDLPAVQFDAVAQLDGESGDVPLK (SEQ ID NO:16)

# Figure 6

**Name:** Arr1
**Nucleotide:** AE003657
**GI:** 10728850
**Transcript:** CT17964
**Sequence:**

ggcgtataccatcgagaacgagcgcgaaacgttaaaggcacatccaaagtttaaactatttccgcagagattttgata
aacagctccaaaatggtggtcaatttcaaggtgttcaagaagtgttccccgaacaacatgatcacgctctacatgaacaggcgtgattttgt
agattccgtgactcaggtggaacccattgatggaatcattgtgctggacgatgagtacgtgcgccagaaccgcaagatcttcgtgcagttg
gtctgcaatttccgatatgggcgcgaggacgacgagatgatcggtctgcggttccagaaggaactgaccctggtctcgcagcaggtgtgc
ccaccccagaagcaggacatccagttgaccaagatgcaggagcgtctgctgaagaagcttggctccaatgcctatcccttcgtgatgca
gatgccaccaagctcgccggcctcggtggttctccagcagaaggccagtgacgagagccagccctgcggagtccagtacttcgtaaag
atctttaccggagacagcgactgcgatcgatcgcatcgcaggagcaccattaacctgggcatccgcaaggtgcagtacgcaccgacca
agcagggcatccagccctgcaccgtcgttcgcaaggacttccttctgtcgcccggagagctcgaactggaggtcaccctcgacaagcag
ctgtaccatcacggcgagaagatctcggtgaacatctgcgtgcgcaacaactccaacaaggtggtgaagaagatcaaggccatggtgc
agcagggcgtcgatgtggtcctgttccagaacggtcagttccgcaacacgatcgccttcatggagacgagcgagggatgtcccctgaac
ccgggatccagcctgcagaaggtcatgtatctggtgcccaccctggtggccaattgcgaccgcgcaggcatcgccgttgagggtgatatc
aagcgcaaagacacagctctggcctcgaccacacttattgccagtcaggatgcgagggatgcctttggcataattgtttcatatgctgtgaa
ggtcaagcttttcttgggagccctgggcggcgagctctgcgctgagctaccatttattctgatgcacccgaagccaagtcgcaaggcccaa
ctggaagccgagggctccattgaggcctaaactgaaagggctacctcaaccaacgaaaaaaatggcgtatttctacaagtcaaaccga
ttttgtagatcctaaaaaatgctgatgttgctgaaatgttctgaactgcagtcgtcgtacttttcctatatagcaaatccaatatccatatattgtat
gtgtgtatgtgtattatatttataactcaactaacaattaaatatgaacagagtttatgtat (SEQ ID NO:17)

**Protein:** AAF53644
**Protein GI:** 7298421

MVVNFKVFKKCSPNNMITLYMNRRDFVDSVTQVEPIDGIIVLDDEYVRQNRKIFVQL
VCNFRYGREDDEMIGLRFQKELTLVSQQVCPPQKQDIQLTKMQERLLKKLGSNAYPFVMQMPPS
SPASVVLQQKASDESQPCGVQYFVKIFTGDSDCDRSHRRSTINLGIRKVQYAPTKQGIQPCTVVR
KDFLLSPGELELEVTLDKQLYHHGEKISVNICVRNNSNKVVKKIKAMVQQGVDVVLFQNGQFRNTI
AFMETSEGCPLNPGSSLQKVMYLVPTLVANCDRAGIAVEGDIKRKDTALASTTLIASQDARDAFGII
VSYAVKVKLFLGALGGELCAELPFILMHPKPSRKAQLEAEGSIEA (SEQ ID NO:18)

# Figure 7

**Name:** CG9150
**Nucleotide:** AE003612
**GI:** 7297037
**Transcript:** CT26200
**Sequence:**

atggagcggtggcagaacaaactggctgtggtaacaggagccagcggaggcataggcgccgcttgtgctcgggc
catgatcggcgctggactacgggtggtgggcctggcacgtcgggaggccaagttgaaagagctcagggagagtctgccccgggagct
gcaggcgaacttcataccgcggcgctgcgacgtctccaaggaagatcaggtgcagagctcttttgactggatcgaacgggagctggag
ggcgccgacgtgctgctgaacaatgctggcattactcgcgagacggaactggtcaccccgagcaatacgcagaagcttaaggaggtc
atagacaccaacgtaatgggcgtgatttggtgtacccgcgaggcgttcaataacatgaaacggcgaggtggcgaaggtcacgttctcatc
atcaacagcatagccggacatcaggtgctcaacttcatcgacgttttgccatcgttcaatatatatccggccaccaagtttgccatcaccgcc
atcacagagacatatcggcaggaatttcagctgcactcgaacaaaatccgggttactggcatctgtcctggtgcggtgaacacgaacatc
ttcccggaagagatccatttttacgtcaaagacatggccagactcgaaccagcgaacattgcggacgcagtgatgtatgctctgcgaact
ccgcctcatgttcaggtgagcataatcactattatgtattaa (SEQ ID NO:19)

**Protein:** AAF52338
**Protein GI:** 7297069

MERWQNKLAVVTGASGGIGAACARAMIGAGLRVVGLARREAKLKELRESLPRELQ
ANFIPRRCDVSKEDQVQSSFDWIERELEGADVLLNNAGITRETELVTPSNTQKLKEVIDTNVMGVI
WCTREAFNNMKRRGGEGHVLIINSIAGHQVLNFIDVLPSFNIYPATKFAITAITETYRQEFQLHSNKI

RVTGICPGAVNTNIFPEEIHFYVKDMARLEPANIADAVMYALRTPPHVQVSIITIMY (SEQ ID NO:20)

# Figure 8

**Name:** CG11102
**Nucleotide:** AE003493
**GI:** 10728232
**Transcript:** CT31063
**Sequence:**

atgcgtggcagtattctggatgccgaggcgcgcacctcaatcaacatttggatatatctcaagagcttggttatcctgttc
gatattgcctggctggcggtgggatccgtttggctggggcattactacaccactgcaccgatcgatgatcccaaaaaggtctacatagcaa
ttatcatctgcaattgggctctggtggtgatcaccctaatcacgatatggtgcaccttcgatgctgccggaagatcctgggtaaagatgaaga
agtaccagcgatcaatgcgcgaaacagagtctcggtttaactacaagcggagcaacagtatgaaccgcaactggcgacaaaggaaa
gtgatgcgagcctaccaggatagttgggatcatcgctgccgcctgctattctgctgcatgggctcctcggagcgcaaccgaaactcattca
cggacatcgcccgtctgctgagcgacttctttcgggagttggacgtggtgccgtcggatgtggtggccggattagttctgcttcgcaagtttca
gcgtctcgaacgcgaggccattgtgaggcagcgcaaaaatggcacctatgagttcctcagcggcgtaccgattacggagcgaacccag
ttcctagcactcaacgatgccaaaaactatgacttctttcaaacggtcatccactatatgtactttgcccagggagcctacggttggcccatgt
acgtcatcatcaatcgcagcaagatgtggcatttggtgccggaactgaaatgctttggctgctgttgcggtacaagtgatgatacagaggtt
atccaggacaattgctgcctgtgcaactatgcagcgctgaagaagacgctccagctgggcgacatcgatatcgtttacgccacctatcac
gtggatgtgggcgagacgcccttctttgtggccatcgactatacacatcgggcggttgtgatcagcatacgtggtacccttagcatgaagga
catacttacggatctgaacgccgagggcgaagttctgccgctgcagccgccgcgtgacgattggctgggccacaagggaatggtgcaa
gcggcgatctacatacgcaacaagctgcaggaggagaatctcatcgagagggcactgcaacggaatccggaccgtcagacgcacac
ctttgatctggtgctggtgggccattcgctgggcgccggaacagcggccatactcgccattctccttaagcccgagcatccaacgctccagt
gcttcagttactctccgccaggaggtctgctcagtatgccagccgtggagtactcaaagtcgttcatcacctcggtggtgctgggcaaggat
gtagtgccgcgcatcggtctcaatcaaatggaggccctgcgagcggaccttatcaatgccattcagcgcagcgtggatcccaagtggaa
gaccatttcctgctcggtcatctgctgtggctgtggacccgaacccacttccgtggtcaacatgtccggccaggacacgcacatcaatcagt
accaggaggagcgtggcactgcccgttcgaccagcgctcatccgacggacagctcgatagctttaactctgcaccagcccttatatccgc
ccggtcgcatcattcacatagtgcgacatcatcccaagcccgacgagtga (SEQ ID NO:21)

**Protein:** AAF48320
**Protein GI:** 7292929

MKEIKKKSKPKRKPTRMEVEESEPEMPFSYKTQPDEGESVNGEAHTSDDGDEMEL
ATFKAASKKGVIYISNLPKHMTLTRLREILGEYGAIGRAFLRSQKLSRKPHNFLFAEGWVEFKSKRV
AKQIVPLLNYKQISTHKKSPFYYSLWIMEYLPRFKWARLNELMNIVPVTNSQNHLKFLNKKAKKAK
KVKKAKKALADRLLELVLASSI (SEQ ID NO:22)

# Figure 9

**Name:** Smr
**Nucleotide:** AE003490
**GI:** 7292788
**Transcript:** CT4722
**Sequence:**

ttacagagcgcagtgtgtgttgcaaagaaaaagtgcaatcaaaataaaaataaaataaaaatgcaaacgcattaag
gcaaatgctgttgccacgttgcagcaacaacagcaaaccaaaaataaagaaagaaataaagaagcaacttacacaattaagttagtta
aactttaagagaaaaagttacaaaattatcgaaaattagtgctaactttaaagcgaaatttttttgtggggcacttccgagagaatgtcgcag
tgcaataatgttgcaactgtcggcagcagcaacacaagcaacagcagcaacacgttaacaacagcaacaacaacaaaacaaagca
aatcaatcaattctgcggccaacaacaagtgtttaagccaaaagagttcggcatgaaatggaagcagctatacgctccacttcagtgcag
gtcggtgctccgccgcccacaaacaacaacaacagcagcagcagcagcagcagcaacagcaacaacaacaacactagcaacaat
aacaacaatagcagcaacagcggcaacaacaacacaccaaacggtgcgcaacagcagcaacaacaacaccatcagcagcagc
agcaacatcatcagcagcaacgtgttgttgccagcagcaaccagcaacagcaacaacaacaactacagcaacagcagcagcagca
gcagcaacaacagcagcagcagcagcagcagcagcatgataatggaccaacgtcccatcatttgcatcaatcatctatcagcatctcta
atcagcagaataaagcgacgcaacagcagcagcagcagcagcagcagcaacaccaaaagcagcaacaacaacaggcttccagc
atgtccgcgtatcagcaacgcctgccatcaaatgcagcatccattcacaggcgcccccactggtcttacagggccctcgagcagcagca
gcagtatgcgaagcaggcggctcatctgcagcagcagcagcagcaccagtcgcaccaacagcagcagcagcagcagcaacagga
ccaacgcaccaacctgcatctgcaaatccatcaccaccaccaacagcagcagcagcagcagcagcagcagcaacaacaacagca
acagcagcaacagcagcagcagcaaaaacagcagcaacatcatatgcagcagcagcagcagcaacaacctttgtcaccacctcac
ccacccggcagcagcagcaacagtagtagtgcagcagcggcagcagcagcagcggcagcggctgcggcggccgtcaatcctggtt
acccgccatcatccgcagcagcggcggcagttaactccggttacccgccacggcccccgcagcatcgttttatccagaacacgggctat
agcattgccccggcgcctacctaccgagataatccctactcgcgccacacccaaattcagcagcagcagcagcagcagcagcaaca
acagcagcagcagcaacagcagcagcaacaacagcagcaggctgcagcgtcgatgcccgaatatcagagggcagcagctcgagc
ggctgtggcggcggtatcggcgggtaaaggcaacgtttctggccaaagttcgaacagcagcagcagtagcagcggaggtggaggtgg
aggaggaagtgccggcggatcagctccacccggcggcggtgttgtccaggtatcgcaatccggcggagtgctggtcatggaggccatg
ccccactatgccagtcaaccaaatagtaatccctcccagcagcagcaacagcagcatcagcagcagcaaggcggcaatcccagtgg
cgcgggtgcaacatctggtgctggcggcggtggaggcggcagcggtggcagcgtaatggtcgggagtctggggcgcatcctgatgccc
catccacaggctctgcaatacaccagcgagtacctgaccaatgccacagcggccgttgctgcggcgatggtcaaccagcgacagcattt
gcagctgcaacagcagcagcagcagcaacatccgcctgagccatttggcggccagcagccgtacaagaagcaacggcttagcgag
gccaatgccaataatatgaatcacctgccaccacacccgcagcagcaacaccagcagcagcagcagcagcagcagcaacatcagg
cgcaccagcagcaacatcagcgatcctcaccagcgcaagtgcaacagcaacagcagcaacagatgaatagcagtcggcagagtca
caatgatatgtgccgacaagtggtcaccacgccaatgggcatgcagctcaaggtggagacgctgccacagcagcaacagaagcagc
agcagcaccaacagcagcagcagcagcagcagcagcagggccgatctcagccagtggtgagcagcatgtccaccgtggtgagcca
accagtgggcacggttaccgttaccacggcgggattatcggcgagccacagcggcagcagtggcaatgttgccgccggattgggtacg
ggaaatacgggatccgccagcaccgaggcctatcatcctcaggtggaggccatttcgccgacattgcccagcgatagttcgattgagga
gcgcggaaggaccagcgccaaagaggatcttctcatgcaaatccaaaaggttgacaatgagatcaaatccgctgagacgacaatgga
aacgttgcgcaaaaaggagaaatccctcatggaggaggccgccctggccaaagagcagagggccgccaaagagttgaacgataac
aacaatgatcaggaaccgatggtggaactatcgtggcgcagccaaatgctagcggagaagatctatgcggccaatcggaagacggcc
caagcgcaacattccatgctgcagaatgcagcggccgatgaatcgtctccgggttccgttgccggcagaccatggttgccattgtataacc
agccactcgatgtcgaggcacttgccatgctaatacgccagcatcagagtcaaattcgtgcgccgctacttttgcacatacgaaaattgaa
ggccgaacgctgggcgcacaatcagggactggtggagaagtacaccaaggatcaggcggattggcagcgacgatgcgagcgtatg
gaggccagtgccaagaggaaagcacgcgaggcaaaaaatcgcgaattcttcgaaaaggtcttcacggagctaaggaaacagcgcg
aggataaggaacgtttcaatcgcgtcggttcgcggattaaatccgaagcggatctcgaggagatcatggatggcctgcaggagcaggc
gttggaggataaaaagatgcgatcctatgcggttataccgcctctgatgcacgatgcccgccagaggcgatgcgcctatcataatgaaaa
tggacttatcgaggatatggtggcagttcatcaacagcgtaaggccctcaacatgtggaccgccggcgagaaggagacctttaaggag
aagtatttgcagcatccgaaaaactttggagccattgcggctagtttggatcgcaaatcgccacaggattgcgttcgctactactacctcag
caagaagaccgagaattacaagcagctgcttcgaaaatcccgccaacggacgcgcagttcgcgtaatccagccaaggctcaggccg
cacagccgcagtgcattatcgattcgatgaccactggcgttatgacgcgattgcagcgcgaacagcaacaaaagtccggcggtcgttcat
ccgccgttgctgagcgtgagcgtgcggaacgtgccgccgagcgtgagcgtgtggcagagaaagcggcagctgatgctgccaaagcg
gctgaaagtgccgccgaaaaggcaagcgccgccactaaggctgtcgaagcaacggctgccggtgaaaaggtggccaaagcggctg
cagcagcggcggcggcagcagcaactaccgccacaaccgcaacaacaaccacatcatcatcaacatcatcatcatcatcatcagcat
catcagcatcaacagcatcatcatcgacggcaagtccagcgacattggcaggaatagccgcagataaaacggatgccggcaaaacg
gctagcgccagcgataagaatgcagcaacagctggtggaccaacggcaacgggaactccgacagcagcaactacacctgcaacag
caacagcaccaccagagatctcagcgggcggcgaggccaagagcaaaaatgccgaggaggaggcagctgccacagccggagca

gcaacggtggccactgccggtacaccggcaaccggagcaagtgcggccagtgctggcgaggcgacaacggcaacgggcgccacg
gcgacagctgccgccaagggtgttggcaaaccggaaacagcgactgagccagcgggaacggctgctaagggagctgactctcggcc
agatgccaacgatccgctggccaaaactgcttcgaaggctatcaatgccgagggctataatgctatcggtggcaacagcagcagcagc
agcagcaatgcaacgggcgcctctgctccagtgcagggcgttaccttgaatggctttaagcctggctatcaaaccgtggtcatggccaatg
taaaggcatccacgggtggcgatgattctggtgcgaacgctggtggagcagcacccggatcgttagcagcaacgaatgcatcgattgcg
acgagtggcgataaaatcgtcaagacaacgccatcatccagggcgccaaattctacatcttcaacggcggcgaatgagagtagtagtg
gtgccggagtgaatacctatggccacaccgcaacaacggcgggcaattatcttggacagaagctgaaggctgcccaagttgagggtttg
ggagctggcaatgaattgcattcggatgttagtgaatccaagaggaaacgattcgaactgaattccggtgaggcaggtggaaatgcaac
gtcagctatgaccaatagcagcaccagtggcagcatgaacatcagcaatagtcacggattgaaggccaatgccaaggatggatcgatg
atggccaaaacatcgatggcatccacatcgtcggcatcggttgttgtcacatctacgccatcggcatcctcatcgtcattgtcatccgcgtcgt
ccatgctgttgataagtgccgcatccgtaatgtccacggcagcgggtgcaacgtcctcgtccacggcgacaacaacggccacagcttcg
gcgatctcgttgccgctattggcggatggcagtggcaactcgatggtcaatgcaaacgagattctcgccctcgatggcaaagacaaattg
gccagctgctttgtgtgcaaggcggaggcgtgtccccgcacccgtccactaaagaaggggcgtggccagcagtatggcattccggacg
aaacaataccggcgggtgcccgcgtctgcaatagctgtcaatgcaaatcggtgcgcagccgctatccaaattgtccgctacccacatgcc
caaatcccaaggatcgagcccagcgtctgcggaacattccctcccgtctcttcgaactggcgcccgaggtgcgtgatccgttgatggccg
aatttcaaataccaccgcatgcaacacgctgctgttcggcctgcctaatgcgcattcgtcgcaagctcgatccgcagctgaatctcaccgat
ggctccagtggcggtgccggcagcggcagtggcggcgatgagacggatgtcagtacctcgtcgtgtgatgaacgagagccgggcgga
tccgataccgcttccgttgagagtccggagaatttgcagcggcacaagtcccttacgatggtcaaacagcaacagcaacagcagcaac
agcagcagcagcaacaacagcagcagcagcagcaacagcaacagcaactatcgcagccacagccacctccgccagctccgcag
cagcagaagggttcgtctggacgaggtggtgatcagggtacaccactgattatcacaccaacccgaatgagcagcaagtcgggatcgg
gcggtgcgcaaacagcgggcgacaatgaacgcctactgccacccgccgctggccaggcacccaagaagcaaaagaccagcgag
gaatacgattcctcggccacggaaacggcggatgaggagaacgaaaactcgccggccaatcgtcagagtcccaaggtactattccat
ggccatggacatggacatggtggtcatgccaataatgtggctggtctgcagccgccggtggccaatatggggacgggtggtggtgttcaa
ccaggtggagctgctgggcaacaggtcaacggaccgattagtatgcgacgcgaggcggtcaacaatgttcaggattgcgtattctcggta
attgagcgttcgctgaagcacaagggtccgcaaccgaagggcggacagggtcagcagcagggtcaggggcaaggacagggccag
ggtcagggtcagactccaggtcaaagccagtccccatcccagcagcaacagcagcagcagcagcagcaatctgccaataatctgga
acgcaaggagcttaccattgtaagggaataccgccaggatccgggtatactgaagcagcagcagcagcagcagcaggctggcggag
caccgcccacctcggctgccggtagtttgccgcatggcacctccgtacagaagctaacaacccggccagctgcagtggcaccaccgcc
accggctcatccactgacacccaccagcactggatgtgcgggcagcaacaacggaaccagcgatagcctagccaccttatcggtggtc
aattcgcacatgggcatggtgggaattggtcatccgggcccgatggcccacgcaagttccgccggaggaatcggtgtggacaaggcga
ccataacgccggttgtcaagagctctagtggatcatcgaagagcggcggcggcagtgcgtccagtcactcgacagccacaccgccgg
agaccataatctacaatgtgccagtggcgcatccgcaacgtggtataccaccaccgtcgcagcactcggtacatccggcccatccgtcg
catacacagcatccggcccatccgcagcattcgtcgcatggtcagcatacgcagctgcaagtgccggaaccggagccgcaaacattgg
acttgagcatcaagaagccgccgcgcgatggccattcgccgcacacgggagccggaggatctagctcgtctggctctggatcgggtgg
accgtcctcatcggatcgacatcatggcccaccaccgccgaccatgtcgatgaagcacattgtacgctctggtggcatgtatcgcggtgac
accgtaactgttccatcgctggctgcacccagttcgtatctgtatcccacacggagtgtcaagaccattggcggcggtggcgtggtgcccg
gtgtgctgcccggtgtgcctggtagtgcactatatctacaaccagtaccagtgcccgtacccatttccatttccggtcaaggccagttgccgc
cgagagctggacaaccgccaccggcacagccgccatccggcaggggtgtggccaaggtgccacccaagctaagtccccagcaggc
ccatcacttgcatcccagtcacggacactcgccatcgcagcagcagcaacagcagcagcaacagcagcaacagcagcagcaacag
gcggctgcagcacaacaacaattgctggtgaaaagcggctcaatcacccatggaacaccggccaatagtgcccagcaacagattatc
gttcatgcaccagcgacggcagcagcagcaccctcctccctgctcagtcccaagttcgatggcttggtcaggcaaacgacaccagagg
gtgtgggttccgttggacccggaggagcaagtggcagcggcaagcatggctcgataacgcagggtacaccgttgcacatgccaccgc
atcatttggagagcaaacggccatacgagagctactacaagagctcgcagcgtcacagtcccgcccaacagccgggtggcaatcagc
aattgccgccgccgccgcagcaatcgtctccacaggcgccacctccgcagggctatggtgtcggtgtgagctcaccctatgcccgatcgc
cgttcgccggtgtcgtggaacagccgcaggtgctgagcacgcgacaaattgtgatgcacgactacataacctcacagcagatgcaggg
acagcaacagcagcaacagcagcagcagcagcaacagcgcaacatgtcgcgcggcagcagtgccagtggcggtggaggcggcg
gtggttccgacaaggagtcaccatcgccacgaaacagtgtgggcagcgccagtggtttcgcctatggcggcgacaaggaatcggcacc
ccgtggccgaccggagtactccagtcgtgcatcgccagccgatcatgttaatagtggtcacgatgcattggcctcgtttgtggacgtggccg
tgcagcagccgcagttaccggtgccgtcgcaaaaggatgacaagtcgccgggtccatcaacggcgccgggacaagtgcccggatctg
gtccaccacttggaccatcacctctgccgccacacgctgtggtcggcgtggcgcagccaccgccgccgaccgcacaccacgatcaacg
gtatcgcgatctgaccctgcatcaccatcatcatacactggtgcagcagcagattgcgcaacaacagcattatcgtagcctcaatgtggcg
gcccaggtcgatatgcagcggcagatggatcaggcgaaacgtgtgatgcgccaccaacagcaccaggtccagcagcagcagcagc
agcaacagcagcagcagcacaaccatgccctggaacgggacagggagatgcaggaacggatgcgcgaacgggatcgtgagcgg
gaaagggaaagggagcgtgagcagcgggaacgagaacgtgaacgagaacgcgaacgtgaacgggagcgcgagcgacgtgaac
aggatcgtgcgcggcgtgtggtggccgaagagcgggagcacgatagtcgacgcatggagcgcatgttcgccggcaatgtggtcactgg
atcaggtggcgccggcggcggaggaccgtcgcccggtcaatttctgcgggcttcggttccggaaactggtccaccacgctcaatcccag
atcgcgaacgggaatcgtactatcgtcaggcacacggcggtccagctccagaggatacgcccgggcaactgagcgcccagagtctga

tcgatgccatcatcaagcatgagatcaatcgttccaatgatgccaccgctggcccgggtcgcgaatttccgcgcccatcgttcgttcatgctc
cgttgccgccacgtggttccggatcgggaggcggcaccggtacacgatcctcaccggccaatgtgctccatccgatgtacctgcgcgatc
tacgccagccgctcgacggcggagcgggatcaatgcttacggcggagaacaatggcaagcccagctcgtcgggatcgcccagtgtga
ttaatattgatttggatcaggagcgcatctcagcggcagcggcagcagttgcccagcagcagcagcagcagcaagctccgcctccgtcg
caatcgtcgcagtctagatccgtgcacggtcagctaaggacaccgacctcgcaagctggcggatcagcgcccagtccacagcagattc
atacgaagagcattacctttggggaactaaccgattcgataattaccagtgactacggcaccaatcctcatctgcggccaccatatatggc
ctatttgcaggagacgcagtcaattctgccgccggatcgctggaagcagaaccgtcgtatgcaacagaaggccgaggaagcgaagca
ccattcccagcagcagcagcagcagcagcaacaccagcagcaacatcacgcccagcagcagcagcagcagcagcagcaacaccatg
cccagcaacatcatccgcagatgccgggcactggctctggctcggctccaggtggtgctggtcagggcggtggatcgggcggcccggg
tagcggtggtggtggcgccggcagagccagcacacctggcgaggatgggcgcaacataatccgaatgccacaggcggtcagtccgc
gaaaatttaatcatgagatgatgctacatcatgtgatgggaacgacgggagcgggtggtgaagctggtcagttcttcctacccagccgtgt
cgttctgcccgagcagagaggaacgcccagcggtggtggtggagcgcctggagccggaggtcctggatccggaggcggtgctaccac
cattgaaaaatatgtaaagacccgcattgctgaagtgatgcgcgatgatattggttatggaaagaatcggactgtcgaggtgcgaacaga
ggatgaagtaaccgccgatatggtggcacattcgcatgccgccgtccatgctgcacatgtggcgcacgcagcccatgtcgcccatgccg
ctgctatggagttgcagcacagaagcaaggaaccaccgccgccagagatcagtgtgtcacgtaagacgcccaaccaatacgaggtg
gtagacgccagtggcgaccaggcggtggcaccacctggtgcctttgccaaggccaaggcagcgcatgccctgagtgaactgggtgca
gtcggtggtggggtgtcattggtggtgggcggcggctctggaggaattgcaggcggaccaggtggtgtctcagtcggtgtcggtgtaccgg
gcggcggcggaccaggaagcggtggcggcggcggtggcggtcacaattccagttcatcgcaagcatctgctgcggttgccgctgccgtt
gcagcggcggccagtgagtccaagccgctgctgttgtccaagtacgatgcactcagcgacgaagattagtt (SEQ ID NO:23)

**Protein:** AAF48196
**Protein GI:** 7292802

MKWKQLYAPLQCRSVLRRPQTTTTAAAAAAATATTTTLATITTIAATAATTTHQTVRNSSN
NNTISSSSNIISSNVLLPAATSNSNNNNYSNSSSSSSNNSSSSSSSSMIMDQRPIICINHLSASLISRI
KRRNSSSSSSSSNTKSSNNNRLPACPRISNACHQMQHPFTGEALEQQQQYAKQAAHLQQQQQH
QSHQQQQQQQQQDQRTNLHLQIHHHHQQQQQQQQQQQQQQQQQQQQQQQQKQQQHHMQQ
QQQQQPLSPPHPPGSSSNSSSAAAAAAAAAAAAAAVNPGYPPSSAAAAAVNSGYPPRPPQHRFI
QNTGYSIAPAPTYRDNPYSRHTQIQQQQQQQQQQQQQQQQQQQQQQQQQAAASMPEYQRAAAR
AAVAAVSAGKGNVSGQSSNSSSSSSGGGGGGGSAGGSAPPGGGVVQVSQSGGVLVMEAMPH
YASQPNSNPSQQQQQQHQQQQGGNPSGAGATSGAGGGGGGSGGSVMVGSLGRILMPHPQAL
QYTSEYLTNATAAVAAAMVNQRQHLQLQQQQQQQHPPEPFGGQQPYKKQRLSEANANNMNHL
PPHPQQQHQQQQQQQQQHQAHQQQHQRSSPAQVQQQQQQQMNSSRQSHNDMCRQVVTTP
MGMQLKVETLPQQQQKQQQHQQQQQQQQQQGRSQPVVSSMSTVVSQPVGTVTVTTAGLSAS
HSGSSGNVAAGLGTGNTGSASTEAYHPQVEAISPTLPSDSSIEERGRTSAKEDLLMQIQKVDNEIK
SAETTMETLRKKEKSLMEEAALAKEQRAAKELNDNNNDQEPMVELSWRSQMLAEKIYAANRKTA
QAQHSMLQNAAADESSPGSVAGRPWLPLYNQPLDVEALAMLIRQHQSQIRAPLLLHIRKLKAERW
AHNQGLVEKYTKDQADWQRRCERMEASAKRKAREAKNREFFEKVFTELRKQREDKERFNRVGS
RIKSEADLEEIMDGLQEQALEDKKMRSYAVIPPLMHDARQRRCAYHNENGLIEDMVAVHQQRKAL
NMWTAGEKETFKEKYLQHPKNFGAIAASLDRKSPQDCVRYYYLSKKTENYKQLLRKSRQRTRSS
RNPAKAQAAQPQCIIDSMTTGVMTRLQREQQQKSGGRSSAVAERERAERAAERERVAEKAAAD
AAKAAESAAEKASAATKAVEATAAGEKVAKAAAAAAAAAATTATTATTTTSSSTSSSSSSASSAST
ASSSTASPATLAGIAADKTDAGKTASASDKNAATAGGPTATGTPTAATTPATATAPPEISAGGEAK
SKNAEEEAAATAGAATVATAGTPATGASAASAGEATTATGATATAAAKGVGKPETATEPAGTAAK
GADSRPDANDPLAKTASKAINAEGYNAIGGNSSSSSNATGASAPVQGVTLNGFKPGYQTVVMA
NVKASTGGDDSGANAGGAAPGSLAATNASIATSGDKIVKTTPSSRAPNSTSSTAANESSSGAGVN
TYGHTATTAGNYLGQKLKAAQVEGLGAGNELHSDVSESKRKRFELNSGEAGGNATSAMTNSSTS
GSMNISNSHGLKANAKDGSMMAKTSMASTSSASVVVTSTPSASSSSLSSASSMLLISAASVMSTA
AGATSSSTATTTATASAISLPLLADGSGNSMVNANEILALDGKDKLASCFVCKAEACPRTRPLKKG
RGQQYGIPDETIPAGARVCNSCQCKSVRSRYPNCPLPTCPNPKDRAQRLRNIPSRLFELAPEVRD
PLMAEFQIPPHATRCCSACLMRIRRKLDPQLNLTDGSSGGAGSGSGGDETDVSTSSCDEREPGG
SDTASVESPENLQRHKSLTMVKQQQQQQQQQQQQQQQQQQQQQQQQQLSQPQPPPPAPQQQK
GSSGRGGDQGTPLIITPTRMSSKSGSGGAQTAGDNERLLPPAAGQAPKKQKTSEEYDSSATETA
DEENENSPANRQSPKVLFHGHGHGHGGHANNVAGLQPPVANMGTGGGVQPGGAAGQQVNGPI
SMRREAVNNVQDCVFSVIERSLKHKGPQPKGGQGQQQGQGQGQGQGQGQTPGQSQSPSQQ
QQQQQQQQSANNLERKELTIVREYRQDPGILKQQQQQQQAGGAPPTSAAGSLPHGTSVQKLTT
RPAAVAPPPPAHPLTPTSTGCAGSNNGTSDSLATLSVVNSHMGMVGIGHPGPMAHASSAGGIGV
DKATITPVVKSSSGSSKSGGGSASSHSTATPPETIIYNVPVAHPQRGIPPPSQHSVHPAHPSHTQH

PAHPQHSSHGQHTQLQVPEPEPQTLDLSIKKPPRDGHSPHTGAGGSSSSGSGSGGPSSSDRHH
GPPPPTMSMKHIVRSGGMYRGDTVTVPSLAAPSSYLYPTRSVKTIGGGGVVPGVLPGVPGSALY
LQPVPVPVPISISGQGQLPPRAGQPPPAQPPSGRGVAKVPPKLSPQQAHHLHPSHGHSPSQQQ
QQQQQQQQQQQQAAAAQQQLLVKSGSITHGTPANSAQQIIVHAPATAAAAPSSLLSPKFDGL
VRQTTPEGVGSVGPGGASGSGKHGSITQGTPLHMPPHHLESKRPYESYYKSSQRHSPAQQPGG
NQQLPPPPQQSSPQAPPPQGYGVGVSSPYARSPFAGVVEQPQVLSTRQIVMHDYITSQQMQGQ
QQQQQQQQQQQRNMSRGSSASGGGGGGGSDKESPSPRNSVGSASGFAYGGDKESAPRGRP
EYSSRASPADHVNSTPSPHRTPPPQRQGVIQRHNTGSKPPSPAAPPPSRMHMPPYQYAPSGHD
ALASFVDVAVQQPQLPVPSQKDDKSPGPSTAPGQVPGSGPPLGPSPLPPHAVVGVAQPPPPTAH
HDQRYRDLTLHHHHHTLVQQQIAQQQHYRSLNVAAQVDMQRQMDQAKRVMRHQQHQVQQQQ
QQQQQQQHNHALERDREMQERMRERDREREREREREQREREREREREREREREREREQDRAR
RVVAEEREHDSRRMERMFAGNVVTGSGGAGGGGPSPGQFLRASVPETGPPRSIPDRERESYYR
QAHGGPAPEDTPGQLSAQSLIDAIIKHEINRSNDATAGPGREFPRPSFVHAPLPPRGSGSGGGTG
TRSSPANVLHPMYLRDLRQPLDGGAGSMLTAENNGKPSSSGSPSVINIDLDQERISAAAAAVAQQ
QQQQQAPPPSQSSQSRSVHGQLRTPTSQAGGSAPSPQQIHTKSITFGELTDSIITSDYGTNPHLR
PPYMAYLQETQSILPPDRWKQNRRMQQKAEEAKHHSQQQQQQHQQQHHAQQQQQQQQH
HAQQHHPQMPGTGSGSAPGGAGQGGGSGGPGSGGGGAGRASTPGEDGRNIIRMPQAVSPRK
FNHEMMLHHVMGTTGAGGEAGQFFLPSRVVLPEQRGTPSGGGGAPGAGGPGSGGGATTIEKY
VKTRIAEVMRDDIGYGKNRTVEVRTEDEVTADMVAHSHAAVHAAHVAHAAHVAHAAAMELQHRS
KEPPPPEISVSRKTPNQYEVVDASGRRSAGSGSVSVSVSGANSHHSPYHPPAAAYAPSTYAFPY
SALNVPGAAGGLPPHQPLQLAHQAVAPPGAFAKAKAAHALSELGAVGGGVSLVVGGGSGGIAGG
PGGVSVGVGVPGGGGPGSGGGGGGGHNSSSSQASAAVAAAVAAAASESKPLLLSKYDALSDE
D (SEQ ID NO:24)

# Figure 10

**Name:** CG8045
**Nucleotide:** AE003721
**GI:** 7300335
**Transcript:** CT24072
**Sequence:**

gagtcaattaagggcaaagactcgagcggtgagcgtgcggctctactcccatatatacatatatatatatatatatattc
aacatcctaaccgatagaaaagtagcaacagcagcccagttatgtgcttgccaaatgcaattaacaactgattgatttggaaactcagca
gcgataatcgagttgaaaatcaaccaaataatacactaaatggattgaaagtgtcgcagtggcaaaagtgttgttagctagttagtgtgtctt
gtgtgctgttactgtgcgttccaaggattaaagtcgtggaggagaatggaacacgatagcttcgatgatcccattttcggggagttcggcgg
cgggccactcaatccactgggcgccaagccactgatgcccaccacaacggccatgcatcccgtgatgctgggcagtgtccacgagttgt
gcagccaacagcagcagcagcagcagcagcaacgattgcccgactgcaacaccatactgccgaatggcggaggaggaggagcag
gatctggcggcgctggaggatcgcccaactatgtgaccaagctggattttgtcaacaagatgggctgctactcgcccagccagaagtac
gagtacattagtgcgccgcaaaaactggtggagcagcagcagcagcagcagcatcatcatcaacatcatcagcactacacggccacg
cccccgcccagccacaatggcatcctgctgcacaagcaatcctcccagcagcaacagcagcagcagcagcaacagctggtgggcatcctg
gactatcatccgctcaatggcaagctggactactcctcgtcgcccaaagatcagtacgagccgcagcagctgcagcatctgtacggcgg
cagtccgcatcatctggaccatttggatcatggcagtgatggcttgctccaggactcctcgcccgtcatgatcaacggaggatccgctggtg
gcaagctaaagaagcccgatgagatgtgttcgcagttggaggcgggtggtgctggagttactccgccaagtagttcctccacggtggtta
atggcaccaccaatggcactggcaacgccaactcctcctcctccgctggcgtcggcattcaggggggcggcgggcacagcggggggcg
tggcagctcctgcagccaaaaaggacggaagcagcagcaagaagaagggcgatcccaatggcatcaagaagaagaagacgaga
accacctttactgcctaccagctggaggagttggagcgtgcctttgaacgtgctccgtatccggatgttttcgcccgcgaggagctggccat
caagctgaatctgagcgaatcccgtgtgcaggtgtggttccagaaccgacgggccaagtggcgcaagcacgagccaccgcgcaaga
cgggctacatcaagacgagcactccgccgacggcgacactgaatcccggaaccctagccccaccattcactagctacccgcagacga
cgacggtcacgccgccgggcagcatggacagctggaccagctaccagacgccctacgaactgacgccccagttcagcctgctctcgc
cggcggccagtccgtatggcacctattccggccagtacggtgcctatgtgcacgaaagccagctcttcccgatgcgtcactacgagtacg
gtagtcccacgcgcatggaaatgggcgccacaaccggttcggtggccggaaacggagatgagtcggtggccaatggcggcagctatc
agacggcggaactgcagaccgcccagcagcagcaactggccgacggcacactggtcaccgtccatgcccaccaacagcagcagc
agcagcagcagcaactcaatggcaagtacctgagcgccgaggaggccaaatacgtgcacctgcagtgccatcagagtggaggtggt
ctggagctaagtcccggcgccagctgtcatctggtggaggcgcagggccagcactatgtgaccacggcgacgggaggagctgcatcc
gcgggcggcaccagcagcgatgacaacgacagcggcggcatgcagactgtgatcaagagcgaggaggtctcccaacagcagcag
cagcagcagggacagagctacgtcctgccgcctttttttgcactgagagaaatgctcacacaccggcgacagcaacaacaccagcgac
gctgggccacgcccatcagcacgccgaacgcccgtttccgtcggccatttgaagggatggaggcgtggcgtgtgcagcatttccaactg
tgtttctccgactgaaagtggggctccagaaaagcggattcccaaagataaagcctcataagtagtagaattcatcatcggattacttaaa
actagttgctttcaaaaaatttggcgagagtccacaaattttatatttaatttgataatattgtttttttagtttatatttccaatcccttggagtttttcta
gccagtcccacacagtttctagccactgttttcatattgccataggttccttttaggtttaatgacgcaagttgcaacaacaacgggaatccac
aagtcttgccaaatcataatgaggttattatgcctgtctaagtctaggttaatctagggctaggggctctgcaacgagagccccgcccccag
aatgcattccaaatgtgccttaccaaaacgggtgacgagtgacctccctccaaattggaaagcttcgttgcccaacaaacttagttttagtta
ttctactcgtagactaagttccatttgctgtttttgctgtagttagacaataaagtgctttttatattgtatactagctcatttggctttacaaattttatta
cttactaattttgtaaaatggcttgcaaattttgttacaactacattcaatttagtttaatttctaattgtaagcaattacataaactaggacactttgc
aaatgtatataatagacgtataaataaatcgtattttgtaccaataattgatcagcttccaattcaaaaaaggaacaacataaaagactcat
aaaaacaaaactgatggttaatacacttgtttcgaagacaagtattaaaagtaatgcatagaaaataatatagcagattcacaacataatt
aatcgctatggagcgacttcagattctttgaaagtaatcaaaatcaatgccaatcagaactgagcaaggaatcacaaatgggatttcaggt
acagtacgaaataagaaaatactttacggactcaatagcaaagcaaatagtgcctattttcacatggactcagttccctgctccaaaaccg
cctgccaatcgatgcctttgaagaatggatgagcctgcaattggtcgtagtccaaacgtagctccggagaaagctgcagcagttgctcca
gc (SEQ ID NO:25)

**Protein:** AAF55517
**Protein GI:** 7300358

MEHDSFDDPIFGEFGGGPLNPLGAKPLMPTTTAMHPVMLGSVHELCSQQQQQQQQR
LPDCNTILPNGGGGGAGSGGAGGSPNYVTKLDFVNKMGCYSPSQKYEYISAPQKLVEQQQQQQ
HHHQHHQHYTATPPPSHNGILLHKQSSQQQQQQQQQLVGILDYHPLNGKLDYSSSPKDQYEPQ
QLQHLYGGSPHHLDHLDHGSDGLLQDSSPVMINGGSAGGKLKKPDEMCSQLEAGGAGVTPPSS
SSTVVNGTTNGTGNANSSSSAGVGIQGAAGTAGGVAAPAAKKDGSSSKKKGDPNGIKKKKTRTT
FTAYQLEELERAFERAPYPDVFAREELAIKLNLSESRVQVWFQNRRAKWRKHEPPRKTGYIKTST
PPTATLNPGTLAPPFTSYPQTTTVTPPGSMDSWTSYQTPYELTPQFSLLSPAASPYGTYSGQYGA

YVHESQLFPMRHYEYGSPTRMEMGATTGSVAGNGDESVANGGSYQTAELQTAQQQLADGTL VTVHAHQQQQQQQQLNGKYLSAEEAKYVHLQCHQSGGGLELSPGASCHLVEAQGQHYVTTA TGGAASAGGTSSDDNDSGGMQTVIKSEEVSQQQQQQQGQSYVLPPFLH (SEQ ID NO:26)

**Transcript:** CT24102
**Sequence:**
ttcggaattcttggtttttttccaagagagttatgtccctggtgccagaagcctctacctcgagagcgcccaagtactgcta cttcttcaagaccctgctggtggaggaactggagctggaaacttcctaccacaacctgcactacggtcagtgcgcattaattggacgtctgg cgttcaaggcgaatcagtaccgcctagaaaacgtgcgcgttaagtgtttgccaaagaagtactccttgggcgagggcaccgtatccttgct tattcttggccttacccatgacaaagtcgtcgaaaatcgcgtgagtaccggccgctactgcattgtgcgtggtgaagtggtgctacataatgt gcagcatcccaaaggtgccaaactgaccgccggcggtgtctacgacaagatcaatagtctgtccaacgatccgttggcccaaaaacag tatctcagcgcgctacttgccacctacagaccggccatcgacttatggtacatccaggtcatcgacagagccgaggacttgctgactcgca ggctggagatgagaagtctgatcgagaaatgaacacacaccacctgaccacctcgcgaaccaatccgcagttctatatattcagtcagct ctttggtaacgatttcgactggaataattgcaattggggggcattcaaaacgttacctggaaagaattctggcagcgtgaggggcatgag ctgctcgagcaaaagtggcacaagcggtttcccgaatacaaagatcttatcgaggtggtttcgtcggaggagcaaggcctttggcagga actgtgggagaagcacgccagcgattacagtgaaaagttctggtatatatttagctgtgcctttgagaactaccaacacgatttggccaaat catttggccttgacgtcgctgaaaactcagaagatgttgaggaggacttgcagaacctaagccttacaaataagccaataaaatcgaaa cagcaaagggtcacagagaaagaggaggacgaatcatacttgactgccaacgacgatcccgaggattgtgacgaggaacaacagc tgcgccttctcggattgccaacatcatttgcgtcgaaggcagatataattaattatagaaaggaaagaaagacacagtacgaactatctag cagcgatagcgaggacgatcatgatctgttgaccatggagaacaacgaaaacgaggctctgcacggcgttcagagcggttctgttaaa aagaaaaaacgacggcaaaggcaaataaaaaaactcaaggccaggatgccagagtgcatgcaagaggataacaacaaaatggtc aagtactgggtcaagcgattctcgctgttctcccgcttcgatcagggcattcggctggaccgcgagagctggttctcggtgacacccgaga agattgccaaacaaacggcccgacgactggcctgcgacgtgatcgtagatgctttctgtggatgcggcggcaatgccattcagtttgcaa acacctgtggacgcgtaattgctgtggacatcgatgccgagaagttggccatggccaagcataatgccggcatttatggtgtggcccataa gatcgagtttatccacgccgattttctgcagtttgcggccagcacaaagctgcgcccaaatgtggttttcctgagccctccgtggggtggtcct gactaccaaaagcaggccaccctttgatatcgaaacaggtctcctgcccgtgggtgctagccaactgatgcagctgtcccgtagcctggca tctgacgtagctttctttctgccacgcaacgccaatatgaagcaggtggtcgctctaagtggagttgggcagcagtgcgaggtggagcaca actatctggacacccgaatggttgcgctcactgcttattatggtaaaggaatcataaagggttcagtgggagaagatgagtag (SEQ ID NO:27)

**Protein:** AAF55519
**Protein GI:** 7300360
MTERENNVYKAKLAEQAERYDEMVEAMKKVASMDVELTVEERNLLSVAYKNVIGA RRASWRIITSIEQKEENKGAEEKLEMIKTYRGQVEKELRDICSDILNVLEKHLIPCATSGESKVFYYK MKGDYHRYLAEFATGSDRKDAAENSLIAYKAASDIAMNDLPPTHPIRLGLALNFSVFYYEILNSPDR ACRLAKAAFDDAIAELDTLSEESYKDSTLIMQLLRDNLTLWTSDMQAEEVDPNAGDGEPKEQIQD VEDQDVS (SEQ ID NO:28)

**Transcript:** CT24092
**Sequence:**
ccgccgcccgatacggtcacaccgctcgagaagtagagaaaaaagaaggcgacgaaccacaggcagcaagc aaaatcgacgccgcacgtaccgggagcatttaaataatttttctgtggattaattttaccgaaaaggcttcaacacaatgactgagcgcga gaacaatgtgtacaaggcaaagctggccgaacaggccgagcgctacgacgaaatggtggaggccatgaagaaggtcgcctccatgg acgtagagctgaccgtcgaggagcgaaatctgctgtcggtggcgtacaagaatgtgattggagcacgccgtgcctcgtggcgcatcatc acctcgatcgaacagaaggaggagaacaagggggccgaggagaaattggagatgatcaaaacctaccgcggacaggtggagaag gagctgcgcgacatctgctcggatatactgaacgtgctcgagaagcatctcattccatgcgccacatccggcgaaagcaaagtattctact ataagatgaagggcgactaccatcgctacctggccgaattcgccaccggctccgaccgcaaggatgcggcagagaactcgctgattgc ctacaaggcggccagcgatattgccatgaacgatctgccaccaacacaccccatccgtttgggcttggcattgaacttctcggtgttctact atgagattctcaactcgccggaccgcgcttgccgcttggcgaaagccgctttcgatgatgccattgccgagttggatacactgagcgaaga gagctacaaagactcgacactcatcatgcagctgctgcgcgacaacctcacattatggacgtccgatatgcaggcagaagaccccaac gcaggtgatggcgagcccaaagagcaaatccaggatgttgaggatcaggacgtgtcgtaatttaagtaaacccccgcccttccatttaaa acacattatataccttctaaagtaaatataataccatataatatatatgcatacaacaacaacaacaaaatcaacaacaacacgacaaca acttcaagaacaacaacatggaaccaatgcggcaacaacaaccatagcagtcgatcaccacaacagcagcacccgtatgtgtatcaa catatttgcaacatgataacagcaagcaaagcagcaggaacacaaattgtcatccacaataaacagcatggaaaacaacagcacaa aaacagaaaaaaaaaacaactatccaagcaaaatgtttgaaaattgggcggcctggtgggcgtggcgcggctgttgctgcggcccag aatcgaatcgcaacataaaaaattcgcggtactattaagttggcgtcgcttcgcaatttggctccttaacgttagaaaactgcaacatcaagt

ctactacatttgaattttttgcattcgagaaaccaagcattttattatatttgaataaatattatatttaattatgagaagcaaaaacgaaacaaat aaaattcattt (SEQ ID NO:29)

**Protein:** AAF55523
**Protein GI:** 7300364
MVHPGHRQSRGLADSQAGDEKSDREMNTHHLTTSRTNPQFYIFSQLFGNDFDWNNCN WGGIQNVTWKEFWQREGHELLEQKWHKRFPEYKDLIEVVSSEEQGLWQELWEKHASDYSEKF WYIFSCAFENYQHDLAKSFGLDVAENSEDVEEDLQNLSLTNKPIKSKQQRVTEKEEDESYLTAND DPEDCDEEQQLRLLGLPTSFASKADIINYRKERKTQYELSSSDSEDDHDLLTMENNENEALHGVQ SGSVKKKKRRQRQIKKLKARMPECMQEDNNKMVKYWVKRFSLFSRFDQGIRLDRESWFSVTPE KIAKQTARRLACDVIVDAFCGCGGNAIQFANTCGRVIAVDIDAEKLAMAKHNAGIYGVAHKIEFIHA DFLQFAASTKLRPNVVFLSPPWGGPDYQKQATFDIETGLLPVGASQLMQLSRSLASDVAFFLPRN ANMKQVVALSGVGQQCEVEHNYLDTRMVALTAYYGKGIIKGSVGEDE (SEQ ID NO:30)

# Figure 11

**Name:** CG10420
**Nucleotide:** AE003751
**GI:** 7301280
**Transcript:** CT29244
**Sequence:**

ctgaaatctgatttaaaaactctcacgaaacttacttaaaaccaggtgaaataaagactgaatacattctggaggcaa
gacaagctgaagcagtatgagtggcaagcaggtagtcatccttctgggcagtgtcctgatcctgggatgcttacaagtggcagcggcaac
ggaaacggataacaaaaccaacgatttcgtggccaccgacgaatggcagacgattgcggaaggtcaagctattccaaggggcctgca
cgtgcgcatcaacctacaaacggggctcaaggaagccaaactcttagacgaaagcgaacgtggcacgtcgctgcagagtcaaccgg
atgatcagaatgctcgggaatctcacgatgacaacgaacccctggctctggactataaaccggacataatcgaggagtctatacggcgg
gtcaaggagcagaagaagagctatgccgagctacgcaaagcgtacaaagagttccagaagaactttcgcaccgacggagaactgat
cgtccagttaatcgatcagtttcggaacttcagcagaacgccgctagagtcagagatgcgctccaagctggactgcctggaaaatctgga
gtatttgctccatcagatagacaatgccttgatgttcattgataatggggggattggatgatgtactactgcccattgttgtgaacgataccagta
catccctgagagtgtcggccatgcgtgtgttgggctcgctggcgagcaacaatcccaaggcccagatcaaggtgttcgaaaagaatttcg
ggtctcatctggctcagattctgaccagttccggaaatgtcggtgagatctccgctgcattgcacgcctttggagccttgttgcggaaatttccg
ctagcccagcagcgagtgctttccacctcgggtacacaggctctgatcaaggtgctccagagtccggatgtggagctgcggagcaaggc
gaaggtagtgactctcataagcgacctagtgctggagaagcggtcagtgctggatgtcagcaaagatgatcccgaagcctcatccacaa
tggcgcaatatgtgctcttggacttcgagtcgtggctgaaaacgccgggctactgcgcggcggtggacactgtgctaaccaaggagttcct
gctcctcctagagcaaccggaggtggtggaacagtttgccaccgcattggagaccaccgaagatatgtgcaccagcacgtggtcgcaa
agttccggtctcaggcatgcactattaaccgttcgcaatcggtatgccaacagcacggacgagtaccgactggaggtgtcccagattttgg
ccaaactgtgcgagagattgttcaacaagcccaagcacaccgaactgtaa (SEQ ID NO:31)

**Protein:** AAF56422
**Protein GI:** 7301293

MSGKQVVILLGSVLILGCLQVAAATETDNKTNDFVATDEWQTIAEGQAIPRGLHVRI
NLQTGLKEAKLLDESERGTSLQSQPDDQNARESHDDNEPLALDYKPDIIEESIRRVKEQKKSYAEL
RKAYKEFQKNFRTDGELIVQLIDQFRNFSRTPLESEMRSKLDCLENLEYLLHQIDNALMFIDNGGL
DDVLLPIVVNDTSTSLRVSAMRVLGSLASNNPKAQIKVFEKNFGSHLAQILTSSGNVGEISAALHAF
GALLRKFPLAQQRVLSTSGTQALIKVLQSPDVELRSKAKVVTLISDLVLEKRSVLDVSKDDPEASST
MAQYVLLDFESWLKTPGYCAAVDTVLTKEFLLLLEQPEVVEQFATALETTEDMCTSTWSQSSGLR
HALLTVRNRYANSTDEYRLEVSQILAKLCERLFNKPKHTEL (SEQ ID NO:32)

# Figure 12

**Name:** Hsc70-2
**Nucleotide:** AE003698
**GI:** 10726497
**Transcript:** CT23565
**Sequence:**

atgggtaaaattccggccatcggcatcgacttgggcaccacctactcctgtgtgggagtttggcagaacagcaaggtg
gagatcatcgccaacgatcagggaaaccgcaccacgccctcgtatgtggcattcaacgagacggagcgcttgattggcgatccggcca
agaaccaggtggccatgaatgccaagaacacggttttcgatgccaagcgattgattggacggaagttcgacgacaagaagatacagg
aggacctgaagctctggcccttcaaggtcatcaatgagaagggaaagccgaagatcgaggtggagttcaagggcgaacggaagcga
tttgcccccgaggagatcagttcgatggtgctgaccaagatgcgcgaaatcgccgaggtctatctgggtggcaaggtgaaggacgcggt
ggtcacagtgccggcctacttcaatgatagccagcgtcaggcgaccaaggatgcgggctccattgctggactgaatgtgctgaggatcat
caatgagcccactgcggcagctctggcctatgggctggataagaatcttaagggagagcgaaatgtgctcatcttcgacttgggcggcgg
caccttcgacgtctccgtactgagcatcgatgagggttccctgttcgaggtgaaggccacggccggcgatacccatttgggtggtgaggat
ttcgacaaccggttggtcaatcattttgccgaggaattcaagcggaagcacaagtcggacatcaagggaaatgccagagcgttgagac
gcctgagaacggcttgtgaacgggcgaagaggacgttgtcctccagcacggaggcatccttggagattgatgccctgcacgagggcatt
gacttttactcgaaaatttcgagagcgcgtttcgaggagctgaacatggacctcttccggtccaccatgcagccggtggagcgggcactg
agcgatgccaagatggacaagaaggccattcatgatgtggtcctggtgggcggctccacgcgtattcccaagattcagaaactactgca
agatctgtttggtggcaagcagctaaatctttcaatcaatcccgatgaggcggtggcttatggagccgccgtacaggctgccattctcaccg
gcgtgggcagctcacagatccaggacctgctcctcgtggacgtggctcccctttcgctgggcatcgagaccgctggcggcgtgatgacca
acttggtggagcgcaatgcccgcattccctgcaagcagcagcagatcttcaccacctacagtgacaaccagaatgcggtgaccattcag
gtgtacgagggcgagcgggccatgaccaaggataacaatctactgggcacctttaacctcaccggcattccgccagctcctcggggagt
gccccagatcgaggtggccttcgacttgaatgcggatggcatcctgaatgtgaatgccaaggacaacagcaccgggaagagcgaaaa
gatcaccatttccaacgacaagggcagactttccaaagcggaaatcgatcgcatgctctcggaggcggagaagtacaaggtcgagga
cgatcgtcagagggagcgggttcagtccaagaacaatctcgaggcatacatctacgcatgtcgccaggccgtcgacgacgctccatcc
ggcgttctttccgaaaccgaacgctccaaggtgcgcgacaagtgctcctccgaggcatcctggctggacaagaactcgctggccgaga
aggaggagttcgagagtcacctgaaagagtgccagcgcatctgtactccaataatgtcgaaaatccatggcggcaaaaagggcaaat
catcgatgggcaagggttcccatcccactgtggaggaggtggatggatagtacgatccaccgacctaa (SEQ ID NO:33)

**Protein:** AAF54899
**Protein GI:** 7299717

MGKIPAIGIDLGTTYSCVGVWQNSKVEIIANDQGNRTTPSYVAFNETERLIGDPAKNQVAM
NAKNTVFDAKRLIGRKFDDKKIQEDLKLWPFKVINEKGKPKIEVEFKGERKRFAPEEISSMVLTKMR
EIAEVYLGGKVKDAVVTVPAYFNDSQRQATKDAGSIAGLNVLRIINEPTAAALAYGLDKNLKGERN
VLIFDLGGGTFDVSVLSIDEGSLFEVKATAGDTHLGGEDFDNRLVNHFAEEFKRKHKSDIKGNARA
LRRLRTACERAKRTLSSSTEASLEIDALHEGIDFYSKISRARFEELNMDLFRSTMQPVERALSDAK
MDKKAIHDVVLVGGSTRIPKIQKLLQDLFGGKQLNLSINPDEAVAYGAAVQAAILTGVGSSQIQDLL
LVDVAPLSLGIETAGGVMTNLVERNARIPCKQQQIFTTYSDNQNAVTIQVYEGERAMTKDNNLLGT
FNLTGIPPAPRGVPQIEVAFDLNADGILNVNAKDNSTGKSEKITISNDKGRLSKAEIDRMLSEAEKY
KVEDDRQRERVQSKNNLEAYIYACRQAVDDAPSGVLSETERSKVRDKCSSEASWLDKNSLAEKE
EFESHLKECQRICTPIMSKIHGGKKGKSSMGKGSHPTVEEVDG (SEQ ID NO:34)

# Figure 13

**Name:** CG10805
**Nucleotide:** AE003615
**GI:** 7297167
**Transcript:** CT30278
**Sequence:**

atgagtactgcgttggcacagcagctgcagaaactggctgcgccgcagtcgtcggtaactttggcagatgcacgaag
tcgcgcctccatactcttcgatcccaaggaggcggccaccaaggatcggcggtccatttatgagatcgggttaacgggactgcaggagct
gaccgactttaatccggccttcaaggagttccagctaacgttgttcgatgaggccacattgacgctggagcgttccgtggagctgccggag
attaacaagatgctggacgcggccattgcaaagtttctgcgccttctttcgccgtaccttctgctccgccccgcacacatggccttcgaatggt
tgctgcgacgattccaggttcacgagtataaccgtagcgaagttatggccctgattctgccctatcacgagacaatgatctttgtccagatcg
ttaagaccatgaggctccggtcatcggatggcgactggtactggctgcgcccgttgcagcgtcctggcgttccgctagccaagactgctatt
attaacagagccgccagcaatcccgccttcctcggcttcatctgccaaagtacccagaaggcggttaaggaactgggtcctcgagccca
ccaactgcaagcccagatcaacttctatgccaccgtcgtagttggcgccctgcaaacggccaaaccactgcaggactggcacatcacc
actattctggagtcgctgcttcgtggcctgatttccgataatatcgactttatggcggccgcctatgtgatcgttgctcagcttgtttcccgcacca
agctcaagagcaaggtgtgcaatgccctgctggaacgcgtggccaactgcccgtttgagaggcttcacagtgagtcgctgctcctgcttgt
ctgcatctatggcaaacagcaagccgcattgccgcactttaagccggaaaccatactcaatctggttgggaagaagtggcttatctccact
ctgtcctccctggccaaaggcaacatcgccatccagtctatctgcatgccactcatgaccggagccgtggccgccattcgggacgatgac
gcctcttctaactcatgcaaactgttcttggacaacctgctgtcagaggtgccaatgccaaagccgactgctcagcaactgatcaattgctttt
tggatacttatgtagagactgcgatcgatgcaccagagcccatggaaactaactcaaacgaagatgacgataccattgtaattgattcgg
atgatgagatcgagacggaaaagaccacctttcaagcctggtattccacttacctcgagaagctggagagacgctatccagaagcttttg
atctgagcgttaaagaggccttgagatcgaaatcctccacaagcaatcgccagaaggccttaaagttggctttgggcttccgtctcaacac
tactgatgaaaaagcgaagcacgcctacgaaaaactataccactacagcgctgactggcgacttagtgctgttcagaaactactccaga
atttgaatgtgactaaaaagcgggagagaagcgttaagcttctgcaggaatgtctgcccgataggataaatgatgatagcggtgctgtggt
ctcaaccttgctatcattgcctaccgaagagctcgccgagatgttgggtccgttgcctctggctcaaaccctatgtcatctgctgtatcgtgctc
agtccgaaaaggatgaggaatggcaacctgtggttcccctcgctgttcgtcacttgacctctgccttggtgagcggaagctatgacacaaa
tttggtgctattagcgctgatgccgcttctcttccctggcgaagcattagctgaacatcagcacaaggctctgcggattctacttggcagtgatt
ttgtcagtaaagttccgttcttggcagaacttaaagtgagcaacaaattcagcgactttaatgtcggtgagcaccgtcagcacttcctggaca
tcatagcgagcagcaatcaggaactgagtagtcaggaacgtgcgttgctccaaagcgtagaagatcatggcggtgagttgtacattcag
aaagcctcccagctgacgcacctgttactcctgctaactgcctacgccaagagggaactacagcctcgggaaagccttcacatgctgga
gaaaattgggctttatagtcggcgcctccagttccgtgtcgtcaatggtagtcaaaacacacagaattgtgcacctctacaattgtatgtggat
ttcctattgactttggttaagaacaccaaatggacagctctagcatccactccttggaatcagatgaccgatgagcttcgcctctgtctgcgcc
tgctggaaataatatgcgcccaagtgttttcggaaaaagctgatcagccagagcgtcaggagtggacacgagccctgcagcaaagtttg
caactaatcctgcctgaagctcaagacagattggaagtgctatcaaacttctatgtgttcgagcgactgccagaattgtggcctcgagactc
tgactatgccgtgttccggttgcaagggtttattattctagaagcggtgctgtccaatccgaaatcccaaatagactgcggtctggttcatgtgc
tgcgggtggccaacgcctgtggatctcccttacaaactctgcgagtgcaggccattaacattctgcaattaatcagtaatcgcaagctggtg
agccatgtggagcaattggtgcgttcgctgctgcagcgcaagagtgagttgagcatggatcatgaacaatatgccctgattctatacaccat
tctggaaccagagaaggcaactgccaaggaaagactcgtgctgtcaaaactgaaacgatctgttttggctttggcctccgatccgaagca
gtctcccatttgtacggcgtctttgcttgcggccctaaagcacgtcaacgatgagaatttcttaaacgaattgctgccgctgggtcttgactccc
tgaagacgataactgcgggagaagacaaccagaatataaaacagctgccatggccacatagcgaaatctacaagtcggtgatagaa
aggtttgaggggcgtgttgcgctgaatgtattgctgcgaaaggatctggcttggaaattgtttgaagacagcttcgcccagtatgacacctat
gtccaacttgaacagaagctacagcctcttccctgcgtcctgctaaacagcctcacgccggagacctttgagcaaatgcatgccaagcac
aagattgctctaatcaagctgatagtggagtcggctaccaattcggacaatgacagcatattcttggctagccatcgtttgctgaagcgctgc
cgtttggattgccagccactcgtaccgatcctgttggagatggccaacaccaaggtggagaagaaacaacccgtcaaacgacgatcag
tacaggcaacccaactggatctaaccagcccatactggaagcagggaatgacccttctcgagctgttggagcacaagaaacagctggt
tggagcggagctgctgatcccaccgctcttcgaacttctacaggcctgtcttacgatggaagagcatagtgccgccgagtatcccaagca
gctgattctctcaagcttgctgcactgctgtcagacggcacagtcggccggtgtgcagctcgttaaggctatgcctgaatctagcttccgcat
cgagctggtggtgcaatccttgaggaatacccgaaatccgcagacccagcagcatgcgctgcttttcctgactcactgtgctggcatgtatc
cgcaacaagttctacacaagatcgttgaaatctttacgttcgttggatccacggtggcgcgacacgacgatgccttcagcctgcatataattc
acaatgtggtggagtcgattatacccattctgcttctgaataccggacataatgagctggttatacccgtgcttaaggtgttcgctgatatttgc
acggacgtgccggtccatcggagattgccattgtatgctaccctgttccgagttctggagcccaaggagcacctttggcagttcttgtgcatta
tctttgagtctcaagtgcttctggagcaagttccacaaaaagtgtccacagacaagtcgcggctggactttgcccgtgaactgacgttgatgt
tcgaagatccgacggtcgcaattcagacgtgcatccgtctattggattacttggccaagttgccggcaaccaaaagcagtctttcgggtgg
atcaggctcctctgtgctaagcacggaacaacagctctttgatgttcggacacggacgttcaagcaactgcggcactacaagtacctcatc
atggacttcctttctggaattagcagctgcaacgaatgggagaagaagatgaagcgtcctgatcccaacgagctacttccgtattatcagg

aattcatcctaaagacactcgcgtatgtgggcgtacttaatggagcattggaagcagcctccgaaacgccttctctggaaaagttctggcg
cgtgctagctaaccatgcacacgatgtcttggataacgccatcggtcttttggcaccgcaacatttcatcagtgtgataaccgagctactgaa
gcatgatcatgtgtatgtgcgcattaaggtgatggacttgttggtgaccaagctaagtccgtcgagcgattacttccagcaatccaacgctga
acactttggagtgcttttcgctcctttgcaagagattatcaacggaatcctcgagggcagttccaacagtgcgcagcaggcgaagcttcaa
cagactgcgttgcatgccctacagttgttggccctccgtcacggacgcgactacatcgaggagtgtcgctctcttctggccaccttgaccaa
gatcaccaaacgacgtgcgaacgtgcccaaggcggtagtgggcaatgtggtgctgaccctcgtcgaaatttgcgcaagtcttaaggccc
atgccttggcccaattgcccaagttcgctccccaacttactgagttactgaaggaacaggttcaccaaatggcctccctcaaacaaggacc
agattacgtctgttccacccttgtcacagcactccacaagctcttcaaggcgttgccgcttttcttgggaccctacttggtggacattatcggag
gcttggcgcgcctttcagtgcaattggaaaatccacagctgctgcaggataagcgcacacaggtgcttaagcagaaacttgcggacgttt
ggagtgcagttgcccagggcgtcgaggtgcgcattcttgtgcccagttgcgccaaggcgttctccagtctattagagcagcaagcgtacg
acgagctaggacatctaatgcagcagctgttgctgcagagcgtccgacacaattcagccgctcaattgcaaccggttcaggatcccctaa
gcgagctcttcctgcaagcactcaacttccggctgcaagtgcgtggacttggtttgcagaggcaattggtttcggatgtagaggcttccataa
ccgagacctttgtgacgtggatcctaaagttgtcagagaccagtttccgtcccatgtactctcgggtacataagtgggcactggagagtacc
tctcgggaaacgcgtctcacctacttcctgctgaccaaccggatcgccgaagctctaaaatctttgttcgtgctatttgccagcgatttcgtgg
aagactcttcgcgcttgctgacggagcacaactcaatccgtcccgagttcgaagtcgaagaaagggaagatgatgtggacctgttaatgg
caatactgaacactctgcatcatgtgttcttgtactgcagcgaggacttcattaatgaccaccgcttcaacgtgttaatgccaccgctggtcaa
ccaattagaaaacgacctggtattgggaaatgagtcgcttcagcaagttctcagtaactgcattgcccagtttgcggtggccaccaacgat
gtgatgtggaagcagctcaacagccaagtgctcctaaagacacgcacatccaatcccgaagtgcggattctggcattcaacagttgtgtg
gctatcgcgcgcaaactgggtgaaagctatgccgccctgttgccagagacagttccattcatcgctgagctcctggaggacgagcatcaa
cgtgtggagaagaacacccggactggagtgcaggagctagaaactattttaggcgagtctgtgcaaaagtatctataa (SEQ ID NO:35)

**Protein:** AAF52447
**Protein GI:** 7297181

MSTALAQQLQKLAAPQSSVTLADARSRASILFDPKEAATKDRRSIYEIGLTGLQELTDFNP
AFKEFQLTLFDEATLTLERSVELPEINKMLDAAIAKFLRLLSPYLLLRPAHMAFEWLLRRFQVHEYN
RSEVMALILPYHETMIFVQIVKTMRLRSSDGDWYWLRPLQRPGVPLAKTAIINRAASNPAFLGFICQ
STQKAVKELGPRAHQLQAQINFYATVVVGALQTAKPLQDWHITTILESLLRGLISDNIDFMAAAYVIV
AQLVSRTKLKSKVCNALLERVANCPFERLHSESLLLLVCIYGKQQAALPHFKPETILNLVGKKWLIS
TLSSLAKGNIAIQSICMPLMTGAVAAIRDDDASSNSCKLFLDNLLSEVPMPKPTAQQLINCFLDTYV
ETAIDAPEPMETNSNEDDDTIVIDSDDEIETEKTTFQAWYSTYLEKLERRYPEAFDLSVKEALRSKS
STSNRQKALKLALGFRLNTTDEKAKHAYEKLYHYSADWRLSAVQKLLQNLNVTKKRERSVKLLQE
CLPDRINDDSGAVVSTLLSLPTEELAEMLGPLPLAQTLCHLLYRAQSEKDEEWQPVVPLAVRHLTS
ALVSGSYDTNLVLLALMPLLFPGEALAEHQHKALRILLGSDFVSKVPFLAELKVSNKFSDFNVGEH
RQHFLDIIASSNQELSSQERALLQSVEDHGGELYIQKASQLTHLLLLLTAYAKRELQPRESLHMLEK
IGLYSRRLQFRVVNGSQNTQNCAPLQLYVDFLLTLVKNTKWTALASTPWNQMTDELRLCLRLLEII
CAQVFSEKADQPERQEWTRALQQSLQLILPEAQDRLEVLSNFYVFERLPELWPRDSDYAVFRLQ
GFIILEAVLSNPKSQIDCGLVHVLRVANACGSPLQTLRVQAINILQLISNRKLVSHVEQLVRSLLQRK
SELSMDHEQYALILYTILEPEKATAKERLVLSKLKRSVLALASDPKQSPICTASLLAALKHVNDENFL
NELLPLGLDSLKTITAGEDNQNIKQLPWPHSEIYKSVIERFEGRVALNVLLRKDLAWKLFEDSFAQY
DTYVQLEQKLQPLPCVLLNSLTPETFEQMHAKHKIALIKLIVESATNSDNDSIFLASHRLLKRCRLDC
QPLVPILLEMANTKVEKKQPVKRRSVQATQLDLTSPYWKQGMTLLELLEHKKQLVGAELLIPPLFE
LLQACLTMEEHSAAEYPKQLILSSLLHCCQTAQSAGVQLVKAMPESSFRIELVVQSLRNTRNPQT
QQHALLFLTHCAGMYPQQVLHKIVEIFTFVGSTVARHDDAFSLHIIHNVVESIIPILLLNTGHNELVIP
VLKVFADICTDVPVHRRLPLYATLFRVLEPKEHLWQFLCIIFESQVLLEQVPQKVSTDKSRLDFARE
LTLMFEDPTVAIQTCIRLLDYLAKLPATKSSLSGGSGSSVLSTEQQLFDVRTRTFKQLRHYKYLIMD
FLSGISSCNEWEKKMKRPDPNELLPYYQEFILKTLAYVGVLNGALEAASETPSLEKFWRVLANHAH
DVLDNAIGLLAPQHFISVITELLKHDHVYVRIKVMDLLVTKLSPSSDYFQQSNAEHFGVLFAPLQEII
NGILEGSSNSAQQAKLQQTALHALQLLALRHGRDYIEECRSLLATLTKITKRRANVPKAVVGNVVL
TLVEICASLKAHALAQLPKFAPQLTELLKEQVHQMASLKQGPDYVCSTLVTALHKLFKALPLFLGPY
LVDIIGGLARLSVQLENPQLLQDKRTQVLKQKLADVWSAVAQGVEVRILVPSCAKAFSSLLEQQAY
DELGHLMQQLLLQSVRHNSAAQLQPVQDPLSELFLQALNFRLQVRGLGLQRQLVSDVEASITETF
VTWILKLSETSFRPMYSRVHKWALESTSRETRLTYFLLTNRIAEALKSLFVLFASDFVEDSSRLLTE
HNSIRPEFEVEEREDDVDLLMAILNTLHHVFLYCSEDFINDHRFNVLMPPLVNQLENDLVLGNESL
QQVLSNCIAQFAVATNDVMWKQLNSQVLLKTRTSNPEVRILAFNSCVAIARKLGESYAALLPETVP
FIAELLEDEHQRVEKNTRTGVQELETILGESVQKYL (SEQ ID NO:36)

# Figure 14

**Name:** eIF-4a
**Nucleotide:** AE003612
**GI:** 7297037
**Transcript:** CT26044
**Sequence:**

agcctcttttgcttggttttaacaaatcagtagcagcacgtgtataaaaagtgccaaataattttcagaaaatggatgacc
gaaatgagatacctcaggatggccccgccagcatggaacccgagggcgtcatcgagtccacctggcacgaggtgtacgacaacttcg
atgacatgaacttgcgcgaggagttgctgcgcggcatctacggttatggtttcgagaagccgtcggccatccagcagcgcgccatcattcc
ctgtgtgaggggtcgcgatgtcattgcccaggcccagtcgggaactggcaagactgccaccttctcgattgctatccttcagcaaatcgac
acgagcattcgcgagtgccaggcgctgatcctggcccccactcgcgagttggccacgcagatccagcgcgtggtgatggcgctcggcg
agtacatgaaggtgcactcgcacgcctgcattggcggcactaacgtgcgcgaagacgcccgcatcttggaatccggttgccatgtggtgg
tgggcactcctggccgcgtctacgacatgatcaaccgcaaggtgctgcgcacccagtacatcaagctgttcgtgctggatgaggccgatg
agatgttgtcccgcggtttcaaggatcagatccaggatgtcttcaagatgctgcccccagatgtgcaggtcatcctgctgtccgccaccatg
ccgccggatgtgctcgaagtgagccgttgcttcatgcgtgatcccgtcagcatcctggttaagaaggaggaactgacccttgagggtatca
agcagttttacgtcaacgtgaagcaggagaactggaaactgggcacccttgcgatctgtacgatacgctgtccatcacccagtcggtaat
cttctgcaacacccgtcgcaaggtggaccaactgacccaggagatgtctatccacaacttcaccgtctcggccatgcacggcgacatgg
agcagcgtgatcgcgaggtcatcatgaaacaattccgttcgggctcgtctcgtgttctgattaccactgatttactggcgcgcggtattgatgt
gcagcaggtgtcgttggtcatcaactatgatctgccctcgaaccgcgagaactacattcatagaattggtcgcggtggtcgtttcggtcgca
agggtgttgcgatcaactttattacagatgatgatcgacgaatccttaaggatattgaacagttctaccacacaacaattgaggaaatgcct
gctaatattgccgatttgatttaaatttttctgctgagccgcaaaacaactaagaaagaattctaaacaaaaagtgagtgagcaaaacagc
aaacaagaactgcgaaatcgataaccgagcgcaactataaattattcaaagatcaacaagtcaagttaaatatttattattcaaatattaat
tcgaaaaggcagccgaccgaccacccaaaaaaaggactaaaccaggacagagcagcaacatcttcagcagcggcgcagcagca
gtaacatcagcagcagcggaagcggcagcaacaccaccaccaattgccgattagccggaacccctctgtatccatttagttctctgtgtat
catcaattttaaaactgcgttgtcaaccagcaacaacaaatatatac (SEQ ID NO:37)

**Protein:** AAF52317
**Protein GI:** 7297048

MDDRNEIPQDGPASMEPEGVIESTWHEVYDNFDDMNLREELLRGIYGYGFEKPSAI
QQRAIIPCVRGRDVIAQAQSGTGKTATFSIAILQQIDTSIRECQALILAPTRELATQIQRVVMALGEY
MKVHSHACIGGTNVREDARILESGCHVVVGTPGRVYDMINRKVLRTQYIKLFVLDEADEMLSRGF
KDQIQDVFKMLPPDVQVILLSATMPPDVLEVSRCFMRDPVSILVKKEELTLEGIKQFYVNVKQENW
KLGTLCDLYDTLSITQSVIFCNTRRKVDQLTQEMSIHNFTVSAMHGDMEQRDREVIMKQFRSGSS
RVLITTDLLARGIDVQQVSLVINYDLPSNRENYIHRIGRGGRFGRKGVAINFITDDDRRILKDIEQFY
HTTIEEMPANIADLI (SEQ ID NO:38)

# Figure 15

**Name:** ACXA
**Nucleotide:** AE003638
**GI:** 7297983
**Transcript:** CT38108
**Sequence:**

taaaaaaaatactacttaaaaatatgaacaaaaaaaacacttacaaaacgccgtacagggaaaggtttaggcgat
gtaatctggaatatactaatgaacgcctttgggaacaaagttatttgaaggccagatgtaaggagttgcacttagaggaggaatatatgaa
atataagattcgtttgatgatcagttcccttaccgtttttgtccccatgttaatactactgatcgtagctcttcaagtaatcatatggtcatttaccga
gtatacaaaatacatctatataaatgcgatatttgacttgggatcattaattttagtcacgggcctactaagcataaatttctttgaagactttgta
atccgccacagatgggtcatgactttcacctcgactttgtcggcatatgttgtggtattgggtgatatagcctttaacacttattattattacaaatc
caactggcctcttaataccttatacgatgtctttgtgctctgcatgatctacatgttcctgccaataccttcgagcaaggcagcagctctgctag
ccatatctgttagtttaacatatgtgatatattttattcacttcatggcgtttaacgagcataatgttgctaaatatgttcatggtctggatatagtatc
aatcgatttttttcactacctgggcttcaatatgatgggaatattcttcagaatcatgaacgacaccatggtgcgctcgtcgttcttggaccgata
ccagttcattacggaggagatttggctacgccaagctcgccgtcaagaatccttgctgctggatagcatacttccaccacagattgcgaag
cccattcagaaaagcattaaggaaaagatcatacagcccgataacgacttctaccatctgggcacttcgcggacagcagaaaacttcat
gtccatccagattcacaacgatgtcagtatcccttatgctgatctggtgaactacacccaattgaccacaacattgacggtggagaagctgg
tgaaggtcctgcacgacctctacgccagatttgatttggctgccttatctttcaaggtgcagcgcatcaagttccttggcgattgctactactgtg
tggcgggtttgggggaatctgatcctgatcacgccaccatggctgtgtctctgggaatttccatgattgctaacattaaggaggtgagtgtga
atcgttctttgaatattggcatgcgaattggtgtccattcgggaactttgtttgcaggtgtaatcggcaaggccaagctacagtacgacatatg
gggtgccgacgtgaatattgccagtcgtctggaagcaaccggaagtcccgggtatgtgcatgttagtggacgaactctgagcagcttgaa
cgctgaggaatacaatatatatccgggcacggaatcggcccagaaggatcccgtactgcagaaacatccgatgagcacctacctcctc
accgctataccatccctcgattcggataaaacaattagcatagttgagggcgttcctaacctggaccttcaaaccgtgggatctaaccgaa
aatcacagattttgaagcctaacttactgtcccatgagatgcgtgaggaatttagaaagatgccggtggggggtttcaaatttcaattcccttg
gtcgcgaagagaaagaaatttgaaaaacgagaaagtcgaacgcgatttgggcatttttttgtgtcgcatttaaggacaaatctgtggaatgg
aattatttgcatcagcccgattatattttaaatattcagtggcgctcggctggggaattggttgctgtcttatttatatccagtccgtgaacaactc
agatatcttttatacgggtgttgtcattaacattatcgcgtttttcgtcctgactttcctactattcatttgctggtacaagaaagtgtgctggtggcat
agtggacaaaacgagcacaggagttacggcaaactcagctgcgccatattccacttgtttgaaaagatccagcacagttttgtccttcgcc
tcactgtctacatgatgataatattgtgctattacatggtgatctccttgatattaatgagctgtgaaaaggaccaatatgagttggacattatag
agagcaagctctacaactatgacatggatccgtttacctgcttccacccctgggcctatacgaacatgatggcccttatcctgggaatgagtt
acaccttcgccccgcattcccttcgccctcaagacctttatcggttgcgccgaagcagtggttttcgtcctggttgtgtgtttccagtacgcgttcat
tttcgagcacagtgttaccacatcgccctatttaaaggctgaaatagctcattcctgcagggtgtgcatgatgttgattactatgtacgctaagg
agcgtcagtcagagttcaacactaaaatgaactacaagctcaacctggacttgcaaaacaagcaaaaatcggccgatgtaactaacca
gtccatcataatcctgcttaacaacattcttccatctcatgttgtcgaggtatatctcagctctatagccaaacacgaactctattatgaaaacta
tcgaatggtatcagtcatgtttgccatgctcacaaactttcaaatggatttgcccagcttaagagtgctaaatgatatcatcacagcgttcgac
agattgctttctgcgtacaagcaatattacgtagtcgagaagatcaaagtcgtcggatgcacttatatggcagcctgtggattggactttagc
ctgattgaaaaccttgatagcaactcgaattttggcagtacatcattgtcatccgaattagaacaggtgcgttctcgtttggagtcatctataaa
agagaagaaccatgatgaggtggcttttataatggctacgtttgctctggacctgatgcgcgttctgtcggtgtgcaacaaggcttacgccg
gagaaccatttgatcgggctttgtccaccggcgagataaggatcggtatctctacgggtcagataatggctggagtggtaggcgcttccca
gccgcactacgacatctgggggaacccggtcaatatggcctcccgaatggaatcgactggtttgtctggacatatacaggttacaaagga
gactgcccaaacgctcgaggagttcgatgttatgtgctattatcgcggcttaacctttgtgaagggtcgtggtgaaattcccacctattttgtgg
gcatcgataaagacctaaagttcatgtccaagaaaatcgatagactttctgagaacccatctaatccagatctaaaccatgacctggacg
gctcctaagacagatacaaattttttcggcaaattcttctcaatatggaattcgaatcgcaaatgcatattgaaggcttcgtttctcaaattttga
gatcgtagttcttgtctgtaaaatttgagatgcgtagccttacaaaatgatgtggactcaaatcttgtaaaaattatgtggaaaatacatatttgt
aatacctt (SEQ ID NO:39)

**Protein:** AAF53228
**Protein GI:** 10728753

MNKKNTYKTPYRERFRRCNLEYTNERLWEQSYLKARCKELHLEEEYMKYKIRLMIS
SLTVFVPMLILLIVALQVIIWSFTEYTKYIYINAIFDLGSLILVTGLLSINFFEDFVIRHRWVMTFTSTLS
AYVVVLGDIAFNTYYYYKSNWPLNTLYDVFVLCMIYMFLPIPSSKAAALLAISVSLTYVIYFIHFMAFN
EHNVAKYVHGLDIVSIDFFHYLGFNMMGIFFRIMNDTMVRSSFLDRYQFITEEIWLRQARRQESLLL
DSILPPQIAKPIQKSIKEKIIQPDNDFYHLGTSRTAENFMSIQIHNDVSILYADLVNYTQLTTTLTVEKL
VKVLHDLYARFDLAALSFKVQRIKFLGDCYYCVAGLGESDPDHATMAVSLGISMIANIKEVSVNRSL
NIGMRIGVHSGTLFAGVIGKAKLQYDIWGADVNIASRLEATGSPGYVHVSGRTLSSLNAEEYNIYP

GTESAQKDPVLQKHPMSTYLLTAIPSLDSDKTISIVEGVPNLDLQTVGSNRKSQILKPNLLSHEMRE
EFRKMPVGGFKFQFPWSRRERNLKNEKVERDLGIFCVAFKDKSVEWNYLHQPDYIFKYSVALGW
GIGCCLIYIQSVNNSDIFYTGVVINIIAFFVLTFLLFICWYKKVCWWHSGQNEHRSYGKLSCAIFHLF
EKIQHSFVLRLTVYMMIILCYYMVISLILMSCEKDQYELDIIESKLYNYDMDPFTCFHPWAYTNMMA
LILGMSYTFARIPFALKTFIGCAEAVVFVLVVCFQYAFIFEHSVTTSPYLKAEIAHSCRVCMMLITMY
AKERQSEFNTKMNYKLNLDLQNKQKSADVTNQSIIILLNNILPSHVVEVYLSSIAKHELYYENYRMV
SVMFAMLTNFQMDLPSLRVLNDIITAFDRLLSAYKQYYVVEKIKVVGCTYMAACGLDFSLIENLDSN
SNFGSTSLSSELEQVRSRLESSIKEKNHDEVAFIMATFALDLMRVLSVCNKAYAGEPFDRALSTGE
IRIGISTGQIMAGVVGASQPHYDIWGNPVNMASRMESTGLSGHIQVTKETAQTLEEFDVMCYYRG
LTFVKGRGEIPTYFVGIDKDLKFMSKKIDRLSENPSNPDLNHDLDGS (SEQ ID NO:40)

# Figure 16

**Name:** CG15117
**Nucleotide:** AE003797
**GI:** 10727456
**Transcript:** CT34995
**Sequence:**

cggtgcgtccgcttcgcctcgcggtttatagtttcaaatagtttgtttttgtaattaccggcaaaacacttgaaccgattatta
atagtcgcgaaatgactgccaatttctaaccacttgtgttcgagtttaattggaaacgaaaatagttcctaagtgcacaaaaccgattccgg
attttaattatactcgcaaagttacatcgatcattcgccggaatcctgcacggaatactcgaatatggctctgtccatcttctccctggtcactgg
tctttatgtcctgcacttctccatagctctgatcctggtcaacaaagaggtgccccaaacgcgcggaatgctgtaccccagggaatcggag
acgcgggaggtgcgatctctggacgggatctggaactttgtgcgctccgaccaggcgaatcccacgcagggcgtccgggatgagtggt
atgccaaggagttgagcaagagcaggcccacaatacccatgccggtgccggcatcctataatgacataaccacggacaatttgaggg
atcacgtgggcacagtgtggtacgaccgcaagttctttgtgccccgatcgtggtcaaaggatcagaggatatggctgcgattcggcagtgtt
cactatgaggcatatgtgtggatcaacggccagaaggtggtgaagcatgaaatgggtcacctacccttcgaggcggaggtcaccgatctt
ctgagctatggagccgagaatcggatcaccgttatgtgcgacaatgctctgattcagacaactgtgccgcagggcaggattacggaagtg
cccaacgatggtggcatgaccattgtgcagagctacacctttgacttctttaactatgcgggaatccacaggagcgtgcatctttacaccac
gccccgtacttttatcgaggaggttgaggtcaccacaaacctttcaaaggatgccaccgttggcgaagtgttctatagcgtaagcgtgaatg
gcagcgctgccaacgaggcagataatgtcttgcaaatccaggccaatttgtacgataaggatggcatattggtggcgaatgccacgtcgg
accaaaaacttggcggcaagctgcaagtgaacccggtgaagccctggtggccctacctaatgcattccgaaccgggttatctgtaccag
ctggagatcaaattgctggcgaccaacgatgagctcctggatgtctaccgactgaaagtgggaatacgcacactaagttggaacagcca
gcagttcctgatcaacggcaagcccgtttacttccgtggttttggacgacacgaagattcggatatcaggggtaagggtctggacaatgca
ctgatggtgcgcgatttcaatctgctcaagtggattggagcgaatgcgtatcgcacgtcgcattatccctactccgaggagtccatgcagttt
gcggatgagcacggcatcatgatcatcgacgaatgtcccagtgtggatacagagttgagttattccgacacctcgaagggattcttactaat
gctcttggcttcgattttcagaaactttagccaggagctgctgggcaagcacaaatcgtcgctggagcagctcatccatcgggatcggaat
catccaagtgtggtgatgtggtccattgccaatgaaccccggactggtagcgtgagtgcggattcgtactttgaattagtggccaacttcaca
cggtctctggataaaacccgacccatcacggctgccattgctgtttcaaacacccaggataaggcgggccgctctctggacatcatcagct
tcaatcgctacaatgcctggtactcaaatgccggacgtctggacatgataacccagaatgtgatcgacgaggccattgcctggaataaac
gctacaacaaacccattatcatgtctgaatatggagcagataccttggagggcttgcatatgcaacctgcttatgtgtggtcggaggagttcc
agacggaggtgttttcccgccattttaaggccttcgatgagctgcgcaagaagggctggttcattggcgagtttgtatggaactttgcggactt
taagacagcacaaagctacacccgcgtgggaggcaataaaaagggtgttttcacccgcgcgcgccaacccaaagcggcagctcatct
ccttcgcaaaagatactttgcattgggcagagatttggaccagtgcagcttcccggaagatctcttcacctacatcgccgatctgatatccta
atggttgggagccagttgatggtgcccaaagtattcggccactgtaaataagtttaccgagaaaaactttatcctcggacttgcattttagccc
gactaccattccgctgcattgccctgtacaatataaaatggttagctctagttgattgtatttttgtatgtatttgtatgtttaagagaataaatgaa
agtgcattatttatatttaccac (SEQ ID NO:41)

**Protein:** AAF57602
**Protein GI:** 7302519

MALSIFSLVTGLYVLHFSIALILVNKEVPQTRGMLYPRESETREVRSLDGIWNFVRSDQAN
PTQGVRDEWYAKELSKSRPTIPMPVPASYNDITTDNLRDHVGTVWYDRKFFVPRSWSKDQRIWL
RFGSVHYEAYVWINGQKVVKHEMGHLPFEAEVTDLLSYGAENRITVMCDNALIQTTVPQGRITEV
PNDGGMTIVQSYTFDFFNYAGIHRSVHLYTTPRTFIEEVEVTTNLSKDATVGEVFYSVSVNGSAAN
EADNVLQIQANLYDKDGILVANATSDQKLGGKLQVNPVKPWWPYLMHSEPGYLYQLEIKLLATND
ELLDVYRLKVGIRTLSWNSQQFLINGKPVYFRGFGRHEDSDIRGKGLDNALMVRDFNLLKWIGAN
AYRTSHYPYSEESMQFADEHGIMIIDECPSVDTENFSQELLGKHKSSLEQLIHRDRNHPSVVMWSI
ANEPRTGSVSADSYFELVANFTRSLDKTRPITAAIAVSNTQDKAGRSLDIISFNRYNAWYSNAGRL
DMITQNVIDEAIAWNKRYNKPIIMSEYGADTLEGLHMQPAYVWSEEFQTEVFSRHFKAFDELRKK
GWFIGEFVWNFADFKTAQSYTRVGGNKKGVFTRARQPKAAAHLLRKRYFALGRDLDQCSFPEDL
FTYIADLIS (SEQ ID NO:42)

# Figure 17

**Name:** BG:DS01759.2
**Nucleotide:** AE003642
**GI:** 7298121
**Transcript:** CT35241
**Sequence:**

atgcagtactcctatctactggcaatctgtgtcagcttgctgcttctgaagctgaagggagcagctgccacttctttatccg
ccgaaccgactccggaacttttgaatgcggccaagcaaataggtttagatgaagttggcttgcgagaaatgtggcacagccagcagccg
ctggttatcagaactcaaaacgatgctggtcaagtcaccactttgacctatgagttaggtccggatttcagatcgattttaagacgcaaggtct
cagagggaatactgcctgctaggctagaggataattcaggctctaagccccagcaagattgggagtatgtgccgagtgccggtcgtcagt
tccgtcccattggagattccaacttcggtacaggtaatttcgagacaaagatatttagcactgaatttccgagatttccgaattgggagctgcc
agcgggagttactccgaaagttacaaccaagacggaaacggatagctcgggacgcaaggtgaccaccacaactagaacttatagcg
gcattctggtcccaggcagtaatgtattcaagcagatattccccgacatctccagagagcctggtccaattaacacagactcaaaccatcc
agccgagaatcctaacacctcagtcttttccccatctcgttccccatctactggacctattgatttcaactatgtatataattcccgaccaagcc
aaagggaacccgtctttgtgccagtgattgactcaaccaccacgcagcggacttcagttccccggttcagttcagcattaccaagagacg
acggatcagatcgagtcatagatgattatttgaaacaagtgaatctgagtgcctcagaaattgagaattcgaatggtgaagtggtcaagac
tattgtggataagaatggcagaattctaactgccagatttgttctaagctcagtcaaaggaaccaacgaaggatctcagcagcagaggcc
gaccaagtaaatagctcattataaatattaatatatggaaatcatcgcaatattttgtaaacaatgcttagcgcagtttaataaataagcttatc
agcctgggctaaacccagaaattgtaataaaatttctaaatcaatctatcactgaaa (SEQ ID NO:43)

**Protein:** AAF53376
**Protein GI:** 7298138

MQYSYLLAICVSLLLLKLKGAAATSLSAEPTPELLNAAKQIGLDEVGLREMWHSQQP
LVIRTQNDAGQVTTLTYELGPDFRSILRRKVSEGILPARLEDNSGSKPQQDWEYVPSAGRQFRPIG
DSNFGTGNFETKIFSTEFPRFPNWELPAGVTPKVTTKTETDSSGRKVTTTTRTYSGILVPGSNVFK
QIFPDISREPGPINTDSNHPAENPNTSVFSPSRSPSTGPIDFNYVYNSRPSQREPVFVPVIDSTTTQ
RTSVPRFSSALPRDDGSDRVIDDYLKQVNLSASEIENSNGEVVKTIVDKNGRILTARFVLSSVKGT
NEGSQQQRPTK (SEQ ID NO:44)

# Figure 18

**Name:** TepIII
**Nucleotide:** AE003618
**GI:** 7297264
**Transcript:** CT21827
**Sequence:**

ctttcaagcggacaacaggtgatcgcgactgtttcgcagaaaaggtgaaaatcgcgtacaaccgagagcgagtaa
agtcgccccaaaaacgcgcacctcgtcttgggcaatctacggtgcttttattttggtgctgctgcaatctggcgtgagagtgagatagtgttcg
gcagcggtagtgcgagtgagaagcagcaacgcgcctgcccacggcaataaagttcgacttgatttttgttttaatatgtgatgccaatacac
aattcgacaatcgagaaaacgtgtttacacttaatttgctaacgtgatttaaagacaaaccaaaaatgcgcctccagggagcggatatgg
gcgctattccagtgctaatcttagtcaccgcttgtttgctttgccaaacatctgcccagggtttatactccatcattgctccgaatactttgcgtccg
aactcgcaatttcatgtggctgtgagcttgcacaatgcacctgaatctgccacatttaaggtgggaatcctggggagctcttacacggacttt
aagaccgtggagttacggcccttttccacacagctattgcactttgagattcccgccttaagaactgacagatatcgcctaactgctgaggg
ccttggaggagttcagtttaccaatgagactcaactgcactttgagtccaaacaacatacggttttggttcagacggacaagagcatctaca
agccaggcgatttggttcactatcgagttcttattttggatgccaatctgaaacccgcgcgtggctatggacgtgtgcatgtggatatcaagg
actccggtgataacattatacgcagctacaaagacattcgtctgaccaattccatttactcgaacgaactgcgattgtcggactccccgcgtt
ttggcacttggtccattgtcgtggatgtttcggatcaagagcatacgcaaactttcgagatccttgatcatattctaccaaaattcgttgtagaca
tcgatacacccaagcacgctatttataaggatgggaaaatagcagccacagtcagagcgcactatgcatttggccagccaattgtgggtg
aagccaccctatccatttatcccactttctttggctcgctccaaccttttgttaacgatctcatcacccgaaaagttgtgccaattgatggtaatg
cgtactttgagttcgatatagagaatgagctccatctgaagcaggactatgagcgacaatacctttggatgccctcgttgaagaaaagtca
actggttcggtgcaaaactattcaacagtcttgacactgcacttgaaccattatcgcgtggaggccgtcaaggtgcccagctactatattcct
ggagtgccctttgaagctacagctcgcatagcccgcaatgatggcggccaattgagggacttcaatccccaaatcaccgcatatttgacg
aatgtctatggcagcagtgagatgtacaaccgtactgcctatagcctggatgccagtggagagatcaagatgaagttcactgttccgattg
gagatcgagatgaatttcattccataatagttgactaccagggcgtgatcagtgaggaaatttctgtggtcgtacgctcctttgcgcccattaa
atattttatgtaccaagttgtcggtcgcggcgatattatactgtctcgcaatgtggatgtaagtaaatatttttttgtaccagaagtttattatgctttat
cgatttggtaacttttccaaggtggcccctgggactttccacacaatcaaattcctggcatcatttgcaatgatgccgagagccaatctgctcg
tatatacggtaatagatggggagtttgtttacgatgagcaggtcatacagcttgaagaaaatctactcaatgcggtacaagttgacgctccc
attagagcacctcctggtcaagatattgacataggcataagcaccaaaccctactcatatgtgggcctcatgttagtggatcaaaatgcaa
acgaccttaggagtggtcatgatttaacccacaaacgactgatggatgctctgcgttcatacgaactgagcgatgtgaacactccaatggg
atcacctggcaaggagtctggtgtgataaccatgtccaatacggattactttatcgaaaaggaagctgagagcaatccggctttggatcga
gaggtttccaccgggcccgaggaagacaaattaacgactgtgcgaaaaaccgacattggacctgcccacaagatcgaggttaatacgt
tgcctcctggaaaaggccgatacgccttttcttataccccaaagcccttttggcataatccaagggtccatgtgatgcgcgatcctgcggata
cgtggctcttcttaaacatctccgccagcagcgatggaaggaattcaatccacagaaggattcccagtgagatgacatcctgggtggtttc
cgcctttgctctggatccggtgaacgggttgggattatctccgcccaatggaaaattggaggcctacaaagagttttacataagcaccgagt
tgccctactccataaaaagagatgaactcattgccattccgtttgtggtgcacaacaatagggacagtgatttgaacgtggaggtcacgtttt
acaactcagcgctggactttgatttcccacaactggatcctaaggccaccaaccaaccaaaggtggaactctacagacgtagatccttgc
aggtaccgggacgatccgcccgctccgtgtccttcatcgtgactccgaagagagtggggcccctcttggtcaaggcaatggctgcctcctc
ccaggctggcgatacagtcgaacaaaatctgctggtggagcaccctggagccatggagcggatcaacagaggattcctcttcgaactta
actccaatgcccagaatagaaggaacgtcaccattgcggttccacgcaacgccattccggaatccacacgtattgaagtatcagccgttg
gcgatctcattggcagcttagtgggtaatctggatagccttatccttctgccaacaggctgcggcgaacagaccatgcgccttcgtcaactc
acgcccgaagtggaactgcgtgccacaaacaatctggctataggctaccagagaattctttattatcgccatgagaatggtgccttcagtg
catttggattagatatcaagcggagttccacctggctgacagcgtacgtggctcgctcacttcgacaagcggctccattcactcaagtggat
agcaatgtcctgcaaaaggcactaacctatttgggaagtgttcaatcagcaaacggtggcttcgaggagcgtggcgacgtattcgagag
atttggcgacgatggcatcagtctgactgcatttgtgacactggctctgatggaaaatgtggatctgtatcccgaatatcgaaacaacatca
acaaagccctggactttatcaccaggggcttagatggatcaaatggcctcaagtggtggaacaaaacggcgcccgctggtgagcaaca
atcaccctggtataatgccactcgtagcgtcaacattgagatctccgcttacgccgccttggctctactggaaaataacttggtgggcgatgc
cctgcccgtcttgaactggttgatggaccagcgaaatcccaaaggtggttttgtggcatcgcaggatactgtagtcggccttcaggcgcttct
catgttcgccgaacgcttttccagtcagggcaataatctccaaatcggcttccattacggagaaggcgctgaaacgataattaatgtgaatg
ccgaaaactcactagccctgcaaacggtggagctgccaaacaatcttaaaaacttgagtgtttcggccactggtcggggaatggcattgg
cccaagtcagctacacctataacacaaacgtgaccagtgcctggccacgatttgtactcgatcccactgtgaatcggaactcccatgcgg
actacctccatctttcggcctgtgccagctttgtttccgtggtgggcgagaacgagcagcgctccaacatggccgtgatggaggttcacctg
cccagcggtttcgtcgtggacagggatacacttcccaccctggagtcgagtgagcgcatcaagaaggtcgagacccagaacaggaac
accaaagtggtcatctactttgattacctggacagaagggaggtgtgtcccaccctgcacgcttacaagacggtcaaggttacaaaacat
cgaccggtggcagtggtcatgtacgactactatgacagcgctcgccgtgccagacagttctacagggcacccaaatccaatatctgtgac
atttgcgagcacgccaactgcggtgacctatgcgaaaaggccgagaagcgcgagtccaagcgacctgatgactacacggcgatagc

gggtcatagttcagggtcaagacacacagccattccactggcatctgtggttatggttctgtcgatgcttttgaaaacccttagctgttagacg atactgttaccgccacttaacgtggaaccaatatggaaatcgaagctttaacaacacccactcacatagtccctaagttagcctaatgaat gccgtgaactaaacctgttctaagtacatctaacgatgctaaagtgttgccaataaagtgttataacgtttactaaaaccag (SEQ ID NO:45)

**Protein:** AAF52542
**Protein GI:** 7297279

MRLQGADMGAIPVLILVTACLLCQTSAQGLYSIIAPNTLRPNSQFHVAVSLHNAPESATFK VGILGSSYTDFKTVELRPFSTQLLHFEIPALRTDRYRLTAEGLGGVQFTNETQLHFESKQHTVLVQ TDKSIYKPGDLVHYRVLILDANLKPARGYGRVHVDIKDSGDNIIRSYKDIRLTNSIYSNELRLSDSPR FGTWSIVVDVSDQEHTQTFEILDHILPKFVVDIDTPKHAIYKDGKIAATVRAHYAFGQPIVGEATLSIY PTFFGSLQPFVNDLITRKVVPIDGNAYFEFDIENELHLKQDYERQYLLDALVEEKSTGSVQNYSTVL TLHLNHYRVEAVKVPSYYIPGVPFEATARIARNDGGQLRDFNPQITAYLTNVYGSSEMYNRTAYSL DASGEIKMKFTVPIGDRDEFHSIIVDYQGVISEVGKIPSKHLHSKNYITAKVLNDRPTVNQEISVVVR SFAPIKYFMYQVVGRGDIILSRNVDVAPGTFHTIKFLASFAMMPRANLLVYTVIDGEFVYDEQVIQL EENLLNAVQVDAPIRAPPGQDIDIGISTKPYSYVGLMLVDQNANDLRSGHDLTHKRLMDALRSYEL SDVNTPMGSPGKESGVITMSNTDYFIEKEAESNPALDREVSTGPEEDKLTTVRKTDIGPAHKIEVN TLPPGKGRYAFSYTPKPFWHNPRVHVMRDPADTWLFLNISASSDGRNSIHRRIPSEMTSWVVSA FALDPVNGLGLSPPNGKLEAYKEFYISTELPYSIKRDELIAIPFVVHNNRDSDLNVEVTFYNSALDFD FPQLDPKATNQPKVELYRRRSLQVPGRSARSVSFIVTPKRVGPLLVKAMAASSQAGDTVEQNLLV EHPGAMERINRGFLFELNSNAQNRRNVTIAVPRNAIPESTRIEVSAVGDLIGSLVGNLDSLILLPTG CGEQTMVNFVPNLIVLRYLGRLRQLTPEVELRATNNLAIGYQRILYYRHENGAFSAFGLDIKRSST WLTAYVARSLRQAAPFTQVDSNVLQKALTYLGSVQSANGGFEERGDVFERFGDDGISLTAFVTLA LMENVDLYPEYRNNINKALDFITRGLDGSSNLHAMAIGTYVLSRANHNAKAAFLQRLDSMATNKD GLKWWNKTAPAGEQQSPWYNATRSVNIEISAYAALALLENNLVGDALPVLNWLMDQRNPKGGFV ASQDTVVGLQALLMFAERFSSQGNNLQIGFHYGEGAETIINVNAENSLALQTVELPNNLKNLSVSA TGRGMALAQVSYTYNTNVTSAWPRFVLDPTVNRNSHADYLHLSACASFVSVVGENEQRSNMAV MEVHLPSGFVVDRDTLPTLESSERIKKVETQNRNTKVVIYFDYLDRREVCPTLHAYKTVKVTKHRP VAVVMYDYYDSARRARQFYRAPKSNICDICEHANCGDLCEKAEKRESKRPDDYTAIAGHSSGSR HTAIPLASVVMVLSMLLKTLSC (SEQ ID NO:46)

# Figure 19

**Name:** Hsc70-4
**Nucleotide:** AE003708
**GI:** 10726541
**Transcript:** CT13958
**Sequence:**

cgcaaattttgtacgggtgtaattcagaaaaaaacgccagccagtttgatcgaaggtgcggcagattaaaagtgaag tagcaattaaacggttatattttagtactttctaagaaacaacacacaagatgtctaaagctcctgctgttggtattgatttgggcaccacctac tcgtgcgtgggcgtgttccagcatggcaaggtcgagatcatcgccaacgaccagggtaatcgtaccactccatcctatgttgccttcaccg atacggagcgtctgatcggagatgccgccaagaaccaggtggcgatgaacccgacccagacgatcttcgacgccaagcgcttgattgg tcgcaagttcgatgatgccgccgtgcagtctgacatgaagcactggcccttcgaggtggtcagcgccgatggcaagcccaagatcgagg tgacctacaaggacgagaagaagaccttcttccccgaggagatctcttcgatggtgcttaccaagatgaaggagaccgccgaggcctat ctgggcaagactgtgaccaacgcggtcatcaccgtgccggcctacttcaacgactctcagcgtcaggcgaccaaggacgccggcacc atcgccggtctgaacgtgctgcgtatcatcaacgagcccactgccgctgctatcgcttacggtctggacaagaaggctgttggagagcgc aacgtgctcatcttcgatctgggcggcggcaccttcgatgtgtccatcctgtcgatcgatgacggtatctttgaggtcaagtccacggccgga gatacgcatctgggtggtgaggacttcgacaaccgtctggtcacccacttcgtgcaggagttcaagcgcaagcacaagaaggatctgac caccaacaagcgtgctctgcgtcgtctgcgcaccgcttgcgagcgtgcaaagcgtaccctgtcgtcctccacccaggccagcattgagat cgactctctgttcgagggtaccgacttctacacctcgattactcgtgcccgtttcgaggagttgaacgctgatctgttccgcagcaccatgga ccccgtggagaaggctctgcgtgacgccaagctggacaagtcggtcatccacgacattgtgctggtcggtggctccacccgtatccccaa ggtgcagcgcctgctgcaggatctgttcaatggcaaggagctgaacaagtcgatcaatcccgatgaggctgtggcctacggtgctgccgt ccaggccgccattctgcacggcgacaagtcgcaggaggtgcaggatctgctgctgctcgatgtgactcctctgtccctgggtatcgaaac cgctggcggtgtgatgagcgtgttgatcaagcgcaacaccaccatcccgaccaagcagacccagaccttcaccacctactcggacaac cagcccggtgtgctgatccaggtgtacgagggagagcgtgccatgaccaaggacaacaacctgctcggcaagttcgagctgtcgggc atcccccccgcaccacgtggtgtgccccagatcgaggtcaccttcgatatcgatgccaacggtatcctcaacgtgactgccctggagcgtt cgaccaacaaggagaacaagatcaccattaccaacgacaagggtcgtctctccaaggaggacatcgagcgcatggtcaacgaggcc gagaagtaccgcaacgaggatgagaagcagaaggagaccattgccgccaagaacggcctcgagtcgtactgcttcaacatgaaggc caccctcgacgaggataacctgaagaccaagatctcggactctgaccgcaccacaatcctggacaagtgcaacgagaccatcaagtg gctggatgccaaccagctggctgacaaggaggagtacgagcaccgccagaaggaactggagggtgtgtgcaacccgatcattacca agctataccagggcgccggtttcccacccggtggcatgcccggcggtcccggaggtatgcccggagcggctggtgccgctggcgctgc cggagccggcggtgctggccccaccatcgaggaggtcgactaaaccattcacccccacacctcaatgcaaccatacagtaacagttct ccaaacaatttaccaaccaaacacagtagaagagttgcttaaacaaacttggattctaaattaggaactcgccaaacatacaaacgggt caaacgacaactgctccccgtagttcattatataaattataaattcatactagaacaaaaccaaacacactgctccataatagtatatattat attattgtcatcgatcgatcatccaaactatttgcttgtacttgaattcaataaataaat (SEQ ID NO:47)

**Protein:** AAF55150
**Protein GI:** 7299978

MSKAPAVGIDLGTTYSCVGVFQHGKVEIIANDQGNRTTPSYVAFTDTERLIGDAAKNQVA MNPTQTIFDAKRLIGRKFDDAAVQSDMKHWPFEVVSADGKPKIEVTYKDEKKTFFPEEISSMVLTK MKETAEAYLGKTVTNAVITVPAYFNDSQRQATKDAGTIAGLNVLRIINEPTAAAIAYGLDKKAVGER NVLIFDLGGGTFDVSILSIDDGIFEVKSTAGDTHLGGEDFDNRLVTHFVQEFKRKHKKDLTTNKRAL RRLRTACERAKRTLSSSTQASIEIDSLFEGTDFYTSITRARFEELNADLFRSTMDPVEKALRDAKLD KSVIHDIVLVGGSTRIPKVQRLLQDLFNGKELNKSINPDEAVAYGAAVQAAILHGDKSQEVQDLLLL DVTPLSLGIETAGGVMSVLIKRNTTIPTKQTQTFTTYSDNQPGVLIQVYEGERAMTKDNNLLGKFEL SGIPPAPRGVPQIEVTFDIDANGILNVTALERSTNKENKITITNDKGRLSKEDIERMVNEAEKYRNED EKQKETIAAKNGLESYCFNMKATLDEDNLKTKISDSDRTTILDKCNETIKWLDANQLADKEEYEHR QKELEGVCNPIITKLYQGAGFPPGGMPGGPGGMPGAAGAAGAAGAGGAGPTIEEVD (SEQ ID NO:48)

# Figure 20

**Name:** CG7069
**Nucleotide:** AE003738
**GI:** 10726692
**Transcript:** CT21853
**Sequence:**

tttcggtgtatcaaaatttattcaaaattcatccatacgcaagaatccttagtccagggtccacagtgcccatatgttccac
ttgcctgttaaacctaagatatcgctgctatgccagcaaatgcaagaaagagaagaaaccaccgaagttccgactggaccgcggacca
ttcgcctcccagttggattaccagtcgcgactgcagtaccaagcacctgccctgagtatccccctcagcagcatcatatgcaccattggac
cctcatccaacagcccagaaaagctattggagctcatccgggctgggatgcgggtcgtgcgtatgaacttctctcacggctctcacgagta
tcactgccagacaatacaggcggctcgtaaggccatcgccatgtacgtggagcaaacgggtctgcccagaacattggcaattgctctgg
acaccaagggtccagagatccggacgggcaaactggccggtggcaatgatagggctgagatagagctcaagaccggagacaaagt
gacattgagcaccaagaaggagatggcagacaagagtaacaaggataatatctatgtggattatcagaggctacctcaactggtcaaa
ccaggaaatcgggtatttgtagacgacggattgattgcattgatcgtgaaggaatcaaagggagatgaagtgatctgtcaggtggagaac
ggtggcaagctgggctcccacaagggaatcaatttgccaggagttccggtagatcttcccagcgtaacggagaaggataagcaggatct
taaatttggggccgagcaaaaggtggacatgatcttcgcatcgttcatacgagacgcaaatgctctgaaggaaatccgccaagtacttgg
accggctggagcgtgtataaagatcatctctaagattgaaaaccaccaaggtctggtaaacatagacgacattatccgtgaatcagatgg
cataatggtggcccgaggtgatatgggcattgagatacccacggaggatgttcccctagcccagaaatcgattgtggctaagtgcaacaa
ggtgggcaagcctgtgatctgtgccacccagatgatggagtcgatgacgaacaagccgcgacccactcgtgccgaagcctcggatgtg
gccaacgctattttcgatggctgcgatgccgtaatgctgtccggtgaaacggccaagggcaagtatccggtggagtgcgtccagtgcatg
gccaggatctgtgccaaagtagaagcagttctatggtacgaaagcctgcagaacagccttaaaagggaaatccggaccagtgctgccg
accacatttcggcagttaccacggcgatcgcggaggcggccacggtgggccaagcccgggcaattgtggtggccagtccgtgctccat
ggtggcccaaatggtcagtcacatgaggccgccatgtccaattgtcatgctcacgggtaacgaatccgaggcggcccagtctctgctcttt
cgcggcatctacccactcctcgttgaggaaatggttattggcagctttaactttcgacgcatcatgcagtcgggcctgaagctgatgggcaa
gatggacatcttggagccgggtcagaaaggatcggtggtgctggttaatgccatgtcggcagaaaagatcaccttccggctgttcactatc
cgtcagcagaccaaggaagaacgcgatcaggatgagcgatgccgaaaattggccctagagcagagctgcaaggagagggctgaa
aaggaggaatgcagaaagctccagcaagcggaagaatgtcagaagcaaaaactggccaaaaaatgtaaacagttcgaagagaag
caaaaggtctgccccaaaaagaatgatactccgaagaatgattgtccgaagaaagattgtccgaagaaagaatgtcccaagcaagat
gacgaaatctcaaagtgcaggcaaatgcaagaagcggaagctgaagaaaggaagtgcaaagaagagtttgaacagatgtgcaaatt
agctgaagaaaaaaggaaagaagctgaaaaatgcaggaaagcggacgaagaacgaaggaaagaggaagctgaaaagtgcag
gaaattggaagaagataggaaatgcaaattggctgaagagaagaaaaggaatgaagaagaacttaagataatagaagcagaagtg
gccaaactggaagctgctgagaaggccaagcgtttgaaggaggaagagaaaaagaaggaagaactaatgaagtgcaagcaacga
aatgaagcgaaaaaaaagagggaagaggctgaaaggtgcaagagaaaggaaagagaaagggagctagctgaaatggaaaaca
aatggaaacaggttgcggagaaaagaaagagaaagaaggcagcagagatgtgcaggaaaatagaggatgcaaaagaaaaagc
cgcggcagaatcggctgataagattctgaaagctgtgtgcgaaaaactaaagcaatctctctcggatccggacaaatcaaaaaagggc
aagaaataaagtgaaagctaatggcagatttatatggaacattttaacttttaatacggtgttacacaccgaattccaaattattatataaaac
tattggaatttttaaattttct (SEQ ID NO:49)

**Protein:** AAF55980
**Protein GI:** 10726696

MRVVRMNFSHGSHEYHCQTIQAARKAIAMYVEQTGLPRTLAIALDTKGPEIRTGKLAGGN
DRAEIELKTGDKVTLSTKKEMADKSNKDNIYVDYQRLPQLVKPGNRVFVDDGLIALIVKESKGDEVI
CQVENGGKLGSHKGINLPGVPVDLPSVTEKDKQDLKFGAEQKVDMIFASFIRDANALKEIRQVLGP
AGACIKIISKIENHQGLVNIDDIIRESDGIMVARGDMGIEIPTEDVPLAQKSIVAKCNKVGKPVICATQ
MMESMTNKPRPTRAEASDVANAIFDGCDAVMLSGETAKGKYPVECVQCMARICAKVEAVLWYES
LQNSLKREIRTSAADHISAVTTAIAEAATVGQARAIVVASPCSMVAQMVSHMRPPCPIVMLTGNES
EAAQSLLFRGIYPLLVEEMVIGSFNFRRIMQSGLKLMGKMDILEPGQKGSVVLVNAMSAEKITFRLF
TIRQQTKEERDQDERCRKLALEQSCKERAEKEECRKLQQAEECQKQKLAKKCKQFEEKQKVCPK
KNDTPKNDCPKKDCPKKECPKQDDEISKCRQMQEAEAEERKCKEEFEQMCKLAEEKRKEAEKC
RKADEERRKEEAEKCRKLEEDRKCKLAEEKKRNEEELKIIEAEVAKLEAAEKAKRLKEEEKKKEEL
MKCKQRNEAKKKREEAERCKRKERERELAEMENKWKQVAEKRKRKKAAEMCRKIEDAKEKAAA
ESADKILKAVCEKLKQSLSDPDKSKKGKK (SEQ ID NO:50)

# Figure 21

**Name:** ACXE
**Nucleotide:** AE003638
**GI:** 7297983
**Transcript:** CT38110
**Sequence:**

atgctgcatatattcgttacctccgtgcactgtgccctactttagcaacgattgagcgccgatcaattatttatttcgatgta
gccctttccataggatgcgcattggtccttatacttgtgctgagtgttaacttctgcgatgaatttattgcgaaacacacgtggtacatgtatgcc
agctcgatatttgcctccctgactttggtattcgctgacctaactgaatcaatttaccacacgtatgcgcacagctggatactgggcacatttta
cgacacctacataatatacatgatatacatgttcctgccgatccacttcatcagcggtgcagtcctgctggctctgttggtgtccggtctctatat
actctactttgtgatcttcattgcccagggcttcgcccagtttgcgtctgcactattctcggtcggcggaatgtcggtggacattgtgcactatctg
tgcctaaatcttgtaggcattttctaccgcgttatgaacgacacggtggtgcgctcctcctttctggaccgacaccagtacatcaaggagaag
atttggctgcgcaacgcacggctgcaggagaagcagttgctggacagcatcctgccgccgcagatctccctgcccctgcagaaagaca
ttcagggcaggatcgtaatggcaaagcaaggtattcactcctggaccgccatggagcgcacaatggccatacagatccatcccgatgtct
ccatcctgtatgcagacgtcgtgaactacactcacttgactaccacactgacggtggagatgttggtgaaggtcctgcacgatctctacggc
agattcgatttggctgcctatcgctacaaggtgcagcgcatcaagttcctgggggactgctactactgtgtggcgggtctgtcggaccccga
tcccgatcacgccaataactgtgttatcctaggtctttccatgatcaaccacattatggaggtgcgggacattcacggcttggacataaatat
gcgcattggagttcattcgggcaatctgtttgctggggtcatcggggaggccaagctgcagttcgatatatggggattggatgttaccatagc
caatgttctagaatcaactggagtgccgggatgtgtgcacataagtggtgccaccttaaacaatctagacgttaatcgcttcgatattgagg
atggacctgaagaagccagggatcatcctcttctgaaaaaataccgaataagatcatatataattcgacaagacttgcatatggatgacg
aagatagtgacgagtttctcggcgatctgcattcaatttctctgtgcaatatgggagctcagccaagaataagtgattctgctaatcaaagtct
aagggcgcttttccacgaagaattgcgggaagagtttcgcaaaatgccgatgagctgccaacgcgaagaattcgagatggcatatatcg
aggaaagactctttcactatgaacaggaggcccatatatgctttcatccctgggtgacgaccaatatgttatccctgatgatttgtttgaccttc
acattcgcccacatacccatcatggtcaagacggcagtggccattttggagacgctcgcctatctgctgttgattttcttccagttcgatttcgtc
ttccatcacagcgttacgacaaacccctatttcaaatcggagtacgctcatgccctgctaatatgcatcacctttctaattatgtttgtaaagga
gcgccaaatagagttcaccaacaaagtaaacttcaactggcgtgttgatctgcgaaaggaggagaatgccgccagccttacaaatcatt
cgatcataattattctcaacaacatactgccatctcatatcgtggatgtgtatctcaattctttggccaaacatgaactctatttcgaaaactatc
gaatggtgtctgtcatgtttgccatgctaattaactttgaaatggatcttcgaagcttaagggtgctcaatgagattattgctgagtttgatacctt
gttgctgttttataaggagtactacaccgttgagaaaatcaaaatcgtcggatgcacctacatggcggcgtgtggacttgatcttaactttgcc
ggctccactaaattcaatgaagaacaaagccgaagaattttgttccagcagagcaatgaggaccttgatgaagtggtctttgtgatgacct
cgtatgcgctggacatgatgcgaaccctggccaagagcaatgaggcgtaccagagtattgctggtgatcgcaatattacggatggaaca
atcgccatcggcatctccagcggtgaggtgatggccggcattgtgggtgcctcgcagccgcactacgatatatggggcaatccagtcaac
atggcctcgcggatggaatccacggggctgccaggacacatccatgtaacggaagagacgtccgaaattctgcaacagttcggcatta
cctgttcatatcgcggcatgacttttgttaaggggcgcggcaaaataccaacctatttggtgggtatcgatgagaaccttaacttcattccaca
gaaggcaacgcgatttcccagtcaccaggagcgcagtacggttatatcgttgcagtcaacctatactcatgcggagaataataacagtat
agctagcacgtctaggcacactttgcaaagtctataa (SEQ ID NO:51)

**Protein:** AAF53229
**Protein GI:** 7297987

MPRSLGNCQLNYSKERMWEPGYLKAKCAELRLESEFRLYRIRLWKSYLLTFFMLHIFVTS
VHCALLLATIERRSIIYFDVALSIGCALVLILVLSVNFCDEFIAKHTWYMYASSIFASLTLVFADLTESIY
HTYAHSWILGTFYDTYIIYMIYMFLPIHFISGAVLLALLVSGLYILYFVIFIAQGFAQFASALFSVGGMS
VDIVHYLCLNLVGIFYRVMNDTVVRSSFLDRHQYIKEKIWLRNARLQEKQLLDSILPPQISLPLQKDI
QGRIVMAKQGIHSWTAMERTMAIQIHPDVSILYADVVNYTHLTTTLTVEMLVKVLHDLYGRFDLAA
YRYKVQRIKFLGDCYYCVAGLSDPDPDHANNCVILGLSMINHIMEVRDIHGLDINMRIGVHSGNLFA
GVIGEAKLQFDIWGLDVTIANVLESTGVPGCVHISGATLNNLDVNRFDIEDGPEEARDHPLLKKYRI
RSYIIRQDLHMDDEDSDEFLGDLHSISLCNMGAQPRISDSANQSLRALFHEELREEFRKMPVSAFS
PKRLLGICRFNTGKEVPAHQNLNICLTFTDPILERAYLKQTDYMYKYSIILSASVGCSLVYIELMDTQ
MICSSCFVLPASVATIQCILALIAWYKKYCWTRYGRNNVPHHYNGFSCFIFRIHDKILNSLPIRICIYL
FLMISSFFMSCQREEFEMAYIEERLFHYEQEAHICFHPWVTTNMLSLMICLTFTFAHIPIMVKTAVAI
LETLAYLLLIFFQFDFVFHHSVTTNPYFKSEYAHALLICITFLIMFVKERQIEFTNKVNFNWRVDLRKE
ENAASLTNHSIIIILNNILPSHIVDVYLNSLAKHELYFENYRMVSVMFAMLINFEMDLRSLRVLNEIIAE
FDTLLLFYKEYYTVEKIKIVGCTYMAACGLDLNFAGSTSTNRKESIPPTEFNEEQSRRILFQQSNED
LDEVVFVMTSYALDMMRTLAKSNEAYQSIAGDRNITDGTIAIGISSGEVMAGIVGASQPHYDIWGN

PVNMASRMESTGLPGHIHVTEETSEILQQFGITCSYRGMTFVKGRGKIPTYLVGIDENLNFIPQKAT
RFPSHQERSTVISLQSTYTHAENNNSIASTSRHTLQSL (SEQ ID NO:52)

# Figure 22

**Name:** EG:52C10.5
**Nucleotide:** AE003802
**GI:** 10727480
**Transcript:** CT19856
**Sequence:**

acacagctgttttgttaaacaaaacagaattattcgccaattgtttacaaaaacactcaaaaagagtctaagcaaaca
attggcaagcaagtgaagactggctagtctcagattaaattattccataagtgatggacagccaggcaccgatcgatgatctactgctgga
cattgtagacaatgcttggcgcatggaagttctacccttcgaccagattctagtgccacgcgaaaagctacccgatccagaggcggacgg
cggtgattcacacctgacggtcagcgagcaggagcaaaagtggactgatctggcgttgggctcgctggcaccggacgcagccctcatc
gatcaactgaacatcacctctatctaaccaaggaccgtgagtccacgggcgagtgccaccgcctcgccaaccaacctattagcaccac
aagcccaactcctaacaccccgacaacatgactagcaacaaccaaaacaatagctcaagccaccagcacctacactccccctccaa
actaaccgaatttgcccgcaatttcgaagacaagccggaatcgctctttggccgagtggtaaacaagatccagaatgtatacaaccaga
gctacaataccgtcaacgatatatcaagtgggagcagcagcagttcctccacgcagccagtgcaagtggtgggcaaatcacagttcttc
agtgactcccagacttccaccgctgagattgccgatgtggaaacctctagccagagctcagttcgcccccagccacccaccacgctcag
cataaggaccaatagtgagacacgtggcacaagcacctccagcaataccgctgcagaggactccgaaaccagcgaccgcgtcgaa
acgctgccgctgccaaccagcgaagcgaatcaggggcgcaccgtttctaacgtcctcaaacacataagcaatattgtggccacaaaaa
acaataacgatctgcgaaactacaaggacacggagctgcagcgcttttggatgcccgattccaaggctaaggaatgctatgattgctctc
aaaagttctccaccttccggaggaaacaccactgtcgattgtgtggccaaatattctgctcaaagtgctgcaaccaagtggtgcctggcat
gataattcgctgcgatggcgatctcaaagtgtgcaactactgctccaagatagtgctcacattcctgaagtcctcaagctctgagatgggac
aggatatgcaggagctgcagcagcacttaagtaacaaactggaagtacaagatagtggcagctcgcttgcaaaacatccccagatgca
aagggcacccccttccgcgtaagacatcagtgggctatcaagaggagcgattcagttcgcatccaacttacacaacccttccatcgacga
tcgcaagaatatcctgcagcagtcaaattcattaattaccctgcacgaggaaatgcagcgtgatctgcctgctcaaaattgtggtcagcgtc
tgattgagtttctcaatagtaataataaatcagcaaacgaggtgcaggcggtggccatactgaatgctatgctagccgctggtttcctcgag
ccaatcgtacccgatcccgagcaaatggacttcgattcgtcgctacactataagttctccaaaagcagcagttcggatacctcccgaacg
atgagtccgcaatttgaggcgaatccccatgcggaaccacaacctccaaagtcaatggatcaatcagctgaagaaaaggagaaggag
ctagaaaatgaactggaaaacgatcgatgctataccacagcaacttctaagcttcttgcctcctattgcgaacacgaagagcagctactg
gcacagatgttgcgcgcacataatttggaccaggaatgggacaaggtgcttcagatgctttgttccacggcagcgaatcacttcaagcca
gagcattgtagtaacgatctcatggacatacgtaactatgtaaactttaaaaaggtgccgggtggcagacgcaaggactcgaagattgttc
atggagtggcattctctaaaaatgtcgcccataaagatatggcaactcatgtgccctttcctcgcatactgctgctgcagtgtcccattgtgtac
gagcgtattgagggaaagtttgtgaccattgagaccgtgctgctccaggaaaaggaataccttcgaaacgtttgcgcccgcattatgagct
tcaagcccaacgttgtgcttgtccacaagaatgtggcgggcattgcccaggaccttttgagatcctatgaagttacccttgttctggatgtaaa
gctttcggtaatggagcgtctgtcacgcaccctgcagtgcgacattgtcagttcaattgagtcgaatataaccatgcccaagctgggctattg
caatgattttacatacgcaactataatggcaaaacgttgatgttctttgagaagctgaccaatccccggggatacacctgtctcctcagagg
tgggagcaatgctgaactgacgagggtcaagcgggtggcatcggccctgctctttgcacgctacaactggcgcttggaaatgtcattcctg
ctgaacgagttcgcccaaccacttagtccaaaaccttcgatattcgactccaaggaaacaagccccaaaacggaaacggaggcagaa
cttcgatctaagcgaccaattattttagagcgcaagtccgaggataagatcaccaccatcgtcagcgagaatgtgtctgatttcacggatcc
attgcgtgcatcacaagccgaagccctgtccacttcgccttgtgctccacccgtggttgaggcgctagctgtggaaccgcgttacgacaac
agattccgcacggcactcagttccacgctgctctctgtgagtcccttcctgacatttcccctaccttatttggaaacggaacagggaaggaa
atgcaagctgcgtaagctgtttcccgcagagctatacttttccaaacaatggtcccggaccggattggagaggccagatagcatgggaga
cggcgaagcagggaaatctgagccaggaaacaaagagaaccagatgcagctacttccagcccatgattttgtgctgatgaagatcaca
gcacctgctagcagtcgagacattcagtcgaagctagctgagttccgctcctttggcggtcggttacccaagggcaaggctccgatgctcc
gaccgaaaaaaaagaacgccgaagtgatccagcgaccgcagaaggtcagtgaggagcaactgtacaaggatgcccttgatccgca
gaaccatcaacgtctaccggtgctcttctgcagcttccattacaatcccaagggcgtttcgtcgttctgcaagctgcccatgctgctggacatg
aagttctacgggcaatatgacatcatgctggagcagttcctccaacgttactgctgtctttttaactccatgtgcccgtcgtgcaatctgccgat
gctgggtcatgttcgtcgatatgtacactcgttgggttgcgtacatgtctacctaaccgaggacctaacccgttccgaccccacgcgtatata
ctttacctcctggtgcagcatatgcaatgccactacacccactattccgctttccgatgccgcaaagtgcctttccctcgccaagtaccttgag
atgcgcttccatggccacgcctacaagagacgacctccttcgacggacgcagaacaaggtggcactgtatgtgaacactcattgcatcgt
gattatgtgcaccacttcagtttccgcggtgttggcgccaaattccaatatacgcccgttgaggtttgggagacggatcttccttcgttgactgt
gcaactggatttgccgcaaccatttcaaagtgcccaagtccaggaggaaattaaaaacttttcgataaagggtcacgaggtttacaacag
gattcacgagcgtattgctgacctcgccaccgaggaagagaactcaccactggtgcagcatctgaaaaccatgctcacgcatgaccagt
ttatcttcaagcagaagattgagatcgtacacacactgctcacggataacagggctactgcctacgacactagcgatgctttggccatggc
aagaagagctctggcagagagcattgagctgtggggaccgcggttgcaggagatcgagaagctgaccgccaagcaggcgcatcaca
tcgactctggaaccatctgcacggaggaactaagaccggaacaggttcagactgcagattcttctaaagttaccacctctagtttgccaaa
ggagaatgatcccctggagtgtccaagcgaggacactgaaactggcgcatcaaacagtcagactgttttggacaagaatttctctattgac

cagatgttggcctctaccgttaacgtttactcggataaaaagtctataaggaagattcttacccagcttctgccatctggtaatcaggttaacc
ctttgcagtcgccatttccggcacaagatcatctgacgcttccattgggcagtattcccattcacgtgagagaaaccgatctcagttcagtgat
tgcatacagtttgacctcgatggattatcaaaaggcaatagacgaggcggaggccaactccaatgctgcccattcaagtccgcagttaaa
gcgaaaaattccattagcggaaagcgttagcgacgccgaagacagtccaagcctttcgcgtacttcgagcaacacaagtgccgctccc
aatgcatcggttccttctccagctaccgcggcctccgagtcggaggaaaaaagcaaggaacgcataaagcagccaccgagtccgcat
atcacactggcattccaagatcacagctgccagttccagtgtaagatctactttgcccgcgagtttgatgcaatgcggtcgaagagtttgaa
gcctcccaagctagacaaatccctgtaccgccggctggagaagagcaagatgcgggaggagctgagaatctcacagagtcgcaccg
ggtccgaaatggagctggttcgcaaacccagcgacgttggtgcgcctcgaaccactgaggatgattccaatcaggaggaggacgctag
gatagcattggcgcgaagcctttgcaaaagtgtccaatgggaggcgaggggcggtaaatcgggatcccggttttgcaagacgcttgacg
atcgtttcgtgctcaaagagatgaactcaagggacatgaccatctttgagccatttgctccgaaatactttgagtacatcgacaggtgccag
cagcaacaacagcccacattgctggccaagatctttggtgtcttcagagtcagcgtgaagaaaaaggattctttcgtggagcgttctgtgat
ggtcatggagaatctgttttacggctgcaatattgaaaacaaatttgatcttaagggctcagagaggaatcgcttggtggatcccagcaatc
agcaaggtgaaattgtcttgctggatgagaatctggttcagatgtcctggtcaaagcctttgtatgtgctgtcccatagcaaaactgtcctgag
ggatgccatccagcgagattcgtccttcctggaaaaaaacctcgtcatggactattcacttttggtgggcttggacaagaaaaatggcgtctt
ggtacttggcatcatagattacattcgaacctttacgctggataaaagagtggaatccattataaaaggatcgggaatccttggaggcaag
ggaaaggatccgaccgtggtcaatccggagcgctacaagcagcgctttatcgatgcgatggatcgctatttcctcacagttccggatcgtt
gggagggcctttccaaggtctaatccccttcacccacccacacacacacacacacacacacacaaagtgcacacgttagttaactta
acatagctatagacgcaataaagtagacgccaatcc (SEQ ID NO:53)

**Protein:** AAF57789
**Protein GI:** 7302711
MTSNNQNNSSSHQHLHSPSKLTEFARNFEDKPESLFGRVVNKIQNVYNQSYNTVNDISS
GSSSSSSTQPVQVVGKSQFFSDSQTSTAEIADVETSSQSSVRPQPPTTLSIRTNSETRGTSTSSN
TAAEDSETSDRVETLPLPTSEANQGRTVSNVLKHISNIVATKNNNDLRNYKDTELQRFWMPDSKA
KECYDCSQKFSTFRRKHHCRLCGQIFCSKCCNQVVPGMIIRCDGDLKVCNYCSKIVLTFLKSSSSE
MGQDMQELQQHLSNKLEVQDSGSSLAKHPQMQRAPLPRKTSVGYQEERFSSHPTYTTLSIDDR
KNILQQSNSLITLHEEMQRDLPAQNCGQRLIEFLNSNNKSANEVQAVAILNAMLAAGFLEPIVPDPE
QMDFDSSLHYKFSKSSSSDTSRTMSPQFEANPHAEPQPPKSMDQSAEEKEKELENELENDRCYT
TATSKLLASYCEHEEQLLAQMLRAHNLDQEWDKVLQMLCSTAANHFKPEHCSNDLMDIRNYVNF
KKVPGGRRKDSKIVHGVAFSKNVAHKDMATHVPFPRILLLQCPIVYERIEGKFVTIETVLLQEKEYL
RNVCARIMSFKPNVVLVHKNVAGIAQDLLRSYEVTLVLDVKLSVMERLSRTLQCDIVSSIESNITMP
KLGYCNDFYIRNYNGKTLMFFEKLTNPRGYTCLLRGGSNAELTRVKRVASALLFARYNWRLEMSF
LLNEFAQPLSPKPSIFDSKETSPKTETEAELRSKRPIILERKSEDKITTIVSENVSDFTDPLRASQAEA
LSTSPCAPPVVEALAVEPRYDNRFRTALSSTLLSVSPFLTFPLPYLETEQGRKCKLRKLFPAELYFS
KQWSRTGLERPDSMGDGEAGKSEPGNKENQMQLLPAHDFVLMKITAPASSRDIQSKLAEFRSFG
GRLPKGKAPMLRPKKKNAEVIQRPQKVSEEQLYKDALDPQNHQRLPVLFCSFHYNPKGVSSFCK
LPMLLDMKFYGQYDIMLEQFLQRYCCLFNSMCPSCNLPMLGHVRRYVHSLGCVHVYLTEDLTRS
DPTRIYFTSWCSICNATTPTIPLSDAAKCLSLAKYLEMRFHGHAYKRRPPSTDAEQGGTVCEHSLH
RDYVHHFSFRGVGAKFQYTPVEVWETDLPSLTVQLDLPQPFQSAQVQEEIKNFSIKGHEVYNRIH
ERIADLATEEENSPLVQHLKTMLTHDQFIFKQKIEIVHTLLTDNRATAYDTSDALAMARRALAESIEL
WGPRLQEIEKLTAKQAHHIDSGTICTEELRPEQVQTADSSKVTTSSLPKENDPLECPSEDTETGAS
NSQTVLDKNFSIDQMLASTVNVYSDKKSIRKILTQLLPSGNQVNPLQSPFPAQDHLTLPLGSIPIHV
RETDLSSVIAYSLTSMDYQKAIDEAEANSNAAHSSPQLKRKIPLAESVSDAEDSPSLSRTSSNTSA
APNASVPSPATAASESEEKSKERIKQPPSPHITLAFQDHSCQFQCKIYFAREFDAMRSKSLKPPKL
DKSLYRRLEKSKMREELRISQSRTGSEMELVRKPSDVGAPRTTEDDSNQEEDARIALARSLCKSV
QWEARGGKSGSRFCKTLDDRFVLKEMNSRDMTIFEPFAPKYFEYIDRCQQQQQPTLLAKIFGVFR
VSVKKKDSFVERSVMVMENLFYGCNIENKFDLKGSERNRLVDPSNQQGEIVLLDENLVQMSWSK
PLYVLSHSKTVLRDAIQRDSSFLEKNLVMDYSLLVGLDKKNGVLVLGIIDYIRTFTLDKRVESIIKGS
GILGGKGKDPTVVNPERYKQRFIDAMDRYFLTVPDRWEGLSKV (SEQ ID NO:54)

# Figure 23

**Name:** gatA
**Nucleotide:** AE003724
**GI:** 10726610
**Transcript:** CT18575
**Sequence:**

cgaaaattgggtgaataagttaaaatccagctgctttcaaactgaaaagaatgcggcgtcacttacagtggagcatta
agcagctaacagcgagttactcagatggtcagctgtctcctcgccgggtgaccgaagatgctctgcaagatgccctgcggtggaagaca
ctgaatgcctttgtgcgcctcacgccggaacaggcggggcagcaggcccaggacgcggagcagcgctataagctaaggcaaccgatt
agtgatcttgatggagtcaccattgccatcaaggacaacttttgcaccaaagatgtacacaccacctgtgcctcacggatgctgcaagactt
tgtgcctccctatgatgccactgtgtgctccagacttaaacaagccggcgctgtaattctgggcaagacgaatatggatcagtttgccatgg
gcgccggcactgtggactccctgtacgggccgactaagaacatctggagcgaggaccttaacaaggatcactggcgcatcgcgggcg
gcagttctggaggctctgcatccgccgtagccgcgggtctttgttatgcagcaattggttcggacacgggtggctccacccgaaacccggc
ttcgtactgcggcgtggtgggtctaaagcccacctacggcctcgtctcccgccacggtcttattccgctggtcaattccatggacgtgcccgg
catttttgcccgctccgtcagcgattgcgtggcggtactgaatacagtggcagggccggataaactggactccacaaccatcaggcagcc
ttttaccaggttacatctgccggaagttgggcaaattgacctgagcactgtgcgcataggaataccaaaggaataccattgccacggtcttt
ccgccgaagttctggaaacgtggtctaaggtggccgatttgctggagtgtagcggtgcctccgtcagacaggtttccctgcccaatacggc
agcaagtatattcgtgtacaccattcttaatcagtgcgaggtggccagcaacatggcaaggtacgatggaatcgagtacggccatcgggc
aacagacgagcgcagcacggagcaattgtatgccctatccagggccgagggattcaacgatgtggttaagacacgcatactcactgga
aactttctgctgctgaaaaagaactatgatcactactttgagaaggctttgcgggtgcgccgccttattgcagaggactttgcaagggtgtttg
attcctcggccaaagaggagcgcgtggacatccttctcacgccgacaactcttacagaggctccgctgtacaaggacttcgcctccctga
ccaacagggatcagtgtgccgtgcaggacttttgcactcagccagccaacatggctggaattcccgccgtgtctataccgattcgcctgtcc
caggccggattgcccttgagccttcagctgatgagcaacagcctcaacgagcagttactgctcacagtggcacgatggatagaggcgca
ggtggaattcgacagcctagagcactcgcaacagtataaggccagtttgtaa (SEQ ID NO:55)

**Protein:** AAF55624
**Protein GI:** 7300468

MRRHLQWSIKQLTASYSDGQLSPRRVTEDALQDALRWKTLNAFVRLTPEQAGQQAQDA
EQRYKLRQPISDLDGVTIAIKDNFCTKDVHTTCASRMLQDFVPPYDATVCSRLKQAGAVILGKTNM
DQFAMGAGTVDSLYGPTKNIWSEDLNKDHWRIAGGSSGGSASAVAAGLCYAAIGSDTGGSTRNP
ASYCGVVGLKPTYGLVSRHGLIPLVNSMDVPGIFARSVSDCVAVLNTVAGPDKLDSTTIRQPFTRL
HLPEVGQIDLSTVRIGIPKEYHCHGLSAEVLETWSKVADLLECSGASVRQVSLPNTAASIFVYTILN
QCEVASNMARYDGIEYGHRATDERSTEQLYALSRAEGFNDVVKTRILTGNFLLLKKNYDHYFEKA
LRVRRLIAEDFARVFDSSAKEERVDILLTPTTLTEAPLYKDFASLTNRDQCAVQDFCTQPANMAGIP
AVSIPIRLSQAGLPLSLQLMSNSLNEQLLLTVARWIEAQVEFDSLEHSQQYKASL (SEQ ID NO:56)

# Figure 24

**Name:** CG17149
**Nucleotide:** AE003514
**GI:** 10727803
**Transcript:** CT33310
**Sequence:**

ccagaacggtataaacaatttttatattttgcacttttaaatgtcggaaaatcaatctgtgagccaacctaatatgaaaag
cgacgcatggatcagctgatacagcatttgataataatacttcattgtcatcagagaagcagggagtttagaccaaaaaaaaaaaactaca
cccgcagtccgatcctcaaaacgtgtgataataaaatcacggaacaatcagaaaaatatcgaaagaataatgcggcaatattccggttt
ggtggactaaagaattcgtgattttcgaacgtgagctagctttattggaattggaaatgagttcgagaaaaatttaaacgggttccacttttga
agggccaacttttcagtgtcccggtggcagttccataatccggagatgaaacccacccagttcggaggcagtagctccaagatgaccga
gccaattgagtatgtgacgctaattagcgacgattccgatggtgagcccacgcccaaaagaaacgtcaatcacccgccgtcggcattgtc
agcacccaatcctggtcagaaacaaaagcacccggatgaggactcaaatgacgcacctgccaccagcgatgagcgacgcacaagt
aggcgcaatcggccaaaagtggattatagtaacaggcctagtggaagtggagacaccgcctccaatgacaagtcaggatcggcatcc
atgggacctaacaaccagcaagcagagcgtcgcagccaaagccaaacccgcaagtcagaagccaatgctacctcctcctccgtgtcc
ggacccagtgccggaaattccagaccgtcccagaacggagattccaaggaccgggatgccggcacgcccacggttttgtctggccaa
gagggcgccgtcttccaatctcgcctgccatttaacaaaatgacacccaacgaagaggcctgctttccggacatcagtcgctcgggcattc
tcggccatcgggtattcctcaacattcgtaacagcctcttgcacatgtgggtggacaatcccaagatccagctgagcttcgagatcgccctg
aagaacctgccgccgccattcgacagcgagcccagtttggtaaggcgagtacactcgtttctggagcgtcatggcttcattaacttcggcat
ctttaagcgacttaagcctatacccgccaaaaagctgggcaaagtcatagtgattggggcagggatctccggcttagcagtggcacacc
agttgcagcagttcggcatggatgtgatcgtcttggaagcgcgggatcgggtagggggacggatatctacgtttcggaagaacagctatat
tgccgatgtgggagcaatggtggttaccggcgtctatggcaatccaatgacgatcctcagcaagcagatcggtatggacctagttcccatc
cagcagacatgtccactttacggcccagatggaaagcccgtgcccaaggagaaggatgacgtgatcgagagagaatttaaccgactg
ctggaatcagccagctacctgtcccaccgccttgattttaactacgccggcgattgcccagtttcgctgggcgatgccttggagtggataatc
agtatgcaggagatgcaggtgatgcacaaacgggggcagcacatgcaggagatcatagccacccaaacgaagatcattgaacagc
gtcgtcgactgaaaacccttagagataccatcggcaaactaaagaatgagcacttggcaatgatcaaccagcggaaacccaaaggca
cggatggcgatctgaaatactgttaccaagagtttaacattcggaacacccagatcaaaatggaggaaacgatcagtacgtttcacgatc
tccatgccgaggaaaagcagatgttagccaaactccacgaactggagcagaaccgacctagcgatgtgtacctctcgtcccgcgaccg
cctgatcttggactggcattttgccaacttggaatttgctaatgccacccgactaaataacctgtcgttgaagcactgggatcaggacgatga
cttcgagtttattggccaccacacaactgtgcggaacggttattcatgtgtgcctgtagctcttactgaaaacctggacatccgtgtcaatagc
gccgtcaaggagataaagtacggcaccaaaggtgtggaagtagtggctgaaaacttgaagacatccaattcgcagatgacatacaag
gcagaccttgtcgtctgcacacttactctgggcgtcctcaaagtggcggtagcacataaggagtcacagcagagtaacacagtcaagttc
gacccgccattgccggactggaaacagcaggccatcaaaaggctgggatttgggaatctcaataaggttgtgctctgcttcgatcgcatttt
ttgggatcccaacgctaatctcttcggacacgtgggcagcacgacggcgagtagaggagaaatgtttctgttctggagtataagctcatctc
cggtgttgctcgctctagtcgccggaatggcagcgaatctggtggagagcgtgacggatgacatcataatcgggcgttgtatgtcggtgct
aaaaaacatcttcgggaatacctctgttccacaacccaaggaaacagtggtaacgcgttggcgcagcgatccctgggcccgtgggtcgt
acagctacgtatccgttggttcctcgggcagtgactacgatctgctggcggcgccggtcattccgccgtccagtaaggatgcggaaggatt
gccacggctattcttcgcgggtgagcatacgatacggaattatccggccactgtgcatggagcgtacttgagcggattgcgcgaggcggg
acgtattgcggattactatctcggctatccagagggaacaccgccggacattggctactcggtggctgaggcggccaatctggtgtccgtg
ggaaatgttgtcaagctgcgcgacctctcgcccaatctatctgactcctccccgtcgtcaaagaagtcggaggagaattcaaactcaaac
actgccgactctacggagctacagtaacaatgtatttagcgtgatggatttggtccccagcagttactcctagagcgtttagttttgtatcatac
aactggggctcgctgtttaaaacgaagctaggccgctgtaaatacctaagactattcaactaagcatagaccactttcattcaaccaattat
aaattaagataaaaagcaataacacccaacaaagcaaatcctcttcagtgtttccttcagatcaaagtaaaccaacctctgtgcgcctata
attgatcaacaaatgcaatcatctatcataactttccgtaccacttacccgccaatgaggagtatatcttttaagtatattatcgcctataagatt
gttttcaataagtattttgtaaattaaattgaaaatagtaaattaaattgacgccgatatttttaattaggttaatttaatttaagtacaaatttcatat
tacttccaacttaaaaacactttcaatttttttaatatcttattgttatcttgataaaatacgatgtagaaaaatgtgtacgcaaatcatggcataa
attgagttttaaaatataatgtaagctt (SEQ ID NO:57)

**Protein:** AAF49052
**Protein GI:** 7293682

MKPTQFGGSSSKMTEPIEYVTLISDDSDGEPTPKRNVNHPPSALSAPNPGQKQKHPDED
SNDAPATSDERRTSRRNRPKVDYSNRPSGSGDTASNDKSGSASMGPNNQQAERRSQSQTRKS
EANATSSSVSGPSAGNSRPSQNGDSKDRDAGTPTVLSGQEGAVFQSRLPFNKMTPNEEACFPDI
SRSGILGHRVFLNIRNSLLHMWVDNPKIQLSFEIALKNLPPPFDSEPSLVRRVHSFLERHGFINFGIF
KRLKPIPAKKLGKVIVIGAGISGLAVAHQLQQFGMDVIVLEARDRVGGRISTFRKNSYIADVGAMVV

TGVYGNPMTILSKQIGMDLVPIQQTCPLYGPDGKPVPKEKDDVIEREFNRLLESASYLSHRLDFNY
AGDCPVSLGDALEWIISMQEMQVMHKRGQHMQEIIATQTKIIEQRRRLKTLRDTIGKLKNEHLAMIN
QRKPKGTDGDLKYCYQEFNIRNTQIKMEETISTFHDLHAEEKQMLAKLHELEQNRPSDVYLSSRD
RLILDWHFANLEFANATRLNNLSLKHWDQDDDFEFIGHHTTVRNGYSCVPVALTENLDIRVNSAVK
EIKYGTKGVEVVAENLKTSNSQMTYKADLVVCTLTLGVLKVAVAHKESQQSNTVKFDPPLPDWKQ
QAIKRLGFGNLNKVVLCFDRIFWDPNANLFGHVGSTTASRGEMFLFWSISSSPVLLALVAGMAAN
LVESVTDDIIGRCMSVLKNIFGNTSVPQPKETVVTRWRSDPWARGSYSYVSVGSSGSDYDLLAA
PVIPPSSKDAEGLPRLFFAGEHTIRNYPATVHGAYLSGLREAGRIADYYLGYPEGTPPDIGYSVAE
AANLVSVGNVVKLRDLSPNLSDSSPSSKKSEENSNSNTADSTELQ (SEQ ID NO:58)

**Transcript:** CT38086
**Sequence:**
tttatattttgcacttttaaatgtcggaaaatcaatctgtgagccaacctaatatgaaaagcgacgcatggatcagctgat
acagcatttgataataatacttcattgtcatcagagaagcagggagtttagaccaaaaaaaaaaactacacccgcagtccgatcctcaaa
acgtgtgataataaaatcacggaacaatcagaaaaatatcgaaagaataatgcggcaatattccggtttggtggactaaagaattcgtga
ttttcgaacggccaacttttcagtgtcccggtggcagttccataatccggagatgaaacccacccagttcggaggcagtagctccaagatg
accgagccaattgagtatgtgacgctaattagcgacgattccgatggtgagcccacgcccaaaagaaacgtcaatcacccgccgtcgg
cattgtcagcacccaatcctggtcagaaacaaaagcacccggatgaggactcaaatgacgcacctgccaccagcgatgagcgacgc
acaagtaggcgcaatcggccaaaagtggattatagtaacaggcctagtggaagtggagacaccgcctccaatgacaagtcaggatcg
gcatccatgggacctaacaaccagcaagcagagcgtcgcagccaaagccaaacccgcaagtcagaagccaatgctacctcctcctc
cgtgtccggacccagtgccggaaattccagaccgtcccagaacggagattccaaggaccgggatgccggcacgcccacggttttgtct
ggccaagagggcgccgtcttccaatctcgcctgccatttaacaaaatgacacccaacgaagaggcctgctttccggacatcagtcgctcg
ggcattctcggccatcgggtattcctcaacattcgtaacagcctcttgcacatgtgggtggacaatcccaagatccagctgagcttcgagat
cgccctgaagaacctgccgccgccattcgacagcgagcccagtttggtaaggcgagtacactcgtttctggagcgtcatggcttcattaac
ttcggcatctttaagcgacttaagcctatacccgccaaaaagctgggcaaagtcatagtgattggggcagggatctccggcttagcagtgg
cacaccagttgcagcagttcggcatggatgtgatcgtcttggaagcgcgggatcgggtagggggacggatatctacgtttcggaagaac
agctatattgccgatgtgggagcaatggtggttaccggcgtctatggcaatccaatgacgatcctcagcaagcagatcggtatggacctag
ttcccatccagcagacatgtccactttacggcccagatggaaagcccgtgcccaaggagaaggatgacgtgatcgagagagaatttaa
ccgactgctggaatcagccagctacctgtcccaccgccttgattttaactacgccggcgattgcccagtttcgctgggcgatgccttggagtg
gataatcagtatgcaggagatgcaggtgatgcacaaacgggggcagcacatgcaggagatcatagccacccaaacgaagatcattg
aacagcgtcgtcgactgaaaacccttagagataccatcggcaaactaaagaatgagcacttggcaatgatcaaccagcggaaaccca
aaggcacggatggcgatctgaaatactgttaccaagagtttaacattcggaacacccagatcaaaatggaggaaacgatcagtacgttt
cacgatctccatgccgaggaaaagcagatgttagccaaactccacgaactggagcagaaccgacctagcgatgtgtacctctcgtccc
gcgaccgcctgatcttggactggcatttgccaacttggaatttgctaatgccacccgactaaataacctgtcgttgaagcactgggatcag
gacgatgacttcgagtttattggccaccacacaactgtgcggaacggttattcatgtgtgcctgtagctcttactgaaaacctggacatccgt
gtcaatagcgccgtcaaggagataaagtacggcaccaaaggtgtggaagtagtggctgaaaacttgaagacatccaattcgcagatga
catacaaggcagaccttgtcgtctgcacacttactctgggcgtcctcaaagtggcggtagcacataaggagtcacagcagagtaacaca
gtcaagttcgacccgccattgccggactggaaacagcaggccatcaaaaggctgggatttgggaatctcaataaggttgtgctctgcttcg
atcgcatttttgggatcccaacgctaatctcttcggacacgtgggcagcacgacggcgagtagaggagaaatgtttctgttctggagtata
agctcatctccggtgttgctcgctctagtcgccggaatggcagcgaatctggtggagagcgtgacggatgacatcataatcgggcgttgtat
gtcggtgctaaaaaacatcttcgggaatacctctgttccacaacccaaggaaacagtggtaacgcgttggcgcagcgatccctgggccc
gtgggtcgtacagctacgtatccgttggttcctcgggcagtgactacgatctgctggcggcgccggtcattccgccgtccagtaaggatgcg
gaaggattgccacggctattcttcgcgggtgagcatacgatacggaattatccggccactgtgcatggagcgtacttgagcggattgcgcg
aggcgggacgtattgcggattactatctcggctatccagagggaacaccgccggacattggctactcggtggctgaggcggccaatctg
gtgtccgtgggaaatgttgtcaagctgcgcgacctctcgcccaatctatctgactcctccccgtcgtcaaagaagtcggaggagaattcaa
actcaaacactgccgactctacggagctacagtaacaatgtatttagcgtgatggatttggtccccagcagttactcctagagcgtttagtttt
gtatcatacaactggggctcgctgtttaaaacgaagctaggccgctgtaaatacctaagactattcaactaagcatagaccactttcattca
accaattataaattaagataaaaagcaataacacccaacaaagcaaatcctcttcagtgtttccttcagatcaaagtaaaccaacctctgt
gcgcctataattgatcaacaaatgcaatcatctatcataactttccgtaccacttacccgccaatgaggagtatatcttttaagtatattatcgc
ctataagattgttttcaataagtattttgtaaattaaatttgaaaatagtaaattaaattgacgccgatatttttaattaggttaatttaatttaagtac
aaatttcatattacttccaacttaaaaacactttcaattttttaatatcttattgttatcttgataaaatacgatgtagaaaaatgtgtacgcaaatc
atggcataaattgagttttaaaatataatgtaagctt (SEQ ID NO:59)

**Protein:** AAF49051
**Protein GI:** 7293681
MKPTQFGGSSSKMTEPIEYVTLISDDSDGEPTPKRNVNHPPSALSAPNPGQKQKHPDED
SNDAPATSDERRTSRRNRPKVDYSNRPSGSGDTASNDKSGSASMGPNNQQAERRSQSQTRKS

EANATSSSVSGPSAGNSRPSQNGDSKDRDAGTPTVLSGQEGAVFQSRLPFNKMTPNEEACFPDI
SRSGILGHRVFLNIRNSLLHMWVDNPKIQLSFEIALKNLPPPFDSEPSLVRRVHSFLERHGFINFGIF
KRLKPIPAKKLGKVIVIGAGISGLAVAHQLQQFGMDVIVLEARDRVGGRISTFRKNSYIADVGAMVV
TGVYGNPMTILSKQIGMDLVPIQQTCPLYGPDGKPVPKEKDDVIEREFNRLLESASYLSHRLDFNY
AGDCPVSLGDALEWIISMQEMQVMHKRGQHMQEIIATQTKIIEQRRRLKTLRDTIGKLKNEHLAMIN
QRKPKGTDGDLKYCYQEFNIRNTQIKMEETISTFHDLHAEEKQMLAKLHELEQNRPSDVYLSSRD
RLILDWHFANLEFANATRLNNLSLKHWDQDDDFEFIGHHTTVRNGYSCVPVALTENLDIRVNSAVK
EIKYGTKGVEVVAENLKTSNSQMTYKADLVVCTLTLGVLKVAVAHKESQQSNTVKFDPPLPDWKQ
QAIKRLGFGNLNKVVLCFDRIFWDPNANLFGHVGSTTASRGEMFLFWSISSSPVLLALVAGMAAN
LVESVTDDIIIGRCMSVLKNIFGNTSVPQPKETVVTRWRSDPWARGSYSYVSVGSSGSDYDLLAA
PVIPPSSKDAEGLPRLFFAGEHTIRNYPATVHGAYLSGLREAGRIADYYLGYPEGTPPDIGYSVAE
AANLVSVGNVVKLRDLSPNLSDSSPSSKKSEENSNSNTADSTELQ (SEQ ID NO:60)

# Figure 25

**Name:** CG2905
**Nucleotide:** AE003787
**GI:** 10728163
**Transcript:** CT9844
**Sequence:**

atgctcaaccttatgaaaaattgtcccaaggaagccgctcatctcagaaaagaacttttaattgctgcccgtcatatattt
gctacagatttacgtcaaaaatttattccatcaatcgaacaattgtttgatgaggatttgcttattggaaaaggagttacattggattctattcgg
ccattggcgtattcaactttagctgacctagctcaccacgttcgccaaagcctaaatatagacgttttaataaaggcagtgaatctcttttcaa
aaaatgttcatgatgaatcacttgctgtgggaatacaaactatgtcatgcaagcttttgttaaatctagttgattgtttacgtcatcacagtgaaa
cagaaccacaaagatcaaaagctctactctcaaaactgttaaaagtgtttgtaaaaaaatttgaaactatagctaaaattcagcttccatta
ataattcaaaagtgcaaagggcacgcattttctggagcattggtaaattcatctggcaacgcttccttatcacacattaacgcgccagatcta
aaagacgacatatccaacatccaagtatctgcatctggatcacaatggatatattccgtcaatgtcgctgaatttcgaagcttagtaaaaac
ccttgttggtggagtaaaaacaataacttggggttttttaactcaaagtttcagctaactgataccaaactggccaaccatgaaaaaattttg
gacctgaaatcgtatgttcttacatcgacttggtttactatgcgatggaggctctagatatatacactataaacgttaatccaaatcaacagag
aacgtctgggttgatttccagaagtaaagaagaaaaagaagtcttggagcattttagtggcatatttttaatgatgcactcccaaaactttca
agaaatattttctactacgattaatttttggtggaaaggatctacaaaaatcagtccttacaagtgatagcgaactcctttttagcaaatcctac
tacttcacctctgtttgccactgttctagtggaatatcttttgaacaaaatggaagaaatgggatcaaacttggaacgctccaacttgtatctac
gattattcaagttggtattcggcagcgtaagtctttttccagtcgaaaatgaacagatgttgcgtccacatcttcataaaatagtcaatagatcc
atggaattggctttgatatcggaggagccgtataactatttttacttctgagggcattgtttagatcgattggaggaggaagccatgacctatt
ataccaagagtttctgccacttttaccaaacttattagaaggattaaatcgcctacagagtggatttcataagcagcacatgcgagatcttttc
gtggaactttgccttacagttccggtccgcttgtcatctttactgccctatttaccaatgttgatggacccattagtttctgcgttaaacggatctcc
aacattaataagtcagggcctgcgaaccttagagctttgcgttgacaatttgcagccagactttttatatgatcatatacaaccagtaagagc
tgctttaatgcaagcattgtggaaaactttaagaaatcaggataacgccgctttggttgcctttcgcgttctaggaaaatttggtggcggcaat
cgaaaaatgatggtggagccacaagctttgtcgtatattataaacgacaagcccaccatttccattgtaacttattttcaggaatacgaaac
accaatagattttcctgttgatgaagcgattaagtccgcctttagagcattgggatcaaattctactgaccagttttataggaggcaaagctgg
gaagtaatcaggtgcttcttagctgcttttattagtttggatgatgaaaaacatatgctgctgaaactttttacacatgtagattttgttgaaaataa
aattatgaattggtctacgtttcaacataaagccgggaacgaaactgtacgagaaactcatcagacagccctcataggtatgttggttgcat
cagcaacaaaggacttgcgtgattcagtgtgcccagtaatggccgctgttgtgaggcattatactatggtagctattgcacagcaggccgg
acctttttccacaaaaaggctaccaagccactcatggaattgaccccatgatattaatagatgctttagcctcttgcatgggtcatgaagaaa
aggagttatgcaaacccggaatagcttgtatgggtattatacttgacactgcaacaaatattatgggtaataaagacagagcgtgtaaattg
cccataatacagtatctagcagaaaaaatggtatccttatgctatgatcggccatggtactcaaaagtaggcggttgccaagcaattcaatt
tttatgcaaacatatgtctttgcgagcccttttcaaaatctctttaatttttaaaagctttcatgtttgtattaatggacctagagggagacgtttcta
atggggctattgaaataacaaaaagttatatgaaaagcatgttggaaatttgtttaacgccaataaatgaatgttataagaatatagatctaa
aggacttacaagccaaagccacttatgaagttattcacgaattagtgcggcacatcacaagccctaataccattgttagagaagaatctat
ggtactacttaaacatattggtacaatacaatccaaaaccgtttctgaagttatggatcctcataaagacgttctcgctgacattataccgcca
aagaagcatttattgagacatcaacccgcaaatgcgcaaatcggacttatggatggcaatacattctgcacaactttggagccaagactttt
tacaattgatttgacgaatacctaccacaaattatttttcacgagttacttacactaagtgaagccgaagatgccactttagcaaagttggac
tgttacaaaaacgttccgaatcttataccacttaggactagtgcgttgcgagctttagcagcatgccactatataagtgatatcggatacaaa
gaaaaaataataaatataattttaaagtaatggaaagtgataaatcagaacttcagacaactgcgtttcattgcatgaagcattttataactg
gagttactctggaaaaagaaaaggtacaatctgcaatgcgcccactgctattaaaattgggcgaccacagaaatttatcgataccagca
ataaaacgcttatcttactttactcagatctttcctcaaatgttcaatgagaagttaagtgagcaaattttgcaacattgctctaaaattatggaa
atatttgttagtgaatacaaatccacaagtccaaacgttaatttttttgcatcatcgaaaggtggagaatatgaacagaaaattgtcatactaa
tcgaaatgtttttctatatatcagcatcagtaaaatacattgaaaagctgtgccaacttgttttaaaaacggaaaaaaacttaatgattgaggc
atcgagtccatatcgagaagccctaattaagtttcttcaacgatttccaacagaaactgtggatttatttttaacggaatctcttatgatagatcc
gcaatggaatcgtctttttatatacttgttgaaacacgaaacgggagtatcatttcgagcagttattaaaagtagtcggtataataatttaatac
attatttaaatacacacacagaatttccagaagcactcaaatatgaaattcagcaccaagccgttttaataatttttacattaatggaatcaga
cgatcaatggataccaacacgacaagatattgttgacgcccttaaaaattgttggcagaattatttaagtacattaagctctgaagacgttct
gtgtgacttatggcatttgattggaaaaatacttttgcattatttttctaacaatacaaatgacattgaactgctgtttcaattattacgtgctttatgc
ttccgatttataccggatgtctatttcctgcgggacttcttgcaacacacagtagcacaaagttttacggttaactggaaacgaaacgcattctt
ttactttgttgaaaactttaataacagctttctttcagaagagctaaaggcaaaaataataacagcagtcataataccctgttttgctgtaagttt
cgataaaggtgaaggaaataaactaattggggctcctccgaccccgtatcaagaagatgaaaaaaatattgtgtccgtttttattaataaa
gtgtttgacccagataaacagtatgatgatgcagtaagaattgctctattacaactagcatgcttattggtagaacgtgcatctcagcacata
catgacggagatgctaataataagcgccaaggcaataagcttagacgactcatgacctttgcctggccctgtttgctgagtaaaagttcagt
ggatcctactgcacgataccacggtcacttattactttcgcatataatcgcaagattggccattcacaagaaaatagtttacaagtatttcatt

ccttgttgaaaggacacgcattagaggccagatctattgttaaacaggcacttgatgttctaacgccagctatgccgttgcgaatggaggat
ggaaatactatgctaacacactggacaaaaaaaattatagttgaagaaggtcacgctatgcagcagttatttcatattttgcagctcattattc
gtcattataaagtgtattttccggttcgacatcaactagtgcaacacttgattaactatatgcagcgccttgggtttcctcctactgcatctattga
acataaaaaacttgctgtggacttagcagaagttattataaaatgggaacttcatcgtattaaggatgatcgtgaaactaaaacagatgga
accgaagaggagttaatacaggaaagctcagtaaaacgatcagggatcgatttagtagaaactcgaaaaaagtcattcgatattataag
agaaacaacagtccaaggaaagtgcgttatgctattaaaaatggcgatgcgtcccgagatctggcctcaaccatttgatattaagcttaatt
ggcttgataaagttcttgctacagtggaaacccctcatcacaacttaaacaacatatgtactggaatagatttttaacatttttaactactatac
taagtccagatcagttggtgtcaattatacgaccggttcaacgaggcctgtccttgtgtataattcatcaaaatacacgaattgtgcggttaat
gcatatgtttttgacgcgaataatggcaatttttccacctgacacccaacacaagcatgaagatcttgatttgttatataccgctgttagtaaaa
tgatcgctgaaaacctaacaagttacgaaaagagtccacaaccgaatgcttcttcgctttttggtaccttaatgattttgaaagcgtgcactac
taataatgcaagttatattgatcgaattttagtccagtttataagggttctgaatcatcttacaagggatcatattaacaccattggtggtaacac
tgttattagccaatctcctgattcaaatgctttacctttagaactcttagttctctctttggaattaatcaagaataggatctttgtaatgagtgtgga
aatcagaaaacttttcataggcaccattttggttagtcttatagaaaagagcacggaggtaaaaattataaaatgtataattaaaatgctgga
tgaatggattaaaacgaaagagccaaatgttatgacacaagttccttctattcgcgaaaagtcggccttgctggtaaagttaatgcaaaac
gttgaaaagaaatttactgatgaaatagagcttaatatacaattcttggaaatcataaattttatatatagagatgaaattcttaaacaaactg
agttaacaaacaaactagagggagctttttaaacggattacgttttcaaaatccaaacgtacgctcgaaatttttgagattttagactcatca
atgcggcgtagacttcacgatcgattgttatatataatttgttcccaagcttgggacacaattggttcccattattggataaaacaatgcattga
attgcttattttaacagccaatacaatgatgcaaattcaatgttcaaatgaacaatttaaaatacccagcattacttcagtcattccagtgaatt
catcagaaacacaggaaaattcctttgtatccttcttatcctctcattccgaatcttttgacattatacaaactgttgatgataaagacgacgtgt
atgatatcgatttaaatgctgatcgcaaagaagattgtcaacaaatactaccaaatcgacgtgttactcttgttgaactagtttacaagcaag
ctgaatttttagaagcaaaccgaaatattaggaccgaccagatgcttgtcgccacatctcagctatgtcatattgatacacagttagctcaaa
gcgtatggttatctatgtttccacgtatttggagtatattcactgaagatcaaaggtgtaatatcacaaaagaactgattccctttttatcgtctgg
aactaacgttaatcaaaaagactgccatccaagtacattaaatacttttgtagagagtttaactaaatgtgcgccacccatatatattccacct
aatttattagcatacctaggcaaatctcataacctatggcatagagctatacttgttttagaagatatggccgttaatcaatcaatgcaatcca
aggatattgatggcggtgaaaatcaattctctgacttggatgtacaacaatcaaataatatatttgattcactttcaaaaatgtattcttcgatgc
atgaggaagatctttgggctggcctatggcttaaatttgcacactacccggaaacaaatatagctgtttcgtatgagcaaatgggatttttcga
agaagcccaaggtgcctatgatctagcaatgaccaaatttaaacaagatctaagtaatggtgtagttaatacatatgttaatagtgaattatt
attgtgggaaaatcactggatgcgatgtgctaaagaattgaaccaatgggacattttactggactatgcccaaactaataaggacaaaaa
tatgtttttgattctggaaagttcgtggcgcgtacctgattggaatttgatgaaaatcgcactggctaaaacagaacaatgctatttaaaacact
acggctttaaaatcaacctttacaaagggtatttgagtattctccaccaagaagaaaggcaaacaggcaatatcgaacgatatgttgaaat
tgcatccagcttatgcattcgtgaatggcgtcgattgccgaacatagtttcacatattcatttgccatatcttcaagcatcacaacaaattatgg
agcttcatgaagcaagtcaaatccatcagggacttgctcaatcgcgcaacaattcacttcacgatatgaaagctatcgtgaaaacttggcg
taatcgtttacctattatttctgatgacttatcgcattggagtgacatatttacatggagacaacatcactaccaaataataacacaacacctag
aacaacaatcggatcaaggaagtacaatgctaggagttcacgcatcagcacaagctataatttcttttggaaaaatagctcggaaacac
aatttgactggtgtttgtcaggagacgttgtccaggatatatacaattccgtctgttcctatcgtggattgttttcagaaaattcggcagcaagta
aaatgctacctgcaaatgccctcaacatctggaaaaaatgaaattaatgaagccttggaagtaattgagtccacgaatttaaaatacttca
ctggtgaaatgaatgctgaattttacgctctaaaggggctattattagcacaaattggaagatcagaagaggctggaaaatcatttagtgttg
ctgctcagcttcatgatggtcttaccaaagcctgggcaatgtggggtgactatatggaacaaatatttttaaaagaaaggaaaatcacatta
gccgtcgatgctttaatttgttatttacaagcaagcagaaatcaaattgaaagcaaaacccgaaaatatattgcaaaagttttgtggtttctgtc
ttatgataataatactaaaatcctcataagcactttagaaaagcatgtggcaggcattccaccctcttattggctaccatggattcctcagttgc
tctgttgcttagaacagttcgaaggggatgttatattaaatctcttaagccaaattggacgcctttatcctcaagcggtatatttcccgattcgga
ctttatatttgactttaaaaatcgaacaacgcgaaaaacataaaactgctgaacaggctgtaaaaagttcatgctcgaacatcgatggaac
tactttaagctttggaaggggagcaagtcacggaaacattccatcaataaatcccattaaagcaactccgcccatgtggcgctgctctaag
gtgatgcaattacagagagaagtacatccaacaatattaagttcattggaaggaattgtagaccaaatggtttggtttagagaaagctgga
cagaggaagttcttcgacaactacgccaaggcctaattaaatgctatgccatagcctttgaaaaaagggatactgttcaacattctaccata
acacctcacacgttgcattttgtcaaaaagctgggttctacgtttggcattggaatagaaaatgttccgggatcagtaacctcctcaatttctaa
ttcagcagcctcggagtctcttgctcgacgcgcccaagttacttttcaagatccagtatttcaaaaaatgaaggagcaattcactaatgactt
cgatttttcaaaacctggtgccatgaaattgcacaacttgatatcaaaattaaaaacatggataaaagtcctggagactaaagttaaaaaa
ttacccacgtcctttttgatagaagacaagtgtaggtttttatcaaactttagtcagaagacagctgaggttgaacttcctggagaattgttaatt
cccttatcatctcattattatgtaagaatcgcaagattcatgccgcgtgtggaaattgtacaaaaaaataacacagcagcgcgtaggttatat
ataagaggtactaacgggaaaatctatccgtaccttgtagttcttgattcaggtttgggagatgctcgccgagaggaaagagttttgcagtta
aaacgcatgttgaattactatttagaaaaacaaaaagagacaagtcgaagatttcttaacataacggtaccaagggttgtcccgatatcgc
cccaaatgagattggcagaagataacccaaacagtatttcattgttaaaaatatttaaaaaatgctgtcaaagtatgcaggttgactacgac
atgccaatagttaagtattatgaccgtctttctgaagtacaggcaagaggcactccaactacacataccctattgagagaaatattctctgaa
attcaatggactatggtcccaaaaacattactaaagcattgggctttgaaaacattcttggcggctactgacttttggcatttccgaaaaatgc
ttaccctgcagttggctttggcattttatgcgaacacgctttgaatcttactcgactgaatgcggatatgatgtaccttcatcaagactcaggac
ttatgaacatatcttattttaagtttgatgtaaatgatgataagtgccagcttaatcaacaccgacctgtaccatttcgcctgactccgaatgttg

gtgaattcataacacattttggaataactggacctttatctgcagcaattgtggcaacggctcggtgttttattcaaccaaattacaaattaagc
tcaatattacaaaccattttaagagatgaaataatagccctgcaaaaaaaaggattcagagaatgtaaactaatcgaaggctctgaaga
ccgttattccgatggaaattgtatggagcactcagtaaacattgtgaattcagcggtggatatcataatgacgcgttttaataaaatatcttattt
tgatagcattgaaaataagaagatttccgtgctcgttcaatcggcaactaacattgataatctttgtcgtatggatcctgcttggcatccctggct
ataa (SEQ ID NO:61)

**Protein:** AAF57342
**Protein GI:** 7302249

MLNLMKNCPKEAAHLRKELLIAARHIFATDLRQKFIPSIEQLFDEDLLIGKGVTLDSIRPLAY
STLADLAHHVRQSLNIDVLIKAVNLFSKNVHDESLAVGIQTMSCKLLLNLVDCLRHHSETEPQRSKA
LLSKLLKVFVKKFETIAKIQLPLIIQKCKGHAFSGALVNSSGNASLSHINAPDLKDDISNIQVSASGSQ
WIYSVNVAEFRSLVKTLVGGVKTITWGFFNSKFQLTDTKLANHEKIFGPEIVCSYIDLVYYAMEALDI
YTINVNPNQQRTSGLISRSKEEKEVLEHFSGIFLMMHSQNFQEIFSTTINFLVERIYKNQSLQVIANS
FLANPTTSPLFATVLVEYLLNKMEEMGSNLERSNLYLRLFKLVFGSVSLFPVENEQMLRPHLHKIV
NRSMELALISEEPYNYFLLLRALFRSIGGGSHDLLYQEFLPLLPNLLEGLNRLQSGFHKQHMRDLF
VELCLTVPVRLSSLLPYLPMLMDPLVSALNGSPTLISQGLRTLELCVDNLQPDFLYDHIQPVRAALM
QALWKTLRNQDNAALVAFRVLGKFGGGNRKMMVEPQALSYIINDKPTISIVTYFQEYETPIDFPVD
EAIKSAFRALGSNSTDQFYRRQSWEVIRCFLAAFISLDDEKHMLLKLFTHVDFVENKIMNWSTFQH
KAGNETVRETHQTALIGMLVASATKDLRDSVCPVMAAVVRHYTMVAIAQQAGPFPQKGYQATHGI
DPMILIDALASCMGHEEKELCKPGIACMGIILDTATNIMGNKDRACKLPIIQYLAEKMVSLCYDRPW
YSKVGGCQAIQFLCKHMSLRALFQNLFNFLKAFMFVLMDLEGDVSNGAIEITKSYMKSMLEICLTPI
NECYKNIDLKDLQAKATYEVIHELVRHITSPNTIVREESMVLLKHIGTIQSKTVSEVMDPHKDVLADII
PPKKHLLRHQPANAQIGLMDGNTFCTTLEPRLFTIDLTNTYHKLFFHELLTLSEAEDATLAKLDCYK
NVPNLIPLRTSALRALAACHYISDIGYKEKIINIIFKVMESDKSELQTTAFHCMKHFITGVTLEKEKVQ
SAMRPLLLKLGDHRNLSIPAIKRLSYFTQIFPQMFNEKLSEQILQHCSKIMEIFVSEYKSTSPNVNFF
ASSKGGEYEQKIVILIEMFFYISASVKYIEKLCQLVLKTEKNLMIEASSPYREALIKFLQRFPTETVDL
FLTESLMIDPQWNRLFIYLLKHETGVSFRAVIKSSRYNNLIHYLNTHTEFPEALKYEIQHQAVLIIFTL
MESDDQWIPTRQDIVDALKNCWQNYLSTLSSEDVLCDLWHLIGKILLHYFSNNTNDIELLFQLLRAL
CFRFIPDVYFLRDFLQHTVAQSFTVNWKRNAFFYFVENFNNSFLSEELKAKIITAVIIPCFAVSFDKG
EGNKLIGAPPTPYQEDEKNIVSVFINKVFDPDKQYDDAVRIALLQLACLLVERASQHIHDGDANNKR
QGNKLRRLMTFAWPCLLSKSSVDPTARYHGHLLLSHIIARLAIHKKIVLQVFHSLLKGHALEARSIVK
QALDVLTPAMPLRMEDGNTMLTHWTKKIIVEEGHAMQQLFHILQLIIRHYKVYFPVRHQLVQHLINY
MQRLGFPPTASIEHKKLAVDLAEVIIKWELHRIKDDRETKTDGTEEELIQESSVKRSGIDLVETRKKS
FDIIRETTVQGKCVMLLKMAMRPEIWPQPFDIKLNWLDKVLATVETPHHNLNNICTGIDFLTFLTTIL
SPDQLVSIIRPVQRGLSLCIIHQNTRIVRLMHMFLTRIMAIFPPDTQHKHEDLDLLYTAVSKMIAENLT
SYEKSPQPNASSLFGTLMILKACTTNNASYIDRILVQFIRVLNHLTRDHINTIGGNTVISQSPDSNAL
PLELLVLSLELIKNRIFVMSVEIRKLFIGTILVSLIEKSTEVKIIKCIIKMLDEWIKTKEPNVMTQVPSIRE
KSALLVKLMQNVEKKFTDEIELNIQFLEIINFIYRDEILKQTELTNKLEGAFLNGLRFQNPNVRSKFFE
ILDSSMRRRLHDRLLYIICSQAWDTIGSHYWIKQCIELLILTANTMMQIQCSNEQFKIPSITSVIPVNS
SETQENSFVSFLSSHSESFDIIQTVDDKDDVYDIDLNADRKEDCQQILPNRRVTLVELVYKQAEFLE
ANRNIRTDQMLVATSQLCHIDTQLAQSVWLSMFPRIWSIFTEDQRCNITKELIPFLSSGTNVNQKD
CHPSTLNTFVESLTKCAPPIYIPPNLLAYLGKSHNLWHRAILVLEDMAVNQSMQSKDIDGGENQFS
DLDVQQSNNIFDSLSKMYSSMHEEDLWAGLWLKFAHYPETNIAVSYEQMGFFEEAQGAYDLAMT
KFKQDLSNGVVNTYVNSELLLWENHWMRCAKELNQWDILLDYAQTNKDKNMFLILESSWRVPD
WNLMKIALAKTEQCYLKHYGFKINLYKGYLSILHQEERQTGNIERYVEIASSLCIREWRRLPNIVSHI
HLPYLQASQQIMELHEASQIHQGLAQSRNNSLHDMKAIVKTWRNRLPIISDDLSHWSDIFTWRQH
HYQIITQHLEQQSDQGSTMLGVHASAQAIISFGKIARKHNLTGVCQETLSRIYTIPSVPIVDCFQKIR
QQVKCYLQMPSTSGKNEINEALEVIESTNLKYFTGEMNAEFYALKGLLLAQIGRSEEAGKSFSVAA
QLHDGLTKAWAMWGDYMEQIFLKERKITLAVDALICYLQASRNQIESKTRKYIAKVLWFLSYDNNT
KILISTLEKHVAGIPPSYWLPWIPQLLCCLEQFEGDVILNLLSQIGRLYPQAVYFPIRTLYLTLKIEQR
EKHKTAEQAVKSSCSNIDGTTLSFGRGASHGNIPSINPIKATPPMWRCSKVMQLQREVHPTILSSL
EGIVDQMVWFRESWTEEVLRQLRQGLIKCYAIAFEKRDTVQHSTITPHTLHFVKKLGSTFGIGIENV
PGSVTSSISNSAASESLARRAQVTFQDPVFQKMKEQFTNDFDFSKPGAMKLHNLISKLKTWIKVLE
TKVKKLPTSFLIEDKCRFLSNFSQKTAEVELPGELLIPLSSHYYVRIARFMPRVEIVQKNNTAARRLY
IRGTNGKIYPYLVVLDSGLGDARREERVLQLKRMLNYYLEKQKETSRRFLNITVPRVVPISPQMRL
AEDNPNSISLLKIFKKCCQSMQVDYDMPIVKYYDRLSEVQARGTPTTHTLLREIFSEIQWTMVPKTL
LKHWALKTFLAATDFWHFRKMLTLQLALAFLCEHALNLTRLNADMMYLHQDSGLMNISYFKFDVN

DDKCQLNQHRPVPFRLTPNVGEFITHFGITGPLSAAIVATARCFIQPNYKLSSILQTILRDEIIALQKK
GFRECKLIEGSEDRYSDGNCMEHSVNIVNSAVDIIMTRFNKISYFDSIENKKISVLVQSATNIDNLCR
MDPAWHPWL (SEQ ID NO:62)

# Figure 26

**Name:** CG2336
**Nucleotide:** AE003674
**GI:** 10727121
**Transcript:** CT7778
**Sequence:**

gtaacggttaagtttaccttttaaatatttaaatctattaagtttgtaatatttccgattttaatgatgtacaacaaccgaccca
cggctatcagtctagaagaggaggttcaatatatctataagtacccttttgctgccgactccactgattgattatggatttatctacaatctggttt
gcaacaagcctcgggatgtcagtgtgccgttgatcctagaaattgagacagagttcaagtccaaagacagtgctcctgagaagccaaag
aaggtgcctaggctttcataccattcccgatcgagttcaatgagtgaagatgaaccggaaccggaagtgcaagttaaataccagtggaa
ctcgccgcggctgatgattctctgcgaagatggagacatcaattgcgctttgcactatctcgttgagtctcttcacgatcccttcgcctgcaatg
cggtggccactcttttcttgcaggaatccatattggaggagttcgtggatcgcataagggatcgcctggaaccgttgagcacggatatctcc
gggcatccggtttatataatgacactggagagaataggtcacttacaggcaaagagaatagttggaaatcccaagactgttccagaaaat
gctagtcccatgctggtctacgaccttagccatcgttacttggctgatggacccaccggggtcataaccctgcacacctttcgaaccatgaa
ggaggccgttgagctgcaggctaaggaaccacttaactttacctctgtgtgcatttggaacgaaaaactggcagccgcctacgaactggt
ggcccgattaagtccgctgatcttcacgattaattgttactacgttaatctgaacgaaatcactctgcccttcgtttgcaactttaactccgcaaa
gattatcgacggctaccactacgagtcattgacctttaagggaaagcgcaaagtggttgtccatccagttggcaccatctgggcaaagttg
gcccgcgaggcactcgtccagtactga (SEQ ID NO:63)

**Protein:** AAF54111
**Protein GI:** 7298905

MSEDEPEPEVQVKYQWNSPRLMILCEDGDINCALHYLVESLHDPFACNAVATLFLQESIL
EEFVDRIRDRLEPLSTDISGHPVYIMTLERIGHLQAKRIVGNPKTVPENASPMLVYDLSHRYLADGP
TGVITLHTFRTMKEAVELQAKEPLNFTSVCIWNEKLAAAYELVARLSPLIFTINCYYVNLNEITLPFV
CNFNSAKIIDGYHYESLTFKGKRKVVVHPVGTIWAKLAREALVQY (SEQ ID NO:64)

# Figure 27

**Name:** TER94
**Nucleotide:** AE003831
**GI:** 10727672
**Transcript:** CT7768
**Sequence:**

ctgcctcatctttgaatttgactaaaagtgccgatttccagctcaacgaatagtccaagtcgcgccgaattgtacaattaa
gttaaaaagttcaacgtcaactagtttatcatggcagattccaagggtgaagatctggctacggcgatcctgaagcgcaaggaccgtccc
aaccgactgattgtggaagaggcgcagaatgacgacaactctgtggtgtcgctatcgcaggcgaagatggatgagctgcagctcttccgt
ggcgacaccgtgattctgaagggcaagcgccgaaaggagacggtgtgcatcgtgctctcggacgacacctgtcccgacgagaagatc
cgcatgaaccgcgtggtgcgcaacaacctgtgcgtccatctttcagatgtggtttccgtgcagtcctgcccggacgtcaagtacggcaaac
gggtgcgcatcctgcccatcgacgaaagcaccgaaggcgttaccggcaacctgttcgagatctatctgaagccgtacttcctagaggcct
atcggccaatccacatgggcgacaactttattgtgcgcgccgccatgcgtcctattgagttcaaggtggtgctgaccgacccggagcccta
ctgcatcgtagctcccgagacggtaatcttctgcgacggtgatccaattaagcgcgaggaggaggaggagtccctaaacgccgttggct
acgatgatatcggtggttgccgcaagcagctggcccagatcaaggagatggttgagttgcctttgcggcatccatcgctcttcaaggccatt
ggtgtgaagccaccgcgtggtattcttatgtacggtcccccgggtaccggtaagaccctgattgccagggctgtggccaacgagaccgga
gcctttttcttcctgattaacggaccggagatcatgtccaaactggccggagagtcggaatcgaatctgcgcaaggccttcgaggaagcc
gagaaaaactcgccggctatcatcttcatcgatgagatcgacgctattgcccccaagcgtgacaagacccacggtgaggtggagcgtcg
cattgtgtcgcagcttttgaccctgatggatggcatgaagaagagctcccatctgatcgttatggctgctaccaacaggcccaactccattga
cccggccctgcgtcgctttggacgttttgatcgtgagattgacatcggcattccagatgccactggacggctggaggtgctgcgcattcaca
ccaagaacatgaagctgcatgatgatgttgacctggagcagattgctgccgaatcccacggacatgtgggcgctgatctggcttcactctg
ctctgaggctgcccttcagcaaatccgtgagaagatggacctcatcgatttggaagacgataagatcgatgccgaggtgctggcgtcgct
ggccgtgacaatggagaacttccgctacgctatgaccaagtccagtccatcggcgctgcgtgaaactgttgttgaggtgcccaacaccac
ctggacagacatcggaggtctggagagcgtcaagaaggaattgcaggagctggtgcaatacccagtggagcatccggacaagttcttg
aagtttggcatgcagcccagtcgcggtgtccttttctacggacccccctggttgcggtaagacgctgctggccaaggccattgccaacgagt
gccaggcgaacttcatctcagtcaagggtcccgaactgctgaccatgtggttcggcgagtctgaggccaacgtgcgcgacatcttcgata
aggcccgctcggcggctccttgtgtgctcttcttcgacgagctggactcgatcgccaaagcccgtggcggtaatgtcggcgatgctggcgg
cgccgctgatcgtgtgatcaatcagatccttaccgaaatggatggcatgggagccaagaagaatgtgttcatcatcggagccaccaatcg
cccggacattatcgatccggccatcctccgtccgggccgtctggatcagttgatctatattccgctgcccgacgacaaatcgcgtgaggcta
tcctgaaggctaacttgcgcaagtcgccgctggccaaggaagtagaccttacctacatcgccaaggtgacgcagggcttctctggcgcc
gatctgaccgagatctgccagcgggcttgcaagctggccatccgacaggccatcgaggctgaaattcgtcgcgagaaggagcgcgcc
gaaaatcagaactccgcgatggacatggacgaggacgatccagtgccggagattactagcgcccacttcgaggaggccatgaagttc
gctcgccgttcggtgtccgataatgacatcaggaagtacgagatgtttgcccagactttgcagcagtcccgtggattcggacagaacttca
gattccccggtcaaaccggcaacacctcaggatctggtaacaacttgccagttaactcgcccggcgacaacggcgacgatgatctttac
agttagatttctagttttacaccgcaaaacaaaaccacaaaatggaactttttaaatgaatttttccgtaaaaccgagcaagcaaaaccaa
tcacattcgcatcctggacgcggtaacttactacagcaaccattggctaagacatccagccgatggcgaaatcagatgacatacccgatt
caatattcacttctttgataccatccttactcttggtcgcggatacgtgcagtaggagaaggttcccatttggatttgggctcgcctaatccgaa
accattttaattggcgaactattccgcgcctcacactaatcattcgtgcattgtcggtagagtggctccgatatatgagtttctataaacataatt
attaacgatacacatacacacatacatttaaagataaatacatatattaaacaccgtaaaattataagtgtatttggagctacatttttaagtg
aat (SEQ ID NO:65)

**Protein:** AAF58863
**Protein GI:** 7303816

MADSKGEDLATAILKRKDRPNRLIVEEAQNDDNSVVSLSQAKMDELQLFRGDTVILKGKR
RKETVCIVLSDDTCPDEKIRMNRVVRNNLCVHLSDVVSVQSCPDVKYGKRVRILPIDESTEGVTGN
LFEIYLKPYFLEAYRPIHMGDNFIVRAAMRPIEFKVVLTDPEPYCIVAPETVIFCDGDPIKREEEEESL
NAVGYDDIGGCRKQLAQIKEMVELPLRHPSLFKAIGVKPPRGILMYGPPGTGKTLIARAVANETGA
FFFLINGPEIMSKLAGESESNLRKAFEEAEKNSPAIIFIDEIDAIAPKRDKTHGEVERRIVSQLLTLMD
GMKKSSHLIVMAATNRPNSIDPALRRFGRFDREIDIGIPDATGRLEVLRIHTKNMKLHDDVDLEQIA
AESHGHVGADLASLCSEAALQQIREKMDLIDLEDDKIDAEVLASLAVTMENFRYAMTKSSPSALRE
TVVEVPNTTWTDIGGLESVKKELQELVQYPVEHPDKFLKFGMQPSRGVLFYGPPGCGKTLLAKAI
ANECQANFISVKGPELLTMWFGESEANVRDIFDKARSAAPCVLFFDELDSIAKARGGNVGDAGGA
ADRVINQILTEMDGMGAKKNVFIIGATNRPDIIDPAILRPGRLDQLIYIPLPDDKSREAILKANLRKSPL
AKEVDLTYIAKVTQGFSGADLTEICQRACKLAIRQAIEAEIRREKERAENQNSAMDMDEDDPVPEIT

SAHFEEAMKFARRSVSDNDIRKYEMFAQTLQQSRGFGQNFRFPGQTGNTSGSGNNLPVNSPGD
NGDDDLYS (SEQ ID NO:66)

**Transcript:** CT7776
**Sequence:**
ccgatttccagctcaacgaatagtccaagtcgcgccgaattgtacaattaagttaaaaagttcttgaagtttggcatgca
gcccagtcgcggtgtccttttctacggaccccctggttgcggtaagacgctgctggccaaggccattgccaacgagtgccaggcgaacttc
atctcagtcaagggtcccgaactgctgaccatgtggttcggcgagtctgaggccaacgtgcgcgacatcttcgataaggcccgctcggcg
gctccttgtgtgctcttcttcgacgagctggactcgatcgccaaagcccgtggcggtaatgtcggcgatgctggcggcgccgctgatcgtgt
gatcaatcagatccttaccgaaatggatggcatgggagccaagaagaatgtgttcatcatcggagccaccaatcgcccggacattatcg
atccggccatcctccgtccgggccgtctggatcagttgatctatattccgctgcccgacgacaaatcgcgtgaggctatcctgaaggctaac
ttgcgcaagtcgccgctggccaaggaagtagaccttacctacatcgccaaggtgacgcagggcttctctggcgccgatctgaccgagat
ctgccagcgggcttgcaagctggccatccgacaggccatcgaggctgaaattcgtcgcgagaaggagcgcgccgaaaatcagaactc
cgcgatggacatggacgaggacgatccagtgccggagattactagcgcccacttcgaggaggccatgaagttcgctcgccgttcggtgt
ccgataatgacatcaggaagtacgagatgtttgcccagactttgcagcagtcccgtggattcggacagaacttcagattccccggtcaaa
ccggcaacacctcaggatctggtaacaacttgccagttaactcgcccggcgacaacggcgacgatgatctttacagttagatttctagtttta
caccgcaaaacaaaaccacaaaatggaactttttaaatgaatttttccgtaaaaccgagcaagcaaaaccaatcacattcgcatcctgg
acgcggtaacttactacagcaaccattggctaagacatccagccgatggcgaaatcagatgacatacccgattcaatattcacttctttgat
accatccttactcttggtcgcggatacgtgcagtaggagaaggttcccatttggatttgggctcgcctaatccgaaaccattttaattggcgaa
ctattccgcgcctcacactaatcattcgtgcattgtcggtagagtggctccgatatatgagtttctataaacataattattaacgatacacatac
acacatacatttaaagataaatacatatattaaacaccgtaaaattataagtgtatttggagctacatttttaagtgaat (SEQ ID
NO:67)

**Protein:** AAF58864
**Protein GI:** 7303817
MQPSRGVLFYGPPGCGKTLLAKAIANECQANFISVKGPELLTMWFGESEANVRDIFDKAR
SAAPCVLFFDELDSIAKARGGNVGDAGGAADRVINQILTEMDGMGAKKNVFIIGATNRPDIIDPAIL
RPGRLDQLIYIPLPDDKSREAILKANLRKSPLAKEVDLTYIAKVTQGFSGADLTEICQRACKLAIRQAI
EAEIRREKERAENQNSAMDMDEDDPVPEITSAHFEEAMKFARRSVSDNDIRKYEMFAQTLQQSR
GFGQNFRFPGQTGNTSGSGNNLPVNSPGDNGDDDLYS (SEQ ID NO:68)

# Figure 28

**Name:** CG6313
**Nucleotide:** AE003746
**GI:** 10726739
**Transcript:** CT19662
**Sequence:**

atggcctactctatttctccggcatctccacttccctcgcccactggcagcggcaactatgtcgatcagcagctgcaaca
gcagtccatggatgttgctctacagcgcaagacggccatggacgatttccgtggcatgttggagacggcggtaaatggtccaaggggca
gaaaagacctggctcttaacacaccacagctgaacttcttcaaggacggctggcatatggtgggagtgcacaatttctttggtgatcagcc
aaagtcgcccactgaaacaccgcctgaaatggaggagactaccatgtcctcaccgaccgaagcagatcgtctcggatcggagcctcg
ggccgagatgaagaacttggccacgctctgctcggccgcagcagcagctgctgctgtggcagcggttaacaaggatcaggttgagatta
gttcggatcttgagagcgaatgcgaggatgatgcagaagacgcggaggtggacaacgatggtgaagtagactacatcgacgaagac
gaaggtggtggagaaggcgaagaagaagaagatgatgcagatgatgacgagttcttcttggacgagcctgatagcgaccaaggaact
ggcaacaataataacaattccaaaagcggcgccagttcgttgccattgaaacagcgcaaaatggccactcggttggaaaacctaatcct
aaactcgcagacagtgtgcgacttcccgcctgaacttagcaactcggaactggttcatgtcctgccccaaataagcaatctcaaggcagc
ggccaacagcaacgcggctctgaacagcgtactccagcagcagttggcagcagcctccgcggcagcggcgcacgccaaagcgtctg
tagtccaccagaagcagcagcatggagagggagatcagcaatgcgaagatgacggcagtgccagcgcaagtgagctatattcgggtt
tggagcactttgccaatgatggcgaaatggaggatatattccaggaattggcaagtagcctaaactatcccgagcttgccgagtttagcctc
aatcagatgtgcaagggtcgatttgctggaaattgggcgcaatcttccggcaagtggactggtcaggagcagttggtgggtgtggtaaggt
cgccgggtctcattaacccaggcgatgttccgcaggatgcccagcgacaggcgaatcttgtcctcctcgactatcccatgcagcaaaaca
tccaactagagcagcggctactcgatgctgaggaaatgcacctgcagcaacaccagcaaacaccgctctccttactgccctttacggatg
agcagcagcagcagcttcatcaccaagctttgtccaatgcatccgatttcagcaacaccaacagcttgccctggaaaacgatccagaa
ctaaagcagcagcttcagcagaactccaacgcgcgcataattaaagctgttgctgcccagcatcagcagcagcctccaaccaacttcgtt
tacaacgtggaaagcggcgacaagaatgctccgccagtgcaattgctcttccagttgccaccacacatggcgcaacatcaggcgcagc
aacagcagggcgttggagagcctcttaccgaacagcaacagcagcagttacacgctgagcaggcgcatctctttcagcatcgaactgg
cggtcagcgtccgcccacccagagtgagttagagcaggtggctcaagagctattgcttcagcgaagcggccaggtgccggcaggagct
cctgttgttggtgttcaggcaattccactcaagcaaaaacactttaacctgcatccgccgccgtgtccacccacctgtgtccagcatcaggtg
gccacgcagacgcatcctgcttctgttgtagtgccgcagcctgctgtaggatatacacaattcgctcttcaggcctctcagcaacagcaaat
gcaacaaaacgagctctccatttggccgatggccacgcctactcccgcgcccagcagcggtgtaagctcgaccaagtcgatgcccggc
ggcattgccaaaaaggccattgacaagcagtcgcgcaaggaacgtcgttgcgtggtgcgccagacaccagcaggcattcaagagaa
caccaaactccatctacagcctcaggtcgcaacagcccagcaacaatttctagtgcagaaccagttagcagttgcgaccaccgtgagttt
ggacaagactatcgaaatagattcagagacggaatccaaccacgacacggcgctaaccttggcttgtgccggcggtcatgaagagctg
gtggaactgttgatcaataggggagcaaacatcgagcaccgtgataagaagggattcacaccgcttatactagccgctaccgctggcca
cgacaaagtcgtggacattctgctcaagcacagcgctgagttggaggctcaatcagagcgtacgaaggatacaccgttgtccctggcat
gttctggcggccgatacgaggtggtggaacttctgcttagcgttggtgccaacaaggagcaccgcaatgtatctgattacactccactgag
cttggcagccagtggggctatgtgaacatcattaaactgttgcttagccatggagcagagatcaattcgcgaacgggcagcaaactgg
gcatttcaccgcttatgctagccgctatgaatggtcatacgccggcggttaagttgcttttagatcagggatcggacataaatgcccagatcg
agacgaatcgcaatacggccttgactttggcttgcttccagggcagacacgaggtcgtaagtctgctgcttgaccgacgggccaatgtgg
agcatcgagctaagacgggcctgaccccactcatggaagccgcgtcaggcggttacatagaggttggtcgcgttctgctggacaagggt
gcggatgtgaatgctgctccggtgccgacgtccagggatacggctctaacaattgccgccgacaagggccatcaaaagttcgtggagct
cctgctatctcgtaatgccagcgtagaggtaaaaaataagaagggtaactccccactctggctggctgcccatggtggtcacctgagtgtg
gttgagcttttgtacgaccataatgctgacattgactcacaggataatcgacgtgtttcctgtctgatggctgccttccgcaaggggcacacc
aagattgtgaagtggatggtgcagtatgtgtcccagtttccctctgaccaggagatgattcgcttcattggtaccataagtgacaaggagctg
atagacaagtgttttgactgcatgaagatcctgcgtagcgccaaagaggcccaggccgtcaaggccaataagaatgcttcgatccttttgg
aggagctggatttggagcggactcgcgaagagagccgcaaagctgccgccgcccgtcgtcgtgagcgcaagaagaagaagaagat
ggagaagaaagaggagaagcgccgtcaacaacagggcaatgggcctggcggagacgatatgcaaggcgatgacgatgacgcca
gtgacaaggatgatgattccgacaaggacgatgaagacgaggaggcagcgccggccgccgcccgcgaggagggagactctggcat
cgatcagggctcgtgctccagcggagacaccaaaggtgcgcgcttcggtggcagtcagtccgctcaggctgcggaagcggcagccaa
ttccgtgtccaccaacagtcagggaaagaagaacaagaagcaggcgaaaaacaaggtgttgatatcggtggaaccaactcaaccag
taatcacatcgaactccgtcctcaagggcgtctgtgcgaagaagcatcccgcagtggaagttgtaaagcaacctcctgccacacaacag
gctgctcctcttaagcggcaactcgatgtaaagaaggaggaacctgcgctcaaaaagaaggaagagaaaaacagctcgtccagcag
cagcagcaagcgtgagaaagagaatcttgcgcccaaggaggttgcactgccagccaagcagcagcccagtagctccagcaaactgc
agagcagcgagtctgcgagcaacataaacagcagcaccgctaccaacaccagtagcgccaatactactcggaaggaagttgcaaa
gccagcgtcacaaactgcgagtgccaccactttgaatcctgcaaagcgcaccgaagtcgatggctggaaggaagtagtccgcaaaag
tagcgcccagcagaccacagcggtgggagcgagtggagccccactgcctgtcacagccaccagttcggccaccagtgtgcaacatca

tccgcaccaccacctagccaacagctccagcaacagctcaagctccctgaccaccagcactactacagcagcgtcttcggttcccgag
atgacgtgcaagaaggtgcaagtgcccgtaaatgccatctccagggttattggacgaggtggaagcaacattaacgccattcgggccac
cactggtgctcacatcgaggtggagaagcagggcaagaaccaatcagagcgttgcatcacgatcaagggcttaaccgatgctacaaa
acaggcacatatgctcattttggcactaatcaaggatcccgatgtggacatattgcaaatgctgcccaggattaacagcagtattaagcag
gcgtctagtggcggagcaagcaccccgatgtccgtgggaacttgggacaatcgcaccgctgccggtgtgaatgcgtataccttttcttctgc
cgcgtccaccacctccacatcctccagctcgtcagctagctctactacaccagcgggagcttcgtacagcaatgcgcacaagcagcacc
aacagcagccgcagtcagtgaagggcccaagtggacggtcatcaacgtcggtcaagtctaatggcagtagcaccaaggtatcggcttc
gagtggatcgggttcccggagcggcagggctggcagtagctatcttgcccaacagcagcctggtcgcagctctggaggtggctcttcaa
atggcgtgatcaagagcaagtcagaaagctcttccaaatccctgccagctgcacaaaagagcagtaccactttgggtaaatcatcgact
gtgtcaccgggtgcacagaatttcgcaaaggcagcggctattggacagtcctcgcccaaaaaggctgagggtggtgctacatctgcggt
ggtcacctctgccggtggacgcagcagtggcgtggtggctccatttggacgtggcaagcctgtagctggccaaggaggacctgcagca
acggcggcttccaacgttgcccagctgggaagtgtgagtggcaacagcaacatattggctggaccaattggcacctttaatgtagcggat
gtggctgctgtgaatgcagccgcggcagcaggagcagcagcagctaccaacagcaatgtgaaacccattgctcccattgcaccgccc
agtaagcgagttggatctcccacccaagtccagcaacagcatcaaacacagcagcagcaacaacagcaactaccccagcccgcac
cagttcctggcccacagccacaacaacagccgcttcagcagcagcaacaacaacaagctcctcagcagcagccacaacagccaaa
ccagcaacagcaaccacagacgtcccagcaaaatctcgtgatcaatacaaacctactgaacgatctgatggccgccagtgcagcaaa
caccaccagcgatagcttcagtgcccagctagcagccaagttgtctagcgcatattccctgttcagtgactaccagcagtcgcagtgggg
caagttgggtgatccaggcatcggcggtggagcaggagctgttggcgatggtctgccgcaagcggatgcttccaaggcaccaggatac
aatcgcaacatccttagctcgcccgttggcagttccaaggcctcgtcgaatcactccacctcgcctcctgtgggtaatgtgatccaacagca
gcagcagcagcaaccgcaatctagccaacaagctctcaacattatcaccagtggaccaggagggcctgctacagcaccagccagatc
accgatggtgtccgccaacgagggcaatcccgctgtgggtcaaccctccatgaatggaacccaaggattgggtgagacggctcctgctc
attcaccaggcgttatcaaaccgcccacggccacagttcccatccagcgtcatgtgcccatgccgatctctgcgccggaggccggagca
ccgcccacatttggagcgattggttccaacccagctagcggtaacaattctgcggctgcccaagccgcagctgccgccgccgcctcggc
gatgatcgatcgtcagcagcagaatttgcagaatctgcagactttgcaaaatttgcaaaggatggtgggagcctcgcagcagcagcagc
cacaacagcagctgaactatccaatggaccccacatcgtcgttcatcgtggatgctaataacgtactgcgcctgaatccacgcgtcatcttt
ccgcaaggcaacaccaagccaccgcagccaccgccgcagggtggaacgcagtcgaatgtgtttggaggaaatccaggcagacaac
cacctggaacgggtgccagacagccaggaggagcagctgcacagcgttggtatggcggcactctggagtatccttcatacacgggccg
tgatatgctgcacctggagaatggtgccggcggaatggcgggtatgggctcaccatctgccatgtcgcccaaccacgacgacattcgca
agatgccgcgccccataggcactgagcgagccgcatcatggaagtacaacaactttaacgttggaggcccgtcacttaacatggaaga
tgctctagccagtgtgttgccccccatgggcacatgagcttaaggctcagcctccaggtttgcagcagccgcctcctccgccgcagtcgcag
cagcaacagcaacaaccgctcaactggctaaagcagcagccgcagcagcagcagtacagggcctacaacaacggaccctatccgc
agcagcagcagcagcatgagccaatgaatatgcccatggactaccacaacatgcaggcacctccaaatatgagccagcagcagcag
cagcacgtcaacttgatgccctcctacggctaccagcattttgtgggcgctcctggagccgttgacatctccgcacatatgccggacaagat
ggaggtgtgggatcaccacgataaacacatgccctggaccaactataccaccaactggtccaactga (SEQ ID NO:69)

**Protein:** AAF56267
**Protein GI:** 7301133

MEAASAGHLDIVKLLLNHNADVNAHCATGNTPLMFACAGGQVDVVKVLLKHGANVEEQN
ENGHTPLMEAASAGHVEVAKVLLEHGAGINTHSNEFKESALTLACYKGHLDMVRFLLQAGADQE
HKTDEMHTALMEASMDGHVEVARLLLDSGAQVNMPTDSFESPLTLAACGGHVELATLLIERGANI
EEVNDEGYTPLMEAAREGHEEMVALLLSKGANINATTEETQETALTLACCGGFMEVAAFLIKEGA
NLELGASTPLMEASQEGHTDLVSFLLKKKANVHAETQTGDTALTHACENGHTDAAGVLLSYGAEL
EHESEGGRTPLMKACRAGHLCTVKFLIQKGANVNKQTTSNDHTALSLACAGGHQSVVELLLKNN
ADPFHKLKDNSTMLIEASKGGHTRVVELLFRYPNISPTENAASANVTQAAPTSNQPGPNQMRQKI
MKQQLQHQLQQLNAPPGLHELSEAARASNQQHFHQQQFSSAGNGSSNIVAMGTGDFLDAGELQ
LTATAGMSAGAGTSTTGSETGMEEYGEVGGIDLTTLGAQQQEGLIAKSRLFHLQQQQQQQQQ
QQQQQQQQQQQQQQQQQQQQPPAAGQHQLVPCKHFDLDMEHINSLQPPQKAPPAPPVLFHT
VCQQPVMQQQQQQLQPGQLKLKAMLPNRNRALKTAEVVEFIDCPVDQQQPGEQVRTQPLGED
GKTPQFACAGEDPRLQRRRGFMPELKKGELPPESSSSDPNELALKAGGTVDQ (SEQ ID NO:70)

# Figure 29

**Name:** aur
**Nucleotide:** AE003693
**GI:** 10726473
**Transcript:** CT10220
**Sequence:**

agtcgtgaatgttttacccaatatcggctttcattgttaactgaccaaaattgtaatctgttctgttagttgtcgagtgcctgtg
ccgtcatgtcccatccgtctgaccatgtgctgcgtcccaaggaaaatgctccgcacagaatgccagaaaaatcagctgcagtgcacaat
atgcaaaagaacttgctattgggaaagaagcccaacagcgagaatatggctccagctagcaaacccttgccaggatcctctggcgcttt
gatcaggaagccaggcctaggaggctccaattccatagcttcctccgaaggaaacaacttccaaaaacccatggtgccgtccgtgaag
aagaccacatcagagtttgctgctccggctccagtagcacctatcaagaagcctgagtcactttccaagcagaaacccactgctgcgag
ctctgaatcctccaaggaactgggtgcggccagcagctccgctgaaaaagagaaaaccaagactgaaacccagccccagaagccg
aagaagacctgggagctcaacaactttgatattggtcgcctgctgggacggggcaagtttggcaacgtttatttggcgcgggaaaaggaa
tcccagttcgtggtggccctaaaagtgctctttaagcgccagattggcgagtccaatgtggagcaccaggtgcgtcgtgagatcgagatac
aatcgcacctacgtcatccgcatatcctgcgtctgtatgcctactttcacgacgacgtgcgcatatatctgatcttggagtatgcgccacaag
gaacgcttttcaacgccttgcaggcacagccgatgaaacgtttcgatgaacgccagtcggccacctatattcaggcgttgtgctcggcgct
gctctatctgcatgagcgggacatcatacacagggacatcaagccggagaacttgctgctcgggcacaagggcgtcctgaagatcgcc
gacttcggttggtccgtgcacgagccgaactccatgcgcatgacactgtgcggcactgtcgactacttgccacccgaaatggttcagggc
aagccgcacacgaaaaacgtggatctatggagcctgggtgtgctctgctttgagctgctagtgggccacgctcccttctactcgaagaact
atgacgagacctacaagaagattctcaaggtggactacaaactgccggagcacatttccaaggcagcatcccatctcatttccaagctgc
tggtccttaatccgcagcaccgcctaccgctggatcaggtgatggtgcatccttggatcctggcgcacacgcagtga (SEQ ID NO:71)

**Protein:** AAF54723
**Protein GI:** 7299536

MSHPSDHVLRPKENAPHRMPEKSAAVHNMQKNLLLGKKPNSENMAPASKPLPGSSGALI
RKPGLGGSNSIASSEGNNFQKPMVPSVKKTTSEFAAPAPVAPIKKPESLSKQKPTAASSESSKEL
GAASSSAEKEKTKTETQPQKPKKTWELNNFDIGRLLGRGKFGNVYLAREKESQFVVALKVLFKRQ
IGESNVEHQVRREIEIQSHLRHPHILRLYAYFHDDVRIYLILEYAPQGTLFNALQAQPMKRFDERQS
ATYIQALCSALLYLHERDIIHRDIKPENLLLGHKGVLKIADFGWSVHEPNSMRMTLCGTVDYLPPEM
VQGKPHTKNVDLWSLGVLCFELLVGHAPFYSKNYDETYKKILKVDYKLPEHISKAASHLISKLLVLN
PQHRLPLDQVMVHPWILAHTQ (SEQ ID NO:72)

# Figure 30

**Name:** Pk91C
**Nucleotide:** AE003723
**GI:** 10799498
**Transcript:** CL90028
**Sequence:**

atggaaaacgccgatgcgacttcgcaatcggatgtccacatcgactacaaaacccccaagaagacccactcgctg
atcgacagtgagcagttgctggacaagatcaacatccttaccaccaagccggagaatcactcccagaatgccaagcttcccactatcaa
ggatttgtcatcattaagccaatcagccgcggcgcctttggaaaagtcttcctgggctacaagaacaacgactcgaagcggctgttcgcc
atcaaggtgatgcgcaagtcagagatgatcaacaagaacatggtgtcccaggtaatcacggagcggaatgccctggccctctcccgctc
tcagttctgtgtcagtctcttctactccctgcaatcgctctcctacgtctacctggtgatggagtacatggtgggcggggatctcaagtcactgct
ggccatgtttgggtattttgacgagcccacggcccggttctacgtggccgagatggtgatggcgctgcagtatctgcatcagcacggaattg
tccacagagatattaaaccggacaacatgttgctctcctccagtggccatgtgaagcttactgattttgggttgagcaaaatcgatatgcgaa
gagatctggaaatatcagaccttataaactgctcaccgaatttaaacgcccgcacgccgggccagcttttgtcgcttacttcgcacctgtcttt
tgggtcagagaagaaactgaatgacttcggttctgtttcaagtggacaaaacaacggaatgggctcggtggccacggggacatcccact
tgctgcaggccatcaacaagcatagtctgataatggagctgtcggatagcgagggcgacacctcgctcaatgatgcagagaagacgag
cgatagcaagatatctggtgtttccccctttcttttctgccgaggaggccaatgaatccattactcatacctgcactacaaacgtaaatcccca
ggatagcagctcgtcttgttcgttccacacttgtaactcggccgacctgagcaagtgctcaccaccactggagtcaaaggacggtgctgct
gcgggcaatgcgatccccagcaagcggcgcgtggaattcgtcttggatgcggcaccctgtcagggctgcaagctggccgagcaagac
agcagcaacatggccaccaatgatggcaaacatttgcccaagatagataacgcaatcgaggcttcgtttgagttttccatggtgcgcagg
cgatcggtcgacgagcgaaatcgcatctcaaagggggccggaggattcaggggtgtccagtcgcaagggcgacgactattccagctgc
cacttgaatctgaatagcgagagcacggcttcgtcgattgagaagaatgtggacaacctaagccaatccaaggaggattttagctgttca
gattactcgcgcagttataacgtgaccaacggcaacgagatgagtggcatcaacatgaactcgccgtttcggaatctgtccaagcacttta
aacgccccgacttcctgcgcggcatgaagcgaaagatcaatttggtcaatcgctccgacaacatgtccagcatggataccgatggctgc
agttcaagcaatggaagtaccaacaccggactaacacaggaaattgaaatccttaatattggcagcagcacgcccaagaagcgcaag
gcccgttcctcgcccatccgtggtgtgctaaaggtgcgctctttatccgatgacgaaatgcccataaatcatttgcttggtccggaagcaaat
gtggccaacgtggtcttctctacgcccgtatcttcacagaagttgccacgtcgggatggtggtcttcttggaaagctcaaggccactcggttc
gctcttccgctgtccattgagaacaagaaacgggagcatgcgaccgccgataagatgtcaggcattcagtaccatctgaaactgtccgac
gatcccacgatgtcgcctatcaatcatggagccggcaatctacccaaagacgccgaaaaacgttaacatcaataccccgttccgcacacc
gaaatcagtgcgtcgcggggctcgcgtgtctaatgagcgcatcttgggcacacctgattatctggcgccggagttactgctgaagcaaggt
cacggtccggccgtcgactggtgggctctgggcgtctgtttctacgagttcatgaccggcattccaccctttaacgacgagacacctcaga
aagtatttgacaacattctgaataagaacatcgaatggccagaaggtgatgaggcattatccgtcgaatccatggaggctgtggaactgct
cctgaccatggatcccaatgagcgaccggccgcaaaggaagtgcaacaaatgcgtcactttgcttgcatcgactgggagaatataggc
aataccgagcccccatttgtcccgacaccagacaatcccactgatacgggatatttcgatgcgcgcaacaatcttcagcacctgcagctgt
ccaattttgccctagaagaatga (SEQ ID NO:73)

**Protein:** AAF55594
**Protein GI:** 7300437

MENADATSQSDVHIDYKTPKKTHSLIDSEQLLDKINILTTKPENHSQNAKLPTIKDFVIIKPIS
RGAFGKVFLGYKNNDSKRLFAIKVMRKSEMINKNMVSQVITERNALALSRSQFCVSLFYSLQSLSY
VYLVMEYMVGGDLKSLLAMFGYFDEPTARFYVAEMVMALQYLHQHGIVHRDIKPDNMLLSSSGH
VKLTDFGLSKIDMRRDLEISDLINCSPNLNARTPGQLLSLTSHLSFGSEKKLNDFGSVSSGQNNGM
GSVATGTSHLLQAINKHSLIMELSDSEGDTSLNDAEKTSDSKISGVSPFFSAEEANESITHTCTTNV
NPQDSSSSCSFHTCNSADLSKCSPPLESKDGAAAGNAIPSKRRVEFVLDAAPCQGCKLAEQDSS
NMATNDGKHLPKIDNAIEASFEFSMVRRRSVDERNRISKGPEDSGVSSRKGDDYSSCHLNLNSES
TASSIEKNVDNLSQSKEDFSCSDYSRSYNVTNGNEMSGINMNSPFRNLSKHFKRPDFLRGMKRKI
NLVNRSDNMSSMDTDGCSSSNGSTNTGLTQEIEILNIGSSTPKKRKARSSPIRGVLKVRSLSDDE
MPINHLLGPEANVANVVFSTPVSSQKLPRRDGGLLGKLKATRFALPLSIENKKREHATADKMSGIQ
YHLKLSDDPTMSPINHGAGNLPKTPKNVNINTPFRTPKSVRRGARVSNERILGTPDYLAPELLLKQ
GHGPAVDWWALGVCFYEFMTGIPPFNDETPQKVFDNILNKNIEWPEGDEALSVESMEAVELLLTM
DPNERPAAKEVQQMRHFACIDWENIGNTEPPFVPTPDNPTDTGYFDARNNLQHLQLSNFALEE
(SEQ ID NO:74)

# Figure 31

**Name:** Top2
**Nucleotide:** AE003663
**GI:** 10728874
**Transcript:** CT28703
**Sequence:**

cttgccagctctaaaaccagcaatacttctcatttgttgtcggttgtgaaaagttgttaaatgtgctgtgcgtattttaatagtt
agttgagaactgtacaagttttagctaaaggcagcagacgcgtggggcacgtacaagtcgaaaattgtagtgcaccgcctcgtgtatcgc
ctgcaacatagagttttgccgcacttcggttgtcggcggcagcaagaaaaggccacaaatacttggcaatttttaaccagatggagaacg
gaaacaaggccctgtccatcgaacagatgtaccagaagaagtcgcagctggagcacatcctgctgcgacccgactcgtacatcggatc
cgtggagttcaccaaggagctgatgtgggtgtatgacaactcccaaaaccgcatggtgcagaaggagatctctttcgtgcccggcctcta
caagatattcgacgagattcttgtgaatgcggcagataacaagcagcgagacaagagcatgaacaccattaagatcgacatcgatccg
gagcgcaatatggtgtccgtgtggaacaacggccagggcattccggtgaccatgcacaaggagcagaagatgtacgttccaacgatga
tctttggtcatctgctgacctcgtcgaactacaacgacgatgagaagaaggtcactggcggcaggaacggatacggagcgaagctctgc
aacatattctccaccagcttcaccgttgagactgccacgagggaatacaagaaaagtttcaagcagacttggggaaacaacatggaa
aagcctccgatgtgcagatcaaggacttcaatggcactgactacacacgcatcacattcagtcccgatctggccaagttcaagatggacc
gtctcgatgaagatattgtggctctaatgtcgcgtcgcgcctacgatgtggccgcctcatccaagggagtatccgtcttcttaaacggcaaca
aactgggtatagtccacgaggttgccaacgagcgttgggaggtggcttgctgtccttcagatcgaggcttccaacaggtctcgtttgtcaact
cgatagctacctacaagggcggtcggcatgtggaccatgtggtagacaatctcattaagcagctgctcgaggttctgaagaaaaagaac
aaaggtggcatcaacatcaagccattccaggtccgaaaccatctgtgggtttttgtcaactgcttgatagagaaccccactttcgactcgca
gaccaaggagaacatgactctgcaacaaaagggcttcggttccaagtgtaccctctcggaaaagttcatcaacaacatgtccaagtctg
gcatcgtggagtctgtgctggcatgggccaagttcaaggcccaaaatgacattgccaagacgggcggtcgcaagtcaagcaagatcaa
gggcattcccaagctggaggacgctaacgaggcgggtggaaagaactcgattaaatgcaccctcatcctcaccgagggagactcagc
caagtcattggccgtatccggtttgggcgtgatcggacgagatctctacggcgtgttcccgcttaggggtaaacttctaaatgtgcgggaag
ctaatttcaagcagctttcggagaatgccgaaatcaacaatttatgcaagataattggcttgcaatacaaaaagaagtacctcactgagga
cgatctaaaaactctgcgctatggcaaagtgatgatcatgacagatcaggatcaggatggctcccacatcaagggtctcttgatcaacttta
tccacaccaattggccagagcttctgcgtctgcccttcctcgaggagttcattacaccaattgtgaaggcaaccaagaaaaacgaggagc
tgtcattctactcgctacccgaattcgaggagtggaaaaatgatacagctaatcaccatacgtacaatataaagtactataagggtttgggt
acttcgacctccaaggaggcgaaagagtattttcaagacatggatcgccatcgcatcttatttaagtacgatggatcggtggatgatgaga
gcattgttatggccttctccaagaagcacatcgagtcgcgaaaggtatggcttaccaaccacatggacgaggtgaagcgtcgcaaggag
ctcggtttgccagagcgatatctctacaccaagggcaccaagagcatcacctatgcagactttatcaatctggagttggtgctgttctcgaat
gctgacaatgagcgctccattcctagtctggtggatggcttgaagccgggtcagcggaaggtgatgttcacttgcttcaagaggaatgaca
agcgtgaggtgaaggtggcccagctatccggatcagtggcggagatgtcagcctatcaccacggagaggtatccctacagatgacaat
cgtgaatctggctcagaattttgtgggtgccaacaacattaatctgcttgagccacgcggtcaatttggtacccgcttgtcgggaggcaaag
attgcgccagcgctcgttacattttcactataatgtctcccttgacgcgactcatctaccatcctttggacgatccacttttggattaccaggtgg
acgatggccaaaagatcgagccactatggtatctgcccatcataccgatggtattggtaaacggagccgagggcataggaactggatg
gtccacgaagatatccaactacaatcctcgtgagattatgaaaaatctaaggaagatgataaacggacaagagccaagtgtgatgcatc
cgtggtacaagaactttttaggacgcatggagtatgtttcggatggtcgttatattcagactggtaacattcaaattttgtccggaaaccgtttag
aaatcagtgaactccctgtgggcgtatggacgcaaaactacaaggaaaatgtcctggagcctttatcaaacggcaccgaaaaggttaa
gggtattatttccgagtacagggagtatcatacagacaccaccgttcgctttgtgatcagtttcgcacctggagaatttgagcgcattcatgca
gaagaaggtggtttctaccgagtgttcaaacttaccacgacgctgtccaccaaccagatgcatgcgttcgaccagaacaactgtctgcga
cgcttccccaccgcgatcgatatccttaaagagtattacaaactgcgacgagagtactacgcccgccggagggactttttggtcggccag
ctcacggcgcaggctgatcgcctcagtgatcaggcgcgctttattctcgaaaagtgtgagaagaagctggtggtggagaacaagcagcg
caaggccatgtgtgatgagttgttgaagcgtggatatcgtcccgatcccgtcaaggagtggcagcgccgcattaaaatggaggatgccg
agcaagctgacgaagaggacgaagaagaagaggaggcagctcccagtgtcagctcaaaggctaagaaggaaaaggaagtcgatc
cggaaaaggcctttaagaaactaactgatgtcaaaaagtttgactaccttctgggtatgtccatgtggatgttgacagaggagaagaaaa
acgagctactcaaacagcgcgacactaaactgtccgagttggaaagtctgcgcaagaagacgccggagatgctttggctggatgatttg
gatgccctcgaatcgaagttaaatgaagtagaggagaaggagcgcgcagaagagcaggggatcaatctcaagaccgcgaaggcttt
gaagggccagaagtcggcctcagctaagggcagaaaggttaagtcaatgggaggcggagcgggtgccggagacgtattcccagatc
ccgatggagaacccgtcgagtttaagatcaccgaagaaatcattaagaaaatggcagcggctgccaaggtagctcaggcggctaagg
agccgaagaaacccaaagagcccaaggagccaaaagtgaagaaggaaccgaagggcaagcagattaaagccgaacctgatgcc
agcggcgatgaggtggacgaatttgatgcaatggtcgagggcggctctaagacctcacccaaggccaagaaggcggttgtcaagaag
gagccgggagaaaaaaagccgcgccaaaagaaggagaacggcggcgggctcaagcagagcaagattgacttcagcaaggcca
aggcgaaaaagagcgacgatgacgtggaggaagtaactcctcgtgcagagcgtcctggacgtcgacaggccagtaagaagataga
ctacagctcgctcttctcggatgaggaggaagacggtggcaacgttggctctgatgatgatggcaatgccagcgatgatgattcgcccaa

gcgtccagctaagcgtggacgagaggatgagagcagtggaggagccaaaaagaaagctccgcccaagaagaggcgagctgtaatt gaaagcgatgatgatgatatcgagattgatgaagatgatgatgatgattctgattttaattgttaa (SEQ ID NO:75)

**Protein:** AAF53802
**Protein GI:** 7298585
MENGNKALSIEQMYQKKSQLEHILLRPDSYIGSVEFTKELMWVYDNSQNRMVQKEISFVP GLYKIFDEILVNAADNKQRDKSMNTIKIDIDPERNMVSVWNNGQGIPVTMHKEQKMYVPTMIFGHL LTSSNYNDDEKKVTGGRNGYGAKLCNIFSTSFTVETATREYKKSFKQTWGNNMGKASDVQIKDF NGTDYTRITFSPDLAKFKMDRLDEDIVALMSRRAYDVAASSKGVSVFLNGNKLGVRNFKDYIDLHI KNTDDDSGPPIKIVHEVANERWEVACCPSDRGFQQVSFVNSIATYKGGRHVDHVVDNLIKQLLEV LKKKNKGGINIKPFQVRNHLWVFVNCLIENPTFDSQTKENMTLQQKGFGSKCTLSEKFINNMSKS GIVESVLAWAKFKAQNDIAKTGGRKSSKIKGIPKLEDANEAGGKNSIKCTLILTEGDSAKSLAVSGL GVIGRDLYGVFPLRGKLLNVREANFKQLSENAEINNLCKIIGLQYKKKYLTEDDLKTLRYGKVMIMT DQDQDGSHIKGLLINFIHTNWPELLRLPFLEEFITPIVKATKKNEELSFYSLPEFEEWKNDTANHHT YNIKYYKGLGTSTSKEAKEYFQDMDRHRILFKYDGSVDDESIVMAFSKKHIESRKVWLTNHMDEV KRRKELGLPERYLYTKGTKSITYADFINLELVLFSNADNERSIPSLVDGLKPGQRKVMFTCFKRND KREVKVAQLSGSVAEMSAYHHGEVSLQMTIVNLAQNFVGANNINLLEPRGQFGTRLSGGKDCAS ARYIFTIMSPLTRLIYHPLDDPLLDYQVDDGQKIEPLWYLPIIPMVLVNGAEGIGTGWSTKISNYNPR EIMKNLRKMINGQEPSVMHPWYKNFLGRMEYVSDGRYIQTGNIQILSGNRLEISELPVGVWTQNY KENVLEPLSNGTEKVKGIISEYREYHTDTTVRFVISFAPGEFERIHAEEGGFYRVFKLTTTLSTNQM HAFDQNNCLRRFPTAIDILKEYYKLRREYYARRRDFLVGQLTAQADRLSDQARFILEKCEKKLVVE NKQRKAMCDELLKRGYRPDPVKEWQRRIKMEDAEQADEEDEEEEEAAPSVSSKAKKEKEVDPE KAFKKLTDVKKFDYLLGMSMWMLTEEKKNELLKQRDTKLSELESLRKKTPEMLWLDDLDALESKL NEVEEKERAEEQGINLKTAKALKGQKSASAKGRKVKSMGGGAGAGDVFPDPDGEPVEFKITEEII KKMAAAAKVAQAAKEPKKPKEPKEPKVKKEPKGKQIKAEPDASGDEVDEFDAMVEGGSKTSPKA KKAVVKKEPGEKKPRQKKENGGGLKQSKIDFSKAKAKKSDDDVEEVTPRAERPGRRQASKKIDY SSLFSDEEEDGGNVGSDDDGNASDDDSPKRPAKRGREDESSGGAKKKAPPKKRRAVIESDDDDI EIDEDDDDDSDFNC (SEQ ID NO:76)

# Figure 32

**Name:** alpha-Est1
**Nucleotide:** AE003671
**GI:** 10727101
**Transcript:** CT1044
**Sequence:**

attgttcggttctcattgcgcgattcagttgttcctcgtcgatcgcggcccgcggaagtgttcatttcactatcgtgccccgc
cggattgtgccgcccattctgacccttgagtcttctgctcacatagttagctgtgaatataattggagtcgcgagcggataaatggagatccg
agtgggagtgggcgatctgctgaaaatgggcaccaagctcattggccacaagatcgtccagtatcgccttggcacaaagcagacgaag
gtggtctgcaccagggatggccaggtgcgcggacatcgccgaaggacactctacgacgaggagatgtacttcgccttcgagggaatcc
cctttgcacagccgccggtgggggagctgcgcttccgagccccccagccaccacatccctggttgggggtgcgggattgcacctatccg
cgggccaagccgatgcaaaagcacttcgtgctcagcatcgtcgaaggcagcgaggattgcctgtacctgaacgtatattccaagcgcct
gagatcggacaagccgctgcccgtgatcgtgtggatctatggcggtggattccagttcggcgaggctggtcgagatttctacagtccagac
tactttatgcagcaagacgtagtggttgtcacattcaattatagggtgggcgcattgggcttcctcagcctcgcggatcgcgacttggatgtgc
ccggaaatgctggtctcaaggaccaagtgatggcccttcgctggatcagtcagaacatagcccaattcaatggagatccccaaaatata
accgtgatgggcgagagtgcgggagcagcctccgttcacgcgttgatgaccaccgagcaaacccgagggctgttccacaaggccatc
atgcaatcgggatccatgttctgcgagtgggccaatgagccaagtggcaggtgggcataccgactggcctgccaattgggatactcggg
cagtgagaacgagaaggaagtgtttcgataccttcagaaagcacccgcctccgaaatggccgcacagggtattactcttgtctcccaag
aggaacggcggcagtacgtcctgtttccctttactcccgttgtggaaccatatatcaccagggattgcgttttgccccgttgccacagggaga
tgctgccggaagcttggggtaatgacctgccgctgatcctgggcggcaactcctttgaaggtctgttctcctaccagagtaccctgcacgac
gaggaacacatgctgagtgcttttgaggtcctaattcctcgggagatcagggagaagagtacccagtcgcatcttaaggatctgctacgtc
aatttaaggtggataacttcgatgatgcgactcgagggcgaatggagttcaatgagtgcctgcacatactgtccgttaaacacttttggcatg
gcattcatcgcactgtattggcccggctcagccacgcccccgcaacgcccacctacctgtaccgcttcgatgtggactcgccccatttcaat
cacttccggcaggtgatgtgcggaaaacacgttcgcggagttagtcatgccgacgatctctcctaccttttctatcacattttggccaacaag
gtggataagtcctcgatggagtaccaaaccatacagagactggtgggcatgtgggtggcattcgcccgaaacgacaatcccaattgtcc
acagatcggcccgaccacatgggaggcactagatgagaaaggtccgcagatgtgtctcaatattggaaaacaactggagttcattgtgc
tgccggagtcgaaacagaatcggatttgggatagactatacgataaaaacgacttgttttag (SEQ ID NO:77)

**Protein:** AAG22202
**Protein GI:** 10727102

MRDCAIYSVIKTKSGPVRGVKRNTIWGGSYFSFEKIPFAKPPVGDLRFKAPEAVEPWDQE
LDCTSPADKPLQTHMFFRKYAGSEDCLYLNVYVKDLQPDKLRPVMVWIYGGGYQVGEASRDMY
SPDFFMSKDVVIVTVAYRLGALGFLSLDDPQLNVPGNAGLKDQIMALRWVQQNIEAFGGDSNNITL
FGESAGGASTHFLALSPQTEGLIHKAIVMSGSVLCPWTQPPRNNWAYRLAQKLGYTGDNKDKAIF
EFLRSMSGGEIVKATATVLSNDEKHHRILFAFGPVVEPYTTEHTVVAKQPHELMQNSWSHRIPMM
FGGTSFEGLLFYPEVSRRPATLDEVGNCKNLLPSDLGLNLDPKLRENYGLQLKKAYFGDEPCNQA
NMMKFLELCSYREFWHPIYRAALNRVRQSSAPTYLYRFDHDSKLCNAIRIVLCGHQMRGVCHGD
DLCYIFHSMLSHQSAPDSPEHKVITGMVDVWTSFAAHGDPNCESIKSLKFAPIENVTNFKCLNIGD
QFEVMALPELQKIEPVWNSFYAPNKL (SEQ ID NO:78)

# Figure 33

**Name:** Nrk
**Nucleotide:** AE003819
**GI:** 10727582
**Transcript:** CT13296
**Sequence:**

atggtgctgaaatggggggccaatttggctgtcctggggctgtgcgtgtttctctttgccagcgccacgcacgcgaactc
cctgaacgccatcgaggagcccgtcacccggcgacaccaccagcggcatcacgagcgcgagcgggaggagaacggctactgcgc
cccgtacagcggcaaggtgtgcaaggaatacctcaccggccaggtgtggtacagtctggaggatcccactggcgggtggaagaacga
gcaggtgaccacggcgctctgggacgagcttatctccgatcttacgggtctgtgtcgcgaagcagccgagaaaatgctctgcgcctatgc
gtttcccaactgccacatggagggcggtcgagcggtgaaggctcctctctgcttcgaggattgccaggccacgcatctccagttctgctac
aacgactgggtgctcatcgaggagaagaaggagcgaaatatgttcatcaagagccgcggccacttccggctacccaactgctcctcctt
gccgcactacaacgcttccatgcggcgacccaactgctcctacatcggtctcaccgaactcaaggagtccgaagtgagctacgattgcc
gcaatggaaacggacgcttctacatgggcacaatgaacgtgtccaagtcgggcattccctgccagcgctgggacactcagtacccgca
caagcacttccagccaccactggtcttccatcagctcctggagggcgaaaactactgccggaatgctggcggtgaggagccgcatccct
ggtgctacactgtggatgaatcagtgcgctggcagcactgcgatatacccatgtgtccggattatgtggaccccaatgctgtcgatttgaac
acgcccatcaagatggagaagttcttcacgccatcgatgatctttctcttggctggaataggtttcgtggccattgtgaccctgcacttgatgat
attgctagtctataagttgtccaagcacaaggattactctcagcctgcgggagcagccactgccgaatgcagtgtttccatgcgtggagga
ggagattgtggcggcaatctgaacaccagtagagaaaccctcggaggcaatggaaacacgaacaccttggcaaaatggggcaccat
caggagcacggccacaatacacagcaattgcgtggcccttactacggtgaccaatgtgtctgatgcgaagggcacgaaaccgaatgc
acgcctggagaagttggagtacccacgcggggatatagtgtatgtgagatcattgggtcaaggagccttcggtcgcgtcttccaggccag
ggctcctggacttgttcccgatcaggaagatctactagtcgctgttaagatgctaaaggacgacgccagcgaccagatgcagatggatttc
gagcgcgaggcctgtttgctggccgagttcgatcatcccaatatcgtgaggctgctggggggtgtgcgccttggcagacccatgtgcctgct
cttcgagtacatggctcctggcgatctaagcgagttcttgcgcgcctgctccccatatgccacacaccaggcgccgacacaggatcgtctg
cagttgaacgagctacatctgctgcagatggcggccaacattgcagcgggcatgctgtatctttcggagagagaaaattcgtccaccgggatt
tggccaccaggaattgcctgatcaacgagcacatggcggtaaagatcgccgactttgggctctcgcacaagatctattgcaggactatta
caaaggcgatgagaacgacttcatcccgatccgctggatgccacttgagagcatactgtacaacaagttctcgcttgagtcggatgtgtgg
gcatacggcatctgtctgtgggaggtcttctccttcgccttgcagccctactttgggttaacccacgaggaggtgatcaaatacatcaagga
gggcaacgtactcggctgtccggacaacacgccgctctccgtctacgcgctgatgcgtcgctgctggaaccgcaagcccagtgagcga
cctggcttcgccgagatcaaccactgcatccagcacagcatcgccgagagcgagtgcaaggcaatgctctaggggattgccggagaa
gtga (SEQ ID NO:79)

**Protein:** AAF58420
**Protein GI:** 7303361

MAAGQWVGVVERVLRGMVLKWGANLAVLGLCVFLFASATHANSLNAIEEPVTRRHHQR
HHEREREENGYCAPYSGKVCKEYLTGQVWYSLEDPTGGWKNEQVTTALWDELISDLTGLCREA
AEKMLCAYAFPNCHMEGGRAVKAPLCFEDCQATHLQFCYNDWVLIEEKKERNMFIKSRGHFRLP
NCSSLPHYNASMRRPNCSYIGLTELKESEVSYDCRNGNGRFYMGTMNVSKSGIPCQRWDTQYP
HKHFQPPLVFHQLLEGENYCRNAGGEEPHPWCYTVDESVRWQHCDIPMCPDYVDPNAVDLNTP
IKMEKFFTPSMIFLLAGIGFVAIVTLHLMILLVYKLSKHKDYSQPAGAATAECSVSMRGGGDCGGNL
NTSRETLGGNGNTNTLAKWGTIRSTATIHSNCVALTTVTNVSDAKGTKPNARLEKLEYPRGDIVYV
RSLGQGAFGRVFQARAPGLVPDQEDLLVAVKMLKDDASDQMQMDFEREACLLAEFDHPNIVRLL
GVCALGRPMCLLFEYMAPGDLSEFLRACSPYATHQAPTQDRLQLNELHLLQMAANIAAGMLYLSE
RKFVHRDLATRNCLINEHMAVKIADFGLSHKIYLQDYYKGDENDFIPIRWMPLESILYNKFSLESDV
WAYGICLWEVFSFALQPYFGLTHEEVIKYIKEGNVLGCPDNTPLSVYALMRRCWNRKPSERPGFA
EINHCIQHSIAESECKAML (SEQ ID NO:80)

# Figure 34

**Name:** otk
**Nucleotide:** AE003823
**GI:** 10727617
**Transcript:** CT25769
**Sequence:**

cgcgtgcagttcagattgagtttgaggttcgattggattcgagatatccaagttccttttgcgctacgttttgtttagccgagtt
tgccccgaactaaaatcacccaccaaagccggttcgggatgcgatgatgccaaggatgcagctgatgcagccgggctcttgtatctttaa
gatacaaattaagttgatttgttgcgctcgcgttctcttgggtaattgaaccccacagttgggctccttgcgccctctgccgcgcggtcttcggtc
ttcggtcttcgagcatttcgttgcattttgtgtatctagatacctttgtcggtatccgtatccgtcgcttgtcaatctggtcgctgataaaaaagcaa
acgcgaacgcagccacgaaacataaaccagccgtcaacggaaaataaaagtaaaaagcaacaaacagcaagtggcgtatcagttc
gtgtgttctcggtgataaatgcatttcatgcgttccaactatgtaaatagcaatttaatttgagtgtccacagtaaacatcaaaaaaccgccct
ggactccagtcaccaccaccaacaacaccatccccggtggagcccagtgatcccattgaaaccccggtaaacccctgaaaagggca
gtcgttgtcatcgtcgtcatcgtcgtcgtcgtcgtacgtctgtgccatccaaatattgaatatttacaaatgcgttgagtgcattttcgagtggccg
gcacttacagtgactatctggtggatgtgcgagtgtattcatcggattgccagcagcataaaacttcgttttcgatactgatccaatacgcgag
tgcttatatatttttgtgtcgctaattgagaccaaaggtgtcgaagtgcggcggttatacatatacgcagatgactgctaggatgatctcaatat
gcggcctggtcatggctttgatgatggcttccgttttggccagctcatcgcgtttccagcgagtgccccaaagtcagtccgtggtggaaaatg
aatccgtgaaattcgagtgcgaatcgaccgactcctacagtgaactgcactacgactggttgcataatggtcatcggattgcgtatgacaa
gcgtgtccatcaaatcggttccaatttgcatatcgaggcggtgcggcgcacggaggatgttggcaactatgtttgcatcgcgacaaatttgg
ccagcggcgccagggaggcgtctccgcccgccaaactgagcgtgatatatcttgaatcggccagtgttcagctgctgggcagcaatcgc
aacgagctgctgctcaagtgccacgtggagggcgcctcaggagatttggaaccgctagagatcgaatggtatcgcaacagcgagaaa
cttagcacctggaagaacgtccagctggatcagcatcgcctgataatccgacagccgggaagcgaggacgatggcctgtaccgctgca
ccgcctccaatgcggcgggtcgagtgatgagcaagcagggctatgtctaccagtccagtgtgaagtgcctgccccgacttcctaggcgc
aagaacgaaaagatgatggagtcctgggacaaacagaccttcctgtgccgcggtaagcgcggtggagctgctggactagaggcacta
cccgccgctcccgaggatctgcgcattgtgcaaggacccattggtcagtcgattatcaaggagggtgagcccacggctttgacctgtctgt
atgaactgcccgacgagctgaagaaccagcgcattcaactccgttggcgcaaagatggcaaacttttgcgtcaagtggaactgggtggc
tcggcgcccattccaggtcactccttcgattccggcaaggatgctctgttgcgtgaggacgcccgtctagtgctgcacaagcagaatggca
ccctaagctttgccagcattattgccagtgatgcgggtcagtaccagtgtcagttgcagctagaagctcatgctccgattaactcatctcctgg
catcttggaggtcatcgagcagctaaagtttgttcctcagcccacgtccaagaatttggagctggatgcagtggtggccaaggttcactgca
aggcgcagggcactccaacgccgcaggtgcaatgggttagggatggggaaaataccactttacccgaccatgtagaagtggatgcca
atggaacactgattttcagaaacgttaactcggaacatcgcggcaactacacttgcttggccaccaacagccaaggacagataaatgcc
actgtggctataaatgtggtggtcactcccaaatttagtgtgcccccagtgggacccatcgagacttcggagcagggcaccgtggtgatgc
attgccaggcgattggggaccccaagccgacaattcagtgggacaaggatctaaagtatctgagtgagaacaatacggaccgcgaaa
gatttaggttcctggagaacggcactctggagatacggaatgtccaggtggaggacgagggtagctacggctgcacaattggaaatagt
gcaggactcaagcgagaggatgtccagttggtagtgaagacaaccggtgacggtttcgctccagaggaatccggaggtgatggcttcct
ggtcacccgagctgtactgatcaccatgacggtggccctggcctacatagtgctcgtggtgggcctgatgctgtggtgtcgctatcgccgtc
aagccagaaaagcccgtttaaacgatctgagcacaaaggaagctggcggggatcagccggatgttgctggcaatggcaaaggctccg
agcaggagccctgcctgtctaagcagcacaatggccacagcaaatccagatccaagtccagtggagatgcccagaaatccgatgaca
cagcctgcagccagcagtcgagggcctcaaagaaatcggcacatatttacgagcaactggctctgccaagatccgggctcagtgaact
catacaaattggacgcggcgagtttggggatgtgtttgtgggcaaacttaaggctaccttagtcacctcgccctcggacaaggatgcggat
acggaaaagcagcacagcaacagtgaaaatggcagtggaggcagcggaagtggaagcaccacgctaagtactctaaatgagaag
cgtcgctccaagacctccatggacgacattgaggagatcaaggaggaggagcaggatcagcacaatcaatcgggtctcgagcagctc
gttctggtcaaagccctgaataaagtgaaggatgaacaggcctgccaggagtttcgacgacagttagatctgctgcgcgccatctcgcac
aagggagtagtacgtctgtttggcctgtgtcgcgaaaaggatccgcactacatggtgctggagtacacggattggggcgatctcaagcag
ttcctgctggccaccgccggaaaagtgaacactgccaccgcgggcagctcctcaccgccgccactcaccaccagtcaggttttggccgt
cgcctatcaaattgcccgaggaatggacgccatctacagagctcgcttcacccatagggatctggccactcgcaactgcgtaatttccagt
gagtttatagtgaaggtatcctatccggctctctgcaaggacaagtatagccgcgagtaccacaaacaccgcaacacgctgctcccgatc
cgctggctggcgcccgagtgcatccaggaagacgagtacaccaccaagagcgacatctttgcctacggtgtggtggtgtgggagctcttc
aaccaggccaccaagctgccccacgaggagctgaccaacgagcaggtggtgcagcgttcccaggcgggctccttggagtggtcagtg
gccgaggcgacgcccgatagcctgcgagagatattgctgtcctgttgggtgtccaatccgaaggagcgaccttcgttcagccaactggga
gctgctctcagcaaggcgatgcagagtgccgagaagtgaggccagactgatggctcgatcatgccaaaaagtaattcgtagtattgtctt
agtgtctgcacgcccgagttttgctatgcgctcagaaacctagtcttaaggatcgaaggataactgcgcctgtttaacatgcgcattgcgca
cttggtggcacacagaatgaagttaagcagctactgtacaaacacaatgtatggaaatggggatatcttcgtgaatagtattgcgtggtgct
gcatattttaggtatgcagctaaaatgtacataataactgccacaaatatgcatttaaaccagttgaagcatttctaggattagatcatttcaa
ccttcgtcagcatttaataatcgacttctaagctaaattagtaaataaaataaaatataaatacacaagcatatgcatcacacaaaacaac

gagcgatttcattgttttcaaacattgagaaactaagccaaattttaagtttagcattaaatataaataacacacaccacccacatattttatca caagcctagttatacactttttaatgcgtaagaaacaaacagccgaaacaattttatgactagccatttaagctttgatgcatgtttacaaaac atttaaataaactcgaataaaataaatttaatcagaa (SEQ ID NO:81)

**Protein:** AAF58596
**Protein GI:** 7303541
MTARMISICGLVMALMMASVLASSSRFQRVPQSQSVVENESVKFECESTDSYSELHYDW LHNGHRIAYDKRVHQIGSNLHIEAVRRTEDVGNYVCIATNLASGAREASPPAKLSVIYLESASVQLL GSNRNELLLKCHVEGASGDLEPLEIEWYRNSEKLSTWKNVQLDQHRLIIRQPGSEDDGLYRCTAS NAAGRVMSKQGYVYQSSVKCLPRLPRRKNEKMMESWDKQTFLCRGKRGGAAGLEALPAAPEDL RIVQGPIGQSIIKEGEPTALTCLYELPDELKNQRIQLRWRKDGKLLRQVELGGSAPIPGHSFDSGKD ALLREDARLVLHKQNGTLSFASIIASDAGQYQCQLQLEAHAPINSSPGILEVIEQLKFVPQPTSKNL ELDAVVAKVHCKAQGTPTPQVQWVRDGENTTLPDHVEVDANGTLIFRNVNSEHRGNYTCLATNS QGQINATVAINVVVTPKFSVPPVGPIETSEQGTVVMHCQAIGDPKPTIQWDKDLKYLSENNTDRER FRFLENGTLEIRNVQVEDEGSYGCTIGNSAGLKREDVQLVVKTTGDGFAPEESGGDGFLVTRAVLI TMTVALAYIVLVVGLMLWCRYRRQARKARLNDLSTKEAGGDQPDVAGNGKGSEQEPCLSKQHN GHSKSRSKSSGDAQKSDDTACSQQSRASKKSAHIYEQLALPRSGLSELIQIGRGEFGDVFVGKLK ATLVTSPSDKDADTEKQHSNSENGSGGSGSGSTTLSTLNEKRRSKTSMDDIEEIKEEEQDQHNQ SGLEQLVLVKALNKVKDEQACQEFRRQLDLLRAISHKGVVRLFGLCREKDPHYMVLEYTDWGDL KQFLLATAGKVNTATAGSSSPPPLTTSQVLAVAYQIARGMDAIYRARFTHRDLATRNCVISSEFIVK VSYPALCKDKYSREYHKHRNTLLPIRWLAPECIQEDEYTTKSDIFAYGVVVWELFNQATKLPHEEL TNEQVVQRSQAGSLEWSVAEATPDSLREILLSCWVSNPKERPSFSQLGAALSKAMQSAEK (SEQ ID NO:82)

# Figure 35

**Name:** cad
**Nucleotide:** AE003668
**GI:** 10799497
**Transcript:** CT5072
**Sequence:**

ttgacgtcgccacgcacggaataacatttcaaattagtagattagccctgcaaaacaaaaatccatctcgtgagtgag
tgctgaaaaatagcgtttcgaaatattatcgatatctcggtcgtatgttactgtgctcccaaaaaacacatcaccagccagtgataactgtaa
agattgacaaagcaaaatatatccattataacccaaactgaaactccagaaaatgtgcaactaaaacatattggccatcaaagtgataa
gacttagagattaacgccggattattggacaccggaccgagcactgggactatcttcggagaaagcgcacctaatctaacctcaaaatg
gtttcgcactactacaacacactgccctacacacaaaagcacagtgccgccaatttggcctatgcctcagcggcgggccagccatggaa
ctggacgcccaactaccaccacacgccgccgaaccaccagttcctgggcgatgtggactcctcgcatgccgcccaccacgcggccgct
gcccaccagatgtactacaactcccatcacatgttccactcggcggcggcggcctctgccggggagtggcactcacccgcctcgagcac
ggcggataacttcgttcagaatgtgcccacgtcggcccaccagctgatgcagcagcaccaccatcatcacgcccatgcctccagcagtt
ccgccagttccgggagcagcagtagcggtggtgcaccgggtgcgccccagctcaacgagaccaacagcagcatcggcgtcggagg
agcaggcggtggcggtggtgttggcggggccaccgatggaggaccaggttccgcaccacccaaccatcagcagcacatcgccgaag
gtctgccgtcgccaccgattaccgtttcgggctcggaaatctctagcccgggggcgccgacttctgcctcgtcgccgcatcatcacttggca
caccatttgagtgccgtcgccaacaacaacaacaacaacaacaacaacaacaacaatagcccatccacccataacaacaacaacaataa
caattcggtgagcaacaacaacaggacatcgccatcgaagccgccatacttcgactggatgaagaagcccgcctatccagcacaacc
acaaccaggcaaaacccgcaccaaggataagtaccgcgtggtgtacaccgacttccagcgcctggagctggagaaggagtactgca
cctcccgctacatcaccatccggcggaagagcgagctcgcccagacgctgtcgctgtcggagcgccaggttaagatctggttccagaac
cgccgcgccaaggagcgcaagcagaacaagaagggtagcgatccgaacgtgatgggcgtgggagtgcagcatgcggactacagc
cagctgctggacgccaaagcgaagctggagccgggcctgcacctgtcccactcgctggcccactcgatgaaccccatggcggcgatg
aacattcccgccatgcgcctgcatccccacctggccgcccacagccactccttggcggcggtggcggcgcactcacaccagctgcagc
agcagcacagtgcgcagatgtccgctgcggcggcagtgggcacgctctcgatgtga (SEQ ID NO:83)

**Protein:** AAF53923
**Protein GI:** 7298711

MVSHYYNTLPYTQKHSAANLAYASAAGQPWNWTPNYHHTPPNHQFLGDVDSSHAAHH
AAAAHQMYYNSHHMFHSAAAASAGEWHSPASSTADNFVQNVPTSAHQLMQQHHHHHAHASSS
SASSGSSSSGGAPGAPQLNETNSSIGVGGAGGGGVGGATDGGPGSAPPNHQQHIAEGLPSPP
ITVSGSEISSPGAPTSASSPHHHLAHHLSAVANNNNNNNNNNNSPSTHNNNNNNNSVSNNNRTS
PSKPPYFDWMKKPAYPAQPQPGKTRTKDKYRVVYTDFQRLELEKEYCTSRYITIRRKSELAQTLS
LSERQVKIWFQNRRAKERKQNKKGSDPNVMGVGVQHADYSQLLDAKAKLEPGLHLSHSLAHSM
NPMAAMNIPAMRLHPHLAAHSHSLAAVAAHSHQLQQQHSAQMSAAAAVGTLSM (SEQ ID NO:84)

# Figure 36

**Name:** rut
**Nucleotide:** AE003497
**GI:** 10728252
**Transcript:** CT26958
**Sequence:**

gttgcttctcttgcagcccctgatcttggcggccccaatccctttggcaggcatcggaaggagccgctcggcagcggc
agctgcggcggcgccgcatggatcatgccgtgaaggcgacgcgcggccgcccgttgaatacgctgcgcttcgagaacgatgagctgg
aatgcctctaccagcggtatacgctcaagctgcagcgcttctcggtgctgggcgtggtggctctggtcttcgtcctctgcggcgtgatggcgg
ccctttcgctgaccttcaacaacgcggccacctttcacaacatcttcaatgcgatcgtttgtggactgtttgccgtggtgctggtgctgctgcag
tgttccgtgatcaaggaccaccacctgcccacattgtgctacggaatcctgctattcacggccagcatctgtgtggtgtcgatgcccacattg
ggcagcgtgttcccggtggacaccaaggaggtgatggccgagggcgtctggcagatcgtgttcgtggtcttcctcgcttacgccatgatgc
cgctgcagatttgggaggcggtggcctttggcatcgccctgccctcggtgcacataagtctaacggtctataagattttcacggatgcgctgc
gctacctggagtataatcaactgattgcgaacattgtcatctttattggtgtaaatgtggccggtctcgtggtgaacatcatgatggagcgcgc
acagcgtcgcacatttctcgatacacgcaactgcatcgcatcccgtctggagatccaggatgagaacgagaaactggaacgtctgttgct
atcggtgctgccgcagcacgtggccatgcagatgaagaacgacatcctgtcgccggtggctgggcaatttcatcgcatctacattcagaa
gcacgagaatgtgagcatcctttcgcggacattgtcggattcacggtgctctcctcgcagtgtagtgcccaggagctggtccgcctgctca
acgagctctttggccgcttcgatcagctggcacacgataatcactgtttgcgtatcaagatcctgggcgactgttactattgtgtctccggattg
ccagaacctcgcaaggatcacgccaaatgcgccgtggaaatgggactcgatatgatagatgccattgccaccgtggtggaggccaccg
acgtcatcctgaacatgcgcgtgggcatccacacgggccgtgtgctctgcggggtcttgggccttcgcaagtggcagttcgacgtgtggtc
aaacgatgtgaccctggccaatcacatggagtccggcggggaaccgggtcgggtgcacgtaacccgcgccaccctggactccttgtcc
ggcgaatacgaggtggaggctggccacggcgacgaacggtcctcgtatctgcgcgaccatggtgtggacacgttcttcatcgttccgccg
ccacatcgacgcaagccactgatgctcaacacactgggcgttcgctcggcgatcggcagtcgacggaagttgtcgttcagaaacgtgtc
caatgtggtcatgcagctgctgcacacgatcaagttctcggaaccggtgccgttctccaatatagccaccggatcgttcccctcagcggcc
agcgccttgggtggcggagtttcagtcggcggtggtggaggaggaggaggaggaggcgtggcacggggtagcacctgcgaggcgaa
ctctggaaacgttcaggtgtcggagaagggatcgcgcaaggtgatacgtctgcaaaagatcttgcatgcaacaccgccaccgcacggc
atgggctatggcagcgtcgtcggcagcggcggcggtgtggacagcggtatttccggtggtggtggctgtgtttccggtggtattgccggtgg
gggcggtgttcaagtgaccgtaggcacaaatcccaactctacagcgagtacgattagtcgcattcataggcacaatcacaaaaacaata
aatcccagtcgaaggtggcggacaaattcaagaggccattccggaagcggcactcggtggccgcccaccatcagccaacgaatcgg
gtgaatcgattcctttcccaggcgatcaatgcccggtcggtggactgcgacaaatcggagcatgtggatcgattgaccctgcgattccgac
agagcgacatggaacgcgagtatcacaaggatttcgatctgggcttcactacggccatgggttgctccctgctgttgctcatcctcggtgca
gcgctccaggtgactgctctgccgcgcactctcatcctgctgctgctctttctctttgccttcatttgggtgagcgccattctgatgctcctgctcgc
cgtgcgtctcaaatggatcatctgggatatttcggagagcttttcgctgcgcatggccatcacgattttcacggtcatactcatctattccgtgg
gacaagtgaatgtgtttacgtgcgtttcggatcatccatgctcgggcaatgggacgacctccttccagaacgactctcatcgcaagtgctcg
ctgccgcagtacgtgtccctatcggctgcctttgcctttctatctgtatcggtattcctgaggctgcccatcatattcaaatcgctcctagttctggg
catgggcaccatctatggccttttcatagagctatcgcatcagaatatctttgagtgctacgacaaccgggtcaacgcctcgattccacttca
cttgatctcgctggcccgcattgccatcttcatgattgccattttggtgcatggacgcctggtggagggcactgcccggctggacttcctctgg
cagctgcaggcgtcgcaggagaagaaggagatggatgtgctacaggagtccaataagcgcattctacacaatttgctgcccgctcatgt
ggctgcacattttctcgatgcgcaatttcgcaacaacatggaactctaccaccagagctatgccaaagtgggcgtcatctttgcaagcgtac
cgaacttcaacgagttttacacggaaatggatggctccgatcagggtcttgagtgcctccgtctgctcaacgagattattgcggactttgatg
agctgctcaaggaggatcgtttccgcggcatcgacaagatcaagaccgttggtagcacttatatggctgtcgtgggactcatacccgagta
caaaatccagcctaacgatccgaactcagtgcggcgccacatgacagcgctcattgagtatgtcaaggccatgaggcactcactccaa
gagataaacagtcactcgtacaataacttcatgctgcgcgtgggcatcaacattggacccgttgtggcgggcgtaattggagctcgaaag
ccacaatatgacatttggggcaatacggtgaacgtggccagtcgcatggactccaccggagttcccggctactcgcaagtaactcaaga
ggtggtcgacagcctggtggggtcgcactttgaattccgctgtcgtggcaccattaaggtgaagggcaaaggcgacatggtcacatatttct
tgtgtgacagcggcaacaaatcgctgaacggagaagtgcgcaacgccatgtccctgccacagagtctccatgcgccggattactatatg
aaggtgtcgcagtttccggagaatcgcgtcaataccgacacctatagcaaaaaggagaatggacatctgtacgctggcaacggcgtcg
aggagcagcagttgttgctgcagcatcagcacaaacagcacgatccgctaccgcttcctgctcctccgcctccggttcaccatcatttgcat
cagcagcaacagcagcgtttgaacagcaagctgcaaaagcaaccgatcttcatggccaacggtggtctgcccaatatccgggagaat
ggcaatggtcacaatggagagcatcagcagcagcagcagcagcagcagcaacatcagcagcagcaacagcagcagcagcagca
cggtggctttatggtggcaacgacgacaccgccggctgcagtggcggttcccctccagccccagcaccatcaactccagttccagcacc
cgcaccagcatccactcccgtcagcagtgtcggttccagtgcagcaccagatcctactccaccaccagctccagctccagcaccagccc
gttccttcggtaatgttgcgcgagtttaatattattgagaatccgacttccggtggccggcatcagcagatggagcagctgccgccgcatca
cggcagcctcgacctatcgggaatgggaatgggtgtgggggctggcgtgctcggcagcgactgcttcatgatgccacggcgagatcgc
gaacgcacatatgtgccgcccttaaaccagcatggccatcatccaccgcatcacctccattcgaatctgaatctgaatcagagccagcac

ccgccatcgttcaccagcctgggctatggccaatgccgggagagcgagccgctattgcatgccagcagcgtcgcgccggtagccaaga
tcatgcccatgcagcatgcgccaaagtacgagccaccacgttacacttctccccacaccatgttgtcgcaacagcaccagcagcagca
gcagcagcagcatcagcatcagcagccgcaatcgcaatctgcccaggatcagcagacacatcccgcccaggacccacatcctttgca
gcggcagtacgccatgtattcgcagcagccgcagctgccgccgaagccagtgctgagaacgtacatgaagccgttgccgaagctgcc
gacggatctggaggagagccgcgacatgtcctcgacggatgatctgagctcacgaccacactcgccctcgatgagcagctcggatgag
agctactcgaagaccacagagggcgaaggtgaaggcgatgaggactcgccgcgcatggtcaatggtggccacctgcatcatcgcaat
ggctaccacctgcccgccggtggtctggtcaacccattgcaatggctctacccctgcgacattcaggtggatcccacctctccggtcgtcga
catggcccacctgcatgactttgagctaagctccaccaccgagagccagggccatcacactaacagcaacacgacgagcaacacac
aacacaagggcgacagctgcaacagcttcgactttcagaaggcagcagttggaacggcagcgggtgcggcaatcgcgaccaagtcg
cccttcgagcgtgaactgcaacgtctgcttaacgaatcctcgcgagctcgttgcctggcgactgcgacaacgacggctggagccatctcta
ccacagatcagacggccagcaatggaagccgtgagttgagctactccctcagcaatgggaagctgagctctgcgaatggacatggcgt
tggaggatccggatccggatccggatcgggatcaggttcaggttcggcggtgggcaatggcagcggtggcagcggcagcagcaatgg
caatctcagcggcggcagcggcagcaactcgaacagtggcaacaacaacagcagtcaccacaagacggagcagcagcagaatat
ggatcacgagcatctggccggtggcaaattgctgggcagtaatagcttcatgatcgccaaacatccggtgggcttggaagccatcaagg
agataacgcgcaacaagaatccctcggagtccagccaaatgcaaacctcggacacggagtcctgcgagattctgcacgagaaccgc
aaccagatgcatgtgctggccatgctggagatgcatacggccaaggagctgaatggcagccatgcgcatcacggtcagcatcaccagc
agccacagaggacgcatcgccagcgaccgcgctccaaggagctgcagtacagccacgagagtctggacggactggatggagcggt
gcagtcgcagtcgcagcagcgacaccaacgctatcatcaccaccatcaccaccaacagaggcagcagcaacagcagcgctacaac
catgtgcaggagcaggaggagcgtgacgacaccgaggacaacctggccgatgaggagtttgaggacgacgaagtgggtcgtgatgt
gcgacagaagcgcctacaaaagtccgagctcaatcacaaacgatcggaggttgcaacagaggcaggcaatcaccacgacgacga
ggtggaggaggaggacgacgatgacgacgaagaggaggaccaccggaatggcgggcgggaggcggcaccgctgactaacggc
agcatgcgcggcctggaggccaatgtgataaacgacgagctgaagtatggagcaacccatctcaatcaccagtccatggactccaatc
cgttggagagccaatcggagtggtcggatgacgattgtcgtgaggaagcaacaggggggagccgagtccacgggctacattacggatg
agcccggactggagaacatctcgttgctgaacgaagctggtctcaccgatgccgagggcgctctctcggatgtcaactcgctgtataacg
cgccggacgtggacgacacttcggtgtccagccgggccagctcccgcctgcttagcctggactcgctgagcggcctctacgactgcgat
ctggactccaagcacgagctggccatcgtcaatgcctcgcacaagatctccagtaaatttgggcagcctctatcaccggcacagcagca
acatcaacaacagcagcaacagcaacagcagcaacagcagcaacaccaccaacagcagctgcagcagaatccgcagcacactc
aagcccaatcgcatttggctccggttcagtttcagtccgccgaggagttgagagagtaaagattgaaagttcgtgtgaagcaggtggagc
gttaaacactcaaacgttgttattgctatatgcaaatggtgtctaataatttacaataaaccaacctaaactatagtaaactaaatctagaata
ggtgacagatatgcatatatcgaaatatatatatatat (SEQ ID NO:85)

**Protein:** AAF48388
**Protein GI:** 7293001

MDHAVKATRGRPLNTLRFENDELECLYQRYTLKLQRFSVLGVVALVFVLCGVMAALSLTF
NNAATFHNIFNAIVCGLFAVVLVLLQCSVIKDHHLPTLCYGILLFTASICVVSMPTLGSVFPVDTKEV
MAEGVWQIVFVVFLAYAMMPLQIWEAVAFGIALPSVHISLTVYKIFTDALRYLEYNQLIANIVIFIGVN
VAGLVVNIMMERAQRRTFLDTRNCIASRLEIQDENEKLERLLLSVLPQHVAMQMKNDILSPVAGQF
HRIYIQKHENVSILFADIVGFTVLSSQCSAQELVRLLNELFGRFDQLAHDNHCLRIKILGDCYYCVSG
LPEPRKDHAKCAVEMGLDMIDAIATVVEATDVILNMRVGIHTGRVLCGVLGLRKWQFDVWSNDVT
LANHMESGGEPGRVHVTRATLDSLSGEYEVEAGHGDERSSYLRDHGVDTFFIVPPPHRRKPLML
NTLGVRSAIGSRRKLSFRNVSNVVMQLLHTIKFSEPVPFSNIATGSFPSAASALGGGVSVGGGGG
GGGGGVARGSTCEANSGNVQVSEKGSRKVIRLQKILHATPPPHGMGYGSVVGSGGGVDSGISG
GGGCVSGGIAGGGGVQVTVGTNPNSTASTISRIHRHNHKNNKSQSKVADKFKRPFRKRHSVAAH
HQPTNRVNRFLSQAINARSVDCDKSEHVDRLTLRFRQSDMEREYHKDFDLGFTTAMGCSLLLLIL
GAALQVTALPRTLILLLLFLFAFIWVSAILMLLLAVRLKWIIWDISESFSLRMAITIFTVILIYSVGQVNV
FTCVSDHPCSGNGTTSFQNDSHRKCSLPQYVSLSAAFAFLSVSVFLRLPIIFKSLLVLGMGTIYGLF
IELSHQNIFECYDNRVNASIPLHLISLARIAIFMIAILVHGRLVEGTARLDFLWQLQASQEKKEMDVL
QESNKRILHNLLPAHVAAHFLDAQFRNNMELYHQSYAKVGVIFASVPNFNEFYTEMDGSDQGLEC
LRLLNEIIADFDELLKEDRFRGIDKIKTVGSTYMAVVGLIPEYKIQPNDPNSVRRHMTALIEYVKAMR
HSLQEINSHSYNNFMLRVGINIGPVVAGVIGARKPQYDIWGNTVNVASRMDSTGVPGYSQVTQEV
VDSLVGSHFEFRCRGTIKVKGKGDMVTYFLCDSGNKSLNGEVRNAMSLPQSLHAPDYYMKVSQF
PENRVNTDTYSKKENGHLYAGNGVEEQQLLLQHQHKQHDPLPLPAPPPPVHHHLHQQQQQRLN
SKLQKQPIFMANGGLPNIRENGNGHNGEHQQQQQQQQHQQQQQQQQHGGFMVATTTPPA
AVAVPLQPQHHQLQFQHPHQHPLPSAVSVPVQHQILLHHQLQLQHQPVPSVMLREFNIIENPTSG
GRHQQMEQLPPHHGSLDLSGMGMGVGAGVLGSDCFMMPRRDRERTYVPPLNQHGHHPPHHL
HSNLNLNQSQHPPSFTSLGYGQCRESEPLLHASSVAPVAKIMPMQHAPKYEPPRYTSPHTMLSQ
QHQQQQQQHQHQQPQSQSAQDQQTHPAQDPHPLQRQYAMYSQQPQLPPKPVLRTYMKPLP

KLPTDLEESRDMSSTDDLSSRPHSPSMSSSDESYSKTTEGEGEGDEDSPRMVNGGHLHHRNGY
HLPAGGLVNPLQWLYPCDIQVDPTSPVVDMAHLHDFELSSTTESQGHHTNSNTTSNTQHKGDSC
NSFDFQKAAVGTAAGAAIATKSPFERELQRLLNESSRARCLATATTTAGAISTTDQTASNGSRELS
YSLSNGKLSSANGHGVGGSGSGSGSGSGSGSAVGNGSGGSGSSNGNLSGGSGSNSNSGNNN
SSHHKTEQQQNMDHEHLAGGKLLGSNSFMIAKHPVGLEAIKEITRNKNPSESSQMQTSDTESCEI
LHENRNQMHVLAMLEMHTAKELNGSHAHHGQHHQQPQRTHRQRPRSKELQYSHESLDGLDGA
VQSQSQQRHQRYHHHHHHQQRQQQQQRYNHVQEQEERDDTEDNLADEEFEDDEVGRDVRQK
RLQKSELNHKRSEVATEAGNHHDDEVEEEDDDDDEEEDHRNGGREAAPLTNGSMRGLEANVIN
DELKYGATHLNHQSMDSNPLESQSEWSDDDCREEATGGAESTGYITDEPGLENISLLNEAGLTDA
EGALSDVNSLYNAPDVDDTSVSSRASSRLLSLDSLSGLYDCDLDSKHELAIVNASHKISSKFGQPL
SPAQQQHQQQQQQQQQQQQHHQQQLQQNPQHTQAQSHLAPVQFQSAEELRE (SEQ ID NO:86)

# Figure 37

**Name:** CG8002
**Nucleotide:** AE003511
**GI:** 10728334
**Transcript:** CT24042
**Sequence:**

atgtgtgcacctcctcttgacctttcgcaggatccggaggacttctaccgcctggatccacagcgatcggccgccgaa
aatgcattcgaaatatactcgatgctctgtttggaggagacgcgggacacgaagcgcctgtttctgctcaacgcgctggcctcgctctgtttg
ggggcccgaaagggctcccaccacagcaatatacgcttcaccaccgaggagctgctctactgccttagtgcctcactggtgcacaccttc
acccaagtgcgagcggcggcactgcgaacgatccggtatgccattagcacgcccaatgacatcaagacgttcaatgcactgcagctgc
agcacctgctctgccgctccatcgatctaatgctgaagaacgacgacgagcgcgtccaggcactgaagctggtccgcaagatgctggc
catcgcgccggaggacatcagtccggcggtggtgcgctgcctggtctcgctggcggatagcggcatcgaggagaacgacaatctgttgc
gtgcctgcctggccaccctggcagagttcgctgtgctcaatccggcgctgctgatcgtctgcggcggcgtcacctccataacccgcaatgt
gctagagtgccacaatccccgcatcgctgagagcctgtgcggcgtgcttctgtacctgctcgagtggccgcaaacgaggaacatctgcg
gtgtgcggctggactgcctggcagctccttattgcgatttcacctaccggcagagtatcgtcgataagaacaaggatgcgcgcgaacttcg
ctacaccagctgtcggctggcactgctgtctgtgctgcgcagctggacgggcaccctcgaattctgtgatcctagcaaaccctcaggcctg
aaagcaatagtcgacgctctgtatttgaaccagatcgaagtcagaaaagcaatattggatttgctgtacgagctgctcacgctgccgcagc
caacctggacggatgactatgcggtggcgttgcaggctgtggatccgtcggacttccaggacacgtggctgctgagcaatggtttcgtgtc
cgccgaagggcgctccatttgccaacgctggcggcgcgtgcaccgagcgtagtagagcagcacctggcgctgatgctgtactgtttcct
cgagactggactgctcaatgcgctcgtcgaggttgtggtgagcagcgatcagttcgtctcggtacaggctacggtgctgattggcaagatc
ctgcagctaatgcacacccacctgcccccggatatttgctgtacgagtccggcactgcccacgctggtgagtcatgccacattggggaac
cagcaggccaatgcagcggtggccgctctgcaaaactaccagaagttgctgcgccagcggccggcgtcgcgcagcttattcctagaca
gcattatccagggcggtgctctcatccagacgcgactgtttcgtcggcacctcaacgtgcaggagcaagcgggtcctgtgctacagttgca
ggaaacggcggaggcggcggcatttgcggtaccgcctgtcactcagtttcgcggcaccctcgatcgttctcggctggattctgtctcctcca
gcgacgagtccaactcacaggcttcgacctcgtcgaggtccagttttcgactgaagcgtaagttccttccgcaagccttatatgataacttcc
gagccttcaatcggctgctgacagactcccgtgtgctgagccaagcggacgcgcatctttgggactgggatgttattaccaccatactgaa
gtcgaatttaatccgcaaactggactacacccaaggaaagttcctgaagcgactcgtggacttctacaagccgcgcaataatcggttctct
caccaggatttggtgcctggacagaggctgcctacatatgtcagtgccggattggatttaattgacgtcctcctgtccagcaatgagcttgaa
tgtatgcgctttataaccgactacttttcggacatcagccaacagctggcggccgtcaccacatcgaatcgtgcacacgactgtctatttagt
ccacagcatatgaataacacgatgtgtcagcagtatttcctctacatcggacgcatgtgccgaaccgtaaaaggcatcgaagtactcaaa
aataccaccgtatttgaatacttgatcaatttggtgcgggtgacggaccacgtctgctatgtgaagctaattgtgtccggcctgaactacagct
acgagaagctaccacgtcaggtgctggaaaaggcgcttacgtcggcgaagacacgcggacgtctgtactccacccagttcatggcagt
gctgctgcgtgcccgtctgcctcactttgaggtatggggcattccattgattatcaaccagacgcgggattcggaccgtagcgtggggctgg
cagccatggatgtactggaggaggcctgccacgacaagtactacctggaggagattgtcagtcggtggcccaatctaacgcatcgtggt
gatgcaggacggctgctgatggcccgctattattcactgccccgcggcctgaacagtacgatggcacgaatcgaggatgagatccgcta
ctggcggaatgggtacaacaagcgctacgtgctcctcgtggaagccgacacccactcaagcctgacgctgcacatccgcaatgaggat
ggctactactcgcgtcgcaattgcaaccagagaccacagactgtaccaccaaacgttgcacctcatctgtacgggcaaatggcacaga
ctggccagggtatgacagctctgcggaagcacggagacctgccgcagctattggaactgctcagacgcgccaaatgcacggacgatg
ccgagtgccttgaactgaaggccgccatttgggcactggcccacgcctcaacccactccaatggaattgaatactttgtcgaactgaatgc
cagactatacgagaagcttattgttctggtgaccaaatgcgaggtatattcggtgagggccacctgttttagtgccttaggtcttatcgcaggc
acacaggcaggtgccaatatcctttttaagttgaactggctgagcgtgcgacatgacaagaacacaatgtggcccgtgcatcagccgga
ggattggatgtccagccagtacacaccggtgcgacattactacgaggacgtgccggcggacaatatgaccatgatggatgactatatag
aaagattctacgaaacgggctcagacttttggcacatgctcgccgatcagactgatgcgagtggtggtggtgggggtgtagccggcaca
gtcgtcggtatgcatcctatagtgcaggatccaaatgcaacgattacgaccacggacagtgttcgcaccacgaccgatgatgtgcacccc
agaatgttggttggagtggctgccaaatcaaagactttgccagaaggtagcaatctgcgtcagggcaagcaccaacgctcgctctccga
atcgaagaccacggatgtgatcagcttgttgggcagtggtgtaggtacgatgtcgggagctgttctctacccaacgccgtatcagcatcga
atacgctacaactcgtgcacggattcaaacacttccggcgttagcagctgcgaatcggtgaccggacgcactgcagctgcatatgccgc
cgcagccaatgagttacagcaattccctttgagtcctattccaagcatgtccaatctgctcgagagcgatgagctgtttcgtcgaaatcagct
agccaccagtatgctgccctccaccctcagtcctatggcgatgaagggctatgtccagctacgctccctgcgcaagcacagccgacccgt
attctccgaatcttcggcggaattctacgactttgcggaaatactggacacaccagaggttcagatgcgcaagctcgactggacgcattcc
caccgtcggcttaaggtacgcagcttggaccgccagctaagtgatgtttatagacgcctctccgcggacgagttgaatgtgcttctgcccac
gcttaatgctcccaagttcctgcctcctaatgatttgaaagggccatgctatgcgggtatctgcttgcctaagaacgttctggacctgtttccga
cgcgtaacctcagtcgaacgtacgtttcgcgggatatccaggaccaagatatagtgggcataaatctgctgaacaccatgctacggccac
agtgtctcaatgactcactcaccaatgagggcggcgacgagtcatcggttatatcatcgctgtcggacgtctcgtctgcaagtcggcgaca
aacacgacaattgcagcaaggcgccaagcactcgcgaagtctgtgtctccattgtgcccgctccccgcgacagcagcgtaatgacagc

ggcagccatagtaatggtggaggaggcgcatccctcgctccctgcgagctgtacagcagtgcagcagcagctctggtggcagccggca
tccaggctggaccagttttggctaaaaagagtggctcatctgcccaaggagcttcagcggcacttggcgccgacatcagcttccattcccc
agagagcatgctgagcgaggatagcctgccggacaggcttacggcctccattctgtacaacgtgcagcgtctggccaatcccgttagtgc
caagcagtcgaaaatggctctgttggagcttaagcagaaacacccgcacgcctttcaggatatttgcctttattcggaggcctgcaaaacg
ataggccggagtagctatcggatgatagcccgccgcttcctgcaggagctctttctggaccttaattttgactcgttctacgtggagcctcagc
tcataatcggggcacgtaagttttccgctgaggaaaaagcagaggccacggcccccacggcaatgcaggccatgccgtttaagcaaac
gattctcaagcctaaaccggcggcccaactggctagcgtttatgagactagttgggagaacctgctgatggaccagtcgccacggataa
acaagtcatcgcctgccgatcaaccggccaatgctcaaacgaaagcaaaggcccggaaccagctgcacattgacgatacggagagtt
cgttgtccagtaccgatggcgaaaatgattccggtgaggatgtttgcgacggcagtggagccggcacccaggccagctcggtgaccacc
gctcatacagccctgcccacaggaaacagcaatcgtagcagcattggcacatgcacagcaagccagctggtgccaggtgcggcaact
tcaaactccgctggtgctgggacaagcgcatctgataatgctagtacaagtctaaagaatgaaaaccggcaattcacacgcggacgatt
ctatacgctggagctcgatttgagctgcaccaagaacaagtttcccatcaagaatcgcagccagcgaacaccaacgcctccaccaatcg
aaagtttaatcgagtccaagcccacggttcagacagatgtgaatacaagtgcgctgccaattatgcagcggaccggtggcagcaatgac
tcctcgtcagccctaagatattccactgtgaccaagagcttgtcggcatcgatggccaggccggtgggcagcctgtactgcgagaagcgc
ttgcagagctccaaatcggaagcggtcctcgatgaggtggcgaaggcgagaagagcatccggtggtgtgggactgacaactgtgcgtg
acaactcctag (SEQ ID NO:87)

**Protein:** AAF48942
**Protein GI:** 7293569

MASQHSSWRFGKRSKLQLRIKVSQDPEDFYRLDPQRSAAENAFEIYSMLCLEETRDTKR
LFLLNALASLCLGARKGSHHSNIRFTTEELLYCLSASLVHTFTQVRAAALRTIRYAISTPNDIKTFNAL
QLQHLLCRSIDLMLKNDDERVQALKLVRKMLAIAPEDISPAVVRCLVSLADSGIEENDNLLRACLAT
LAEFAVLNPALLIVCGGVTSITRNVLECHNPRIAESLCGVLLYLLEWPQTRNICGVRLDCLAAPYCD
FTYRQSIVDKNKDARELRYTSCRLALLSVLRSWTGTLEFCDPSKPSGLKAIVDALYLNQIEVRKAIL
DLLYELLTLPQPTWTDDYAVALQAVDPSDFQDTWLLSNGFVSAEGRSILPTLAARAPSVVEQHLAL
MLYCFLETGLLNALVEVVVSSDQFVSVQATVLIGKILQLMHTHLPPDICCTSPALPTLVSHATLGNQ
QANAAVAALQNYQKLLRQRPASRSLFLDSIIQGGALIQTRLFRRHLNVQEQAGPVLQLQETAEAAA
FAVPPVTQFRGTLDRSRLDSVSSSDESNSQASTSSRSSFRLKRKFLPQALYDNFRAFNRLLTDSR
VLSQADAHLWDWDVITTILKSNLIRKLDYTQGKFLKRLVDFYKPRNNRFSHQDLVPGQRLPTYVSA
GLDLIDVLLSSNELECMRFITDYFSDISQQLAAVTTSNRAHDCLFSPQHMNNTMCQQYFLYIGRMC
RTVKGIEVLKNTTVFEYLINLVRVTDHVCYVKLIVSGLNYSYEKLPRQVLEKALTSAKTRGRLYSTQ
FMAVLLRARLPHFEVWGIPLIINQTRDSDRSVGLAAMDVLEEACHDKYYLEEIVSRWPNLTHRGDA
GRLLMARYYSLPRGLNSTMARIEDEIRYWRNGYNKRYVLLVEADTHSSLTLHIRNEDGYYSRRNC
NQRPQTVPPNVAPHLYGQMAQTGQGMTALRKHGDLPQLLELLRRAKCTDDAECLELKAAIWALA
HASTHSNGIEYFVELNARLYEKLIVLVTKCEVYSVRATCFSALGLIAGTQAGANILFKLNWLSVRHD
KNTMWPVHQPEDWMSSQYTPVRHYYEDVPADNMTMMDDYIERFYETGSDFWHMLADQTDASG
GGGGVAGTVVGMHPIVQDPNATITTTDSVRTTTDDVHPRMLVGVAAKSKTLPEGSNLRQGKHQR
SLSESKTTDVISLLGSGVGTMSGAVLYPTPYQHRIRYNSCTDSNTSGVSSCESVTGRTAAAYAAA
ANELQQFPLSPIPSMSNLLESDELFRRNQLATSMLPSTLSPMAMKGYVQLRSLRKHSRPVFSESS
AEFYDFAEILDTPEVQMRKLDWTHSHRRLKVRSLDRQLSDVYRRLSADELNVLLPTLNAPKFLPP
NDLKGPCYAGICLPKNVLDLFPTRNLSRTYVSRDIQDQDIVGINLLNTMLRPQCLNDSLTNEGGDE
SSVISSLSDVSSASRRQTRQLQQGAKHSRSLCLHCARSPRQQRNDSGSHSNGGGGASLAPCEL
YSSAAAALVAAGIQAGPVLAKKSGSSAQGASAALGADISFHSPESMLSEDSLPDRLTASILYNVQR
LANPVSAKQSKMALLELKQKHPHAFQDICLYSEACKTIGRSSYRMIARRFLQELFLDLNFDSFYVE
PQLIIGARKFSAEEKAEATAPTAMQAMPFKQTILKPKPAAQLASVYETSWENLLMDQSPRINKSSP
ADQPANAQTKAKARNQLHIDDTESSLSSTDGENDSGEDVCDGSGAGTQASSVTTAHTALPTGNS
NRSSIGTCTASQLVPGAATSNSAGAGTSASDNASTSLKNENRQFTRGRFYTLELDLSCTKNKFPIK
NRSQRTPTPPPIESLIESKPTVQTDVNTSALPIMQRTGGSNDSSSALRYSTVTKSLSASMARPVGS
LYCEKRLQSSKSEAVLDEVAKARRASGGVGLTTVRDNS (SEQ ID NO:88)

# Figure 38

**Name:** CG10335
**Nucleotide:** AE003542
**GI:** 10727968
**Transcript:** CT29022
**Sequence:**

atggagcggaaactgcacagtggaatgcaccatgccacgctgcggcagctgcaggagtcgggctgcgagatagc
gccgcacaatctcatgtatccggtgttcatcgtgagcaacgacgacgatgtccagccaattgccagcatgccaggaatctcgagattcgg
actgaatcgcctgaaggagcacctggaaccgctggtggccaagggactgtcgtcggtgctgctctttggcgttgtggacccggacatgaa
ggatgagcaggcctccaatgcggattcagcaaagaatccagtcgttctcgcactgccgaagctccgggaatggtttccggatctgctgat
agcctgtgatgtgtgcatctgcccgtactcctcgcacggtcactgcggcctgctgggcgaaacgggtctggagaacggacccagcataa
agcggatagccgagatcgccgtggcttatgccaaagctggagcacacattgtggctccatcggacatgatggacaaccgggtgaaggc
cattaagcaggccctcatcgatgcccagatgaacagtgtatccctgctggcgtactccgccaagttcacatccaatttctatggacccttccg
ggaggcagcacaatctgcaccgaaattcggtgaccggaggtgctatcaactgcccagtggctccagaagcttagcaatgagagccatt
caacgtgatgtcgccgagggagccgacatgctgatggtgaaaccgggaatgccctatctggatatcctacgatccacaaaggattcctat
ccgtatcacacgctgtacgtctaccaggtttccggggagttcgctatgctgtatcatgccgccaaggcgggagcctttgacctcaaggatgc
cgtgctggaggccatgaagggctttcgacgagctggtgccgattgcatcatcacctactacacacccctttctgctggacattatcggaaagg
tcaagtaa (SEQ ID NO:89)

**Protein:** AAF49936
**Protein GI:** 7294597

MERKLHSGMHHATLRQLQESGCEIAPHNLMYPVFIVSNDDDVQPIASMPGISRFGLNRLK
EHLEPLVAKGLSSVLLFGVVDPDMKDEQASNADSAKNPVVLALPKLREWFPDLLIACDVCICPYSS
HGHCGLLGETGLENGPSIKRIAEIAVAYAKAGAHIVAPSDMMDNRVKAIKQALIDAQMNSVSLLAYS
AKFTSNFYGPFREAAQSAPKFGDRRCYQLPSGSRSLAMRAIQRDVAEGADMLMVKPGMPYLDIL
RSTKDSYPYHTLYVYQVSGEFAMLYHAAKAGAFDLKDAVLEAMKGFRRAGADCIITYYTPFLLDIIG
KVK (SEQ ID NO:90)

# Figure 39

**Name:** CG8070
**Nucleotide:** AE003834
**GI:** 10727693
**Transcript:** CT8068
**Sequence:**
accacacgtgcgtttactttgcgaaccaaacagaacgtggttgaaaataatcgtagtttttatactgttataacggctcac
catggtgcggcccaacaacaaccagctgccggagaaccttccgcagttgcagaacctcatcaagcgggatccggagtcgtatagcgat
gagttccacatccagtaccaacactttcttagcttgctggaagtttttgcgctgaatcccagcgaagaaaacaaatccctggatgacatcgtc
atgtttgtcgcccaggtggctcagtgctatccggccgtctgcgaggagttcccaaagcgtctttccgatttgctgaagaactatgccaccgttc
tggacccggctatgcgtaactgctttgtgaaagctctaatcctgctgagaaacaagaacctagtgcccgcgctggacatactggaactgtt
ctttcagttgctgcggtgtccggacaagaatctgcgcacctttctgcaaacgcacattgtgacggacatcaagaatatgaatgccaagcac
aaggacatgaagctcaattcgagcctgcaggcattcatgtactccatgctgaaggatgccaatccgaaggcagccaagatgtccgctga
tatcatgatcgagctgtacaagaaaaacatatggaatgattctaagacggtgaatgtcatagccactgtggggtgcttctctaaggtaacaa
aagtactggtcacctcgctcaagttcttcctcggccacgacgaagaggatgaagaggaggacactgacagcgagaacgaggtggacc
ttaagggtgctctgatggccaatcgagtcaataagaaaaccaagaagcggaccaagcaactggcgcagatcaaaaagcaagcggtg
aaggcccagaaaaagaaaaagaacgctccagcctttaacttctcaggcattcacctggtgcacaatccgcagggaatggccgaaggg
ctctttaagcagctgcaggccaccaacgagcgatttgaggtgaagctaatgcatttggacgttatttcccggctgatcggcatccacgacct
cttccttttcggtttctatccgtacataacacgctttctgcaaccccaccaacgccaggtgacacgtgtgctccagtttgccgcccaagcgtcc
cacgaactggttcccggagacatcatcgagcctatcctaaagacgatcgccaacaacttcattactgaacgcaactccagcgatgtgatg
gccataggtctgaatgcgacgcgtgagatttgcatgcgatgtcccctggccatgggcgaggatttgctgcaggacctggcaatgtacaaa
acgtacaaggagaagtcggtgatgatggcggcacggtcgctaatcacactctacagggaacagctgcctgccctgttgcacaaaaagg
accgaggcaggcagacagaggcccaggccgaacgaaaggttcgagcctacggagagcgcgaagtccacgacactgtcttgggtgc
agaggctctgctcaaggactctaagactattgacatagagagcgaagacgatacggactcaaacgatggcgtggaaagtggcgaaga
gtccgcaaaggccaagaaggaaaagaaagatatgaggatccttaaccaaaaggaggctgcacaggaactggctcttactcgcatatt
cacggacgaagatttcaagcgcatcaacgcggctaatcttaagaagaccgtaaccagtgcccgcaagagaccacttgaacaggatcg
cgccgagttcgtcaagctgaacagcatcgagatgatctacaagaagcgcaagcacgacaaggagtcgcggctggagactgtgcagg
cgggccggcaggatcgcgagcgctttggctggaaggatgggcgcgtgaacgagcactgttccaagacgaaccgcgagaagcgcaa
aaccaagaactttggcatgctgcgacacaaggctcgctccaaggttaagaagagcttcaaggacaagcagcaggcgctgagaaaac
atctgctgcatcagaaaaagatgaagtag (SEQ ID NO:91)

**Protein:** AAF58986
**Protein GI:** 7303942
MVRPNNNQLPENLPQLQNLIKRDPESYSDEFHIQYQHFLSLLEVFALNPSEENKSLDDIVM
FVAQVAQCYPAVCEEFPKRLSDLLKNYATVLDPAMRNCFVKALILLRNKNLVPALDILELFFQLLRC
PDKNLRTFLQTHIVTDIKNMNAKHKDMKLNSSLQAFMYSMLKDANPKAAKMSADIMIELYKKNIWN
DSKTVNVIATVGCFSKVTKVLVTSLKFFLGHDEEDEEEDTDSENEVDLKGALMANRVNKKTKKRT
KQLAQIKKQAVKAQKKKKNAPAFNFSGIHLVHNPQGMAEGLFKQLQATNERFEVKLMHLDVISRLI
GIHDLFLFGFYPYITRFLQPHQRQVTRVLQFAAQASHELVPGDIIEPILKTIANNFITERNSSDVMAIG
LNATREICMRCPLAMGEDLLQDLAMYKTYKEKSVMMAARSLITLYREQLPALLHKKDRGRQTEAQ
AERKVRAYGEREVHDTVLGAEALLKDSKTIDIESEDDTDSNDGEWVNVAHSDGEGGGADDDEED
EDEDEDDDDEDEDEENSNDEENEDEDNSDEGVESGEESAKAKKEKKDMRILNQKEAAQELALTR
IFTDEDFKRINAANLKKTVTSARKRPLEQDRAEFVKLNSIEMIYKKRKHDKESRLETVQAGRQDRE
RFGWKDGRVNEHCSKTNREKRKTKNFGMLRHKARSKVKKSFKDKQQALRKHLLHQKKMK
(SEQ ID NO:92)

## Figure 40

**Name:** CG7460
**Nucleotide:** AE003523
**GI:** 10727853
**Transcript:** CT22955
**Sequence:**

atgagttccgcgtcattgcgtctgcttagtaaaactaggcaaaccgctcgcattgtgatcgtgggcgcgggatccgctg
gaatagctgcggccaccaggcttctggatctcggtttccgaaatgtgctcctcctcgaggcggaagatagaatcggcggtcgcgtccaca
cgattccctttgccgataacgtcgtcgacctgggcgcccagtggtgccatggcgagaaaggaaatgtagtatacgacaaggtcaaggac
ctgaacctcctggaggtcacggaaccgcactatgaaacctttagatgcgttcgatccaacaaagaagtcctacccgacgacctagcaga
tcaactgaaaactatagctgatatgtcaataccagaccggcaggcagagctcctggacttcgagggttcgttgggagactacatcaatatg
aagtactggaaggagctggccaagcttccgcccatagatcgtaccattgcggaggagttcctggaggtgtttcacaaattcgaagcatcc
gtggaggcagcggatcacctgtttgaagtatccggaaagggtcacttggagtactggctatgcgaaggcgagctgctcctcaattggaag
gacaagggatacaaaagattcctcaaactactcatgaaagcgccagaggatcagtccgaagatcttggtatcctcaaggatcatgtgcg
gctaaacagacgtattgctgagattaactggaaaggagctgatgagcttaccgtgcgttgctggaatggcgaggtaatcacagcggatca
tgtcatatgcactgtttccttgggagttctgaaggagcagcatccgaaactttttgttcccgccctgccagctgccaaatttgagaatcctccgc
tgccaggcgattggcctggcttcaattgtctttggctaaaggaggacctggaggagctgcgggcttcggaactcttttggctggagagtgtgt
tcggcttttatccagtctccaggcagccacgcatcctgcagggctggatcattggcccacacgcccggcacatggaaacgctcaccgag
gagagggtcctggagggtctgctgtggcttttccgcaagttcctgccatttgaaactgcccatccagtgcgaatgctgcgcactcaatggcat
gcgaatcccaacttccgaggcagctacaccttccgttccacctacacggatgcgctgcgaaccggtgcttgggatctggaagctccgctc
caggatgtgtgcggcaggccacgcctccagttcgccggagaatccacccacaagcacttctactcaacggtccatggagccgttgagac
gggatggcgggaggcggaacgattgcacctctactaccgggcgcgaacatctcagctatga (SEQ ID NO:93)

**Protein:** AAF49310
**Protein GI:** 7293951

MSSASLRLLSKTRQTARIVIVGAGSAGIAAATRLLDLGFRNVLLLEAEDRIGGRVHTIPFAD
NVVDLGAQWCHGEKGNVVYDKVKDLNLLEVTEPHYETFRCVRSNKEVLPDDLADQLKTIADMSIP
DRQAELLDFEGSLGDYINMKYWKELAKLPPIDRTIAEEFLEVFHKFEASVEAADHLFEVSGKGHLE
YWLCEGELLLNWKDKGYKRFLKLLMKAPEDQSEDLGILKDHVRLNRRIAEINWKGADELTVRCWN
GEVITADHVICTVSLGVLKEQHPKLFVPALPAAKVRAIEGLKLGTVDKFFLEFENPPLPGDWPGFN
CLWLKEDLEELRASELFWLESVFGFYPVSRQPRILQGWIIGPHARHMETLTEERVLEGLLWLFRKF
LPFETAHPVRMLRTQWHANPNFRGSYTFRSTYTDALRTGAWDLEAPLQDVCGRPRLQFAGEST
HKHFYSTVHGAVETGWREAERLHLYYRARTSQL (SEQ ID NO:94)

# Figure 41

**Name:** CG17735
**Nucleotide:** AE003605
**GI:** 10727172
**Transcript:** CT28225
**Sequence:**

atggcacaacaaccgccaagtggaccgccgcatgcaggtggacagcaaataacgccaggtgcaaactctgcaa
atctaagcattgtagcggccgctttgagcgccgctcgtgacgtcgggggaggatccgacggaggaggatcagcgggaggcgcaacgc
cagctactggagcatcggcttcatccgtaggaaacaccagcgccgtgggcgcgtcgagcagcagcaacagcagtgccggccaagca
gccagcagcaacagcaacaatgtgactgcaacaggatcggggtcggctcctggcggaggacccacgagtaccgggacgaccagtg
gcactcagcatggtagtggatcgggggcagctgcagccgttgattcggaaagcgatgacagtgaggtcggtcgactacaggctttgctcg
aagcccgaggactgccgccccatttattcggtgcccttggcccaagggtgacacatattctccaccgaaccattggtaacagcagcagttc
caaggccaatcaacttctgcaaggattacaatcccacgacgaatcccagcagctgcaggctgccattgaaatgtgccagatgctcgtgat
gggcaatgaggacaccctagccggttttccaatcaagcaagtggtacctgctctaatacaactccttcgtatggagcacaactttgatataa
tgaacaatgcatgtagggcattggcatacatgctagaagctttgccccgctcgtcaggcaccgtggtggaggcggttccagtctttctagag
aaacttcaggtcatccaatgcatggatgtggctgagcaaagtttatcggccttggagattctgtcacggcgtcacaacaaggccatcctgc
aggcaaacggaatttcggcctgcctcacctatttggacttcttttcgattgtggctcagcgggcagcattggcgattgcggctaattgctgcct
aaacatgcatccggaggaattccactttgtggcggaaagcttgcctctactagctcgcttgctctcccagcaagataaaaaatgcattgaa
agcgtttgttctgcattctgtcgtctagtagagagtttccagcacgacggtcagcgcttgcagcaaatcgctagtccggacctgttgaagaac
tgccagcaattactgctggtaacaccagccatcctgaatacaggaaccttcacagccgtcgttcggatgctaagtctaatgtgttgcagctgt
ccagacttggcaatttctttactaaggaacgatatagcggccaccctgctttatttgctcaccggaaatgcagagccggctgctgccagtgct
acccacgtggagctgatctcacgctcgccttcagagctgtacgaacttacctgcctcattggggagttaatgccgcgcctacctttggatgg
aatctttgctgtggactcactgctggacagaccaacgcttaatacccaggaccaggtgcattggcagtggcgcgatgatcgcggctcctgg
cacaactattctacaattgattcgcgcctgatcgaggccgccaaccagagcagcgaggacgagataagcctaagcacctttggacgca
cctacactgtcgatttcatgccatgcagcagataaatgaggacacgggcactacacgacccgttcaacgtcgtcttaaccataactatgtg
gctcccatgtctgcgggccaggacttgactacaacatcggctggatcggcggccgcgggaggagcttccacgtccgctgcagcagcgg
cagcgtcctccaacaacaataacaataataataataatccaccaggcaatagtgtcaacctcaatcaagtaaagcgccgtccatctctgg
atgctagaatcgcatgccttaaggaggagcggggtctggctgcggactttataaaacacatctttaacgtactgtacgaagtatacagctct
tctgccggaccaaatgtgcgttacaaatgcctgcgcgcccttctgcggatggtgtactatgccacgccggagttgttgcgtcaggtgctgaa
gtatcagctcgtctctagccacattgctggcatgctaggaagcaatgatttgcgcatcgttgtgggagctcttcaaatggctgagatcttaatg
cgccaacttcccgatgtctttggcacgcactttcgtcgtgagggagtgatctaccagtttactcagctcactgatccaaataatccgatatgcg
cgaatccctcgccgaaaccgcttagcgcaacagcaacgcccaccgcaaatgcaggcggatcacaatcggctcctgcctccgccaaca
gcctgcaagtaaatccatttttcatggatagtgctccgggtttgtcgagtgcttccacgactcccagcagtagcaagcatcaatcgtacagtg
tgaagagcttctcacatgccatgaacgccctaacagccagcgccaaaggaactccttctggagcgttggacgccacaagctcatctacc
acagctggtggttataattacagcagctcggcaccgtcttcttcctccggagcacccgctgcctactttgtgacccaacagggagatccacg
ccagtacgtccactttcagcagccggccgttccagcaccaccaccgcagcaggaactgttaccatcaggagttcagcaacaagggcag
caggtgcctcaggtgatctaccagccacatcatcaacagcctgcacacttagtgctggcctccaccagcagtggcgctgcctcgtcgtctt
cgtcttcgtcctcttcgtcctcggcctctgcactgcagcacaagatgacagacatgctaaagcgaaaagcgcctccgaagcgtaaatcgc
aaagcggtggcagggcaaaatctagacaagaagatgcagcagtcgcaccagcgggatctggaccagggggagcgcctccttcttcat
ccggctcagctatgcatgaacttcttagccgtgctacaagtcttggtagcgggaacggtggcaggagtacgccaaattcgggaggtggct
ctggaagctccaagtcccggtttaacgccggaaactcgtccaatgccggatcgagcaaatcttcgttcttggcatcgcttaatccagctcgc
tggggccgtcaaactcatcaaaatcatcaccatcaccaccagcagtcccagcagcagcatcatggtctttctaaagattccggcaactcg
aactcaacaggttccggctccggagctggtttagcatacacggtgagccaacatggcgctggtagtggagccggaggactgaacgctg
cagccgttgcggcgagtataagtaagagcatttcccacgccaatctcctagcggcggccaacagagagagggcgcgtcagtgggtccg
cgaacaggctgtggattttgtaaagcgttacactgagcaggaggccaaaaggagcaaggcggcatccgagagtggtgccactcagag
cgggagtagtggagtgggattatcgtcgacgggcaatactccattgtccacagcgggcagtaccaatgtcctagagcgtttgtccagcatt
ctgtttaagcttaacgggagctaccacgactgtttagatgctctcctcgagctaaagaccattcttttggagagtgacatctctccctttgaggt
aaatcattcgggcctgatcaaagccatgctcaactatatgactagcgagactggactggttgagcgcgatgcacgtctgcgcagctttatg
cacgtattcgctggccttccattagagcctctgctgcaaaacgtcggccagatgccaactatcgagccgatagccttcggagcctttgtggc
caagctaaatggttgtgtcacgcaactagaacagtttcctgttaaggtacacgactttcctgccggtcctggtggaaggtccaatcagagtg
ctctgcgtttcttcaacacccatcagcttaagtgcaacctgcaacgccatccgcagtgcaataacctacgtcagtggaagggcggaactgt
aaagatcgaccccttggccatggtccaggccatcgagcgttacctggtggtgcgcggctatggtggcattcgtgcagactccgacgacga
cagtgaagaggatatggatgacaatgtggcggcagtggtgttgtcccaggcgagctttagacacaagttgcagtttacgattggggatcac
gtgctgccctataacatgacggtttatcaggcggtcaagcagttctcgccactggtcagtgaacaacctgagacggacaacgagtcgga
gactcttttaggaaacgctagtatatgggtgcagcagcatactattcactatcgtcctgtagaggaggaagtgacttcgggagcggctgcg

ggagcagcatccagcagcagttcgtgtagcagtggcgtgcaaaaacagcagagctcttcaagctccgcatccagctgtgtaaacgctac
ctcttcgtgctcctcatcttctggagtggcaagcggtggcgggtcctgatgttgtgaccgtacaagacgcctcattggatgcactgtgtatgct
gcgagttattcatgctctaaatcgtcactgggaccatctctacggttgtgtggtgcgccaaaacattattccccagtcagactttatacatccta
agattatggccaaggcgaatcgtcagctgcaggatccgcttgtcatcatgacaggcaatctaccacagtggcttccccagataggcatgg
cctgtcccttcctcttcccattcgagactcgtcacctgcttttctacgcaaccagctttgatcgggatcgcgctttgcagcgtctgttggacactac
ccccgatcttaatgcagcggagtcttcagagcgcgtggctcctcgccttgatcgccgcaagcgagcgatttcccggacagagatactcaa
acaggcagagcacatcctacaggactttgggcactcgaaggcactgctggaaattcagtacgagaatgaggtcggaacgggtctggg
acctacattggaattctacgctttggtttctgctgagctgcagcgtaccgaccttggcctatggaatggcagtgatagctacaaacagaactc
tgtgaccattgtagacgtagtcaaggccaacagcgctgtgctgcacatcgaggatgctcttgaggcaaccaccacggaccagaacactc
cagcagtagcaggagcgtcgcttgtgagcagcagcaccaccactacaacaacaaccgcacaacaacaccagcatccgcccacacg
ctctagcagtcgttcgcacgtcttgcgtagcggagctggccaacagccggtggagcacagctcctctagcgccggcgccaatgagaatg
ctttaaatatggttatagcacagcaatttagtgatacaaactctgctaacccagcagcgattgataatcccagcagcaccactaccgcaac
aacggttgtacagcataataccactacgaataactcaagcattatcacgactacaaccacgacgagttatgtgcatgcggtgcatggcct
gttccctttgcctctgggtaaatcttcgaagcttccgcagatgactaaggccaaggccaaattcaaattcttgggaaagttcatggcaaagg
cggtgatggacagtcgcatgttggatttgccattttctttgccattctatcgctggttagtcagcgaagagcattcaatcggcctggccgatctg
atgcgtgtggccccggaggtccaaaacactctagtgcgactccaagacttggttcgccagcgggagtacattctatccgatccaaacatc
gatgccatggaaaagactgaaaagattgaacagctagacttggatggctgtcccattgctgacctaggcttggactttgtattgcccggac
atgccaatatagagttgtgtcggggcggtcgcgatacacctgtcactgtgcacaaccttcaccagtacatctccctggtcacatactggttcc
ttatcgagggtgtccagaaacagtttgaggctttgcgtgaaggttttgattccgtttttccaatccaacggttgcgaatgttctatccagaggagt
tggagtgcgttttctgtggatcgggcagcgagcagcagcattcgcgatgggaaatcaagatgctgcaggagagctgccgcactgatcat
ggattccaccaggactcgcaagctatccaatatctgtatgagatcctggcctcttacaaccgcgacgaacagcgcgccttcttacagtttgt
gactggatcaccacgccttccgactggaggattcaaggcccttacgccaccactgactattgtacgcaagacgttggatgagaaccaaa
accctaacgattacttaccatctgtgatgacctgtgtcaactatctaaagttgcccgactactctagtcgcgaggtgatgaggcagaagctg
aaagtggctgctaacgaaggcagcatgtctttccacctctcataagcaagcaacaaaccaaaacccaaccgaaccccgctttccacaa
acaaccagatttacagcaacacaaatcggaaactacaaaaatactacatggaatatattaaggagggtatattccggatggattaattgg
atgggggtcctatttgcatttttctttcgatcgttagtttcgccgcgcgcgttttcccaagctccttaaattttcatttggcaagcgcgtgtcatact
aaatttcaatataaatatataagtataatatatttttattaccaagttttgcaaaaaagttaaacggaaacacggataacatcgagaaagaa
aataatgatggacctctcacacaaaatgttacctttaaaaaatttgaatagta (SEQ ID NO:95)

**Protein:** AAF52092
**Protein GI:** 7296816

MAQQPPSGPPHAGGQQITPGANSANLSIVAAALSAARDVGGGSDGGGSAGGATPATGA
SASSVGNTSAVGASSSSNSSAGQAASSNSNNVTATGSGSAPGGGPTSTGTTSGTQHGSGSGAA
AAVDSESDDSEVGRLQALLEARGLPPHLFGALGPRVTHILHRTIGNSSSSKANQLLQGLQSHDES
QQLQAAIEMCQMLVMGNEDTLAGFPIKQVVPALIQLLRMEHNFDIMNNACRALAYMLEALPRSSG
TVVEAVPVFLEKLQVIQCMDVAEQSLSALEILSRRHNKAILQANGISACLTYLDFFSIVAQRAALAIA
ANCCLNMHPEEFHFVAESLPLLARLLSQQDKKCIESVCSAFCRLVESFQHDGQRLQQIASPDLLK
NCQQLLLVTPAILNTGTFTAVVRMLSLMCCSCPDLAISLLRNDIAATLLYLLTGNAEPAAASATHVEL
ISRSPSELYELTCLIGELMPRLPLDGIFAVDSLLDRPTLNTQDQVHWQWRDDRGSWHNYSTIDSR
LIEAANQSSEDEISLSTFGRTYTVDFHAMQQINEDTGTTRPVQRRLNHNYVAPMSAGQDLTTTSA
GSAAAGGASTSAAAAAASSNNNNNNNNNPPGNSVNLNQVKRRPSLDARIACLKEERGLAADFIK
HIFNVLYEVYSSSAGPNVRYKCLRALLRMVYYATPELLRQVLKYQLVSSHIAGMLGSNDLRIVVGA
LQMAEILMRQLPDVFGTHFRREGVIYQFTQLTDPNNPICANPSPKPLSATATPTANAGGSQSAPA
SANSLQVNPFFMDSAPGLSSASTTPSSSKHQSYSVKSFSHAMNALTASAKGTPSGALDATSSSTT
AGGYNYSSSAPSSSSGAPAAYFVTQQGDPRQYVHFQQPAVPAPPPQQELLPSGVQQQGQQVP
QVIYQPHHQQPAHLVLASTSSGAASSSSSSSSSSSASALQHKMTDMLKRKAPPKRKSQSGGRAK
SRQEDAAVAPAGSGPGGAPPSSSGSAMHELLSRATSLGSGNGGRSTPNSGGGSGSSKSRFNA
GNSSNAGSSKSSFLASLNPARWGRQTHQNHHHHHQQSQQQHHGLSKDSGNSNSTGSGSGAG
LAYTVSQHGAGSGAGGLNAAAVAASISKSISHANLLAAANRERARQWVREQAVDFVKRYTEQEA
KRSKAASESGATQSGSSGVGLSSTGNTPLSTAGSTNVLERLSSILFKLNGSYHDCLDALLELKTILL
ESDISPFEVNHSGLIKAMLNYMTSETGLVERDARLRSFMHVFAGLPLEPLLQNVGQMPTIEPIAFG
AFVAKLNGCVTQLEQFPVKVHDFPAGPGGRSNQSALRFFNTHQLKCNLQRHPQCNNLRQWKGG
TVKIDPLAMVQAIERYLVVRGYGGIRADSDDDSEEDMDDNVAAVVLSQASFRHKLQFTIGDHVLPY
NMTVYQAVKQFSPLVSEQPETDNESETLLGNASIWVQQHTIHYRPVEEEVTSGAAAGAASSSSSC
SSGVQKQQSSSSSASSCVNATSSCSSSSGVASGGGSLTKKAHKSSSKFMRKKTELWHEGIAPVV
ISALKPFLSSSLPADVVTVQDASLDALCMLRVIHALNRHWDHLYGCVVRQNIIPQSDFIHPKIMAKA
NRQLQDPLVIMTGNLPQWLPQIGMACPFLFPFETRHLLFYATSFDRDRALQRLLDTTPDLNAAESS

ERVAPRLDRRKRAISRTEILKQAEHILQDFGHSKALLEIQYENEVGTGLGPTLEFYALVSAELQRTD
LGLWNGSDSYKQNSVTIVDVVKANSAVLHIEDALEATTTDQNTPAVAGASLVSSSTTTTTTTAQQH
QHPPTRSSSRSHVLRSGAGQQPVEHSSSSAGANENALNMVIAQQFSDTNSANPAAIDNPSSTTT
ATTVVQHNTTTNNSSIITTTTTTTSYVHAVHGLFPLPLGKSSKLPQMTKAKAKFKFLGKFMAKAVMD
SRMLDLPFSLPFYRWLVSEEHSIGLADLMRVAPEVQNTLVRLQDLVRQREYILSDPNIDAMEKTEK
IEQLDLDGCPIADLGLDFVLPGHANIELCRGGRDTPVTVHNLHQYISLVTYWFLIEGVQKQFEALRE
GFDSVFPIQRLRMFYPEELECVFCGSGSEQQHSRWEIKMLQESCRTDHGFHQDSQAIQYLYEILA
SYNRDEQRAFLQFVTGSPRLPTGGFKALTPPLTIVRKTLDENQNPNDYLPSVMTCVNYLKLPDYS
SREVMRQKLKVAANEGSMSFHLS (SEQ ID NO:96)

# Figure 42

**Name:** Gycalpha99B
**Nucleotide:** AE003770
**GI:** 7301790
**Transcript:** CT3044
**Sequence:**

cattttcaggcgtgcgaaaaaattcctcagcgctccaagaatttccacagcaattttggtcactgctttaaagtgagcat
aaagccaagaggccagcgaaagaatttttatttcttaaccaaatcgaatagaggccacccgagaggcgagcacgcgacatggcgtgt
cctttcttcaggcgtgcggactcgctgacacgccagccctcggtcatcgccgagcctggtggccactgggcgctcgaggacgaggagct
ctcggacgacgccctgaccctcacccacctccagatggccatccagctgctgacggcgccgtcgaacgaggatctcaacacggctgta
acgtctttggtggccaagtaccgccagaactggcccaatatccacaagctcaaactggatccgcaaaccttcaagagctgtgccaactat
gactacttggcggacatccaggagctgctgctgaagatggacgaggccagcgccagcgagattctggtgctgctcggcgaggagctga
tcacctgctgctgcacaggtatcattgaaagagccttccgctgcctgggcaccgatctgcaggaattcctgggctccctcgatggcgtttac
gatgtgctgaagctgcaggaggaggacgtcaccgataccggattcgtgtgtgccggcgagggcgagctgatcttcacctcggagcgcc
cggtcattgcctggttgctcctgggctccctgaaggcgctcacccgcatgctctacaaggtggacgtgaacatcaagatcgagcccgtgg
agggcgatgcccggcggtatcgctacctcttctcgctggtcaaggacaactcccagaccatgctgatgggtcgtcccacttccgtgagcaa
aaccatcccggaaacggtgcagcggagcaactcgagcaacgcctccgacctgcaaatgaactcgtccagcttctgcaagatgtttccct
ggcacttcatcatgaacgagcagctggaactggtgcagttgggcaggggattcagcaaattgtacaagccctacatggcggactttggtt
gccaggcgaccacctactttgatttcaaacgacccaagggattgaccatgaaattccgggacatcgtgaggcgcacctacacgcctttcc
tgatcggtttgaataatccgccgggcgctgtggatttcccggccattggactggagatcaagggacagatggtgcactgtcccgagtccaa
ttcgttgctcttcattggatccccccttcctggacggcctggatgggttgacctgcaacggactcttcatctcggacataccgctgcacgacgcc
accagggaggtgatcctggtgggcgaacaggctcgggcccaggatggactgcgccggcgcatggacaagatcaagaacagcattga
ggaggccaactcggcggtgaccaaggagcggaagaagaacgtcagtctgctgcacctcatctttcccgcggagatcgccgagaagctt
tggctgggctcctccattgacgccaagacgtatcccgatgttacgatcctgttcagcgacatcgttggcttcactagcatctgctcgcgggcc
actcccttcatggtgatcagcatgctggaggggctgtacaaggacttcgacgagttctgcgacttcttcgacgtgtacaaggtggagaccat
cggggatgcctactgcgtggccagtggactccaccgagcctccatctacgacgcccacaaggttgcctggatggccctgaagatgatcg
acgcctgctcgaagcacattacccacgacggcgagcaaattaaaatgaggatcggcctgcacacgggcactgtcttggcgggagtggt
gggcaggaagatgcccaggtattgcctcttcgggcacagcgtcaccatcgccaacaagttcgagtcgggcagcgaggcgctgaagatt
aatgttagcccaaccaccaaggattggctgaccaagcacgagggctttgagtttgagctgcagccgcgggatccatcgttcctgcccaag
gaattccccaatcccggcggcaccgagacctgctacttcctggagagcttccgcaacccggctttggacagcgagctgccgctggtgga
gcacatcaatgtgtccatgaagaccatatccgagggcggcgatgcctagaccccccgtagctccaatccccccgtagaccacccacac
gctcagtagtcctagttcggtgcacttgtattgtggttttgcctgttgcctgcttagcctagaatcaataaaatatcccattcgaatggttgcatttc
agcatagagatgaacacattttattggtt (SEQ ID NO:97)

**Protein:** AAF56917
**Protein GI:** 7301807

MACPFFRRADSLTRQPSVIAEPGGHWALEDEELSDDALTLTHLQMAIQLLTAPSNEDLNT
AVTSLVAKYRQNWPNIHKLKLDPQTFKSCANYDYLADIQELLLKMDEASASEILVLLGEELITCCCT
GIIERAFRCLGTDLQEFLGSLDGVYDVLKLQEEDVTDTGFVCAGEGELIFTSERPVIAWLLLGSLKA
LTRMLYKVDVNIKIEPVEGDARRYRYLFSLVKDNSQTMLMGRPTSVSKTIPETVQRSNSSNASDLQ
MNSSSFCKMFPWHFIMNEQLELVQLGRGFSKLYKPYMADFGCQATTYFDFKRPKGLTMKFRDIV
RRTYTPFLIGLNNPPGAVDFPAIGLEIKGQMVHCPESNSLLFIGSPFLDGLDGLTCNGLFISDIPLHD
ATREVILVGEQARAQDGLRRRMDKIKNSIEEANSAVTKERKKNVSLLHLIFPAEIAEKLWLGSSIDA
KTYPDVTILFSDIVGFTSICSRATPFMVISMLEGLYKDFDEFCDFFDVYKVETIGDAYCVASGLHRA
SIYDAHKVAWMALKMIDACSKHITHDGEQIKMRIGLHTGTVLAGVVGRKMPRYCLFGHSVTIANKF
ESGSEALKINVSPTTKDWLTKHEGFEFELQPRDPSFLPKEFPNPGGTETCYFLESFRNPALDSELP
LVEHINVSMKTISEGGDA (SEQ ID NO:98)

# Figure 43

**Name:** CG13893
**Nucleotide:** AE003468
**GI:** 7291959
**Transcript:** CT33427
**Sequence:**

atggatgatgcgttggttggaacccacgacgattacttcttggtgcgctggctaagagctcgaaaatggaatctggagg
cagcggagaaaatgctgagagctagtttgaaaacacgcgccatgtggaatgtggacaacatcgagaagtgggatccaccgaaggcat
tgcaggagtatctgccatacggcctcatgggctacgacaacgaaggatcaccggtgctggtctgcccctttgccaacttcgacatgtgggg
catgatgcactgcgtcacgcggtttgagttccaaaagtacctggtcctgctgctcgagcgcttcatgaagatcgcctacgatcagagtcaaa
agcacggatggcgagcccgccagctggttgtatttttcgacatgcaggatgtgaacctgaagcagtacgcctggcgaccggctgccgaa
tgtgtgatctctacggtgaagcaatacgaggccaacttcccggagctcctcaagatgtgctacatcatcaacgcacccaagctcttctcggt
ggcgttcaacattgttaagaaattcctggacgagaacaccaccagcaagatcgtgatctacaaatccggagtggaccgctggcaggag
caactcttctcccatgtgaaccgaaaggcatttccgaaggcctggggcggcgagatggtggacaggaacggagatccccagtgcaag
gcactgatggtctggggaggcaagctgccggaggagctttacattgatcagagtagccagcagagcgatcgagactttgtggaggccc
aggttcccaagggcgacaagttgaagctgcactttaaggtcaacgttgaggagcagaagatcctctcctgggagttccgcacctttgacta
cgacatcaaattcggcatctacagcgtggacgacaagacgggcgagaagcgcagcgaggtgcccctgggaaccgtgtactccaacg
agatggacgagattggttacatctctacacgaccgaacaccacttacacggtggttttcgacaactccgccagctacctgcgcagcaaga
agctccgctactgggtggacctcatctctgaggaggaggagggcatatccgagctgaccacccagatggacaacactcagattgcgaa
tcagcagtga (SEQ ID NO:99)

**Protein:** AAF47396
**Protein GI:** 7291980

MSGPLPEISEEQRAILEKFRKQMDDALVGTHDDYFLVRWLRARKWNLEAAEKMLRASLK
TRAMWNVDNIEKWDPPKALQEYLPYGLMGYDNEGSPVLVCPFANFDMWGMMHCVTRFEFQKY
LVLLLERFMKIAYDQSQKHGWRARQLVVFFDMQDVNLKQYAWRPAAECVISTVKQYEANFPELLK
MCYIINAPKLFSVAFNIVKKFLDENTTSKIVIYKSGVDRWQEQLFSHVNRKAFPKAWGGEMVDRNG
DPQCKALMVWGGKLPEELYIDQSSQQSDRDFVEAQVPKGDKLKLHFKVNVEEQKILSWEFRTFD
YDIKFGIYSVDDKTGEKRSEVPLGTVYSNEMDEIGYISTRPNTTYTVVFDNSASYLRSKKLRYWVD
LISEEEEGISELTTQMDNTQIANQQ (SEQ ID NO:100)

# Figure 44

**Name:** CG18176
**Nucleotide:** AE003551
**GI:** 10728019
**Transcript:** CT41038
**Sequence:**

tgaaaacctcaataaacacgatttatctgcgataaatgtgagtgaggaaacctgtaaccatgtctcacctgaccggca
cccgcgtgagcaccttcaacgagagcttccttaacgaaaatgagcacgactcgaatgcggtgctcatggaactggacaagggactgcg
gagcaccaagcagggaatccagtgcgaggcggtcgtccgctttccgcgcctcttcgagaagtatccgtttcccattctcatcaactcgtcgtt
cattaagctggccgattactttgtcagcggatcgaatctgctgcgcttctgggtgctccgtgtttgccagcagagcgagaatcatctggacaa
gatcctcaacattgacagctttgtgcgctgtatctttgtggtgatgcactccaacgatccggtggcgcgtgctctcctcctgcgcaccttgggc
gccgtttcccgtgtgattcccgagaagcaacaggttcatcatgccattcgacgtgctctggatagccacgataccgtcgaggtggaggctg
ccatctatgccagcagctgctttgccgcccagtcaagttcctttgccatcagcatgtgcgccaagatttcggacatgatcgaatcgcttcagg
tgccggtgccaatgaagctgctcctgattcccgtgttgcgacacatgcatcacgaggctaccacagcgtcactggttagcagactgtgcat
ggatctgctgcccaaatatccagcccagagctttgtggtggccatcatagacacgctcactcaattgtcatcacgaacgctggtgggagtg
cccggtcagctggatgttctgctggatttcatgcaggacttaaggacaccagtacgcatacaggtgctgcgatctttcaacgagttggccgg
acgtcagagcgttcatgcctggcccaagccagccatcaaagcgctcattgatcgttttgaattgtgtaccaattccaaggagcagttcctctt
cctctcaatcctgctgaagttaagcgagtgcccgcttacttgccagcagctgcttcgcgagcatcgcgtagccctgctccgtttgtgcatcca
gtgcattagcaagctggacgactacaccaccgccactcaggccatggcagttttgtcggttctagtagcatttggtttaaaaaaaaagggc
agcggcgagcaggtggacgatattcttcatatggtcaacctgcacatggaaggattgctcctttgcacggccaagcggtcggagtgcacc
cgcgatctccgtagggtgctgacatacggtataaggatcactaaagccaacgctgaatttggaacatccttcattgggatcgtgaccaata
gcctgggagataagggtgcctatccgccagccaatgcggaacttatgtgcgaggcactggcgggtctgtgtgagcactttcagctgcgta
agtacgccttttccaccgcagaagatttgatagtagacgaaaacgcgatggacaccgatgagttgccccgcccaagataaatcccatg
ctagctcgtttgccgctcattctgcacaagctgaacaccattatcgaccaggagaattgcgatcaacagttgcgcagcgtggagatcctga
gttctctggttctacaaaccaccatgggctgttacctgccgcaaaaggttgttcagtgctttgagaaatgcctcggtaggcttaactgctggac
gctgtataggattgcacgcactgctagtcgctatggtcaccactatgtggctgctcacatttacaccaaggtctcccagattgtgatcagcgat
catatgcactatttcctggtggcactgtcgcagatctcgcaggcggagtgcattcttaactatggcttggaatacgcctacatgcgggacaat
tatgcaccaaaggtggcaccggaacctctaattccgctgatgaagcgcttggaaatggctagtaatctctatcagcaagcgctggccagc
ctgcgagctggctcctcgccccagcatccgtgcacctttcagttggagtatctgaagataagggcgcagtttctgcagacccttcacctggct
gtgaccgtaaagaatgcccaggtgattgtgccgccaccggcaatagctggaagtttggcccagaactcgcgcgactatctgcaaaagttt
ggtcatgtcacgaaccagctgcgcaagctggtgaaggcactaaaggcctgcgaggagacttacgctaggctatacaaatcctctttcgat
gcggatcacgtgacgctggagtttctggaggtggctgaatttcagtgcgctcttttcgcccacatcatcgagtcgatttgctacgccacgcctc
cggaaccgcctgtatttctaaccacaggcgatcaccccggagacgcgctactttgccgcaagctgccagcgcatggagcagatgcaaaa
aaacctgccacaggagccggccaatgccaagaccattagtaaccgacatttggatgtcatcattgcgcagatcgagatcataacaaag
acaccgctctgcctgccgcgttacttcttccagattctgcagtccacacagatcaagctatcggtgagtccgcagccaaggagcgccacc
gaacccgtgaatgtccagtcgggcagtaatctggttatcaaggtcgagggtgtcctgcagcactttagcaagcagaagaaacacttccgt
cgcgtggagtccgttcaattaagcctcacctcacagctaatcacgccgccaccgcgatcctcgcaagagctgcccaagcagggcgcca
acgatactgtgaccctcaatcagattgtaaagccccagcgcgatttcctctccggcagcttcctgttgcccatctccaatggcgggcacttcc
aggtgacgctggaaacattcgtggtggacgagaatggcattacctggtgcactggccccaaatcctcgatggtggtgcgggtgctggag
gatcctagcaagcagggcgctccagcgccgagcacttcccaggcagtgggacagacgaggaggttttaa (SEQ ID NO:101)

**Protein:** AAF50214
**Protein GI:** 7294884

MSHLTGTRVSTFNESFLNENEHDSNAVLMELDKGLRSTKQGIQCEAVVRFPRLFEKYPFP
ILINSSFIKLADYFVSGSNLLRFWVLRVCQQSENHLDKILNIDSFVRCIFVVMHSNDPVARALLLRTL
GAVSRVIPEKQQVHHAIRRALDSHDTVEVEAAIYASSCFAAQSSSFAISMCAKISDMIESLQVPVPM
KLLLIPVLRHMHHEATTASLVSRLCMDLLPKYPAQSFVVAIIDTLTQLSSRTLVGVPGQLDVLLDFM
QDLRTPVRIQVLRSFNELAGRQSVHAWPKPAIKALIDRFELCTNSKEQFLFLSILLKLSECPLTCQQ
LLREHRVALLRLCIQCISKLDDYTTATQAMAVLSVLVAFGLKKKGSGEQVDDILHMVNLHMEGLLL
CTAKRSECTRDLRRVLTYGIRITKANAEFGTSFIGIVTNSLGDKGAYPPANAELMCEALAGLCEHF
QLRKYAFSTAEDLIVDENAMDTDELPPPKINPMLARLPLILHKLNTIIDQENCDQQLRSVEILSSLVL
QTTMGCYLPQKVVQCFEKCLGRLNCWTLYRIARTASRYGHHYVAAHIYTKVSQIVISDHMHYFLV
ALSQISQAECILNYGLEYAYMRDNYAPKVAPEPLIPLMKRLEMASNLYQQALASLRAGSSPQHPCT
FQLEYLKIRAQFLQTLHLAVTVKNAQVIVPPPAIAGSLAQNSRDYLQKFGHVTNQLRKLVKALKACE
ETYARLYKSSFDADHVTLEFLEVAEFQCALFAHIIESICYATPPEPPVFLTTGDHPETRYFAASCQR

MEQMQKNLPQEPANAKTISNRHLDVIIAQIEIITKTPLCLPRYFFQILQSTQIKLSVSPQPRSATEPVN
VQSGSNLVIKVEGVLQHFSKQKKHFRRVESVQLSLTSQLITPPPRSSQELPKQGANDTVTLNQIVK
PQRDFLSGSFLLPISNGGHFQVTLETFVVDENGITWCTGPKSSMVVRVLEDPSKQGAPAPSTSQA
VGQTRRF (SEQ ID NO:102)

# Figure 45

**Name:** CG8858
**Nucleotide:** AE003823
**GI:** 10727617
**Transcript:** CT25438
**Sequence:**

atggaaactggaccgaatgcagaaattgagcttctggagcgcgtcctgttgcgtctgggaaatgcggactcggatga
gaagctggaagccgctgtgggcaagttcctgaccccggtgatcctcaaaatgaactcgccccacaatgtggtgcgcacaaaggtcgtag
aagtgctcacccatatcaagaggcgcctgtcgtcgcgcggacatgtgcaaatacccgtagaggcactgctggatcagtatgccgcccag
gatagcagcaccttcctcaagaacttcgccatcatattcattaccatgggctttccgcgcctggcgttggaacaacagactgcgctggcca
gcaaagtggtgggctgcgaggacaagctggagagctaccaggacaagctgttcgccctgctgctgcccgtactgtcggacatgaagatt
cccgacgacccgacgcagcgctcaaagctacttgatctgcaggacaagccagccatctgtgcaaactttttggcactgctccaggatgtg
ctcctcctgccctatggcatcacccaggaacaggatgtgcctccgggtctgagcccgtacagttttacacgcatcatcgcctacaattggcg
ggccgaagaactggaaaaggtcaagaagggaatagttcgtttcctgtgcgccagtgtgtttagtgatttgcagatattcgtgccactggtggt
ggcttccgcggacacacgtttcagcgtagccactccagcaatcacagagctgagcaagctctgtacaatgctggatttcagcaacccag
cggtgactgccccgttgtacactctgttcgctggcaaccaaagcaagataactgaccgccagacacgtccctgctgcgcccgagtgcgc
caaaaattgctgcagtacctcatcaagtgccgtggcaaaagcattaacgtcaccaagggcttgcaggtgacctttgatggcttctttggcac
aaacaccaaccagaagtgcaaggtcctagcgctacagtttatggaactgctcctcagagatggtccccgcgagctggtctcgaaggtttc
caaggtaatacttaccggcatcaccaaaatcattggacgtgactccaacgaaccgaacgatgtacaaaatgcagcatattccgctttggc
gcaacacgctcgcagttttccgcaagacgtaagccaggatctgaaactagtccttggatacttcaacaacctggccagctgtgcaccaga
attgcattcatccataagggaagcattggtttcgatggctcccgcattcgcttggaagaccaaggagaagagcgatgcaatggaggtgga
taagaatgtcgattcctcaagcgtgaaactggatggccaacagcacctgctattggccatgctgctggacaatgctgagtccaaagtgca
gatcgtgcagaatgtgaccagtgttttcctcaccagctgctatccggagtactatgcaccagccagatacctttttgctactcatcgcaggaga
gcgaaattccctacgcgagaatgtgaccacttacttgtacggcacctcgaagaaggatcacataaattacagcatgctctcttccatcgatc
attctaagaaccgcataagcagcgagtctataagtgactttaatcacttgtccctggagcagcgtagggttatgctacccagcttccaggtg
atgatgtcgcacgtccatgagatggccgaaaaacgcctaaaaaagaacacggcctgcgtggtagttggccgtacaaagctgccctaca
gcctggaggtatacgaggagcttctagattatctgcgtctgtgcttgtggtactcggctggagtggttgccgctcccggcgacgagaagtac
acccatgagctgcgcgagtacatcaccttgcattacgaagatacggaggataatgcactccatcaatatgcgcagtttgtgcagcgaagc
gtagaggccaagagaagtgaatccaatctcatttgcctatatgatctgttgaatgccgctccggaactctttgctgctaagcagctgcatttgc
tggaaccactgggcaacactttgaaagatgtatcggagactatgcgtatcaacgtggctcaagtctatggtattctctgggcttttggtctctcc
gatgagaaattcgatgaagaagtgggtgaatgcctgaatagcttaacgcaaaaaacattggagcacaagcatggatggcttctcgtggt
gggtcacacgttcaatcgcaaaattgctatgcttaagcaggagaataagaccaaggactttgctgcctggccgcagtttgttaatgcggta
aaaataatctcaaaatgcctctgtgaatcccaatggttgctggtgtcagcagcagtgaaatgcatctctatgatcggcaaggctgtggaaat
accaaatgtccccgtggagatccaggttccatccaaagatggagacgacgacgacgaggagatgaccgagtatacgagcatagattc
ctcgacaaaacttgttatctttggcgtcgtctttcaattactgcgaagctcctcagcgcgtcaaaagattcgcgaggactcagccagatgtctc
ggatatctggccattggcgacggcgaacacttcaccaaacgcaacctggataagtttcttaccctgaccaagattcaaaaggatgctgctc
taaacatagccatttcggaagcgatcgtaaacacactttgtggctatgatgtgaacaaggggcaacccgatgagaagtttgttaataagca
ctgcaacgatgacgactttgatcagttcctgaactctctaatacgtctggtcaccgatcccaatccgcattcccgtcaggccatatccgtgtgg
cttctagctgtggtaaagcactgcagccaacgtccagctgtattggccaaaaaagagctgttgcagttcgcttttaccgaacttctttcagatg
acagcgagtttgttcaggacgtggcttctcgcggcctggggctggtctactcgatatcggattctggaagccaaagtgatttggcaaactcg
ctgttggatcaactgattgggggaaaacgaaaggtcaatcaagtaaccgcagacactgaactctttgccgaaggaatgctgggtaaaac
tccaacgggaggcaacattaccacctacaaggagctgtgttccctggcatccgatctcaatcagccggacatgatctatcagttcatgcag
ctggccaatcataatgccacatggaccagcaaactgggtgctgctttcggattgaaaacccttctgccgagtcaaggcagaagatggag
ccatatctgggcaagatcattccgcgcctctatcggtacaagtacgatcccacacccaagattcagaactcgatgatctccatttgggatac
gattgtgactgactcgaaggaagtgaccgagcgttactactgggaaatattacgcgagctgctggacaatctcacttgcaaggagtggcg
agtgcgaatcgcctgctgcctggcagtaagggatctcctaaaccgacccaacgggcttaagctacgctcggaggagccggttcgtcgtg
ctctccccgataacagtatggaagtggatgaggtccctgagccggagctgaaagagctatggttccagctgtttcgtgtgatggatgacatt
catgagggcacacgggtcgctgcccacggaactgcttcattcctcggcaagctgtgtgtcatcgcatcgtcatcggatcatggcaaatccg
ggacagccgtagcgtcatcgatactgccatttctgctagaaaccggagtgggtcacaaagtgcccgagatacgtagagttagcattaaga
ccatttccgacatgattgattcctcgggatcgcttattgccccgcatctcgccaccctgattccctgcctgctacgagccactggcgagttgga
gaacaccaagctctcctacgtatccactcgcttgggtgcggacaacgaggcccaggaggcggtggatacgctacgagcggaggccgc
caagtcgcttcataccatggaaaccattgggaagtgtgtccgttatatcgactatagcgttttggagaagatgacgcccgaagtgctggag
ctcatgaagggtagtgtcaacctcggaaccaagattggctgtgcccattttgtttgtctgattagcattcgcttaggcaaggagatgacaccat
tggtgggaaaatacattcgtgcctgctttgtgggcatcaagaatcgcaacgccactgtgcgcaagtacaacgccagtgccattggccacct
gttgggcttggccaaagagcagtcgattaaaagtctattcactaagctggaagagttatatgccgagcagccaggaaatcgctccattgc

acttaccattcagtccattaacaagcgtcaccatgaactgctcaaggactacatggacagtatgttgccgctgatcttctttgccatgcacga
agagcccaatgaagagacgaaagctaatgtcgagctgtggaaggatctatggcacgacgttagcccaggtgacgcgggcatccgtctc
aatttaaatgtgattatacccaaactggagtcatctctaaccgatgccagctggtcgcgtaaagctcaggcagcgaatgcgatccaaacg
atagccactcgcctaagtagctccttggatgagcccgaccgattgaggctgatcaaactccttctaagcggtctgcaaggacgcacttttga
gggcaaagagcggctattgcaagctttggccgcactcaccaaaggattggatcggaaccatcaaatctgttccagcattatcgacgcgg
ccatgagggaagcaagaaagagggaacctgtttaccgcaccatggccctggcttctcttggcgaaattctcgatcaactggaagcggat
cgctttgaggaagtgtacaacatgagctggaacctgctggaaaagaaggaactgcgcaaggagtcggacgacgaggacgagccca
acaccagtcaggaactgtctgccgacgagcgcaacaagcgggctcaaacactcaatcgactaaaggaggttgtgtgggagacactgg
gcaaatcctggccgcgtcactccatcgagacgcagcaccgctaccaactcttctttgccgaaaactgtacgagcattctggcggagagca
ctcgcccagtgcaagtcagtctgctggcagcactcactaagtacatcgaaagacttcatgtctttgatgagtctgctcaagtgccggatttga
ctcagataaaccaggagaagaagatcaagacagaggagacggcgccaaaaacccgagaggccattgtcgagaagatatgcacag
atgttttggctgcagttgctcttgccgctggagttcctcattccggtttgaaaaaggaggcgctcaacatcgttcttatgctgatcaagcggctg
agcggaagcggaaacggaaaccagcagccactgaggttgatcaagcagagctttgaatcgaatttggaaaagttccagcgcgattcg
gcgccggaaatccggtgtcgcatcaaggacatcgaagacaagctaagcaagttgaatccacagaattaa (SEQ ID NO:103)

**Protein:** AAF58554
**Protein GI:** 7303499

METGPNAEIELLERVLLRLGNADSDEKLEAAVGKFLTPVILKMNSPHNVVRTKVVEVLTHI
KRRLSSRGHVQIPVEALLDQYAAQDSSTFLKNFAIIFITMGFPRLALEQQTALASKVVGCEDKLESY
QDKLFALLLPVLSDMKIPDDPTQRSKLLDLQDKPAICANFLALLQDVLLLPYGITQEQDVPPGLSPY
SFTRIIAYNWRAEELEKVKKGIVRFLCASVFSDLQIFVPLVVASADTRFSVATPAITELSKLCTMLDF
SNPAVTAPLYTLFAGNQSKITDRQTRPCCARVRQKLLQYLIKCRGKSINVTKGLQVTFDGFFGTNT
NQKCKVLALQFMELLLRDGPRELVSKVSKVILTGITKIIGRDSNEPNDVQNAAYSALAQHARSFPQ
DVSQDLKLVLGYFNNLASCAPELHSSIREALVSMAPAFAWKTKEKSDAMEVDKNVDSSSVKLDG
QQHLLLAMLLDNAESKVQIVQNVTSVFLTSCYPEYYAPARYLLLLIAGERNSLRENVTTYLYGTSKK
DHINYSMLSSIDHSKNRISSESISDFNHLSLEQRRVMLPSFQVMMSHVHEMAEKRLKKNTACVVV
GRTKLPYSLEVYEELLDYLRLCLWYSAGVVAAPGDEKYTHELREYITLHYEDTEDNALHQYAQFV
QRSVEAKRSESNLICLYDLLNAAPELFAAKQLHLLEPLGNTLKDVSETMRINVAQVYGILWAFGLS
DEKFDEEVGECLNSLTQKTLEHKHGWLLVVGHTFNRKIAMLKQENKTKDFAAWPQFVNAVKIISK
CLCESQWLLVSAAVKCISMIGKAVEIPNVPVEIQVPSKDGDDDEEMTEYTSIDSSTKLVIFGVVFQ
LLRSSSARQKIREDSARCLGYLAIGDGEHFTKRNLDKFLTLTKIQKDAALNIAISEAIVNTLCGYDVN
KGQPDEKFVNKHCNDDDFDQFLNSLIRLVTDPNPHSRQAISVVLLAVVKHCSQRPAVLAKKELLQ
FAFTELLSDDSEFVQDVASRGLGLVYSISDSGSQSDLANSLLDQLIGGKRKVNQVTADTELFAEG
MLGKTPTGGNITTYKELCSLASDLNQPDMIYQFMQLANHNATWTSKLGAAFGLKTLSAESRQKME
PYLGKIIPRLYRYKYDPTPKIQNSMISIWDTIVTDSKEVTERYYWEILRELLDNLTCKEWRVRIACCL
AVRDLLNRPNGLKLRSEEPVRRALPDNSMEVDEVPEPELKELWFQLFRVMDDIHEGTRVAAHGT
ASFLGKLCVIASSSDHGKSGTAVASSILPFLLETGVGHKVPEIRRVSIKTISDMIDSSGSLIAPHLATLI
PCLLRATGELENTKLSYVSTRLGADNEAQEAVDTLRAEAAKSLHTMETIGKCVRYIDYSVLEKMTP
EVLELMKGSVNLGTKIGCAHFVCLISIRLGKEMTPLVGKYIRACFVGIKNRNATVRKYNASAIGHLL
GLAKEQSIKSLFTKLEELYAEQPGNRSIALTIQSINKRHHELLKDYMDSMLPLIFFAMHEEPNEETKA
NVELWKDLWHDVSPGDAGIRLNLNVIIPKLESSLTDASWSRKAQAANAIQTIATRLSSSLDEPDRL
RLIKLLLSGLQGRTFEGKERLLQALAALTKGLDRNHQICSSIIDAAMREARKREPVYRTMALASLGE
ILDQLEADRFEEVYNMSWNLLEKKELRKESDDEDEPNTSQELSADERNKRAQTLNRLKEVVWETL
GKSWPRHSIETQHRYQLFFAENCTSILAESTRPVQVSLLAALTKYIERLHVFDESAQVPDLTQINQE
KKIKTEETAPKTREAIVEKICTDVLAAVALAAGVPHSGLKKEALNIVLMLIKRLSGSGNGNQQPLRLI
KQSFESNLEKFQRDSAPEIRCRIKDIEDKLSKLNPQN (SEQ ID NO:104)

# Figure 46

**Name:** Ac76E
**Nucleotide:** AE003515
**GI:** 10733346
**Transcript:** CT23940
**Sequence:**

tcgaaagcggttgagaagcgattttcaggcggtcgcgtttttcgcgcgtttccttgcatcggaaatagcaagtgtgcgcc
tgtgtgtgtgtgtgtgcgagtgtgtaaatgtgttaatgtgtatttttaaacaaaatgtgccattgggaaatgtctccgcatctatgcaatgcacaa
atatatatagataaatagatttattggttcaaatgattaggaaacaaacaacaacagaggattatacgagcagcaattagagttggatttctc
catttccgggaggaaggaaggaagtcggcaacaagtgttggatccccaggagcagaagtcgcaaggacttgcgtccagggtgaggc
aatcaggacgcgcagctggcggctgtgaggagtgccactagctacacgccaccgcttttcgccatggtaaatcacaatgcggaaactgc
gaaaacgggcaatggcacaaatgcaacagcgaatctgattgttaaggccgacggaaatgcaacgcagccgaaggcgatgacgtcat
cggcggccaggatgaatgacgccctttcggcatctttggccgatttgagcgagcaggaaaatggaaccacgggtgaggctgaggacat
ccacctaaacgatttgtatacccgctaccgccagaggctccgcaagtcgcttttcagatcgggattgttaacttcgctgctggcatgtgttgtgt
ccataataattggcatcgtttacggccagcacctggtgcagaccatgctcttagtcctggctgccctaatctcgggatccattctcacggccct
gcagttcccggcggtgctgagctccccggcagccgccttggccttcgccatcgtcaccaccttctcactgggcaccattgcggccatcacc
ggggacgaactggctcccctgcccatgtacgccctcttcctgtgcatccactccatgctgcccatttcgtggcccgtctccgtggtcctggctc
tattcatgaccgccattcacatcgtttaccgcatcggcacaagtcccgactacgcgcccaatttgccaatgctcttcggggagattgtgatgtt
ggccagcgcctccgtttccggtctctactaccgcatcatgtcggacgcagcccacaaccggacggtggacggaaccaggacgggaatc
gaacagcgcgtgaagctcgagtgcgagcgggagcagcaggagcaattgctcctcagcgttattcccgcctacatcgccgccgaggtga
aacgcagcatcatgctgaaaatggcagatgcctgtcagagagccggaggacaggcgtccacttcggccacccggttccatgagctcca
cgtccagcggcacacgaatgtcaccattctgttcgcggatattgtcaacttcacgcccctttcaagttccctgacggccagcgatttggtgaa
gaccctgaacgatctgtttggacgattcgatcaaatagctcaggagaaccaatgcctacggatcaagatcctgggggattgttattactgtg
tgtccggcctgcccatttcccggccccaacatgcaaccaactgtgtcaacatgggcctgcagatgatcgatgccatcagacacgtgcgtg
aggcgaccggcataaatgtcgacatgcgaattggcatccacactggcaacgtactctgcggcgtccttggcctacgcaagtggcaatttg
atgtttggagcgacgacgttacattggccaaccacatggagagcggcggtgtcgctgggcgagtccacatcacgaagcaaactttggac
ttcctgggcgacaagttcgaggtggagcagggcgagggtggcaatcgagatgcgtatctggcggatcacaaggttgaatcgtatctcatt
gtgccaccgaaaccagcatacacctacagcgttccacgcgtggtggaatgcattgagcagaacgatcccagtcccaccaccgaggag
accaaggagatcaaggagacggatcaatcccatgaagccaccgatgtcgccgatgtccttcttccggtaactgtggctccaccgcctgc
aatcgttgacgagaagatgtcacccacctcgatcaatagccaggaggcacctttgcatgcccctctcgcctcagccgcctccatgtccatt
aaggagctgtccgaggaggaggacgaggctgatgaggccactgccgtcaccgagccgctgatgcacagggatcaggatggcaaga
atgacaaggagccgaaggcaaatggtggccatcgcggcagtggtgattccgccgcctccgagtcggtggccaagtccgctgccctttcc
ctgcccgccgatgatctgctcagtatgagtgggtcagagagcggcatctccaacagcggagcccaggcgcaatcctcgaatccggcga
gtgtcacacccaccgcagccgctccagccggaggagccgcctccaacagcttgacggtggccgaggccccggagagatctcgtcgg
aagttgtccgttcaaggtctgatgtccttcgccgacagacgacgctcctccggagccttcatcgaaggacgaaagctgtccatccacagc
ggagaaagtttccgcagtcatgcgggtaagtagcatcgccatgattgaatcaaatttactgccgccggagcgtaaatgtttcaattttaatttc
ttcgggccgcccacggagctgaagccgttcaccatgtggtaccgcaatacgccccgggaggccatgtaccgcgcccagccggatacg
cacttccggttcgacctgatatgcgccttcgtcctcttcctctcgctagccgtcgtccagctgattgtaatcgaattgaacctggccctccttgga
tccctcctggccagcttcgtctcgctggccctcttcctctacctgagcaacatgtccgtgccggacgtgcacgcctccactacggagcgaaa
tggtcccggccaggtggtggccagcagtcgctacttacggctggccatgttcgttgtggtcaacattctcatttcatcttgcgcggtgtttagtgt
gataaactacactgtgcctgacggggtaagcaaggagccatcatccaatcagaccatcctggagagcaatttttcgagtgtgttcgtaaac
tccaccctggaggatgtgcagctgtgggaaatcgattatgccatacccattgcgcccgtatttctgtattgctgtgccattagtttggctgcaatt
tccgccttcctgcgatccggatttattctcaagctgattgccatgctggtggctgtgatcgcacaggtgacagttcttgggtacagtgatctcttt
gagatgtacaacgatgccaacatcacacacggcttgccacttgagatcaagggcttcctttttgcttctggtgatcatcttggtgcttcacactct
ggatcgccagggtgaatatgtggctcgcacagatttcctctggaaggccaagttgaaggtggagcaggaggaggtggaaaccatgcgt
ggcattaacaagattctgctggagaacatcctgccggcccacgtggccacccatttcctgcacctggaacgctccacggagctctaccac
gagagctactcctgcgtggccgtcatgttcgcctccattcccaactacaaggagttctacgatgagacggatgtgaacaaacagggcttg
gagtgcctgcgtctgttgaacgaaatcatttgtgattttgataagcttctcttgaaaccaaagttcagtggaatcgaaaagataaagactata
gctagcacatatatgtgtgcctcgggcttgagacccggcaaggaggacggcgctacggacgagaagcgcacggaggagcacaacgt
ggtcattttggtggagtttgcgattgctttgatgtccatactggactcgattaatcgcgagtccttccaacgattccgtctccgcatcggactcaa
tcacggtccggtgattgccggcgtgattggtgcccagaaacctcagtacgacatctggagcaacacggtcaacgtggcctcccgcatgg
actcatgtggcgtaatgggacgacttcagacgacggaaaacacggccaagatattgatgaccgccggctatgagtgcgagtgccgagg
cctgacttatgtaaagggcaagggcaatctagtcacctactttgtaaagacacctttcgatgggaaattgtagatcaccgatcgtcacccag
ttgctgttgatactatttaatctatttaatggatatttatgaacatgtacacgtagcctaggccaaaagtcacttagaagcatcttcttatttattcttt
gcgattatcatttagttgtaatggattgcaatcgcaggcatttaaagtacgttgctcatcacgcgcacagttgagtattgttagcaacaatttgttt

ttagttagttggtagtgttaaattgttattgactatttcgtacgatttgttagctaatcttatgcgaagccatcacttaaacagcgaatatttatatttat atacgattcgaattaaattatgatagacaatgaaatgcaatttgaacagccccgccccaatccagaaacatatctattgtctactatatagta tctgctcgttttcattcttatctgagactcttaagatctatttccctctccactgcgaacagcaaacactaaaaaaaaatcagtatgtttaaactat tcgatttgattaaacgacactagttaaattctgtagggagaatcaattaaagctctatatattttttactcggatgtttttactactactaaactatt cactacttcatcttttgatatatttatgtatataactatgtaagagaaagccaccaaagcgaactactctataagatacatctatgagtacctat cgcatcccaacaaaccactcgatacaatacctccgatgaaaactcacctgcttagatgcgatattatcaaccaaactctcaagattcaaa actgatttcgaagtatctgcgctggaggaatacaataaatgaacgggatgtttgttaacacactaaacacgctgcagtagattcattgaaca atatttgatattatcagaaaatttaattgattttgattaaatgataaataaaggaaatcaataaaatattattatgtattc (SEQ ID NO:105)

**Protein:** AAF49089
**Protein GI:** 10733351

MVNHNAETAKTGNGTNATANLIVKADGNATQPKAMTSSAARMNDALSASLADLSEQENG TTAEDIHLNDLYTRYRQRLRKSLFRSGLLTSLLACVVSIIIGIVYGQHLVQTMLLVLAALISGSILTALQ FPAVLSSPAAALAFAIVTTFSLGTIAAITGDELAPLPMYALFLCIHSMLPISWPVSVVLALFMTAIHIVY RIGTSPDYAPNLPMLFGEIVMLASASVSGLYYRIMSDAAHNRTVDGTRTGIEQRVKLECEREQQE QLLLSVIPAYIAAEVKRSIMLKMADACQRAGGQASTSATRFHELHVQRHTNVTILFADIVNFTPLSS SLTASDLVKTLNDLFGRFDQIAQENQCLRIKILGDCYYCVSGLPISRPQHATNCVNMGLQMIDAIRH VREATGINVDMRIGIHTGNVLCGVLGLRKWQFDVWSDDVTLANHMESGGVAGRVHITKQTLDFL GDKFEVEQGEGGNRDAYLADHKVESYLIVPPKPAYTYSVPRVVECIEQNDPSPTTEETKEIKETDQ SHEATDVADVLLPVTVAPPPAIVDEKMSPTSINSQEAPLHAPLASAASMSIKELSEEEDEADEATAV TEPLMHRDQDGKNDKEPKANGGHRGSGDSAASESVAKSAALSLPADDLLSMSGSESGISNSGA QAQSSNPASVTPTAAAPAGGAASNSLTVAEAPERSRRKLSVQGLMSFADRRRSSGAFIEGRKLSI HSGESFRSHAGHVTRNRPSSKMTKYVECWGADRPFANIAESKLVKNIGLASIAMIESNLLPPERKC FNFNFFGPPTELKPFTMWYRNTPREAMYRAQPDTHFRFDLICAFVLFLSLAVVQLIVIELNLALLGS LLASFVSLALFLYLSNMSVPDVHASTTERNGPGQVVASSRYLRLAMFVVVNILISSCAVFSVINYTV PDGVSKEPSSNQTILESNFSSVFVNSTLEDVQLWEIDYAIPIAPVFLYCCAISLAAISAFLRSGFILKLI AMLVAVIAQVTVLGYSDLFEMYNDANITHGLPLEIKGFLLLLVIILVLHTLDRQGEYVARTDFLWKAK LKVEQEEVETMRGINKILLENILPAHVATHFLHLERSTELYHESYSCVAVMFASIPNYKEFYDETDV NKQGLECLRLLNEIICDFDKLLLKPKFSGIEKIKTIASTYMCASGLRPGKEDGATDEKRTEEHNVVIL VEFAIALMSILDSINRESFQRFRLRIGLNHGPVIAGVIGAQKPQYDIWSNTVNVASRMDSCGVMGR LQTTENTAKILMTAGYECECRGLTYVKGKGNLVTYFVKTPFDGKL (SEQ ID NO:106)

# Figure 47

**Name:** CG17010
**Nucleotide:** AE003635
**GI:** 7297915
**Transcript:** CT37753
**Sequence:**

atttttcccccaaccaaaatctctgttaagttttgttcaaaatttccggcttacgcagccctttcaaatgggaaagaagctg
gatgtggttgtcttcgggacggcaaacatcgaatatataacatatgtcccggaattgccgaaacccggtgaacttgtcgccggaacgtaca
tggagacctgtttcggtggcaagggagccaatcagtgtgtggcagctgccaaattgggggcctccaccgtcctggtcgccaagttgggca
aggacgaatcgggcgatgactacctaaaccacctgcaacagcacgaggttgatgtgacgcacgtccagcaggtggagaacaatccc
acgggcatgagtgaaatcgcggtttctgagaatggagatcagtacaagatcaacgtggctggtgcgaatgtatttctcacggccaaggat
gtgacccgggctaagaagtcctttcaggatgcgaaggtgcttctctgccaactggaaacggatatgaatgccaccatgtgtgcgttgcgtc
agttcaaggggggtttccctgctccacatgtcgccaatgcgaaaagatattccaaatgccatgattggcttgcctactattctggttgcgaatca
agaggcagctgccaacctaaccgatatggaagaagtgttaacccctgatcaggcgcgaaaagcagctgaaactcttatcgcaaaggg
agccaagagcgtgatcattaccatgggcgagcaaggagccgtttacatgtccaagaagagcaaagatatgtgcacccatgtgcccgcc
tccgatgtgccgcacttggccgatccctcgggtgccggtgatgcctttatgggcagcttggcctatcacatagctcgatttcccaaactgtcca
cagaacatcatatcagtgcggcacactcctgcgccgcctactccatgggccgccgcggcactcagccaagttttccgggcaaggaaag
cgccaaaagtgacctatgctattcgacgcccacgtttagtgtcattccaccttctagtccgcaacatgaagatgataatatggtaacaaaga
cgccaccgccttcaatggtagtccctacgcccgaagaagcacactcccaaaaggaagttgtggaagaaccgcaaaaacctgcacctc
ctccaccggaggatccggttgtggaagcacctccaccaacgacctccgaggaacctccgaaagcagtggaaacctccaatgaacctc
caaagcctgcggagaccactgatgaacctccaaagcctgcggagaccgcagatcaagctcctgtgaaacccgtggagactgctaatg
agccacctgccaacaaagcataa (SEQ ID NO:107)

**Protein:** AAF53182
**Protein GI:** 7297937

MGKKLDVVVFGTANIEYITYVPELPKPGELVAGTYMETCFGGKGANQCVAAAKLGASTVL
VAKLGKDESGDDYLNHLQQHEVDVTHVQQVENNPTGMSEIAVSENGDQYKINVAGANVFLTAKD
VTRAKKSFQDAKVLLCQLETDMNATMCALRQFKGVSLLHMSPMRKDIPNAMIGLPTILVANQEAA
ANLTDMEEVLTPDQARKAAETLIAKGAKSVIITMGEQGAVYMSKKSKDMCTHVPASDVPHLADPS
GAGDAFMGSLAYHIARFPKLSTEHHISAAHSCAAYSMGRRGTQPSFPGKESAKSDLCYSTPTFSV
IPPSSPQHEDDNMVTKTPPPSMVVPTPEEAHSQKEVVEEPQKPAPPPPEDPVVEAPPPTTSEEPP
KAVETSNEPPKPAETTDEPPKPAETADQAPVKPVETANEPPANKA (SEQ ID NO:108)

# Figure 48

**Name:** tkv
**Nucleotide:** AE003609
**GI:** 7296952
**Transcript:** CT33585
**Sequence:**

ccataatttacaccactcatgttacgctaaagttttattgagttccattaatttacgactttctatggtcgtaattaagattatcc
tttgctcgcgctccgcaattgccgccgattttccatgaatggcagctttttatcggacctgcactcgcataattcattttatcattttgacagattcg
gaatggaatagcgactttcctcgcccacccaacacgcgtacgagatttcaattgcccgtcgagtggcacacatatattataccagctctcg
aaatgagacgcatcataaataataactaatgttttgataagaccccaatgagcgccaaccactttttctgtttatcaaattacgtatgaagtttg
ctcattaattttggcaaagtgagtgtaggggaaacatgggtagtgaattcagtgcgaatgttcagccagacgtcgaagctatggccgccgc
cgctttgagtggcatggaaatgggatctggaccaggatcagagggatatgaggatgccgacaacgagaaaagcaagaccgtggaaa
atgcccgctccctaacctgctactgcgatggcagttgtccggacaatgtaagcaatggaacctgcgagaccagacccggtggcagttgct
tcagcgcagtccaacagctttacgatgagacgaccgggatgtacgaggaggagcgtacatatggatgcatgcctcccgaagacaacg
gtggttttctcatgtgcaaggtagccgctgtaccccacctgcatggcaagaacattgtctgctgcgacaaggaggacttctgcaaccgtgac
ctgtaccccacctacacacccaagctgaccacaccagcgccggatttgcccgtgagcagcgagtccctacacacgctggccgtctttgg
ctccatcatcatctccctgtccgtgtttatgctgatcgtggctagcttatgtttcacctacaagcgacgcgagaagctgcgcaagcagccacg
tctcatcaactcaatgtgcaactcacagctgtcgcctttgtcacaactggtggaacagagttcgggctccggatcgggattaccattgctggt
gcaaagaaccattgccaagcagattcagatggtgcgactggtgggcaaaggacgatatggcgaggtctggctggccaaatggcgcga
tgagcgggtggccgtcaagaccttctttacgaccgaagaggcttcttggttccgcgagactgaaatctatcagacagtgctgatgcgacac
gacaatatcttgggcttcattgccgccgatatcaagggtaatggtagctggacacagatgttgctgatcaccgactaccacgagatgggca
gcctacacgattacctctcaatgtcggtgatcaatccgcagaagctgcaattgctggcgttttcgctggcctccggattggcccacctgcacg
acgagattttcggaacccctggcaaaccagctatcgctcatcgcgatatcaagagcaagaacattttggtcaagcggaatgggcagtgc
gctattgctgacttcgggctggcagtgaagtacaactcggaactggatgtcattcacattgcacagaatccacgtgtcggcactcgacgct
acatggctccagaagtattgagtcagcagctggatcccaagcagtttgaagagttcaaacgggctgatatgtattcagtgggtctcgttctgt
gggagatgacccgtcgctgctacacacccgtatcgggcaccaagacgaccacctgcgaggactacgccctgccctatcacgatgtggt
gccctcggatcccacgttcgaggacatgcacgctgttgtgtgcgtaaagggtttccggccgccgataccatcacgctggcaggaggatga
tgtactcgccaccgtatccaagatcatgcaggagtgctggcacccgaatcccaccgttcggctgactgccctgcgcgtaaagaagacgct
ggggcgactggaaacagactgtctaatcgatgtgcccattaagattgtctaagcagctgtttgtagtctcgttttaggtttaacctaagttgcatt
tagttttctgtttttgttcaacttctgttgaatacgcagttccgaccttttgtccggaaattcatagttaggttctaatcgtaagttgcttgtacgtgagc
ggagtaaagcagaggatccttcagaggtgcctatactacggttacttaaaccgtcgcactgattgtaagctagagtttagatataagttggc
aggagctagttactaaggccccttgcaaaatctttggttagaaaaatgctttaactatttgagaaatatagggagtattttggatgacacctatt
catgtcgatgtacataaatatgagatcaaaacaaaaaatcaatcgaaagaatcgaaatttatatgtaacatacattaatttttgtgccagcttt
tgtacgctttaactaaacgagttaagtcgcccattctttgacccccgaggctttagcctcaacatagaacatgtccttatatgttttgtttgtatcta
gtgtttaagtgtaaactttgcagactttgtgcaagtaaatgctgccttttgtaaaatccacggaattagttgccttaagacaaagtgtaaagac
aggagccaacagacaggagcccgactcccttcctccccttttagtagcacatatttttgtatttataaccagagatatatagaactggcagtt
gcgtatctcgagaagtgcacttgtgtcggctcttttgttttgtgtaaatagcaaagatgtttataggccgctaaccatacatatatatagtatatat
attatatattatcgttataagcgattctgtaaatactatatatgtatctttatctgtaaccgtttaacttatatcttatcgttcttatcatgtaaccttgttta
agctagagttagatgactagcctgtaagagcaacaagtaaactatatgaactagatggatatgtatctttcagttttttttctgtttcattattactg
ctcgcagaccacaagaactaaagttttgtattaaagcggttagttaatttatttaaaaataataaaaaattataattaaaccca (SEQ ID NO:109)

**Protein:** AAF52230
**Protein GI:** 7296958

MRLQLVALELQATSGTADGLFEEIALEALTRSEFSANVQPDVEAMAAAALSGMEMGSGP
GSEGYEDADNEKSKTVENARSLTCYCDGSCPDNVSNGTCETRPGGSCFSAVQQLYDETTGMYE
EERTYGCMPPEDNGGFLMCKVAAVPHLHGKNIVCCDKEDFCNRDLYPTYTPKLTTPAPDLPVSS
ESLHTLAVFGSIIISLSVFMLIVASLCFTYKRREKLRKQPRLINSMCNSQLSPLSQLVEQSSGSGSGL
PLLVQRTIAKQIQMVRLVGKGRYGEVWLAKWRDERVAVKTFFTTEEASWFRETEIYQTVLMRHDN
ILGFIAADIKGNGSWTQMLLITDYHEMGSLHDYLSMSVINPQKLQLLAFSLASGLAHLHDEIFGTPG
KPAIAHRDIKSKNILVKRNGQCAIADFGLAVKYNSELDVIHIAQNPRVGTRRYMAPEVLSQQLDPKQ
FEEFKRADMYSVGLVLWEMTRRCYTPVSGTKTTTCEDYALPYHDVVPSDPTFEDMHAVVCVKGF
RPPIPSRWQEDDVLATVSKIMQECWHPNPTVRLTALRVKKTLGRLETDCLIDVPIKIV (SEQ ID NO:110)

# Figure 49

**Name:** dnt
**Nucleotide:** AE003662
**GI:** 10728868
**Transcript:** CT38779
**Sequence:**

aactcggccgggaaaagttgaaaaattattaacaagcagctgccagacagctaaaacagtcataaactgaagaaa
acccgcggaaacaacgtttaaaatgaaatgtgcgtgaaaaaatagtcgaattgttatttataagaccaaaataaagtgaattgcccggtta
aacaatgcttttaatgaaattttggtgctaaaacgacgccgtcgcactcgcactctcgctctttctctctcgcggcgtccgtgtctctctctcccc
caccccccgcgactttggattcgcttttgtgctggtcattcgttcgagattgtttacgaatagccgtgtgtgataagagtgcaaaaactggcaaa
cggaacagaatatctgatggaatctgtgaataaatgcggtaaaagcgcatccacaagaaattgtacggtgaaaatgagccggaaaatg
tgggtgctttcgctgctggcgctggcagcgctacaactgcactcgggcagcgaagtggctgcacatttgaatgttttcctaaatcccgtgga
agtaatgcgtttattaggcgtctctgctgaggtttactacgttcgcgaaggtcatataaataactacgctttgaacttcattgtaccagttccggc
gaatgtcaaggacattagtttcacctggcagtcgctggcggggcgtggccttccctacagcatcaatgtggtcagctccgatcaggaagtg
cttcctcgaccggcgatcaatgtgtcacacagtggggaaatccccacgaccatacaaacctggtccattgccctcaagtgcagcggattg
aaagcggcggaggtggatgtgactgtcagcctggaagtggtgctcaaccggtcactgaacaatgtaactcatctggtgtttcggaggaag
aagatctgtcttatgaatgacagtgccgaggatttgtcggaggatgtggacgatccccagttgctggagaccgtaatgttaccacccactgg
attgatcaccctggtcgttggtgtttctgtggccatgggatctgtgtgcctgctcctcatgattgcctattgcgtcaagggagcagccaacaag
aggcagcatcatcaacacggaggacagcccatgaggacttcgagctttcagagactaaatacccatccgccctgccagtcttccatggg
atcggctgcttatatgacgccttcgattatagctccaattcatggctcctccttgccacggaaagttccagtttccgtagagcagcaacatccg
gaggagcttcatcgccgaatttccgagttgaccgtggagcgctgcagggtacgactatcgagtctcctgcaggaaggaacgtttggacga
gtttatcgtggcacctacaacgacactcaggatgttctggtcaaaacggtggcccagcatgctagccaaatgcaggtgcttctccttctcca
ggaaggaatgctgctctatggcgcctctcaccctggaattctctcggttctcggcgtttccattgaggatcacaccacaccgtttgtcctctatc
ccgctctgaacaatacccgcaacttaaagcaattcctgctggatccagcttgtgctcgcactgtgaccaccatccaaattgtgatgatggcc
tcgcaactgtccatggcattggatcatctgcacagccatggagtggtgcataaggatattgccacaaggaactgcgttattgacgatcagct
gagggtgaagctctccgatagctccctgtcccgcgatctcttcccctcggactacaactgcctgggggacagcgaaaacaggccagtta
agtggatgtccctggaggcactgcagcacaaacagttctccgaggccagcgattcgtgggccttcggagttctgatgtgggagttgtgcac
ctccgctaagcaaccctacgccgaggtggatcccttcgagatggagcactatctcaaggacgggtatcgtctggcccagcccttcaactgc
cccgatgagctgtttacgatcatggcctattgttgggccctgttacctgccgagcggccgaccttcgcccagctgcagtcttgtctctccgaatt
ctattcacaaatcacgcgctatgtctaggacaaaggtcatcaatgtcatccgcatgggaatcggctcttttgaagagttaaggagtttaggct
tagttgaataaagtcctctagcttactccactttgttatgacaagatttgccaaatcgaatgtttagcccttccttggtccagcatctgtacatata
gtttacacattcagctctttaagtaactatctttaagcttactctacgataaactcaactcttaccacttagatgaaatacatagttcagcttgcac
agttctccttgtaatccatcaacactagctaaaacaaaggccgaaatcaaatatagtgatatttataaatagagatagcgatgtctataagat
tacacatacatatttatgatgcatataatacatgttgtatgtttagcatttttgacaatttaccgtttatacgagagatcaaacatgatatacaaat
atatac (SEQ ID NO:111)

**Protein:** AAF53783
**Protein GI:** 7298565

MESVNKCGKSASTRNCTVKMSRKMWVLSLLALAALQLHSGSEVAAHLNVFLNPVEVMRL
LGVSAEVYYVREGHINNYALNFIVPVPANVKDISFTWQSLAGRGLPYSINVVSSDQEVLPRPAINVS
HSGEIPTTIQTWSIALKCSGLKAAEVDVTVSLEVVLNRSLNNVTHLVFRRKKICLMNDSAEDLSEDV
DDPQLLETVMLPPTGLITLVVGVSVAMGSVCLLLMIAYCVKGAANKRQHHQHGGQPMRTSSFQR
LNTHPPCQSSMGSAAYMTPSIIAPIHGSSLPRKVPVSVEQQHPEELHRRISELTVERCRVRLSSLL
QEGTFGRVYRGTYNDTQDVLVKTVAQHASQMQVLLLLQEGMLLYGASHPGILSVLGVSIEDHTTP
FVLYPALNNTRNLKQFLLDPACARTVTTIQIVMMASQLSMALDHLHSHGVVHKDIATRNCVIDDQL
RVKLSDSSLSRDLFPSDYNCLGDSENRPVKWMSLEALQHKQFSEASDSWAFGVLMWELCTSAK
QPYAEVDPFEMEHYLKDGYRLAQPFNCPDELFTIMAYCWALLPAERPTFAQLQSCLSEFYSQITR
YV (SEQ ID NO:112)

# Figure 50

**Name:** ACXD
**Nucleotide:** AE003474
**GI:** 10727242
**Transcript:** CT6304
**Sequence:**

ggttggagtcggtcgcgtgtgttgatagcagctccggcgggcaattaagtcggacggaatcgaatcggggcgagcg
cttatcgcggcaaaagagtaatttgtgatacctgtccgccgtgtattggcttgttggagcgcaacgcgcttaagtggtgttaatttttattaattat
tatcgtatttattacccaacgaaagtgaagtgcgaaatcggtcgcctcaagtgcccggccgtacgtgcaaagcccgtaaataaattacac
agccatatagccctgatccttacctctacactcgctctcgcccaccgcagctgataatgcagacgaattccgcctgatcgattcccattccca
tttcgattgcgatttggagtgcgagtgcgggcgaaataggcagccagcctctgattctgagtagctgccaccgaaaatgagcccgccaca
ttgatcatctgatctcccatccacccaatccaatctgaactgaactgaactggcgccatcatggactcctacttcgactcggccatcgagtac
aatcgatccaaccccatcagcctggccaggaccagcagccaggcccaaaccgtgcagaacgagaagaactgggagtggtcgtacct
tgtgagaaaatgtcgcaacctggagctggaggactcgtacgacctatacatgcgtcgcctgcgcgtcggctacctgtcgctgttcatcttcat
ccacgtggcggtgacggtgatccacacgctgctgctgctgaccacgccggagatcaagtacgtgtacgtggacatggtggcctacatgtg
ctccggcctggtcatctgggtggtgctgggcgtgaactttcgcagcgagctggtgagcaagcacggctgggtggtctacgccacctcctgg
ctggcggtgtgtgtaatggtcctgatggacatcggactgaatgtttaccacgccaccagccacaacgacatcctgaatccgatctacgatg
cgtatacgctgtacgccatatacatgttcatgccggtgccgtacctgctgcagccgtttgtccttggatcggcagtcacctttgctacataata
aactacagcttcgtgatcaccgccaaggatgacaaccaaatgcacagcatcctgaacgaggccatatacctgagctgtgtcaacctact
gggcatttctttcgcctgatgagggatatagcgctacgcaccaccttcctcgatcgccggcagtatgtggaggagaatctcctgttgcgctat
gccagggatcaggagcggagtctcttgcttagcatcctgcctgcccagatcgccgatcggctgcaggaggatgtgaagaaccgcatcga
gcgatccaagcagcagcaccagcagcagtcgcaggtggatctgcgcaggagtgcggatagccagacccttaaaaggtggcgacaa
ccggaccatggcaccctcttcattgagccccacgaagatgtcaccgtcctctacgcggatgtggtcaactacacccacttgaccaccacc
ctggatgtgaagaagctagtggaggctctgcacgatctcttcgtgcgtttcgacattgccagcgaggagtacaacgtgctgaggatcaagt
tcctgggcgattgctactactgcgtggctgggctggccaatcccaatgcggatcacgccaagtgctgcgtggatctgggactgcgtatgatc
aaggacattcgagatgtcagagagaagcgccatctgaatatcgacatgcgaatcggcgtccactcgggcgatgtcctatccggcgtaatt
ggagccgccaagtggcagtttgacatatggtcgaaggacgtggatattgccaatcgcctggaggccactggagccacaggacgagtgc
atgtgagccagaagaccctatccctgctggatggcgagtacttcttcgaggacggcaccgaaaaggctcgagaggatccggtgctgca
gaagcacggcattcgcaccttccttatcaagtcactgcgcgccccccatgcatgacccgcgtcgcaggatgagggagcggcaggtgaag
aagctgagcgaggccagcaaggccaactttatgcacaactccacgctccaccagtacaaccaggtgcgcaaccaggccaagctgga
gatgtgtcgcgagctggacaagatgcccatcggcaggatacagctcaccaaagtcttccggcgaagcactcggctcacccaggacga
aatcgaagaagagacctttcgacgcaacatcagctcctgctgtctgttctttcgcatccgcaactgggagttccagtatatcaaggagccgg
atgtgatgctcaagtacagcattgctttggcttgggtcatttacatgggtctgctgaccatccagctgctgagcaaggatgctcgctatcacta
ctggtacatagatggcaccacgattatcctgctcaccatgctgctgatcgtgtcctggtacaagaagctctggatcatgtacatgtccgatgc
ggatgtgtcctcgccgaacggccggctcagtggcttcctcttccgcatgtcggatgagatgcagcgcaatgtggtcatccgggtggtgatgt
acttcctgatcatcttctcctattgcgcggtggccattatgcaagtggtgggatgcagcagtgacgaggactacgcggatctggatccgacc
tacgacgagcgggttcagtgctttcatccttggatactcaccaattgcatgaccctggtaattggcatgtccttcctcttcacgcgaatccccttc
atcatcaagtcctgcttctctgctttgataatgatcggctacgcggtgctggtggtgtccgagttcaacttcatctacgccaacagcccctccac
caatgtgaacttcaacgccaagtactcgcatatcctgctgatgatcataacgttcggcatttttcacttgatggagcggcagacggagttcatt
gccaaagtggattacaactggaaacggcagctaattaagaagcaggaggacgccctgatcaccaatgacaccattaaagtgctcctca
ccaatattctgcccacacatgttggtaagtttgccaagtggccagctggtgacatactaactccatccgcccatccagccgacttctatctgtc
gaatcaactgcagaacgagctctactacgaggagtacgataatgtggccgtgatgttcgcctcgatcaaaaacttcgacaccgacaaga
tcggactgcgggtgctcaatgagattatctgcgactttgacgatgtgttgaacaagtactcgcagagtctgcgcgtggagaagatcaaggt
ggccaactggacgtacatggcggcctgtggcctagacgtctctcgttcggagcaggtgaacgccccgcagatgaagttccgcaacgtttc
cctcatgcccaacgggcgcaggagtcgctacgacggagcacgaagctccaatgcggacggagtgcagcgggtgccgtacggaaac
ggcagcaacatagccctggatctcgacttggagcggggtcagtacgagggcaacgtgatcaccagcgggccgcgcatcagtagcacc
cacaacgggagcagcagcaacgaggtggtgcgcgtgatggccgagttcgccctggacctgatgcgcacgatgcgccgcttcaacacg
gagaacatgcagacggagtacgagggcagcaccgactacggcatgctgaggatcggcatctcgcacggcagggccatggccgggg
tggtgggcatctcgaagccccactatgacatctggggcaatcccgtgaacatggcctcgcgcatggactccaccggcgtgcccggccag
attcaggtcaccgagaacacggcgcttaagctgcgcgagtttaacattcagtgcaactaccgcggcatgaccttcgtcaaggggcgtggc
aacattcccacctacatcatcggcatcgacagcgaataccagttcctgccacatcgcccggccgcgccaaccaaagaagactagctga
aatttaccagagattagatttaggacatgcatgtatgtaaatatagagtataataggtttaggacagtcgcaaataaaagctgtaattcgaac
t (SEQ ID NO:113)

**Protein:** AAF47621

**Protein GI:** 7292211

MDSYFDSAIEYNRSNPISLARTSSQAQTVQNEKNWEWSYLVRKCRNLELEDSYDLYMRR
LRVGYLSLFIFIHVAVTVIHTLLLLTTPEIKYVYVDMVAYMCSGLVIWVVLGVNFRSELVSKHGWVVY
ATSWLAVCVMVLMDIGLNVYHATSHNDILNPIYDAYTLYAIYMFMPVPYLLQPFVLGSAVTFCYIINY
SFVITAKDDNQMHSILNEAIYLSCVNLLGIFFRLMRDIALRTTFLDRRQYVEENLLLRYARDQERSLL
LSILPAQIADRLQEDVKNRIERSKQQHQQQSQVDLRRSADSQTLKRWRQPDHGTLFIEPHEDVTV
LYADVVNYTHLTTTLDVKKLVEALHDLFVRFDIASEEYNVLRIKFLGDCYYCVAGLANPNADHAKC
CVDLGLRMIKDIRDVREKRHLNIDMRIGVHSGDVLSGVIGAAKWQFDIWSKDVDIANRLEATGATG
RVHVSQKTLSLLDGEYFFEDGTEKAREDPVLQKHGIRTFLIKSLRAPMHDPRRRMRERQVKKLSE
ASKANFMHNSTLHQYNQVRNQAKLEMCRELDKMPIGRIQLTKVFRRSTRLTQDEIEEETFRRNISS
CCLFFRIRNWEFQYIKEPDVMLKYSIALAWVIYMGLLTIQLLSKDARYHYWYIDGTTIILLTMLLIVSW
YKKLWIMYMSDADVSSPNGRLSGFLFRMSDEMQRNVVIRVVMYFLIIFSYCAVAIMQVVGCSSDE
DYADLDPTYDERVQCFHPWILTNCMTLVIGMSFLFTRIPFIIKSCFSALIMIGYAVLVVSEFNFIYANS
PSTNVNFNAKYSHILLMIITFGIFHLMERQTEFIAKVDYNWKRQLIKKQEDALITNDTIKVLLTNILPTH
VADFYLSNQLQNELYYEEYDNVAVMFASIKNFDTDKIGLRVLNEIICDFDDVLNKYSQSLRVEKIKV
ANWTYMAACGLDVSRSEQVNAPQMKFRNVSLMPNGRRSRYDGARSSNADGVQRVPYGNGSNI
ALDLDLERGQYEGNVITSGPRISSTHNGSSSNEVVRVMAEFALDLMRTMRRFNTENMQTEYEGS
TDYGMLRIGISHGRAMAGVVGISKPHYDIWGNPVNMASRMDSTGVPGQIQVTENTALKLREFNIQ
CNYRGMTFVKGRGNIPTYIIGIDSEYQFLPHRPAAPTKED (SEQ ID NO:114)

# Figure 51

**Name:** Aats-ala-m
**Nucleotide:** AE003567
**GI:** 10728137
**Transcript:** CT14952
**Sequence:**

ttatctcgagcatttttaattgccggtgcggttttaattgaactgcggcatgtacaactcggccaagcagctgcagcgcgt
gctgacagcgcgcgagattcgtaaaaccttttggatcatttcaccgtcaatcatggccacaagttcgttcgctccagcccggtggtgcccttt
tgcgatcccactgtggctttcgttaacgcgggcatgaatcagttcaagtcggtgttcctgggcacagcagcggcgccacacaagcgggttg
tcaactcccagaagtgcgtccgcgttgggggaaagcacaatgatctctccgtggtgggcacggatggctatcatcacacgttctttgagat
gctgggcaactggtcctttggggattacttcaagcgggaggcgtgtgcaatggccctggagctgctccgtggtccctacaacattgatcca
ggtcgtctatacgtcacatactttgctggcgacaaggtgctgggcattccagccgatctggagtgtttcgagatctggaggagcctgggcttt
cccgcctcgcgaatactgccctttggctgcgccgacaattttgggaaatgggcgctacagggccgtgtggtccctgcaccgaaatccaca
tagatcatcgcccggatttgggcagcgtggagcagcgagccaagttggtgaacgcaggtcgttccgatctcacagagctttggaatcttgt
ctttatacagtacaatcgtcatgccgatggcagcatctctcaattgcccgctcatcatgtggacaccggcatgggttttgagcggctaacggc
tgtgcttcagaataagtcttctaactatgatacggacttgttcacgccgatatttgatggcatccagcaggcggccaagactccggtttacagt
ggcagctttccggatggtggaaatgcagcagtactcgacaccagctataggatactagccgatcatgcacgcatggtgaccgcctgtttg
gccgacggcatgcttccagatcaaaatcagaaattgcgtcgcgtgctgcgcaaagctctcaacatttcggagcatgtgtttgcccacgaca
aattgctgacccagctagttcctattgtggtggagacacttggtgaagcctatccagagatggcggctaagcagcaggctgttatcgacttg
atctgccacgagcaggaggtctacaagaatctaagagagagttcttccaaagcgtttgcagaggtgcttatggagtttcccaatctggatg
acatcgatctgatggagtgcccgggattcgtgcccgcctatcgagagttgcagatgcagcgctgcaagttttccaataacacaataccag
gggacttcctttacaagctgaccgatacctacggcctgaccgaggagagtttcctcaagctggctgaattggagaacatgaactgtgatttg
gagcgctacagggcggaggtcagtctggcgaagctcaaagccaagggcaaccaaagggagactgctggaggatcactctgcgatct
ggccacggagcagcgtattgctgaagctcaggcgatgctaacgaagcgtctgacacccaccgacaacagtcacaagtacacctactc
cttcgacaaggagagtgattcataccaaatacccccactaaagactcgagtcctcggaatgctcctcaacgatgcggaagtgtcacgaa
cccaaggcagtcgcatacagcaacccttcaccgaccttatttccattgtgaccgctggcagcaatttctactatgaatccggaggtcaaca
atcggatggcggaaagatccttgtgagcaatcaccaacagccagaccatcctcattcactagacgtaatcggtgtaaagcatctcaacg
attgcgtagttcacatctgcaagttatccagtcccaccgacgcctttcagctggccattggggatgaggtggagctgcaggtggatgcgca
gcagagacagctcaacacgtgccatcacacagccacgcatctactaaacgccgccatccgatcgcttttcaagaaggtcacctaccag
gtctccagttctgtgagcagcgatcagtgcaagctggagctgggactcctgggcaagcgcattcaaaagaccgatgtacagctcatcga
ggatctgatcaatcgcgttatctgctctgctgcgccggttgaagttcaattgctgagtgcagctgaagttctggagcagaatgacatcacaat
ggtgcccggcgaggtttatcctgagcagggattgcgtctagtcaacgtggagagtcctgaactgcagctgagctccaaggaactgtgctg
cggaacgcatgcaaccaacaccagtgagctttcgtgcttctgcatcgtgaatctcaagcaaaccaatcgtgcgagattcgcatttaccgcc
gttgctggccaggcggcggaaaatgtgctgaaaacggctgccctgctgcgacaccgcgttgatctcctcgagaagcagttccaaactga
caaactcacaaacgctacggaggcggaactgcagacgattcggcacaacatgttgcacacggacattaaactgccatacgcttttaag
atggacacgctggaacgcatcaccgagatgctgaagagaataaaagattcatcgcgcacaaccttaaaagaatttgtggacgtagaga
tgcgcacgctgctgcaggagaagccgttggacacacaccccttcattctgcactacatcaccagttccgcgctcgtggaggagattcctttg
caacgagccaccaagctgtgcccggatcggcccatcctcgtaatcagcgtgtgtgatagcgtggtcaaggctcgctgttgtgtaccagaa
aagtgtatcacggagaaattcaacgcttccgcctggctgcaatcctttgccgacacctttaatggacaaatagccgcgcccaagggccaa
aatccgcaggccgtgtgcaacatgaagggtcgacgggtgagcaatctgttcgaggaacagttggagcaggcaatgtccaaggcacat
gcatacgctaagctttatctataggtataagttgtgattaccctccaaaatgtgctagctgtaagcttgccctttgaataattgctcatgtgagga
gcgagtaaatggcgaagttttaaggaa (SEQ ID NO:115)

**Protein:** AAF50804
**Protein GI:** 7295490

MYNSAKQLQRVLTAREIRKTFLDHFTVNHGHKFVRSSPVVPFCDPTVAFVNAGMNQFKS
VFLGTAAAPHKRVVNSQKCVRVGGKHNDLSVVGTDGYHHTFFEMLGNWSFGDYFKREACAMAL
ELLRGPYNIDPGRLYVTYFAGDKVLGIPADLECFEIWRSLGFPASRILPFGCADNFWEMGATGPC
GPCTEIHIDHRPDLGSVEQRAKLVNAGRSDLTELWNLVFIQYNRHADGSISQLPAHHVDTGMGFE
RLTAVLQNKSSNYDTDLFTPIFDGIQQAAKTPVYSGSFPDGGNAAVLDTSYRILADHARMVTACLA
DGMLPDQNQKLRRVLRKALNISEHVFAHDKLLTQLVPIVVETLGEAYPEMAAKQQAVIDLICHEQE
VYKNLRESSSKAFAEVLMEFPNLDDIDLMECPGFVPAYRELQMQRCKFSNNTIPGDFLYKLTDTY
GLTEESFLKLAELENMNCDLERYRAEVSLAKLKAKGNQRETAGGSLCDLATEQRIAEAQAMLTKR
LTPTDNSHKYTYSFDKESDSYQIPPLKTRVLGMLLNDAEVSRTQGSRIQQPFTDLISIVTAGSNFYY
ESGGQQSDGGKILVSNHQQPDHPHSLDVIGVKHLNDCVVHICKLSSPTDAFQLAIGDEVELQVDA

QQRQLNTCHHTATHLLNAAIRSLFKKVTYQVSSSVSSDQCKLELGLLGKRIQKTDVQLIEDLINRVI
CSAAPVEVQLLSAAEVLEQNDITMVPGEVYPEQGLRLVNVESPELQLSSKELCCGTHATNTSELS
CFCIVNLKQTNRARFAFTAVAGQAAENVLKTAALLRHRVDLLEKQFQTDKLTNATEAELQTIRHNM
LHTDIKLPYAFKMDTLERITEMLKRIKDSSRTTLKEFVDVEMRTLLQEKPLDTHPFILHYITSSALVEE
IPLQRATKLCPDRPILVISVCDSVVKARCCVPEKCITEKFNASAWLQSFADTFNGQIAAPKGQNPQ
AVCNMKGRRVSNLFEEQLEQAMSKAHAYAKLYL (SEQ ID NO:116)

# Figure 52

**Name:** gek
**Nucleotide:** AE003463
**GI:** 7291737
**Transcript:** CT13314
**Sequence:**

aaacggaacactacaattcgacaacagttcggcttttctttttgcactatttcggataaatatggtttaggccagacggag
caataactaagatggaatacgaatcttccgaaatcagtgatataaccacaggaagctgcaagaaaaggctcacatttctgaaatgtattct
aagcgacaccacaagcgatcaaaaatgggcggccgagttcggcgaggacacggaaggacatcagttttcgttggactatctgctggac
accttcatcgttctctatgacgagtgcagcaactcctccctgcgcagggagaagggcgtctctgatttcctcaaactatccaaacccttcgtg
cacatagtgcgaaagcttcgactaagccgagatgactttgacatactcaagatcatcggacgcggtgcctttggagaagtgtgcgtggttc
agatgatatccaccgaaaaggtgtatgccatgaagattctgaacaagtgggagatgctgaagagggccgagacggcctgcttccgcga
ggagcgggatgtgttggttttcggcgaccggcagtggatcaccaaccttcactatgcctttcaagacaatattaacctgtatctggtgatgga
ctattactgcggcggagacctgcttacattgctcagcaaattcgaggacaagctgcccgaggacatggccaagttctacatcacagagat
gatcctggcaatcaatagtattcaccagattaggtacgttcacagggacatcaagccggataatgtactgctcgataagcgcggtcacgtg
cgcctggctgactttggatcctgcctgcgcctggacaaggatggcaccgtacagtccaacgtggccgtgggcacacccgactacatttcc
ccggaaattttgagggccatggaggacggaaagggacgctacggcacggagtgtgattggtggtcactcggagtgtgcatgtacgagat
gctctacggagagacgcccttctacgcagaaagtttggtggaaacctatggcaagatcatgaaccaccaaaactgtttcaacttaccgtc
acaagaaaccctaaattacaaggtatcggagacgtctcaggacttgctctgcaaactgatatgtattcccgagaaccgacttggtcagaa
cggcatccaagacttcatggaccacccctggtttgtgggcatcgactggaagaacataaggcagggaccagcaccgtatgtgccggaa
gtgtccagtccaacagatacctccaacttcgatgtggacgacaatgacgtccggttaacggactccattccgccgtcagccaatcctgcctt
ctctggatttcacttgcccttcatcgggtttaccttctcgctgaccagcagctccaccttggactcgaagaagaaccagagctcgggcttcgg
agatgataccttggacaccataagcagtccccagttggccattctgccgagcaacaactcggagacaccggtggactccgttcagctga
aggcactaaacgatcagcttgcggctttgaagcaggaaaaggcagagttgtcaaagcaacacaacgaggttttcgaacggctcaaaa
ctcaagactctgaacttcaggacgccatctctcaacgaaacatcgccatgatggagtactcggaggtcactgagaaactctcggagctgc
gaaatcagaagcagaagctctcccgccaagtcagggacaaggaggaggagcttgatggtgccatgcagaagaacgacagcctgcg
gaacgaactgagaaagtcggacaaaacgcgaagggaattggagcttcacattgaggatgctgtcatcgaggctgccaaagaaaaga
agctgcgggagcatgcggaggactgctgcaggcagctgcagatggagctgcggaagggctcctcgagtgtggagacaactatgccac
tcagcatttcctccgagatgtcgtcatacgagatcgaacgccttgaactccagttctcggaaaagctaagtcatcagcagacgcgccaca
atatggagttggaagcactgcgcgagcagttcagcgagctagagaacgcgaatttggcactcaccaaggagttgcaacaaacacagg
aaaggctgaagtacactcaaatggagtcgattaccgattccgccgaaacgcttttggaattgaagaagcagcatgatttggagaaaagct
catggttcgaggaaaagcagagattaagctccgaggtgaatctcaagtcaaagagtcttaaagaacttcaagcggaggacgacgagat
cttcaaggagttgcgaatgaaacgggaggccatcaccttgtgggagcggcaaatggcggagattattcaatgggtgtccgatgagaaag
acgcgcgaggctacttgcaggccttggccaccaaaatgacggaggagttggagtatctgaagcatgtgggtaccttcaacaacaacggt
gtggacaataagaactggaggaaccggcgctctcagaagctggacaagatggagctgctcaacctgcagagcgcgctgcaaaggga
gattcaggccaagaacatgatatcggatgagctgagtcaaaccagatcggacctcatatccacacagaaagaggttcgagactacaaa
aagcggtacgactccatactgcacgatttccagaagaaggaaaccgaactgcgggacttgcagaagggaggcttggaatactccgagt
cgttcctcaacaaatcaacgcatcatgggctaagcagcgccttcttccgcgacatgtcaaagaactccgagattatagattctgccgagag
ctttggcaacgaatccggcgacaatttcactcccaacttcttccagtccggcaattcgggcatgctcttcaactacgagccaaagtatgcgg
gtaagaacaacaaggatcattcgtccatgaaagaagcctcggtcagcgatttgtcccgcgaggagagtgaccagctggtcaaggaatct
cagaagaaagtgcccggcaacacggcgatccatcagttcctggtgcgcacattcagtagtcccacaaaatgcaatcactgcacatcgct
gatggtggggctcacacgacaaggcgttgtgtgcgagatctgcggattcgcctgtcacacgatctgctgccaaaaggtgcccaccacgtg
ccccgtgcccatggatcagacgaaacggccgctgggaatcgatcccaccaggggaatcggcacggcatacgagggctacgtaaagg
tccccaagtcgggggtgattaagcgaggatggattcggcagttcgtagttgtttgcgactttaagttgttcctctatgacatttcgccggatcgg
tgtgcattgcccagcgtgagcgtgtcccaagtgctggatatgagggatcccgagttctcggtgggtagtgtgcgcgaaagcgatgtcatcc
atgccgccaagaaagatgtgccatgtattttcaagataaaaaccgcccttatcgatggcggtctttcgctgaacacccttatgctcgccgac
aacgagtcggagaagtccaagtgggtcattgccctgggagaactacatcgaatattgaaacgaaacagcttaccaaatactgctatattt
aaagttaacgagattctggacaataccctgtctttaataagaaacgcattgtgttctgtcatcatatatccaaatcaaatactcttgggcaccg
aagacggcctgttctacattaatctggaccagtacgagatcgctcgtattggcgaaagcaagaagattcttcagctatggtacattgaaga
ggagcagatcctcgtcattctctgcggaaagcagcgcaacttgcgtttattacctataagggcattagaggctagtgatgtcgagtggatca
aggtggtcgaatcgaagaactgcatatctgcttgcactggaataatccgccgcttccccaatatcgtttactcatttatcatcgctctaaagcg
gccgaataaccacacacaaatcgtagtttacgaaatcaatagaacgcgcacaagacaccagaagacttgcgaattcacaattggctac
atggcgcagcacctgcagattctgtccgacatgcgattggttgtggctcaccaaagcggatttaccgcatactttctgcgcggagaagcaa
ctgcaatgtctttggttcatccggagaaccagttatgtgcatttttgaactactccggagtggatgcggtcagggtcattgaaattctgtgcccc
agcggcggcaatttcggggagtacttactggtgttccagactctggccatatacgtagacctgcaagggcgaaagtctcgggataggga

aattatgtatccagccttccctacgtatataaccttttgtgatggtcacttgctggtgttttcggatacacatttggatatattcaatacgcagactg
ccgaatgggtgcagtcgattggattgaagcaatcacttcccttaaacaatttgggaaacgtggtgctgtcctcagtcaatgacactccgctg
attgtttacctatccaatattcacacaaagggcctttgcaataccgtgacggaaatcgaaagggacttccgagcataaagcggagattctc
tatcagggagataaacaagaccatcaaaagtgatcgtagatcaaagatgatatcagcaccgactaatttcaaccacatttctcacatggg
gccaggggatggcattcaaaatcaacgactcctagacttaccaaccacgctcgaaacagccgatcaagcctgcagtccaataatacatt
cgctgagttgcataccccagagtagaaaatccaattttctagaacaagtggatgcaaactccgatgactatggcaatgacaacattatctc
cagaacgcccagtccaatggcatcgtccttcatggacggcttaagcaataatgattaacttcagtcaatctctacacgtgtaatttaatcgatt
ttttaagataactgtttgtaataaacatatttaatttctcgaatatcaa (SEQ ID NO:117)

**Protein:** AAF47163
**Protein GI:** 7291742
MEYESSEISDITTGSCKKRLTFLKCILSDTTSDQKWAAEFGEDTEGHQFSLDYLLDTFIVLY
DECSNSSLRREKGVSDFLKLSKPFVHIVRKLRLSRDDFDILKIIGRGAFGEVCVVQMISTEKVYAMK
ILNKWEMLKRAETACFREERDVLVFGDRQWITNLHYAFQDNINLYLVMDYYCGGDLLTLLSKFED
KLPEDMAKFYITEMILAINSIHQIRYVHRDIKPDNVLLDKRGHVRLADFGSCLRLDKDGTVQSNVAV
GTPDYISPEILRAMEDGKGRYGTECDWWSLGVCMYEMLYGETPFYAESLVETYGKIMNHQNCFN
LPSQETLNYKVSETSQDLLCKLICIPENRLGQNGIQDFMDHPWFVGIDWKNIRQGPAPYVPEVSSP
TDTSNFDVDDNDVRLTDSIPPSANPAFSGFHLPFIGFTFSLTSSSTLDSKKNQSSGFGDDTLDTISS
PQLAILPSNNSETPVDSVQLKALNDQLAALKQEKAELSKQHNEVFERLKTQDSELQDAISQRNIAM
MEYSEVTEKLSELRNQKQKLSRQVRDKEEELDGAMQKNDSLRNELRKSDKTRRELELHIEDAVIE
AAKEKKLREHAEDCCRQLQMELRKGSSSVETTMPLSISSEMSSYEIERLELQFSEKLSHQQTRHN
MELEALREQFSELENANLALTKELQQTQERLKYTQMESITDSAETLLELKKQHDLEKSSWFEEKQ
RLSSEVNLKSKSLKELQAEDDEIFKELRMKREAITLWERQMAEIIQWVSDEKDARGYLQALATKMT
EELEYLKHVGTFNNNGVDNKNWRNRRSQKLDKMELLNLQSALQREIQAKNMISDELSQTRSDLIS
TQKEVRDYKKRYDSILHDFQKKETELRDLQKGGLEYSESFLNKSTHHGLSSAFFRDMSKNSEIIDS
AESFGNESGDNFTPNFFQSGNSGMLFNYEPKYAGKNNKDHSSMKEASVSDLSREESDQLVKES
QKKVPGNTAIHQFLVRTFSSPTKCNHCTSLMVGLTRQGVVCEICGFACHTICCQKVPTTCPVPMD
QTKRPLGIDPTRGIGTAYEGYVKVPKSGVIKRGWIRQFVVVCDFKLFLYDISPDRCALPSVSVSQV
LDMRDPEFSVGSVRESDVIHAAKKDVPCIFKIKTALIDGGLSLNTLMLADNESEKSKWVIALGELHR
ILKRNSLPNTAIFKVNEILDNTLSLIRNALCSVIIYPNQILLGTEDGLFYINLDQYEIARIGESKKILQLW
YIEEEQILVILCGKQRNLRLLPIRALEASDVEWIKVVESKNCISACTGIIRRFPNIVYSFIIALKRPNNHT
QIVVYEINRTRTRHQKTCEFTIGYMAQHLQILSDMRLVVAHQSGFTAYFLRGEATAMSLVHPENQL
CAFLNYSGVDAVRVIEILCPSGGNFGEYLLVFQTLAIYVDLQGRKSRDREIMYPAFPTYITFCDGHL
LVFSDTHLDIFNTQTAEWVQSIGLKQSLPLNNLGNVVLSSVNDTPLIVYLSNIHTKGLLQYRDGNRK
GLPSIKRRFSIREINKTIKSDRRSKMISAPTNFNHISHMGPGDGIQNQRLLDLPTTLETADQACSPIIH
SLSCIPQSRKSNFLEQVDANSDDYGNDNIISRTPSPMASSFMDGLSNND (SEQ ID NO:118)

# Figure 53

**Name:** CG3216
**Nucleotide:** AE003452
**GI:** 10726992
**Transcript:** CT10631
**Sequence:**

atgcatctgcttgggatcagcatccacttcttcttcctgatgtacgtcaactgctttagtgcccatccgaatcccaggcgca atgatataacctgggatgatctgaacaaggatattagtttggactccactacttccttggcggggctcaatgccagtgatgcgggcctagag caaagaatgtacgagaggagcagggaatccaagtctacgcagctttctagatacactgaggtgggcgaaatgggctccacaatgcgtg tctataacgttggcgttctgatggcttctcatttggattctccattcgatctggagcgttgtggaccggccgttgacttggccttggatgagattaa caaggtttcctgaagcctcacaacataacactactgaaaaagaagggaagctatccatcctgttcgggtgctcgggctcctggactggct gcggacatgtacttccaggacgacgtaatcgcgtttattggaccagcctgcgcctttgcccttgagccggtggcacgtttggccgcctattgg aacaagcccatcatcaccggcatgggagatcagccgccttcctcggaaggtgagctgacggtcacttccggaatcctgggaaggatac acaagtggaagaatgagaatacgggcatgttcaaggacaagtccaagtacccgacgctcacccggatgtcctactgccagtgccgcct catcctggtctttgccagcgtcattcggcagttcaactggaatcacgttgccctgctggtggacagatcggagctcttctcgtggacggtggg caagaatctggagtacggtctgcggcaggagggactcttgagcttcgtcaaggagctcaacggcaacgaggaggaggtgtacgaaaa ctatctaaaggacgccagcatgtatgccagagtggtcatcctgtcggttcgcggcgttcttgtgcgaaaattcatgttggcagcccacagcct gggcatgaccaatggcgagtgggtgttcctggacgtggaaatctttcagagcgaatactggggcgacaagggttgggagatgaaggat gagcacgatgccaaggcgcggaaggcatacgaagccctcctgagagtcagtctcctgcagccgacaagtcccaagttccaggactttg ccgacaatgtgagggagaatgcgctctacgattacaactacacgtttggcgagggcgaggaggtgaacttctttattggagccttctacga tggcgtctacctgctgggaatggccttgaacgagaccctcaccgagggtggtgacatccgggatggggtcaacatcaccaggcggatgt ggaatcgcaccttcgaaggtatcacggggcacgtgcgcatcgacgacaacggtgaccgggatgctgattactccattctcgatctggatc ccatcaatggaaagttctccgttgtggcccattattccggtgttcacaagcagatgaagctcagcaaggagctgaataatatgtcctggcgc gtacgacccgatgatgtcctcatcgagatgggcggcatgtttggttcgaagggaggcttgcagcgcctggatgtggagaacatatcgctgc agcagttcggtatccattcgggacgcgcatcgatagccagcttcacgtcgctgccgccgcaagtgtacaccaccattggccagttcaagg gcgaacgggtggccatcaagaaggtgaacgtgaagaaggtggacctgacgccgcagctcctttgggagatcaagcaggcgcgggat gtgagccacgagaatacggtgcgtttcgtgggcgcctgcatcgatctgccgcgacccaccgtgctcatcctcaccgaatattgctcgagg ggcagtctgaaggatgtgctggagaacgaggccatcgagttggactggaactttcgcatgtctctcatccacgacattgtcaagggcatga actatctgcacaatagcgatgtggctgcccatggaaagctgaggtcttgcaattgcctgatagacggacgattcgttctcaagatctccgac tttggactgagaacgctgaccacgccctctgattttgtgcgggatcagaactactacctcaccacgcagcgcggcgatgtgtactcctttgg cattattctggaggagatcgtcaaccgtggtggaccctaccaggaggcgcgccagcagatggacgttcacacgatcctgcacaaggtgc gccagtgcaatggattccgtccgctcatcagagaacgcgagtgtccacctgatctgctggagctaatggagaagtgctgggcagacaat caggaggagcggcccactttctccaccatacgcagtaatattcgcaccataatgaaaggcttctgtgagaatctaatggatgatctactga atcgcatggagcagtatgccaacaatctcgagagtctcgtggaggagaaaacccgccagttgagcctggaaaagcagcgtacggagg agctgctctaccaaatcctgccgcgtcctgtggcccagcaactgatggccggcgatcttgtggagccggaggagttcagcagcgtgacc atatactttagtgacatcgttggcttcaccgaactgtgcgcccgtagcagtcccatggacgtggtcaacttcctgaatgacttgtatagcacat ttgaccgaatcattggcttctacgacgtctacaaagtggaaacgattggtgatgcctatctggtggtttctggattgccggagcccaatggtg acaagcatgcccgcgagattgctctaatggccctggacatcctacgagcagtcagctccttcaatctccgccataaaccggaatacaag atacagattcgcattgggatgcattcgggttccgtgtgcgccggagtcgtgggcaagaagatgccgcactactgtttgtttggcgacacggt caacacggcgtcgagaatggagagcactggccagccgggcaagatacatgtgtcctcggccaccaaggccatcctggacaagttcgg caccttccaaatggagcagcgcggcgatgtcgagctaaagggcaagggcacggtcaccacctactggctgaatagcacctccgaggg cgaggcgcgtcctccgactccgcagatcctgaccaccgatgaggtgcccttcccgctgctcttcgccggcatgggaaagtag (SEQ ID NO:119)

**Protein:** AAF46649
**Protein GI:** 7291217

MHLLGISIHFFFLMYVNCFSAHPNPRRNDITWDDLNKDISLDSTTSLAGLNASDAGLEQRM YERSRESKSTQLSRYTEVGEMGSTMRVYNVGVLMASHLDSPFDLERCGPAVDLALDEINKVFLKP HNITLLKKKGSYPSCSGARAPGLAADMYFQDDVIAFIGPACAFALEPVARLAAYWNKPIITGMGDQ PPSSEGELTVTSGILGRIHKWKNENTGMFKDKSKYPTLTRMSYCQCRLILVFASVIRQFNWNHVAL LVDRSELFSWTVGKNLEYGLRQEGLLSFVKELNGNEEEVYENYLKDASMYARVVILSVRGVLVRK FMLAAHSLGMTNGEWVFLDVEIFQSEYWGDKGWEMKDEHDAKARKAYEALLRVSLLQPTSPKF QDFADNVRENALYDYNYTFGEGEEVNFFIGAFYDGVYLLGMALNETLTEGGDIRDGVNITRRMWN RTFEGITGHVRIDDNGDRDADYSILDLDPINGKFSVVAHYSGVHKQMKLSKELNNMSWRVRPDDV LIEMGGMFGSKGGLQRLDVENISLQQFGIHSGRASIASFTSLPPQVYTTIGQFKGERVAIKKVNVKK

VDLTPQLLWEIKQARDVSHENTVRFVGACIDLPRPTVLILTEYCSRGSLKDVLENEAIELDWNFRM
SLIHDIVKGMNYLHNSDVAAHGKLRSCNCLIDGRFVLKISDFGLRTLTTPSDFVRDQNYYLTTQRG
DVYSFGIILEEIVNRGGPYQEARQQMDVHTILHKVRQCNGFRPLIRERECPPDLLELMEKCWADN
QEERPTFSTIRSNIRTIMKGFCENLMDDLLNRMEQYANNLESLVEEKTRQLSLEKQRTEELLYQILP
RPVAQQLMAGDLVEPEEFSSVTIYFSDIVGFTELCARSSPMDVVNFLNDLYSTFDRIIGFYDVYKVE
TIGDAYLVVSGLPEPNGDKHAREIALMALDILRAVSSFNLRHKPEYKIQIRIGMHSGSVCAGVVGKK
MPHYCLFGDTVNTASRMESTGQPGKIHVSSATKAILDKFGTFQMEQRGDVELKGKGTVTTYWLN
STSEGEARPPTPQILTTDEVPFPLLFAGMGK (SEQ ID NO:120)

## Figure 54

**Name:** CG5653
**Nucleotide:** AE003553
**GI:** 10728037
**Transcript:** CT17866
**Sequence:**

atggaaaaatgccgtgcctcctctaggataatcataatcggagcaggagtttcgggcatagccgccgccactcgact
cctgcaaaacaatttccagaatgttcagatcctggaggcggaggatcgcattggcgggcggatcaacaccgtgtattttggtgacaacgtc
atcgatttgggagcccagtggtgtcatggaaagcagcaaaattgtgtatacgacatggtcaaggatatgggtatcctgcacgagacaggc
gactattacagtcccataaagagggtgagatccaacaaggaggtggttccacatgagttggcctgcaggattcacgacattgctgtgaag
agcatgccgagtggacctcacccggttgtgggatcctttggcacccacctgactcagaccttctggcgcaagatcgagtccgagctaccc
caagtgaatagggatgtggctagtgaggccttgaatacatttgcgaagcatgaaagctctattattggtgcggacaatcttttcgaggtctcc
gttagggaacacatcgagtaccacgaatgtgatggcgacaagctgctccactggggaacgaagggttaccggcgcttcctgcggctgct
gatgaaggtcagcgcggatacgccagaagagctgggtctactggaaggacgtattcaacttgatatgaaggtcatcaagattgagttggc
gtgcccccgtaaggtaatcctgcgctgccaggatggggattacttcgaggcggaccacgtcatctgcaccgtttccctgggagtcctgcag
gagcagcacgagaaactgttcgtcccgccgctgcccgcagccaaggtgaatgccattcgcagcctcaccctgggcactgtgaacaaac
tgtatttggagtatgagaagcaacctcttcctgacggctgggtgggtttcttctgcttttggctggaagaggatctgatagagctgcgaaagac
ggaatatttctgggtggagggtataaccggtgtacacatgatcacttgtcagccccgaatgttgatggcctgggtgaatggtccgcatggac
gccacatggagaccctgtccgatgagaaagtccttgagggcttgtactggcttttccgaaagttcctcactttcgaaattccgccgccaaag
cgtttcgttcgcagctcgtggttttcgaatccgaatttccgtggcagttggagttatcgtggagttatggccgatgaaaggaatacgggcccct
gggatctagagtctcctgtgttgggcgaagatggtcatttaggcctccttttttgccggagaagcgtctagcaggaatcacttttccaccgttca
cggagctgtggaggcgggctatcgggaggcagaccggctcattgatcattacacctcatgcagtgtcagtgcctga (SEQ ID
NO:121)

**Protein:** AAF50345
**Protein GI:** 7295017

MEKCRASSRIIIIGAGVSGIAAATRLLQNNFQNVQILEAEDRIGGRINTVYFGDNVIDLGAQ
WCHGKQQNCVYDMVKDMGILHETGDYYSPIKRVRSNKEVVPHELACRIHDIAVKSMPSGPHPVV
GSFGTHLTQTFWRKIESELPQVNRDVASEALNTFAKHESSIIGADNLFEVSVREHIEYHECDGDKL
LHWGTKGYRRFLRLLMKVSADTPEELGLLEGRIQLDMKVIKIELACPRKVILRCQDGDYFEADHVIC
TVSLGVLQEQHEKLFVPPLPAAKVNAIRSLTLGTVNKLYLEYEKQPLPDGWVGFFCFWLEEDLIEL
RKTEYFWVEGITGVHMITCQPRMLMAWVNGPHGRHMETLSDEKVLEGLYWLFRKFLTFEIPPPK
RFVRSSWFSNPNFRGSWSYRGVMADERNTGPWDLESPVLGEDGHLGLLFAGEASSRNHFSTV
HGAVEAGYREADRLIDHYTSCSVSA (SEQ ID NO:122)

# Figure 55

**Name:** CG17740
**Nucleotide:** AE003822
**GI:** 10727606
**Transcript:** CT39323
**Sequence:**

atgcttcgccatatcgtgctcctcattctgctgtcttccgaagtcagaggagtctgcaatcggataccccaaggtgccagt
ggccctcgatcgcctgtggatgataactttaagatccttatcgatggcaatccagagacctatgtgccagaacatcagtacaatgtttcgttat
cctgtccaataaacatgaaattcgtgagcttcaccttggtggtcgaggctgaggatccgtcggctgccttcggtggtcaggatatgaccggt
cactttgaactgctgggagtcggagacacccgattcagtaccagttgtgagaacatggtggagaacacaaatacgaatgccaagatcta
tatcgcggtatcctggatagcaccacgtaatccggacagcggttgtgtactgattaaggctggtgtggttcaacatcgtgatgtgtggtttctg
gacgatggcttcctgaccaagcggatttgtcccgaggaaatcgacgaactgaacagcttaacaccacccttggaaacttgctgtgcctgtg
atgaagccaaatatgagattgttctggagcgaaagtgggcaaggaacacccattggaaggactttccctctgaggactggcgcacacgc
ttgggcgaggtgattggtgcttcacatagccacagctatcgctactgggcctacggaggaagagcgagcaagggcatgaaggagttgg
ccgagcacggagctacacgtacccttgagaatgagatcagggagaatactcagaatggcgaggtgagaactataataaaggctcctg
gaattccctatcgtccgaaaacatttggcacaaccctggccaatgctcgtctggatccagtacaccatcagatatcgctagcagccaagat
tgatccttcacccgattggatactgggcgtggctggattggagctttgcttgagcaattgtacgtggctggagcgaaagtcctcagatgagcc
acaagtgccaccggatgtggtgcgccgtataacatcttcgtatcccagcgaccatcgttcgccattttacgatgacaccggtgccccaatg
aaacccttggccacactctacttgacccggaagaaactctacattagggagtgcgaagaggtattagttccccaggaaggaccgctgga
gtgcgctgtacacccttggaacgagtggaccaactgcagcacgcgatgtggtcagggctactctcatcgtcagcgtagcttcaagaatcc
caatctagctgccaactttaactgcgataggcgcttggaggagttccgccaatgccagggaacccaatgtggggcagcggaggaggaa
ctggaaggcgaggagcctggcctgggcatggaaaatcccccaagtagtggctcaatggccgagtgccagctgaccaactggggcga
gtggtcaccgtgcacgaagacatgcggcagaggatcttccacacgtacccgggactattacaatccgcaggccaggcagcgctgtttgt
ccgtgatgcgaataccgctcgaggaaactcgggagtgcattggatcagattgtggtggcaccattccggataatggcgaactcgatggctt
tccggaaccagatgtgttcggagagacggatcacggcggttacatgggcaaaaccacttcttggaacagtaaacaggagcattccagtc
taggttccggcaaccaggcgtggaatagaaagccctacaattcgtggaaagataaatctctttccaaccctagagataacgcacaggag
tccccaaacttaccgttcgatcagatagaaaatcctcagagacctgcgtataatccatctgacaatatgggtggttatgacaagggctttcct
ccgacaaatagctacagcaatgacaagcttgtgggcaatagacccggctacaatgacttcaattcggactataacaacaaggacgctta
caatgattacactaccccctagttttacatctcgcactccctttggaagtcggtatcccaatcagcaggagactgtggagggaaataccaacg
atatcaatggcaactataatgtggttcaagattattgctttgaaaagccatatgcctcgactcttccctgtttcgccaaaccggtggtggagag
caattactggttctacgatcacgaagatcacgagtgcaagatcttcaccacggacaactgtgatcagaatagaaaccgattccgtaccct
gacggcctgcgagggcacctgtctgcagccccaaataaacatggagcgatccgaaaacgatgccatggacttgtacggaaggagatc
ctatgaccaaaaacaaacgagagatcatataagcaaacaggaggtggatcctatgatcaaccagaagataggtcctatgacttatcga
cagcaggaggtagatcctatggagaaaccagtgaagcaggtgatattggagaacccatgtcgcagaccaggagcagatacgacaca
tccagacgcgaacacgaacatgtggtggtcaagtggtgctcctctcgtggagctacttctgcttgggtccctggtgattggtagagtcagctc
cctgatatgcacgcgccgtccagcgaacacgggctcgcccaaatctccggtggatgagaactttatgataagcgtgtcgggtaatccgga
aacgtacattctcggccaggaatacaacgtctctcttaatgccttcaatggccaccgctacattagctttatcatggcactggaaaacgaga
atggtgactttagctacaacgatgacttgggtcgctttgagctgagcgacttgattgagacccggttcagtccgaactgcatcaacatggtgg
agaacaccaatacgaactccaagacgcacatgcacttgacttgggtggcgcccagtgagccgggcagtggctgcgttctgatccgggc
cacagttcagcagcatcgcgaggtgtggcacatggatgatggcggtttaacaaaacgcatctgcgaggaggtcaccgatgatgtggaa
agtcagccaactgctccggcggcagtggatgtgccctgctgtgcatgcgatgaggcgcgctatgagcttatatttgaaggcgtctggtcca
ggaacctgcatcccaaggactttcccactcgaggctgggagactcgattctgtgagcttctgggagccgcccacagttcggattatcgtttct
gggagtccggagaactggccagcgtggctatgaaggaatatgcggaacattgcagttcgcgtctgctggagcgagagttcagcattaatt
ttcgggatcaaaagatacggacgataatcaaggcacggggcccatcgtatcccaacataagcagcaagacaatggcctcggtgcgag
tagatcccattcaccacatggtgtcctttgcctccaagatagaaccctctccggattggattgtgggcgttactggactggaactatgcctgcg
caactgcacatggatggaggaaaaagtgatcaacctatatccgtgggatgtgggcacagattcgggtcccacctatacgtcatccgatca
gccccaagttccgccagatgtcattagacgcatgcgttccgactttcccaatgatccgcgctctcctttctacgacatatccggaactcccatg
aaacccatggccattctgacggtgaagcgccagcgaatctacgagagaagatgtgcagatgaggactccaacaacgatcccgatgtg
ccgcgtgaatgtttcacccatccatggtccagttggagcgactgctcttccaaatgcggggttggcatgcagtatagaagacgtgtctataa
gcaaccggagctcgcaagggtctacaattgcaatgaggcgcagtatgaggagcgcgagtgccagggcgagatgtgtggtcaaaataa
tctcatgcgggagccggaggaatttgaggacctggagccagatccaaggaggattggttatcaaccgtcacaacaacgccgtgccgaa
tgcgaactcagttcctggggcagctggagcccatgtagtgtaacctgtggcgatggttacgagatgcgccagaggcagtaccttaatccc
caagcagagttcgattgccagggtgtacatcgcatggagctgcaggaaacgcgaaaatgctctggtcgagattgccttggtagcttacca
ggatcctacagcaccgacatggatagtccctatggcggggcttctccaggacgaattggaggcaatatgtatggaccgcaacttgaacc
cgaggcagagaactttggtgactacgatgactcacgaaatggagcagttggtggattcgaagtgggcaatcttaagggcaacattggca

attgggccttgaacaggcaaacaaatttgggaagagatgttccgtcgcttaggcccacaaactacgatagagcccgccagcaagcaga
aaaatcagaacgaaccacgtggtctgggcagtcacgtcccgatgatccgcgcttggaggatgtggaacgtgctccttggcatcgtcaaa
ggccctccaacaaatacggctcaatcgtagaggcgcccgaaatggtttcgccaagggaaccatggcaaagggcaaacaactttggca
atccggacatgtatacacctggaggagatgtggacccctcgctgtcctaccgctgcttccaaatggttcagacggctcagccacgctgcca
cgatcaaactattacgggtcacttttggttctacaacttctgtgcggatgagtgcatgctgtatgccgcggatcagtgcgatcggaatgtgaac
aagttcaggcggtgggaggattgcgagaaatgccggcagccaggaatggctgcactgcagcaggagcacaccaattcgccacagtg
cgaggatttccgtgccatttggcaggcggagcagcgggctcgggaggaaaagcggccatcaaaccgcaggcgcaactatggcgattc
cagatatcggactagaaattag (SEQ ID NO:123)

**Protein:** AAF58535
**Protein GI:** 7303479
MWWSSGAPLVELLLLGSLVIGRVSSLICTRRPANTGSPKSPVDENFMISVSGNPETYILG
QEYNVSLNAFNGHRYISFIMALENENGDFSYNDDLGRFELSDLIETRFSPNCINMVENTNTNSKTH
MHLTWVAPSEPGSGCVLIRATVQQHREVWHMDDGGLTKRICEEVTDDVESQPTAPAAVDVPCC
ACDEARYELIFEGVWSRNLHPKDFPTRGWETRFCELLGAAHSSDYRFWESGELASVAMKEYAEH
CSSRLLEREFSINFRVTPCLKRLVGSTLCYIFPLTGSKDTDDNQGTGPIVSQHKQQDNGLGASRSH
SPHGVLCLQDRTLSGLDCGRYWTGTMPAQLHMDGGKSDQPISVGCGHRFGSHLYVIRSAPSSA
RCH (SEQ ID NO:124)

# Figure 56

**Name:** Tepi
**Nucleotide:** AE003649
**GI:** 7298255
**Transcript:** CT40635
**Sequence:**

atgtcctcgaagtcggttaggtttgacattccgaagctaacagaaggcgattacgagctaaaagttatgggtagcgga
ggcatcgagttccagaattccaccaagctgagctttgcgcccgacttaaattggttatacattcaatcggacaaggccacctataagcccg
gcgataagatccaatttagggtactcttcctagacaagaacacccgacctgccgtaatcgataagcccattaaaattgagatccgcgatg
gagaccagaatttaattaagagctggaaagatatcaagccggcaaagggtgtctattccggagagctccagctgtccgacagacctgta
ctaggcaactggaccgtaaccgccacggtgcaggacgagggcaaagtgactaatgtgctagtggtggacaagtatgtggttccgaagtt
cgaggtggtcgtattaaccgctaagaatgtggcggctagtgctgggtatattagggcaacgataaaagccaggtacacattcaagaaac
ccgtaaagggccacgtcgtcgctacgatcgagggcagctcgaccgagcagagcctgcccattgatggagaggtgaatgtggagtttcc
catttcagcgactgccaagagacttttgaaaataacggccattgttaccgaggagctgacggacatcaaacacaatggtactgcctacgt
gacagtccaccagcaccgtcataagctggaagatctttttggccaacgcattataggccaggtgtatcgtctgaatttaagactgtggttag
aaacttggacggttctcctgtcatggactcttctaagatggttaacttcaacgtcttgccacaactaaggaagctacttaaaatcaatattatat
ccgatacctatcttccttactttattctcactgttgtcgcacgcggtaacattgttctcagtctttttcaagagatgaaagaaaaaaaaaagagtc
aggaaattgagttcgagccaacctttgcactggtgcctcaagctacgatatttgtccattacattatcgatggagttttgatgtctgacgagaa
aactgtcgatatcgagagggactttgagaacacaattgaaatattaacaacaaacgaagcattgccaagggatgaagtgagcctaaag
gtgaaaaccaatccccattctttcgtcggactccttggagtggaccagagtgtgctacttttaaggtcaggaaatgacctgaaccgtgatcta
atattgaacaatctcgccacgtactccaccgatttggtgatcctaacaaatgcgaatataaatatctacaggagctcgggtggctgttatacc
aatcctgcaaagaactcaggtcctgttcccattgtcggttcaaccagagcacaagcttcacttccaccagttcgtaagctctttcccgaaaca
tggctgttttcaaatattactgacgtgggcgccaacggtgaatatatcattaaagaaactgttcccgatacgttgacctcttgggtgatcacag
gcttttcccttagccctcaatccggccttgcagtgaccaggaatccgagcagaattcgtgtgttccaaccattctttattaccaccaacttgccg
tactctgtaaagcggggtgaagtgattgccattcccgtgatcgtctttaactacttgggaatggatgtgaaggcgaaagtattaatggataac
tctgacggtcagtacgaatttattgagacaaccaacaaaaatgtgtcccagtatcttaggggagtccgaaggaaaaaaacattatggattc
ccgcgaatacaggacgtggcatttcattcatgatccgcccaaagaaagtgggcctgactactctgaaaatcaccgccatttcgaaatatgc
cggtgacagactccatcaaatactgaaagtggaggccgatggcgtccaaaagtacgtcaataaggccgtattaattaacgttcaaaggtt
gaatcgtcgtagtttggcaccaccggaaaaaacaataatcatagaaaaagccgacaatgttattgaaggttccgagactgtagaattcga
agtgtgcggaactagccaagcgcctcaactggaacaccttgacgatctggttcacttgccctgcggatgtggagagcagaacatgtttaa
ctttgtgcccagtatactggcgttaagctatttaaaagcaaaaaatcggcaggatcaagagatagaaaataaagctaagcgttatgtgga
aactggttaccagatagaactcaactacaaacgtaacgacggttcctttagcgcttgggggcaacacgatgctttaggcagcacatggct
cactgcatatgtgatacggtcctttcaccaggccgccaaatacatcgacattgacaaaaatgtgttggtagctggacttgatttcctcgtgtcc
cgacagagtacagatggaaagtttaaagaattgggcatggtaatacacaacagtcatggaagtccattggcacttacatcatttgtcctgct
gactttcttcgaaaacgaggaatatatgcctaaatataagcatgtaatcgatagggctgtggaatttgtggttacggaggtgcaccagtcaa
atgagccgtatgatctggccattgccgccctggccctgtcattagcaagaaaccgcaatgcctataaagttctggataagttggataaattg
gccactcggcgaggtgatcacaaatggtggaccggttctgataagtgtaagagcagcgaagtagagaccacctcttatgtcctgctcgcc
cttctcgagcataatatatcggatgaacccaaaccgattgtcgattggctgattagcaagcgcaacagcaacggaggttttgtctcctccca
ggacaccgttgtgggtataatggccttaaccaagtacgaactccaatcacatgcctcgaccgaagctattgacatagagttttggcatttga
acgaagataaaaaacatgtaagggtaactaaagaaaatgaatttaaagtccagactcatcagcttcctgaaaacacaaacgaggtga
agttgttggccaaaggtcaggggcgtgcacaagttcagctgacgtaccgctacaacgtggccaccaaggaggcgagacccagcttca
aattaacgactacggttaagaagtcccataaaggccgactgatccttggcatttgtggcacgtacacccccatcgcagcctccgagagga
ataagaccaccaatatggctttgatgcaggtgcagctaccatctggttatgtttgcgatatcgagccctttgccgatatcgaagccatttcaga
cgtaaagcgtgtggagaccaaaaacgaagacaccgaggttcatatctatttcgaaaagctgtcacctggtgaccgcaagtgcctgaccc
tagaggccatatacacccatgcggtggccaatctgaaaccttcttgggttcggctctatgattactatgccaccgaacgcagtgctaccgaa
ttctatcacgtggacacttctctgtgtgacatttgccacggcaacgagtgcggaaatatgtgctaa (SEQ ID NO:125)

**Protein:** AAF53490
**Protein GI:** 10728811

MLWLILSSTILHCVLLSNANGLYSVLAPKTLRSNSAYNVVVAIHNTTRTTEVSVSLTGPSLN
SRKYVDVQSMSSKSVRFDIPKLTEGDYELKVMGSGGIEFQNSTKLSFAPDLNWLYIQSDKATYKP
GDKIQFRVLFLDKNTRPAVIDKPIKIEIRDGDQNLIKSWKDIKPAKGVYSGELQLSDRPVLGNWTVT
ATVQDEGKVTNVLVVDKYVVPKFEVVVLTAKNVAASAGYIRATIKARYTFKKPVKGHVVATIEGSS
TEQSLPIDGEVNVEFPISATAKRLLKITAIVTEELTDIKHNGTAYVTVHQHRHKLEDLFWPTHYRPG
VSSEFKTVVRNLDGSPVMDSSKMVNFNVLCCQVSKNFSASLQNSIATEHIMLPETCQSCLVTSTF

DTAENIERYIYKLNKPLMIAINTKKPQLRKLLKINIISDTYLPYFILTVVARGNIVLSLFQEMKEKKKSQ
EIEFEPTFALVPQATIFVHYIIDGVLMSDEKTVDIERDFENTIEILTTNEALPRDEVSLKVKTNPHSFV
GLLGVDQSVLLLRSGNDLNRDLILNNLATYSTDLVILTNANINIYRSSGGCYTNPGYTNCTGSLIGR
TMFKNEPTKNSGPVPIVGSTRAQASLPPVRKLFPETWLFSNITDVGANGEYIIKETVPDTLTSWVIT
GFSLSPQSGLAVTRNPSRIRVFQPFFITTNLPYSVKRGEVIAIPVIVFNYLGMDVKAKVLMDNSDGQ
YEFIETTNKNVSQYLRGVRRKKTLWIPANTGRGISFMIRPKKVGLTTLKITAISKYAGDRLHQILKVE
ADGVQKYVNKAVLINVQRLNRRSLAPPEKTIIIEKADNVIEGSETVEFEVCGTSQAPQLEHLDDLVH
LPCGCGEQNMFNFVPSILALSYLKAKNRQDQEIENKAKRYVETGYQIELNYKRNDGSFSAWGQH
DALGSTWLTAYVIRSFHQAAKYIDIDKNVLVAGLDFLVSRQSTDGKFKELGMVIHNSHGSPLALTSF
VLLTFFENEEYMPKYKHVIDRAVEFVVTEVHQSNEPYDLAIAALALSLARNRNAYKVLDKLDKLATR
RGDHKWWTGSDKCKSSEVETTSYVLLALLEHNISDEPKPIVDWLISKRNSNGGFVSSQDTVVGIM
ALTKYELQSHASTEAIDIEFWHLNEDKKHVRVTKENEFKVQTHQLPENTNEVKLLAKGQGRAQVQ
LTYRYNVATKEARPSFKLTTTVKKSHKGRLILGICGTYTPIAASERNKTTNMALMQVQLPSGYVCDI
EPFADIEAISDVKRVETKNEDTEVHIYFEKLSPGDRKCLTLEAIYTHAVANLKPSWVRLYDYYATER
SATEFYHVDTSLCDICHGNECGNMC (SEQ ID NO:126)

# Figure 57

**Name:** for
**Nucleotide:** AE003579
**GI:** 10727349
**Transcript:** CT43154
**Sequence:**

attcgtccggcaggcggcgtagcgagaaaacgtgtgtggcgcatgcgcatgcgctcggttgcgaaaagtgcacctg
gatttgaaattaagccacaagaataacagaaatcaaaactcccccacttcgtgtttggccatttcttcggctgtcgtatcgtgattttcaattcg
ccctcgtgtgtgcttaacggtattatagagcattttgcatatggcaaccgctgccagtgaattaataataaaagccacaaaccagacaatta
ccatgccttgcgcatatgcctttgagctatttccaacaataatgcttacataaatagcagctctggttgatgttctaaatagcaagtaaacaaa
accgcacaattttcccatgctcggtttttccgctcggctgtaattatgttgaaagagatcgggaaacgcttcgtgttaatttctttgaactgctcgc
aaattcttggaataaagtgaattaaaagtgaaagtgaattctttaagcactcgcattaagtgagggaaataagcttaaaaacacccacgg
acttaaagagaatgagacgcagaaacggagacttggattttgctctttttctcgaattgaacgcaaagatcacttgaagaattccggcctga
aaaccgtaggcatcgccattcccagtgactcttcaaatattccgtgcgcgaaactgcagagctctttggttatgcaaaagacctccgaaaat
aacaaacaacaggcacaaaaacataaactaaacgctggtggagccaaagacaaacgaatcacgtcgctgcagctggtcaagtgaa
atttttctttcgtaatgaaaatcaaacattatccgggcaaagccgtcgatgcgagtctctcgctggagggcagcagtgccatgggtgccctgt
acgaggccaattggctgagggctgcgaatcagcccgccgctccagccacaacgggcaccaaattgagcaggcagagcagctccgcc
ggcagcagtttcctcatcgagggcatctccgccctgagcaagtaccaaatgaccctggagaatatccggcagctggagctgcagtcgcg
tgacaaacgcatagcgagcacgattaaggaactcagcggctataggccatcggcactgcagcatcatcagcagcagcagatgcataa
cgtttgggtggccgaggatcaggatcaggaacacgaagaactggaagatgcttcggagggaaaggagaagttggctagtatccagga
accccctgcagttaaccactatgtcctggacccgacggagagaccaagggttcccagaccccggcaacagttctccgtgaaaccgcctt
ccttgcgtcgctcccaaaccatgtcgcaaccgcccagttatgccaccttgagatcgcccccgaaaatcaaggaaaatcttagcaaaagtt
cctccgcctactccacattctcctcggctgcagaggattcccaggatcaagtagtgatctgccaacagccacagagactgatggctccgc
cacccagagaaccgcctccggagccacccaaaagggtatcgaaacccctgagcagatcgcaaacttcggttcagcggtacgccacg
gttaggatgcccaaccagaccacttcgttcagcagaagtgtggtcaggtctagggattccaccgcctcccagagacgtctcagtttggagc
aggccatcgagggactcaaactggagggggagaaggctgttcgtcagaagagtccacaaatctcaccagctgccagcagtaatggca
gctccaaggatctcaacggggagggtttctgtatcccacgaccgcgcctcatcgttccagtgcacacatatgcccgtcgacgcaggacgg
gaaatctcaaggagcaatccagcggtgggcaggaggaggaagcagagaaggatggacgcgtggaggtttcccgcgagggcaagta
cctctccacattgtcgggagcgaaggtccttggcgaattggcgatcctgtacaactgccagcggacggcgaccatcaccgcgatcaccg
agtgcaacctgtgggccatcgagcgccagtgctttcagaccatcatgatgcgaacgggcctgatccggcaggcggagtacagcgatttc
ctcaagagtgtgcccatcttcaaagacctggcggaagacacgctcatcaaaatctccgatgttttggaggagacgcactaccagcgtggc
gactacatagtgcgccaaggcgcccgaggcgataccttcttcatcatatccaagggaaaagtgcgagtgacgatcaagcagcaggac
acgcaggaggagaagttcattcgcatgctgggcaaaggggatttctttggagagaaggctctccagggcgatgatctgcgcacggcga
atattatttgcgagtccgccgatggcgtcagttgtctggtcatcgatcgcgagaccttcaatcagctaatttccaatctagacgagatcaagc
atcgctacgacgacgagggcgccatggaacgcagaaagatcaacgaggaattccgggacattaacctcacagatctgcgcgtcatcg
caaccccttggagttggaggcttcggtcgcgtagagctggtccaaactaatggagatagctccaggtccttcgcgctcaagcagatgaaaa
agtcacagattgtggagacgcgtcagcagcaacacatcatgtccgagaaggagatcatgggcgaggccaattgccagttcatcgtgaa
gctgttcaagaccttcaaggacaagaagtacctgtacatgctgatggagagttgcctgggcggagagctctggacgattctacgggaca
agggcaacttcgacgacagcaccacccgcttctacacggcatgtgtggtggaggcctttgattacttgcactcgcgtaacatcatctaccg
cgatcttaagccggagaacctgctgctcaatgaacgaggatatgtgaagctggtggactttggctttgccaagaagctgcagacgggca
ggaagacctggactttctgcggcactccagagtacgtggctcccgaggtgattctcaaccggggccacgacatcagtgcggattactggt
cgctgggagtgctcatgttcgagttacttactggtacccctccattcacgggctcggatcccatgcgcacctacaacattatacttaagggca
tcgacgccatcgaattcccaaggaatatcacccgcaatgccagcaacctgatcaagaagctctgtcgcgacaatccagccgagcgtttg
ggctaccagcgtgggggaatcagcgagatccaaaagcacaaatggttcgatggcttctattggtggggcctgcagaactgcacactgga
accgcccattaagcccgccgtgaaaagcgttgtggatacaacaaactttgatgactatcctcccgatcctgagggtccgccgccagatga
tgtcactggatgggacaaggacttctgaggagaatcagaacccgtttcctagacgatgctctctaaacgcttctgctgcagaaaaccagg
aggatatgaaagccagggaagaaaaattgatcttaagtgcgccatatgtacgccaaagccaacagcaacagtcagcagctcgcatcg
aaaagctgccacaaaaaaaaacaaagaaacgtagcagtcgcaaggtcaagggccgacacaaaagcacaatcatccatcgtcgtag
ctccattgagatttatagatacgtctccgtgatgttataaccatgatgtgcaacgcaatgaatttattaacgagtttataactattattttataatg
aggatatatgtgtctagttcgcttggaattgatgtaaattgtaagtaggtctgtgactctgtttcagagctctgttagccatgtgcattgtataaatt
cagctatttgtatctattaaatatttttaacataattattacacatcattgttaaagcatacaaatcgggttgccttatagtctgtaagagaacattt
gaaagcaacatttgaccaagatcttccgtcacacatttcttaaaattctatgtggcctctctactgtctttcattagtcttagcgatcatgtctattat
atttacgaataacatgcccttttaattgtctttttaagtaaatc (SEQ ID NO:127)

**Protein:** AAF51082

**Protein GI:**10727350

MRFCFDRLCFATKRPAQNSNSNAPHSSTTVDAPPRPADVDVATVPVATPAPPPQQPVS
NLFYADYQKLQPAIIDRDWERDRDTDTDTRSEAKPPDIVEHIEPVEEQRQIHTQIQSPAEIQIQIPPT
PPAPSIQIQIQQRYRRHSSAEDRNLNTRRNDSNITEALRKAASMQQEPNANYQFPTDLGLVSIVNN
NNNTNTHPSGSNSGSNNNSNINNNLVGGIVTLPAAGGLIGLEHTASGLRLIPAPPTHSDVLTHTLIY
GTPPSGAQQLNQDPRSLLHQQELQLQQRYQQLQQLQAQTQGLYTSQGSPVLYHQPSPGSSQP
VAIPGATCHSPTQLQPPNTLNLQQQMQSLRISGCTPSGTGGSATPSPVGLVDPNFIVSNYVAASP
QEERFIQIIQAKELKIQEMQRALQFKDNEIAELKSHLDKFQSVFPFSRGSAAGCAGTGGASGSGAG
GSGGSGPGTATGATRKSGQNFQRQRALGISAEPQSESSLLLEHVSFPKYDKDERSRELIKAAILD
NDFMKNLDLTQIREIVDCMYPVKYPAKNLIIKEGDVGSIVYVMEDGRVEVSREGKYLSTLSGAKVL
GELAILYNCQRTATITAITECNLWAIERQCFQTIMMRTGLIRQAEYSDFLKSVPIFKDLAEDTLIKISD
VLEETHYQRGDYIVRQGARGDTFFIISKGKVRVTIKQQDTQEEKFIRMLGKGDFFGEKALQGDDLR
TANIICESADGVSCLVIDRETFNQLISNLDEIKHRYDDEGAMERRKINEEFRDINLTDLRVIATLGVG
GFGRVELVQTNGDSSRSFALKQMKKSQIVETRQQQHIMSEKEIMGEANCQFIVKLFKTFKDKKYL
YMLMESCLGGELWTILRDKGNFDDSTTRFYTACVVEAFDYLHSRNIIYRDLKPENLLLNERGYVKL
VDFGFAKKLQTGRKTWTFCGTPEYVAPEVILNRGHDISADYWSLGVLMFELLTGTPPFTGSDPMR
TYNIILKGIDAIEFPRNITRNASNLIKKLCRDNPAERLGYQRGGISEIQKHKWFDGFYWWGLQNCTL
EPPIKPAVKSVVDTTNFDDYPPDPEGPPPDDVTGWDKDF (SEQ ID NO:128)

**Transcript:** CT42452
**Sequence:**

gcgcatgcgcatgcgctcggttgcgaaaagtgcacctggatttgaaattaagccacaagaataacagaaatcaaaa
ctcccccacttcgtgtttggccatttcttcggctgtcgtatcgtgattttcaattcgccctcgtgtgtgcttaacggcctgaaaaccgtaggcatcg
ccattcccagtgactcttcaaatattccgtgcgcgaaactgcagagctctttggttatgcaaaagacctccgaaaataacaaacaacaggc
acaaaaacataaactaaacgctggtggagccaaagacaaacgaatcacgtcgctgcagctggtcaagtgaaatttttcttcgtaatgaa
aatcaaacattatccgggcaaagccgtcgatgcgagtctctcgctggagggcagcagtgccatgggtgccctgtacgaggccaattggc
tgagggctgcgaatcagcccgccgctccagccacaacgggcaccaaattgagcaggcagagcagctccgccggcagcagtttcctca
tcgagggcatctccgccctgagcaagtaccaaatgaccctggagaatatccggcagctggagctgcagtcgcgtgacaaacgcatagc
gagcacgattaaggaactcagcggctataggccatcggcactgcagcatcatcagcagcagcagatgcataacgtttgggtggccgag
gatcaggatcaggaacacgaagaactggaagatgcttcggagggaaaggagaagttggctagtatccaggaacccccctgcagttaac
cactatgtcctggacccgacggagagaccaagggttcccagaccccggcaacagttctccgtgaaaccgccttccttgcgtcgctccca
aaccatgtcgcaaccgcccagttatgccaccttgagatcgcccccgaaaatcaaggaaaatcttagcaaaagttcctccgcctactccac
attctcctcggctgcagaggattcccaggatcaagtagtgatctgccaacagccacagagactgatggctccgccacccagagaaccg
cctccggagccacccaaaagggtatcgaaacccctgagcagatcgcaaacttcggttcagcggtacgccacggttaggatgcccaacc
agaccacttcgttcagcagaagtgtggtcaggtctagggattccaccgcctcccagagacgtctcagtttggagcaggccatcgagggac
tcaaactggagggggagaaggctgttcgtcagaagagtccacaaatctcaccagctgccagcagtaatggcagctccaaggatctcaa
cggggagggtttctgtatcccacgaccgcgcctcatcgttccagtgcacacatatgcccgtcgacgcaggacgggaaatctcaaggagc
aatccagcggtgggcaggaggaggaagcagagaagggtaaggggtggaaggatttctatgttttaagtcaggataggcatagtagcttt
tatatcaataggataggacagaattatgattatgattacccaattaattttaatatatttctccagatggacgcgtggaggtttcccgcgaggg
caagtacctctccacattgtcgggagcgaaggtccttggcgaattggcgatcctgtacaactgccagcggacggcgaccatcaccgcga
tcaccgagtgcaacctgtgggccatcgagcgccagtgctttcagaccatcatgatgcgaacgggcctgatccggcaggcggagtacag
cgatttcctcaagagtgtgcccatcttcaaagacctggcggaagacacgctcatcaaaatctccgatgttttggaggagacgcactaccag
cgtggcgactacatagtgcgccaaggcgcccgaggcgataccttcttcatcatatccaagggaaaagtgcgagtgacgatcaagcagc
aggacacgcaggaggagaagttcattcgcatgctgggcaaaggggatttctttggagagaaggctctccagggcgatgatctgcgcac
ggcgaatattatttgcgagtccgccgatggcgtcagttgtctggtcatcgatcgcgagaccttcaatcagctaatttccaatctagacgagat
caagcatcgctacgacgacgagggcgccatggaacgcagaaagatcaacgaggaattccgggacattaacctcacagatctgcgcg
tcatcgcaaccccttggagttggaggcttcggtcgcgtagagctggtccaaactaatggagatagctccaggtccttcgcgctcaagcagat
gaaaaagtcacagattgtggagacgcgtcagcagcaacacatcatgtccgagaaggagatcatgggcgaggccaattgccagttcatc
gtgaagctgttcaagaccttcaaggacaagaagtacctgtacatgctgatggagagttgcctgggcggagagctctggacgattctacgg
gacaagggcaacttcgacgacagcaccacccgcttctacacggcatgtgtggtggaggcctttgattacttgcactcgcgtaacatcatct
accgcgatcttaagccggagaacctgctgctcaatgaacgaggatatgtgaagctggtggactttggctttgccaagaagctgcagacgg
gcaggaagacctggactttctgcggcactccagagtacgtggctcccgaggtgattctcaaccggggccacgacatcagtgcggattact
ggtcgctgggagtgctcatgttcgagttacttactggtacccctccattcacgggctcggatcccatgcgcacctacaacattatacttaagg
gcatcgacgccatcgaattcccaaggaatatcacccgcaatgccagcaacctgatcaagaagctctgtcgcgacaatccagccgagcg
tttgggctaccagcgtggggggaatcagcgagatccaaaagcacaaatggttcgatggcttctattggtggggcctgcagaactgcacact
ggaaccgcccattaagcccgccgtgaaaagcgttgtggatacaacaaactttgatgactatcctcccgatcctgagggtccgccgccag
atgatgtcactggatgggacaaggacttctgaggagaatcagaacccgtttcctagacgatgctctctaaacgcttctgctgcagaaaacc

aggaggatatgaaagccagggaagaaaaattgatcttaagtgcgccatatgtacgccaaagccaacagcaacagtcagcagctcgc
atcgaaaagctgccacaaaaaaaaacaaagaaacgtagcagtcgcaaggtcaagggccgacacaaaagcacaatcatccatcgtc
gtagctccatttgagatttatagatacgtctccgtgatgttataaccatgatgtgcaacgcaatgaatttattaacgagtttataactattattttat
aatgaggatatatgtgtctagttcgcttggaattgatgtaaattgtaagtaggtctgtgactctgtttcagagctctgttagccatgtgcattgtata
aattcagctatttgtatctattaaatatttttaacataattattacacatcattgttaaagcatacaaatcgggttgccttatagtctgtaagagaac
atttgaaagcaacatttgaccaagatcttccgtcacacatttcttaaaattctatgtggcctctctactgtctttcattagtcttagcgatcatgtcta
ttatatttacgaataacatgcc (SEQ ID NO:129)

**Protein:** AAG22251
**Protein GI:** 10727351
MQSLRISGCTPSGTGGSATPSPVGLVDPNFIVSNYVAASPQEERFIQIIQAKELKIQEMQR
ALQFKDNEIAELKSHLDKFQSVFPFSRGSAAGCAGTGGASGSGAGGSGGSGPGTATGATRKSG
QNFQRQRALGISAEPQSESSLLLEHVSFPKYDKDERSRELIKAAILDNDFMKNLDLTQIREIVDCMY
PVKYPAKNLIIKEGDVGSIVYVMEDGRVEVSREGKYLSTLSGAKVLGELAILYNCQRTATITAITECN
LWAIERQCFQTIMMRTGLIRQAEYSDFLKSVPIFKDLAEDTLIKISDVLEETHYQRGDYIVRQGARG
DTFFIISKGKVRVTIKQQDTQEEKFIRMLGKGDFFGEKALQGDDLRTANIICESADGVSCLVIDRETF
NQLISNLDEIKHRYDDEGAMERRKINEEFRDINLTDLRVIATLGVGGFGRVELVQTNGDSSRSFAL
KQMKKSQIVETRQQQHIMSEKEIMGEANCQFIVKLFKTFKDKKYLYMLMESCLGGELWTILRDKG
NFDDSTTRFYTACVVEAFDYLHSRNIIYRDLKPENLLLNERGYVKLVDFGFAKKLQTGRKTWTFCG
TPEYVAPEVILNRGHDISADYWSLGVLMFELLTGTPPFTGSDPMRTYNIILKGIDAIEFPRNITRNAS
NLIKKLCRDNPAERLGYQRGGISEIQKHKWFDGFYWWGLQNCTLEPPIKPAVKSVVDTTNFDDYP
PDPEGPPPDDVTGWDKDF (SEQ ID NO:130)

**Transcript:** CT43152
**Sequence:**
acaaacacccatccgagtggatccaatagcggcagcaacaacaacagcaatatcaacaataatctggtgggtggc
attgtgaccttgccggcggctggtggtctcataggtctggagcatacggcgtcgggtctgcgactgataccagcaccgcctacccactcgg
atgtgctgacccacacgctgatctatggcacaccgccctcgggcgcccagcaattgaaccaggatccccgaagccttctgcaccaaca
ggagctgcagctacagcagcgctatcagcaattgcagcagcttcaggcgcagacgcagggcctgtacaccagccaaggaagtccggt
gctgtatcaccaacccagtccgggatcgagtcaaccggtggccatacccggagccacttgccattcgcccacgcaactgcagccgccc
aacacgctgaacctgcagcagcagatgcagagtctgcggatctcgggatgcacgcccagcggtacgggtggctcggccacgccctcg
ccggtgggcctggtggatccgaatttcatagtcagcaactatgtggccgcctcgccgcaggaggagcgcttcattcagatcattcaggcca
aggagctcaagatacaggaaatgcaaagggccctccagttcaaggacaacgaaatagccgagctaaagtcgcacttggacaaattcc
agagtgtctttcccttcagccgtggcagtgcggccggttgtgcgggcacgggcggagcgtcgggatctggagctggcggaagtggtggc
agtggtcccggcaccgccacaggtgccacacgcaagtcgggtcagaatttccagaggcagagggcattgggtatctccgccgagcca
cagagcgagtcctcgctgctcctcgagcacgtcagctttcccaaatacgacaaggatgagcgctcccgtgaacttatcaaggctgccata
ttggataatgatttcatgaagaatctggatctgacgcagatccgcgagatcgttgactgcatgtatccggttaagtatccagccaagaatctg
atcatcaaggagggagatgtcggaagcatcgtttatgtcatggaagatggacgcgtggaggtttcccgcgagggcaagtacctctccac
attgtcgggagcgaaggtccttggcgaattggcgatcctgtacaactgccagcggacggcgaccatcaccgcgatcaccgagtgcaac
ctgtgggccatcgagcgccagtgctttcagaccatcatgatgcgaacgggcctgatccggcaggcggagtacagcgatttcctcaagagt
gtgcccatcttcaaagacctggcggaagacacgctcatcaaaatctccgatgtttggaggagacgcactaccagcgtggcgactacata
gtgcgccaaggcgcccgaggcgataccttcttcatcatatccaagggaaaagtgcgagtgacgatcaagcagcaggacacgcagga
ggagaagttcattcgcatgctgggcaaaggggatttctttggagagaaggctctccagggcgatgatctgcgcacggcgaatattatttgc
gagtccgccgatggcgtcagttgtctggtcatcgatcgcgagaccttcaatcagctaatttccaatctagacgagatcaagcatcgctacga
cgacgagggcgccatggaacgcagaaagatcaacgaggaattccgggacattaacctcacagatctgcgcgtcatcgcaacccttgg
agttggaggcttcggtcgcgtagagctggtccaaactaatggagatagctccaggtccttcgcgctcaagcagatgaaaaagtcacagat
tgtggagacgcgtcagcagcaacacatcatgtccgagaaggagatcatgggcgaggccaattgccagttcatcgtgaagctgttcaaga
ccttcaaggacaagaagtacctgtacatgctgatggagagttgcctgggcggagagctctggacgattctacgggacaagggcaacttc
gacgacagcaccacccgcttctacacggcatgtgtggtggaggcctttgattacttgcactcgcgtaacatcatctaccgcgatcttaagcc
ggagaacctgctgctcaatgaacgaggatatgtgaagctggtggactttggctttgccaagaagctgcagacgggcaggaagacctgg
actttctgcggcactccagagtacgtggctcccgaggtgattctcaaccggggccacgacatcagtgcggattactggtcgctgggagtgc
tcatgttcgagttacttactggtacccctccattcacgggctcggatcccatgcgcacctacaacattatacttaagggcatcgacgccatcg
aattcccaaggaatatcacccgcaatgccagcaacctgatcaagaagctctgtcgcgacaatccagccgagcgtttgggctaccagcgt
gggggaatcagcgagatccaaaagcacaaatggttcgatggcttctattggtggggcctgcagaactgcacactggaaccgcccattaa
gcccgccgtgaaaagcgttgtggatacaacaaactttgatgactatcctcccgatcctgagggtccgccgccagatgatgtcactggatgg
gacaaggacttctgaggagaatcagaacccgtttcctagacgatgctctctaaacgcttctgctgcagaaaaccaggaggatatgaaag
ccagggaagaaaaattgatcttaagtgcgccatatgtacgccaaagccaacagcaacagtcagcagctcgcatcgaaaagctgccac

aaaaaaaaacaaagaaacgtagcagtcgcaaggtcaagggccgacacaaaagcacaatcatccatcgtcgtagctccatttgagattt atagatacgtctccgtgatgttataaccatgatgtgcaacgcaatgaatttattaacgagtttataactattattttataatgaggatatatgtgtct agttcgcttggaattgatgtaaattgtaagtaggtctgtgactctgtttcagagctctgttagccatgtgcattgtataaattcagctatttgtatcta ttaaatatttttaacataattattacacatcattgttaaagcatacaaatcgggttgccttatagtctgtaagagaacatttgaaagcaacatttg accaagatcttccgtcacacatttcttaaaattctatgtggcctctctactgtctttcattagtcttagcgatcatgtctattatatttacgaataacat gcc (SEQ ID NO:131)

**Protein:** AAG22252
**Protein GI:** 10727352

MKIKHYPGKAVDASLSLEGSSAMGALYEANWLRAANQPAAPATTGTKLSRQSSSAGSSF LIEGISALSKYQMTLENIRQLELQSRDKRIASTIKELSGYRPSALQHHQQQMHNVWVAEDQDQE HEELEDASEGKEKLASIQEPPAVNHYVLDPTERPRVPRPRQQFSVKPPSLRRSQTMSQPPSYATL RSPPKIKENLSKSSSAYSTFSSAAEDSQDQVVICQQPQRLMAPPPREPPPEPPKRVSKPLSRSQT SVQRYATVRMPNQTTSFSRSVVRSRDSTASQRRLSLEQAIEGLKLEGEKAVRQKSPQISPAASSN GSSKDLNGEGFCIPRPRLIVPVHTYARRRRTGNLKEQSSGGQEEEAEKDGRVEVSREGKYLSTLS GAKVLGELAILYNCQRTATITAITECNLWAIERQCFQTIMMRTGLIRQAEYSDFLKSVPIFKDLAEDT LIKISDVLEETHYQRGDYIVRQGARGDTFFIISKGKVRVTIKQQDTQEEKFIRMLGKGDFFGEKALQ GDDLRTANIICESADGVSCLVIDRETFNQLISNLDEIKHRYDDEGAMERRKINEEFRDINLTDLRVIA TLGVGGFGRVELVQTNGDSSRSFALKQMKKSQIVETRQQQHIMSEKEIMGEANCQFIVKLFKTFK DKKYLYMLMESCLGGELWTILRDKGNFDDSTTRFYTACVVEAFDYLHSRNIIYRDLKPENLLLNER GYVKLVDFGFAKKLQTGRKTWTFCGTPEYVAPEVILNRGHDISADYWSLGVLMFELLTGTPPFTG SDPMRTYNIILKGIDAIEFPRNITRNASNLIKKLCRDNPAERLGYQRGGISEIQKHKWFDGFYWWGL QNCTLEPPIKPAVKSVVDTTNFDDYPPDPEGPPPDDVTGWDKDF (SEQ ID NO:132)

**Transcript:** CT43158
**Sequence:**

tgtgtgcgtgccacttagaaacgcaacgcaagtggaaagactcgagaaaacaaaagtgcaggccgtctaaattgat ggcgaagtttcgtgtgccgcctgcatttcatttataaatatattttcggccttaattctgtgcttcctgtgaattaacgaggaaactgaagagtgc gcgagcgagacagccgatcacaaatcaacaagtggaaacgaagaaactttcttcgcaaaagccggctgatcgcaacaacaagaag aaaagagagcgattctctgcgactggaaccataacatttacatcgaccctggaatttcttcacttttcgtccaacatggctcccaacgcctgg ctgattgagttttccgtggcggctgttgatcaggaaagccgcctggatgcgtttctgctttgatcggctgtgcttcgcaaccaagcggccagcc caaaactccaacagcaatgcaccacacagcagcaccacagtagatgcaccaccacgtccagcggatgtagatgtagccactgtacca gtagcaacaccagcaccaccaccacaacagccagttagtaatctgttctatgccgactaccagaagctgcagccggcgataatcgatc gggactgggagcgggacagagatacagatacagataccaggagcgaggccaagccaccggacattgtggagcacatagaacccgt agaggagcagagacagatacacacacagatacagtcgccggcagagatacagatacagatacccccgacgccccctgccccatcg atacagatacagatacagcagcgctatcgccggcacagcagtgccgaagatcgcaatctgaacacgaggcgaaacgattcgaatatt acggaggcgttacgaaaagcagccagtatgcagcaggagccgaacgccaactatcagttcccaaccgacttgggtctggtgagcatc gtcaacaataataataacacaaacacccatccgagtggatccaatagcggcagcaacaacaacagcaatatcaacaataatctggtg ggtggcattgtgaccttgccggcggctggtggtctcataggtctggagcatacggcgtcgggtctgcgactgataccagcaccgcctaccc actcggatgtgctgacccacacgctgatctatggcacaccgccctcgggcgcccagcaattgaaccaggatccccgaagccttctgcac caacaggagctgcagctacagcagcgctatcagcaattgcagcagcttcaggcgcagacgcagggcctgtacaccagccaaggaag tccggtgctgtatcaccaacccagtccgggatcgagtcaaccggtggccatacccggagccacttgccattcgcccacgcaactgcagc cgcccaacacgctgaacctgcagcagcagatgcagagtctgcggatctcgggatgcacgcccagcggtacgggtggctcggccacgc cctcgccggtgggcctggtggatccgaatttcatagtcagcaactatgtggccgcctcgccgcaggaggagcgcttcattcagatcattca ggccaaggagctcaagatacaggaaatgcaaagggccctccagttcaaggacaacgaaatagccgagctaaagtcgcacttggaca aattccagagtgtctttcccttcagccgtggcagtgcggccggttgtgcgggcacgggcggagcgtcgggatctggagctggcggaagtg gtggcagtggtcccggcaccgccacaggtgccacacgcaagtcgggtcagaatttccagaggcagagggcattgggtatctccgccga gccacagagcgagtcctcgctgctcctcgagcacgtcagctttcccaaatacgacaaggatgagcgctcccgtgaacttatcaaggctgc catattggataatgatttcatgaagaatctggatctgacgcagatccgcgagatcgttgactgcatgtatccggttaagtatccagccaaga atctgatcatcaaggagggagatgtcggaagcatcgtttatgtcatggaagatggacgcgtggaggtttcccgcgaggcaagtacctct ccacattgtcgggagcgaaggtccttggcgaattggcgatcctgtacaactgccagcggacggcgaccatcaccgcgatcaccgagtg caacctgtgggccatcgagcgccagtgctttcagaccatcatgatgcgaacgggcctgatccggcaggcggagtacagcgatttcctca agagtgtgcccatcttcaaagacctggcggaagacacgctcatcaaaatctccgatgttttggaggagacgcactaccagcgtggcgac tacatagtgcgccaaggcgcccgaggcgataccttcttcatcatatccaagggaaaagtgcgagtgacgatcaagcagcaggacacg caggaggagaagttcattcgcatgctgggcaaaggggatttctttggagagaaggctctccagggcgatgatctgcgcacggcgaatatt atttgcgagtccgccgatggcgtcagttgtctggtcatcgatcgcgagaccttcaatcagctaatttccaatctagacgagatcaagcatcgc tacgacgacgagggcgccatggaacgcagaaagatcaacgaggaattccgggacattaacctcacagatctgcgcgtcatcgcaacc

cttggagttggaggcttcggtcgcgtagagctggtccaaactaatggagatagctccaggtccttcgcgctcaagcagatgaaaaagtca cagattgtggagacgcgtcagcagcaacacatcatgtccgagaaggagatcatgggcgaggccaattgccagttcatcgtgaagctgtt caagaccttcaaggacaagaagtacctgtacatgctgatggagagttgcctgggcggagagctctggacgattctacgggacaagggc aacttcgacgacagcaccacccgcttctacacggcatgtgtggtggaggcctttgattacttgcactcgcgtaacatcatctaccgcgatctt aagccggagaacctgctgctcaatgaacgaggatatgtgaagctggtggactttggctttgccaagaagctgcagacgggcaggaaga cctggactttctgcggcactccagagtacgtggctcccgaggtgattctcaaccggggccacgacatcagtgcggattactggtcgctggg agtgctcatgttcgagttacttactggtacccctccattcacgggctcggatcccatgcgcacctacaacattatacttaagggcatcgacgc catcgaattcccaaggaatatcacccgcaatgccagcaacctgatcaagaagctctgtcgcgacaatccagccgagcgtttgggctacc agcgtggggggaatcagcgagatccaaaagcacaaatggttcgatggcttctattggtggggcctgcagaactgcacactggaaccgcc cattaagcccgccgtgaaaagcgttgtggatacaacaaactttgatgactatcctcccgatcctgagggtccgccgccagatgatgtcact ggatgggacaaggacttctgaggagaatcagaacccgtttcctagacgatgctctctaaacgcttctgctgcagaaaaccaggaggatat gaaagccagggaagaaaaattgatcttaagtgcgccatatgtacgccaaagccaacagcaacagtcagcagctcgcatcgaaaagct gccacaaaaaaaaacaaagaaacgtagcagtcgcaaggtcaagggccgacacaaaagcacaatcatccatcgtcgtagctccattt gagatttatagatacgtctccgtgatgttataaccatgatgtgcaacgcaatgaatttattaacgagtttataactattattttataatgaggatat atgtgtctagttcgcttggaattgatgtaaattgtaagtaggtctgtgactctgtttcagagctctgttagccatgtgcattgtataaattcagctatt tgtatctattaaatattttaacataattattacacatcattgttaaagcatacaaatcgggttgccttatagtctgtaagagaacatttgaaagca acatttgaccaagatcttccgtcacacatttcttaaaattctatgtggcctctctactgtctttcattagtcttagcgatcatgtctattatatttacga ataacatgcc (SEQ ID NO:133)

**Protein:** AAG22253
**Protein GI:** 10727353

MKIKHYPGKAVDASLSLEGSSAMGALYEANWLRAANQPAAPATTGTKLSRQSSSAGSSF LIEGISALSKYQMTLENIRQLELQSRDKRIASTIKELSGYRPSALQHHQQQMHNVWVAEDQDQE HEELEDASEGKEKLASIQEPPAVNHYVLDPTERPRVPRPRQQFSVKPPSLRRSQTMSQPPSYATL RSPPKIKENLSKSSSAYSTFSSAAEDSQDQVVICQQPQRLMAPPPREPPPEPPKRVSKPLSRSQT SVQRYATVRMPNQTTSFSRSVVRSRDSTASQRRLSLEQAIEGLKLEGEKAVRQKSPQISPAASSN GSSKDLNGEGFCIPRPRLIVPVHTYARRRRTGNLKEQSSGGQEEEAEKDGRVEVSREGKYLSTLS GAKVLGELAILYNCQRTATITAITECNLWAIERQCFQTIMMRTGLIRQAEYSDFLKSVPIFKDLAEDT LIKISDVLEETHYQRGDYIVRQGARGDTFFIISKGKVRVTIKQQDTQEEKFIRMLGKGDFFGEKALQ GDDLRTANIICESADGVSCLVIDRETFNQLISNLDEIKHRYDDEGAMERRKINEEFRDINLTDLRVIA TLGVGGFGRVELVQTNGDSSRSFALKQMKKSQIVETRQQQHIMSEKEIMGEANCQFIVKLFKTFK DKKYLYMLMESCLGGELWTILRDKGNFDDSTTRFYTACVVEAFDYLHSRNIIYRDLKPENLLLNER GYVKLVDFGFAKKLQTGRKTWTFCGTPEYVAPEVILNRGHDISADYWSLGVLMFELLTGTPPFTG SDPMRTYNIILKGIDAIEFPRNITRNASNLIKKLCRDNPAERLGYQRGGISEIQKHKWFDGFYWWGL QNCTLEPPIKPAVKSVVDTTNFDDYPPDPEGPPPDDVTGWDKDF (SEQ ID NO:134)

**Transcript:** CT43160
**Sequence:**

attcgtccggcaggcggcgtagcgagaaaacgtgtgtggcgcatgcgcatgcgctcggttgcgaaaagtgcacctg gatttgaaattaagccacaagaataacagaaatcaaaactcccccacttcgtgtttggccatttcttcggctgtcgtatcgtgattttcaattcg ccctcgtgtgtgcttaacggtattatagagcattttgcatatggcaaccgctgccagtgaattaataataaaagccacaaaccagacaatta ccatgccttgcgcatatgcctttgagctatttccaacaataatgcttacataaatagcagctctggttgatgttctaaatagcaagtaaacaaa accgcacaattttcccatgctcggtttttccgctcggctgtaattatgttgaaagagatcgggaaacgcttcgtgttaatttctttgaactgctcgc aaattcttggaataaagtgaattaaaagtgaaagtgaattctttaagcactcgcattaagtgagggaaataagcttaaaaacacccacgg acttaaagagaatgagacgcagaaacggagacttggattttgctctttttctcgaattgaacgcaaagatcacttgaagaattccggcctga aaaccgtaggcatcgccattcccagtgactcttcaaatattccgtgcgcgaaactgcagagctctttggttatgcaaaagacctccgaaaat aacaaacaacaggcacaaaaacataaactaaacgctggtggagccaaagacaaacgaatcacgtcgctgcagctggtcaagtgaa atttttcttcgtaatgaaaatcaaacattatccgggcaaagccgtcgatgcgagtctctcgctggagggcagcagtgccatgggtgccctgt acgaggccaattggctgagggctgcgaatcagcccgccgctccagccacaacgggcaccaaattgagcaggcagagcagctccgcc ggcagcagtttcctcatcgagggcatctccgccctgagcaagtaccaaatgaccctggagaatatccggcagctggagctgcagtcgcg tgacaaacgcatagcgagcacgattaaggaactcagcggctataggccatcggcactgcagcatcatcagcagcagcagatgcataa cgtttgggtggccgaggatcaggatcaggaacacgaagaactggaagatgcttcggagggaaaggagaagttggctagtatccagga accccctgcagttaaccactatgtcctggacccgacggagagaccaagggttcccagaccccggcaacagttctccgtgaaaccgcctt ccttgcgtcgctcccaaaccatgtcgcaaccgcccagttatgccaccttgagatcgcccccgaaaatcaaggaaaatcttagcaaaagtt cctccgcctactccacattctcctcggctgcagaggattcccaggatcaagtagtgatctgccaacagccacagagactgatggctccgc cacccagagaaccgcctccggagccacccaaaagggtatcgaaacccctgagcagatcgcaaacttcggttcagcggtacgccacg gttaggatgcccaaccagaccacttcgttcagcagaagtgtggtcaggtctagggattccaccgcctcccagagacgtctcagtttggagc

aggccatcgagggactcaaactggagggggagaaggctgttcgtcagaagagtccacaaatctcaccagctgccagcagtaatggca
gctccaaggatctcaacggggagggtttctgtatcccacgaccgcgcctcatcgttccagtgcacacatatgcccgtcgacgcaggacgg
gaaatctcaaggagcaatccagcggtgggcaggaggaggaagcagagaagggtaaggggtggaaggatttctatgttttaagtcagg
ataggcatagtagcttttatatcaataggataggacagaattatgattatgattacccaattaattttaatatattttctccagatggacgcgtgg
aggtttcccgcgagggcaagtacctctccacattgtcgggagcgaaggtccttggcgaattggcgatcctgtacaactgccagcggacgg
cgaccatcaccgcgatcaccgagtgcaacctgtgggccatcgagcgccagtgctttcagaccatcatgatgcgaacgggcctgatccgg
caggcggagtacagcgatttcctcaagagtgtgcccatcttcaaagacctggcggaagacacgctcatcaaaatctccgatgttttggag
gagacgcactaccagcgtggcgactacatagtgcgccaaggcgcccgaggcgataccttcttcatcatatccaagggaaaagtgcgag
tgacgatcaagcagcaggacacgcaggaggagaagttcattcgcatgctgggcaaaggggatttctttggagagaaggctctccaggg
cgatgatctgcgcacggcgaatattatttgcgagtccgccgatggcgtcagttgtctggtcatcgatcgcgagaccttcaatcagctaatttcc
aatctagacgagatcaagcatcgctacgacgacgagggcgccatggaacgcagaaagatcaacgaggaattccgggacattaacct
cacagatctgcgcgtcatcgcaacccttggagttggaggcttcggtcgcgtagagctggtccaaactaatggagatagctccaggtccttc
gcgctcaagcagatgaaaaagtcacagattgtggagacgcgtcagcagcaacacatcatgtccgagaaggagatcatgggcgaggc
caattgccagttcatcgtgaagctgttcaagaccttcaaggacaagaagtacctgtacatgctgatggagagttgcctgggcggagagctc
tggacgattctacgggacaagggcaacttcgacgacagcaccacccgcttctacacggcatgtgtggtggaggcctttgattacttgcact
cgcgtaacatcatctaccgcgatcttaagccggagaacctgctgctcaatgaacgaggatatgtgaagctggtggactttggctttgccaa
gaagctgcagacgggcaggaagacctggactttctgcggcactccagagtacgtggctcccgaggtgattctcaaccggggccacgac
atcagtgcggattactggtcgctgggagtgctcatgttcgagttacttactggtacccctccattcacgggctcggatcccatgcgcacctac
aacattatacttaagggcatcgacgccatcgaattcccaaggaatatcacccgcaatgccagcaacctgatcaagaagctctgtcgcga
caatccagccgagcgtttgggctaccagcgtgggggaatcagcgagatccaaaagcacaaatggttcgatggcttctattggtggggcct
gcagaactgcacactggaaccgcccattaagcccgccgtgaaaagcgttgtggatacaacaaactttgatgactatcctcccgatcctga
gggtccgccgccagatgatgtcactggatgggacaaggacttctgaggagaatcagaacccgtttcctagacgatgctctctaaacgctt
ctgctgcagaaaaccaggaggatatgaaagccagggaagaaaaattgatcttaagtgcgccatatgtacgccaaagccaacagcaa
cagtcagcagctcgcatcgaaaagctgccacaaaaaaaaacaaagaaacgtagcagtcgcaaggtcaagggccgacacaaaagc
acaatcatccatcgtcgtagctccatttgagatttatagatacgtctccgtgatgttataaccatgatgtgcaacgcaatgaatttattaacgag
tttataactattattttataatgaggatatatgtgtctagttcgcttggaattgatgtaaattgtaagtaggtctgtgactctgtttcagagctctgttag
ccatgtgcattgtataaattcagctatttgtatctattaaatatttttaacataattattacacatcattgttaaagcatacaaatcgggttgccttata
gtctgtaagagaacatttgaaagcaacatttgaccaagatcttccgtcacacatttcttaaaattctatgtggcctctctactgtctttcattagtct
tagcgatcatgtctattatatttacgaataacatgcc (SEQ ID NO:135)

**Protein:** AAG22254
**Protein GI:** 10727354

MKIKHYPGKAVDASLSLEGSSAMGALYEANWLRAANQPAAPATTGTKLSRQSSSAGSSF
LIEGISALSKYQMTLENIRQLELQSRDKRIASTIKELSGYRPSALQHHQQQMHNVWVAEDQDQE
HEELEDASEGKEKLASIQEPPAVNHYVLDPTERPRVPRPRQQFSVKPPSLRRSQTMSQPPSYATL
RSPPKIKENLSKSSSAYSTFSSAAEDSQDQVVICQQPQRLMAPPPREPPPEPPKRVSKPLSRSQT
SVQRYATVRMPNQTTSFSRSVVRSRDSTASQRRLSLEQAIEGLKLEGEKAVRQKSPQISPAASSN
GSSKDLNGEGFCIPRPRLIVPVHTYARRRRTGNLKEQSSGGQEEEAEKDGRVEVSREGKYLSTLS
GAKVLGELAILYNCQRTATITAITECNLWAIERQCFQTIMMRTGLIRQAEYSDFLKSVPIFKDLAEDT
LIKISDVLEETHYQRGDYIVRQGARGDTFFIISKGKVRVTIKQQDTQEEKFIRMLGKGDFFGEKALQ
GDDLRTANIICESADGVSCLVIDRETFNQLISNLDEIKHRYDDEGAMERRKINEEFRDINLTDLRVIA
TLGVGGFGRVELVQTNGDSSRSFALKQMKKSQIVETRQQQHIMSEKEIMGEANCQFIVKLFKTFK
DKKYLYMLMESCLGGELWTILRDKGNFDDSTTRFYTACVVEAFDYLHSRNIIYRDLKPENLLLNER
GYVKLVDFGFAKKLQTGRKTWTFCGTPEYVAPEVILNRGHDISADYWSLGVLMFELLTGTPPFTG
SDPMRTYNIILKGIDAIEFPRNITRNASNLIKKLCRDNPAERLGYQRGGISEIQKHKWFDGFYWWGL
QNCTLEPPIKPAVKSVVDTTNFDDYPPDPEGPPPDDVTGWDKDF (SEQ ID NO:136)

# Figure 58

**Name:** Ac13E
**Nucleotide:** AE003499
**GI:** 10728265
**Transcript:** CT26306
**Sequence:**

gaattcaaatcgaatcgaataacgtacgcatagcgcgagaggcaaatttccaattgccaataaataacgaagaaaa
tacgacgaaaatgcttggactctaagtgaattgtttattttaacgttattgtcgcgcatttgccgaccattcgaatatatgtgcaatgtttaactca
cgttcatggaatttgtgtggattgttattcgtcgcgccgccagtcggtgagctccagggcgatgccgccgggcgttctggtcaacgatagcc
gtgcgaactccaccgatgacatccagatcgccttggcgccccacatccagacctacctgagccagacgggccggcggcactcctgctg
cagcgtgatgctcccagtggcattcgagcgggcggcggccaaatcctggctggaccccaagttcgattccccggtcctcgaggagcagt
accaggccagcgtctttccacacgtccgcatgcgatacaggttcaccctctcgtacatcctgctctgctcgctgatgtggtgcctgtactttgtg
gtggatggcggatcggaggacttctggcgcccaatctctagctccttttcgatgctctcgctggtcaccatcatggcgttgtgcttcacccattg
ggacctgtacagggagcaccgaacggtgacctcggcggtaactgcgctgctactttgcggcgcctcattggcattcctcacctacacggg
ccgggcgttcagtccgctgggtcatttcgccatctgcctggagatcgttctgctgatctacacggctctaccgatgcccttgtggctgggcgcc
agtacggcgattagctattcgattgccttcgagatggtctcgcacatggtaatcggatgcagtgcgattcatggaggtcccatgcacggcgg
aggtggagcagctgggggatcaggaatggaagccaacggagatcccagcaataggatactcatactgcggataatggcccatctgag
tgtgcatttggtgggcgtgcatgtgctgatcatgaatctggtgcgcatgcgcggcaccttcatgaaggtgggtcagaatctcctcgtgcgtcgt
caattggagatggaaaagcagctcaaggagaagatgatacactcggtgatgccgccgaaagtggccgatatgctgctcaatgagggtg
gtccgtcgggtctggatgccggtggattgccaccggaatcccactacatgcgaccacgtgcctccaacgatgtgaagtccctgttccgacc
attccacatgcacagcatggagaacgtgagcattctattcgcggacatcgttggcttcacccgcatgtcctccacgaaaacagccgaaca
gttagtggagatactcaacgatctcttcgagcgttttgacgacctttgctcgctgagtggctgcgagaagatctccacgctgggcgattgcta
ctactgcgtgtccggttgtccagagccacgggcggatcacgccatttgctgtgtggaaatgggtctgggcatgattgatgccatgcgctgctt
cgatgcccagcgccacgagggtgtcaagatgcgagtgggcgtccacacgggaacagttctctgcgggatcgtcggcacgaggcgggt
gaagttcgatgtgtggagcaacgacgtcagcctggccaataaaatggaatcctcgggcaaaccggagcaggtgcacatctcgcagga
gacatccagctttttgggcgatgcctactacttggaggagggcgaggaggtgtttggtcatcgtacttacttcgtggtgggaagacgacgcg
acttcacacgaaccaatagcctgagtcccagcatgcctgcgaatgccactggcagttccctgctcttgcccggcgcccatggcgcctccct
ttcccagagtgccaccaatgtgtcggcggtgcagccgaacgttcctccggcctcgccggtgggtcagctatccagctcgctgaatccctcg
ccggtactctccatgcgaccgcgtctcacctcgttgagcatgaagatgcgtaagaagtcgcagagccgcgaccgggatgtggaacgtgg
catcatccatccggcggcagcgggaattcctccggtgattgtggtgcgggaacgaccgaagatcatcattaccaccaagtccctgcccg
gcagtctggactctgatgaacaaccaccagtcagtccaccgttgctaccgccggcaaatccaccagcggccgaaacgcgatcgaagat
caagctaaaggtgtggaaaattcctcgattccttaagcgatttgaggatttaaccaacaggagcagctgtggcggcagctcctccgccggt
catctggacaaggaaagagagagggagcgggagaaggagaaggaggagctgcaccagcataatcaactgaatcccgaggagac
cttggcattcatggacaaccagatgcaaaatggcaacggttgtggctatcagcagttgcccgttttggtagagtccaaccatcgcactcag
accctggacattccggccgctcgtccggtgctccatcatgcagctacatccacggcactggcaagcagtgtgctcagatcgccggaggc
gggagtcagtggaggcgggggttgctgttctcctggtcagtactccatgtacgatgacatcatcgatgtgcgatcctacatcagtcagtcac
ggagcgatatatctccattcggtcgttcgggcagctatagatcgcaatgtggccgccagtcaacgggaggggtgaacggagcaggacc
agcaggagttggggccgaaggaagagctggtacctcccagacagcgccgccagtagagcaatcgccactcccgcgaccacgagca
tccaccttggcaaccggtagacccacaccgaatagcctggaaccgggcccttcctcaacgagtccctgttgcttgccagctccgggcaac
tccgtcggaggtggtggcatctttccgcccacgcactcccgccagtcgagcatctgcccatcggccacgtcgcgcaaggattccgggatc
aagagcaactcgcgcagatcctcgatccaacagcaaatctatgcactcaaccagacggcgattagtcagcacagggtctccggttacttt
accagctccacgtccagtatctccaatttgggagaggtccagggactgggtcttccgctggcggtacaaccgccaccgcctcttttgatgcc
cgcctgctcttcgcaaatggcggatccactggccgcctgcctgcagcaattgcgcaagcaatcggatcttcagcttatccgttgcgttcggg
ataatgccaggtcgcagaggagctatctggtcaaaccgccgttgcggaaattcagtttatacttcaagtcacggcagttggaacgcgatttc
cgctcgaaggcccatcgttttggagcggaaaatgagacggaaggaccgccgactttggccacgccacgctataatacctacattgatat
attcgtggggattgcggtttatctgtgcatctccgtctcgctgtttttgatgacccagaacacggtgtcgcccagcttccggctgtgggtgacctt
gttctcctgcttcacaggcatccaagtgttcgccctcttcctcttcacaaggcaaatgtgtcgccggcagagcggtagcaatgttagcaatcg
attccgttccaagtcgacgaccagtgaggatttggagcgggatgagcgaggagctgcaccgggaggaagaccacattttgagtcctgtg
ccgatcgcattttcgaggccatatccagctggtatccgtggcacatttgcctggccgttctgatggccatgccagtgctgttgatcatcgccaa
ctttctgctcctggacttggagcaactggaggcgtttgagtaccattatggattcctgatctttgtgtgcatcgtgcacttctgcaatttcacacag
ctgaattgttgggtgcggaatgtactggccttttggcagccctctgctttattggcattgcagtgtcccagttgatggtctacagtcacaatcgg
agtgaccagcagcaggatcaggaggcgtcgaatttcatccaggagatcaagtggtttcaggactatcatgtggaaatctatctggatctgc
tgctcatcctggtgttggtctggttccttaatcgcgaatttgagattggttatcggctgaccttctacggcaatgcggtggccaatcaggataag
gtccgagtgcagaacatgaagaaccaggccgacatgctgctccacaacattatacccaagcacgtggctgagcatctgaagaacacg
gccaagtactcggagaaccatcacaacatagccatcatctttgcctcgatcgtgaacttcaatgagatgtacgacgagagttatctgggtg

gcaaggagttcctgcgagtgctcaacgagctgattggtgactttgatgagctgctctcacgtccggaattccgggctgtggagaagatcaa gacaattggttcgaccttcatggcggccagtggattggatccctcgcatcgcggcacgggggacgaacatattcacacactaatggagtt ctcaatcgccatgcaggaggtggtggatgccttcaacaaggatctgttggaattcaatctcatcctgcggattggcatgaacattggcgatgt gacggcgggagtaatcggtactagcaagctgtattacgatatttggggcgatgcagtcaatgtggcctccagaatggactccaccgggct acccaatcgcattcaggtgggcaaggattgcctgcccttcctcaccaatcgctatgagttcgagccacgtggcagtgtctatgtaaagggc aaagatcacatggaggtgttcctctacacgacacgaagagataatccactggatgatgacgaggctgataaggttgaacaattgggcac caagaagatggagcacgaggggcgggatgaggatgtggtgcagggcgagcagcaagcggaggacaaagacgaggaggaggag gaagaggaggaggatgatgacttgcactccagtgagaccaccacgctcttcaagtcacaggaatcgctgcaggcgaatggtggcagc cacttggcacccaacgccgcagccaccagccacacccacacaattaccctaaccaacaacaacaaccataacagcagcaacatca acaacaacattgccaccagcaacacaacaacaacagccaacaacaactcaatgccagtggaaacttaggaaaattgcggggtagc aaggcacttgaaaaatggtttcaaaattgcatttaatgactctaacggtagggcagcaagtttcccggacaaccagaccatccgtcgtcga caaccacaagtggctaagagtatggagctgttgccagaaaaccaaaccagatccaggggataaaaaatcggtggggcggggcgag gaattacccgaaggggtggggggttgaaaattaaaaaaagcaaccgtgtttttgtgatataaatgaatatgaatagatagaaacgttagg aacgatagaatcgggtaaaagttgctagtgattgattggttaattgattgatccatcgattgattgttgttaactgcgtttttcgggatggctaacg tgttggagcattgaaactccaaacagaagcagaccattcggcatatgcaaaatatgtgtgtgtttaagtgttactagccgccagttgactcat ctgcacaaccaaacgaagtgctttctttccattctaccactgttttttttcaaatagaattaactacacttaattaaactaatggaaatatgtggc tttaaatggtagtagcgaaatatagatgtggattgtatgtagattcaatgtaccatagagttaacccactatgcatataagtaactaaataaca attttttgagccaacgaatggatattacctgtatgtattggcccgttgttcgatgttgatcaatgtgtgtatataaatgtacgtagctaaagttaaa cgattgctgattgtgtttcgcagacgaagatctgttgccatataagcggggtttttggggtcggattatttcggatttcgaagaatcataaccaa atgataatttcgtcgtctgtttttagctattacatcgcagtaaatggcgtggaaattggtttcccagtgtagcttcatatatttttttaaagttttttttct agtaaagcagttatacgatctacatatgtatgtagttataacatgcagcttttacataaaatgtaccccagcaaacgaccgaaaagtatgca ccaaaatgcttgccatacacacacaaagacacgcactcacccaccaaaaaaaaaagctttcaccacactcacgcacacacacacac acacacacaccaacccccaagacaccggaaaaaataattttatagcctttgtataattttttatacaaacacaccttttgtgcaatgcaac attgccagtttctaatggtaaacttaagcagaactctatttccgccacgaaaaaaa (SEQ ID NO:137)

**Protein:** AAF48468
**Protein GI:** 7293083

MPPGVLVNDSRANSTDDIQIALAPHIQTYLSQTGRRHSCCSVMLPVAFERAAAKSWLDPK FDSPVLEEQYQASVFPHVRMRYRFTLSYILLCSLMWCLYFVVDGGSEDFWRPISSSFSMLSLVTI MALCFTHWDLYREHRTVTSAVTALLLCGASLAFLTYTGRAFSPLGHFAICLEIVLLIYTALPMPLWL GASTAISYSIAFEMVSHMVIGCSAIHGGPMHGGGGAAGGSGMEANGDPSNRILILRIMAHLSVHLV GVHVLIMNLVRMRGTFMKVGQNLLVRRQLEMEKQLKEKMIHSVMPPKVADMLLNEGGPSGLDAG GLPPESHYMRPRASNDVKSLFRPFHMHSMENVSILFADIVGFTRMSSTKTAEQLVEILNDLFERFD DLCSLSGCEKISTLGDCYYCVSGCPEPRADHAICCVEMGLGMIDAMRCFDAQRHEGVKMRVGVH TGTVLCGIVGTRRVKFDVWSNDVSLANKMESSGKPEQVHISQETSSFLGDAYYLEEGEEVFGHR TYFVVGRRRDFTRTNSLSPSMPANATGSSLLLPGAHGASLSQSATNVSAVQPNVPPASPVGQLS SSLNPSPVLSMRPRLTSLSMKMRKKSQSRDRDVERGIIHPAAAGIPPVIVVRERPKIIITTKSLPGSL DSDEQPPVSPPLLPPANPPAAETRSKIKLKVWKIPRFLKRFEDLTNRSSCGGSSSAGHLDKERER EREKEKEELHQHNQLNPEETLAFMDNQMQNGNGCGYQQLPVLVESNHRTQTLDIPAARPVLHHA ATSTALASSVLRSPEAGVSGGGGCCSPGQYSMYDDIIDVRSYISQSRSDISPFGRSGSYRSQCGR QSTGGVNGAGPAGVGAEGRAGTSQTAPPVEQSPLPRPRASTLATGRPTPNSLEPGPSSTSPCC LPAPGNSVGGGGIFPPTHSRQSSICPSATSRKDSGIKSNSRRSSIQQQIYALNQTAISQHRVSGYF TSSTSSISNLGEVQGLGLPLAVQPPPPLLMPACSSQMADPLAACLQQLRKQSDLQLIRCVRDNAR SQRSYLVKPPLRKFSLYFKSRQLERDFRSKAHRFGAENETEGPPTLATPRYNTYIDIFVGIAVYLCI SVSLFLMTQNTVSPSFRLWVTLFSCFTGIQVFALFLFTRQMCRRQSGSNVSNRFRSKSTTSEDLE RDERGAAPGGRPHFESCADRIFEAISSWYPWHICLAVLMAMPVLLIIANFLLLDLEQLEAFEYHYGF LIFVCIVHFCNFTQLNCWVRNVLAFLAALCFIGIAVSQLMVYSHNRSDQQQDQEASNFIQEIKWFQ DYHVEIYLDLLLILVLVWFLNREFEIGYRLTFYGNAVANQDKVRVQNMKNQADMLLHNIIPKHVAEH LKNTAKYSENHHNIAIIFASIVNFNEMYDESYLGGKEFLRVLNELIGDFDELLSRPEFRAVEKIKTIGS TFMAASGLDPSHRGTGDEHIHTLMEFSIAMQEVVDAFNKDLLEFNLILRIGMNIGDVTAGVIGTSKL YYDIWGDAVNVASRMDSTGLPNRIQVGKDCLPFLTNRYEFEPRGSVYVKGKDHMEVFLYTTRRD NPLDDDEADKVEQLGTKKMEHEGRDEDVVQGEQQAEDKDEEEEEEEEDDDLHSSETTTLFKSQ ESLQANGGSHLAPNAAATSHTHTITLTNNNNHNSSNINNNIATSNTTTTANNNSMPVET (SEQ ID NO:138)

# Figure 59

**Name:** CG2667
**Nucleotide:** AE003676
**GI:** 7298935
**Transcript:** CT9029
**Sequence:**

ttagtgctgactttccggttgctctttgacacgtactcgacttttccggacttttggttttcgcaactaatcaaagcctctctctt
gcagccgaacgaagcacttcccaatcttcagaccctaaaggggtccaagtcgagcttgtttatggggtccactttattcggtttcgatcacttg
gcttccgcagaaaaggacaaggaggaaaagagcggcaaagacaaggagaagacgcccaccgacgagaccaaccgcaagttgc
ccatcatcaatcctctagtgcgactgcccaactggccaaatcttgccaatggcggtggctttatatccaaatgtcttttggccaatgctgatac
cctctgcgccgccgtcagtcccctaatggacccggatgagacactgctggccggttaccatgagaagtgcgtgatgaacaactactttgg
catcggcatcgatgccaagatctcgctggacttccacaacaagcgtgaggaacaccccgaaaagtgccgatcacgagcccggaacta
catgtggtatggggttctgggatccaagcagcttctgcagaagacctgcaagaatctggagcagcgagtacagctcgagtgcgacggtc
agaggattccgctgccggaactgcagggaattgtgatactgaacatacccagcttcatgggcggtaccaatttctggggcagtagcaaga
aggatgacatattcctgccgcccagcttcgatgatcgggtgctagaagtggtggctgtcttcggatcggtgcagatggctgcctcgcgtctg
attaatctacaacatcatcggatcgcccagtgccaaagcgtacagatcaatatcctgggagacgaggagatacccatccaggtggacg
gagaagcctggcttcagccacctggaatgattcgcattctgcacaagaaccgagtgcagatgctatgcaggaacagaagcttggagctc
tcgctgaagagctggcaggagaagcagcgccagcacagcatctccatccagagggatgcatcttctacagcttcagagcatgccaact
ccacggacgaggtaatctccgaacgtgaatgctacgtgctcctcaacttcatcgaagccgtcagctcgttggtgaagtgggtcaagttcctg
attatctcgcatccggccctgcagcacgacctatacgaggtggcctgccgggccagtgaagccctggaatcgattcatccgcagggcaa
actactcgaaggtccttcgttgcgcaccaagttggtggaagtcatagactcatcgcggcagctttacgatgatgcatgcaccctgctgcgcg
accgaggtcatagtttgattctccgtgaggatttggaaacaaagctcagcgcggcattggccaacatggaaatggagctgaaaaagtgct
ccgtgcagaagtgcattgatggaaagctgcgggcctactttaatgttctggcgcccaacgaagagtccgatggccgccgaaagtctcga
cctttctgggtgagattgcgttccggttcgaccgccggtcagcaggcgtttaagccacctttgaccaacacccgcgaggcggccaacaact
ggagcgtcaacgaggtggtcacctggttggaaacgatgcagctgtcggagtacgtggacagcttcctgaagaacgacattcgcggcaa
ggaactgcttaccctgggcaggcgtgacctcaaggatcttggcgtggttaaggtgggacacgtcaagcgtatactgcaggcaataaagg
atctcagcgagaactag (SEQ ID NO:139)

**Protein:** AAF54154
**Protein GI:** 7298950

MTRGSCISSPIPAYCAFTAEQNGQRRLSLLFYTDVSRFYGFIFLQVETRRAGTAKIAMQHR
ERIITPTRSLVLCAESRREMEDWLGSLKTATAPQRPRGDSFLIEQHDILSNHHHWYATSHARPTYC
NVCRDALSGVTSHGLSCEVCKCKVHKRCAAKSIANCKWTTLASVGKDIIEQADGIIMPHQWMEGN
LPVSSMCAVCKKTCGSVLRLQDWRCLWCRATVHVACRPQMAVACPIGPAKLSVVPPTSVHSIST
DDAWDVASPKGNFSPLLVFVNSKSGDNQGVKFLRRFKQLLNPAQVFDLISTGPSLGLRLFRHFEM
FRILVCSGDGSVGWVLSEIDRFNMHKQCQVAVMPLGTGNDLARVLGWGSSCDDDTHLPQILERY
ESASTKMLDRWSIMVFEKAIPVPKTPKMSISTEQEAMLTGMVTSANHHLRFIVETNDTQTLIRSTR
NLCDTVDDLVCRISEHHKDDEQLAVKCDILRQKLNMLLDALQEEEIGAHSGDDLIATIRSLIARSIPV
TPGSNAYLLNPNISIEKTEKDQINTKERRNSRSLRSSEKEALQCRANSVKRAIYNVVEHSEPGRPK
RYQRKLSITPFEALKLPTASGESTPCTSPLPIIPPINIISPTMETSRLTCISPLPDTRRDSVDENFFNSI
NLPAPRQFADSRRSSGVEVIQEIEEGANGETVYRKSRMSLTGGANIDDAGNRLSPCSDAGENTPT
ERKVDFLRVPIHTGEPIVDPLCDYRPHEVFERTYYMTREMDKGKEKDKEKDKPVEIDKEKDTCVE
KEGSMPAEKLVHTCNLQVPGVVVTPNPQNVYSSASITIIDTDAQTTTEQSSSDDLGGEASDVLSAI
SNEECSVASEIFDKQDAGQTVGDIIQNMDASNFTHIDSPETSDETEAMPGESIMDDISSVLGHDITY
ALQDNTLTDDTTTLCSEHAGPPKPPRKKSLSALSRTQAHPRRRNSSPPRMARLARMDSDDNPQQ
FGFENIVFEIDNRCDDQKMREPPRYCSLAQFVEGNDIARQSFKQLMLEQHRGGDNDSDYPEHEQ
TPTNKGANLLATTSEDELSTQTAIKIEIQDIDATVRNLNSSMKPNTILTTSTSPTKKSGHGQDISVVV
RPPTPLRGDCIKPTVSLLPVSSGGAMTVSMTCSGMLGVRAMNASEIRRHSSHAPGLAVREFDKD
KDRRHSGFNPNQLTLDPEHARFLSSSPAASRRISCGSLFKKNKKIGTKRSYGLFSVRFFVVAEPDF
RLATLALIRPLIPLLVLTFRLLFDTYSTFPDFWFSQLIKASLLQPNEALPNLQTLKGSKSSLFMGSTLF
GFDHLASAEKDKEEKSGKDKEKTPTDETNRKLPIINPLVRLPNWPNLANGGGFISKCLLANADTLC
AAVSPLMDPDETLLAGYHEKCVMNNYFGIGIDAKISLDFHNKREEHPEKCRSRARNYMWYGVLG
SKQLLQKTCKNLEQRVQLECDGQRIPLPELQGIVILNIPSFMGGTNFWGSSKKDDIFLPPSFDDRV
LEVVAVFGSVQMAASRLINLQHHRIAQCQSVQINILGDEEIPIQVDGEAWLQPPGMIRILHKNRVQM
LCRNRSLELSLKSWQEKQRQHSISIQRDASSTASEHANSTDEVISERECYVLLNFIEAVSSLVKWV

KFLIISHPALQHDLYEVACRASEALESIHPQGKLLEGPSLRTKLVEVIDSSRQLYDDACTLLRDRGH
SLILREDLETKLSAALANMEMELKKCSVQKCIDGKLRAYFNVLAPNEESDGRRKSRPFWVRLRSG
STAGQQAFKPPLTNTREAANNWSVNEVVTWLETMQLSEYVDSFLKNDIRGKELLTLGRRDLKDL
GVVKVGHVKRILQAIKDLSEN (SEQ ID NO:140)

# Figure 60

**Name:** CG7842
**Nucleotide:** AE003525
**GI:** 10727872
**Transcript:** CT23794
**Sequence:**

ccttcaacaaaaatgttggccgcccgcagattactacgatcaccgcggattaccggtgccctaagctggtcccgctgg
agcagcgatgccgcgaaggccacaacagaaacctccgcccttttgcagaacgcggagaagcggcagcaactgctcaacgagctttct
gagccgctggaacagaaaggccggcccgccatcgatcccaaggaaacaagtgtgatgctctttcccggtcagggcacccagtacgtg
ggcatggccaaagacctgctccggttcccgggcgcccgtcgcatcttcgagctggccaacgaggtgctgaaatacgatctgctcaagat
ctgcctggagggaccacgtgagaaactgaatcgcacggagcacgctcagctggctgtgatggtatcctcgctggcagcactggagcag
ttgcgtgaggagcgacccaaggccattgaaacgtgtgtagctgccgcaggctttagtttgggcgagattaccgcattggtgtacgcggatg
ccctgcctttcgacaaggcattgcgattggtgcaggtgcgggccaccgccatgcaggcggcatgtgaccaggcagccggagccatggc
gatgactctctacggaccggataccaatctgggagaggcctgcgctcgtgcgcagcaatggtgtctggacaaaggagtggagtcgccct
actgtggcatcgccaactacatgtatccgcactgcaaggtagtggccggcaacgtagaggccctggagttcctggaacaaaatgccaa
gtccttcaaaatccggcggatgaagagattggctgtcagtggcgcctttcacactcctctaatgcaaagcgccgtggagccatttaccaaa
gccctgaaaacggtgcgacttcaggaccccgtcattagagtgtactccaatgtggatggcaaaccctatcgccatgccaagcacatccta
acgcagctgcccaagcaaatcgtgaggcccgtcaagtgggagcagactctccacgagatgtacgagcgcaagcagggcgtcgacttt
ccacgcaccttcgaatgcggtcctggcaagggcctggtccaggtcctggaaaaagtaaatgccaaggccgctcaatccagctttaatgtt
atagcctaa (SEQ ID NO:141)

**Protein:** AAF49377
**Protein GI:** 7294021

MLAARRLLRSPRITGALSWSRWSSDAAKATTETSALLQNAEKRQQLLNELSEPLEQKGR
PAIDPKETSVMLFPGQGTQYVGMAKDLLRFPGARRIFELANEVLKYDLLKICLEGPREKLNRTEHA
QLAVMVSSLAALEQLREERPKAIETCVAAAGFSLGEITALVYADALPFDKALRLVQVRATAMQAAC
DQAAGAMAMTLYGPDTNLGEACARAQQWCLDKGVESPYCGIANYMYPHCKVVAGNVEALEFLE
QNAKSFKIRRMKRLAVSGAFHTPLMQSAVEPFTKALKTVRLQDPVIRVYSNVDGKPYRHAKHILTQ
LPKQIVRPVKWEQTLHEMYERKQGVDFPRTFECGPGKGLVQVLEKVNAKAAQSSFNVIA (SEQ
ID NO:142)

# Figure 61

**Name:** CG17486
**Nucleotide:** AE002751
**GI:** 7289853
**Transcript:** CT38665
**Sequence:**
aacgtcaataatgtgtggaatattttgttcagtcgtgaacaatgttcctttaaactcctttaatatttctagcgctctgaaagta
attctgaaaaaccgcggtcctgatgtgcaagacgaggtggttatagattattgttttggaaaaattttatttgctggcttcgtgttatggcaacaa
ggggaaagtgtacaaaagcaacctgtggtggaggacgattttatatttcttttcaatggagacatttacaatacttcaaagcctgaatatatgt
ctgacacgacttggatagcggaaaggctagctgaatgccgttgtaaagaacagaatattttgaagatacttaagcgattggagggaccac
attgtttaataatttatgacaaacgagaacaaatcctttactttagtcgcgacgcacttggaagaaattcactattaattgaacgcatttgtaatg
gcttccaattattgagcacatcacattttgaagataaaaaaatatcactggaattgcccccattgggtctttttcgagttaaactaaatgatctg
aattcgtgcgtattgtatccatggcagcccctcaataattattcgactcagcttttacgcaaccttgatcttgcgattgggtggaaaactgcagt
agagagtccaatgtcgccagattggatgttaaattgcaaccccgcatttagttataacttctatgaatttcaatacatcaagaataacgtgaa
cctttataaaaacttaatagatcagtcagaaattaagtatactctatctattctccatacactgctatccgattcgataaaaaatcgtgttagaa
acatggctccattttgcagactctgcatgcaaaaattaaatatatcgcctccatgcagacacgcaaaattgtgtattctattttcgggtggcctt
gactgtacaattttggccttgctggccaaccaatttgtaccagtaaatgaacccatcgaacttattaatgttgctttcgaaagcgttaagggcc
aaaatatatcggaaaagctttttgatgtaccagatagaaaaacgtcattagtatctgtcaatgaactaaaacagctttgtccggagaggtgc
tggaatctactgaaagtgaatgtaactcgacttgaattgcagcagcacctaacatcacgtataaagcatcttatatatccgctagatacggt
attagatgagagtcttggatgtgccttctggtttgcttcgcattgcactcactctactgctagagtcgctcttatcggaagcggggcagatgaac
tatttggtgggtatacccgctatcgcaacagttatagccgttgtctgggaaacgatctggagcgccaacttgctgtgcaaaacgaacttgaa
agggactggcaacgtatttcggcccgtaatttagccagagatgaccgcattattgcagatactggtaaaacagcaagatccccattcatcg
aagagaactttgtaaaatttataagatcgttagaagtgtatcaaaaatgttgctttggtttcccggaaggagtcggcgataaattacttctgag
actgtatggctatcaaatcggtttgcgagatgttgtgttcttaaagaaaagggctattcaatttgggtcccgaattgcaaacaaaaaacaaa
atggctctcatcaatctgataatctttaa (SEQ ID NO:143)

**Protein:** AAF45462
**Protein GI:** 7289856
MCGIFCSVVNNVPLNSFNISSALKVILKNRGPDVQDEVVIDYCFGKILFAGFVLWQQGESV
QKQPVVEDDFIFLFNGDIYNTSKPEYMSDTTWIAERLAECRCKEQNILKILKRLEGPHCLIIYDKREQ
ILYFSRDALGRNSLLIERICNGFQLLSTSHFEDKKISLELPPLGLFRVKLNDLNSCVLYPWQPLNNYS
TQLLRNLDLAIGWKTAVESPMSPDWMLNCNPAFSYNFYEFQYIKNNVNLYKNLIDQSEIKYTLSILH
TLLSDSIKNRVRNMAPFCRLCMQKLNISPPCRHAKLCILFSGGLDCTILALLANQFVPVNEPIELINV
AFESVKGQNISEKLFDVPDRKTSLVSVNELKQLCPERCWNLLKVNVTRLELQQHLTSRIKHLIYPLD
TVLDESLGCAFWFASHCTHSTARVALIGSGADELFGGYTRYRNSYSRCLGNDLERQLAVQNELE
RDWQRISARNLARDDRIIADTGKTARSPFIEENFVKFIRSLEVYQKCCFGFPEGVGDKLLLRLYGY
QIGLRDVVFLKKRAIQFGSRIANKKQNGSHQSDNL (SEQ ID NO:144)

# Figure 62

**Name:** CG6969
**Nucleotide:** AE003740
**GI:** 10726705
**Transcript:** CT21569
**Sequence:**

atgacggacgaaacaacgccgcttaccgatgccgttcccagtggctccggatatgttgtcctgccgccgtatcaggga ccggagcgtgtctttccgggtggtgtttctccgcgtgcgcgacgcaacaagatgcgacagttccagtgctgcatgggcatcacctttatagc gattgtatttaccgccctgtgcctggcactggtgttcagtgattccctgggcggagcggatggaggtcccagcttttttttcgtcgtcaatggatc ggatagcgaattggccccaaataggccactgccagacgaaccggctgcggaatgggcgctgcagcaggcggcactgggtcatcatg atggtgctcaggcagtcagtgctgggattaaggcactgggagatcgggaaatactggaggagggtctgcagccgaacgaggtgaatac gccgtccttccgacactatcgttccctgagcacaaatccagaggcaaggaagctggcacgtcgcggctatgtggagaaccaggccacc atagacattgccaagaggtttaactacaccaaacagccgggacgcagcaacattggttggggacccaagatagttctgccagatcctac ggtactccgtttggaatgcgatttcaatgcgcgctacagaagatccaccggggtgtgtaacaataaacaacatcccaggacctacggag catcgatggttccatatcgtcgcatggtgtcgccggactatgcggatggtattgctgctccgcgagttagtcatcatggacggttaccgccgg ctcgtcaggtctctctgaaaatccatcgttccagctacgaaacggactcgaattttacggtaatgctggccgtttttggtcagttcatggaccat gacatcacagccacatcgctgaccacttcgcaggagggtgagtccatcgattgttgtgtggctgccacccgcgagcagcatccggaatg ctatccggttgatattctgcccgatgatccctactacaagcagtacaacatcagttgcatgaatttcgtgagatctgctccagcgccaacggg tagatttggtccacggatgcagctgaaccaggccacggcatttatcgatgcctccgtcgtgtacggcaatctggagcagcggcaaaatca gctgcgcagctttatcaacggatctcttcggatgtttgtcacggatgatggtcgccagctattgcccatttcctcgaatccggcagatggctgc aatcgtgtccagatgactcgactgggtaaatactgtttcgagtccggcgatgatcgggccaatgagaacctgttgctcacatctatgcatttg ctgtgggctagacatcacaactatttggctcgacaattgcaggagcagaatccacattgggaggatgagcggctctaccaggaagcccg aaagattcttggtgctcagatggcacacatcacctacaatgagtttttgccagtgctgctggggaagaatatcagcgaggccaagggcttg ctgcctgccaaacacaatctaaatgctccggatacgtatgacccggaggtggatccaagtattgccaattgctttgcagcagccgcatttcg atttgcacacactcttctaccgggattgtttaatatctcgagggataacagcactcccgaggccatcgaactgcacaagatgctcttcaatcc attctcgctctgggcggagcatggcattgaccacgccctgatgacagcggccaatacgccggtgatgcaagtggatcgcttcttttcactgg aagtcacgcaaaagcttttcgaaggcaccgcagaggatagggtgcctctttgtggactcgatttggtatccttaaacattcagaggggtcg ggatcatggcataccctcgtatccagtcttccgtcgccattgccgcctgccgacggtggacacgtgggaggagatgtcacaggccattga caacgctactctggactccatccggcagatttatgaatctccgcaggatgtggacgtctatacgggtgcacttagtgagccgccactggatg gagctatttttggtccgttgctcagctgcatggtttccgaccagttcctacgcctcaaactaggtgactcccattggtacgaacggaaaatgg gcccacagaagttcaccaaagctcaattggcggagatctacaagaccagtctggccgccatcatttgtcgcaactcagatggaatcacc cgagtgagggagcatgttatgcagagactgcgcgatggaggtaatccccatgtggattgccaggacctggagggattccatttcaatttcg aaccctggtccgagaagcaacagccccaggatcttcatagtgccggcataagcaggggatccacttcggtgcgagtaatgtccaaggc aaatcatcaagcccacaacgtaaccctacatattgataaaggaatctagttag (SEQ ID NO:145)

**Protein:** AAF56043
**Protein GI:** 7300903

MTDETTPLTDAVPSGSGYVVLPPYQGPERVFPGGVSPRARRNKMRQFQCCMGITFIAIV FTALCLALVFSDSLGGADGGPSFFFVVNGSDSELAPNRPLPDEPAAEWALQQAALGHHDGAQAV SAGIKALGDREILEEGLQPNEVNTPSFRHYRSLSTNPEARKLARRGYVENQATIDIAKRFNYTKQP GRSNIGWGPKIVLPDPTVLRLECDFNARYRRSTGVCNNKQHPRTYGASMVPYRRMVSPDYADGI AAPRVSHHGRLPPARQVSLKIHRSSYETDSNFTVMLAVFGQFMDHDITATSLTTSQEGESIDCCV AATREQHPECYPVDILPDDPYYKQYNISCMNFVRSAPAPTGRFGPRMQLNQATAFIDASVVYGNL EQRQNQLRSFINGSLRMFVTDDGRQLLPISSNPADGCNRVQMTRLGKYCFESGDDRANENLLLT SMHLLWARHHNYLARQLQEQNPHWEDERLYQEARKILGAQMAHITYNEFLPVLLGKNISEAKGLL PAKHNLNAPDTYDPEVDPSIANCFAAAAFRFAHTLLPGLFNISRDNSTPEAIELHKMLFNPFSLWAE HGIDHALMTAANTPVMQVDRFFSLEVTQKLFEGTAEDRVPLCGLDLVSLNIQRGRDHGIPSYPVF RRHCRLPTVDTWEEMSQAIDNATLDSIRQIYESPQDVDVYTGALSEPPLDGAIFGPLLSCMVSDQF LRLKLGDSHWYERKMGPQKFTKAQLAEIYKTSLAAIICRNSDGITRVREHVMQRLRDGGNPHVDC QDLEGFHFNFEPWSEKQQPQDLHSAGISRGSTSVRVMSKANHQAHNVTLHIDKGI (SEQ ID NO:146)

# Figure 63

**Name:** CG12262
**Nucleotide:** AE003558
**GI:** 10728071
**Transcript:** CT16549
**Sequence:**

tttcgctggcagccaccgacgagttagcaaggcgacctagttaaaactcaggaaaatacgaaaataaacccacatt
caaatggcgttcctcaacaagcttgctgcgcctgccctgcgccagttggtgtcccaaagtcgcgcctacgccgccgtgtcacacgtttcgcc
caatggcacctctttcgcactcaccgaggatcagctgcagctgcaggaactggcacgtaagttcacccgtgaggagatcatcccggtgg
ccgcccagtacgacaagagcggcgagtacccgtggcccatcatcaagaaggcctgggaactgggcctgatgaacaaccacattcctg
ctgatattggtggcctggatctcgatgtgttcaccacctgcttgtcggccgaggagttggcctatggctgcactggcatcatgaccgccttaga
ggctagtggtctgggccaaactcctgtgatcctgtccggtaacaaggaacagaagaaaaagtacctgggccgcctgctggaggagcca
cttgtcgccgcctattgtgttacagagcccggagcaggatccgatgtgtccggcatcaagacacgcgccgagaagaagggtgatgagtg
ggtaatcaacggccagaagatgtggatcaccaacggtggtgtggcgaactggtactttgtgctggcccgcaccaatccggatcccaagt
gcccgcccagcaaggccttcaccgggttcattgtggagcgcgacagtcccggactaacgcccggccgcaaggagttgaacatgggac
agcgcgcctccgacacgcgtggcatcaccttcgaggatgtgcgcgtgcccaaggaaaatgtgctgattggcgagggtgccggtttcaaa
attgccatgggcacctttgataagacgcgtcctccagttgctgccggagctgtgggtttggcccagcgttgcttggatgaggctcttaaatac
gcgctggaacgcaagacctttggcgtgcccatcgcttaccaccaggctgtgcagttcatgctggctgatatggccatcggtgtggagacat
cccgtctggcgtggcgtctctccgcctgggaaatcgaccagggacgccgcaacagctactatgcctccattgccaagtgccatgccgcc
gatatggccaacaagattgcctccgatgccgtccagatctttggaggcaacggcttcaacagcgagtatcccgtggagaagctgatgcgt
gatgccaagatctaccaaatctacgaaggtacttctcagatccagcgcctcatcatttcccgaaacatgtacgaggctgctaagggtcaag
cctaagtcagttttaactaggattcctcatctccattcacctcatcagttgtcatgctcattgtagacacattcacaaatccgattaataaagtac
aataagtaaataattacaagaaaatataaatca (SEQ ID NO:147)

**Protein:** AAF50524
**Protein GI:** 7295201

MAFLNKLAAPALRQLVSQSRAYAAVSHVSPNGTSFALTEDQLQLQELARKFTREEIIPVAA
QYDKSGEYPWPIIKKAWELGLMNNHIPADIGGLDLDVFTTCLSAEELAYGCTGIMTALEASGLGQT
PVILSGNKEQKKKYLGRLLEEPLVAAYCVTEPGAGSDVSGIKTRAEKKGDEWVINGQKMWITNGG
VANWYFVLARTNPDPKCPPSKAFTGFIVERDSPGLTPGRKELNMGQRASDTRGITFEDVRVPKEN
VLIGEGAGFKIAMGTFDKTRPPVAAGAVGLAQRCLDEALKYALERKTFGVPIAYHQAVQFMLADM
AIGVETSRLAWRLSAWEIDQGRRNSYYASIAKCHAADMANKIASDAVQIFGGNGFNSEYPVEKLM
RDAKIYQIYEGTSQIQRLIISRNMYEAAKGQA (SEQ ID NO:148)

# Figure 64

**Name:** fray
**Nucleotide:** AE003722
**GI:** 10726601
**Transcript:** CT23267
**Sequence:**

gctgaattttggttgtatatttatttcgtgccgtcgaatcgcttcgctaagctgactttttgactttttcgtgtagtttgcactcgaa
aggagaaagaaatcagccgaaatatatcccccgaagatattggcgatagcccggttggcgtatatattccagtgaatcggtggattgcca
gcaaagcagccaaaagcaagaacaacatcgaaaaacgtttcgtcacgctgagagagcagtggaatttttcgcaacaaatacaattaa
aagttgcacgcagaagtggaggaagaggcgacgaaagtcagaaaatcaatttgtgaataatacgaaatcggtgcaagataagttccc
gtgtgaaattgctttttcggcgattggattttcatcgattttgcaaagggaaaacagaggtggcacacatcgccaacaacaacaacaacca
gaaccaaagcaaagaatcccaagaaagagatccgaaggatacaggaatctttggatgttttgccaagaaggaagagcagcagcata
atcctaaaaagaaacaatcagaaggacccagagagagttccaactcgggcagctataaagtccagcaaacggagcaagaacaacg
aagggaatcggagaacgagcatccatccgtttaccgccagcaggacgaggagtttcatccgataaaaaaggaggagccccagaaaa
ccccacccatatccaagcaaccccgtccgcggaataccccaagatcgcaaagtcagccggctattaacaaccgacgacgtccagga
accccgccgcctcacacagtcgcacctggaaacggaaccgcaagcagcaggagcatgacctccatacccgccaatctgtctagcaat
aatgttgctggtgcagcaaccttaccaggagctgcggcaccgccagagaaatatacatggccgaactccaaggacgattacgagctga
gggacgtcattggtgtgggtgccacagccgttgtccatggggcttactgtattccaaggaacgagaagtgcgccattaagcgcatcaacct
ggagaagtggaacacatcgatggacgagctgctcaaggagattcaggccatgtcctcctgttttcacgagaacgtggtgacctatcacac
ctcatttgtggttagggaagagctctggttggtgctaagacttctagaaggcggttctttgctggacatcatcaagcacaagatgcgaacgtc
caactgcaaacagggtgtcttcgacgaagccaccattgccacggtactgaaggaagtcctaaagggattggaatatttccattcgaacgg
gcaaatccatcgtgatatcaaggccggcaatattttgattggtgacgatggcaccattcaaatcgctgacttcggtgtgagtgcatggctggc
cactggcagggatttgtccaggcagaaagtgcgccacacctttgtgggcacaccgtgttggatggctcccgaggtgatggagcaggatc
atggctatgactttaaggcggacatttggtcctttggaataacagctatcgaaatggccacgggcactgcaccataccataaatacccgcc
catgaaggtgctaatgctgaccctgcaaaatgatccgcccaccttggacacgggtgcggacgataaagatcaatataaggcgtatggca
agacattccgcaagatgatcgtcgagtgcttgcaaaaggagccatcgaaacgtcccacggctagtgagcttttgaagcacgctttcttcaa
gaaggccaaggatcgcaaatatctcacccagacgttgctgcaatctgggcccagtatggagaccagggttcacaaggcggcgaaacg
ccaaccaggagcctcgggccgtttgcatcgcacggtgacaggcgaatgggtctggtctagcgaagaggaggacaatggtggctctgcc
accggttccggaacaggagacagaaaacatccatcttcggactcggattccgaggatagacccatgaatcgattggaaagagccgact
catcggacagcgaccgggaagagccttctcccgaaataacccacagtgtttcctcagccacagtgacgcctggagctccagctgctgct
ggtgctggtgcagcccaagagctaacagctggcattgctcaattaccactacctagcgaagcggccggcgaggcgccaccagtcaatc
ttgtccttcgcatgcgcaacttgcgacgtgaacttcacgacattcgcttcgagttcgttgtgggcaaggacactgccgagggtatcgctacag
aactggtagacgccggactcgttgatgcgctggatacccaaccgatggcaacgcatctggatcaactgatagctgccagtgcaacgatg
aaaacgattactttccaattgagctccggcgtgcagccgggcgaagtgcccgacgaacgatcactggtgggatatgcgcagatctccatc
acggactagtg (SEQ ID NO:149)

**Protein:** AAF55567
**Protein GI:** 10726604

MTSIPANLSSNNVAGAATLPGAAAPPEKYTWPNSKDDYELRDVIGVGATAVVHGAYCIPR
NEKCAIKRINLEKWNTSMDELLKEIQAMSSCFHENVVTYHTSFVVREELWLVLRLLEGGSLLDIIKH
KMRTSNCKQGVFDEATIATVLKEVLKGLEYFHSNGQIHRDIKAGNILIGDDGTIQIADFGVSAWLAT
GRDLSRQKVRHTFVGTPCWMAPEVMEQDHGYDFKADIWSFGITAIEMATGTAPYHKYPPMKVLM
LTLQNDPPTLDTGADDKDQYKAYGKTFRKMIVECLQKEPSKRPTASELLKHAFFKKAKDRKYLTQ
TLLQSGPSMETRVHKAAKRQPGASGRLHRTVTGEWVWSSEEEDNGGSATGSGTGDRKHPSSD
SDSEDRPMNRLERADSSDSDREEPSPEITHSVSSATVTPGAPAAAGAGAAQELTAGIAQLPLPSE
AAGEAPPVNLVLRMRNLRRELHDIRFEFVVGKDTAEGIATELVDAGLVDALDTQPMATHLDQLIAA
SATMKTITFQLSSGVQPGEVPDERSLVGYAQISITD (SEQ ID NO:150)

# Figure 65

**Name:** CG6879
**Nucleotide:** AE003749
**GI:** 10726756
**Transcript:** CT21312
**Sequence:**

atgcccgcctcggagcggtaccgcacccacgacggcacctgcaacaacaaacgccggccgcgatggggtgccg
cccagatgcccttcaaccgcttcttgccgccagagtacggtgatggcgtggacactgttcggagttccgccgatggcagcaccttgtcctcg
tcgcgattcgttagccttttggtgcacggagcgcgggagggcgaggcaccgctcaccctgatgatcgcccagtggggtcagatgctagac
cacgacatgacctcgacggctcagccgcgttccatcaatggttccatacccagttgctgtggcggcaaggacttccatcccgcctgctttcc
catcaaagtgcccctggacgatccttggctggcgccgctcaaggttcgctgcctggagttcctgcgctcggctcctgcccagcgacgcga
ctgtgtgctgtcctggcgggagcagaccaaccaggtgacctcatacatcgacgcctcgcccatttactcgaacagcgccaagtcctcgga
caatgcgagagttttccggcatgggctgctcgtctatgggcgcggtgatcccgccgaggatgtgtgccaacgcggcgctattgccaccaa
gtgcattcgttccggagatggacgcagtggtgagcagccgggattgttggccatgcaccacgtctgggtggggaacacaaccgaattg
ccatggagctgagcgaactgaatcctcactggagcgacgagaaggtgtaccaggaaacgcgcaggattgtgggcgccatgtttcagca
catcaccttccgggagtttctgccggtcattttgggcagggaggtggtcaagctcttcgatctggagctgatgccctcgggctactacgaacg
ttatagttcaaaggtcaatcccacggtggccaatgcctttgcagccgccgccttccgttttggccactcgctggtccagaactcctacactcg
ctgcgatcgtcatcacaatgtgatcaataacaatgtcagcctgcatgaggagttccagcgaggtgacattggttcggcgggctcactgcat
cgtctgctacgtggcttggcctcccaaagggctctgaagagggatgagttcattacccccgagctgaccaatcacctgttccagactcctg
gctttccattcggcttggacctggctgccatcaacattcaacggggcagggatcatggcattgctccgtacagcgcctggcgagtgccctgt
ggtctaagtcccatcctcagttgggatgacttcgctaacgtggtgggtccggagtccgccaagcgcattggccacgcctatcgcagcgtgc
acgacatcgatctgtttgtgggcgggatcgcggaacgtcctgtggtgggtggcctggtgggtcccaccttcgcctgcatcatcgcccagca
gtttagtaactccaggcggggtgaccgcttttggtacgagaatggtggcttcgagagctccttcacgcccgcccaactgcactccttgcgcc
gcgtgtccttggcccaagtgctctgccgcacggtgggtggtggcaccctccagccccacatctttattccagcggagttcgaggacaatga
gagacagacctgtggtgtgggcagtctaagtcccatcgacctaagtccgtggttggagcaggatccctttcacaatcagcaggtgcccga
acaggtctttaccattgggcaacccgagttgggatccgttcagatcataaaggaggacaaggcgggcttctccaaccgggtgaaaccca
tcaagccacaggtggaggttagtccctcgtcacttagcggcttccatcgacccaccaacgaaaacttgaccaaagttagtgacaaacttg
acctaagacgcaagacggtgaccaaaaagaacaataccaacaacagacaacagaccactagaaaacccgtgggtggagtaaaca
acaagctggacaagtcccccaaaatcactttaaacatcaagagcgtaaatgtgagaagaccagagggatcgggactcaaagaagg
gtgatcatcaacaatgtgcccgtagagctgcgaagctctggaggtaatgccacggctgaggagacagaaccaggaaatttgacagac
gaagacgacgagtttcacgacgacgcaggcgaagtggtggacatggaggacatagtggaagtgagggctagcaaggatgatccatc
gaaactgaccaagacggacttaaaccaaaacagaaatgattcgaagacaactgaaacagaacagactgaaaagaacactaccatc
gaacccacggaagattctactgatgaaccaaaaactgaaagacgtgacgccagaaagatggaacctcatccagcagcaaagactcc
ggacaagacagcagactctagactattgcacaaccaacgcaaccgtaccactaccccaagggacttccaagccagctccaccgtgag
cctgaaacgcttcacgcgacagacgaaggcaccgaaggttagtaagcccaccaaaagggccgaggaactgagacagtaccagaa
cagaccgctctacgagagaccccaaaaggtggtggtcaacggaccgaacgccgaccagtacgagattgagatcaacatacgacag
acgaacaagaatcccgcatcacgaccatcgacttctgactacgcgcaatacacgactgccaacaaaccgtactataactccaactacg
caccacactacatggactacgccagtaccacaccaattccattgcccagctatggctaccatcaaccgattggggagatcagtaaccag
ggagtaagaacgaagccaccgaccatcatttatctgaacgacaacgacgaccgacggaccacaactcgggcgccgaacatcttcca
aaactttctgacctttgccacgaacagtttcaatcccttggggataagacgacccatgcccacgactccatccccgatctcgcagagccac
aaggagccaggacagttcgagaacaatgtggttcacagtccgatcagtaactcgcacatactgactggaggaggtccgtccgcctcgta
ctcaccgcgaccgccgtcggtgcactcgtatccggtggccaccagttcccagattggtggcaatcccggatcagggttcctgcacgcttcc
agcagcgcagttagtgcagggcactttggcagcatttcgggagtggccactgccaatggagcggacagcacatttagcttcaacatacg
gccacgtcccagtccggatttgggttcagtagtgagctcctatccacgaccggtttcaagtcagggcggaggttcatcttacagacctgact
acgctcctacccagagcgaactgtactatgacagagaactgatcactgaccgaaatcccagcccattttccggatcaagttccgcccaaa
gaccacatcaacagacattctacggacggagcccggaactcaaggaagtgggcaacaatagcaacgaactgacccatacggagca
caaactgtacctacaggactacgactatgccagcagaacgctttctaatctgaccacggacgagagtcaccaaactgatgacgacacc
gattacgtatacgacgaagacatagccagcaatgacctggataagacgaagacccgaaaggagacggacctcaacgaattgaccac
aaaaaagttcgaccaggacggctacatgcgaccacaacttatgcgagacgcgacccacaactcgaccggatacaacgtgacctcact
ggacgacaactacgtaatgcccatgctggagaacaggacgcggacgaactatgtgaccgaggtgcccaagcccatgctgtccacctc
cagccggagtgtgacgaacagcaaggtgaccgtctttccggacagcctagggaaacagttggccaacgacatccttgcggacgaggc
gacggacgactacgtggacgcgaatgggcatacagaagcgcgactaaaggcccagaaactaaagcaactggccagtgtggcgttcg
caccaattaccgtactgaccaaaccggacagaccggacaactgggtgatctacaacaaggccagcgaggagccaccactgccaca
accgccagcagtcaacatggatgtggcgcccagcggagaggtgcccactccgatcaagaacttcaacaatgcgtggctaaagcagga

tctaaaggctcggccggagacaccacctaccagattggcggaagaggtgaccacggtggcggcagcggacagtatctagaaatgcc
aaattagccctagattgattatttatttatttatacttcaaaggcaagaattcaatccaactat (SEQ ID NO:151)

**Protein:** AAF56334
**Protein GI:** 7301203
MPASERYRTHDGTCNNKRRPRWGAAQMPFNRFLPPEYGDGVDTVRSSADGSTLSSSR
FVSLLVHGAREGEAPLTLMIAQWGQMLDHDMTSTAQPRSINGSIPSCCGGKDFHPACFPIKVPLD
DPWLAPLKVRCLEFLRSAPAQRRDCVLSWREQTNQVTSYIDASPIYSNSAKSSDNARVFRHGLLV
YGRGDPAEDVCQRGAIATKCIRSGDGRSGEQPGLLAMHHVWVGEHNRIAMELSELNPHWSDEK
VYQETRRIVGAMFQHITFREFLPVILGREVVKLFDLELMPSGYYERYSSKVNPTVANAFAAAAFRF
GHSLVQNSYTRCDRHHNVINNNVSLHEEFQRGDIGSAGSLHRLLRGLASQRALKRDEFITPELTN
HLFQTPGFPFGLDLAAINIQRGRDHGIAPYSAWRVPCGLSPILSWDDFANVVGPESAKRIGHAYRS
VHDIDLFVGGIAERPVVGGLVGPTFACIIAQQFSNSRRGDRFWYENGGFESSFTPAQLHSLRRVS
LAQVLCRTVGGGTLQPHIFIPAEFEDNERQTCGVGSLSPIDLSPWLEQDPFHNQQVPEQVFTIGQ
PELGSVQIIKEDKAGFSNRVKPIKPQVEVSPSSLSGFHRPTNENLTKVSDKLDLRRKTVTKKNNTN
NRQQTTRKPVGGVNNKLDKSPKITLNIKSVNVRRPEGSGLKRRVIINNVPVELRSSGGNATAEETE
PGNLTDEDDEFHDDAGEVVDMEDIVEVRASKDDPSKLTKTDLNQNRNDSKTTETEQTEKNTTIEP
TEDSTDEPKTERRDARKMEPHPAAKTPDKTADSRLLHNQRNRTTTPRDFQASSTVSLKRFTRQT
KAPKVSKPTKRAEELRQYQNRPLYERPQKVVVNGPNADQYEIEINIRQTNKNPASRPSTSDYAQY
TTANKPYYNSNYAPHYMDYASTTPIPLPSYGYHQPIGEISNQGVRTKPPTIIYLNDNDDRRTTTRAP
NIFQNFLTFATNSFNPLGIRRPMPTTPSPISQSHKEPGQFENNVVHSPISNSHILTGGGPSASYSPR
PPSVHSYPVATSSQIGGNPGSGFLHASSSAVSAGHFGSISGVATANGADSTFSFNIRPRPSPDLG
SVVSSYPRPVSSQGGGSSYRPDYAPTQSELYYDRELITDRNPSPFSGSSSAQRPHQQTFYGRSP
ELKEVGNNSNELTHTEHKLYLQDYDYASRTLSNLTTDESHQTDDDTDYVYDEDIASNDLDKTKTR
KETDLNELTTKKFDQDGYMRPQLMRDATHNSTGYNVTSLDDNYVMPMLENRTRTNYVTEVPKP
MLSTSSRSVTNSKVTVFPDSLGKQLANDILADEATDDYVDANGHTEARLKAQKLKQLASVAFAPIT
VLTKPDRPDNWVIYNKASEEPPLPQPPAVNMDVAPSGEVPTPIKNFNNAWLKQDLKARPETPPTR
LAEEVTTVAAADSI (SEQ ID NO:152)

# Figure 66

**Name:** CG11594
**Nucleotide:** AE003480
**GI:** 10727290
**Transcript:** CT34844
**Sequence:**

ctcgttgcgactggttgtgcgacaggtgtcggaataaataaaagcaaaaacattcgacggcttttgtttgcgctgataaa
caacctgcgcatatgtacaagcaaagcaaaatactgctcgaataattgtttgttcaaaacaatcaggtgtgccttttataaaactaaaagttt
ctaatttctaaacgagtaggtgtgcgcgcttctaataaaaaatttcggtattaaaaattaagttgttttaaaattaaatgctcttcagctttttaaga
tgcagcagaactattgtgctaaagaaaataaacaagcacaaatcacaaattacggtaaatgcaaaaatataaacaaaaacgtgcctga
aagatacggggttactgcgatcgtggtttaaataaaatcaaaagatacaaaaccatagcgatacaatacagatgcagatacatgtgcgtc
tgtggagtttggaaaatcaaaaattataccaaaaataaaccataattgatataaaaaaagcttatcatcatttttctatatttattgcctctgaca
attgtcgataacaattaagaatataaatatctcacttcttctgtggcaaattaagatatttcataactgtaactcaattataagtaatcaaatgag
atctttagaaaccggtacttgggttaatttacacagtttctagctgtacagggtctctaggcgttcgcatatcaggttgcttatcagttcgcgtccg
acgctcgacgacttcgcttcgcttttggtctgggattgggtttgggttttcgcatcgagccaataacatttatttgaccgccaaggtgattagagg
cgtctgtgacggagacggcgacttggaaatgtcatccggaccgtttttttgtgggcgtggacgtgggcacgggcagtgcgagggcggccct
ggtcgcctgcgatgggcgtgtcctggagcaggcggtgcagacgatccaaacgtggaatcctgagccgggctactacaaccagtcatcc
gataatatatggcagtccatctgccaggtggtgaagaaagtcattggcggcgtcgataagtcaaaagtcaagggcattggcttcgatgcc
acctgctccttggtggttctgggcccccagggatcgccgctgacggtgagcaagtccggtgaggcggagcagaacataatcctctggatg
gaccatcgtgccgagcaggaaacccaagagatcaatgccttcaagcactcgctgctgaaatatgtgggtggtcaggtttcgctggaaatg
gaggttcccaaattgctgtggctgaagaggaatctgtcacagacttttggaaatatttggagggtctttgacctgcccgactttctgacctggc
gagccactggagtggacacccgatcactttgctcggtggtgtgcaagtggaactacgatgcagcgaatggcagttggaacaaggagttc
ctgaagcaggcggacttggaggagttgacacagaataacttcgagaaattgggcagtgatgtccagccgcctggccgaacggtgggc
aaaggactcacggccaaggctgctggggaactgggcttatccgccggcactgtggtgagcacctccctaatcgatgcccatgctggcgc
cttgggaatgtttggatgccgctcaaaggagtccaagggtgccgacgatgtccagggtaaaatggctctgattgccggtacatccacctgc
cacatgagcatcacccggaaggcatgcttcgcacaaggtgtttggggtccctatcaggatgccattattcccggctacttcctaaacgagg
gtggtcagagcatcgcaggacatctcctagatcacgtcctcaagtcgcatgaatcctacgccgaactgaaatcccaattgggagaggat
aagtttatctaccagcatcttaataagttgctgccggaactggcagcagctcgaggattaagccaggtgggctgtctcacccaggatgtcc
atgtctggccggacttgcatggcaatcggtcgcccatcgcagatcccacgctgcgaggagtgattactggactggacatgacgcgagga
actgaatctctggccatcaaatacttggcgttcgtccaagctctggcttacggcacacgtcacattattgaaaacctttaccagtacggtaga
gctccctttcaaacgctacttttctgcgggggattggccaagaatcctctgtacgtacaatgtcatgcggacatttgcaatctgcccgcactga
ttccggatgaacaggaaatggttctggtgggcgctgctgccttgggagccgctgcctccggccacttcgacagcttggagagcgcctcga
aatccatgggtggcactggccagctggtgaagccgaacgcggaaacgcttgagttccacaatcgcaagtacaaggtgttcctgcagctg
ctggagaaccaacgacagtacaggcgcattatgcaggcagacacaaagtgattttttttcattccgatgtcaagactttcaataattttaaat
aaatgccgtacatgagcattgtaacaaataaaaagtttggcgcacacaggt (SEQ ID NO:153)

**Protein:** AAF47823
**Protein GI:** 7292419

MSSGPFFVGVDVGTGSARAALVACDGRVLEQAVQTIQTWNPEPGYYNQSSDNIWQSIC
QVVKKVIGGVDKSKVKGIGFDATCSLVVLGPQGSPLTVSKSGEAEQNIILWMDHRAEQETQEINAF
KHSLLKYVGGQVSLEMEVPKLLWLKRNLSQTFGNIWRVFDLPDFLTWRATGVDTRSLCSVVCKW
NYDAANGSWNKEFLKQADLEELTQNNFEKLGSDVQPPGRTVGKGLTAKAAGELGLSAGTVVSTS
LIDAHAGALGMFGCRSKESKGADDVQGKMALIAGTSTCHMSITRKACFAQGVWGPYQDAIIPGYF
LNEGGQSIAGHLLDHVLKSHESYAELKSQLGEDKFIYQHLNKLLPELAAARGLSQVGCLTQDVHV
WPDLHGNRSPIADPTLRGVITGLDMTRGTESLAIKYLAFVQALAYGTRHIIENLYQYGRAPFQTLLF
CGGLAKNPLYVQCHADICNLPALIPDEQEMVLVGAAALGAAASGHFDSLESASKSMGGTGQLVKP
NAETLEFHNRKYKVFLQLLENQRQYRRIMQADTK (SEQ ID NO:154)

# Figure 67

**Name:** S6kII
**Nucleotide:** AE003574
**GI:** 10803726
**Transcript:** CT34367
**Sequence:**

aagcaacacagctgtgcggattgcgaagcggatagtaaagcagacggcgtacgaaacgtgggcgcattaggaaa
gcgctgcttgttggccgtaggagcagcagggaaaccagagaaatccggagaggcaacagcgcagtcgactgcgacgcccacgcag
cggaagtaataaaaaagaaaagtgaaatcggaagtagggagagttcggtggaggaggagcagcaggaggagttggaaacgcagg
gggaactcgggctctagcattgtgcatcaggatctggaaacaaaggaaccgctaggagcagttgcctcacaacgctagcagccaccg
aagtcatgccgctggccgattcgcaaaaggatctccgccagcagcctcagcagcagcagcagcagcagcatgtgtcctccaccagca
gcagcaacaatgcggagcagcaatgcagcagcagcggattgggtctgcagctgcgccagcgcatgcaaattacctcgtccggctgca
gcagcctggcggtcacgcccatggagcacacgcccaccgaggacgatgagagtggcggaggcaacagcggtgtcacttcctcggtg
accactgtgacatcatcgcaacgccgccagcagctgcaacaggtgcaacagcaatctgctctgcaagcggccctcgagcagcatcac
atctcaccaacagcagacttggattccgctaggaaaaggccgacgcatcgaaccttgtgcccgccgccagaacttatggagctgagtga
ttccgagtcccagggaggcgtggaaactggcggtaggagagaaggggctactgggcgaagtgcacctgatttagaagacaccgagc
ccctatatgaaacagaaaacgaattcgagcttaaggaagtcatcaaggagggtcacgacaaggccgatccttcccagttcgagctccta
cgggttctgggcgaaggtagctttggaaaggtgtttctagtgcgaaagatcataggcaaagatgcaggaacactctatgccatgaaggtg
ctcaaaaaggccaccctaaaagtaaaagatcgcgtaaggagcacaaatgaacgaaaaatactagcggacgtgggtcatgctttcatc
gtacgtcttcactatgccttccaaactcccggaaaactctacttgatactggatttcttcgtggcggtgatctgtttacccgtctatccaaagaa
gtaatgtttacggaagaagatgtcaagttctatttagcggaactggcgctagctatgaatcacctacacacattgggcattatctacagggat
ctgaaaccggaaaatattctactggacgagcatggccatatagccttgacggactttggtctatccaagcagcctttggatggctcaaaaa
catatagcttttgtggaaccgtagaatacatggcgccggagatcgtgaaccgaaagggacacgattttgccgctgattggtggagtttcgg
ggtgctcatgtacgaaatgttaacggggaatttaccctttcatggccaaacccgccaagagactatgaatcagatccttagaagtaagctg
ggcatgccggagaatttgtcgccagaggcgcaatccctgctacgtgctctcttcaaaagaaacccccagaatcgtttgggtgcgggtgcc
caaggaattctggacatcaaggcgcactgctttttgccaccatcgactgggtgagattagaacgaaagcaggtgcgtccgcctttcatac
cggcggttagccgtgacgatgcctttttactttgatgtggagtacacctcaaagtcccccagggattctccgggtggcccgatctccgcatctg
cccatgagatcttccgcgggttcagctttgtggctcctgtccttctggaaggtcagtgtgctggctcgaatatgtgctccaccagcgccagtcc
tctgcatagtatagctcctattcctgctgctccagtgggagcccctcgaacattacctggtgttcttcccggaaacttccatgcggaatataatc
tactgcaagaactgggacgtggaaccttttcagtttgtcggttgtgcgagcatcgagcctccaagaaacattacgcagtaaaagtaatcga
aaaggcagctgtggccgcagcatccacatccacttctgccgattgttgggaggaggtggagattatgctgaggtacggcaaccacccaa
atatcgtcactctgtactctgtttacgaggatgcggggtccgcatatcttgtgatggagctgcttaagggtggcgagcttctcgatcggatactt
gccgtgggccagatgtgcgagagtgaggccagcgcggtgttaaggacaattgcatctgcggtagcatatctccatgaacatggcgtggtc
catcgagatcttaagccttcaaatatgatatatgccagtatgcggcaaactcccgagaccctaaagctctgcgatttgggtttcgcgaagca
gctgcgcgcggacaacggcctcctgatgacgccatgctacacagccaattttgtggctcccgaggttctaaagagacagggctatgacct
ggcttgcgacatctggtcgctcggtgtgctgctttacatcatgttatccggccggacgcctttcgccagcactccaaatgattcaccggacgt
aatactgaagcgcatcggatcaggacaaattgacttcacaagcagtcgctgggcactgatcagtgtgccggccaaagaacttttgcgtca
gatgctacacatagtaccggagaatcggccgacggcggcgcgaatacttgagcacgactggctgcgggagcaattcgccggcggcgt
acagcttacagagtatgcggtggcgcccggatcccaactttcgctgggcgcccagcagcagcagcagaatcacatctccatggccttaa
gaggcgctgttgatgccactttccgggctattgccataccccaggcggcgaatgtgggacccgtagaactttccatgctcgccaagaggc
gggccaaagatcgagccaacctgcactcctaatcctgggcggctgcatggtgtccgcggcgccaggccaagcggcagatagtccgac
tacttttccgaatctatagtattctaatccaatgctgctgtgccaatccgcgtcgtcttgtcaaggacaatgcgcagaagcttggttcacatcca
caaacgctgggcaatcctcgccaacgctgccgcagcgccaagctgcgtgtggtttaggttttgggggaatcggcctaacta (SEQ ID NO:155)

**Protein:** AAF50945
**Protein GI:** 7295638

MPLADSQKDLRQQPQQQQQQQHVSSTSSSNNAEQQCSSSGLGLQLRQRMQITSSGCS
SLAVTPMEHTPTEDDESGGGNSGVTSSVTTVTSSQRRQQLQQVQQQSALQAALEQHHISPTADL
DSARKRPTHRTLCPPPELMELSDSESQGGVETGGRREGATGRSAPDLEDTEPLYETENEFELKE
VIKEGHDKADPSQFELLRVLGEGSFGKVFLVRKIIGKDAGTLYAMKVLKKATLKVKDRVRSTNERKI
LADVGHAFIVRLHYAFQTPGKLYLILDFLRGGDLFTRLSKEVMFTEEDVKFYLAELALAMNHLHTLG
IIYRDLKPENILLDEHGHIALTDFGLSKQPLDGSKTYSFCGTVEYMAPEIVNRKGHDFAADWWSFG
VLMYEMLTGNLPFHGQTRQETMNQILRSKLGMPENLSPEAQSLLRALFKRNPQNRLGAGAQGIL
DIKAHCFFATIDWVRLERKQVRPPFIPAVSRDDAFYFDVEYTSKSPRDSPGGPISASAHEIFRGFSF

VAPVLLEGQCAGSNMCSTSASPLHSIAPIPAAPVGAPRTLPGVLPGNFHAEYNLLQELGRGTFSV
CRLCEHRASKKHYAVKVIEKAAVAAASTSTSADCWEEVEIMLRYGNHPNIVTLYSVYEDAGSAYLV
MELLKGGELLDRILAVGQMCESEASAVLRTIASAVAYLHEHGVVHRDLKPSNMIYASMRQTPETLK
LCDLGFAKQLRADNGLLMTPCYTANFVAPEVLKRQGYDLACDIWSLGVLLYIMLSGRTPFASTPN
DSPDVILKRIGSGQIDFTSSRWALISVPAKELLRQMLHIVPENRPTAARILEHDWLREQFAGGVQLT
EYAVAPGSQLSLGAQQQQQNHISMALRGAVDATFRAIAIPQAANVGPVELSMLAKRRAKDRANLH
S (SEQ ID NO:156)

# Figure 68

**Name:** CG11714
**Nucleotide:** AE003544
**GI:** 10727982
**Transcript:** CT36751
**Sequence:**

atttcgacacttctacagaaagactttcctatcagctaaattttctgttcgaaatcgtcgggaaaaactggatcgccttgcg
taagatgagtatctggcccattcgaacactaccaaattccaaaatgcgtagttgcatgatggagctcggtcgcaccaatcgccacacgga
ttgcacatttataattgaggatgagagcggtggcagtcaatcgtttccctgccacaaattgctattcagctgcgcctccgatgtgttcgatcgc
atgctctatggcgactacattgagtctactagcggagttgtcagactaaatgatgtacaaccggatatatttgagaaattccgagactatgtct
acggctatgagtgcgataaactgcagaagtacgactttgatactctgattcgcctctgtgagtttgctaacaaatatctggtgcaatctctcga
agaggactgtgtaaaggatttgctgatacgcaagaacacttttgacatgggagagttacttcgtctattccagtgtgcgcatcgcatgaatcg
caagtcgttaataaatcagatagcctgggagctaaagtgcaccttcaagagcaccttggaccactctggcgtatacgaattcaattgcgag
gtatttaaacactatatcgaagtgattgctagcaaaatatcggaggcagatcgtttccgtctgctggaaatgtatctaaaatataatggtatcg
aggaattggaaagcgcggggcaagtggatagccaggatgcaactgaagccaacaccaccaccatcaccaccaccaacgaagtgg
aaaatcaagaatcggaacttccgtccactagctgcgttccgctcaaaactgaatgcagttttgccgttccaaataagaaggcaagttttgtc
agcgatctacttgcactgattgattttggcaaactttcgcccaaggaattctacgatggacccggcaaatccaatttccttagcctggctgag
aagtacgagcatatgtatcaaatcgccaagaactgcgttacggccaaagatgaactgcagctgaagatggcattgaccacggaacag
aagccccccctaccttccgaatcccgacgcatagttcatatgggaggaagcttaatgcgcgacgaaccaacggcttcttcggttacctcgc
attacctttcccgacccatccgtcgatatcgcgcctggtcagcacacagtgacgtctaa (SEQ ID NO:157)

**Protein:** AAF50053
**Protein GI:** 7294716

MSIWPIRTLPNSKMRSCMMELGRTNRHTDCTFIIEDESGGSQSFPCHKLLFSCASDVFDR
MLYGDYIESTSGVVRLNDVQPDIFEKFRDYVYGYECDKLQKYDFDTLIRLCEFANKYLVQSLEEDC
VKDLLIRKNTFDMGELLRLFQCAHRMNRKSLINQIAWELKCTFKSTLDHSGVYEFNCEVFKHYIEVI
ASKISEADRFRLLEMYLKYNGIEELESAGQVDSQDATEANTTTITTTNEVENQESELPSTSCVPLKT
ECSFAVPNKKASFVSDLLALIDFGKLSPKEFYDGPGKSNFLSLAEKYEHMYQIAKNCVTAKDELQL
KMALTTEQKPPLPSESRRIVHMGGSLMRDEPTASSVTSHYLSRPIRRYRAWSAHSDV (SEQ ID NO:158)

# Figure 69

**Name:** CG3534
**Nucleotide:** AE003715
**GI:** 7300193
**Transcript:** CT11741
**Sequence:**

cggaagcagtttgaacatgggtccgccaaagcagctgaaacgaacctttcttggcttcgacttgagcacgcaaaagc
tcaaagccgtcctgctgggctccagtttggaggtggttgccgcggctgaagtgaaattcgacaccgacttgccggagttccggaccaccg
gtggagccaacgctggatccatcaaaaatgaatactttgtacaaccagtgatgtgggtgaaggccatggacattgtcctggaccgactgg
ttatgcagcaagcggatcttagcaccgtagctgccatcagtggctccggacagcagcacggatcgctctactggtccaaacatggaatca
acacgctgcaaaacctcgattccgagaagttccttcacgcacaaatcgacgactccgcctttgtggtaaatcgtactccgatttggatggac
gccaccaccaccaaacagtgcctagagatggagatggctgttggtggcaaacttaacatggtggaactgacgggctcgaagtgctacg
aacgttttacgggtccccaaattagaaagatatatcagcaacgctgccacgcctacgaagaggccaaccgaatctcattggtcagcagtt
ttatatcatcgctatttctgggctccgtcgctccaattgacttcagtgatggttccggcatgaatctgctggacatccgcaagaagaactggtcc
aaagaatgcctaaatgtttgtgcacccgatttggacaagcgcctggacattccggtcagtccaaattcgatttaggcaacgtctctccgtac
tttgtggagcgctttagtttttcgcccgattgcaaagtggctgctagcacgggggacaatccttcggcgctgtctggaatgttggtgggcagca
gctggcttaccatttccatgggcacaagtgacaccctaatgatgagcttgaaggagccgctaaactgggaggagggtcatatactctgcc
atccaactgaaacggaagaattcatgggccttctatgttttagaaatgcttcattggtccgtgaggaaatgaacaagaagacaaccggcg
gcgattgggataaattcaatgagtatctcgactccacgccccgtggaaactttgggaacatggccgtgcactttaacgacatggaaatcatt
cccaaggcgcagggtattctgcgatggaacagggaaatggatcccagttccccggacgcggctaggggtgtaatcaaattcagctcgc
cccaaatcgaaattcgagccctagttgaaggtcagatgctgcatcaccgggctgtggctgaagaccttggctacaaattcggacccgag
acacaaatccttgtcacaggaggcgcatctgttaacaaatcaatcctgcaaacaattgccgatgtgttcaatgctcccgtccatatccagga
tgaaggatttgaggctgccctactgggagccgcttatcgatccgcctacgctctgtaccttcaagaggcaggtgatggggtgacgcctctct
gttaccgagattatatcctaagtctgacaagtaacaaactgagccttgtatgcgaaccacacaaggacagcgaggaaatctacgctcca
atgctacaacgatatcgcgaaatggcgcgcgtcctgtccaatcctaacacataaactctccgaaagcccaacgtaacattccagtttatac
tccgtacattcacaaactgattccttcttaaacgtctcctgtaattgacagaacttaagtgcaattaacaaattctgtgattgacgatttattacaa
atttatgcgaaaccatatacaaataaatttttatatatggccc (SEQ ID NO:159)

**Protein:** AAF55371
**Protein GI:** 7300206

MGPPKQLKRTFLGFDLSTQKLKAVLLGSSLEVVAAAEVKFDTDLPEFRTTGGANAGSIKN
EYFVQPVMWVKAMDIVLDRLVMQQADLSTVAAISGSGQQHGSLYWSKHGINTLQNLDSEKFLHA
QIDDSAFVVNRTPIWMDATTTKQCLEMEMAVGGKLNMVELTGSKCYERFTGPQIRKIYQQRCHAY
EEANRISLVSSFISSLFLGSVAPIDFSDGSGMNLLDIRKKNWSKECLNVCAPDLDKRLDIPVSPNSIL
GNVSPYFVERFSFSPDCKVAASTGDNPSALSGMLVGSSWLTISMGTSDTLMMSLKEPLNWEEGH
ILCHPTETEEFMGLLCFRNASLVREEMNKKTTGGDWDKFNEYLDSTPRGNFGNMAVHFNDMEIIP
KAQGILRWNREMDPSSPDAARGVIKFSSPQIEIRALVEGQMLHHRAVAEDLGYKFGPETQILVTG
GASVNKSILQTIADVFNAPVHIQDEGFEAALLGAAYRSAYALYLQEAGDGVTPLCYRDYILSLTSNK
LSLVCEPHKDSEEIYAPMLQRYREMARVLSNPNT (SEQ ID NO:160)

## Figure 70

**Name:** CG7335
**Nucleotide:** AE003514
**GI:** 10727803
**Transcript:** CT22623
**Sequence:**

atgaaaaagcaaatcgtaagggagttcataaccgtaaagaaggtacttcacctgccgccggagccaccccccaccg
ccgccacctcctaagcgccacgttctggctgtgggcagctgcaccctggacatgatcaccatcgtggatctgcctctttttccaggccaggt
gcaacgcaccacggagggaagctggcggcgcggcggtcccgctgccaatatatgcactgtgtggcggcgactgggcttggagtgcga
attcctcggagttttgagcagggttagggcgttcgagagcctgttgaatggatttcaatcgcagggaatcgacatttcgcattgcccgctgac
gaatcaccggcctgcgcaccgcagcatcattgtccaacgtaacgtggatacgcgaaccatgctcgagttcgccaatgccaaccaggag
ctcacctaccagcagtttgtgggcgccgtggactaccagaagtactcgtggatacacttcgaatgtcgtaatcccgttgagatgctgcgcat
ggtcctggcggtcatacaattcaacgagcgctgccccgagtcaaggatcacactctctgtggatctggacaacttgcgaccggccaccat
gttgatggccagtatggtggactacgtcttcgcccgaaagaccatgatgcgtacgtactgttttatgaatggacgcgaggttgtttgggcgatt
aggaatgaaatgcgaagagcacggactcaatgggaaaaaagccagcccaaaaagatgccctatctacctcctgatcccccagatga
gcattccaacggatattgcccgggaccgctgaataaccaacccgtcgtcatatacaataattatatggaaggcgccagttgcctgatggcc
gacgacacctacttcaaggtgggctcccagattccgcccaaattagtggacgttgtggccgttaacgacaccttctcggcggccgtgatat
atgcactgattaaggtcaagatgcggctgagggatgccatcgagtacggaacccgggcgtcgtctctgaagttgacgggaaacggattc
gatgtgctgcggtgtatgcccaaagatctgattggttgttactatacttaa (SEQ ID NO:161)

**Protein:** AAF49067
**Protein GI:** 7293697

MKKQIVREFITVKKVLHLPPEPPPPPPPPKRHVLAVGSCTLDMITIVDLPLFPGQVQRTTE
GSWRRGGPAANICTVWRRLGLECEFLGVLSRVRAFESLLNGFQSQGIDISHCPLTNHRPAHRSIIV
QRNVDTRTMLEFANANQELTYQQFVGAVDYQKYSWIHFECRNPVEMLRMVLAVIQFNERCPESR
ITLSVDLDNLRPATMLMASMVDYVFARKTMMRTYCFMNGREVVWAIRNEMRRARTQWEKSQPK
KMPYLPPDPPDEHSNGYCPGPLNNQPVVIYNNYMEGASCLMADDTYFKVGSQIPPKLVDVVAVN
DTFSAAVIYALIKVKMRLRDAIEYGTRASSLKLTGNGFDVLRCMPKDLIGCYYT (SEQ ID NO:162)

## Figure 71

**Name:** CG11275
**Nucleotide:** AE003456
**GI:** 7291355
**Transcript:** CT31473
**Sequence:**

ttgcgctcgacggttgtcgctattctggttctcgctcaagttatccgaactcttggaacagctgggtagtctggtctggcctg
gcgcataaagacgcgatatcggtcaagttcagacgaagaagacgacgacgcggcgatcactcaattagcctcaccgtgagggcgcgt
gaaattcacttttaaatttcccccaactcgattttcacatacacaaattgcttttgttacgattctcgcgtgtgcttggttgaaaaggaatttgtcac
agtgccaaaagatgagcatctcggcggtggatcgcaaagagccgggcagtgtgagcagcggtctgggtaggcgatacgggcagctg
ctccgtggagccaagtacacagattgcgtgttccacgtgtgcgaggagcaagtgaaatgccacaagctcatcctgagctccgccagtcc
cgttttcgaggcaatgttctttggtcccatgcaaaacaacgagcccgagccggaaatcgaaatccacgacataagctctgccatcttcaag
gtgctggtggagtacatctacacgggcgtcgttgactacaatggcctggagctggtggcctgcattgagctgtactacgccgctgagaagt
atctgctggaggagctgatcgccgacacgctgatggttatcacccgcaagctgcgatttgccaatatcttgcccgctctagagctaagtgttt
gcatgggcctcgacgggttgctggaggtctgcatgaccttttttcatgcgatgctgtgttaataatggacagtatatgagccaccttaaggagc
actatgtgcacgtgtccaaggagtgtgtgaaggccataatagcggcgtgcaaggaaccgcacaagttactcatctggtatgtttatgagtg
gacacgacaggagtgcgagcagttgggtctgggtcccagcgatgccgacttagtagtgcacgacttgggtggcaaaacaagcgactgg
cctgcggcttcgggtgctagtgatttggaatcacctgccctaactccggtggttttggtggagcgctgctattacaaggcctgcaggcccttta
cagtggatgcagaaactccgctgtttcgcttgcgtctaaagtgctctagatttatctccttgatgggcttggtcctgaacagcaggctgactccc
aacctattgggtcacgtccagcagctggagtaccgcgaatcgctgcggctggacctttgcgaggtgcccgctgaaagcgagggtccggc
cacgtcgcccgtatggagtcacgttgtccagagccagaataccaagtacaactgcaacctgcatcttggctggcgacgcgaagaggcct
gtgtgctgaatccggagctgacctatgagctgcagatccgctgggacagcagtgcttacggcgccgagtatccgtgcagcctgcagtcct
gccaggcggacggaattcggttcactgacgaggacagcttcagcggaagtctcgtcaagggactgcgctatgccaacctggtgtag
(SEQ ID NO:163)

**Protein:** AAF46814
**Protein GI:** 7291386

MSISAVDRKEPGSVSSGLGRRYGQLLRGAKYTDCVFHVCEEQVKCHKLILSSASPVFEA
MFFGPMQNNEPEPEIEIHDISSAIFKVLVEYIYTGVVDYNGLELVACIELYYAAEKYLLEELIADTLMV
ITRKLRFANILPALELSVCMGLDGLLEVCMTFFMRCCVNNGQYMSHLKEHYVHVSKECVKAIIAAC
KEPHKLLIWYVYEWTRQECEQLGLGPSDADLVVHDLGGKTSDWPAASGASDLESPALTPVVLVE
RCYYKACRPFTVDAETPLFRLRLKCSRFISLMGLVLNSRLTPNLLGHVQQLEYRESLRLDLCEVPA
ESEGPATSPVWSHVVQSQNTKYNCNLHLGWRREEACVLNPELTYELQIRWDSSAYGAEYPCSL
QSCQADGIRFTDEDSFSGSLVKGLRYANLV (SEQ ID NO:164)

# Figure 72

**Name:** CG16726
**Nucleotide:** AE003551
**GI:** 10728019
**Transcript:** CT37213
**Sequence:**

atggatatggagtatataactagcagtagtggcaacataactgccactacagaagcagatttcagttcatctttgggcg
aatccaatgtcacggaatacaatacaacggaaatggacgcgaatgaatcagctggcgaggatgaggagatgctaaggatagcctttttt
atagggcacttcgtgcatcaatactatataccagtgctttgctgcacgggcagcattggcaatatcctctccgtgtttgtcttctttaggaccaa
gttgagaaagttgtcctccagcttttacctggccgctctcgcagtgagcgacacctgcttcctggccggactcttcgcacagtggctgaacttc
ctcaatgtggatatctataatcaaaactactttgccagttcttcacgttcttcagctacctggccagcttctgctccgtctggtttgtggtggccttc
accgtggagcgcttcattgccgtcatctatccactgaagcgccagaccatgtgcaccgtgcgccgggccaagatcgtcctgttttgtttgac
actcgtgggatgtctgcactgtctgccctatatagtcattgccaagccggtgtttatgcccaaattgaacacgaccatttgcgatctcaactcg
gaatacaaattccctgaatttttcatttgggttgccactagcaaccagcggcacaacggaaatttatctaccatttcccgcaccaatatttggct
ttaccaaaggtaa (SEQ ID NO:165)

**Protein:** AAF50229
**Protein GI:** 7294899

MDMEYITSSSGNITATTEADFSSSLGESNVTEYNTTEMDANESAGEDEEMLRIAFFIGHFV
HQYYIPVLCCTGSIGNILSVFVFFRTKLRKLSSSFYLAALAVSDTCFLAGLFAQWLNFLNVDIYNQN
YFCQFFTFFSYLASFCSVWFVVAFTVERFIAVIYPLKRQTMCTVRRAKIVLFCLTLVGCLHCLPYIVI
AKPVFMPKLNTTICDLNSEYKFPEFFIWVATSNQRHNGNLSTISRTNIWLYQR (SEQ ID NO:166)

# Figure 73

**Name:** CG7514
**Nucleotide:** AE003482
**GI:** 10727313
**Transcript:** CT2166
**Sequence:**

atggcgtacagcatcgaaaagaaatccatacccggctacatgatgtacattaatggcggtctggccggaatgctggg
cacctgcattgtgcagccattggatttggtgaagaccagaatgcagatatcagccacgacgggcgagtacaagagctccttcgattgcct
gctaaaggtttttaaaaacgagggcatacttgcgttgtataatggattgagtgcgggattgatgcgacaggctacgtacacgacagcccga
atgggtttctaccaaatggagatcgacgcctatcgcaagcagttcaatgcaccgcccactgtgttggccagcatgggtatgggcattctgg
ccggtgctttcggtgccatgttcggcaatccggcggaggtggccctcatccggatgatgtccgataaccgattgccaccagcggagaggc
gaaactacacgggtgtgttgaacgccctttgtgcggatcgtcaaggatgagggtgttatcaccctctggaaaggatgcatgcccactgtggg
tcgggccatgatcgtcaacatggttcagctggcctcgtactcgcagttaaaggcagcattttcggaatacttttcgggactttcgcttcacatcg
ctgccgccatgatgtccggtctattgaccaccattgcctcaatgccactggacatggccaaaacgcgcatccagcagcaaaagacggcc
gagtacaagggcaccatggatgtcctgatgaaggtctcaaagaatgagggtatcgcctccctgtggaagggcttcacgccctatttgtgtc
gcttaggaccgcacacggtgtttgcctttatattcctggaacaactcaccaaggcctacaagcacattgtgctcggcgacgattctgaatcg
aacatctaa (SEQ ID NO:167)

**Protein:** AAF47931
**Protein GI:** 7292529

MAYSIEKKSIPGYMMYINGGLAGMLGTCIVQPLDLVKTRMQISATTGEYKSSFDCLLKVFK
NEGILALYNGLSAGLMRQATYTTARMGFYQMEIDAYRKQFNAPPTVLASMGMGILAGAFGAMFG
NPAEVALIRMMSDNRLPPAERRNYTGVLNAFVRIVKDEGVITLWKGCMPTVGRAMIVNMVQLASY
SQLKAAFSEYFSGLSLHIAAAMMSGLLTTIASMPLDMAKTRIQQQKTAEYKGTMDVLMKVSKNEGI
ASLWKGFTPYLCRLGPHTVFAFIFLEQLTKAYKHIVLGDDSESNI (SEQ ID NO:168)

# Figure 74

**Name:** CG17283
**Nucleotide:** AE003717
**GI:** 7300241
**Transcript:** CT38253
**Sequence:**

atgcggtgcaggatcttggtgctgtgtgcattcatatgcctccttgtaattttgaccgaggccaggcagaggaaagttaa
ccattccgctgatagaagatcattggcagtcagaagtggacacaggaatgagcggagtagtgctcttcatggaagaggattggctgcac
gatcaaggaatcaaaggaaacgcagattaaatgctgccaagagaagcaggaggaagcgtaacttggctagaaagtcaaatcggaa
aattagggccaagaccgagtcaatcgttagaagtgattctagttacgctactttaccattggacttccaacagaacttcgtgcgaaccaccg
acaacctgcgatcggagaaggccttttggccaatcgatatggctttagttttgccaaaagttctggaacggctactttgaaaaacacggcg
aacatggagtatacctgcaaaatgaacattggtacacccaagcagaagttcacagttcttccggacaccggaagttcgaatatttgggtgc
caggaccgcattgcaagagtaaggcctgcaagaaacacaaacaatatcatccggccaagtcaagtacctatgttaagaatggaaatc
ctttgccatcacctacggttcgggtagtgtggcaggagttctggccaaggatacggtgagaatagctgggctggttgtgaccaatcaaactt
tcgccatgaccaccaaggaaccgggaaccactttcgtgacctcaaatttcgatggtatactagggctgggatatcgatccatagcggtgg
acaatgtgaagaccctggtgcagaatatgtgctcggaggatgtgatcaccagttgcaagtttgccatttgcatgaagggcggcggcagttc
ctctcgaggtggtgcaataatcttcggcagctccaatacaagtgcttatagcggatccaatagctacacatacactccggtgaccaaaaa
gggttactggcagttcactctgcaggacatttacgtgggcggcaccaaagtgagcggctcggtgcaggccattgtggattccggaacttca
ttgatcacagctccgacggccatctacaacaagatcaacaaggtcatcggatgcagggctacctccagcggagagtgttggatgaagtg
cgccaagaaaatacccgacttcacctttgtcatcgcgggcaagaagttcgtcgtcaagggtaacaaaatgaaattaaaggtgaggacca
acagaggcaggacagtctgcatatcggcggtaactgaagttcccgacgagccagtgatcctgggcgatgccttcatccggcatttctgca
cggaattcgacttggccaacaatcgcattggattcgctgccactacgtattccacttag (SEQ ID NO:169)

**Protein:** AAF55416
**Protein GI:** 7300253

MRCRILVLCAFICLLVILTEARQRKVNHSADRRSLAVRSGHRNERSSALHGRGLAARSRN
QRKRRLNAAKRSRRKRNLARKSNRKIRAKTESIVRSDSSYATLPLDFQQNFVRTTDNLRSEKAFLA
NRYGFSFAKSSGTATLKNTANMEYTCKMNIGTPKQKFTVLPDTGSSNIWVPGPHCKSKACKKHK
QYHPAKSSTYVKNGKSFAITYGSGSVAGVLAKDTVRIAGLVVTNQTFAMTTKEPGTTFVTSNFDGI
LGLGYRSIAVDNVKTLVQNMCSEDVITSCKFAICMKGGGSSRGGAIIFGSSNTSAYSGSNSYTYT
PVTKKGYWQFTLQDIYVGGTKVSGSVQAIVDSGTSLITAPTAIYNKINKVIGCRATSSGECWMKCA
KKIPDFTFVIAGKKFVVKGNKMKLKVRTNRGRTVCISAVTEVPDEPVILGDAFIRHFCTEFDLANNRI
GFAATTYST (SEQ ID NO:170)

# Figure 75

**Name:** BcDNA:GH07626
**Nucleotide:** AE003581
**GI:** 10727365
**Transcript:** CT11871
**Sequence:**

cagcattccgtcgttgtcgtggttttacaagccggtacgctacgtttgaatcccaaagaacataactgtgtgtggcagag
taattttcaaaaactaatagctactaactaagctaataacggcagtcaacggagaacttcgagtcgaaagcagtttcagtttctggattataa
cggtttccccattcagtcaggaccaactgtaaaggaacaccccccacgaaactgaattcgaatcctccgcgcagccctccgcccgctcc
actccaagaactcgaaaaacagctgtcgaggaatcgcctgatttctagcccctacaactaaacctgttgcaagccgtggaccagccgca
tcctgttgctttcaagcccaacaattgcagcacccatcagccccaacattcacccagcacccagaacaacggaaagagcgctcgcgcc
aatcgtcgtcgacgccgcagacgcaacaagcagcagcagaattcgcagcaacctgacgccttccgtgataccgacgcgcagctaaca
ccgagcacctcctcgtccaccccgcactctgcacccaacgatagacaggaccacctaaactcccaattcaatcccgaatccatcaatat
gcccgcccgattcgccgaggaagtcatcaccgctgagcccgcacaacgtgccgccccccaattggacctgggtggtggtcactatgtgc
cccgtcagcagcacctcaacgatgagatcgccatcaccggtttctccggccgtctgccggagagctccaccatcgaggagttcaagcag
aatcttttcgacggcgtcgacatggtcaacgatgatccccgccgttgggagcgtggtctgtatggacttcccgacaggattggcaagctca
aggatagcgatctggagaactttgaccaacagttcttcggtgtgcaccaaaagcaggctgagtgcatggacccactgctgcgcatgctgc
tcgagctcacccatgaagctattattgacgctggcctaaacccaagtgatctgcgcggttcccgcactggtgtgtacatcggtgtgtccaact
cggagactgagcagcactggtgcagcgatgctgaccgggttaacggctacggcttgacaggatgtgcccgtgccatgtttgccaaccgc
atctccttcacctttgatttcaagggacccagctacagcattgacaccgcttgctccagttctctgtacgccttggaacaggctttctccgatatg
cgcgaaggaaaggtcgacaacgccctggtcgctggagctggactgatcctcaagcccaccatgtcgctgcagttcaagcgactgaatat
gttgagcccggacggcagctgcaaggccttcgatgagtctggcaatggatacgtccgttccgatggatgtgtggtgctgctcctgcagcgc
acctctgcagccaggcgtgtgtatgcctccatcctcaatgtgcgcaccaatacggatggtttcaaggagcagggcatcacataccctattg
gcaagatgcaaaatcgcctgatccgcgagacctacgaggagattggtcttaaccccgccgatgtggtttacgtggaggcacacggtacc
ggaaccaaggtgggcgatccccaggaggtgaactctatcactgacttcttctgcaaggaccgtacgacccctctgttgatcggatcggtca
agtccaacatgggtcactcggaacccgcttccggtgtctgctctgtggccaagattctgatcgccatggaggagggcgtcattcccggtaa
cttgcactacaacaagccgaacccagatctttatggactcgttgatggacgtctcaaggtggtcgacaggaatctgccttggaacggtggc
atcatcggcctgaactcctttggttttggaggagccaacgctcacgttatcttaaagtcgaaccccaagcccaaggctttgacccccaagg
atggagcactgaaagtggtcctcgcctctggacgcacctttgaggctgtggagcagttgctcgagtccgcctctactaatgccgatgatgat
gagtacctgcaactgatcaacgagatccactcaaaggctattccgaatcacttcttccgtggctacggagtggtgagcagcaagggcacc
caccaacgtgaggtgatcgagtccaacgacgataaacgccctatttggtacatctactccggcatgggcagccagtgggctagcatggc
caaggatctgatgaagatcgaggccttcgccaagaccattcagaggtgtgccgatgtcctaaagcccgagggagtggatctaatcgatgt
cctgacccgttccacggacaagagcttcgaaaacattctgaactctttcatctcgattgctgccatgcaagtggctcttaccgatttgctcagc
tcgctgggcatccaccccgacggaattgtgggacactcggtgggagagcttggatgcgcctacgccgatggctgcttcaccccggagca
gaccgtgctggctgcttactggcgaggaaagagcattctggacactcaactggccaagggcaagatggccgctgtgggattgagctggg
aagatgcgcacagccgtgtgcccagcgattgcttcccggtgtgccacaacagcgaggacaactgcaccatctctggcccggaggcttcc
atcgaggctctggtcgctaaactgaacgcggagggagtgttcgccaaggcagtgaactcatccggctacgctttccacagcaagtacatc
gctgaagctggacccaaactacgcaagagtctggagaagatcattcccaacgctaagaaccgtaccgcccgctggatcagtaccagc
atcccagaatccgcttggaacacaccggtggccaagcaatcctcggccgcctaccacgtgaacaatctgctgtcgccagtgctcttccac
gaggcccttcagcacgttcccaagaacgccatttccgtggagattgctccccacggcctgctccaggctatcctcaagcgcgccctgggtc
ctgatgccaccaatctgagcttggtgaagcgcggacacgagaacaacgttgagttcttcctcaccaatgtgggcaaactctttgccgccgg
agcccagcctcaggtacttactctagtgcgtcctatcagctacccggtgggtcgtggaacccccatgttgaactccaaggtcggatgggac
cacacccagaagtggctggtggccaagttcggcaaggaaacttcttctggtgagaccatcgtggaagtcgatctctccaaggaggatgat
gctttcctggctggacacacgatcgacggacgtatcctcttcccggccactggctacatgactctggcttggcagacattcgctaagatgca
gggaagtgagttccacaagaccccggtggtaatggagaacctcgtgttccaccgtgccactattcttaacaagaacgcagtggtcaagttt
ggcattaacttcttcgatggaactggcgcttttgagatttgtgagagcggtagcttggcggtttctggaaaaataaccattccagagtctattga
caacgaggagttgcccctggaggagcaaactcccagcgctgttgccaaggagctaggcaccaacgatgtctacaaggagctgcgcct
ccgaggctacgactatggtggaattttccgcggcattgttcgttctgacaccgtggcctccactggaaagcttcagtgggtggacaactgga
ttagtttcatggacaccatgttgcagttcagtatccttagcaagaaccttcgtgagctgtacctgcccactcgcatcgagcgagcggtgatta
atcccgccaagcactttgaattgctgagcgccctcaccaaggaggaacaggttgagactggactacctgtgcagtggtacagcgacatt
aacgtgatcaagagtgcgggcgtggagctgcgcggtctaaaggccaatctcgcccagcgtcgtcctggcacacaggcacctcccactct
ggagcgctaccagtttgtgcccaacatcaacaccacggatctaaatgaaaactctgagaaggctcgcctgcacgccttggatgtggccat
ccaggtgatcatcgagaactccagcggagccgtcaagctaaagggcgtggaattggccaatggacgcaaccccgacgttttggttgcc
aaccgcctgctgcagattatcgagggtgagcccgtgcttactggtgatgtggctgtggtcacctcgaacaacaatgaagagaccataacc
gccgccctaggagattcaggagttcgagtggtgtccaaggacgtgctgaaggaaccggtagagcagaactgtcacttcgtctttggcatc

gatgtcctttcccgaccggacaccaagaccctggagaactccattgccagcattcgcgagaacggtttcctgatccttgaggagacgctg
cccacttacaccaagacaggccgtgccctgctgaccaaatttggattcgttgccgttcaggaacagagtctgggagccacccgtgtcctg
gtcctcgcccgcaaggctgttgatctgaaaactcgcaagtccgttgtggttgtggccaccgagcagaactttaactgggtggatgacttgaa
ggccgctctggctactgctgctacggaggagcagtatgtgtacgtggtttgccagggagaggagctgtttggagctgtgggtctgatgacgt
gcatcaagaacgagaatggcggcaaactggctcgcctggtcttcgtgcaagatgccaaggctgagaagttctcccttacatcgaccctgt
accgccagcagctggagaaggacctcatctcgaacgtgctgaagaacggcgcttggggcactttccgtcacctgaaactggagaccca
gcaggccactctccaagtggagcatgcctatgtaaacgctctggtcaagggtgatcttgcttccctcaagtggatcgaggctgcccaggcg
gataccgctgcaactgtcgacaagaacctggaaacttgcaccgtctactatgcgcccatcaacttccgtgacgttatgttgacctctggtaa
gctggccgcggatgccttgcctggagatctggctgagcaagattgtgtgctgggtctggagttcgctggtcgcgatacccagggtcgtcgtg
taatggccatggtgccggctaaatcacttgctaccacctgcgtggcaagcaagcgcatgatgtggcaaatccctgagaagtggaccatg
gaagaggcttcgaccgttccttgcgtgtactccaccgtctactacgctctggttgtgcgaggacaaatgaagaagggtgagaagatcctca
ttcacgctggctccggaggagttggtcaggcggccatctctgtggctctggcccatggcttgaccgtgttcaccacggtgggcagcaagga
aaagcgcgagttcctgctgaagcgattccccaaactgcaggagcgtaacatcggcaactcccgagacacctccttcgagcagttggtcc
tgcgcgagaccaagggacgcggagtcgatctggtgctgaactcgttatctgaagagaaactgcaggcctcgatccgctgtttgggtctga
atggacgcttcctggagattggcaagttcgatttgagcaacaacagcccacttggaatgtccgttttcctcaagaacacctccttccacggc
attttgcttgacagtgtgatggagggcgaggaggagatgcaaaaccaggttgtctccctggtcgctgagggcatcaagaccggagccgtt
gtaccccttccgacctcggtgtttaacgaccagcaggtggagcaggccttccgtttcatggcctctggcaagcacatcggcaaggtagtga
tcaaggtgcgtgatgaggaggcaggcaagaaggcgctgcagcccaagccgcgcctgatcaacgccattccacgcacatacatgcac
ccggagaagagttacatcctggttggaggtcttggcggattcggtttggagctgaccaactggttggtgacgcgtggagcccgctacattgt
gctgacctctcgttccggagttaagaccggctaccagggtctgatgatccgtcgctggcaggaacgtggcgtaaaggtggtgattgacac
cagtgatgtgaccaccgccgctggagctaaaaagctgctggagaatagtaacaagctggctttggtcggaggcatcttcaacctggcgg
ctgttctccgcgatgctctgatcgaggatcagaccgccaaggacttcaagacagtggctgatcccaaggtgacggccaccaagtacctg
gatcagttctctcgcgatatctgcactgagctggactacttcatctgcttctccagtgtttcctgcggtcgtggtaacattggtcagaccaactac
ggattggccaactctgcaatggagcgaatttgcgagcagcgccaggtgagcggattcccaggcaccgccatccaatggggagccatcg
gagacaccggtctggtgctggagaacttgggtgacaatgacaccgtgattggaggaaccctgccccagaggatgccctcttgcctgcag
accatcgacctgttcctgcaacagccccatccggttgttgcctccatggttgtggccgagaagcgcaagtcggatcagtcggctggagtca
gcctaattgccaccattgccaacatccttggtctccgggacaccaagaatatccaggacggagcatcgcttgctgatctcggaatggactc
cctgatgagcgctgagatcaaacagactttggagcgaaactttgacattgttctgtccgcccaggagatccgtcagctcacctttggagccc
tcaaggccatggatggtggtgctgatgtcaagccagccgcagctgctcccgcagccgctgctggagtcccagaggcaaacattacctct
ggtggatcctcgcgcacagctagtcccatgggcgatggaacgcaggtggtgttcaccacgtccctcatccccaccgaggcaattgtaca
actggacacaaaggcacctgcaaacagcaaacagagccccatcttcttcatttccccgatcgaggggttcgcctccgcactggagccgc
tggccaagcgattggaggtgcccgcctatggactgcagtacacggaagccgtgccctccgattctctggaatcagcagccaaattcttcat
caagcagctgcgcaccgtccagccaaagggtccctacaaactagccggatactccttcggttgcctgctcacctacgtgatggccggtatt
ctggaagagaccaacgaggtggccaatgtgatcatgctggacggagccccaagctacgtcaactggtacacgagcagcttcaagcag
cgttacactgacggcaccaacgccgataacgacaaccagagctacggcttggcctactttggaatcgtgttggccaacattgactacaaa
gcgctggtgcgtctgctaatagtgatccccacatgggaggagaaactcgagaggttcgctgagctgatgagcaatgagatcacccagcc
cgtggaaacgatcaagaaatccgccactctgttctacaagaaactggagctggccgatggctaccagcccactctgaagctcaagacg
aatgtgaccttggtcaagcccacagacaactctgccaagctggacgaggactatcgccttaaggaggtctgcacaaagcccgtggaag
tacacactgttgagggcaaccaccgcaccttcctcatcgaggatcagtccctgaagaccatccagagcatactgaagcgtctgttcaact
aagctagaaagaggattagaataagagttgtgaatgtggcataggcccactacccacacgatcatcaccctctcaccgtcactcaccca
atactcgggttctatgcaccatgtggaaccacggaacacatcgacacctagttagcatacttagtgaatgaatttacgttgttgagctgcaag
gaatggctcgccccatcccagttacgcacagctagaagagataagttaacagattggatttgcgtcgatcggattgaatctgcctaactgct
gtcttgcagggctgtaggcttcggtccaaaattaagtctacagtctctgcgattccgggcagttgaaatgatgcaagatgagaggctcagtc
cataacacttaacgctttgtgcaccgtacaatttagtacccgacattccttgagagctagacggcaccagacgtcccaacttaccaaatata
tcttttatgctctatctataatgtatgtcgcatattctagttttattgcctagttgtaagcctacagtttgtagtcgcgcatgtaatgaatttcattcctcg
aactaacccaagaaagagactacaacattcgttatctctcttgatgtataaaataatcgcccaaaaattatcactcatgaatattgtggatatt
aaaattacaccatactaaaaaat (SEQ ID NO:171)

**Protein:** AAF51148
**Protein GI:** 7295848

MPARFAEEVITAEPAQRAAPQLDLGGGHYVPRQQHLNDEIAITGFSGRLPESSTIEEFKQ
NLFDGVDMVNDDPRRWERGLYGLPDRIGKLKDSDLENFDQQFFGVHQKQAECMDPLLRMLLEL
THEAIIDAGLNPSDLRGSRTGVYIGVSNSETEQHWCSDADRVNGYGLTGCARAMFANRISFTFDF
KGPSYSIDTACSSSLYALEQAFSDMREGKVDNALVAGAGLILKPTMSLQFKRLNMLSPDGSCKAF
DESGNGYVRSDGCVVLLLQRTSAARRVYASILNVRTNTDGFKEQGITYPIGKMQNRLIRETYEEIG
LNPADVVYVEAHGTGTKVGDPQEVNSITDFFCKDRTTPLLIGSVKSNMGHSEPASGVCSVAKILIA
MEEGVIPGNLHYNKPNPDLYGLVDGRLKVVDRNLPWNGGIIGLNSFGFGGANAHVILKSNPKPKA

LTPKDGALKVVLASGRTFEAVEQLLESASTNADDDEYLQLINEIHSKAIPNHFFRGYGVVSSKGTH
QREVIESNDDKRPIWYIYSGMGSQWASMAKDLMKIEAFAKTIQRCADVLKPEGVDLIDVLTRSTDK
SFENILNSFISIAAMQVALTDLLSSLGIHPDGIVGHSVGELGCAYADGCFTPEQTVLAAYWRGKSIL
DTQLAKGKMAAVGLSWEDAHSRVPSDCFPVCHNSEDNCTISGPEASIEALVAKLNAEGVFAKAVN
SSGYAFHSKYIAEAGPKLRKSLEKIIPNAKNRTARWISTSIPESAWNTPVAKQSSAAYHVNNLLSPV
LFHEALQHVPKNAISVEIAPHGLLQAILKRALGPDATNLSLVKRGHENNVEFFLTNVGKLFAAGAQP
QVLTLVRPISYPVGRGTPMLNSKVGWDHTQKWLVAKFGKETSSGETIVEVDLSKEDDAFLAGHTI
DGRILFPATGYMTLAWQTFAKMQGSEFHKTPVVMENLVFHRATILNKNAVVKFGINFFDGTGAFEI
CESGSLAVSGKITIPESIDNEELPLEEQTPSAVAKELGTNDVYKELRLRGYDYGGIFRGIVRSDTVA
STGKLQWVDNWISFMDTMLQFSILSKNLRELYLPTRIERAVINPAKHFELLSALTKEEQVETGLPVQ
WYSDINVIKSAGVELRGLKANLAQRRPGTQAPPTLERYQFVPNINTTDLNENSEKARLHALDVAIQ
VIIENSSGAVKLKGVELANGRNPDVLVANRLLQIIEGEPVLTGDVAVVTSNNNEETITAALGDSGVR
VVSKDVLKEPVEQNCHFVFGIDVLSRPDTKTLENSIASIRENGFLILEETLPTYTKTGRALLTKFGFV
AVQEQSLGATRVLVLARKAVDLKTRKSVVVVATEQNFNWVDDLKAALATAATEEQYVYVVCQGE
ELFGAVGLMTCIKNENGGKLARLVFVQDAKAEKFSLTSTLYRQQLEKDLISNVLKNGAWGTFRHL
KLETQQATLQVEHAYVNALVKGDLASLKWIEAAQADTAATVDKNLETCTVYYAPINFRDVMLTSGK
LAADALPGDLAEQDCVLGLEFAGRDTQGRRVMAMVPAKSLATTCVASKRMMWQIPEKWTMEEA
STVPCVYSTVYYALVVRGQMKKGEKILIHAGSGGVGQAAISVALAHGLTVFTTVGSKEKREFLLKR
FPKLQERNIGNSRDTSFEQLVLRETKGRGVDLVLNSLSEEKLQASIRCLGLNGRFLEIGKFDLSNN
SPLGMSVFLKNTSFHGILLDSVMEGEEEMQNQVVSLVAEGIKTGAVVPLPTSVFNDQQVEQAFRF
MASGKHIGKVVIKVRDEEAGKKALQPKPRLINAIPRTYMHPEKSYILVGGLGGFGLELTNWLVTRG
ARYIVLTSRSGVKTGYQGLMIRRWQERGVKVVIDTSDVTTAAGAKKLLENSNKLALVGGIFNLAAV
LRDALIEDQTAKDFKTVADPKVTATKYLDQFSRDICTELDYFICFSSVSCGRGNIGQTNYGLANSA
MERICEQRQVSGFPGTAIQWGAIGDTGLVLENLGDNDTVIGGTLPQRMPSCLQTIDLFLQQPHPV
VASMVVAEKRKSDQSAGVSLIATIANILGLRDTKNIQDGASLADLGMDSLMSAEIKQTLERNFDIVL
SAQEIRQLTFGALKAMDGGADVKPAAAAPAAAAGVPEANITSGGSSRTASPMGDGTQVVFTTSLI
PTEAIVQLDTKAPANSKQSPIFFISPIEGFASALEPLAKRLEVPAYGLQYTEAVPSDSLESAAKFFIK
QLRTVQPKGPYKLAGYSFGCLLTYVMAGILEETNEVANVIMLDGAPSYVNWYTSSFKQRYTDGTN
ADNDNQSYGLAYFGIVLANIDYKALVRLLIVIPTWEEKLERFAELMSNEITQPVETIKKSATLFYKKLE
LADGYQPTLKLKTNVTLVKPTDNSAKLDEDYRLKEVCTKPVEVHTVEGNHRTFLIEDQSLKTIQSIL
KRLFN (SEQ ID NO:172)

## Figure 76

**Name:** CG16752
**Nucleotide:** AE003434
**GI:** 10728478
**Transcript:** CT36050
**Sequence:**

atgccgcgtgtgaggcggtgcacggcgtatattgcattgctggcgtttctgcaccaactgcccagattcttcgacagga
cgtacatgccgctggtgatcgagtggaacggcagcccaacggaggtgtgccacttggagacgtcgatgtgggtgcacgattacattgga
gtggacctatactacacaagctactatctgttccgggtgctgttcgtccacctgctgccctgcattatcctggttaccctgaacattctgctgttcg
cggcgatgcggcaggcacaggagcgccgaaagctcctcttccgggagaaccggaagaaggagtgcaagaaactgagggagacca
attgcaccacgctgatgctaattgtggtcgtgtcggtgttccttctagccgagatacccattgctgtggtcactgcgatgcacatcgtcagtagc
ctgattatcgagttcctggactatggactggccaacatttgcatcatgctgacgaacttcttcctggtattcagctatccgatcaacttcggcattt
actgcggcatgtcgcgccagtttcgggagactttcaaggagatattcctcggtcggctgatggccaaaaaggatagctccacaaaatact
cgatcgttaatggcgcccgcacctgcaccaacaccaacgagacggtcctctagtaattggtgatgttggtgccgcggcggggcagcagc
gaccatcgtcgcagctccactagcaccaccaccaccaccaccaccaagaccattggtgggtcaatgatcatcggtggtgaggcctcgg
cgcagcaccagcatctggtaacgcaccatctgcagacccactcacagcccagtcagcagcggcgggtcagcaccatggacatcatca
ccgaggagcggattttgtaaactggatgcgagaatcggatgggttcggaatcgattgggtgcggctatcggaaatcagagatcccaatat
actgttgaaaactatcggctgcctgccgaggagcgcaatttacaatacagttctttattcgtcaatggtctcagcatgaaattttaacagaattt
gcaattgcaactaatccagcaaaaatcaaaagaactccactacacaaatggtttttttcgaacagagctaagaagtaaggtttcaggag
aaaactacaatacataggtacacaggaacttgtttaattaaaaacactaatatatcaatatcaaaataaccaaaaatgcgtagagtatttg
gaatgatgcagtctgaattttgatggattttgcaaatgagcgattttcacttttaaataacttaaggcagttta (SEQ ID NO:173)

**Protein:** AAF46037
**Protein GI:** 7290587

MDNYTDVLYQYRLAPSASPEMEMELADPRQMVRGFHLPTNESQLEIPDYGNESLDYPNY
QQMVGGPCRMEDNNISYWNLTCDSPLEYAMPLYGYCMPFLLIITIISNSLIVLVLSKKSMATPTNFV
LMGMAICDMLTVIFPAPGLWYMYTFGNHYKPLHPVSMCLAYSIFNEIMPAMCHTISVWLTLALAVQ
RYIYVCHAPMARTWCTMPRVRRCTAYIALLAFLHQLPRFFDRTYMPLVIEWNGSPTEVCHLETSM
WHDYIGVDLYYTSYYLFRVLFVHLLPCIILVTLNILLFAAMRQAQERRKLLFRENRKKECKKLRET
NCTTLMLIVVVSVFLLAEIPIAVVTAMHIVSSLIIEFLDYGLANICIMLTNFFLVFSYPINFGIYCGMSRQ
FRETFKEIFLGRLMAKKDSSTKYSIVNGARTCTNTNETVL (SEQ ID NO:174)

## Figure 77

**Name:** Rpt1
**Nucleotide:** AE003840
**GI:** 10727757
**Transcript:** CT3016
**Sequence:**

aaattgttcagtcattctcgccgtaaaacaaaaggaaaacatcgcataaactcattttttgccttaaaaaccgtacaatt
gcaatcgaataagatgccggactacctgggcgacgaccagcgcaaggtgaagcacgatgagaaggaggacaaggagatcaagtc
cctcgacgaaggcgacattgagcttctaaagacttatgggcagagccagtatcacaaatccatcaagagcatcgaggaggacattcaa
aaggctgtgaagcaggtgaacgagctgactggaatcaaggaaagcgacacgggtctggcgccaccagcgctctgggatttggccgcc
gacaagcagatcctgcaaaacgagcaaccgctgcaggttgcccgatgcaccaagatcatcaacgcggattccgacgaccccaagtat
atcatcaatgttaagcagttcgccaagttcgtggtggacctagctgactcggtggcccctactgacatagaagagggaatgcgtgtgggtg
tcgaccgcaacaagtaccagatccacattccgctgcctcccaagattgatcctacggttaccatgatgcaggttgaggacaagccggac
gtcacctacagcgatgtgggcggctgcaaggaacaaatcgaaaagctgcgcgaggtggtggagacgccactgctgcacccggaaaa
gttcgtcaatctgggtattgagccgcccaaaggtgtgctactgtttggcccaccgggaacgggaaagaccctgtgtgcccgcgccgttgcc
aaccgcacggacgcttgctttatccgcgtcattggctctgagctggtgcaaaagtacgtgggtgagggcgctcgtatggtgcgcgagctctt
cgaaatggcgcgttccaaaaaggcttgtctcatcttcttcgacgaaattgatgctatcggtggtgcccgattcgacgatggggccggcggc
gacaacgaggtgcagcgcaccatgttggagctgattaaccaactggatggcttcgatccgcgcggaaatatcaaggtgctgatggccac
caaccgacccgacacgttggatccggcgctcatgcgtcccggtcgtctggaccgaaaggtggagttcggcctgcctgaccaggacggtc
gctcgcacatctttaagattcacgcccgctccatgtccgtggagcgagatattcgcttcgatctgctggcgcgtctatgtcccaactcgactgg
tgctgagatccggtcagtatgcacggaagcgggcatgttcgccattcgggcgcgtcgcaaggtggccacagagaaggatttcctcgaag
ccgtcaagaaggttatcaagagctacgccaagttcagcgccactccacgctacatgacctacaactaa (SEQ ID NO:175)

**Protein:** AAF59219

**Protein GI:** 7304183
MPDYLGDDQRKVKHDEKEDKEIKSLDEGDIELLKTYGQSQYHKSIKSIEEDIQKAVKQVNE
LTGIKESDTGLAPPALWDLAADKQILQNEQPLQVARCTKIINADSDDPKYIINVKQFAKFVVDLADS
VAPTDIEEGMRVGVDRNKYQIHIPLPPKIDPTVTMMQVEDKPDVTYSDVGGCKEQIEKLREVVETP
LLHPEKFVNLGIEPPKGVLLFGPPGTGKTLCARAVANRTDACFIRVIGSELVQKYVGEGARMVREL
FEMARSKKACLIFFDEIDAIGGARFDDGAGGDNEVQRTMLELINQLDGFDPRGNIKVLMATNRPDT
LDPALMRPGRLDRKVEFGLPDQDGRSHIFKIHARSMSVERDIRFDLLARLCPNSTGAEIRSVCTEA
GMFAIRARRKVATEKDFLEAVKKVIKSYAKFSATPRYMTYN (SEQ ID NO:176)

# Figure 78

**Name:** wts
**Nucleotide:** AE003775
**GI:** 7301969
**Transcript:** CT4806
**Sequence:**

atgcatccagcgggcgaaaaaaggggcggtcgccccaatgataaatacacggcggaagccctcgagagcatca
agcaggacctaacccgatttgaagtacaaaataaccataggaataatcagaattacacacctctgcgatacacggcgaccaacggac
gcaacgatgcacttactcctgactatcaccacgccaagcagccgatggagccgccaccctccgcctctcctgctccggacgtggtcatac
cgccgccgcccgccattgtaggtcagcccggagccggctccatatccgtatccggtgtgggcgttggagtggtgggtgtggcgaacgga
cgtgtgccaaagatgatgacggccctaatgccaaacaaactgatccggaagccgagcatcgaacgggacacggcgagcagtcacta
cctgcgctgcagtccggctctggactccggagccggtagctcccgatcggacagcccccattcgcaccacacccaccagccgagctcg
aggacggtgggtaatccaggtggaaatggtggattttctccgtcgccaagcggtttcagtgaggtggctccaccggcgccgccgccacgc
aatcccaccgcctgcagcgcggccacgccccccaccgccagtgccgccccactagccaggcgtacgtgaagcggcgatcaccggccct
gaacaaccgcccgccggcgatagcgccacccactcagcggggcaactcacctgtaataacccagaacgggctgaagaacccgcag
cagcagttgacgcagcagctgaagtccctgaacctatacccaggcggaggcagtggagcagttgtggagccaccgccgccctaccta
attcaaggcggagccggaggagcagcaccgccgccgccaccacccagttacacggcctccatgcagtcgcggcagtcgcccacaca
atcccaacaatcggactacaggaaatccccgagcagtgggatatactcggccacctcggcgggctcgccgagccccataactgtgtcc
ctgccgccggcgccgctggcgaagccacaaccacgagtctaccaggccaggagtcagcagccgatcatcatgcagagtgtgaagag
cacgcaggtccaaaagcccgtgctgcaaacagcagtggcgccccaatcgccatcgagtgcctcggccagcaattcaccagtccacgt
gctggccgctccaccctcttaccctcagaagtccgcggcagtggtgcagcagcagcaacaggcagcagcggcggcccaccagcagc
agcatcagcaccagcaatccaaaccaccaacgcccaccacaccgcccttggtgggtctgaatagcaagcccaattgcctggagccac
cgtcctatgccaagagcatgcaggccaaggcggccacggtggtgcagcagcagcaacagcagcagcagcagcaacaacaggtcc
agcagcagcaggtgcaacagcagcagcagcagcagcaacagcaactgcaggccttgagggtgctccaggcacaggctcagaggg
agcgggatcaacgggagcgggatcaacgggagcgggaacgggatcagcagaagctggccaacggaaatcctggccggcagatgc
ttccgccgccgccctatcagagcaacaacaacaacaacagcgagatcaaaccgccgagctgcaacaacaacaacatacagataag
caacagcaacctggcgacgacaccacccattccgcctgccaaatacaataacaactcctccaacacgggcgcgaatagctcgggcg
gcagcaacggatccaccggcaccaccgcctcctcgtcgaccagctgcaagaagatcaagcacgcctcgcccatcccggagcgcaag
aagatctccaaggagaaggaggaggagcgcaaggagttccgcatcaggcagtactcgccgcaagccttcaagttcttcatggagcag
cacatagagaacgtgatcaagtcgtatcgccagcgcacgtatcgcaagaatcagctggagaaggagatgcacaaagtgggactgccc
gatcagacccaaatcgagatgaggaaaatgctgaaccaaaaggagagcaactacattcgattgaagcgcgccaagatggacaaga
gcatgttcgtcaaactgaagcccattggagtgggtgcatttggcgaggtaacgctggtgagcaaaatcgatacctcgaaccatttgtatgc
gatgaaaaccctgcggaaagcggacgttctcaagcggaatcaggtggcacacgtgaaggccgagagggatatcctcgcggaagccg
acaataactgggtggtgaagttgtactacagcttccaggacaaggataatctgtactttgtgatggactacataccaggtggtgatctgatgt
cgctgctcatcaaactgggcatttcgaggaggaactggccagattctacatcgccgaggtcacctgcgccgtggacagcgttcacaaaa
tgggcttcattcacagagacatcaagcctgacaacatactcatcgatagggacggacacataaagctcaccgactttggcctgtgcacgg
gattccgatggacgcacaactcgaagtactaccaggagaacggcaatcactcgcgccaggactcgatggagccctgggaggaatact
ccgagaacggaccgaagcccaccgtgctggagaggcgacggatgcgcgatcaccaaagagtcctggcccactcgctggtgggcac
cccgaactacatagctcccgaggtgctggagagaagtgggtacacgcagctgtgcgactactggagcgtgggcgtcatcctctatgaga
tgctggtgggtcagccgcccttctggccaacagtccgctggaaacgcaacaaaaggtcatcaactgggagaaaaccctgcatattccg
ccgcaggccgagttatcccgcgaggctacggacttgataaggaggctctgtgcgtcggctgacaagcggctgggcaagagcgtggac
gaggtcaagagccacgacttcttcaagggcatcgactttgcggacatgcggaagcagaaggcgccctatataccggaaatcaagcac
ccgacggacacatccaactttgatcccgtggatccggagaagctgcgctcgaatgactccaccatgagcagcggcgatgatgtcgacc
agaacgaccgcaccttccacggctttttcgaatttaccttccgtcgcttcttcgacgacaagcagccgccggatatgacggacgatcaggc
gccggtttacgtctgaa (SEQ ID NO:177)

**Protein:** AAF57085
**Protein GI:** 7301980

MHPAGEKRGGRPNDKYTAEALESIKQDLTRFEVQNNHRNNQNYTPLRYTATNGRNDAL
TPDYHHAKQPMEPPPSASPAPDVVIPPPPAIVGQPGAGSISVSGVGVGVVGVANGRVPKMMTAL
MPNKLIRKPSIERDTASSHYLRCSPALDSGAGSSRSDSPHSHHTHQPSSRTVGNPGGNGGFSPS
PSGFSEVAPPAPPPRNPTACSAATPPPPVPPTSQAYVKRRSPALNNRPPAIAPPTQRGNSPVITQ
NGLKNPQQQLTQQLKSLNLYPGGGSGAVVEPPPPYLIQGGAGGAAPPPPPPSYTASMQSRQSP
TQSQQSDYRKSPSSGIYSATSAGSPSPITVSLPPAPLAKPQPRVYQARSQQPIIMQSVKSTQVQK
PVLQTAVAPQSPSSASASNSPVHVLAAPPSYPQKSAAVVQQQQAAAAAHQQQHQHQQSKPPT

PTTPPLVGLNSKPNCLEPPSYAKSMQAKAATVVQQQQQQQQQQQQVQQQQVQQQQQQQQQ
LQALRVLQAQAQRERDQRERDQRERERDQQKLANGNPGRQMLPPPPYQSNNNNNSEIKPPSC
NNNNIQISNSNLATTPPIPPAKYNNNSSNTGANSSGGSNGSTGTTASSSTSCKKIKHASPIPERKKI
SKEKEEERKEFRIRQYSPQAFKFFMEQHIENVIKSYRQRTYRKNQLEKEMHKVGLPDQTQIEMRK
MLNQKESNYIRLKRAKMDKSMFVKLKPIGVGAFGEVTLVSKIDTSNHLYAMKTLRKADVLKRNQVA
HVKAERDILAEADNNWVVKLYYSFQDKDNLYFVMDYIPGGDLMSLLIKLGIFEEELARFYIAEVTCA
VDSVHKMGFIHRDIKPDNILIDRDGHIKLTDFGLCTGFRWTHNSKYYQENGNHSRQDSMEPWEEY
SENGPKPTVLERRRMRDHQRVLAHSLVGTPNYIAPEVLERSGYTQLCDYWSVGVILYEMLVGQP
PFLANSPLETQQKVINWEKTLHIPPQAELSREATDLIRRLCASADKRLGKSVDEVKSHDFFKGIDFA
DMRKQKAPYIPEIKHPTDTSNFDPVDPEKLRSNDSTMSSGDDVDQNDRTFHGFFEFTFRRFFDDK
QPPDMTDDQAPVYV (SEQ ID NO:178)

# Figure 79

**Name:** CG1582
**Nucleotide:** AE003484
**GI:** 7292554
**Transcript:** CT4155
**Sequence:**

atggacgaatcctcgagaaagcacatggaggactgcttcctacgcagtcccagcgacatccgtaccaccaacgtgc
ccgcggcgcctaaatcgcaggcggctgacaacaagtctcggaaaacggagctccacgtccttcggctcaacgatgaatcccgccagat
gaccatggacacgctgcgccaaatccacggcccggaattccagctggacgacatcagcaagtacaaggatcgtggacgcggaggtc
atggcgtgaagcactcctactggcaggatcgcggcaccctggtggtgcagagtgtccagggcgtgagctcaacgcgtggcaacgaca
gtgatggtgatcgcctgcgccgatatgccttggccaaactggagaactacggatttcaggctgtccactgccttgaagcttatgagcactgc
tccggcgacacggaagccgctctactgctgctctaccgtcgctatatgagaattcccgatgaggagcaactgactctcgagccacccagc
gagcaagaaatactggacatgcgcgccgacgagaaggaggccctggagtccatcttcgataaggcgtacgaggagcgcgaggcga
atcgcgtgtggaacctaaagtttcgcatcgaccatctgctggcccacagtccgtcggaggtgcgaaaggccagggaggcggtcctggcc
gccgctgctgctgctgcccaggcggcgctcgacaagaagaaaaagcctcctctacgttgtcgcaatttcgatcgtgacggcacctgtaag
tacggtcccaagtgccggttcgctcacctgccgcaacaacccactgaatcggacacgaccaaaaaggatagcgttgacgagaacgat
aacgagctgtggtttcacgtggaagtgcgctttccgccaggcagtcgctatccatatgaggcgcctttcatctacctgaaaaccacttgcca
cgacataccgcacgaactgcgcctccggtatgcccgccatctctacaaggaggccaaagagatatgccgggatggcattccctgtgtat
acagcatatgcgacctgctgcagtccaatgaacagctggccggacgactagacacgtctgcttttccctcgcccaagcgatcactcttcta
cgatgagccggcgggcggtggagtgaatccggatgccgagcatcatcaatcaccgaaaccatcgcactacgcacgtggtcaaacgtc
gcgaaatgatggaggccaccagcgaaacgtggaagcgcagacgagagagaaccgtcgccttttgcaacagttcgtggagcgacgaa
aggaagaacgctaccagaagatcattgatggacgaaaacagctgcccgccttcgccgaaattgaacgtattctggcgctaatagaaagt
tcgccggtagtggtaatatctggtgaaacgggttgcggcaagagtacccaagtgccgcagttcattttggacaattggttcttccgcgcactt
caactgccagcaaaggagaacttgccccacgtggaaatcatctgcacgcaaccgcgacgactctcagccatcggagtggcggagcg
agtggctgccgagcgtctggatcgcattggtcagctggtgggctaccagatccgactggaaaacaaggtgtcgcaaagcacgcgactc
agtttttgcaccacgggcatcttgttgcggcgactggcctcggatccgctactaggaagcgttacccacgtcatagtggacgaagtacacg
agcgatctgaggagtccgatttcctactgctaattctcaagaatctgctgcgagagcgcaaggatctaaaagttattctcatgtcggccacg
ctcaatgccgccctcttttcggattactttggcggagctcccgtgctagatattcctggtcgaacgtttccagtacagcagctctttttggaggat
atcctggagatgagcgactttgtcatggaatacgacactaagtactgccgcaagcttaaaaagcaggagcaggaaatactggagcgtg
agctggagtacgctgatgtgcaggcttcgggagaagcgccaggcaaaaagatcaaggatgaaaagctaactctggcggagacttatc
agcgatacgcggaatatagcaagcccacgtgcaagagtatttacctgatggagcccatgactataaatccagaactgatcgaatccgta
ctgaaatacattgttgaaggttctcacgattggccgcgcgaaggaaccatcctcatcttcctgcctggtttcggggagatccaatccgtgcac
gattccttgctggacaatgcactcttttccccacgcgccggcaaatttatcctggtgccattgcactcggccctttctggcgaggatcaggcgc
tagtcttcaaaaaggccccgcccggtaaacgaaagatcgtgttgagcacaaacatagctgagacatcggtgaccatcgacgactgtgtg
ttcgtggtggactgcggcctgatgaaggagaagtgtttcgactcaaaccgcaacatggagtcactggacctagtctgggtgtctcgtgcca
atgcaaagcagcgcaagggtcgtgccggccgtgtgatgccgggtgtgtgtatccacctatacaccagctaccgctatcaataccacattct
ggcccaaccggtgccagagatccagcgagtgccactcgagcaaattgtgctgcgcatcaagacgttgcaaacgttcgcctcacgcaac
acgctctctgttctgctggagacactggaggcgccaacagaggacagtgtactaggtgcattgacacgattgagggatgtgggcgcgctc
gatgcagaggatcaattaaccccgcttggtcatcatttggcagcccttcctgtcgacgtgcgcatcggcaagctgatgctgtatggagcgat
cttccagtgtctggacagcgtgctgaccatcgccgcctgcctgagcaacaagtcgcccttcgtaagtccgctcaacaagcgcacggagg
cggacaagtgcaaacggatgtttgcactgggcaacagcgatcatttgaccgtcctgaatgcgtataggaaatggctggatgttgcgcgaa
ggggaaactatgcagccagcagaaactacgccagtgagcacttcctgtcgctgaacacacttgaaacgatagctgatctcaagtatcaa
tacctggagctactcgtttccattggctttgtgccgatcgatgtgccgcgtaggcgcaaaaatgcctgcgacaatatactgacactaacagg
tgtggagcagaaccacaacggtgataacaacaggctgctgacctcgctcctctgcgccgccctctatccaaacatcgtgaagattatgac
gccggatcgcgtctacatccaaacagctggtggtgctgtgccgcgtgagccgagccaccatgatctgcgcttcaagacgcgcggcgatg
gttacgtgaagattcatccgtcgtcggttaactcgcaggtgtccgtcttccaggcgccgttcctcgtcttccaggagaaggtacgcaccagc
gccatctacatccgcgactgttcgatgttgccgctcatcgccatggtcctgtttgccggcagcgattttaaggtggagctacacgatggggac
ttcctttttctgctggaaagcggttggattatattgaaggcccacgatctagagactgctgagatggtacagtgcctgcgggcggagatgatc
aagctgctcgaggagaagattcgcgatccgtgccttaatctgctgcaccacaaaaacggctgccgtatgatcgccaacatcgttcacttga
ttagtaaaaacagctga (SEQ ID NO:179)

**Protein:** AAF47973
**Protein GI:** 7292573

MDESSRKHMEDCFLRSPSDIRTTNVPAAPKSQAADNKSRKTELHVLRLNDESRQMTMDT
LRQIHGPEFQLDDISKYKDRGRGGHGVKHSYWQDRGTLVVQSVQGVSSTRGNDSDGDRLRRYA

LAKLENYGFQAVHCLEAYEHCSGDTEAALLLLYRRYMRIPDEEQLTLEPPSEQEILDMRADEKEAL
ESIFDKAYEEREANRVWNLKFRIDHLLAHSPSEVRKAREAVLAAAAAAAQAALDKKKKPPLRCRNF
DRDGTCKYGPKCRFAHLPQQPTESDTTKKDSVDENDNELWFHVEVRFPPGSRYPYEAPFIYLKT
TCHDIPHELRLRYARHLYKEAKEICRDGIPCVYSICDLLQSNEQLAGRLDTSAFPSPKRSLFYDEPA
GGGVNPDAEHHQSPKPSHYARGQTSRNDGGHQRNVEAQTRENRRLLQQFVERRKEERYQKIID
GRKQLPAFAEIERILALIESSPVVVISGETGCGKSTQVPQFILDNWFFRALQLPAKENLPHVEIICTQ
PRRLSAIGVAERVAAERLDRIGQLVGYQIRLENKVSQSTRLSFCTTGILLRRLASDPLLGSVTHVIV
DEVHERSEESDFLLLILKNLLRERKDLKVILMSATLNAALFSDYFGGAPVLDIPGRTFPVQQLFLEDI
LEMSDFVMEYDTKYCRKLKKQEQEILERELEYADVQASGEAPGKKIKDEKLTLAETYQRYAEYSK
PTCKSIYLMEPMTINPELIESVLKYIVEGSHDWPREGTILIFLPGFGEIQSVHDSLLDNALFSPRAGK
FILVPLHSALSGEDQALVFKKAPPGKRKIVLSTNIAETSVTIDDCVFVVDCGLMKEKCFDSNRNMES
LDLVWVSRANAKQRKGRAGRVMPGVCIHLYTSYRYQYHILAQPVPEIQRVPLEQIVLRIKTLQTFA
SRNTLSVLLETLEAPTEDSVLGALTRLRDVGALDAEDQLTPLGHHLAALPVDVRIGKLMLYGAIFQC
LDSVLTIAACLSNKSPFVSPLNKRTEADKCKRMFALGNSDHLTVLNAYRKWLDVARRGNYAASRN
YASEHFLSLNTLETIADLKYQYLELLVSIGFVPIDVPRRRKNACDNILTLTGVEQNHNGDNNRLLTSL
LCAALYPNIVKIMTPDRVYIQTAGGAVPREPSHHDLRFKTRGDGYVKIHPSSVNSQVSVFQAPFLV
FQEKVRTSAIYIRDCSMLPLIAMVLFAGSDFKVELHDGDFLFLLESGWIILKAHDLETAEMVQCLRA
EMIKLLEEKIRDPCLNLLHHKNGCRMIANIVHLISKNS (SEQ ID NO:180)

# Figure 80

**Name:** CG12289
**Nucleotide:** AE003544
**GI:** 10727982
**Transcript:** CT19318
**Sequence:**

tcgatttaaattaagggcagagaatcgaagaaagaagtagaggaggatctggatacgaaatcagagctttagaca
atcgccaggatgacgcaatggtgtgctatcagtgtaatgaaggccgtgctctgcgtcggctgcacggaggtggactacgtgaccaccatg
gataaggtcgacttccagtgcgacaggtgcagcacccagaatggaatgcttcagcgcagcggaagatcctcgaatgttagtacggccct
gcgtctgttgggcgccaatgtggagttgttcggtgtgctcacccgtaatcccacctatcgcatgattctggatgatctcgagcagcggggcat
tgatattagccattgtacattcacggacgcaagtcctcccttttccacaattgtccaagatcgctggacgcgtcgttgcacggtggtgtattgcg
acaagtcatttccatatgttacggcagcggatttccgcaaattggatctcgatcaatacggctgggtgaattttgaggcacggagtccacgtg
aaacttgcgatatgatacgcctaatcctcgatcacaacaacgggcgggatgaacgggatcggatagtggtatcgttgcagattttcaattcc
ttcgaacaggtcgtgcatctgattgccatgtgcgactatgtgttcgtcaccaagtatatagccgagtccagaggctggaagtcaccgctgga
agcctgcgagggtgtcaatgcgctactgcgcatgccaaggaacattagaattcgcagaccctgcatcattgtgccgtggagcagcctgg
gagcaggatgtatgaccacggaaggggagtacttcgaagtgccagcacacaagccgaaaaagatcatcgatcgggtgggcgatagt
gattgcttcacggcgggcttcatttatgccgcttttgtgcggcaaaggcgattggccgaagccgtggacttcgccaatgctgtggccagctat
aagttgggctatgtgggtttcgattgcctgggtcatttgccaatcgatgcggttaagctgcccgttagaatgaggtgtggcagtgaggtgtcct
ccgacgatgaggataacggcgatcagttgaatagctgcaagaagcacctgttacgcccgattgagttcctgccggaaaagtcctccattg
cacccactgagaaggaagttccgatcgagacttcgcaactttctagctag (SEQ ID NO:181)

**Protein:** AAF50065
**Protein GI:** 7294728

MKAVLCVGCTEVDYVTTMDKVDFQCDRCSTQNGMLQRSGRSSNVSTALRLLGANVELF
GVLTRNPTYRMILDDLEQRGIDISHCTFTDASPPFSTIVQDRWTRRCTVVYCDKSFPYVTAADFRK
LDLDQYGWVNFEARSPRETCDMIRLILDHNNGRDERDRIVVSLQIFNSFEQVVHLIAMCDYVFVTK
YIAESRGWKSPLEACEGVNALLRMPRNIRIRRPCIIVPWSSLGAGCMTTEGEYFEVPAHKPKKIIDR
VGDSDCFTAGFIYAAFVRQRRLAEAVDFANAVASYKLGYVGFDCLGHLPIDAVKLPVRMRCGSEV
SSDDEDNGDQLNSCKKHLLRPIEFLPEKSSIAPTEKEVPIETSQLSS (SEQ ID NO:182)

# Figure 81

**Name:** Pepck
**Nucleotide:** AE003799
**GI:** 10727469
**Transcript:** CT30577
**Sequence:**

ttactcgtggccagagtaaatggaaagatcaagtgaaaagcctactttcagtggtctgaaaatcaagttgatgtttgac
aaaaaactgtggaagaacatatgaacgcaaagtcctcgacagtagaacactaattgtgataattactcaaattgtgataagcgaattaag
caaagtgccaaaaaccctttcacgcgcagaaaaatctaataaaaatattccaaactttttttcgagaaaaattcataaaaaacaacacaa
acaaaatgcctgagctcattgaacaaagcaaaattatctccggcaatgtctgcggtctgccccagttgcacaagctgcgccaggacaatt
gcggtctgtacagccacatccgtggcattcccatctcctatggaaatgtggatttgctgaccaccggtgtccgtgccttcgtggaggagggc
atcgccctctgccagcccgaccaggtgcacatctgcgatggcagcgagcaggagaacaaggtgctcatcaagagcctcttggaggctg
gcaccattgtgccgctgcccaagtacgacaattgctggctggcccgcaccaatccggcggatgtggcccgcgtcgagtcgcgcactttca
tctgcaccgagcggcgggaggagacgattcccactccagtggagggtgtcaagggaaccctgggcaactggatctcgcccagcgaca
tggatgctgcagtgcagcagcgattccccggctgcatgaagggtcgcaccatgtacgtggtgcccttcagcatgggtcctgtgggctcccc
gctctctaagatcggaatcgaactgacggactctgcttacgtggtggcctccatgaggatcatgacacgcatgggagccgctgtcttgcgc
cagctggccaagaaggaggaattcgtccgcgccctacactctgtgggcgcccctgccaacggacaggtggagcagccatcctggcctt
gtgatcccgaacgcaccatcatcctgcacaagcccgccgagaaccttattgtgtcttacggatcgggctacggcggcaactcgctgctgg
gcaagaagtgcttcgccctgaggattggcagcaccattgccaagcaggagggctggctggccgagcacatgttgattctgggcatcacc
gatcccaagggcgagaagaagtacatcactgccgcctttccctcggcctgtggcaagaccaatctggccatgctgaatccctcgttggcc
aactacaaggtggaatgcgtgggcgacgacattgcgtggatgaagtttgactcgcagggagtgctgcgtgccatcaacccagagaatg
gattctttggtgtggctccaggaacctcaatggagaccaatcccattgccatgaacactgttttcaagaacaccatcttcacgaacgtggcct
ccacctctgatggcggtgtgttctgggagggcatggaaagcagtctggctcccaatgtccaaatcactgactggttgggcaaaccctgga
ccaaggattccggcaagcccgctgcgcatcccaactcccgcttctgcactccagccgcccagtgcccgattatcgatgaagcctgggaa
gatccggcgggagtgcccatctccgccatgctcttcggaggacgtcgtcccgccggagtgccccctaatctatgaggctcgggactggacc
cacggagtcttcattggagcagccatgaggagtgaggccactgctgcggccgagcacaagggcaaggtgatcatgcacgatccgttcg
ccatgaggcccttctttggctacaactttggcgactacgtggcccactggctgagcatggagaagcgtggtcaggtgccaaagatcttcca
cgtcaactggttccgcaagagtgccgagggcaagttcatgtggcccggatacggagagaactcccgtgtgctggaatggatcctgcgca
gggtcaatggcgaatcctgctacgtggactcggccattggacacattcccgccgagggtgccctaaacctcgatggcatgaaggataag
gtggacgtgaaggagatcttctcgctgcccaaggagttctggtcgcaggaggtcaaggatattcgcacctacttcgagtcacaggtgggc
gccgatcttccggccagcatttaccagcagctggacgagctatcttcccgcgtagacaacctgtaagaaggatcaatgaggagagtccc
atctacagcatctttacctgccccacacacctgctcacccactgtgctatcccgtactgaccccatcaccaacccaatttgttaacctgtcctaa
atacctgtccccaatacccttatttagtcgtttctctagttcttaagccgaagacttccaaactatttattatataagcagccataagattagcaagt
aggccatacaaacagagaaaattaaaaaaaaaataaaacaacacgattttataacctac (SEQ ID NO:183)

**Protein:** AAF57676
**Protein GI:** 7302595

MPELIEQSKIISGNVCGLPQLHKLRQDNCGLYSHIRGIPISYGNVDLLTTGVRAFVEEGIAL
CQPDQVHICDGSEQENKVLIKSLLEAGTIVPLPKYDNCWLARTNPADVARVESRTFICTERREETIP
TPVEGVKGTLGNWISPSDMDAAVQQRFPGCMKGRTMYVVPFSMGPVGSPLSKIGIELTDSAYVV
ASMRIMTRMGAAVLRQLAKKEEFVRALHSVGAPANGQVEQPSWPCDPERTIILHKPAENLIVSYG
SGYGGNSLLGKKCFALRIGSTIAKQEGWLAEHMLILGITDPKGEKKYITAAFPSACGKTNLAMLNPS
LANYKVECVGDDIAWMKFDSQGVLRAINPENGFFGVAPGTSMETNPIAMNTVFKNTIFTNVASTS
DGGVFWEGMESSLAPNVQITDWLGKPWTKDSGKPAAHPNSRFCTPAAQCPIIDEAWEDPAGVPI
SAMLFGGRRPAGVPLIYEARDWTHGVFIGAAMRSEATAAAEHKGKVIMHDPFAMRPFFGYNFGD
YVAHWLSMEKRGQVPKIFHVNWFRKSAEGKFMWPGYGENSRVLEWILRRVNGESCYVDSAIGHI
PAEGALNLDGMKDKVDVKEIFSLPKEFWSQEVKDIRTYFESQVGADLPASIYQQLDELSSRVDNL
(SEQ ID NO:184)

## Figure 82

**Name:** CG5665
**Nucleotide:** AE003591
**GI:** 10726906
**Transcript:** CT17896
**Sequence:**

atgcattttcagtacatgacgccgtgccagaactacagtgtgcccctgctggaggcctcgaagctgtggaagcactct
cgattcagcaagggacgtaaggtggtaattctggccaccggatggaccaacaccgtaaacgaatcctcggccatttcgatgatctctaag
gcattcatgtgtcgtggcgatgtgaattttgtgattgtagatgcggctgactatgtggacaccttctacgcgtggtcggcgctgaacacggattt
aattggagagcatattggagttggtctgacccaccttatagagctgaccccacttcgaaatattcacttgattggacattcactaggagctca
cattatgggcactgctggtcggactttaagaagcttaccggaaaacttatacccaggataactggcttggatccggccaagccatgtttca
ggcgggaaaagattctgccaggattgacgcgtggcgatgccaagctagtggacataatccacaccaacattggtattttggccaagcgtg
gacccctcggtgatgtggacttctatcccggcggagcacatcccatccagccaggctgcctaaccatcggatgctctcatacccgagccg
ttgagtatttcgccgagagtgcatatccacaccaagagaagaactttatgggcaagaagtgcgcttcttgggatgagctaagaaaacggg
attgttctgctggcatagtttccccgatgggctatcgaatgaatccccaagccaggggcatctactacgtagatgtcaatggctggccccctt
acggccgaaactcggagaacactgttgatcctaggcttaaaacgtgctacttgtgccggaggtga (SEQ ID NO:185)

**Protein:** AAF51579
**Protein GI:** 7296289

MHFQYMTPCQNYSVPLLEASKLWKHSRFSKGRKVVILATGWTNTVNESSAISMISKAFM
CRGDVNFVIVDAADYVDTFYAWSALNTDLIGEHIGVGLTHLIELTPLRNIHLIGHSLGAHIMGTAGRT
FKKLTGKLIPRITGLDPAKPCFRREKILPGLTRGDAKLVDIIHTNIGILAKRGPLGDVDFYPGGAHPIQ
PGCLTIGCSHTRAVEYFAESAYPHQEKNFMGKKCASWDELRKRDCSAGIVSPMGYRMNPQARGI
YYVDVNGWPPYGRNSENTVDPRLKTCYLCRR (SEQ ID NO:186)

## Figure 83

**Name:** CG7285
**Nucleotide:** AE003520
**GI:** 10727839
**Transcript:** CT22465
**Sequence:**

atgacagccgattcagaggcaaatgccacaaattggtataacacgaacgagagcttatataccacggaactgaac
catagatggattagtggtagttccacaattcagccagaggagtccctttatggcactgatttgcccacctatcaacattgcatagccacgcg
gaattcctttgctgacttgttcactgtggtgctctacggatttgtgtgcattatcggattatttggcaacaccctggtgatctacgtggtgttgcgcttt
tccaaaatgcaaacggtcacgaatatatatatcctgaatctggcggtggcagacgagtgcttcctgattggaatacccttttctgctgtacaca
atgcgaatttgcagctggcgattcggggagtttatgtgcaaagcctacatggtgagcacatccatcacctccttcacctcgtcgatttttctgct
catcatgtccgcggatcgatatatagcggtatgccacccgatttcctcgccacgatatcgaactctgcatattgccaaagtggtctcagcgat
tgcctggtcaacttcagcggtcctcatgctgcccgtgatcctttatgccagcactgtggagcaggaggatggcatcaattactcgtgcaacat
aatgtggccagatgcgtacaagaagcattcgggcaccaccttcatactgtacacatttttcctaggattcgccacaccgctgtgctttatcctg
agtttctactacttggttataaggaaactgcgatcggtgggtcccaaaccaggaacgaagtccaaggagaagaggcgggctcacagga
aggtcactcgactggtactgacggtggccctgattcactcgaatcccgcgcaaagggacctctcccgactggaaatactcattttcctacttc
tgggggcactggtttactcgaattcggcggtgaatcccatactttatgccttcctaagtgagaacttccggaagagcttcttcaaggcctttac
ctgtatgaataagcaggatatcaacgctcaactccagctggagcccagtgttttcaccaaacagggcagtaaaaagaggggtggctcca
agcgcctgttgaccagcaatccgcagattcctccactgctgccactgaatgcgggtaacaacaattcatcgaccaccacatcctcgacca
cgacagcggaaaagaccggaaccacggggacacagaaatcatgcaattccaatggcaaagtgacagctccgccggagaatttgatt
atatgtttgagcgagcagcaggaggcattttgcaccaccgcgagaagaggatcgggcgcagtgcagcagacagatttgtaa (SEQ
ID NO:187)

**Protein:** AAF49259
**Protein GI:** 7293895

MTADSEANATNWYNTNESLYTTELNHRWISGSSTIQPEESLYGTDLPTYQHCIATRNSFA
DLFTVVLYGFVCIIGLFGNTLVIYVVLRFSKMQTVTNIYILNLAVADECFLIGIPFLLYTMRICSWRFGE
FMCKAYMVSTSITSFTSSIFLLIMSADRYIAVCHPISSPRYRTLHIAKVVSAIAWSTSAVLMLPVILYA

STVEQEDGINYSCNIMWPDAYKKHSGTTFILYTFFLGFATPLCFILSFYYLVIRKLRSVGPKPGTKSK
EKRRAHRKVTRLVLTVALIHSNPAQRDLSRLEILIFLLLGALVYSNSAVNPILYAFLSENFRKSFFKAF
TCMNKQDINAQLQLEPSVFTKQGSKKRGGSKRLLTSNPQIPPLLPLNAGNNNSSTTTSSTTTAEKT
GTTGTQKSCNSNGKVTAPPENLIICLSEQQEAFCTTARRGSGAVQQTDL (SEQ ID NO:188)

# Figure 84

**Name:** bt
**Nucleotide:** AE003843
**GI:** 10726313
**Transcript:** CT3598
**Sequence:**

gttaaagaggaggcaaagcctgcggctcccaaacttaaggccaaagcaaaagcaaaaccgaaatacgaagag
ctaccagaaattccggactatgaacgtccacagcttgaaaagtatgaaaaatccgattttacaccatccgactttgctcgtgatctggaaat
accaaacaagatggaaaaacccataatagacagcggaaagaaagaacctgcggttcttgcacagaaaaatggaattcccaaaaag
acggacattattgaacagtacgcagacgaacctaagggcttgaaagtcggcaaaggtaaacttcctgatgaaggagacggtcgagatg
gcgctgtgctcaaaccagtcatcattgaacccgagaaggaaatattggatctcggaaataaaaaaaataatcaggcaaatgcaacgga
gcagttctctcccacaaaagcagtcaaggcacaagtcgctgacttgaagaagcctgaaactttagcaacccttgaggataactatgagc
ggcccgttttagaaaaatatgatccttttagcattgataagaccaaatcagagaaatccacaccatccattatcaccccggacattagaggt
cccgaagtaaagttgcctgtgcaagaaactaaggaggagaagcaaaaggtccctaaaatgcaaccacctgctccaggtgacccaccc
aagatcgaagtgattcgggagaagcgaccatcactggcaccagagcccccaagtcgccgcggatctcttataccccccgctgatactg
gtcgacgaccatctctgatcatcaatgatgagcggcgcagaccgtcgattgatgtacgccgcccaagcgtgcaagatctagaagatctta
ttaacaagccatccacacccttacgcgatgtcggcgatggcggtcctccttctatcgttgatgtccaagagtcttactctgttgtggaagactc
gactgcttacctcactgttggcgtcgaaggtagtccagcacctacgttcaagttctacaagggagttagcgagattcttgagggcggtcggtt
taagttcctcacagatgggcaaacaaacacaattacattgtgtatgcgtaagtgcaagcccaatgatgaaagcaagtacaagatagttgtt
tcgaacattcacggtgaggattcagccgaaatgcaactctatgtatctgattcaagtggtatggacttccgtgccatgctcaaaaaacgtcgt
tatcaaaaatgggacaaagacgaacaggaccccaattggggcgaccttaaagaaacagaaaaaccgttgccggcccttaaaaaagt
tgaaaggaaggttgagtcgttcctcagtccacttatcgatcaatttgcaaaagagggaaaggacaaaaaggttgtttttgaagccagattc
agcaaacccaattgcaagcccaagtggctgtttcgaaaagacgaggtgtttaccggcagtaaatttaaatttaaacaagaaaatgatactt
atcagttgattattacgacaccgaaagtcgaggacaccggcaagtacacgattgaaatcggcggtgtttcaagtacagcatttttgaatgtc
gaagaagccgatcccacctacacttttacgaaaccccttaaaaagaaacttgagggattcactcagcatgagaccactcttgagtgctcc
gtcagcagcagcatggctaatgtgcattggtttaagaataacaccaaattggagtccgatgatcctcgctatcttatctccaaagatataaac
ggtaacctcaagcttattattaaggattctgtgctcgatgatgcggggctctacagatgtcaactggataagcaacctgataagactgagtgt
aaccttaaggtcacagagtacccgtacaagttcgtcaaggttctgaaatcacaacagtgtattgagaaagacacagtgacgctcgcctgc
gaaatcgatgatgctatgggtgaagtgcagtggttacgtaacggcgaagaaatcaagcccgataaacgtattcaaattgtaaaagatggt
cgaaagcgaaagctagtcatcaaggactgcaaggttaccgatgccggccaattcaaatgtacaacgaatgccgacaccacagagtcg
gaaattattattaactatcaaaaccgatttaataaaaaactcaaggatactgaagctgttgagagagaaaaattgattctggacatcgagct
tcaggatcaaacagcaccgtgtgactggaaatttaacggagagcccatagtaccgagtgaaagtattgaaatcaagaacatgggtggc
ggcaagcatcaactcatatttagcagcctggatatgtctaatgagggcgaaataacttgcgagtctggacagctaagttcgaaatgcaag
ctttctattcgcaagggagaaagtagaccaaacatagactgtcctgataaattttctggtcctatcagtgcccctgtgcttctagaagtgccatt
taaagtttccggtacaaagcaaacaccagtagaggctaaactcttcaaggacggaaagccattgcccgtgaaggatgtggaggttgca
gttaccgatgacaaggtcacatttaaaataaaaaagccgtcaagagatttatctgggccatatcaaataaaaatatccaatggacaaggt
gaagataccaaagatgtacagattatttgtcaagatgttccacagccccctcaggatgtggacattacagacgtctatcaaacatcctgtgt
agtaagcttcaatccccccgtctgacgatggtggtacacctataacgaaatatgtgatcgaacggcaggacttaagcaaaaaacatggttg
ggagtctgtcgctgaggttttacctagtgaaccgtgtctaaaaaaaaattgatgacttaatcccaaaaaagcagtacagattccgcattcgtg
ctgtcaacgcaatcggccaatcagatccagccacattcaaaaataccattcttgcaaaagacccatgggatgagcctggaaaaccgaa
ggcagtcgatcttaccgactgggataaagaccatgcagatctgaagtgggaagcccccgagactgatggcggggatcctataactgcat
atattgtagaatacaaagaaaagttctctaacgactgggtttccggcaaggaagttgacggcgatgctagaactgctaccgttgacggtct
aaaagaggggcaacagtatgaattccgagtccgtgctgttaatagggcaggcccaggtgaacccagcgacaagactaaatccattatt
gcaaaatgccgttttgttaagcccttcattgttggagaaggtcttaaaaatgtaaccgttaaaaaggggcaaactattcggttcgacattaaat
atgacggtgagccagagccagcagcaacatgggtaaagggaacagacaatttaaagtttgacaaccagcgcatttgcttagaccagtt
ggaaaggaactcatccattactattaaaaaatccgtgcgcaaggacacaggcaaatacaaattagtactgtcgaacagctcagggaca
atcgagtccgaggctcaagttgtggttcttgacaggccacttcctcctggtggaccatttgagccggaagagattcgcgccagccatataa
aaatgaaatggaaacgtccagatgatgacggtggttgtgaaattagtggatatgcattagagcgcatggacgaagagacgggccgctg
gattccagctggagaagtgggtccgaatgagacttcattcgattttaagggactaaccccaaataagaaatacaagtttcgcgtcaaggct
attaataaagagggcgagtcagagccacttgagacattcgacgcaatcgtggcaagaaatccatacgatcctccaagtcctcccagcca
gccagttatcgatgattacgataacaagagtgtccttttaaaatggaagcgtcctccgtcagatggcggacgcccaattacccattatatcgt
tgaaataaaagacaaattcgcaccttcctggagtgaggtggcaaagaccgatgatcccaaccctgagtgcaacgtagagggtcttaaa
gaaaaaatggtgtaccaattccgagttagagccgttaacaaagcaggcccctccgagccttcccaaccaactgacaatcacttgtgcaa
acacaaaaatcttaagccgcaaatcgatcggtctacttttaaacgagtaacaataaagagcggccgaactcacaaatggtcggttgatgt
ccttggggaaccaataccagagctgcattggagctggagggatgatatcccattaaccaacggagacagaataaagatagaaaatgtc

gactatcatactgattttagcattacaaatgttttgcgtaaggatagtggcttttatacattaaaagcagagaaccgcaatggcattgaccgag
agaccgttgaattagttgttctaggaaagccaagctcgccaaaaggacccccttgctgtaagcgatgtaactgcaagtggatgcaaacttca
atggaagaagcctgaagatgacggtggagtgccaataaaggaatacgttgttgaaaaaatggacacagcaactggaaaatgggtccg
agttggaagatctcctggtgaaaaagagccccctagctttgacgttactggcttaagtctgggatcagagtacatgttccgtgtatctgctgtt
aacgaggaaggagagtcggaacctcttactactttggtaggggtcgtggctaaagatccatttgatgagccaaataaaccaggaactcc
agaagttactgattatgataatcaaagtataagcttaaaatgggctgcacctaataatgacggaggagccccaattcaaaagtacataat
agaaaaaaagaataaaaataagacagaatgggaaaaagctctagaaataccgggagaccaactggaagccacagttgccggactt
caggaatatggagaatatcaatttcgagtaatagcagtaaacaaagccggactttcgccccccatcagatgctagtgttccccaaatagtga
agtacaaaaaattgaagccccgtattgaccgatcaaacttaaagccgttgctcattcgagccggcaagccaataaggtacgatgttaatgt
tcgtggtgaacctgcacccgtaataacgtggtatcaaaatgataaagagctcaagccagaagaacttcctagcagtagcgaaatcaag
aacatcccatataatacaaagatttcaatcattgaaactgttcgtaagcatactggaatatacaagataatagctgttaacgaacacgggc
aggatgaagccacagttgaggttaatattctagctcctccaagcaaaccaaggggtcccttggatgttaaagatgttactaaagacagctg
taagctcaaatggaaaaaaccagaagatgatggaggaaaacctatttcagcatatcaggttgaaaagtttgataaaaaaacaaggtcgtt
gggtccctcttggtcgaacgtctgccaatgataccgaatttgatgtaaagggcctccaggaaggccatgagtatcagtttcgcgtcaaggc
aattaatgaagagggggaatccgatcccctggactccgatgatagcatcatagctaaaaatccctatgatgctgctagtaagccaggtac
acctaacattgtggattataatgagcatatggtaaaattgaagtgggaagcacccaggtctgacgggggcgccccaatatcaggttacat
cattgaaaaaaaagataagttctctcctatttgggatgaaatcctcagcaccaatacatccgttcccgaagcaaccgttgaaggtcttgttga
aggcaatatctaccagtttcgtgttcgggcagttaataaggccggtttcagcgatccaagtgatgcaacagagcctcatttggccaagcca
agaaatctaaagccatacataaacagagacaaaatgaagcctattaaggttcgcgctggtcaacccgttaaatttgatgtcgatgtcaag
ggagaaccggcgccttctttaacatggttcctaaaagaaaccgagttaacgtctacgggtcaagtacgtctagaaaacatagattacaat
accaaacttacattgcttgatacggatcgcaagcaaagtggccagtataagttacgtgctgaaaatataaatggtgttgacgaagctgtagt
tgaagtcattatattagataagccttcaaaacctgaaggaccgattgaagtctccgacattcataaagaggggtgcaagcttaaatggagg
aaacctaaagatgatggaggtatacctattactggctatgtaatagaaaaaatggataccgccactggtaaatgggttcctgctggttccgt
agatccagaaaaatatgatattgagataaagggcttggatccaaatcatcgctaccaatttagagtgaaggctgtcaacgaggagggtg
aatcagaacctcttgaaactgaatcggctattactgccaagaatccattcgacgtgtctgctccccctggtctcccagagcttgaagattggg
atgagcatcatgtcaaactaaaatgggaaccacccattcgggatgggggctctcctattaccaattatataatcgaagtaatggataaaga
ttcaggggaatttgtaaaggcagttgaaactgatagtccagtttgtaagggcgtcgtaaagaaacttgaagaaggacagcaatacaaattt
agagttcgagccgtaaacaaggctggtccatcggacccatctgaacaaacgaattggcatgtggccaagcctcgattcctgaaacctcat
attgatcgagtcaatttaaaacccgtgattgtaaaaactggtttatcaattagcttggatattaacataagaggagagccagctccgaaagtt
gaatggttcttcaataattcatctgttacaagcgacgaacatagtgtaaaaattgataatgttgattacaaatacgaaattctttgttatgcgtgca
cagagatctcaaagtgggaaatacattatcaaagcaacaaacgaagttggtgaagacgaagcagaactggaagtcacagttttgggc
aagccaggaaaaccaaaaggtcccctgcaagtcaatgacataacaaaacacagttgcaagcttaagtgggaaaagccagatgatga
tggtggttcacccatcgactactatgaaattgaaaaacttgatcctcacacgggtcagtggttaccatgtggaaaaagtaccgagccggag
gccaaggtaattggtcttcatgaaggaaaggcatataaattccgagtaagggctgtaaacaaggaaggagaatctgaggacttggaaa
ccgaaaaaccaattatagcgaaaaatccatatgatgaaccagatagaccagggaagcctgaaccaactaattgggataaagatttcgt
cgacttagcctgggatccgccgaaaaacgatggtggtgcaccaattcaaaaatatgttattcaaatgcgagataagtctggacgcgcatg
ggtggactctgcaaccgtgcctggggataagtgtaatggtaccgtaactggcgtagaagagggccatgaatacgagttccgtattgttgct
gttaataaggcgggacctagtgatccaagcgatgtttcaaaatcagtgatagcgaaacctcgttttcttaaacctcacattgaccgcaagaa
cctacaaaagaaaattatgcgcagcggtcaaatgcttcatattgacgctttaataaaggcggaacctccagctaaagtaacatggaccta
caataaaacagaaattaaaacttcagatcacataaaaattgaaaacgaggactataaaacgacattcattatgccgaaagtgaaacgc
gctgatcgaggcatttacatcgtcactgccaaaaacgactcaggttccgacaccgtagaagttgagctggaagtactgtgcaagccaagt
aaaccgaaagggcctctagctgtttctaacgtaacagccgaaaccttacacctaaaatgggaaaagcccgaagatgatggcggtgacc
ctattgaacaatatttagtcgaacgcatggatacggaaacaggtcgatgggtacctgtacttacaactaaaacaccggaagccgatgtta
caggtcttacggaaggaaaggaatatctgtttagagtaaaagcagtcaattccgaaggcgaatcagagcctttggtgaccgatattccaa
caaaggccaaaaaatcctttcgatgcggccgacaccccaggtaagccgcaaattgttgattggtcaggtaaccactgtgatctaaagtgga
gggcacccgaagacgatgggggggcatccatcactggctatattgtagaaagaaaggatcccaatacaggaaagtggcaaaaggca
cttgaaacaagtacacctgactgtaaggcacgcgtaaatgacttaatcgcaggaaacaaatatcagttcagaataatggctgtcaacaa
ggcaggaaagagcaagccatccgagccgtccgaccagatgaccgcaaaggaccgatttgctccacccaaaattgatagaacaaaca
ttaaggacataaccatcaaggctggacaacacattcgtttcgacattaaggtctctggagagcccccggctacaaaagtttggcttcacaa
caaagcccgacttgaaaatgacgatagtaactacaatattgatatggaatcatatcgcacaaaactgaccgtgccaatttcaaagcgattc
cattcaggaaaatatactcttaaggcagagaatgaatctggacgggatgaggcttcatttgaagtcatagtcttagacaaaccgggcccc
ccagagggtcccttacgagtcacagatgtgcacaaagaagggtgcaagctgaaatggaacgctcccttggacgatggaggtttgccaat
cgaccattacattattgagaaaatggacgttgagagtgggcgttggttgccatctggtcgctttaaggaatctttcgcagagttgaataatttg
gagcctagtcatgagtacaaattccgagtgcttgccgtaaatactgaaggtgaatctgaaccattaactggagaacagtctgttattgccaa
gaatccttttgatgagcccggaaagcccggaactccggaagcagtggactgggataaagatcatgtcgatttggtttggaggccaccaat
taacgatggtggatcgcctatcactggctatgtagttgaaaaacgtgagaaaggtactgacaagtggataaagggaacagaaattactat
cccgtgccttggcgaagagtgcaaagctactgtgccaaccctaaatgaaaattgtgaatacgaattccgagtcaaagcaattaatgctgct

gggcctggtgaaccatctgacgcaagcaagcctatcattacaaagccaagaaagttggcccccaaaattgaccgcaaaaatattcgta
cctataacttcaagtctggagagccgatatttttggacatcaatattagtggagagcccgcaccggatgttacttggaatcaaaacaacaa
gtctgttcaaaccacatcatttagtcatattgagaaccttccgtataacaccaaatacataaataacaaccccgaacggaaagatacggg
actctacaagatctcagctcataacttttacgggcaggatcaagtcgagtttcaaattaatattattacgaaaccaggcaagcctgaaggtc
cattagaagtatcagaagtacacaaggatggttgtaagctgaaatggaaaaaacccaaggacgatggaggtgaacctgttgaaagcta
tttggttgagaaattcgacccagatacgggtatttggttacctgttggcaggtctgacggacctgagtacaacgttgatggcttagtcccaggt
catgattacaagttccgggtaaaagctgtaaacaaggaaggggaatcggagccactggaaacgttgggttcaataattgccaaggatcc
atttagcgttccaacaaagcctggagttccagaacctaccgattggactgcaaataaggtagaacttgcttggccagaaccagcaagtga
tggtggatctccgatacaaggctatatagttgaggtaaaagacaaatacagccctctttgggaaaaggctttggaaaccaattccccaact
ccaactgcaacggttcaaggtctcattgaaggaaatgaataccagttccgagtagttgccttgaataagggtggcctatcggagccttcag
acccaagcaaaatatttacagctaagccgcgctatcttgcccccaagatagaccgtagaaatctacgtaatattactctgtcgtccggaac
cgctctgaaattagatgcaaatataacaggtgaacccgctccaaaagtagagtggaaactttccaattaccatttgcaatcagggaaaaa
tgttacaatcgaaactcctgactactatacaaaattagttattagaccaacgcagcgaagtgattctggagaatatttggtcacggcgacta
acacttctggaaaagatagtgtgcttgtaaatgtagttatcacagataaaccctcacctcctaacggaccacttcaaatatcagatgtgcata
aagagggttgccaccttaaatggaaacgtccgagcgatgatgggggaacgccaattgagtatttccaaattgacaaattagagcccgaa
actggttgctggattccttcttgtcgatctacagaaccccaagttgatgttacaggtctttctccgggaaatgaatataagttccgagtatcagct
gtgaatgccgaaggtgaatcacagcccttggtaggcgatgaatctatagttgcaagaaatcctttcgatgaacctggtaagcccgagaac
cttaaagcaactgattgggataaggatcacgtggacttggcgtggaccccacctctaattgatggcggatcaccaatttcttgctatattatcg
aaaagcaggataaatatggaaagtgggagcgggcccttgacgtccctgctgatcaatgcaaagcgactattccagatcttgttgagggc
caaacctacaaattccgagtttccgcggtcaacgcagctgggactggagagccatctgattctactccgccaataattgcgaaggctcgc
aataaaccacccattatagatagatccagcctagtagaagtgcgtattaaagctggacagtcctttacatttgattgtaaggtatctggggag
ccagcgcctcagacaaaatggctacttaagaaaaaggaagtttattcaaaggacaacgttaaggtaactaatgttgactacaataccaa
actaaaagtaaatagtgcaaccagatctgattccggtatatacacagtgtttgcagaaaacgcaaacggagaagacagtgcagatgtta
aggtaactgtcattgacaaacctgcaccgccaaacggaccgcttaaggttgatgaaataaattccgagtcttgtactctgcactggaatcc
accagacgacgatggaggacaaccaatagataattatgtcgttgaaaaattagatgaaacaacaggacgttggatacctgccggggaa
actgacgggccagtcaccgcacttaaagtcggtggccttacccctggccataaatacaaattccgagtacgtgccaaaaatcgtcaggg
aacttcagaaccacttacaacagcacaagccattattgcgaaaaatccattcgatgtacctacaaaaccaggaacaccaaccataaaa
gactttgacaaagaatttgttgatctggaatggactagaccagaggctgatggtggatctccaataaccgggtatgtagttgagaagcggg
acaaattttctcctgattgggaaaaatgcgctgaaatcagtgatgacatcaccaatgctcacgttcctgacttgattgaaggccttaaatatg
aattccgtgtcagagcagtaaacaaagccggtccaggctcaccaagtgatgcaactgaaacacatgtggctcgcccaaaaacacac
ccccaaaaatagatcgaaacttcatgtccgatattaaaattaaagctggaaatgtatttgaatttgatgtaccagtaactggggagcctttgc
caagcaaagattggacccacgaaggcaatatgatcataaacacagaccgagtaaaaatttccaattttgatgaccgcactaagataag
aatacttgatgcgaagcgatccgacacaggcgtctatacattaacagcacgtaacataaacggtacagataggcacaatgtaaaagta
acaatcctggatgctccaagtgtgccggaaggcccattacgtaatggcgatgttagcaaaaactctatagtacttcgatggaggccaccc
aaggatgatggtggctcggagattactcattatgttgtggaaaaaatggataatgaggccatgcgctgggttcccgttggtgattgcactgat
actgaaatcagagcggacaatctaattgaaaatcatgattatagttttagggtaagggctgttaataagcaaggacaatcacaaccactca
caacttctcagcctataacagcaaaagatccttattcacatccagataagcctggccaaccgcaagctacagattggggtaaacattttgt
ggacttggagtggtccactccaaagagagatggaggagcacccatttcttcgtatattatagaaaaagaccaaagtttggtcaatggga
acgagctgctgttgtgctaggtgataattgtaaggctcatgtaccggaacttacaaatggtggagaatacgagtttcgggtgatcgccgtca
atcggggaggtcctagcgatccatctgatccgtctagcaccattatatgcaagccacgtttccttgctccattctttgacaagtcacttctaaac
gacatcacggtccatgctggaaagcgattagggtggacattaccaatcgaggcgtctccaagaccattaatcacttggctatataatggaa
aagaaatcggttcaaattcacgaggcgagtccggtcttttccaaaacgaactaacatttgaaattgtatcctcacttagaagcgacgaagg
tcgttatactttaatattaaagaatgagcatggatcatttgatgcgtctgctcatgcaactgtattagatcgaccatcgccacccaaaggtcctc
tggatataacaaagataacaagggatggctgccacctaacctggaatgttccggatgatgacggtggttcgcctatattgcattatattatcg
aaaaaatggatttatctcgaagcacatggtccgatgctggcatgagcacacatatagtccacgatgtcactcgtcttgtccaccgcaagga
gtacttgttccgtgttaaagcagtcaatgcaattggggaatccgatcccttagaagcagtaaatactataattgctaaaaacgaatttgacga
gcctgatgcgcctggaaaaccaattattactgattgggatcgtgaccatatagacttgcaatgggctgtcccaaaaagtgacgggggagc
tccaattagtgaatacatcatacagaaaaaggaaaagggcagcccttactggactaatgtacgacatgttccatcaaataagaacacaa
ccacaattccagaactaactgagggtcaagagtatgaatttagagttattgctgttaatcaagcaggccaatctgaaccttcagagccgtct
gatatgattatggcaaagccgcgctacctgccaccaaaaataataactccactaaatgaagtgcgcattaaatgcgggctcatatttcaca
cagatatacattttatcggtgagccagcaccagaagcgacgtggacacttaatagcaatccactattgtcgaatgacagatcaacaattac
atcaatcggtcaccactctgtagtacatactgttaactgtcaaaggtctgactccggaatctaccacctattacttcgtaatagttctggaatcg
atgaaggcagcttcgaacttgtagtactcgaccgcccggggccgccagagggtccaatggaatatgaagaaataactgcgaactctgtg
actatatcatggaaaccaccaaaagataatggaggatctgaaatatcttcttatgttatagaaaagcgtgatcttacccatggcggtgggtg
ggtaccagcagtaaattatgtaagtgcaaaatacaaccatgccgtggttcccagactgctggaaggaacaatgtacgagcttagagttat
ggccgaaaacctacaagggcgctccgatccacttacttccgaccaaccggttgttgcaaaatctcagtacacggtgccaggagctcctg
gaaagccagaactaactgatagcgacaagaatcatattacaattaaatggaagcaaccaattagtaatggaggatcccccattattggtt

acgacattgaacgtcgcgacgtaaacacaggacgatggataaagataaatggacaaccagtacctactgctgaatatcaagacgaca
gagtaacatcgaaccatcaatatcaatacagaatatcggcggttaatgctgccggcaacgggaagacttccgaaccttcggccatattca
atgctcgaccactgcgtgaaaagccaagattttattttgatggcttaataggaaagcgcattaaagttcgtgcaggtgaaccagtaaatttaa
atattcccatatctggggcacctactccgacaattgaatggaagcgaggggatctcaaattagaggaaggcaagcgaatatcttatgaga
ctaactcggagcgtactctatttcgcattgatgattcaaacagacgggactctggaaaatacactgttaccgctgctaatgagtttggtaagg
ataccgctgacattgaagttattgtcgtagataagccatcgccacctgaaggacctttgagttatacggagacagcaccagatcacatttctt
tgcattggtattcccccaaggacgatggtggcagcgacattacggggtacataatcgagttcactgagtttggtgtagatgattggaagcca
gtgccaggcacttgtcccaacaccaactttacagtaaagaatctagtagagggaaaaaaatatgtttttcgtattagagctgaaaatatcta
cggagcttccgaagctctcgagggtaagccggtgttggctaagtcgccttttgatccacccggtgcaccatctcagccaacaatttctgcat
atacaccaaactctgctaatttggagtggcatcctccagacgattgtggtggtaagcctattacggggtacatcgtagagcgacgcgaacg
cgggggtgagtggattaagtgcaacaattacccaacaccgaatacatcctatacagtatccaatcttcgggatggagctcgttatgagttcc
gggtcctcgcagtaaatgaggctgggcctggccacccgtcaaagccttctgatccaatgactgctgaacatcagcgctatcgtccagatc
cgccggagccaccaaaaccggatcgtattactaggaatggtgtcacattatcttggcgtccgcctcggacggatggcaagtccagaata
aaagggtattatgtcgaaatgcgtcccaaaaacgggaaggattggaaaactgttaacgatatacctattaattccactgtatacacagtac
caagtcttaaggaaggagaagagtactcattccgtgttgttgctgaaaacgaagttggtcgttcagatccatctaagccatcgcagcccatt
acaattgaggagcaaccaaataagccttgcatggaactcggaaaggtccgagatatagtgtgtagggctggtgatgattttagcattcacg
taccctacctagctttccccaagcccaacgcattttggtattctaatgacaatatgctggatgacaataaccgtgtgcacaaacatttgactga
tgatgcagcatctgttgtggtaaaaaactcgaaacgtgcagactcaggtcaatatagactccaattaaagaatacgtcaggattcgatacg
gccaccattaatgttagggttctggatagaccatctccaccgacacgcctacgtgctgatgagttttctggagattctctaacattgtattggaa
tccaccaaacgatgacggtggttccgctattcaaaattatattattgaaaaaaaagaagctcgttcgtccacatggtctaaggtcagcagctt
ttgcacagttccttttgttagaattcgaaaccttgtactcaataaagagtacgatttccgtgtgatagctgaaaataaatatggtcaatccgatc
cggccaatacatctgagcctatacttgcaagacatccctttgatatccccaatactcccggtattccccatggtattgactcgacagaagata
gtataacaatagcatggacgaagcctaaacatgatggtggatctcccataactggatatattattgaaaaacgattattgagtgatgacaa
atggaccaaggcagttcatgcactttgtccagacctttcttgtaaaattccaaatcttattgaaaatgctgaatacgaatttagggtggccgca
gtaaatgcagccggacagtcggcctatagcggttccagcgatctcatcttttgccgccgaccacctcatgcgccaaagattacttcagattt
gtcaattcgggatatgacagtaattgcaggagatgaattccgtataacagtaccttatcacgccagtcctcgcccaactgcatcatggagttt
aaatggcttagaagtaattcccggcgagcgtattaaatttgattctaacgattatgcttcaatgtattacaacaaatcagctaaacgagatga
aactggatcgtacaccattacacttaccaataacaaaggttcagatactgcttcatgtcacgtcacggtggtagataggccattaccacctc
agggtccattgaatgcctacgacattacccccgacacttgcaccttggcctggaagacgcctttggatgatggaggttcaccgattacaaat
tatgtcgttgaaaaattagataacagtggttcatgggtaaagattagcagttttgttcgtaacactcactacgatgtcatgggcctggaacctc
actacaaatataacttccgcgtaagagcagaaaaccaatatggtttgtctgatcctttggatattattgaacctattgttgccaaacatcaattt
acagtcccagatgagcctggtcagccaaaggttattgactgggactctggaaatgtaactttgatttggacccgaccattaagtgatggag
ggtcccgcatacaaggctatcaaattgaatatcgggatattttaaacgattcgtcttggaatgcgtatgactacattataaaggacactaagt
atcaactttacaacttaattaacggcagtgaatacgaattccgtataaaggccaagaacgctgcaggcttaagtaagccatcttcaccatc
acttagatttaagctgaaaggaaaattcaccgtgccatcacctcctggagccccacaagtaaccagagtcggcaaaaactatgtcgacct
aaagtgggagaaaccccttaagagatggtggctcccgcattacgggttacattattgaaagaagggacatcggtggagccgtatgggtaa
agtgtaatgattacaatgttttggatactgaatacaccgttatgaacctcatcgagatgggtgattacgaatttcgagtatttgctgtcaattccg
ctggacgttcagaaccaagcttatgtactatgccaatcaaggtttgcgaggtactaggtggaaagaagcctgattggattacacgactgca
agacaaggtagcacccttggcaaggattatactttacaatgcgccgcctcgggtaagccaagtccaaccgcaaggtggcttcgcaatg
gaaaagaaattcaaatgaatgggggccgaatgacatgcgacagtaaagatggggtcttccgactgcacatatctaacgtgcaaactgg
agacgatggtgactacacatgtgaggcaatgaattctctcggatttgttaacacatcaggttatctaaaaattggatcaccaccaattattaa
caggtgcccaagcgaactaaaattaccagaaggagacaattcgaagatcaagattttctactctggcgaccagccactaactgtgatatt
aaagaaaaataatgaagtcatatgtgacagtaatgatgatacgcatgttaaggtaaacatatttgacgactatgtagcgatttacattcgtaa
tatcgtaaaaagtgatggtggtccatatcaaatagaattcacgaatgaatctggtagtgccacaggggagttttatgtacacatcactggcat
gccttcagcacccactggacctatgggtatctcttacattaacaaaaactcctgtatgttaaactggcgtccccccttcttacgatggtggtctta
aagttagtcactacgtcatcgagagagaaaagatgtgtcatctccacactggataactgtttctagtacctgcaaagataccgccttcaatgtac
aaggtcttattgaaaaccaagaatacattttccgagtgatggctgtaaatgagaacggaatgggtccaccactagaggggttaaatccaat
tcgagctaaagacccaattgacccaccatcgccaccgggcagtcctcaaatcactgagatcggaggagactttgtgcatttggaatggg
aaaaaccggaatcggatggcggtgctcacattcaaggctattggatcgataagcgtgaagtaggcagcaacacatggcagcgtgtaaa
cgctaccatctgtgctgccaatcaaataaactgtatcaatctcattgaaggtcgacagtacgagtttcgaattttgctcagaatgtagcaggc
ctttcaacagaatcgtccgcttcacaagcagtcaagataatagatccacaggcggcttcgcctccattgattgtgaaaccgctacgcgatg
ccaattgcattcaaaaccacaatgcccaattcacatgcaccataaatggcgttcctaaaccaacgatatcttggtataaaggagctcgcga
aattagtaatggcgcccggtatcacatgtactcagaaggtgacaatcacttcctaaatattaatgacgtattcggcgaagacgctgatgaat
atgtttgtcgcgctgtaaacaaggctggagcaaaatcaacacgcgccactctggccattatgactgctccaaaattaaacgttcctccaag
atttagagacactgcttacttcgataagggtgaaaatgttgtaatcaaaattcccttcactggcttcccaaaaccacgtatacactgggttagg
gacggtgagaatattgaatccggtggtcattacactgttgaggttaaagaaagacatgccgttcttatcattcgtgatggatcgcacttggact
cgggaccttataggataacagcggagaatgaacttggttcggatacagctataatacaagtgcaaattagtgatcgtccagatcccccac

gattccctttaatagaaagtattggcacagagtcattgtctcttagttggaaagcacctgtatgggacggttgcagcgacataacgaattact
acgtagagcggcgggagcacccattatcttcgtggatacgagtcggaaatactaggtttacgtctatggcagtcagcggcttgactccagg
aaaggagtacgatttccgcatatttgctgataatgtatatggacggtctgatgcttcagacacaagtactctaatcaagactaaggagtctgtt
aaaaagaaaccaattgaacgtaaatgggaaattgatgcaaatggacgaaaactgcgcggaaaagcagatggaccagttaaagacta
cgattcatatgtgtttgatatttactccaagttcgttccgcaacccgtggaaatatcccagcaaagtgtatatgataggtatgatatccttgagg
aaatcggaacaggggcatttggagtagtacatcgctgtcgtgagagaagcacaggaaatatttttgctgccaagttcattccagtatcaca
ctcagttgagaaagacttgatacgtcgtgaaatagatataatgaatcaactgcatcatcagaagcttatcaacttacacgatgctttcgaag
acgacgatgaaatgatcctaatactggaatttttatctggtggtgaattatttgaacgcattactgctgaaggttacgtaatgacggaagctga
agttataaactatatgaggcaaatttgcgagggcatcagacacatgcacgaacaaaatattatccatttggacataaaaccagagaatat
aatgtgtcaaactcgttctagcacaaatgttaaacttatcgactttggactagcgacgcgcctcgatccaaatgaagttgttaaaataacaac
aggcacggctgagttcgctgcaccagagatcgtcaacagagagccagtcggcttttacactgatatgtgggctactggagtactctcctat
gttctgttaagcggattatcgccctttgctggtgataatgatgttcaaactctcaaaaacgtaaaggcttgcgattgggacttcgacgtagaat
cctttaaatacatttctgaagaagctaaagattttataagaaaattgctcgttagaaacaaagaaaagcgtatgacggctcacgaatgtcttc
tgcatccttggctgactggagaccatagtgctatgaaacaggaaatcaaccgtgatcgttatctagcctatcgagaaaaattgagaagaa
aatatgaagacttcgagaggttcctgctgccgattggccggctttctgaatactcatccttaagaaaattgctgatggagaaatataagatcc
atgatgctgttttcgataggcgtcaagcagcgccacgttttgtaattcgaccatcttcacaattttgctatgagggccaaagcgtaaagttctac
tgcagatgcattgcaatagccacacctactctcacttggtcacataataatatcgagttgcgacagagtgtcaagtttatgaaacgatacgtt
ggagatgattactacttcatcattaaccgagttaagttggacgatcgaggagaatatattatacgcgcagaaaatcactatggctctagaga
agaagtcgttttcctaaatgttcagcccttaccaaaagaacagccgaggtaccgaacggaatcaactccggtgcgcagacgtgaaccctt
gccgtacacattctggcaggaagagtcagagacggcgcccagtttcacattccttttgcggccacgcgtcatgcaagcccgcgatacatg
caaactactgtgttgcttaagtggcaagccagtacctaatgtaaggtggtacaaggacggccgagaacttagcaaatacgagtatgcgat
gactcactcagatggagttgtcaccatggaaataattgactgcaagccatccgattcaggaaaatacagctgtaaggcaacaaactgtca
cggaacagacgaaacagattgtgttgtgattgttgaaggagaatgggttacgcctgagcaggctcaactcgcccacaattttctttactcag
gagataggaaatacatagagcagccaattaagccagctcccctcccaatagtgacatcccggcagtatacatcatcttcagtccaaaac
acatctgaaccacagggtgacaaagtaaatgtgtccaactcaaactcatcaggcatttcaaacaagaagaagtatgcatcaaatagttta
caggccccaggaagcccgtcaagatcacgaagcgctaccaaagagcttatacttccaccagatgattcattaatgtgcaaaccggaattt
acgaagccccttcacgatttaacaattcacgatggagaacagttaatattaacttgctatgtaaaaggagatccagagcctcaaatctcttg
gtcgaaaaatggaaagtccctatcatcttctgatattttggacttaaggtataaaaatggtattgcaactttaaccatcaacgaagtgtttcctg
aagacgagggagttatcacttgcacggccacaaattctgttggagccgtggaaactaaatgcaaactaacaatacagcccctggataaa
aacataaacaagcgaaaagtaaatgccggcgacaacgcgccgaaaatagttagccacttggagtccagatttgttagggatggcgatg
cagtgaatctggcttgtcgcataattggagctcaacatttcgatgtcgtatggctgcataataacaaagagataaagccgagcaaggatttc
caatacaccaacgaggccaatatatatcgattacaaattgcagagatatttcccgaagatggcggcacctacacttgcgaggccttcaat
gatataggcgaatcattcagtacctgcacaataaatgtaacagtgccaggagacgagactaagcagccttcctttgtaaagtttcccacgt
cagtttctgtacttgagggagagggtaccacatttgaatgcgaaatagattctgagctcctaaatttggtttggctgaaagatgggaaaccca
ttgacgaaactcttccacgttactcatttacaaaggatggccatcgatactcttttgcagtggcaaaatgtaatatggacgatgtcggtcaata
ccaagctaaagcggtatctgggaaggccgaaagtatttgtgcgttctcgatgaacgttcacacagcggaatccgcaaatccttggcaatta
ttgtccataacttcaaatttcaaaagagcttgtgcgagatttttcccgcagcgacgttaaattttatgatcagaaaacaaagtcaatggttgtttg
tgttctttggctgtatagataatacatgggggccttcctgtagaaaggacctggaaactttaacagacatcagtctgcgagtgctcttccgag
ggtggttagagacccacacggacaatgggggtaaactgttcactctgagcgcggatccaccaagcttggtcgcatataaggcctatttgta
tgacggggatcgctgctga (SEQ ID NO:189)

**Protein:** AAF59316
**Protein GI:** 10726323
MGVAEDFAPSFVKKPQLHQEDDGNRLIFECQLLSSPKPDIEWFRSDNKVVEDVRTKFKIQ
PVGENKYTVVLELDDVVETDAGLYKVKAKNKSGEVSASINLNFTPADEPKEKQIDGFAPTFAKKPA
IRQEEDGKRLLFECRVNADPIPAIIWFHNGAAVKESERHKITVDKDVHSYFATLEILNVTVEDAGKY
KVNAKNELGESNATISLNFDNLVIGAYSHFQIALYGYFNSIGDEAPVPESAEGIKPTFTERPVIRQSE
DGGNVTFECRCVGDPTPTVTWSHGETELNESNRYKMSLTMDQKLYHIACLEISSVVSSDQGEYR
AQAKNKHGSGVATINLNFESGSKKIPDGKSPRFPKKPTIRQEEDLLIMECVLEAHPVPDIVWYCSE
KEICNNQRTKMTRKAITKDSYILTLEIQNPTKEDGGNYRCNAINMYGESNANIALNFQGASDANGF
APSFIEKPRIIPNESGTLITMKCKCKAKPEPTVTWYRGQDLVEKSKKIKINTTVIAEDTYELTLEIKDP
GATDGGTYRCNVKNEYGESNANLNLNIEAEPEPEGEGPTFIEKPRIVSENNGKLVIMECKVKADPK
PDVIWFRNGEVIKESNKIKTFIEQRGDQYYIKLELLDPQLEDSGLYKCNIKNTLGELNANLTLNIEIVP
VIKDKPKIIKIIKKRTVVIECTVASKFEPKCTWYKETSTVKESKRHVYQVEQTKEGEFAVKLEINDVE
ESDKGAYKLVASNEKGEAVSQIVNLVDIPEEERKPCKPEISRKLADQKVAESKTFELLVSLSQTDR
KCKVEWYKGSTVIRETKDITTTFDGTTARLTFSSARTEHTSNYKVIVTNEVGKDESSCKITVEKVAK
KKEEKPKEKEKTKNEKEVEQKEMEEDKNESGQSVAQTEGRINIEQISEGDPKEELTVKEEILDKRD

TQEVKESSVELQDSAGHEVPEPKKATSDSKLDQSNQKNLDKKHKTDQSESKTNKNVSLAEPIKSN
KQESEEQQATEQIGLKKVDRKASIVSVKEEISSDVRRKSTIKAKEEITVDDKKASSRRSSLAVEESN
TESRRSSIIDKKPLEQVDNKPIDANKNPQPLKEEIPRLKPAEKRRTSKVIEEPKPDEGLPKLRKASIA
QVKEEAKPAAPKLKAKAKAKPKYEELPEIPDYERPQLEKYEKSDFTPSDFARDLEIPNKMEKPIIDS
GKKEPAVLAQKNGIPKKTDIIEQYADEPKGLKVGKGKLPDEGDGRDGAVLKPVIIEPEKEILDLGNK
KNNQHADKPTVLDIIKQRRRSSIRNLMTKEPIQNESFLGVVLKPVIKDTREQAAPQQAIQLTKANAT
EQFSPTKAVKAQVADLKKPETLATLEDNYERPVLEKYDPFSIDKTKSEKSTPSIITPDIRGPEVKLPV
QETKEEKQKVPKMQPPAPGDPPKIEVIREKRPSLAPEPPSRRGSLIPPADTGRRPSLIINDEKKLRP
GEVMDTRRRRPSIDVRRPSVQDLEDLINKPSTPLRDVGDGGPPSIVDVQESYSVVEDSTAYLTVG
VEGSPAPTFKFYKGVSEILEGGRFKFLTDGQTNTITLCMRKCKPNDESKYKIVVSNIHGEDSAEMQ
LYVSDSSGMDFRAMLKKRRYQKWDKDEQDPNWGDLKETEKPLPALKKVERKVESFLSPLIDQFA
KEGKDKKVVFEARFSKPNCKPKWLFRKDEVFTGSKFKFKQENDTYQLIITTPKVEDTGKYTIEIGG
VSSTAFLNVEEADPTYTFTKPLKKKLEGFTQHETTLECSVSSSMANVHWFKNNTKLESDDPRYLIS
KDINGNLKLIIKDSVLDDAGLYRCQLDKQPDKTECNLKVTEYPYKFVKVLKSQQCIEKDTVTLACEI
DDAMGEVQWLRNGEEIKPDKRIQIVKDGRKRKLVIKDCKVTDAGQFKCTTNADTTESEIIINYQNRF
NKKLKDTEAVEREKLILDIELQDQTAPCDWKFNGEPIVPSESIEIKNMGGGKHQLIFSSLDMSNEGE
ITCESGQLSSKCKLSIRKGESRPNIDCPDKFSGPISAPVLLEVPFKVSGTKQTPVEAKLFKDGKPLP
VKDVEVAVTDDKVTFKIKKPSRDLSGPYQIKISNGQGEDTKDVQIICQDVPQPPQDVDITDVYQTS
CVVSFNPPSDDGGTPITKYVIERQDLSKKHGWESVAEVLPSEPCLKKIDDLIPKKQYRFRIRAVNAI
GQSDPATFKNTILAKDPWDEPGKPKAVDLTDWDKDHADLKWEAPETDGGDPITAYIVEYKEKFSN
DWVSGKEVDGDARTATVDGLKEGQQYEFRVRAVNRAGPGEPSDKTKSIIAKCRFVKPFIVGEGLK
NVTVKKGQTIRFDIKYDGEPEPAATWVKGTDNLKFDNQRICLDQLERNSSITIKKSVRKDTGKYKL
VLSNSSGTIESEAQVVVLDRPLPPGGPFEPEEIRASHIKMKWKRPDDDGGCEISGYALERMDEET
GRWIPAGEVGPNETSFDFKGLTPNKKYKFRVKAINKEGESEPLETFDAIVARNPYDPPSPPSQPVI
DDYDNKSVLLKWKRPPSDGGRPITHYIVEIKDKFAPSWSEVAKTDDPNPECNVEGLKEKMVYQFR
VRAVNKAGPSEPSQPTDNHLCKHKNLKPQIDRSTFKRVTIKSGRTHKWSVDVLGEPIPELHWSW
RDDIPLTNGDRIKIENVDYHTDFSITNVLRKDSGFYTLKAENRNGIDRETVELVVLGKPSSPKGPLA
VSDVTASGCKLQWKKPEDDGGVPIKEYVVEKMDTATGKWVRVGRSPGEKEPPSFDVTGLSLGS
EYMFRVSAVNEEGESEPLTTLVGVVAKDPFDEPNKPGTPEVTDYDNQSISLKWAAPNNDGGAPI
QKYIIEKKNKNKTEWEKALEIPGDQLEATVAGLQEYGEYQFRVIAVNKAGLSPPSDASVPQIVKYK
KLKPRIDRSNLKPLLIRAGKPIRYDVNVRGEPAPVITWYQNDKELKPEELPSSSEIKNIPYNTKISIIET
VRKHTGIYKIIAVNEHGQDEATVEVNILAPPSKPRGPLDVKDVTKDSCKLKWKKPEDDGGKPISAY
QVEKFDKKQGRWVPLGRTSANDTEFDVKGLQEGHEYQFRVKAINEEGESDPLDSDDSIIAKNPYD
AASKPGTPNIVDYNEHMVKLKWEAPRSDGGAPISGYIIEKKDKFSPIWDEILSTNTSVPEATVEGLV
EGNIYQFRVRAVNKAGFSDPSDATEPHLAKPRNLKPYINRDKMKPIKVRAGQPVKFDVDVKGEPA
PSLTWFLKETELTSTGQVRLENIDYNTKLTLLDTDRKQSGQYKLRAENINGVDEAVVEVIILDKPSK
PEGPIEVSDIHKEGCKLKWRKPKDDGGIPITGYVIEKMDTATGKWVPAGSVDPEKYDIEIKGLDPN
HRYQFRVKAVNEEGESEPLETESAITAKNPFDVSAPPGLPELEDWDEHHVKLKWEPPIRDGGSPI
TNYIIEVMDKDSGEFVKAVETDSPVCKGVVKKLEEGQQYKFRVRAVNKAGPSDPSEQTNWHVAK
PRFLKPHIDRVNLKPVIVKTGLSISLDINIRGEPAPKVEWFFNNSSVTSDEHSVKIDNVDYNTKFFVM
RAQRSQSGKYIIKATNEVGEDEAELEVTVLGKPGKPKGPLQVNDITKHSCKLKWEKPDDDGGSPI
DYYEIEKLDPHTGQWLPCGKSTEPEAKVIGLHEGKAYKFRVRAVNKEGESEDLETEKPIIAKNPYD
EPDRPGKPEPTNWDKDFVDLAWDPPKNDGGAPIQKYVIQMRDKSGRAWVDSATVPGDKCNGTV
TGVEEGHEYEFRIVAVNKAGPSDPSDVSKSVIAKPRFLKPHIDRKNLQKKIMRSGQMLHIDALIKAE
PPAKVTWTYNKTEIKTSDHIKIENEDYKTTFIMPKVKRADRGIYIVTAKNDSGSDTVEVELEVLCKPS
KPKGPLAVSNVTAETLHLKWEKPEDDGGDPIEQYLVERMDTETGRWVPVLTTKTPEADVTGLTE
GKEYLFRVKAVNSEGESEPLVTDIPTKAKNPFDAADTPGKPQIVDWSGNHCDLKWRAPEDDGGA
SITGYIVERKDPNTGKWQKALETSTPDCKARVNDLIAGNKYQFRIMAVNKAGKSKPSEPSDQMTA
KDRFAPPKIDRTNIKDITIKAGQHIRFDIKVSGEPPATKVWLHNKARLENDDSNYNIDMESYRTKLT
VPISKRFHSGKYTLKAENESGRDEASFEVIVLDKPGPPEGPLRVTDVHKEGCKLKWNAPLDDGGL
PIDHYIIEKMDVESGRWLPSGRFKESFAELNNLEPSHEYKFRVLAVNTEGESEPLTGEQSVIAKNP
FDEPGKPGTPEAVDWDKDHVDLVWRPPINDGGSPITGYVVEKREKGTDKWIKGTEITIPCLGEEC
KATVPTLNENCEYEFRVKAINAAGPGEPSDASKPIITKPRKLAPKIDRKNIRTYNFKSGEPIFLDINIS
GEPAPDVTWNQNNKSVQTTSFSHIENLPYNTKYINNNPERKDTGLYKISAHNFYGQDQVEFQINIIT
KPGKPEGPLEVSEVHKDGCKLKWKKPKDDGGEPVESYLVEKFDPDTGIWLPVGRSDGPEYNVD
GLVPGHDYKFRVKAVNKEGESEPLETLGSIIAKDPFSVPTKPGVPEPTDWTANKVELAWPEPASD
GGSPIQGYIVEVKDKYSPLWEKALETNSPTPTATVQGLIEGNEYQFRVVALNKGGLSEPSDPSKIF

TAKPRYLAPKIDRRNLRNITLSSGTALKLDANITGEPAPKVEWKLSNYHLQSGKNVTIETPDYYTKL
VIRPTQRSDSGEYLVTATNTSGKDSVLVNVVITDKPSPPNGPLQISDVHKEGCHLKWKRPSDDGG
TPIEYFQIDKLEPETGCWIPSCRSTEPQVDVTGLSPGNEYKFRVSAVNAEGESQPLVGDESIVARN
PFDEPGKPENLKATDWDKDHVDLAWTPPLIDGGSPISCYIIEKQDKYGKWERALDVPADQCKATIP
DLVEGQTYKFRVSAVNAAGTGEPSDSTPPIIAKARNKPPIIDRSSLVEVRIKAGQSFTFDCKVSGEP
APQTKWLLKKKEVYSKDNVKVTNVDYNTKLKVNSATRSDSGIYTVFAENANGEDSADVKVTVIDK
PAPPNGPLKVDEINSESCTLHWNPPDDDGGQPIDNYVVEKLDETTGRWIPAGETDGPVTALKVG
GLTPGHKYKFRVRAKNRQGTSEPLTTAQAIIAKNPFDVPTKPGTPTIKDFDKEFVDLEWTRPEADG
GSPITGYVVEKRDKFSPDWEKCAEISDDITNAHVPDLIEGLKYEFRVRAVNKAGPGSPSDATETHV
ARPKNTPPKIDRNFMSDIKIKAGNVFEFDVPVTGEPLPSKDWTHEGNMIINTDRVKISNFDDRTKIRI
LDAKRSDTGVYTLTARNINGTDRHNVKVTILDAPSVPEGPLRNGDVSKNSIVLRWRPPKDDGGSEI
THYVVEKMDNEAMRWVPVGDCTDTEIRADNLIENHDYSFRVRAVNKQGQSQPLTTSQPITAKDP
YSHPDKPGQPQATDWGKHFVDLEWSTPKRDGGAPISSYIIEKRPKFGQWERAAVVLGDNCKAHV
PELTNGGEYEFRVIAVNRGGPSDPSDPSSTIICKPRFLAPFFDKSLLNDITVHAGKRLGWTLPIEAS
PRPLITWLYNGKEIGSNSRGESGLFQNELTFEIVSSLRSDEGRYTLILKNEHGSFDASAHATVLDRP
SPPKGPLDITKITRDGCHLTWNVPDDDGGSPILHYIIEKMDLSRSTWSDAGMSTHIVHDVTRLVHR
KEYLFRVKAVNAIGESDPLEAVNTIIAKNEFDEPDAPGKPIITDWDRDHIDLQWAVPKSDGGAPISE
YIIQKKEKGSPYWTNVRHVPSNKNTTTIPELTEGQEYEFRVIAVNQAGQSEPSEPSDMIMAKPRYL
PPKIITPLNEVRIKCGLIFHTDIHFIGEPAPEATWTLNSNPLLSNDRSTITSIGHHSVVHTVNCQRSDS
GIYHLLLRNSSGIDEGSFELVVLDRPGPPEGPMEYEEITANSVTISWKPPKDNGGSEISSYVIEKRD
LTHGGGWVPAVNYVSAKYNHAVVPRLLEGTMYELRVMAENLQGRSDPLTSDQPVVAKSQYTVP
GAPGKPELTDSDKNHITIKWKQPISNGGSPIIGYDIERRDVNTGRWIKINGQPVPTAEYQDDRVTS
NHQYQYRISAVNAAGNGKTSEPSAIFNARPLREKPRFYFDGLIGKRIKVRAGEPVNLNIPISGAPTP
TIEWKRGDLKLEEGKRISYETNSERTLFRIDDSNRRDSGKYTVTAANEFGKDTADIEVIVVDKPSPP
EGPLSYTETAPDHISLHWYSPKDDGGSDITGYIIEFTEFGVDDWKPVPGTCPNTNFTVKNLVEGKK
YVFRIRAENIYGASEALEGKPVLAKSPFDPPGAPSQPTISAYTPNSANLEWHPPDDCGGKPITGYI
VERRERGGEWIKCNNYPTPNTSYTVSNLRDGARYEFRVLAVNEAGPGHPSKPSDPMTAEHQRY
RPDPPEPPKPDRITRNGVTLSWRPPRTDGKSRIKGYYVEMRPKNGKDWKTVNDIPINSTVYTVPS
LKEGEEYSFRVVAENEVGRSDPSKPSQPITIEEQPNKPCMELGKVRDIVCRAGDDFSIHVPYLAFP
KPNAFWYSNDNMLDDNNRVHKHLTDDAASVVVKNSKRADSGQYRLQLKNTSGFDTATINVRVLD
RPSPPTRLRADEFSGDSLTLYWNPPNDDGGSAIQNYIIEKKEARSSTWSKVSSFCTVPFVRIRNLV
LNKEYDFRVIAENKYGQSDPANTSEPILARHPFDIPNTPGIPHGIDSTEDSITIAWTKPKHDGGSPIT
GYIIEKRLLSDDKWTKAVHALCPDLSCKIPNLIENAEYEFRVAAVNAAGQSAYSGSSDLIFCRRPPH
APKITSDLSIRDMTVIAGDEFRITVPYHASPRPTASWSLNGLEVIPGERIKFDSNDYASMYYNKSAK
RDETGSYTITLTNNKGSDTASCHVTVVDRPLPPQGPLNAYDITPDTCTLAWKTPLDDGGSPITNYV
VEKLDNSGSWVKISSFVRNTHYDVMGLEPHYKYNFRVRAENQYGLSDPLDIIEPIVAKHQFTVPDE
PGQPKVIDWDSGNVTLIWTRPLSDGGSRIQGYQIEYRDILNDSSWNAYDYIIKDTKYQLYNLINGSE
YEFRIKAKNAAGLSKPSSPSLRFKLKGKFTVPSPPGAPQVTRVGKNYVDLKWEKPLRDGGSRITG
YIIERRDIGGAVWVKCNDYNVLDTEYTVMNLIEMGDYEFRVFAVNSAGRSEPSLCTMPIKVCEVLG
GKKPDWITRLQDKVAPFGKDYTLQCAASGKPSPTARWLRNGKEIQMNGGRMTCDSKDGVFRLHI
SNVQTGDDGDYTCEAMNSLGFVNTSGYLKIGSPPIINRCPSELKLPEGDNSKIKIFYSGDQPLTVIL
KKNNEVICDSNDDTHVKVNIFDDYVAIYIRNIVKSDGGPYQIEFTNESGSATGEFYVHITGMPSAPT
GPMGISYINKNSCMLNWRPPSYDGGLKVSHYVIERKDVSSPHWITVSSTCKDTAFNVQGLIENQE
YIFRVMAVNENGMGPPLEGLNPIRAKDPIDPPSPPGSPQITEIGGDFVHLEWEKPESDGGAHIQGY
WIDKREVGSNTWQRVNATICAANQINCINLIEGRQYEFRIFAQNVAGLSTESSASQAVKIIDPQAAS
PPLIVKPLRDANCIQNHNAQFTCTINGVPKPTISWYKGAREISNGARYHMYSEGDNHFLNINDVFG
EDADEYVCRAVNKAGAKSTRATLAIMTAPKLNVPPRFRDTAYFDKGENVVIKIPFTGFPKPRIHWV
RDGENIESGGHYTVEVKERHAVLIIRDGSHLDSGPYRITAENELGSDTAIIQVQISDRPDPPRFPLIE
SIGTESLSLSWKAPVWDGCSDITNYYVERREHPLSSWIRVGNTRFTSMAVSGLTPGKEYDFRIFA
DNVYGRSDASDTSTLIKTKESVKKKPIERKWEIDANGRKLRGKADGPVKDYDSYVFDIYSKFVPQP
VEISQQSVYDRYDILEEIGTGAFGVVHRCRERSTGNIFAAKFIPVSHSVEKDLIRREIDIMNQLHHQK
LINLHDAFEDDDEMILILEFLSGGELFERITAEGYVMTEAEVINYMRQICEGIRHMHEQNIIHLDIKPE
NIMCQTRSSTNVKLIDFGLATRLDPNEVVKITTGTAEFAAPEIVNREPVGFYTDMWATGVLSYVLLS
GLSPFAGDNDVQTLKNVKACDWDFDVESFKYISEEAKDFIRKLLVRNKEKRMTAHECLLHPWLTG
DHSAMKQEINRDRYLAYREKLRRKYEDFERFLLPIGRLSEYSSLRKLLMEKYKIHDAVFDRRQAAP
RFVIRPSSQFCYEGQSVKFYCRCIAIATPTLTWSHNNIELRQSVKFMKRYVGDDYYFIINRVKLDDR
GEYIIRAENHYGSREEVVFLNVQPLPKEQPRYRTESTPVRRREPLPYTFWQEESETAPSFTFLLRP

RVMQARDTCKLLCCLSGKPVPNVRWYKDGRELSKYEYAMTHSDGVVTMEIIDCKPSDSGKYSCK
ATNCHGTDETDCVVIVEGEWVTPEQAQLAHNFLYSGDRKYIEQPIKPAPLPIVTSRQYTSSSVQNT
SEPQGDKVNVSNSNSSGISNKKKYASNSLQAPGSPSRSRSATKELILPPDDSLMCKPEFTKPLHD
LTIHDGEQLILTCYVKGDPEPQISWSKNGKSLSSSDILDLRYKNGIATLTINEVFPEDEGVITCTATN
SVGAVETKCKLTIQPLDKNINKRKVNAGDNAPKIVSHLESRFVRDGDAVNLACRIIGAQHFDVVWL
HNNKEIKPSKDFQYTNEANIYRLQIAEIFPEDGGTYTCEAFNDIGESFSTCTINVTVPGDETKQPSF
VKFPTSVSVLEGEGTTFECEIDSELLNLVWLKDGKPIDETLPRYSFTKDGHRYSFAVAKCNMDDV
GQYQAKAVSGKAESICAFSMNVHTAES (SEQ ID NO:190)

# Figure 85

**Name:** CG8795
**Nucleotide:** AE003699
**GI:** 10726505
**Transcript:** CT25350
**Sequence:**

atgctgcccactaacagttccggtgtcctggcaaccgatctacaactgtttcacaatgagaaatttctcttaaatctgaca
caagtgctgaacatctccgcggacaacctgaccagccttctccagggcctggagccggaggagcttttgcccacggtgaccccgatgac
accactttcactgctggccaccctaagcgtgggctacgccctcatatttatcgccggcgttttgggcaacctcatcacatgcattgtcatttcgc
ggaacaactttatgcacacggccaccaacttttatctgtttaacctcgctatatccgacatgattttgttatgctcaggaatgccgcaggacctc
tataacctctggcacccggataattatcctttcagtgacagcatctgcatattggagagcgttctctcggaaacggcggccaatgcgacagt
tctaaccattaccgcgttcacagtcgaacgatatattgccatttgtcatccgttcaggcagcacacgatgtccaagttgtcacgggccgtaaa
gttcatatttgccatctggatagctgccctttttgctggccctgccccaagccattcagttctccgtggtgatgcagggcatgggaacatcgtgc
acgatgaaaaacgactttttgcccatgtgtttgctgtgtcgggcttcctgttctttggcggacccatgacggccatctgcgtgctctatgtcctca
tcggggtgaagttgaaacggagccgactcctgcaggcgcttcccaggcgatgttacgatgtaaaccgggggataagcgcccaaacgc
gagtcatccggatgctggtggcggtggccgtggccttcttcatctgctgggcccccttcacgcccagcggctgatggcggtctatggatcc
acctcgggcattgagtcccagtggttcaacgacgtgttcagcatcctcgactatacgtccggtgttctctacttcctctcaacttgcatcaaccc
gctgctctacaacatcatgagccacaagtttcgtgaggcttttaaggtaactttggctagacactttggacttggtggcaaaaatcagggaa
gggggcttcctcatacttacagcgctcttcgacgtaatcaaacgggttcactgcggctgcacacaacggacagcgttcgcaccacaatga
cttccatggcgacgaccaccactgggctaaatggctctgccaatggcagtggcaatggaacgacaacgggtcagtcggtgcgcctgaa
ccgcgtgtccttggacagtgtccagatgcagggtcagaatcgcagccggcaagacctcttcgataatccgcgtcgcatgctccagacgc
aaatatcgcagctgtcatcggtgggcgatgcccattccctcttggaggaggatttgcagtttcccggggagccactgcagcgccagccca
cgatgtgctccattgacgagctcacagatgacttggccatctcgcggtcacgccttaaacttacgcgcatcacccgcccaccaggaggtgt
gacggggggcgtggcaggaggcagcacaacaggggccgcggggtcgggggggcgtgagcggtgacgagtcgagtggcaaagtgc
gcaaggcgaaagtgaaagtgctcaagagctcaagcccgttcaagggtctcaggaccaaattcaattggcgtgccagacgcaaaggca
gccacaaaccgcacgaaaagggggcgacggtgaatggaggcgacactgaagagcgggccgccttttaa (SEQ ID NO:191)

**Protein:** AAF54929
**Protein GI:** 7299748

MAVKMLPTNSSGVLATDLQLFHNEKFLLNLTQVLNISADNLTSLLQGLEPEELLPTVTPMT
PLSLLATLSVGYALIFIAGVLGNLITCIVISRNNFMHTATNFYLFNLAISDMILLCSGMPQDLYNLWHP
DNYPFSDSICILESVLSETAANATVLTITAFTVERYIAICHPFRQHTMSKLSRAVKFIFAIWIAALLLAL
PQAIQFSVVMQGMGTSCTMKNDFFAHVFAVSGFLFFGGPMTAICVLYVLIGVKLKRSRLLQALPR
RCYDVNRGISAQTRVIRMLVAVAVAFFICWAPFHAQRLMAVYGSTSGIESQWFNDVFSILDYTSGV
LYFLSTCINPLLYNIMSHKFREAFKVTLARHFGLGGKNQGRGLPHTYSALRRNQTGSLRLHTTDSV
RTTMTSMATTTTGLNGSANGSGNGTTTGQSVRLNRVSLDSVQMQGQNRSRQDLFDNPRRMLQ
TQISQLSSVGDAHSLLEEDLQFPGEPLQRQPTMCSIDELTDDLAISRSRLKLTRITRPPGGVTGGV
AGGSTTGAAGSGGVSGDESSGKVRKAKVKVLKSSSPFKGLRTKFNWRARRKGSHKPHEKGATV
NGGDTEERAAF (SEQ ID NO:192)

# Figure 86

**Name:** CG10967
**Nucleotide:** AE003540
**GI:** 10727955
**Transcript:** CT30713
**Sequence:**

ccaccacttcgcttagttgtgttttcgtcttgccttttccactagtctctctcgcgaagtagttggaaaaatcaccttttgcaaa
gtttttcgctgggaccaaaaagctacatttttcaaaaaccgaaaggaaattacgctgatcgtccgtgtgtgcgtgcgtgtgtatgtgagagag
tgtgtgccatcgatcctgcaagtgtgtgtttgtacgcgagtgtggggccgcaaattaaaagcaataccacactgaaaaccgagataatcgc
acacggaaaagaacggtttaattcaattacaagtgaaattagagtgtgtgcccgataattgtcaactggccaggtggacggaaaagacg
gtggtggtgttacgtgaaaatttcggagatcgtcgagggccagagaatcacatttagtggctggccaaaggggaaagaaattgattttcca
ttttccacaattgcaattggaagcaaaagcaaagcagaagagcgaagaaaataaaaggggagagcaagcgtgcgtgcacgagcga
aagagagtgtggagcgcgagggggtgtacgactgtgagagagagagagggagagtgggagagagcagcacttccaggcgtcgcat
cctcctcctttttcgcatgcaatgaatattgtcggggaatatgaatacagctccaaggatatgctgggccatggcgcctttgccgtcgtctaca
aaggacgtcatcgcaagaaacacatgccggtggccatcaagtgcatcacgaagaagggacaactgaagacgcagaatctgctcggc
aaggagatcaagatcctgaaggagctcaccgagctgcaccatgagaatgtggtggctctgctggactgcaaggagtcccaggattgcgt
cagcctggtcatggagtattgcaatggcggcgacttggcggattatctgagtgtcaaggggacgctcagcgaggataccgttagactcttc
ctcgtgcaactagctggtgctatgaaagcactttataccaaaggaattgtgcatcgtgatctcaagccacaaaacattttgctatcgcacaat
tatggcaaaacattgccagctccatcgaaaataaccctgaaaattgcggattttgggtttgcgcgattcctgaacgagggcgccatggcag
ccactctgtgcggctctcccatgtatatggcgcccgaggtgatcatgtcgctgcagtacgactccaaggcggatctctggtcgctgggaac
gattgtctaccagtgtttgactggcaaggcgccattctatgcacaaacgcccaacgaactgaagtcctactacgagcagaatgctaacctg
gcaccaaaaattccaagtggcgtgtcaccggatctgagggatctgctgctgtgcctgctgcgccgcaactccaaggatcgcatctcgtac
gagagcttctttgttcaccgcttccttcagggcaagaaggcggccgtgtcgccagttgacatgccaccgcttggcggaacaccacctgcca
aggccaagagccccttgcagcagcagctggagcaggaactgaagctggtcaagctggccgagcaacagcagaaggagcgcgagg
agcaggaggcccaggaggacgagaacacggtgtccgtggtggccaatccggccatttgtgccaccatcaccaacgtaggtgtcctgtg
cgacagtgagaacaacagcggttcttgcagctcccacgaggactccgatgactttgtgctggtgccaaagaaccttccggaggaccagc
gacagggtctggcccaagtccaagcccagcccgcttccggtggccaacgtccacagcagcaacaaaatcaatccagtccgccacgtc
ccagcagtctgcccatctctgaacccaaaccagtaccggctcccgctcggcggcaggtggccaggcccgggccgttaacggtagccac
actgggtggccagcagataccccgatcgcagccaattagcgtaaagcaaccgcgaccggatcagcgcaagagcagtgtgagcagtg
acattaactcgatctcaccgccggcggtgcagtttgccattggaacgccgcctacacgtatgcgttctgcctctggtggatcgctgtcggag
acaccgccgccgcatgcaccgagcacctggcaggtgtcgccaggacactcgcaatcgccgttgcgtcgaagcggcaatagctcgcca
gttctgccatccgctgcgcttaccaaattgcccactttgggcagtccaacaatgctggtggcgccgggatcattgggctcgattggttcggcg
ggcagtggatcggagaacaacaatcagcatcacatgctgggaccgcgagcttttacgctgcccgaactgggggccactggtggcctac
atagtctgctggacacaggagccggcggcggtggggaaccacatgcattccaggccccagaactgtccgaagagacgctgatggatc
gcgagcacaacgagacattgtccaagctgaactttgtgctggcgctcaccgattgtatccaggaagttgccgattcgcgatgcgcaccgct
ctccacgttcatggtggccggcagtcagtcggcggcgcaagcggcgtcagcggatgcacagcagataccgccgcacgcacctgagca
ctgcaaacgggccgagcgcctggtgctgctcgtccggggactgcagctcctatcgtccgggatgaatctggcttcccagcagctaagca
atggccagctaaagccgtcgtccaatgtgaagaatgctttgctcaccatgaacgccaaataccggagcatgctgttcgaatcgaaacgcc
tcaatggcagcggactattgcagaaggcgaatgcatttaacataacggcggacaagattctctatgactacgcactggatatgtgccagg
ccgctgctctggatgagctactgaagaacacgaagaattgcttcgaacgttacaacacggcacacattctgcttcattcgttggtgcaaaa
gtgcaatcatccgcaggacaaaatgatgctgaacaaatatcgcgatgccgttgagaagcgtttgagcatattgcagcagcatggctatat
atacatgaccgatgagaatgcttag (SEQ ID NO:193)

**Protein:** AAF49878
**Protein GI:** 7294537

MNIVGEYEYSSKDMLGHGAFAVVYKGRHRKKHMPVAIKCITKKGQLKTQNLLGKEIKILKE
LTELHHENVVALLDCKESQDCVSLVMEYCNGGDLADYLSVKGTLSEDTVRLFLVQLAGAMKALYT
KGIVHRDLKPQNILLSHNYGKTLPAPSKITLKIADFGFARFLNEGAMAATLCGSPMYMAPEVIMSLQ
YDSKADLWSLGTIVYQCLTGKAPFYAQTPNELKSYYEQNANLAPKIPSGVSPDLRDLLLCLLRRNS
KDRISYESFFVHRFLQGKKAAVSPVDMPPLGGTPPAKAKSPLQQQLEQELKLVKLAEQQKEREE
QEAQEDENTVSVVANPAICATITNVGVLCDSENNSGSCSSHEDSDDFVLVPKNLPEDQRQGLAQ
VQAQPASGGQRPQQQQNQSSPPRPSSLPISEPKPVPAPARRQVARPGPLTVATLGGQQIPRSQ
PISVKQPRPDQRKSSVSSDINSISPPAVQFAIGTPPTRMRSASGGSLSETPPPHAPSTWQVSPGH
SQSPLRRSGNSSPVLPSAALTKLPTLGSPTMLVAPGSLGSIGSAGSGSENNNQHHMLGPRAFTLP
ELGATGGLHSLLDTGAGGGGEPHAFQAPELSEETLMDREHNETLSKLNFVLALTDCIQEVADSRC

APLSTFMVAGSQSAAQAASADAQQIPPHAPEHCKRAERLVLLVRGLQLLSSGMNLASQQLSNGQ
LKPSSNVKNALLTMNAKYRSMLFESKRLNGSGLLQKANAFNITADKILYDYALDMCQAAALDELLK
NTKNCFERYNTAHILLHSLVQKCNHPQDKMMLNKYRDAVEKRLSILQQHGYIYMTDENA (SEQ ID
NO:194)

# Figure 87

**Name:** CG3809
**Nucleotide:** AE003694
**GI:** 10726480
**Transcript:** CT12693
**Sequence:**

tcacatcaatcaaattaaacagttcgatacagtaaaacagcatgaacgacaaataggcgtagaatccatcaggtgta
ggagttctcaagaatcggcgagggtctaatattgggaatgtccaggcgggtttttggcctcagtataaaatcattccgattgttccgaagaac
tccgatccatcccccagcattccagcatttttctggtggcgtctttcgaacttcctttaggagtttctgcgattgtcaggaacaccgaagaatgg
atctaccagagggaatacttatgggctttggcaaccccctgctggacatcacctgcaccgttgaggataatgtgatcctggagaagtacgg
cctggaagcaaatgctgcgatcattgctgatgaaaagcacgatgccctgtttgatgagttgatgaacatggaaaatgtcatctattcggcgg
gtggagcctgtcagaactcgatgcggatcttccagtggatcgtgcagacaccatttcgagctgtgttcatagggtcagtggcaaggataa
gctaggtgatcgcatcgaaaagagggccaaatccgatggcctgctaacactttatcagctgaaggaagagctgccgacaggctcttgtg
ctgtgatcatcaacggtccaaaccgctcattagtggccaacttgggcgctgcctctctcttcagcgacgactggattgacgaggatgataac
atctgcgccttggatcgcgccgagtacttctactttacgggcttctttctggccgtttgtccacctgccgtggagcgggtagctaggatgtgctg
cgagaccaatcgaattatgatcctcaacttcagcgccgtgtttgtgctgcagatgcaaaaggaagccttgggcaatatcctgcagtacgtg
gatataatcatctgcaataaggaagaggccattgcatttagcgacaccaacgactggaagacgaagaatatctttgagatcggatctagg
ctgcagcaaatgcccaaggaaaatacccgaccaagactagtaatgattacggatgcggtgtgtcctgttctcgtcttccaggataacgatc
gcgttctggaataccccgttccacccgtaaagcagggcgagattttcgacaccaatggttgtggcgacgccttcgtcggcggattcctggc
catgtacgtccaaaggatgcccctggattactgcatccgaacggggatttcgcatcccagcaggtcctacacgtggtcggtgttcagatcg
ataagctgccaaagtttagcgagaaatgcatatga (SEQ ID NO:195)

**Protein:** AAF54757
**Protein GI:** 7299571

MSRRVFGLSIKSFRLFRRTPIHPPAFQHFSGGVFRTSFRSFCDCQEHRRMDLPEGILMGF
GNPLLDITCTVEDNVILEKYGLEANAAIIADEKHDALFDELMNMENVIYSAGGACQNSMRIFQWIVQ
TPFRAVFIGSVGKDKLGDRIEKRAKSDGLLTLYQLKEELPTGSCAVIINGPNRSLVANLGAASLFSD
DWIDEDDNICALDRAEYFYFTGFFLAVCPPAVERVARMCCETNRIMILNFSAVFVLQMQKEALGNI
LQYVDIIICNKEEAIAFSDTNDWKTKNIFEIGSRLQQMPKENTRPRLVMITDAVCPVLVFQDNDRVL
EYPVPPVKQGEIFDTNGCGDAFVGGFLAMYVQRMPLDYCIRTGIFASQQVLHVVGVQIDKLPKFS
EKCI (SEQ ID NO:196)

# Figure 88

**Name:** Ack
**Nucleotide:** AE003480
**GI:** 10727290
**Transcript:** CT34843
**Sequence:**

aaaaataccaaaagtgtttggccgtcgcgcattttcaattgcaaattaagcgcagctaactgcgattaacgacgatgttt
ccgcattagaaagaaaatcaagctgatttcagcgagttaacatattgaaaaagtaaagaattctcgctcttttcggttcttcgtggcacctgc
gatttgcgttgctgttttttcgtgcatttcgattcgtacacacgcacacatacacccagacccaaacggccgtgcagcgaatgcgtgtgtgtgt
gaaattgtgcaaaaacgtgaaacgatcgagtgggatggtcggtgggggaggggctgagcgctgttgaaagcggaatattcattgttttctc
gtgtgtgcactataaattaatatttacgtgtctgcgtcatgaggggaccactaaccgcccgccatcaaaatcagcagccaagcagacagc
tgctcatttggcgagtgtaaacaaagcagccaacacacacacacacacacgcacgcatacaatcgtttacattgcggcgactgcgacg
ccaactgcgctgtcttcaactcgaatatcgaatttcacatctttaagccagaaatctccatatccaccgcataaaccacttccatctgccgca
acatcttttgtctggacaacctgttcgcttacagacaggaagtttgccaattttctcaagtttagccaagtgcagcgactgcgacgtccgcgtt
gacgtcgacgctcctcagccagcgtcgcagcagctgcgcagctgtttaatattcggaaatgacctccacttcggcggtggatggtgggctc
gggagcgagacggcctggctggaggatctgctgcgggaggtgcagctggagcagtttctggacaggatacgggacgacctacaggtg
acccggctggcgcacttcgactacgtgctacctgacgatctggagcgatgtggtctggggaaaccggctattcgacggctgatggaggc
agtgcggaaaaagaaggcccaccagtggcgcaagaacatcctgtccaaactaattggcggtggcaagcaaccctcgtccaagaagc
agtcctccgccgcccgggaatccagtcagggcaatggcacccagcttacctgccttatccacgaaaaggacatcacaatgggactgaa
actgggcgacggatcattcggtgtggtgcgtcgcggcgaatggagtgcttcgccggcgggcaaggtcatcccggtggccgtcaaggtgc
tgaagtcggacaacctcacccaaccgggcatcatcgatgacttcttccgcgaggttcaggctatgcacgccctggatcatgcgaatctggt
gcggctttacggcgtcgtcttatcccagccgatgatgatgattacagagctggcggagcgcgggtcgctgctggatacgttgcgaaaaca
atgccgccacacctcgctgaccatcatctggaactggtcggtgcagattgtgactggcatggcctatctggaacagaagcgctttctacatc
gcgatctggcatgtcggaatgtcctcctggcagctggaaacaagatcaagatcggagattttggcctaatgcgtgccttgccccaagagg
atgattgctatgtgatgtctgagcataagaaggtgccttttccctggtgcgctcctgaatcgctgcgcttccggcagttctcgcacgcctctgac
acctggatgtttggcgtgacgctgtgggagatgtttagctttggcgaagatccatgggtgggactcaatggatcgcagatcctgcgcaaaat
cgatcgcgaaggcgagcgactgcatcagccggatgcctgtccaccggacgtttatgctatgatgctgcaatgctgggataaaactccagc
agagcgacccacttttgctgctctcaaagaatatttggccagtatgtcgcctcctgtgatgcgtgcttcccgaagtcaccacgagtccaagg
gactgcagatcgagcctggcgacacaatcgccattatcgatggacggcacgagctgaaattgataaagggccagaaccagcgaacat
tcgatataggaatctttcccagaaacctactggagcagcgcaaagtgggtgctgccggagatgtggtaatgcgaagcagcgttggaaat
ggttcgtcgtcttcgccattcggcttttgttggggcggagcagctgcaatggccaacggagatgatcgacagcgaaagtgtgcctcgatga
caaaccagccccatgccaaggagcgcaagtccacgtccagcaagcagtttgcgtacaacaaactggttaatgattccgctactggtcttc
agcgacgaaatgcagtgaagcacaaaggagtggtggtgggcccgcagcgtcctccgccgccacaatttcaacaggagggcatactc
atcgacatctcgcccgatatgcgacccatagccgaagctggaacaggaggagctaagggagcaggcgatagttcctcactgcaggcg
gacagctcgttttgcatactagacgctcccatagatgtgcccacttatgccggctccagtggatcgggggatcttaatgtctcgcccacgtac
tacaatgagcagccgcagttcgactttgatccggccaagatgacagcttcaccagggcgcctgcaaccgccgccgtaccagatgccac
caacttactcgaatacaatggagtttgtccaaaaacgagaccttcaccaacagcagctagcgacgcctgtaagggagcgagatcccttc
gacaccacaaacgttgaaacgactgtggcactctactcgaatttcaaccagtctctggaagcagcttctccaccagcaccgatttataata
gtccttcggtcaggaagagccttttcggtggctccaagtcgaataaagaaaacatacctgccctggaatcagcggctatgcagctaaatct
cagtaatcttacattggagcgacacgacgccacctgcattcagccagtggagcctgtacctgctcctcctggtgatggagtactgctggac
aagtccttcatcgccgaactggagaaggacatgtacagcaacggcaaaacagagcgcaggaggagtaccagcgcaattctacgca
gatgtatgccagcaaggatatggtgtataagcagaatctcacacccttaaagaacggtgcagcacccgggtcagttcactcgaatcattc
cagtccctcttcgaccgcgtcgcccaagcagaacaatgtggaggcagcagctgctgcggcagctaccacgcaaagtgtggtgaatcgt
atttggtatgagcaagtggcgtccacgcagtccgagtactatgcccagccgccgacagagcaggcagaggagcagatctaccaaaac
catcgccaccagcagcagcagcagcaggagttgaaccattcgtttgttgcaatatccaatcgagtggtggcacccaaaaacaatgcgta
ctcctcaaccgcctcgctgtacgatgcagtggcggccagcacggcgggctccacatactacggccaagtgccgaatggcagtggagct
gttctgtacgatgaagtgactcaggatgactacctgcggccaacgcgaccagctccattggcgccacctcctctctccgcgcagcagatc
caacgaaggatggagaagatgcggctgcagcaacagcagcagctggacggagcccatcagctgtatgcgccagtgccctcggacta
cggtcgcgagcaggagaagctccagcagctaatgcaagagctgggcagctccgcggtggagcaggatgtgcgcaatgcactgcgtg
cggccagcggagatgttggactagccacgcggcactacaaaatcgaccaactggcgaggcttggggtggctgggcgaccacagtgc
gaacaggcgctgcagcagaccaactggagcttagaggtggcagctgaactactactgaatgccggctaaacccacaaactacaacca
tgtattaccgcctcgaatgtaattgctttagttttacgtgctgaatattcttttaaacttagctaacgatgtatcgaatctttatcaaaatactcctag
gtgaatgcgcgaatgcaaacacattgtcaacgactgtacaatttgttctatttaatgtaagcgatcgtataaaattattttataattgttattcgat
gtccgcattttctgagatgccccgaatattttattcacactcgaaattgaatctttcgaatgaagacctcggttcgcttcggccggttaaggtca
aaaatgtcaaacgtatgcaacaattcatttgtttatcgaataaattatatgaaattgttacaaaa (SEQ ID NO:197)

**Protein:** AAF47839
**Protein GI:** 10727294

MTSTSAVDGGLGSETAWLEDLLREVQLEQFLDRIRDDLQVTRLAHFDYVLPDDLERCGL
GKPAIRRLMEAVRKKKAHQWRKNILSKLIGGGKQPSSKKQSSAARESSQGNGTQLTCLIHEKDIT
MGLKLGDGSFGVVRRGEWSASPAGKVIPVAVKVLKSDNLTQPGIIDDFFREVQAMHALDHANLVR
LYGVVLSQPMMMITELAERGSLLDTLRKQCRHTSLTIIWNWSVQIVTGMAYLEQKRFLHRDLACR
NVLLAAGNKIKIGDFGLMRALPQEDDCYVMSEHKKVPFPWCAPESLRFRQFSHASDTWMFGVTL
WEMFSFGEDPWVGLNGSQILRKIDREGERLHQPDACPPDVYAMMLQCWDKTPAERPTFAALKE
YLASMSPPVMRASRSHHESKGLQIEPGDTIAIIDGRHELKLIKGQNQRTFDIGIFPRNLLEQRKVGA
AGDVVMRSSVGNGSSSSPFGFCWGGAAAMANGDDRQRKCASMTNQPHAKERKSTSSKQFAY
NKLVNDSATGLQRRNAVKHKGVVVGPQRPPPPQFQQEGILIDISPDMRPIAEAGTGGAKGAGDSS
SLQADSSFCILDAPIDVPTYAGSSGSGDLNVSPTYYNEQPQFDFDPAKMTASPGRLQPPPYQMPP
TYSNTMEFVQKRDLHQQQLATPVRERDPFDTTNVETTVALYSNFNQSLEAASPPAPIYNSPSVRK
SLFGGSKSNKENIPALESAAMQLNLSNLTLERHDATCIQPVEPVPAPPGDGVLLDKSFIAELEKDM
YSNGQNRAQEEYQRNSTQMYASKDMVYKQNLTPLKNGAAPGSVHSNHSSPSSTASPKQNNVEA
AAAAAATTQSVVNRIWYEQVASTQSEYYAQPPTEQAEEQIYQNHRHQQQQQQELNHSFVAISNR
VVAPKNNAYSSTASLYDAVAASTAGSTYYGQVPNGSGAVLYDEVTQDDYLRPTRPAPLAPPPLSA
QQIQRRMEKMRLQQQQQLDGAHQLYAPVPSDYGREQEKLQQLMQELGSSAVEQDVRNALRAA
SGDVGLATRHYKIDQLARLGVAGRPQCEQALQQTNWSLEVAAELLLNAG (SEQ ID NO:198)

# Figure 89

**Name:** Abl
**Nucleotide:** AE003526
**GI:** 10727878
**Transcript:** CT13380
**Sequence:**

atggggctcagcagggcaaggacaggggcgcccactcgggaggaggcggctcgggggcccccgtcagctgc
atcggcctctccagcagcccagtggcctccgtttccccccactgcatctccagttccagcggcgtcagcagcgccccccctcggcgggggc
tccacgctccgtggctcccgcatcaagtcctcgtcctccggcgtggccagtggcagcggatcgggcggaggcggtggcggatccggatc
ggggctgagccagcgtagtggcggacacaaggatgcacgctgcaatcccaccgtgggtctcaacatattcaccgagcataacgaagc
cctgctgcagtcgcgtccattacctcacattccggccggcagcacggcggcctctcttctggcggatgccgctgagctgcagcagcatca
gcaggattccggtggactgggactgcagggctcctccctgggcggtggtcacagttcgactacatccgtgtttgaatccgcccatcggtgg
acctcgaaggagaacctactggcccccggacccgaggaggatgatccgcaactttttgtggcgctatacgatttccaagccggcggaga
gaatcaattgagtctgaagaaaggcgagcaggtgcgcatacttagctacaacaaatcgggggagtggtgcgaggcgcactcggactc
cggaaacgttggatgggtgccctccaactatgtcacgccgctcaattcgctggagaagcactcctggtaccacgggcctatctcacgcaa
cgccgccgagtatcttctcagctccggaatcaatggcagtttcctggtccgtgaaagtgaaagttcaccgggtcaaaggagcatcagtttg
agatacgagggtcgcgtctatcactaccgcatctcagaggatcccgatgggaaagtcttcgttacccaggaggccaagtttaacactctg
gccgagctggtgcatcatcacagcgtgccccatgagggtcacggcctgatcacaccgctcctatatccggcgcccaagcagaacaagc
ccaccgtctttccgctgagtcccgagccggatgagtgggagatctgccggacggacatcatgatgaagcacaagctaggcggcggtca
gtacggagaggtctacgaggccgtttggaagcggtatggcaatacggtggctgttaaaacgctcaaggaggacaccatggcactgaag
gacttcctcgaagaggcggccatcatgaaggaaatgaagcaccctaatctggtgcagctcattggtgtttgcaccagagaaccaccgttc
tatatcatcaccgagtttatgtcgcacggcaatctgttggacttcctgcgctccgccggacgcgaaacgctcgatgcagtagcgttgctgtac
atggccactcagatagcgtcgggaatgagctacctggagtcgcgcaactacattcatcgcgatctcgctgcccgcaattgcctggtgggtg
acaacaagctggtcaaggtggcggatttcggcctggcacgtttgatgcgggacgacacgtatacagcacatgccggagccaagttccc
gatcaaatggaccgcaccggagggtctggcctacaacaagtttagcaccaaatcagacgtttgggccttcggagtcttgctgtgggaaat
cgccacgtatggaatgtcaccgtatccggccatcgatctgaccgatgtgtaccacaagctagacaagggctatcgcatggagcgaccgc
caggctgcccgccggaggtgtacgacttgatgcgccagtgctggcagtgggatgccaccgacaggcccacgttcaagagcatacacc
atgcgctggagcacatgtttcaggaatcgtccatcaccgaagcggtggaaaagcagctgaacgccaacgccaccagcgcgagcagct
ccgctccgagcacatcgggcgtggccaccggcggaggagccacaaccacgacggcggccagcggctgcgcttcctcatcctcggcc
accgcctcgctaagtctcacaccgcagatggtgaagaagggtttacccggcggtcaggccctcacgccgaacgcccaccacaacgat
ccgcaccagcagcaggccagcacgcccatgtcagaaaccggatccacttccaccaagctaagcactttctccagccagggcaaggg
caatgtccagatgcgtcgcaccaccaacaagcagggcaaacaggcgcccgccccaccaaagcgaaccagcctgctctcgagcagt
cgggactccacttatcgcgaggaggatccagccaacgccagatgcaatttcatcgacgacctcagcacgaatggactagcccgggac
atcaacagtttgacgcagcggtacgattccgaaacagatccggcagccgacccggacacagatgccacgggcgatagtctggagcag
agtctgagccaagtgatagccgctccggtcaccaacaagatgcagcattctcttcacagcggaggaggaggaggaggaataggtcctc
gatcctcgcagcaacacagctccttcaagcgaccgactggaacacccgtgatgggtaaccgaggactagagacccggcagagcaag
cggtcccagctccattcacaggctccgggtccaggaccgccatcaactcaaccgcatcatggcaacaacggcgtggtgaccagtgctc
atcccatcactgtgggtgcgctcgatgtgatgaatgtcaagcaggtggtgaaccgctacggaacactaccaaagggtgccagaatcggt
gcctatctggacagtcttgaggatagcagtgaggctgctcctgctcttccggcaactgctccttctctgccaccagccaatggacacgccac
gcctccggctgccagactaaatccgaaggccagccccattccgccacagcaaatgatcaggagcaactcgtcgggcggagtgaccat
gcaaaacaatgcggctgccagcttaaacaagcttcagcgtcatcgcaccactaccgaaggcaccatgatgacgttctcctccttccgggc
gggcggttccagtagctcacccaagcggagtgcctcgggagtggcttcaggagtccagccagctctggccaaccttgagtttccaccgcc
tccgttggacttgcctccgccgcccgaggaattcgaaggcggaccaccacctcctccaccggcgccggagagcgctgtgcaggccatc
cagcagcacttgcatgcccagctgccaaacaatggcaacattagcaatggaaacggaacaaacaacaacgacagcagccacaacg
atgttagcaacatagctcccagtgtggaggaggccagctccagattcggtgtgtctctgagaaaacgcgagccctccaccgactcctgca
gctcgctgggcagtccacccgaggatctcaaggaaaagctaatcaccgaaatcaaggcggccggcaaggatactgctccggcctcac
atctggccaacggctcgggcatcgctgtcgtggaccccgtctccctgcttgtcaccgaactagccgagagcatgaacctgccaaagccg
ccgccgcagcagcagcaaaagctgaccaacggcaatagtactgggtccggattcaaggctcagctgaagaaagtcgagcccaaaa
agatgagcgcgccaatgcccaaggcggagccggcaaataccatcatcgacttcaaggctcatctgcgccgggtggacaaggaaaag
gagccagcaactccagctccagctccagctactgtagccgtagccaacaatgccaactgcaatacaacgggcactttgaaccggaag
gaggacggcagcaagaagttctcgcaggccatgcaaaagactgaaatcaaaatcgacgtaacaaactcgaatgtggaggcggatgc
gggagcagcgggcgagggcgatctcggcaagcggcgaagcacagatgacgaggagcagtcgcacacggagggcctggatcgg
gaggccaaggatcagcagacatgacccagtcgctgtacgagcagaagccacagatccagcaaaagccagcggtgccgcacaagc
caacaaagctaaccatctacgccacgcctattgccaaactgaccgaaccagccagctccgccagctccacacagatatcacgggaga
gcattctggagctggttggcctgctagagggctcgctcaagcacccggtgaatgccatcgctggatctcagtggctgcagctgagtgaca

agctcaacatcctgcacaattcgtgcgtgatcttcgcggagaacggagcgatgccgccgcactccaagttccagttccgggagctggtca
cgcgcgtggaagcacagtcgcagcacttgcgctccgccggcagcaagaacgtccaggacaacgagcgcctggtggccgaagtcggt
cagtcgctgcgtcagatctccaatgcgcttaacaggtaa (SEQ ID NO:199)

**Protein:** AAF49431
**Protein GI:** 7294076
MGAQQGKDRGAHSGGGGSGAPVSCIGLSSSPVASVSPHCISSSSGVSSAPLGGGSTLR
GSRIKSSSSGVASGSGSGGGGGGGSGSGLSQRSGGHKDARCNPTVGLNIFTEHNEALLQSRPLP
HIPAGSTAASLLADAAELQQHQQDSGGLGLQGSSLGGGHSSTTSVFESAHRWTSKENLLAPGPE
EDDPQLFVALYDFQAGGENQLSLKKGEQVRILSYNKSGEWCEAHSDSGNVGWVPSNYVTPLNSL
EKHSWYHGPISRNAAEYLLSSGINGSFLVRESESSPGQRSISLRYEGRVYHYRISEDPDGKVFVT
QEAKFNTLAELVHHHSVPHEGHGLITPLLYPAPKQNKPTVFPLSPEPDEWEICRTDIMMKHKLGG
GQYGEVYEAVWKRYGNTVAVKTLKEDTMALKDFLEEAAIMKEMKHPNLVQLIGVCTREPPFYIITE
FMSHGNLLDFLRSAGRETLDAVALLYMATQIASGMSYLESRNYIHRDLAARNCLVGDNKLVKVAD
FGLARLMRDDTYTAHAGAKFPIKWTAPEGLAYNKFSTKSDVWAFGVLLWEIATYGMSPYPAIDLT
DVYHKLDKGYRMERPPGCPPEVYDLMRQCWQWDATDRPTFKSIHHALEHMFQESSITEAVEKQ
LNANATSASSSAPSTSGVATGGGATTTTAASGCASSSSATASLSLTPQMVKKGLPGGQALTPNA
HHNDPHQQQASTPMSETGSTSTKLSTFSSQGKGNVQMRRTTNKQGKQAPAPPKRTSLLSSSRD
STYREEDPANARCNFIDDLSTNGLARDINSLTQRYDSETDPAADPDTDATGDSLEQSLSQVIAAPV
TNKMQHSLHSGGGGGGIGPRSSQQHSSFKRPTGTPVMGNRGLETRQSKRSQLHSQAPGPGPP
STQPHHGNNGVVTSAHPITVGALDVMNVKQVVNRYGTLPKGARIGAYLDSLEDSSEAAPALPATA
PSLPPANGHATPPAARLNPKASPIPPQQMIRSNSSGGVTMQNNAAASLNKLQRHRTTTEGTMMT
FSSFRAGGSSSSPKRSASGVASGVQPALANLEFPPPPLDLPPPPEEFEGGPPPPPPAPESAVQAI
QQHLHAQLPNNGNISNGNGTNNNDSSHNDVSNIAPSVEEASSRFGVSLRKREPSTDSCSSLGSP
PEDLKEKLITEIKAAGKDTAPASHLANGSGIAVVDPVSLLVTELAESMNLPKPPPQQQQKLTNGNS
TGSGFKAQLKKVEPKKMSAPMPKAEPANTIIDFKAHLRRVDKEKEPATPAPAPATVAVANNANCN
TTGTLNRKEDGSKKFSQAMQKTEIKIDVTNSNVEADAGAAGEGDLGKRRSTGSINSLKKLWEQQP
PAPDYATSTILQQQPSVVNGGGTPNAQLSPKYGMKSGAINTVGTLPAKLGNKQPPAAPPPPPPN
CTTSNSSTTSISTSSRDCTSRQQASSTIKTSHSTQLFTDDEEQSHTEGLGSGGQGSADMTQSLYE
QKPQIQQKPAVPHKPTKLTIYATPIAKLTEPASSASSTQISRESILELVGLLEGSLKHPVNAIAGSQW
LQLSDKLNILHNSCVIFAENGAMPPHSKFQFRELVTRVEAQSQHLRSAGSKNVQDNERLVAEVGQ
SLRQISNALNR (SEQ ID NO:200)

# Figure 90

**Name:** CG7362
**Nucleotide:** AE003706
**GI:** 10726534
**Transcript:** CT22669
**Sequence:**

atgctacctacgggaaacagctttccgaagaataatgggaaaataatgcctctaattatcattaataaatcgagagac
aagtcaacaaacccggccgtcgagtcaacaacttggttgcgcttcaaggcagccaaggaaaaacgtttcctttggccaatggattggat
gacggccaaacaatgcgtccagtttggacaaacatttgcagcagcaaacccatttcatgcccattcccaacttcccacgttgcacggcag
gaacccaagaagtttcggctagaccgtggaccctacctttcccagctggactaccaatctcgtctgcagttccaagcaccagctctgaggtt
acccccttaccagcattatatgcaccattggaccctcatccagccagcccgaggttcttcttaatctcattcacgctgggatgaaggtggtccg
attggacttctccgacggcacccacgattgccattgccaggctatccaggcggctcgtaaagccatcgccatgtatgcggaggaaacgg
gtcttcccaggagcttggccattgccctggacaccaagggtccggtaatcaatccacagggtgtagctgctgatttaaacgccataaccga
acaagacaaactggatctcaagtttggagcggatcaaaaggttgacatgatcttcgcatcgttcatccgcgatgccaaagctttgaaaga
aattcgccaggcactgggtgcctgtccatcaagtgagcacataaagatcatttccaagatcgaaagtcaacaggctctggcgaacatag
atgagataatccgcgaatccgatggcataatggtggcccttgggaatatgggcaacgaaatagcactggaggctgtaccgctggcccag
aaatcgattgtggccaagtgcaacaaggttggaaagcctgtgatctgtgccaatcaaatgatgaattcgatgataaccaagccacgtccc
actcgcgccgaatcctctgatgtggcaaacgcaatcttggatggttgtgatgcccttgtgttgtcagatgaaacggccaagggtaagtaccc
ggtgcaatgtgtgcagtgcatggccagaatatgcgccaaagtcgagtcggttttatggtacgagagcattcagaacaacctcaagagcg
aagtccggatcaacgccgccgatcacatctcggcggtaagcacggcaattgcagaagcggcaacggtgagtcaggctcaggcaata
gtggtggccagtccgtgctccattgtgtcccagatggtcagtcaaatgaggccgccgtgtcctatcgtgttgctcacaggctgcccacatga
ggccgctcaatccttgctttttcgtggcgtttatccactccttgtcaaggaaatggtttatggcagcgtaaactactgtcgcatcatgcaatcgg
gcttaaagatattggccaagttggacatcctgaaggcaggccaaaggggaactttggtgctggtcaatgccatgtcggcagacaagctc
accttcaggctgttcacttttgcacagccaactgaggcagagcgagaagaagaacgctgccgaaagttggccaggaagcagcgatgc
aaggaaatgtctgtaaataaatcctgtccaaatccaaagagcagtaagtctgaaatatctataaagtccgatgaaaaagatggtaccagt
tcaaagaaagaatttcctaaggacgaagaagtttcgaaatgtttacaactagaaaatgaaattcagcagaaacggggtgaagattctgta
atgaacaaagaaaatgaaaatctctgccacaacgtggcattgaagtgcttacatttgaagaatgaaagacaagctcaatcggaagctat
gatgaaacagacggaaaaacttaaaaaagcggaagatcctagaaaaaacaatgggacccttaaggatagttttaaccgtaagaaaa
aagacgataaaacctcgaagtccgataaaaaggatggttccagctcaaagaaagatcgtcctaaccacaaggaagtctggaaatgttt
acagcggagtcaagatttggtaatggcagaagaaaatgaatatctctgttcaaaaaaatcaaaagaatgtcccaataaggacgacgag
gccttgaagtgcttacagctgaaaaaacaaagacaagctcgagcggaagctgagatgaaaaagaaggcagaactaaaaaaaaata
atagtttaagccataagaaagaagacgataaagcaccaaactgcaacaaacttaaaaaacaaggcccaaagaagaataagtgcag
agtagtcaaaatttcgaggacatccgaaaaggataattatattccgaagaaagaatatcctaggatcttcaatatagaaaaggctcagcc
aaaaactgtggtcaaacacagtaaagagttggcaaagtcctgcgatattgtactcgaaaactgcaagaccgtaaaacgcaaaaactgc
aagactgtaaaattagaaaaatgtaagacagtaaaacttgaaaactgcaaaataacaaaacttaagaagtgcaaaacgatcaagctc
gaaaattgtaagcagccgcgaagagttaggcaaagaactaatcttactaaaaaggcatacactttcagtactagagatacgaggaaaa
aaataattccaccaaagcgttatcgcgttgtacgcggctctgcaattgttaaacaactaaaggaaaataaagccagcgagagtccaaca
gctgcgagcagattgtcgagtttcgctgaatgcgatagatttagcgagagagaaaagatacatttacggagagcgaatcgctga
(SEQ ID NO:201)

**Protein:** AAF55096
**Protein GI:** 7299922

MLPTGNSFPKNNGKIMPLIIINKSRDKSTNPAVESTTWLRFKAAKEKRFLLANGLDDGQTM
RPVWTNICSSKPISCPFPTSHVARQPQNSDFFIKFYIYLVCITQIIFFFKIRKYAGNLCQRASLCVVSP
CPNFPLSLRFRCYADKCKRKMEPKKFRLDRGPYLSQLDYQSRLQFQAPALRLPLTSIICTIGPSSS
QPEVLLNLIHAGMKVVRLDFSDGTHDCHCQAIQAARKAIAMYAEETGLPRSLAIALDTKGPVINPQ
GVAADLNAITEQDKLDLKFGADQKVDMIFASFIRDAKALKEIRQALGACPSSEHIKIISKIESQQALA
NIDEIIRESDGIMVALGNMGNEIALEAVPLAQKSIVAKCNKVGKPVICANQMMNSMITKPRPTRAES
SDVANAILDGCDALVLSDETAKGKYPVQCVQCMARICAKVESVLWYESIQNNLKSEVRINAADHIS
AVSTAIAEAATVSQAQAIVVASPCSIVSQMVSQMRPPCPIVLLTGCPHEAAQSLLFRGVYPLLVKE
MVYGSVNYCRIMQSGLKILAKLDILKAGQRGTLVLVNAMSADKLTFRLFTFAQPTEAEREEERCRK
LARKQRCKEMSVNKSCPNPKSSKSEISIKSDEKDGTSSKKEFPKDEEVSKCLQLENEIQQKRGED
SVMNKENENLCHNVALKCLHLKNERQAQSEAMMKQTEKLKKAEDPRKNNGTLKDSFNRKKKDD
KTSKSDKKDGSSSKKDRPNHKEVWKCLQRSQDLVMAEENEYLCSKKSKECPNKDDEALKCLQL
KKQRQARAEAEMKKKAELKKNNSLSHKKEDDKAPNCNKLKKQGPKKNKCRVVKISRTSEKDNYIP

KKEYPRIFNIEKAQPKTVVKHSKELAKSCDIVLENCKTVKRKNCKTVKLEKCKTVKLENCKITKLKK
CKTIKLENCKQPRRVRQRTNLTKKAYTFSTRDTRKKIIPPKRYRVVRGSAIVKQLKENKASESPTAA
SRLSSFAECDRFSEREKIHLRRANR (SEQ ID NO:202)

# Figure 91

**Name:** Cyp9f2
**Nucleotide:** AE003695
**GI:** 7299572
**Transcript:** CT17908
**Sequence:**

acgcttagatcgccaagctagggtcaacatgttgtgggagttcttcgccctatttgcgatcgctgccgccttgttctaccgt
tgggcgtccgccaacaatgatttcttcaaggataggggcatcgcctatgaaaagccagtgctctactttggcaacatggccggcatgttcct
gcggaagagggccatgtttgacatagtctgtgatctctacaccaaaggcggaagtaagaaattttttggtatctttgagcagcgacaacca
cttttaatggtgcgcgacccagatcttatcaagcagataaccataaaggacttcgatcactttatcaatcatcgcaatgtgtttgccaccagc
agcgatgatgatccacatgacatgagtaaccttttcggcagctcgctgttctccatgcgagatgctcggtggaaggatatgcgaagcactct
gagtccggcatttacaggcagcaaaatgcgtcagatgttccagctgatgaaccaggtggccaaggaggccgtggactgtcttaagcagg
atgattcaagggttcaggagaatgaactggacatgaaggactactgcacacgattcaccaatgatgtgatcgcctcgacagcctttggact
gcaagtgaactcgttcaaggatcgcgaaaataccttctatcagatgggaaaaaagttgaccacatttacattcctgcagagcatgaagttc
atgttgttcttcgcattgaagggtcttaataagatccttaaggtcgagcttttcgataggaaaagcactcagtatttcgtgcgtctggtgctcgat
gctatgaaataccgacaggagcacaatattgtacgtcccgacatgataaacatgctgatggaggctcgtggtatcatccagacggagaa
gaccaaggcatctgccgttcgcgagtggagcgatcgcgacattgtcgcccagtgtttcgtgttcttctttgctggcttcgagacttcggcggtg
ctgatgtgcttcaccgctcacgagctaatggagaaccaggatgtgcagcagaggctatacgaggaggtacagcaagtggaccaagac
ctggagggcaaagaactgacctatgaggcaattatgggcatgaagtatctggatcaggtcgtcaacgaggttctgcgaaagtggccggc
ggcaattgccgtcgatcgagaatgcaacaaggatatcaccttcgatgtggatggccagaaagtcgaggtcaagaagggcgatgtcatct
ggctgcctacatgcggcttccatcgtgatcccaagtactttgagaaccctatgaagttcgatccggaacgttttagcgatgagaacaagga
gtctattcagccattcacatacttcccctttggcctgggtcaacgcaactgcattggttcccgattcgctctacttgaagcaaaggctgttatcta
ctatcttttgaaggactaccgctttgcccctgccaagaaatcctgcattccactagagctcattacttccggttttcagttgtcacccaaaggcg
gcttttggattaaacttgttcaaaggaactaa (SEQ ID NO:203)

**Protein:** AAF54803
**Protein GI:** 7299618

MLWEFFALFAIAAALFYRWASANNDFFKDRGIAYEKPVLYFGNMAGMFLRKRAMFDIVCD
LYTKGGSKKFFGIFEQRQPLLMVRDPDLIKQITIKDFDHFINHRNVFATSSDDDPHDMSNLFGSSLF
SMRDARWKDMRSTLSPAFTGSKMRQMFQLMNQVAKEAVDCLKQDDSRVQENELDMKDYCTRF
TNDVIASTAFGLQVNSFKDRENTFYQMGKKLTTFTFLQSMKFMLFFALKGLNKILKVELFDRKSTQ
YFVRLVLDAMKYRQEHNIVRPDMINMLMEARGIIQTEKTKASAVREWSDRDIVAQCFVFFFAGFET
SAVLMCFTAHELMENQDVQQRLYEEVQQVDQDLEGKELTYEAIMGMKYLDQVVNEVLRKWPAAI
AVDRECNKDITFDVDGQKVEVKKGDVIWLPTCGFHRDPKYFENPMKFDPERFSDENKESIQPFTY
FPFGLGQRNCIGSRFALLEAKAVIYYLLKDYRFAPAKKSCIPLELITSGFQLSPKGGFWIKLVQRN
(SEQ ID NO:204)

# Figure 92

**Nucleotide:** AB002369
**Sequence:**
gacgtcccagcggctcaggcgctgcccagcgccggccctggcagggagcctggtggggtggcggaggggggagactgtgccgtggag
ggcctcgccatgtcctgctgcccgacttccttgtgaaacctcctagtagaaaaagttcttgggacaaacttcatttgacttcaccataaggga
aagaagagagccttgttggacactgaagaagggacggggatttctcctcctaatctgggcctcttgtcatggatgaagagactcggcaca
gccttgagtgcatccaggccaatcagatctttcccaggaagcagctgatccgggaggatgagaatcttcaggttcctttccttgaacttcatg
gagagagcacagagtttgtgggccgtgccgaggatgccatcattgccctttccaattacagacttcacatcaagttcaaggagtctcttgtta
atgttccattacagcttatagaaagtgttgaatgccgagatatatttcagcttcatttgacttgcaaagactgcaaagttatcaggtgtcagttttc
aacctttgagcagtgtcaagagtggctgaagagactgaacaacgcaatccgaccacctgctaaaatagaagatctcttctcatttgcatac
catgcttggtgcatggaggtctatgccagtgaaaaagagcaacatggagacctgtgcagaccaggggagcatgtaacttcaaggtttaa
aaacgaggtggagaggatgggttttgatatgaacaacgcctggaggatttccaacatcaatgagaagtacaaattatgtggtagctatcct
caagagctcatagtgcctgcctggatcactgacaaagaactggaaagtgtatcaagtttcaggtcctggaagcgcatccctgccgtcatct
acaggcaccagagcaatggagctgtcattgcccgctgtggacagccagaggttagctggtggggctggcgaaatgcagatgatgagc
atctggtacagtcagtagccaaagcttgtgcctctgactcccgatcgagtggcagcaagctgtcaactaggaacacttctcgagactttccc
aatgggggagacctttctgacgtggagttcgattcttctctgtcaaatgcttcaggagcagagagtttagccatccaaccgcagaagcttttg
atcttggatgcacgctcctatgcagctgctgtggcaaaccgagccaaaggaggaggctgcgaatgcccagagtattacccaaactgtga
agttgtgtttatggggatggcaaacattcattctattcggaggagttttcagtctctgcggttgctgtgcactcagatgccagatccgggaaatt
ggctatcagctcttgaaagcacaaaatggctccatcacttgtctgtgcttctgaaatcagcgcttctggtagtgcatgctgtggatcaggatca
gcggccggtgctagtacactgctcagatggctgggaccgcacccccccagattgtggcattggctaagctcttgctggacccttattaccga
accatagagggtttccaggtcctcgtggaaatggagtggctggattttggccataaatttgctgaccggtgtggtcatggggagaactcgga
tgatctgaatgaacgttgcccagtgtttctgcagtggcttgactgtgttcatcagcttcagaggcaatttccttgctcttttgagttcaatgaagcat
tccttgtgaaactggtgcagcatacctattcctgcctgtttggaacattcctgtgcaacaacgccaaggagagagaggggaaaagcatactca
ggaacggacatgttccgtgtggtcacttcttcgggcaggcaacaaggctttcaaaaacctactgtattcctctcagtcagaagccgtgctgt
accctgtgtgccatgtgcgtaacctgatgctgtggagtgcagtgtacctgccctgcccatccccaaccacccctgtggacgacagctgtgc
accatacccagccccaggcaccagccctgatgatcccccccctgagccggctaccaaagactagatcatacgacaatctgaccacagc
ctgtgacaacacagtgcctctggccagccggcgctgcagcgaccccagcctgaacgagaagtggcaggagcaccggcgctcactag
agctgagcagcctggctggccctggagaggatcccctttctgccgacagcctagggaagcccaccagagtgccggggggtgccgagc
tttctgttgcagccggagtagctgaggggcagatggagaacatcttgcaggaggccaccaaagaggagagtggagtagaggaacctg
cccacagggcaggcattgagatacaggagggtaaagaggaccctctcttagaaaaggagagcaggaggaagacacctgaggcctc
agccattggacttcaccaagacccagaactgggtgatgctgctctgaggagccatctggatatgagctggcctctgttctcacagggcattt
ctgaacagcagagtgggctcagtgttctcctcagttctctccaggtccccccagggggagaggattccctggaggtccctgtggagcagttt
cgaatagaagagattgcagagggtagggaggaagcagttcttccaatcccagtagatgcaaaagttggctatggtacctcacagtcatgt
tctctgctaccttcccaagtccctttgagaccagaggaccaaacgtggacagttctacagacatgttagtggaagataaggtgaagtcagt
aagtgggccccaaggtcatcatagatcttgccttgtaaatagtggcaaggacaggcttcctcagaccatggaacccagcccttcagagac
aagcctggtcgagaggccccaagtggggtctgtggtgcataggacttcccttggcagcactctcagcctgacacgttccccttgtgccttgc
ctttagccgaatgtaaagaggggcttgtgtgcaatggtgccccagagactgaaaacagggcctcagagcagcccccaggtcttagcac
cctccagatgtaccccacacccaatgggcattgcgccaatggggaggctggtaggagcaaggactcactgagccgtcagctgtctgcta
tgagctgcagctctgcccacttacactcaaggaacttgcaccacaagtggctgcatagccactcaggaaggccatctgcaaccagcagc
cccgaccagccttcccgcagccacctggacgatgatggcatgtcagtgtacacagacacgatccaacagcgcctgcgtcagattgagtc
aggccaccagcaggaagtagaaactttgaagaaacaagtccaggagctgaagagtcgcctggagagccagtacctgaccagctccc
tacactttaatggagactttggggatgaggtgacttcaatccccgactcggaaagcaatctggatcagaactgtttgtctcgctgcagcaca
gagattttctctgaagccagctgggagcaggtggataaacaggacacagagatgacccgttggcttcctgaccacctggccgcccactg
ctatgcgtgcgacagtgccttctggcttgccagcaggaagcaccactgcaggaattgtgggaacgtattctgctccagttgttgtaaccaga
aggttccagttcccagccagcagctctttgaacccagtcgagtatgcaagtcttgctatagcagcctacatcccacaagctccagcattgac
cttgaactggataagcccattgctgccacttccaactgaagctcagtgacctgggtgggcagtggccaagctgctgttcctatgacaggcc
cactcaacctgggcagaccgagaggcccgtgcactttggaatgggagcgtggaaccacctgtacagagtgacagatttgggatgcacc
actggattgtagattgatttttctttcctgtcccccctactccctccctaccttttccatcctcctctctgccttcaaaaaaggaaactttcccttggttg
tcttaattttttttttttttgatggaagaccaagggttgccaggcccactgtaactgccgagctgcctgctgtcacgtgacactgagggatggctt
gtttcttccgggtgggaggatggtggtcagagccaggagtatggagatctgagaccgtgagcagggaagaaagccagtgctaacatgc
aaccattcctcacgccaccgccacatcagagctggttgggaaccctttgctgctggggaggctggaagcatattccccaagagcactgcc
ctgggcatcatctccctcctgcgaggagctgagccagtcccctcacagatggataaatgaggctgatgtttggagggagaggcacacgg
taaatggcatccccttaggcgcctctttgggaaaggaaagtggatgctcctttgaggcaggcgaggggctgggagtgggtagccgtcatg
ttgtcccccgtgggatcccatttttaacttgacccagattgtcttgggctccttttacttcagggggctgctttcctgggccaggctgtgtagcact
tcccaccctcaggcatgagtacagactggtcaaaatgtttatgcagtcaaggccaaagcctgccctgagcccagacacacctgggtccc

cattctggggcctggtcccctaaacaatctcttcctcagccagcacagggaaatccagactcagggttccagaaatcccccgttcccaaa
ccaaaccaatcatggatcttccctttaaggggtagaatcagcctttagagttacaagcccctggaaaagggagcaggtcccatacaatca
gcctttctcctcttctcttcttgggacaggaataactgctgagcagtccccctttgccactcctggctgtctgagtgggcacttgtctggctgctgg
ccagcagtgaagggccacttggtgacttcgtagggttccttagagaagtgacatggccttgaggagattcagggcacctttcctcagtgag
ctttgatatggtccaaaaacagccttaaatcaagaatatttgtggaggtaggtgtggggaaggttggagaagagttaggtttgtgctttgtaat
cttggcatccatgttttgtgcctgccctccctcttggggaggccatgcttgactctgctgaaagctgctaactggtgacagggtggatcatggt
gaggactaggggtaaggtcagggaatgcttaagctctggccctgccctgtattcctcttccccttggtagcagttccctgaccaagggcaag
gtgtgtctcaggaaggtggttctgtgcatgcctgggtgtggttaaggcgtggcccagaaggcttccctggtgctctcagtccaggcaaagcc
cagagcatctgagcacgtctctgacttccagtggcaacacagtttgaacgtggtgaaacagggtgtagttcttttccacattctgtgtcatcta
gtcgtgcaggtagggaaaaagttggtctaattgaagattgtgcatttcctagtgacaggtgccaagaggttatgatacgggtttcttgggtctg
atgtacagtgtgaataaatgcttgcagctcttagctctttttgatcgatgaagcactttttattaatattttccttgttaaaggaggaaccgtaact
ctccatagctgtacatataaccctttctcctaaagaggagtcagtcagtgctcctatatttttcatttttgtcaaagcaagaagtaaatactttag
aattgttaaatatataaataaagcaaataaagttg (SEQ ID NO:205)

**Q9UEG3**
MDEETRHSLECIQANQIFPRKQLIREDENLQVPFLELHGESTEFVGRAEDAIIALSNYRLHIKFKESL
VNVPLQLIESVECRDIFQLHLTCKDCKVIRCQFSTFEQCQEWLKRLNNAIRPPAKIEDLFSFAYHAW
CMEVYASEKEQHGDLCRPGEHVTSRFKNEVERMGFDMNNAWRISNINEKYKLCGSYPQELIVPA
WITDKELESVSSFRSWKRIPAVIYRHQSNGAVIARCGQPEVSWWGWRNADDEHLVQSVAKACAS
DSRSSGSKLSTRNTSRDFPNGGDLSDVEFDSSLSNASGAESLAIQPQKLLILDARSYAAAVANRA
KGGGCECPEYYPNCEVVFMGMANIHSIRRSFQSLRLLCTQMPDPGNWLSALESTKWLHHLSVLL
KSALLVVHAVDQDQRPVLVHCSDGWDRTPQIVALAKLLLDPYYRTIEGFQVLVEMEWLDFGHKFA
DRCGHGENSDDLNERCPVFLQWLDCVHQLQRQFPCSFEFNEAFLVKLVQHTYSCLFGTFLCNNA
KERGEKHTQERTCSVWSLLRAGNKAFKNLLYSSQSEAVLYPVCHVRNLMLWSAVYLPCPSPTTP
VDDSCAPYPAPGTSPDDPPLSRLPKTRSYDNLTTACDNTVPLASRRCSDPSLNEKWQEHRRSLE
LSSLAGPGEDPLSADSLGKPTRVPGGAELSVAAGVAEGQMENILQEATKEESGVEEPAHRAGIEI
QEGKEDPLLEKESRRKTPEASAIGLHQDPELGDAALRSHLDMSWPLFSQGISEQQSGLSVLLSSL
QVPPRGEDSLEVPVEQFRIEEIAEGREEAVLPIPVDAKVGYGTSQSCSLLPSQVPFETRGPNVDSS
TDMLVEDKVKSVSGPQGHHRSCLVNSGKDRLPQTMEPSPSETSLVERPQVGSVVHRTSLGSTLS
LTRSPCALPLAECKEGLVCNGAPETENRASEQPPGLSTLQMYPTPNGHCANGEAGRSKDSLSRQ
LSAMSCSSAHLHSRNLHHKWLHSHSGRPSATSSPDQPSRSHLDDDGMSVYTDTIQQRLRQIESG
HQQEVETLKKQVQELKSRLESQYLTSSLHFNGDFGDEVTSIPDSESNLDQNCLSRCSTEIFSEAS
WEQVDKQDTEMTRWLPDHLAAHCYACDSAFWLASRKHHCRNCGNVFCSSCCNQKVPVPSQQL
FEPSRVCKSCYSSLHPTSSSIDLELDKPIAATSN (SEQ ID NO:206)

# Figure 93

**Nucleotide:** X74008
**Sequence:**
aggaagtagggagcggggtggcagggggggacccgccgcggctgctgccaccgccgccaccaccgcctctgctcgtggcgtggg
aaaggaggtgtgagtcccgggcgcgagccgcggcggcgccgctgcgggagggtcggcggtgggaaggcgatggcggatttagata
aactcaacatcgacagcattatccaacggctgctggaagtgagagggtccaagcctggtaagaatgtccagcttcaggagaatgaaatc
agaggactgtgcttaaagtctcgtgaaatctttctcagtcagcctatcctactagaacttgaagcaccactcaaaatatgtggtgacatccat
ggacaatactatgatttgctgcgactttttgagtacggtggtttcccaccagaaagcaactacctgtttcttggggactatgtggacaggggga
aagcagtcattggagacgatctgcctcttactggcctacaaaataaaatatcctgagaattttttcttctcagagggaaccatgaatgtgcca
gcatcaacagaatttatggattttatgatgaatgtaaaagaagatacaacattaaactatggaaaactttcacagactgttttaactgtttacc
gatagcagccatcgtggatgagaagatattctgctgtcatggaggtttatcaccagatcttcaatctatggagcagattcggcgaattatgcg
accaactgatgtaccagatcaaggtcttctttgtgatcttttgtggtctgaccccgataaagatgtcttaggctggggtgaaaatgacagagg
agtgtccttcacatttggtgcagaagtggttgcaaaatttctccataagcatgatttggatcttatatgtagagcccatcaggtggttgaagatg
gatatgaatttttttgcaaagaggcagttggtcactctgttttctgcgcccaattattgcggagagtttgacaatgcaggtgccatgatgagtgtg
gatgaaacactaatgtgttcttttcagattttaaagcctgcagagaaaaagaagccaaatgccacgagacctgtaacgcctccaaggggt
atgatcacaaagcaagcaaagaaatagatgtcgttttgacactgcctagtcgggacttgtaacatagagtatataaccttcatttttaagact
gtaatgtgtactggtcagcttgctcagatagatctgtgtttgtgggggcccttccttccatttttgatttagtgaatggcatttgctggttataacagc
aaatgaaagactcttcactccaaaaagaaaagtgttttgttttttaattctctgttccttttgcaaacaattttaatgatggtgttaaagctgtacac
cccaggacagtttatcctgtctgaggagtaagtgtacaattgatctttttaattcagtacaacccataatcatgtaaatgctcattttctttagga
cataaagagagccctagggtgctctgaatctgtacatgttcttgtcataaaatgcatactgttgatacaaaccactgtgaacatttttttatttgag
aattttgtttcaaagggattgctttttcctctcattgtcttgttatgtacaaactagtttttatagctatcaacattaggagtaactttcaaccttgccag
catcactggtatgatgtatatttaattaaagcacacttttccccgaccgtatacttaaaatgacaaagccattcttttaaatatttgtgactctttcct
aaagccaaagtttctgttgaattatgttttgacacacccctaagtacaaggtggtatggttgtatacacatgctgccttcttggggattcaaaaa
caggtttttgattttgaatagcaattagtgatatagtgctgtttaagctactaacgataaaaggtaataacattttatacaatttccatatagtctatt
cattaagtaatctttttacagttgcatcaggcctgaacccgtccattcagaaagcttcaaattatagaaacaatactgttctatacgagtgacc
gattatgctttctttggcctacattctttattctgcggtgaagttgaggcttataagttaaaacaaaggaactaacttactgtccaccagtttatac
agaactcacagtacctatgactttttttaaactaagatctgttaaaaaagaaatctgtttcaacagatgaccgtgtacaataccgtgtggtgaa
aat (SEQ ID NO:207)

**P36873**
MADLDKLNIDSIIQRLLEVRGSKPGKNVQLQENEIRGLCLKSREIFLSQPILLELEAPLKICGDIHGQY
YDLLRLFEYGGFPPESNYLFLGDYVDRGKQSLETICLLLAYKIKYPENFFLLRGNHECASINRIYGF
YDECKRRYNIKLWKTFTDCFNCLPIAAIVDEKIFCCHGGLSPDLQSMEQIRRIMRPTDVPDQGLLC
DLLWSDPDKDVLGWGENDRGVSFTFGAEVVAKFLHKHDLDLICRAHQVVEDGYEFFAKRQLVTL
FSAPNYCGEFDNAGAMMSVDETLMCSFQILKPAEKKKPNATRPVTPPRGMITKQAKK (SEQ ID NO:208)

# Figure 94

Nucleotide: U29344
**Sequence:**
cggccgtcgacacggcagcggccccggcctccctctccgccgcgcttcagcctcccgctccgccgcgctccagcctcgctctccgccgc
ccgcaccgccgcccgcgccctcaccagagcagccatggaggaggtggtgattgccggcatgtccgggaagctgccagagtcggaga
acttgcaggagttctgggacaacctcatcggcggtgtggacatggtcacggacgatgaccgtcgctggaaggcggggctctacggcctg
ccccggcggtccggcaagctgaaggacctgtctaggtttgatgcctccttcttcggagtccaccccaagcaggcacacacgatggaccct
cagctgcggctgctgctggaagtcacctatgaagccatcgtggacggaggcatcaacccagattcactccgaggaacacacactggcg
tctgggtgggcgtgagcggctctgagacctcggaggccctgagccgagaccccgagacactcgtgggctacagcatggtgggctgcca
gcgagcgatgatggccaaccggctctccttcttcttcgacttcagagggcccagcatcgcactggacacagcctgctcctccagcctgatg
gccctgcagaacgcctaccaggccatccacagcgggcagtgccctgccgccatcgtggggggcatcaatgtcctgctgaagcccaac
acctccgtgcagttcttgaggctggggatgctcagccccgagggcacctgcaaggccttcgacacagcggggaatgggtactgccgctc
ggagggtgtggtggccgtcctgctgaccaagaagtccctggcccggcgggtgtacgccaccatcctgaacgccggcaccaatacagat
ggcttcaaggagcaaggcgtgaccttcccctcaggggatatccaggagcagctcatccgctcgttgtaccagtcggccggagtggcccc
tgagtcatttgaatacatcgaagcccacggcacaggcaccaaggtgggcgaccccccaggagctgaatggcatcacccgagccctgtg
cgccacccgccaggagccgctgctcatcggctccaccaagtccaacatggggcacccggagccagcctcggggctggcagccctgg
ccaaggtgctgctgtccctggagcacgggctctgggcccccaacctgcacttccatagccccaaccctgagatcccagcgctgttggatg
ggcggctgcaggtggtggaccagcccctgcccgtccgtggcggcaacgtgggcatcaactcctttggcttcgggggctccaacgtgcac
atcatcctgaggcccaacacgcagccgcccccgcacccgccccacatgccaccctgccccgtctgctgcgggccagcggacgcacc
cctgaggccgtgcagaagctgctggagcagggcctccggcacagccaggacctggctttcctgagcatgctgaacgacatcgcgctgt
ccccgaccaccgccatgcccttccgtggctacgctgtgctgggtggtgagcgcggtggcccagaggtgcagcaggtgcccgctggcga
gcgcccgctctggttcatctgctctgggatgggcacacagtggcgcgggatggggctgagcctcatgcgcctggaccgcttccgagattc
catcctacgctccgatgaggctgtgaaccgattcggcctgaaggtgtcacagctgctgctgagcacagacgagagcacctttgatgacat
cgtccattcgtttgtgagcctgactgccatccagataggcctcatagacctgctgagctgcatggggctgaggccagatggcatcgtcggc
cactccctgggggaggtggcctgtggctacgccgacggctgcctgtcccaggaggaggccgtcctcgctgcctactggaggggacagt
gcatcaaagaagcccatctcccgccgggcgccatggcagccgtgggcttgtcctgggaggagtgtaaacagcgctgcccccggcggt
ggtgcccgcctgccacaactccaaggacacagtcaccatctcgggacctcaggccccggtgtttgagttcgtggagcagctgaggaag
gagggtgtgtttgccaaggaggtgcggaccggcggtatggccttccactcctacttcatggaggccatcgcacccccactgctgcaggag
ctcaagaaggtgatccgggagccgaagccacgttcagcccgctggctcagcacctctatccccgaggcccagtggcacagcagcctg
gcacgcacgtcctccgccgagtacaatgtcaacaacctggtgagccctgtgctgttccaggaggccctgtggcacgtgcctgagcacgc
ggtggtgctggagatcgcgccccacgccctgctgcaggctgtcctgaagcgtggcctgaagccgagctgcaccatcatcccccctgatga
agaaggatcacagggacaacctggagttcttcctggccggcatccggaggctgcacctctcaggcatcgacgccaaccccaatgccttg
ttcccacctgtggagttcccagctccccgaggaactcccctcatctccccactcatcaagtgggaccacagcctggcctgggacgtgccg
gccgccgaggacttccccaacggttcaggttccccctcagccgccatctacaacatcgacaccagctccgagtctcctgaccactacctg
gtggaccacaccctcgacggtcgcgtcctcttccccgccactggctacctgagcatagtgtggaagacgctggcccgacccctgggcctg
ggcgtcgagcagctgcctgtggtgtttgaggatgtggtgctgcaccaggccaccatcctgcccaagactgggacagtgtccctggaggta
cggctcctggaggcctcccgtgccttcgaggtgtcagagaacggcaacctggtagtgagtgggaaggtgtaccagtgggatgaccctga
ccccaggctcttcgaccacccggaaagccccacccccaaccccacggagcccctcttcctggcccaggctgaagtttacaaggagctg
cgtctgcgtggctacgactacggccctcatttccagggcatcctggaggccagcctggaaggtgactcggggaggctgctgtggaagga
taactgggtgagcttcatggacaccatgctgcagatgtccatcctgggctcggccaagcacggcctgtacctgcccacccgtgtcaccgc
catccacatcgaccctgccacccacaggcagaagctgtacacactgcaggacaaggcccaagtggctgacgtggtggtgagcaggtg
gctgagggtcacagtggccggaggcgtccacatctccgggctccacactgagtcggccccgcggcggcagcaggagcagcaggtgc
ccatcctggagaagttttgcttcacttcccacacggaggaggggtgcctgtctgagcgcgctgccctgcaggaggagctgcaactgtgca
aggggctggtgcaggcactgcagaccaaggtgacccagcaggggctgaagatggtggtgcccggactggatggggcccagatcccc
cgggacccctcacagcaggaactgccccggctgttgtcggctgcctgcaggcttcagctcaacgggaacctgcagctggagctggcgc
aggtgctggcccaggagaggcccaagctgccagaggaccctctgctcagcggcctcctggactccccggcactcaaggcctgcctgg
acactgccgtggagaacatgcccagcctgaagatgaaggtggtggaggtgctggccggccacggtcacctgtattcccgcatcccagg
cctgctcagcccccatcccctgctgcagctgagctacacggccaccgaccgccacccccaggccctggaggctgcccaggccgagct
gcagcagcacgacgttgcccagggccagtgggatcccgcagaccctgcccccagcgccctgggcagcgccgacctcctggtgtgcaa
ctgtgctgtggctgccctcggggacccggcctcagctctcagcaacatggtggctgccctgagagaagggggctttctgctcctgcacaca
ctgctccgggggcacccctcgggacatgtggccttcctcacctccactgagccgcagtatggccagggcatcctgagccaggacgcgtg
ggagagcctcttctccagggtgtccgtgcgcctggtgggcctgaagaagtccttctacggctccacgctcttcctgtgccgccggcccaccc
cgcaggacagccccatcttcctgccggtggacgataccagcttccgctgggtggagtctctgaagggcatcctggctgacgaagactctt
cccggcctgtgtggctgaaggccatcaactgtgccacctcgggcgtggtgggcttggtgaactgtctccgccgagagcccggcggaacg
ctccggtgtgtgctgctctccaacctcagcagcacctcccacgtcccggaggtggacccgggctccgcagaactgcagaaggtgttgca

gggagacctggtgatgaacgtctaccgcgacggggcctggggggctttccgccacttcctgctggaggaggacaagcctgaggagcc
gacggcacatgcctttgtgagcaccctcacccggggggacctgtcctccatccgctgggtctgctcctcgctgcgccatgcccagcccacc
tgccctggcgcccagctctgcacggtctactacgcctccctcaacttccgcgacatcatgctggccactggcaagctgtcccctgatgccat
cccagggaagtggacctcccaggacagcctgctaggtatggagttctcgggccgagacgccagcggcaagcgtgtgatgggactggt
gcctgccaagggcctggccacctctgtcctgctgtcaccggacttcctctgggatgtgccttccaactggacgctggaggaggcggcctcg
gtgcctgtcgtctacagcacggcctactacgcgctggtggtgcgtgggcgggtgcgccccggggagacgctgctcatccactcgggctcg
ggcggcgtgggccaggccgccatcgccatcgccctcagtctgggctgccgcgtcttcaccaccgtggggtcggctgagaagcgggcgt
acctccaggccaggttcccccagctcgacagcaccagcttcgccaactcccgggacacatccttcgagcagcatgtgctgtggcacacg
ggcgggaagggcgttgacctggtcttgaactccttggcggaagagaagctgcaggccagcgtgaggtgcttggctacgcacggtcgctt
cctggaaattggcaaattcgacctttctcagaaccacccgctcggcatggctatcttcctgaagaacgtgacattccacggggtcctactgg
atgcgttcttcaacgagagcagtgctgactggcgggaggtgtgggcgcttgtgcaggccggcatccgggatggggtggtacggcccctc
aagtgcacggtgttccatggggcccaggtggaggacgccttccgctacatggcccaagggaagcacattggcaaagtcgtcgtgcagg
tgcttgcggaggagccggaggcagtgctgaaggggggccaaacccaagctgatgtcggccatctccaagaccttctgcccggcccaca
agagctacatcatcgctggtggtctgggtggcttcggcctggagttggcgcagtggctgatacagcgtggggtgcagaagctcgtgttgac
ttctcgctccgggatccggacaggctaccaggccaagcaggtccgccggtggagggcccagggcgtacaggtgcaggtgtccaccag
caacatcagctcactggagggggcccggggcctcattgccgaggcggcgcagcttgggcccgtgggcggcgtcttcaacctggccgtg
gtcttgagagatggcttgctggagaaccagaccccagagttcttccaggacgtctgcaagcccaagtacagcggcaccctgaacctgga
cagggtgacccgagaggcgtgccctgagctggactactttgtggtcttctcctctgtgagctgcgggcgtggcaatgcgggacagagcaa
ctacggctttgccaattccgccatggagcgtatctgtgagaaacgccggcacgaaggcctcccaggcctggccgtgcagtggggcgcc
atcggcgacgtgggcatttggtggagacgatgagcaccaacgacacgatcgtcagtggcacgctgccccaggccatggcgtcctgcct
ggaggtgctggacctcttcctgaaccagccccacatggtcctgagcagctttgtgctggctgagaaggctgcggcctatagggacaggg
acagccagcgggacctggtggaggccgtggcacacatcctgggcatccgcgacttggctgctgtcaacctggacagctcactggcgga
cctgggcctggactcgctcatgagcgtggaggtgcgccagacgctggagcgtgagctcaacctggtgctgtccgtgcgcgaggtgcggc
aactcacgctccggaaactgcaggagctgtcctcaaaggcggatgaggccagcgagctggcatgccccacgcccaaggaggatggt
ctggcccagcagcagactcagctgaacctgcgctccctgctggtgaacccggagggccccaccctgatgcggctcaactccgtgcaga
gctcggagcggcccctgttcctggtgcacccaatcgagggctccaccaccgtgttccacagcctggcctcccggctcagcatccccacct
atggcctgcagtgcacccgagctgcgccccttgacagcatccacagcctggctgcctactacatcgactgcatcaggcaggtgcagccc
gagggcccctaccgcgtggccggctactcctacggggcctgcgtggcctttgaaatgtgctcccagctgcaggcccagcagagcccag
cccccacccacaacagcctcttcctgttcgacggctcgcccacctacgtactggcctacacccagagctaccgggcaaagctgacccca
ggctgtgaggctgaggctgagacggaggccatatgcttcttcgtgcagcagttcacggacatggagcacaacagggtgctggaggcgct
gctgccgctgaagggcctagaggagcgtgtggcagccgccgtggacctgatcatcaagagccaccagggcctggaccgccaggagc
tgagctttgcggcccggtccttctactacaagctcggtgccgctgagcagtacacacccaaggccaagtaccatggcaacgtgatgctac
tgcgcgccaagacgggtggcgcctacggcgaggacctgggcgcggattacaacctctcccaggtatgcgacgggaaagtatccgtcc
acgtcatcgagggtgaccaccgcacgctgctggagggcagcggcctggagtccatcatcagcatcatccacagctccctggctgagcc
acgcgtgagcgtgcgggagggctaggcccgtgcccccgcctgccaccggaggtcactccaccatccccaccccaccccaccccaccc
ccgccatgcaacgggattgaagggtcctgccggtgggaccctgtccggcccagtgccactgccccccgaggctgctagatgtaggtgtt
aggcatgtcccacccacccgccgcctcccacggcacctcggggacaccagagctgccgacttggagactcctggtctgtgaagagccg
gtggtgcccgttcccgcaggaactgggctgggcctcgtgcgcccgtggggtctgcgcttggtctttctgtgcttggatttgcatatttattgcatt
gctggtagagacccccaggcctgtccaccctgccaagactcctcaggcagcgtgtgggtcccgcactctgcccccatttccccgatgtccc
ctgcgggcgcgggcagccacccaagcctgctggctgcggcccccctctcggccaggcattggctcagccngctgagtggggggtcgtgg
gccagtccccgaggagctgggcccctgcacaggcacacagggcccggccacacccagcggcccccgcacagccacccgtgggg
tgctgcccttatcgcccggcgccgggcaccaactccatgtttggtgtttgtctgtgtttgtttttcaagaaatgattcaaattgctgcttggattttga
aatttactgtaactgtcagtgtacacgtctggaccccgtttcatttttacaccaatttggtaaaaatgctgctctcagcctcccacaattaaaccg
catgtgatctcccc (SEQ ID NO:209)

**Q16702**

MEEVVIAGMSGKLPESENLQEFWDNLIGGVDMVTDDDRRWKAGLYGLPRRSGKLKDLSRFDASF
FGVHPKQAHTMDPQLRLLLEVTYEAIVDGGINPDSLRGTHTGVWVGVSGSETSEALSRDPETLVG
YSMVGCQRAMMANRLSFFFDFRGPSIALDTACSSSLMALQNAYQAIHSGQCPAAIVGGINVLLKP
NTSVQFLRLGMLSPEGTCKAFDTAGNGYCRSEGVVAVLLTKKSLARRVYATILNAGTNTDGFKEQ
GVTFPSGDIQEQLIRSLYQSAGVAPESFEYIEAHGTGTKVGDPQELNGITRALCATRQEPLLIGSTK
SNMGHPEPASGLAALAKVLLSLEHGLWAPNLHFHSPNPEIPALLDGRLQVVDQPLPVRGGNVGIN
SFGFGGSNVHIILRPNTQPPPAPAPHATLPRLLRASGRTPEAVQKLLEQGLRHSQDLAFLSMLNDI
ALSPTTAMPFRGYAVLGGERGGPEVQQVPAGERPLWFICSGMGTQWRGMGLSLMRLDRFRDSI
LRSDEAVNRFGLKVSQLLLSTDESTFDDIVHSFVSLTAIQIGLIDLLSCMGLRPDGIVGHSLGEVAC
GYADGCLSQEEAVLAAYWRGQCIKEAHLPPGAMAAVGLSWEECKQRCPPAVVPACHNSKDTVTI
SGPQAPVFEFVEQLRKEGVFAKEVRTGGMAFHSYFMEAIAPPLLQELKKVIREPKPRSARWLSTSI

PEAQWHSSLARTSSAEYNVNNLVSPVLFQEALWHVPEHAVVLEIAPHALLQAVLKRGLKPSCTIIP
LMKKDHRDNLEFFLAGIRRLHLSGIDANPNALFPPVEFPAPRGTPLISPLIKWDHSLAWDVPAAED
FPNGSGSPSAAIYNIDTSSESPDHYLVDHTLDGRVLFPATGYLSIVWKTLARPLGLGVEQLPVVFE
DVVLHQATILPKTGTVSLEVRLLEASRAFEVSENGNLVVSGKVYQWDDPDPRLFDHPESPTPNPT
EPLFLAQAEVYKELRLRGYDYGPHFQGILEASLEGDSGRLLWKDNWVSFMDTMLQMSILGSAKH
GLYLPTRVTAIHIDPATHRQKLYTLQDKAQVADVVVSRWLRVTVAGGVHISGLHTESAPRRQQEQ
QVPILEKFCFTSHTEEGCLSERAALQEELQLCKGLVQALQTKVTQQGLKMVVPGLDGAQIPRDPS
QQELPRLLSAACRLQLNGNLQLELAQVLAQERPKLPEDPLLSGLLDSPALKACLDTAVENMPSLK
MKVVEVLAGHGHLYSRIPGLLSPHPLLQLSYTATDRHPQALEAAQAELQQHDVAQGQWDPADPA
PSALGSADLLVCNCAVAALGDPASALSNMVAALREGGFLLLHTLLRGHPSGHVAFLTSTEPQYGQ
GILSQDAWESLFSRVSVRLVGLKKSFYGSTLFLCRRPTPQDSPIFLPVDDTSFRWVESLKGILADE
DSSRPVWLKAINCATSGVVGLVNCLRREPGGTLRCVLLSNLSSTSHVPEVDPGSAELQKVLQGDL
VMNVYRDGAWGAFRHFLLEEDKPEEPTAHAFVSTLTRGDLSSIRWVCSSLRHAQPTCPGAQLCT
VYYASLNFRDIMLATGKLSPDAIPGKWTSQDSLLGMEFSGRDASGKRVMGLVPAKGLATSVLLSP
DFLWDVPSNWTLEEAASVPVVYSTAYYALVVRGRVRPGETLLIHSGSGGVGQAAIAIALSLGCRV
FTTVGSAEKRAYLQARFPQLDSTSFANSRDTSFEQHVLWHTGGKGVDLVLNSLAEEKLQASVRC
LATHGRFLEIGKFDLSQNHPLGMAIFLKNVTFHGVLLDAFFNESSADWREVWALVQAGIRDGVVR
PLKCTVFHGAQVEDAFRYMAQGKHIGKVVVQVLAEEPEAVLKGAKPKLMSAISKTFCPAHKSYIIA
GGLGGFGLELAQWLIQRGVQKLVLTSRSGIRTGYQAKQVRRWRAQGVQVQVSTSNISSLEGARG
LIAEAAQLGPVGGVFNLAVVLRDGLLENQTPEFFQDVCKPKYSGTLNLDRVTREACPELDYFVVF
SSVSCGRGNAGQSNYGFANSAMERICEKRRHEGLPGLAVQWGAIGDVGILVETMSTNDTIVSGT
LPQAMASCLEVLDLFLNQPHMVLSSFVLAEKAAAYRDRDSQRDLVEAVAHILGIRDLAAVNLDSSL
ADLGLDSLMSVEVRQTLERELNLVLSVREVRQLTLRKLQELSSKADEASELACPTPKEDGLAQQQ
TQLNLRSLLVNPEGPTLMRLNSVQSSERPLFLVHPIEGSTTVFHSLASRLSIPTYGLQCTRAAPLDS
IHSLAAYYIDCIRQVQPEGPYRVAGYSYGACVAFEMCSQLQAQQSPAPTHNSLFLFDGSPTYVLA
YTQSYRAKLTPGCEAEAETEAICFFVQQFTDMEHNRVLEALLPLKGLEERVAAAVDLIIKSHQGLD
RQELSFAARSFYYKLGAAEQYTPKAKYHGNVMLLRAKTGGAYGEDLGADYNLSQVCDGKVSVHV
IEGDHRTLLEGSGLESIISIIHSSLAEPRVSVREG (SEQ ID NO:210)

# Figure 95

**Nucleotide:** AB025194
**Sequence:**
tggctgagccagcagctgcagcagctgcgggagtggccgggtggccggcgggtgccagccgccatggaggccgtgccccgcatgcc
catgatctggctggacctgaaggaggccggtgactttcacttccagccagctgtgaagaagttcgtcctgaagaattatggagagaaccc
agaagcctacaatgaagaactgaagaagctggagttgctcagacagaatgctgtccgtgtcccacgagactttgagggctgtagtgtcct
ccgcaagtacctcggccagcttcattacctgcagagtcgggtccccatgggctcgggccaggaggccgctgtccctgtcacctggacag
agatcttctcaggcaagtctgtggcccatgaggacatcaagtacgagcaggcctgtattctctacaaccttggagcgctgcactccatgctg
ggggccatggacaagcgggtgtctgaggagggcatgaaggtctcctgtacccatttccagtgcgcagccggcgccttcgcctacctacg
ggagcacttccctcaagcctacagcgtcgacatgagccgccagatccttacgctcaacgtcaacctcatgctgggccaggctcaggagt
gcctcctggagaagtcgatgttggacaacaggaagagctttctggtggcccgcatcagtgcacaggtggtagattactacaaggaggca
tgccgggccttggagaaccccgacactgcctcactgctgggccggatccagaaggactggaagaaacttgtgcagatgaagatctact
acttcgcagccgtggctcatctgcacatgggaaagcaggccgaggagcagcagaagttcggggagcgggttgcatacttccagagcg
ccctggacaagctcaatgaagccatcaagttggccaagggccagcctgacactgtgcaagacgcgcttcgcttcactatggatgtcattg
gggggaaagtacaattctgccaagaaggacaacgacttcatttaccatgaggctgtcccagcattggacacccttcagcctgtaaaagga
gcccccttggtgaagcccttgccagtgaaccccacagacccagctgttacaggccctgacatctttgccaaactggtacccatggctgccc
acgaggcctcgtcactgtacagtgaggagaaggccaagctgctccgggagatgatggccaagattgaggacaagaatgaggtcctgg
accagttcatggattcaatgcagttggatcccgagacggtggacaaccttgatgcctacagccacatcccaccccagctcatggagaagt
gcgcggctctcagcgtccggcccgacactgtcaggaaccttgtacagtccatgcaagtgctgtcaggtgtgttcacggatgtggaggcttc
cctgaaggacatcagagatctgttggaggaggatgagctgctagagcagaagtttcaggaggcggtgggccaggcaggggccatctc
catcacctccaaggctgagctggcagaggtgaggcgagaatgggccaagtacatggaagtccatgagaaggcctccttcaccaacag
tgagctgcaccgtgccatgaacctgcacgtcggcaacctgcgcctgctcagcgggccgcttgaccaggtccgggctgccctgcccacac
cggccctctccccagaggacaaggccgtgctgcaaaacctaaagcgcatcctggctaaggtgcaggagatgcgggaccagcgcgtgt
ccctggagcagcagctgcgtgagcttatccagaaagatgacatcactgcctcgctggtcaccacagaccactcagagatgaagaagttg
ttcgaggagcagctgaaaaagtatgaccagctgaaggtgtacctggagcagaacctggccgcccaggaccgtgtcctctgtgcactga
cagaggccaacgtgcagtacgcagccgtgcggcgggtactcagcgacttggaccaaaagtggaactccacgctgcagaccctggtgg
cctcgtatgaagcctatgaggacctgatgaagaagtcgcaggagggcagggacttctacgcagatctggagagcaaggtggctgctct
gctggagcgcacgcagtccacctgccaggcccgcgaggctgcccgccagcagctcctggacagggagctgaagaagaagccgccg
ccacggcccacagccccaaagccgctgctgccccgcagggaggagagtgaggcagtggaagcaggagacccccctgaggagctg
cgcagcctcccccctgacatggtggctggcccacgactgcctgacaccttcctgggaagtgccaccccgctccactttcctcccagcccct
tccccagctccacaggcccaggaccccactatctctcaggccccttgccccctggtacctactcgggccccacccagctgatacagccca
gggccccagggccccatgcaatgcccgtagcacctgggcctgccctctacccagcccctgcatacacaccggagctgggccttgtgccc
cgatcctccccacagcatggcgtggtgagcagtccctatgtgggggtagggccggccccaccagttgcaggtctcccctcggccccacct
cctcaattctcaggccccgagttggccatggcggttcggccagccaccaccacagtagatagcatccaggcgcccatccccagccaca
cagccccacggccaaaccccacccctgctcctcccccgccctgcttccctgtgcccccaccgcagccactgcccacgccttacacctac
cctgcaggggctaagcaacccatcccagcacagcaccacttctcttctgggatccccgcaggttttccagccccaaggattgggccccag
ccccagccccatcctcagccccatccttcacaagcgtttgggcctcagcccccacagcagccccttccactccagcatccacatctcttcc
caccccaggccccaggactcctacccccacaatcccccctacccctatgcccctcagcctggggtcctggggcagccgccaccccccct
acacacccagctctacccaggtcccgctcaagaccctctgccagcccactcaggggctctgcctttccccagccctgggccccctcagcc
tccccatcccccactggcatatggtcctgccccttctaccagacccatgggcccccaggcagcccctcttaccattcgagggccctcgtctg
ctggccagtccacccctagtccccacctggtgccttcacctgccccatctccagggcctggtccggtacccctcgcccccagcagcag
aaccacccccttgcctgcgccgaggcgccgcagctgcagacctgctctcctccagcccggagagccagcatggcggcactcagtctcct
gggggtgggcagcccctgctgcagcccaccaaggtggatgcagctgagggtcgtcggccgcaggccctgcggctgattgagcgggac
ccctatgagcatcctgagaggctgcggcagttgcagcaggagctggaggcctttcggggtcagctggggggatgtgggagctctggacac
tgtctggcgagagctgcaagatgcgcaggaacatgatgcccgaggccgttccatcgccattgcccgctgctactcactgaagaaccggc
accaggatgtcatgccctatgacagtaaccgtgtggtgctgcgctcaggcaaggatgactacatcaatgccagctgcgtggaggggctct
ccccatactgcccccgctagtggcaacccaggccccactgcctggcacagctgctgacttctggctcatggtccatgagcagaaagtgt
cagtcattgtcatgctggtttctgaggctgagatggagaagcaaaaagtggcacgctacttccccaccgagaggggccagcccatggtg
cacggtgccctgagcctggcattgagcagcgtccgcagcaccgaaacccatgtggagcgcgtgctgagcctgcagttccgagaccag
agcctcaagcgctctcttgtgcacctgcacttccccacttggcctgagttaggcctgcccgacagccccagcaacttgctgcgcttcatcca
ggaggtgcacgcacattacctgcatcagcggccgctgcacacgcccatcattgtgcactgcagctctggtgtgggccgcacgggagcctt
tgcactgctctatgcagctgtgcaggaggtggaggctgggaacggaatccctgagctgcctcagctggtgcggcgcatgcggcagcag
agaaagcacatgctgcaggagaagctgcacctcaggttctgctatgaggcagtggtgagacacgtggagcaggtcctgcagcgccatg
gtgtgcctcctccatgcaaacccttggccagtgcaagcatcagccagaagaaccaccttcctcaggactcccaggacctggtcctcggtg
gggatgtgcccatcagctccatccaggccaccattgccaagctcagcattcggcctcctgggggggttggagtccccggttgccagcttgcc

aggccctgcagagcccccaggcctcccgccagccagcctcccagagtctaccccaatcccatcttcctccccacccccccttttcctcccc
actacctgaggctccccagcctaaggaggagccgccagtgcctgaagcccccagctcggggcccccctcctcctccctggaattgctgg
cctccttgaccccagaggccttctccctggacagctccctgcggggcaaacagcggatgagcaagcataactttctgcaggcccataac
gggcaagggctgcgggccacccggccctctgacgaccccctcagccttctggatccactctggacactcaacaagacctgaacaggttt
tgcctacctggtccttacactacatcatcatcatctcatgcccacctgcccacacccagcagagcttctcagtgggcacagtctcttactccc
atttctgctgcctttggccctgcctggcccagcctgcacccctgtggggtggaaatgtactgcaggctctgggtcaggttctgctcctttatggg
acccgacatttttcagctctttgctattgaaataataaaccaccctgttctgtgaaaaaaaaa (SEQ ID NO:211)

**Q9H3S7**
MEAVPRMPMIWLDLKEAGDFHFQPAVKKFVLKNYGENPEAYNEELKKLELLRQNAVRVPRDFEG
CSVLRKYLGQLHYLQSRVPMGSGQEAAVPVTWTEIFSGKSVAHEDIKYEQACILYNLGALHSMLG
AMDKRVSEEGMKVSCTHFQCAAGAFAYLREHFPQAYSVDMSRQILTLNVNLMLGQAQECLLEKS
MLDNRKSFLVARISAQVVDYYKEACRALENPDTASLLGRIQKDWKKLVQMKIYYFAAVAHLHMGK
QAEEQQKFGERVAYFQSALDKLNEAIKLAKGQPDTVQDALRFTMDVIGGKYNSAKKDNDFIYHEA
VPALDTLQPVKGAPLVKPLPVNPTDPAVTGPDIFAKLVPMAAHEASSLYSEEKAKLLREMMAKIED
KNEVLDQFMDSMQLDPETVDNLDAYSHIPPQLMEKCAALSVRPDTVRNLVQSMQVLSGVFTDVE
ASLKDIRDLLEEDELLEQKFQEAVGQAGAISITSKAELAEVRREWAKYMEVHEKASFTNSELHRAM
NLHVGNLRLLSGPLDQVRAALPTPALSPEDKAVLQNLKRILAKVQEMRDQRVSLEQQLRELIQKD
DITASLVTTDHSEMKKLFEEQLKKYDQLKVYLEQNLAAQDRVLCALTEANVQYAAVRRVLSDLDQ
KWNSTLQTLVASYEAYEDLMKKSQEGRDFYADLESKVAALLERTQSTCQAREAARQQLLDRELK
KKPPPRPTAPKPLLPRREESEAVEAGDPPEELRSLPPDMVAGPRLPDTFLGSATPLHFPPSPFPS
STGPGPHYLSGPLPPGTYSGPTQLIQPRAPGPHAMPVAPGPALYPAPAYTPELGLVPRSSPQHG
VVSSPYVVGVGPAPPVAGLPSAPPPQFSGPELAMAVRPATTTVDSIQAPIPSHTAPRPNPTPAPPP
PCFPVPPPQPLPTPYTYPAGAKQPIPAQHHFSSGIPAGFPAPRIGPQPQPHPQPHPSQAFGPQPP
QQPLPLQHPHLFPPQAPGLLPPQSPYPYAPQPGVLGQPPPPLHTQLYPGPAQDPLPAHSGALPF
PSPGPPQPPHPPLAYGPAPSTRPMGPQAAPLTIRGPSSAGQSTPSPHLVPSPAPSPGPGPVPPR
PPAAEPPPCLRRGAAAADLLSSSPESQHGGTQSPGGGQPLLQPTKVDAAEGRRPQALRLIERDP
YEHPERLRQLQQELEAFRGQLGDVGALDTVWRELQDAQEHDARGRSIAIARCYSLKNRHQDVMP
YDSNRVVLRSGKDDYINASCVEGLSPYCPPLVATQAPLPGTAADFWLMVHEQKVSVIVMLVSEAE
MEKQKVARYFPTERGQPMVHGALSLALSSVRSTETHVERVLSLQFRDQSLKRSLVHLHFPTWPE
LGLPDSPSNLLRFIQEVHAHYLHQRPLHTPIIVHCSSGVGRTGAFALLYAAVQEVEAGNGIPELPQL
VRRMRQQRKHMLQEKLHLRFCYEAVVRHVEQVLQRHGVPPPCKPLASASISQKNHLPQDSQDL
VLGGDVPISSIQATIAKLSIRPPGGLESPVASLPGPAEPPGLPPASLPESTPIPSSSPPPLSSPLPEA
PQPKEEPPVPEAPSSGPPSSSLELLASLTPEAFSLDSSLRGKQRMSKHNFLQAHNGQGLRATRP
SDDPLSLLDPLWTLNKT (SEQ ID NO:212)

## Figure 96

**Nucleotide:** M27507
**Sequence:**
gcgaagcggccggcctgggcgccgactgcagagccgggaggctggtggtcatgccggggttcctggttcgcatcctccttctgctgctgg
ttctgctgcttctgggccctacgcgcggcttgcgcaatgccacccagaggatgtttgaaattgactatagccgggactccttcctcaaggatg
gccagccatttcgctacatctcaggaagcattcactactcccgtgtgccccgcttctactggaaggaccggctgctgaagatgaagatggc
tgggctgaacgccatccagacgtatgtgccctggaactttcatgagccctggccaggacagtaccagttttctgaggaccatgatgtggaa
tattttcttcggctggctcatgagctgggactgctggttatcctgaggcccgggccctacatctgtgcagagtgggaaatgggaggattacct
gcttggctgctagagaaagagtctattcttctccgctcctccgacccagattacctggcagctgtggacaagtggttgggagtccttctgccc
aagatgaagcctctcctctatcagaatggagggccagttataacagtgcaggttgaaaatgaatatggcagctactttgcctgtgattttgac
tacctgcgcttcctgcagaagcgctttcgccaccatctggggg atgatgtggttctgtttaccactgatggagcacataaaacattcctgaaa
tgtggggccctgcagggcctctacaccacggtggactttggaacaggcagcaacatcacagatgctttcctaagccagaggaagtgtga
gcccaaaggacccttgatcaattctgaattctatactggctggctagatcactggggccaacctcactccacaatcaagaccgaagcagt
ggcttcctccctctatgatatacttgcccgtggggcgagtgtgaacttgtacatgtttataggtgggaccaattttgcctattggaatggggcca
actcaccctatgcagcacagcccaccagctacgactatgatgccccactgagtgaggctggggacctcactgagaagtattttgctctgc
gaaacatcatccagaagtttgaaaaagtaccagaaggtcctatccctccatctacaccaaagtttgcatatggaaaggtcactttggaaaa
gttaaagacagtgggagcagctctggacattctgtgtccctctgggcccatcaaaagcctttatcccttgacatttatccaggtgaaacagc
attatgggtttgtgctgtaccggacaacacttcctcaagattgcagcaacccagcacctctctcttcacccctcaatggagtccacgatcgag
catatgttgctgtggatgggatcccccagggagtccttgagcgaaacaatgtgatcactctgaacataacagggaaagctggagccactc
tggaccttctggtagagaacatgggacgtgtgaactatggtgcatatatcaacgattttaagggtttggtttctaacctgactctcagttccaat
atcctcacggactggacgatctttccactggacactgaggatgcagtgcgcagccacctgggggctggggacaccgtgacagtggcc
accatgatgaagcctgggcccacaactcatccaactacacgctcccggccttttatatggggaacttctccattcccagtgggatcccaga
cttgccccaggacacctttatccagtttcctggatggaccaagggccaggtctggattaatggctttaaccttggccgctattggccagcccg
gggccctcagttgaccttgtttgtgccccagcacatcctgatgacctcggccccaaacaccatcaccgtgctggaactggagtgggcacc
ctgcagcagtgatgatccagaactatgtgctgtgacgttcgtggacaggccagttattggctcatctgtgacctacgatcatccctccaaacc
tgttgaaaaaagactcatgcccccacccccgcaaaaaaacaaagattcatggctggaccatgtatgatgatgaaagcctgtgtctttgag
ggattctaccctgaacatacctcacagatcctccctgtcatgccacatttcactgattggaatgtggaaatggaaaaggaatttaggatgtgc
attttcacctgaggtttccctgcatccctgcagtgccaaagccccaccttcagggaccacctggaatgtgtgagggctgacagcacagtaa
cgtgcatacatatctgcagggctggaatggaagctttaaaggtggtagtgatttttatttggaagaatcatgttacctttttgttaaataaaatttg
tactcaaatg (SEQ ID NO:213)

**P16278**
MPGFLVRILLLLLVLLLLGPTRGLRNATQRMFEIDYSRDSFLKDGQPFRYISGSIHYSRVPRFYWKD
RLLKMKMAGLNAIQTYVPWNFHEPWPGQYQFSEDHDVEYFLRLAHELGLLVILRPGPYICAEWEM
GGLPAWLLEKESILLRSSDPDYLAAVDKWLGVLLPKMKPLLYQNGGPVITVQVENEYGSYFACDF
DYLRFLQKRFRHHLGDDVVLFTTDGAHKTFLKCGALQGLYTTVDFGTGSNITDAFLSQRKCEPKG
PLINSEFYTGWLDHWGQPHSTIKTEAVASSLYDILARGASVNLYMFIGGTNFAYWNGANSPYAAQ
PTSYDYDAPLSEAGDLTEKYFALRNIIQKFEKVPEGPIPPSTPKFAYGKVTLEKLKTVGAALDILCPS
GPIKSLYPLTFIQVKQHYGFVLYRTTLPQDCSNPAPLSSPLNGVHDRAYVAVDGIPQGVLERNNVI
TLNITGKAGATLDLLVENMGRVNYGAYINDFKGLVSNLTLSSNILTDWTIFPLDTEDAVRSHLGGW
GHRDSGHHDEAWAHNSSNYTLPAFYMGNFSIPSGIPDLPQDTFIQFPGWTKGQVWINGFNLGRY
WPARGPQLTLFVPQHILMTSAPNTITVLELEWAPCSSDDPELCAVTFVDRPVIGSSVTYDHPSKPV
EKRLMPPPPQKNKDSWLDHV (SEQ ID NO:214)

# Figure 97

**Nucleotide:** L04685
**Sequence:**
atgggcgacaaagggacgcgggtgttcaagaaggccagtccaaatggaaagctcaccgtctacctgggaaagcgggactttgtggac
cacatcgacctcgtggaccctgtggatggtgtggtcctggtggatcctgagtatctcaaagagcggagagtctatgtgacgctgacctgcg
ccttccgctatggccgggaggacctggatgtcctgggcctgacctttcgcaaggacctgtttgtggccaacgtacagtcgttcccaccggcc
cccgaggacaagaagcccctgacgcggctgcaggaacgcctcatcaagaagctgggcgagcacgcttacccctttcacctttgagatcc
ctccaaaccttccatgttctgtgacactgcagccggggcccgaagacacggggaaggcttgcggtgtggactatgaagccaaagccttct
gcgcggagaatttggaggagaagatccacaagcggaattctgtgggtctggtcatccggaaggttcagtatgccccagagaggcctgg
cccccagcccacagccgagaccaccaggcagttcctcatgtcggacaagcccttgcacctagaagcctctctggataaggagatctatt
accatggagaacccatcagcgtcaacgtccacgtcaccaacaacaccaacaagacggtggagaagatcaagatctcagtgcgccag
tatgcagacatctgccttttcaacacagctcagtacaagtgccctgttgccatggaagaggctgatgacactgtggcacccagctcgacgtt
ctgcaaggtctacacactgacccccttcctagccaataaccgagagaagcggggcctcgccttggacgggaagctcaagcacgaaga
cacgaacttggcctctagcaccctgttgagggaaggtgccaaccgtgagatcctggggatcattgtttcctacaaagtgaaagtgaagctg
gtggtgtctcggggcggcctgttgggagatcttgcatccagcgacgtggccgtggaactgcccttcaccctaatgcaccccaagcccaaa
gaggaacccccgcatcgggaagttccagagaacgagacgccagtagataccaatctcatagaacttgacacaaatgatgacgacatt
gtatttgaggactttgctcgccagagactgaaaggcatggaggatgacaaggaggaagaggaggatggtaccggctctccgcggctca
acgacaga (SEQ ID NO:215)

**P49407**
MGDKGTRVFKKASPNGKLTVYLGKRDFVDHIDLVDPVDGVVLVDPEYLKERRVYVTLTCAFRYGR
EDLDVLGLTFRKDLFVANVQSFPPAPEDKKPLTRLQERLIKKLGEHAYPFTFEIPPNLPCSVTLQPG
PEDTGKACGVDYEVKAFCAENLEEKIHKRNSVRLVIRKVQYAPERPGPQPTAETTRQFLMSDKPL
HLEASLDKEIYYHGEPISVNVHVTNNTNKTVKKIKISVRQYADICLFNTAQYKCPVAMEEADDTVAP
SSTFCKVYTLTPFLANNREKRGLALDGKLKHEDTNLASSTLLREGANREILGIIVSYKVKVKLVVSR
GGLLGDLASSDVAVELPFTLMHPKPKEEPPHREVPENETPVDTNLIELDTNDDDIVFEDFARQRLK
GMKDDKEEEEDGTGSPQLNNR (SEQ ID NO:216)

# Figure 98

**Nucleotide:** BC002731
**Sequence:**
ggcacgaggcccaagcaggtcggcggcggcggcaggagagcggccgggcgtcagctcctcgacccccgtgtcggctagtccagc
gaggcggacgggcggcgtgggcccatggccaggcccggcatggagcggtggcgcgaccggctggcgctggtgacgggggcctcgg
ggggcatcggcgcggccgtggcccgggccctggtccagcagggactgaaggtggtgggctgcgcccgcactgtgggcaacatcgag
gagctggctgctgaatgtaagagtgcaggctaccccgggactttgatcccctacagatgtgacctatcaaatgaagaggacatcctctcc
atgttctcagctatccgttctcagcacagcggtgtagacatctgcatcaacaatgctggcttggcccggcctgacaccctgctctcaggcag
caccagtggttggaaggacatgttcaatgtgaacgtgctggccctcagcatctgcacacgggaagcctaccagtccatgaaggagcgg
aatgtggacgatgggcacatcattaacatcaatagcatgtctggccaccgagtgttacccctgtctgtgacccacttctatagtgccaccaa
gtatgccgtcactgcgctgacagagggactgaggcaagagcttcgggaggcccagacccacatccgagccacgtgcatctctccaggt
gtggtggagacacaattcgccttcaaactccacgacaaggaccctgagaaggcagctgccacctatgagcaaatgaagtgtctcaaac
ccgaggatgtggccgaggctgttatctacgtcctcagcaccccccgcacacatccagattggagacatccagatgaggcccacggagca
ggtgacctagtgactgtgggagctcctccttccctccccacccttcatggcttgcctcctgcctctggattttaggtgttgatttctggatcacggg
ataccacttcctgtccacaccccgaccaggggctagaaaatttgtttgagatttttatatcatcttgtcaaattgcttcagttgtaaatgtgaaaa
atgggctggggaaaggaggtggtgtccctaattgttttacttgttaacttgttcttgtgcccctgggcacttggcctttgtctgctctcagtgtcttcc
ctttgacatgggaaaggagttgtggccaaaatccccatcttcttgcacctcaacgtctgtggctcagggctggggtggcagagggaggcct
tcaccttatatctgtgttgttatccagggctccagacttcctcctctgcctgccccactgcaccctctccccccttatctatctccttctcggctcccc
agcccagtcttggcttcttgtcccctcctggggtcatccctccactctgactctgactatggcagcagaacaccagggcctggcccagtgga
tttcatggtgatcattaaaaagaaaaatcgcaaccaaaaaaaaaaaaaaaaaa (SEQ ID NO:217)

**Q9BUC7**
MFSAIRSQHSGVDICINNAGLARPDTLLSGSTSGWKDMFNVNVLALSICTREAYQSMKERNVDDG
HIININSMSGHRVLPLSVTHFYSATKYAVTALTEGLRQELREAQTHIRATCISPGVVETQFAFKLHD
KDPEKAAATYEQMKCLKPEDVAEAVIYVLSTPAHIQIGDIQMRPTEQVT (SEQ ID NO:218)

# Figure 99

**Nucleotide:** AB014559
**Sequence:**
ggccttggggagcccaggatggaggtggcggtcgcggcggcgggccgagccctgcggcgggcgggaggaggtgagcaccaggcc
cactgagcctctgcagagccaccagccatgcccgggatcgtggtgttccggcggcgctggtctgtgggcagtgatgacctcgtcctacca
gccatcttcctctttctcctgcataccacctggtttgtgatcctgtccgtggtgctcttcggcctggtctataacccgcacgaggcctgctccctga
acctggtggaccacggccgcggctacctgggcatcctgctgagctgcatgatcgctgagatggccatcatctggctgagcatgcgcggg
ggcatcctctacacggagccccgtgactccatgcagtacgtgctctacgtgcgcctggccatcctggtgatcgagttcatctacgccatcgt
gggcatcgtctggctcactcagtactacacctcctgcaacgacctcactgccaagaatgtcaccctcggaatggttgtctgcaactgggta
gtcatcctcagtgtgtgcatcactgtcctctgcgtcttcgaccccacgggccgcacctttgtcaagctgagagccaccaagaggaggcagc
gtaacctgcggacctacaacctgcggcaccgcttagaggagggtcaagccaccagctggtcgcgccggctcaaagtgttcctctgctgc
acgcggacgaaggactcccagtcagatgcctactcagaaatcgcctacctctttgcggagttcttccgggaccttgacattgtgccatccg
acatcattgctggcctggtgctgctccggcagcggcagcgggccaagcgcaacgccgtgctggacgaggcaaacaatgacatcttggc
cttcctgtctgggatgccggtgaccagaaacaccaagtacctcgacctcaagaattcacaagagatgctccgctacaaagaggtctgcta
ctacatgctctttgccctggctgcctacgggtggcccatgtacctgatgcggaagcccgcctgcggcctctgccaactggctcggtcctgctc
gtgttgcctgtgtcctgcgaggccgcggttcgcccctggagtcaccatcgaggaagacaactgctgtggctgtaatgccattgccatccgg
cgccacttcctggacgagaacatgactgcggtggacatcgtctatacctcctgccatgatgcggtctatgaaacgcccttctacgtggcggt
ggaccatgacaagaagaaagtggtgatcagtatccggggggaccctgtcccccaaggatgccctgactgacctgacgggtgatgctgag
cgcctccccgtggaggggcaccacggcacctggctgggccacaagggtatggtcctctcagctgagtacatcaagaagaaactggag
caggagatggtcctgtcccaggcctttgggcgagacctgggccgcggaaccaaacactacggcctgattgtggtgggccactccctggg
cgcgggcactgctgccatcctctccttccttctgcgcccacagtatccgaccctcaagtgctttgcctactccccgccaggggggcctgctgag
tgaggatgcaatggagtattccaaggagttcgtgactgctgtggttctgggcaaagacctcgtccccaggattggcctctctcagctggaag
gcttccgcagacagctcctggatgtcctgcagcgaagcaccaagcccaaatggcggatcatcgtgggggccaccaaatgcatccccaa
gtcggagctgcctgaggaggtagaggtgaccaccctggccagcacgcggctctggacccaccccagcgacctaactatagccctctca
gccagcactccactctacccgcccggccgcatcatccacgtggtccacaaccaccctgcagagcagtgctgctgctgtgagcaggagg
agcccacatactttgccatctggggcgacaacaaggccttcaatgaggtgatcatctcgccagccatgctgcatgagcacctgccctatgt
ggtcatggagggggctcaacaaggtgctggagaactacaacaaggggaagaccgctctgctctctgcagccaaggtcatggtgagccct
accgaggtggacctgactcctgagctcatcttccagcagcagccactccccacggggccgcccatgcccactggccttgccctggagct
gccgactgcagaccaccgcaacagcagcgtcaggagcaagtcccagtctgagatgagcctggagggcttctcggagggggcggctgct
gtcgccagtggttgcggcggcggcccgccaggacccggtggagctgctgctgctgtctacccaggagcggctggcggcggagctgca
ggcccggcgggcaccactggccaccatggagagcctctcggacactgagtccctgtacagcttcgactcgcgccgctcctcaggcttcc
gcagcatccggggctcccccagcctccacgctgtgctggagcgtgatgaaggccacctcttctacattgaccctgccatccccgaggaa
aacccatccctgagctcgcgcactgagctgctggcggccgacagcctgtccaagcactcacaggacacgcagcccctggaggcggcc
ctgggcagtggcggcgtcactcctgagcggcccccccagtgctgcggccaatgacgaggaggaagaggttggcggtgggggtggcgg
gccggcctcccgcggggagctggcgctgcacaatgggcgcctgggggactcgcccagtcctcaggtgctggaattcgccgagttcatcg
acagcctcttcaacctggacagcaagagcagctccttccaagacctctactgcatggtggtgcccgagagccccaccagtgactacgct
gagggccccaagtcccccagccagcaagagatcctgctccgtgcccagttcgagcccaacctggtgcccaagcccccacggctctttg
ccggctcagccgacccctcctcgggcatctcactctcgccctccttcccgctcagctcctcgggtgagctcatggacctgacgcccacggg
cctcagtagccaggaatgcctggcggctgacaagatccggacttctacccccactggccacggagccagccccgccaagcaagatga
gctggtcatctcagcacgctagcaccccagttgcgtggccagccgggcccaggcaggagcaggtggccctgtgggcacctggtgcctg
ccccctgccgggcagctttaaggacagacccccaggggcagtttagcctcaggcacaggcatcgctgctgagctgggggtccgcatcc
ctacctcagcttaggacccccagagccaaggtggctgggatctggccccacagatggggaaagatggggaagggtgtggagtgggg
aggagcctgggcagcctgctgggtgggccacactcagcctgactgccctccatgggggcattctggcacccccctgctccaggacaggc
catgggcaagctgcctcccatcactgcctgctggctgctctcccaggggccaggtgtgaagagcagtgcccccgacacatgtattctcat
ctgtggtccaggccggcatcgtcctggccacccccccagatctggtgcctgctggccggcccccctggggtgcccctgccgaggtggcctgc
agtgctgtacatgtttacagaagctgctgggcttggctcaggatgtgttctgggcttgcaagccccccgcccaatcatgtgttcagtagccgtc
ctctgagcagggcccaaggcagccaggggcctggagggggccagaggagggtggggtcagggccgccccttctctgccttgtgcctctc
atgctgcctcctctgcccatgggtcctgggcacccaggcctgccctgcctgctggctacttcctggcttaccttctacccccaaggatcctcac
cacccaaagggtggtgggcactgctgtgaccaccccagctgcagagtcagtgccctgggtggaaggaaggcactgagagcccccttc
ctctgagggccccacctcaccccttggtgtcacccccaccacgcctaggcagctctgggccctgggatctggaaccaacacacccctgtt
cccctcagctttccctcctcgctggcctgggcaccctcctgggagcaggccttcctccctcccacccccaatgtcctgttggtaggaggtggg
gccaagagtggggtatggtgggccttggctggagacctctgtccactgcccagggaggggcctggggctgggagcagtcccggtttagc
ctgaggtccccatagggcttcctcccctgctgggtttgggaagcagttagggagatagcgacccggagtttccccagaagcggggtggg
agggtgtgcatgctagtgttggcgcgtatgcatgtgcatgagtgtgcaccgttcctaaggaaggggcctctggggctgcccaccctacctg
ccctgcctgcctgctgcccctcccagcctgccaagaaaacggtaggggagcatgatggggcctttgaggcagggtcgcagggacaag

ctcagctttaggcaccatctgttcccatcacgcctgctgctgtgacccgttttggaaaactggtgtgtaccgaggcgctgactgcacggctga
ccgcctgctcgtgccttcattctgcagcggcatggtccctcccattctggctccacctgcagcctccctgggtggcctaggctcccccgacca
agagacctccctctcatgatcactggtacctgggggcctgaattctggcccccggctccccacacagctgggactggcctggatggctgtc
ctgggagcccctgcccaccctgacggagctgggcctcccctcatcctctgtaactcccgccttcaccagactcaaggacaccctggccct
gctgaggcatacagagcttcagcccagcacagaagcaagacaaaatcagtggctcttagagtttagaaaacaagacagactctcagat
gaaagatctgacaagcaccgtggccagtcacagggagagacttgatgtctggccttttaattcctcctctgccagggtgggtcctgggacc
tctaatgtgggcatgtcgtccaccccaggacgagccatcagggacagacccccaccccccaaggctgcagccacaccatgtttcaggc
ttggggctggggcaggcttgggctcaatcctgggcacccaggggcagcccacccctaacctggctcctacccaccttgcccttgaaggat
gggcctgctgcacgtctccctcctccaccccataccacactggggggtctgagccaccccccctcagccccgttcggctcagaccgacccc
cactccatccccagacctgcagcacaagtgcgcgggcctgtcctcccaggggcctgggcgactccatatgcaatcagtagcgagcagc
cgggccccacagaccctcatgcactctcttacgtgccattctccccagactttttttgtacttaatgtatgaaagatccaaactaatattgctgta
aaaaggagagacaaattaatatagcttattctataaat (SEQ ID NO:219)

**Q9Y4D2; BAA31634**
ASAEPPAMPGIVVFRRRWSVGSDDLVLPAIFLFLLHTTWFVILSVVLFGLVYNPHEACSLNLVDHG
RGYLGILLSCMIAEMAIIWLSMRGGILYTEPRDSMQYVLYVRLAILVIEFIYAIVGIVWLTQYYTSCND
LTAKNVTLGMVVCNWVVILSVCITVLCVFDPTGRTFVKLRATKRRQRNLRTYNLRHRLEEGQATS
WSRRLKVFLCCTRTKDSQSDAYSEIAYLFAEFFRDLDIVPSDIIAGLVLLRQRQRAKRNAVLDEANN
DILAFLSGMPVTRNTKYLDLKNSQEMLRYKEVCYYMLFALAAYGWPMYLMRKPACGLCQLARSC
SCCLCPARPRFAPGVTIEEDNCCGCNAIAIRRHFLDENMTAVDIVYTSCHDAVYETPFYVAVDHDK
KKVVISIRGTLSPKDALTDLTGDAERLPVEGHHGTWLGHKGMVLSAEYIKKKLEQEMVLSQAFGR
DLGRGTKHYGLIVVGHSLGAGTAAILSFLLRPQYPTLKCFAYSPPGGLLSEDAMEYSKEFVTAVVL
GKDLVPRIGLSQLEGFRRQLLDVLQRSTKPKWRIIVGATKCIPKSELPEEVEVTTLASTRLWTHPSD
LTIALSASTPLYPPGRIIHVVHNHPAEQCCCCEQEEPTYFAIWGDNKAFNEVIISPAMLHEHLPYVV
MEGLNKVLENYNKGKTALLSAAKVMVSPTEVDLTPELIFQQQPLPTGPPMPTGLALELPTADHRN
SSVRSKSQSEMSLEGFSEGRLLSPVVAAAARQDPVELLLLSTQERLAAELQARRAPLATMESLSD
TESLYSFDSRRSSGFRSIRGSPSLHAVLERDEGHLFYIDPAIPEENPSLSSRTELLAADSLSKHSQD
TQPLEAALGSGGVTPERPPSAAANDEEEEVGGGGGGPASRGELALHNGRLGDSPSPQVLEFAE
FIDSLFNLDSKSSSFQDLYCMVVPESPTSDYAEGPKSPSQQEILLRAQFEPNLVPKPPRLFAGSAD
PSSGISLSPSFPLSSSGELMDLTPTGLSSQECLAADKIRTSTPTGHGASPAKQDELVISAR (SEQ
ID NO:220)

# Figure 100

**Nucleotide:** AF044209
**Sequence:**
ccaagatggcggccaaggtggcgaagcagcagccgcggcggcggcggctggagtgagcgtccgactcgccgcgccgaacga
ggtcccggtgtagggccgcgcgccgtggccgcgtcccactcctcaggccggggcgcacgtcggctcccacgcttagccagctcccggt
ggtttcctagaaacatgattgtttattggcattgatctcacagtctggtgaggacttctttactgataatgtcaagttcaggttatcctcccaacca
aggagcattcagcacagaacaaagtcgttatcctcctcactctgtccagtatacatttcccaacacccgccaccagcaggagttcgcagtc
cctgattatcgttcctctcatcttgaagtgagtcaggcatcacagcttttgcagcaacagcagcagcaacagcttcgaaggcgaccttccttg
ctttcagaatttcacccaggttctgacaggcctcaagaaaggagaactagttatgaaccgtttcatccaggcccatccccagtggatcatga
ttcactggaatcgaagcgaccacgtctggaacaggtttctgattctcattttcagcgtgtcagtgctgcggttttgcctttagtgcacccgctgcc
agaagggctgagggcttctgcagatgctaagaaggatccagcattcggaggcaaacatgaagctccatcctctccaatttcggggcaac
catgtggagatgatcaaaatgcttcaccttcaaaactctcaaaggaagagttaatacagagtatggatcgtgtagatcgagaaattgcaa
aagtagaacagcagatccttaaactgaaaaagaaacaacaacagcttgaagaagaggcagctaaacctcctgagcctgagaagccc
gtgtcccctcctcctgtggagcagaaacaccgcagtattgtccaaattatttatgatgagaatcggaaaaaagcagaagaagctcataaa
atttttgaaggtcttggcccaaaagttgaactgccactgtataaccagccatcagataccaaggtgtaccatgagaacatcaagacaaac
caggtgatgaggaaaaaactcattttatttttaaaagaagaaatcatgcaagaaaacaaagggaacaaaaaatctgccagcgttatgat
cagctcatggaggcatgggagaaaaaagtggacagaatagaaaataatcctcggaggaaagctaaagaaagcaaaacaagggaa
tactatgaaaagcagtttccagaaattcgaaaacaaagagaacagcaagaaagatttcagcgagttgggcagaggggagctggtcttt
cagccaccattgctaggagtgagcatgagatttctgaaattattgatggctctctgagcaggagaataatgagaaacaaatgcggcagc
tctctgtgattccacctatgatgtttgatgcagaacaaagacgagtcaagttcattaacatgaatgggcttatggaggaccctatgaaagtgt
ataaagataggcagtttatgaatgtttggactgaccatgaaaaggagatctttaaggacaagtttatccagcatccaaaaaactttggacta
attgcatcatacttggagaggaagagtgttcctgattgtgttttgtattactatttaaccaagaaaaatgagaattataaagccctcgtcagaa
ggaattatgggaaacgcagaggcagaaaccagcaaattgctcgaccctcgcaagaagaaaaagtagaagaaaaagaagaggata
aagcagaaaaaacagaaaaaaaagaagaagaaaagaaagatgaagaggaaaaagatgaaaaagaagactccaaagaaaata
ccaaggaaaaggacaagatagatggtacagcagaagaaactgaggaaagagagcaagccacaccccgggggcgaaagactgc
caacagtcagggccgccgtaagggccggatcaccaggtccatgacaaacgaagctgcagctgccagtgctgcagccgcagcggcta
ctgaagagcccccaccacctctgccaccgccaccagaacccatttctacagagcctgtggagacctctcgatggacagaagaagaaat
ggaagttgctaaaaaaggtctagtagaacatggtcgtaactgggcagcaattgctaaaatggtgggaacgaaaagtgaagctcaatgta
aaaacttctattttaactataaaaggcgacacaatcttgacaacctcttacagcagcataaacagaaaacttcacgaaaacctcgtgaag
agcgagatgtgtctcaatgtgaaagtgtcgcttccactgtttctgctcaggaggatgaagatattgaagcctccaatgaagaagaaaatcc
agaagacagcgaagttgaagctgtcaagcccagcgaggacagtcctgaaaatgctacttctcgaggaaacacagaacctgcggttga
gcttgagcccaccacggaaactgcacccagtacatctccctccttagcagttccaagtacaaaaccagctgaagatgaaagtgtggaga
cccaggtgaatgacagcatcagtgctgagacagcagagcagatggatgtagatcagcaggagcacagtgctgaagagggttctgtttgt
gatcccccacccgctaccaaagctgactctgtggacgttgaagtgagggtgccagaaaaccatgcatctaaagttgaaggtgataatac
caaagaaagagacttggatagagccagtgagaaggtggaacctagagatgaagatttggtggtagctcagcaaataaatgcccaaag
gcccgagccccagtcagacaatgattccagtgccacgtgcagcgctgatgaggatgtggatggagagccagagaggcagagaatgttt
cctatggactcaaagccttcactgttaaaccccactggatctatactcgtctcatctccgttaaaaccaaatccactggatctgccacagcttc
agcatcgagctgctgttatcccaccaatggtatcctgcaccccatgtaacataccaattggaaccccagtgagcggctatgctctctaccag
cgacacattaaagcaatgcatgagtcagcactcctggaggagcagcggcagagacaagaacagatagatttggaatgtagaagttcta
caagtccatgtggcacatccaagagtccaaacagagagtgggaagtccttcagcctgctccacatcaattgataactaatctccctgaag
gcgttcggcttccgacaactcgaccaaccaggccaccgcccccctctcatcccgtcatccaaaaccacagtggcttcagaaaaaccatctt
ttataatgggaggctccatctcacagggaacaccaggcacttatttgacttctcataatcaggcttcctacactcaagaaacacccaagcc
gtcagtaggatctatctctcttggactgccacggcaacaggaatctgccaaatcagctactttgccctacatcaagcaggaagaattttctcc
ccgaagccaaaactcacaacctgagggtctgttggtcagggcccaacatgaaggtgtagtcagaggtaccgcaggagccatacaaga
aggaagtataactcggggaactccaaccagcaaaatttcagtggagagcattccatccctacggggctctatcactcagggcaccccgg
ctctgccccagactggcataccaacagaggctttggtgaaggggtccatttcgagaatgcccattgaagacagcagtcctgagaaaggc
agagaggaagctgcatccaaaggccatgttatttatgaaggcaaaagtggacatatcttgtcatatgataatattaagaatgcccgagaa
gggactaggagtccaagaacagctcatgaaatcagtttaaagagaagctatgaatcagtggaaggaaatataaagcaagggatgtca
atgagggagtctcctgtatcagcaccgttagaggggctgatatgccgagcattacccaggggggagtcctcattctgacctcaaagaaagg
actgtattgtctggctccataatgcaggggacaccaagagcaacaactgaaagctttgaagatggccttaaatatcccaaacaaattaaa
agggaaagtcctcccatacgagcatttgaaggtgccattaccaaaggaaaaccatatgatggcatcaccaccatcaaagaaatggggc
gttccattcatgagattccaaggcaagatattttaactcaggaaagtcggaaaactccagaagtggtccagagcacacggccgataattg
agggttccatttcccagggcacaccaataaagtttgacaacaactcaggtcaatctgccatcaaacacaatgtcaaatccttaatcacggg
gcctagcaaactatcccgtggaatgcctccgctggaaattgtgccagagaacataaaagtggtagaacggggaaaatatgaggatgtg
aaagcaggcgagaccgtgcgttcccggcacacgtcagtggtaagctctggcccctccgttcttaggtccacactgcatgaagctcccaaa

gcacaactgagccctgggatttatgatgacaccagtgcacggaggacccctgtgagttatcaaaacaccatgtccagaggctcacccat
gatgaacagaacttctgatgttacaattcctcctaacaagtctaccaatcatgaaaggaaatcgacactgacccctacccagagggaaa
gtatcccagcgaagtctccagtgcctggggtggaccctgtcgtgagccacagtccgtttgatccccatcacagaggcagcactgcaggc
gaggtttattggagccacctgcccacgcaattggatccagccatgccttttcacagggctttggatcctgcagcggctgcttacctgtttcaga
gacagctttcaccaactccaggttacccaagtcagtatcagctttacgcaatggagaacacaagacagacaatcttaaatgattacattac
ctcacaacagatgcaagtgaacttgcgtccagatgtggccagaggactctccccaagagagcagccactgggtctcccatacccagca
acgagaggaatcattgacctgaccaatatgcctccaacaattttagtgcctcatccaggggggaacaagcactcctcccatggacagaatc
acttatattcctggtacacagattactttccctcccaggccgtacaactctgcttccatgtctccaggacacccaacacaccttgcagctgctg
caagtgctgagagggaacgggaacgggagcgggagaaggagcgggagcgggaacggattgctgcagcttcctccgacctctacctg
cggccaggctcagaacagcctggccgacctggcagtcatggatatgttcgctccccttcccttcagtaagaactcaggagaccatgttgc
aacagagacccagtgttttccaaggaaccaatggaaccagtgtaatcacacctttggatccaactgctcagctacgaatcatgccactgc
ctgctgggggcccttcaataagccaaggcctgccagcctcccgttacaacactgctgcggatgccctggctgctcttgtggatgctgcagct
tctgcaccccagatggatgtgtccaaaacaaaagagagtaagcatgaagctgccaggttagaagaaaatttgagaagcaggtcagca
gcagttagtgaacagcagcagctagagcagaaaaccctggaggtggagaagagatctgttcagtgtttatacacttcttcagcctttccaa
gtggcaagccccagcctcattcttcagtagtttattctgaggctgggaaagataaagggcctcctccaaaatccagatatgaggaagagct
aaggaccagagggaagactaccattactgcagctaacttcatagacgtgatcatcacccggcaaattgcctcggacaaggatgcgagg
gaacgtggctctcaaagttcagactcttctagtagcttatcttctcacaggtatgaaacacctagcgatgctattgaggtgataagtcctgcca
gctcacctgcgccaccccaggagaaactgcagacctatcagccagaggttgttaaggcaaatcaagcggaaaatgatcctaccagac
aatatgaaggaccattacatcactatcgaccacagcaggaatcaccatctccccaacaacagctgccccccttcttcacaggcagaggga
atggggcaagtgcccaggacccatcggctgatcacacttgctgatcacatctgtcaaattatcacacaagatttgctagaaatcaagtttc
ctcgcagactccccagcagcctcctacttctacattccagaactcaccttctgctttggtatctacacctgtgaggactaaaacatcaaaccg
ttacagcccagaatcccaggctcagtctgtccatcatcaaagaccaggttcaagggtctctccagaaaatcttgtggacaaatccagggg
aagtaggcctggaaaatccccagagaggagtcacgtctcttccgagccctacgagcccatctccccaccccaggttccggttgtgcatga
gaaacaggacagcttgctgctcttgtctcagaggggcgcagagcctgcagagcagaggaatgatgcccgctcaccagggagtataag
ctacttgccttcattcttcaccaagcttgaaaatacatcacccatggttaaatcaaagaagcaggagatttttcgtaagttgaactcctctggtg
gaggtgactctgatatggcagctgctcagccaggaactgagatctttaatctgccagcagttactacgtcaggctcagttagctctagaggc
cattcttttgctgatcctgccagtaatcttgggctggaagacattatcaggaaggctctcatgggaagctttgatgacaaagttgaggatcatg
gagttgtcatgtcccagcctatgggagtagtgcctggtactgccaacacctcagttgtgaccagtggtgagacacgaagagaggaaggg
gacccatcacctcattcaggaggagtttgcaaaccaaagctgatcagcaagtcaaacagcaggaaatctaagtctcctatacctgggca
aggctacttaggaacggaacggccctcttcagtctcctctgtacattcagaaggggattaccataggcagacgccagggtgggcctggg
aagacaggccctcttcaacaggctcaactcagtttccttataaccctctgactatgcggatgctcagcagtactccaccaacaccgattgca
tgtgctccctctgcggtgaaccaagcagctcctcaccaacagaacaggatctgggagcgagagcctgccccactgctctcagcacagta
cgagaccctgtcggatagtgatgactgaactgcacaaagtgaggggaacagggtgcaggagagggatctctagtttttgtggtttaattttt
agtagcaggtcaaaaacctgccctcctgtgacttattccctgagacttttcaggagagccagcccacagatgatgaagaaatgatggaag
ttcatttggagagtcaaatgggaaaaaaacaaacaaaaaactgcctttgatacaggcaattcagtggactataataatagtggagggttg
agatgtagagtttttaaaaagtgaacagttgctgttcttacatctgtaaagaaaaccataatgtctttaaatcactcttctgtaaatagatgacct
ttttgcagtgtaaaaaaaaaaaaaaaaaaaaaaaaa (SEQ ID NO:221)

**O75376**

MSSSGYPPNQGAFSTEQSRYPPHSVQYTFPNTRHQQEFAVPDYRSSHLEVSQASQLLQQQQQQ
QLRRRPSLLSEFHPGSDRPQERRTSYEPFHPGPSPVDHDSLESKRPRLEQVSDSHFQRVSAAVL
PLVHPLPEGLRASADAKKDPAFGGKHEAPSSPISGQPCGDDQNASPSKLSKEELIQSMDRVDREI
AKVEQQILKLKKKQQQLEEEAAKPPEPEKPVSPPPVEQKHRSIVQIIYDENRKKAEEAHKIFEGLGP
KVELPLYNQPSDTKVYHENIKTNQVMRKKLILFFKRRNHARKQREQKICQRYDQLMEAWEKKVDR
IENNPRRKAKESKTREYYEKQFPEIRKQREQQERFQRVGQRGAGLSATIARSEHEISEIIDGLSEQ
ENNEKQMRQLSVIPPMMFDAEQRRVKFINMNGLMEDPMKVYKDRQFMNVWTDHEKEIFKDKFIQ
HPKNFGLIASYLERKSVPDCVLYYYLTKKNENYKALVRRNYGKRRGRNQQIARPSQEEKVEEKEE
DKAEKTEKKEEEKKDEEEKDEKEDSKENTKEKDKIDGTAEETEEREQATPRGRKTANSQGRRKG
RITRSMTNEAAAASAAAAAATEEPPPPLPPPPEPISTEPVETSRWTEEEMEVAKKGLVEHGRNWA
AIAKMVGTKSEAQCKNFYFNYKRRHNLDNLLQQHKQKTSRKPREERDVSQCESVASTVSAQEDE
DIEASNEEENPEDSEVEAVKPSEDSPENATSRGNTEPAVELEPTTETAPSTSPSLAVPSTKPAEDE
SVETQVNDSISAETAEQMDVDQQEHSAEEGSVCDPPPATKADSVDVEVRVPENHASKVEGDNTK
ERDLDRASEKVEPRDEDLVVAQQINAQRPEPQSDNDSSATCSADEDVDGEPERQRMFPMDSKP
SLLNPTGSILVSSPLKPNPLDLPQLQHRAAVIPPMVSCTPCNIPIGTPVSGYALYQRHIKAMHESAL
LEEQRQRQEQIDLECRSSTSPCGTSKSPNREWEVLQPAPHQLITNLPEGVRLPTTRPTRPPPPLIP
SSKTTVASEKPSFIMGGSISQGTPGTYLTSHNQASYTQETPKPSVGSISLGLPRQQESAKSATLPY
IKQEEFSPRSQNSQPEGLLVRAQHEGVVRGTAGAIQEGSITRGTPTSKISVESIPSLRGSITQGTPA

LPQTGIPTEALVKGSISRMPIEDSSPEKGREEAASKGHVIYEGKSGHILSYDNIKNAREGTRSPRTA
HEISLKRSYESVEGNIKQGMSMRESPVSAPLEGLICRALPRGSPHSDLKERTVLSGSIMQGTPRAT
TESFEDGLKYPKQIKRESPPIRAFEGAITKGKPYDGITTIKEMGRSIHEIPRQDILTQESRKTPEVVQ
STRPIIEGSISQGTPIKFDNNSGQSAIKHNVKSLITGPSKLSRGMPPLEIVPENIKVVERGKYEDVKA
GETVRSRHTSVVSSGPSVLRSTLHEAPKAQLSPGIYDDTSARRTPVSYQNTMSRGSPMMNRTSD
VTIPPNKSTNHERKSTLTPTQRESIPAKSPVPGVDPVVSHSPFDPHHRGSTAGEVYWSHLPTQLD
PAMPFHRALDPAAAAYLFQRQLSPTPGYPSQYQLYAMENTRQTILNDYITSQQMQVNLRPDVAR
GLSPREQPLGLPYPATRGIIDLTNMPPTILVPHPGGTSTPPMDRITYIPGTQITFPPRPYNSASMSP
GHPTHLAAAASAERERERERERERERERIAAASSDLYLRPGSEQPGRPGSHGYVRSPSPSVRTQ
ETMLQQRPSVFQGTNGTSVITPLDPTAQLRIMPLPAGGPSISQGLPASRYNTAADALAALVDAAAS
APQMDVSKTKESKHEAARLEENLRSRSAAVSEQQQLEQKTLEVEKRSVQCLYTSSAFPSGKPQP
HSSVVYSEAGKDKGPPPKSRYEEELRTRGKTTITAANFIDVIITRQIASDKDARERGSQSSDSSSSL
SSHRYETPSDAIEVISPASSPAPPQEKLQTYQPEVVKANQAENDPTRQYEGPLHHYRPQQESPSP
QQQLPPSSQAEGMGQVPRTHRLITLADHICQIITQDFARNQVSSQTPQQPPTSTFQNSPSALVST
PVRTKTSNRYSPESQAQSVHHQRPGSRVSPENLVDKSRGSRPGKSPERSHVSSEPYEPISPPQV
PVVHEKQDSLLLLSQRGAEPAEQRNDARSPGSISYLPSFFTKLENTSPMVKSKKQEIFRKLNSSG
GGDSDMAAAQPGTEIFNLPAVTTSGSVSSRGHSFADPASNLGLEDIIRKALMGSFDDKVEDHGVV
MSQPMGVVPGTANTSVVTSGETRREEGDPSPHSGGVCKPKLISKSNSRKSKSPIPGQGYLGTER
PSSVSSVHSEGDYHRQTPGWAWEDRPSSTGSTQFPYNPLTMRMLSSTPPTPIACAPSAVNQAA
PHQQNRIWEREPAPLLSAQYETLSDSDD (SEQ ID NO:222)

# Figure 101

**Nucleotide:** U28936
**Sequence:**
atggatgatcgagaggatctggtgtaccaggcgaagctggccgagcaggctgagcgatacgacgaaatggtggagtcaatgaagaa
agtagcagggatggatgtggagctgacagttgaagaaagaaacctcctatctgttgcatataagaatgtgattggagctagaagagcctc
ctggagaataatcagcagcattgaacagaaagaagaaaacaagggaggagaagacaagctaaaaatgattcgggaatatcggcaa
atggttgagactgagctaaagttaatctgttgtgacattctggatgtactggacaaacacctcattccagcagctaacactggcgagtccaa
ggttttctattataaaatgaaaggggactaccacaggtatctggcagaatttgccacaggaaacgacaggaaggaggctgcggagaac
agcctagtggcttataaagctgctagtgatattgcaatgacagaacttccaccaacgcatcctattcgcttaggtcttgctctcaatttttccgta
ttctactacgaaattcttaattcccctgaccgtgcctgcaggttggcaaaagcagcttttgatgatgcaattgcagaactggatacgctgagtg
aagaaagctataaggactctacacttatcatgcagttgttacgtgataatctgacactatggacttcagacatgcagggtgacggtgaaga
gcagaataaagaagcgctgcaggacgtggaagacgaaaatcagtgagacataagccaacaagagaaacca (SEQ ID NO:223)

**P42655**
MDDREDLVYQAKLAEQAERYDEMVESMKKVAGMDVELTVEERNLLSVAYKNVIGARRASWRIISS
IEQKEENKGGEDKLKMIREYRQMVETELKLICCDILDVLDKHLIPAANTGESKVFYYKMKGDYHRY
LAEFATGNDRKEAAENSLVAYKAASDIAMTELPPTHPIRLGLALNFSVFYYEILNSPDRACRLAKAA
FDDAIAELDTLSEESYKDSTLIMQLLRDNLTLWTSDMQGDGEEQNKEALQDVEDENQ (SEQ ID NO:224)

# Figure 102

**Nucleotide:** AJ299442
**Sequence:**
gttatcctgtacaggtggagctggtcgccgccgcggcgctggaattgtgggagttgtgtctgccactcggctgccggaggccgaaggtccc
tgactatggctccccagagcctgccttcatctaggatggctcctctgggcatgctgcttgggctgctgatggccgcctgcttcaccttctgcctc
agtcatcagaacctgaaggagtttgccctgaccaacccagagaagagcagcaccaaagaaacggagagagaaaagaaaccaaagcc
gaggaggagctggatgccgaagtcctggaggtgttccacccgacgcatgagtggcaggcccttcagccagggcaggctgtccctgcag
gatcccacgtacggctgaatcttcagactggggaaagagaggcaaaactccaatatgaggacaagttccgaaataatttgaaaggcaa
aaggctggatatcaacaccaacacctacacatctcaggatctcaagagtgcactggcaaaattcaaggagggggcagagatggaga
gttcaaaggaagacaaggcaaggcaggctgaggtaaagcggctcttccgccccattgaggaactgaagaaagactttgatgagctga
atgttgtcattgagactgacatgcagatcatggtacggctgatcaacaagttcaatagttccagctccagtttggaagagaagattgctgcg
ctctttgatcttgaatattatgtccatcagatggacaatgcgcaggacctgctttcctttggtggtcttcaagtggtgatcaatgggctgaacagc
acagagcccctcgtgaaggagtatgctgcgtttgtgctgggcgctgcctttttccagcaaccccaaggtccaggtggaggccatcgaaggg
ggagccctgcagaagctgctggtcatcctggccacggagcagccgctcactgcaaagaagaaggtcctgtttgcactgtgctccctgctg
cgccacttcccctatgcccagcggcagttcctgaagctcggggggctgcaggtcctgaggaccctggtgcaggagaagggcacggag
gtgctcgccgtgcgcgtggtcacactgctctacgacctggtcacggagaagatgttcgccgaggaggaggctgagctgacccaggaga
tgtccccagagaagctgcagcagtatcgccaggtacacctcctgccaggcctgtgggaacagggctggtgcgagatcacggcccacct
cctggcgctgcccgagcatgatgcccgtgagaaggtgctgcagacactgggcgtcctcctgaccacctgccgggaccgctaccgtcag
gacccccagctcggcaggacactggccagcctgcaggctgagtaccaggtgctggccagcctggagctgcaggatggtgaggacga
gggctacttccaggagctgctgggctctgtcaacagcttgctgaaggagctgagatgaggccccacaccaggactggactgggatgcc
gctagtgaggctgaggggtgccagcgtgggtgggcttctcaggcaggaggacatcttggcagtgctggcttggccattaaatggaaacct
gaaggccnnnnnaaaaaaaaaaaannnaanannaaaaaaanaaaaaaaanaaaaaaaaaaaannanaaaaaaaaaaaa
nnnaaaaaaaaaaaaaaaaaa (SEQ ID NO:225)

**Q9H173**
MAPQSLPSSRMAPLGMLLGLLMAACFTFCLSHQNLKEFALTNPEKSSTKETERKETKAEEELDAE
VLEVFHPTHEWQALQPGQAVPAGSHVRLNLQTGEREAKLQYEDKFRNNLKGKRLDINTNTYTSQ
DLKSALAKFKEGAEMESSKEDKARQAEVKRLFRPIEELKKDFDELNVVIETDMQIMVRLINKFNSSS
SSLEEKIAALFDLEYYVHQMDNAQDLLSFGGLQVVINGLNSTEPLVKEYAAFVLGAAFSSNPKVQV
EAIEGGALQKLLVILATEQPLTAKKKVLFALCSLLRHFPYAQRQFLKLGGLQVLRTLVQEKGTEVLA
VRVVTLLYDLVTEKMFAEEEAELTQEMSPEKLQQYRQVHLLPGLWEQGWCEITAHLLALPEHDAR

EKVLQTLGVLLTTCRDRYRQDPQLGRTLASLQAEYQVLASLELQDGEDEGYFQELLGSVNSLLKE
LR (SEQ ID NO:226)

# Figure 103

**Nucleotide:** AB034951
**Sequence:**
gcttccttcgttattggagccaggcctacaccccagcaaccatgtccaagggacctgcagttggtattgatcttggcaccacctactcttgtgt
gggtgttttccagcacggaaaagtcgagataattgccaatgatcagggaaaccgaaccactccaagctatgtcgcctttacggacactga
acggttgatcggtgatgccgcaaagaatcaagttgcaatgaaccccaccaacacagtttttgatgccaaacgtctgattggacgcagattt
gatgatgctgttgtccagtctgatatgaaacattggccctttatggtggtgaatgatgctggcaggcccaaggtccaagtagaatacaaggg
agagaccaaaagcttctatccagaggaggtgtcttctatggttctgacaaagatgaaggaaattgcagaagcctaccttgggaagactgtt
accaatgctgtggtcacagtgccagcttactttaatgactctcagcgtcaggctaccaaagatgctggaactattgctggtctcaatgtactta
gaattattaatgagccaactgctgctgctattgcttacggcttagacaaaaaggttggagcagaaagaaacgtgctcatctttgacctggga
ggtggcacttttgatgtgtcaatcctcactattgaggatggaatctttgaggtcaagtctacagctggagacacccacttgggtggagaagat
tttgacaaccgaatggtcaaccattttattgctgagtttaagcgcaagcataagaaggacatcagtgagaacaagagagctgtaagacgc
ctccgtactgcttgtgaacgtgctaagcgtaccctctcttccagcacccaggccagtattgagatcgattctctctatgaaggaatcgacttct
atacctccattacccgtgcccgatttgaagaactgaatgctgacctgttccgtggcaccctggacccagtagagaaagcccttcgagatgc
caaactagacaagtcacagattcatgatattgtcctggttggtggttctactcgtatccccaagattcagaagcttctccaagacttcttcaatg
gaaaagaactgaataagagcatcaaccctgatgaagctgttgcttatggtgcagctgtccaggcagccatcttgtctggagacaagtctg
agaatgttcaagatttgctgctcttggatgtcactcctctttcccttggtattgaaactgctggtggagtcatgactgtcctcatcaagcgtaatac
caccattcctaccaagcagacacagaccttcactacctattctgacaaccagcctggtgtgcttattcaggtttatgaaggcgagcgtgcca
tgacaaaggataacaacctgcttggcaagtttgaactcacaggcatgccaggaggaatgcctggggatttcctggtggtggagctcctc
cctctggtggtgcttcctcagggcccaccattgaagaggttgattaagccaaccaagt (SEQ ID NO:227)

**P11142**
MSKGPAVGIDLGTTYSCVGVFQHGKVEIIANDQGNRTTPSYVAFTDTERLIGDAAKNQVAMNPTN
TVFDAKRLIGRRFDDAVVQSDMKHWPFMVVNDAGRPKVQVEYKGETKSFYPEEVSSMVLTKMK
EIAEAYLGKTVTNAVVTVPAYFNDSQRQATKDAGTIAGLNVLRIINEPTAAAIAYGLDKKVGAERNV
LIFDLGGGTFDVSILTIEDGIFEVKSTAGDTHLGGEDFDNRMVNHFIAEFKRKHKKDISENKRAVRR
LRTACERAKRTLSSSTQASIEIDSLYEGIDFYTSITRARFEELNADLFRGTLDPVEKALRDAKLDKSQ
IHDIVLVGGSTRIPKIQKLLQDFFNGKELNKSINPDEAVAYGAAVQAAILSGDKSENVQDLLLLDVTP
LSLGIETAGGVMTVLIKRNTTIPTKQTQTFTTYSDNQPGVLIQVYEGERAMTKDNNLLGKFELTGIP
PAPRGVPQIEVTFDIDANGILNVSAVDKSTGKENKITITNDKGRLSKEDIERMVQEAEKYKAEDEKQ
RDKVSSKNSLESYAFNMKATVEDEKLQGKINDEDKQKILDKCNEIINWLDKNQTAEKEEFEHQQK
ELEKVCNPIITKLYQSAGGMPGGMPGGFPGGGAPPSGGASSGPTIEEVD (SEQ ID NO:228)

# Figure 104

**Nucleotide:** AX067150
**Sequence:**
ggttgggctccttggtaccatgtgggaagcgctgtgaagagttgttgccttccaagatatacccaaattcccagttccagcccgtgtcattaa
aactccgctggcgtgaaagatgacgtccttagcccagcagctgcaacgactcgccctccctcaaagtgatgccagcctcttatcyagaga
tgaagttgcttctttgttatttgaccctaaggaagcggccacaatcgacagggacaccgccttcgccattggatgtactggcctggaagagtt
gcttggaattgatccttcctttgagcagtttgaagcaccgttgttcagtcagctagcaaaaaccttggagcgaagtgttcagaccaaagcagt
aaacaaacagttggatgaaaacatttcattattccttattcacttgtcgccttacttcctgcttaagccagcacagaagtgtctggagtggttgat
tcacaggttccatatacatctctataatcaagatagcctcattgcttgtgttctgccataccacgagacaagaatatttgtgcgagtcatacag
cttctaaaaattaataattcaaagcacagatggttctggttgttgccagttaagcaatctggagtgccgttagctaaaggaactttgattaccc
actgctacaaagatcttggattcatggatttcatttgcagtttggtgacaaaatctgtgaaggtttttgctgagtacccgggcagctcagctcag
ttgagggtgctcttggctttctatgcttctaccatagtgtcggcgctggtagctgcagaggacgtatcagacaatatcatcgccaaactatttcc
ctatatccaaaagggattgaaatcatctttaccagattacagagctgcaacatacatgataatatgtcagatttctgtgaaagtgaccatgga
aaatacctttgtgaattcattggcatcacagatcatcaaaacattgaccaagattccctctttgatcaaggatgggttaagttgcttgatagtgc
tcctgcagagacagaagccagagagccttgggaaaaagccattccctcacttatgtaatgttcctgatcttattacaatacttcatgggatttc
tgaaacttacgatgtcagtcctcttctgcgttacatgcttccccatctggtcgtctccatcattcatcatgttacaggagaagaaactgaagga
atggatggtcaatctacaagagacacttagaagctatacttacaaaaatatcactgaagaacaacttagaccatttgttggctagccttct
atttgaagagtatatttcatatagttcacaggaagaaatggattctaataaagtgtctttgcttaatgaacaatttcttccactcattagacttttag
aaagcaaataccccagaacattagatgttgtattagaggaacacttaaaggaaattgcagatctgaaaaaacaagagcttttccatcagtt
tgtttctctttctacaagtggaggaaagtatcagtttttagcagattctgatacttctttgatgctcagcctgaatcatccacttgctcctgtgagaat
tctggccatgaatcatttgaaaaagatcatgaaaacatcaaaggagggtgttgatgaatctttcataaaagaagctgttttagcccgattag
gtgatgataatatagatgttgttttgtcggctataagtgcttttgagattttcaaagaacacttcagttcagaagtgacgatttcaaatcttctgaat
ctctttcaaagagcagaactttcaaagaatggagaatggtacgaggtacttaagatagccgctgacatattaattaaagaagagatactg
agtgaaaatgatcagttgtcaaatcaggtggttgtatgtttgctgccatttgtggttatcaataatgatgatacggaatctgctgagatgaaaatt
gctatatatttatcaaaatcaggaatctgctccctgcaccctctattaagaggctgggaagaagctcttgaaaatgtaattaaaagcacaaa
gccaggaaaactaatcggtgtagcaaatcagaagatgattgagttgttggctgataatataaatttaggagatccttcttcaatgttaaagat
ggtggaggatttgataagcgtgggtgaggaggagtcctttaacctgaagcagaaagtaacgtttcatgtgatcctgtctgtgctcgtctcttgtt
gttcatctttaaaagaaacccactttccatttgcgataagagtcttcagtttgttgcagaaaaaaataaagaagcttgaaagtgtcattactgc
agtggaaatcccctcagaatggcacattgaactgatgttagacagagggatcccagtagagctgtgggcacattatgtagaagagctca
acagcactcagagggtggccgtggaggactcggtttttcttgtattttccttgaaaaaatttatttatgcactgaaagctcctaaatcttttcctaa
aggtgatatatggtggaatcctgaacaactgaaagaagacagcagggactatctgcacttgctcattgggctgtttgagatgatgctcaatg
gtgccgatgctgttcatttcagagttctgatgaaacttttcataaaggtkcatctagaagatgtttttcagttattcaagttctgttctgttttatggac
ctatggttctagcctttcaaatccactaaactgcagtgtgaaaacagtgctgcagactcaagctctttatgtgggctgtgcaatgctttcttctca
gaagacacagtgtaaacaccaactggcatccatatcttctccagtggtgacatctttactcattaacctgggaagccccgtaaaagaagtt
cgtagggctgccattcagtgtctccaggccctcagtggagtggcatccccgttttatctgataatagatcatttgatttctaaagcagaggaga
tcacttcagatgctgcctatgttattcaggatttggctactttatttgaggaactacagagagaaaagaaactgaaatctcatcagaagttgtct
gaaactttgaaaaacttacttagttgtgtgtatagttgcccatcttatatagcaaaagatttgatgaaagtacttcagggagtcaacggtgaga
tggtgctttctcagctattgcctatggctgaacaactgctagaaaagatccagaaggagcccacagctgtgctgaaagatgaggccatggt
tctgcatctcactctgggaaagtataatgaattttcagtttcccttttaaatgaggatccgaagagtctagatatatttataaaagctgtgcacac
aacaaaggaactttacgcgggaatgccaaccattcagatcacagcccttgaaaagattacaaaaccatttttgcagccatatcagatga
aaaagttcagcagaagcttttaagaatgttgtttgatttattggtgaactgtaaaaactcacattgtgctcagactgtcagcagtgtttttaaagg
gatttccgttaatgctgaacaagtccgaatagaactggagccaccagataaagctaaacccttgggcacagttcagcaaaaaagaagg
caaaaaatgcagcagaaaaaatcacaagatctagaatctgttcaggaagttggaggttcttactggcaaagagtaactctcatcctggaa
ttactgcagcacaaaaagaagctcagaagtcctcagatattggtgccaactctttttaacttgctatcaagatgtttagaacccttgccacaa
gagcagggaaatatggaatacaccaaacaattaattcttagttgtctgctcaacatctgccaaaaactatctccagatggtggcaaaatac
ccaaagatattttagatgaggagaagttcaacgtggagttgatagttcagtgcatccgcctttcggagatgccgcagacccatcaccatgc
ccttttacttttgggcactgttgctggaatatttccggataaagttttacacaatatcatgtctatttttacatttatgggagccaatgtcatgcgcct
agatgatacttacagttttcaagttattaacaagacagtgaaaatggttattcccgcacttattcagtctgatagtggagattctatagaagtttc
aagaaacgttgaagagattgtggtaaaaatcattagtgtatttgtggatgcgctgccacacgtcccggagcacaggcgcctgcccatcctt
gttcaacttgttgatacactgggtgcagagaaattcctctggattctcctcatcttgctttttgaacagtatgtcacaaaaacagtgctggcggct
gcctatggcgaaaaggatgctattttagaagcagacactgaattttggttttcagtctgttgtgagtttagtgtccagcatcagatacaaagctt
gatgaatatcctccagtacttactaaagctgccagaggaaaaagaagaaaccattcccaaagcagtgtcatttaataagagtgaatcac
aagaagaaatgctacaggtttttaatgtagagactcacactagcaagcaactgcggcattttaaatttttgtcagtgtccttcatgtctcagctc
ctgtcttccaataattttctgaaaaaggtagttgagagtggtggtcctgagattttaaaaggccttgaagagaggttgctggagaccgttctcg
gctatatcagtgcagttgcacagtccatggaaaggaacgcagacaaactcaccgtgaagttctggcgcgcgctccttagtaaagcttacg

acctgttagataaggtcaatgccttgctgcccacagagacattcattcctgtgatcagagggctggtgggcaatcccctgccatctgttcgcc
gcaaagcgctggaccttttgaataacaagctgcagcaaaatatatcctggaagaagacaatagttacccgtttcctaaaactggttccaga
ccttttggccattgtgcagcgtaagaaaaaggaaggggaagaagaacaagcaatcaacagacagacagcgttgtataccttaaagcttt
tatgcaagaattttggtgcagaaaatccagatccttttgtcccagtgctgarcactgctgtgaaactgattgctccagagagaaaggaggag
aagaatgtcytgggaagcgcgctgctgtgcatagcagaggtgacctccaccctggaggcgctggccatcccccagcttcccagcctgat
gccatcgttgctgacaacaatgaagaacaccagcgagctggtctccagcgaggtctacctgctcagtgccttggctgctctgcagaaggtt
gtggagactctcccgcacttcatcagcccctatctggaaggcattctctcccaggtgattcatctggagaaaatcactagtgaaatgggttct
gcgtcacaggctaatatccgtctcacatctcttaaaaagacactggctaccacacttgcaccccgagtcctgttgcccgccatcaaaaaaa
cttacaagcagattgagaagaactggaagaatcacatgggtccgtttatgagcatcttgcaagagcatattggggygatgaagaaggaa
gagctcacctcccatcagtctcagctaaccgcctttttcctggargccctggacttccgagcccagcactctgagaacgatctggaggaagt
tggaaaaacggaaaattgtatcattgactgtctagtagccatggttgtcaaactttccgaggtcacattcaggcccctgttcttcaagctgtttg
attgggctaaaacagaagatgccccaaaggacaggttgttgacatttacaacttggcagattgcattgctgaaaagctgaaagggcttttt
actctgtttgccggccacttagtgaagcctttgctgacaccttgraccaggtgaacatctccaaaacagatgaagcatttttgactctgaaa
atgaccctgaaaagtgctgcttgctgttgcagtttattttgaactgtttatacaaaatcttccttttgatacccagcattttataagtaaagagaga
gcagragccttgatgatgcctctggtggatcagctggaaaacaggcttgggggagaagagaaattccaggaacgggtgacaaagcac
ctgataccatgcatcgcacagttttcrgtggccatggcggatgactctcttggaaaccactgaactaccagattctgctaaagacgagaga
ctcctcgcctaaggttcgatttgctgctttgattactgtgttagcactggctgaaaaactaaaggagaattatattgtcttgctaccagaatccatt
cctttcttagcagagttgatggaagatgaatgtgaagaagtagaacatcagtgccaaaagactattcagcaactggaaactgtcctggga
gagccactccagagctatttctaagactttctgtggtgtttcatactctactcagagttcacactcatatttcatatttttatttttgggtgttgggtgcc
atgttacttttggtgccttaatacacctacttggattacttacaaatgttttatcacttcgttacaaaatccccacctggcttgtgctgccacataag
cctctcctgcctatcgtatagagctgcagaaagagtaaatgatacacggtatttttatac (SEQ ID NO:229)

**Q9H583**
MTSLAQQLQRLALPQSDASLLSRDEVASLLFDPKEAATIDRDTAFAIGCTGLEELLGIDPSFEQFEA
PLFSQLAKTLERSVQTKAVNKQLDENISLFLIHLSPYFLLKPAQKCLEWLIHRFHIHLYNQDSLIACV
LPYHETRIFVRVIQLLKINNSKHRWFWLLPVKQSGVPLAKGTLITHCYKDLGFMDFICSLVTKSVKV
FAEYPGSSAQLRVLLAFYASTIVSALVAAEDVSDNIIAKLFPYIQKGLKSSLPDYRAATYMIICQISVK
VTMENTFVNSLASQIIKTLTKIPSLIKDGLSCLIVLLQRQKPESLGKKPFPHLCNVPDLITILHGISETY
DVSPLLRYMLPHLVVSIIHHVTGEETEGMDGQIYKRHLEAILTKISLKNNLDHLLASLLFEEYISYSSQ
EEMDSNKVSLLNEQFLPLIRLLESKYPRTLDVVLEEHLKEIADLKKQELFHQFVSLSTSGGKYQFLA
DSDTSLMLSLNHPLAPVRILAMNHLKKIMKTSKEGVDESFIKEAVLARLGDDNIDVVLSAISAFEIFK
EHFSSEVTISNLLNLFQRAELSKNGEWYEVLKIAADILIKEEILSENDQLSNQVVVCLLPFVVINNDD
TESAEMKIAIYLSKSGICSLHPLLRGWEEALENVIKSTKPGKLIGVANQKMIELLADNINLGDPSSML
KMVEDLISVGEEESFNLKQKVTFHVILSVLVSCCSSLKETHFPFAIRVFSLLQKKIKKLESVITAVEIP
SEWHIELMLDRGIPVELWAHYVEELNSTQRVAVEDSVFLVFSLKKFIYALKAPKSFPKGDIWWNPE
QLKEDSRDYLHLLIGLFEMMLNGADAVHFRVLMKLFIKVHLEDVFQLFKFCSVLWTYGSSLSNPLN
CSVKTVLQTQALYVGCAMLSSQKTQCKHQLASISSPVVTSLLINLGSPVKEVRRAAIQCLQALSGV
ASPFYLIIDHLISKAEEITSDAAYVIQDLATLFEELQREKKLKSHQKLSETLKNLLSCVYSCPSYIAKD
LMKVLQGVNGEMVLSQLLPMAEQLLEKIQKEPTAVLKDEAMVLHLTLGKYNEFSVSLLNEDPKSL
DIFIKAVHTTKELYAGMPTIQITALEKITKPFFAAISDEKVQQKLLRMLFDLLVNCKNSHCAQTVSSV
FKGISVNAEQVRIELEPPDKAKPLGTVQQKRRQKMQQKKSQDLESVQEVGGSYWQRVTLILELLQ
HKKKLRSPQILVPTLFNLLSRCLEPLPQEQGNMEYTKQLILSCLLNICQKLSPDGGKIPKDILDEEKF
NVELIVQCIRLSEMPQTHHHALLLLGTVAGIFPDKVLHNIMSIFTFMGANVMRLDDTYSFQVINKTV
KMVIPALIQSDSGDSIEVSRNVEEIVVKIISVFVDALPHVPEHRRLPILVQLVDTLGAEKFLWILLILLF
EQYVTKTVLAAAYGEKDAILEADTEFWFSVCCEFSVQHQIQSLMNILQYLLKLPEEKEETIPKAVSF
NKSESQEEMLQVFNVETHTSKQLRHFKFLSVSFMSQLLSSNNFLKKVVESGGPEILKGLEERLLET
VLGYISAVAQSMERNADKLTVKFWRALLSKAYDLLDKVNALLPTETFIPVIRGLVGNPLPSVRRKAL
DLLNNKLQQNISWKKTIVTRFLKLVPDLLAIVQRKKKEGEEEQAINRQTALYTLKLLCKNFGAENPD
PFVPVLNTAVKLIAPERKEEKNVLGSALLCIAEVTSTLEALAIPQLPSLMPSLLTTMKNTSELVSSEV
YLLSALAALQKVVETLPHFISPYLEGILSQVIHLEKITSEMGSASQANIRLTSLKKTLATTLAPRVLLP
AIKKTYKQIEKNWKNHMGPFMSILQEHIGVMKKEELTSHQSQLTAFFLEALDFRAQHSENDLEEVG
KTENCIIDCLVAMVVKLSEVTFRPLFFKLFDWAKTEDAPKDRLLTFYNLADCIAEKLKGLFTLFAGHL
VKPFADTLNQVNISKTDEAFFDSENDPEKCCLLLQFILNCLYKIFLFDTQHFISKERAEALMMPLVD
QLENRLGGEEKFQERVTKHLIPCIAQFSVAMADDSLWKPLNYQILLKTRDSSPKVRFAALITVLALA
EKLKENYIVLLPESIPFLAELMEDECEEVEHQCQKTIQQLETVLGEPLQSYF (SEQ ID NO:230)

# Figure 105

**Nucleotide:** BC012547

**Sequence:**

ggggcttttcagtcgggcgctgagtggtttttcggatcatgtctggtggctccgcggattataacagcagagaacatggcggcccagaggg
aatggaccccgatggtgtcatcgagagcaactggaatgagattgttgataactttgatgatatgaatttaaaggagtctctccttcgtggcatc
tatgcttacggttttgagaagccttccgctattcagcagagagctattattccctgtattaaagggtatgatgtgattgctcaagctcagtcaggt
actggcaagacagccacatttgccatttccatcctgcaacagttggagattgagttcaaggagacccaagcactagtattggccccccacc
agagaactggctcaacagatccaaaaggtaattctggcacttggagactatatgggagccacttgtcatgcctgcattggtggaacaaat
gttcgaaatgaaatgcaaaaactgcaggctgaagcaccacatattgttgttggtacacccgggagagtgtttgatatgttaaacagaagat
acctttctccaaaatggatcaaaatgtttgttttggatgaagcagatgaaatgttgagccgtggttttaaggatcaaatctatgagattttccaa
aaactaaacacaagtattcaggttgtgttgctttctgccacaatgccaactgatgtgttggaagtgaccaaaaaattcatgagagatccaatt
cgaattctggtgaaaaaggaagaattgacccttgaaggaatcaaacagttttatattaatgttgagagagaggaatggaagttggataca
ctttgtgacttgtacgagacactgaccattacacaggctgttatttttctcaatacgaggcgcaaggtggactggctgactgagaagatgcat
gccagagacttcacagtttctgctctgcatggtgacatggaccagaaggagagagatgttatcatgagggaattccggtcagggtcaagt
cgtgttctgatcactactgacttgttggctcgcgggattgatgtgcaacaagtgtctttggttataaattatgatctacctaccaatcgtgaaaac
tatattcacagaattggcagagggggtcgatttgggaggaaaggtgtggctataaactttgttactgaagaagacaagaggattcttcgtga
cattgagactttctacaatactacagtggaggagatgcccatgaatgtggctgaccttatttaattcctgggatgagagttttggatgcagtgct
cgctgttgctgaataggcgatcacaacgtgcattgtgcttctttcttgggaatatttgaatcttgtctcaatgctcataacggatcagaaataca
gatttgatagcaaagcgacgttagtcgtgagctcttgtgaggaaagtcattggctttatcctctttagagttagactgttggggtgggtataaa
agatggggtctgtaaaatctttctttcttagaaatttatttcctagttctgtagaaatggttgtattagatgttctctatcatttaataatatacttgtgga
ctaaaagatataagtgctgtataaaatcagccaattatgttaaactagcatatctgcctttattgtgtttgtcattagcctgagtagaaaggccttt
aaaattttttagaaagcatttgaatgcattttgtttggtattgtatttattcaataaagtatttaattagtgtaaaaaaaaaaaaaaaaaaaaa
aaaaaaa (SEQ ID NO:231)

**Q96EA8**

MSGGSADYNSREHGGPEGMDPDGVIESNWNEIVDNFDDMNLKESLLRGIYAYGFEKPSAIQQRAI
IPCIKGYDVIAQAQSGTGKTATFAISILQQLEIEFKETQALVLAPTRELAQQIQKVILALGDYMGATCH
ACIGGTNVRNEMQKLQAEAPHIVVGTPGRVFDMLNRRYLSPKWIKMFVLDEADEMLSRGFKDQIY
EIFQKLNTSIQVVLLSATMPTDVLEVTKKFMRDPIRILVKKEELTLEGIKQFYINVEREEWKLDTLCDL
YETLTITQAVIFLNTRRKVDWLTEKMHARDFTVSALHGDMDQKERDVIMREFRSGSSRVLITTDLL
ARGIDVQQVSLVINYDLPTNRENYIHRIGRGGRFGRKGVAINFVTEEDKRILRDIETFYNTTVEEMP
MNVADLI (SEQ ID NO:232)

# Figure 106

**Nucleotide:** AB028983
**Sequence:**
ggagcctaccataagcacctcatggaactcgctcttcagcaaacatatcaggacacctgtaattgcatcaagtcgcggatcaagttggaa
tttgaaaaacgtcaacaggagcggcttctgctctccctgctgccggcccacatcgccatggagatgaaagcggagatcatccagaggct
gcagggccccaaggcgggccagatggagaacacaaataacttccacaacctgtatgtgaagcggcatacaaacgtgagcatcttata
cgctgacatcgttggctttacccggctggcaagtgactgctccccgggagaactagtccacatgctgaatgagctctttggaaagtttgatc
aaattgcaaaggagaatgaatgcatgagaattaaaattttaggagactgctactactgtgtatctggactccctatatctctccctaaccatg
ccaagaactgtgtgaaaatggggctggacatgtgtgaagccataaagaaagtgagggatgctactggagttgatatcaacatgcgcgtg
ggcgtgcattctgggaatgtcctgtgtggcgtgattggtctgcagaagtggcaatatgatgtgtggtcacatgatgtgaccttggccaaccac
atggaagctggaggggtccctggacgtgttcacatttcttctgtcaccctggagcacttgaatggcgcttataaagtggaggagggagatg
gtgacattagggacccatatttaaaacagcacctggtgaaaacctactttgtgatcaaccccaagggagaacgacggagcccccagca
tctcttcagacctcgccacacccttgatggagccaaaatgagggcctcggtccgcatgacccggtacttggagtcctgggggcagcca
agccctttgcacacctacatcacagggacagcatgaccacagagaacggcaagatcagcaccacggatgtacccatgggtcagcata
attttcaaaatcgcaccttaagaaccaagtcacaaaagaagagatttgaagaagaattgaatgaaaggatgattcaagcaattgatggg
attaatgcacagaagcaatggctcaagtctgaagacattcagagaatctcactgcttttctataacaaagtactagaaaaagagtaccgg
gccacggcactgccagcgttcaagtattatgtgacttgtgcctgtctcatattcttctgcatcttcattgtgcagattctcgtgctgccaaaaacgt
ctgtcctgggcatctcctttggggctgcgtttctcttgctggccttcatcctcttcgtctgctttgctggacagcttctgcaatgcagcaaaaaagc
ctctcccctgctcatgtggcttttgaagtcctcgggcatcattgccaaccgcccctggccacggatctctctcacgatcatcaccacagccat
catattaatgatggccgtgttcaacatgtttttcctgagtgactcagaggaaacaatccctccaactgccaacacaacaaacacaagcttttc
agcctcaaataatcaggtggcgattctgcgtgcgcagaatttatttttcctcccgtactttatctacagctgcattctgggactgatatcctgttcc
gtgttcctgcgggtaaactatgagctgaagatgttgatcatgatggtggccttggtgggctacaacaccatcctactccacacccacgccca
cgtcctgggcgactacagccaggtcttatttgagagaccaggcatttggaaagacctgaagaccatgggctctgtgtctctctctatattcttc
atcacactgcttgttctgggtagacagaatgaatattactgtaggttagacttcttatggaagaacaaattcaaaaaagagcgggaggaga
tagagaccatggagaacctgaaccgcgtgctgctggagaacgtgcttcccgcgcacgtggctgagcacttcctggccaggagcctgaa
gaatgaggagctataccaccagtcctatgactgcgtctgtgtcatgtttgcctccattccggatttcaaagaattttatacagaatccgacgtg
aacaaggagggcttggaatgccttcggctcctgaacgagatcatcgctgactttgatgatcttctttccaagccaaaattcagtggagttgaa
aagattaagaccattggcagcacatacatggcagcaacaggtctgagcgctgtgcccagccaggagcactcccaggagcccgagcg
gcagtacatgcacattggcaccatggtggagtttgcttttgccctggtagggaagctggatgccatcaacaagcactccttcaacgacttca
aattgcgagtgggtattaaccatggacctgtgatagctggtgtgattggagctcagaagccacaatatgatatctggggcaacactgtcaat
gtggccagtaggatggacagcaccggagtcctggacaaaatacaggttaccgaggagacgagcctcgtcctgcagaccctcggatac
acgtgcacctgtcgaggaataatcaacgtgaaaggaaagggggacctgaagacgtactttgtaaacacagaaatgtcaaggtcccttc
ccagagcaacgtggcatcctgaagagtcaccttcattttggcaagaagactgtattttcaggaaggtatcacacactttctgactgcaacttc
tgtcccttgtttttgatgtgcgtgctgtctgtcctatggagcctctgcagactcgttctcgtgacccagtggcataccgtttggtgtctgatgtgtgcc
cagatcgttctgccacttgcactgtgcttgctcctaagcaaaagggaaaaggagcgcgcgtgatagaagaaaagcactgggagaacta
acagaggagaaaggtgaaacacacacacattcttaaggcaataaaactaggggggtgtatattatcttctggtgcatgttcttttctggaaaa
tatggtagctcgccaaccgcatctgctcatctgatattcaaacacacagtattcgtgaataagttgattctgtcccccacgtggactctgtgctc
acccattgtctcattgccagtggtgtccaagggcccccgttgggacccacggctctcgtccctctgctccgtgtgtctcatgccagcagcacg
tcgccatccgtcaccagaattagtcctcacagcctaggaccagttttgtatcaaactcgtctgatgttttgatgccatttgtcttttgtaaagttaat
tcattaaaagtttatgtactttgatttacagtgcctgtatcttttattttcctgtcttctttctcctgtggtttgctccagaattaaggtttgttttccatccatt
ctccctttgacacagttgtttcagaaagagctctccagaagccaatattgagatgtaattcagattaggacacagtgtgtgacgcagataa
ctggttactcagctccctggaaagcaggcaagcatgttgaatgtatctagtggtctgattttaatttgggcatctctagagaacgctttcaggg
aaaaatactttaatagtaaaaagattctctgcgagcaacagtgccccctccgtccactacgctcctgtctccaagaatgttttgctagagcta
acagacatagactgcaaaagaataatttggaatcagctatgcaaatcagtctcacaatagcgtgagctaactgagagaagtactaagac
ccacaaactgcctgttaagtctgagaaggctaaagaagacacacagccaacgttcatgcatttttaaagacagaaggccttgaagaattt
gttcttgtaaatccaacacaagttgtttggtacttttaacataaagaaatcatactttgccaaatagtgaaaagtagagcaatcgtgtataagc
taatgtttaaaagcaaaactgcaaattgtagcccagttggtcaaacttgttttcttttataactcatggcaggcatctgtaagaagtagagaac
ccagatgatctcttaggaagccttttattcgtgggaactcgaacttgaagcacaagttcctggtttgaatcctggctctgatttttactggctgtgt
gactttgaacacatctcttagtccctcttaggagtacttccttattggcaacttatggaatcgctagtgattaaacgaggcaatgactgtgaga
gagcctggcaggtgccccgtggtacattcacagcacgggcacagctgctgtgccaggactgtgactcattcccagtaaaaggcacttatc
gaagctgataaccgtccttcatcaccgaagtgtgagtagagcatgacttatttagtattctgcctcaatggggaatttttgatcctgtaatcac
aactcagcattggccttaatatacctaaatctccaaaaacagtgattaaagcaagagaattattacaagggcttttctctttcctctaactcatt
cttcacggatgccgtagcgtttccgtgagctcaaactggccttggtgtaaaatgtgtaaggatgagcagcaggcgtgcctcgtgggttcttcc
tctgttacatcctgctacactcatctgcaggtcaccttagttcacctaccctgagtgaacacccccagctgggtggtccaccaagttctcataa
acagagtccctcccattcccccacggggtgcaccgaacttgggtttgcgctaaaaagaactcaaaaggagaactgtgctctcccaaagc

catatcaccagtcttaccaaacaataggcttttaaaagcactgagtcattgtcagaatccactatgggaagctctgtgtgtacctggtcccttc
taggtgtggtcccataggagcagccttagcatcccctgtgaacttatcaaaaatgcaaattctcaggccccaacctgaaggaggaccctg
aatttgagatccccggggctggggcccagcacaactgctgtttagtgaacaggttctccaggtgattctgatccctgatcaagcttcagacct
ccctccctccccagtgtttttgcaggtgaggaaacaggggcagagtaattcaggcacatgtgttcagagttccacagttgattagcagttga
ggccaggctagaatggaaaactgcctgttccttgaatctgaaagagcattattcctggtcaaagctccttaaagttctgggcagctaaaagc
atccctgtgagcaaaaatgccaggcagaaaactggcagtgcacctctcatcagcccaggtcccagtgccattggcttcaagaaaaaaa
aaaatctctccccaatgctctccttaacctttaagtcttacaggaagcctctcatagaaattgcctccagtccagtttcccaaaaaccccagtg
ttttacatatctgtttaggagtgtctaagttttgtcatcaatccacgatgttattcttccttcccaactcactgtgctcctaaaggcagcaaccattca
tctttcctttgctctggatacaccgaatgaccaggtaacatcatcaggccgggtgcagtggctcctataatcccgatattttgggaggccgag
gagagaggatcacttaagcccaggagtctgagaccagcctgggcaacatagcaagacccccatctctgcaaaaaaataagaaaatta
gcttggcatggtggcacgtgcctgtagtcccagctacatgggaggctgaggtgggaggatcacttgagcccaggaagtccagaacgtag
tgagccttgattataccactgcactccagcctgggtgacagagcgagactctgtctcag (SEQ ID NO:233)

**Q08462**
GAYHKHLMELALQQTYQDTCNCIKSRIKLEFEKRQQERLLLSLLPAHIAMEMKAEIIQRLQGPKAG
QMENTNNFHNLYVKRHTNVSILYADIVGFTRLASDCSPGELVHMLNELFGKFDQIAKENECMRIKIL
GDCYYCVSGLPISLPNHAKNCVKMGLDMCEAIKKVRDATGVDINMRVGVHSGNVLCGVIGLQKW
QYDVWSHDVTLANHMEAGGVPGRVHISSVTLEHLNGAYKVEEGDGDIRDPYLKQHLVKTYFVINP
KGERRSPQHLFRPRHTLDGAKMRASVRMTRYLESWGAAKPFAHLHHRDSMTTENGKISTTDVP
MGQHNFQNRTLRTKSQKKRFEEELNERMIQAIDGINAQKQWLKSEDIQRISLLFYNKVLEKEYRAT
ALPAFKYYVTCACLIFFCIFIVQILVLPKTSVLGISFGAAFLLLAFILFVCFAGQLLQCSKKASPLLMWL
LKSSGIIANRPWPRISLTIITTAIILMMAVFNMFFLSDSEETIPPTANTTNTSFSASNNQVAILRAQNLF
FLPYFIYSCILGLISCSVFLRVNYELKMLIMMVALVGYNTILLHTHAHVLGDYSQVLFERPGIWKDLK
TMGSVSLSIFFITLLVLGRQNEYYCRLDFLWKNKFKKEREEIETMENLNRVLLENVLPAHVAEHFLA
RSLKNEELYHQSYDCVCVMFASIPDFKEFYTESDVNKEGLECLRLLNEIIADFDDLLSKPKFSGVEK
IKTIGSTYMAATGLSAVPSQEHSQEPERQYMHIGTMVEFAFALVGKLDAINKHSFNDFKLRVGINH
GPVIAGVIGAQKPQYDIWGNTVNVASRMDSTGVLDKIQVTEETSLVLQTLGYTCTCRGIINVKGKG
DLKTYFVNTEMSRSLSQSNVAS (SEQ ID NO:234)

**Nucleotide:** Z35309
**Sequence:**
gactggctgcagccgcagtcttggtggaggaggtggtgaccaccaccgctcctccacctgcatccggctgcgggactgcggcggccgc
gtttgccctgcagaccctgcaccccgggacgcggctcatctgtcattagcaccggcactaagctcccaccgctcagcgacttggtccgcc
gcaagctccgccgcaggctttgtccgctagcgctcggctgagtctgggcgggcgggaaacctgggctagggcgaggcggggcccctg
gacatgcctttctccacgtccgcctcctcgaccctattgtaagcggagaaacttagtgtgcgaggcaggcaggggacgtccccatttgatg
tcccgtaccctccaccccttcggatcgaggtagtaaagaggctcctgtaggaaactgactgcctctatgattgcggcctcttgggggatttt
gcgtttagcccgaaagttggctttgccaaaagacgcacgggtaggaagggcgaaaaggaaaccctgtattccgtcgcgctgggctctcc
gagtccgtgcgcaaagcggcctacgagtcctggctccgcacctgcagaggacaagagccaatgcctaaaaaagaacagcggagga
accggctggcgcggccagctggaacgctggatcgcagtgcgcccagggaaggccgggggcgcccgccggccctagccctcagtggt
cctctcccacgccggcccgcgcgtgcctctgcctacaagacctggggcgtcctggccagatctggatggcaggtccctccgccacccccc
ggcccggttccgggggcgtggcttggcgcgggggcgggtttaagtcaccgcgggtgtctgaccactctgacaggtctccaaatttctccca
gtcgcctggcgcccgcggtgcgtttcagagctccagggtggcacgcggcggcgcttccctagatccagaggcgtctctgtcgacttccac
gcggcctcgggcctcccttctcctccaaaccttcgcctcatccgccaagcttcggttctcagcctcagatatccgcaccggcggcttctcttcg
ttctcggagctcttggctttggagccctcaccacttttccttcccccgcctctcctgatcctcctagctccgagccaaatggactccaaagaac
gaaataaaggggatgagaactgtgtgctgcgacccttcgaaagcacagctgaaagcgttgacctcgtcttatagatcaggctgggaccct
ggggcgagagtccccacacccccctccgggagggatgcttctggccagagccagcgctgcgctgtcagtccttgctcccgaactaggaa
agagcctaggagggagcctcagcatacccccttcctccaaattaactatttggtgaattgttagcgccgaggctaccacctctccaaccctgt
cgcggggcgcgccgccacctcaccgtgacccccctcctcctccttctttgccaccgcccccagctccgcccctgctccccatcccggcgca
atggagttctccgaagggcgatgattccagccacatctgctaacttcgcacccatcgctgccgccggtcaccgccggccaggccccctgc
agccgcggagcagtgggcgtccaaagcccagtgcagcagccaggacccgcccgacgcgcagcagaagcacggcgcccaggcgc
ttaggcgtctcttggagagcaaaggctgcgccaaaacgctgagcctagaatcaaccaaggagcctgagcccaggaaggggctgcgtg
gctcacagcgctgcggctcctgaggacaaatagccactgccgctgcgtacccaagctgcgccggctggcgggagagcagcacgcaa
ggacgccgaggtccgccgcgatcttccaggtgcccttgcccctgggcacagtatgacccgacctacagggagccctagcgcagggct
cctgcaacgggtcagcctaggataaaaaggatccttgccaagctcctaccaggccgcctttgagtctttaggaacccctcctccggctgcc
tccccaaggttctgggcctccttccctgcggcccagagccatggagctctccgatgtgcgctgccttacaggcagcgaggaactctacac
catccacccgacgcccccggccggcgacggcaggagcgcctcccggccgcagcggctgctgtggcagacggcggtgcgacacatc
acggagcagcgcttcattcacgggcaccggggaggcagcggcagcgggagtggaggctcgggcaaagcctcggaccctgcgggcg

gcggccccaaccaccacgcgccgcagctgtcaggcgactcggcgctgcccctctactcgctgggcccgggagagcgagcgcacagc
acctgcggcaccaaagtcttcccggaacgcagcgggagcggcagtgccagcggcagcggaggcgggggcgacctgggcttcctgc
accttgactgtgcccctagcaactcggatttctttcttaatgggggctatagctaccgaggggtcattttccccaccctgcgcaactccttcaaa
tctcgggatttggaacgcctctaccagcgctatttcttgggccaaaggcgcaaatcggaagtggtgatgaacgtgctggacgtgctgacca
aactcactctcttggtcctacacttgagcctggcctcggcccccatggacccgctcaagggcatcctgctgggcttcttcaccggcattgagg
tagtgatctgcgccctggtggtggtcaggaaggacaccacctcccacacgtacctgcagtacagcggcgtggtcacctgggtggccatg
accacccagatcctggcagcaggcctcggctacgggctcctgggcgacggcataggctacgtgctcttcacgctcttcgccacctacagt
atgctgccgctgccgctcacctgggccatcctggccggcctgggcacctcgctgctgcaggtcatcctccaagtggtcataccccggctgg
cggtcatttccatcaaccaggttgtggcccaggcagtgctattcatgtgtatgaacacagctggaatcttcatcagttacctgtcagaccggg
cccagcgccaagctttcctggagactcggaggtgtgtggaggccaggctgcgcctggagacagagaaccaaagacaggagcggctc
gtgctttctgtgctcccccggtttgttgtcctggaaatgatcaacgacatgaccaatgtggaagatgagcacctgcagcaccagttccatcgg
atctacatccatcgctatgagaacgtcagtattctttttgcagatgttaaaggatttaccaacctctccacgaccttgtctgctcaggagctggtc
aggatgctcaacgagctctttgccagatttgatcgactggcccatgagcatcactgccttcgtattaaaatcctgggggactgctactactgc
gtgtctggacttcctgagccccgccaggaccatgcccactgctgtgttgaaatgggtctcagcatgatcaaaaccatcaggtatgtgcggtc
aaggacaaaacacgatgttgacatgaggattggaatccactccggctcggtgctgtgcggtgttttgggactacggaagtggcagtttgat
gtctggtcttgggatgtggatattgcaaacaaactcgaatctggaggaatccccgggaggattcacatttccaaagccacgctggactgtct
caacggtgactataacgtggaagagggccatggtaaagagaggaatgaattcctgaggaagcataatatcgaaacttacttaattaagc
agcctgaggacagtctgctgtccttgcctgaagatatcgtcaaggagtcagtgagctcctcagaccggagaaacagtggggccacattc
actgaaggatcctggagccctgaactgccctttgataatatcgtggggaaacagaatactctggctgccctaacaagaaattcaataaatc
tgcttccaaaccatcttgcacaagctttgcatgtccagtctgggcctgaggaaattaacaagagaatagaacataccatcgacttgcggag
tggcgataaattgagaagagagcatatcaagccattctcactgatgtttaaagactccagcctggagcacaagtattctcaaatgagggat
gaagtgttcaagtcaaacttggtctgtgcatttatcgttcttctatttatcacggcaatacaaagtttgcttccttcttcaagagtgatgccaatgac
catccagttctccattctgattatgctgcactcggctctggtcctcatcaccacagcagaggattataaatgtttgcccctcatcctccggaaaa
cttgctgttggattaatgagacctatttggcccggaacgtcatcatctttgcatccattttgattaatttcctgggtgccatcttaaatatcctgtggt
gtgattttgacaagtcgatacccttgaagaacctgactttcaattcctcagctgtgtttacagatatctgctcctacccagagtactttgtcttcac
gggggtgttggccatggtgacctgtgcagttttcctccggctgaactccgtcctgaagctggcagtgctgctgatcatgattgccatctatgcc
ctgctcactgagaccgtctacgcaggcctctttctgcgttatgacaacctcaaccacagtggagaagatttcctggggaccaaggaggtat
cactgctactgatggccatgttcctcctggctgtgttctaccatggacagcagctggagtacacagcccgcctggacttcctttggcgagtac
aggccaaagaggagatcaatgagatgaaggagctgagggaacacaatgagaacatgctccggaatatcttacccagccatgtggcc
cgccatttcctagagaaggaccgagacaatgaggagctgtattctcaatcctatgatgctgttggggtgatgtttgcctccatcccaggatttg
cggacttttactctcagactgaaatgaataaccagggagtggaatgcctgcgcttgctcaatgagatcattgctgacttcgatgagttgcttg
gtgaagaccgatttcaagacattgaaaagattaagaccattggcagcacctacatggccgtgtcaggcctgtcacctgaaaaacagcaa
tgtgaagacaagtggggacatttgtgtgctctggctgacttctcactcgccctgacagaaagcatacaggagatcaacaagcattcattca
acaattttgaactccggattggcatcagccacggctcagtggtagctggcgttatcggcgctaagaaaccacagtatgacatttggggcaa
aactgtgaacctggcaagccgaatggacagcacgggggttagtggccggatccaagtcccagaggagacctatctcatcctgaagga
ccagggctttgcctttgattaccgaggggagatctatgtgaagggtatcagtgaacaggaaggaaaaatcaaaacgtactttcttctggga
agagtccaacccaacccattcatcttgcccccaagaagactgcctgggcagtactccctggccgcggttgtcctgggacttgtccagtccc
tcaataggcaaaggcagaagcagctactcaatgagaacaacaacacaggaatcatcaagggtcattacaaccggcggactttgttgtc
acccagcggcacagagcctggagcccaggctgaaggcaccgacaaatctgatttgccataaaagcatttctttctgttttttttttttttgtattt
cttttatatataaaataaatatactaataaaaggtttaatttttttagaacaaaaaaaaaaaaaaaaaaaaaaaaaaaaaaaaaaaa
aaaaaaaaccccaaaaaaaaaaaaaaa (SEQ ID NO:235)

**P40145**

MELSDVRCLTGSEELYTIHPTPPAGDGRSASRPQRLLWQTAVRHITEQRFIHGHRGGSGSGSGG
SGKASDPAGGGPNHHAPQLSGDSALPLYSLGPGERAHSTCGTKVFPERSGSGSASGSGGGGDL
GFLHLDCAPSNSDFFLNGGYSYRGVIFPTLRNSFKSRDLERLYQRYFLGQRRKSEVVMNVLDVLT
KLTLLVLHLSLASAPMDPLKGILLGFFTGIEVVICALVVVRKDTTSHTYLQYSGVVTWVAMTTQILAA
GLGYGLLGDGIGYVLFTLFATYSMLPLPLTWAILAGLGTSLLQVILQVVIPRLAVISINQVVAQAVLF
MCMNTAGIFISYLSDRAQRQAFLETRRCVEARLRLETENQRQERLVLSVLPRFVVLEMINDMTNV
EDEHLQHQFHRIYIHRYENVSILFADVKGFTNLSTTLSAQELVRMLNELFARFDRLAHEHHCLRIKIL
GDCYYCVSGLPEPRQDHAHCCVEMGLSMIKTIRYVRSRTKHDVDMRIGIHSGSVLCGVLGLRKW
QFDVWSWDVDIANKLESGGIPGRIHISKATLDCLNGDYNVEEGHGKERNEFLRKHNIETYLIKQPE
DSLLSLPEDIVKESVSSSDRRNSGATFTEGSWSPELPFDNIVGKQNTLAALTRNSINLLPNHLAQAL
HVQSGPEEINKRIEHTIDLRSGDKLRREHIKPFSLMFKDSSLEHKYSQMRDEVFKSNLVCAFIVLLFI
TAIQSLLPSSRVMPMTIQFSILIMLHSALVLITTAEDYKCLPLILRKTCCWINETYLARNVIIFASILINFL
GAILNILWCDFDKSIPLKNLTFNSSAVFTDICSYPEYFVFTGVLAMVTCAVFLRLNSVLKLAVLLIMIAI
YALLTETVYAGLFLRYDNLNHSGEDFLGTKEVSLLLMAMFLLAVFYHGQQLEYTARLDFLWRVQA

KEEINEMKELREHNENMLRNILPSHVARHFLEKDRDNEELYSQSYDAVGVMFASIPGFADFYSQT
EMNNQGVECLRLLNEIIADFDELLGEDRFQDIEKIKTIGSTYMAVSGLSPEKQQCEDKWGHLCALA
DFSLALTESIQEINKHSFNNFELRIGISHGSVVAGVIGAKKPQYDIWGKTVNLASRMDSTGVSGRIQ
VPEETYLILKDQGFAFDYRGEIYVKGISEQEGKIKTYFLLGRVQPNPFILPPRRLPGQYSLAAVVLGL
VQSLNRQRQKQLLNENNNTGIIKGHYNRRTLLSPSGTEPGAQAEGTDKSDLP (SEQ ID NO:236)

# Figure 107

**Nucleotide:** M15182
**Sequence:**
ggtggccgagcgggggaccgggaagcatggcccgggggtcggcggttgcctgggcggcgctcgggccgttgttgtggggctgcgcgc
tggggctgcagggcgggatgctgtacccccaggagagcccgtcgcgggagtgcaaggagctggacggcctctggagcttccgcgccg
acttctctgacaaccgacgccggggcttcgaggagcagtggtaccggcggccgctgtgggagtcaggccccaccgtggacatgccagt
tccctccagcttcaatgacatcagccaggactggcgtctgcggcattttgtcggctgggtgtggtacgaacgggaggtgatcctgccggag
cgatggacccaggacctgcgcacaagagtggtgctgaggattggcagtgcccattcctatgccatcgtgtgggtgaatggggtcgacac
gctagagcatgagggggggctacctccccttcgaggccgacatcagcaacctggtccaggtggggcccctgccctcccggctccgaatc
actatcgccatcaacaacacactcacccccaccaccctgccaccagggaccatccaatacctgactgacacctccaagtatcccaagg
gttactttgtccagaacacatattttgactttttcaactacgctggactgcagcggtctgtacttctgtacacgacacccaccacctacatcgat
gacatcaccgtcaccaccagcgtggagcaagacagtgggctggtgaattaccagatctctgtcaagggcagtaacctgttcaagttgga
agtgcgtcttttggatgcagaaaacaaagtcgtggcgaatgggactgggacccagggccaacttaaggtgccaggtgtcagcctctggt
ggccgtacctgatgcacgaacgccctgcctatctgtattcattggaggtgcagctgactgcacagacgtcactggggcctgtgtctgacttct
acacactccctgtggggatccgcactgtggctgtcaccaagagccagttcctcatcaatgggaaacctttctatttccacggtgtcaacaag
catgaggatgcggacatccgagggaagggcttcgactggccgctgctggtgaaggacttcaacctgcttcgctggcttggtgccaacgct
ttccgtaccagccactacccctatgcagaggaagtgatgcagatgtgtgaccgctatgggattgtggtcatcgatgagtgtcccggcgtgg
gcctggcgctgccgcagttcttcaacaacgtttctctgcatcaccacatgcaggtgatggaagaagtggtgcgtagggacaagaaccacc
ccgcggtcgtgatgtggtctgtggccaacgagcctgcgtcccacctagaatctgctggctactacttgaagatggtgatcgctcacaccaa
atccttggacccctcccggcctgtgacctttgtgagcaactctaactatgcagcagacaagggggctccgtatgtggatgtgatctgtttgaa
cagctactactcttggtatcacgactacgggcacctggagttgattcagctgcagctggccacccagtttgagaactggtataagaagtatc
agaagcccattattcagagcgagtatggagcagaaacgattgcagggtttcaccaggatccacctctgatgttcactgaagagtaccaga
aaagtctgctagagcagtaccatctgggtctggatcaaaaacgcagaaaatatgtggttggagagctcatttggaattttgccgatttcatga
ctgaacagtcaccgacgagagtgctggggaataaaaaggggatcttcactcggcagagacaaccaaaaagtgcagcgttccttttgcg
agagagatactggaagattgccaatgaaaccaggtatccccactcagtagccaagtcacaatgtttggaaaacagcccgtttacttgagc
aagactgataccacctgcgtgtcccttcctccccgagtcagggcgacttccacagcagcagaacaagtgcctcctggactgttcacggca
gaccagaacgtttctggcctgggttttgtggtcatctattctagcagggaacactaaaggtggaaataaaagattttctattatggaaataaa
gagttggcatgaaagtcgctactg (SEQ ID NO:237)

**P08236**
MARGSAVAWAALGPLLWGCALGLQGGMLYPQESPSRECKELDGLWSFRADFSDNRRRGFEEQ
WYRRPLWESGPTVDMPVPSSFNDISQDWRLRHFVGWVWYEREVILPERWTQDLRTRVVLRIGS
AHSYAIVWVNGVDTLEHEGGYLPFEADISNLVQVGPLPSRLRITIAINNTLTPTTLPPGTIQYLTDTS
KYPKGYFVQNTYFDFFNYAGLQRSVLLYTTPTTYIDDITVTTSVEQDSGLVNYQISVKGSNLFKLEV
RLLDAENKVVANGTGTQGQLKVPGVSLWWPYLMHERPAYLYSLEVQLTAQTSLGPVSDFYTLPV
GIRTVAVTKSQFLINGKPFYFHGVNKHEDADIRGKGFDWPLLVKDFNLLRWLGANAFRTSHYPYA
EEVMQMCDRYGIVVIDECPGVGLALPQFFNNVSLHHHMQVMEEVVRRDKNHPAVVMWSVANEP
ASHLESAGYYLKMVIAHTKSLDPSRPVTFVSNSNYAADKGAPYVDVICLNSYYSWYHDYGHLELIQ
LQLATQFENWYKKYQKPIIQSEYGAETIAGFHQDPPLMFTEEYQKSLLEQYHLGLDQKRRKYVVG
ELIWNFADFMTEQSPTRVLGNKKGIFTRQRQPKSAAFLLRERYWKIANETRYPHSVAKSQCLENS
PFT (SEQ ID NO:238)

# Figure 108

**Nucleotide:** AF410459
**Sequence:**
ctaaactcgaattaagagggaaaaaaaatcagggaggaggtggcaagccacaccccacggtgcccgcgaacttccccggcagcgg
actgtagcccaggcagacgccgtcgagatgcagggcccaccgctcctgaccgccgcccacctcctctgcgtgtgcaccgccgcgctgg
ccgtggctcccgggcctcggtttctggtgacagccccagggatcatcaggcccggaggaaatgtgactattggggtggagcttctggaac
actgcccttcacaggtgactgtgaaggcggagctgctcaagacagcatcaaacctcactgtctctgtcctggaagcagaaggagtctttg
aaaaaggctcttttaagacacttactcttccatcactacctctgaacagtgcagatgagatttatgagctacgtgtaaccggacgtacccag
gatgagattttattctctaatagtacccgcttatcatttgagaccaagagaatatctgtcttcattcaaacagacaaggccttatacaagccaa
agcaagaagtgaagtttcgcattgttacactcttctcagattttaagccttacaaaacctctttaaacattctcattaaggaccccaaatcaaat
ttgatccaacagtggttgtcacaacaaagtgatcttggagtcatttccaaaacttttcagctatcttcccatccaatacttggtgactggtctattc
aagttcaagtgaatgaccagacatattatcaatcatttcaggtttcagaatatgtattaccaaaatttgaagtgactttgcagacaccattatatt
gttctatgaattctaagcatttaaatggtaccatcacggcaaagtatacatatgggaagccagtgaaaggagacgtaacgcttacatttttac
ctttatccttttggggaaagaagaaaaatattacaaaaacatttaagataaatggatctgcaaacttctcttttaatgatgaagagatgaaaa
atgtaatggattcttcaaatggactttctgaatacctggatctatcttcccctggaccagtagaaattttaaccacagtgacagaatcagttaca
ggtatttcaagaaatgtaagcactaatgtgttcttcaagcaacatgattacatcattgagtttttgattatactactgtcttgaagccatctctcaa
cttcacagccactgtgaaggtaactcgtgctgatggcaaccaactgactcttgaagaaagaagaaataatgtagtcataacagtgacac
agagaaactatactgagtactggagcggatctaacagtggaaatcagaaaatggaagctgttcagaaaataaattatactgtcccccaa
agtggaacttttaagattgaattcccaatcctggaggattccagtgagctacagttgaaggcctatttccttggtagtaaaagtagcatggca
gttcatagtctgtttaagtctcctagtaagacatacatccaactaaaaacaagagatgaaaatataaaggtgggatcgccttttgagttggtg
gttagtggcaacaaacgattgaaggagttaagctatatggtagtatccaggggacagttggtggctgtaggaaaacaaaattcaacaatg
ttctctttaacaccagaaaattcttggactccaaaagcctgtgtaattgtgtattatattgaagatgatggggaaattataagtgatgttctaaaa
attcctgttcagcttgtttttaaaaataagataaagctatattggagtaaagtgaaagctgaaccatctgagaaagtctctcttaggatctctgt
gacacagcctgactccatagttgggattgtagctgttgacaaaagtgtgaatctgatgaatgcctctaatgatattacaatggaaaatgtggt
ccatgagttggaactttataacacaggatattatttaggcatgttcatgaattcttttgcagtctttcaggaatgtggactctgggtattgacagat
gcaaacctcacgaaggattatattgatggtgtttatgacaatgcagaatatgctgagaggtttatggaggaaaatgaaggacatattgtag
atattcatgacttttctttgggtagcagtccacatgtccgaaagcattttccagagacttggatttggctagacaccaacatgggttacaggatt
taccaagaatttgaagtaactgtacctgattctatcacttcttgggtggctactggttttgtgatctctgaggacctgggtcttggactaacaact
actccagtggagctccaagccttccaaccatttttcatttttttgaatcttccctactctgttatcagaggtgaagaatttgctttggaaataactat
attcaattatttgaaagatgccactgaggttaaggtaatcattgagaaaagtgacaaatttgatattctaatgacttcaaatgaaataaatgcc
acaggccaccagcagacccttctggttcccagtgaggatggggcaactgttctttttcccatcaggccaacacatctgggagaaattcctat
cacagtcacagctctttcacccactgcttctgatgctgtcacccagatgattttagtaaaggctgaaggaatagaaaaatcatattcacaatc
catcttattagacttgactgacaataggctacagagtaccctgaaaactttgagtttctcatttcctcctaatacagtgactggcagtgaaaga
gttcagatcactgcaattggagatgttcttggtccttccatcaatggcttagcctcattgattcggatgccttatggctgtggtgaacagaacatg
ataaattttgctccaaatatttacattttggattatctgactaaaaagaaacaactgacagataatttgaaagaaaaagctctttcatttatgag
gcaaggttaccagagagaacttctctatcagagggaagatggctctttcagtgcttttgggaattatgacccttctgggagcacttggttgtca
gcttttgttttaagatgtttccttgaagccgatccttacatagatattgatcagaatgtgttacacagaacatacacttggcttaaaggacatcag
aaatccaacggtgaattttgggatccaggaagagtgattcatagtgagcttcaaggtggcaataaaagtccagtaacacttacagcctata
ttgtaacttctctcctgggatatagaaagtatcagcctaacattgatgtgcaagagtctatccatttttggagtctgaattcagtagaggaatttc
agacaattatactctagcccttataacttatgcattgtcatcagtggggagtcctaaagcgaaggaagctttgaatatgctgacttggagagc
agaacaagaaggtggcatgcaattctgggtgtcatcagagtccaaactttctgactcctggcagccacgctccctggatattgaagttgca
gcctatgcactgctctcacacttcttacaatttcagacttctgagggaatcccaattatgaggtggctaagcaggcaaagaaatagcttgggt
ggttttgcatctactcaggataccactgtggctttaaaggctctgtctgaatttgcagccctaatgaatacagaaaggacaaatatccaagtg
accgtgacggggcctagctcaccaagtcctgtaaagtttctgattgacacacacaaccgcttactccttcagacagcagagcttgctgtggt
acagccaatggcagttaatatttccgcaaatggttttggatttgctatttgtcagctcaatgttgtatataatgtgaaggcttctgggtcttctagaa
gacgaagatctatccaaaatcaagaagcctttgatttagatgttgctgtaaaagaaaataaagatgatctcaatcatgtggatttgaatgtgt
gtacaagcttttcgggcccgggtaggagtggcatggctcttatggaagttaacctattaagtggctttatggtgccttcagaagcaatttctctg
agcgagacagtgaagaaagtggaatatgatcatggaaaactcaacctctatttagattctgtaaatgaaacccagttttgtgttaatattcct
gctgtgagaaactttaaagtttcaaatacccaagatgcttcagtgtccatagtggattactatgagccaaggagacaggcggtgagaagtt
acaactctgaagtgaagctgtcctcctgtgacctttgcagtgatgtccagggctgccgtccttgtgaggatggagcttcaggctcccatcatc
actcttcagtcattttt atttctgtttcaagcttctgtactttatggaactttggctgtgatttatttttaaaggactctgtgtaacactaacatttccagt
agtcacatgtgattgttttgttttcgtagaagaatactgcttctattttgaaaaaagagtttttttctttctatggggttgcaggatggtgtacaaca
ggtcctagcatgtatagctgcatagatttcttcacctgatctttgtgtggaagatcagaatgaatgcagttgtgtgtctatattttcccctcacaaa
atcttttagaatttttttggaggtgtttgttttctccagaataaaggtattactttagaaataggtattctcctcatttgtgaaagaaatgaacctaga
ttcttaagcattattacacatccatgtttgcttaaagatggatttccctgggaatgggagaaaacagccagcaggaggagcttcatctgttccc

ttcccacctccaacctagccctactgcccaccccaccccaacccaccccatgcccagtggtctcagtagatacttcttaactggaaattcttt
cttttcagaatctaggtggtgaattttttttaagtggcacggtctttttctgcttgaaatctgatcacaccccccagccattgccctccctctcttttcc
tctgtagagaaatgtgaggggcagtacatttactgtgcttttcacaccatctcagaggttgaggagcatactgaaaattgccctggggggtg
ctgggtgtgctgtctccttcccacatcctcagccccacaccagctctatttcaggggtgagagtcagagagcactgcaatatgtgcttcatgg
gatttcgattcgaagatcctagaccagggagacactgtgagccagggatacaacaaaatactaggtaagtcactgcagaccgacctcc
ctgcagtttgggaaagaagctgggtttgtggagaatcagagcatcttgacatgactgctgacctaaagatccctggcattggccagggatc
ctgtggaacctcttctagttcaggggtgtgagcattagactgccagttgtctagtgacatctgatgcttgctgtgaactttttaagatccccgaatc
ctgagcacctcaatctttaattgccctgtattccgaagggtaatataatttatctggatggaaattttaaagatgaatcccccttttttcttttcttctct
cttttctttccttctccctttcttctttgccttctaaatatactgaaatgatttagatatgtgtcaacaattaatgatcttttattcaatctaagaaatggttt
agtttttctctttagctctatggcatttcactcaagtggacaggggaaaaagtaattgccatgggctccaaagaatttgctttatgtttttagctattt
aaaaataaatccatcaaaaataaagtatgcaaatgtatctttt (SEQ ID NO:239)

**Q8TDJ3**
MQGPPLLTAAHLLCVCTAALAVAPGPRFLVTAPGIIRPGGNVTIGVELLEHCPSQVTVKAELLKTAS
NLTVSVLEAEGVFEKGSFKTLTLPSLPLNSADEIYELRVTGRTQDEILFSNSTRLSFETKRISVFIQT
DKALYKPKQEVKFRIVTLFSDFKPYKTSLNILIKDPKSNLIQQWLSQQSDLGVISKTFQLSSHPILGD
WSIQVQVNDQTYYQSFQVSEYVLPKFEVTLQTPLYCSMNSKHLNGTITAKYTYGKPVKGDVTLTF
LPLSFWGKKKNITKTFKINGSANFSFNDEEMKNVMDSSNGLSEYLDLSSPGPVEILTTVTESVTGIS
RNVSTNVFFKQHDYIIEFFDYTTVLKPSLNFTATVKVTRADGNQLTLEERRNNVVITVTQRNYTEY
WSGSNSGNQKMEAVQKINYTVPQSGTFKIEFPILEDSSELQLKAYFLGSKSSMAVHSLFKSPSKTY
IQLKTRDENIKVGSPFELVVSGNKRLKELSYMVVSRGQLVAVGKQNSTMFSLTPENSWTPKACVI
VYYIEDDGEIISDVLKIPVQLVFKNKIKLYWSKVKAEPSEKVSLRISVTQPDSIVGIVAVDKSVNLMNA
SNDITMENVVHELELYNTGYYLGMFMNSFAVFQECGLWVLTDANLTKDYIDGVYDNAEYAERFM
EENEGHIVDIHDFSLGSSPHVRKHFPETWIWLDTNMGYRIYQEFEVTVPDSITSWVATGFVISEDL
GLGLTTTPVELQAFQPFFIFLNLPYSVIRGEEFALEITIFNYLKDATEVKVIIEKSDKFDILMTSNEINA
TGHQQTLLVPSEDGATVLFPIRPTHLGEIPITVTALSPTASDAVTQMILVKAEGIEKSYSQSILLDLTD
NRLQSTLKTLSFSFPPNTVTGSERVQITAIGDVLGPSINGLASLIRMPYGCGEQNMINFAPNIYILDY
LTKKKQLTDNLKEKALSFMRQGYQRELLYQREDGSFSAFGNYDPSGSTWLSAFVLRCFLEADPYI
DIDQNVLHRTYTWLKGHQKSNGEFWDPGRVIHSELQGGNKSPVTLTAYIVTSLLGYRKYQPNIDV
QESIHFLESEFSRGISDNYTLALITYALSSVGSPKAKEALNMLTWRAEQEGGMQFWVSSESKLSD
SWQPRSLDIEVAAYALLSHFLQFQTSEGIPIMRWLSRQRNSLGGFASTQDTTVALKALSEFAALMN
TERTNIQVTVTGPSSPSPVKFLIDTHNRLLLQTAELAVVQPMAVNISANGFGFAICQLNVVYNVKAS
GSSRRRRSIQNQEAFDLDVAVKENKDDLNHVDLNVCTSFSGPGRSGMALMEVNLLSGFMVPSEA
ISLSETVKKVEYDHGKLNLYLDSVNETQFCVNIPAVRNFKVSNTQDASVSIVDYYEPRRQAVRSYN
SEVKLSSCDLCSDVQGCRPCEDGASGSHHHSSVIFIFCFKLLYFMELWL (SEQ ID NO:240)

# Figure 109

**Nucleotide:** AB034951
**Sequence:**
gcttccttcgttattggagccaggcctacaccccagcaaccatgtccaagggacctgcagttggtattgatcttggcaccacctactcttgtgt
gggtgttttccagcacggaaaagtcgagataattgccaatgatcagggaaaccgaaccactccaagctatgtcgcctttacggacactga
acggttgatcggtgatgccgcaaagaatcaagttgcaatgaaccccaccaacacagtttttgatgccaaacgtctgattggacgcagattt
gatgatgctgttgtccagtctgatatgaaacattggccctttatggtggtgaatgatgctggcaggcccaaggtccaagtagaatacaaggg
agagaccaaaagcttctatccagaggaggtgtcttctatggttctgacaaagatgaaggaaattgcagaagcctaccttgggaagactgtt
accaatgctgtggtcacagtgccagcttactttaatgactctcagcgtcaggctaccaaagatgctggaactattgctggtctcaatgtactta
gaattattaatgagccaactgctgctgctattgcttacggcttagacaaaaaggttggagcagaaagaaacgtgctcatctttgacctggga
ggtggcacttttgatgtgtcaatcctcactattgaggatggaatctttgaggtcaagtctacagctggagacacccacttgggtggagaagat
tttgacaaccgaatggtcaaccattttattgctgagtttaagcgcaagcataagaaggacatcagtgagaacaagagagctgtaagacgc
ctccgtactgcttgtgaacgtgctaagcgtaccctctcttccagcacccaggccagtattgagatcgattctctctatgaaggaatcgacttct
atacctccattacccgtgcccgatttgaagaactgaatgctgacctgttccgtggcaccctggacccagtagagaaagcccttcgagatgc
caaactagacaagtcacagattcatgatattgtcctggttggtggttctactcgtatccccaagattcagaagcttctccaagacttcttcaatg
gaaaagaactgaataagagcatcaaccctgatgaagctgttgcttatggtgcagctgtccaggcagccatcttgtctggagacaagtctg
agaatgttcaagatttgctgctcttggatgtcactcctctttcccttggtattgaaactgctggtggagtcatgactgtcctcatcaagcgtaatac
caccattcctaccaagcagacacagaccttcactacctattctgacaaccagcctggtgtgcttattcaggtttatgaaggcgagcgtgcca
tgacaaaggataacaacctgcttggcaagtttgaactcacaggcatgccaggaggaatgcctggggggatttcctggtggtggagctcctc
cctctggtggtgcttcctcagggcccaccattgaagaggttgattaagccaaccaagt (SEQ ID NO:241)

**P11142**
MSKGPAVGIDLGTTYSCVGVFQHGKVEIIANDQGNRTTPSYVAFTDTERLIGDAAKNQVAMNPTN
TVFDAKRLIGRRFDDAVVQSDMKHWPFMVVNDAGRPKVQVEYKGETKSFYPEEVSSMVLTKMK
EIAEAYLGKTVTNAVVTVPAYFNDSQRQATKDAGTIAGLNVLRIINEPTAAAIAYGLDKKVGAERNV
LIFDLGGGTFDVSILTIEDGIFEVKSTAGDTHLGGEDFDNRMVNHFIAEFKRKHKKDISENKRAVRR
LRTACERAKRTLSSSTQASIEIDSLYEGIDFYTSITRARFEELNADLFRGTLDPVEKALRDAKLDKSQ
IHDIVLVGGSTRIPKIQKLLQDFFNGKELNKSINPDEAVAYGAAVQAAILSGDKSENVQDLLLLDVTP
LSLGIETAGGVMTVLIKRNTTIPTKQTQTFTTYSDNQPGVLIQVYEGERAMTKDNNLLGKFELTGIP
PAPRGVPQIEVTFDIDANGILNVSAVDKSTGKENKITITNDKGRLSKEDIERMVQEAEKYKAEDEKQ
RDKVSSKNSLESYAFNMKATVEDEKLQGKINDEDKQKILDKCNEIINWLDKNQTAEKEEFEHQQK
ELEKVCNPIITKLYQSAGGMPGGMPGGFPGGGAPPSGGASSGPTIEEVD (SEQ ID NO:242)

# Figure 110

**Nucleotide:** M23725
**Sequence:**
ggctgaggcagtggctccttgcacagcagctgcacgcgccgtggctccggatcttcttcgtctttgcagcgtagcccgagtcggtcagcgc
cagaggacctcagcagccatgtcgaagccccatagtgaagccgggactgccttcattcagacccagcagctgcacgcagccatggctg
acacattcctggagcacatgtgccgcctggacattgattcaccacccatcacagcccggaacactggcatcatctgtaccattggcccag
cttcccgatcagtggagacgttgaaggagatgattaagtctggaatgaatgtggctcgtctgaacttctctcatggaactcatgagtaccatg
cggagaccatcaagaatgtgcgcacagccacggaaagctttgcttctgaccccatcctctaccggcccgttgctgtggctctagacactaa
aggacctgagatccgaactgggctcatcaagggcagcggcactgcagaggtggagctgaagaagggagccactctcaaaatcacgc
tggataacgcctacatggaaaagtgtgacgagaacatcctgtggctggactacaagaacatctgcaaggtggtggaagtgggcagcaa
gatctacgtggatgatgggcttatttctctccaggtgaagcagaaaggtgccgacttcctggtgacggaggtggaaaatggtggctccttgg
gcagcaagaagggtgtgaaccttcctgggctgctgtggacttgcctgctgtgtcggagaaggacatccaggatctgaagtttggggtcg
agcaggatgttgatatggtgtttgcgtcattcatccgcaaggcatctgatgtccatgaagttaggaaggtcctgggagagaagggaaaga
acatcaagattatcagcaaaatcgagaatcatgaggggttcggaggtttgatgaaatcctggaggccagtgatgggatcatggtggctc
gtggtgatctaggcattgagattcctgcagagaaggtcttccttgctcagaagatgatgattggacggtgcaaccgagctgggaagcctgt
catctgtgctactcagatgctggagagcatgatcaagaagccccgccccactcgggctgaaggcagtgatgtggccaatgcagtcctgg
atggagccgactgcatcatgctgtctggagaaacagccaaaggggactatcctctggaggctgtgcgcatgcagaacctgattgcccgt
gaggcagaggctgccatctaccacttgcaattatttgaggaactccgccgcctggcgcccattaccagcgaccccacagaagccaccg
ccgtgggtgccgtggaggcctccttcaagtgctgcagtggggccataatcgtcctcaccaagtctggcaggtctgctcaccaggtggcca
gataccgcccacgtgcccccatcattgctgtgacccggaatccccagacagctcgtcaggcccacctgtaccgtggcatcttccctgtgct
gtgcaaggacccagtccaggaggcctgggctgaggacgtggacctccgggtgaactttgccatgaatgttggcaaggcccgaggcttct
tcaagaagggagatgtggtcattgtgctgaccggatggcgccctggctccggcttcaccaacaccatgcgtgttgttcctgtgccgtgatgg
accccagagcccctcctccagcccctgtcccaccccccttcccccagcccatccattaggccagcaacgcttgtagaactcactctgggctg
taacgtggcactggtaggttgggacaccagggaagaagatcaacgcctcactgaaacatggctgtgtttgcagcctgctctagtgggaca
gcccagagcctggctgccccatcatgtggccccacccaatcaagggaagaaggaggaatgctggactggaggcccctggagccaga
tggcaagagggtgacagcttcctttcctgtgtgtactctgtccagttcctttagaaaaaatggatgcccagaggactcccaaccctggcttgg
ggtcaagaaacagccagcaagagttaggggccttagggcactgggctgttgttccattgaagccgactctggccctggcccttacttgcttc
tctagctctctaggcctctccagtttgcacctgtccccaccctccactcagctgtcctgcagcaaacactccaccctccaccttccattttcccc
cactactgcagcacctccaggcctgttgccgc (SEQ ID NO:243)

**P14786**
MSKPHSEAGTAFIQTQQLHAAMADTFLEHMCRLDIDSPPITARNTGIICTIGPASRSVETLKEMIKS
GMNVARLNFSHGTHEYHAETIKNVRTATESFASDPILYRPVAVALDTKGPEIRTGLIKGSGTAEVEL
KKGATLKITLDNAYMEKCDENILWLDYKNICKVVEVGSKIYVDDGLISLQVKQKGADFLVTEVENG
GSLGSKKGVNLPGAAVDLPAVSEKDIQDLKFGVEQDVDMVFASFIRKASDVHEVRKVLGEKGKNI
KIISKIENHEGVRRFDEILEASDGIMVARGDLGIEIPAEKVFLAQKMMIGRCNRAGKPVICATQMLES
MIKKPRPTRAEGSDVANAVLDGADCIMLSGETAKGDYPLEAVRMQNLIAREAEAAIYHLQLFEELR
RLAPITSDPTEATAVGAVEASFKCCSGAIIVLTKSGRSAHQVARYRPRAPIIAVTRNPQTARQAHLY
RGIFPVLCKDPVQEAWAEDVDLRVNFAMNVGKARGFFKKGDVVIVLTGWRPGSGFTNTMRVVPV
P (SEQ ID NO:244)

# Figure 111

**Nucleotide:** Z35309
**Sequence:**
gactggctgcagccgcagtcttggtggaggaggtggtgaccaccaccgctcctccacctgcatccggctgcgggactgcggcggccgc
gtttgccctgcagaccctgcaccccgggacgcggctcatctgtcattagcaccggcactaagctcccaccgctcagcgacttggtccgcc
gcaagctccgccgcaggctttgtccgctagcgctcggctgagtctgggcgggcgggaaacctgggctagggcgaggcggggcccctg
gacatgcctttctccacgtccgcctcctcgaccctattgtaagcggagaaacttagtgtgcgaggcaggcaggggacgtccccattttgatg
tcccgtaccctccaccccccttcggatcgaggtagtaaagaggctcctgtaggaaactgactgcctctatgattgcggcctcttggggggatttt
gcgtttagcccgaaagttggctttgccaaaagacgcacgggtaggaagggcgaaaaggaaaccctgtattccgtcgcgctgggctctcc
gagtccgtgcgcaaagcggcctacgagtcctggctccgcacctgcagaggacaagagccaatgcctaaaaaagaacagcggagga
accggctggcgcggccagctggaacgctggatcgcagtgcgcccagggaaggccgggggcgcccgccggccctagccctcagtggt
cctctcccacgccggcccgcgcgtgcctctgcctacaagacctggggcgtcctggccagatctggatggcaggtccctccgccacccccc
ggcccggttccgggggcgtggcttggcgcgggggcgggtttaagtcaccgcgggtgtctgaccactctgacaggtctccaaatttctccca
gtcgcctggcgcccgcggtgcgtttcagagctccagggtggcacgcggcggcgcttccctagatccagaggcgtctctgtcgacttccac
gcggcctcgggcctcccttctcctccaaaccttcgcctcatccgccaagcttcggttctcagcctcagatatccgcaccggcggcttctcttcg
ttctcggagctcttggctttggagccctcaccactttttccttcccccgcctctcctgatcctcctagctccgagccaaatggactccaaagaac
gaaataaaggggatgagaactgtgtgctgcgacccttcgaaagcacagctgaaagcgttgacctcgtcttatagatcaggctgggaccct
ggggcgagagtccccacacccccctccgggagggatgcttctggccagagccagcgctgcgctgtcagtccttgctcccgaactaggaa
agagcctaggagggagcctcagcataccccttcctccaaattaactatttggtgaattgttagcgccgaggctaccacctctccaaccctgt
cgcggggcgcgccgccacctcaccgtgacccccctcctcctccttctttgccaccgcccccagctccgcccctgctccccatcccggcgca
atggagttctccgaagggcgatgattccagccacatctgctaacttcgcacccatcgctgccgccggtcaccgccggccaggccccctgc
agccgcggagcagtgggcgtccaaagcccagtgcagcagccaggacccgcccgacgcgcagcagaagcacggcgcccaggcgc
ttaggcgtctcttggagagcaaaggctgcgccaaaacgctgagcctagaatcaaccaaggagcctgagcccaggaaggggctgcgtg
gctcacagcgctgcggctcctgaggacaaatagccactgccgctgcgtacccaagctgcgccggctggcgggagagcagcacgcaa
ggacgccgaggtccgccgcgatcttccaggtgccctttgcccctgggcacagtatgacccgacctacagggagccctagcgcagggct
cctgcaacgggtcagcctaggataaaaaggatccttgccaagctcctaccaggccgcctttgagtctttaggaacccctcctccggctgcc
tccccaaggttctgggcctccttccctgcggcccagagccatggagctctccgatgtgcgctgccttacaggcagcgaggaactctacac
catccacccgacgcccccggccggcgacggcaggagcgcctcccggccgcagcggctgctgtggcagacggcggtgcgacacatc
acggagcagcgcttcattcacgggcaccggggaggcagcggcagcgggagtggaggctcgggcaaagcctcggaccctgcgggcg
gcggccccaaccaccacgcgccgcagctgtcaggcgactcggcgctgcccctctactcgctgggcccgggagagcgagcgcacagc
acctgcggcaccaaagtcttcccggaacgcagcgggagcggcagtgccagcggcagcggaggcgggggcgacctgggcttcctgc
accttgactgtgcccctagcaactcggatttctttcttaatgggggctatagctaccgaggggtcattttccccaccctgcgcaactccttcaaa
tctcgggatttggaacgcctctaccagcgctatttcttgggccaaaggcgcaaatcggaagtggtgatgaacgtgctggacgtgctgacca
aactcactctcttggtcctacacttgagcctggcctcggcccccatggacccgctcaagggcatcctgctgggcttcttcaccggcattgagg
tagtgatctgcgccctggtggtggtcaggaaggacaccacctcccacacgtacctgcagtacagcggcgtggtcacctgggtggccatg
accacccagatcctggcagcaggcctcggctacgggctcctgggcgacggcataggctacgtgctcttcacgctcttcgccacctacagt
atgctgccgctgccgctcacctgggccatcctggccggcctgggcacctcgctgctgcaggtcatcctccaagtggtcataccccggctgg
cggtcatttccatcaaccaggttgtggcccaggcagtgctattcatgtgtatgaacacagctggaatcttcatcagttacctgtcagaccggg
cccagcgccaagctttcctggagactcggaggtgtgtggaggccaggctgcgcctggagacagagaaccaaagacaggagcggctc
gtgctttctgtgctcccccggtttgttgtcctggaaatgatcaacgacatgaccaatgtggaagatgagcacctgcagcaccagttccatcgg
atctacatccatcgctatgagaacgtcagtattctttttgcagatgttaaaggatttaccaacctctccacgaccttgtctgctcaggagctggtc
aggatgctcaacgagctctttgccagatttgatcgactggcccatgagcatcactgccttcgtattaaaatcctgggggactgctactactgc
gtgtctggacttcctgagccccgccaggaccatgcccactgctgtgttgaaatgggtctcagcatgatcaaaaccatcaggtatgtgcggtc
aaggacaaaacacgatgttgacatgaggattggaatccactccggctcggtgctgtgcggtgttttgggactacggaagtggcagtttgat
gtctggtcttgggatgtggatattgcaaacaaactcgaatctggaggaatccccgggaggattcacatttccaaagccacgctggactgtct
caacggtgactataacgtggaagagggccatggtaaagagaggaatgaattcctgaggaagcataatatcgaaacttacttaattaagc
agcctgaggacagtctgctgtccttgcctgaagatatcgtcaaggagtcagtgagctcctcagaccggagaaacagtggggccacattc
actgaaggatcctggagccctgaactgccctttgataatatcgtggggaaacagaatactctggctgccctaacaagaaattcaataaatc
tgcttccaaaccatcttgcacaagctttgcatgtccagtctgggcctgaggaaattaacaagagaatagaacataccatcgacttgcggag
tggcgataaattgagaagagagcatatcaagccattctcactgatgtttaaagactccagcctggagcacaagtattctcaaatgagggat
gaagtgttcaagtcaaacttggtctgtgcatttatcgttcttctatttatcacggcaatacaaagtttgcttccttcttcaagagtgatgccaatgac
catccagttctccattctgattatgctgcactcggctctggtcctcatcaccacagcagaggattataaatgtttgcccctcatcctccggaaaa
cttgctgttggattaatgagacctatttggcccggaacgtcatcatctttgcatccattttgattaatttcctgggtgccatcttaaatatcctgtggt
gtgattttgacaagtcgataccttgaagaacctgactttcaattcctcagctgtgtttacagatatctgctcctacccagagtactttgtcttcac
gggggtgttggccatggtgacctgtgcagttttcctccggctgaactccgtcctgaagctggcagtgctgctgatcatgattgccatctatgcc

ctgctcactgagaccgtctacgcaggcctctttctgcgttatgacaacctcaaccacagtggagaagatttcctggggaccaaggaggtat
cactgctactgatggccatgttcctcctggctgtgttctaccatggacagcagctggagtacacagcccgcctggacttcctttggcgagtac
aggccaaagaggagatcaatgagatgaaggagctgagggaacacaatgagaacatgctccggaatatcttacccagccatgtggcc
cgccatttcctagagaaggaccgagacaatgaggagctgtattctcaatcctatgatgctgttggggtgatgtttgcctccatcccaggatttg
cggacttttactctcagactgaaatgaataaccagggagtggaatgcctgcgcttgctcaatgagatcattgctgacttcgatgagttgcttg
gtgaagaccgatttcaagacattgaaaagattaagaccattggcagcacctacatggccgtgtcaggcctgtcacctgaaaaacagcaa
tgtgaagacaagtggggacatttgtgtgctctggctgacttctcactcgccctgacagaaagcatacaggagatcaacaagcattcattca
acaattttgaactccggattggcatcagccacggctcagtggtagctggcgttatcggcgctaagaaaccacagtatgacatttggggcaa
aactgtgaacctggcaagccgaatggacagcacgggggttagtggccggatccaagtcccagaggagacctatctcatcctgaagga
ccagggctttgcctttgattaccgaggggagatctatgtgaagggtatcagtgaacaggaaggaaaaatcaaaacgtactttcttctggga
agagtccaacccaacccattcatcttgcccccaagaagactgcctgggcagtactccctggccgcggttgtcctgggacttgtccagtccc
tcaataggcaaaggcagaagcagctactcaatgagaacaacaacacaggaatcatcaagggtcattacaaccggcggactttgttgtc
acccagcggcacagagcctggagcccaggctgaaggcaccgacaaatctgatttgccataaaagcattttcttctgtttttttttttttgtattt
cttttatatataaaataaatatactaataaaaaggtttaattttttttagaacaaaaaaaaaaaaaaaaaaaaaaaaaaaaaaaaaaaaa
aaaaaaaaaccccaaaaaaaaaaaaaaa (SEQ ID NO:245)

**P40145**

MELSDVRCLTGSEELYTIHPTPPAGDGRSASRPQRLLWQTAVRHITEQRFIHGHRGGSGSGSGG
SGKASDPAGGGPNHHAPQLSGDSALPLYSLGPGERAHSTCGTKVFPERSGSGSASGSGGGGDL
GFLHLDCAPSNSDFFLNGGYSYRGVIFPTLRNSFKSRDLERLYQRYFLGQRRKSEVVMNVLDVLT
KLTLLVLHLSLASAPMDPLKGILLGFFTGIEVVICALVVVRKDTTSHTYLQYSGVVTWVAMTTQILAA
GLGYGLLGDGIGYVLFTLFATYSMLPLPLTWAILAGLGTSLLQVILQVVIPRLAVISINQVVAQAVLF
MCMNTAGIFISYLSDRAQRQAFLETRRCVEARLRLETENQRQERLVLSVLPRFVVLEMINDMTNV
EDEHLQHQFHRIYIHRYENVSILFADVKGFTNLSTTLSAQELVRMLNELFARFDRLAHEHHCLRIKIL
GDCYYCVSGLPEPRQDHAHCCVEMGLSMIKTIRYVRSRTKHDVDMRIGIHSGSVLCGVLGLRKW
QFDVWSWDVDIANKLESGGIPGRIHISKATLDCLNGDYNVEEGHGKERNEFLRKHNIETYLIKQPE
DSLLSLPEDIVKESVSSSDRRNSGATFTEGSWSPELPFDNIVGKQNTLAALTRNSINLLPNHLAQAL
HVQSGPEEINKRIEHTIDLRSGDKLRREHIKPFSLMFKDSSLEHKYSQMRDEVFKSNLVCAFIVLLFI
TAIQSLLPSSRVMPMTIQFSILIMLHSALVLITTAEDYKCLPLILRKTCCWINETYLARNVIIFASILINFL
GAILNILWCDFDKSIPLKNLTFNSSAVFTDICSYPEYFVFTGVLAMVTCAVFLRLNSVLKLAVLLIMIAI
YALLTETVYAGLFLRYDNLNHSGEDFLGTKEVSLLLMAMFLLAVFYHGQQLEYTARLDFLWRVQA
KEEINEMKELREHNENMLRNILPSHVARHFLEKDRDNEELYSQSYDAVGVMFASIPGFADFYSQT
EMNNQGVECLRLLNEIIADFDELLGEDRFQDIEKIKTIGSTYMAVSGLSPEKQQCEDKWGHLCALA
DFSLALTESIQEINKHSFNNFELRIGISHGSVVAGVIGAKKPQYDIWGKTVNLASRMDSTGVSGRIQ
VPEETYLILKDQGFAFDYRGEIYVKGISEQEGKIKTYFLLGRVQPNPFILPPRRLPGQYSLAAVVLGL
VQSLNRQRQKQLLNENNNTGIIKGHYNRRTLLSPSGTEPGAQAEGTDKSDLP (SEQ ID NO:246)

**Nucleotide:** D25538
**Sequence:**

tgaggaactgcgtgtggagtcagcccagtctggatgcacaggaggatgctggcggcacagtgagtgaggcctggtgccagagctgtgc
ggacccccttgttggccatggagcagcaggcccagaggccctctccccagccctgcttgcctgcctcggagaggacagaggcctaggcc
cacgggggagggtgttggcagacagatgccctccaggccctggggcctccttaacggccccttaacgacacgcgtgccaagggtgga
ggatgccagccaaggggcgctacttcctcaacgagggcgaggagggccctgaccaagatgcgctctacgagaagtaccagctcacc
agccagcatgggccgctgctgctcacgctcctgctggtggccgccactgcctgcgtggccctcatcatcattgccttcagccaggggggacc
cctccagacaccaggccattctgggcatggcgttcctggtgctggcggtgtttgcggccctctctgtgctgatgtacgtcgagtgtctcctgcg
gcgctggctcagggccttggcgctgctcacctgggcctgcttggtggcgctgggctatgtgctggtgttcgacgcatggacaaaggcggcc
tgtgcgtgggagcaggtgcccttcttcctgttcattgtcttcgtggtgtacacactactgcccttcagcatgcggggcgctgtcgccgttggggc
cgtctccactgcctcccacctcctggtgctcggttctttgatgggaggcttcacgacacccagtgtccgggtggggctgcagctgctggcca
acgcagtcatcttcctgtgtgggaacctgacaggcgccttccacaagcaccaaatgcaggatgcgtcccgggacctcttcacctacactgt
gaagtgcatccagatccgccggaagctgcgcatcgagaagcgccagcaggagaacctgctgctgtcagtgcttccggcccacatctcc
atgggcatgaagctggccatcatcgaacggctcaaggagcatggtgaccgtcgctgcatgcctgacaacaacttccacagcctctacgt
caagaggcaccagaatgtcagcatcctctatgcggacatcgtgggcttcacgcagctggccagcgactgttctcccaaggagctggtggt
ggtgctgaatgagctctttggcaagttcgaccagatcgccaaggccaacgagtgcatgcgaatcaagatcctcggcgactgctactactg
tgtatcgggcctgcccgtgtcgctgcctacccacgcccggaactgcgtgaagatggggctggacatgtgccaggccatcaagcaggtgc
gggaggccacgggcgtggacatcaacatgcgtgtgggcatacactcggggaatgtgctgtgcggggtcatcgggctgcgcaagtggca
gtatgacgtgtggtcccacgacgtgtccctggccaaccggatggaggcagccggagtacccggccgggtgcacatcacggaggccac
gctaaagcacctggacaaggcgtacgaggtggaggatgggcacgggcagcagcgggacccctacctcaaggagatgaacatccgc

acctacctggtcatcgacccccggagccagcagccacccccgcccagccaacacctccccaggcccaaggggacgcggccctga
agatgcgggcgtcagtgcgcatgacccggtacctcgagtcctgggggcggcacggccctttgcacatctcaaccaccgtgagagcgt
gagcagtggtgagacccacgtccccaacgggcggaggcctaagagcgttccccagcgccaccgccggaccccagacagaagcatg
tcccccaaggggcggtcggaggatgactcgtacgatgacgagatgctgtcagccattgaggggctcagctccacgaggccctgctgctc
caagtccgatgacttctacacctttgggtccatcttcctggagaagggctttgagcgcgagtaccgcctggcacccatcccccgggcccgc
cacgactttgcctgcgccagcctgatcttcgtctgcatcctgctcgtccatgtcctgctcatgcccaggacggcggcactgggtgtgtccttcg
ggctggtggcctgtgtactggggctggtgctgggcctgtgctttgccaccaagttctcgaggtgctgcccagctcgggggacgctctgcact
atctctgagagggtggagacacagcccctgctgaggctgaccctggccgtcctgaccatcggcagcctgctcactgtggccatcatcaac
ctgcccctgatgcctttccaagttccagagctgcctgttggcaatgagacaggcctactggccgcgagcagcaagacaagagccctgtgt
gagcccctcccgtactacacctgcagctgtgtcctgggcttcatcgcctgctcggtcttcctgaggatgagcctggagccaaaggttgtgctg
ctgacagtggccctggtggcctacctggtgctcttcaacctctccccatgctggcagtgggactgctgcggccaaggcctgggcaacctca
ccaagcccaacggcaccaccagtggcacccctagctgttcctggaaggacctgaagaccatgaccaatttctacctggtcctgttctacat
caccctgcttacactctccagacagattgactattactgccgcttggactgcctatggaagaagaagttcaagaaggagcacgaggagttt
gagaccatggagaacgtgaaccgccttcttctggagaacgtcctgccagcccacgtggctgcccactttatcggtgacaagttaaacgag
gactggtaccatcagtcctatgactgcgtctgtgtcatgtttgcctccgtgccggacttcaaagtgttctacacagagtgcgatgtcaacaaa
gaagggctggagtgcctacgcctgctcaatgagatcattgccgacttcgacgagctcctactgaagcccaagttcagcggcgtggagaa
gatcaagaccatcggcagcacgtacatggcagctgcagggctcagcgtcgcctcagggcacgagaaccaggagctggagcggcag
catgcccacattggtgtcatggtggagttcagcatcgccctgatgagtaagctggacggcatcaacaggcactccttcaactccttccgcct
ccgcgtcggcataaaccatgggcctgtgattgctggagtgattgggcccgaaaacctcagtatgacatctggggaaacactgtcaatgt
ggccagccgaatggaaagcactggagaacttgggaaaatccaggttaccgaggagacctgcaccatcctccagggcctcgggtactct
tgtgaatgccgtggcctgatcaacgtcaaaggcaaaggcgagctgaggacttactttgtctgtacggacactgccaagtttcaggggctgg
ggctgaactgagggctcctgctggattccgaaaaggccgggaagccagtctccttccctgaagcaagcccaggagaagactctccgcc
ccacgccaatcccaaaggcatgcagatggctgtgcatgttggcttctttggacctgcactggaggatttctcagacacatgcaccagattct
ggctcgaagcagccactgagccataatgcgcaggggaggccagaagctctgtgcctggtctgtaacagtttccaggccagctggagaa
tgttcactggttcggggctgactttgagatctttgttccctgaggtgccaggcaggcaactttagcacatgatgaaacagacttccacctca
gtggcctgtgggcacgcacaagtgaggtctgtttttctagacaccaaggggagtaagctgagctgtctagcacggattggagactccctc
tccctggtgggcctggcaatgacagcatttctcacagaggcattctggtaaatgaagctgaaaggggtgttttacatctgtaaacggtttcaa
acaggtagagagaaaaacaccacaattaacactgttactttttgccttgtctggcatgtttgttttaaatgaatacattaatgggtttttatcctttt
gaatgacttttcagacactagacataaatctcttccctccagtgtatgctctgcctttttaaccactgacatgtaaggaggactactgtctagca
tcagcttatggggtcagctggctgtggggatagagtcctgaggaatgtggtcacagcaagaaggcggggagcagcagagccttgccttt
gaatgaggcagcttgtgaggcaagcattctggagagaggtgctttgaaagtaaggtgcggcctttcacctcttccttgattactcacacatctt
tgcgttctcccctgccgtccttcaactgtatcttacttttcttaccagaaaggaatggagtctgtttagagacaacttggacaacctgtgagtgc
atctcttctttcctttagtcttcacagctaactctggagagcttcaaaactagaaggatctactccgcatgggtgcatgcagaggctcctggatc
tgggaagcccgccccctcacaaatgctgagccgttcttgctctgaaactgcgtgagtcaaggcaaatgcaaaaagccaggttttggggat
gtgtcttactgtgcttcaacttcccaaggaattgaaagtcaacctaactgtaacaacagggtgagaaatgaccaaactgcccgtgactttttc
tgaatggacttcataaccggaagacttaaccggtggcctcatcaccagagcatcgccaggatttctaatgcactcagtttccctacatagca
gggattcttagctaggtgtccccatgaaccccgtaaagttctacacaaagtcttgcatacaggagcctttacaagatgattatacagggttgc
agattgggtgactgaccagacttgttggggtcctgggatgagttgccccgggctgcaaattaagagtacagctaagtgcgggggtggcgg
tggagggaacgaaaattgaacctgtctgcctgtgctgtgtcgtgtggctttatcagcccgaggaagggcaggtgtattctaatttgcacaaa
ggtgctgggtagactagtggcagctctcatgtgctgcacataagtggaatcagtatgaatagaagaacttgctgtataaaggaatttcatgg
caacaatgctggtaagggcaattagcctcgcttaagttgcctttttacacaccaaaactttttacatgaagggctggtttcacatgaatactat
actgaaatctgtgccacaccaaaactttttacatgaagggctggtttcacatgaatactatactgaaatctgtgctctcaagatctagcagtga
ccagggctgcccggcgggggctctcctggcaagtcaggaaggtttctgttgctaatataacatagaaacacattagtgcactgggcctctc
tgaggtcagcatatttgtactcttggaatatttgtttttttcttcagtaacaacagaaaccccagttgggagtttaacaaataactgactaccact
cactcatgcatttttatttccaattaaagcaaagcactgtgctgtgctcagataataatagtttgtaagtaaaagtttttagttttcagtgttcaggtt
atagaatataactgaccataaaaattacctgcaggtattttctttttatgaacttgtttttaaattaccaagtaattactggtgtcattttgttttatgac
agacacacgtatctaacaaacaaacaaacagtgaccttctccatgggtcaaggacttccttacaatttctcctgagttaactttgtgaaaat
aatacctaaggttttctggcttattgaggaaatttcctaacaaacaaacaaacaaacaaacagaagagaagatcattaaccactgtatact
ttgtgtatataataggtcagtgtaaagaaatatgatttgaggtggtgcatgcaagtaactagggtttattctatataatgaatatttatagatctgt
aacatttgtttcaaaatgctgtttcatttttataaagtaccagtgtttagctgcttttatacattaaattagcaatttgaaaaactc (SEQ ID NO:247)

**P51828**

MPAKGRYFLNEGEEGPDQDALYEKYQLTSQHGPLLLTLLLVAATACVALIIIAFSQGDPSRHQAILG
MAFLVLAVFAALSVLMYVECLLRRWLRALALLTWACLVALGYVLVFDAWTKAACAWEQVPFFLFIV
FVVYTLLPFSMRGAVAVGAVSTASHLLVLGSLMGGFTTPSVRVGLQLLANAVIFLCGNLTGAFHKH
QMQDASRDLFTYTVKCIQIRRKLRIEKRQQENLLLSVLPAHISMGMKLAIIERLKEHGDRRCMPDN

NFHSLYVKRHQNVSILYADIVGFTQLASDCSPKELVVVLNELFGKFDQIAKANECMRIKILGDCYYC
VSGLPVSLPTHARNCVKMGLDMCQAIKQVREATGVDINMRVGIHSGNVLCGVIGLRKWQYDVWS
HDVSLANRMEAAGVPGRVHITEATLKHLDKAYEVEDGHGQQRDPYLKEMNIRTYLVIDPRSQQPP
PPSQHLPRPKGDAALKMRASVRMTRYLESWGAARPFAHLNHRESVSSGETHVPNGRRPKSVPQ
RHRRTPDRSMSPKGRSEDDSYDDEMLSAIEGLSSTRPCCSKSDDFYTFGSIFLEKGFEREYRLAP
IPRARHDFACASLIFVCILLVHVLLMPRTAALGVSFGLVACVLGLVLGLCFATKFSRCCPARGTLCTI
SERVETQPLLRLTLAVLTIGSLLTVAIINLPLMPFQVPELPVGNETGLLAASSKTRALCEPLPYYTCS
CVLGFIACSVFLRMSLEPKVVLLTVALVAYLVLFNLSPCWQWDCCGQGLGNLTKPNGTTSGTPSC
SWKDLKTMTNFYLVLFYITLLTLSRQIDYYCRLDCLWKKKFKKEHEEFETMENVNRLLLENVLPAH
VAAHFIGDKLNEDWYHQSYDCVCVMFASVPDFKVFYTECDVNKEGLECLRLLNEIIADFDELLLKP
KFSGVEKIKTIGSTYMAAAGLSVASGHENQELERQHAHIGVMVEFSIALMSKLDGINRHSFNSFRL
RVGINHGPVIAGVIGARKPQYDIWGNTVNVASRMESTGELGKIQVTEETCTILQGLGYSCECRGLI
NVKGKGELRTYFVCTDTAKFQGLGLN (SEQ ID NO:248)

# Figure 112

**Nucleotide:** AB023198
**Sequence:**
accttcagttcctccaagtcctggaagactgagacaaggggaagaaagcaagataagtgcgatggatgcatctccacggaatatttctcc
aggacttcagaatggagaaaaagaggatcgcttcttaacaactttgtccagccagagctccaccagttctactcatctccaattgcctacgc
cacctgaagtcatgtctgaacagtcagtgggagggccccctgagctagatacagccagcagttccgaagatgtgtttgatgggcatttgct
gggatccacagacagccaagtgaaggaaaagtcaaccatgaaagccatctttgcaaatttgcttccaggaaatagctataatcctattcc
atttcctttgatccagataaacactacttaatgtatgaacatgaacgagtgcccattgcagtctgcgagaaggaacccagctccatcattgc
ttttgctctcagttgtaaagaataccgaaatgccttagaggaattgtctaaagcgactcagtggaacagtgccgaagaagggcttccaaca
aatagtacttcagatagcagaccaaagagtagcagccctatcagattacctgaaatgagtggaggacagacaaatcgtacaacagaa
acagaaccacaaccaaccaaaaaggcttctggaatgttgtccttcttcagagggacagcagggaaaagccccgatctctcttcccagaa
gagagagaccttacgtggagcagatagtgcttactaccaggttggcagacaggcaaggaggggaccgagaatcaaggcgttgagc
ctcaagatgaagtagatggaggagatacgcaaaagaagcaactcataaatcctcatgtggaacttcaattttcagatgctaatgccaagtt
ttactgtcggctctactatgcgggagagtttcataagatgcgtgaagtgattctggacagcagtgaggaagatttcattcgttccctctcccact
catcaccctggcaggcccggggaggcaaatcaggagctgccttctatgcaactgaggatgatagatttattttgaagcaaatgcctcgtct
ggaagtccagtccttcctcgactttgcaccacattacttcaattatattacaaatgctgttcaacaaaagaggcccacggcgttggccaaaat
tcttggagtttacagaattggttataagaactctcagaacaacactgagaagaagttagatctccttgtcatggaaaatcttttctacgggaga
aagatggcacaggtttttgatttgaagggctctcttaggaatcggaatgtaaaaactgacactggaaaagagagttgtgatgtggtcctgct
agatgaaaatctcctaaagatggttcgagacaaccctctatatattcgttctcattccaaagctgtgctgagaacctcgatccatagtgactc
ccatttcctttctagccacctcattatagattattctttgctggttgggcgagatgatactagcaatgagctagtagttggaattatagattatattc
gaacatttacatgggacaaaaagcttgagatggttgtgaaatcaacaggaattttaggtggacaaggtaaaatgccaacagtggtgtctc
cggagttgtacaggactaggttttgtgaggcaatggacaagtatttcctaatggtaccagaccactggacaggcttgggtctgaattgctga
aatcaagcacatatttgaaatggactgtgaaggaaaaggggacaggaacaaaggaccaaaaataagctacatgttttatttcttcatcgt
gttcaccactgtatgccaaggcttttcagttctgtgtctgtttagactgtccgtaatggaatggtaaaactccatgaatttgcactttggtttttgata
cctgtggagctgtctgtacgttgggaagtggcatgaaaatttcttaagctaaaatacagacatgtttcaaagggctaaagttggagatgagt
agatagggtgaaaaatgggttaaatttgctagcttaattgttttaagaagaaaacagtgtctcataaattgactatcctggcatcacatttaac
atgttatctacttagaaagcatttgtagagctgctgaatttgttttgtgtttttctgtaataatttaatgttacttattatcagaatttctgaaacctttaca
aaaattctgatttattccattaatggccagttaaacacgtgggcatttattgttttattgaggaatttgacttaaactgggaatcctgtcatgttgttt
atctttccagcttgcctgtttttgagtatgtttgatgtttttaaaattttgtcttctctgtggatgacaggaggctacagcaattaactttaagcctccttt
tagagatatttttaaagcttgtttaaaatttttgtgcaattcatatattaaattgcacttacttgcatacgctcatattctagggtttttctctatttttagg
gtatcatagtaaatcattagtaaatgagtctgtagttactaaaccctaatggaataattattaatgaaagatttttgaaatataaaaaataaatt
aggcccaatccaagaaattgagtgagaaggaaacacttgttttattcacagaggtaaagtgtcttttcaatataaccagcaatttaggtggc
atctataaaataaaaaatttctactgtggacatccccttttccaactttctacataatggctagttctgactactaagaaatgttaagaaatagg
ccaagtgcggtggctcacgcctataatcctatcactttgggaggcctaggcaggcagatcacctgaggtcaggagttcaagaccagcctg
gccaacatgaggaaaccccatctctactaaaaagacaaaaaattagctgggcatggtggcatatacctgtaaccccaactacttgggtg
gctgaggcaggagaattgtttgaacctgggaggcggaggctgcagtgagctgggattacaccattgcattccagcctgggcaacagag
caagactgtctcaaaaaacaaaacgaaacaaaaaaagaaagttattcttagtaaggaacttcttgtttaatagcatttttgtttattttaaaaa
gtgatcagaagtagtaaactatctttgaggaaatactgtaaccccagaatatttcctcttgacttctttttgtaacaaggataatttagggatttat
aaagttgtaaggatttcactgttttcggactgcctataataatagcacattaaccttcacataataagaaatctggacaagttcagttacacag
tatgatgaatacttgaattaggaacattgtggaaaatttgctttagagaatcaaggcagtagtttggtatttggtgcttattaaaaatgtggtttgtt
ttgaactggaagcaagttgaccaaggacttatgactaatgtgatgctaagttccacttggccccttttaaaaacgtgtatgtgccttttgaagat
acacaaaacactgaggatttagttttgaaatcaaagactattaaaggagctgtacagaggtaaaaaaataaatgtggaacattattaactt
attttgtgtctaggaacaatggatttgtatctgatttaaaatgccaacactgttttgtctctgttcattttttctgtgaggatacttaaggttattattcct
gtctgtttcctgtactcccctagtcatgagcacttgaagtacaaggtgtctccccctaggtgcaattaggttgtttcttgttttagtttcaattctatg
tgcatagcaggaatgctcccacaggaatggcttctgacaataatctgtcctgttgattttgttttccttgcccatgacttgaacaactgtgttttaaa
gtactgtagtctagtaggtaactttgtggcaaaaattttcaatataatacattctgaaacaatagttgctgccttgcaaaggtaatctctcattta
aaattggacagtattaatgaaggggaaatatacaatttatttctattgagtggtagaactatatgtctggtcccttgctgctcttgtttaggccact
atcatagatatatttcaaatattgtactactcagtgttaagtattgaatgactgtttccctttccttcaaggcctagagtatattctgaaaatttagga
atgaggaagaaatcttaatacttccttccttaacatacaacatgagtcccgagaataattgatagtagcaagagaaaactatgtcagtaac
atgttgctttgtataaaaatcttatttataaatgtgaagctttttgatgccatcaaaacttattaaaaaataggatttactttttctaattctgacctaa
gaaaaataatgagaacaagctgttgcaagctcttttgtagtctattgaatattttatagatattcaaaatttcctacaaactataatttttccatga
tttagcagtgagtgatttctagctttggctcttattaggtattgtaaatagtagggttatatcgatatcagcttttgtgatggcattgtggtcatcagc
ttcatgacattttacccatttgcagtgatcctgtgtaaaactgccaaggaaagtaattacctgtaggagtttgctgagcttgaagagtgaaaac
tgttgtgaatgagcctgatcataaaacggaccaggccattcattattcctcaagtgttaatatactgacttatgcagtattcaaaccatctagtg
caatgtttttgttttgtttttttttttttggtaacacaggtgcagtgtattatagaaaaaataaaaactacaatcattagcagttttaatactgctgtgt

cagttttgtaaaaaatgtacattatgtcttttgacatgttgaattttaaactagggaaatgacattgtaaatcatagtagcctcttttaatttaatatg
aaaaatgccactatattgaaagtacttaatgtattgtatatatttctctactttggttctagctattttatatgattgacatgttatttaaaagataactg
ccttgaacttttggagacttgtactgtaaataaagaaatcttaacaataaactcagaatctactt (SEQ ID NO:249)

**Q9Y2I7**
PSVPPSPGRLRQGEESKISAMDASPRNISPGLQNGEKEDRFLTTLSSQSSTSSTHLQLPTPPEVM
SEQSVGGPPELDTASSSEDVFDGHLLGSTDSQVKEKSTMKAIFANLLPGNSYNPIPFPFDPDKHY
LMYEHERVPIAVCEKEPSSIIAFALSCKEYRNALEELSKATQWNSAEEGLPTNSTSDSRPKSSSPIR
LPEMSGGQTNRTTETEPQPTKKASGMLSFFRGTAGKSPDLSSQKRETLRGADSAYYQVGQTGK
EGTENQGVEPQDEVDGGDTQKKQLINPHVELQFSDANAKFYCRLYYAGEFHKMREVILDSSEED
FIRSLSHSSPWQARGGKSGAAFYATEDDRFILKQMPRLEVQSFLDFAPHYFNYITNAVQQKRPTA
LAKILGVYRIGYKNSQNNTEKKLDLLVMENLFYGRKMAQVFDLKGSLRNRNVKTDTGKESCDVVL
LDENLLKMVRDNPLYIRSHSKAVLRTSIHSDSHFLSSHLIIDYSLLVGRDDTSNELVVGIIDYIRTFTW
DKKLEMVVKSTGILGGQGKMPTVVSPELYRTRFCEAMDKYFLMVPDHWTGLGLNC (SEQ ID NO:250)

# Figure 113

**Nucleotide:** AL136679
**Sequence:**
agtgacaattaaagatggctgcgcccatgtaacatcactagcgaccggtgacctctttttccccttgcctggctcctgtggtggcaggctgg
gcacgaggaccatgctgggccggagcctccgagaagtttctgcggcactgaaacaaggccaaattacaccaacagagctctgtcaaa
aatgtctctctcttatcaagaaggccaagtttctaaatgcctacattactgtgtcagaagaggtggccttaaaacaagctgaagaatcagaa
aagagatataagaatggacagtcacttggggatttagatggaattcctattgcagtaaaagacaatttcagcacttctggcattgagacaa
catgtgcatcaaatatgctgaaaggttatataccaccttataatgctacagtagttcagaagttgttggatcagggagctctactaatgggaa
aaacaaatttagatgagtttgctatgggatctgggagcacagatggtgtatttggaccagttaaaaaccccctggagttattcaaaacgatat
agagaaaagaggaagcagaatccccacagcgagaatgaagattcagactggctgataactggaggaagcccaggtgggagtgcag
ctgctgtatcggcgttcacatgctacgcggctttaggatcagatacaggaggatcgaccagaaatcctgctgcccactgtgggcttgttggtt
tcaaaccaagctatggcttagtttcccgtcatggtctcattcccctggtgaattcgatggatgtgccaggaatcttaaccagatgtgtggatgat
gcagcaattgtgttgggtgcactggccggacctgaccccagggactctaccacagtacatgaacctattaataaaccattcatgcttccca
gtttggcagatgtgagcaaactatgtataggaattccaaaggaatatcttgtaccggaattatcaagtgaagtacagtctctttggtccaaag
ctgctgacctctttgagtctgagggggccaaagtaattgaagtatcccttcctcacaccagttattcaattgtctgctaccatgtattgtgcacat
cagaagtggcatcgaatatggcaagatttgatgggctacaatatggtcacagatgtgacattgatgtgtccactgaagccatgtatgctgca
accagacgagaaggatttaatgatgtggtgagaggaagaattctctcaggaaacttttcttattaaaagaaaactatgaaaattattttgtca
aagcacagaaagtgagacgcctcattgctaatgactttgtaaatgcttttaactctggagtagatgtcttgctaactcccaccaccttgagtga
ggcagtaccatacttggagttcatcaaagaggacaacagaacccgaagtgcccaggatgatattttacacaagctgtaaatatggcag
gattgccagcagtgagtatccctgttgcactctcaaaccaagggttgccaataggactgcagtttattggacgtgcgttttgtgaccagcagc
ttcttacagtagccaaatggtttgaaaaacaagtacagtttcctgttattcaacttcaagaactcatggatgattgttcagcagtccttgaaaat
gaaaagttagcctctgtctctctaaaacagtaaacatatcttacaaattaaaatgacttttaggctgggtgcagtggctcacacctgtaatccc
agcactttgggaggccaaggcgagcggatcatgaggtcagaagatctagaacagcctggtcaacatggtgaaaccccgtctctactaa
aaatacaaaaattagccaggcttagtggcgggcatctgtagtcccagctactcaggaggctgaggcaggagaatcacttgaaccctgga
ggtggaggttgcagtgagccgagatcatgccactgcactgcactccagcctgggtgacaaagcaagactgtgtctcaaaataaataaat
aaaataaaataaaatgacgtacagagattctatattctagagagtcaaatggtcttgctcaattcttgtaattaggttcttgttaatacagtcatt
ccatggaattactttttaaaattcctgtgacaattaataataaataacgtgtcagcatttagtaagcatccactaagtgtacaatacttctacaat
aacacaagatacctgttcctcaaagacaatgcattctgccataatgttcattaaagagtttacagtaaaaataagattagggataaacttctc
aaaaattgtacatctgtgtaactaaagcactaacaaaaacatgaatagtccttctagaggtaacttggatagcctaggcaggcaacttatc
atgtggtgaaggccgcctcaggggttgttaaaaatgcacagaaacaattgagtgcgattattggcttctgagcgctgagcagagcaggtg
gaagaggaactttgagcacaggaggaaatgcaaccagtcagggcccagaatcatgcaaatctcaggggtatgcctctctggggagga
gctccacttgcagggactccttttatttccctaagaaagagctgaaatgactgagaactttcctttcctccttagagttacaattttacttctgctat
tccggagcccatgcctagaagccagaacaactccatgttacactgagttcatgctcctatttactgatcacaaatgagctcattaatgtcatc
gaaacatttattgtaacctaacagaccatcacagattggaaacttggtagatagcagagcatggtattagtgaaaaaggttcaaaataca
caagtaacatacactctgaaaaacatgcagataatttgctgatgaagcagaagaggggatgcgcatggcaagaacttgccttacccca
gattctctatatctcatggtttcctttcctcttgactgtctttacgagtgtttttttatttgggaccctcgagcccagagatattaatggatatctgtattc
aatatttgacaaaatctaatggaaaccatccatttactcatgataaggcttcatcactggatttctgtgtcttcactagaacaccattgtcatctc
atattgatcaggtattttaatctagcacttacatattgttgataaatgaaagctgaattgttacttaataaattcactttgtttagcaaaaaaaaaa
aaaaaaaa (SEQ ID NO:251)

**Q9H0R6**
MLGRSLREVSAALKQGQITPTELCQKCLSLIKKAKFLNAYITVSEEVALKQAEESEKRYKNGQSLG
DLDGIPIAVKDNFSTSGIETTCASNMLKGYIPPYNATVVQKLLDQGALLMGKTNLDEFAMGSGSTD
GVFGPVKNPWSYSKRYREKRKQNPHSENEDSDWLITGGSPGGSAAAVSAFTCYAALGSDTGGS
TRNPAAHCGLVGFKPSYGLVSRHGLIPLVNSMDVPGILTRCVDDAAIVLGALAGPDPRDSTTVHEP
INKPFMLPSLADVSKLCIGIPKEYLVPELSSEVQSLWSKAADLFESEGAKVIEVSLPHTSYSIVCYHV
LCTSEVASNMARFDGLQYGHRCDIDVSTEAMYAATRREGFNDVVRGRILSGNFFLLKENYENYFV
KAQKVRRLIANDFVNAFNSGVDVLLTPTTLSEAVPYLEFIKEDNRTRSAQDDIFTQAVNMAGLPAV
SIPVALSNQGLPIGLQFIGRAFCDQQLLTVAKWFEKQVQFPVIQLQELMDDCSAVLENEKLASVSL
KQ (SEQ ID NO:252)

# Figure 114

**Nucleotide:** AL031428
**Sequence:**
cggcgcgcgggcagcgtgaagcgaggcgaggcaaggcttttcggacccacggagcgacagagcgagcggcccctacggccgtcg
gcggcccggcggcccgagatgttatctgggaagaaggcggcagccgcggcggcggcggctgcagcggcagcaaccgggacggag
gctggccctgggacagcaggcggctccgagaacgggtctgaggtggccgcgcagcccgcgggcctgtcgggcccagccgaggtcg
ggccgggggcggtgggggagcgcacaccccgcaagaaagagcctccgcgggcctcgcccccccgggggcctggcggaaccgccg
gggtccgcagggcctcaggccggccctactgtcgtgcctgggtctgcgacccccatggaaactggaatagcagagactccggagggg
cgtcggaccagccggcgcaagcgggcgaaggtagagtacagagagatggatgaaagcttggccaacctctcagaagatgagtattat
tcagaagaagagagagaaatgccaaagcagagaaggaaaagaagcttcccccaccacccccctcaagccccacctgaggaagaaaat
gaaagtgagcctgaagaaccatcggggcaagcaggaggacttcaagacgacagttctggagggtatggagacggccaagcatcag
gtgagggtggcattagatgcttgccacttagtagcaagagccgccaagtaggaattgatgcttgcaagtgtttttggctgtggtatccacatttt
agccagaagcgcagatgtcctttgctttcttcgttaggtgtggagggcgcagctttccagagccgacttcctcatgaccggatgacttctcaa
gaagcagcctgttttccagatattatcagtggaccacaacagacccagaaggtttttcttttcattagaaaccgcacactgcagttgtggttgg
ataatccaaagattcagctgacatttgaggctactctccaacaattagaagcaccttataacagtgatactgtgcttgtccaccgagttcaca
gttatttagagcgtcatggtcttatcaacttcggcatctataagaggataaaacccctaccaactaaaaagacaggaaaggtaattattata
ggctctggggtctcaggcttggcagcagctcgacagttacaaagttttggaatggatgtcacacttttggaagccagggatcgtgtgggtgg
acgagttgccacatttcgcaaaggaaactatgtagctgatcttggagccatggtggtaacaggtcttggagggaatcctatggctgtggtca
gcaaacaagtaaatatggaactggccaagatcaagcaaaaatgcccactttatgaagccaacggacaagctgttcctaaagagaaag
atgaaatggtagagcaagagtttaaccggttgctagaagctacatcttaccttagtcatcaactagacttcaatgtcctcaataataagcctg
tgtcccttggccaggcattggaagttgtcattcagttacaagagaagcatgtcaaagatgagcagattgaacattggaagaagatagtga
aaactcaggaagaattgaaagaacttcttaataagatggtaaatttgaaagagaaaattaaagaactccatcagcaatacaaagaagc
atctgaagtaaagccacccagagatattactgccgagttcttagtgaaaagcaaacacagggatctgaccgccctatgcaaggaatatg
atgaattagctgaaacacaaggaaagctagaagaaaaacttcaggagttggaagcgaatcccccaagtgatgtatatctctcatcaaga
gacagacaaatacttgattggcattttgcaaatcttgaatttgctaatgccacacctctctcaactctctcccttaagcactgggatcaggatg
atgactttgagttcactggcagccacctgacagtaaggaatggctactcgtgtgtgcctgtggctttagcagaaggcctagacattaaactg
aatacagcagtgcgacaggttcgctacacggcttcaggatgtgaagtgatagctgtgaatacccgctccacgagtcaaacctttatttataa
atgcgacgcagttctctgtacccttcccctgggtgtgctgaagcagcagccaccagccgttcagtttgtgccacctctccctgagtggaaaa
catctgcagtccaaaggatgggatttggcaaccttaacaaggtggtgttgtgttttgatcgggtgttctgggatccaagtgtcaatttgttcggg
catgttggcagtacgactgccagcaggggtgagctcttcctcttctggaacctctataaagctccaatactgttggcactagtggcaggaga
agctgctggtatcatggaaaacataagtgacgatgtgattgttggccgatgcctggccattctcaaagggatttttggtagcagtgcagtacc
tcagcccaaagaaactgtggtgtctcgttggcgtgctgatccctgggctcggggctcttattcctatgttgctgcaggatcatctggaaatgac
tatgatttaatggctcagccaatcactcctggcccctcgattccaggtgccccacagccgattccacgactcttctttgcgggagaacatacg
atccgtaactacccagccacagtgcatggtgctctgctgagtggctgcgagaagcgggaagaattgcagaccagtttttgggggccatg
tatacgctgcctcgccaggccacaccaggtgttcctgcacagcagtccccaagcatgtga (SEQ ID NO:253)

**Q9NUH3**
RRAGSVKRGEARLFGPTERQSERPLRPSAARRPEMLSGKKAAAAAAAAAAAATGTEAGPGTAG
GSENGSEVAAQPAGLSGPAEVGPGAVGERTPRKKEPPRASPPGGLAEPPGSAGPQAGPTVVPG
SATPMETGIAETPEGRRTSRRKRAKVEYREMDESLANLSEDEYYSEEERNAKAEKEKKLPPPPPQ
APPEEENESEPEEPSGQAGGLQDDSSGGYGDGQASGEGGIRCLPLSSKSRQVGIDACKCFWLW
YPHFSQKRRCPLLSSLGVEGAAFQSRLPHDRMTSQEAACFPDIISGPQQTQKVFLFIRNRTLQLW
LDNPKIQLTFEATLQQLEAPYNSDTVLVHRVHSYLERHGLINFGIYKRIKPLPTKKTGKVIIIGSGVSG
LAAARQLQSFGMDVTLLEARDRVGGRVATFRKGNYVADLGAMVVTGLGGNPMAVVSKQVNMEL
AKIKQKCPLYEANGQAVPKEKDEMVEQEFNRLLEATSYLSHQLDFNVLNNKPVSLGQALEVVIQL
QEKHVKDEQIEHWKKIVKTQEELKELLNKMVNLKEKIKELHQQYKEASEVKPPRDITAEFLVKSKH
RDLTALCKEYDELAETQGKLEEKLQELEANPPSDVYLSSRDRQILDWHFANLEFANATPLSTLSLK
HWDQDDDFEFTGSHLTVRNGYSCVPVALAEGLDIKLNTAVRQVRYTASGCEVIAVNTRSTSQTFI
YKCDAVLCTLPLGVLKQQPPAVQFVPPLPEWKTSAVQRMGFGNLNKVVLCFDRVFWDPSVNLFG
HVGSTTASRGELFLFWNLYKAPILLALVAGEAAGIMENISDDVIVGRCLAILKGIFGSSAVPQPKETV
VSRWRADPWARGSYSYVAAGSSGNDYDLMAQPITPGPSIPGAPQPIPRLFFAGEHTIRNYPATVH
GALLSGLREAGRIADQFLGAMYTLPRQATPGVPAQQSPSM (SEQ ID NO:254)

# Figure 115

**Nucleotide:** AF076974
**Sequence:**
cgcgccggggcctggtgctcggtcggcgggtgctgccgctttaagcgggggcgggactgcgcgcggccgagcggttgcgacgagggc
tcggctgggggtcgccggggtcgcgggccgggcctgcaggagccgggccgccgaggtcggggctggttgaactcatggacctgatact
tttctcttgagaagcaaaccagcccaaaagaaaaatggcgtttgttgcaacacagggggccacggtggttgaccagaccactttgatgaa
aaagtaccttcagtttgtggcagctctcacagatgtgaatacacctgatgaaacaaagttgaaaatgatgcaagaagttagtgaaaattttg
agaatgtcacgtcatctcctcagtattctacattcctagaacatatcatccctcgattccttacatttctccaagatggagaagttcagtttcttca
ggagaaaccagcacagcaactgcggaagctcgtacttgaaataattcatagaataccaaccaacgaacatcttcgtcctcacacaaaa
aatgttttgtctgtgatgtttcgctttttagagacggaaaatgaagaaaatgttcttatttgtctaagaataattattgagctacacaaacagttca
ggccaccgatcacacaagaaattcatcatttctggattttgtgaaacagatttacaaggagcttccaaaagtagtgaaccgctactttgag
aaccctcaagtgatccccgagaacacagtgcctcccccagaaatggttggtatgataacaacgattgctgtgaaagtcaacccggagcg
tgaggacagtgagactcgaacacattccatcattccgaggggatcactttctctgaaagtgttggcagaattgcccattattgttgttttaatgt
atcagctctacaaactgaacatccacaatgttgttgctgagtttgtgcccttgatcatgaacaccattgccattcaggtgtctgcacaagcga
ggcaacataagctttacaacaaggagttgtatgctgacttcattgctgctcagattaaaacattgtcatttttagcttacattatcaggatttacc
aggagttggtgactaagtattctcagcagatggtgaaaggaatgctccagttactttcaaattgtccagcagagactgcacacctcagaaa
ggagcttctgattgctgccaaacacatcctcaccacagagctgagaaaccagttcattccttgcatggacaagctgtttgatgaatccatact
aattggctcaggatatactgccagagagagactctaaggcccctcgcctacagcacgctggccgacctcgtgcaccatgtccgccagcacc
tgcccctcagcgacctctccctcgccgtccagctcttcgccaagaacatcgacgatgagtccctgcccagcagcatccagaccatgtcctg
caagctcctgctgaacctggtggactgcatccgttccaagagcgagcaggagagtggcaatgggagagacgtcctgatgcggatgctg
gaggttttcgttctcaaattccacacaattgctcggtaccagctctctgccattttaagaagtgtaagcctcagtcagaacttggagccgtgg
aagcagctctgcctggggtgcccactgcccctgcagctcctggccctgctccctccccagcccctgtccctgccccacctccacccccgcc
cccacccccacctgccacccctgtgaccccggcccccgtgcctcccttcgagaagcaaggagaaaaggacaaggaagacaagcag
acattccaagtcacagactgtcgaagtttggtcaaaaccttggtgtgtggtgtcaagacaatcacgtggggcataacatcatgcaaagcac
ctggtgaagctcagttcattcccaacaagcagttacaacccaaagagacacagatttacatcaaacttgtgaaatatgcaatgcaagcttt
agatatttatcaggtccagatagcaggaaatggacagacatacatccgtgtggccaactgccagactgtgagaatgaaagaggagaag
gaggtattggagcatttcgctggtgtgttcacaatgatgaaccccttaacgttcaaagaaatcttccaaactacggtcccttatatggtggag
agaatctcaaaaaattatgctcttcagattgttgccaattccttcttggcaaatcctactacctctgctctgtttgctacgattctggtggaatatct
ccttgatcgcctgccagaaatgggctccaacgtggagctctccaacctgtacctcaagctgttcaagctggtctttggctctgtctccctcttg
cagctgaaaatgaacaaatgctgaagcctcacttgcacaagattgtgaacagctctatggagctcgcgcagactgccaaggaaccctta
caactacttcttgctgctacgggcgctgtttcgctctattggtggaggtagccacgatctcttgtatcaggagttcttgcctctccttccaaacctc
ctgcaagggctgaacatgcttcagagtggcctgcacaagcagcacatgaaggacctctttgtggagctgtgtctcaccgtccctgtgcggc
tgagctcgcttttgccgtacctgcccatgcttatggatcccttggtgtctgcactcaatgggtctcagacattggtcagccaaggcctcaggac
gctggagctgtgtgtggacaacctgcagcccgacttcctctacgaccacatccagccggtgcgcgcagagcttatgcaggctctgtggcg
caccttacgcaaccctgctgacagcatctcccacgtggcctaccgtgtgctcggtaagtttggcggcagtaacaggaagatgctgaagga
gtcgcagaagctgcactacgttgtgaccgaggttcagggccccagcatcactgtggagttttccgactgcaaagcttctctccagctcccca
tggagaaggccattgaaactgctctggactgcctgaaaagcgccaacactgagccctactaccggaggcaggcgtgggaagtgatca
aatgcttcctggtggccatgatgagcctggaggacaacaagcacgcactctaccagctcctggcacaccccaactttacagaaaagac
catccccaatgttatcatctcacatcgctacaaagcccaggacactccagcccggaagactttgagcaggccctgacaggcgccttcat
gtctgctgtcattaaggacctgcggcccagcgccctgccctttgtcgccagcttgatccgccactatacgatggtggcagtcgcccagcagt
gtggcccctttcttgctgccttgctaccaggtgggcagccagcccagcacagccatgtttcacagtgaagaaaatggctcgaaaggaatgg
atcctttggttctcattgatgcaattgctatttgtatggcatatgaagaaaaggagctttgcaaaatcggggaggtggccctagctgtgatattt
gatgttgcaagtatcatcctgggctccaaggagagggcctgccagctgcccctgttttcttacatcgtggagcgcctgtgtgcatgttgttatg
aacaggcgtggtatgcaaagctgggggtgtggtgtctattaagtttctcatggagcggctgcctctcacttgggttctccagaaccagcag
acattcctgaaagcacttctctttgtcatgatggacttaactggagaggtttccaatggggcagtcgctatggcaaagaccacgctggagca
gcttctgatgcggtgcgcaacgcctttaaaagacgaggagagagccgaagagatcgtggccgcccaggaaaagtctttccaccatgtg
acacacgacttggttcgagaagtcacctctccaaactccactgtgaggaagcaggccatgcattcgctgcaggtgttggcccaggtcact
gggaagagtgtcacggtgatcatggaaccccacaaagaggtcctgcaggatatggtcccccctaagaagcacctgctccgacaccag
cctgccaacgcacagattggcctgatggaggggaacacgttctgtaccacgttgcagcccaggctcttcacaatggaccttaacgtggtg
gagcataaggtgttctacacagagctgttgaatttgtgtgaggctgaagattcagctttaacaaagctgccctgttataaaagccttccgtca
ctcgtacctttacgaattgcggcattaaatgcacttgctgcctgcaattaccttcctcagtccagggagaaaatcatcgctgcactcttcaaag
ccctgaattccaccaatagtgagctccaagaggccggagaagcctgtatgagaaagtttttagaaggtgctaccatagaagtcgatcaa
atccacacacatatgcgacctttgctgatgatgctgggagattaccggagcttgacgctgaatgttgtgaatcgcctgacttcggtcacgag
gctcttcccaaattccttcaatgataaatttgtgatcagatgatgcaacatctgcgcaagtggatggaagtggtggtgatcacccacaaag
ggggccagaggagcgacggaaacgaaatgaagatttgctcagcaattataaacctttttcatctgatcccggctgctcctcagacactggt
gaagcctttgctagaggttgtcatgaaaacggagcgggcgatgctgatcgaggcggggagtccattccgagagcccctgatcaagttcct

gactcgacatccctcgcagacagtggagctgttcatgatggaagccacactgaacgatccccagtggagcagaatgtttatgagtttttaa
aacacaaagacgccagacctctgcgggatgtgctggctgccaaccccaacaggttcatcaccctgctgctgccggggggtgcccagac
ggctgtgcgccccggttcgcccagcaccagcaccatgcgcctggacctccagttccaggccatcaagatcataagcattatagtgaaaa
acgatgactcctggctggccagccagcactctctggtgagccagttgcgacgtgtgtgggtgagtgagaacttccaagagaggcaccgc
aaggagaacatggcagccaccaactggaaggagcccaagctgctggcctactgcctgctgaactactgcaaaaggaattacggagat
atagaattgctgttccagctgctccgagcctttactggtcgttttctctgcaacatgacattcttaaaagagtatatggaggaagagattcccaa
aaattacagcatcgctcagaaacgtgccctgttctttcgctttgtagacttcaacgaccccaacttcggagatgaattaaaagctaaagttct
gcagcatatcttgaatcctgctttcttgtacagctttgagaagggggaaggagagcagctcttgggacctcccaatccagaaggagataac
ccagaaagcatcaccagtgtgtttattaccaaggtcctggaccccgagaagcaggcggacatgctggactcgctgcggatctacctgctg
cagtacgccacgctgctggtggagcacgcccccccaccacatccatgacaacaacaagaaccgcaacagcaagctgcgccgcctcat
gaccttcgcctggccctgcctgctctccaaggcctgcgtggacccagcctgcaagtacagcggacacttgctcctggcgcacattatcgcc
aaattcgccatacacaagaagatcgtcctgcaggtttttcatagtctcctcaaggctcacgcaatggaagctcgagcgatcgtcagacag
gcgatggccattctgaccccggcggtgccggccaggatggaggacgggcaccagatgctgacccactggacccggaagatcattgtg
gaggaggggcacaccgtcccgcagctggtccacattctgcacctgatagtgcaacacttcaaggtgtactacccggtacggcaccactt
ggtgcagcacatggtgagcgccatgcagaggctgggcttcacgcccagtgtcaccatcgagcagaggcggctggccgtggacctgtct
gaagtcgtcatcaagtgggagctgcagaggatcaaggaccagcagccggattcagatatggacccaaattccagtggagaaggagtc
aattctgtctcatcctccattaagagaggcctgtccgtggattctgcccaggaagtgaaacgctttaggacggccaccggagccatcagtg
cagtctttgggaggagccagtcgctacctggagcagactctctcctcgccaagcccattgacaagcagcacacagacactgtggtgaac
ttccttatccgcgtggcctgtcaggttaatgacaacaccaacacagcggggtcccctggggaggtgctctctcgccggtgtgtgaaccttct
gaagactgcgttgcggccagacatgtggcccaagtccgaactcaagctgcagtggttcgacaagctgctgatgactgtggagcagcca
aaccaagtgaactatgggaatatctgcacgggcctagaagtgctgagcttcctgctaactgtcctccagtccccagccatcctcagtagctt
caaacctctgcagcgtggaattgccgcctgcatgacatgtggaaacaccaaggtgttgcgagccgtccacagccttctctcgcgcctgat
gagcattttcccaacagagccgagtacttccagtgtggcctccaaatatgaagagctggagtgcctctacgcagccgtcggaaaggtcat
ctatgaagggctcaccaactacgagaaggccaccaatgccaatccctcccagctcttcgggacccttatgatcctcaagtctgcctgcag
caacaaccccagctacatagacaggctgatctccgtctttatgcgctccctgcagaagatggtccgggagcatttaaaccctcaggcagc
gtcaggaagcaccgaagccacctcaggtacaagcgagctggtgatgctgagtctggagctggtgaagacgcgcctggcagtgatgag
catggagatgcggaagaacttcatccaggccatcctgacatccctcatcgaaaaatcaccagatgccaaaatcctccgggctgtggtca
aaatcgtggaagaatgggtcaagaataactccccaatggcagccaatcagacacctacactccgggagaagtccattttgcttgtgaag
atgatgacttacatagaaaaacgctttccggaagaccttgaattaaatgcccagtttttagatcttgttaactatgtctacagggatgagaccc
tctctggcagcgagctgacggcgaaacttgagcctgcctttctctctgggctgcgctgtgcccagccactcatcagggcaaagtttttcgag
gtttttgacaactccatgaaacgtcgtgtctacgagcgcttgctctatgtgacctgttcgcagaactgggaagccatggggaaccacttctgg
atcaagcagtgcattgagctgcttctggccgtgtgtgagaagagcacccccattggcaccagctgccaaggagccatgctcccgtccatc
accaacgtcatcaacctggccgatagccacgaccgtgccgccttcgccatggtcacacatgtcaagcaggagccccgggagcgggag
aacagcgagtccaaagaggaggatgtagagatagacatcgaactagctcctggggatcagaccagcacgcccaaaaccaaagaac
tttcagaaaaggacattggaaaccagctgcacatgctaaccaacaggcacgacaagtttctggacactctccgagaggtgaagactgg
agcgctgctcagcgctttcgttcagctgtgccacatttccacgacgctggcagagaagacgtgggtccagcttttccccagattgtggaaga
tcctctctgacagacagcagcatgcactcgcgggtgagataagtccatttctgtgcagcggcagtcaccaggtgcagcgggactgccag
cccagcgcgctgaactgctttgtggaagccatgtcccagtgcgtgccgccaatccccatccgaccctgcgtcctgaagtacctggggaag
acacacaacctctggttccggtccacgctgatgttggagcaccaggcttttgaaaagggtctgagtcttcagattaagccgaagcaaaca
acggagttttatgagcaggagagcatcaccccgccgcagcaggagatactggattcccttgcggagctttactccctgttacaagaggaa
gatatgtgggctggtctgtggcagaagcggtgcaagtactcggagacagcgactgcgattgcttacgagcagcacgggttctttgagcag
gcacaagaatcctatgaaaaggcaatggataaagccaaaaaagaacatgagaggagtaacgcctcccctgctattttccctgaatacc
agctctgggaagaccactggattcgatgctccaaggaattgaaccagtgggaagccctgacggagtacggtcagtccaaaggccacat
caacccctacctcgtcctggagtgcgcctggcgggtgtccaactggactgccatgaaggaggcgctggtgcaggtggaagtgagctgtc
cgaaggagatggcctggaaggtgaacatgtaccgcggatacctggccatctgccaccccgaggagcagcagctcagcttcatcgagc
gcctggtggagatggccagcagcctggccatccgcgagtggcggcggctgccccacgtagtgtcccacgtgcacacgcctctcctaca
ggcagcccagcaaatcatcgaactccaggaagctgcacaaatcaacgcaggcttacagccaaccaacctgggaaggaacaacagc
ctgcacgacatgaagacggtggtgaagacctggaggaaccgactgcccatcgtgtctgacgacttgtcccactggagcagcatcttcatg
tggaggcagcatcattaccaggcgattgtaactgcctatgagaatagctctcagcatgatcccagttcaaataacgctatgcttggggttcat
gcatcagcttcagcgatcatccagtatggaaaaatcgcccggaaacaaggactggtcaatgtagctctggatatattaagtcggattcata
ctattccaactgttcctatcgtggattgcttccagaagattcgacagcaagttaaatgctacctccagctggcaggcgtcatgggcaaaaac
gagtgcatgcagggccttgaagttattgaatctacaaatttaaaatacttcacaaaagagatgacagccgaattttatgcactgaagggaa
tgttcttggctcagatcaacaagtccgaggaggcaaacaaagccttctctgcagctgtgcagatgcacgatgtgctggtgaaagcctggg
ccatgtggggcgactacctggagaacatctttgtgaaggagcggcagctgcacctgggcgtgtctgccatcacctgctacctgcacgcct
gccggcatcagaacgagagcaaatcgaggaaatacttagccaaggtgctgtggcttttgagttttgatgatgacaaaaacactttggcag
atgccgtcgacaagtactgcattggtgtgccacccatccagtggctggcctggatcccacagctgctcacctgcctggttggctcggaggg
aaagctgctcttgaacctcattagccaggttggacgcgtgtatccccaagcggtctactttcccatccggaccctgtacctgaccctgaaaat

agaacagcgggaacgctacaagagcgatccagggcccataagagcaacagcacccatgtggcgctgcagccgaatcatgcacatg
cagcgagagctccaccccacccttctgtcttccctggaaggcatcgtcgatcagatggtctggttcagagaaaattggcatgaagaggttct
caggcagctccaacagggcctggcgaaatgttactccgtggcgtttgagaaaagtggagcggtgtccgatgctaaaatcaccccccaca
ctctcaatttgtgaagaagttggtgagcacgtttggggtgggcctggagaatgtgtccaacgtctcgaccatgttctccagcgcagcctctg
agtctctggcccggcgggcgcaggccactgcacaagaccctgtctttcagaagctgaaaggccagttcacgacggattttgacttcagcg
ttccaggatccatgaagcttcataatcttatttctaagttgaaaaagtggatcaaaatcttggaggccaagaccaagcaactccccaaattct
tcctcatagaggaaaagtgccggttcttgagcaatttctcggcacagacagctgaagtggaaattcctggggagtttctgatgccaaagcc
aacgcattattacatcaagattgcacggttcatgccccgggtagagattgtgcagaagcacaacaccgcagcccggcggctgtacatcc
ggggacacaatggcaagatctacccatacctcgtcatgaacgacgcctgcctcacagagtcacggcgagaggagcgtgtgttgcagct
gctgcgtctgctgaacccctgtttggagaagagaaaggagaccaccaagaggcacttgtttttcacagtgccccgggttgtggcagtttcc
ccacagatgcgcctcgtggaggacaacccctcttcactttcccttgtggagatctacaagcagcgctgcgccaagaagggcatcgagca
tgacaaccccatctcccgttactatgaccggctggctacggtgcaggcgcggggaacccaagccagccaccaggtcctccgcgacatc
ctcaaggaggttcagagtaacatggtgccgcgcagcatgctcaaggagtgggcgctgcacaccttccccaatgccacggactactgga
cgttccggaagatgttcaccatccagctggctctgataggcttcgcggaattcgtcctgcatttaaatagactcaaccccgagatgttacaga
tcgctcaggacactggcaaactgaatgttgcctactttcgatttgacataaacgacgcgactggagacctggatgccaaccgtcctgtccc
atttcgactcacgcccaacatttctgagtttctgaccaccatcggggtctccggcccgttgacagcgtccatgattgcggtcgcccggtgcttc
gcccagccaaactttaaggtggatggcattctgaaaacggttctccgggacgagatcattgcttggcacaaaaaaacacaagaggaca
cgtcctctcctctctcggccgccgggcagccagagaacatggacagccagcaactggtgtccctggttcagaaagccgtcaccgccatc
atgacccgcctgcacaacctcgcccagttcgaaggcggggaaagcaaggtgaacaccctggtggccgcggcaaacagcctggaca
atctgtgccgcatggaccccgcctggcacccctggctgtgactgtggccgccacggcacgcgggaatgtgaagggcgctccgggctctg
agcccgcagcttttacgacttctccctgcgtcgttccttatattcacagaagccccatagtttcactgggttgcggttattttcctggtagtttgcgt
gtaagaaagggagaatatagttttagaggaagctgaactatgacgatgctgggcgaacggtttgggaaatggcagagctgaaacttattc
caagctttcaaaataatcttttaagaagccaggattctccggtctggaatttctgagtgagtccttttttatggtgtcctccctctgtgaatgtaca
ggcggaactgtacgaacagctcccttccatccattttaactctttcggaaataacacctcacagcagcttcgtgcttttgtacagacctttgta
acaagtgtacagaaaactcatttgtttgagaaacaggagttgatgaacccatcatgctggtttttctctgagcacaaagttttaggctgtaca
cagccagccttgggaatctcgttgagcgttcggcgtggatccacggggccaggccaccctgcgggaggccacacgcatccacttcggat
tcagtgggtgaagacagaactctgagagtctgcaggcggctcctgtgcttttttatttctggctcttcggatgtcttctagacatttactatcactgc
acctgaagaaaaaatcactttaccttcctaatttaaaaagacaaaacagaaatgtacgttccttcgctagctttagtctttctgttcccatttttat
aaatctgagcattgataatgttctatctaaatttgtacagtgtgatttttttt (SEQ ID NO:255)

**Q9Y6H4**

MAFVATQGATVVDQTTLMKKYLQFVAALTDVNTPDETKLKMMQEVSENFENVTSSPQYSTFLEHII
PRFLTFLQDGEVQFLQEKPAQQLRKLVLEIIHRIPTNEHLRPHTKNVLSVMFRFLETENEENVLICL
RIIIELHKQFRPPITQEIHHFLDFVKQIYKELPKVVNRYFENPQVIPENTVPPPEMVGMITTIAVKVNP
EREDSETRTHSIIPRGSLSLKVLAELPIIVVLMYQLYKLNIHNVVAEFVPLIMNTIAIQVSAQARQHKL
YNKELYADFIAAQIKTLSFLAYIIRIYQELVTKYSQQMVKGMLQLLSNCPAETAHLRKELLIAAKHILT
TELRNQFIPCMDKLFDESILIGSGYTARETLRPLAYSTLADLVHHVRQHLPLSDLSLAVQLFAKNIDD
ESLPSSIQTMSCKLLLNLVDCIRSKSEQESGNGRDVLMRMLEVFVLKFHTIARYQLSAIFKKCKPQS
ELGAVEAALPGVPTAPAAPGPAPSPAPVPAPPPPPPPPPPATPVTPAPVPPFEKQGEKDKEDKQT
FQVTDCRSLVKTLVCGVKTITWGITSCKAPGEAQFIPNKQLQPKETQIYIKLVKYAMQALDIYQVQIA
GNGQTYIRVANCQTVRMKEEKEVLEHFAGVFTMMNPLTFKEIFQTTVPYMVERISKNYALQIVANS
FLANPTTSALFATILVEYLLDRLPEMGSNVELSNLYLKLFKLVFGSVSLFAAENEQMLKPHLHKIVN
SSMELAQTAKEPYNYFLLLRALFRSIGGGSHDLLYQEFLPLLPNLLQGLNMLQSGLHKQHMKDLF
VELCLTVPVRLSSLLPYLPMLMDPLVSALNGSQTLVSQGLRTLELCVDNLQPDFLYDHIQPVRAEL
MQALWRTLRNPADSISHVAYRVLGKFGGSNRKMLKESQKLHYVVTEVQGPSITVEFSDCKASLQL
PMEKAIETALDCLKSANTEPYYRRQAWEVIKCFLVAMMSLEDNKHALYQLLAHPNFTEKTIPNVIIS
HRYKAQDTPARKTFEQALTGAFMSAVIKDLRPSALPFVASLIRHYTMVAVAQQCGPFLLPCYQVG
SQPSTAMFHSEENGSKGMDPLVLIDAIAICMAYEEKELCKIGEVALAVIFDVASIILGSKERACQLPL
FSYIVERLCACCYEQAWYAKLGGVVSIKFLMERLPLTWVLQNQQTFLKALLFVMMDLTGEVSNGA
VAMAKTTLEQLLMRCATPLKDEERAEEIVAAQEKSFHHVTHDLVREVTSPNSTVRKQAMHSLQVL
AQVTGKSVTVIMEPHKEVLQDMVPPKKHLLRHQPANAQIGLMEGNTFCTTLQPRLFTMDLNVVEH
KVFYTELLNLCEAEDSALTKLPCYKSLPSLVPLRIAALNALAACNYLPQSREKIIAALFKALNSTNSE
LQEAGEACMRKFLEGATIEVDQIHTHMRPLLMMLGDYRSLTLNVVNRLTSVTRLFPNSFNDKFCD
QMMQHLRKWMEVVVITHKGGQRSDGNEMKICSAIINLFHLIPAAPQTLVKPLLEVVMKTERAMLIE
AGSPFREPLIKFLTRHPSQTVELFMMEATLNDPQWSRMFMSFLKHKDARPLRDVLAANPNRFITL
LLPGGAQTAVRPGSPSTSTMRLDLQFQAIKIISIIVKNDDSWLASQHSLVSQLRRVWVSENFQERH
RKENMAATNWKEPKLLAYCLLNYCKRNYGDIELLFQLLRAFTGRFLCNMTFLKEYMEEEIPKNYSI

AQKRALFFRFVDFNDPNFGDELKAKVLQHILNPAFLYSFEKGEGEQLLGPPNPEGDNPESITSVFI
TKVLDPEKQADMLDSLRIYLLQYATLLVEHAPHHIHDNNKNRNSKLRRLMTFAWPCLLSKACVDPA
CKYSGHLLLAHIIAKFAIHKKIVLQVFHSLLKAHAMEARAIVRQAMAILTPAVPARMEDGHQMLTHW
TRKIIVEEGHTVPQLVHILHLIVQHFKVYYPVRHHLVQHMVSAMQRLGFTPSVTIEQRRLAVDLSEV
VIKWELQRIKDQQPDSDMDPNSSGEGVNSVSSSIKRGLSVDSAQEVKRFRTATGAISAVFGRSQS
LPGADSLLAKPIDKQHTDTVVNFLIRVACQVNDNTNTAGSPGEVLSRRCVNLLKTALRPDMWPKS
ELKLQWFDKLLMTVEQPNQVNYGNICTGLEVLSFLLTVLQSPAILSSFKPLQRGIAACMTCGNTKV
LRAVHSLLSRLMSIFPTEPSTSSVASKYEELECLYAAVGKVIYEGLTNYEKATNANPSQLFGTLMIL
KSACSNNPSYIDRLISVFMRSLQKMVREHLNPQAASGSTEATSGTSELVMLSLELVKTRLAVMSM
EMRKNFIQAILTSLIEKSPDAKILRAVVKIVEEWVKNNSPMAANQTPTLREKSILLVKMMTYIEKRFP
EDLELNAQFLDLVNYVYRDETLSGSELTAKLEPAFLSGLRCAQPLIRAKFFEVFDNSMKRRVYERL
LYVTCSQNWEAMGNHFWIKQCIELLLAVCEKSTPIGTSCQGAMLPSITNVINLADSHDRAAFAMVT
HVKQEPRERENSESKEEDVEIDIELAPGDQTSTPKTKELSEKDIGNQLHMLTNRHDKFLDTLREVK
TGALLSAFVQLCHISTTLAEKTWVQLFPRLWKILSDRQQHALAGEISPFLCSGSHQVQRDCQPSAL
NCFVEAMSQCVPPIPIRPCVLKYLGKTHNLWFRSTLMLEHQAFEKGLSLQIKPKQTTEFYEQESIT
PPQQEILDSLAELYSLLQEEDMWAGLWQKRCKYSETATAIAYEQHGFFEQAQESYEKAMDKAKK
EHERSNASPAIFPEYQLWEDHWIRCSKELNQWEALTEYGQSKGHINPYLVLECAWRVSNWTAMK
EALVQVEVSCPKEMAWKVNMYRGYLAICHPEEQQLSFIERLVEMASSLAIREWRRLPHVVSHVHT
PLLQAAQQIIELQEAAQINAGLQPTNLGRNNSLHDMKTVVKTWRNRLPIVSDDLSHWSSIFMWRQ
HHYQAIVTAYENSSQHDPSSNNAMLGVHASASAIIQYGKIARKQGLVNVALDILSRIHTIPTVPIVDC
FQKIRQQVKCYLQLAGVMGKNECMQGLEVIESTNLKYFTKEMTAEFYALKGMFLAQINKSEEANK
AFSAAVQMHDVLVKAWAMWGDYLENIFVKERQLHLGVSAITCYLHACRHQNESKSRKYLAKVLW
LLSFDDDKNTLADAVDKYCIGVPPIQWLAWIPQLLTCLVGSEGKLLLNLISQVGRVYPQAVYFPIRT
LYLTLKIEQRERYKSDPGPIRATAPMWRCSRIMHMQRELHPTLLSSLEGIVDQMVWFRENWHEEV
LRQLQQGLAKCYSVAFEKSGAVSDAKITPHTLNFVKKLVSTFGVGLENVSNVSTMFSSAASESLA
RRAQATAQDPVFQKLKGQFTTDFDFSVPGSMKLHNLISKLKKWIKILEAKTKQLPKFFLIEEKCRFL
SNFSAQTAEVEIPGEFLMPKPTHYYIKIARFMPRVEIVQKHNTAARRLYIRGHNGKIYPYLVMNDAC
LTESRREERVLQLLRLLNPCLEKRKETTKRHLFFTVPRVVAVSPQMRLVEDNPSSLSLVEIYKQRC
AKKGIEHDNPISRYYDRLATVQARGTQASHQVLRDILKEVQSNMVPRSMLKEWALHTFPNATDY
WTFRKMFTIQLALIGFAEFVLHLNRLNPEMLQIAQDTGKLNVAYFRFDINDATGDLDANRPVPFRLT
PNISEFLTTIGVSGPLTASMIAVARCFAQPNFKVDGILKTVLRDEIIAWHKKTQEDTSSPLSAAGQPE
NMDSQQLVSLVQKAVTAIMTRLHNLAQFEGGESKVNTLVAAANSLDNLCRMDPAWHPWL (SEQ ID NO:256)

# Figure 116

**Nucleotide:** AC004472
**Sequence:**
atggcttctggagccgattcaaaaggtgatgacctatcaacagccattctcaaacagaagaaccgtcccaatcggttaattgttgatgaag
ccatcaatgaggacaacagtgtggtgtccttgtcccagcccaagatggatgaattgcagttgttccgaggtgacacagtgttgctgaaagg
aaagaagagacgagaagctgtttgcatcgtcctttctgatgatacttgttctgatgagaagattcggatgaatagagttgttcggaataacctt
cgtgtacgcctaggggatgtcatcagcatccagccatgccctgatgtgaagtacggcaaacgtatccatgtgctgcccattgatgacacag
tggaaggcattactggtaatctcttcgaggtataccttaagccgtacttcctggaagcgtatcgacccatccggaaaggagacatttttcttgt
ccgtggtgggatgcgtgctgtggagttcaaagtggtggaaacagatcctagcccttattgcattgttgctccagacacagtgatccactgcg
aaggggagcctatcaaacgagaggatgaggaagagtccttgaatgaagtagggtatgatgacattggtggctgcaggaagcagctag
ctcagataaaggagatggtggaactgcccctgagacatcctgccctctttaaggcaattggtgtgaagcctcctagaggaatcctgctttac
ggacctcctggaacaggaaagaccctgattgctcgagctgtagcaaatgagactggagccttcttcttcttgatcaatggtcctgagatcat
gagcaaattggctggtgagtctgagagcaaccttcgtaaagcctttgaggaggctgagaagaatgctcctgccatcatcttcattgatgag
ctagatgccatcgctcccaaaagagagaaaactcatggcgaggtggagcggcgcattgtatcacagttgttgaccctcatggatggccta
aagcagagggcacatgtgattgttatggcagcaaccaacagacccaacagcattgacccagctctacggcgatttggtcgctttgacag
ggaggtagatattggaattcctgatgctacaggacgcttagagattcttcagatccataccaagaacatgaagctggcagatgatgtggac
ctggaacaggtagccaatgagactcacgggcatgtgggtgctgacttagcagccctgtgctcagaggctgctctgcaagccatccgcaa
gaagatggatctcattgacctagaggatgagaccattgatgccgaggtcatgaactctctagcagttactatggatgacttccggtgggcct
tgagccagagtaacccatcagcactgcgggaaaccgtggtagaggtgccacaggtaacctgggaagacatcgggggcctagaggat
gtcaaacgtgagctacaggagctggtccagtatcctgtggagcacccagacaaattcctgaagtttggcatgacaccttccaagggagtt
ctgttctatggacctcctggctgtgggaaaactttgttggccaaagccattgctaatgaatgccaggccaacttcatctccatcaagggtcctg
agctgctcaccatgtggtttggggagtctgaggccaatgtcagagaaatctttgacaaggcccgccaagctgccccctgtgtgctattctttg
atgagctggattcgattgccaaggctcgtggaggtaacattggagatggtggtggggctgctgaccgagtcatcaaccagatcctgacag
aaatggatggcatgtccacaaaaaaaaatgtgttcatcattggcgctaccaaccggcctgacatcattgatcctgccatcctcagacctgg
ccgtcttgatcagctcatctacatcccacttcctgatgagaagtcccgtgttgccatcctcaaggctaacctgcgcaagtccccagttgccaa
ggatgtggacttggagttcctggctaaaatgactaatggcttctctggagctgacctgacagagatttgccagcgtgcttgcaagctggccat
ccgtgaatccatcgagagtgagattaggcgagaacgagagaggcagacaaacccatcagccatggaggtagaagaggatgatcca
gtgcctgagatccgtcgagatcactttgaagaagccatgcgctttgcgcgccgttctgtcagtgacaatgacattcggaagtatgagatgttt
gcccagacccttcagcagagtcggggctttggcagcttcagattcccttcagggaaccagggtggagctggccccagtcagggcagtgg
aggcggcacaggtggcagtgtatacacagaagacaatgatgatgacctgtatggctaa (SEQ ID NO:257)

**P55072**
MASGADSKGDDLSTAILKQKNRPNRLIVDEAINEDNSVVSLSQPKMDELQLFRGDTVLLKGKKRR
EAVCIVLSDDTCSDEKIRMNRVVRNNLRVRLGDVISIQPCPDVKYGKRIHVLPIDDTVEGITGNLFE
VYLKPYFLEAYRPIRKGDIFLVRGGMRAVEFKVVETDPSPYCIVAPDTVIHCEGEPIKREDEEESLN
EVGYDDIGGCRKQLAQIKEMVELPLRHPALFKAIGVKPPRGILLYGPPGTGKTLIARAVANETGAFF
FLINGPEIMSKLAGESESNLRKAFEEAEKNAPAIIFIDELDAIAPKREKTHGEVERRIVSQLLTLMDGL
KQRAHVIVMAATNRPNSIDPALRRFGRFDREVDIGIPDATGRLEILQIHTKNMKLADDVDLEQVANE
THGHVGADLAALCSEAALQAIRKKMDLIDLEDETIDAEVMNSLAVTMDDFRWALSQSNPSALRET
VVEVPQVTWEDIGGLEDVKRELQELVQYPVEHPDKFLKFGMTPSKGVLFYGPPGCGKTLLAKAIA
NECQANFISIKGPELLTMWFGESEANVREIFDKARQAAPCVLFFDELDSIAKARGGNIGDGGGAAD
RVINQILTEMDGMSTKKNVFIIGATNRPDIIDPAILRPGRLDQLIYIPLPDEKSRVAILKANLRKSPVAK
DVDLEFLAKMTNGFSGADLTEICQRACKLAIRESIESEIRRERERQTNPSAMEVEEDDPVPEIRRD
HFEEAMRFARRSVSDNDIRKYEMFAQTLQQSRGFGSFRFPSGNQGGAGPSQGSGGGTGGSVY
TEDNDDDLYG (SEQ ID NO:258)

# Figure 117

**Nucleotide:** AB014597
**Sequence:**
gcgaggaagaagaggacgacgacgacgaggaagaggaggtttctgaggtggagtctttcattttggatcaggatgatttggaaaatcca
atgctggaaacagcttccaagttgctcttatcaggtactgctgatggtgcagacctcaggacagtagatccagaaacacaggctagactg
gaagctttactagaagctgcaggaataggaaaattgtccacggctgatggtaaagcctttgcagatcctgaagtacttcggaggttgacat
cgtctgttagttgtgcgttggatgaagctgctgctgcacttacccgtatgagagctgaaagcacagcaaatgcagggcagtcggacaacc
gcagtttggcagaagcctgttcagaaggagatgtaaatgctgtgcgaaagttactcattgaagggcgaagtgtaaatgaacacacagag
gaaggggagagcctcctttgtttagcttgttctgctggatactatgagcttgcacaggttttgttggcaatgcatgcaaatgtggaagataggg
gaatcaaaggtgacattacatctttaatggctgctgctaatggaggacatgtcaaaattgtgaagttgctgctagctcataaagcagatgtta
atgcacagtcttcaacaggcaatacagcacttacatatgcttgtgctggaggctatgtagatgttgtaaaggtgctcttggaatccggtgcta
gtattgaggaccataatgaaaatggtcatacccctcttatggaagctggaagtgctggacatgtggaagtagccagattgctgctagaaaa
tggggctggcattaatacgcattctaatgaatttaaagagagtgcccttaccttagcttgttacaaaggacacttagagatggtgcgatttctttt
ggaagcaggcgcggatcaagagcataaaacagatgaaatgcacactgctctgatggaggcttgcatggatggccatgttgaagtagct
aggttacttcttgacagcggtgcccaagtgaacatgcctgctgattcatttgagtcaccattaactttggctgcatgtggtgggcatgtggaact
tgcggctttacttattgaaagaggagctagcctggaagaggtcaatgatgaaggttatacaccattgatggaagcagctcgagaaggac
atgaagaaatggtggcattacttcttggtcaaggagcaaatatcaatgcacagacagaagaaactcaagaaactgccttgactctggctt
gctgtggaggctttctggaagtggcagactttctaattaaggcaggagccgatatagaactagggtgttctacccctttaatggaagctgctc
aagagggtcatttggagttagttaaatacttattagctgcaggagctaacgttcatgcaacaacagcaacaggggatacagcactaacat
atgcctgtgaaaatggtcatactgatgtagcagatgtcttacttcaggcaggcgcagatctggaacatgaatctgaaggtggaagaactcc
tttaatgaaagctgcaagagctggtcatgtttgtactgttcagttcttaattagtaaaggagcgaatgtgaatagaaccacagctaataatga
ccatactgtactgtccctggcttgtgcagggggtcatctggcagtggtggaactacttttggctcatggggcagatcctactcaccgtttgaaa
gatggctcaactatgttgatagaagcagcaaaaggtggccatacaagtgttgtttgctatctcttggattatcctaataacttgctttcagcccct
ccaccagatgtcactcagttaactcccccatcccacgatttaaatagggctcctcgtgtaccagttcaagcactgcccatggttgttccacctc
aggagcctgacaaaccacctgccaatgttgccaccactcttcccatcaggaataaagctgcttctaaacaaaagtccagcagccatttgc
cagcaaacagccaggatgtacagggttacatcaccaatcagtctccagagagcattgtagaagaggctcagggaaagttaacagaac
tggaacagaggataaaagaagccatagaaaagaatgcacagctgcagtccttggaactggctcatgctgaccaacttaccaaggaga
agatcgaggagctcaacaaaacaagggaggaacaaattcagaagaaacaaaagattttggaggaactacagaaagtagaacgag
agttacaactgaaaactcagcagcagctaaaaaagcagtatctagaggttaaagctcaaagaattcaacttcagcaacagcagcaac
agtcttgccaacacctgggattactaactcctgttggagttggagagcagctttctgagggagactatgcacggttacagcaagtggatcct
gttttacttaaagatgaaccccagcagactgctgctcagatgggttttgcgccaatccagcctctggcgatgcctcaagctttgcctctggcg
gcaggtcccttgcctccagggtccatcgcaaatcttacagaactgcaaggagtgatagttggacagccagtactgggccaagcacagtt
ggcagggctggggcaaggaattctgacagaaacacaacaagggttaatggtagccagccctgctcagaccctcaatgacacgctgga
tgacatcatggcagtcagtggaagagcatctgcaatgtcaaacactcctacccacagtattgctgcatccatttcccaacctcagactcca
actccaagtcctatcatctctccttcagccatgcttcctatctaccctgccattgatattgatgcacagactgagagtaatcatgacacggcgct
aacacttgcctgtgctggtggccacgaggaactggtacaaacactgctagagagaggagctagtatagagcaccgagacaagaaag
gttttactccactcatcttggctgccacagctggtcatgttggtgttgtggaaatattgctggacaatggtgcagacattgaagcccagtctgaa
agaaccaaggacacaccactctccttggcttgttctgggggaagacaggaggtggtggagctattgttagctcgaggggcaaataaaga
gcacaggaatgtttctgattacacacctctaagtctggctgcttctggtggctatgtgaacatcatcaaaatattactaaatgcaggagctga
gattaactctagaactggtagcaaattgggcatctctcctctgatgttagcagctatgaatgggcatacagctgctgttaagctcctgttagac
atgggctctgacataaatgctcagatagaaaccaatcggaacactgcccttactttagcctgcttccaaggaagaactgaagtggttagtct
tctgcttgatagaaaagcaaatgttgaacacagagctaagactggtctcacaccactaatggaagctgcctctggtggatatgcggaggt
gggccgagttcttttggataaaggtgctgatgttaatgcccctccagttccctcctcaagagatacagctttaaccatagcagcagataaag
ggcattacaaattctgtgagcttcttattggcaggggagctcatattgatgtacgtaacaagaaggggaacactccattgtggctagcagca
aatggtggacacctcgatgtggttcagttactggtgcaagcaggtgcagatgtggatgcagcagataaccgcaagataactcctcttatgg
cagcatttagaaagggtcatgtgaaggtggtgcgctacttagtcaaagaagtcaatcagtttccatcagattctgaatgtatgagatacata
gcaaccatcactgataaggagatgctgaagaagtgtcatctttgtatggagtcaatagtacaagccaaagatagacaggctgctgaagc
aaacaaaaacgccagcattttgttagaggagttagacttggaaaagttaagggaagaaagtcggaggctggctttggctgcgaaaaga
gaaaaaagaaaagagaagagaaggaagaaaaaggaagaacaaagaaggaaactagaagaaattgaagccaaaaataaaga
gaactttgaactccaagctgctcaagaaaaagaaaagcttaaagttgaagatgagcctgaagtcttgacagaacctccaagtgccaca
accactactaccataggtatatctgcaacctggacaactttggcaggttctcatggtaaaagaaataataccataactacaaccagttcaa
agaggaaaaacaggaaaaataaaattactccagaaaacgttcaaattatatttgatgatccactaccaatttcatacagtcagccagaga
aggtgaatggagagtccaagagcagcagtaccagcgagagtggggacagtgataacatgaggatttccagctgcagcgatgaaagt
agtaacagcaacagcagtcgtaagagtgacaatcattcaccagctgtggtcactaccactgtgagcagcaaaaagcagccatcagttct
tgttacatttccaaaggaagagagaaaatctgtttctggcaaggcttcaataaaattgtcagaaactatcagtgaagggaccagtaattctct

atctacttgtacaaaatctggtccatctcccctttcttctccaaatgggaagttaacagtagcaagtcctaagcgtgggcaaaagagggaag
aaggatggaaagaagttgtaagaaggtcaaagaaagtatctgttccatcaactgtgatatccagagtgattggaagaggaggctgtaat
atcaatgctattcgggagtttactggtgcacacatagatattgataaacagaaagacaagactggagaccggataatcactataaggggt
ggcactgaatcaacaagacaagcaactcaattgattaatgctttgatcaaggatccagacaaagaaattgatgaacttattccaaagaat
cgtttgaaaagctcctcagcaaattccaaaatagggtcatcagcacctaccaccactgctgctaacacttccttaatgggaattaaaatgac
aactgtagctctgtcatcaacatctcaaactgccacagcactcactgtgcctgcaatttcttctgcatccactcacaaaaccattaagaaccc
agtgaataatgtgaggcctggttttccagtttctcttccattagcatatcctcctccacagtttgcacatgctttgcttgctgctcagactttccagc
agatccgtccaccaaggttgcccatgacccactttggaggtacttttccaccagctcaatccacttggggtccgtttcctgtcaggcctttgag
ccctgccagagctactaactcgcctaagcctcacatggtgcctcgccatagcaatcagaatagcagtggttctcaggtgaattcagcaggt
tctttaacttcaagcccaacaactacaaccagttcatcagcttcaacggtgcctggtacatctacaaatggcagtccaagttcaccttctgtcc
gaaggcagctttttgtcacagttgtgaagacatccaatgccaccacaacaacagtcacaaccacggcaagcaacaacaacactgcac
ccacaaatgccacatatcctatgcctactgccaaagaacactatccagtatcatccccatcttccccatcaccaccagcccagccaggag
gggtttctagaaacagccctttggattgtggatcagcatctccaaataaagtggcatcttcctccgaacaggaagcaggtagtccaccagt
agtagaaacaacaaacactagacctccaaacagcagcagttcttctgggagttcatcagctcattctaatcagcaacaacctccgggatc
tgtttctcaggaaccaagaccacctcttcagcagtctcaggttcctcccccggaagttagaatgactgttcctcctttagcaacaagttctgctc
cagtggcggtgccttctactgccccagtgacttaccctatgcctcagacaccaatgggatgcccccagcctactcctaaaatggaaaccc
ctgctattagaccaccccctcatggcacaactgcccctcacaagaattcagcttcagtgcaaaattcatctgttgcagtcctcagtgtcaatc
acattaaaagacctcacagtgttccctcttctgtccagctaccttcgaccttaagtacacaaagtgcttgtcagaattcagtacatccagcaa
ataagcctattgctcccaatttcagtgccccccttaccatttgggccctttagcacattgtttgaaaacagccctacttctgctcatgccttctgggg
aggatctgttgtttcatctcagtcaacaccagaatctatgctatcaggaaaatcctcatatttgccaaattcagatcctttacatcagtctgatac
ttccaaagctccaggttttagaccaccattacagagacctgctccaagtccctcaggtattgtcaatatggactcgccatatggttctgtaaca
ccttcttcaacacatttgggaaactttgcttcaaacatttcaggaggtcagatgtacggacctggggcaccccttggaggagcacccgcag
ctgctaactttaacagacaacatttttccccgcttagtttgttgactccgtgttcatcagcatcaaatgattcttctgcacagtcagtatcctcggg
agttcgtgcaccatctcctgccccatcatcagtaccgttagggtcagaaaagcccagcaatgtgtctcaggacaggaaagttccagtccct
attgggactgaacgttctgcacgtatcaggcaaactggaacgtcagctccatctgttattgggagcaatttgtctacatcagtaggacatagt
ggcatctggtcctttgaagggattggtggcaatcaagacaaagtagactggtgtaaccctgggatgggaaatcctatgatccacagaccg
atgtctgacccaggagtattttcacaacatcaagcaatggagcgagatagtacaggaattgtaactccttctggtacattccatcagcatgtt
cctgcaggctacatggactttcctaaagttggggggtatgcctttttctgtgtatgggaatgcaatgattcctccagtagcacctatccctgatggt
gctggaggacccatatttaatggccctcatgctgcagacccttcttggaactcactgataaagatggtttccagctccacggaaaataatgg
ccctcaaacggtgtggactggaccctgggcacctcacatgaacagtgtgcatatgaaccagcttggctga (SEQ ID NO:259)

**O75179; BAA31672**

EEEEDDDDEEEEVSEVESFILDQDDLENPMLETASKLLLSGTADGADLRTVDPETQARLEALLEAA
GIGKLSTADGKAFADPEVLRRLTSSVSCALDEAAAALTRMRAESTANAGQSDNRSLAEACSEGDV
NAVRKLLIEGRSVNEHTEEGESLLCLACSAGYYELAQVLLAMHANVEDRGIKGDITSLMAAANGG
HVKIVKLLLAHKADVNAQSSTGNTALTYACAGGYVDVVKVLLESGASIEDHNENGHTPLMEAGSA
GHVEVARLLLENGAGINTHSNEFKESALTLACYKGHLEMVRFLLEAGADQEHKTDEMHTALMEAC
MDGHVEVARLLLDSGAQVNMPADSFESPLTLAACGGHVELAALLIERGASLEEVNDEGYTPLMEA
AREGHEEMVALLLGQGANINAQTEETQETALTLACCGGFLEVADFLIKAGADIELGCSTPLMEAAQ
EGHLELVKYLLAAGANVHATTATGDTALTYACENGHTDVADVLLQAGADLEHESEGGRTPLMKAA
RAGHVCTVQFLISKGANVNRTTANNDHTVLSLACAGGHLAVVELLLAHGADPTHRLKDGSTMLIE
AAKGGHTSVVCYLLDYPNNLLSAPPPDVTQLTPPSHDLNRAPRVPVQALPMVVPPQEPDKPPAN
VATTLPIRNKAASKQKSSSHLPANSQDVQGYITNQSPESIVEEAQGKLTELEQRIKEAIEKNAQLQS
LELAHADQLTKEKIEELNKTREEQIQKKQKILEELQKVERELQLKTQQQLKKQYLEVKAQRIQLQQ
QQQQSCQHLGLLTPVGVGEQLSEGDYARLQQVDPVLLKDEPQQTAAQMGFAPIQPLAMPQALPL
AAGPLPPGSIANLTELQGVIVGQPVLGQAQLAGLGQGILTETQQGLMVASPAQTLNDTLDDIMAVS
GRASAMSNTPTHSIAASISQPQTPTPSPIISPSAMLPIYPAIDIDAQTESNHDTALTLACAGGHEELV
QTLLERGASIEHRDKKGFTPLILAATAGHVGVVEILLDNGADIEAQSERTKDTPLSLACSGGRQEVV
ELLLARGANKEHRNVSDYTPLSLAASGGYVNIIKILLNAGAEINSRTGSKLGISPLMLAAMNGHTAA
VKLLLDMGSDINAQIETNRNTALTLACFQGRTEVVSLLLDRKANVEHRAKTGLTPLMEAASGGYAE
VGRVLLDKGADVNAPPVPSSRDTALTIAADKGHYKFCELLIGRGAHIDVRNKKGNTPLWLAANGG
HLDVVQLLVQAGADVDAADNRKITPLMAAFRKGHVKVVRYLVKEVNQFPSDSECMRYIATITDKE
MLKKCHLCMESIVQAKDRQAAEANKNASILLEELDLEKLREESRRLALAAKREKRKEKRRKKKEE
QRRKLEEIEAKNKENFELQAAQEKEKLKVEDEPEVLTEPPSATTTTTIGISATWTTLAGSHGKRNN
TITTTSSKRKNRKNKITPENVQIIFDDPLPISYSQPEKVNGESKSSSTSESGDSDNMRISSCSDESS
NSNSSRKSDNHSPAVVTTTVSSKKQPSVLVTFPKEERKSVSGKASIKLSETISEGTSNSLSTCTKS
GPSPLSSPNGKLTVASPKRGQKREEGWKEVVRRSKKVSVPSTVISRVIGRGGCNINAIREFTGAHI

DIDKQKDKTGDRIITIRGGTESTRQATQLINALIKDPDKEIDELIPKNRLKSSSANSKIGSSAPTTTAA
NTSLMGIKMTTVALSSTSQTATALTVPAISSASTHKTIKNPVNNVRPGFPVSLPLAYPPPQFAHALL
AAQTFQQIRPPRLPMTHFGGTFPPAQSTWGPFPVRPLSPARATNSPKPHMVPRHSNQNSSGSQ
VNSAGSLTSSPTTTTSSSASTVPGTSTNGSPSSPSVRRQLFVTVVKTSNATTTTVTTTASNNNTAP
TNATYPMPTAKEHYPVSSPSSPSPPAQPGGVSRNSPLDCGSASPNKVASSSEQEAGSPPVVETT
NTRPPNSSSSSGSSSAHSNQQQPPGSVSQEPRPPLQQSQVPPPEVRMTVPPLATSSAPVAVPS
TAPVTYPMPQTPMGCPQPTPKMETPAIRPPPHGTTAPHKNSASVQNSSVAVLSVNHIKRPHSVPS
SVQLPSTLSTQSACQNSVHPANKPIAPNFSAPLPFGPFSTLFENSPTSAHAFWGGSVVSSQSTPE
SMLSGKSSYLPNSDPLHQSDTSKAPGFRPPLQRPAPSPSGIVNMDSPYGSVTPSSTHLGNFASNI
SGGQMYGPGAPLGGAPAAANFNRQHFSPLSLLTPCSSASNDSSAQSVSSGVRAPSPAPSSVPL
GSEKPSNVSQDRKVPVPIGTERSARIRQTGTSAPSVIGSNLSTSVGHSGIWSFEGIGGNQDKVDW
CNPGMGNPMIHRPMSDPGVFSQHQAMERDSTGIVTPSGTFHQHVPAGYMDFPKVGGMPFSVY
GNAMIPPVAPIPDGAGGPIFNGPHAADPSWNSLIKMVSSSTENNGPQTVWTGPWAPHMNSVHM
NQLG (SEQ ID NO:260)

# Figure 118

**Nucleotide:** D84212
**Sequence:**
atggaccgatctaaagaaaactgcatttcaggacctgttaaggctacagctccagttggaggtccaaaacgtgttctcgtgactcagcaatt
tccttgtcagaatccattacctgtaaatagtggccaggctcagcgggtcttgtgtccttcaaattcttcccagcgcgttcctttgcaagcacaaa
agcttgtctccagtcacaagccggttcagaatcagaagcagaagcaattgcaggcaaccagtgtacctcatcctgtctccaggccactga
ataacacccaaaagagcaagcagcccctgccatcgcacctgaaaataatcctgaggaggaactggcatcaaaacagaaaaatgaa
gaatcaaaaagaggcagtggctttggaagactttgaaattggtcgccctctgggtaaaggaaagtttggtaatgtttatttggcaagagaaa
agcaaagcaagtttattctggctcttaaagtgttatttaaagctcagctggagaaagccggagtggagcatcagctcagaagagaagtag
aaatacagtcccaccttcggcatcctaatattcttagactgtatggttatttccatgatgctaccagagtctacctaattctggaatatgcaccac
ttggaacagtttatagagaacttcagaaactttcaaagtttgatgagcagagaactgctaacttatataacagaattgcaaatgccctgtctta
ctgtcattcgaagagagttattcatagagacattaagccagagaacttacttcttggatcagctggagagcttaaaattgcagattttgggtg
gtcagtacatgctccatcttccaggaggaccactctctgtggcaccctggactacctgcccccctgaaatgattgaaggtcggatgcatgatg
agaaggtggatctctggagccttggagttctttgctatgaatttttagttgggaagcctccttttgaggcaaacacataccaagagacctaca
aaagaatatcacgggttgaattcacattccctgactttgtaacagagggagccagggacctcatttcaagactgttgaagcataatcccag
ccagaggccaatgctcagagaagtacttgaacacccctggatcacagcaaattcatcaaaaccatcaaattgccaaaacaaagaatc
agctagcaaacagtcttag (SEQ ID NO:261)

**O60445**
MDRSKENCISGPVKATAPVGGPKRVLVTQQFPCQNPLPVNSGQAQRVLCPSNSSQRIPLQAQKL
VSSHKPVQNQKQKQLQATSVPHPVSRPLNNTQKSKQPLPSAPENNPEEELASKQKNEESKKRQ
WALEDFEIGRPLGKGKFGNVYLAREKQSKFILALKVLFKAQLEKAGVEHQLRREVEIQSHLRHPNIL
RLYGYFHDATRVYLILEYAPLGTVYRELQKLSKFDEQRTATYITELANALSYCHSKRVIHRDIKPENL
LLGSAGELKIADFGWSVHAPSSRRTTLCGTLDYLPPEMIEGRMHDEKVDLWSLGVLCYEFLVGKP
PFEANTYQETYKRISRVEFTFPDFVTEGARDLISRLLKHNPSQRPMLREVLEHPWITANSSKPSNC
QNKESASKQS (SEQ ID NO:262)

**Nucleotide:** AF008552
**Sequence:**
atggcccagaaggagaactcctacccctggccctacggccgacagacggctccatctggcctgagcaccctgccccagcgagtcctcc
ggaaagagcctgtcaccccatctgcacttgtcctcatgagccgctccaatgtccagcccacagctgcccctggccagaaggtgatggag
aatagcagtgggacacccgacatcttaacgcggcacttcacaattgatgactttgagattgggcgtcctctgggcaaaggcaagtttggaa
acgtgtacttggctcgggagaagaaaagccatttcatcgtggcgctcaaggtcctcttcaagtcccagatagagaaggagggcgtggag
catcagctgcgcagagagatcgaaatccaggcccacctgcaccatcccaacatcctgcgtctctacaactatttttatgaccggaggagg
atctacttgattctagagtatgccccccgcggggagctctacaaggagctgcagaagagctgcacatttgacgagcagcgaacagccac
gatcatggaggagttggcagatgctctaatgtactgccatgggaagaaggtgattcacagagacataaagccagaaaatctgctcttagg
gctcaagggagagctgaagattgctgacttcggctggtctgtgcatgcgccctccctgaggaggaagacaatgtgtggcaccctggacta
cctgcccccagagatgattgaggggcgcatgcacaatgagaaggtggatctgtggtgcattggagtgctttgctatgagctgctggtgggg
aacccaccctttgagagtgcatcacacaacgagacctatcgccgcatcgtcaaggtggacctaaagttccccgcttctgtgcccacggga
gcccaggacctcatctccaaactgctcaggcataacccctcggaacggctgcccctggcccaggtctcagcccacccttgggtccgggc
caactctcggagggtgctgcctccctctgcccttcaatctgtcgcctga (SEQ ID NO:263)

**O60446**
MAQKENSYPWPYGRQTAPSGLSTLPQRVLRKEPVTPSALVLMSRSNVQPTAAPGQKVMENSSG
TPDILTRHFTIDDFEIGRPLGKGKFGNVYLAREKKSHFIVALKVLFKSQIEKEGVEHQLRREIEIQAHL
HHPNILRLYNYFYDRRRIYLILEYAPRGELYKELQKSCTFDEQRTATIMEELADALMYCHGKKVIHR
DIKPENLLLGLKGELKIADFGWSVHAPSLRRKTMCGTLDYLPPEMIEGRMHNEKVDLWCIGVLCYE
LLVGNPPFESASHNETYRRIVKVDLKFPASVPTGAQDLISKLLRHNPSERLPLAQVSAHPWVRANS
RRVLPPSALQSVA (SEQ ID NO:264)

# Figure 119

**Nucleotide:** AK027719
**Sequence:**
atggatcccaccgcgggaagcaagaaggagcctggaggaggcgcggcgactgaggagggcgtgaataggatcgcagtgccaaag
ccgccctccattgaggaattcagcatagtgaagcccattagccggggcgccttcgggaaagtgtatctggggcagaaaggcggcaaatt
gtatgcagtaaaggttgttaaaaaagcagacatgatcaacaaaaatatgactcatcaggtccaagctgagagagatgcactggcactaa
gcaaaagcccattcattgtccatttgtattattcactgcagtctgcaaacaatgtctacttggtaatggaatatcttattggggggagatgtcaagt
ctctcctacatatatatggttattttgatgaagagatggctgtgaaatatatttctgaagtagcactggctctagactaccttcacagacatgga
atcatccacagggacttgaaaccggacaatatgcttatttctaatgagggtcatattaaactgacggattttggcctttcaaaagttactttgaa
tagagatattaatatgatggatatccttacaacaccatcaatggcaaaacctagacaagattattcaagaaccccaggacaagtgttatcg
cttatcagctcgttgggatttaacacaccaattgcagaaaaaaatcaagaccctgcaaacatcctttcagcctgtctgtctgaaacatcaca
gctttctcaaggactcgtatgccctatgtctgtagatcaaaaggacactacgccttattctagcaaattactaaaatcatgtcttgaaacagttg
cctccaacccaggaatgcctgtgaagtgtctaacttctaatttactccagtctaggaaaaggctggccacatccagtgccagtagtcaatcc
cacaccttcatatccagtgtggaatcagaatgccacagcagtcccaaatgggaaaaagattgccaggaaagtgatgaagcattgggcc
caacaatgatgagttggaatgcagttgaaaagttatgcgcaaaatctgcaaatgccattgagacgaaaggtttcaataaaaaggatctgg
agttagctctttctcccattcataacagcagtgcccttcccaccactggacgctcttgtgtaaaccttgctaaaaaatgcttctctggggaagttt
cttgggaagcagtagaactggatgtaaataatataaatatggacactgacacaagtcagttaggtttccatcagtcaaatcagtgggctgt
ggattctggtgggatatctgaagagcaccttgggaaaagaagtttaaaaagaaatttgagttggttgactccagtccttgtaaaaaaattat
acagaataaaaaaacttgtgtagagtataagcataacgaaatgacaaattgttatacaaatcaaaatacaggcttaacagttgaagtgca
ggaccttaagctatcagtgcacaaaagtcaacaaaatgactgtgctaataaggagaacattgtcaattcttttactgataaacaacaaaca
ccagaaaaattacctataccaatgatagcaaaaaaccttatgtgtgaactcgatgaagactgtgaaaagaatagtaagagggactactt
aagttctagtttctatgttctgatgatgatagagcttctaaaaatatttctatgaactctgattcatcttttcctggaatttctataatggaaagtccat
tagaaagtcagcccttagattcagatagaagcattaaagaatcctcttttgaagaatcaaatattgaagatccacttattgtaacaccagatt
gccaagaaaagacctcaccaaaaggtgtcgagaaccctgctgtacaagagagtaaccaaaaaatgttaggtcctcctttggaggtgctg
aaaacgttagcctctaaaagaaatgctgttgcttttcgaagttttaacagtcatattaatgcatccaataactcagaaccatccagaatgaac
atgacttctttagatgcaatggatatttcgcgtgcctacagtggttcatatcccatggctataacccctactcaaaaaagaagatcctgtatgc
cacatcagaccccaaatcagatcaagtcgggaactccataccgaactccgaagagtgtgagaagaggggtggcccccgttgatgatg
ggcgaattctaggaaccccagactaccttgcacctgagctgttactaggcagggcccatggtcctgcggtagactggtgggcacttggag
tttgcttgtttgaatttctaacaggaattcccccctttcaatgatgaaacaccacaacaagtattccagaatattctgaaaagagatatcccttgg
ccagaaggtgaagaaaagttatctgataatgctcaaagtgcagtagaaatacttttaaccattgatgatacaaagagagctggaatgaaa
gagctaaaacgtcatcctctcttcagtgatgtggactgggaaaatctgcagcatcagactatgcctttcatcccccagccagatgatgaaac
agatacctcctattttgaaaccaggaatactgctcagcacctgaccgtatctggatttagtctgtag (SEQ ID NO:265)

**Q96SJ5**
MDPTAGSKKEPGGGAATEEGVNRIAVPKPPSIEEFSIVKPISRGAFGKVYLGQKGGKLYAVKVVKK
ADMINKNMTHQVQAERDALALSKSPFIVHLYYSLQSANNVYLVMEYLIGGDVKSLLHIYGYFDEEM
AVKYISEVALALDYLHRHGIIHRDLKPDNMLISNEGHIKLTDFGLSKVTLNRDINMMDILTTPSMAKP
RQDYSRTPGQVLSLISSLGFNTPIAEKNQDPANILSACLSETSQLSQGLVCPMSVDQKDTTPYSSK
LLKSCLETVASNPGMPVKCLTSNLLQSRKRLATSSASSQSHTFISSVESECHSSPKWEKDCQESD
EALGPTMMSWNAVEKLCAKSANAIETKGFNKKDLELALSPIHNSSALPTTGRSCVNLAKKCFSGE
VSWEAVELDVNNINMDTDTSQLGFHQSNQWAVDSGGISEEHLGKRSLKRNFELVDSSPCKKIIQN
KKTCVEYKHNEMTNCYTNQNTGLTVEVQDLKLSVHKSQQNDCANKENIVNSFTDKQQTPEKLPIP
MIAKNLMCELDEDCEKNSKRDYLSSSFLCSDDDRASKNISMNSDSSFPGISIMESPLESQPLDSDR
SIKESSFEESNIEDPLIVTPDCQEKTSPKGVENPAVQESNQKMLGPPLEVLKTLASKRNAVAFRSF
NSHINASNNSEPSRMNMTSLDAMDISRAYSGSYPMAITPTQKRRSCMPHQTPNQIKSGTPYRTPK
SVRRGVAPVDDGRILGTPDYLAPELLLGRAHGPAVDWWALGVCLFEFLTGIPPFNDETPQQVFQN
ILKRDIPWPEGEEKLSDNAQSAVEILLTIDDTKRAGMKELKRHPLFSDVDWENLQHQTMPFIPQPD
DETDTSYFETRNTAQHLTVSGFSL (SEQ ID NO:266)

# Figure 120

**Nucleotide:** J04088
**Sequence:**
atggaagtgtcaccattgcagcctgtaaatgaaaatatgcaagtcaacaaaataaagaaaaatgaagatgctaagaaaagactgtctgt
tgaaagaatctatcaaaagaaaacacaattggaacatattttgctccgcccagacacctacattggttctgtggaattagtgacccagcaa
atgtgggtttacgatgaagatgttggcattaactatagggaagtcacttttgttcctggtttgtacaaaatctttgatgagattctagttaatgctgc
ggacaacaaacaaagggacccaaaaatgtcttgtattagagtcacaattgatccggaaaacaatttaattagtatatggaataatggaaa
aggtattcctgttgttgaacacaaagttgaaaagatgtatgtcccagctctcatatttggacagctcctaacttctagtaactatgatgatgatg
aaaagaaagtgacaggtggtcgaaatggctatggagccaaattgtgtaacatattcagtaccaaatttactgtggaaacagccagtaga
gaatacaagaaaatgttcaaacagacatggatggataatatgggaagagctggtgagatggaactcaagcccttcaatggagaagatt
atacatgtatcacctttcagcctgatttgtctaagtttaaaatgcaaagcctggacaaagatattgttgcactaatggtcagaagagcatatga
tattgctggatccaccaaagatgtcaaagtctttcttaatggaaataaactgccagtaaaaggatttcgtagttatgtggacatgtatttgaag
gacaagttggatgaaactggtaactccttgaaagtaatacatgaacaagtaaaccacaggtgggaagtgtgtttaactatgagtgaaaaa
ggctttcagcaaattagctttgtcaacagcattgctacatccaagggtggcagacatgttgattatgtagctgatcagattgtgactaaacttgt
tgatgttgtgaagaagaagaacaagggtggtgttgcagtaaaagcacatcaggtgaaaaatcacatgtggatttttgtaaatgccttaattg
aaaacccaacctttgactctcagacaaaagaaaacatgactttacaacccaagagctttggatcaacatgccaattgagtgaaaaatttat
caaagctgccattggctgtggtattgtagaaagcatactaaactgggtgaagtttaaggcccaagtccagttaaacaagaagtgttcagct
gtaaaacataatagaatcaagggaattcccaaactcgatgatgccaatgatgcaggggggccgaaactccactgagtgtacgcttatcctg
actgagggagattcagccaaaactttggctgtttcaggccttggtgtggttgggagagacaaatatggggtttccctcttagaggaaaaat
actcaatgttcgagaagcttctcataagcagatcatggaaaatgctgagattaacaatatcatcaagattgtgggtcttcagtacaagaaaa
actatgaagatgaagattcattgaagacgcttcgttatgggaagataatgattatgacagatcaggaccaagatggttcccacatcaaag
gcttgctgattaattttatccatcacaactggccctctcttctgcgacatcgttttctggaggaatttatcactcccattgtaaaggtatctaaaaa
caagcaagaaatggcattttacagccttcctgaatttgaagagtggaagagttctactccaaatcataaaaaatggaaagtcaaatattac
aaaggtttgggcaccagcacatcaaaggaagctaaagaatactttgcagatatgaaaagacatcgtatccagttcaaatattctggtcctg
aagatgatgctgctatcagcctggcctttagcaaaaaacagatagatgatcgaaaggaatggttaactaatttcatggaggatagaagac
aacgaaagttacttgggcttcctgaggattacttgtatggacaaactaccacatatctgacatataatgacttcatcaacaaggaacttatctt
gttctcaaattctgataacgagagatctatcccttctatggtggatggtttgaaaccaggtcagagaaaggttttgtttacttgcttcaaacgga
atgacaagcgagaagtaaaggttgcccaattagctggatcagtggctgaaatgtcttcttatcatcatggtgagatgtcactaatgatgacc
attatcaatttggctcagaattttgtgggtagcaataatctaaacctcttgcagcccattggtcagtttggtaccaggctacatggtggcaagg
attctgctagtccacgatacatctttacaatgctcagctctttggctcgattgttatttccaccaaaagatgatcacacgttgaagtttttatatgat
gacaaccagcgtgttgagcctgaatggtacattcctattattcccatggtgctgataaatggtgctgaaggaatcggtactgggtggtcctgc
aaaatccccaactttgatgtgcgtgaaattgtaaataacatcaggcgtttgatggatggagaagaacctttgccaatgcttccaagttacaa
gaacttcaagggtactattgaagaactggctccaaatcaatatgtgattagtggtgaagtagctattcttaattctacaaccattgaaatctca
gagcttcccgtcagaacatggacccagacatacaaagaacaagttctagaacccatgttgaatggcaccgagaagacacctcctctcat
aacagactatagggaataccatacagataccactgtgaaatttgttgtgaagatgactgaagaaaaactggcagaggcagagagagtt
ggactacacaaagtcttcaaactccaaactagtctcacatgcaactctatggtgctttttgaccacgtaggctgtttaaagaaatatgacacg
gtgttggatattctaagagactttttttgaactcagacttaaatattatggattaagaaaagaatggctcctaggaatgcttggtgctgaatctgct
aaactgaataatcaggctcgctttatcttagagaaaatagatggcaaaataatcattgaaaataagcctaagaaagaattaattaaagttct
gattcagaggggatatgattcggatcctgtgaaggcctggaaagaagcccagcaaaaggttccagatgaagaagaaaatgaagaga
gtgacaacgaaaaggaaactgaaaagagtgactccgtaacagattctggaccaaccttcaactatcttcttgatatgccccttggtatttaa
ccaaggaaaagaaagatgaactctgcaggctaagaaatgaaaaagaacaagagctggacacattaaaaagaaagagtccatcag
atttgtggaaagaagacttggctacatttattgaagaattggaggctgttgaagccaaggaaaaacaagatgaacaagtcggacttcctg
ggaaagggggggaaggccaaggggaaaaaaacacaaatggctgaagttttgccttctccgcgtggtcaaagagtcattccacgaataa
ccatagaaatgaaagcagaggcagaaaagaaaaataaaaagaaaattaagaatgaaaatactgaaggaagccctcaagaagatg
gtgtggaactagaaggcctaaaacaaagattagaaaagaaacagaaaagagaaccaggtacaaagacaaagaaacaaactacat
tggcatttaagccaatcaaaaaaggaaagaagagaaatccctggcctgattcagaatcagataggagcagtgacgaaagtaattttgat
gtccctccacgagaaacagagccacggagagcagcaacaaaaacaaaattcacaatggatttggattcagatgaagatttctcagattt
tgatgaaaaaactgatgatgaagattttgtcccatcagatgctagtccacctaagaccaaaacttccccaaaacttagtaacaaagaactg
aaaccacagaaaagtgtcgtgtcagaccttgaagctgatgatgttaagggcagtgtaccactgtcttcaagccctcctgctacacatttccc
agatgaaactgaaattacaaacccagttcctaaaaagaatgtgacagtgaagaagacagcagcaaaaagtcagtcttccacctccact
accggtgccaaaaaaagggctgccccaaaaggaactaaaagggatccagctttgaattctggtgtctctcaaaagcctgatcctgccaa
aaccaagaatcgccgcaaaaggaagccatccacttctgatgattctgactctaattttgagaaaattgtttcgaaagcagtcacaagcaag
aaatccaagggggagagtgatgacttccatatggactttgactcagctgtggctcctcgggcaaaatctgtacgggcaaagaaacctata
aagtacctggaagagtcagatgaagatgatctgttttaa (SEQ ID NO:267)

**P11388**
MEVSPLQPVNENMQVNKIKKNEDAKKRLSVERIYQKKTQLEHILLRPDTYIGSVELVTQQMWVYD
EDVGINYREVTFVPGLYKIFDEILVNAADNKQRDPKMSCIRVTIDPENNLISIWNNGKGIPVVEHKVE
KMYVPALIFGQLLTSSNYDDDEKKVTGGRNGYGAKLCNIFSTKFTVETASREYKKMFKQTWMDN
MGRAGEMELKPFNGEDYTCITFQPDLSKFKMQSLDKDIVALMVRRAYDIAGSTKDVKVFLNGNKL
PVKGFRSYVDMYLKDKLDETGNSLKVIHEQVNHRWEVCLTMSEKGFQQISFVNSIATSKGGRHVD
YVADQIVTKLVDVVKKKNKGGVAVKAHQVKNHMWIFVNALIENPTFDSQTKENMTLQPKSFGSTC
QLSEKFIKAAIGCGIVESILNWVKFKAQVQLNKKCSAVKHNRIKGIPKLDDANDAGGRNSTECTLILT
EGDSAKTLAVSGLGVVGRDKYGVFPLRGKILNVREASHKQIMENAEINNIIKIVGLQYKKNYEDEDS
LKTLRYGKIMIMTDQDQDGSHIKGLLINFIHHNWPSLLRHRFLEEFITPIVKVSKNKQEMAFYSLPEF
EEWKSSTPNHKKWKVKYYKGLGTSTSKEAKEYFADMKRHRIQFKYSGPEDDAAISLAFSKKQIDD
RKEWLTNFMEDRRQRKLLGLPEDYLYGQTTTYLTYNDFINKELILFSNSDNERSIPSMVDGLKPGQ
RKVLFTCFKRNDKREVKVAQLAGSVAEMSSYHHGEMSLMMTIINLAQNFVGSNNLNLLQPIGQFG
TRLHGGKDSASPRYIFTMLSSLARLLFPPKDDHTLKFLYDDNQRVEPEWYIPIIPMVLINGAEGIGT
GWSCKIPNFDVREIVNNIRRLMDGEEPLPMLPSYKNFKGTIEELAPNQYVISGEVAILNSTTIEISEL
PVRTWTQTYKEQVLEPMLNGTEKTPPLITDYREYHTDTTVKFVVKMTEEKLAEAERVGLHKVFKL
QTSLTCNSMVLFDHVGCLKKYDTVLDILRDFFELRLKYYGLRKEWLLGMLGAESAKLNNQARFILE
KIDGKIIIENKPKKELIKVLIQRGYDSDPVKAWKEAQQKVPDEEENEESDNEKETEKSDSVTDSGPT
FNYLLDMPLWYLTKEKKDELCRLRNEKEQELDTLKRKSPSDLWKEDLATFIEELEAVEAKEKQDE
QVGLPGKGGKAKGKKTQMAEVLPSPRGQRVIPRITIEMKAEAEKKNKKKIKNENTEGSPQEDGVE
LEGLKQRLEKKQKREPGTKTKKQTTLAFKPIKKGKKRNPWSDSESDRSSDESNFDVPPRETEPR
RAATKTKFTMDLDSDEDFSDFDEKTDDEDFVPSDASPPKTKTSPKLSNKELKPQKSVVSDLEADD
VKGSVPLSSSPPATHFPDETEITNPVPKKNVTVKKTAAKSQSSTSTTGAKKRAAPKGTKRDPALN
SGVSQKPDPAKTKNRRKRKPSTSDDSDSNFEKIVSKAVTSKKSKGESDDFHMDFDSAVAPRAKS
VRAKKPIKYLEESDEDDLF (SEQ ID NO:268)

**Nucleotide:** X68060
**Sequence:**
atggccaagtcgggtggctgcggcgcgggagccggcgtgggcggcggcaacggggcactgacctgggtgaacaatgctgcaaaaa
aagaagagtcagaaactgccaacaaaaatgattcttcaaagaagttgtctgttgagagagtgtatcagaagaagacacaacttgaaca
cattcttcttcgtcctgatacatatattgggtcagtggagccattgacgcagttcatgtgggtgtatgatgaagatgtaggaatgaattgcagg
gaggttacctttgtgccaggtttatacaagatctttgatgaaattttggttaatgctgctgacaataaacagagggataagaacatgacttgtat
taaagtttctattgatcctgaatctaacattataagcatttggaataatgggaaaggcattccagtagtagaacacaaggtagagaaagttta
tgttcctgctttaatttttggacagcttttaacatccagtaactatgatgatgatgagaaaaaagttacaggtggtcgtaatggttatggtgcaaa
actttgtaatattttcagtacaaagtttacagtagaaacagcttgcaaagaatacaaacacagttttaagcagacatggatgaataatatgat
gaagacttctgaagccaaaattaaacattttgatggtgaagattacacatgcataacattccaaccagatctgtccaaatttaagatggaaa
aacttgacaaggatattgtggccctcatgactagaagggcatatgatttggctggttcgtgtagaggggtcaaggtcatgtttaatggaaag
aaattgcctgtaaatggatttcgcagttatgtagatctttatgtgaaagacaaattggatgaaactggggtggccctgaaagttattcatgagc
ttgcaaatgaaagatgggatgtttgtctcacattgagtgaaaaaggattccagcaaatcagctttgtaaatagtattgcaactacaaaaggt
ggacggcacgtggattatgtggtagatcaagttgttggtaaactgattgaagtagttaagaaaaagaacaaagctggtgtatcagtgaaa
ccatttcaagtaaaaaaccatatatgggtttttattaattgccttattgaaaatccaacttttgattctcagactaaggaaaacatgactctgcag
cccaaaagttttgggtctaaatgccagctgtcagaaaaatttttaaagcagcctctaattgtggcattgtagaaagtatcctgaactgggtga
aatttaaggctcagactcagctgaataagaagtgttcatcagtaaaatacagtaaaatcaaaggtattcccaaactggatgatgctaatga
tgctggtggtaaacattccctggagtgtacactgatattaacagagggagactctgccaaatcactggctgtgtctggattaggtgtgattgg
acgagacagatacggagtttttccactcaggggcaaaattcttaatgtacgggaagcttctcataaacagatcatggaaaatgctgaaata
aataatattattaaaatagttggtctacaatataagaaaagttacgatgatgcagaatctctgaaaaccttacgctatggaaagattatgatta
tgaccgatcaggatcaagatggttctcacataaaaggcctgcttattaatttcatccatcacaattggccatcacttttgaagcatggttttcttg
aagagttcattactcctattgtaaaggcaagcaaaaataagcaggaactttccttctacagtattcctgaatttgacgaatggaaaaaacat
atagaaaaccagaaagcctggaaaataaagtactataaaggattgggtactagtacagctaaagaagcaaaggaatattttgctgatat
ggaaaggcatcgcatcttgtttagatatgctggtcctgaagatgatgctgccattaccttggcatttagtaagaagaagattgatgacagaa
aagaatggttaacaaattttatggaagaccggagacagcgtaggctacatggcttaccagagcaatttttatatggtactgcaacaaagca
tttgacttataatgatttcatcaacaaggaattgattctcttctcaaactcagacaatgaaagatctataccatctcttgttgatggctttaaacct
ggccagcggaaagttttatttacctgtttcaagaggaatgataaacgtgaagtaaaagttgcccagttggctggctctgttgctgagatgtcg
gcttatcatcatggagaacaagcattgatgatgactattgtgaatttggctcagaactttgtgggaagtaacaacattaacttgcttcagcctat
tggtcagtttggaactcggcttcatggtggcaaagatgctgcaagccctcgttatattttcacaatgttaagcactttagcaaggctactttttcct
gctgtggatgacaacctccttaagttcctttatgatgataatcaacgtgtagagcctgagtggtatattcctataattcccatggttttaataaatg
gtgctgagggcattggtactggatgggcttgtaaactacccaactatgatgctagggaaattgtgaacaatgtcagacgaatgctagatgg

cctggatcctcatcccatgcttccaaactacaaaaactttaaaggcacgattcaagaacttggtcaaaaccagtatgcagtcagtggtgaa
atatttgtagtggacagaaacacagtagaaattacagagcttccagttagaacttggacacaggtatataaagaacaggttttagaaccta
tgctaaatggaacagataaaacaccagcattaatttctgattataaagaatatcatactgacacaactgtgaaatttgtggtgaaaatgact
gaagagaaactagcacaagcagaagctgctggactgcataaagtttttaaacttcaaactactcttacttgtaattccatggtactttttgatc
atatgggatgtctgaagaaatatgaaactgtgcaagacattctgaaagaattctttgatttacgattaagttattacggtttacgtaaggagtg
gcttgtgggaatgttgggagcagaatctacaaagcttaacaatcaagcccgtttcattttagagaagatacaagggaaaattactatagag
aataggtcaaagaaagatttgattcaaatgttagtccagagaggttatgaatctgacccagtgaaagcctggaaagaagcacaagaaa
aggcagcagaagaggatgaaacacaaaaccagcatgatgatagttcctccgattcaggaactccttcaggcccagattttaattatatttt
aaatatgtctctgtggtctcttactaaagaaaaagttgaagaactgattaaacagagagatgcaaaagggcgagaggtcaatgatcttaa
aagaaaatctccttcagatctttggaaagaggatttagcggcatttgttgaagaactggataaagtggaatctcaagaacgagaagatgtt
ctggctggaatgtctggaaaagcaattaaaggtaaagttggcaaacctaaggtgaagaaactccagttggaagagacaatgccctcac
cttatggcagaagaataattcctgaaattacagctatgaaggcagatgccagcaaaaagttgctgaagaagaagaagggtgatcttgat
actgcagcagtaaaagtggaatttgatgaagaattcagtggagcaccagtagaaggtgcaggagaagaggcattgactccatcagttcc
tataaataaaggtcccaaacctaagagggagaagaaggagcctggtaccagagtgagaaaaacacctacatcatctggtaaacctag
tgcaaagaaagtgaagaaacggaatccttggtcagatgatgaatccaagtcagaaagtgatttggaagaaacagaacctgtggttattc
caagagattctttgcttaggagagcagcagccgaaagacctaaatacacatttgatttctcagaagaagaggatgatgatgctgatgatg
atgatgatgacaataatgatttagaggaattgaaagttaaagcatctcccataacaaatgatggggaagatgaatttgttccttcagatgggt
tagataaagatgaatatacattttcaccaggcaaatcaaaagccactccagaaaaatctttgcatgacaaaaaaagtcaggattttggaa
atctcttctcatttccttcatattctcagaagtcagaagatgattcagctaaatttgacagtaatgaagaagattctgcttctgtttttcaccatcatt
tggtctgaaacagacagataaagttccaagtaaaacggtagctgctaaaaagggaaaaccgtcttcagatacagtccctaagcccaag
agagccccaaaacagaagaaagtagtagaggctgtaaactctgactcggattcagaatttggcattccaaagaagactacaacacca
aaaggtaaaggccgaggggcaaagaaaaggaaagcatctggctctgaaaatgaaggcgattataaccctggcaggaaaacatcca
aaacaacaagcaagaaaccgaagaagacatcttttgatcaggattcagatgtggacatcttcccctcagacttccctactgagccaccttc
tctgccacgaaccggtcgggctaggaaagaagtaaaatattttacagagtctgatgaagaagaagatgatgttgattttgcaatgtttaatta
a (SEQ ID NO:269)

**Q02880**

MAKSGGCGAGAGVGGGNGALTWVTLFDQNNAAKKEESETANKNDSSKKLSVERVYQKKTQLEH
ILLRPDTYIGSVEPLTQFMWVYDEDVGMNCREVTFVPGLYKIFDEILVNAADNKQRDKNMTCIKVSI
DPESNIISIWNNGKGIPVVEHKVEKVYVPALIFGQLLTSSNYDDDEKKVTGGRNGYGAKLCNIFSTK
FTVETACKEYKHSFKQTWMNNMMKTSEAKIKHFDGEDYTCITFQPDLSKFKMEKLDKDIVALMTR
RAYDLAGSCRGVKVMFNGKKLPVNGFRSYVDLYVKDKLDETGVALKVIHELANERWDVCLTLSEK
GFQQISFVNSIATTKGGRHVDYVVDQVVGKLIEVVKKKNKAGVSVKPFQVKNHIWVFINCLIENPTF
DSQTKENMTLQPKSFGSKCQLSEKFFKAASNCGIVESILNWVKFKAQTQLNKKCSSVKYSKIKGIP
KLDDANDAGGKHSLECTLILTEGDSAKSLAVSGLGVIGRDRYGVFPLRGKILNVREASHKQIMENA
EINNIIKIVGLQYKKSYDDAESLKTLRYGKIMIMTDQDQDGSHIKGLLINFIHHNWPSLLKHGFLEEFI
TPIVKASKNKQELSFYSIPEFDEWKKHIENQKAWKIKYYKGLGTSTAKEAKEYFADMERHRILFRYA
GPEDDAAITLAFSKKKIDDRKEWLTNFMEDRRQRRLHGLPEQFLYGTATKHLTYNDFINKELILFSN
SDNERSIPSLVDGFKPGQRKVLFTCFKRNDKREVKVAQLAGSVAEMSAYHHGEQALMMTIVNLA
QNFVGSNNINLLQPIGQFGTRLHGGKDAASPRYIFTMLSTLARLLFPAVDDNLLKFLYDDNQRVEP
EWYIPIIPMVLINGAEGIGTGWACKLPNYDAREIVNNVRRMLDGLDPHPMLPNYKNFKGTIQELGQ
NQYAVSGEIFVVDRNTVEITELPVRTWTQVYKEQVLEPMLNGTDKTPALISDYKEYHTDTTVKFVV
KMTEEKLAQAEAAGLHKVFKLQTTLTCNSMVLFDHMGCLKKYETVQDILKEFFDLRLSYYGLRKE
WLVGMLGAESTKLNNQARFILEKIQGKITIENRSKKDLIQMLVQRGYESDPVKAWKEAQEKAAEED
ETQNQHDDSSSDSGTPSGPDFNYILNMSLWSLTKEKVEELIKQRDAKGREVNDLKRKSPSDLWK
EDLAAFVEELDKVESQEREDVLAGMSGKAIKGKVGKPKVKKLQLEETMPSPYGRRIIPEITAMKAD
ASKKLLKKKKGDLDTAAVKVEFDEEFSGAPVEGAGEEALTPSVPINKGPKPKREKKEPGTRVRKT
PTSSGKPSAKKVKKRNPWSDDESKSESDLEETEPVVIPRDSLLRRAAAERPKYTFDFSEEEDDDA
DDDDDDNNDLEELKVKASPITNDGEDEFVPSDGLDKDEYTFSPGKSKATPEKSLHDKKSQDFGNL
FSFPSYSQKSEDDSAKFDSNEEDSASVFSPSFGLKQTDKVPSKTVAAKKGKPSSDTVPKPKRAPK
QKKVVEAVNSDSDSEFGIPKKTTTPKGKGRGAKKRKASGSENEGDYNPGRKTSKTTSKKPKKTS
FDQDSDVDIFPSDFPTEPPSLPRTGRARKEVKYFAESDEEEDDVDFAMFN (SEQ ID NO:270)

# Figure 121

**Nucleotide:** M55040
**Sequence:**
atgaggcccccgcagtgtctgctgcacacgccttccctggcttccccactccttctcctcctcctctggctcctgggtggaggagtgggggct
gagggccgggaggatgcagagctgctggtgacggtgcgtgggggccggctgcggggcattcgcctgaagacccccgggggccctgt
ctctgctttcctgggcatcccctttgcggagccacccatgggaccccgtcgctttctgccaccggagcccaagcagccttggtcaggggtg
gtagacgctacaaccttccagagtgtctgctaccaatatgtggacaccctatacccaggttttgagggcaccgagatgtggaaccccaac
cgtgagctgagcgaggactgcctgtacctcaacgtgtggacaccatacccccggcctacatcccccacccctgtcctcgtctggatctatg
gggggtggcttctacagtggggcctcctccttggacgtgtacgatggccgcttcttggtacaggccgagaggactgtgctggtgtccatgaac
taccgggtgggagcctttggcttcctggccctgccggggagccgagaggccccgggcaatgtgggtctcctggatcagaggctggccct
gcagtgggtgcaggagaacgtggcagccttcgggggtgacccgacatcagtgacgctgtttggggagagcgcgggagccgcctcggt
gggcatgcacctgctgtccccgcccagccggggcctgttccacagggccgtgctgcagagcggtgcccccaatggaccctgggccacg
gtgggcatgggagaggcccgtcgcagggccacgcagctggcccaccttgtgggctgtcctccaggcggcactggtgggaatgacaca
gagctggtagcctgccttcggacacgaccagcgcaggtcctggtgaaccacgaatggcacgtgctgcctcaagaaagcgtcttccggtt
ctccttcgtgcctgtggtagatggagacttcctcagtgacaccccagaggccctcatcaacgcgggagacttccacggcctgcaggtgctg
gtgggtgtggtgaaggatgagggctcgtattttctggtttacggggccccaggcttcagcaaagacaacgagtctctcatcagccgggccg
agttcctggccggggtgcgggtcggggttccccaggtaagtgacctggcagccgaggctgtggtcctgcattacacagactggctgcatc
ccgaggacccggcacgcctgagggaggccctgagcgatgtggtgggcgaccacaatgtcgtgtgccccgtggcccagctggctgggc
gactggctgcccagggtgcccgggtctacgcctacgtctttgaacaccgtgcttccacgctctcctggcccctgtggatgggggtgcccca
cggctacgagatcgagttcatctttgggatccccctggacccctctcgaaactacacggcagaggagaaaatcttcgcccagcgactgat
gcgatactgggccaactttgcccgcacaggggatcccaatgagccccgagaccccaaggccccacaatggcccccgtacacggcgg
gggctcagcagtacgttagtctggacctgcggccgctggaggtgcggcgggggctgcgcgcccaggcctgcgccttctggaaccgcttc
ctccccaaattgctcagcgccaccgacacgctcgacgaggcggagcgccagtggaaggccgagttccaccgctggagctcctacatg
gtgcactggaagaaccagttcgaccactacagcaagcaggatcgctgctcagacctgtga (SEQ ID NO:271)

**P22303**
MRPPQCLLHTPSLASPLLLLLLWLLGGGVGAEGREDAELLVTVRGGRLRGIRLKTPGGPVSAFLGI
PFAEPPMGPRRFLPPEPKQPWSGVVDATTFQSVCYQYVDTLYPGFEGTEMWNPNRELSEDCLY
LNVWTPYPRPTSPTPVLVWIYGGGFYSGASSLDVYDGRFLVQAERTVLVSMNYRVGAFGFLALP
GSREAPGNVGLLDQRLALQWVQENVAAFGGDPTSVTLFGESAGAASVGMHLLSPPSRGLFHRA
VLQSGAPNGPWATVGMGEARRRATQLAHLVGCPPGGTGGNDTELVACLRTRPAQVLVNHEWH
VLPQESVFRFSFVPVVDGDFLSDTPEALINAGDFHGLQVLVGVVKDEGSYFLVYGAPGFSKDNES
LISRAEFLAGVRVGVPQVSDLAAEAVVLHYTDWLHPEDPARLREALSDVVGDHNVVCPVAQLAG
RLAAQGARVYAYVFEHRASTLSWPLWMGVPHGYEIEFIFGIPLDPSRNYTAEEKIFAQRLMRYWA
NFARTGDPNEPRDPKAPQWPPYTAGAQQYVSLDLRPLEVRRGLRAQACAFWNRFLPKLLSATDT
LDEAERQWKAEFHRWSSYMVHWKNQFDHYSKQDRCSDL (SEQ ID NO:272)

# Figure 122

**Nucleotide:** AF006464
**Sequence:**
atgagagagctcgtcaacattccactggtacatattcttactctggttgccttcagcggaactgagaaacttccaaaagctcctgtcatcacc
actcctcttgaaacagtggatgccttagttgaagaagtggctactttcatgtgtgcagtggaatcctacccccagcctgagatttcctggacta
gaaataaaattctcattaaactctttgacacccggtacagcatccgggagaatgggcagctcctcaccatcctgagtgtggaagacagtg
atgatggcatttactgctgcacggccaacaatggtgtgggaggagctgtggagagttgtggagccctgcaagtgaagatgaaacctaaa
ataactcgccctcccataaatgtgaaaataatagagggattaaaagcagtcctaccatgtactacaatgggtaatcccaaaccatcagtgt
cttggataaagggagacagccctctcagggaaaattcccgaattgcagttcttgaatctgggagcttgaggattcataacgtacaaaagg
aagatgcaggacagtatcgatgtgtggcaaaaaacagcctcgggacagcatattccaaagtggtgaagctggaagttgaggtttttgcca
ggatcctgcgggctcctgaatcccacaatgtcacctttggctcctttgtgaccctgcactgtacagcaacaggcattcctgtccccaccatca
cctggattgaaaacggaaatgctgtttcttctgggtccattcaagagagtgtgaaagaccgagtgattgactcaagactgcagctgtttatca
ccaagccaggactctacacatgcatagctaccaataagcatggggagaagttcagtactgccaaggctgcagccaccatcagcatagc
agaatggagtaaaccacagaaagataacaaaggctactgcgcccagtacagaggggaggtgtgtaatgcagtcctggcaaaagatg
ctcttgtttttctcaacacctcctatgcggaccctgaggaggcccaagagctactggtccacacggcctggaatgaactgaaagtagtgag
cccagtctgccggccagctgctgaggctttgttgtgtaaccacatcttccaggagtgcagtcctggagtagtgcctactcctattcccatttgc
agagagtactgcttggcagtaaaggagctcttctgcgcaaaagaatggctggtaatggaagagaagacccacagaggactctacagat
ccgagatgcatttgctgtccgtgccagaatgcagcaagcttcccagcatgcattgggaccccacggcctgtgccagactgccacatctag
attataacaaagaaaacctaaaaacattcccaccaatgacgtcctcaaagccaagtgtggacattccaaatctgccttcctcctcctcttctt
ccttctctgtctcacctacatactccatgactgtaataatctccatcatgtccagctttgcaatatttgtgcttcttaccataactactctctattgctg
ccgaagaagaaaacaatggaaaaataagaaaagagaatcagcagcagtaaccctcaccacactgccttctgagctcttactagatag
acttcatcccaaccccatgtaccagaggatgccgctccttctgaaccccaaattgctcagcctggagtatccaaggaataacattgaatat
gtgagagacatcggagagggagcgtttggaagggtgtttcaagcaagggcaccaggcttacttccctatgaaccttttcactatggtggca
gtaaagatgctcaaagaagaagcctcggcagatatgcaagcggactttcagagggaggcagccctcatggcagaatttgacaaccccta
acattgtgaagctattaggagtgtgtgctgtcgggaagccaatgtgcctgctctttgaatacatggcctatggtgacctcaatgagttcctccg
cagcatgtcccctcacaccgtgtgcagcctcagtcacagtgacttgtctatgagggctcaggtctccagccctgggccccccacccctctcct
gtgctgagcagctttgcattgccaggcaggtggcagctggcatggcttacctctcagaacgtaagtttgttcaccgagatttagccaccagg
aactgcctggtgggcgagaacatggtggtgaaaattgccgactttggcctctccaggaacatctactcagcagactactacaaagctaat
gaaaacgacgctatccctatccgttggatgccaccagagtccattttttataaccgctacactacagagtctgatgtgtgggcctatggcgtg
gtcctctgggagatcttctcctatggcctgcagccctactatgggatggcccatgaggaggtcatttactacgtgcgagatggcaacatcctc
tcctgccctgagaactgccccgtggagctgtacaatctcatgcgtctatgttggagcaagctgcctgcagacagacccagtttcaccagtat
tcaccgaattctggaacgcatgtgtgagagggcagagggaactgtgagtgtctaa (SEQ ID NO:273)

**O15146**
MRELVNIPLVHILTLVAFSGTEKLPKAPVITTPLETVDALVEEVATFMCAVESYPQPEISWTRNKILIK
LFDTRYSIRENGQLLTILSVEDSDDGIYCCTANNGVGGAVESCGALQVKMKPKITRPPINVKIIEGLK
AVLPCTTMGNPKPSVSWIKGDSPLRENSRIAVLESGSLRIHNVQKEDAGQYRCVAKNSLGTAYSK
VVKLEVEVFARILRAPESHNVTFGSFVTLHCTATGIPVPTITWIENGNAVSSGSIQESVKDRVIDSRL
QLFITKPGLYTCIATNKHGEKFSTAKAAATISIAEWSKPQKDNKGYCAQYRGEVCNAVLAKDALVF
LNTSYADPEEAQELLVHTAWNELKVVSPVCRPAAEALLCNHIFQECSPGVVPTPIPICREYCLAVK
ELFCAKEWLVMEEKTHRGLYRSEMHLLSVPECSKLPSMHWDPTACARLPHLDYNKENLKTFPPM
TSSKPSVDIPNLPSSSSSFSVSPTYSMTVIISIMSSFAIFVLLTITTLYCCRRRKQWKNKKRESAAV
TLTTLPSELLLDRLHPNPMYQRMPLLLNPKLLSLEYPRNNIEYVRDIGEGAFGRVFQARAPGLLPY
EPFTMVAVKMLKEEASADMQADFQREAALMAEFDNPNIVKLLGVCAVGKPMCLLFEYMAYGDLN
EFLRSMSPHTVCSLSHSDLSMRAQVSSPGPPPLSCAEQLCIARQVAAGMAYLSERKFVHRDLAT
RNCLVGENMVVKIADFGLSRNIYSADYYKANENDAIPIRWMPPESIFYNRYTTESDVWAYGVVLW
EIFSYGLQPYYGMAHEEVIYYVRDGNILSCPENCPVELYNLMRLCWSKLPADRPSFTSIHRILERM
CERAEGTVSV (SEQ ID NO:274)

# Figure 123

**Nucleotide:** U33635
**Sequence:**
atgggagctgcgcggggatccccggccagaccccgccggttgcctctgctcagcgtcctgctgctgccgctgctgggcggtacccagac
agccattgtcttcatcaagcagccgtcctcccaggatgcactgcaggggcgccgggcgctgcttcgctgtgaggttgaggctccgggccc
ggtacatgtgtactggctgctcgatggggcccctgtccaggacacggagcggcgtttcgcccagggcagcagcctgagctttgcagctgt
ggacccgctgcaggactctggcaccttccagtgtgtggctcgggatgatgtcactggagaagaagcccgcagtgccaacgcctccttca
acatcaaatggattgaggcaggtcctgtggtcctgaagcatccagcctcggaagctgagatccagccacagacccaggtcaaacttcgtt
gccacattgatgggcaccctcggcccacctaccaatggttccgagatgggacccccctttctgatggtcagagcaaccacacagtcagc
agcaaggagcggaacctgacgctccggccagctggtcctgagcatagtgggctgtattcctgctgcgcccacagtgcttttagccaggctt
gcagcagccagaacttcaccttgagcattgctgatgaaagctttgccagggtggtgctggcaccccaggacgtggtagtagcgaggtatg
aggaggccatgttccattgccagttctcagcccagccacccccgagcctgcagtggctctttgaggatgagactcccatcactaaccgca
gtcgcccccccacacctccgcagagccacagtgtttgccaacgggtctctgctgctgacccaggtccggccacgcaatgcagggatctac
cgctgcattggccaggggcagagggggcccacccatcatcctggaagccacacttcacctagcagagattgaagacatgccgctatttga
gccacgggtgtttacagctggcagcgaggagcgtgtgacctgccttcccccccaagggtctgccagagcccagcgtgtggtgggagcac
gcgggagtccggctgcccacccatggcagggtctaccagaagggccacgagctggtgttggccaatattgctgaaagtgatgctggtgt
ctacacctgccacgcggccaacctggctggtcagcggagacaggatgtcaacatcactgtggccactgtgccctcctggctgaagaagc
cccaagacagccagctggaggagggcaaacccggctacttggattgcctgacccaggccacaccaaaacctacagttgtctggtaca
gaaaccagatgctcatctcagaggactcacggttcgaggtcttcaagaatgggaccttgcgcatcaacagcgtggaggtgtatgatggg
acatggtaccgttgtatgagcagcaccccagccggcagcatcgaggcgcaagccgtgctccaagtgctggaaaagctcaagttcacac
caccaccccagccacagcagtgcatggggtttgacaaggaggccacggtgccctgttcagccacaggccgagagaagcccactatta
agtgggaacgggcagatgggagcagcctcccagagtgggtgacagacaacgctgggaccctgcattttgcccgggtgactcgagatg
acgctggcaactacacttgcattgcctccaacgggccgcagggccagattcgtgcccatgtccagctcactgtggcagtttttatcaccttca
aagtggaaccagagcgtacgactgtgtaccagggccacacagccctactgcagtgcgaggcccaggggggaccccaagccgctgatt
cagtggaaaggcaaggaccgcatcctggaccccaccaagctgggacccaggatgcacatcttccagaatggctccctggtgatccatg
acgtggcccctgaggactcaggccgctacacctgcattgcaggcaacagctgcaacatcaagcacacggaggcccccctctatgtcgt
ggacaagcctgtgccggaggagtcggagggccctggcagccctcccccctacaagatgatccagaccattgggttgtcggtgggtgcc
gctgtggcctacatcattgccgtgctgggcctcatgttctactgcaagaagcgctgcaaagccaagcggctgcagaagcagcccgaggg
cgaggagccagagatggaatgcctcaacggtgggcctttgcagaacggcagccctcagcagagatccaagaagaagtggccttga
ccagcttgggctccggccccgcggccaccaacaaacgccacagcacaagtgataagatgcacttcccacggtctagcctgcagcccat
caccacgctggggaagagtgagtttggggaggtgttcctggcaaaggctcagggcttggaggagggagtggcagagaccctggtactt
gtgaagagcctgcagagcaaggatgagcagcagcagctggacttccggagggagttggagatgtttgggaagctgaaccacgccaa
cgtggtgcggctcctggggctgtgccgggaggctgagccccactacatggtgctggaatatgtggatctggaagacctcaagcagttcct
gaggatttccaagagcaaggatgaaaaattgaagtcacagcccctcagcaccaagcagaaggtggccctatgcacccaggtagccct
gggaatggagcacctgtccaacaaccgctttgtgcataaggacttggctgcgcgtaactgcctggtcagtgcccagagacaagtgaagg
tgtctgccctgggcctcagcaaggatgtgtacaacagtgagtactaccacttccgccaggcctgggtggcgctgcgctggatgtcccccg
aggccatcctggagggtgacttctctaccaagtctgatgtctgggcctccggtgtgctgatgtgggaagtgtttacacatggagagatgccc
catggtgggcaggcagatgatgaagtactggcagatttgcaggctgggaaggctagacttcctcagcccgagggctgcccttccaaact
ctatcggctgatgcagcgctgctgggccctcagccccaaggaccggccctccttcagtgagattgccagcgccctgggagacagcacc
gtggacagcaagccgtga (SEQ ID NO:275)

**Q13308**
MGAARGSPARPRRLPLLSVLLLPLLGGTQTAIVFIKQPSSQDALQGRRALLRCEVEAPGPVHVYW
LLDGAPVQDTERRFAQGSSLSFAAVDPLQDSGTFQCVARDDVTGEEARSANASFNIKWIEAGPV
VLKHPASEAEIQPQTQVKLRCHIDGHPRPTYQWFRDGTPLSDGQSNHTVSSKERNLTLRPAGPE
HSGLYSCCAHSAFSQACSSQNFTLSIADESFARVVLAPQDVVVARYEEAMFHCQFSAQPPPSLQ
WLFEDETPITNRSRPPHLRRATVFANGSLLLTQVRPRNAGIYRCIGQGQRGPPIILEATLHLAEIED
MPLFEPRVFTAGSEERVTCLPPKGLPEPSVWWEHAGVRLPTHGRVYQKGHELVLANIAESDAGV
YTCHAANLAGQRRQDVNITVATVPSWLKKPQDSQLEEGKPGYLDCLTQATPKPTVVWYRNQMLI
SEDSRFEVFKNGTLRINSVEVYDGTWYRCMSSTPAGSIEAQAVLQVLEKLKFTPPPQPQQCMGF
DKEATVPCSATGREKPTIKWERADGSSLPEWVTDNAGTLHFARVTRDDAGNYTCIASNGPQGQI
RAHVQLTVAVFITFKVEPERTTVYQGHTALLQCEAQGDPKPLIQWKGKDRILDPTKLGPRMHIFQN
GSLVIHDVAPEDSGRYTCIAGNSCNIKHTEAPLYVVDKPVPEESEGPGSPPPYKMIQTIGLSVGAA
VAYIIAVLGLMFYCKKRCKAKRLQKQPEGEEPEMECLNGGPLQNGQPSAEIQEEVALTSLGSGPA
ATNKRHSTSDKMHFPRSSLQPITTLGKSEFGEVFLAKAQGLEEGVAETLVLVKSLQSKDEQQQLD

FRRELEMFGKLNHANVVRLLGLCREAEPHYMVLEYVDLEDLKQFLRISKSKDEKLKSQPLSTKQK
VALCTQVALGMEHLSNNRFVHKDLAARNCLVSAQRQVKVSALGLSKDVYNSEYYHFRQAWVALR
WMSPEAILEGDFSTKSDVWASGVLMWEVFTHGEMPHGGQADDEVLADLQAGKARLPQPEGCP
SKLYRLMQRCWALSPKDRPSFSEIASALGDSTVDSKP (SEQ ID NO:276)

# Figure 124

**Nucleotide:** U51096
**Sequence:**
atgtacgtgagctacctcctggacaaggacgtgagcatgtaccctagctccgtgcgccactctggcggcctcaacctggcgccgcagaa
cttcgtcagcccccccgcagtacccggactacggcggttaccacgtggcggccgcagctgcagcgcagaacttggacagcgcgcagtc
cccggggccatcctggccggcagcgtatggcgccccactccgggaggactggaatggctacgcgcccggaggcgcggccgccgcc
aacgccgtggctcacgcgctcaacggtggctccccggccgcagccatgggctacagcagccccgcagactaccatccgcaccacca
cccgcatcaccacccgcaccacccggccgccgcgccttcctgcgcttctgggctgctgcaaacgctcaaccccggccctcctgggcccg
ccgccaccgctgccgccgagcagctgtctcccggcggccagcggcggaacctgtgcgagtggatgcggaagccggcgcagcagtcc
ctcggcagccaagtgaaaaccaggacgaaagacaaatatcgagtggtgtacacggaccaccagcggctggagctggagaaggagt
ttcactacagtcgctacatcaccatccggaggaaagccgagctagccgccacgctggggctctctgagaggcaggttaaaatctggtttc
agaaccgcagagcaaaggagaggaaaatcaacaagaagaagttgcagcagcaacagcagcagcagccaccacagccgcctcc
gccgccaccacagcctccccagcctcagccaggtcctctgagaagtgtcccagagcccttgagtccggtgtcttccctgcaagcctcagt
gtctggctctgtccctggggttctggggccaactggggggtgctaaaccccaccgtcacccagtga (SEQ ID NO:277)

**Q99626**
MYVSYLLDKDVSMYPSSVRHSGGLNLAPQNFVSPPQYPDYGGYHVAAAAAAAANLDSAQSPGP
SWPAAYGAPLREDWNGYAPGGAAAAANAVAHGLNGGSPAAAMGYSSPADYHPHHHPHHHPHH
PAAAPSCASGLLQTLNPGPPGPAATAAAEQLSPGGQRRNLCEWMRKPAQQSLGSQVKTRTKDK
YRVVYTDHQRLELEKEFHYSRYITIRRKAELAATLGLSERQVKIWFQNRRAKERKINKKKLQQQQ
QQPPQPPPPPPQPPQPQPGPLRSVPEPLSPVSSLQASVSGSVPGVLGPTGGVLNPTVTQ (SEQ
ID NO:278)

# Figure 125

**Nucleotide:** AF250226
**Sequence:**
atgtcatggtttagtggcctcctggtccctaaagtggatgaacggaaaacagcctggggcgaacgcaatgggcagaagcgttcgcggcg
ccgtggcactcgggcaggtggcttctgcacgccccgctatatgagctgcctccgggatgcagagccacccagccccacccctgccggc
ccccctcggtgcccctggcaggatgacgccttcatccggagggcggcccaggcaagggcaaggagctggggctgcgggcagtggc
cctgggcttcgaggataccgaggtgacaacgacagcgggcgggacggctgaggtggcgcccgacgcggtgcccaggagtgggcga
tcctgctggcgccgtctggtgcaggtgttccagtcgaagcagttccgttcggccaagctggagcgcctgtaccagcggtacttcttccagat
gaaccagagcagcctgacgctgctgatggcggtgctggtgctgctcacagcggtgctgctggctttccacgccgcacccgcccgccctc
agcctgcctatgtggcactgttggcctgtgccgccgccctgttcgtggggctcatggtggtgtgtaaccggcatagcttccgccaggactcc
atgtgggtggtgagctacgtggtgctgggcatcctggcggcagtgcaggtcgggggcgctctcgcagcagacccgcgcagcccctctgc
gggcctctggtgccctgtgttctttgtctacatcgcctacacgctcctccccatccgcatgcgggctgccgtcctcagcggcctgggcctctcc
accttgcatttgatcttggcctggcaacttaaccgtggtgatgccttcctctggaagcagctcggtgccaatgtgctgctgttcctctgcaccaa
cgtcattggcatctgcacacactatccagcagaggtgtctcagcgccaggcctttcaggagacccgcggttacatccaggcccggctcca
cctgcagcatgagaatcggcagcaggagcggctgctgctgtcggtattgccccagcacgttgccatggagatgaaagaagacatcaac
acaaaaaaagaagacatgatgttccacaagatctacatacagaagcatgacaatgtcagcatcctgtttgcagacattgagggcttcacc
agcctggcatcccagtgcactgcgcaggagctggtcatgaccctgaatgagctctttgcccggtttgacaagctggctgcggagaatcact
gcctgaggatcaagatcttgggggactgttactactgtgtgtcagggctgccggaggcccgggccgaccatgcccactgctgtgtggaga
tgggggtagacatgattgaggccatctcgctggtacgtgaggtgacaggtgtgaatgtgaacatgcgcgtgggcatccacagcgggcgc
gtgcactgcggcgtccttggcttgcggaaatggcagttcgatgtgtggtccaatgatgtgaccctggccaaccacatggaggcaggaggc
cgggctggccgcatccacatcactcgggcaacactgcagtacctgaacggggactacgaggtggagccaggccgtggtggcgagcg
caacgcgtacctcaaggagcagcacattgagactttcctcatcctgggcgccagccagaaacggaaagaggagaaggccatgctggc
caagctgcagcggactcgggccaactccatggaagggctgatgccgcgctgggttcctgatcgtgccttctcccggaccaaggactcca
aggccttccgccagatgggcattgatgattccagcaaagacaaccggggcacccaagatgccctgaaccctgaggatgaggtggatg
agttcctgagccgtgccatcgatgcccgcagcattgatcagctgcggaaggaccatgtgcgccggtttctgctcaccttccagagagagg
atcttgagaagaagtactcccggaaggtggatccccgcttcggagcctacgttgcctgtgccctgttggtcttctgcttcatctgcttcatccag
cttctcatcttcccacactccaccctgatgcttgggatctatgccagcatcttcctgctgctgctaatcaccgtgctgatctgtgctgtgtactcctg
tggttctctgttccctaaggccctgcaacgtctgtcccgcagcattgtccgctcacgggcacatagcaccgcagttggcatcttttccgtcctgc
ttgtgtttacttctgccattgccaacatgttcacctgtaaccacaccccccatacggagctgtgcagcccggatgctgaatttaacacctgctga
catcactgcctgccacctgcagcagctcaattactctctgggcctggatgctcccctgtgtgagggcaccatgcccacctgcagctttcctga
gtacttcatcgggaacatgctgctgagtctcttggccagctctgtcttcctgcacatcagcagcatcgggaagttggccatgatctttgtcttgg
ggctcatctatttggtgctgcttctgctgggtcccccagccaccatctttgacaactatgacctactgcttggcgtccatggcttggcttcttccaa
tgagaccctttgatgggctggactgtccagctgcagggagggtggccctcaaatatatgacccctgtgattctgctggtgtttgcgctggcgct
gtatctgcatgctcagcaggtggagtcgactgcccgcctagacttcctctggaaactacaggcaacaggggagaaggaggagatggag
gagctacaggcatacaaccggaggctgctgcataacattctgcccaaggacgtggcggcccacttcctggcccgggagcgccgcaat
gatgaactctactatcagtcgtgtgagtgtgtggctgttatgtttgcctccattgccaacttctctgagttctatgtggagctggaggcaaacaat
gagggtgtcgagtgcctgcggctgctcaacgagatcatcgctgactttgatgagattatcagcgaggagcggttccggcagctggaaaa
gatcaagacgattggtagcacctacatggctgcctcagggctgaacgccagcacctacgatcaggtgggccgctcccacatcactgccc
tggctgactacgccatgcggctcatggagcagatgaagcacatcaatgagcactccttcaacaatttccagatgaagattgggctgaaca
tgggcccagtcgtggcaggtgtcatcggggctcggaagccacagtatgacatctgggggaacacagtgaatgtctctagtcgtatggaca
gcacgggggtccccgaccgaatccaggtgaccacggacctgtaccaggttctagctgccaagggctaccagctggagtgtcgaggggt
ggtcaaggtgaagggcaaggggggagatgaccacctacttcctcaatgggggccccagcagttaa (SEQ ID NO:279)

**O43306**
MSWFSGLLVPKVDERKTAWGERNGQKRSRRRGTRAGGFCTPRYMSCLRDAEPPSPTPAGPPR
CPWQDDAFIRRGGPGKGKELGLRAVALGFEDTEVTTTAGGTAEVAPDAVPRSGRSCWRRLVQV
FQSKQFRSAKLERLYQRYFFQMNQSSLTLLMAVLVLLTAVLLAFHAAPARPQPAYVALLACAAALF
VGLMVVCNRHSFRQDSMWVVSYVVLGILAAVQVGGALAADPRSPSAGLWCPVFFVYIAYTLLPIR
MRAAVLSGLGLSTLHLILAWQLNRGDAFLWKQLGANVLLFLCTNVIGICTHYPAEVSQRQAFQETR
GYIQARLHLQHENRQQERLLLSVLPQHVAMEMKEDINTKKEDMMFHKIYIQKHDNVSILFADIEGFT
SLASQCTAQELVMTLNELFARFDKLAAENHCLRIKILGDCYYCVSGLPEARADHAHCCVEMGVDM
IEAISLVREVTGVNVNMRVGIHSGRVHCGVLGLRKWQFDVWSNDVTLANHMEAGGRAGRIHITRA
TLQYLNGDYEVEPGRGGERNAYLKEQHIETFLILGASQKRKEEKAMLAKLQRTRANSMEGLMPR
WVPDRAFSRTKDSKAFRQMGIDDSSKDNRGTQDALNPEDEVDEFLSRAIDARSIDQLRKDHVRR
FLLTFQREDLEKKYSRKVDPRFGAYVACALLVFCFICFIQLLIFPHSTLMLGIYASIFLLLLITVLICAVY

SCGSLFPKALQRLSRSIVRSRAHSTAVGIFSVLLVFTSAIANMFTCNHTPIRSCAARMLNLTPADITA
CHLQQLNYSLGLDAPLCEGTMPTCSFPEYFIGNMLLSLLASSVFLHISSIGKLAMIFVLGLIYLVLLLL
GPPATIFDNYDLLLGVHGLASSNETFDGLDCPAAGRVALKYMTPVILLVFALALYLHAQQVESTAR
LDFLWKLQATGEKEEMEELQAYNRRLLHNILPKDVAAHFLARERRNDELYYQSCECVAVMFASIA
NFSEFYVELEANNEGVECLRLLNEIIADFDEIISEERFRQLEKIKTIGSTYMAASGLNASTYDQVGRS
HITALADYAMRLMEQMKHINEHSFNNFQMKIGLNMGPVVAGVIGARKPQYDIWGNTVNVSSRMD
STGVPDRIQVTTDLYQVLAAKGYQLECRGVVKVKGKGEMTTYFLNGGPSS (SEQ ID NO:280)

# Figure 126

**Nucleotide:** AB082530
**Sequence:**
ggcaaacacaattcttcctcattcacatagccatcatttacactgcttgccaaccctaatgaatatggctgcatcctttgatatccccaaggaa
aagagactgcgagccagtgcagccttgaactgtttaaaacgcttccatgaaatgaagaaacgaggacctaagccttatagtcttcatttag
accacattattcagaaagcaattgcaacacaccagaaacgggatcagtatctccgagttcagaaagatatatttatccttaaggatacag
aggaagctcttttaattaaccttagagatagccaagtccttcaacataaagagaatcttgaatggaattggaatcttatagggaccattcttaa
gtggccaaatgtaaatctaagaaactataaagatgaacagttacacaggtttgtacgaagactactttatttttacaagcccagcagtaaatt
atatgccaacctggatctggattttgccaaggccaaacagctcacggttgtaggttgccagtttacagaatttcttcttgaatctgaagaggat
gggcaaggctacttagaagatctagtaaaggatattgttcagtggctcaatgcttcatctggaatgaaacccgaaagaagtcttcaaaata
atggtttattgaccacccttagtcaacactacttttatttattggaacactttcttgccaccctcatggagttaaaatgctggaaaaatgcagtgt
atttcagtgtctccttaatctttgctccttgaaaaaccaagatcacttgctaaaacttactgtttctagcttggactatagcagagatggattggct
agagtcatcctttccaaaattttaactgcagctactgatgcctgcagactctatgcaacaaaacatttaagggtattattgagagctaatgttg
aattctttaataattggggaattgagttgttagtgacccagctacatgataaaaacaaaacgatttcctctgaagctcttgatatcctcgatgaa
gcatgtgaagacaaggccaatcttcatgctctcattcagatgaaaccagcgttatcccaccttggagacaagggtttgcttctcctgctgag
atttctctccattccaaaaggattttcctatctgaatgaaagaggttatgtagcaaaacaattggaaaagtggcacagggaatacaactcca
aatatgttgacttgattgaggaacaactcaatgaagcacttactacttaccggaagcctgttgatggtgataactatgttcgtcggagtaacc
aaagattacagcgtcctcacgtctacctgcctatacacctttatggacaactagtacaccataaaacaggctgccatttgttggaagtacag
aatattattacagaactctgtcgtaatgttcgtacaccagatttggataagtgggaagaaattaaaaaactgaaagcatctctttgggccttg
ggaaatatcggctcatcaaattggggtctcaatttgctacaggaagaaaacgtgattccagatatactaaaacttgcaaaacagtgtgaag
ttctttccatcagagggacctgtgtatatgtacttgggctcatagctaaaaccaaacaaggctgtgatattctaaaatgtcacaactgggatg
ctgtgaggcatagtcgcaaacatctgtggccagtggttccagatgatgtggaacaactctgtaatgaactttcatctatcccaagcactctaa
gtttgaactcggagtcaaccagctctagacataatagtgaaagtgaatctgtgccatcgagtatgttcatattggaggatgaccggtttggca
gcagctctactagtacatttttccttgatatcaatgaagatacagagccaacattttatgaccgatctggacccataaaggataaaaattcatt
ccctttctttgcttctagtaaacttgtgaagaatcgtatcttaaattcgcttactttgcctaacaaaaaacatcgtagtagcagtgatccaaaagg
agggaaattatcatctgaaagtaagacaagcaacaggcgaatcagaacacttacggagcccagtgttgattttaatcatagtgatgatttt
acacccatatccactgtacagaaaacattacaattagagacttcatttatggggaataagcacattgaagacactggtagtacaccaagc
attggagaaaatgacttaaaattcaccaagaattttggtacagagaatcacagagaaaatacaagccgagagaggttagtagtagaaa
gttcaacgagctcacatatgaagatacgtagccaaagtttcaatacagacactacaacaagtggcataagttcaatgagctcaagtccttc
acgagagacagtaggtgtagatgctacaactatggacacagactgtggaagcatgagtactgtggtaagtactaaaactattaagacaa
gccactatttgacgccacagtctaaccatctgtctctctccaaatcaaattcggtgtccctggtgcctccaggttcttctcatacgcttcctagaa
gagcacagtcccttaaagcaccctctattgctacaattaaaagtctagcagattgtaactttagttacacaagttctagagatgcttttggctat
gctacactgaaaagactacagcaacaaagaatgcatccatccttatctcactctgaagctttggcatctccagcaaaagatgtgctatttac
tgataccatcaccatgaaggccaacagttttgagtccagattaacaccaagcaggttcatgaaagccttaagttatgcatcattagataaa
gaagatttattgagtcctattaatcaaaataccctgcaacgatcttcctcagtgcggtccatggtgtccagtgccacatatgggggttcagatg
attacattggtcttgctctcccggtggatataaatgatatattccaggtaaaggatattccctattttcagacaaaaaacataccaccacatgat
gatcgaggtgcaagagcatttgcccatgatgcaggaggtcttccatctggaactggaggtcttgtaaaaaattcttttcacttgctacgacag
cagatgagtcttacggaaataatgaattcaatccattcagatgcctctctgtttttagaaagtacagaagacactggactacaggaacatac
agatgataactgcctttattgtgtctgtattgaaattctgggtttccagcccagcaaccaactgagtgcaatatgtagtcattcagactttcaag
atattccatattctgattggtgtgagcagactatccataatcctttagaagtggttccctctaagttttcggggatttctggatgcagtgatggggt
gtctcaagaaggctcagctagcagcaccaaaagcacagaattgttactaggtgttaaaacaattccagatgatacaccaatgtgccgtat
actccttcgcaaagaagttctaagattagtcattaatttgagtagttcagtttcaactaaatgtcatgagactgggcttttaacaattaaggaga
agtatcctcaaacatttgatgacatatgcctttactctgaggtttcccatttgctgtcacactgcacattcagacttccgtgtcggaggttcataca
agaattatttcaagatgtacagtttctacaaatgcatgaagaagcagaggctgtgttggcaacaccaccaaagcaacctatagttgataca
tctgctgaatcctga (SEQ ID NO:281)

**Q8NCM6; BAC02708**
ANTILPHSHSHHLHCLPTLMNMAASFDIPKEKRLRASAALNCLKRFHEMKKRGPKPYSLHLDHIIQK
AIATHQKRDQYLRVQKDIFILKDTEEALLINLRDSQVLQHKENLEWNWNLIGTILKWPNVNLRNYKD
EQLHRFVRRLLYFYKPSSKLYANLDLDFAKAKQLTVVGCQFTEFLLESEEDGQGYLEDLVKDIVQ
WLNASSGMKPERSLQNNGLLTTLSQHYFLFIGTLSCHPHGVKMLEKCSVFQCLLNLCSLKNQDHL
LKLTVSSLDYSRDGLARVILSKILTAATDACRLYATKHLRVLLRANVEFFNNWGIELLVTQLHDKNK
TISSEALDILDEACEDKANLHALIQMKPALSHLGDKGLLLLLRFLSIPKGFSYLNERGYVAKQLEKW
HREYNSKYVDLIEEQLNEALTTYRKPVDGDNYVRRSNQRLQRPHVYLPIHLYGQLVHHKTGCHLL
EVQNIITELCRNVRTPDLDKWEEIKKLKASLWALGNIGSSNWGLNLLQEENVIPDILKLAKQCEVLSI

RGTCVYVLGLIAKTKQGCDILKCHNWDAVRHSRKHLWPVVPDDVEQLCNELSSIPSTLSLNSEST
SSRHNSESESVPSSMFILEDDRFGSSSTSTFFLDINEDTEPTFYDRSGPIKDKNSFPFFASSKLVKN
RILNSLTLPNKKHRSSSDPKGGKLSSESKTSNRRIRTLTEPSVDFNHSDDFTPISTVQKTLQLETSF
MGNKHIEDTGSTPSIGENDLKFTKNFGTENHRENTSRERLVVESSTSSHMKIRSQSFNTDTTTSGI
SSMSSSPSRETVGVDATTMDTDCGSMSTVVSTKTIKTSHYLTPQSNHLSLSKSNSVSLVPPGSSH
TLPRRAQSLKAPSIATIKSLADCNFSYTSSRDAFGYATLKRLQQQRMHPSLSHSEALASPAKDVLF
TDTITMKANSFESRLTPSRFMKALSYASLDKEDLLSPINQNTLQRSSSVRSMVSSATYGGSDDYIG
LALPVDINDIFQVKDIPYFQTKNIPPHDDRGARAFAHDAGGLPSGTGGLVKNSFHLLRQQMSLTEI
MNSIHSDASLFLESTEDTGLQEHTDDNCLYCVCIEILGFQPSNQLSAICSHSDFQDIPYSDWCEQTI
HNPLEVVPSKFSGISGCSDGVSQEGSASSTKSTELLLGVKTIPDDTPMCRILLRKEVLRLVINLSSS
VSTKCHETGLLTIKEKYPQTFDDICLYSEVSHLLSHCTFRLPCRRFIQELFQDVQFLQMHEEAEAVL
ATPPKQPIVDTSAES (SEQ ID NO:282)

## Figure 127

**Nucleotide:** M13928
**Sequence:**
atgcagccccagtccgttctgcacagcggctacttccacccactacttcgggcctggcagacagccaccaccaccctcaatgcctccaac
ctcatctaccccatctttgtcacggatgttcctgatgacatacagcctatcaccagcctcccaggagtggccaggtatggtgtgaagcggct
ggaagagatgctgaggcccttggtggaagagggcctacgctgtgtcttgatctttggcgtccccagcagagttcccaaggacgagcggg
gttccgcagctgactccgaggagtccccagctattgaggcaatccatctgttgaggaagaccttccccaacctcctggtggcctgtgatgtc
tgcctgtgtccctacacctcccatggtcactgcgggctcctgagtgaaaacggagcattccgggctgaggagagccgccagcggctggc
tgaggtggcattggcgtatgccaaggcaggatgtcaggtggtagccccgtcggacatgatggatggacgcgtggaagccatcaaagag
gccctgatggcacatggacttggcaacagggtatcggtgatgagctacagtgccaaatttgcttcctgtttctatggccctttccgggatgca
gctaagtcaagcccagcttttggggaccgccgctgctaccagctgccccctggagcacgaggcctggctctccgagctgtggaccggga
tgtacgggaaggagctgacatgctcatggtgaagccgggaatgccctacctggacatcgtgcgggaggtaaaggacaagcaccctga
cctccctctcgccgtgtaccacgtctctggagagtttgccatgctgtggcatggagcccaggccggggcatttgatctcaaggctgccgtact
ggaggccatgactgccttccgcagagcaggtgctgacatcatcatcacctactacacaccgcagctgctgcagtggctgaaggaggaat
ga (SEQ ID NO:283)

**P13716**
MQPQSVLHSGYFHPLLRAWQTATTTLNASNLIYPIFVTDVPDDIQPITSLPGVARYGVKRLEEMLR
PLVEEGLRCVLIFGVPSRVPKDERGSAADSEESPAIEAIHLLRKTFPNLLVACDVCLCPYTSHGHC
GLLSENGAFRAEESRQRLAEVALAYAKAGCQVVAPSDMMDGRVEAIKEALMAHGLGNRVSVMS
YSAKFASCFYGPFRDAAKSSPAFGDRRCYQLPPGARGLALRAVDRDVREGADMLMVKPGMPYL
DIVREVKDKHPDLPLAVYHVSGEFAMLWHGAQAGAFDLKAAVLEAMTAFRRAGADIIITYYTPQLL
QWLKEE (SEQ ID NO:284)

# Figure 128

**Nucleotide:** AK001360
**Sequence:**
atgtttatggcacagattagtcactgctacccagagtacctaagtaattttcctcaagaggtgaaagatcttctctcctgcaatcataccgtatt
ggatccagatctgcgaatgacattttgcaaagctttgatcttgctgagaaataagaatctcatcaatccatcaagcctgctagaactcttctttg
aactttttcgttgccatgataaacttctgcgaaagactttatacacacatattgtgactgatatcaagaatataaatgcaaaacacaagaaca
ataaagtgaatgtagtattgcaaaatttcatgtacaccatgttaagagatagcaatgcaaccgcagccaagatgtctttagatgtaatgattg
aactctacagaaggaacatctggaatgatgcaaaaactgtcaatgttatcacaactgcatgtttctctaaggtcaccaagatattagttgcc
gctttgacattctttcttgggaaagatgaagatgaaaaacaggacagtgactccgaatctgaggatgatggaccaacagcaagagacct
gctagtacaatatgctacagggaagaaaagttccaaaaacaagaaaaagttggaaaaggcaatgaaagtgctcaagaaacaaaaa
aagaagaaaaaaccagaggtgtttaacttttcagccattctcttgattcatgatccccaagattttgcggaaaaactactaaagcagcttga
gtgctgtaaggagaggtttgaagtgaagatgatgctcatgaaccttatctccagattggtgggaattcatgagcttttcctcttcaatttctatcc
ctttttgcaaaggtttctgcagccccaccaaagagaagtaaccaagatccttctgtttgctgcacaagcatctcatcacctagtaccccag
agattattcaatcattgcttatgactgtggcaaacaattttgttaccgacaagaactctggagaagtcatgacagtaggaatcaatgctataa
aggagataacagctcgatgtcctctggccatgactgaagaacttctccaagacctggctcagtataaaacacacaaggataagaatgta
atgatgtctgctagaactttgattcacctcttccgaacactgaatcctcagatgctgcagaagaaattccggggtaagcctacagaggcctc
catagaagcaagagtacaagaatatggagaattagatgctaaagattacattccaggagcagaagttctggaagttgagaaagaaga
gaatgctgaaaatgatgaagatggatgggaaagtaccagtctcagtgaggaggaggatgctgatggtgaatggattgatgtgcaacact
cttccgatgaagaacagcaagaaatctccaagaagctgaacagcatgcccatggaggagcggaaggccaaagctgcagccatcag
cactagccgagttttaactcaggaagacttccagaaaatccgcatggcccaaatgagaaaagaacttgatgctgcccccgggaaatgc
cagaagaggaaatacattgaaatagacagtgatgaagagcccaggggtgaattacttctcttcgggacattgaacgccttcataaaaag
ccaaagtctgacaaagagacaagactagcaactgcaatggctggaaagacagaccgaaaagaatttgtgaggaagaaaaccaaaa
caaatccatttccagttcgacaaataaagagaagaaaaaacagaagaactttatgatgatgcggtatagccagaatgtccggtcaaaa
aataagcgttccttccgagaaaaacagttggcactacgagatgcacttttgaaaaagagaaaaagaatgaagtaa (SEQ ID NO:285)

**Q9NVU7**
MFMAQISHCYPEYLSNFPQEVKDLLSCNHTVLDPDLRMTFCKALILLRNKNLINPSSLLELFFELFR
CHDKLLRKTLYTHIVTDIKNINAKHKNNKVNVVLQNFMYTMLRDSNATAAKMSLDVMIELYRRNIW
NDAKTVNVITTACFSKVTKILVAALTFFLGKDEDEKQDSDSESEDDGPTARDLLVQYATGKKSSKN
KKKLEKAMKVLKKQKKKKKPEVFNFSAILLIHDPQDFAEKLLKQLECCKERFEVKMMLMNLISRLV
GIHELFLFNFYPFLQRFLQPHQREVTKILLFAAQASHHLVPPEIIQSLLMTVANNFVTDKNSGEVMT
VGINAIKEITARCPLAMTEELLQDLAQYKTHKDKNVMMSARTLIHLFRTLNPQMLQKKFRGKPTEA
SIEARVQEYGELDAKDYIPGAEVLEVEKEENAENDEDGWESTSLSEEEDADGEWIDVQHSSDEE
QQEISKKLNSMPMEERKAKAAISTSRVLTQEDFQKIRMAQMRKELDAAPGKCQKRKYIEIDSDEE
PRGELLSLRDIERLHKKPKSDKETRLATAMAGKTDRKEFVRKKTKTNPFSSSTNKEKKKQKNFMM
MRYSQNVRSKNKRSFREKQLALRDALLKKRKRMK (SEQ ID NO:286)

# Figure 129

**Nucleotide:** AY033889
**Sequence:**
atgcaaagttgtgaatccagtggtgacagtgcggatgaccctctcagtcgcggcctacggagaaggggacagcctcgtgtggtggtgatc
ggcgccggcttggctggcctggctgcagccaaagcacttcttgagcagggtttcacggatgtcactgtgcttgaggcttccagccacatcg
gaggccgtgtgcagagtgtgaaacttggacacgccacctttgagctgggagccacctggatccatggctcccatgggaaccctatctatc
atctagcagaagccaacggcctcctggaagagacaaccgatggggaacgcagcgtgggccgcatcagcctctattccaagaatggcg
tggcctgctaccttaccaaccacggccgcaggatccccaaggacgtggttgaggaattcagcgatttatacaacgaggtctataacttgac
ccaggagttcttccggcacgataaaccagtcaatgctgaaagtcaaaatagcgtgggggtgttcacccgagaggaggtgcgtaaccgc
atcaggaatgaccctgacgacccagaggctaccaagcgcctgaagctcgccatgatccagcagtacctgaaggtggagagctgtgag
agcagctcacacagcatggacgaggtgtccctgagcgccttcggggagtggaccgagatccccggcgctcaccacatcatcccctcgg
gcttcatgcgggttgtggagctgctggcggagggcatccctgcccacgtcatccagctagggaaacctgtccgctgcattcactgggacc
aggcctcagcccgccccagaggccctgagattgagccccggggtgagggcgaccacaatcacgacactggggagggtggccaggg
tggagaggagccccggggggcaggtgggatgaggatgagcagtggtcggtggtggtggagtgcgaggaccgtgagctgatcccgg
cggaccatgtgattgtgaccgtgtcgctaggtgtgctaaagaggcagtacaccagtttcttccggccaggcctgcccacagagaaggtgg
ctgccatccaccgcctgggcattggcaccaccgacaagatctttctggaattcgaggagcccttctggggccctgagtgcaacagcctac
agtttgtgtgggaggacgaagcggagagccacaccctcacctacccacctgagctctggtaccgcaagatctgcggctttgatgtcctcta
cccgcctgagcgctacggccatgtgctgagcggctggatctgcggggaggaggccctcgtcatggagaagtgtgatgacgaggcagtg
gccgagatctgcacggagatgctgcgtcagttcacagggaaccccaacattccaaaacctcggcgaatcttgcgctcggcctggggca
gcaacccttacttccgtggctcctattcatacacgcaggtgggctccagcggggcggatgtggagaagctggccaagcccctgccgtac
acggagagctcaaagacagcgcccatgcaggtgctgttttccggtgaggccacccaccgcaagtactattccaccacccacggtgctct
gctgtccggccagcgtgaggctgcccgcctcattgagatgtaccgagacctcttccagcaggggacctga (SEQ ID NO:287)

**Q96QT3**
MQSCESSGDSADDPLSRGLRRRGQPRVVVIGAGLAGLAAAKALLEQGFTDVTVLEASSHIGGRV
QSVKLGHATFELGATWIHGSHGNPIYHLAEANGLLEETTDGERSVGRISLYSKNGVACYLTNHGR
RIPKDVVEEFSDLYNEVYNLTQEFFRHDKPVNAESQNSVGVFTREEVRNRIRNDPDDPEATKRLK
LAMIQQYLKVESCESSSHSMDEVSLSAFGEWTEIPGAHHIIPSGFMRVVELLAEGIPAHVIQLGKPV
RCIHWDQASARPRGPEIEPRGEGDHNHDTGEGGQGGEEPRGGRWDEDEQWSVVVECEDRELI
PADHVIVTVSLGVLKRQYTSFFRPGLPTEKVAAIHRLGIGTTDKIFLEFEEPFWGPECNSLQFVWE
DEAESHTLTYPPELWYRKICGFDVLYPPERYGHVLSGWICGEEALVMEKCDDEAVAEICTEMLRQ
FTGNPNIPKPRRILRSAWGSNPYFRGSYSYTQVGSSGADVEKLAKPLPYTESSKTAPMQVLFSGE
ATHRKYYSTTHGALLSGQREAARLIEMYRDLFQQGT (SEQ ID NO:288)

# Figure 130

**Nucleotide:** D28476
**Sequence:**
atgtccaaccggcctaataacaatccagggggggtcactgcgacgttcacagaggaacactgccggggcccaaccacaagacgactc
aataggaggaagaagctgcagttcatcatctgctgtgatagttccacaaccagaggatccagacagagccaatacttcagaaagacaa
aaaacggggcaggtgcctaagaaagacaattctcgaggagtgaagcgcagtgctagtccagactacaacaggaccaattctcctagct
ctgcaaaaaaaccaaaagcacttcagcatactgaatctccctcagaaacaaataagccacatagtaagtcaaagaagagacatttaga
ccaggagcaacaactgaaatctgcacaatcaccatcaacaagcaaggctcataccaggaagagtggggccactggcggttcacgga
gtcagaaaagaaaaaggacagagagttcttgtgtaaagagtggctccgggtctgaatcaactggtgcagaagagagatctgcgaaac
ctaccaagctggcttcaaaatcagccacctcagccaaagctgggtgtagcaccatcactgattcttcttctgctgcctctacttcctcctcgtctt
ctgctgtagcctcggcctcctccactgtaccaccaggtgccagagtgaaacaaggaaaagatcagaacaaggccaggcgttcccgttc
agcgtccagtcccagccccagaagaagtagcagggaaaaggaacagagtaaaactggtggctcttcaaaatttgattggctgctcgtt
tcagccctaaagttagccttcctaaaacaaaactgtctcttccagggtcttctaagtcagagacatcaaaacctggaccttctggattacagg
ccaaattagcaagtttaagaaaatctacgaagaaacgcagtgagtctccacctgctgagctccccagtttgaggcggagcacacgcca
aaagaccacgggctcctgtgctagtaccagtcggcgaggctctggcctgggcaaaagaggagcagctgaagctcgtcgacaggaga
aaatggcagaccctgaaagcaaccaggaggcagtaaattcttcagctgctcggacagatgaagctccccaaggagctgcaggggctg
ttggcatgaccacctctggggagagtgaatcagatgattccgagatgggacgtttgcaagctttgttagaggcaaggggtcttccccctcac
ctatttggtcctcttggtcctcggatgtcacagcttttccatagaacaattggaagtggagctagttctaaggcccagcagctactacaaggat
tgcaagccagtgatgaaagtcaacagcttcaggcagttattgagatgtgtcagttactggtcatgggaaatgaggagacactgggagggt
ttcctgtcaagagtgttgttccagctttgattacgttacttcagatggagcacaattttgatattatgaaccatgcttgtcgagccttaacatacat
gatggaagcacttcctcgatcttctgctgttgtagtagatgctattcctgtcttttagaaaagctgcaagttattcagtgtattgatgtggcagag
caggccttgactgccttggagatgttgtcacggagacatagtaaagccattctacaggcgggtggtttggcagactgcttgctgtacctaga
attcttcagcataaatgcccaaagaaatgcattagcaattgcagctaattgctgccagagtatcacgccagatgaatttcattttgtggcaga
ttcactcccattgctaacccaaaggctaacacatcaggataaaaagtcagtagaaagcacttgcctttgttttgcacgcctagtggacaact
tccagcatgaggagaatttactccagcaggttgcttccaaagatctgcttacaaatgttcaacagctgttggtagtgactccacccatttaag
ttctgggatgtttataatggtggttcgcatgttttctctgatgtgttccaactgtccaactttagctgttcaacttatgaaacaaaacattgcagaaa
cgcttcactttctcctgtgtggtgcctccaatggaagttgtcaggaacagattgatcttgttccacgaagccctcaagagttgtatgaactgac
atctctgatttgtgaacttatgccatgtttaccaaaagaaggcatttttgcagttgataccatgttgaagaagggaaatgcacagaacacaga
tggtgcgatatggcagtggcgtgatgatcggggcctctggcatccatataacaggattgacagccggatcattgagcaaatcaatgagga
cacgggaacagcacgtgccattcagagagaaaacctaacccgttagccaatagtaacactagtggatattcagagtcaaagaaggatgat
gctcgagcacagcttatgaaagaggatccggaactggctaagtcttttattaagacattatttggtgttctttatgaagtgtatagttcctcagca
ggacctgcggtcagacataagtgccttagagcaattcttaggataatttattttgcggatgctgaacttctgaaggatgttctgaaaaatcatg
ctgtttcaagtcacattgcttccatgctgtcaagccaagacctgaagatagtagtgggagcacttcagatggcagaaattttaatgcagaag
ttacctgatatttttagtgtttacttcagaagagaaggtgtaatgcatcaagtaaaacacttagcagaatcagagtctttgttgacaagtccacc
aaaggcatgtacgaatggatcgggatccatgggatccacaacttcagtcagcagtgggacagccacagctgccactcatgctgcagct
gacttgggatcacccagcttgcagcacagcagggatgattctttagatctcagccctcaaggtcgattaagtgatgttctaaagagaaaac
gactgccaaaacgagggccaagaaggccaaagtactcacctccaagagatgatgacaaagtagacaatcaagctaaaagccccac
cactactcagtcacctaaatcttctttcctggcaagcttgaatccaaaaacatggggaaggttaagtacacagtccaacagcaacaacatt
gagccagcacggactgcgggaggtagtggccttgccagggctgcctcaaaggataccatctccaataatagagaaaaaattaaaggtt
ggattaaggagcaggcacataaatttgtagaacgttatttcagttctgagaatatggatggaagcaaccctgcattgaatgtccttcagaga
ctttgtgctgcaaccgaacaactcaacctccaggtggatggtggagctgagtgccttgtagaaatccgtagcatagtctcagagtcagatgt
ttcatcatttgaaatccaacatagtggatttgtgaagcagctgttgctttatttgacatctaaaagtgaaaaggatgctgtgagcagagagatc
agattaaagcgatttcttcatgtatttttttcttctccacttcctggagaagagcccattggaagagtggaaccagtgggtaatgcacctttgttg
gcattagttcacaagatgaacaactgcctcagccagatggaacaatttccagtcaaagtacatgatttccctagtggaaatgggacagga
ggcagcttttctctcaacagaggatcacaggctttaaaattttcaacacacatcaattaaaatgccagttacaaaggcatccagactgtgc
aaatgtgaagcagtggaagggtggacctgtcaagattgaccctctggctttggtacaagccatcgagagatacctttgtagttagagggtat
ggaagagtaagagaagatgatgaagacagcgatgacgatggatcagatgaggaaatagatgagtctctggctgctcagttcctaaattc
aggaaatgtaagacacaggctgcagttttatattggagaacatttgctgccgtataacatgactgtgtatcaggcagtacggcagtttagtat
acaggctgaagatgaaagagaatccacagatgatgagagcaatcctctaggcagagctggtatttggacaaagactcatacaatatggt
ataaacctgtgagagaggatgaagaaagtaataaagattgtgttggtggtaaaagaggaagagcccaaacagctccaacgaaaactt
cccctagaaatgcaaaaaagcatgatgagttatggcacgatggagtgtgcccatcagtatcaaatcctttagaagtttacctcattcccaca
ccacctgaaaatataacatttgaagacccgtcattagatgtgatccttcttttaagagttttacatgctatcagtcgatactggtattacttgtatg
ataatgcaatgtgcaaggaaattattccaactagtgaatttattaacagtaagttaacagcaaaagcaaataggcaacttcaagatccttta
gtaatcatgacaggaaacatcccaacatggcttactgagctaggaaaaacctgcccatttttctttcctttgatacccggcaaatgctttttat
gtaactgcatttgatcgggaccgagcaatgcaaagattacttgataccaacccagaaatcaaccagtctgattctcaagatagcagagttg

cacctagattggatagaaaaaaacgtactgtgaaccgagaggagctgctgaaacaggcggagtctgtgatgcaggacctcggcagct
cacgggccatgttagaaatccagtatgaaaatgaggttggtacaggtcttgggcctacactggagttttatgcgcttgtatctcaggaactac
agagagctgacttgggtctttggagaggtgaagaagtaactcttagcaatccaaaagggagccaagaagggaccaagtatattcaaaa
cctccagggcctgtttgcgcttccctttggtaggacagcaaagccagctcatatcgcaaaggttaagatgaagtttcgcttcttaggaaaatt
aatggccaaggctatcatggatttcagattggtggaccttccccttggcttacccttttataaatggatgctacggcaagaaacttcactgaca
tcacacgatttgtttgacatcgacccagttgtagccagatcagtttatcacctagaagacattgtcagacagaagaaaagacttgaacaag
ataaatcccagaccaaagagagtctacagtatgcattagaaaccttgactatgaatggctgctcagttgaagatctaggactggatttcact
ctgccagggtttcccaatatcgaactgaagaaaggagggaaggatataccagtcactatccacaatttagaggagtatctaagactggtt
atattctgggcactaaatgaaggcgtttctaggcaatttgattcgttcagagatggatttgaatcagtcttcccactcagtcatcttcagtacttct
acccggaggaactggatcagctcctttgtggcagtaaagcagacacttgggatgcaaagacactgatggaatgctgtaggcctgatcat
ggttatactcatgacagtcgggctgtgaagtttttgtttgagattctcagtagttttgataatgagcagcagaggttatttctccagtttgtgactgg
tagcccaagattgcctgttggaggattccggagtttgaatccacctttgacaattgtccgaaagacgtttgaatcaacagaaaacccagatg
acttcttgccctctgtaatgacttgtgtgaactatcttaagttgccggactattcaagcattgagataatgcgtgaaaaactgttgatagcagca
agagaagggcagcagtcgttccatctttcctga (SEQ ID NO:289)

**Q14669**
MSNRPNNNPGGSLRRSQRNTAGAQPQDDSIGGRSCSSSSAVIVPQPEDPDRANTSERQKTGQV
PKKDNSRGVKRSASPDYNRTNSPSSAKKPKALQHTESPSETNKPHSKSKKRHLDQEQQLKSAQS
PSTSKAHTRKSGATGGSRSQKRKRTESSCVKSGSGSESTGAEERSAKPTKLASKSATSAKAGCS
TITDSSSAASTSSSSSAVASASSTVPPGARVKQGKDQNKARRSRSASSPSPRRSSREKEQSKTG
GSSKFDWAARFSPKVSLPKTKLSLPGSSKSETSKPGPSGLQAKLASLRKSTKKRSESPPAELPSL
RRSTRQKTTGSCASTSRRGSGLGKRGAAEARRQEKMADPESNQEAVNSSAARTDEAPQGAAG
AVGMTTSGESESDDSEMGRLQALLEARGLPPHLFGPLGPRMSQLFHRTIGSGASSKAQQLLQGL
QASDESQQLQAVIEMCQLLVMGNEETLGGFPVKSVVPALITLLQMEHNFDIMNHACRALTYMMEA
LPRSSAVVVDAIPVFLEKLQVIQCIDVAEQALTALEMLSRRHSKAILQAGGLADCLLYLEFFSINAQR
NALAIAANCCQSITPDEFHFVADSLPLLTQRLTHQDKKSVESTCLCFARLVDNFQHEENLLQQVAS
KDLLTNVQQLLVVTPPILSSGMFIMVVRMFSLMCSNCPTLAVQLMKQNIAETLHFLLCGASNGSCQ
EQIDLVPRSPQELYELTSLICELMPCLPKEGIFAVDTMLKKGNAQNTDGAIWQWRDDRGLWHPYN
RIDSRIIEQINEDTGTARAIQRKPNPLANSNTSGYSESKKDDARAQLMKEDPELAKSFIKTLFGVLY
EVYSSSAGPAVRHKCLRAILRIIYFADAELLKDVLKNHAVSSHIASMLSSQDLKIVVGALQMAEILMQ
KLPDIFSVYFRREGVMHQVKHLAESESLLTSPPKACTNGSGSMGSTTSVSSGTATAATHAAADLG
SPSLQHSRDDSLDLSPQGRLSDVLKRKRLPKRGPRRPKYSPPRDDDKVDNQAKSPTTTQSPKSS
FLASLNPKTWGRLSTQSNSNNIEPARTAGGSGLARAASKDTISNNREKIKGWIKEQAHKFVERYFS
SENMDGSNPALNVLQRLCAATEQLNLQVDGGAECLVEIRSIVSESDVSSFEIQHSGFVKQLLLYLT
SKSEKDAVSREIRLKRFLHVFFSSPLPGEEPIGRVEPVGNAPLLALVHKMNNCLSQMEQFPVKVH
DFPSGNGTGGSFSLNRGSQALKFFNTHQLKCQLQRHPDCANVKQWKGGPVKIDPLALVQAIERY
LVVRGYGRVREDDEDSDDDGSDEEIDESLAAQFLNSGNVRHRLQFYIGEHLLPYNMTVYQAVRQ
FSIQAEDERESTDDESNPLGRAGIWTKTHTIWYKPVREDEESNKDCVGGKRGRAQTAPTKTSPR
NAKKHDELWHDGVCPSVSNPLEVYLIPTPPENITFEDPSLDVILLLRVLHAISRYWYYLYDNAMCKE
IIPTSEFINSKLTAKANRQLQDPLVIMTGNIPTWLTELGKTCPFFFPFDTRQMLFYVTAFDRDRAMQ
RLLDTNPEINQSDSQDSRVAPRLDRKKRTVNREELLKQAESVMQDLGSSRAMLEIQYENEVGTGL
GPTLEFYALVSQELQRADLGLWRGEEVTLSNPKGSQEGTKYIQNLQGLFALPFGRTAKPAHIAKV
KMKFRFLGKLMAKAIMDFRLVDLPLGLPFYKWMLRQETSLTSHDLFDIDPVVARSVYHLEDIVRQK
KRLEQDKSQTKESLQYALETLTMNGCSVEDLGLDFTLPGFPNIELKKGGKDIPVTIHNLEEYLRLVI
FWALNEGVSRQFDSFRDGFESVFPLSHLQYFYPEELDQLLCGSKADTWDAKTLMECCRPDHGY
THDSRAVKFLFEILSSFDNEQQRLFLQFVTGSPRLPVGGFRSLNPPLTIVRKTFESTENPDDFLPSV
MTCVNYLKLPDYSSIEIMREKLLIAAREGQQSFHLS (SEQ ID NO:290)

# Figure 131

**Nucleotide:** X63282
**Sequence:**
atgtctcgaaggaagatttcgtccgagtccttcagctccctgggctccgactacctggagaccagcccggaggaggagggggagtgccc
cctgtctaggctctgctggaatggcagccggagcccgcccgggccgctggagcccagcccggccgcagctgccgctgccgccgcccc
ggccccgaccccggctgcttctgccgccgccgccgctgccactgccggggccaggagggtgcagcgccggaggcgggtcaacctgg
actcgctgggcgagagcatcagccgcctgacggcgccctcgcctcagacgatacagcagactctcaagaggacactgcagtattatga
acatcaagttattggttacagggatgcagaaaagaatttccacaatatctctaacagatgctcctatgcagaccactccaacaaagaaga
aattgaagatgtctcaggaattcttcagtgtactgctaatatactcggtttgaagtttgaggaaattcaaaaaagatttggtgaagagttctttaa
tatatgctttcatgagaatgagagagtccttcgagctgtaggtggcactttgcaggactttttaacggctttgatgctttgttggaacacattaga
acttcttttggaaaacaggccactctggagtcaccatctttcctatgcaaagagctccctgaaggtactctcatgctccactacttccaccctc
accatattgtggggtttgcaatgctggggatgattaaggctgcaggaaagaagatctatcggctggatgtggaagtggaacaggttgcaa
atgagaagctatgctctgatgtttcaaacccaggcaattgtagctgtcttactttccttatcaaagaatgtgaaaatactaatatcatgaagaa
ccttccacagggaacctcccaagttcctgcggacctcagaattagcatcaacaccttctgtagagccttccctttccacttgatgtttgatccc
agcatgtcagtccttcagttgggggaaggtctaaggaagcagcttcgatgtgacactcacaaagtgctcaagtttgaggactgcttcgaga
ttgtatctccaaaggttaatgccacctttgaaagggtcctgctgcgactgtctaccccgtttgtgattagaaccaagcctgaggcttctggctct
gaaaataaagacaaggtgatggaagtcaaaggacaaatgatccatgttccagaatcaaattccattttatttttgggctctccatgtgtggac
aagttggatgaactcatgggccgagggctacatctctcagacatccctatccatgatgccacccgagatgtcattttggttggtgagcaggc
aaaggcccaagatgggttgaagaaaaggatggataaattaaaggcaactttagaaagaactcaccaggccctggaagaagagaaa
aagaagacagtggatcttctatattctattttccctggtgatgtagcccagcaattatggcaagggcagcaagtacaggccagaaagtttga
tgatgtcaccatgctcttttcagacattgttggcttcacagccatatgtgcccagtgtactcccatgcaagtaatcagcatgctgaatgaactgt
acaccagatttgaccaccagtgtggatttttggatatttataaggtggaaacaataggtgatgcctactgtgttgcagcagggctccacaga
aaaagcctctgccatgctaaacccattgctctgatggccttgaagatgatggaactttcagaagaggtgctgacacctgatggaagaccg
attcagatgaggataggaattcactcaggctccgtgctggctggagttgttggggtgcgaatgccacgttattgcctgtttggaaataatgtca
cactggcaagcaaattcgagtcgggaagtcaccctcggcgcatcaatgtcagcccaaccacttaccaattattaaaacgagaagaaag
tttcacattcattccgcggtctcgtgaagagcttccagacaactttccaaaggaaattcctgggatctgctatttcctggaggtaaggactggt
ccaaagccaccaaagccttctctttcttcgtcgagaataaaaaaggtttcctacaacatcggcaccatgttcctccgggagacaagcctct
ga  (SEQ ID NO:291)

**P33402**
MSRRKISSESFSSLGSDYLETSPEEEGECPLSRLCWNGSRSPPGPLEPSPAAAAAAAAPAPTPAA
SAAAAAATAGARRVQRRRRVNLDSLGESISRLTAPSPQTIQQTLKRTLQYYEHQVIGYRDAEKNF
HNISNRCSYADHSNKEEIEDVSGILQCTANILGLKFEEIQKRFGEEFFNICFHENERVLRAVGGTLQ
DFFNGFDALLEHIRTSFGKQATLESPSFLCKELPEGTLMLHYFHPHHIVGFAMLGMIKAAGKKIYRL
DVEVEQVANEKLCSDVSNPGNCSCLTFLIKECENTNIMKNLPQGTSQVPADLRISINTFCRAFPFH
LMFDPSMSVLQLGEGLRKQLRCDTHKVLKFEDCFEIVSPKVNATFERVLLRLSTPFVIRTKPEASG
SENKDKVMEVKGQMIHVPESNSILFLGSPCVDKLDELMGRGLHLSDIPIHDATRDVILVGEQAKAQ
DGLKKRMDKLKATLERTHQALEEEKKKTVDLLYSIFPGDVAQQLWQGQQVQARKFDDVTMLFSDI
VGFTAICAQCTPMQVISMLNELYTRFDHQCGFLDIYKVETIGDAYCVAAGLHRKSLCHAKPIALMAL
KMMELSEEVLTPDGRPIQMRIGIHSGSVLAGVVGVRMPRYCLFGNNVTLASKFESGSHPRRINVS
PTTYQLLKREESFTFIPRSREELPDNFPKEIPGICYFLEVRTGPKPPKPSLSSSRIKKVSYNIGTMFL
RETSL  (SEQ ID NO:292)

# Figure 132

**Nucleotide:** AL096881
**Sequence:**
atgagcggcagagtcggcgatctgagccccaggcagaaggaggcattggccaagtttcgggagaatgtccaggatgtgctgccggcc
ctgccgaatccagatgactattttctcctgcgttggctccgagccagaagcttcgacctgcagaagtcggaggccatgctccggaagcatg
tggagttccgaaagcaaaaggacattgacaacatcattagctggcagcctccagaggtgatccaacagtatctgtcaggggtatgtgtg
gctatgacctggatggctgcccagtctggtacgacataattggacctctggatgccaagggtctgctgttctcagcctccaaacaggacctg
ctgaggaccaagatgcgggagtgtgagctgcttctgcaagagtgtgcccaccagaccacaaagttggggaggaaggtggagaccatc
accataatttatgactgcgaggggcttggcctcaagcatctctggaagcctgctgtggaggcctatggagagtttctctgcatgtttgaggaa
aattatcccgaaacactgaagcgtctttttgttgttaaagcccccaaactgtttcctgtggcctataacctcatcaaacccttcctgagtgagga
cactcgtaagaagatcatggtcctgggagcaaattggaaggaggttttactgaaacatatcagccctgaccaggtgcctgtggagtatgg
gggcaccatgactgaccctgatggaaaccccaagtgcaaatccaagatcaactacggggggtgacatccccaggaagtattatgtgcga
gaccaggtgaaacagcagtatgaacacagcgtgcagatttcccgtggctcctcccaccaagtggagtatgagatcctcttccctggctgtg
tcctcaggtggcagtttatgtcagatggagcggatgttggttttgggattttcctgaagaccaagatgggagagaggcagcgggcagggg
agatgacagaggtgctgcccaaccagaggtacaactcccacctggtccctgaagatgggaccctcacctgcagtgatcctggcatctat
gtcctgcggtttgacaacacctacagcttcattcatgccaagaaggtcaatttcactgtggaggtcctgcttccagacaaagcctcagaaga
gaagatgaaacagctgggggcaggcaccccgaaataa (SEQ ID NO:293)

**O76054**
MSGRVGDLSPRQKEALAKFRENVQDVLPALPNPDDYFLLRWLRARSFDLQKSEAMLRKHVEFRK
QKDIDNIISWQPPEVIQQYLSGGMCGYDLDGCPVWYDIIGPLDAKGLLFSASKQDLLRTKMRECEL
LLQECAHQTTKLGRKVETITIIYDCEGLGLKHLWKPAVEAYGEFLCMFEENYPETLKRLFVVKAPKL
FPVAYNLIKPFLSEDTRKKIMVLGANWKEVLLKHISPDQVPVEYGGTMTDPDGNPKCKSKINYGGD
IPRKYYVRDQVKQQYEHSVQISRGSSHQVEYEILFPGCVLRWQFMSDGADVGFGIFLKTKMGER
QRAGEMTEVLPNQRYNSHLVPEDGTLTCSDPGIYVLRFDNTYSFIHAKKVNFTVEVLLPDKASEEK
MKQLGAGTPK (SEQ ID NO:294)

# Figure 133

**Nucleotide:** BC033918
**Sequence:**
atggcgtcaaactcaactaagtctttcctggcagatgccggctatggcgaacaggaactggatgccaactctgcccttatggaattggaca
aaggcctaagatctggcaaacttggtgaacagtgtgaagcagttgttcgctttcccagactttttcagaagtatccattccctattcttatcaatt
ctgcattcctaaagttagctgatgttttcagagttggaaataatttcctgaggctatgtgttcttaaagttacccaacaaagtgagaaacatttgg
agaagattctaaatgtggatgaatttgtgaagagaatttttctgtgattcatagtaatgatcctgtggcaagagccatcaccctccggatgttg
ggaagtctggcatcaataattcctgagaggaagaatgctcatcatagtattcgtcagagtttagattcacatgataatgtagaagttgaagct
gctgtttttgctgctgcaaacttctctgcacagtcaaaggattttgctgtaggaatctgtaacaaaatcagtgaaatgattcaaggtttagcgac
accagtagacttgaagctaaaattgatacccattctacagcacatgcaccatgatgcaatcttggcttccagtgctcgtcagcttttacaaca
gctggtcacatcctatccgtccaccaaaatggtgattgtgtctttgcacactttcactctgcttgcagcgtcatctttggttgatacacctaagca
gattcagcttctgttgcagtatttgaagaatgatcccaggaaggcagtaaagagacttgctattcaagatctgaaattacttgctaataaaac
accacatacttggagtagggagaatattcaggcactttgtgagtgtgccctccagactccttatgacagcttaaaactagggatgttgtctgtc
ctttccacactatcagggaccatcgccatcaaacattacttcagtatagttccaggaaatgtgagttcttctcccagatcttctgatttagtcaaa
ttagcccaagagtgctgttaccataataacaggggcattgcagctcatggagttagagtcctaactaatataactgtttcttgtcaagaaaag
gatcttttggcactggaacaagatgctgtctttggcctggaatccctactggtactttgtagtcaagatgatagtccaggtgctcaggccacttt
aaagattgctctaaactgtatggtgaagttggccaagggcaggccccgtcttagccagtcagtagttgagaccttgttgactcaattgcaca
gtgctcaagacgctgcccggatttgatgtgccattgcctggcagccattgccatgcaactgccggtgctgggtgatgggatgcttggtgac
ctcatggagctgtacaaggtgattggacgatcagccacagacaagcaacaagaacttctggtgagtttggctactgtgatttttgttgcaagt
cagaaggcattgtctgtggaaagtaaggcagtaattaagcagcagcttgaaagtgtctccaatggatggactgtataccgtattgccagac
aggcttccagaatgggtaatcatgacatggccaaagagctttatcagagtttgctgactcaggttgcctcagaacatttctacttctggctaaa
tagtttgaaggagttttcacatgcagaacagtgtctcactgggttgcaagaggaaaattatagttcagcactttcttgcattgctgaatctttaaa
attctatcacaaagggattgcttccttaacagcagctagtacaccactgaatcctttaagctttcagtgtgaatttgtaaaactcaggattgacc
ttttacaagccttctctcaacttatctgtacttgtaatagcctgaagacaagcccaccacctgcaattgccacaacaattgccatgaccttagg
aaatgacctccagaggtgtggtcgcatctccaatcagatgaaacagtccatggaagaatttcgaagccttgcttctcgatatggagatcttta
ccaggcatcttttgatgctgactcagcaactttgaggaatgttgaactacagcagcagagctgtttactgatatctcatgcaatagaagccct
gatttggatccagaatcagcaagtttccaggaatatggatctactggaacagcccatgctgatagtgaatatgaaagaagaatgatgtct
gtatataatcatgtcttggaggaggtagaatcactcaatcggaaatataccccctgtttcttatatgcacacagcatgcctctgcaatgccatca
ttgctttgctgaaagttcccctttctttccagagatattttttccagaaactacagtctaccagcatcaagcttgctctgtcaccatcgccccgga
atcctgcagagcccattgctgtccagaataaccagcagctggcgctaaaggtagagggagtggttcagcacggatctaaaccaggact
cttccgcaaaattcagtctgtctgtctgaatgtttcttccacactgcagagtaaatctggacaagactacaagatacccattgacaacatgac
caatgagatggagcaaagggttgaacctcataatgattacttcagtactcaatttctgttgaactttgctatccttggaacacacaacattaca
gtggaatcttctgtgaaagatgccaatggtatagtatggaagactggtcccagaactaccatatttgtaaaatccctggaagacccttattcc
cagcaaattcgcttacaacagcagcaagcccagcagccattacagcagcagcagcaacgcaatgcctacacacggtttta (SEQ
ID NO:295)

**Q8N4K7; AAH33918**
MASNSTKSFLADAGYGEQELDANSALMELDKGLRSGKLGEQCEAVVRFPRLFQKYPFPILINSAFL
KLADVFRVGNNFLRLCVLKVTQQSEKHLEKILNVDEFVKRIFSVIHSNDPVARAITLRMLGSLASIIP
ERKNAHHSIRQSLDSHDNVEVEAAVFAAANFSAQSKDFAVGICNKISEMIQGLATPVDLKLKLIPIL
QHMHHDAILASSARQLLQQLVTSYPSTKMVIVSLHTFTLLAASSLVDTPKQIQLLLQYLKNDPRKAV
KRLAIQDLKLLANKTPHTWSRENIQALCECALQTPYDSLKLGMLSVLSTLSGTIAIKHYFSIVPGNVS
SSPRSSDLVKLAQECCYHNNRGIAAHGVRVLTNITVSCQEKDLLALEQDAVFGLESLLVLCSQDDS
PGAQATLKIALNCMVKLAKGRPRLSQSVVETLLTQLHSAQDAARILMCHCLAAIAMQLPVLGDGML
GDLMELYKVIGRSATDKQQELLVSLATVIFVASQKALSVESKAVIKQQLESVSNGWTVYRIARQAS
RMGNHDMAKELYQSLLTQVASEHFYFWLNSLKEFSHAEQCLTGLQEENYSSALSCIAESLKFYHK
GIASLTAASTPLNPLSFQCEFVKLRIDLLQAFSQLICTCNSLKTSPPPAIATTIAMTLGNDLQRCGRIS
NQMKQSMEEFRSLASRYGDLYQASFDADSATLRNVELQQQSCLLISHAIEALILDPESASFQEYGS
TGTAHADSEYERRMMSVYNHVLEEVESLNRKYTPVSYMHTACLCNAIIALLKVPLSFQRYFFQKL
QSTSIKLALSPSPRNPAEPIAVQNNQQLALKVEGVVQHGSKPGLFRKIQSVCLNVSSTLQSKSGQ
DYKIPIDNMTNEMEQRVEPHNDYFSTQFLLNFAILGTHNITVESSVKDANGIVWKTGPRTTIFVKSL
EDPYSQQIRLQQQQAQQPLQQQQQRNAYTRF (SEQ ID NO:296)

# Figure 134

**Nucleotide:** AB002366
**Sequence:**
agctaatcaatgaatacaaagaggaccctaaactactgtcaatggcatattcagctgttggaaaactctccagtcggatgccacatttattc
actaaggatatagcgcttgtgcagcagctttttgaagccctttgcaaggaagagcctgagactcgacttgctattcaagaagctttatctatg
atggttggagcgtatagtactttggaaggggcacagcgaactctcatggaggcacttgtggcttcgtacttaataaagcctgaagttcaagtt
cgacaagtggctgtgaaatttgccagtacggtgtttccctcagatcatatcccttccagatatttgctgctactggctgcaggagatccacgtg
aagaagttcatggagaagcacaacgcgtattaaggtgtcttccaggtagaaacagaaaagaaagtacttctgagcagatgccttccttcc
cagaaatggtttattacatccaagaaaaggcttctcatcgaatgaaaactccagtcaagtacatgaccgggaccactgtccttccatttaac
ccagcagcctttggagagatcgttctgtacttgcgcatgtgccttgcgcacagtgcgggggtggtgcccacctctcagagtttggctgatatg
caggatcatgccccagccattgggcgctacatacggactttaatgtcaagcgggcagatggcaccctcatcatctaacaagagtgggga
gactaaccctgtccagatctacattggcctgcttcagcagctgttagcaggtgttggaggtttgccggttatgtactgtctattggaagctgtgtc
agtgtatccagaaaagctggctaccaaatttgtagacaaaacagaatggataaagagtctgatgaataacagtaaagaagaaatgcgc
gaactggcagcgttgttttattctgtagtggtatcaacagtgtcggggaatgagttgaaatcaatgatagaacagcttataaagactacaaa
agacaatcacagcccggagatacagcatggatccttgcttgcattgggattcacggtgggaaggtatttggctaaaaagaaaatgagaat
gtcagagcaacaagacctggagagaaatgctgacaccctccctgatcaagaggaactcattcagagtgctacagaaacaataggctc
atttttggacagtacatcacccctcctggcaattgctgcctgcacagccctgggtgaaattggcagaaatggtccacttccaatccccagtg
agggatctggctttaccaaattgcatcttgtagaaagcttactaagtagaataccttccagtaaagaaacaaataagatgaaagaacgag
caatccaaacactgggatattttccagttggggatggagattttcctcaccagaaactcctcttgcaaggtctgatggattctgtggaggcca
agcagatagaacttcagttcactattggcgaagccattaccagtgctgcaataggaactagttctgtggctgcccgagatgcctggcaaat
gactgaagaggaatatactccacctgctggagccaaagtgaatgatgtggttccatgggtgttggatgtgattttaaataaacatatcatca
gccccaacccacacgtgaggcaagcagcctgcatctggctcctttcccttgtcaggaagctaagtacccacaaagaagtgaagtctcatc
ttaaagaaattcaaagtgcatttgtttcagttctatcagaaaatgatgaacttagccaagatgttgcatcaaagggccttgggttggtttatgaa
ctaggcaatgaacaagatcaacaggaattggtttctacacttgtggaaacacttatgactggcaaaagagttaaacatgaagtttctggag
agacagtggtatttcaaggggggagctcttggcaaaacaccagatggtcagggcctttctacttacaaggaactttgttctctggcaagtgat
cttagccagccagatctggtgtataaatttatgaatttagccaaccatcatgcaatgtggaactctaggaagggtgctgcttttggttttaatgta
attgctaccagagctggagagcagctggctccttttctgcctcagctagttcctcgactttatcgttaccagtttgatcccaaccttggcattcga
caggccatgacaagtatttggaatgcgttggtcactgacaaatccatggtggataaatatttgaaagaaattcttcaagatttggttaagaac
cttacaagcaatatgtggcgagttcgagaatccagctgtttagctttgaatgatttattgagaggaagacccttagatgacatcattgataaac
ttccagaaatttgggaaacgctttttagagtacaagatgatatcaaggaatctgtacgaaaagcggcagaactagctctgaaaactctgag
caaggtctgtgtgaaaatgtgtgaccctgccaaaggagcagctggccagagaaccatcgctgcccttctgccttgccttctggacaaagg
aatgatgagcaccgtgacggaagttcgagccctcagcattaacacccttgtgaagatcagcaaaagtgcaggagccatgttgaaaccg
catgcaccaaaactcattccagctctgctagagtccttaagtgtattggagccccaagttctcaattatttgagcctccgggcgacagagca
agaaaaggctgcgatggatagtgctcggcttagtgctgccaaatcttctccaatgatggaaacaatcaacatgtgcctgcaataccttgatg
tgtcagtgctgggcgagctagttcctaggttgtgtgaactgatcagaagtggtgtaggtcttggaactaagggtggctgtgccagtgtcattgt
gtcattaactactcagtgtcctcaggacctaacaccttactcaggtaaacttatgagtgctttgctgagtggcctgacagatcggaacagtgt
gattcagaaatcttgtgcatttgctatgggccatttagttcggacctcacgggatagcagcactgaaaaactcctgcagaagctcaatgggt
ggtatatggagaaagaagaacctatctacaagacctcttgtgctttgactattcatgctattggacgatacagccctgatgtattaaagaatc
atgcaaaagaagtcctgcctctggcatttttaggcatgcatgaaattgctgatgaggagaaatccgaaaaagaagaatgtaatttatggac
cgaagtgtggcaggaaaacgtacctggatcctttggtggcattcgattatacctgcaggagttaattactattacccagaaggctttgcagtc
tcagtcctggaaaatgaaagcccagggtgcaattgccatggcatcaattgcaaaacagactagctctctagtacctccatatctcggaatg
atactgaccgcattgctgcaaggcctggctggaagaacgtgggcaggaaaggaggagctattgaaagccattgcctgtgtggtgacag
cttgcagtgcagagctggaaaagtctgtgcccaatcaacccagcacaaatgaaattcttcaagctgttctgaaggaatgtagcaaagag
aatgtcaaatacaagattgtagcaatcagctgtgcagctgatatcttgaaggccaccaaagaggacagattccaggagttctctaacattg
tcatacctctcatcaagaagaactcacttgaaagcagtggggtccggacaaccaaaaatgaagaggagaatgaaaaggaaaaggag
ctccagctggaatatctgctgggtgcctttgaaagcctgggcaaagcctggccgcgaaacgcggagacccaacgttgttatcgtcagga
gctgtgcaaactgatgtgtgaacggctaaaactcagcacgtggaaagtgcagctaggagtcctgcaatcaatgaatgcctttttcagggg
ttaatgcttttggaagaagaacatgccgatcctgaggctttggctgaaattctgcttgaaacttgtaaatcaatcacatattctttagaaaataa
gacctactcatctgtgagaacagaagctttatctgtgatagaattgctgcttaaaaaacttgaagaatctaaacagtgggaatgtttgacatct
gaatgcagagtgctcctaattgagtctttagctactatggagccagacagcagacctgaactgcaggagaaagcagcgttactgaagaa
aacacttgaaaatctggaataa  (SEQ ID NO:297)

**O15074**
LINEYKEDPKLLSMAYSAVGKLSSRMPHLFTKDIALVQQLFEALCKEEPETRLAIQEALSMMVGAY
STLEGAQRTLMEALVASYLIKPEVQVRQVAVKFASTVFPSDHIPSRYLLLLAAGDPREEVHGEAQR
VLRCLPGRNRKESTSEQMPSFPEMVYYIQEKASHRMKTPVKYMTGTTVLPFNPAAFGEIVLYLRM
CLAHSAGVVPTSQSLADMQDHAPAIGRYIRTLMSSGQMAPSSSNKSGETNPVQIYIGLLQQLLAG
VGGLPVMYCLLEAVSVYPEKLATKFVDKTEWIKSLMNNSKEEMRELAALFYSVVVSTVSGNELKS
MIEQLIKTTKDNHSPEIQHGSLLALGFTVGRYLAKKKMRMSEQQDLERNADTLPDQEELIQSATETI
GSFLDSTSPLLAIAACTALGEIGRNGPLPIPSEGSGFTKLHLVESLLSRIPSSKETNKMKERAIQTLG
YFPVGDGDFPHQKLLLQGLMDSVEAKQIELQFTIGEAITSAAIGTSSVAARDAWQMTEEEYTPPAG
AKVNDVVPWVLDVILNKHIISPNPHVRQAACIWLLSLVRKLSTHKEVKSHLKEIQSAFVSVLSENDE
LSQDVASKGLGLVYELGNEQDQQELVSTLVETLMTGKRVKHEVSGETVVFQGGALGKTPDGQGL
STYKELCSLASDLSQPDLVYKFMNLANHHAMWNSRKGAAFGFNVIATRAGEQLAPFLPQLVPRLY
RYQFDPNLGIRQAMTSIWNALVTDKSMVDKYLKEILQDLVKNLTSNMWRVRESSCLALNDLLRGR
PLDDIIDKLPEIWETLFRVQDDIKESVRKAAELALKTLSKVCVKMCDPAKGAAGQRTIAALLPCLLDK
GMMSTVTEVRALSINTLVKISKSAGAMLKPHAPKLIPALLESLSVLEPQVLNYLSLRATEQEKAAMD
SARLSAAKSSPMMETINMCLQYLDVSVLGELVPRLCELIRSGVGLGTKGGCASVIVSLTTQCPQDL
TPYSGKLMSALLSGLTDRNSVIQKSCAFAMGHLVRTSRDSSTEKLLQKLNGWYMEKEEPIYKTSC
ALTIHAIGRYSPDVLKNHAKEVLPLAFLGMHEIADEEKSEKEECNLWTEVWQENVPGSFGGIRLYL
QELITITQKALQSQSWKMKAQGAIAMASIAKQTSSLVPPYLGMILTALLQGLAGRTWAGKEELLKAI
ACVVTACSAELEKSVPNQPSTNEILQAVLKECSKENVKYKIVAISCAADILKATKEDRFQEFSNIVIP
LIKKNSLESSGVRTTKNEEENEKEKELQLEYLLGAFESLGKAWPRNAETQRCYRQELCKLMCERL
KLSTWKVQLGVLQSMNAFFQGLMLLEEEHADPEALAEILLETCKSITYSLENKTYSSVRTEALSVIE
LLLKKLEESKQWECLTSECRVLLIESLATMEPDSRPELQEKAALLKKTLENLE (SEQ ID NO:298)

# Figure 135

**Nucleotide:** AB028983
**Sequence:**
ggagcctaccataagcacctcatggaactcgctcttcagcaaacatatcaggacacctgtaattgcatcaagtcgcggatcaagttggaa
tttgaaaaacgtcaacaggagcggcttctgctctccctgctgccggcccacatcgccatggagatgaaagcggagatcatccagaggct
gcagggccccaaggcgggccagatggagaacacaaataacttccacaacctgtatgtgaagcggcatacaaacgtgagcatcttata
cgctgacatcgttggctttacccggctggcaagtgactgctccccgggagaactagtccacatgctgaatgagctctttggaaagtttgatc
aaattgcaaaggagaatgaatgcatgagaattaaaattttaggagactgctactactgtgtatctggactccctatatctctccctaaccatg
ccaagaactgtgtgaaaatggggctggacatgtgtgaagccataaagaaagtgagggatgctactggagttgatatcaacatgcgcgtg
ggcgtgcattctgggaatgtcctgtgtggcgtgattggtctgcagaagtggcaatatgatgtgtggtcacatgatgtgaccttggccaaccac
atggaagctggaggggtccctggacgtgttcacatttcttctgtcaccctggagcacttgaatggcgcttataaagtggaggagggagatg
gtgacattagggacccatatttaaaacagcacctggtgaaaacctactttgtgatcaaccccaagggagaacgacggagcccccagca
tctcttcagacctcgccacacccttgatggagccaaaatgagggcctcggtccgcatgacccggtacttggagtcctgggggcagcca
agccctttgcacacctacatcacagggacagcatgaccacagagaacggcaagatcagcaccacggatgtacccatgggtcagcata
attttcaaaatcgcaccttaagaaccaagtcacaaaagaagagatttgaagaagaattgaatgaaaggatgattcaagcaattgatggg
attaatgcacagaagcaatggctcaagtctgaagacattcagagaatctcactgcttttctataacaaagtactagaaaaagagtaccgg
gccacggcactgccagcgttcaagtattatgtgacttgtgcctgtctcatattcttctgcatcttcattgtgcagattctcgtgctgccaaaaacgt
ctgtcctgggcatctcctttggggctgcgtttctcttgctggccttcatcctcttcgtctgctttgctggacagcttctgcaatgcagcaaaaagc
ctctcccctgctcatgtggcttttgaagtcctcgggcatcattgccaaccgcccctggccacggatctctctcacgatcatcaccacagccat
catattaatgatggccgtgttcaacatgtttttcctgagtgactcagaggaaacaatccctccaactgccaacacaacaaacacaagcttttc
agcctcaaataatcaggtggcgattctgcgtgcgcagaatttatttttcctcccgtactttatctacagctgcattctgggactgatatcctgttcc
gtgttcctgcgggtaaactatgagctgaagatgttgatcatgatggtggccttggtgggctacaacaccatcctactccacacccacgccca
cgtcctgggcgactacagccaggtcttatttgagagaccaggcatttggaaagacctgaagaccatgggctctgtgtctctctctatattcttc
atcacactgcttgttctgggtagacagaatgaatattactgtaggttagacttcttatggaagaacaaattcaaaaaagagcgggaggaga
tagagaccatggagaacctgaaccgcgtgctgctggagaacgtgcttcccgcgcacgtggctgagcacttcctggccaggagcctgaa
gaatgaggagctataccaccagtcctatgactgcgtctgtgtcatgtttgcctccattccggatttcaaagaattttatacagaatccgacgtg
aacaaggagggcttggaatgccttcggctcctgaacgagatcatcgctgactttgatgatcttctttccaagccaaaattcagtggagttgaa
aagattaagaccattggcagcacatacatggcagcaacaggtctgagcgctgtgcccagccaggagcactcccaggagcccgagcg
gcagtacatgcacattggcaccatggtggagtttgctttttgccctggtagggaagctggatgccatcaacaagcactccttcaacgacttca
aattgcgagtgggtattaaccatggacctgtgatagctggtgtgattggagctcagaagccacaatatgatatctggggcaacactgtcaat
gtggccagtaggatggacagcaccggagtcctggacaaaatacaggttaccgaggagacgagcctcgtcctgcagaccctcggatac
acgtgcacctgtcgaggaataatcaacgtgaaaggaaagggggacctgaagacgtactttgtaaacacagaaatgtcaaggtccctttc
ccagagcaacgtggcatcctga (SEQ ID NO:299)

**Q08462**
GAYHKHLMELALQQTYQDTCNCIKSRIKLEFEKRQQERLLLSLLPAHIAMEMKAEIIQRLQGPKAG
QMENTNNFHNLYVKRHTNVSILYADIVGFTRLASDCSPGELVHMLNELFGKFDQIAKENECMRIKIL
GDCYYCVSGLPISLPNHAKNCVKMGLDMCEAIKKVRDATGVDINMRVGVHSGNVLCGVIGLQKW
QYDVWSHDVTLANHMEAGGVPGRVHISSVTLEHLNGAYKVEEGDGDIRDPYLKQHLVKTYFVINP
KGERRSPQHLFRPRHTLDGAKMRASVRMTRYLESWGAAKPFAHLHHRDSMTTENGKISTTDVP
MGQHNFQNRTLRTKSQKKRFEEELNERMIQAIDGINAQKQWLKSEDIQRISLLFYNKVLEKEYRAT
ALPAFKYYVTCACLIFFCIFIVQILVLPKTSVLGISFGAAFLLLAFILFVCFAGQLLQCSKKASPLLMWL
LKSSGIIANRPWPRISLTIITTAIILMMAVFNMFFLSDSEETIPPTANTTNTSFSASNNQVAILRAQNLF
FLPYFIYSCILGLISCSVFLRVNYELKMLIMMVALVGYNTILLHTHAHVLGDYSQVLFERPGIWKDLK
TMGSVSLSIFFITLLVLGRQNEYYCRLDFLWKNKFKKEREEIETMENLNRVLLENVLPAHVAEHFLA
RSLKNEELYHQSYDCVCVMFASIPDFKEFYTESDVNKEGLECLRLLNEIIADFDDLLSKPKFSGVEK
IKTIGSTYMAATGLSAVPSQEHSQEPERQYMHIGTMVEFAFALVGKLDAINKHSFNDFKLRVGINH
GPVIAGVIGAQKPQYDIWGNTVNVASRMDSTGVLDKIQVTEETSLVLQTLGYTCTCRGIINVKGKG
DLKTYFVNTEMSRSLSQSNVAS (SEQ ID NO:300)

**Nucleotide:** D25538
**Sequence:**
atgccagccaaggggcgctacttcctcaacgagggcgaggagggccctgaccaagatgcgctctacgagaagtaccagctcaccag
ccagcatgggccgctgctgctcacgctcctgctggtggccgccactgcctgcgtggccctcatcatcattgccttcagccagggggacccc
tccagacaccaggccattctgggcatggcgttcctggtgctggcggtgtttgcggccctctctgtgctgatgtacgtcgagtgtctcctgcggc
gctggctcagggccttggcgctgctcacctgggcctgcttggtggcgctgggctatgtgctggtgttcgacgcatggacaaaggcggcctgt
gcgtgggagcaggtgcccttcttcctgttcattgtcttcgtggtgtacacactactgcccttcagcatgcggggcgctgtcgccgttggggccg
tctccactgcctcccacctcctggtgctcggttctttgatgggaggcttcacgacacccagtgtccgggtggggctgcagctgctggccaac
gcagtcatcttcctgtgtgggaacctgacaggcgccttccacaagcaccaaatgcaggatgcgtcccgggacctcttcacctacactgtga
agtgcatccagatccgccggaagctgcgcatcgagaagcgccagcaggagaacctgctgctgtcagtgcttccggcccacatctccatg
ggcatgaagctggccatcatcgaacggctcaaggagcatggtgaccgtcgctgcatgcctgacaacaacttccacagcctctacgtcaa
gaggcaccagaatgtcagcatcctctatgcggacatcgtgggcttcacgcagctggccagcgactgttctcccaaggagctggtggtggt
gctgaatgagctctttggcaagttcgaccagatcgccaaggccaacgagtgcatgcgaatcaagatcctcggcgactgctactactgtgt
atcgggcctgcccgtgtcgctgcctacccacgcccggaactgcgtgaagatggggctggacatgtgccaggccatcaagcaggtgcgg
gaggccacgggcgtggacatcaacatgcgtgtgggcatacactcggggaatgtgctgtgcggggtcatcggctgcgcaagtggcagt
atgacgtgtggtcccacgacgtgtccctggccaaccggatggaggcagccggagtacccggccgggtgcacatcacggaggccacg
ctaaagcacctggacaaggcgtacgaggtggaggatgggcacgggcagcagcgggacccctacctcaaggagatgaacatccgca
cctacctggtcatcgacccccggagccagcagccaccccccgcccagccaacacctccccaggcccaaggggggacgcggccctgaa
gatgcgggcgtcagtgcgcatgacccggtacctcgagtcctggggggcggcacggccctttgcacatctcaaccaccgtgagagcgtg
agcagtggtgagacccacgtccccaacgggcggaggcctaagagcgttccccagcgccaccgccggaccccagacagaagcatgt
cccccaagggggcggtcggaggatgactcgtacgatgacgagatgctgtcagccattgaggggctcagctccacgaggccctgctgctc
caagtccgatgacttctacacctttgggtccatcttcctggagaagggctttgagcgcgagtaccgcctggcacccatcccccgggcccgc
cacgactttgcctgcgccagcctgatcttcgtctgcatcctgctcgtccatgtcctgctcatgcccaggacggcggcactgggtgtgtccttcg
ggctggtggcctgtgtactggggctggtgctgggcctgtgctttgccaccaagttctcgaggtgctgcccagctcgggggacgctctgcact
atctctgagagggtggagacacagcccctgctgaggctgaccctggccgtcctgaccatcggcagcctgctcactgtggccatcatcaac
ctgcccctgatgcctttccaagttccagagctgcctgttggcaatgagacaggcctactggccgcgagcagcaagacaagagccctgtgt
gagcccctcccgtactacacctgcagctgtgtcctgggcttcatcgcctgctcggtcttcctgaggatgagcctggagccaaaggttgtgctg
ctgacagtggccctggtggcctacctggtgctcttcaacctctccccatgctggcagtgggactgctgcggccaaggcctgggcaacctca
ccaagcccaacggcaccaccagtggcacccctagctgttcctggaaggacctgaagaccatgaccaatttctacctggtcctgttctacat
caccctgcttacactctccagacagattgactattactgccgcttggactgcctatggaagaagaagttcaagaaggagcacgaggagttt
gagaccatggagaacgtgaaccgccttcttctggagaacgtcctgccagcccacgtggctgcccactttatcggtgacaagttaaacgag
gactggtaccatcagtcctatgactgcgtctgtgtcatgtttgcctccgtgccggacttcaaagtgttctacacagagtgcgatgtcaacaaa
gaagggctggagtgcctacgcctgctcaatgagatcattgccgacttcgacgagctcctactgaagcccaagttcagcggcgtggagaa
gatcaagaccatcggcagcacgtacatggcagctgcagggctcagcgtcgcctcagggcacgagaaccaggagctggagcggcag
catgcccacattggtgtcatggtggagttcagcatcgccctgatgagtaagctggacggcatcaacaggcactccttcaactccttccgcct
ccgcgtcggcataaaccatggcctgtgattgctggagtgattggggcccgaaaacctcagtatgacatctggggaaacactgtcaatgt
ggccagccgaatggaaagcactggagaacttgggaaaatccaggttaccgaggagacctgcaccatcctccagggcctcgggtactct
tgtgaatgccgtggcctgatcaacgtcaaaggcaaaggcgagctgaggacttactttgtctgtacggacactgccaagtttcaggggctgg
ggctgaactga (SEQ ID NO:301)

**P51828**
MPAKGRYFLNEGEEGPDQDALYEKYQLTSQHGPLLLTLLLVAATACVALIIIAFSQGDPSRHQAILG
MAFLVLAVFAALSVLMYVECLLRRWLRALALLTWACLVALGYVLVFDAWTKAACAWEQVPFFLFIV
FVVYTLLPFSMRGAVAVGAVSTASHLLVLGSLMGGFTTPSVRVGLQLLANAVIFLCGNLTGAFHKH
QMQDASRDLFTYTVKCIQIRRKLRIEKRQQENLLLSVLPAHISMGMKLAIIERLKEHGDRRCMPDN
NFHSLYVKRHQNVSILYADIVGFTQLASDCSPKELVVVLNELFGKFDQIAKANECMRIKILGDCYYC
VSGLPVSLPTHARNCVKMGLDMCQAIKQVREATGVDINMRVGIHSGNVLCGVIGLRKWQYDVWS
HDVSLANRMEAAGVPGRVHITEATLKHLDKAYEVEDGHGQQRDPYLKEMNIRTYLVIDPRSQQPP
PPSQHLPRPKGDAALKMRASVRMTRYLESWGAARPFAHLNHRESVSSGETHVPNGRRPKSVPQ
RHRRTPDRSMSPKGRSEDDSYDDEMLSAIEGLSSTRPCCSKSDDFYTFGSIFLEKGFEREYRLAP
IPRARHDFACASLIFVCILLVHVLLMPRTAALGVSFGLVACVLGLVLGLCFATKFSRCCPARGTLCTI
SERVETQPLLRLTLAVLTIGSLLTVAIINLPLMPFQVPELPVGNETGLLAASSKTRALCEPLPYYTCS
CVLGFIACSVFLRMSLEPKVVLLTVALVAYLVLFNLSPCWQWDCCGQGLGNLTKPNGTTSGTPSC
SWKDLKTMTNFYLVLFYITLLTLSRQIDYYCRLDCLWKKKFKKEHEEFETMENVNRLLLENVLPAH
VAAHFIGDKLNEDWYHQSYDCVCVMFASVPDFKVFYTECDVNKEGLECLRLLNEIIADFDELLLKP
KFSGVEKIKTIGSTYMAAAGLSVASGHENQELERQHAHIGVMEFSIALMSKLDGINRHSFNSFRL

RVGINHGPVIAGVIGARKPQYDIWGNTVNVASRMESTGELGKIQVTEETCTILQGLGYSCECRGLI NVKGKGELRTYFVCTDTAKFQGLGLN (SEQ ID NO:302)

# Figure 136

**Nucleotide:** AJ404857
**Sequence:**
atggcggcgtctggggaaccccagaggcagtggcaagaggaggtggcggcggtggtagtggtgggctcctgcatgaccgacctggtc
agtcttacttctcgtttgccaaaaactggagaaaccatccatggacataagttttttattggctttggagggaaaggtgccaaccagtgtgtcc
aagctgctcggcttggagcaatgacgtccatggtgtgtaaggttggcaaagattcttttggcaatgattatatagaaaacttaaaacagaat
gatatttctacagaatttacatatcagactaaagatgctgctacaggaactgcttctataattgtcaataatgaaggccagaatatcattgtcat
agtggctggagcaaatttacttttgaatacggaggatctgagggcagcagccaatgtcattagcagagccaaagtcatggtctgccagct
cgaaataactccagcaacttctttggaagccctaacaatggcccgcaggagtggagtgaaaaccttgttcaatccagcccctgccattgct
gacctggatccccagttctacaccctctcagatgtgttctgctgcaatgaaagtgaggctgagattttaactggcctcacggtgggcagcgct
gcagatgctggggaggctgcattagtgctcttgaaaaggggctgccaggtggtaatcattaccttaggggctgaaggatgtgtggtgctgtc
acagacagaacctgagccaaagcacattcccacagagaaagtcaaggctgtggataccacgggtgctggtgacagctttgtgggagct
ctggccttctacctggcttactatccaaatctgtccttggaagacatgctcaacagatccaatttcattgcagcagtcagtgtccaggctgcag
gaacacagtcatcttacccttacaaaaaagaccttccgcttactctgttttga (SEQ ID NO:303)

**Q9H477**
MAASGEPQRQWQEEVAAVVVVGSCMTDLVSLTSRLPKTGETIHGHKFFIGFGGKGANQCVQAA
RLGAMTSMVCKVGKDSFGNDYIENLKQNDISTEFTYQTKDAATGTASIIVNNEGQNIIVIVAGANLLL
NTEDLRAAANVISRAKVMVCQLEITPATSLEALTMARRSGVKTLFNPAPAIADLDPQFYTLSDVFCC
NESEAEILTGLTVGSAADAGEAALVLLKRGCQVVIITLGAEGCVVLSQTEPEPKHIPTEKVKAVDTT
GAGDSFVGALAFYLAYYPNLSLEDMLNRSNFIAAVSVQAAGTQSSYPYKKDLPLTLF (SEQ ID
NO:304)

# Figure 137

**Nucleotide:** D89675
**Sequence:**
atgcttttgcgaagtgcaggaaaattaaatgtgggcaccaagaaagaggatggtgagagtacagcccccaccccccgtccaaaggtctt
gcgttgtaaatgccaccaccattgtccagaagactcagtcaacaatatttgcagcacagacggatattgtttcacgatgatagaagaggat
gactctgggttgcctgtggtcacttctggttgcctaggactagaaggctcagattttcagtgtcgggacactcccattcctcatcaaagaagat
caattgaatgctgcacagaaaggaacgaatgtaataaagacctacaccctacactgcctccattgaaaaacagagattttgttgatggac
ctatacaccacagggctttacttatatctgtgactgtctgtagtttgctcttggtccttatcatattattttgttacttccggtataaaagacaagaaa
ccagacctcgatacagcattgggttagaacaggatgaaacttacattcctcctggagaatccctgagagacttaattgagcagtctcagag
ctcaggaagtggatcaggcctccctctgctggtccaaaggactatagctaagcagattcagatggtgaaacagattggaaaaggtcgct
atggggaagtttggatgggaaagtggcgtggcgaaaaggtagctgtgaaagtgttcttcaccacagaggaagccagctggttcagaga
gacagaaatatatcagacagtgttgatgaggcatgaaaacattttgggtttcattgctgcagatatcaaagggacagggtcctggacccag
ttgtacctaatcacagactatcatgaaaatggttccctttatgattatctgaagtccaccaccctagacgctaaatcaatgctgaagttagcct
actcttctgtcagtggcttatgtcatttacacacagaaatctttagtactcaaggcaaaccagcaattgcccatcgagatctgaaaagtaaaa
acattctggtgaagaaaaatggaacttgctgtattgctgacctgggcctggctgttaaatttattagtgatacaaatgaagttgacataccacc
taacactcgagttggcaccaaacgctatatgcctccagaagtgttggacgagagcttgaacagaaatcacttccagtcttacatcatggct
gacatgtatagttttggcctcatcctttgggaggttgctaggagatgtgtatcaggaggtatagtggaagaataccagcttccttatcatgacct
agtgcccagtgacccctcttatgaggacatgagggagattgtgtgcatcaagaagttacgcccctcattcccaaaccggtggagcagtga
tgagtgtctaaggcagatgggaaaactcatgacagaatgctgggctcacaatcctgcatcaaggctgacagccctgcgggttaagaaa
acacttgccaaaatgtcagagtcccaggacattaaactctga (SEQ ID NO:305)

**O00238**
MLLRSAGKLNVGTKKEDGESTAPTPRPKVLRCKCHHHCPEDSVNNICSTDGYCFTMIEEDDSGLP
VVTSGCLGLEGSDFQCRDTPIPHQRRSIECCTERNECNKDLHPTLPPLKNRDFVDGPIHHRALLIS
VTVCSLLLVLIILFCYFRYKRQETRPRYSIGLEQDETYIPPGESLRDLIEQSQSSGSGSGLPLLVQRT
IAKQIQMVKQIGKGRYGEVWMGKWRGEKVAVKVFFTTEEASWFRETEIYQTVLMRHENILGFIAA
DIKGTGSWTQLYLITDYHENGSLYDYLKSTTLDAKSMLKLAYSSVSGLCHLHTEIFSTQGKPAIAHR
DLKSKNILVKKNGTCCIADLGLAVKFISDTNEVDIPPNTRVGTKRYMPPEVLDESLNRNHFQSYIMA
DMYSFGLILWEVARRCVSGGIVEEYQLPYHDLVPSDPSYEDMREIVCIKKLRPSFPNRWSSDECL
RQMGKLMTECWAHNPASRLTALRVKKTLAKMSESQDIKL (SEQ ID NO:306)

**Nucleotide:** Z22535
**Sequence:**
atgactcagctatacatttacatcagattattgggagcctatttgttcatcatttctcgtgttcaaggacagaatctggatagtatgcttcatggca
ctgggatgaaatcagactccgaccagaaaaagtcagaaaatggagtaaccttagcaccagaggataccttgcctttttaaagtgctattg
ctcagggcactgtccagatgatgctattaataacacatgcataactaatggacattgctttgccatcatagaagaagatgaccagggaga
aaccacattagcttcagggtgtatgaaatatgaaggatctgattttcagtgcaaagattctccaaaagcccagctacgccggacaataga
atgttgtcggaccaatttatgtaaccagtatttgcaaccccacactgccccctgttgtcataggtccgtttttgatggcagcattcgatggctggtt
ttgctcatttctatggctgtctgcataattgctatgatcatcttctccagctgcttttgttacaaacattattgcaagagcatctcaagcagacgtcg
ttacaatcgtgatttggaacaggatgaagcatttattccagttggagaatcactaaaagaccttattgaccagtcacaaagttctggtagtgg
gtctggactacctttattggttcagcgaactattgccaaacagattcagatggtccggcaagttggtaaaggccgatatggagaagtatgga
tgggcaaatggcgtggcgaaaaagtggcggtgaaagtattctttaccactgaagaagccagctggtttcgagaaacagaaatctaccaa
actgtgctaatgcgccatgaaaacatacttggtttcatagcggcagacattaaaggtacaggttcctggactcagctctatttgattactgatt
accatgaaaatggatctctctatgacttcctgaaatgtgctacactggacaccagagccctgcttaaattggcttattcagctgcctgtggtct
gtgccacctgcacacagaaatttatggcacccaaggaaagcccgcaattgctcatcgagacctaaagagcaaaaacatcctcatcaag
aaaaatgggagttgctgcattgctgacctgggccttgctgttaaattcaacagtgacacaaatgaagttgatgtgcccttgaataccagggt
gggcaccaaacgctacatggctcccgaagtgctggacgaaagcctgaacaaaaaccacttccagccctacatcatggctgacatctac
agcttcggcctaatcatttgggagatggctcgtcgttgtatcacaggagggatcgtggaagaataccaattgccatattacaacatggtacc
gagtgatccgtcatacgaagatatgcgtgaggttgtgtgtgtcaaacgtttgcggccaattgtgtctaatcggtggaacagtgatgaatgtct
acgagcagttttgaagctaatgtcagaatgctgggcccacaatccagcctccagactcacagcattgagaattaagaagacgcttgcca
agatggttgaatcccaagatgtaaaaatctga (SEQ ID NO:307)

**P36894**
MTQLYIYIRLLGAYLFIISRVQGQNLDSMLHGTGMKSDSDQKKSENGVTLAPEDTLPFLKCYCSGH
CPDDAINNTCITNGHCFAIIEEDDQGETTLASGCMKYEGSDFQCKDSPKAQLRRTIECCRTNLCNQ

YLQPTLPPVVIGPFFDGSIRWLVLLISMAVCIIAMIIFSSCFCYKHYCKSISSRRRYNRDLEQDEAFIP
VGESLKDLIDQSQSSGSGSGLPLLVQRTIAKQIQMVRQVGKGRYGEVWMGKWRGEKVAVKVFFT
TEEASWFRETEIYQTVLMRHENILGFIAADIKGTGSWTQLYLITDYHENGSLYDFLKCATLDTRALL
KLAYSAACGLCHLHTEIYGTQGKPAIAHRDLKSKNILIKKNGSCCIADLGLAVKFNSDTNEVDVPLN
TRVGTKRYMAPEVLDESLNKNHFQPYIMADIYSFGLIIWEMARRCITGGIVEEYQLPYYNMVPSDP
SYEDMREVVCVKRLRPIVSNRWNSDECLRAVLKLMSECWAHNPASRLTALRIKKTLAKMVESQD
VKI (SEQ ID NO:308)

**Nucleotide:** L02911
**Sequence:**
atggtagatggagtgatgattcttcctgtgcttatcatgattgctctcccctcccctagtatggaagatgagaagcccaaggtcaaccccaaa
ctctacatgtgtgtgtgtgaaggtctctcctgcggtaatgaggaccactgtgaaggccagcagtgcttttcctcactgagcatcaacgatggc
ttccacgtctaccagaaaggctgcttccaggtttatgagcagggaaagatgacctgtaagaccccgccgtcccctggccaagccgtgga
gtgctgccaaggggactggtgtaacaggaacatcacggcccagctgcccactaaaggaaaatccttccctggaacacagaatttccact
tggaggttggcctcattattctctctgtagtgttcgcagtatgtcttttagcctgcctgctgggagttgctctccgaaaatttaaaaggcgcaacc
aagaacgcctcaatccccgagacgtggagtatggcactatcgaagggctcatcaccaccaatgttggagacagcactttagcagatttat
tggatcattcgtgtacatcaggaagtggctctggtcttccttttctggtacaaagaacagtggctcgccagattacactgttggagtgtgtcgg
gaaaggcaggtatggtgaggtgtggaggggcagctggcaaggggaaaatgttgccgtgaagatcttctcctcccgtgatgagaagtcat
ggttcagggaaacggaattgtacaacactgtgatgctgaggcatgaaaatatcttaggtttcattgcttcagacatgacatcaagacactcc
agtacccagctgtggttaattacacattatcatgaaatgggatcgttgtacgactatcttcagcttactactctggatacagttagctgccttcga
atagtgctgtccatagctagtggtcttgcacatttgcacatagagatatttgggacccaagggaaaccagccattgcccatcgagatttaaa
gagcaaaaatattctggttaagaagaatggacagtgttgcatagcagatttgggcctggcagtcatgcattcccagagcaccaatcagctt
gatgtggggaacaatccccgtgtgggcaccaagcgctacatggcccccgaagttctagatgaaaccatccaggtggattgtttcgattctt
ataaaagggtcgatatttgggcctttggacttgttttgtgggaagtggccaggcggatggtgagcaatggtatagtggaggattacaagcca
ccgttctacgatgtggttcccaatgacccaagttttgaagatatgaggaaggtagtctgtgtggatcaacaaaggccaaacatacccaaca
gatggttctcagacccgacattaacctctctggccaagctaatgaaagaatgctggtatcaaaatccatccgcaagactcacagcactgc
gtatcaaaaagactttgaccaaaattgataattccctcgacaaattgaaaactgactgttga (SEQ ID NO:309)

**Q04771**
MVDGVMILPVLIMIALPSPSMEDEKPKVNPKLYMCVCEGLSCGNEDHCEGQQCFSSLSINDGFHV
YQKGCFQVYEQGKMTCKTPPSPGQAVECCQGDWCNRNITAQLPTKGKSFPGTQNFHLEVGLIIL
SVVFAVCLLACLLGVALRKFKRRNQERLNPRDVEYGTIEGLITTNVGDSTLADLLDHSCTSGSGSG
LPFLVQRTVARQITLLECVGKGRYGEVWRGSWQGENVAVKIFSSRDEKSWFRETELYNTVMLRH
ENILGFIASDMTSRHSSTQLWLITHYHEMGSLYDYLQLTTLDTVSCLRIVLSIASGLAHLHIEIFGTQ
GKPAIAHRDLKSKNILVKKNGQCCIADLGLAVMHSQSTNQLDVGNNPRVGTKRYMAPEVLDETIQ
VDCFDSYKRVDIWAFGLVLWEVARRMVSNGIVEDYKPPFYDVVPNDPSFEDMRKVVCVDQQRP
NIPNRWFSDPTLTSLAKLMKECWYQNPSARLTALRIKKTLTKIDNSLDKLKTDC (SEQ ID NO:310)

# Figure 138

**Nucleotide:** S59184
**Sequence:**
atgcgtggggcggcgcggctggggcggccgggccggagttgcctcccggggcccgcgctgagggccgccgccgcgcccgccctgct
gcttgctcgttgcgctgttgccgctgctgccggcctgcgtgccgccgcccgtccgcggcccccggagctgcagtcggcttccgcggggccc
agcgtgagtctctacctgagcgaggacgaggtgcgccggctgatcggtcttgatgcagaactttattatgtgagaaatgaccttattagtca
ctacgctctatcctttaatctgttagtacccagtgagacaaatttcctgcacttcacctggcatgcgaagtccaaggttgaatataagctggga
ttccaagtggacaatgttttggcaatggatatgccccaggtcaacatttctgttcaggggggaagttccacgcactttatcagtgtttcgggtag
agctttcctgtactggcaaagtagattctgaagttatgatactaatgcagctcaacttgacagtaaattcttcaaaaaatttaccgtcttaaattt
taaacgaaggaaaatgtgctacaaaaaacttgaagaagtaaaaacttcagccttggacaaaaacactagcagaactatttatgatcctgt
acatgcagctccaaccacttctacgcgtgtgttttatattagtgtaggggtttgttgtgcagtaatatttctcgtagcaataatattagctgttttgca
ccttcataatatgaaaaggattgaactggatgacagcattagtgccagcagtagttcccaagggctgtctcagccatccacccagacgac
tcagtatctgagagcagacacgcccaacaatgcaactcctatcaccagttatcctaccttgcggatagagaagaacgacttgagaagtgt
cactcttttggaggccaaaggcaaggtgaaggatatagcaatatccagagagaggataactctaaaagatgtactccaagaaggtacttt
tgggcgtattttccatgggatttaatagatgaaaaagatccaaataaagaaaaacaagcatttgtcaaaacagttaaagatcaagcttctg
aaattcaggtgacaatgatgctcactgaaagttgtaagctgcgaggtcttcatcacagaaatcttcttcctattactcatgtgtgtatagaaga
aggagaaaagcccatggtgatattgccttacatgaattgggggaatcttaaattgtttttacgacagtgcaagttagtagaggccaataatc
cacaggcaatttctcagcaagacctggtacacatggctattcagattgcctgtggaatgagctacctggccagaagggaagtcatccaca
aagacctggctgccaggaactgtgtcattgatgacacacttcaagttaagatcacagacaatgccctctccagagacttgttccccatgga
ctatcactgtctgggggacaatgaaaacaggccagttcgttggatggctcttgaaagtctggttaataacgagttctctagcgctagtgatgt
gtgggcctttggagtgaacagcttgtgggaactcatgactctgggccagactccctacacgttggacattgaccccttcgagatggcggca
tacctgaaagatggttaccgaatagcccagccaatcacctgtcctgatgaattatttgctgtgatggcctgttgctgggccttagatccagag
gagaggcccaggtttcagcagctggtacagtgcctaacagagtttcatgcagccctgggggcctacgtctga (SEQ ID NO:311)

**P34925**
MRGAARLGRPGRSCLPGPALRAAAAPALLLARCAVAAAAGLRAAARPRPPELQSASAGPSVSLY
LSEDEVRRLIGLDAELYYVRNDLISHYALSFNLLVPSETNFLHFTWHAKSKVEYKLGFQVDNVLAM
DMPQVNISVQGEVPRTLSVFRVELSCTGKVDSEVMILMQLNLTVNSSKNFTVLNFKRRKMCYKKL
EEVKTSALDKNTSRTIYDPVHAAPTTSTRVFYISVGVCCAVIFLVAIILAVLHLHNMKRIELDDSISAS
SSSQGLSQPSTQTTQYLRADTPNNATPITSYPTLRIEKNDLRSVTLLEAKGKVKDIAISRERITLKDV
LQEGTFGRIFHGILIDEKDPNKEKQAFVKTVKDQASEIQVTMMLTESCKLRGLHHRNLLPITHVCIE
EGEKPMVILPYMNWGNLKLFLRQCKLVEANNPQAISQQDLVHMAIQIACGMSYLARREVIHKDLAA
RNCVIDDTLQVKITDNALSRDLFPMDYHCLGDNENRPVRWMALESLVNNEFSSASDVWAFGVTL
WELMTLGQTPYVDIDPFEMAAYLKDGYRIAQPINCPDELFAVMACCWALDPEERPKFQQLVQCLT
EFHAALGAYV (SEQ ID NO:312)

# Figure 139

**Nucleotide:** AB028983
**Sequence:**
ggagcctaccataagcacctcatggaactcgctcttcagcaaacatatcaggacacctgtaattgcatcaagtcgcggatcaagttggaa
tttgaaaaacgtcaacaggagcggcttctgctctccctgctgccggcccacatcgccatggagatgaaagcggagatcatccagaggct
gcagggccccaaggcgggccagatggagaacacaaataacttccacaacctgtatgtgaagcggcatacaaacgtgagcatcttata
cgctgacatcgttggctttacccggctggcaagtgactgctccccgggagaactagtccacatgctgaatgagctctttggaaagtttgatc
aaattgcaaaggagaatgaatgcatgagaattaaaattttaggagactgctactactgtgtatctggactccctatatctctccctaaccatg
ccaagaactgtgtgaaaatggggctggacatgtgtgaagccataaagaaagtgagggatgctactggagttgatatcaacatgcgcgtg
ggcgtgcattctgggaatgtcctgtgtggcgtgattggtctgcagaagtggcaatatgatgtgtggtcacatgatgtgaccttggccaaccac
atggaagctggaggggtccctggacgtgttcacatttcttctgtcaccctggagcacttgaatggcgcttataaagtggaggagggagatg
gtgacattagggacccatatttaaaacagcacctggtgaaaacctactttgtgatcaaccccaagggagaacgacggagcccccagca
tctcttcagacctcgccacacccttgatggagccaaaatgagggcctcggtccgcatgacccggtacttggagtcctgggggggcagcca
agccctttgcacacctacatcacagggacagcatgaccacagagaacggcaagatcagcaccacggatgtacccatgggtcagcata
attttcaaaatcgcaccttaagaaccaagtcacaaaagaagagatttgaagaagaattgaatgaaaggatgattcaagcaattgatggg
attaatgcacagaagcaatggctcaagtctgaagacattcagagaatctcactgcttttctataacaaagtactagaaaaagagtaccgg
gccacggcactgccagcgttcaagtattatgtgacttgtgcctgtctcatattcttctgcatcttcattgtgcagattctcgtgctgccaaaaacgt
ctgtcctgggcatctcctttggggctgcgtttctcttgctggccttcatcctcttcgtctgctttgctggacagcttctgcaatgcagcaaaaaagc
ctctcccctgctcatgtggcttttgaagtcctcgggcatcattgccaaccgcccctggccacggatctctctcacgatcatcaccacagccat
catattaatgatggccgtgttcaacatgtttttcctgagtgactcagaggaaacaatccctccaactgccaacacaacaaacacaagcttttc
agcctcaaataatcaggtggcgattctgcgtgcgcagaatttatttttcctcccgtactttatctacagctgcattctgggactgatatcctgttcc
gtgttcctgcgggtaaactatgagctgaagatgttgatcatgatggtggccttggtgggctacaacaccatcctactccacacccacgccca
cgtcctgggcgactacagccaggtcttatttgagagaccaggcatttggaaagacctgaagaccatgggctctgtgtctctctctatattcttc
atcacactgcttgttctgggtagacagaatgaatattactgtaggttagacttcttatggaagaacaaattcaaaaaagagcgggaggaga
tagagaccatggagaacctgaaccgcgtgctgctggagaacgtgcttcccgcgcacgtggctgagcacttcctggccaggagcctgaa
gaatgaggagctataccaccagtcctatgactgcgtctgtgtcatgtttgcctccattccggatttcaaagaattttatacagaatccgacgtg
aacaaggagggcttggaatgccttcggctcctgaacgagatcatcgctgactttgatgatcttctttccaagccaaaattcagtggagttgaa
aagattaagaccattggcagcacatacatggcagcaacaggtctgagcgctgtgcccagccaggagcactcccaggagcccgagcg
gcagtacatgcacattggcaccatggtggagtttgcttttgccctggtagggaagctggatgccatcaacaagcactccttcaacgacttca
aattgcgagtgggtattaaccatggacctgtgatagctggtgtgattggagctcagaagccacaatatgatatctggggcaacactgtcaat
gtggccagtaggatggacagcaccggagtcctggacaaaatacaggttaccgaggagacgagcctcgtcctgcagaccctcggatac
acgtgcacctgtcgaggaataatcaacgtgaaaggaaagggggacctgaagacgtactttgtaaacacagaaatgtcaaggtccctttc
ccagagcaacgtggcatcctga (SEQ ID NO:313)

**Q08462**
GAYHKHLMELALQQTYQDTCNCIKSRIKLEFEKRQQERLLLSLLPAHIAMEMKAEIIQRLQGPKAG
QMENTNNFHNLYVKRHTNVSILYADIVGFTRLASDCSPGELVHMLNELFGKFDQIAKENECMRIKIL
GDCYYCVSGLPISLPNHAKNCVKMGLDMCEAIKKVRDATGVDINMRVGVHSGNVLCGVIGLQKW
QYDVWSHDVTLANHMEAGGVPGRVHISSVTLEHLNGAYKVEEGDGDIRDPYLKQHLVKTYFVINP
KGERRSPQHLFRPRHTLDGAKMRASVRMTRYLESWGAAKPFAHLHHRDSMTTENGKISTTDVP
MGQHNFQNRTLRTKSQKKRFEEELNERMIQAIDGINAQKQWLKSEDIQRISLLFYNKVLEKEYRAT
ALPAFKYYVTCACLIFFCIFIVQILVLPKTSVLGISFGAAFLLLAFILFVCFAGQLLQCSKKASPLLMWL
LKSSGIIANRPWPRISLTIITTAIILMMAVFNMFFLSDSEETIPPTANTTNTSFSASNNQVAILRAQNLF
FLPYFIYSCILGLISCSVFLRVNYELKMLIMMVALVGYNTILLHTHAHVLGDYSQVLFERPGIWKDLK
TMGSVSLSIFFITLLVLGRQNEYYCRLDFLWKNKFKKEREEIETMENLNRVLLENVLPAHVAEHFLA
RSLKNEELYHQSYDCVCVMFASIPDFKEFYTESDVNKEGLECLRLLNEIIADFDDLLSKPKFSGVEK
IKTIGSTYMAATGLSAVPSQEHSQEPERQYMHIGTMVEFAFALVGKLDAINKHSFNDFKLRVGINH
GPVIAGVIGAQKPQYDIWGNTVNVASRMDSTGVLDKIQVTEETSLVLQTLGYTCTCRGIINVKGKG
DLKTYFVNTEMSRSLSQSNVAS (SEQ ID NO:314)

**Nucleotide:** Z35309
**Sequence:**
atggagctctccgatgtgcgctgccttacaggcagcgaggaactctacaccatccaccccgacgcccccggccggcgacggcaggagc
gcctcccggccgcagcggctgctgtggcagacggcggtgcgacacatcacggagcagcgcttcattcacgggcaccggggaggcag
cggcagcgggagtggaggctcgggcaaagcctcggaccctgcgggcggcggccccaaccaccacgcgccgcagctgtcaggcga
ctcggcgctgcccctctactcgctgggcccgggagagcgagcgcacagcacctgcggcaccaaagtcttcccggaacgcagcggga
gcggcagtgccagcggcagcggaggcgggggcgacctgggcttcctgcaccttgactgtgcccctagcaactcggatttcttcttaatgg
gggctatagctaccgaggggtcattttccccaccctgcgcaactccttcaaatctcgggatttggaacgcctctaccagcgctatttcttgggc
caaaggcgcaaatcggaagtggtgatgaacgtgctggacgtgctgaccaaactcactctcttggtcctacacttgagcctggcctcggcc
cccatggacccgctcaagggcatcctgctgggcttcttcaccggcattgaggtagtgatctgcgccctggtggtggtcaggaaggacacc
acctcccacacgtacctgcagtacagcggcgtggtcacctgggtggccatgaccacccagatcctggcagcaggcctcggctacgggc
tcctgggcgacggcataggctacgtgctcttcacgctcttcgccacctacagtatgctgccgctgccgctcacctgggccatcctggccggc
ctgggcacctcgctgctgcaggtcatcctccaagtggtcataccccggctggcggtcatttccatcaaccaggttgtggcccaggcagtgct
attcatgtgtatgaacacagctggaatcttcatcagttacctgtcagaccgggcccagcgccaagctttcctggagactcggaggtgtgtgg
aggccaggctgcgcctggagacagagaaccaaagacaggagcggctcgtgctttctgtgctcccccggtttgttgtcctggaaatgatca
acgacatgaccaatgtggaagatgagcacctgcagcaccagttccatcggatctacatccatcgctatgagaacgtcagtattctttttgca
gatgttaaaggatttaccaacctctccacgaccttgtctgctcaggagctggtcaggatgctcaacgagctctttgccagatttgatcgactg
gcccatgagcatcactgccttcgtattaaaatcctgggggactgctactactgcgtgtctggacttcctgagccccgccaggaccatgccca
ctgctgtgttgaaatgggtctcagcatgatcaaaaccatcaggtatgtgcggtcaaggacaaaacacgatgttgacatgaggattggaatc
cactccggctcggtgctgtgcggtgttttgggactacggaagtggcagtttgatgtctggtcttgggatgtggatattgcaaacaaactcgaat
ctggaggaatccccgggaggattcacatttccaaagccacgctggactgtctcaacggtgactataacgtggaagagggccatggtaaa
gagaggaatgaattcctgaggaagcataatatcgaaacttacttaattaagcagcctgaggacagtctgctgtccttgcctgaagatatcgt
caaggagtcagtgagctcctcagaccggagaaacagtggggccacattcactgaaggatcctggagccctgaactgccctttgataata
tcgtggggaaacagaatactctggctgccctaacaagaaattcaataaatctgcttccaaaccatcttgcacaagctttgcatgtccagtct
gggcctgaggaaattaacaagagaatagaacataccatcgacttgcggagtggcgataaattgagaagagagcatatcaagccattct
cactgatgtttaaagactccagcctggagcacaagtattctcaaatgagggatgaagtgttcaagtcaaacttggtctgtgcatttatcgttctt
ctatttatcacggcaatacaaagtttgcttccttcttcaagagtgatgccaatgaccatccagttctccattctgattatgctgcactcggctctgg
tcctcatcaccacagcagaggattataaatgtttgcccctcatcctccggaaaacttgctgttggattaatgagacctatttggcccggaacgt
catcatctttgcatccattttgattaatttcctgggtgccatcttaaatatcctgtggtgtgattttgacaagtcgatacccttgaagaacctgacttt
caattcctcagctgtgtttacagatatctgctcctacccagagtactttgtcttcacgggggtgttggccatggtgacctgtgcagttttcctccgg
ctgaactccgtcctgaagctggcagtgctgctgatcatgattgccatctatgccctgctcactgagaccgtctacgcaggcctctttctgcgtta
tgacaacctcaaccacagtggagaagatttcctggggaccaaggaggtatcactgctactgatggccatgttcctcctggctgtgttctacc
atggacagcagctggagtacacagcccgcctggacttcctttggcgagtacaggccaaagaggagatcaatgagatgaaggagctga
gggaacacaatgagaacatgctccggaatatcttacccagccatgtggcccgccatttcctagagaaggaccgagacaatgaggagct
gtattctcaatcctatgatgctgttggggtgatgtttgcctccatcccaggatttgcggacttttactctcagactgaaatgaataaccagggagt
ggaatgcctgcgcttgctcaatgagatcattgctgacttcgatgagttgcttggtgaagaccgatttcaagacattgaaaagattaagaccat
tggcagcacctacatggccgtgtcaggcctgtcacctgaaaaacagcaatgtgaagacaagtggggacatttgtgtgctctggctgacttc
tcactcgccctgacagaaagcatacaggagatcaacaagcattcattcaacaattttgaactccggattggcatcagccacggctcagtg
gtagctggcgttatcggcgctaagaaaccacagtatgacatttggggcaaaactgtgaacctggcaagccgaatggacagcacgggg
gttagtggccggatccaagtcccagaggagacctatctcatcctgaaggaccagggctttgcctttgattaccgaggggagatctatgtga
agggtatcagtgaacaggaaggaaaaatcaaaacgtactttcttctgggaagagtccaacccaacccattcatcttgcccccaagaaga
ctgcctgggcagtactccctggccgcggttgtcctgggacttgtccagtccctcaataggcaaaggcagaagcagctactcaatgagaac
aacaacacaggaatcatcaagggtcattacaaccggcggactttgttgtcacccagcggcacagagcctggagcccaggctgaaggc
accgacaaatctgatttgccataa (SEQ ID NO:315)

**P40145**
MELSDVRCLTGSEELYTIHPTPPAGDGRSASRPQRLLWQTAVRHITEQRFIHGHRGGSGSGSGG
SGKASDPAGGGPNHHAPQLSGDSALPLYSLGPGERAHSTCGTKVFPERSGSGSASGSGGGGDL
GFLHLDCAPSNSDFFLNGGYSYRGVIFPTLRNSFKSRDLERLYQRYFLGQRRKSEVVMNVLDVLT
KLTLLVLHLSLASAPMDPLKGILLGFFTGIEVVICALVVVRKDTTSHTYLQYSGVVTWVAMTTQILAA
GLGYGLLGDGIGYVLFTLFATYSMLPLPLTWAILAGLGTSLLQVILQVVIPRLAVISINQVVAQAVLF
MCMNTAGIFISYLSDRAQRQAFLETRRCVEARLRLETENQRQERLVLSVLPRFVVLEMINDMTNV
EDEHLQHQFHRIYIHRYENVSILFADVKGFTNLSTTLSAQELVRMLNELFARFDRLAHEHHCLRIKIL
GDCYYCVSGLPEPRQDHAHCCVEMGLSMIKTIRYVRSRTKHDVDMRIGIHSGSVLCGVLGLRKW
QFDVWSWDVDIANKLESGGIPGRIHISKATLDCLNGDYNVEEGHGKERNEFLRKHNIETYLIKQPE
DSLLSLPEDIVKESVSSSDRRNSGATFTEGSWSPELPFDNIVGKQNTLAALTRNSINLLPNHLAQAL
HVQSGPEEINKRIEHTIDLRSGDKLRREHIKPFSLMFKDSSLEHKYSQMRDEVFKSNLVCAFIVLLFI

TAIQSLLPSSRVMPMTIQFSILIMLHSALVLITTAEDYKCLPLILRKTCCWINETYLARNVIIFASILINFL
GAILNILWCDFDKSIPLKNLTFNSSAVFTDICSYPEYFVFTGVLAMVTCAVFLRLNSVLKLAVLLIMIAI
YALLTETVYAGLFLRYDNLNHSGEDFLGTKEVSLLLMAMFLLAVFYHGQQLEYTARLDFLWRVQA
KEEINEMKELREHNENMLRNILPSHVARHFLEKDRDNEELYSQSYDAVGVMFASIPGFADFYSQT
EMNNQGVECLRLLNEIIADFDELLGEDRFQDIEKIKTIGSTYMAVSGLSPEKQQCEDKWGHLCALA
DFSLALTESIQEINKHSFNNFELRIGISHGSVVAGVIGAKKPQYDIWGKTVNLASRMDSTGVSGRIQ
VPEETYLILKDQGFAFDYRGEIYVKGISEQEGKIKTYFLLGRVQPNPFILPPRRLPGQYSLAAVVLGL
VQSLNRQRQKQLLNENNNTGIIKGHYNRRTLLSPSGTEPGAQAEGTDKSDLP (SEQ ID NO:316)

# Figure 140

**Nucleotide:** D32050
**Sequence:**
atggactctactctaacagcaagtgaaatccggcagcgatttatagatttcttcaagaggaacgagcatacgtatgttcactcgtctgccac
catcccattggatgaccccactttgctctttgccaatgcaggcatgaaccagtttaaacccattttcctgaacacaattgacccatctcacccc
atggcaaagctgagcagagctgccaatacccagaagtgcatccgggctgggggcaaacaaaatgacctggacgatgtgggcaagg
atgtctatcatcacaccttcttcgagatgctgggctcttggtcttttggagattactttaaggaattggcatgtaagatggctctggaactcctcac
ccaagagtttggcattcccattgaaagactttatgttacttactttggcggggatgaagcagctggcttagaagcagatctggaatgcaaac
agatctggcaaaatttggggctggatgacaccaaaatcctcccaggcaacatgaaggataacttctgggagatgggtgacacgggccc
ctgtggtccttgcagtgagatccactacgaccggattggtggtcgggacgccgcacatcttgtcaaccaggacgaccctaatgtgctggag
atctggaaccttgtgttcatccagtataacagggaagctgatggcattctgaaacctcttcccaagaaaagcattgacacagggatgggcc
tggaacgactggtatctgtgctgcagaataagatgtccaactatgacactgaccttttgtcccttactttgaagccattcagaagggcacag
gtgcccgaccatacactgggaaagttggtgctgaggatgccgatgggattgacatggcctaccgggtgctggctgaccatgctcggacc
atcactgtggcactggctgatggtggccggcctgacaacacagggcgtggatatgtgttgagacggattctccgccgagctgtccgatac
gcccatgaaaagctcaatgccagcaggggcttctttgctacgttagtggatgttgtcgtccagtccctgggagatgcatttcctgagctgaag
aaggacccagacatggtgaaggacatcattaatgaagaagaggtgcagtttctcaagactctcagcagagggcgtcgcatcctggaca
ggaaaattcagagcctgggagacagcaagaccattcccggagacactgcttggctcctctatgacacctatgggtttccagtggatctga
ctggactgattgctgaagagaagggcctggtggtagacatggatggctttgaagaggagaggaaactggcccagctgaaatcacagg
gcaagggagctggtggggaagacctcattatgctggacatttacgctatcgaagagctccgggcacggggtctggaggtcacagatgat
tccccaaagtacaattaccatttggactccagtggtagctatgtatttgagaacacagtggctacggtgatggctctgcgcagggagaaga
tgttcgtggaagaggtgtccacaggccaggagtgtggagtggtgctggacaagacctgtttctatgctgagcaaggaggccagatctatg
acgaaggctacctggtgaaggtggatgacagcagtgaagataaaacagagtttacagtgaagaatgctcaggtccgaggagggtatgt
gctacacattggaaccatctacggtgacctgaaagtgggggatcaggtctggctgtttattgatgagccccgacgaagacccatcatgag
caaccacacagctacgcacattctgaacttcgccctgcgctcagtgcttggggaagctgaccagaaaggctcattggttgctcctgaccgc
ctcagatttgactttactgccaagggagccatgtccacccaacagatcaagaaggctgaagagattgctaatgagatgattgaggcagcc
aaggccgtctatacccaggattgcccccctggcagcagcgaaagccatccagggcctacgggctgtgtttgatgagacctatcctgaccct
gtgcgagtcgtctccattggggtcccggtgtccgagttgctggatgacccctctgggcctgctggctccctgacttctgttgagttctgtgggg
aacgcacctgcggaactcgagtcatgcaggagcttttgtgatcgtgacggaagaagccattgccaagggtatccggaggattgtggctgt
cacaggtgccgaggcccagaaggccctcaggaaagcagagagcttgaagaaatgtctctctgtcatggaagccaaagtgaaggctca
gactgctccaaacaaggatgtgcagagggagatcgctgaccttggagaggccctggccactgcagtcatcccccagtggcagaagga
tgaattgcgggagactctcaaatccctaaagaaggtcatggatgacttggaccgagccagcaaagccgatgtccagaaacgagtgtta
gagaagacgaagcagttcatcgacagcaaccccaaccagcctcttgtcatcctggagatggagagcggcgcctcagccaaggccctg
aatgaagccttgaagctcttcaagatgcactcccctcagacttctgccatgctcttcacggtggacaatgaggctggcaagatcacgtgcct
gtgtcaagtcccccagaatgcagccaatcggggcttaaaagccagcgagtgggtgcagcaggtgtcaggcttgatggacggtaaaggt
ggtggcaaggatgtgtctgcacaggccacaggcaagaacgttggctgcctgcaggaggcgctgcagctggccacttccttcgcccagct
gcgcctcggggatgtaaagaactga (SEQ ID NO:317)

**P49588**
MDSTLTASEIRQRFIDFFKRNEHTYVHSSATIPLDDPTLLFANAGMNQFKPIFLNTIDPSHPMAKLS
RAANTQKCIRAGGKQNDLDDVGKDVYHHTFFEMLGSWSFGDYFKELACKMALELLTQEFGIPIER
LYVTYFGGDEAAGLEADLECKQIWQNLGLDDTKILPGNMKDNFWEMGDTGPCGPCSEIHYDRIG
GRDAAHLVNQDDPNVLEIWNLVFIQYNREADGILKPLPKKSIDTGMGLERLVSVLQNKMSNYDTDL
FVPYFEAIQKGTGARPYTGKVGAEDADGIDMAYRVLADHARTITVALADGGRPDNTGRGYVLRRI
LRRAVRYAHEKLNASRGFFATLVDVVVQSLGDAFPELKKDPDMVKDIINEEEVQFLKTLSRGRRIL
DRKIQSLGDSKTIPGDTAWLLYDTYGFPVDLTGLIAEEKGLVVDMDGFEEERKLAQLKSQGKGAG
GEDLIMLDIYAIEELRARGLEVTDDSPKYNYHLDSSGSYVFENTVATVMALRREKMFVEEVSTGQE
CGVVLDKTCFYAEQGGQIYDEGYLVKVDDSSEDKTEFTVKNAQVRGGYVLHIGTIYGDLKVGDQV
WLFIDEPRRRPIMSNHTATHILNFALRSVLGEADQKGSLVAPDRLRFDFTAKGAMSTQQIKKAEEIA
NEMIEAAKAVYTQDCPLAAAKAIQGLRAVFDETYPDPVRVVSIGVPVSELLDDPSGPAGSLTSVEF
CGGTHLRNSSHAGAFVIVTEEAIAKGIRRIVAVTGAEAQKALRKAESLKKCLSVMEAKVKAQTAPN
KDVQREIADLGEALATAVIPQWQKDELRETLKSLKKVMDDLDRASKADVQKRVLEKTKQFIDSNP
NQPLVILEMESGASAKALNEALKLFKMHSPQTSAMLFTVDNEAGKITCLCQVPQNAANRGLKASE
WVQQVSGLMDGKGGGKDVSAQATGKNVGCLQEALQLATSFAQLRLGDVKN (SEQ ID NO:318)

# Figure 141

**Nucleotide:** AF128625
**Sequence:**
atgtcggccaaggtgcggctcaagaagctggagcagctgctcctggacgggccctggcgcaacgagagcgccctgagcgtggaaac
gctgctcgacgtgctcgtctgcctgtacaccgagtgcagccactcggccctgcgccgcgacaagtacgtggccgagttcctcgagtgggc
taaaccatttacacagctggtgaaagaaatgcagcttcatcgagaagactttgaaataattaaagtaattggaagaggtgcttttggtgagg
ttgctgttgtcaaaatgaagaatactgaacgaatttatgcaatgaaaatcctcaacaagtgggagatgctgaaaagagcagagaccgcg
tgcttccgagaggagcgcgatgtgctggtgaacggcgactgccagtggatcaccgcgctgcactacgcctttcaggacgagaaccacct
gtacttagtcatggattactatgtgggtggtgatttactgaccctgctcagcaaatttgaagacaagcttccggaagatatggcgaggttctac
attggtgaaatggtgctggccattgactccatccatcagcttcattacgtgcacagagacattaaacctgacaatgtccttttggacgtgaatg
gtcatatccgcctggctgactttggatcatgtttgaagatgaatgatgatggcactgtgcagtcctccgtggccgtgggcacacctgactaca
tctcgccggagatcctgcaggcgatggaggacggcatgggcaaatacgggcctgagtgtgactggtggtctctgggtgtctgcatgtatg
agatgctctatggagaaacgccgttttatgcggagtcactcgtggagacctatgggaagatcatgaaccatgaagagcgattccagttccc
atcccatgtcacggatgtatctgaagaagcgaaggacctcatccagagactgatctgcagtagagagaacgccggctggggcagaatgga
atagaggatttcaaaaagcatgcgtttttgaaggtctaaattgggaaaatatacgaaacctagaagcaccttatattcctgatgtgagcagt
ccctctgacacatccaacttcgacgtggatgacgacgtgctgagaaacacggaaatattacctcctggttctcacacaggcttttctggatta
catttgccattcattggtttacattcacaacggaaagctgtttttctgatcgaggctctctgaagagcataatgcagtccaacacattaaccaa
agatgaggatgtgcagcgggacctggagcacagcctgcagatggaagcttacgagaggaggattcggaggctggaacaggagaag
ctggagctgagcaggaagctgcaagagtccacccagaccgtgcagtccctccacggctcatctcgggccctcagcaattcaaaccgag
ataaagaaatcaaaaagctaaatgaagaaatcgaacgcttgaagaataaaatagcagattcaaacaggctggagcgacagcttgag
gacacagtggcgcttcgccaagagcgtgaggactccacgcagcggctgcgggggctggagaagcagcaccgcgtggtccggcagg
agaaggaggagctgcacaagcaactggttgaagcctcagagcggttgaaatcccaggccaaggaactcaaagatgcccatcagcag
cgaaagctggccctgcaggagttctcggagctgaacgagcgcatggcagagctccgtgcccagaagcagaaggtgtcccggcagctg
cgagacaaggaggaggagatggaggtggccacgcagaaggtggacgccatgcggcaggaaatgcggagagctgagaagctcag
gaaagagctggaagctcagcttgatgatgctgttgctgaggcctccaaggagcgcaagcttcgtgagcacagcgagaacttctgcaagc
aaatggaaagcgagctggaggccctcaaggtgaagcaaggaggccggggagcgggtgccaccttagagcaccagcaagagatttc
caaaatcaaatccgagctggagaagaaagtcttattttatgaagaggaattggtcagacgtgaggcctcccatgtgctagaagtgaaaa
atgtgaagaaggaggtgcatgattcagaaagccaccagctggccctgcagaaagaaatcttgatgttaaaagataagttagaaaagtc
aaagcgagaacggcataacgagatggaggaggcagtaggtacaataaaagataaatacgaacgagaaagagcgatgctgtttgatg
aaaacaagaagctaactgctgaaaatgaaaagctctgttcctttgtggataaactcacagctcaaaatagacagctggaggatgagctg
caggatctggcagccaagaaggagtcagtggcccactgggaagctcagattgcggaaatcattcagtgggtcagtgacgagaaagat
gcccggggttaccttcaagctcttgcttccaagatgaccgaagagctcgaggctttgaggagttctagtctggggtcaagaacactggacc
cgctgtggaaggtgcgccgcagccagaagctggacatgtccgcgcggctggagctgcagtcggccctggaggcggagatccgggcc
aagcagcttgtccaggaggagctcaggaaggtcaaggacgccaacctcaccttggaaagcaaactaaaggattccgaagccaaaaa
cagagaattattagaagaaatggaaattttgaagaaaaagatggaagaaaaattcagagcagatactgggctcaaacttccagattttca
ggattccatttttgagtatttcaacactgctcctcttgcacatgacctgacatttagaaccagctcagctagtgagcaagaaacacaagctcc
gaagccagaagcgtccccgtcgatgtctgtggctgcatcagagcagcaggaggacatggctcggcccccgcagaggccatccgctgt
gccgttgcccaccacgcaggccctggttctggctggaccgaagccaaaagctcaccagttcagcatcaagtccttctccagccctactca
gtgcagccactgcacctccctgatggttgggctgatccggcagggctacgcctgcgaggtgtgttcctttgcttgccacgtgtcctgcaaag
acggtgcccccccaggtgtgcccaatacctcccgagcagtccaagaggcctctgggcgtggacgtgcagcgaggcatcggaacagcct
acaaaggccatgtcaaggtcccaaagcccacgggggtgaagaagggatggcagcgcgcatatgcagtcgtctgtgagtgcaagctctt
cctgtatgatctgcctgaaggaaaatccacccagcctggtgtcattgcgagccaagtcttggatctcagagatgacgagttttccgtgagct
cagtcctggcctcagatgtcattcatgctacacgccgagatattccatgtatattcagggtgacggcctctctcttaggtgcaccttctaagac
cagctcgctgctcattctgacagaaaatgagaatgaaaagaggaagtgggttgggattctagaaggactccagtccatccttcataaaaa
ccggctgaggaatcaggtcgtgcatgttcccttggaagcctacgacagctcgctgcctctcatcaaggccatcctgacagctgccatcgtg
gatgcagacaggattgcagtcggcctagaagaagggctctatgtcatagaggtcacccgagatgtgatcgtccgtgccgctgactgtaag
aaggtacaccagatcgagcttgctcccagggagaagatcgtaatcctcctctgtggccggaaccaccatgtgcacctctatccgtggtcgt
cccttgatggagcggaaggcagctttgacatcaagcttccggaaaccaaaggctgccagctcatggccacggccacactcaagagga
actctggcacctgcctgtttgtggccgtgaaacggctgatcctttgctatgagatccagagaacgaagccattccacagaaagttcaatga
gattgtggctcccggcagcgtgcagtgcctggcggtgctcagggacaggctctgtgtgggctacccttctgggttctgcctgctgagcatcc
agggggacgggcagcctctaaacctggtaaatcccaatgacccctcgcttgcgttcctctcacaacagtcttttgatgccctttgtgctgtgg
agctcgaaagcgaggagtacctgctttgcttcagccacatgggactgtacgtggacccgcaaggccggagggcacgcgcgcaggagc
tcatgtggcctgcggctcctgtcgcctgtagttgcagccccacccacgtcacggtgtacagcgagtatggcgtggacgtctttgatgtgcgc
accatggagtgggtgcagaccatcggcctgcggaggataaggcccctgaactctgaaggcaccctcaacctcctcaactgcgagcctc
cacgcttgatctacttcaagagcaagttctcgggagcggttctcaacgtgccggacacctccgacaacagcaagaagcagatgctgcgc

accaggagcaaaaggcggttcgtcttcaaggtcccagaggaagagagactgcagcagaggcgagagatgcttagagacccagaatt
gagatccaaaatgatatccaacccaaccaacttcaaccacgtggcccacatgggcccaggcgacggcatgcaggtgctcatggacct
gcctctgagtgctgtgccccccctcccaggaggaaaggccgggccccgctcccaccaacctggctcgccagcctccatccaggaacaa
gccctacatctcgtggccctcatcaggtggatcggagcctagcgtgactgtgcctctgagaagtatgtctgatccagaccaggactttgaca
aagagcctgattcggactccaccaaacactcaactccatcgaatagctccaaccccagcggcccaccgagccccaactccccccaca
ggagccagctccccctcgaaggcctggagcagccggcctgtgacacctga (SEQ ID NO:319)

**Q9Y5S2**

MSAKVRLKKLEQLLLDGPWRNESALSVETLLDVLVCLYTECSHSALRRDKYVAEFLEWAKPFTQL
VKEMQLHREDFEIIKVIGRGAFGEVAVVKMKNTERIYAMKILNKWEMLKRAETACFREERDVLVNG
DCQWITALHYAFQDENHLYLVMDYYVGGDLLTLLSKFEDKLPEDMARFYIGEMVLAIDSIHQLHYV
HRDIKPDNVLLDVNGHIRLADFGSCLKMNDDGTVQSSVAVGTPDYISPEILQAMEDGMGKYGPEC
DWWSLGVCMYEMLYGETPFYAESLVETYGKIMNHEERFQFPSHVTDVSEEAKDLIQRLICSRERR
LGQNGIEDFKKHAFFEGLNWENIRNLEAPYIPDVSSPSDTSNFDVDDDVLRNTEILPPGSHTGFSG
LHLPFIGFTFTTESCFSDRGSLKSIMQSNTLTKDEDVQRDLEHSLQMEAYERRIRRLEQEKLELSR
KLQESTQTVQSLHGSSRALSNSNRDKEIKKLNEEIERLKNKIADSNRLERQLEDTVALRQEREDST
QRLRGLEKQHRVVRQEKEELHKQLVEASERLKSQAKELKDAHQQRKLALQEFSELNERMAELRA
QKQKVSRQLRDKEEEMEVATQKVDAMRQEMRRAEKLRKELEAQLDDAVAEASKERKLREHSEN
FCKQMESELEALKVKQGGRGAGATLEHQQEISKIKSELEKKVLFYEEELVRREASHVLEVKNVKK
EVHDSESHQLALQKEILMLKDKLEKSKRERHNEMEEAVGTIKDKYERERAMLFDENKKLTAENEK
LCSFVDKLTAQNRQLEDELQDLAAKKESVAHWEAQIAEIIQWVSDEKDARGYLQALASKMTEELE
ALRSSSLGSRTLDPLWKVRRSQKLDMSARLELQSALEAEIRAKQLVQEELRKVKDANLTLESKLK
DSEAKNRELLEEMEILKKKMEEKFRADTGLKLPDFQDSIFEYFNTAPLAHDLTFRTSSASEQETQA
PKPEASPSMSVAASEQQEDMARPPQRPSAVPLPTTQALVLAGPKPKAHQFSIKSFSSPTQCSHC
TSLMVGLIRQGYACEVCSFACHVSCKDGAPQVCPIPPEQSKRPLGVDVQRGIGTAYKGHVKVPK
PTGVKKGWQRAYAVVCECKLFLYDLPEGKSTQPGVIASQVLDLRDDEFSVSSVLASDVIHATRRDI
PCIFRVTASLLGAPSKTSSLLILTENENEKRKWVGILEGLQSILHKNRLRNQVVHVPLEAYDSSLPLI
KAILTAAIVDADRIAVGLEEGLYVIEVTRDVIVRAADCKKVHQIELAPREKIVILLCGRNHHVHLYPWS
SLDGAEGSFDIKLPETKGCQLMATATLKRNSGTCLFVAVKRLILCYEIQRTKPFHRKFNEIVAPGSV
QCLAVLRDRLCVGYPSGFCLLSIQGDGQPLNLVNPNDPSLAFLSQQSFDALCAVELESEEYLLCF
SHMGLYVDPQGRRARAQELMWPAAPVACSCSPTHVTVYSEYGVDVFDVRTMEWVQTIGLRRIR
PLNSEGTLNLLNCEPPRLIYFKSKFSGAVLNVPDTSDNSKKQMLRTRSKRRFVFKVPEEERLQQR
REMLRDPELRSKMISNPTNFNHVAHMGPGDGMQVLMDLPLSAVPPSQEERPGPAPTNLARQPP
SRNKPYISWPSSGGSEPSVTVPLRSMSDPDQDFDKEPDSDSTKHSTPSNSSNPSGPPSPNSPHR
SQLPLEGLEQPACDT (SEQ ID NO:320)

# Figure 142

**Nucleotide:** X15357
**Sequence:**
atgccggggccccggcgccccgctggctcccgcctgcgcctgctcctgctcctgctgctgccgccgctgctgctgctgctccggggcagc
cacgcgggcaacctgacggtagccgtggtactgccgctggccaatacctcgtacccctggtcgtgggcgcgcgtgggacccgccgtgg
agctggccctggcccaggtgaaggcgcgccccgacttgctgccgggctggacggtccgcacggtgctgggcagcagcgaaaacgcg
ctgggcgtctgctccgacaccgcagcgcccctggccgcggtggacctcaagtgggagcacaaccccgctgtgttcctgggccccggctg
cgtgtacgccgccgccccagtggggcgcttcaccgcgcactggcgggtcccgctgctgaccgccggcgccccggcgctgggcttcggt
gtcaaggacgagtatgcgctgaccacccgcgcggggcccagctacgccaagctgggggacttcgtggcggcgctgcaccgacggct
gggctgggagcgccaagcgctcatgctctacgcctaccggccgggtgacgaagagcactgcttcttcctcgtggaggggctgttcatgcg
ggtccgcgaccgcctcaatattacggtggaccacctggagttcgccgaggacgacctcagccactacaccaggctgctgcggaccatg
ccgcgcaaaggccgagttatctacatctgcagctcccctgatgccttcagaaccctcatgctcctggccctggaagctggcttgtgtgggga
ggactacgttttcttccacctggatatctttgggcaaagcctgcaaggtggacagggccctgctccccgcaggccctgggagagaggggga
tgggcaggatgtcagtgcccgccaggcctttcaggctgccaaaatcattacatataaagacccagataatcccgagtacttggaattcctg
aagcagttaaaacacctggcctatgagcagttcaacttcaccatggaggatggcctggtgaacaccatcccagcatccttccacgacgg
gctcctgctctatatccaggcagtgacggagactctggcacatggggggaactgttactgatggggagaacatcactcagcggatgtggaa
ccgaagctttcaaggtgtgacaggatacctgaaaattgatagcagtggcgatcgggaaacagacttctccctctgggatatggatcccga
gaatggtgccttcagggttgtactgaactacaatgggacttcccaagagctggtggctgtgtcggggcgcaaactgaactggcccctggg
gtaccctcctcctgacatccccaaatgtggctttgacaacgaagacccagcatgcaaccaagatcacctttccaccctggaggtgctggct
ttggtgggcagcctctccttgctcggcattctgattgtctccttcttcatatacaggaagatgcagctggagaaggaactggcctcggagctgt
ggcgggtgcgctgggaggacgttgagcccagtagccttgagaggcacctgcggagtgcaggcagccggctgaccctgagcgggaga
ggctccaattacggctccctgctaaccacagagggccagttccaagtctttgccaagacagcatattataagggcaacctcgtggctgtga
aacgtgtgaaccgtaaacgcattgagctgacacgaaaagtcctgtttgaactgaagcatatgcgggatgtgcagaatgaacacctgacc
aggtttgtgggagcctgcaccgacccccccaatatctgcatcctcacagagtactgtccccgtgggagcctgcaggacattctggagaat
gagagcatcaccctggactggatgttccggtactcactcaccaatgacatcgtcaagggcatgctgtttctacacaatggggctatctgttcc
catgggaacctcaagtcatccaactgcgtggtagatgggcgctttgtgctcaagatcaccgactatgggctggagagcttcagggacctg
gacccagagcaaggacacaccgtttatgccaaaaagctgtggacggcccctgagctcctgcgaatggcttcacccccctgtgcggggctc
ccaggctggtgacgtatacagctttgggatcatccttcaggagattgccctgaggagtggggtcttccacgtggaaggtttggacctgagcc
ccaaagagatcatcgagcgggtgactcggggtgagcagccccccttccggccctccctggccctgcagagtcacctggaggagttggg
gctgctcatgcagcggtgctgggctgaggacccacaggagaggccaccattccagcagatccgcctgacgttgcgcaaatttaacagg
gagaacagcagcaacatcctggacaacctgctgtcccgcatggagcagtacgcgaacaatctggaggaactggtggaggagcggac
ccaggcatacctggaggagaagcgcaaggctgaggccctgctctaccagatcctgcctcactcagtggctgagcagctgaagcgtggg
gagacggtgcaggccgaagcctttgacagtgttaccatctacttcagtgacattgtgggtttcacagcgctgtcggcggagagcacaccc
atgcaggtggtgaccctgctcaatgacctgtacacttgctttgatgctgtcatagacaactttgatgtgtacaaggtggagacaattggcgat
gcctacatggtggtgtcagggctccctgtgcggaacgggcggctacacgcctgcgaggtagcccgcatggccctggcactgctggatgc
tgtgcgctccttccgaatccgccaccggccccaggagcagctgcgcttgcgcattggcatccacacaggacctgtgtgtgctggagtggtg
ggactgaagatgccccgttactgtctctttggggatacagtcaacacagcctcaagaatggagtctaatggggaagccctgaagatccac
ttgtcttctgagaccaaggctgtcctggaggagtttggtggtttcgagctggagcttcgaggggatgtagaaatgaagggcaaaggcaag
gttcggacctactggctccttggggagagggggagtagcacccgaggctga (SEQ ID NO:321)

**P16066**
MPGPRRPAGSRLRLLLLLLLPPLLLLLRGSHAGNLTVAVVLPLANTSYPWSWARVGPAVELALAQ
VKARPDLLPGWTVRTVLGSSENALGVCSDTAAPLAAVDLKWEHNPAVFLGPGCVYAAAPVGRFT
AHWRVPLLTAGAPALGFGVKDEYALTTRAGPSYAKLGDFVAALHRRLGWERQALMLYAYRPGDE
EHCFFLVEGLFMRVRDRLNITVDHLEFAEDDLSHYTRLLRTMPRKGRVIYICSSPDAFRTLMLLALE
AGLCGEDYVFFHLDIFGQSLQGGQGPAPRRPWERGDGQDVSARQAFQAAKIITYKDPDNPEYLE
FLKQLKHLAYEQFNFTMEDGLVNTIPASFHDGLLLYIQAVTETLAHGGTVTDGENITQRMWNRSFQ
GVTGYLKIDSSGDRETDFSLWDMDPENGAFRVVLNYNGTSQELVAVSGRKLNWPLGYPPPDIPK
CGFDNEDPACNQDHLSTLEVLALVGSLSLLGILIVSFFIYRKMQLEKELASELWRVRWEDVEPSSL
ERHLRSAGSRLTLSGRGSNYGSLLTTEGQFQVFAKTAYYKGNLVAVKRVNRKRIELTRKVLFELK
HMRDVQNEHLTRFVGACTDPPNICILTEYCPRGSLQDILENESITLDWMFRYSLTNDIVKGMLFLH
NGAICSHGNLKSSNCVVDGRFVLKITDYGLESFRDLDPEQGHTVYAKKLWTAPELLRMASPPVRG
SQAGDVYSFGIILQEIALRSGVFHVEGLDLSPKEIIERVTRGEQPPFRPSLALQSHLEELGLLMQRC
WAEDPQERPPFQQIRLTLRKFNRENSSNILDNLLSRMEQYANNLEELVEERTQAYLEEKRKAEAL
LYQILPHSVAEQLKRGETVQAEAFDSVTIYFSDIVGFTALSAESTPMQVVTLLNDLYTCFDAVIDNF

DVYKVETIGDAYMVVSGLPVRNGRLHACEVARMALALLDAVRSFRIRHRPQEQLRLRIGIHTGPVC
AGVVGLKMPRYCLFGDTVNTASRMESNGEALKIHLSSETKAVLEEFGGFELELRGDVEMKGKGK
VRTYWLLGERGSSTRG (SEQ ID NO:322)

**Nucleotide:** AB005647
**Sequence:**
atggcgctgccatcacttctgctgttggtggcagccctggcaggtggggtgcgtcctcccggggcgcggaacctgacgctggcggtggtg
ctgccagaacacaacctgagctatgcctgggcctggccacgggtgggacccgctgtggcactagctgtggaggctctgggccgggcac
tgcccgtggacctgcggtttgtcagctccgaactggaaggcgcctgctctgagtacctggcaccgctgagcgctgtggacctcaagctgta
ccatgaccccgacctgctgttaggtcccggttgcgtgtaccctgctgcctctgtggcccgctttgcctcccactggcgccttcccctgctgactg
cgggtgctgtggcctctggtttttcggctaagaatgaccattatcgtaccctggttcgcactggcccctctgctcccaagctgggtgagtttgtg
gtgacactacacgggcacttcaattggactgcccgtgctgccttgctgtacctggatgctcgcacagatgaccggcctcactacttcaccat
cgagggcgtctttgaggccctgcagggcagcaacctcagtgtgcagcaccaggtgtatgcccgagagccaggggggccccgagcagg
ccacccacttcatccgggccaacgggcgcattgtgtatatctgcggccctctggagatgctgcatgagatcctgcttcaggcccagaggg
agaatctgaccaatggggattatgtcttcttttacctggatgtctttggggagagtctccgtgcaggccccacacgtgctacaggccggccct
ggcaggacaatcgcacccgggaacaggcccaggccctcagagaggcctttcagactgtattggtgatcacgtaccgagaacccccaa
atcctgagtatcaggaattccagaatcgtctgctgataagagcccgggaagactttggtgtggagctgggcccttccctgatgaacctcatc
gctggctgcttctatgatgggatcctgctatatgctgaagtcctgaatgagacaatacaggaaggaggcacccgggaggatggacttcga
attgtggaaaagatgcagggacgaagatatcacggtgtaactgggctggttgtcatggacaagaacaatgaccgagagactgactttgt
cctctgggccatgggagacctggattctggggactttcagcctgcagcccactactcgggagctgagaagcagatttggtggacgggac
ggcctattccctgggtgaaggggggctcctccctcggacaatccccctgtgcctttgacttggacgacccatcctgtgataaaactccacttt
caaccctggcaattgtggctctgggcacaggaatcaccttcatcatgtttggtgtttccagcttcctaattttccgaaagctgatgctggagaa
ggagctggctagcatgttgtggcgtattcgctgggaagaactgcagtttggcaactcagagcgttatcacaaaggtgcaggcagtcgcctc
acactgtcgctgcggggatccagttacggctcgctcatgacagcccatgggaaataccagatctttgccaacaccggtcacttcaaggga
aatgttgtcgccatcaaacatgtgaataagaagcgcattgagctgacccggcaggttctgtttgaactcaaacatatgagagatgttcagtt
caaccatctcactcgcttcattggcgcctgcatagaccctcccaacatttgcattgtcactgaatactgtcctcgtgggagtttacaggatattc
tagaaaatgacagcatcaacttggactggatgtttcgttattcactcattaatgaccttgttaagggcatggcctttctccacaacagcattattt
catcgcatgggagtctcaagtcctccaactgtgtggtggatagtcgttttgtgctcaaaatcacagactatggcctggccagcttccgatcaa
ctgctgaacctgatgacagccatgccctctatgccaagaagctgtggactgccccagaactgctcagtgggaaccccttgccaaccaca
ggcatgcagaaggctgacgtctatagctttgggatcatcctgcaggagatagcacttcgcagtggtcctttctacttggagggcctggacct
cagccccaaagagattgtccagaaggtacgaaatggtcagcggccatatttccggccaagcattgaccggtcccaactgaatgaagag
ctagttttgctgatggagcgatgttgggctcaggacccagctgagcggccagactttggacagattaagggcttcattcggcgctttaacaa
ggagggtggcaccagcatattggacaacctcctgctgcgcatggaacagtatgccaataacttggagaagctggtggaggaacgcaca
caggcctatctggaggaaaaacgcaaggctgaagctctgctctaccaaatcctaccccattcagtggcagagcagttaaaacggggag
agactgtacaggctgaggcctttgacagtgttaccatctacttcagtgacattgttggcttcacagcattgtcagcagagagcacccccatgc
aggtagtgacacttcttaatgacctgtatacctgctttgatgccataattgacaactttgatgtctacaaggtggagacgattggggatgcttac
atggtggtatctggcctcccaggccgaaatggtcaacgccatgcaccagaaattgctcgtatggccctagcattactagatgcagtttcttcc
tttcgcatccgccaccgaccccatgaccagctgaggctacgcataggggtccatactgggccagtctgtgctggggttgttggcctgaaga
tgccccgttattgtctttttggagacacagtgaacactgcttctcgaatggagtctaatggtcaagcgctgaagatccatgtctcctctaccacc
aaggatgccctagatgagctaggatgcttccagctagagcttcgggggggatgtggaaatgaagggaaaaggaaagatgcgaacatac
tggctcttaggagagcggaaaggacctcctggactcctgtaa (SEQ ID NO:323)

**P20594**
MALPSLLLLVAALAGGVRPPGARNLTLAVVLPEHNLSYAWAWPRVGPAVALAVEALGRALPVDLR
FVSSELEGACSEYLAPLSAVDLKLYHDPDLLLGPGCVYPAASVARFASHWRLPLLTAGAVASGFS
AKNDHYRTLVRTGPSAPKLGEFVVTLHGHFNWTARAALLYLDARTDDRPHYFTIEGVFEALQGSN
LSVQHQVYAREPGGPEQATHFIRANGRIVYICGPLEMLHEILLQAQRENLTNGDYVFFYLDVFGES
LRAGPTRATGRPWQDNRTREQAQALREAFQTVLVITYREPPNPEYQEFQNRLLIRAREDFGVELG
PSLMNLIAGCFYDGILLYAEVLNETIQEGGTREDGLRIVEKMQGRRYHGVTGLVVMDKNNDRETD
FVLWAMGDLDSGDFQPAAHYSGAEKQIWWTGRPIPWVKGAPPSDNPPCAFDLDDPSCDKTPLS
TLAIVALGTGITFIMFGVSSFLIFRKLMLEKELASMLWRIRWEELQFGNSERYHKGAGSRLTLSLRG
SSYGSLMTAHGKYQIFANTGHFKGNVVAIKHVNKKRIELTRQVLFELKHMRDVQFNHLTRFIGACI
DPPNICIVTEYCPRGSLQDILENDSINLDWMFRYSLINDLVKGMAFLHNSIISSHGSLKSSNCVVDS
RFVLKITDYGLASFRSTAEPDDSHALYAKKLWTAPELLSGNPLPTTGMQKADVYSFGIILQEIALRS
GPFYLEGLDLSPKEIVQKVRNGQRPYFRPSIDRTQLNEELVLLMERCWAQDPAERPDFGQIKGFI
RRFNKEGGTSILDNLLLRMEQYANNLEKLVEERTQAYLEEKRKAEALLYQILPHSVAEQLKRGETV
QAEAFDSVTIYFSDIVGFTALSAESTPMQVVTLLNDLYTCFDAIIDNFDVYKVETIGDAYMVVSGLP
GRNGQRHAPEIARMALALLDAVSSFRIRHRPHDQLRLRIGVHTGPVCAGVVGLKMPRYCLFGDTV
NTASRMESNGQALKIHVSSTTKDALDELGCFQLELRGDVEMKGKGKMRTYWLLGERKGPPGLL
(SEQ ID NO:324)

# Figure 143

**Nucieotide:** AY033889
**Sequence:**
atgcaaagttgtgaatccagtggtgacagtgcggatgaccctctcagtcgcggcctacggagaaggggacagcctcgtgtggtggtgatc
ggcgccggcttggctggcctggctgcagccaaagcacttcttgagcagggtttcacggatgtcactgtgcttgaggcttccagccacatcg
gaggccgtgtgcagagtgtgaaacttggacacgccacctttgagctgggagccacctggatccatggctcccatgggaaccctatctatc
atctagcagaagccaacggcctcctggaagagacaaccgatggggaacgcagcgtgggccgcatcagcctctattccaagaatggcg
tggcctgctaccttaccaaccacggccgcaggatccccaaggacgtggttgaggaattcagcgatttatacaacgaggtctataacttgac
ccaggagttcttccggcacgataaaccagtcaatgctgaaagtcaaaatagcgtgggggtgttcacccgagaggaggtgcgtaaccgc
atcaggaatgaccctgacgacccagaggctaccaagcgcctgaagctcgccatgatccagcagtacctgaaggtggagagctgtgag
agcagctcacacagcatggacgaggtgtccctgagcgccttcggggagtggaccgagatccccggcgctcaccacatcatcccctcgg
gcttcatgcgggttgtggagctgctggcggagggcatccctgcccacgtcatccagctagggaaacctgtccgctgcattcactgggacc
aggcctcagcccgccccagaggccctgagattgagccccggggtgagggcgaccacaatcacgacactggggagggtggccaggg
tggagaggagccccgggggggcaggtgggatgaggatgagcagtggtcggtggtggtggagtgcgaggaccgtgagctgatcccgg
cggaccatgtgattgtgaccgtgtcgctaggtgtgctaaagaggcagtacaccagtttcttccggccaggcctgcccacagagaaggtgg
ctgccatccaccgcctgggcattggcaccaccgacaagatctttctggaattcgaggagcccttctggggccctgagtgcaacagcctac
agtttgtgtgggaggacgaagcggagagccacaccctcacctacccacctgagctctggtaccgcaagatctgcggctttgatgtcctcta
cccgcctgagcgctacggccatgtgctgagcggctggatctgcggggaggaggccctcgtcatggagaagtgtgatgacgaggcagtg
gccgagatctgcacggagatgctgcgtcagttcacagggaaccccaacattccaaaacctcggcgaatcttgcgctcggcctggggca
gcaacccttacttccgtggctcctattcatacacgcaggtgggctccagcggggcggatgtggagaagctggccaagcccctgccgtac
acggagagctcaaagacagcgcccatgcaggtgctgttttccggtgaggccacccaccgcaagtactattccaccacccacggtgctct
gctgtccggccagcgtgaggctgcccgcctcattgagatgtaccgagacctcttccagcaggggacctga (SEQ ID NO:325)

**Q96QT3**
MQSCESSGDSADDPLSRGLRRRGQPRVVVIGAGLAGLAAAKALLEQGFTDVTVLEASSHIGGRV
QSVKLGHATFELGATWIHGSHGNPIYHLAEANGLLEETTDGERSVGRISLYSKNGVACYLTNHGR
RIPKDVVEEFSDLYNEVYNLTQEFFRHDKPVNAESQNSVGVFTREEVRNRIRNDPDDPEATKRLK
LAMIQQYLKVESCESSSHSMDEVSLSAFGEWTEIPGAHHIIPSGFMRVVELLAEGIPAHVIQLGKPV
RCIHWDQASARPRGPEIEPRGEGDHNHDTGEGGQGGEEPRGGRWDEDEQWSVVVECEDRELI
PADHVIVTVSLGVLKRQYTSFFRPGLPTEKVAAIHRLGIGTTDKIFLEFEEPFWGPECNSLQFVWE
DEAESHTLTYPPELWYRKICGFDVLYPPERYGHVLSGWICGEEALVMEKCDDEAVAEICTEMLRQ
FTGNPNIPKPRRILRSAWGSNPYFRGSYSYTQVGSSGADVEKLAKPLPYTESSKTAPMQVLFSGE
ATHRKYYSTTHGALLSGQREAARLIEMYRDLFQQGT (SEQ ID NO:326)

# Figure 144

**Nucleotide:** AB051390
**Sequence:**
atgaggctgtccccggcgcccctgaagctgagccggactccggcactgctggccctggcgctgcccctggccgcggcgctggccttctc
cgacgagaccctggacaaagtgcccaagtcagagggctactgtagccgtatcctgcgcgcccagggcacgcggcgcgagggctaca
ccgagttcagcctccgcgtggagggcgaccccgacttctacaagccgggaaccagctaccgcgtaacactttcagctgctcctccctcct
acttcagaggattcacattaattgccctcagagagaacagagagggtgataaggaagaagaccatgctgggaccttccagatcataga
cgaagaagaaactcagtttatgagcaattgccctgttgcagtcactgaaagcactccacggaggaggacccggatccaggtgttttggat
agcaccaccagcgggaacaggctgcgtgattctgaaggccagcatcgtacaaaaacgcattatttattttcaagatgagggctctctgac
caagaaactttgtgaacaagattccacatttgatggggtgactgacaaacccatcttagactgctgtgcctgcggaactgccaagtacaga
ctcacattttatgggaattggtccgagaagacacacccaaaggattaccctcgtcgggccaaccactggtctgcgatcatcggaggatcc
cactccaagaattatgtactgtgggaatatggaggatatgccagcgaaggcgtcaaacaagttgcagaattgggctcacccgtgaaaat
ggaggaagaaattcgacaacagagtgatgaggtcctcaccgtcatcaaagccaaagcccaatggccagcctggcagcctctcaacgt
gagagcagcaccttcagctgaattttccgtggacagaacgcgccatttaatgtccttcctgaccatgatgggccctagtcccgactggaac
gtaggcttatctgcagaagatctgtgcaccaaggaatgtggctgggtccagaaggtggtgcaagacctgattccctgggacgctggcacc
gacagcggggtgacctatgagtcacccaacaaacccaccattccccaggagaaaatccggcccctgaccagcctggaccatcctcag
agtcctttctatgacccagagggtgggtccatcactcaagtagccagagttgtcatcgagagaatcgcacggaagggtgaacaatgcaat
attgtacctgacaatgtcgatgatattgtagctgacctggctccagaagagaaagatgaagatgacacccctgaaacctgcatctactcca
actggtccccatggtccgcctgcagctcctccacctgtgacaaaggcaagaggatgcgacagcgcatgctgaaagcacagctggacct
cagcgtcccctgccctgacacccaggacttccagccctgcatgggccctggctgcagtgacgaagacggctccacctgcaccatgtccg
agtggatcacctggtcgccctgcagcatctcctgcggcatgggcatgaggtcccgggagaggtatgtgaagcagttcccggaggacgg
ctccgtgtgcacgctgcccactgaggaaatggagaagtgcacggtcaacgaggagtgctctcccagcagctgcctgatgaccgagtgg
ggcgagtgggacgagtgcagcgccacctgcggcatgggcatgaagaagcggcaccgcatgatcaagatgaaccccgcagatggct
ccatgtgcaaagccgagacatcacaggcagagaagtgcatgatgccagagtgccacaccatcccatgcttgctgtccccatggtccga
gtggagtgactgcagcgtgacctgcgggaagggcatgcgaacccgacagcggatgctcaagtctctggcagaacttggagactgcaat
gaggatctggagcaggtggagaagtgcatgctccctgaatgccccattgactgtgagctcaccgagtggtcccagtggtcggaatgtaac
aagtcatgtgggaaaggccacgtgattcgaacccggatgatccaaatggagcctcagtttggaggtgcaccctgcccagagactgtgca
gcgaaaaaagtgccgcatccgaaaatgccttcgaaatccatccatccaaaagccacgctggagggaggcccgagagagccggcgg
agtgagcagctgaaggaagagtctgaaggggagcagttcccaggttgtaggatgcgcccatggacggcctggtcagaatgcaccaaa
ctgtgcggaggtggaattcaggaacgttacatgactgtaaagaagagattcaaaagctcccagtttaccagctgcaaagacaagaagg
agatcagagcatgcaatgttcatccttgttag (SEQ ID NO:327)

**Q9HCB6**
MRLSPAPLKLSRTPALLALALPLAAALAFSDETLDKVPKSEGYCSRILRAQGTRREGYTEFSLRVE
GDPDFYKPGTSYRVTLSAAPPSYFRGFTLIALRENREGDKEEDHAGTFQIIDEEETQFMSNCPVAV
TESTPRRRTRIQVFWIAPPAGTGCVILKASIVQKRIIYFQDEGSLTKKLCEQDSTFDGVTDKPILDCC
ACGTAKYRLTFYGNWSEKTHPKDYPRRANHWSAIIGGSHSKNYVLWEYGGYASEGVKQVAELG
SPVKMEEEIRQQSDEVLTVIKAKAQWPAWQPLNVRAAPSAEFSVDRTRHLMSFLTMMGPSPDW
NVGLSAEDLCTKECGWVQKVVQDLIPWDAGTDSGVTYESPNKPTIPQEKIRPLTSLDHPQSPFYD
PEGGSITQVARVVIERIARKGEQCNIVPDNVDDIVADLAPEEKDEDDTPETCIYSNWSPWSACSSS
TCDKGKRMRQRMLKAQLDLSVPCPDTQDFQPCMGPGCSDEDGSTCTMSEWITWSPCSISCGM
GMRSRERYVKQFPEDGSVCTLPTEEMEKCTVNEECSPSSCLMTEWGEWDECSATCGMGMKKR
HRMIKMNPADGSMCKAETSQAEKCMMPECHTIPCLLSPWSEWSDCSVTCGKGMRTRQRMLKSL
AELGDCNEDLEQVEKCMLPECPIDCELTEWSQWSECNKSCGKGHVIRTRMIQMEPQFGGAPCP
ETVQRKKCRIRKCLRNPSIQKPRWREARESRRSEQLKEESEGEQFPGCRMRPWTAWSECTKLC
GGGIQERYMTVKKRFKSSQFTSCKDKKEIRACNVHPC (SEQ ID NO:328)

# Figure 145

**Nucleotide:** AF410459
**Sequence:**
atgcagggcccaccgctcctgaccgccgcccacctcctctgcgtgtgcaccgccgcgctggccgtggctcccgggcctcggtttctggtga
cagccccagggatcatcaggcccggaggaaatgtgactattggggtggagcttctggaacactgcccttcacaggtgactgtgaaggcg
gagctgctcaagacagcatcaaacctcactgtctctgtcctggaagcagaaggagtctttgaaaaaggctcttttaagacacttactcttcc
atcactacctctgaacagtgcagatgagatttatgagctacgtgtaaccggacgtacccaggatgagattttattctctaatagtacccgctta
tcatttgagaccaagagaatatctgtcttcattcaaacagacaaggccttatacaagccaaagcaagaagtgaagtttcgcattgttacact
cttctcagattttaagccttacaaaacctctttaaacattctcattaaggaccccaaatcaaatttgatccaacagtggttgtcacaacaaagt
gatcttggagtcatttccaaaacttttcagctatcttcccatccaatacttggtgactggtctattcaagttcaagtgaatgaccagacatattatc
aatcatttcaggtttcagaatatgtattaccaaaatttgaagtgactttgcagacaccattatattgttctatgaattctaagcatttaaatggtacc
atcacggcaaagtatacatatgggaagccagtgaaaggagacgtaacgcttacatttttacctttatccttttggggaaagaagaaaaata
ttacaaaaacatttaagataaatggatctgcaaacttctcttttaatgatgaagagatgaaaaatgtaatggattcttcaaatggactttctgaa
tacctggatctatcttcccctggaccagtagaaattttaaccacagtgacagaatcagttacaggtatttcaagaaatgtaagcactaatgtg
ttcttcaagcaacatgattacatcattgagtttttgattatactactgtcttgaagccatctctcaacttcacagccactgtgaaggtaactcgtg
ctgatggcaaccaactgactcttgaagaaagaagaaataatgtagtcataacagtgacacagagaaactatactgagtactggagcgg
atctaacagtggaaatcagaaaatggaagctgttcagaaaataaattatactgtcccccaaagtggaacttttaagattgaattcccaatcc
tggaggattccagtgagctacagttgaaggcctatttccttggtagtaaaagtagcatggcagttcatagtctgtttaagtctcctagtaagac
atacatccaactaaaaacaagagatgaaaatataaaggtgggatcgccttttgagttggtggttagtggcaacaaacgattgaaggagtt
aagctatatggtagtatccaggggacagttggtggctgtaggaaaacaaaattcaacaatgttctctttaacaccagaaaattcttggactc
caaaagcctgtgtaattgtgtattatattgaagatgatggggaaattataagtgatgttctaaaaattcctgttcagcttgtttttaaaaataagat
aaagctatattggagtaaagtgaaagctgaaccatctgagaaagtctctcttaggatctctgtgacacagcctgactccatagttgggattgt
agctgttgacaaaagtgtgaatctgatgaatgcctctaatgatattacaatggaaaatgtggtccatgagttggaactttataacacaggata
ttatttaggcatgttcatgaattcttttgcagtctttcaggaatgtggactctgggtattgacagatgcaaacctcacgaaggattatattgatggt
gtttatgacaatgcagaatatgctgagaggtttatggaggaaaatgaaggacatattgtagatattcatgacttttctttgggtagcagtccac
atgtccgaaagcattttccagagacttggatttggctagacaccaacatgggttacaggatttaccaagaatttgaagtaactgtacctgatt
ctatcacttcttgggtggctactggttttgtgatctctgaggacctgggtcttggactaacaactactccagtggagctccaagccttccaacca
tttttcatttttttgaatcttccctactctgttatcagaggtgaagaatttgctttggaaataactatattcaattatttgaaagatgccactgaggtta
aggtaatcattgagaaaagtgacaaatttgatattctaatgacttcaaatgaaataaatgccacaggccaccagcagacccttctggttccc
agtgaggatggggcaactgttctttttcccatcaggccaacacatctgggagaaattcctatcacagtcacagctctttcacccactgcttctg
atgctgtcacccagatgatttagtaaaggctgaaggaatagaaaaatcatattcacaatccatcttattagacttgactgacaataggctac
agagtaccctgaaaactttgagtttctcatttcctcctaatacagtgactggcagtgaaagagttcagatcactgcaattggagatgttcttggt
ccttccatcaatggcttagcctcattgattcggatgccttatggctgtggtgaacagaacatgataaattttgctccaaatatttacattttggatt
atctgactaaaaagaaacaactgacagataatttgaaagaaaaagctctttcatttatgaggcaaggttaccagagagaacttctctatca
gagggaagatggctctttcagtgcttttgggaattatgacccttctgggagcacttggttgtcagcttttgttttaagatgtttccttgaagccgatc
cttacatagatattgatcagaatgtgttacacagaacatacacttggcttaaaggacatcagaaatccaacggtgaattttgggatccagga
agagtgattcatagtgagcttcaaggtggcaataaaagtccagtaacacttacagcctatattgtaacttctctcctgggatatagaaagtat
cagcctaacattgatgtgcaagagtctatccatttttggagtctgaattcagtagaggaatttcagacaattatactctagcccttataacttat
gcattgtcatcagtggggagtcctaaagcgaaggaagctttgaatatgctgacttggagagcagaacaagaaggtggcatgcaattctg
ggtgtcatcagagtccaaactttctgactcctggcagccacgctccctggatattgaagttgcagcctatgcactgctctcacacttcttacaa
tttcagacttctgagggaatcccaattatgaggtggctaagcaggcaaagaaatagcttgggtggttttgcatctactcaggataccactgtg
gctttaaaggctctgtctgaatttgcagccctaatgaatacagaaaggacaaatatccaagtgaccgtgacggggcctagctcaccaagt
cctgtaaagtttctgattgacacacacaaccgcttactccttcagacagcagagcttgctgtggtacagccaatggcagttaatatttccgca
aatggttttggatttgctatttgtcagctcaatgttgtatataatgtgaaggcttctgggtcttctagaagacgaagatctatccaaaatcaagaa
gcctttgatttagatgttgctgtaaaagaaaataaagatgatctcaatcatgtggatttgaatgtgtgtacaagcttttcgggcccgggtagga
gtggcatggctcttatggaagttaacctattaagtggctttatggtgccttcagaagcaatttctctgagcgagacagtgaagaaagtggaat
atgatcatggaaaactcaacctctatttagattctgtaaatgaaacccagttttgtgttaatattcctgctgtgagaaactttaaagtttcaaatac
ccaagatgcttcagtgtccatagtggattactatgagccaaggagacaggcggtgagaagttacaactctgaagtgaagctgtcctcctgt
gacctttgcagtgatgtccagggctgccgtccttgtgaggatggagcttcaggctcccatcatcactcttcagtcattttatttctgtttcaagctt
ctgtactttatggaactttggctgtga (SEQ ID NO:329)

**Q8TDJ3**
MQGPPLLTAAHLLCVCTAALAVAPGPRFLVTAPGIIRPGGNVTIGVELLEHCPSQVTVKAELLKTAS
NLTVSVLEAEGVFEKGSFKTLTLPSLPLNSADEIYELRVTGRTQDEILFSNSTRLSFETKRISVFIQT
DKALYKPKQEVKFRIVTLFSDFKPYKTSLNILIKDPKSNLIQQWLSQQSDLGVISKTFQLSSHPILGD
WSIQVQVNDQTYYQSFQVSEYVLPKFEVTLQTPLYCSMNSKHLNGTITAKYTYGKPVKGDVTLTF
LPLSFWGKKKNITKTFKINGSANFSFNDEEMKNVMDSSNGLSEYLDLSSPGPVEILTTVTESVTGIS
RNVSTNVFFKQHDYIIEFFDYTTVLKPSLNFTATVKVTRADGNQLTLEERRNNVVITVTQRNYTEY
WSGSNSGNQKMEAVQKINYTVPQSGTFKIEFPILEDSSELQLKAYFLGSKSSMAVHSLFKSPSKTY
IQLKTRDENIKVGSPFELVVSGNKRLKELSYMVVSRGQLVAVGKQNSTMFSLTPENSWTPKACVI
VYYIEDDGEIISDVLKIPVQLVFKNKIKLYWSKVKAEPSEKVSLRISVTQPDSIVGIVAVDKSVNLMNA
SNDITMENVVHELELYNTGYYLGMFMNSFAVFQECGLWVLTDANLTKDYIDGVYDNAEYAERFM
EENEGHIVDIHDFSLGSSPHVRKHFPETWIWLDTNMGYRIYQEFEVTVPDSITSWVATGFVISEDL
GLGLTTTPVELQAFQPFFIFLNLPYSVIRGEEFALEITIFNYLKDATEVKVIIEKSDKFDILMTSNEINA
TGHQQTLLVPSEDGATVLFPIRPTHLGEIPITVTALSPTASDAVTQMILVKAEGIEKSYSQSILLDLTD
NRLQSTLKTLSFSFPPNTVTGSERVQITAIGDVLGPSINGLASLIRMPYGCGEQNMINFAPNIYILDY
LTKKKQLTDNLKEKALSFMRQGYQRELLYQREDGSFSAFGNYDPSGSTWLSAFVLRCFLEADPYI
DIDQNVLHRTYTWLKGHQKSNGEFWDPGRVIHSELQGGNKSPVTLTAYIVTSLLGYRKYQPNIDV
QESIHFLESEFSRGISDNYTLALITYALSSVGSPKAKEALNMLTWRAEQEGGMQFWVSSESKLSD
SWQPRSLDIEVAAYALLSHFLQFQTSEGIPIMRWLSRQRNSLGGFASTQDTTVALKALSEFAALMN
TERTNIQVTVTGPSSPSPVKFLIDTHNRLLLQTAELAVVQPMAVNISANGFGFAICQLNVVYNVKAS
GSSRRRRSIQNQEAFDLDVAVKENKDDLNHVDLNVCTSFSGPGRSGMALMEVNLLSGFMVPSEA
ISLSETVKKVEYDHGKLNLYLDSVNETQFCVNIPAVRNFKVSNTQDASVSIVDYYEPRRQAVRSYN
SEVKLSSCDLCSDVQGCRPCEDGASGSHHHSSVIFIFCFKLLYFMELWL (SEQ ID NO:330)

# Figure 146

**Nucleotide:** Z92867
**Sequence:**
atgagcgagctagaggaagactttgccaagattctcatgctcaaggaggagaggatcaaagagctggagaagcggctgtcagagaa
ggaggaagaaattcaggagctgaagaggaaactccacaaatgccagtcggtgctcccagtgccctcgacccacatcggcccccgga
ccacccgggcgcagggcatctcggccgagccgcagacgtacaggtccttccacgacctccgacaggcattccggaagttcaccaagt
ccgaaaggtccaaggatcttataaaggaagctatccttgacaatgactttatgaagaacttggagctgtcgcagatccaggagattgtgga
ttgtatgtacccggtggagtatggcaaggacagttgcatcatcaaagaaggagacgtggggtcactggtgtatgtcatggaagatggtaa
ggttgaagttacaaaagaaggtgtgaagttgtgtaccatgggtccaggaaaagtgtttggggaattggctattctttacaactgtacccgga
cagcgaccgtcaagactcttgtaaatgtaaaactctgggccattgatcgacaatgttttcaaacaataatgatgaggacaggactcatcaa
gcataccgagtatatggaatttttaaaaagcgttccaacattccagagccttcctgaagagatcctcagcaagcttgctgatgtccttgaaga
gacccactatgaaaatggagaatatattatcaggcaaggtgcaagaggggacaccttctttatcatcagcaaaggaacggtaaatgtca
ctcgtgaagactcaccgagtgaagacccagtctttcttagaactttaggaaaaggagactggtttggagagaaagccttgcaggggga
gatgtgagaacagcaaacgtaattgctgcagaagctgtaacctgccttgtgattgacagagactcttttaaacatttgattggagggctggat
gatgtttctaataaagcatatgaagatgcagaagctaaagcaaaatatgaagctgaagcggctttcttcgccaacctgaagctgtctgattt
caacatcattgataccettggagttggaggtttcggacgagtagaactggtccagttgaaaagtgaagaatccaaaacgtttgcaatgaag
attctcaagaaacgtcacattgtggacacaagacagcaggagcacatccgctcagagaagcagatcatgcagggggctcattccgattt
catagtgagactgtacagaacatttaaggacagcaaatatttgtatatgttgatggaagcttgtctaggtggagagctctggaccattctcag
ggatagaggttcgtttgaagattctacaaccagattttacacagcatgtgtggtagaagcttttgcctatctgcattccaaaggaatcatttaca
gggacctcaagccagaaaatctcatcctagatcaccgaggttatgccaaactggttgattttggctttgcaaagaaaataggatttggaaa
gaaaacatggactttttgtgggactccagagtatgtagccccagagatcatcctgaacaaaggccatgacatttcagccgactactggtca
ctgggaatcctaatgtatgaactcctgactggcagcccacctttctcaggcccagatcctatgaaaacctataacatcatattgaggggat
tgacatgatagaatttccaaagaagattgccaaaaatgctgctaatttaattaaaaaactatgcagggacaatccatcagaaagattagg
gaatttgaaaaatggagtaaaagacattcaaaagcacaaatggtttgagggctttaactgggaaggcttaagaaaaggtaccttgacac
ctcctataataccaagtgttgcatcacccacagacacaagtaattttgacagtttccctgaggacaacgatgaaccaccacctgatgacaa
ctcaggatgggatatagacttctaa (SEQ ID NO:331)

**Q13976**
MSELEEDFAKILMLKEERIKELEKRLSEKEEEIQELKRKLHKCQSVLPVPSTHIGPRTTRAQGISAEP
QTYRSFHDLRQAFRKFTKSERSKDLIKEAILDNDFMKNLELSQIQEIVDCMYPVEYGKDSCIIKEGD
VGSLVYVMEDGKVEVTKEGVKLCTMGPGKVFGELAILYNCTRTATVKTLVNVKLWAIDRQCFQTI
MMRTGLIKHTEYMEFLKSVPTFQSLPEEILSKLADVLEETHYENGEYIIRQGARGDTFFIISKGTVNV
TREDSPSEDPVFLRTLGKGDWFGEKALQGEDVRTANVIAAEAVTCLVIDRDSFKHLIGGLDDVSN
KAYEDAEAKAKYEAEAAFFANLKLSDFNIIDTLGVGGFGRVELVQLKSEESKTFAMKILKKRHIVDT
RQQEHIRSEKQIMQGAHSDFIVRLYRTFKDSKYLYMLMEACLGGELWTILRDRGSFEDSTTRFYT
ACVVEAFAYLHSKGIIYRDLKPENLILDHRGYAKLVDFGFAKKIGFGKKTWTFCGTPEYVAPEIILNK
GHDISADYWSLGILMYELLTGSPPFSGPDPMKTYNIILRGIDMIEFPKKIAKNAANLIKKLCRDNPSE
RLGNLKNGVKDIQKHKWFEGFNWEGLRKGTLTPPIIPSVASPTDTSNFDSFPEDNDEPPPDDNSG
WDIDF (SEQ ID NO:332)

# Figure 147

**Nucleotide:** AJ133123
**Sequence:**
atggcttccccgccccaccagcagctgctgcatcaccacagcaccgaggtgagctgcgactccagcggggacagcaacagcgtgcg
cgtcaagatcaaccccaagcagctgtcctccaacagccaccccaagcactgcaaatacagcatctcctctagctgcagcagctctggg
gactccgggggcgtcccccggcgagtgggcggcggaggccggctgcgcaggcagaagaagctgccccagctgttcgagagggcct
ccagccgctggtgggaccccaagttcgactcggtgaacctggaggaggcctgcctggagcgctgcttcccgcagacccagcgccggtt
ccggtatgcgctcttctacatcggcttcgcctgccttctgtggagcatctattttgcggtccacatgagatccagactgatcgtcatggtcgccc
ccgcgctgtgcttcctcctggtgtgtgtgggcttctttctgtttaccttcaccaagctgtacgcccggcattacgcgtggacctcgctggctctca
ccctgctggtgttcgccctgaccctggctgcgcagttccaggtcttgacgcctgtctcaggacgcggcgacagctccaaccttacggccac
agcccggcccacagatacttgcttatctcaagtggggagcttctccatgtgcatcgaagtgctctttttgctctataccgtcatgcacttaccttt
gtacctgagtttgtgtctgggggtggcctactctgtccttttcgagacctttggctaccatttccgggatgaagcctgcttcccctcgcccggag
ccggggccctgcactgggagctgctgagcagggggctgctccacggctgcatccacgccatcggggtccacctgttcgtcatgtcccag
gtgaggtccaggagcaccttcctcaaggtggggcaatccattatgcacggaaggacctggaagtggaaaaagccctcaaagagag
gatgattcattccgtgatgccaagaatcatagccgatgacttaatgaagcagggagatgaggagagtgagaattctgtcaagagacatg
ccacctcgagccccaagaacaggaagaaaaagtcttccatccaaaaagctcctatagccttccgcccttttaagatgcagcagatcgaa
gaagtcagtattttatttgcagatatcgtgggcttcaccaagatgagtgccaacaagtctgcccacgccctggtgggtctcctgaacgatctg
ttcggtcgcttcgaccgcctgtgtgaggagaccaagtgtgagaaaatcagcaccctgggagactgttactactgcgtggcgggctgtccc
gagccccgggccgaccatgcctactgctgcatcgagatgggcctgggcatgatcaaggccatcgagcagttctgccaggagaagaag
gagatggtgaacatgagagtcggggtgcacacgcgcaccgtcctttgcggcatcctgggcatgaggaggtttaaatttgacgtgtggtcc
aacgatgtgaacctggccaatctcatggagcagctgggagtggccggcaaagttcacatttctgaggccaccgcaaaatacttagatga
ccggtacgaaatggaagatgggaaagttattgaacggctgggccagagcgtggttgctgaccagttgaaaggtttgaagacatacctga
tatcgggtcagagagccaaggagtctcgctgcagctgtgcagaggccttgctttctggctttgaggtcattgacggctcacaggtgtcctca
ggccctaggggacaggggacagcgtcatcagggaatgtcagtgacttggcgcagactgtcaaaacctttgataaccttaagacctgccc
ttcgtgcggaatcacatttgctcccaaatctgaagccggcgccgagggaggagcacctcaaaacggctgccaagacgagcataaaaa
cagcaccaaggcttctggaggacctaatcccaaaactcagaacgggctcctcagccctccccaagaggagaagctcaccaacagtca
gacttctctgtgtgagatcttgcaggagaagggaaggtgggcaggggtgagcctggaccagtcggctctccttccgctgaggttcaagaa
catccgggagaaaacggacgcccactttgtggacgttatcaaagaagacagcctgatgaaagattactttttaagccgcccattaatcag
ttcagcctgaacttcctggatcaggagctggagcgatcctacaggaccagctatcaggaagaggtcataaagaactcccccgtgaaga
cgtttgctagtcccaccttcagctccctcctggatgtgtttctgtcgaccacagtgtttctgacgctgtccaccacctgcttcctgaagtacgagg
cggccaccgtgcctcccccgcccgccgccctggcggtcttcagtgcagccctgctgctggaggtgctgtccctcgcggtgtccatcaggat
ggtgttcttcctggaggacgtcatggcctgcaccaagcgcctgctggagtggatcgccggctggctaccacgtcactgcatcggggccat
cctggtgtcgcttcccgcactggccgtctactcccatgcaacctccgaatatgagaccaacatacacttcccagtgttcacaggctcggccg
cgctgattgccgtcgtgcactactgtaacttctgccagctcagctcctggatgaggtcctccctcgccaccgtcgtgggggccggccgctg
ctcctgctctacgtctccctgtgcccagacagttctgtattaacttcgccccttgacgcagtacagaatttcagttccgagaggaacccgtgca
atagttcggtgccgcgtgacctccggcggcccgccagcctcatcggccaggaggtggttctcgtcttctttctcctgctcttgttggtctggttcc
tgaatcgcgaatttgaagtcagctaccgcctccactaccacggagacgtggaagcggatcttcaccgcaccaagatccagagcatgcg
ggaccaggcagactggctgctgaggaacatcatcccctaccacgtggctgagcagctgaaggtgtcccagacctactccaagaacca
cgacagcggaggggtgatcttcgccagcatcgtcaacttcagcgagttctacgaggagaactacgagggcggcaaggagtgctaccg
ggtcctcaacgagctcatcggggactttgacgagctcctaagcaagccggactacagcagcatcgagaagatcaagaccatcggagc
cacgtacatggcggcgtcagggctgaacaccgcgcaggcccaggacggcagccacccgcaggagcacctgcagatcctgttcgagtt
cgccaaggagatgatgcgcgtggtggacgacttcaacagcaacatgctgtggttcaacttcaagctccgcgtcggcttcaaccatggcc
cctcacggccggggtcatcggcaccaccaagctgctgtacgacatctggggagacaccgtcaacatcgccagcaggatggacaccac
cggcgtggagtgccgcatccaggtgagcgaagagagctaccgcgtcttgagcaagatgggctatgacttcgactacagagggaccgtg
aatgtcaaggggaaaggccagatgaagacctacctgtacccaaagtgcacggatcacagggtcatcccacagcaccagctgtccatct
ccccagacatccgcgtccaggtggatggcagcatcggacggtctcccacagacgagattgccaacctggtgccttctgtccagtatgtgg
acaagacatctctgggttctgacagcagcacgcaggccaaggatgcccacctgtcccgcaagagaccgtggaaggagcccgtcaaa
gccgaagaaaggggtcgatttggcaaagccatagagaaagacgactgtgacgaaacaggaatagaagaagccaacgaactcacc
aagctcaacgtttcaaagagtgtgtga (SEQ ID NO:333)

**O60503**
MASPPHQQLLHHHSTEVSCDSSGDSNSVRVKINPKQLSSNSHPKHCKYSISSSCSSSGDSGGVP
RRVGGGGRLRRQKKLPQLFERASSRWWDPKFDSVNLEEACLERCFPQTQRRFRYALFYIGFACL
LWSIYFAVHMRSRLIVMVAPALCFLLVCVGFFLFTFTKLYARHYAWTSLALTLLVFALTLAAQFQVL
TPVSGRGDSSNLTATARPTDTCLSQVGSFSMCIEVLFLLYTVMHLPLYLSLCLGVAYSVLFETFGY
HFRDEACFPSPGAGALHWELLSRGLLHGCIHAIGVHLFVMSQVRSRSTFLKVGQSIMHGKDLEVE

KALKERMIHSVMPRIIADDLMKQGDEESENSVKRHATSSPKNRKKKSSIQKAPIAFRPFKMQQIEE
VSILFADIVGFTKMSANKSAHALVGLLNDLFGRFDRLCEETKCEKISTLGDCYYCVAGCPEPRADH
AYCCIEMGLGMIKAIEQFCQEKKEMVNMRVGVHTGTVLCGILGMRRFKFDVWSNDVNLANLMEQ
LGVAGKVHISEATAKYLDDRYEMEDGKVIERLGQSVVADQLKGLKTYLISGQRAKESRCSCAEALL
SGFEVIDGSQVSSGPRGQGTASSGNVSDLAQTVKTFDNLKTCPSCGITFAPKSEAGAEGGAPQN
GCQDEHKNSTKASGGPNPKTQNGLLSPPQEEKLTNSQTSLCEILQEKGRWAGVSLDQSALLPLR
FKNIREKTDAHFVDVIKEDSLMKDYFFKPPINQFSLNFLDQELERSYRTSYQEEVIKNSPVKTFASP
TFSSLLDVFLSTTVFLTLSTTCFLKYEAATVPPPPAALAVFSAALLLEVLSLAVSIRMVFFLEDVMAC
TKRLLEWIAGWLPRHCIGAILVSLPALAVYSHVTSEYETNIHFPVFTGSAALIAVVHYCNFCQLSSW
MRSSLATVVGAGPLLLLYVSLCPDSSVLTSPLDAVQNFSSERNPCNSSVPRDLRRPASLIGQEVVL
VFFLLLLLVWFLNREFEVSYRLHYHGDVEADLHRTKIQSMRDQADWLLRNIIPYHVAEQLKVSQTY
SKNHDSGGVIFASIVNFSEFYEENYEGGKECYRVLNELIGDFDELLSKPDYSSIEKIKTIGATYMAAS
GLNTAQAQDGSHPQEHLQILFEFAKEMMRVVDDFNNNMLWFNFKLRVGFNHGPLTAGVIGTTKL
LYDIWGDTVNIASRMDTTGVECRIQVSEESYRVLSKMGYDFDYRGTVNVKGKGQMKTYLYPKCT
DHRVIPQHQLSISPDIRVQVDGSIGRSPTDEIANLVPSVQYVDKTSLGSDSSTQAKDAHLSPKRPW
KEPVKAEERGRFGKAIEKDDCDETGIEEANELTKLNVSKSV (SEQ ID NO:334)

**Nucleotide:** AF033861
**Sequence:**
atgccgaggaaccagggcttctccgagcccgaatactcggccgagtactcagccgagtactccgtcagcctgccctccgaccctgaccg
cggggtgggccggacccatgaaatctcggtccggaactcgggctcctgcctgtgcctgcctcgcttcatgcggctgactttcgtgccggagt
ccttggagaacctctaccagacctacttcaaaaggcagcgccacgagaccctgctggtgctggtggtctttgcagccctctttgactgctac
gtggtggtcatgtgtgctgtggtcttctccagcgacaagctggctcccctcgccgtggctggaattggactggtgttggacatcatcctcttcgt
gctctgcaaaaaggggctgctcccggaccgggtcacccgcagagtgctgccctacgtgctgtggctgctcataaccgcccagatcttctc
ctacctgggcctgaacttcgcgcgtgcccacgcggctagtgacacggtgggctggcaggtcttctttgtcttctccttcttcatcacgctgcccc
tcagcctcagccccatcgtgatcatctccgtggtctcctgtgtggtgcacacgttggtcctgggggtcaccgtggcccagcagcagcagga
ggagctcaaggggatgcagctgctgcgggagatcctggccaacgtcttcctctacctgtgcgccatcgctgtgggcatcatgtcctactac
atggctgaccgcaagcaccgcaaggccttcctggaggcccgccagtcgctggaggtgaagatgaacctggaagagcagagccagca
gcaggagaacctcatgctttccatcctgcccaagcacgtggctgacgagatgctgaaagacatgaagaaagacgagagccagaagg
accagcagcagttcaacaccatgtacatgtaccgtcacgagaacgtcagcatcctctttgccgacatcgtgggctttacccagctgtcttctg
cctgcagtgcccaggagcttgtgaagctgctcaacgagctctttgcccgctttgacaagctggcagctaaataccaccagctgcggattaa
gatcctgggcgactgctactactgcatctgcggcttgcctgactaccgggaggaccacgccgtctgctccatcctcatggggctggccatg
gtggaggccatctcgtatgtgcgggagaagaccaagactggggtggacatgcgtgtgggggtgcacacgggcaccgtgctgggggc
gtcctgggccagaagcgctggcagtacgacgtgtggtcgactgatgtcactgtagccaacaagatggaggccggcggcatccctgggc
gcgtgcacatctcccagagcaccatggactgcctgaaaggggagtttgatgtggagccaggcgatgggggcagccgctgtgattaccta
gaagagaagggtattgaaacctacctcatcattgcctccaagccagaggtgaagaaaacagccacccagaatggcctcaatggctcg
gccctgcccaatggagcaccagcttcctcaaagtccagctcccctgccctcattgagaccaaggagcccaacgggagtgcccacagca
gtgggtccacgtcggagaagcccgaggagcaggatgcccaggccgacaacccctcattccccaacccacgccggaggctgcgcctg
caggacctggctgaccgagtggtggatgcctctgaagatgagcacgagctcaaccagctgctcaacgaggccctgcttgagcgagagt
ccgcccaagtagtaaagaagagaaacaccttcctcttgtccatgcggttcatggaccccgagatggaaacccgctactcggtggagaag
gagaagcagagtggggctgccttcagctgctcctgcgtcgtcctgctctgcacggccctggtcgagatactcatcgacccctggctaatga
caaactatgtgaccttcatggtgggggagattctgctcctcatcctgaccatctgctccctggctgccatctttccccgggcctttcctaagaag
cttgtggccttctcaacttggattgaccggacccgctgggccaggaacacctgggccatgctcgccatcttcatcctggtgatggcaaatgt
cgtggacatgctcagctgtctccagtactacacgggacccagcaatgcaacggcagggatggagacggagggcagctgcctggaga
accccaagtattacaactatgtggccgtgctgtccctcatcgccaccatcatgctggtgcaggtcagccacatggtgaagctcacgctcatg
ctgctcgtcgcaggcgccgtggccaccatcaacctctatgcctggcgtcccgtctttgatgaatacgaccacaagcgttttcgggagcacg
acttacctatggtggccttagagcagatgcaaggattcaaccctgggctcaatggcactgacaggctgcccctggtgccttccaagtactct
atgacggtgatggtgttcctcatgatgctcagcttctactacttctcccgccacgtagaaaaactggcacggacacttttcttgtggaagattg
aggtccacgaccagaaggaacgtgtctatgagatgcgacgctggaacgaggccttggtcaccaacatgttgcctgagcacgtggcacg
ccatttcctggggtccaagaagagagatgaggagctgtatagccagacgtatgatgagattggagtcatgtttgcctccctgcccaactttg
ctgacttctacacagaggagagcatcaacaatggtggtattgagtgtctgcgtttcctcaatgaaatcatctcagattttgactctctcctggac
aatcccaagttccgggtgatcaccaagatcaaaaccattggcagcacgtatatggcggcttcaggagtcacccccgatgtcaacaccaa
tggctttgccagctccaacaaggaagacaagtccgagagagagcgctggcagcacctggctgacctggccgacttcgcgctggccatg
aaggatacgctcaccaacatcaacaaccagtccttcaataacttcatgctgcgcataggcatgaacaaaggcggggttctggctggggt
catcggagcccggaaaccacactacgacatctggggcaatacagtcaatgtagccagcaggatggagtccacgggggtcatgggca
acattcaggtggtagaagaaacccaagtcatcctccgagagtacggcttccgctttgtgaggcgaggccccatctttgtgaaggggaagg

gggagctgctgaccttcttcttgaaggggcgggataagctagccaccttccccaatggcccctctgtcacactgccccaccaggtggtgga caactcctga (SEQ ID NO:335)

**O60266**
MPRNQGFSEPEYSAEYSAEYSVSLPSDPDRGVGRTHEISVRNSGSCLCLPRFMRLTFVPESLENL YQTYFKRQRHETLLVLVVFAALFDCYVVVMCAVVFSSDKLAPLAVAGIGLVLDIILFVLCKKGLLPD RVTRRVLPYVLWLLITAQIFSYLGLNFARAHAASDTVGWQVFFVFSFFITLPLSLSPIVIISVVSCVVH TLVLGVTVAQQQQEELKGMQLLREILANVFLYLCAIAVGIMSYYMADRKHRKAFLEARQSLEVKMN LEEQSQQQENLMLSILPKHVADEMLKDMKKDESQKDQQQFNTMYMYRHENVSILFADIVGFTQLS SACSAQELVKLLNELFARFDKLAAKYHQLRIKILGDCYYCICGLPDYREDHAVCSILMGLAMVEAIS YVREKTKTGVDMRVGVHTGTVLGGVLGQKRWQYDVWSTDVTVANKMEAGGIPGRVHISQSTMD CLKGEFDVEPGDGGSRCDYLEEKGIETYLIIASKPEVKKTATQNGLNGSALPNGAPASSKSSSPAL IETKEPNGSAHSSGSTSEKPEEQDAQADNPSFPNPRRRLRLQDLADRVVDASEDEHELNQLLNE ALLERESAQVVKKRNTFLLSMRFMDPEMETRYSVEKEKQSGAAFSCSCVVLLCTALVEILIDPWL MTNYVTFMVGEILLLILTICSLAAIFPRAFPKKLVAFSTWIDRTRWARNTWAMLAIFILVMANVVDML SCLQYYTGPSNATAGMETEGSCLENPKYYNYVAVLSLIATIMLVQVSHMVKLTLMLLVAGAVATIN LYAWRPVFDEYDHKRFREHDLPMVALEQMQGFNPGLNGTDRLPLVPSKYSMTVMVFLMMLSFY YFSRHVEKLARTLFLWKIEVHDQKERVYEMRRWNEALVTNMLPEHVARHFLGSKKRDEELYSQT YDEIGVMFASLPNFADFYTEESINNGGIECLRFLNEIISDFDSLLDNPKFRVITKIKTIGSTYMAASGV TPDVNTNGFASSNKEDKSERERWQHLADLADFALAMKDTLTNINNQSFNNFMLRIGMNKGGVLA GVIGARKPHYDIWGNTVNVASRMESTGVMGNIQVVEETQVILREYGFRFVRRGPIFVKGKGELLT FFLKGRDKLATFPNGPSVTLPHQVVDNS (SEQ ID NO:336)

# Figure 148

**Nucleotide:** D63479
**Sequence:**
cgccgcccgaggagtcgtccgacagcgagcccgaggcggagcccggctccccacagaagctcatccgcaaggtgtccacgtcgggt
cagatccgacagaagaccatcatcaaagaggggatgctgaccaaacagaacaattcattccagcgatcaaaaaggagatactttaag
cttcgagggcgaacgctttactatgccaaaacggcaaagtcaatcatatttgatgaggtggatctgacagatgccagcgtagctgaatcca
gtaccaaaaacgtcaacaacagttttacggtcataactccatgcaggaagctcatcttgtgtgctgataacagaaaagaaatggaagatt
ggattgcagcattaaagactgtgcagaacagggagcactttgagcccacccagtacagcatggaccacttctcagggatgcacaattgg
tacgcctgttcccacgcgaggccgacctactgcaatgtgtgccgtgaggctctgtctggggtcacgtcgcacgggctgtcctgcgaggtgt
gcaaatttaaggcccacaagcgctgtgctgtgcgtgcaaccaataactgcaagtggaccacactggcctcgatcgggaaggacatcatt
gaagatgcagatgggattgcaatgccccaccagtggttggaaggaaacctacctgtgagcgccaagtgcactgtgtgcgacaagacct
gtggcagtgtgctgcgcctgcaggactggcgctgcctctggtgcaaggccatggttcacacatcgtgtaaagaatccttgctgaccaagtg
cccacttggcctgtgcaaagtgtcagtcatcccacccacggctctcaacagcatcgactccgatgggttctggaaggccagctgtcctcctt
cttgcacaagcccactgttggtcttcgtcaattcaaaaagtggggacaaccagggtgtgaagttcctcagaagattcaaacagctactaaa
ccccgcccaggtcttcgacctcatgaacggaggcccacacctcggcttacggttattccagaagtttgacacattccggattctggtttgtgg
cggggatggaagtgttggctgggtcctctccgaaatcgacagcctcaaccttcataaacagtgtcagctgggagtgctgccgctcggcac
agggaacgacttggcccgagtactgggctggggctcagcctgcgatgacgacacccagctcccccagatcttggagaagttggagag
agccagcaccaagatgctggacaggtggagcgtcatggcatacgaggccaagctcccccggcaggcctcctcctctaccgtcaccga
agacttcagcgaggattccgaggtacagcagattctcttctatgaagactcggttgcagcccacctttctaaaatcctcacctcggaccagc
actcggtggtcatctcctcggccaaagtgctctgtgagacggtgaaggacttcgtggcacgggtggggaaggcctatgagaagacgacc
gagagctcggaggagtcagaggtcatggccaagaagtgctctgtcctgaaagagaagctggattcccttctcaagaccttggacgatga
gtcccaggcctcgtcctctctgcccaacccgcccccccaccattgccgaggaggctgaagatggagatgggtcgggcagcatctgcggtt
ccaccggagaccgcttggtggcatcagcttgcccggcccggccgcagatattccggcctcgagaacagctcatgctgagagccaacag
cctgaagaaagcaattcgtcagatcatagaacacacagaaaaagctgtcgatgagcagaatgcccagacccaggagcaggagggct
tcgtcctgggcctctctgagtcagaggagaagatggaccacagagtgtgcccaccactgtcccacagcgagagcttcggggtccccaa
ggggaggagccagcgcaaagtgtcgaaatctccgtgtgaaaagctgatcagcaaagggagtctgtccctaggcagttctgcttcccttcc
gccccagccgggaagccgggacggcttgcctgcgctcaacaccaagatcctgtacccaaatgtccgggctggaatgtctggttccttacc
cggtggctcagtcatcagtcgcctgttaattaatgctgatcccttcaactctgaaccagaaaccctagagtattacacggagaaatgtgtcat
gaacaactattttggcattggcctggatgcgaagatatccctggactttaacaacaagcgcgatgagcacccagagaagtgcaggagcc
gaaccaagaacatgatgtggtatggagttcttggaaccaaagagttgctgcacagaacctacaagaacctggagcaaaaggtcttgctg
gagtgtgacgggcgacccatcccactccccagtcttcagggaattgctgtccttaacattcccagctatgccggaggaaccaacttctggg
gggtaccaaggaagatgatactttcgcagctccatcattcgatgacaagattctggaggtggtcgccgtgttcggcagcatgcagatggc
cgtctctcgagtcatcaggctacagcatcatcggatcgcccagtgtcgcacggtgaagatctccatccttggggatgagggcgtgcctgtg
caggtggacggagaggcctgggtccagccgccagggtacattcggattgtccacaagaaccggcacagacactgaccagagaca
gggcatttgagagcaccctgaagtcctgggaagacaagcagaagtgcgagctgccccgccctccatcctgttccctgcacccggagat
gctgtccgaggaggaggccacccagatggaccagtttgggcaggcagcaggggtcctcattcacagtatccgagaaatagctcagtct
caccgggacatggagcaggaactggcccacgccgtcaatgccagctccaagtccatggaccgtgtgtatggcaagcccagaaccac
agaggggctcaactgcagcttcgtcctggaaatggtgaataacttcagagctctgcgcagtgagacggagctgctgctgtctgggaagat
ggccctgcagctggatccgcctcagaaggagcagctggggagtgctcttgccgagatggaccgacagctcaggaggctggcagacac
cccgtggctctgccagtccgcagagcccggcgacgaagagagtgtgatgctggatcttgccaagcgcagtcgcagtggtaaattccgcc
tcgtgaccaagtttaaaaaggagaaaaacaacaagaacaaagaagctcacagtagcctgggagccccggttcacctctgggggaca
gaggaggttgctgcctggctggagcacctcagtctctgtgagtataaggacatcttcacacggcacgacatccggggctctgagctcctgc
acctggagcggagggacctcaaggacctgggcgtgaccaaggtgggccacatgaagaggatcctgtgtggcatcaaggagctgagc
cgcagcgcccccgccgtcgaggcctag (SEQ ID NO:337)

**Q16760**
PPEESSDSEPEAEPGSPQKLIRKVSTSGQIRQKTIIKEGMLTKQNNSFQRSKRRYFKLRGRTLYYA
KTAKSIIFDEVDLTDASVAESSTKNVNNSFTVITPCRKLILCADNRKEMEDWIAALKTVQNREHFEP
TQYSMDHFSGMHNWYACSHARPTYCNVCREALSGVTSHGLSCEVCKFKAHKRCAVRATNNCK
WTTLASIGKDIIEDADGIAMPHQWLEGNLPVSAKCTVCDKTCGSVLRLQDWRCLWCKAMVHTSC
KESLLTKCPLGLCKVSVIPPTALNSIDSDGFWKASCPPSCTSPLLVFVNSKSGDNQGVKFLRRFKQ
LLNPAQVFDLMNGGPHLGLRLFQKFDTFRILVCGGDGSVGWVLSEIDSLNLHKQCQLGVLPLGTG
NDLARVLGWGSACDDDTQLPQILEKLERASTKMLDRWSVMAYEAKLPRQASSSTVTEDFSEDSE
VQQILFYEDSVAAHLSKILTSDQHSVVISSAKVLCETVKDFVARVGKAYEKTTESSEESEVMAKKC
SVLKEKLDSLLKTLDDESQASSSLPNPPPTIAEEAEDGDGSGSICGSTGDRLVASACPARPQIFRP

REQLMLRANSLKKAIRQIIEHTEKAVDEQNAQTQEQEGFVLGLSESEEKMDHRVCPPLSHSESFG
VPKGRSQRKVSKSPCEKLISKGSLSLGSSASLPPQPGSRDGLPALNTKILYPNVRAGMSGSLPGG
SVISRLLINADPFNSEPETLEYYTEKCVMNNYFGIGLDAKISLDFNNKRDEHPEKCRSRTKNMMWY
GVLGTKELLHRTYKNLEQKVLLECDGRPIPLPSLQGIAVLNIPSYAGGTNFWGGTKEDDTFAAPSF
DDKILEVVAVFGSMQMAVSRVIRLQHHRIAQCRTVKISILGDEGVPVQVDGEAWVQPPGYIRIVHK
NRAQTLTRDRAFESTLKSWEDKQKCELPRPPSCSLHPEMLSEEEATQMDQFGQAAGVLIHSIREI
AQSHRDMEQELAHAVNASSKSMDRVYGKPRTTEGLNCSFVLEMVNNFRALRSETELLLSGKMAL
QLDPPQKEQLGSALAEMDRQLRRLADTPWLCQSAEPGDEESVMLDLAKRSRSGKFRLVTKFKKE
KNNKNKEAHSSLGAPVHLWGTEEVAAWLEHLSLCEYKDIFTRHDIRGSELLHLERRDLKDLGVTK
VGHMKRILCGIKELSRSAPAVEA (SEQ ID NO:338)

# Figure 149

**Nucleotide:** AL022237
**Sequence:**
atgagcgtccgggtcgcacgggtagcgtgggtcaggggcttgggcgccagctaccgccgcggcgcctcgagcttcccggtgcctccgc
cgggcgcccagggtgtagcggagctgctgcgagatgcgaccggggcggaggaggaggcgccctgggcggcgacggagcggcga
atgccgggccagtgctccgtgctgctcttcccgggccagggcagccaggtggtgggcatgggccgcggtctgctcaactacccgcgcgt
ccgcgaactctacgccgccgcccgccgcgtgctgggctacgacctgctggaactgagcctgcacgggccgcaggagaccctggaccg
caccgtgcactgtcagcccgcgatcttcgtggcatcgctggccgctgtcgagaaactacatcacctgcagccctcggtgattgagaactgt
gttgctgctgctggattcagtgtgggagagtttgcagccctagtgtttgccggagccatggaatttgctgaaggtttgtatgcagtgaaaatcc
gagctgaggccatgcaggaagcttcagaagctgtccccagtgggatgctgtctgtcctcggccagcctcagtccaagttcaacttcgcctg
tttggaagcccgggaacactgcaagtctttaggcatagagaaccccgtatgtgaagtgtccaactacctctttccagattgcagggtgatttc
aggacaccaagaggctctacggtttctccagaagaattcctctaagtttcatttcagacgcaccaggatgttgccggttagtggcgcattcc
acacccgcctcatggagccagccgtggagcccctgacgcaagctttaaaggcagtcgacattaagaagcctctggtttctgtctactcca
acgtccacgcgcatagatacaggcatcccgggcacatccacaagctgctggcccagcagctggtctccccagtgaagtgggagcaga
cgatgcatgccatatacgaaaggaaaaagggcaggggttcccccaaactttcgaagtaggccctggcaggcagctgggagccatcc
tgaagagctgtaacatgcaggcctggaagtcctacagcgccgtggatgtgctgcagaccctcgaacatgtggacctggaccctcagga
gcccccgagatga (SEQ ID NO:339)

**O95510**
MSVRVARVAWVRGLGASYRRGASSFPVPPPGAQGVAELLRDATGAEEEAPWAATERRMPGQC
SVLLFPGQGSQVVGMGRGLLNYPRVRELYAAARRVLGYDLLELSLHGPQETLDRTVHCQPAIFVA
SLAAVEKLHHLQPSVIENCVAAAGFSVGEFAALVFAGAMEFAEGLYAVKIRAEAMQEASEAVPSG
MLSVLGQPQSKFNFACLEAREHCKSLGIENPVCEVSNYLFPDCRVISGHQEALRFLQKNSSKFHF
RRTRMLPVSGAFHTRLMEPAVEPLTQALKAVDIKKPLVSVYSNVHAHRYRHPGHIHKLLAQQLVS
PVKWEQTMHAIYERKKGRGFPQTFEVGPGRQLGAILKSCNMQAWKSYSAVDVLQTLEHVDLDP
QEPPR (SEQ ID NO:340)

# Figure 150

**Nucieotide:** AK000759
**Sequence:**
atgtgtggcatttgttgttctgtaaacttttctgctgagcatttcagtcaagatttaaaagaggacttactatataatcttaaacagcggggaccc
aatagtagtaaacaattgttaaagtctgatgttaactaccagtgtttattttctgctcacgtcctacacttgaggggtgttttgactacccagcctg
tggaagatgaaagaggcaatgtgtttctatggaatggagaaatttttagtggaataaaggttgaagctgaagagaatgacactcaaattttg
tttaattatctttcctcctgtaagaatgaatctgagattttgtcactcttctcagaagtacaaggtccctggtcatttatatattatcaagcatctagtc
attatttatggtttggtagggattttttggtcgccgtagcttgctttggcattttagtaatttgggcaagagtttctgcctctcttcagttggcacccaa
acatctggattggcaaatcagtggcaagaagttccagcatctggacttttcagaattgatcttaagtctactgtcatttccagatgcattatttta
caactgtatccttggaaatatatttctagggagaatattattgaagaaaatgttaatagcctgagtcaaatttcagcagacttaccagcatttgt
atcagtggtagcaaatgaagccaaactgtatcttgaaaaacctgttgttcctttaaatatgatgttgccacaagctgcattggagactcattgc
agtaatatttccaatgtgccacctacaagagagatacttcaagtctttcttactgatgtacacatgaaggaagtaattcagcagttcattgatgt
cctgagtgtagcagtcaagaaacgtgtcttgtgtttacctagggatgaaaacctgacagcaaatgaagttttgaaaacgtgtgataggaaa
gcaaatgttgcaatcctgttttctgggggcattgattccatggttattgcaacccttgctgaccgtcatattcctttagatgaaccaattgatcttctt
aatgtagctttcatagctgaagaaaagaccatgccaactacctttaacagagaagggaataaacagaaaaataaatgtgaaataccttc
agaagaattctctaaagatgttgctgctgctgctgctgacagtcctaataaacatgtcagtgtaccagatcgaatcacaggaagggcggg
actaaaggaactacaagctgttagcccttcccgaatttggaattttgttgaaattaatgtttctatggaagaactgcagaaattaagaagaac
tcgaatatgtcacttaattcggccattggatacagttttggatgatagcattggctgtgcagtctggtttgcttctagaggaattggttggttagtg
gcccaggaaggagtgaaatcctatcagagcaatgcaaaggtagttctcactggaattggtgcagatgagcaacttgcaggttattctcgtc
atcgtgtccgctttcagtcgcatgggctggaaggattgaataaggaaataatgatggaactgggtcgaatttcttctagaaatcttggtcgtg
atgacagagttattggtgatcatggaaaagaagcaagatttcctttcctggatgaaaatgttgtctcctttctaaattctctgccgatttgggaaa
aagcaaacttgactttaccccgaggaattggtgaaaaattacttttacgccttgcagctgtggaacttggtcttacagcctctgctcttctgccc
aaacgggccatgcagtttggatcaagaattgcaaaaatggaaaaaattaatgaaaaggcatctgataaatgtggacggctccaaatcat
gtccttagaaaatctttctattgaaaaggagactaaattgtaa (SEQ ID NO:341)

**Q9NWL6**
MCGICCSVNFSAEHFSQDLKEDLLYNLKQRGPNSSKQLLKSDVNYQCLFSAHVLHLRGVLTTQPV
EDERGNVFLWNGEIFSGIKVEAEENDTQILFNYLSSCKNESEILSLFSEVQGPWSFIYYQASSHYL
WFGRDFFGRRSLLWHFSNLGKSFCLSSVGTQTSGLANQWQEVPASGLFRIDLKSTVISRCIILQLY
PWKYISRENIIEENVNSLSQISADLPAFVSVVANEAKLYLEKPVVPLNMMLPQAALETHCSNISNVP
PTREILQVFLTDVHMKEVIQQFIDVLSVAVKKRVLCLPRDENLTANEVLKTCDRKANVAILFSGGIDS
MVIATLADRHIPLDEPIDLLNVAFIAEEKTMPTTFNREGNKQKNKCEIPSEEFSKDVAAAAADSPNK
HVSVPDRITGRAGLKELQAVSPSRIWNFVEINVSMEELQKLRRTRICHLIRPLDTVLDDSIGCAVWF
ASRGIGWLVAQEGVKSYQSNAKVVLTGIGADEQLAGYSRHRVRFQSHGLEGLNKEIMMELGRISS
RNLGRDDRVIGDHGKEARFPFLDENVVSFLNSLPIWEKANLTLPRGIGEKLLLRLAAVELGLTASAL
LPKRAMQFGSRIAKMEKINEKASDKCGRLQIMSLENLSIEKETKL (SEQ ID NO:342)

# Figure 151

**Nucleotide:** D86983
**Sequence:**
agccggccgtggtggctccgtgcgtccgagcgtccgtccgcgccgtcggccatggccaagcgctccaggggccccgggcgccgctgc
ctgttggcgctcgtgctgttctgcgcctgggggacgctggccgtggtggcccagaagccgggcgcagggtgtccgagccgctgcctgtgc
ttccgcaccaccgtgcgctgcatgcatctgctgctggaggccgtgcccgccgtggcgccgcagacctccatcctagatcttcgctttaacag
aatcagagagatccaacctggggcattcaggcggctgaggaacttgaacacattgcttctcaataataatcagatcaagaggataccta
gtggagcatttgaagacttggaaaatttaaaatatctctatctgtacaagaatgagatccagtcaattgacaggcaagcatttaagggactt
gcctctctagagcaactatacctgcactttaatcagatagaaactttggacccagattcgttccagcatctcccgaagctcgagaggctatttt
tgcataacaaccggattacacatttagttccagggacatttaatcacttggaatctatgaagagattgcgactggactcaaacacacttcact
gcgactgtgaaatcctgtggttggcggatttgctgaaaacctacgcggagtcggggaacgcgcaggcagcggccatctgtgaatatccc
agacgcatccagggacgctcagtggcaaccatcaccccggaagagctgaactgtgaaaggccccggatcacctccgagccccagga
cgcagatgtgacctcggggaacaccgtgtacttcacctgcagagccgaaggcaaccccaagcctgagatcatctggctgcgaaacaat
aatgagctgagcatgaagacagattcccgcctaaacttgctggacgatgggaccctgatgatccagaacacacaggagacagaccag
ggtatctaccagtgcatggcaaagaacgtggccggagaggtgaagacgcaagaggtgaccctcaggtacttcgggtctccagctcgac
ccacttttgtaatccagccacagaatacagaggtgctggttggggagagcgtcacgctggagtgcagcgccacaggccacccccccgcc
gcggatctcctggacgagaggtgaccgcacacccttgccagttgacccgcgggtgaacatcacgccttctggcgggctttacatacaga
acgtcgtacaggggggacagcggagagtatgcgtgctctgcgaccaacaacattgacagcgtccatgccaccgctttcatcatcgtccag
gctcttcctcagttcactgtgacgcctcaggacagagtcgttattgagggccagaccgtggatttccagtgtgaagccaagggcaacccgc
cgcccgtcatcgcctggaccaagggagggagccagctctccgtggaccggcggcacctggtcctgtcatcgggaacacttagaatctct
ggtgttgccctccacgaccagggccagtacgaatgccaggctgtcaacatcatcggctcccagaaggtcgtggcccacctgactgtgca
gcccagagtcaccccagtgtttgccagcattcccagcgacacaacagtggaggtgggcgccaatgtgcagctcccgtgcagctcccag
ggcgagcccgagccagccatcacctggaacaaggatggggttcaggtgacagaaagtggaaaatttcacatcagccctgaaggattct
tgaccatcaatgacgttggccctgcagacgcaggtcgctatgagtgtgtggcccggaacaccattgggtcggcctcggtgagcatggtgc
tcagtgtgaacgttcctgacgtcagtcgaaatggagatccgtttgtagctacctccatcgtggaagcgattgcgactgttgacagagctataa
actcaacccgaacacatttgtttgacagccgtcctcgttctccaaatgatttgctggccttgttccggtatccgagggatccttacacagttgaa
caggcacgggcgggagaaatctttgaacggacattgcagctcattcaggagcatgtacagcatggcttgatggtcgacctcaacggaac
aagttaccactacaacgacctggtgtctccacagtacctgaacctcatcgcaaacctgtcgggctgtaccgcccaccggcgcgtgaaca
actgctcggacatgtgcttccaccagaagtaccggacgcacgacggcacctgtaacaacctgcagcaccccatgtggggcgcctcgct
gaccgccttcgagcgcctgctgaaatccgtgtacgagaatggcttcaacacccctcggggcatcaacccccaccgactgtacaacggg
cacgcccttcccatgccgcgcctggtgtccaccaccctgatcgggacggagaccgtcacacccgacgagcagttcacccacatgctgat
gcagtggggccagttcctggaccacgacctcgactccacggtggtggccctgagccaggcacgcttctccgacggacagcactgcagc
aacgtgtgcagcaacgaccccccctgcttctctgtcatgatccccccaatgactcccggccaggagcggggcccgctgcatgttcttcg
tgcgctccagccctgtgtgcggcagcggcatgacttcgctgctcatgaactccgtgtacccgcgggagcagatcaaccagctcacctcct
acatcgacgcatccaacgtgtacgggagcacggagcatgaggcccgcagcatccgcgacctggccagccaccgcggcctgctgcgg
cagggcatcgtgcagcggtccgggaagccgctgctccccttcgccaccgggccgcccacggagtgcatgcgggacgagaacgagag
ccccatcccctgcttcctggccggggaccaccgcgccaacgagcagctgggcctgaccagcatgcacacgctgtggttccgcgagcac
aaccgcattgccacggagctgctcaagctgaacccgcactgggacggcgacaccatctactatgagaccaggaagatcgtgggtgcg
gagatccagcacatcacctaccagcactggctcccgaagatcctgggggaggtgggcatgaggacgctgggagagtaccacggctac
gaccccggcatcaatgctggcatcttcaacgccttcgccaccgcggccttcaggtttggccacacgcttgtcaacccactgctttaccggct
ggacgagaacttccagcccattgcacaagatcacctccccttcacaaagctttcttctcccttccggattgtgaatgagggcggcatcg
atccgcttctcaggggctgttcggggtggcggggaaaatgcgtgtgccctcgcagctgctgaacacggagctcacggagcggctgttct
ccatggcacacacggtggctctggacctggcggccatcaacatccagcggggccgggaccacgggatcccaccctaccacgactac
agggtctactgcaatctatcggcggcacacacgttcgaggacctgaaaaatgagattaaaaaccctgagatccgggagaaactgaaa
aggttgtatggctcgacactcaacatcgacctgtttccggcgctcgtggtggaggacctggtgcctggcagccggctgggccccaccctg
atgtgtcttctcagcacacagttcaagcgcctgcgagatggggacaggttgtggtatgagaaccctggggtgttctccccggcccagctga
ctcagatcaagcagacgtcgctggccaggatcctatgcgacaacgcggacaacatcacccgggtgcagagcgacgtgttcagggtgg
cggagttccctcacggctacggcagctgtgacgagatccccagggtggacctccgggtgtggcaggactgctgtgaagactgtaggacc
agggggcagttcaatgccttttcctatcatttccgaggcagacggtctcttgagttcagctaccaggaggacaagccgaccaagaaaaca
agaccacggaaaatacccagtgttgggagacaggggggaacatctcagcaacagcacctcagccttcagcacacgctcagatgcatct
gggacaaatgacttcagagagtttgttctggaaatgcagaagaccatcacagacctcagaacacagataaagaaacttgaatcacggc
tcagtaccacagagtgcgtggatgccgggggcgaatctcacgccaacaacaccaagtggaaaaaagatgcatgcaccatttgtgaatg
caaagacgggcaggtcacctgcttcgtggaagcttgccccccctgccacctgtgctgtccccgtgaacatcccaggggcctgctgtccagt
ctgcttacagaagagggcggaggaaaagccctag (SEQ ID NO:343)

**Q92626**

SRPWWLRASERPSAPSAMAKRSRGPGRRCLLALVLFCAWGTLAVVAQKPGAGCPSRCLCFRTT
VRCMHLLLEAVPAVAPQTSILDLRFNRIREIQPGAFRRLRNLNTLLLNNNQIKRIPSGAFEDLENLKY
LYLYKNEIQSIDRQAFKGLASLEQLYLHFNQIETLDPDSFQHLPKLERLFLHNNRITHLVPGTFNHLE
SMKRLRLDSNTLHCDCEILWLADLLKTYAESGNAQAAAICEYPRRIQGRSVATITPEELNCERPRIT
SEPQDADVTSGNTVYFTCRAEGNPKPEIIWLRNNNELSMKTDSRLNLLDDGTLMIQNTQETDQGI
YQCMAKNVAGEVKTQEVTLRYFGSPARPTFVIQPQNTEVLVGESVTLECSATGHPPPRISWTRG
DRTPLPVDPRVNITPSGGLYIQNVVQGDSGEYACSATNNIDSVHATAFIIVQALPQFTVTPQDRVVI
EGQTVDFQCEAKGNPPPVIAWTKGGSQLSVDRRHLVLSSGTLRISGVALHDQGQYECQAVNIIGS
QKVVAHLTVQPRVTPVFASIPSDTTVEVGANVQLPCSSQGEPEPAITWNKDGVQVTESGKFHISP
EGFLTINDVGPADAGRYECVARNTIGSASVSMVLSVNVPDVSRNGDPFVATSIVEAIATVDRAINST
RTHLFDSRPRSPNDLLALFRYPRDPYTVEQARAGEIFERTLQLIQEHVQHGLMVDLNGTSYHYND
LVSPQYLNLIANLSGCTAHRRVNNCSDMCFHQKYRTHDGTCNNLQHPMWGASLTAFERLLKSVY
ENGFNTPRGINPHRLYNGHALPMPRLVSTTLIGTETVTPDEQFTHMLMQWGQFLDHDLDSTVVAL
SQARFSDGQHCSNVCSNDPPCFSVMIPPNDSRARSGARCMFFVRSSPVCGSGMTSLLMNSVYP
REQINQLTSYIDASNVYGSTEHEARSIRDLASHRGLLRQGIVQRSGKPLLPFATGPPTECMRDENE
SPIPCFLAGDHRANEQLGLTSMHTLWFREHNRIATELLKLNPHWDGDTIYYETRKIVGAEIQHITYQ
HWLPKILGEVGMRTLGEYHGYDPGINAGIFNAFATAAFRFGHTLVNPLLYRLDENFQPIAQDHLPL
HKAFFSPFRIVNEGGIDPLLRGLFGVAGKMRVPSQLLNTELTERLFSMAHTVALDLAAINIQRGRD
HGIPPYHDYRVYCNLSAAHTFEDLKNEIKNPEIREKLKRLYGSTLNIDLFPALVVEDLVPGSRLGPT
LMCLLSTQFKRLRDGDRLWYENPGVFSPAQLTQIKQTSLARILCDNADNITRVQSDVFRVAEFPH
GYGSCDEIPRVDLRVWQDCCEDCRTRGQFNAFSYHFRGRRSLEFSYQEDKPTKKTRPRKIPSVG
RQGEHLSNSTSAFSTRSDASGTNDFREFVLEMQKTITDLRTQIKKLESRLSTTECVDAGGESHAN
NTKWKKDACTICECKDGQVTCFVEACPPATCAVPVNIPGACCPVCLQKRAEEKP (SEQ ID NO:344)

# Figure 152

**Nucleotide:** M91432
**Sequence:**
atggcagcggggttcgggcgatgctgcagggtcctgagaagtatttctcgttttcattggagatcacagcatacaaaagccaatcgacaac
gtgaaccaggattaggatttagtttgagttcaccgaacagcagaaagaatttcaagctactgctcgtaaatttgccagagaggaaatcatc
ccagtggctgcagaatatgataaaactggtgaatatccagtcccccctaattagaagagcctgggaacttggtttaatgaacacacacattc
cagagaactgtggaggtcttggacttggaacttttgatgcttgtttaattagtgaagaattggcttatggatgtacaggggttcagactgctattg
aaggaaattctttggggcaaatgcctattattattgctggaaatgatcaacaaaagaagaagtatttggggagaatgactgaggagccatt
gatgtgtgcttattgtgtaacagaacctggagcaggctctgatgtagctggtataaagaccaaagcagaaaagaaaggagatgagtatat
tattaatggtcagaagatgtggataaccaacggaggaaaagctaattggtatttttattggcacgttctgatccagatcctaaagctcctgct
aataaagcctttactggattcattgtggaagcagataccccaggaattcagattgggagaaaggaattaaacatgggccagcgatgttca
gatactagaggaattgtcttcgaagatgtgaaagtgcctaaagaaaatgttttaattggtgacggagctggtttcaaagttgcaatgggagct
tttgataaaaccagacctgtagtagctgctggtgctgttggattagcacaaagagctttggatgaagctaccaagtatgccctggaaagga
aaactttcggaaagctacttgtagagcaccaagcaatatcatttatgctggctgaaatggcaatgaaagttgaactagctagaatgagttac
cagagagcagcttgggaggttgattctggtcgtcgaaatacctattatgcttctattgcaaaggcatttgctggagatattgcaaatcagttag
ctactgatgctgtgcagatacttggaggcaatggatttaatacagaatatcctgtagaaaaactaatgagggatgccaaaatctatcagatt
tatgaaggtacttcacaaattcaaagacttattgtagcccgtgaacacattgacaagtacaaaaattaa (SEQ ID NO:345)

**P11310**
MAAGFGRCCRVLRSISRFHWRSQHTKANRQREPGLGFSFEFTEQQKEFQATARKFAREEIIPVAA
EYDKTGEYPVPLIRRAWELGLMNTHIPENCGGLGLGTFDACLISEELAYGCTGVQTAIEGNSLGQ
MPIIIAGNDQQKKKYLGRMTEEPLMCAYCVTEPGAGSDVAGIKTKAEKKGDEYIINGQKMWITNGG
KANWYFLLARSDPDPKAPANKAFTGFIVEADTPGIQIGRKELNMGQRCSDTRGIVFEDVKVPKEN
VLIGDGAGFKVAMGAFDKTRPVVAAGAVGLAQRALDEATKYALERKTFGKLLVEHQAISFMLAEM
AMKVELARMSYQRAAWEVDSGRRNTYYASIAKAFAGDIANQLATDAVQILGGNGFNTEYPVEKL
MRDAKIYQIYEGTSQIQRLIVAREHIDKYKN (SEQ ID NO:346)

# Figure 153

**Nucleotide:** AB017642
**Sequence:**
atgtccgaagactcgagcgccctgccctggtccatcaacagggacgattacgagctgcaggaggtgatcgggagtggagcaactgctg
tagtccaagcagcttattgtgcccctaaaaaggagaaagtggcaatcaaacggataaaccttgagaaatgtcaaactagcatggatgaa
ctcctgaaagaaattcaagccatgagtcaatgccatcatcctaatattgtatcttactacacatcttttgtggtaaaagatgagctgtggcttgtc
atgaagctgctaagtggaggttctgttctggatattattaagcacattgtggcaaaaggggaacacaaaagtggagtcctagatgaatcta
ccattgctacgatactccgagaagtactggaagggctggaatatctgcataaaaatggacagatccacagagatgtgaaagctggaaa
cattcttcttggagaagatggctcagtacagattgcagactttggggttagtgcttttttagcaactggtggtgatattacccgaaataaagtga
gaaagacctttgttggcacccccttgttggatggcacctgaagttatggaacaggtccgtggttatgatttcaaagctgatatttggagttttgga
attacagcaattgaattggctacaggggcggctccttatcataaatatccaccaatgaaggttttaatgctgacactgcagaacgatcctcct
tctttggaaactggtgttcaagataaagaaatgctgaaaaaatatggaaaatcatttagaaaaatgatttcattgtgccttcaaaaagatcca
gaaaaaagaccaacagcagcagaactattaaggcacaaattttttccagaaagcaaagaataaagaatttcttcaagaaaaaacattgc
agagagcaccaaccatttctgaaagagcaaaaaaggttcggagagtaccaggttccagtgggcgtcttcataagacagaggatggag
gctgggagtggagtgatgatgaatttgatgaagaaagtgaggaagggaaagcagcaatttcacaactcaggtctccccgagtgaaaga
atcaatatcaaattctgagctctttccaacaactgatcctgtgggtactttgctccaagttccagaacagatctctgctcatctacctcagccag
ctgggcagattgctacacagccaactcaagtctctctcccacccaccgcagagccagcaaaaacagctcaggctttgtcttcaggatcag
gttcacaagaaaccaagatcccaatcagtctagtactaagattaaggaattccaaaaaagaactaaatgatattcgatttgaatttactcct
gggagagatacagcagagggtgtctctcaggaactcatttctgctggcctggtcgacggaagggatttagtaatagtggcagctaatttgc
agaaaattgtggaagaacctcagtcaaatcgatctgtcactttcaaactggcatctggtgtcgaaggctcagatattcctgatgatggtaaa
ctgataggatttgcccagctcagcatcagctaa (SEQ ID NO:347)

**O95747**
MSEDSSALPWSINRDDYELQEVIGSGATAVVQAAYCAPKKEKVAIKRINLEKCQTSMDELLKEIQA
MSQCHHPNIVSYYTSFVVKDELWLVMKLLSGGSVLDIIKHIVAKGEHKSGVLDESTIATILREVLEGL
EYLHKNGQIHRDVKAGNILLGEDGSVQIADFGVSAFLATGGDITRNKVRKTFVGTPCWMAPEVME

QVRGYDFKADIWSFGITAIELATGAAPYHKYPPMKVLMLTLQNDPPSLETGVQDKEMLKKYGKSF
RKMISLCLQKDPEKRPTAAELLRHKFFQKAKNKEFLQEKTLQRAPTISERAKKVRRVPGSSGRLHK
TEDGGWEWSDDEFDEESEEGKAAISQLRSPRVKESISNSELFPTTDPVGTLLQVPEQISAHLPQP
AGQIATQPTQVSLPPTAEPAKTAQALSSGSGSQETKIPISLVLRLRNSKKELNDIRFEFTPGRDTAE
GVSQELISAGLVDGRDLVIVAANLQKIVEEPQSNRSVTFKLASGVEGSDIPDDGKLIGFAQLSIS
(SEQ ID NO:348)

# Figure 154

**Nucleotide:** D86983
**Sequence:**
agccggccgtggtggctccgtgcgtccgagcgtccgtccgcgccgtcggccatggccaagcgctccaggggccccgggcgccgctgc
ctgttggcgctcgtgctgttctgcgcctgggggacgctggccgtggtggcccagaagccgggcgcagggtgtccgagccgctgcctgtgc
ttccgcaccaccgtgcgctgcatgcatctgctgctggaggccgtgcccgccgtggcgccgcagacctccatcctagatcttcgctttaacag
aatcagagagatccaacctggggcattcaggcggctgaggaacttgaacacattgcttctcaataataatcagatcaagaggataccta
gtggagcatttgaagacttggaaaatttaaaatatctctatctgtacaagaatgagatccagtcaattgacaggcaagcatttaagggactt
gcctctctagagcaactatacctgcactttaatcagatagaaactttggacccagattcgttccagcatctcccgaagctcgagaggctatttt
tgcataacaaccggattacacatttagttccagggacatttaatcacttggaatctatgaagagattgcgactggactcaaacacacttcact
gcgactgtgaaatcctgtggttggcggatttgctgaaaacctacgcggagtcggggaacgcgcaggcagcggccatctgtgaatatccc
agacgcatccagggacgctcagtggcaaccatcaccccggaagagctgaactgtgaaaggccccggatcacctccgagccccagga
cgcagatgtgacctcggggaacaccgtgtacttcacctgcagagccgaaggcaaccccaagcctgagatcatctggctgcgaaacaat
aatgagctgagcatgaagacagattcccgcctaaacttgctggacgatgggaccctgatgatccagaacacacaggagacagaccag
ggtatctaccagtgcatggcaaagaacgtggccggagaggtgaagacgcaagaggtgaccctcaggtacttcgggtctccagctcgac
ccacttttgtaatccagccacagaatacagaggtgctggttggggagagcgtcacgctggagtgcagcgcccacaggccaccccccgcc
gcggatctcctggacgagaggtgaccgcacacccttgccagttgacccgcgggtgaacatcacgccttctggcgggctttacatacaga
acgtcgtacaggggacagcggagagtatgcgtgctctgcgaccaacaacattgacagcgtccatgccaccgctttcatcatcgtccag
gctcttcctcagttcactgtgacgcctcaggacagagtcgttattgagggccagaccgtggatttccagtgtgaagccaagggcaacccgc
cgcccgtcatcgcctggaccaagggagggagccagctctccgtggaccggcggcacctggtcctgtcatcgggaacacttagaatctct
ggtgttgccctccacgaccagggccagtacgaatgccaggctgtcaacatcatcggctcccagaaggtcgtggcccacctgactgtgca
gcccagagtcaccccagtgtttgccagcattcccagcgacacaacagtggaggtgggcgccaatgtgcagctcccgtgcagctcccag
ggcgagcccgagccagccatcacctggaacaaggatggggttcaggtgacagaaagtggaaaatttcacatcagccctgaaggattct
tgaccatcaatgacgttggccctgcagacgcaggtcgctatgagtgtgtggcccggaacaccattgggtcggcctcggtgagcatggtgc
tcagtgtgaacgttcctgacgtcagtcgaaatggagatccgtttgtagctacctccatcgtggaagcgattgcgactgttgacagagctataa
actcaacccgaacacatttgtttgacagccgtcctcgttctccaaatgatttgctggccttgttccggtatccgagggatccttacacagttgaa
caggcacgggcgggagaaatctttgaacggacattgcagctcattcaggagcatgtacagcatggcttgatggtcgacctcaacggaac
aagttaccactacaacgacctggtgtctccacagtacctgaacctcatcgcaaacctgtcgggctgtaccgcccaccggcgcgtgaaca
actgctcggacatgtgcttccaccagaagtaccggacgcacgacggcacctgtaacaacctgcagcaccccatgtggggcgcctcgct
gaccgccttcgagcgcctgctgaaatccgtgtacgagaatggcttcaacacccctcggggcatcaacccccaccgactgtacaacggg
cacgcccttcccatgccgcgcctggtgtccaccaccctgatcgggacggagaccgtcacacccgacgagcagttcacccacatgctgat
gcagtggggccagttcctggaccacgacctcgactccacggtggtggccctgagccaggcacgcttctccgacggacagcactgcagc
aacgtgtgcagcaacgacccccccctgcttctctgtcatgatccccccccaatgactcccgggccaggagcggggcccgctgcatgttcttcg
tgcgctccagccctgtgtgcggcagcggcatgacttcgctgctcatgaactccgtgtacccgcgggagcagatcaaccagctcacctcct
acatcgacgcatccaacgtgtacgggagcacggagcatgaggcccgcagcatccgcgacctggccagccaccgcggcctgctgcgg
cagggcatcgtgcagcggtccgggaagccgctgctccccttcgccaccgggccgcccacggagtgcatgcgggacgagaacgagag
ccccatcccctgcttcctggccggggaccaccgcgccaacgagcagctgggcctgaccagcatgcacacgctgtggttccgcgagcac
aaccgcattgccacggagctgctcaagctgaacccgcactgggacggcgacaccatctactatgagaccaggaagatcgtgggtgcg
gagatccagcacatcacctaccagcactggctcccgaagatcctgggggaggtgggcatgaggacgctgggagagtaccacggctac
gaccccggcatcaatgctggcatcttcaacgccttcgccaccgcggccttcaggtttggccacacgcttgtcaacccactgctttaccggct
ggacgagaacttccagcccattgcacaagatcacctccccttcacaaagctttcttctctcccttccggattgtgaatgagggcggcatcg
atccgcttctcaggggggctgttcggggtggcggggaaaatgcgtgtgccctcgcagctgctgaacacggagctcacggagcggctgttct
ccatggcacacacggtggctctggacctggcggccatcaacatccagcggggccgggaccacgggatcccaccctaccacgactac
agggtctactgcaatctatcggcggcacacacgttcgaggacctgaaaaatgagattaaaaaccctgagatccgggagaaactgaaa
aggttgtatggctcgacactcaacatcgacctgtttccggcgctcgtggtggaggacctggtgcctggcagccggctgggccccaccctg
atgtgtcttctcagcacacagttcaagcgcctgcgagatggggacaggttgtggtatgagaaccctggggtgttctccccggcccagctga
ctcagatcaagcagacgtcgctggccaggatcctatgcgacaacgcggacaacatcacccgggtgcagagcgacgtgttcagggtgg
cggagttccctcacggctacggcagctgtgacgagatccccagggtggacctccgggtgtggcaggactgctgtgaagactgtaggacc
agggggcagttcaatgccttttcctatcatttccgaggcagacggtctcttgagttcagctaccaggaggacaagccgaccaagaaaaca
agaccacggaaaatacccagtgttgggagacaggggggaacatctcagcaacagcacctcagccttcagcacacgctcagatgcatct
gggacaaatgacttcagagagtttgttctggaaatgcagaagaccatcacagacctcagaacacagataaagaaacttgaatcacggc
tcagtaccacagagtgcgtggatgccgggggcgaatctcacgccaacaacaccaagtggaaaaaagatgcatgcaccatttgtgaatg
caaagacgggcaggtcacctgcttcgtggaagcttgcccccctgccacctgtgctgtccccgtgaacatcccaggggcctgctgtccagt
ctgcttacagaagagggcggaggaaaagccctag (SEQ ID NO:349)

**Q92626**

SRPWWLRASERPSAPSAMAKRSRGPGRRCLLALVLFCAWGTLAVVAQKPGAGCPSRCLCFRTT
VRCMHLLLEAVPAVAPQTSILDLRFNRIREIQPGAFRRLRNLNTLLLNNNQIKRIPSGAFEDLENLKY
LYLYKNEIQSIDRQAFKGLASLEQLYLHFNQIETLDPDSFQHLPKLERLFLHNNRITHLVPGTFNHLE
SMKRLRLDSNTLHCDCEILWLADLLKTYAESGNAQAAAICEYPRRIQGRSVATITPEELNCERPRIT
SEPQDADVTSGNTVYFTCRAEGNPKPEIIWLRNNNELSMKTDSRLNLLDDGTLMIQNTQETDQGI
YQCMAKNVAGEVKTQEVTLRYFGSPARPTFVIQPQNTEVLVGESVTLECSATGHPPPRISWTRG
DRTPLPVDPRVNITPSGGLYIQNVVQGDSGEYACSATNNIDSVHATAFIIVQALPQFTVTPQDRVVI
EGQTVDFQCEAKGNPPPVIAWTKGGSQLSVDRRHLVLSSGTLRISGVALHDQGQYECQAVNIIGS
QKVVAHLTVQPRVTPVFASIPSDTTVEVGANVQLPCSSQGEPEPAITWNKDGVQVTESGKFHISP
EGFLTINDVGPADAGRYECVARNTIGSASVSMVLSVNVPDVSRNGDPFVATSIVEAIATVDRAINST
RTHLFDSRPRSPNDLLALFRYPRDPYTVEQARAGEIFERTLQLIQEHVQHGLMVDLNGTSYHYND
LVSPQYLNLIANLSGCTAHRRVNNCSDMCFHQKYRTHDGTCNNLQHPMWGASLTAFERLLKSVY
ENGFNTPRGINPHRLYNGHALPMPRLVSTTLIGTETVTPDEQFTHMLMQWGQFLDHDLDSTVVAL
SQARFSDGQHCSNVCSNDPPCFSVMIPPNDSRARSGARCMFFVRSSPVCGSGMTSLLMNSVYP
REQINQLTSYIDASNVYGSTEHEARSIRDLASHRGLLRQGIVQRSGKPLLPFATGPPTECMRDENE
SPIPCFLAGDHRANEQLGLTSMHTLWFREHNRIATELLKLNPHWDGDTIYYETRKIVGAEIQHITYQ
HWLPKILGEVGMRTLGEYHGYDPGINAGIFNAFATAAFRFGHTLVNPLLYRLDENFQPIAQDHLPL
HKAFFSPFRIVNEGGIDPLLRGLFGVAGKMRVPSQLLNTELTERLFSMAHTVALDLAAINIQRGRD
HGIPPYHDYRVYCNLSAAHTFEDLKNEIKNPEIREKLKRLYGSTLNIDLFPALVVEDLVPGSRLGPT
LMCLLSTQFKRLRDGDRLWYENPGVFSPAQLTQIKQTSLARILCDNADNITRVQSDVFRVAEFPH
GYGSCDEIPRVDLRVWQDCCEDCRTRGQFNAFSYHFRGRRSLEFSYQEDKPTKKTRPRKIPSVG
RQGEHLSNSTSAFSTRSDASGTNDFREFVLEMQKTITDLRTQIKKLESRLSTTECVDAGGESHAN
NTKWKKDACTICECKDGQVTCFVEACPPATCAVPVNIPGACCPVCLQKRAEEKP (SEQ ID
NO:350)

# Figure 155

**Nucleotide:** BC014947
**Sequence:**
atgtggctggaccatcgagcagtcagtcaagttaacaggatcaatgagaccaagcacagtgtcctccagtacgtcggggggggtgatgtct
gtggaaatgcaggccccgaaacttctgtggctgaaagagaacttgagagagatttgctgggataaggcgggacatttctttgatctcccgg
acttcttatcgtggaaggcaacaggtgtcacagcacggtctctctgctccctggtgtgtaagtggacatattcagcagagaaaggctggga
cgacagtttctggaaaatgattggtttggaagactttgttgcagataattacagcaaaataggaaaccaagtgctacctcctggagcttctctt
ggaaatgggctcacaccagaggcagcaagagaccttggccttctccctgggattgcggtcgcagcttcactcattgatgcccatgcagga
ggactaggagtgattggggcagatgtgagagggcacggcctcatctgtgaggggcagccagtgacgtcacggctggctgtcatctgtgg
aacgtcttcttgtcacatggggatcagcaaagacccgatttttgtaccaggcgtctgggggccttatttctcagccatggtacctgggttctgg
ctgaatgaaggtggtcagagcgttactggaaaattgatagaccacatggtacaaggccatgctgcttttccagaactacaagtaaaggcc
acagccagatgccagagtatatatgcatatttgaacagtcacctggatctgattaagaaggctcagcctgtgggtttccttactgttgatttac
atgtttggccagatttccatggcaaccggtctcccttagcagatctgacactaaagggcatggtcaccggattgaaactgtctcaggacctt
gatgatcttgccattctctacctggccacagttcaagccattgctttggggactcgcttcattatagaagccatggaggcagcagggcactca
atcagtactcttttcctatgtggaggcctcagcaagaatccccttttgtgcaaatgcatgcggacattactggcatgcctgtggtcctgtcgca
agaggtggagtccgttcttgtgggtgctgctgttctgggtgcctgtgcctcaggggatttcgcttctgtacaggaagcaatggcaaaaatgag
caaagttgggaaagttgtgttcccgagactacaggataaaaaatactatgataagaaataccaagtattcctgaagctggttgaacacca
gaaggagtatttggcgatcatgaatgatgactga (SEQ ID NO:351)

**Q96C11**
MWLDHRAVSQVNRINETKHSVLQYVGGVMSVEMQAPKLLWLKENLREICWDKAGHFFDLPDFLS
WKATGVTARSLCSLVCKWTYSAEKGWDDSFWKMIGLEDFVADNYSKIGNQVLPPGASLGNGLTP
EAARDLGLLPGIAVAASLIDAHAGGLGVIGADVRGHGLICEGQPVTSRLAVICGTSSCHMGISKDPI
FVPGVWGPYFSAMVPGFWLNEGGQSVTGKLIDHMVQGHAAFPELQVKATARCQSIYAYLNSHLD
LIKKAQPVGFLTVDLHVWPDFHGNRSPLADLTLKGMVTGLKLSQDLDDLAILYLATVQAIALGTRFII
EAMEAAGHSISTLFLCGGLSKNPLFVQMHADITGMPVVLSQEVESVLVGAAVLGACASGDFASVQ
EAMAKMSKVGKVVFPRLQDKKYYDKKYQVFLKLVEHQKEYLAIMNDD (SEQ ID NO:352)

# Figure 156

**Nucleotide:** U08316
**Sequence:**
atgccgctggcgcagctggcggacccgtggcagaagatggctgtggagagcccgtccgacagcgctgagaatggacagcaaattatg
gatgaacctatgggagaggaggagattaacccacaaactgaagaagtcagtatcaaagaaattgcaatcacacatcatgtaaaggaa
ggacatgaaaaggcagatccttcccagtttgaacttttaaaagtattagggcagggatcatttggaaaggttttcttagttaaaaaaatctca
ggctctgatgctaggcagctttatgccatgaaggtattgaagaaggccacactgaaagttcgagaccgagttcggacaaaaatggaacg
tgatatcttggtagaggttaatcatccttttattgtcaagttgcattatgcttttcaaactgaagggaagttgtatcttattttggattttctcaggga
ggagatttgtttacacgcttatccaaagaggtgatgttcacagaagaagatgtcaaattctacttggctgaacttgcacttgctttagaccatct
acatagcctgggaataatttatagagacttaaaaccagaaaatatacttcttgatgaagaaggtcacatcaagttaacagatttcggcctaa
gtaaagagtctattgaccatgaaaagaaggcatattctttttgtggaactgtggagtatatggctccagaagtagttaatcgtcgaggtcata
ctcagagtgctgactggtggtcttttggtgtgttaatgtttgaaatgcttactggtacactccctttccaaggaaaagatcgaaaagaaacaat
gactatgattcttaaagccaaacttggaatgccacagtttttgagtcctgaagcgcagagtcttttacgaatgcttttcaagcgaaatcctgca
aacagattaggtgcaggaccagatggagttgaagaaattaaaagacattcattttctcaacgatagactggaataaactgtatagaaga
gaaattcatccgccatttaaacctgcaacgggcaggcctgaagatacattctattttgatcctgagtttactgcaaaaactcccaaagattca
cctggcattccacctagtgctaatgcacatcagctttttcgggggtttagttttgttgctattacctcagatgatgaaagccaagctatgcagac
agttggtgtacattcaattgttcagcagttacacaggaacagtattcagtttactgatggatatgaagtaaaagaagatattggagttggctcc
tactctgtttgcaagagatgtatacataaagctacaaacatggagtttgcagtgaagattattgataaaagcaagagagacccaacagaa
gaaattgaaattcttcttcgttatggacagcatccaaacattatcactctaaaggatgtatatgatgatggaaagtatgtgtatgtagtaacag
aacttatgaaaggaggtgaattgctggataaaattcttagacaaaaatttttctctgaacgagaggccagtgctgtcctgttcactataactaa
aaccgttgaatatcttcacgcacaaggggtggttcatcgagacttgaaacctagcaacattctttatgtggatgaatctggtaatccggaatc
tattcgaatttgtgattttggctttgcaaaacagctgagagcggaaaatggtcttctcatgactccttgttacactgcaaattttgttgcaccaga
ggttttaaaaagacaaggctatgatgctgcttgtgatatatggagtcttggtgtcctactctatacaatgcttaccggttacactccatttgcaaa
tggtcctgatgatacaccagaggaaatattggcacgaataggtagcggaaaattctcactcagtggtggttactggaattctgtttcagaca
cagcaaaggacctggtgtcaaagatgcttcatgtagaccctcatcagagactgactgctgctcttgtgctcagacatccttggatcgtccact
gggaccaactgccacaataccaactaaacagacaggatgcaccacatctagtaaagggtgccatggcagctacatattctgctttgaac
cgtaatcagtcaccagttttggaaccagtaggccgctctactcttgctcagcggagaggtattaaaaaaatcacctcaacagccctgtga
(SEQ ID NO:353)

**P51812**
MPLAQLADPWQKMAVESPSDSAENGQQIMDEPMGEEEINPQTEEVSIKEIAITHHVKEGHEKADP
SQFELLKVLGQGSFGKVFLVKKISGSDARQLYAMKVLKKATLKVRDRVRTKMERDILVEVNHPFIV
KLHYAFQTEGKLYLILDFLRGGDLFTRLSKEVMFTEEDVKFYLAELALALDHLHSLGIIYRDLKPENI
LLDEEGHIKLTDFGLSKESIDHEKKAYSFCGTVEYMAPEVVNRRGHTQSADWWSFGVLMFEMLT
GTLPFQGKDRKETMTMILKAKLGMPQFLSPEAQSLLRMLFKRNPANRLGAGPDGVEEIKRHSFFS
TIDWNKLYRREIHPPFKPATGRPEDTFYFDPEFTAKTPKDSPGIPPSANAHQLFRGFSFVAITSDDE
SQAMQTVGVHSIVQQLHRNSIQFTDGYEVKEDIGVGSYSVCKRCIHKATNMEFAVKIIDKSKRDPT
EEIEILLRYGQHPNIITLKDVYDDGKYVYVVTELMKGGELLDKILRQKFFSEREASAVLFTITKTVEYL
HAQGVVHRDLKPSNILYVDESGNPESIRICDFGFAKQLRAENGLLMTPCYTANFVAPEVLKRQGY
DAACDIWSLGVLLYTMLTGYTPFANGPDDTPEEILARIGSGKFSLSGGYWNSVSDTAKDLVSKML
HVDPHQRLTAALVLRHPWIVHWDQLPQYQLNRQDAPHLVKGAMAATYSALNRNQSPVLEPVGR
STLAQRRGIKKITSTAL (SEQ ID NO:354)

# Figure 157

**Nucleotide:** AK021919
**Sequence:**
atgtctcgggaacccaccccacctctacctggagatatgtctactggtcccatagcagaaagctggtgttacacacaggttaaagtggtaa
aattttcctatatgtggaccattaataacttcagtttttgtcgagaggaaatgggtgaagtgttaaaaagttcaacattttcatctggcccaagtg
acaaaatgaaatggtgcctgagggtaaacccaaagggattagatgatgaaagtaaagactacttgtccttatatttgcttttagtcagctgcc
ccaaaagtgaagttcgagcaaaattcaaattttcccttctgaatgctaaaagggaagaaacaaaagcaatggaaagccaaagagcata
tcgatttgtgcaagggaaggactggggttttaaaaaattcattagaagggactttttgcttgatgaagctaatggtcttttaccagatgacaag
cttacattattttgtgaggtgagtgtggtccaagattcagtaaacatatcaggacatactaatacaaatactttgaaggtgcctgagtgtcgtct
agcagaagatttaggtaatctctgggaaaacacaagatttacagactgcagtttttcgtgagaggacaagaatttaaagctcataaatctg
tgcttgcagctcgatctccagtttttaacgccatgtttgaacatgaaatggaagaaagcaaaaagaatcgagtggaaataaatgatttaga
ccctgaagtttttaaagaaatgatgagattcatttacacagggagagcaccaaaccttgacaaaatggctgacaacttgttggcagctgca
gacaaatatgcactggaacggctgaaggtcatgtgcgaagaagctttgtgtagtaacctctcagtagagaatgttgcagatacccttgtcct
tgcagatttgcacagtgcagaacagttgaaagcacaagccatagactttattaataggtgcagtgtacttcgacaacttgggtgtaaagatg
ggaaaaactggaacagcaaccaagcaaccgacataatggaaagtgaacgggatcgccaacatttgataaagaagttatgaaagcct
atattgaagaccttaaagaaagagaatctgttcccagctataccaccaaagtgtcttttagccttcaactttga (SEQ ID NO:355)

**Q9HAB2**
MSREPTPPLPGDMSTGPIAESWCYTQVKVVKFSYMWTINNFSFCREEMGEVLKSSTFSSGPSDK
MKWCLRVNPKGLDDESKDYLSLYLLLVSCPKSEVRAKFKFSLLNAKREETKAMESQRAYRFVQG
KDWGFKKFIRRDFLLDEANGLLPDDKLTLFCEVSVVQDSVNISGHTNTNTLKVPECRLAEDLGNL
WENTRFTDCSFFVRGQEFKAHKSVLAARSPVFNAMFEHEMEESKKNRVEINDLDPEVFKEMMRF
IYTGRAPNLDKMADNLLAAADKYALERLKVMCEEALCSNLSVENVADTLVLADLHSAEQLKAQAID
FINRCSVLRQLGCKDGKNWNSNQATDIMESERDRQHFDKEVMKAYIEDLKERESVPSYTTKVSFS
LQL (SEQ ID NO:356)

**Nucleotide:** AJ000644
**Sequence:**
atgtcaagggttccaagtcctccacctccggcagaaatgtcgagtggccccgtagctgagagttggtgctacacacagatcaaggtagtg
aaattctcctacatgtggaccatcaataactttagcttttgccgggaggaaatgggtgaagtcattaaaagttctacattttcatcaggagcaa
atgataaactgaaatggtgtttgcgagtaaaccccaaagggttagatgaagaaagcaaagattacctgtcactttacctgttactggtcagc
tgtccaaagagtgaagttcgggcaaaattcaaattctccatcctgaatgccaagggagaagaaaccaaagctatggagagtcaacggg
catataggtttgtgcaaggcaaagactggggattcaagaaattcatccgtagagattttcttttggatgaggccaacgggcttctccctgatg
acaagcttaccctcttctgcgaggtgagtgttgtgcaagattctgtcaacatttctggccagaataccatgaacatggtaaaggttcctgagtg
ccggctggcagatgagttaggaggactgtgggagaattcccggttcacagactgctgcttgtgtgttgccggccaggaattccaggctcac
aaggctatcttagcagctcgttctccggtttttagtgccatgtttgaacatgaaatggaggagagcaaaaagaatcgagttgaaatcaatga
tgtggagcctgaagtttttaaggaaatgatgtgcttcatttacacggggaaggctccaaacctcgacaaaatggctgatgatttgctggcag
ctgctgacaagtatgccctggagcgcttaaaggtcatgtgtgaggatgccctctgcagtaacctgtccgtggagaacgctgcagaaattct
catcctggccgacctccacagtgcagatcagttgaaaactcaggcagtggatttcatcaactatcatgcttcggatgtcttggagacctctgg
gtggaagtcaatggtggtgtcacatccccacttggtggctgaggcataccgctctctggcttcagcacagtgcccttttctgggaccccccac
gcaaacgcctgaagcaatcctaa (SEQ ID NO:357)

**O43791**
MSRVPSPPPPAEMSSGPVAESWCYTQIKVVKFSYMWTINNFSFCREEMGEVIKSSTFSSGANDK
LKWCLRVNPKGLDEESKDYLSLYLLLVSCPKSEVRAKFKFSILNAKGEETKAMESQRAYRFVQGK
DWGFKKFIRRDFLLDEANGLLPDDKLTLFCEVSVVQDSVNISGQNTMNMVKVPECRLADELGGL
WENSRFTDCCLCVAGQEFQAHKAILAARSPVFSAMFEHEMEESKKNRVEINDVEPEVFKEMMCFI
YTGKAPNLDKMADDLLAAADKYALERLKVMCEDALCSNLSVENAAEILILADLHSADQLKTQAVDFI
NYHASDVLETSGWKSMVVSHPHLVAEAYRSLASAQCPFLGPPRKRLKQS (SEQ ID NO:358)

# Figure 158

**Nucleotide:** AB015046
**Sequence:**
atggcggagcacgcccctcgccgctgctgcctgggctgggacttcagcacgcagcaggtaaaggttgttgctgttgatgcagagttgaat
gtcttctatgaggaaagtgtgcattttgacagagatcttccagaatttgggcatgtacttgatgtgcatggtgttcatgtgcacaaggatgggct
gacggtcacttctccagtactaatgtgggtccaggcactggatatcatcttggagaagatgaaggcttcgggcttcgaattctctcaagtcct
agccttgtccggggcgggccagcaacacggaagtatatactggaaggctggagcccagcaggcactgacaagcttatcaccagacct
ccggctacaccagcagcttcaggactgtttctccatcagcgactgcccggtgtggatggactccagcaccacagcccagtgccgccagc
tggaggctgctgtgggtggtgctcaggctctcagctgcctcacggggtcccgtgcctatgagcgttttacagggaaccaaattgcaaaaatt
taccagcagaaccccgaggcctactcacatacagagagaatttctttggtcagtagctttgctgcttccctgttccttggctcttactcccctatt
gactacagtgatggttctggaatgaatttgttgcagatacaggataaagtctggtcccaggcttgccttggtgcctgtgcacctcatttagagg
agaagcttagcccaccagtaccatcatgctcagttgtgggagccatttcttcctacaacgtccagcgctacggatttcctccaggatgcaaa
gtggtggccttcactggggacaacccagcgtcgctggcaggcatgagactggaggaaggtgacattgcggtcagcctgggcaccagtg
acaccctgtttctctggctccaagagcccatgcctgccctggaaggccacatcttctgcaacccggttgactcccagcactacatggcactc
ctgtgctttaaaaatggctccctcatgagagagaagatccgcaacgagtctgtatcccgttcctggagcgatttctctaaggcactgcagtcc
acagagatgggcaacggtggaaacctgggtttttattttgatgtaatggagatcaccccctgaaattattggacgtcataggtttaacacaga
aaaccacaaggttgcagcattccctggggatgtggaggttcgagcactaattgaaggacaattcatggccaagaggattcacgcagaa
ggcctgggctatcgagtcatgtccaagacaaagatttggccacaggaggagcatctcacaatagagaaatcttacaggtgcttgcagat
gtgtttgatgccccggtgtatgttatagacactgccaactcggcctgtgtgggttctgcataccgagcttttcatggtcttgcaggtggaacaga
tgtgcccttttcagaggttgtgaagttagctccaaatcccagactagctgctaccccaagcccgggagcttctcaggtgagagaccatcng
aatttgtttgtagcatttgcattatga (SEQ ID NO:359)

**O75191**
MAEHAPRRCCLGWDFSTQQVKVVAVDAELNVFYEESVHFDRDLPEFGHVLDVHGVHVHKDGLT
VTSPVLMWVQALDIILEKMKASGFEFSQVLALSGAGQQHGSIYWKAGAQQALTSLSPDLRLHQQL
QDCFSISDCPVWMDSSTTAQCRQLEAAVGGAQALSCLTGSRAYERFTGNQIAKIYQQNPEAYSH
TERISLVSSFAASLFLGSYSPIDYSDGSGMNLLQIQDKVWSQACLGACAPHLEEKLSPPVPSCSVV
GAISSYNVQRYGFPPGCKVVAFTGDNPASLAGMRLEEGDIAVSLGTSDTLFLWLQEPMPALEGHI
FCNPVDSQHYMALLCFKNGSLMREKIRNESVSRSWSDFSKALQSTEMGNGGNLGFYFDVMEITP
EIIGRHRFNTENHKVAAFPGDVEVRALIEGQFMAKRIHAEGLGYRVMSKTKILATGGASHNREILQV
LADVFDAPVYVIDTANSACVGSAYRAFHGLAGGTDVPFSEVVKLAPNPRLAATPSPGASQVRDHX
NLFVAFAL (SEQ ID NO:360)

# Figure 159

**Nucleotide:** X78678
**Sequence:**
atggaagagaagcagatcctgtgcgtggggctagtggtgctggacgtcatcagcctggtggacaagtaccctaaggaggactcggaga
taaggtgtttgtcccagagatggcagcgcggaggcaacgcgtccaactcctgcaccgttctctccctgctcggagccccctgtgccttcatg
ggctcaatggctcctggccatgttgctgatttgtcctggatgacctccgccgctattctgtggacctacgctacacagtctttcagaccacag
gctccgtccccatcgccacggtcatcatcaacgaggccagtggtagccgcaccatcctatactatgacaggagcctgccagatgtgtctg
ctacagactttgagaaggttgatctgacccagttcaagtggatccacattgagggccggaacgcatcggagcaggtgaagatgctgcag
cggatagacgcacacaacaccaggcagcctccagagcagaagatccgggtgtccgtggaggtggagaagccacgagaggagctct
tccagctgtttggctacggagacgtggtgtttgtcagcaaagatgtggccaagcacttggggttccagtcagcagaggaagccttgaggg
gcttgtatggtcgtgtgaggaaaggggctgtgcttgtctgtgcctgggctgaggagggcgccgacgccctgggccctgatggcaaattgct
ccactcggatgctttcccgccaccccgcgtggtggatacactgggagctggagacaccttcaatgcctccgtcatcttcagcctctcccagg
ggaggagcgtgcaggaagcactgagattcgggtgccaggtggccggcaagaagtgtggcctgcagggctttgatggcatcgtgtga
(SEQ ID NO:361)

**P50053**
MEEKQILCVGLVVLDVISLVDKYPKEDSEIRCLSQRWQRGGNASNSCTVLSLLGAPCAFMGSMAP
GHVADFVLDDLRRYSVDLRYTVFQTTGSVPIATVIINEASGSRTILYYDRSLPDVSATDFEKVDLTQ
FKWIHIEGRNASEQVKMLQRIDAHNTRQPPEQKIRVSVEVEKPREELFQLFGYGDVVFVSKDVAK

HLGFQSAEEALRGLYGRVRKGAVLVCAWAEEGADALGPDGKLLHSDAFPPPRVVDTLGAGDTFN
ASVIFSLSQGRSVQEALRFGCQVAGKKCGLQGFDGIV (SEQ ID NO:362)

# Figure 160

**Nucleotide:** AK021919
**Sequence:**
atgtctcgggaacccaccccacctctacctggagatatgtctactggtcccatagcagaaagctggtgttacacacaggttaaagtggtaa
aattttcctatatgtggaccattaataacttcagtttttgtcgagaggaaatgggtgaagtgttaaaaagttcaacattttcatctggcccaagtg
acaaaatgaaatggtgcctgagggtaaacccaaagggattagatgatgaaagtaaagactacttgtccttatatttgcttttagtcagctgcc
ccaaaagtgaagttcgagcaaaattcaaattttcccttctgaatgctaaagggaagaaacaaaagcaatggaaagccaaagagcata
tcgatttgtgcaagggaaggactggggttttaaaaaattcattagaagggactttttgcttgatgaagctaatggtcttttaccagatgacaag
cttacattattttgtgaggtgagtgtggtccaagattcagtaaacatatcaggacatactaatacaaatactttgaaggtgcctgagtgtcgtct
agcagaagatttaggtaatctctgggaaaacacaagatttacagactgcagttttttcgtgagaggacaagaatttaaagctcataaatctg
tgcttgcagctcgatctccagtttttaacgccatgtttgaacatgaaatggaagaaagcaaaaagaatcgagtggaaataaatgatttaga
ccctgaagtttttaaagaaatgatgagattcatttacacagggagagcaccaaaccttgacaaaatggctgacaacttgttggcagctgca
gacaaatatgcactggaacggctgaaggtcatgtgcgaagaagctttgtgtagtaacctctcagtagagaatgttgcagataccccttgtcct
tgcagatttgcacagtgcagaacagttgaaagcacaagccatagactttattaataggtgcagtgtacttcgacaacttgggtgtaaagatg
ggaaaaactggaacagcaaccaagcaaccgacataatggaaagtgaacgggatcgccaacattttgataaagaagttatgaaagcct
atattgaagaccttaaagaaagagaatctgttcccagctataccaccaaagtgtcttttagccttcaactttga (SEQ ID NO:363)

**Q9HAB2**
MSREPTPPLPGDMSTGPIAESWCYTQVKVVKFSYMWTINNFSFCREEMGEVLKSSTFSSGPSDK
MKWCLRVNPKGLDDESKDYLSLYLLLVSCPKSEVRAKFKFSLLNAKREETKAMESQRAYRFVQG
KDWGFKKFIRRDFLLDEANGLLPDDKLTLFCEVSVVQDSVNISGHTNTNTLKVPECRLAEDLGNL
WENTRFTDCSFFVRGQEFKAHKSVLAARSPVFNAMFEHEMEESKKNRVEINDLDPEVFKEMMRF
IYTGRAPNLDKMADNLLAAADKYALERLKVMCEEALCSNLSVENVADTLVLADLHSAEQLKAQAID
FINRCSVLRQLGCKDGKNWNSNQATDIMESERDRQHFDKEVMKAYIEDLKERESVPSYTTKVSFS
LQL (SEQ ID NO:364)

**Nucleotide:** AJ000644
**Sequence:**
atgtcaagggttccaagtcctccacctccggcagaaatgtcgagtggccccgtagctgagagttggtgctacacacagatcaaggtagtg
aaattctcctacatgtggaccatcaataactttagcttttgccgggaggaaatgggtgaagtcattaaaagttctacattttcatcaggagcaa
atgataaactgaaatggtgtttgcgagtaaaccccaaagggttagatgaagaaagcaaagattacctgtcactttacctgttactggtcagc
tgtccaaagagtgaagttcgggcaaaattcaaattctccatcctgaatgccaagggagaagaaaccaaagctatggagagtcaacggg
catataggtttgtgcaaggcaaagactggggattcaagaaattcatccgtagagattttcttttggatgaggccaacgggcttctccctgatg
acaagcttaccctcttctgcgaggtgagtgttgtgcaagattctgtcaacatttctggccagaataccatgaacatggtaaaggttcctgagtg
ccggctggcagatgagttaggaggactgtgggagaattcccggttcacagactgctgcttgtgtgttgccggccaggaattccaggctcac
aaggctatcttagcagctcgttctccggtttttagtgccatgtttgaacatgaaatggaggagagcaaaaagaatcgagttgaaatcaatga
tgtggagcctgaagtttttaaggaaatgatgtgcttcatttacacggggaaggctccaaacctcgacaaaatggctgatgatttgctggcag
ctgctgacaagtatgccctggagcgcttaaaggtcatgtgtgaggatgccctctgcagtaacctgtccgtggagaacgctgcagaaattct
catcctggccgacctccacagtgcagatcagttgaaaactcaggcagtggatttcatcaactatcatgcttcggatgtcttggagacctctgg
gtggaagtcaatggtggtgtcacatccccacttggtggctgaggcataccgctctctggcttcagcacagtgcccttttctgggacccccac
gcaaacgcctgaagcaatcctaa (SEQ ID NO:365)

**O43791**
MSRVPSPPPPAEMSSGPVAESWCYTQIKVVKFSYMWTINNFSFCREEMGEVIKSSTFSSGANDK
LKWCLRVNPKGLDEESKDYLSLYLLLVSCPKSEVRAKFKFSILNAKGEETKAMESQRAYRFVQGK
DWGFKKFIRRDFLLDEANGLLPDDKLTLFCEVSVVQDSVNISGQNTMNMVKVPECRLADELGGL
WENSRFTDCCLCVAGQEFQAHKAILAARSPVFSAMFEHEMEESKKNRVEINDVEPEVFKEMMCFI
YTGKAPNLDKMADDLLAAADKYALERLKVMCEDALCSNLSVENAAEILILADLHSADQLKTQAVDFI
NYHASDVLETSGWKSMVVSHPHLVAEAYRSLASAQCPFLGPPRKRLKQS (SEQ ID NO:366)

# Figure 161

**Nucleotide:** D16845
**Sequence:**
atggaaaacgagacagtcagtgaactgaaccaaacacagcttcagccacgagcagtggtggccttagaataccaggtggtcaccatct
tacttgtactcattatttgtggcctgggcattgtaggcaacatcatggtagtcctggttgtcatgagaaccaagcacatgaggaccccccacaa
actgctacctggtgagcctggcagtagctgatctcatggtcttggtggccgcaggcctccccaacataacagacagtatctacggttcctgg
gtctatggctatgttggatgcctctgcattacttacctccagtatttgggaattaatgcatcctcttgttcaataacagcctttaccattgagaggta
catagcaatctgtcaccccatcaaagcccagtttctctgcacattttccagagccaaaaagattatcatctttgtctgggctttcacatctcttta
ctgtatgctctggttcttcttgctggatctcaatattagcacctacaaagatgctattgtgatatcctgtggctacaagatctccaggaattactac
tcacctatttacctaatggactttggtgtcttttatgttgtgccaatgatcctggctaccgtcctctatggattcatagctagaatccttttcttaaatc
ccattccttcagatcctaaagaaaactctaagacatggaaaaatgattcaacccatcagaacacaaatctgaatgtaaatacctctaatag
atgtttcaacagcacagtatcttcaaggaagcaggtcaccaagatgctggcagtggttgtaattctgtttgcccttttatggatgccctacagg
actctagtggttgtcaactcatttctctccagtcctttccaagaaaattggtttttgctcttttgcagaatttgcatttatctcaacagtgccatcaacc
cggtgatttacaatctcatgtcccagaaattccgtgcagccttcagaaagctctgcaactgcaagcagaagccaacagagaaacctgcta
actacagtgtggccctaaattacagcgtcatcaaggagtcagaccatttcagcacagagcttgatgatatcactgtcactgacacttacctg
tctgccacaaaagtgtcttttgatgacacctgcttggcttctgaggtatcctttagccaaagttga (SEQ ID NO:367)

**P34981**
MENETVSELNQTQLQPRAVVALEYQVVTILLVLIICGLGIVGNIMVVLVVMRTKHMRTPTNCYLVSL
AVADLMVLVAAGLPNITDSIYGSWVYGYVGCLCITYLQYLGINASSCSITAFTIERYIAICHPIKAQFL
CTFSRAKKIIIFVWAFTSLYCMLWFFLLDLNISTYKDAIVISCGYKISRNYYSPIYLMDFGVFYVVPMIL
ATVLYGFIARILFLNPIPSDPKENSKTWKNDSTHQNTNLNVNTSNRCFNSTVSSRKQVTKMLAVVVI
LFALLWMPYRTLVVVNSFLSSPFQENWFLLFCRICIYLNSAINPVIYNLMSQKFRAAFRKLCNCKQK
PTEKPANYSVALNYSVIKESDHFSTELDDITVTDTYLSATKVSFDDTCLASEVSFSQS (SEQ ID NO:368)

# Figure 162

**Nucleotide:** X66114
**Sequence:**
atggcggcgacggcgagtgccggggccggcgggatggacgggaagccccgtacctcccctaagtccgtcaagttcctgtttgggggcc
tggccgggatgggagctacagtttttgtccagcccctggacctggtgaagaaccggatgcagttgagcggggaaggggccaagactcg
agagtacaaaaccagcttccatgccctcaccagtatcctgaaggcagaaggcctgaggggcatttacactgggctgtcggctggcctgct
gcgtcaggccacctacaccactacccgccttggcatctataccgtgctgtttgagcgcctgactggggctgatggtactcccccctggctttct
gctgaaggctgtgattggcatgaccgcaggtgccactggtgcctttgtgggaacaccagccgaagtggctcttatccgcatgactgccgat
ggccggcttccagctgaccagcgccgtggctacaaaaatgtgtttaacgccctgattcgaatcacccgggaagagggtgtcctcacactg
tggcggggctgcatccctaccatggctcgggccgtcgtcgtcaatgctgcccagctcgcctcctactcccaatccaagcagttcttactgga
ctcaggctacttctctgacaacatcctgtgccacttctgtgccagcatgatcagcggtcttgtcaccactgctgcctccatgcctgtggacattg
ccaagacccgaatccagaacatgcggatgattgatgggaagccggaatacaagaacgggctggacgtgctgttcaaagttgtccgcta
cgagggcttcttcagcctgtggaagggcttcacgccgtactatgcccgcctgggcccccacaccgtcctcaccttcatcttcttggagcagat
gaacaaggcctacaagcgtctcttcctcagtggctga (SEQ ID NO:369)

**Q02978**
MAATASAGAGGIDGKPRTSPKSVKFLFGGLAGMGATVFVQPLDLVKNRMQLSGEGAKTREYKTS
FHALTSILKAEGLRGIYTGLSAGLLRQATYTTTRLGIYTVLFERLTGADGTPPGFLLKAVIGMTAGAT
GAFVGTPAEVALIRMTADGRLPADQRRGYKNVFNALIRITREEGVLTLWRGCIPTMARAVVVNAA
QLASYSQSKQFLLDSGYFSDNILCHFCASMISGLVTTAASMPVDIAKTRIQNMRMIDGKPEYKNGL
DVLFKVVRYEGFFSLWKGFTPYYARLGPHTVLTFIFLEQMNKAYKRLFLSG (SEQ ID NO:370)

# Figure 163

**Nucleotide:** M84424
**Sequence:**
atgaaaacgctccttcttttgctgctggtgctcctggagctgggagaggcccaaggatcccttcacagggtgcccctcaggaggcatccgt
ccctcaagaagaagctgcgggcacggagccagctctctgagttctggaaatcccataatttggacatgatccagttcaccgagtcctgctc
aatggaccagagtgccaaggaacccctcatcaactacttggatatggaatacttcggcactatctccattggctccccaccacagaacttc
actgtcatcttcgacactggctcctccaacctctgggtcccctctgtgtactgcactagcccagcctgcaagacgcacagcaggttccagcc
ttcccagtccagcacatacagccagccaggtcaatctttctccattcagtatggaaccgggagcttgtccgggatcattggagccgaccaa
gtctctgtggaaggactaaccgtggttggccagcagtttggagaaagtgtcacagagccaggccagacctttgtggatgcagagtttgatg
gaattctgggcctgggatacccctccttggctgtgggaggagtgactccagtatttgacaacatgatggctcagaacctggtggacttgccg
atgttttctgtctacatgagcagtaacccagaaggtggtgcggggagcgagctgatttttggaggctacgaccactcccatttctctgggagc
ctgaattgggtcccagtcaccaagcaagcttactggcagattgcactggataacatccaggtgggaggcactgttatgttctgctccgagg
gctgccaggccattgtggacacagggacttccctcatcactggcccttccgacaagattaagcagctgcaaaacgccattggggcagcc
cccgtggatggagaatatgctgtggagtgtgccaaccttaacgtcatgccggatgtcaccttcaccattaacggagtcccctataccctcag
cccaactgcctacaccctactggacttcgtggatggaatgcagttctgcagcagtggctttcaaggacttgacatccaccctccagctgggc
cctctggatcctgggggatgtcttcattcgacagttttactcagtctttgaccgtgggaataaccgtgtgggactggccccagcagtcccta
a (SEQ ID NO:371)

**P14091**
MKTLLLLLLVLLELGEAQGSLHRVPLRRHPSLKKKLRARSQLSEFWKSHNLDMIQFTESCSMDQS
AKEPLINYLDMEYFGTISIGSPPQNFTVIFDTGSSNLWVPSVYCTSPACKTHSRFQPSQSSTYSQP
GQSFSIQYGTGSLSGIIGADQVSVEGLTVVGQQFGESVTEPGQTFVDAEFDGILGLGYPSLAVGG
VTPVFDNMMAQNLVDLPMFSVYMSSNPEGGAGSELIFGGYDHSHFSGSLNWVPVTKQAYWQIA
LDNIQVGGTVMFCSEGCQAIVDTGTSLITGPSDKIKQLQNAIGAAPVDGEYAVECANLNVMPDVTF
TINGVPYTLSPTAYTLLDFVDGMQFCSSGFQGLDIHPPAGPLWILGDVFIRQFYSVFDRGNNRVGL
APAVP (SEQ ID NO:372)

# Figure 164

**Nucleotide:** U29344
**Sequence:**
atggaggaggtggtgattgccggcatgtccgggaagctgccagagtcggagaacttgcaggagttctgggacaacctcatcggcggtgt
ggacatggtcacggacgatgaccgtcgctggaaggcggggctctacggcctgccccggcggtccggcaagctgaaggacctgtctag
gtttgatgcctccttcttcggagtccaccccaagcaggcacacacgatggaccctcagctgcggctgctgctggaagtcacctatgaagcc
atcgtggacggaggcatcaacccagattcactccgaggaacacacactggcgtctgggtgggcgtgagcggctctgagacctcggag
gccctgagccgagaccccgagacactcgtgggctacagcatggtgggctgccagcgagcgatgatggccaaccggctctccttcttcttc
gacttcagagggcccagcatcgcactggacacagcctgctcctccagcctgatggccctgcagaacgcctaccaggccatccacagcg
ggcagtgccctgccgccatcgtgggggcatcaatgtcctgctgaagcccaacacctccgtgcagttcttgaggctggggatgctcagcc
ccgagggcacctgcaaggccttcgacacagcggggaatgggtactgccgctcggagggtgtggtggccgtcctgctgaccaagaagtc
cctggcccggcgggtgtacgccaccatcctgaacgccggcaccaatacagatggcttcaaggagcaaggcgtgaccttcccctcaggg
gatatccaggagcagctcatccgctcgttgtaccagtcggccggagtggcccctgagtcatttgaatacatcgaagcccacggcacagg
caccaaggtgggcgacccccaggagctgaatggcatcacccgagccctgtgcgccacccgccaggagccgctgctcatcggctccac
caagtccaacatggggcacccggagccagcctcggggctggcagccctggccaaggtgctgctgtccctggagcacgggctctgggc
ccccaacctgcacttccatagccccaaccctgagatcccagcgctgttggatgggcggctgcaggtggtggaccagcccctgcccgtcc
gtggcggcaacgtgggcatcaactcctttggcttcgggggctccaacgtgcacatcatcctgaggcccaacacgcagccgccccccgc
acccgccccacatgccaccctgccccgtctgctgcgggccagcggacgcacccctgaggccgtgcagaagctgctggagcagggcct
ccggcacagccaggacctggctttcctgagcatgctgaacgacatcgcgctgtccccgaccaccgccatgcccttccgtggctacgctgt
gctgggtggtgagcgcggtggcccagaggtgcagcaggtgcccgctggcgagcgcccgctctggttcatctgctctgggatgggcacac
agtggcgcgggatggggctgagcctcatgcgcctggaccgcttccgagattccatcctacgctccgatgaggctgtgaaccgattcggcc
tgaaggtgtcacagctgctgctgagcacagacgagagcacctttgatgacatcgtccattcgtttgtgagcctgactgccatccagatagg
cctcatagacctgctgagctgcatggggctgaggccagatggcatcgtcggccactccctgggggaggtggcctgtggctacgccgacg
gctgcctgtcccaggaggaggccgtcctcgctgcctactggaggggacagtgcatcaaagaagcccatctcccgccgggcgccatggc
agccgtgggcttgtcctgggaggagtgtaaacagcgctgcccccggcggtggtgcccgcctgccacaactccaaggacacagtcacc
atctcgggacctcaggccccggtgtttgagttcgtggagcagctgaggaaggagggtgtgtttgccaaggaggtgcggaccggcggtat
ggccttccactcctacttcatggaggccatcgcacccccactgctgcaggagctcaagaaggtgatccgggagccgaagccacgttcag
cccgctggctcagcacctctatccccgaggcccagtggcacagcagcctggcacgcacgtcctccgccgagtacaatgtcaacaacct
ggtgagccctgtgctgttccaggaggccctgtggcacgtgcctgagcacgcggtggtgctggagatcgcgccccacgccctgctgcagg
ctgtcctgaagcgtggcctgaagccgagctgcaccatcatcccccctgatgaagaaggatcacagggacaacctggagttcttcctggcc
ggcatccggaggctgcacctctcaggcatcgacgccaaccccaatgccttgttcccacctgtggagttcccagctccccgaggaactccc
ctcatctccccactcatcaagtgggaccacagcctggcctgggacgtgccggccgccgaggacttccccaacggttcaggttcccccctca
gccgccatctacaacatcgacaccagctccgagtctcctgaccactacctggtggaccacaccctcgacggtcgcgtcctcttccccgcc
actggctacctgagcatagtgtggaagacgctggcccgaccccctgggcctgggcgtcgagcagctgcctgtggtgtttgaggatgtggtg
ctgcaccaggccaccatcctgcccaagactgggacagtgtccctggaggtacggctcctggaggcctcccgtgccttcgaggtgtcaga
gaacggcaacctggtagtgagtgggaaggtgtaccagtgggatgaccctgaccccaggctcttcgaccacccggaaagccccacccc
caaccccacggagcccctcttcctggcccaggctgaagtttacaaggagctgcgtctgcgtggctacgactacggccctcatttccaggg
catcctggaggccagcctggaaggtgactcggggaggctgctgtggaaggataactgggtgagcttcatggacaccatgctgcagatgt
ccatcctgggctcggccaagcacggcctgtacctgcccacccgtgtcaccgccatccacatcgaccctgccacccacaggcagaagct
gtacacactgcaggacaaggcccaagtggctgacgtggtggtgagcaggtggctgagggtcacagtggccggaggcgtccacatctc
cgggctccacactgagtcggccccgcggcggcagcaggagcagcaggtgcccatcctggagaagttttgcttcacttcccacacggag
gaggggtgcctgtctgagcgcgctgccctgcaggaggagctgcaactgtgcaaggggctggtgcaggcactgcagaccaaggtgacc
cagcaggggctgaagatggtggtgcccggactggatggggcccagatcccccgggacccctcacagcaggaactgccccggctgttg
tcggctgcctgcaggcttcagctcaacgggaacctgcagctggagctggcgcaggtgctggcccaggagaggcccaagctgccagag
gaccctctgctcagcggcctcctggactccccggcactcaaggcctgcctggacactgccgtggagaacatgcccagcctgaagatga
aggtggtggaggtgctggccggccacggtcacctgtattcccgcatcccaggcctgctcagcccccatcccctgctgcagctgagctaca
cggccaccgaccgccacccccaggccctggaggctgcccaggccgagctgcagcagcacgacgttgcccagggccagtgggatcc
cgcagaccctgcccccagcgccctgggcagcgccgacctcctggtgtgcaactgtgctgtggctgccctcggggacccggcctcagctc
tcagcaacatggtggctgccctgagagaagggggctttctgctcctgcacacactgctccgggggcacccctcgggacatgtggccttcct
cacctccactgagccgcagtatggccagggcatcctgagccaggacgcgtgggagagcctcttctccagggtgtccgtgcgcctggtgg
gcctgaagaagtccttctacggctccacgctcttcctgtgccgccggcccaccccgcaggacagccccatcttcctgccggtggacgata
ccagcttccgctgggtggagtctctgaagggcatcctggctgacgaagactcttcccggcctgtgtggctgaaggccatcaactgtgccac
ctcgggcgtggtgggcttggtgaactgtctccgccgagagcccggcggaacgctccggtgtgtgctgctctccaacctcagcagcacctc
ccacgtcccggaggtggacccgggctccgcagaactgcagaaggtgttgcagggagacctggtgatgaacgtctaccgcgacggggc
ctgggggctttccgccacttcctgctggaggaggacaagcctgaggagccgacggcacatgcctttgtgagcaccctcacccgggggg

gacctgtcctccatccgctgggtctgctcctcgctgcgccatgcccagcccacctgccctggcgcccagctctgcacggtctactacgcctc
cctcaacttccgcgacatcatgctggccactggcaagctgtcccctgatgccatcccagggaagtggacctcccaggacagcctgctag
gtatggagttctcgggccgagacgccagcggcaagcgtgtgatgggactggtgcctgccaagggcctggccacctctgtcctgctgtcac
cggacttcctctgggatgtgccttccaactggacgctggaggaggcggcctcggtgcctgtcgtctacagcacggcctactacgcgctggt
ggtgcgtgggcgggtgcgccccggggagacgctgctcatccactcgggctcgggcggcgtgggccaggccgccatcgccatcgccct
cagtctgggctgccgcgtcttcaccaccgtggggtcggctgagaagcgggcgtacctccaggccaggttcccccagctcgacagcacc
agcttcgccaactcccgggacacatccttcgagcagcatgtgctgtggcacacgggcgggaagggcgttgacctggtcttgaactccttg
gcggaagagaagctgcaggccagcgtgaggtgcttggctacgcacggtcgcttcctggaaattggcaaattcgacctttctcagaacca
cccgctcggcatggctatcttcctgaagaacgtgacattccacggggtcctactggatgcgttcttcaacgagagcagtgctgactggcgg
gaggtgtgggcgcttgtgcaggccggcatccgggatggggtggtacggcccctcaagtgcacggtgttccatggggcccaggtggagg
acgccttccgctacatggcccaagggaagcacattggcaaagtcgtcgtgcaggtgcttgcggaggagccggaggcagtgctgaagg
gggccaaacccaagctgatgtcggccatctccaagaccttctgcccggcccacaagagctacatcatcgctggtggtctgggtggcttcg
gcctggagttggcgcagtggctgatacagcgtggggtgcagaagctcgtgttgacttctcgctccgggatccggacaggctaccaggcc
aagcaggtccgccggtggagggcccagggcgtacaggtgcaggtgtccaccagcaacatcagctcactggagggggcccggggcct
cattgccgaggcggcgcagcttgggcccgtgggcggcgtcttcaacctggccgtggtcttgagagatggcttgctggagaaccagaccc
cagagttcttccaggacgtctgcaagcccaagtacagcggcaccctgaacctggacagggtgacccgagaggcgtgccctgagctgg
actactttgtggtcttctcctctgtgagctgcgggcgtggcaatgcgggacagagcaactacggctttgccaattccgccatggagcgtatct
gtgagaaacgccggcacgaaggcctcccaggcctggccgtgcagtggggcgccatcggcgacgtgggcattttggtggagacgatga
gcaccaacgacacgatcgtcagtggcacgctgccccaggccatggcgtcctgcctggaggtgctggacctcttcctgaaccagccccac
atggtcctgagcagctttgtgctggctgagaaggctgcggcctatagggacagggacagccagcgggacctggtggaggccgtggcac
acatcctgggcatccgcgacttggctgctgtcaacctggacagctcactggcggacctgggcctggactcgctcatgagcgtggaggtgc
gccagacgctggagcgtgagctcaacctggtgctgtccgtgcgcgaggtgcggcaactcacgctccggaaactgcaggagctgtcctc
aaaggcggatgaggccagcgagctggcatgccccacgcccaaggaggatggtctggcccagcagcagactcagctgaacctgcgct
ccctgctggtgaacccggagggccccaccctgatgcggctcaactccgtgcagagctcggagcggcccctgttcctggtgcacccaatc
gagggctccaccaccgtgttccacagcctggcctcccggctcagcatccccacctatggcctgcagtgcacccgagctgcgccccttgac
agcatccacagcctggctgcctactacatcgactgcatcaggcaggtgcagcccgagggcccctaccgcgtggccggctactcctacg
ggcctgcgtggcctttgaaatgtgctcccagctgcaggcccagcagagcccagcccccacccacaacagcctcttcctgttcgacggct
cgcccacctacgtactggcctacacccagagctaccgggcaaagctgaccccaggctgtgaggctgaggctgagacggaggccatat
gcttcttcgtgcagcagttcacggacatggagcacaacagggtgctggaggcgctgctgccgctgaagggcctagaggagcgtgtggc
agccgccgtggacctgatcatcaagagccaccagggcctggaccgccaggagctgagctttgcggcccggtccttctactacaagctcg
gtgccgctgagcagtacacacccaaggccaagtaccatggcaacgtgatgctactgcgcgccaagacgggtggcgcctacggcgag
gacctgggcgcggattacaacctctcccaggtatgcgacgggaaagtatccgtccacgtcatcgagggtgaccaccgcacgctgctgg
agggcagcggcctggagtccatcatcagcatcatccacagctccctggctgagccacgcgtgagcgtgcgggagggctag (SEQ ID NO:373)

**Q16702**

MEEVVIAGMSGKLPESENLQEFWDNLIGGVDMVTDDDRRWKAGLYGLPRRSGKLKDLSRFDASF
FGVHPKQAHTMDPQLRLLLEVTYEAIVDGGINPDSLRGTHTGVWVGVSGSETSEALSRDPETLVG
YSMVGCQRAMMANRLSFFFDFRGPSIALDTACSSSLMALQNAYQAIHSGQCPAAIVGGINVLLKP
NTSVQFLRLGMLSPEGTCKAFDTAGNGYCRSEGVVAVLLTKKSLARRVYATILNAGTNTDGFKEQ
GVTFPSGDIQEQLIRSLYQSAGVAPESFEYIEAHGTGTKVGDPQELNGITRALCATRQEPLLIGSTK
SNMGHPEPASGLAALAKVLLSLEHGLWAPNLHFHSPNPEIPALLDGRLQVVDQPLPVRGGNVGIN
SFGFGGSNVHIILRPNTQPPPAPAPHATLPRLLRASGRTPEAVQKLLEQGLRHSQDLAFLSMLNDI
ALSPTTAMPFRGYAVLGGERGGPEVQQVPAGERPLWFICSGMGTQWRGMGLSLMRLDRFRDSI
LRSDEAVNRFGLKVSQLLLSTDESTFDDIVHSFVSLTAIQIGLIDLLSCMGLRPDGIVGHSLGEVAC
GYADGCLSQEEAVLAAYWRGQCIKEAHLPPGAMAAVGLSWEECKQRCPPAVVPACHNSKDTVTI
SGPQAPVFEFVEQLRKEGVFAKEVRTGGMAFHSYFMEAIAPPLLQELKKVIREPKPRSARWLSTSI
PEAQWHSSLARTSSAEYNVNNLVSPVLFQEALWHVPEHAVVLEIAPHALLQAVLKRGLKPSCTIIP
LMKKDHRDNLEFFLAGIRRLHLSGIDANPNALFPPVEFPAPRGTPLISPLIKWDHSLAWDVPAAED
FPNGSGSPSAAIYNIDTSSESPDHYLVDHTLDGRVLFPATGYLSIVWKTLARPLGLGVEQLPVVFE
DVVLHQATILPKTGTVSLEVRLLEASRAFEVSENGNLVVSGKVYQWDDPDPRLFDHPESPTPNPT
EPLFLAQAEVYKELRLRGYDYGPHFQGILEASLEGDSGRLLWKDNWVSFMDTMLQMSILGSAKH
GLYLPTRVTAIHIDPATHRQKLYTLQDKAQVADVVVSRWLRVTVAGGVHISGLHTESAPRRQQEQ
QVPILEKFCFTSHTEEGCLSERAALQEELQLCKGLVQALQTKVTQQGLKMVVPGLDGAQIPRDPS
QQELPRLLSAACRLQLNGNLQLELAQVLAQERPKLPEDPLLSGLLDSPALKACLDTAVENMPSLK
MKVVEVLAGHGHLYSRIPGLLSPHPLLQLSYTATDRHPQALEAAQAELQQHDVAQGQWDPADPA
PSALGSADLLVCNCAVAALGDPASALSNMVAALREGGFLLLHTLLRGHPSGHVAFLTSTEPQYGQ

GILSQDAWESLFSRVSVRLVGLKKSFYGSTLFLCRRPTPQDSPIFLPVDDTSFRWVESLKGILADE
DSSRPVWLKAINCATSGVVGLVNCLRREPGGTLRCVLLSNLSSTSHVPEVDPGSAELQKVLQGDL
VMNVYRDGAWGAFRHFLLEEDKPEEPTAHAFVSTLTRGDLSSIRWVCSSLRHAQPTCPGAQLCT
VYYASLNFRDIMLATGKLSPDAIPGKWTSQDSLLGMEFSGRDASGKRVMGLVPAKGLATSVLLSP
DFLWDVPSNWTLEEAASVPVVYSTAYYALVVRGRVRPGETLLIHSGSGGVGQAAIAIALSLGCRV
FTTVGSAEKRAYLQARFPQLDSTSFANSRDTSFEQHVLWHTGGKGVDLVLNSLAEEKLQASVRC
LATHGRFLEIGKFDLSQNHPLGMAIFLKNVTFHGVLLDAFFNESSADWREVWALVQAGIRDGVVR
PLKCTVFHGAQVEDAFRYMAQGKHIGKVVVQVLAEEPEAVLKGAKPKLMSAISKTFCPAHKSYIIA
GGLGGFGLELAQWLIQRGVQKLVLTSRSGIRTGYQAKQVRRWRAQGVQVQVSTSNISSLEGARG
LIAEAAQLGPVGGVFNLAVVLRDGLLENQTPEFFQDVCKPKYSGTLNLDRVTREACPELDYFVVF
SSVSCGRGNAGQSNYGFANSAMERICEKRRHEGLPGLAVQWGAIGDVGILVETMSTNDTIVSGT
LPQAMASCLEVLDLFLNQPHMVLSSFVLAEKAAAYRDRDSQRDLVEAVAHILGIRDLAAVNLDSSL
ADLGLDSLMSVEVRQTLERELNLVLSVREVRQLTLRKLQELSSKADEASELACPTPKEDGLAQQQ
TQLNLRSLLVNPEGPTLMRLNSVQSSERPLFLVHPIEGSTTVFHSLASRLSIPTYGLQCTRAAPLDS
IHSLAAYYIDCIRQVQPEGPYRVAGYSYGACVAFEMCSQLQAQQSPAPTHNSLFLFDGSPTYVLA
YTQSYRAKLTPGCEAEAETEAICFFVQQFTDMEHNRVLEALLPLKGLEERVAAAVDLIIKSHQGLD
RQELSFAARSFYYKLGAAEQYTPKAKYHGNVMLLRAKTGGAYGEDLGADYNLSQVCDGKVSVHV
IEGDHRTLLEGSGLESIISIIHSSLAEPRVSVREG (SEQ ID NO:374)

# Figure 165

**Nucleotide:** AB083594
**Sequence:**
atggaggcaccacctctcatggttgtgttagaatataagcataactttaaaatagtatccactaattgtatgaggtgctatttggtaatgctgag
gttgtccctcaaaagcattgattcaatcattttcacttccatgggagtgcaaaataaacatatgcctagggcccccataggagccaacgaa
gtcatctttgcttcagagctaaacccagtttttcttctctccacagcaaatatcttgacagtgatcatcctctcccagctggtggcaagaagaca
gaagtcctcctacaactatctcttggcactcgctgctgccgacatcttggtcctctttttcatagtgtttgtggacttcctgttggaagatttcatcttg
aacatgcagatgcctcaggtccccgacaagatcatagaagtgctggaattctcatccatccacacctccatatggattactgtaccgttaac
cattgacaggtatatcgctgtctgccacccgctcaagtaccacacggtctcatacccagcccgcacccggaaagtcattgtaagtgtttaca
tcacctgcttcctgaccagcatcccctattactggtggcccaacatctggactgaagactacatcagcacctctgtgcatcacgtcctcatctg
gatccactgcttcaccgtctacctggtgccctgctccatcttcttcatcttgaactcaatcattgtgtacaagctcaggaggaagagcaattttc
gtctccgtggctactccacggggaagaccaccgccatcttgttcaccattacctccatctttgccacactttgggcccccgcatcatcatgat
tctttaccacctctatggggcgcccatccagaaccgctggctggtacacatcatgtccgacattgccaacatgctagcccttctgaacacag
ccatcaacttcttcctctactgcttcatcagcaagcggttccgcaccatggcagccgccacgctcaaggctttcttcaagtgccagaagcaa
cctgtacagttctacaccaatcataacttttccataacaagtagcccctggatctcgccggcaaactcacactgcatcaagatgctggtgta
ccagtatgacaaaaatggaaaacctataaaagtatccccgtga (SEQ ID NO:375)

**Q8TDU8**
MEAPPLMVVLEYKHNFKIVSTNCMRCYLVMLRLSLKSIDSIIFTSMGVQNKHMPRAPIGANEVIFAS
ELNPVFLLSTANILTVIILSQLVARRQKSSYNYLLALAAADILVLFFIVFVDFLLEDFILNMQMPQVPDK
IIEVLEFSSIHTSIWITVPLTIDRYIAVCHPLKYHTVSYPARTRKVIVSVYITCFLTSIPYYWWPNIWTE
DYISTSVHHVLIWIHCFTVYLVPCSIFFILNSIIVYKLRRKSNFRLRGYSTGKTTAILFTITSIFATLWAP
RIIMILYHLYGAPIQNRWLVHIMSDIANMLALLNTAINFFLYCFISKRFRTMAAATLKAFFKCQKQPV
QFYTNHNFSITSSPWISPANSHCIKMLVYQYDKNGKPIKVSP (SEQ ID NO:376)

# Figure 166

**Nucleotide:** D11094
**Sequence:**
atgccggattacctcggtgccgatcagcggaagaccaaagaggatgagaaggacgacaagcccatccgagctctggatgaggggga
tattgccttgttgaaaacttatggtcagagcacttactctaggcagatcaagcaagttgaagatgacattcagcaacttctcaagaaaattaa
tgagctcactggtattaaagaatctgacactggcctggccccaccagcactctgggatttggctgcagataagcagacactccagagtga
acagccctttacaggttgccaggtgtacaaagataatcaatgctgattcggaggacccaaaatacattatcaacgtaaagcagtttgccaa
gtttgtggtggaccttagtgatcaggtggcacctactgacattgaagaagggatgagagtgggcgtggatagaaataaatatcaaattcac
attccattgcctcctaagattgacccaacagttaccatgatgcaggtggaagagaaacctgatgtcacatacagtgatgttggtggctgtaa
ggaacagattgagaaactgcgagaagtagttgaaaccccattacttcatccagagaggtttgtgaaccttggcattgagcctcccaaggg
cgtgctgctctttggtccacccggtacaggcaagacactctgtgcgcgggcagttgctaatcggactgatgcgtgcttcattcgagttattgg
atctgagcttgtacagaaatacgtcggtgagggggctcgaatggttcgtgaactctttgaaatggccagaacaaaaaaagcctgccttatc
ttctttgatgaaattgatgctattggaggggctcgttttgatgatggtgctggaggtgacaatgaagtgcagagaacaatgttggaactgatca
atcagcttgatggttttgatcctcgaggcaatattaaagtgctgatggccactaacagacctgatactttggatccagcactgatgaggccag
ggagattggatagaaaaattgaatttagcttgcccgatctagagggtcggacccacatatttaagattcacgctcgttcaatgagtgttgaaa
gagatatcagatttgaactgttagcacgactgtgtccaaatagcactggtgctgagattagaagcgtctgcacagaggctggtatgtttgcc
atcagagcacggcgaaaaattgctaccgagaaggatttcttggaagctgtaaataaggtcattaagtcttatgccaaattcagtgctactcc
tcgttacatgacatacaactga (SEQ ID NO:377)

**P35998**
MPDYLGADQRKTKEDEKDDKPIRALDEGDIALLKTYGQSTYSRQIKQVEDDIQQLLKKINELTGIKE
SDTGLAPPALWDLAADKQTLQSEQPLQVARCTKIINADSEDPKYIINVKQFAKFVVDLSDQVAPTDI
EEGMRVGVDRNKYQIHIPLPPKIDPTVTMMQVEEKPDVTYSDVGGCKEQIEKLREVVETPLLHPE
RFVNLGIEPPKGVLLFGPPGTGKTLCARAVANRTDACFIRVIGSELVQKYVGEGARMVRELFEMA
RTKKACLIFFDEIDAIGGARFDDGAGGDNEVQRTMLELINQLDGFDPRGNIKVLMATNRPDTLDPA
LMRPGRLDRKIEFSLPDLEGRTHIFKIHARSMSVERDIRFELLARLCPNSTGAEIRSVCTEAGMFAI
RARRKIATEKDFLEAVNKVIKSYAKFSATPRYMTYN (SEQ ID NO:378)

# Figure 167

**Nucleotide:** AF104413
**Sequence:**
atgaagaggagtgaaaagccagaaggatatagacaaatgaggcctaagacctttcctgccagtaactatactgtcagtagccggcaaa
tgttacaagaaattcgggaatcccttaggaatttatctaaaccatctgatgctgctaaggctgagcataacatgagtaaaatgtcaaccgaa
gatcctcgacaagtcagaaatccacccaaatttgggacgcatcataaagccttgcaggaaattcgaaactctctgcttccatttgcaaatg
aaacaaattcttctcggagtacttcagaagttaatccacaaatgcttcaagacttgcaagctgctggatttgatgaggatatggttatacaag
ctcttcagaaaactaacaacagaagtatagaagcagcaattgaattcattagtaaaatgagttaccaagatcctcgacgagagcagatg
gctgcagcagctgccagacctattaatgccagcatgaaaccagggaatgtgcagcaatcagttaaccgcaaacagagctggaaaggtt
ctaaagaatccttagttcctcagaggcatggcccgccactaggagaaagtgtggcctatcattctgagagtcccaactcacagacagatg
taggaagacctttgtctggatctggtatatcagcatttgttcaagctcaccctagcaacggacagagagtgaaccccccaccaccacctca
agtaaggagtgttactcctccaccacctccaagaggccagactccccctccaagaggtacaactccacctccccccttcatgggaaccaa
actctcaaacaaagcgctattctggaaacatggaatacgtaatctcccgaatctctcctgtcccacctggggcatggcaagagggctatcc
tccaccacctctcaacacttcccccatgaatcctcctaatcaaggacagagaggcattagttctgttcctgttggcagacaaccaatcatcat
gcagagttctagcaaatttaactttccatcagggagacctggaatgcagaatggtactggacaaactgatttcatgatacaccaaaatgttg
tccctgctggcactgtgaatcggcagccaccacctccatatcctctgacagcagctaatggacaaagcccttctgctttacaaacagggggg
atctgctgctccttcgtcatatacaaatggaagtattcctcagtctatgatggtgccaaacagaaatagtcataacatggaactatataacatt
agtgtacctggactgcaaacaaattggcctcagtcatcttctgctccagcccagtcatccccgagcagtgggcatgaaatccctacatggc
aacctaacataccagtgaggtcaaattcttttaataacccattaggaaatagagcaagtcactctgctaattctcagccttctgctacaacag
tcactgcaattacaccagctcctattcaacagcctgtgaaaagtatgcgtgtattaaaaccagagctacagactgctttagcacctacacac
ccttcttggataccacagccaattcaaactgttcaacccagtccttttcctgagggaaccgcttcaaatgtgactgtgatgccacctgttgctg
aagctccaaactatcaaggaccaccaccaccccctacccaaaacatctgctgcaccaaaacccatctgttcctccatacgagtcaatcagt
aagcctagcaaagaggatcagccaagcttgcccaaggaagatgagagtgaaaagagttatgaaaatgttgatagtggggataaagaa
aagaaacagattacaacttcacctattactgttaggaaaaacaagaaagatgaagagcgaagggaatctcgtattcaaagttattctcctc
aagcatttaaattctttatggagcaacatgtagaaaatgtactcaaatctcatcagcagcgtctacatcgtaaaaaacaattagagaatgaa
atgatgcgggttggattatctcaagatgcccaggatcaaatgagaaagatgctttgccaaaaagaatctaattacatccgtcttaaaaggg
ctaaaatggacaagtctatgtttgtgaagataaagacactaggaataggagcatttggtgaagtctgtctagcaagaaaagtagatactaa
ggctttgtatgcaacaaaaactcttcgaaagaaagatgttcttcttcgaaatcaagtcgctcatgttaaggctgagagagatatcctggctga
agctgacaatgaatgggtagttcgtctatattattcattccaagataaggacaatttatactttgtaatggactacattcctggggggtgatatgat
gagcctattaattagaatgggcatctttccagaaaagtctggcacgattctacatagcagaacttacctgtgcagttgaaagtgttcataaaat
gggttttattcatagagatattaaacctgataatattttgattgatcgtgatggtcatattaaattgactgactttggcctctgcactggcttcagat
ggacacacgattctaagtactatcagagtggtgaccatccacggcaagatagcatggatttcagtaatgaatggggggatccctcaagct
gtcgatgtggagacagactgaagccattagagcggagagctgcacgccagcaccagcgatgtctagcacattctttggttgggactccc
aattatattgcacctgaagtgttgctacgaacaggatacacacagttgtgtgattggtggagtgttggtgttattctttttgaaatgttggtgggac
aacctcctttcttggcacaaacaccattagaaacacaaatgaaggttatcaactggcaaacatctcttcacattccaccacaagctaaact
cagtcctgaagcttctgatcttattattaaactttgccgaggacccgaagatcgcttaggcaagaatggtgctgatgaaataaaagctcatcc
attttttaaaacaattgacttctccagtgacctgagacagcagtctgcttcatacattcctaaaatcacacacccaacagatacatcaaatttt
gatcctgttgatcctgataaattatggagtgatgataacgaggaagaaaatgtaaatgacactctcaatggatggtataaaaatggaaagc
atcctgaacatgcattctatgaatttaccttccgaaggttttttgatgacaatggctacccatataattatccgaagcctattgaatatgaataca
ttaattcacaaggctcagagcagcagtcggatgaagatgatcaaaacacaggctcagagattaaaaatcgcgatctagtatatgtttaa
(SEQ ID NO:379)

**O95835**
MKRSEKPEGYRQMRPKTFPASNYTVSSRQMLQEIRESLRNLSKPSDAAKAEHNMSKMSTEDPR
QVRNPPKFGTHHKALQEIRNSLLPFANETNSSRSTSEVNPQMLQDLQAAGFDEDMVIQALQKTNN
RSIEAAIEFISKMSYQDPRREQMAAAAARPINASMKPGNVQQSVNRKQSWKGSKESLVPQRHGP
PLGESVAYHSESPNSQTDVGRPLSGSGISAFVQAHPSNGQRVNPPPPPQVRSVTPPPPPRGQTP
PPRGTTPPPPSWEPNSQTKRYSGNMEYVISRISPVPPGAWQEGYPPPPLNTSPMNPPNQGQRGI
SSVPVGRQPIIMQSSSKFNFPSGRPGMQNGTGQTDFMIHQNVVPAGTVNRQPPPPYPLTAANGQ
SPSALQTGGSAAPSSYTNGSIPQSMMVPNRNSHNMELYNISVPGLQTNWPQSSSAPAQSSPSS
GHEIPTWQPNIPVRSNSFNNPLGNRASHSANSQPSATTVTAITPAPIQQPVKSMRVLKPELQTALA
PTHPSWIPQPIQTVQPSPFPEGTASNVTVMPPVAEAPNYQGPPPPYPKHLLHQNPSVPPYESISK
PSKEDQPSLPKEDESEKSYENVDSGDKEKKQITTSPITVRKNKKDEERRESRIQSYSPQAFKFFM
EQHVENVLKSHQQRLHRKKQLENEMMRVGLSQDAQDQMRKMLCQKESNYIRLKRAKMDKSMF
VKIKTLGIGAFGEVCLARKVDTKALYATKTLRKKDVLLRNQVAHVKAERDILAEADNEWVVRLYYS

FQDKDNLYFVMDYIPGGDMMSLLIRMGIFPESLARFYIAELTCAVESVHKMGFIHRDIKPDNILIDRD
GHIKLTDFGLCTGFRWTHDSKYYQSGDHPRQDSMDFSNEWGDPSSCRCGDRLKPLERRAARQ
HQRCLAHSLVGTPNYIAPEVLLRTGYTQLCDWWSVGVILFEMLVGQPPFLAQTPLETQMKVINWQ
TSLHIPPQAKLSPEASDLIIKLCRGPEDRLGKNGADEIKAHPFFKTIDFSSDLRQQSASYIPKITHPTD
TSNFDPVDPDKLWSDDNEEENVNDTLNGWYKNGKHPEHAFYEFTFRRFFDDNGYPYNYPKPIEY
EYINSQGSEQQSDEDDQNTGSEIKNRDLVYV (SEQ ID NO:380)

**Nucleotide:** AB028019
**Sequence:**
caaacagtgacacttccctggatgccaaagtcctggggagcaaagatgccaccagcagcaagcagcagatgagagccaccccaaa
gttcggaccttatcagaaagccttgagggaaatcagatattccttgttgccttttgctaatgaatcgggcacctctgcagctgcagaagtgaa
ccggcaaatgctgcaggaactggtgaacgcaggatgcgaccaggagatggctggccgagctctcaagcagactggcagcaggagc
atcgaggccgccctggagtacatcagcaagatgggctacctggacccgaggaatgagcagattgtgcgggtcattaagcagacctccc
caggaaaggggctcatgccaaccccagtgacgcggaggcccagcttcgaaggaaccggcgattcgtttgcgtcctaccaccagctga
gcggtacccccctacgagggcccaagcttcggcgctgacggccccacggcgctggaggagatgccgcggccgtacgtggactaccttt
ccccggagtcggcccccacgggcccggccaccagcaccagcacccacccaagggctacggtgccagcgtagaggcagcaggggc
acacttcccgctgcagggcgcgcactacgggcggccgcacctgctggtgcctggggaacccctgggctacggagtgcagcgcagccc
ctccttccagagcaagacgccgccggagaccgggggttacgccagcctgcccacgaagggccagggaggaccgccaggcgccgg
cctcgctttcccaccccctgccgccgggctctacgtgccgcacccacaccacaagcaggccggtcccgtggcccaccagctgcatgtgc
tgggctcccgcagccaggtgttcgccagcgacagcccccgcagagcctgctcactccctcgcggaacagcctcaacgtggacctgtat
gaattgagcagcacctccgtccagcagtggccggctgccaccctggcccgccgggactccctgcagaagccgggcctggaggcgcc
gccgcgcgcgcacgtggccttccggcctgactgcccagtgcccagcaggaccaactccttcaacagccaccagccgcggcccggtcc
gcctggcaaggccgagccctccctgcccgcccccaacaccgtgacggctgtcagcgccgcgcacatcttgcacccggtgaagagcgt
gcgtgtgctgaggccggagccgcagacggctgtggggccctcgcaccccgcctgggtgcccgcgcctgccccggcccccgcccccgc
ccccgccccggctgcggagggcttggacgccaaggaggagcatgccctggcgctgggcggcgcaggcgccttcccgctggacgtgg
agtacggaggcccagaccggagtgcccgcctccgccctacccgaagcacctgctgctgcgcaaagtcggagcagtacgacctggac
agcctgtgcgcaggcatggagcagagcctccgtgcgggccccaacgagcccgagggcggcgacaagagccgcaaaagcgccaa
gggggacaaaggcggaaaggataaaaagcagattcagacctctcccgttcccgtccgcaaaaacagcagagacgaagagaagag
agagtcacgcatcaagagctactcgccatacgcctttaagttcttcatggagcagcacgtggagaatgtcatcaaaacctaccagcaga
aggttaaccggaggctgcagctggagcaagaaatggccaaagctggactctgtgaagctgagcaggagcagatgcggaagatcctct
accagaaagagtctaattacaacaggttaaagagggccaagatggacaagtctatgtttgtcaagatcaaaaccctggggatcggtgcc
tttggagaagtgtgccttgcttgtaaggtggacactcacgccctgtacgccatgaagaccctaaggaaaaaggatgtcctgaaccggaat
caggtggcccacgtcaaggccgagagggacatcctggccgaggcagacaatgagtgggtggtcaaactctactactccttccaagaca
aagacagcctgtactttgtgatggactacatccctggtggggacatgatgagcctgctgatccggatggaggtcttccctgagcacctggc
ccggttctacatcgcagagctgactttggccattgagagtgtccacaagatgggcttcatccaccgagacatcaagcctgataacattttga
tagatctggatggtcacattaaactcacagatttcggcctctgcactgggttcaggtggactcacaattccaaatattaccagaaagggagc
catgtcagacaggacagcatggagcccagcgacctctgggatgatgtgtctaactgtcggtgtggggacaggctgaagaccctagagc
agagggcgcggaagcagcaccagaggtcgctggcacattcactggtggggactccaaactacatcgcacccgaggtgctcctccgca
aagggtacactcaactctgtgactggtggagtgttggagtgattctcttcgagatgctggtggggcagccgcccttttggcacctactccca
cagaaacccagctgaaggtgatcaactgggagaacacgctccacattccagcccaggtgaagctgagccctgaggccagggacctc
atcaccaagctgtgctgctccgcagaccaccgcctggggcggaatggggccgatgacctgaaggcccaccccttcttcagcgccattga
cttctccagtgacatccggaagcagccagcccccctacgttcccaccatcagccaccccatggacacctcgaatttcgaccccgtagatga
agaaagcccttggaacgatgccagcgaaggtagcaccaaggcctgggacacactcacctcgcccaataacaagcatcctgagcacg
cattttacgaattcaccttccgaaggttctttgatgacaatggctacccctttcgatgcccaaagccttcaggagcagaagcttcacaggctg
agagctcagatttagaaagctctgatctggtggatcagactgaaggctgccagcctgtgtacgtgtag (SEQ ID NO:381)

**Q9P2X1**
NSDTSLDAKVLGSKDATSSKQQMRATPKFGPYQKALREIRYSLLPFANESGTSAAAEVNRQMLQ
ELVNAGCDQEMAGRALKQTGSRSIEAALEYISKMGYLDPRNEQIVRVIKQTSPGKGLMPTPVTRR
PSFEGTGDSFASYHQLSGTPYEGPSFGADGPTALEEMPRPYVDYLFPGVGPHGPGHQHQHPPK
GYGASVEAAGAHFPLQGAHYGRPHLLVPGEPLGYGVQRSPSFQSKTPPETGGYASLPTKGQGG
PPGAGLAFPPPAAGLYVPHPHHKQAGPVAHQLHVLGSRSQVFASDSPPQSLLTPSRNSLNVDLY
ELSSTSVQQWPAATLARRDSLQKPGLEAPPRAHVAFRPDCPVPSRTNSFNSHQPRPGPPGKAE
PSLPAPNTVTAVSAAHILHPVKSVRVLRPEPQTAVGPSHPAWVPAPAPAPAPAPAPAAEGLDAKE
EHALALGGAGAFPLDVEYGGPDRSARLRPTRSTCCCAKSEQYDLDSLCAGMEQSLRAGPNEPE
GGDKSRKSAKGDKGGKDKKQIQTSPVPVRKNSRDEEKRESRIKSYSPYAFKFFMEQHVENVIKTY
QQKVNRRLQLEQEMAKAGLCEAEQEQMRKILYQKESNYNRLKRAKMDKSMFVKIKTLGIGAFGE

VCLACKVDTHALYAMKTLRKKDVLNRNQVAHVKAERDILAEADNEWVVKLYYSFQDKDSLYFVM
DYIPGGDMMSLLIRMEVFPEHLARFYIAELTLAIESVHKMGFIHRDIKPDNILIDLDGHIKLTDFGLCT
GFRWTHNSKYYQKGSHVRQDSMEPSDLWDDVSNCRCGDRLKTLEQRARKQHQRSLAHSLVGT
PNYIAPEVLLRKGYTQLCDWWSVGVILFEMLVGQPPFLAPTPTETQLKVINWENTLHIPAQVKLSP
EARDLITKLCCSADHRLGRNGADDLKAHPFFSAIDFSSDIRKQPAPYVPTISHPMDTSNFDPVDEE
SPWNDASEGSTKAWDTLTSPNNKHPEHAFYEFTFRRFFDDNGYPFRCPKPSGAEASQAESSDL
ESSDLVDQTEGCQPVYV (SEQ ID NO:382)

# Figure 168

**Nucleotide:** AF283512
**Sequence:**
atgaaacaggcttccagacagttccagtccattctgcaagagaggcaatcactccctgcttgggaagaaagagaaaccattcttaacttat
tgcgtaagcaccaggtggttgtcataagtggtatgactggatgtgggaaaaccacacaaattccgcagtttattctggatgattctctgagtg
gaccacctgagaaggtagccaacatcatctgtacccaaccccgacgaatctctgcaatctctgttgctgaacgcgttgctaaagaaagag
cagagagggtgggtctgaccgtgggataccagattcggttagaaagtgtcaagtcctcagccaccagactgttatactgcaccacggga
gtgctgctgagaaggctagaaggagatacagctctacaaggagtttcccatatcattgttgatgaagttcatgagaggacagaagaaagt
gacttcttgctgctagttttgaaggacattgtatcgcagaggccaggtcttcaagttattttaatgagtgcaactctaaacgctgagctttttcag
actattttaattcctgccccgtcattactataccaggtcgtacatttcctgttgatcaattttttttggaagatgcaattgctgtgacaaggtatgtatt
acaggatgggagcccatatatgcggtccatgaaacagatttcaaaggaaaagcttaaagcaaggcggaacagaactgcatttgaaga
agtggaagaagacctaaggctctcccttcacctccaggatcaggattctgtcaaagatgcagtgccagatcaacagttagattttaagcag
ctcctggcccgctataaaggggttagcaagtcagtcatcaaaacaatgtccatcatggattttgaaaaggtgaatcttgaattaatagaggc
cttattagagtggattgtggatggaaagcactcctaccctccaggtgctatacttgtatttttaccaggactagcagaaatcaaaatgctttatg
aacagctacagtctaattctcttttcaacaacagacgtagtaatcgatgtgttattcacccacttcattcatctttatccagtgaagagcagcag
gctgtgtttgtaaaacctcctgcaggagtaactaagattataatttccaccaacattgctgagacatccataaccatcgatgatgttgtctatgtt
atcgattctgggaaaatgaaagaaaagagatatgatgccagcaaagggatggaaagtctagaggacacctttgtatctcaagctaatgc
tctacaaaggaaaggccgagcaggccgtgttgcatctggggtctgcttccatgtattcactagccatcactacaatcaccagcttttaaaac
aacagctaccagaaatacaaagagtgccattggaacagctgtgtctaagaattaaaattttagagatgtttagtgctcataatctccagtctg
tgttctctcggctcattgaacctccacacaccgattctcttcgtgcctcaaaaatacgattacgagacttaggagcattaactccagatgaaa
gattgacccctcttgggtatcatttggcctctctgcccgtggatgtgagaattggcaaactaatgttgtttgggtctatcttccgctgtttggatcct
gctctcaccattgctgccagtttggcttttaagtcgccgtttgtatctccctgggataaaaaagaagaagctaaccagaaaaagctggaattt
gcattcgcaaacagtgattatctggcccttctacaagcgtataagggatggcagctaagtacaaaagaaggcgtgcgtgcaagttataatt
actgcagacaaaacttcttgtctggaagagttctgcaggaaatggccagcctcaaacgacaattcacggaactgttgtcggatatagggtt
tgcaagggaagggctcagagcaagggaaattgagaaaagggcccaaggaggagatggtgtcttagatgccacaggagaagaggc
aaactcaaatgctgagaaccccaagctgatatcagcaatgctgtgtgctgctttgtatccaaatgtagtgcaggtgaaaagcccagaagg
aaaatttcagaagaccagtactggagctgtcagaatgcaaccaaaatcagctgagttgaagtttgtcaccaagaacgatggatatgtaca
cattcacccttcatcagtgaactatcaggtgagacactttgacagcccctacctgttgtaccacgagaagataaaaactagtcgagtattca
tccgagactgcagcatggtgtctgtgtacccgctggtcttgtttggaggaggccaagtgaatgtgcagcttcaaagaggagagttcgttgtct
ccctggatgatggttggatccgttttgtagctgcttcccatcaggtggctgaactggtaaaggagcttcgttgtgaacttgatcagcttctccag
gataaaattaaaaacccaagcattgatctgtgtacgtgtcctcgaggatcccggatcatcagcacaattgtgaaacttgtcaccacacaat
aa (SEQ ID NO:383)

**Q8NG17; AAM73547**
MKQASRQFQSILQERQSLPAWEERETILNLLRKHQVVVISGMTGCGKTTQIPQFILDDSLSGPPEK
VANIICTQPRRISAISVAERVAKERAERVGLTVGYQIRLESVKSSATRLLYCTTGVLLRRLEGDTALQ
GVSHIIVDEVHERTEESDFLLLVLKDIVSQRPGLQVILMSATLNAELFSDYFNSCPVITIPGRTFPVD
QFFLEDAIAVTRYVLQDGSPYMRSMKQISKEKLKARRNRTAFEEVEEDLRLSLHLQDQDSVKDAV
PDQQLDFKQLLARYKGVSKSVIKTMSIMDFEKVNLELIEALLEWIVDGKHSYPPGAILVFLPGLAEIK
MLYEQLQSNSLFNNRRSNRCVIHPLHSSLSSEEQQAVFVKPPAGVTKIIISTNIAETSITIDDVVYVID
SGKMKEKRYDASKGMESLEDTFVSQANALQRKGRAGRVASGVCFHVFTSHHYNHQLLKQQLPEI
QRVPLEQLCLRIKILEMFSAHNLQSVFSRLIEPPHTDSLRASKIRLRDLGALTPDERLTPLGYHLASL
PVDVRIGKLMLFGSIFRCLDPALTIAASLAFKSPFVSPWDKKEEANQKKLEFAFANSDYLALLQAYK
GWQLSTKEGVRASYNYCRQNFLSGRVLQEMASLKRQFTELLSDIGFAREGLRAREIEKRAQGGD
GVLDATGEEANSNAENPKLISAMLCAALYPNVVQVKSPEGKFQKTSTGAVRMQPKSAELKFVTKN
DGYVHIHPSSVNYQVRHFDSPYLLYHEKIKTSRVFIRDCSMVSVYPLVLFGGGQVNVQLQRGEFV
VSLDDGWIRFVAASHQVAELVKELRCELDQLLQDKIKNPSIDLCTCPRGSRIISTIVKLVTTQ (SEQ
ID NO:384)

# Figure 169

**Nucleotide:** X78678
**Sequence:**
atggaagagaagcagatcctgtgcgtggggctagtggtgctggacgtcatcagcctggtggacaagtaccctaaggaggactcggaga
taaggtgtttgtcccagagatggcagcgcggaggcaacgcgtccaactcctgcaccgttctctccctgctcggagccccctgtgccttcatg
ggctcaatggctcctggccatgttgctgattttgtcctggatgacctccgccgctattctgtggacctacgctacacagtctttcagaccacag
gctccgtccccatcgccacggtcatcatcaacgaggccagtggtagccgcaccatcctatactatgacaggagcctgccagatgtgtctg
ctacagactttgagaaggttgatctgacccagttcaagtggatccacattgagggccggaacgcatcggagcaggtgaagatgctgcag
cggatagacgcacacaacaccaggcagcctccagagcagaagatccgggtgtccgtggaggtggagaagccacgagaggagctct
tccagctgtttggctacggagacgtggtgtttgtcagcaaagatgtggccaagcacttggggttccagtcagcagaggaagccttgaggg
gcttgtatggtcgtgtgaggaaaggggctgtgcttgtctgtgcctgggctgaggagggcgccgacgccctgggccctgatggcaaattgct
ccactcggatgctttcccgccaccccgcgtggtggatacactgggagctggagacaccttcaatgcctccgtcatcttcagcctctcccagg
ggaggagcgtgcaggaagcactgagattcgggtgccaggtggccggcaagaagtgtggcctgcagggctttgatggcatcgtgtga
(SEQ ID NO:385)

**P50053**
MEEKQILCVGLVVLDVISLVDKYPKEDSEIRCLSQRWQRGGNASNSCTVLSLLGAPCAFMGSMAP
GHVADFVLDDLRRYSVDLRYTVFQTTGSVPIATVIINEASGSRTILYYDRSLPDVSATDFEKVDLTQ
FKWIHIEGRNASEQVKMLQRIDAHNTRQPPEQKIRVSVEVEKPREELFQLFGYGDVVFVSKDVAK
HLGFQSAEEALRGLYGRVRKGAVLVCAWAEEGADALGPDGKLLHSDAFPPPRVVDTLGAGDTFN
ASVIFSLSQGRSVQEALRFGCQVAGKKCGLQGFDGIV (SEQ ID NO:386)

# Figure 170

**Nucleotide:** X92720
**Sequence:**
atggccgcattgtaccgccctggcctgcggcttaactggcatgggctgagccccttgggctggccatcatgccgtagcatccagaccctgc
gagtgcttagtggagatctgggccagcttcccactggcattcgagattttgtagagcacagtgcccgcctgtgccaaccagagggcatcca
catctgtgatggaactgaggctgagaatactgccacactgaccctgctggagcagcagggcctcatccgaaagctccccaagtacaata
actgctggctggcccgcacagaccccaaggatgtggcacgagtagagagcaagacggtgattgtaactccttctcagcgggacacggt
accactcccgcctggtggggcctgtgggcagctgggcaactggatgtccccagctgatttccagcgagctgtggatgagaggtttccagg
ctgcatgcagggccgcaccatgtatgtgcttccattcagcatgggtcctgtgggctccccgctgtcccgcatcggggtgcagctcactgact
cagcctatgtggtggcaagcatgcgtattatgacccgactggggacacctgtgcttcaggccctgggagatggtgactttgtcaagtgtctg
cactccgtgggccagcccctgacaggacaaggggagccagtgagccagtggccgtgcaacccagagaaaaccctgattggccacgt
gcccgaccagcgggagatcatctccttcggcagcggctatggtggcaactccctgctgggcaagaagtgctttgccctacgcatcgcctct
cggctggcccgggatgagggctggctggcagagcacatgctgatcctgggcatcaccagccctgcagggaagaaggcgctatgtgca
gccgccttccctagtgcctgtggcaagaccaacctggctatgatgcggcctgcactgccaggctggaaagtggagtgtgtgggggatgat
attgcttggatgaggtttgacagtgaaggtcgactccgggccatcaaccctgagaacggcttctttggggttgcccctggtacctctgccacc
accaatcccaacgccatggctacaatccagagtaacactatttttaccaatgtggctgagaccagtgatggtggcgtgtactgggagggc
attgaccagcctcttccacctggtgttactgtgacctcctggctgggcaaaccctggaaacctggtgacaaggagccctgtgcacatccca
actctcgattttgtgccccggctcgccagtgccccatcatggacccagcctgggaggccccagagggtgtccccattgacgccatcatcttt
ggtggccgcagacccaaaggggtacccctggtatacgaggccttcaactggcgtcatggggtgtttgtgggcagagccatgcgctctga
gtccactgctgcagcagaacacaaagggaagatcatcatgcacgacccatttgccatgcggcccttttttggctacaacttcggccactac
ctggaacactggctgagcatggaagggcgcaaggggggcccagctgccccgtatcttccatgtcaactggttccggcgtgacgaggcag
ggcacttcctgtggccaggctttggggagaatgctcgggtgctagactggatctgccggcggttagagggggaggacagtgcccgaga
gacacccattgggctggtgccaaaggaaggagccttggatctcagcggcctcagagctatagacaccactcagctgttctccctccccaa
ggacttctgggaacaggaggttcgtgacattcggagctacctgacagagcaggtcaaccaggatctgcccaaagaggtgttggctgagc
ttgaggccctggagagacgtgtgcacaaaatgtga (SEQ ID NO:387)

**Q16822**
MAALYRPGLRLNWHGLSPLGWPSCRSIQTLRVLSGDLGQLPTGIRDFVEHSARLCQPEGIHICDG
TEAENTATLTLLEQQGLIRKLPKYNNCWLARTDPKDVARVESKTVIVTPSQRDTVPLPPGGARGQ
LGNWMSPADFQRAVDERFPGCMQGRTMYVLPFSMGPVGSPLSRIGVQLTDSAYVVASMRIMTR
LGTPVLQALGDGDFVKCLHSVGQPLTGQGEPVSQWPCNPEKTLIGHVPDQREIISFGSGYGGNS

LLGKKCFALRIASRLARDEGWLAEHMLILGITSPAGKKALCAAAFPSACGKTNLAMMRPALPGWK
VECVGDDIAWMRFDSEGRLRAINPENGFFGVAPGTSATTNPNAMATIQSNTIFTNVAETSDGGVY
WEGIDQPLPPGVTVTSWLGKPWKPGDKEPCAHPNSRFCAPARQCPIMDPAWEAPEGVPIDAIIF
GGRRPKGVPLVYEAFNWRHGVFVGRAMRSESTAAAEHKGKIIMHDPFAMRPFFGYNFGHYLEH
WLSMEGRKGAQLPRIFHVNWFRRDEAGHFLWPGFGENARVLDWICRRLEGEDSARETPIGLVPK
EGALDLSGLRAIDTTQLFSLPKDFWEQEVRDIRSYLTEQVNQDLPKEVLAELEALERRVHKM
(SEQ ID NO:388)

# Figure 171

**Nucleotide:** M15856
**Sequence:**
atggagagcaaagccctgctcgtgctgactctggccgtgtggctccagagtctgaccgcctcccgcggaggggtggccgccgccgacc
aaagaagagattttatcgacatcgaaagtaaatttgccctaaggacccctgaagacacagctgaggacacttgccacctcattcccgga
gtagcagagtccgtggctacctgtcatttcaatcacagcagcaaaaccttcatggtgatccatggctggacggtaacaggaatgtatgaga
gttgggtgccaaaacttgtggccgccctgtacaagagagaaccagactccaatgtcattgtggtggactggctgtcacgggctcaggagc
attacccagtgtccgcgggctacaccaaactggtgggacaggatgtggcccggtttatcaactggatggaggaggagtttaactaccctct
ggacaatgtccatctcttgggatacagccttggagcccatgctgctggcattgcaggaagtctgaccaataagaaagtcaacagaattact
ggcctcgatccagctggacctaactttgagtatgcagaagccccgagtcgtctttctcctgatgatgcagattttgtagacgtcttacacacatt
caccagagggtcccctggtcgaagcattggaatccagaaaccagttgggcatgttgacatttacccgaatggaggtacttttcagccagg
atgtaacattggagaagctatccgcgtgattgcagagagaggacttggagatgtggaccagctagtgaagtgctcccacgagcgctcca
ttcatctcttcatcgactctctgttgaatgaagaaaatccaagtaaggcctacaggtgcagttccaaggaagcctttgagaaagggctctgct
tgagttgtagaaagaaccgctgcaacaatctgggctatgagatcaataaagtcagagccaaaagaagcagcaaaatgtacctgaaga
ctcgttctcagatgccctacaaagtcttccattaccaagtaaagattcatttttctgggactgagagtgaaacccataccaatcaggcctttga
gatttctctgtatggcaccgtggccgagagtgagaacatcccattcactctgcctgaagtttccacaaataagacctactccttcctaatttac
acagaggtagatattggagaactactcatgttgaagctcaaatggaagagtgattcatactttagctggtcagactggtggagcagtcccg
gcttcgccattcagaagatcagagtaaaagcaggagagactcagaaaaaggtgatcttctgttctagggagaaagtgtctcatttgcaga
aggaaaggcacctgcggtatttgtgaaatgccatgacaagtctctgaataagaagtcaggctga (SEQ ID NO:389)

**P06858**
MESKALLVLTLAVWLQSLTASRGGVAAADQRRDFIDIESKFALRTPEDTAEDTCHLIPGVAESVAT
CHFNHSSKTFMVIHGWTVTGMYESWVPKLVAALYKREPDSNVIVVDWLSRAQEHYPVSAGYTKL
VGQDVARFINWMEEEFNYPLDNVHLLGYSLGAHAAGIAGSLTNKKVNRITGLDPAGPNFEYAEAP
SRLSPDDADFVDVLHTFTRGSPGRSIGIQKPVGHVDIYPNGGTFQPGCNIGEAIRVIAERGLGDVD
QLVKCSHERSIHLFIDSLLNEENPSKAYRCSSKEAFEKGLCLSCRKNRCNNLGYEINKVRAKRSSK
MYLKTRSQMPYKVFHYQVKIHFSGTESETHTNQAFEISLYGTVAESENIPFTLPEVSTNKTYSFLIY
TEVDIGELLMLKLKWKSDSYFSWSDWWSSPGFAIQKIRVKAGETQKKVIFCSREKVSHLQKGKAP
AVFVKCHDKSLNKKSG (SEQ ID NO:390)

# Figure 172

**Nucleotide:** AF184174
**Sequence:**
atggacatggcggatgagccactcaatggaagccacacatggctatccattccatttgacctcaatggctctgtggtgtcaaccaacacct
caaaccagacagagccgtactatgacctgacaagcaatgcagtcctcacattcatctattttgtggtctgcatcattgggttgtgtggcaaca
cacttgtcatttatgtcatcctccgctatgccaagatgaagaccatcaccaacatttacatcctcaacctggccatcgcagatgagctcttcat
gctgggtctgcctttcttggctatgcaggtggctctggtccactggccctttggcaaggccatttgccgggtggtcatgactgtggatggcatc
aatcagttcaccagcatcttctgcctgacagtcatgagcatcgaccgatacctggctgtggtccaccccatcaagtcggccaagtggagg
agaccccggacggccaagatgatcaccatggctgtgtggggagtctctctgctggtcatcttgcccatcatgatatatgctgggctccggag
caaccagtgggggagaagcagctgcaccatcaactggccaggtgaatctggggcttggtacacagggttcatcatctacactttcattctg
gggttcctggtacccctcaccatcatctgtctttgctacctgttcattatcatcaaggtgaagtcctctggaatccgagtgggctcctctaagag
gaagaagtctgagaagaaggtcacccgaatggtgtccatcgtggtggctgtcttcatcttctgctggcttcccttctacatattcaacgtttcttc
cgtctccatggccatcagccccaccccagcccttaaaggcatgtttgactttgtggtggtcctcacctatgctaacagctgtgccaaccctatc
ctatatgccttcttgtctgacaacttcaagaagagcttccagaatgtcctctgcttggtcaaggtagacaattcaaagtctggagaagaggg
atcatgcctggatatgatctttagaaacaacaaaaatagaaaaaaataa (SEQ ID NO:391)

**Q96TF2**
MDMADEPLNGSHTWLSIPFDLNGSVVSTNTSNQTEPYYDLTSNAVLTFIYFVVCIIGLCGNTLVIYVI
LRYAKMKTITNIYILNLAIADELFMLGLPFLAMQVALVHWPFGKAICRVVMTVDGINQFTSIFCLTVM
SIDRYLAVVHPIKSAKWRRPRTAKMITMAVWGVSLLVILPIMIYAGLRSNQWGRSSCTINWPGESG
AWYTGFIIYTFILGFLVPLTIICLCYLFIIIKVKSSGIRVGSSKRKKSEKKVTRMVSIVVAVFIFCWLPFYI
FNVSSVSMAISPTPALKGMFDFVVVLTYANSCANPILYAFLSDNFKKSFQNVLCLVKVDNSKSGEE
GSCLDMIFRNNKNRKK (SEQ ID NO:392)

# Figure 173

**Nucleotide:** AJ277892
**Sequence:**
atgacaactcaagcaccgacgtttacgcagccgttacaaagcgttgtggtactggagggtagtaccgcaacctttgaggctcacattagtg
gttttccagttcctgaggtgagctggtttagggatggccaggtgatttccacttccactctgcccggcgtgcagatctcctttagcgatggccgc
gctaaactgacgatccccgccgtgactaaagccaacagtggacgatattccctgaaagccaccaatggatctggacaagcgactagta
ctgctgagcttctcgtgaaagctgagacagcaccacccaacttcgttcaacgactgcagagcatgaccgtgagacaaggaagccaagt
gagactccaagtgagagtgactggaatccctacacctgtggtgaagttctaccgggatggagccgaaatccagagctcccttgatttcca
aatttcacaagaaggcgacctctacagcttactgattgcagaagcataccctgaggactcagggacctattcagtaaatgccaccaatag
cgttggaagagctacttcgactgctgaattactggttcaaggtgaagaagaagtacctgctaaaaagacaaagacaattgtttcgactgct
cagatctcagaatcaagacaaacccgaattgaaaagaagattgaagcccactttgatgccagatcaattgcaacagttgagatggtcat
agatggtgccgctgggcaacagctgccacataaaacacctcacaggattcctccgaagccaaagtcaagatccccaacaccaccgtct
attgctgccaaagcacagctggctcggcagcagtccccatcgcccataagacactccccttccccggtcagacacgtgcgggcaccga
ccccatctccggtcaggtccgtgtctccagcagcaagaatctccacatcccccatcaggtctgttaggtctccattgctcatgcgtaagactc
aggcatccaccgtggccacaggtcctgaagtgcctccccccttggaagcaagagggctacgtggcctcctcatctgaggctgagatgaga
gagacaacgctgacaacctctactcagatcaggacagaagagagatgggaagggagatacggtgtccaggagcaagtgaccatca
gtggtgctgcgggtgctgccgccagtgtgtcggccagtgctagctacgcagcagaggctgttgccactggtgctaaagaggtgaaacaa
gatgctgacaaaagtgcagctgttgcgactgttgttgctgccgttgatatggccagagtgagagaaccagtgatcagcgctgtagagcag
actgctcagaggacaaccacgactgctgtgcacatccaacctgctcaagaacaggtaagaaaggaagcggagaagactgctgtaact
aaggtagtagtggccgccgataaagccaaggaacaagaattaaaatcaagaaccaaagaagtaattaccacaaagcaagagcaga
tgcacgtaactcatgagcagataagaaaagaaactgaaaaaacatttgtaccaaaggtagtaatttccgcagctaaagccaaagaaca
agaaactagaatttctgaagaaattactaagaaacagaaacaagtaactcaagaagcaataagacaggaaactgagataactgctgc
atccatggtggtagttgccactgcaaagtccacaaaactagaaacagtcccgggagctcaagaagaaactaccacacaacaagatca
aatgcacctaagttatgaaaagataatgaaggaaactaggaaaacagttgtacctaaagtcatagttgccacacccaaagtcaaagaa
caagatttagtatcaagaggtagagaaggcattactaccaaaagagaacaagtgcaaataactcaggagaagatgagaaaggaagc
cgagaaaactgccttgtctacaatagcagttgctactgctaaagccaaagaacaagaaacaatactgagaactagagaaactatggct
actagacaagaacaaatccaagttacccatggaaaggtggacgttggaaaaaaggctgaagctgtagcaacagttgttgctgcagtag
accaggcccgagtcagagagcccagagagcctgggcatcttgaagaatcctatgctcagcagaccactttggagtacggatataagga
acgcatttccgccgcaaaggtagctgagcctccccaacgtccagcctcagaaccccacgttgtccctaaagcagtcaagcctagagtaa
tccaggctccttctgagactcatatcaaaactactgatcaaaagggaatgcacatatcatcacagatcaagaaaactacagatctaacaa
cggaaagattagtccatgtggataaacgcccccgcacagctagccctcactttactgtttcaaaaatttctgttcctaagacagaacatgga
tatgaggcatcaatagccggtagtgctattgccacattacaaaaagagttgtcagccacatcttctgctcagaagatcaccaaatcggtga
aggctcctactgtgaagcccagtgagactagagtaagggcagagcccacacccttgccacagttccccttcgctgacacaccagatactt
acaagagtgaagctggcgttgaggtgaaaaaggaagtaggggtgagcatcactggcaccaccgtccgtgaagagcgctttgaagtact
gcacggacgcgaagccaaggtaacagaaacagcaagagtaccagcacctgttgaaattcctgttactccaccaactttggtctcgggctt
aaaaaatgtgactgtcatagaaggtgaatctgtcaccttggagtgccacatctctggatacccatccccgacagtgacatggtacaggga
agactaccaaatcgaaagttccattgacttccagataaccttccagagtggaattgctcgtcttatgattcgcgaagcatttgcggaagaca
gcgggcgatttacttgcagtgctgtaaatgaggctggaaccgtcagcacatcctgctacctggctgtgcaggtgtcagaagaatttgaaaa
ggaaaccacagccgtgactgagaaatttactacagaagagagaaacgctttgttgagtcaagagatgtggttatgactgatactagcctcac
agaggaacaagcagggcctggagaacctgccgcgccttactttattacaaaaccagtggtccagaaactggtggaaggtgggagcgt
ggtgtttggatgccaagttggcggcaacccaaagccccatgtatactggaaaaaatctggtgttcctctaaccactggatacagatacaaa
gtgagttacaacaaacaaaccggtgaatgcaagctggtgatttctatgacttttgctgatgatgctggagaatacactattgttgttcgcaata
agcatggagaaacttctgcatctgcttccttgcttgaagaagctgattatgagttactgatgaagtcccagcaagaaatgctttatcagacac
aagtgactgcatttgttcaagaacctaaagttggagaaacagcacctggatttgtatactctgagtatgaaaaagagtatgaaaaagaac
aagccttaattaggaagaaaatggccaaagatactgtagtggtcagaacttatgtagaagatcaggaattccatatttcttcctttgaagag
aggcttattaaagaaattgaatatagaataataaagactacattagaagaacttcttgaagaagatggagaagaaaagatggcagttga
catttctgaatctgaagctgttgaatcaggatttgatttaagaatcaagaattatagaattcttgaggggatgggtgtcacttttcattgcaagat
gtctggatatccattaccaaagattgcttggtacaaagatggcaagcgcatcaaacatggagaaagataccaaatggactttctacaaga
tggcagagctagtctgcgtatacctgttgttcttccagaagatgaaggaatctacactgcatttgccagcaatattaaaggaaatgcaatttg
ctcagggaaattgtatgtggagcctgctgcaccacttggagctccgacttacattcccacactagagccagtgagcagaatcagatctctct
ctccacgttcagtgagcaggtctcctatacgcatgtctcctgcacggatgtcacctgcaaggatgtctcctgcacggatgtcccctgcaaga
atgtcccctggacgtaggctggaggagacagatgagtcacaacttgagagactatataaaccagtctttgtgttaaaacctgtttctttcaaa
tgtttagaagggcaaactgccagatttgacttaaaggttgttggtagacctatgccagagacgttctggtttcatgatggccagcaaattgtca
atgactatacccataaagtagtcattaaagaagatggtactcaatcactaattattgtccctgccacacccagtgattctggggaatggactg
tggttgcccaaaacagggcaggcagatcttcaatttcagtgattttaactgtggaagctgtggaacatcaggtaaaaccgatgtttgtagaa

aaactgaaaaatgtcaatataaaggaaggttcccaacttgaaatgaaagtcagagctacgggtaaccccaaccctgacattgtatggttg
aaaaacagtgacatcattgtgcctcataaatatcccaaaatcagaattgaaggaaccaagggagaagctgcccttaaaatcgattccact
gtcagccaagattctgcctggtatactgcgactgctattaataaagctggcagagacactacaagatgcaaagtaaatgttgaagttgagtt
tgcagagcctgagccagagagaaagttaatcatcccacgggggacatatagagcaaaggagattgcagccccagaactggagcccc
tccatttgcgatatggccaagagcaatgggaagaaggtgatctctatgacaaagagaaacaacagaaaccatttttcaagaaaaaactc
acttccttaagacttaagcgctttgggcctgcccactttgaatgcaggctaacacccattggtgacccaacgatggtggtggagtggctccat
gatggaaagccacttgaagcagccaacaggctccgtatgatcaatgaatttgggtactgcagccttgattatggcgttgcatattctagaga
cagtggtatcattacttgcagagccactaacaaatatggaacagatcacacatctgctacccttattgttaaagatgagaaaagtcttgtgg
aagaatcccaattgcctgaggggaggaaaggcttacagagaattgaagaattagagagaatggctcatgaaggtgcacttacaggtgt
aacaacagatcagaaagaaaagcaaaagccagacattgtcttgtacccagagccagttagagtacttgaaggggagactgcaaggtt
ccgctgcagggtaacaggctaccctcagcccaaagtcaactggtacctcaatggacagctcatccgcaaaagcaaaaggttcagagtt
cgctatgatggtatccattacctggacatcgtggactgcaaatcatatgacacaggtgaagtgaaggtcaccgcggaaaatcctgaaggt
gtgatagagcataaagtgaagcttgagattcaacagagggaagattttaggtctgtccttaggagagctcctgaaccaaggcctgagtttc
acgtacatgaaccaggaaagcttcagtttgaagtacaaaaagtggatagacctgttgacaccactgaaaccaaagaagttgtgaagttg
aaaagggctgaaagaattacccatgaaaaagtgcctgaagagtcggaagagctgcgcagtaaattcaagcgcagaacagaagagg
gctattatgaagccattaccgctgtggagctcaagtctcgaaagaaggatgaatcctatgaggaactcctcaggaagacaaaagatgaa
cttctccactggaccaaagagttaactgaagaggaaaagaaagctcttgccgaagaaggcaaaatcacgattccaacttttaaacctga
caagattgaactaagtcctagtatggaggctccaaaaatcttcgaaagaatccagagccaaacagtgggccaaggatctgatgcacact
tccgggtcagagtcgtggggaaaccagaccccgaatgtgaatggtacaaaaatggtgtcaaaattgaacggtctgaccggatctactgg
tactggcccgaagacaatgtttgtgaattggtcataagagatgtgactgctgaggactctgccagcatcatggtaaaagccatcaacatag
ctggagaaacctccagtcacgcattcttacttgtccaagccaagcaattgatcactttcacacaggaattacaagatgttgttgctaaggaa
aaagacactatggcaacctttgaatgtgaaacttcagaaccatttgtcaaagtgaaatggtataaagatggtatggaggttcatgagggag
ataaatacaggatgcactctgacagaaaggttcacttcctctccatactgaccattgatacgtctgatgctgaagattacagctgtgtacttgt
ggaagatgaaaatgtcaaaacgactgctaaacttattgttgaaggtgcagttgttgagtttgtgaaagaacttcaggacatagaagttccag
aatcatattcaggagaattagagtgcattgtatccccagaaaatatagaaggaaaatggtatcataatgatgtggagcttaaatccaatgg
caaatatacaattacatctcgtcgtggacgtcagaacctcacggtcaaggatgtaaccaaggaggaccagggagaatacagctttgtcat
cgacgggaaaaagacaacctgtaaattaaagatgaaaccccgccccattgctatcctacaaggacttagtgaccaaaaagtctgtgag
ggtgacattgttcagcttgaagttaaagtctccttggaaagtgtggaaggcgtctggatgaaagacggccaagaagtgcagcccagtgac
agggttcacattgtgatagacaaacaatctcatatgctgctcattgaagacatgactaaggaagatgctggaaattactctttcaccattcca
gcccttggcctctccaccagtgggcgtgtctctgtctatagtgtggacgtgataacacctctaaaagatgttaatgtgattgaaggcaccaag
gctgtgcttgaatgtaaggtgtcagtccctgatgtgacttctgttaagtggtacttaaatgatgaacaaatcaagcctgatgaccgtgtacagg
ccattgtgaaaggtactaaacagcgactagtcattaaccgaactcatgcttcagacgaaggaccttacaagctgatagttggcagagttg
aaaccaactgtaatctctctgtagaaaaaattaaaattatcagaggtcttcgtgaccttacctgtacagaaactcaaaatgtggtgtttgaggt
tgagctgtcccactctggaattgatgtcctgtggaattttaaggacaaggaaatcaagcccagttctaaatataaaattgaagcacatggaa
aaatatataaattgacagttctaaatatgatgaaagatgatgaaggaaaatacacattttacgcgggagaaaatatcacatctggaaaact
tactgtggcaggtggggccatctccaagccactcacagatcagaccgtagctgaatcccaggaagctgtgtttgaatgtgaagttgccaa
cccagattccaaaggcgaatggttgagggatggcaaacacctaccactgactaacaacatcagaagtgagtctgatggccacaaaag
gagacttatcattgctgccaccaaattagatgacattggagaatatacctacaaggtggccacctccaaaacatctgccaaactcaaagtt
gaagctgtcaaaattaagaagactctgaagaacctcacagtgacagaaacacaggatgctgttttcactgtcgagcttacacaccctaat
gtcaaaggtgtccagtggatcaaaaatggagttgtgctggaatccaatgaaaagtatgctatctctgtcaaaggaacaatttactctctgag
gattaaaaactgtgccatcgtggatgagtctgtttatggcttcaggcttggaaggcttggagccagtgccagactgcacgtggagactgtca
agatcattaaaaagccaaaggatgtgacagccttggaaaatgccactgttgcctttgaagttagtgtttcccatgacactgttccagtaaaat
ggttccataagaatgtggaaattaagccaagtgacaaacacagactggtctcagaaaggaaagtccacaagctgatgctgcagaacat
ctccccctcagatgctggggaatacacagctgtggtcgggcaattggaatgcaaagcaaaactgtttgtggagacattacatattacaaa
aaccatgaaaaatatcgaggtgcctgagaccaaaactgcctcttttgagtgtgaggtgtcccacttcaatgtcccttccatgtggctgaaga
atggtgtggaaattgagatgagtgaaaagttcaagatagttgtgcagggaaaactccatcagctgatcatcatgaacaccagcacagag
gactcggcagaatacacatttgtctgtggcaatgaccaagtcagtgccaccctgacagtcaccccaatcatgattacttccatgctgaaag
acatcaacgctgaagaaaaagacactattacttttgaggtgacagtgaactatgaaggcatctcttacaaatggttaaagaatggtgtgga
aatcaaatcaactgacaagtgccagatgagaaccaaaaagctcacacactcactgaacatcaggaatgttcactttggggatgctgctg
actacacctttgtggctggaaaagcaacatcaacagccacactttatgtggaagctcgtcatatagaatttaggaaacacattaaggacat
taaggtactggagaagaagcgagccatgtttgaatgtgaagtttctgaacctgacatcactgtacagtggatgaaagatgaccaggaact
gcagatcacagacagaataaagattcagaaggagaaatatgtccaccgccttctgatcccatccacccggatgtctgatgctgggaagt
acacagtggtggcaggaggcaacgtgtcaactgcaaaactctttgtagaaggcagagatgttcgcatccgaagtattaaaaaggaggtt
caggtcattgagaaacagcgtgctgttgttgaatttgaggtcaatgaagacgatgttgatgcccactggtataaagatggcattgaaatcaa
tttccaagttcaagaacgacacaaatatgtagtggagagaagaatccaccgaatgtttatctctgagaccagacagagcgatgcaggag
aatacacctttgtggcaggaaggaacaggagttctgtcactctctatgtcaatgctcctgaaccgccccaagttctgcaggagctccagcct
gtcactgtgcagtctggcaagcctgcccgcttctgtgccgtgatatccggaagaccacagcccaaaatttcctggtacaaggaagagcag

ctgctttccactggcttcaagtgcaaatttcttcatgatgggcaagagtacacgcttttgctaattgaagccttcccagaggatgcggcagtct
atacctgtgaagccaagaatgactatggtgttgccacaacatcagcttcactctcagtggaagttccagaagttgtgtctcctgatcaggaa
atgcctgtttatccacctgccatcatcaccccgcttcaggacactgtcacttctgaagggcagccagcccgttttcaatgccgggtttctggaa
cagatctaaaagtgtcgtggtacagcaaagacaagaaaatcaagccatctcggttctttagaatgactcaatttgaagacacttatcaact
ggaaattgccgaagcttatccagaagatgaaggaacttacacgtttgttgctagtaatgctgtaggccaagtatcaagcacagccaacct
gagtctggaagctcctgaatcaattttgcatgagaggattgaacaagagattgagatggaaatgaaagagttttctagttcttttctgtctgcc
gaggaagaaggacttcatagcgccgaacttcaattatctaaaataaatgaaacacttgaacttttgtctgaatctccagtttactcaactaaa
tttgattccgaaaaggaaggcactggcccaattttcatcaaagaagtgtcaaatgctgatataagcatgggggatgtggctacactgtctgt
aactgtcattggcatccccaaacctaaaattcagtggttctttaatggagtgctattaaccccttctgctgactacaaatttgtttttgacggtgat
gatcatagcctgatcattctgttcaccaaattggaggatgagggagagtatacatgtatggccagtaatgactatggaaagacaatatgta
gtgcctatctaaaaattaattccaaaggagagggtcacaaagacactgaaacagaatcagcagtggcaaaatctctggaaaagctggg
aggtccttgtcctcctcacttccttaaggagttaaaaccaattcgctgtgctcaagggcttcctgccatctttgagtacacagtggttggagagc
ctgcccctactgttacatggttcaaagaaaacaagcagctttgcaccagtgtttattacactatcattcataaccctaatggctctggaactttc
attgtcaatgaccctcagagggaagacagtggcctctatatctgtaaagcagagaatatgttgggtgagtccacctgtgcagcagagctg
cttgtgcttctggaagacacagacatgactgataccccctgcaaagcaaagtccacaccagaggctcctgaggattttccacagacacc
cttaaagggtcccgcagttgaagcacttgactcagagcaggaaattgcaacgtttgtaaaagacaccattttgaaagctgctttaatcaca
gaagaaaaccagcaactatcttatgagcatattgctaaagccaatgaattgagcagtcagcttcctttgggagctcaggaattgcaatcca
ttttggagcaagacaagctcactcctgaaagcaccagggaatttctttgcatcaatggcagtattcactttcagcctctcaaggaaccatctc
ccaacctacagctgcagattgtacagtcccagaaaaccttctccaaagaaggtattctaatgcctgaagagcctgagacacaggcagttc
tatcagataccgagaaaatcttcccaagtgccatgtccatagaacaaattaattcattaacagttgagcctctgaaaactttattagctgaac
ctgaagggaattatccacagtcttcaatagaacctccaatgcattcttatctaacctctgtggctgaggaagtactttcaccaaaagaaaag
acagtatctgacaccaacagagagcaaagagtgactcttcaaaagcaagaggcacaaagtgctctcatcttgagtcagagcttagctga
gggacacgtggagagtctccagagtcctgatgtcatgatctctcaggtaaactatgagcccctagtcccttcagaacactcatgcacagaa
ggaggtaaaattttgatagaaagtgcaaatccactggaaaatgcagggcaagattctgcggtcagaattgaggaaggcaagtccttaag
atttccactagcacttgaagaaaagcaggtactgctcaaagaagagcattctgacaacgtggtgatgcccccagaccaaatcattgagtc
taaaagagagcccgtggcaataaagaaagtgcaggaggtacagggaagggaccttctttctaaggaaagcttgctttctggtattccaga
agagcagagattaaacctgaaaattcaaatctgccgggctttgcaagcagctgtggccagcgagcagccaggtcttttctctgagtggcta
agaaatattgaaaaggtggaggtcgaggctgtaaacatcacccaagagcccagacacatcatgtgcatgtaccttgttacttcggcaaa
gtctgtaacagaagaagtaaccatcattattgaagatgttgatcctcaaatggctaacctgaaaatggaacttagggatgctttgtgtgctatt
atatatgaggaaatagacatcctaacagctgagggtcctagaattcagcaaggagccaaaacaagtttgcaagaagaaatggattctttt
tcaggttcacagaaggttgaacccattactgaaccagaagttgaatctaaatatctgatctcacctgaagaggtcagttattttaacgtgcaa
agtagggttaaatatttggatgccacacctgtcactaaaggggttgcttcagctgttgtctctgacgaaaaacaagatgagagtctgaaacc
atcagaggaaaaagaggagtcttcctctgaaagtggtactgaggaggttgctacagtaaagatacaggaagctgagggtggcttcatca
aagaggatggccccatgatacatacacctttagtggacactgtttctgaggaaggtgatattgtacacctcacaacatccataacaaatgct
aaagaggtgaattggtattttgagaataaactggtgccttcagatgaaaagttcaagtgtttacaagatcaaaatacatatacgctagtcatc
gacaaagtaaataccgaagaccatcaaggagagtatgtctgtgaggccttgaatgacagcggaaaaacagcaacttcagccaaactc
actgtagtaaaaagagctgccccagtgatcaagaggaaaatcgaacccctggaagtagcactgggccacctagccaaattcacctgtg
agatccaaagtgctcccaatgtccggttccagtggtttaaagctggccgagaaatttatgagagtgacaagtgttctattcgatcttcaaagt
atatctccagccttgaaatcctgagaacccaggtggttgactgcggcgagtatacatgcaaagcttccaatgagtatggcagtgtcagctgt
acagccacactaactgtgacagaggcatatccaccaacctttctctccagacctaagtcccttacaacttttgtgggtaaggcagccaagtt
catctgcacagtgacagggactcctgtgatagaaaccatttggcagaaagatggtgctgcactctcaccttcacctaactggaagatttcc
gacgcagaaaacaaacacattttagaactctcaaaccttaccattcaagatagaggagtttattcttgtaaggcttctaacaagtttggagc
agacatctgccaagcagagttgatcatcattgataagccacatttcattaaagaattagagcctgtgcagtccgctatcaataagaaggtcc
accttgagtgccaagtagatgaagacagaaaagtcacagttacgtggagcaaagatgggcaaaaactcccccccagggaaagattata
agatctgttttgaagataaaatagcaacacttgagattcctttggccaaactgaaagattcaggaacctatgtctgtacagcttcaaatgagg
ctggaagcagctcctgctcagccacagtcactgtcagagagccaccatccttcgtgaagaaggtggatccctcttatttaatgctcccaggt
gaatcagcacgcctccattgcaagctgaaaggctcacctgtgatccaggttacttggtttaaaaataacaaagaactcagtgaaagtaac
acagtccgaatgtattttgtcaattctgaggctatacttgatattacggatgtaaaagttgaagacagtgggagttactcatgtgaagcagtga
atgacgtcggcagtgatagctgcagtactgaaatagttatcaaagaacctccttctttcatcaaaactcttgagcctgcagacatagtgaga
ggaacaaatgctctacttcagtgtgaagtctcaggcactggaccatttgaaattagctggttcaaagacaagaaacaaattcgaagtagta
aaaaatacagattgttttctcagaagtctcttgtgtgtctggagatcttttcgtttaatagtgcagatgttggtgaatatgaatgtgttgttgctaatg
aagtcggcaagtgtggctgcatggcaacccacttactcaaagaacctccaacctttgtaaagaaagtagatgatttgattgcactaggag
gacaaaccgttaccctgcaagctgctgtgagagggtcagagcccatttctgtcacatggatgaaaggtcaagaggtcatcagagaagac
ggaaaaatcaaaatgagcttttccaatggtgttgcagtcttgataatccctgatgttcagattagttttggaggcaaatacacgtgcctggctg
aaaatgaagctggaagccaaacatctgttggggaattaatagttaaagaacctgccaaaatcattgagcgagcagaactgatccaggtg
acagcaggagatcccgccacactggagtacacagtggcaggcacgccagaactgaagcccaaatggtacaaagatgggagaccct
tggtcgccagtaaaaaataccgaataagttttaaaaacaatgttgcccagctcaaattttattcagctgagctgcacgacagtggccaatac

acatttgagatttccaatgaagtgggcagcagctcctgtgagactacattcactgtattagatcgagacattgctccattttttaccaaaccctt
gcgcaacgtggatagtgttgttaatggtacctgcagactggactgcaaaattgcaggctccctccccatgagggtgtcctggtttaaggatg
gcaaagaaatagctgcttctgacagatacaggatagcatttgtggaaggcacagcctctttggagatcatcagagtagacatgaatgatg
cagggaatttcacttgtcgagccacaaattccgtgggaagcaaagacagcagtggagccctgattgtgcaagaaccacccagttttgtaa
ctaaacccggctcaaaggatgttctgcctggctcagcagtctgcctgaagagcactttccaaggatctactcctctcacaatcagatggttta
agggcaacaaagagctggtttctggtggaagctgctatattaccaaagaagctttagagagttccctggaactctatttagtaaaaacctct
gattcgggcacgtacacatgtaaagtcagcaatgtcgctggaggggtggaatgcagtgcaaacttgtttgtaaaagaacctgccacatttg
ttgaaaagttagagccatcacagctgttaaagaagggagatgccacccagctagcctgcaaagtaactggtacccctccaattaaaata
acatggtttgcaaatgatagagaaattaaggagagcagcaaacacagaatgtcttttgtggagtctactgcagttctaagactgacagatg
ttggcatcgaagacagtggtgaatatatgtgtgaggcccaaaatgaggctggcagtgaccactgcagtagcattgtaatagtcaaagagt
caccttattttaccaaggaatttaagccaattgaagtcttaaaggagtacgatgtcatgttgctggctgaggtggcaggcactcctcccttga
gatcacttggttcaaagataacacaatcctgcgaagtggtagaaagtataagactttcattcaggatcatctggttagcctgcagatcctca
agtttgtagctgcagatgctggcgaataccagtgtcgggtgaccaatgaggtgggcagcagcatctgcagtgccagggtgactttaagag
aacccccatccttcataaagaagatcgagagtaccagctccctccggggaggcacagctgccttccaggccactctgaagggctccttg
ccaattacggtgacttggctaaaagacagcgatgaaatcactgaagacgataatataagaatgacctttgagaacaatgtggccagtttgt
acctcagtggcattgaagtcaagcatgatgggaaatatgtctgtcaggccaaaaatgatgcaggaatacaaagatgttctgcattactctc
agtaaaagaacctgcaacaatcaccgaggaagctgtgtctatagatgtcacccaaggagacccagccactttgcaggttaaattttcagg
gactaaggagattacagccaaatggtttaaagatggccaagaactgaccctgggctcaaaatataaaatcagtgtcactgatacagtctc
aatactgaagattatttctacagaaaagaaagatagtggagaatatactttcgaggtccaaaatgatgttgggaggagcagctgcaaggc
tagaattaatgtattagatcttatcatacctccttcattcaccaaaaagctgaagaaaatggacagcattaaaggttctttcattgatttagaatg
tatagtggctgggtcacatcccataagcatccagtggttcaaagatgaccaagaaatatcagctagtgaaaagtacaaattctcttttcatg
acaatactgccttcttggaaatcagccagctggaaggtacagacagtgggacatacacttgttctgccacaaataaggcagggcacaac
caatgcagtgggcatctgacagtcaaagaacccccttactttgtggaaaagccacagtcacaagatgtcaatcccaacacaagggtac
agttaaaggctcttgtgggtggcactgcacccatgacaataaagtggtttaaagataacaaagagttacactcaggagcagcccgctca
gtttggaaggatgacacctctaccagtctagagctctttgcagccaaagctaccgattctggaacctacatttgccaactaagcaatgatgtt
ggcacagcaaccagcaaagccactctctttgtaaaagaacctcctcaattcattaagaagcccagtccagtcttagtgctgaggaatgga
cagtcaacaacatttgaatgccaaataacaggcactcctaaaatccgagtgtcttggtatctagacgggaatgaaataacagccattcag
aaacatggcatttccttcattgatggtttagccactttccagatttctggtgccagggtagaaaatagtgggacttatgtgtgtgaagctcgaaa
tgacgcaggcacggcgagctgcagcattgaactcaaagtgaaagaacccccccacctttatcagagagctgaagcctgtggaggtagta
aaatatagtgacgtggagctggagtgtgaagttacgggaacacctccgtttgaagtcacttggctgaagaataacagggaaattcgaag
cagcaaaaaatacacattgaccgacagagtgtctgtgtttaacctccatataaccaagtgtgacccttcagacactggggaataccagtg
cattgtatccaatgaaggcggcagctgctcatgcagtactagagttgccctgaaagaaccaccatcattcattaagaagatagaaaatac
cactactgttttgaaaagttctgccacctttcagagtaccgtggcaggttctcctcctatttctataacctggctaaaggatgatcagattcttgat
gaagatgataatgtctatatttcatttgtggacagtgtggccacacttcaaatcagaagtgtggataatggacacagtgggagatatacctgt
caagccaagaatgagtcaggagttgagaggtgttatgctttccttttagtacaagaaccagctcaaatcgtagagaaagctaaatcagttg
atgttacggagaaagatcccatgaccttggaatgtgttgtggctggaacaccagaactcaaagtgaaatggcttaaagacgggaaacaa
atagttcccagtagatactttcaatgagttttgaaaataacgtggccagttttagaattcagtcagtaatgaagcaggacagcggtcagtac
actttcaaggtggaaaatgacttcggaagcagtagctgtgatgcctacttaagagtgctagatcaaaatattcctccatcattcaccaaaaa
attaaccaaaatggataaagttctgggctcttctattcatatggagtgcaaggtgtctggttcacttcccattagtgctcagtggtttaaggatgg
aaaagaaatatctacaagtgcaaaatacagacttgtgtgccatgagagatctgtgtctctggaagttaataatctggaattagaagatactg
caaattacacatgcaaagtgtcaaatgtagcaggagatgatgcatgcagtggcatcttaactgtgaaagaaccaccaagcttcctagtga
aacctgggcgacagcaagccatacctgattctacagtggaatttaaggcaatactaaaggaacaccaccatttaaaataaaatggttta
aggatgatgtggaacttgtctcaggtcctaaatgtttcattggcttggaagggtcgactagcttcttaaatctctactcagtggatgcttctaaga
ctggacagtatacttgccatgttaccaatgatgttggtagcgactcttgtactacgatgttgcttgtgacagaaccaccaaagtttgtaaagaa
attagaagcctccaaaattgtgaaagcaggtgactcttcacgacttgaatgcaagatagctggatccccagaaatcagagttgtgtggttc
cgaaatgaacatgaacttccagccagtgataagtacagaatgactttcattgactcagtggccgtcatacagatgaacaatctcagtactg
aggacagtggagatttcatttgtgaggctcagaatcccgctggcagcacaagctgcagtaccaaggttatagtaaaagagccacctgtttt
tagcagcttccctcctatagtagaaacccttaaaaatgctgaagtcagtcttgaatgtgaactttcgggaacaccaccgtttgaggtggtatg
gtacaaagacaagcggcaactcagaagcagcaagaaatacaagattgcatccaaaaacttccacacaagtattcacattctcaatgtg
gacacttcagacatcggtgaataccactgcaaagcacagaatgaagtgggaagtgatacttgtgtttgtactgtaaaattgaaagaacca
ccaagatttgtctccaaactgaacagcctcactgttgtagccggagagcctgctgaattacaagcatccatagaaggcgcccagcctatttt
tgtccagtggcttaaagagaaggaagaagtgattagagaaagtgaaaacatcaggattacatttgtggaaaatgttgcaactctacagttt
gcaaaagcagagccagctaatgctggaaagtatatctgccaaatcaagaatgatggtggaatggaggagaacatggccacactgatg
gtcttagagcccgcagtcattgttgagaaggcaggaccgatgacggtgactgtaggagaaacgtgcactctggagtgtaaggtggctgg
cactccggaactctctgttgaatggtacaaggatggaaagctgttgaccagcagccaaaaacacaaatttagcttctacaacaaaatctct
tccttaaggattctctcagttgaaaggcaagatgcaggcacatacactttccaggttcaaaataatgttgggaaaagcagctgcacagctg
tggttgatgtttcagaccgagcagttcctccctctttcacacgaagactgaaaaatactggtggggtgttaggtgcttcttgcatcttggaatgc

aaagtagctggatcatcacctatttcagttgcctggtttcatgagaaaaccaagattgtcagtggagcaaagtaccaaaccacattttcaga
taatgtctgcacattgcagttgaattctctggattcctcagatatgggcaattacacatgcgtggctgctaatgtcgctgggtctgatgaatgtc
gtgcagtgctaactgtacaagaaccaccctcttttgtgaaagaacctgaacctttggaagtactaccaggcaaaaatgtaaccttcacaag
cgttattagaggaacccctccattcaaagtcaactggttcagaggtgccagagaactagtgaaaggagaccggtgcaacatctattttga
agacactgtggcagaactggaattatttaatattgacatatctcagagtggggaatacacctgtgtggtttctaacaacgctggccaagcat
cttgcactacccgtctctttgtgaaagaaccagctgcattttgaagagattaagtgatcattctgtagaaccagggaagtccataattctgga
gagcacctacactggaacacttccaatttctgtcacttggaaaaaggatggttttaacataactacctctgaaaaatgtaacatagtcacaa
cagagaaaacttgtatcctggaaattctgaatagcacaaaaagagatgcagggcagtattcctgcgagattgaaaatgaggcaggaag
ggatgtttgtggagctctggtatctacattagaaccgccttattttgttacggaactggaacctctggaggcagcagttggagattcggtttcttt
acaatgccaagttgctgggacaccagaaattacagtgtcttggtacaaaggagataccaaattacggccaactcctgaatacaggacct
actttacaaacaatgtggccacacttgtttttaataaagtgaacatcaatgacagtggagagtacacatgcaaagcagaaaatagtatag
gaactgcttcttctaagactgtgttcagaattcaagagcgccaactcccaccttcttttgcaagacaattaaaggacattgaacaaactgtgg
ggttacctgttacactcacttgtcgattaaatggctctgcacccatccaagtgtgctggtatagagatggagtacttttaagagacgatgaaa
atctacagacttcatttgtagataatgtggcaactttaaaaattttgcaaactgacttgagtcactctggccagtactcttgctcagcttccaacc
cacttggaacagcatcttctagtgctagactcacagcaagagaacccaagaaatctcccttctttgacatcaagcctgtatctatagatgtta
ttgctggagaaagtgctgattttgagtgtcatgttactggtgctcaaccgatgcgaatcacttggtcaaaagataacaaggagatccgtcctg
gaggaaactatacaatcacatgtgtgggaaacactcctcattgagaattcttaaagtaggcaaaggagactctggtcaatatacttgcca
agcaaccaatgatgttggcaaagacatgtgctcagctcagctcagtgtaaaagaacctccaaagtttgttaagaaattagaagcttcaaa
agttgcaaagcagggagaatccattcaactggaatgtaaaataagtggctccccagaaatcaaagtttcatggttccgaaatgacagcg
aacttcatgaaagttggaaatacaacatgtcattcattaattctgtggcattgcttacgatcaatgaagctagtgctgaagacagcggggact
acatttgtgaggctcataatggtgttggtgacgccagctgcagcacagcgttgacagtgaaagcacctcctgttttcacccagaagccttctc
cagtaggagctcttaaaggttctgatgtgattctccaatgtgaaatttcgggaactcccccatttgaagtagtatgggttaaagatcgaaagc
aagttagaaacagcaagaaatttaaaatcacttcaaaacattttgatacaagtcttcatatccttaatcttgaagcctccgatgtcggggaat
atcactgcaaagctactaatgaggtgggaagtgacacgtgttcttgctctgtcaagttcaaagaacctccacgattcgtgaaaaagctaag
tgacacctcaacccttattggggatgctgttgagttacgggccatagtggagggcttccagccaatttctgttgtctggctgaaagatagagg
tgaagtcatcagagagagtgaaaataccaggatttcattcattgataacattgcaaccctccagctggggagtccagaagcatctaattct
ggaaaatatatatgccaaatcaaaaacgatgctggaatgagagaatgctctgcagtcttgactgtactagaaccagcaagaatcataga
gaagcccgaacccatgactgtcactactggaaatccttttgcattagagtgtgtagtgactggaacaccagaactctcagccaagtggttc
aaagatggaagagaattgtctgcagacagcaaacatcacattacattcatcaataaagtggcttcccttaaaatcccctgtgccgaaatga
gtgacaaaggattatatagctttgaagtgaaaaacagtgttggcaaaagtaactgcactgtatccgtccatgtttctgatcggattgtgcctcc
ttccttcatccgcaagctgaaagacgtgaatgccatcctgggggcctcagttgttttggagtgccgagtctctggctcagccccgatttcagtt
ggctggtttcaggatggaaatgagattgttagtgggcccaaatgtcagtccagcttttcggaaaacgtctgtactttgaatctgagcttgttgga
gccctccgacacaggcatatacacgtgtgtggctgccaatgtagctggttccgatgagtgctcagcagtcctgactgtgcaagaaccacc
atcttttgaacaaacccctgattctgtggaagttttgcctggaatgagcctcacattcaccagtgtcatcagaggcacccctcctttcaaggtc
aagtggtttaaaggcagcagggaactggtgcctggggagtcatgcaacatctctctggaagattttgtcacagaactggagttgtttgaggt
gcagccattagaaagtggagactattcttgcctcgttacaaatgatgctggcagtgcttcctgtaccacacatctttttgtgaaagaaccagc
cacctttgtgaaaagattggctgatttcagtgttgagacaggaagccccatagttctcgaggccacatacactggcacacctccaatctcag
tgagctggataaaggacgagtatcttatttcacaatctgagagatgcagtattactatgacagaaaaatctaccatactggaaattcttgag
agcacaatagaggattatgcacagtacagctgcctgatagaaaatgaggctggtcaagatatctgtgaagctctggtgtctgtcttagaac
caccgtactttattgaacctctggaacatgtggaagcagtcattggagaacctgcaactttacagtgtaaagtggatggaactccagaaatt
agaatttcttggtataaagaacacacaaagctacgatcagctcctgcatataaaatgcaattcaaaaataacgttgcttccttagtaatcaa
caaagtggatcacagtgatgtgggagagtattcatgcaaggcagacaacagtgtgggggcagtcgcttcttcagctgtgcttgttatcaaa
gagcgcaaacttccacctttctttgcaagaaaactgaaagacgttcatgagactctaggcttcccagttgcatttgaatgccgcatcaatgg
ctcagaacctcttcaagtgtcttggtacaaggatggggttcttttgaaagatgatgctaatttgcagacatcttttgttcataatgtagcaactctt
cagattttacaaactgaccagagccacatagggcagtataattgctctgcttctaatcctcttgggactgcttcatccagtgccaagctcattct
ctcagagcatgaagtgcctcctttctttgatctaaagcctgtatcagtagatcttgctcttggagagaagtggtacttttaaatgtcatgtaactgg
gactgcaccaatcaaaatcacttgggccaaagataaccgagagattcgccctggaggcaactacaagatgactttggtagaaaatactg
ccactctgacagttctcaaagtaggcaaaggcgatgccgggcagtacacctgctatgcaagcaacatcgctggaaaagactcttgttctg
ctcatctgggtgtacaagaaccacccaggttcattaagaagctagaaccttcaagaattgtgaaacaggatgaattcacaaggtacgaat
gcaaaatcggtgggtctccagaaatcaaagttttatggtataaggacgagactgaaattcaagagagcagtaaattcagaatgtcattcgt
tgactcggtggctgtgctggaaatgcacaatctcagtgttgaagacagtggagactacacctgtgaggcccacaatgcagcaggcagtg
ccagcagcagcacatccttaaaagttaaagaaccacccattttccgcaaaaagcctcatcctatagagacactgaaaggagctgatgttc
accttgaatgtgagcttcagggcactcccccatttcacgtttcttggtataaagacaagagagaacttaggagcggcaagaagtacaaga
taatgtctgagaacttccttaccagtatccacatcctgaatgtcgatgctgcagacattggggaatatcagtgtaaagccacaaatgatgtg
ggaagcgacacttgcgttggttccatcgctctcaaagcaccaccaagatttgtgaagaagctcagtgacatatctactgtcgttggtaaaga
agttcagctgcagactaccattgaaggcgctgagcccatttcagtggtgtggttcaaagataagggagaaatcgttagagaaagtgacaa
catatggatttcttattcagaaaacattgcaaccttacagtttcaagagtggaaccagccaatgctggaaaatacacttgtcagatcaaaa

acgatgctgggatgcaagagtgcttcgccacgctatccgttctcgagcctgcaacaattgtagaaaagccagaatccataaaagttacca
cgggagacacctgtaccttggagtgtacagtagctggcacccctgaactcagtactaagtggtttaaggatggaaaagagctaacaagt
gacaacaaatacaaaataagcttcttcaacaaagtatctggccttaagatcatcaatgtagcaccgagtgacagtggggtatacagttttg
aggtgcagaaccctgttggcaaagacagctgcacagcttcattgcaggtttcagaccgaaccgttcctccttcattcacaagaaaattgaa
agagacaaatggtctatccggctcctcagttgtaatggagtgtaaagtctatgggtcacctccaatctctgtctcctggttccatgaaggaaat
gagatcagtagtggaaggaaataccagaccaccctgacagataatacttgtgctttaactgtgaacatgctggaagaatcagacagtggt
gactacacatgtatagctactaatatggctggttctgatgaatgtagtgctcctttgactgtgagagaaccaccatcttttgttcagaaacctga
tcccatggatgttttaactgggaccaatgtaactttcacaagtatcgtaaaaggaacacctcctttcagtgttagctggttcaaaggtagcagt
gaactagtaccaggtgacagatgcaacgtgtctttggaggattcagttgctgaactggagttgttcgatgtagatacatcacaaagtggag
aatatacttgcatagttagcaatgaagctggcaaggcttcttgtacaacacatctctacataaaagccccagccaaatttgtgaagaggctg
aatgattacagcatagagaaaggaaaacccctgatcctagagggtacattcactggaactcctcccatttcggttacctggaagaaaaat
ggcataaatgtaactccttctcagaggtgtaatataactacgactgaaaaatcggcaatcctggaaattccaagtagcacagtagaggat
gcaggacaatacaactgctacattgaaaatgcctctggaaaagattcctgttcagcacaaatactcatactagaaccaccgtattttgtcaa
gcagttggagccggttaaggtgtctgttggagattctgcctctctacaatgccagcttgctggaacccctgaaattggggtatcctggtataaa
ggagatacaaaattgagacccactacgacttacaaaatgcattttaggaataatgttgctacactggttttcaaccaggttgatattaatgata
gtggagaatatatctgcaaagctgaaaacagcgtgggagaagtttctgcatcaactttccttaccgttcaagagcaaaaacttccaccatc
attttctcgacaattgagagatgttcaagaaacagttggactgccagttgtttttgattgtgccataagtggatcagaacctatctccgtgtcttg
gtataaagacggcaagccattgaaagacagcccaaatgtacaaacatcattttagacaatacagccacactcaatatttttaaaactgac
cggagccttgcaggccagtattcctgcacagctacaaaccctataggctctgcttcttccagtgccaggctcattctcacagaggggaaga
acccacccttctttgacatccgtcttgcccctgtggatgctgtggtgggagaaagtgctgactttgagtgccacgtcacgggcacacaaccg
ataaaggtcagctgggccaaagacagcagagaaatacgaagtggcggaaagtaccagattagttatctggaaaacagcgcccacct
gacagtcctcaaagtagacaaaggagattctggacaatatacctgctatgctgtgaatgaagtgggaaaagactcttgcacagctcagct
gaatataaaagagcggctcatcccaccaagtttcactaaaagactctcagagacagtagaagaaacagaagggaattctttcaaacttg
agggacgtgtggctggttcccaacctataactgttgcctggtacaaaaataatatagagatacaaccaacttctaactgtgaaataacattc
aagaacaacacgttagtgctgcaagtcagaaaagcaggcatgaacgacgctggtttgtacacatgcaaagtgtccaatgatgcaggct
ctgctctgtgcacgtcttcaatcgtcatcaaagaacctaagaagccacctgtatttgatcagcaccttactccagtaacagtgagtgaagga
gaatacgtgcagctcagctgccatgtccagggatctgagccaattaggatccagtggttgaaggctggcagggaaataaagccttcaga
cagatgcagcttcagctttgctagtgggacagctgtactggaactcagagatgtggctaaagcagattcgggagattatgtgtgtaaagctt
caaatgtggctggaagtgacactaccaaatcaaaagtgaccattaaagacaaaccagctgtggccccagcaaccaagaaagctgcg
gtagatggaagactctttttgtgtcagaacctcagagtatcagagtcgtagaaaaaaccactgcgaccttcattgcaaaagttggaggtga
cccaatcccaaatgttaaatggacaaaagggaagtggagacagctgaaccaaggaggtcgtgttttcatccaccaaaaaggcgatga
agcaaaactggagattagggacaccacaaaaactgattctgggttataccgatgcgtggcatttaacgaacatggtgaaattgaaagta
atgttaacttacaggtggatgaaaggaagaaacaagagaaaattgaaggcgatcttagagcaatgctgaaaaagactccaatcttaaa
gaaaggagctggagaagaagaggaaattgatatcatggaacttctcaaaaatgttgatcctaaagaatatgaaaaatatgcccgcatgt
atggaatcactgacttccgaggtcttcttcaagcatttgaactgctcaagcaaagccaagaagaagagacacatagactggaaattgag
gaaatagagaggtcagagagggacgaaaaggaatttgaggaacttgtatcatttattcagcaaagactgtcacagacagagcctgtca
ctctgatcaaggacattgaaaatcagacagttttgaaagataatgatgctgtctttgaaatcgacattaaaattaattatccagaaatcaagc
tttcatggtacaaaggaactgaaaaactggaacccagtgataaatttgaaataagcattgatggtgaccgacatacactcagagtcaaaa
actgtcaacttaaagaccagggcaattatcgattggtttgtggtccacacatcgctagcgctaaactaactgtaattgagcctgcatgggaa
cggcatcttcaggatgtgactctgaaagaaggccagacttgcaccatgacatgccaattttctgtaccaaatgtaaaatctgaatggttcag
aaatggcagaatccttaaaccccaaggtagacataaaacagaagtggaacacaaagtccataaattgaccattgcagatgttcgagca
gaagaccagggtcagtacacctgcaaatatgaagaccttgaaacttcagcagaactcagaatcgaagctgaaccaattcagtttacaaa
gcgcatacagaacatcgtggtgagtgagcatcagtctgccacctttgagtgtgaagtgtcctttgatgatgccattgtaacatggtacaaag
gaccaacagaactgacagagagccagaaatacaacttcaggaatgatggccgctgccattatatgaccatccacaatgtgaccccag
acgatgaaggtgtctactcggtcatcgctcggctggagccaagaggtgaagcaagaagcacggcagagctgtacctaacgacgaaag
aaatcaaacttgagctgaagcctcctgatattcctgactccagagttccaatcccaaccatgcctatcagagcagtgccaccagaagaaa
tccctcctgtggttgctcctcctatcccccttttgctaccaacacccgaagaaaagaaaccaccaccaaaacgtattgaagttaccaaaaa
ggctgtgaagaaagatgccaaaaaagttgttgcaaagcccaaagagatgacaccacgtgaagagattgtcaagaagcctccacctcct
actaccttaattccagcaaaagctcctgaaatcattgatgtatcctctaaagctgaagaagtaaaaataatgactataaccagaaagaaa
gaggttcagaaagaaaaagaagctgtgtatgagaaaaagcaagcagtccacaaggagaagagagtcttcattgaatctttcgaagaa
ccttatgacgaactggaggtagaaccatacacagagccatttgaacaaccttattatgaagaaccagatgaagactatgaagagattaa
ggtagaagctaaaaaagaagttcacgaggaatgggaagaagattttgaagaagggcaagaatactatgaaaggaagaaggctatg
acgaaggggaggaagagtgggaagaggcttaccaagaaagggaagtaattcaagttcaaaaggaggtctatgaagaatcacatga
gagaaaagttccagccaaagtacctgaaaagaaagcaccaccacctcctaaagttataaagaagccagtaattgaaaaaattgaaaa
gacttctcgaagaatggaggaagaaaaagttcaagtcaccaaagtacctgaagtttcaaagaagattgttccacaaaaaccttcccgga
ctccagtacaggaagaagttattgaagtgaaagtaccagctgtgcatacaaagaagatggttatttcagaagaaaagatgttctttgcttct
cacacagaggaggaggtgtcagtcacagtccccgaggtacaaaaggaaattgttactgaagagaaaattcacgttgccgtttccaaaa

gggttgaaccaccacctaaagtccctgagctacctgagaaaccagctccagaagaagtggcccctgttcctatccctaaaaaagtggag
cccccagcaccaaaagttcctgaggttcccaagaaacccgtgccagaggagaaaaagccagttcctgtgcctaagaaggaacctgct
gctcccccaaaagtcccagaggtgccaaagaaacctgtccctgaagaaaagattcccgttcctgttgcaaagaaaaaggaagctcccc
cagctaaagttcctgaagtacagaagagagttgtgacagaagaaaaaataaccattgtaactcaaagagaggaatctccaccaccag
cagtgccagaaataccaaagaagaaagttcctgaagaaaggaaacctgttcctcggaaggaggaagaagttccaccaccaccaaaa
gtgccagctctgcctaagaaacccgtcccagaggagaaagttgcagtgccagttcctgtcgctaagaaagctcctcctccccgagctga
agtctctaagaaaactgttgtagaagaaaagagatttgttgctgaagaaaaactatccttcgcagttcctcaaagagtggaagtcacgcgg
cacgaagtatctgcagaggaggaatggagttactcagaagaggaggaaggtgtgtccatttcagtttatagagaagaagaaagagag
gaggaggaagaagcagaggttacagaatatgaagtgatggaagagcctgaggaatatgttgtggaagaaaagctgcacattatttcta
agagagtggaagctgagccagctgaagtgacagagaggcaggagaagaaaattgtactgaaaccaaaaattcctgctaaaatagag
gagcctccaccggctaaagttcctgaagcacctaagaaaattgtgccagaaaagaaagttcctgctccagttcctaaaaaggaaaaggt
gcccccacctaaagtgccagaagagccaaagaaaccagttccagaaaaaaaggttcctccaaaagtcattaagatggaagaacctct
cccagccaaagtgactgagaggcacatgcaaattacccaggaagaaaaagttcttgttgctgtaactaaaaaagaggcgcctccaaaa
gcaagagtgccagaggaaccgaagagagctgtcccagaagaaaaagttctgaaactcaaacctaaaagagaggaggaaccacca
gctaaagtgactgaattcagaaaaagagtggttaaagaagaaaaagtatcaattgaagctccaaaaagagaacctcaacccatcaaa
gaagtaactataatggaagagaaagaaagggcttataccctagaagaagaagctgtttcagtacaacgggaagaagaatatgagga
atatgaagaatatgattataaagaatttgaggagtatgaaccaacagaagaatatgaccaatatgaagaatacgaggagcgggagtat
gaacgatatgaagagcatgaagaatacatcacagaaccagagaagcctatccctgtaaagcctgtcccagaagaaccagttcccaca
aaaccaaaggccccaccggctaaagtgctgaagaaagctgtccctgaagaaaaagtaccagtgcccattcctaagaaactcaaacct
ccaccacccaaagtgcctgaagaaccaaagaaagtttttgaggaaaaaatacgtatttcaattaccaaacgtgaaaaagagcaggtga
ctgaacccgctgctaaagtgcccatgaagcccaagagggttgtcgcagaagaaaaagtacctgtccctagaaaagaagtagcaccac
ctgttagagtgccagaagtgcctaaagaacttgaaccagaagaggttgcctttgaagaggaagttgtaacccatgtagaagaatatcttgt
agaagaagaagaagagtacattcatgaagaagaggagttcataactgaggaagaagtggtgccagtgataccagtcaaagtgcctga
ggtacccaggaaacctgttccagaagagaagaaacctgttcctgttcccaagaagaaggaagctccaccggcaaaagtgcctgaggtt
cctaagaagccagaggagaaagttcctgtgcttattcctaaaaaggagaagcctccgccagcaaaagttcctgaagtgcccaagaaac
ctgtgccagaggagaaagtaccagtaccagttcctaaaaaggtggaagctccacctgccaaagtgccagaggtacccaagaagcctg
tgcctgagaagaaggtgccagttcctgctcctaagaaagtggaggctccacctgcaaaagtgccagaggtgcccaagaagctcatccc
agaagaaaagaaaccaacacctgttccgaaaaaagtggaagcaccaccacccaaagtgccaaagaaacgtgaaccagttccagtt
cctgtagctctacctcaggaagaggaagttctatttgaagaagaaattgttcctgaagaggaagttctacctgaggaagaggaagttctac
ctgaggaagaggaagttctacctgaagaagaggaagttctacctgaagaagaggaaattccacctgaggaagaggaagttcctcccg
aagaagaatatgtacctgaggaagaagaatttgtacctgaagaagaagtccttccagaagttaaacctaaggtgccagtacctgcacca
gtgcctgaaattaagaagaaagtgacagagaagaaagtggtcattcccaagaaagaggaggctccccctgccaaagttcctgaggtg
cctaagaaggtggaagaaaaacgaatcattctccctaaagaagaggaagttctaccagttgaagtgactgaggagcctgaagaagag
cctatttcagaagaagaaatcccagaagaaccacctagcatagaggaagttgaagaggtggcaccacctagagtgcctgaagtgatta
agaaagcagtacctgaagcacctactcctgttcctaaaaaagtggaggcaccaccagctaaagtgtcaaagaaaattcctgaggaaaa
agtacctgttcctgttcagaaaaaagaggcacccccagccaaagtgcctgaagtaccaaagaaagtcccagaaaagaaagtccttgtg
cctaaaaaggaagctgttcccccagctaaagggagaactgtccttgaagaaaaagtatcagttgccttccgccaagaagtagtagtaaa
agaaagactagaattagaagtagtagaagcagaagtggaagaaattccggaagaagaagagttccatgaagttgaagaatattttgaa
gaaggcgagtttcatgaagtagaagaattcatcaaattagaacaacatagagttgaagaagaacacagagttgaaaaagttcataggg
taatagaagtttttgaggctgaagaagtggaagtatttgaaaaaccaaaagctccacctaaagggcctgagatatctgagaaaatcatcc
ctccaaaaaaaccgcccactaaagttgttcctcgaaaagagccaccagctaaagtaccggaggtgcctaagaaaattgtggtagaaga
aaaagtacgtgttcctgaagagcccagagttccaccaactaaagtgcctgatgtgctgccaccgaaggaagtggtcccagaaaagaaa
gtaccggtgcctcctgccaaaaagccagaagctccacctcctaaagtgcctgaggctcccaaagaagttgttcctgaaaagaaagtgcc
agtgcctcctcctaaaaagcctgaagtgccacccacaaaagtcccagaggtgccaaaggcagctgtcccagaaaagaaggtgcctga
agctattcctcccaaaccggaaagtcctcccccctgaagtgccagaggctccgaaagaagttgttcctgaaaagaaagtgccagcggctc
ctcctaaaaagcctgaagtcacacctgttaaagtgcctgaggctcccaaagaagttgttcctgaaaagaaagtgccagtgcctcctcctaa
aaagcctgaagtgccacccacaaaagtcccagaggtgccaaaggtagctgtcccagaaaagaaggtgcctgaagctattcctcccaa
accggaaagtcctcccccctgaagtgttcgaggagcctgaggaagttgccctagaagagcctcctgctgaagttgtggaagagccagag
ccagcggcgcctccacaagtgaccgtaccacctaagaaacctgtcccagaaaagaaagcacctgctgtcgttgccaaaaaacctgaa
ctaccaccagtgaaagtgcccgaggtgcccaaggaggttgttcctgaaaagaaggtgcctctggtggttcccaaaaagccagaagccc
cacctgctaaagtgcctgaagttccaaaagaagttgttccagaaaagaaagtagctgttcccaaaaagccagaagtcccaccagcaaa
agtgccagaagtgccaaagaaacctgtcttggaagagaaaccagctgttcctgttccagaaagagcggagtctcctcccccagaagtat
atgaagaacctgaggaaattgctcctgaagaggaaattgctcctgaagaggaaaagccagttcctgttgcagaagaggaggaaccag
aggttccacctccagcagtgcctgaagagcccaagaagatcatcccagagaagaaagttcctgtcatcaaaaaaccagaagcaccgc
ctcctaaagaacctgaacctgaaaaggttattgagaagccaaaactcaaaccaagacccccacctcctccacctgctccacctaagga
agatgtgaaggagaaaatattccaacttaaagctattccaaagaagaaagttcctgaaaaacctcaggttccagaaaaagtggagctta
cacctctgaaagtgcctggaggtgaaaagaaagttcgcaaattacttccggaacgtaaacctgaaccaaaggaagaagttgttctgaaa

agcgttctaagaaaaagacctgaagaagaagaacctaaagtagaacctaaaaaactagaaaaagttaaaaaacctgcagtaccag
aaccaccacctccaaaacctgttgaagaggttgaagtacctactgttacaaaaagggaaaggaagattcctgaaccaacaaaagtgcc
tgaaatcaagccagcaatacctctccctgcacctgaaccgaaaccaaagcccgaagcagaagtgaaaacaatcaaaccacctcctgt
ggaacctgaaccaacccccatcgctgccccagtaacagtgccagtggttggaaagaaagcagaagccaaagcacctaaggaagag
gctgccaagccaaaaggtcctatcaaaggtgtacccaaaaagactccttcaccaatagaagccgaaaggagaaagttaaggccagg
aagtggtggagagaaacctcctgatgaagccccgttcacctaccagctaaaggctgtgccactgaagtttgtgaaagaaatcaaagaca
tcatcttgacagaatcagagttcgttggctcttcagcaatctttgaatgtttggtctccccttccactgcaattacaacctggatgaaagacggt
agcaatatccgtgagagtcccaagcacaggtttattgcagatggtaaagacagaaagctgcacatcattgatgttcaactttccgatgctg
gtgaatacacctgtgttttacgtttgggaaacaaagaaaagacctccacggctaaacttgttgtagaagaacttcctgtgcgttttgtaaaaa
cactggaagaggaagtcacagtggtcaaaggacagccattgtacttgagctgcgagttaaacaaagagcgtgacgtggtctggaggaa
ggatggcaagattgtggtggagaaacctggccgaattgtgccaggcgtcattggcttgatgcgggccctgaccatcaacgatgcagatg
acacagatgctggaacatacacagttactgtggaaaacgccaacaacctggagtgttcatcttgcgtaaaagtagtagaagtcattagag
attggctggtgaaacctatacgagaccagcatgtgaaacccaaggggacagctatttttgcctgtgatatagcaaaagatactccaaaca
ttaagtggttcaaaggatatgatgaaatccctgcggaaccaaatgataagactgaaatactgagagatggaaatcatctgtacctcaaaa
ttaagaatgctatgccagaagatattgctgagtatgcagtggaaattgaaggaaaaagataccctgcaaagctgacacttggagagcgt
gaagttgaactgcttaaaccaatagaggacgttaccatttatgagaaagaaagtgcaagctttgatgcagaaatctcagaggcagacatt
cctggacaatggaaactgaaaggagaacttctaaggccctcacctacttgtgaaatcaaagcagaaggtggaaaacgcttcttaactttg
cgcaaagtcaaactggaccaagctggtgaagtcctctaccaggcccttaatgcaattacaactgccattttgacagtaaaagaaatcgaa
cttgactttgctgtgcccctgaaggatgtcactgttccagaaaggcgacaggctcgattcgaatgtgtcctcacccgagaggcaaatgttat
atggtccaaaggacctgatataattaagtcatctgacaaatttgatatcatcgctgatggaaagaaacatattcttgttattaatgattctcaatt
tgatgatgaaggggtctatactgctgaggtggagggcaagaagacctcagctcggttgtttgtcacaggtataagactgaaattcatgtcac
ctcttgaagatcaaacagtaaaagaaggtgaaacagcaacttttgtttgtgaactttctcatgaaaaaatgcatgtagtctggttcaaaaatg
atgccaaactccatacaagcagaacagtactcatctcttctgagggcaagactcacaaattggaaatgaaagaagtgacattggatgat
atatctcagataaaagctcaagtcaaggagctgagctccacagcacagctgaaggtcttagaggccgatccctacttcactgtgaaatta
catgacaaaactgcagtggagaaggatgagattactttgaagtgtgaagtgagcaaagatgtaccagtgaaatggttcaaagatggtga
agagattgtcccttcacccaaatattctatcaaggcagatggcctgcgccgcatcttaaaaatcaaaaaggcggaccttaaagataaagg
cgaatatgtgtgtgactgtggcacagacaagaccaaggcaaatgttactgttgaggctcgactaataaaagtggaaaagcctctgtacgg
agtagaggtgtttgttggtgaaacagcccactttgaaattgaactttctgaacctgatgttcacggccagtggaagctgaaaggacagccttt
gacagcttcccctgactgtgaaatcattgaggatggaaagaagcatattctgatccttcataactgtcagctgggtatgacaggagaggttt
ccttccaggctgctaatgccaaatctgcagccaatctgaaagtgaaagaattgcctcttatcttcatcacacctctcagtgatgttaaagtcttc
gagaaagatgaggctaagtttgagtgtgaagtatccagggagcccaaaacattccgttggctaaaaggaacccaggaaatcacaggtg
atgacagatttgagcttataaaggatggcactaagcattcaatggtgatcaagtcagctgcttttgaagatgaagcaaaatacatgtttgaa
gctgaagataagcacacaagtggcaaactgatcattgaaggaatccggctcaaattcctcacccctctcaaagatgtaactgccaaaga
gaaggaaagtgctgtatttactgtggagttatctcatgataacatccgagttaaatggttcaagaatgaccagcgcctacacaccaccagg
tcggtctcaatgcaagacgaagggaaaactcattcgatcacattcaaagacctgtctattgatgacacctcccaaattagagtagaagcta
tggggatgagttcagaagctaaactcactgtgcttgagggagacccatattttacaggaaaacttcaagattatactggtgtagagaaaga
tgaagttattctacagtgtgaaattagcaaagcagatgcaccagtgaaatggtttaaggatgggaaggaaataaagccatccaaaaatg
ctgttattaaggcagatggcaagaaacgcatgctaatcctaaagaaagccttgaaatcagatattggacagtacacctgtgactgtggga
cagataagacctcaggaaaacttgacattgaggatcgggaaattaaactggtgcgacccctgcacagtgtggaggtgatggagactga
gacagcacgctttgaaaccgaaatctctgaagatgatatccacgccaactggaaactcaagggagaggccctactccaaacacctgatt
gtgaaattaaggaagaaggcaaaatacactcccttgttttgcacaactgtcgcctggaccagacgggtggggtggatttccaagctgcca
atgttaaatctagtgcccacctccgagttaagccacgagtaattggtcttctgaggcctttaaaggatgtcaccgtgactgcaggggaaaca
gccaccttcgactgcgagctctcctacgaagatatcccagtggaatggtatctcaaagggaagaaactagagcccagcgataaggtggt
cccacgttcagaaggaaaagttcatacacttactctgagggatgtaaagttagaagatgctggggaagtccaactaacagcaaaagattt
caaaactcacgccaacctctttgtgaaagaacccccagttgaattcactaagcctcttgaggaccagacggtcgaagagggagccactg
cagtgctggagtgtgaagtctccagagaaaatgctaaggtgaaatggttcaaaaatgggacagaaatcctcaaaagcaagaagtatga
aattgttgctgatggcagggtcagaaaacttgttatacatgactgtaccccagaggatattaaaacatacacttgtgatgctaaggattttaa
gacttcctgtaacctgaatgtcgtgcctcctcatgtggaattcttaagaccactcaccgaccttcaagttagagaaaaagaaatggctcgatt
tgagtgtgaactttcccgagaaaatgctaaggttaagtggtttaaagatggtgctgaaattaaaaagggcaaaaaatatgacatcatatcc
aagggagcagtgcgcattcttgtcatcaacaaatgtctactggatgatgaagctgaatattcctgtgaagtaaggacagcgagaacttctg
gcatgctgacagttctggaagaagaagctgtctttaccaaaaatcttgccaacattgaagttagtgaaacagacactataaaactggtttgt
gaagtctccaaacctggcgcagaagtgatttggtataaaggggatgaggagatcattgaaacaggaagatatgaaatactgactgaag
gacggaagagaatcctggtcattcagaacgctcaccttgaggatgctggcaactacaactgtcgactcccaagctctcgaaccgatggc
aaagtcaaagtacatgaactggctgctgaatttatctcaaagcctcaaaaccttgaaatacttgaaggagaaaaggctgaatttgtctgctc
tatatcaaaagaaagctttccagtccagtggaagagggatgataagacacttgaatctggagataaatatgacgttattgctgatggtaaa
aagagggtcctagttgtgaaagatgccacattacaagatatgggcacttacgttgtcatggtaggggccgccagagcagcagctcacttg
acagtcattgaaaaactcaggatcgtagttcctcttaaggacacccgggtgaaggaacaacaggaagttgtcttcaactgtgaagtcaat

actgaaggtgccaaagccaaatggttcagaaatgaagaagctatatttgatagttcaaaatacatcattctccaaaaagacctagtctaca
ccctcagaattagagatgcacacttagatgaccaagccaactataatgtgtctttgaccaatcacagaggtgaaaatgttaaaagtgcag
ccaatctaatagtagaagaggaagaccttaggattgttgagcctcttaaagatattgaaacaatggagaagaaatctgtcacattctggtg
caaggtgaatcgtctcaatgtaacactgaagtggaccaaaaatggtgaagaagtgccttttgacaaccgtgtctcatacagagttgataag
tacaagcacatgttaaccattaaagactgtggcttcccagatgaaggtgaatacattgtcactgctggacaagataaatctgttgctgagctt
ctcatcatagaagccccgacagaatttgtggaacacttggaagatcagacagtcactgagttcgatgacgctgtcttctcctgccagctctc
cagagagaaagccaatgtaaaatggtacagaaatgggagagaaatcaaagaaggcaaaaaatacaaatttgaaaaagatggaagt
atacacagactcattataaaagattgcaggctggatgatgagtgtgaatatgcttgcggggtagaagacaggaagtctcgtgctagactttt
tgtggaagaaattcctgttgagatcatcaggcctccacaagatattcttgaagcccctggtgctgatgttgtcttttagcagaactcaataaa
gataaggtggaagtccaatggctaagaaataacatggttgttgtccagggtgataaacaccagatgatgagtgaaggaaagatacatcg
actacagatttgtgatattaagccccgtgaccagggtgaatacagatttattgccaaagacaaagaagccagagctaagcttgaactggc
agctgcaccaaaaatcaagacagctgaccaagaccttgtggttgatgttggcaagcctctgacaatggtggtgccatatgatgcctaccc
caaagcagaagctgaatggtttaaagaaaatgaacctttatctacaaaaaccattgatactacggctgaacaaacttctttcagaattttag
aagccaagaaaggagacaaagggaggtataaaattgtgcttcagaacaaacatggaaaagcagaaggattcatcaatttaaaagttat
tgatgttcctgggccagtacgtaacttagaagtgacagaaacatttgatggtgaagtgagccttgcttgggaagaacctttaactgatggtg
gaagcaaaatcataggttacgttgttgaaagacgtgacattaagagaaagacctgggttctggccacagaccgtgcagagagttgtgag
tttactgtcactggtctacagaaaggaggagttgagtacctattccgtgtgagtgcaagaaacagagttggcactggtgagccagtagaaa
ctgacaatcctgtagaagcaaggagtaaatatgatgttccaggccctcctttgaatgtaaccatcactgatgtgaatcgatttggtgtctcact
gacatgggaaccaccagagtatgatggaggtgctgagatcacaaactacgtcattgaattaagagacaagacttctatcaggtgggata
ctgccatgactgtgagagctgaagacctgtctgcaactgttactgatgtggtagaaggacaggagtacagtttccgagtgagagcccaaa
atcgaattggagttggaaaaccaagtgcagccacacccttcgtcaaagttgctgatccaattgagagaccaagtcctcctgtaaacctaa
cttcctcagatcagactcagtcatcagttcagctcaaatgggaacctcctctgaaagatggaggaagcccaatattaggctatataattgag
cgatgcgaagaaggaaaagataattggattcgttgcaatatgaaacttgtccctgaactgacttacaaggttaccggattggaaaaagga
aataaatatttatatagagtatctgcagaaaataaagctggtgtttcagatccatctgaaattcttggtcctctcaccgctgacgatgcatttgtt
gaaccaacaatggatttaagtgcatttaaagatggtctggaagttattgtcccaaatcctatcacgatcctggttccaagtacaggctatcca
aggccaactgcaacctggtgttttggagataaagtactagaaacaggggaccgggtgaaaatgaagaccttgtctgcctatgccgaactt
gtcatttctccaagtgaacgttcagacaagggcatttatacactgaaattagaaaaccgtgtgaaaacaatttctggggaaattgatgtcaat
gtaattgctcgcccaagtgcacccaaagaattgaaatttggtgatataaccaaggactcagtacatttgacttgggaaccacctgatgatg
atggaggaagtccgttaactggatacgttgttgaaaaacgagaagtcagccggaaaacatggactaaagttatggactttgtgactgatct
agaattcacagttcctgatcttgttcaaggaaaagagtacttatttaaagtttgtgctcgtaacaaatgtggccctggagaacctgcatatgttg
atgaacctgtaaatatgtcaactcctgcaacggtacctgacccaccagagaatgttaaatggagagatcgaacagccaatagcatcttctt
aacatgggatccacctaaaaatgatggtggttcacgcatcaaaggatatatagttgaaagatgtccacgtggttctgataaatgggttgcct
gtggagaacctgttgcagaaacaaaaatggaagtgacaggtcttgaggaaggcaaatggtatgcctaccgcgtgaaggccttaaacag
gcagggtgctagcaaaccaagcagacccacagaggaaatccaggctgtggacacacaagaggccccagaaatcttcctcgatgtga
agctccttgctggtctcactgtaaaagctgggaccaagattgaacttcctgccaccgtaaccggaaaacctgaacctaaaataacttggac
aaaggctgatatgattctgaagcaggacaaaagaattaccattgaaaatgtccctaagaaatccacagtgactattgttgatagtaagag
aagtgacactggcacatatatcattgaggctgtgaatgtgtgtggccgggccactgctgtggtggaagtgaacgtcttagataaacccgga
ccaccagctgcctttgacatcacagatgtaaccaatgagtcatgtcttctaacatggaacccaccacgcgatgatggtggatctaagatca
caaactatgttgtggagagacgagcaactgatagtgaagtgtggcacaagctctcatccaccgtcaaggatacaaacttcaaggccacc
aaattaatccccaataaagagtacatcttcagagttgctgcagaaaacatgtatggtgttggtgaaccagttcaggcctctccaataacagc
caaatatcagtttgatccacctggtcctccaactcgcctagaaccttctgatatcactaaagacgcagtgactctcacatggtgtgagccag
atgatgatggtggcagcccaatcacaggatactgggttgaaagactggatcctgatacagataaatgggttagatgcaataagatgcca
gtaaaggacacaacatacagagtgaaaggtctcactaataagaaaaaatacagattccgtgtgttggctgaaaatcttgctggacctgga
aaaccaagcaaatcaactgaaccaatcttaataaaggatcccatagatcctccatggcccctggaaaaccaactgtaaaagatgtag
gcaaaacatcagtaaggttgaattggacaaaaccagaacatgatggaggtgcaaagattgagtcttatgtcattgaaatgctgaagactg
gaacagatgagtgggtcagagtggcggaaggggttcccaccactcagcacttgctcccagggctcatggaaggacaggaatactcatt
ccgagttagagctgtgaataaggctggggaaagtgaacccagtgaacccagtgaccctgtgctttgccgggagaagctatatcctccatc
accaccacgctggcttgaagttattaatatcacaaaaaatacagcagacctaaaatggacagttcctgagaaagatggagggtcccccca
tcaccaactacattgtggaaaagagagacgtcaggcgaaaaggctggcaaacagtggataccactgtcaaggacaccaagtgcaca
gtcaccccactgactgagggctctttatatgtgttccgagttgctgcagaaaatgctataggacaaagcgactacaccgaaattgaggact
ctgtgctggccaaagacacctttaccactcctggaccaccctacgccctggcagtggttgatgtgacaaaacgacatgttgacctaaagtg
ggagccacctaaaaatgatggtggaagaccaatacagagatatgtcattgagaagaaagaaaggttaggtacccgttgggtgaaagct
ggaaagactgcaggacctgactgtaacttcagagtaactgatgtcatcgaaggaacagaggtccagtttcaggttcgggctgaaaatga
agctggagttggccacccaagtgaacccacagaaatcctatccattgaagatccaacaagtcctccctcaccaccccttgacctacatgt
gactgatgctgggagaaaacacattgccattgcttggaagcctccagagaaaaatggtggaagtcctatcataggataccatgttgaaat
gtgtccagtaggcactgagaaatggatgagagttaattctcgcccaataaaggacttgaaattcaaggttgaagaaggtgttgttcctgac
aaagaatatgtcctgagagtgagagcagtcaatgctattggtgtcagcgagccatctgaaatctctgaaaatgtggttgccaaagaccca

gactgcaagccaacaattgacctggagactcatgacattattgttattgaaggtgaaaagttaagcattcctgttcccttcagagctgtccca
gttccaactgttagttggcataaagatggcaaagaagttaaagcaagtgatagattaacaatgaagaatgatcacatctctgcacaccttg
aagttcccaagagtgtccgtgcagatgccggaatttataccattacactggagaataagctcggctcagcaacagcctcaatcaatgtca
aagtcataggcctacctggaccatgcaaagatattaaagcaagtgacattaccaagagttcttgtaagttaacttgggaacctccagaattt
gatggtggaaccccaattcttcattatgtcctggagcgcagagaagctgggaggagaacatatataccagtcatgtctggtgagaacaaa
ctgtcatggactgtgaaggatctcataccaaatggtgaatacttcttccgtgttaaagcagtcaacaaggttggtggaggagaatatattga
actgaaaaatccagtcattgctcaagatccaaagcaacccccctgatccacctgtagatgtagaggttcataatcctacagcggaggcaat
gactattacatggaagccacctttgtatgatggagggagcaagataatgggctacatcatagagaagattgctaagggtgaagaaaggt
ggaagagatgcaatgaacacctggtaccaatcctgacctatacagcaaaaggacttgaagaggggaaagagtaccaattccgtgtgc
gagcagagaacgccgcgggtattagtgaaccttctcgggctactcctccaaccaaagctgtagatcccattgatgcccccaaagtcattct
gagaacaagcctagaagtgaaacgaggtgatgaaatagcacttgatgcaagtatttctggatcaccttacccaactattacatggataaa
ggatgaaaatgttattgtaccagaggaaattaagaagcgtgcagcacccttggttaggagaaggaagggtgaagttcaagaagaaga
accatttgtcctgcctctgacacagcgtttgagtattgacaacagcaaaaagggagaatctcagctacgcgtccgagattctctccgacctg
accatggtctgtatatgatcaaagttgaaaatgaccacggtattgcaaaagctccttgtactgtcagtgtgttagatacaccgggaccacca
atcaactttgtatttgaagatatcagaaagacctcagtcctttgtaaatgggaaccaccccttgatgatggtggcagtgaaatcataaactac
actttggaaaagaaagacaagacaaaacccgactcagaatggattgttgtcacttcaacacttagacattgcaaatattcagtaacaaaa
ctgattgaaggaaaagagtacctcttccgtgtaagagctgaaaacagatttgggccaggtccaccatgtgtttcaaagccacttgtggcta
aagatccatttggaccacctgatgcaccagataagcccattgtggaagatgttaccagcaacagtatgctagtgaaatggaatgaacca
aaagataatggaagccccattttgggttactggcttgaaaaacgtgaagttaacagtacacattggtctcgtgtcaacaaaagccttctgaa
tgccttgaaagccaatgtagatggcttattagaaggactcacctatgtcttcagagtatgtgctgaaaatgcagctggacctggaaagttca
gtccaccttcagatcccaaaacagcacatgatccaatctctcctcctgggccacctatcccaagagtcactgacacaagctctacaactat
tgaactagaatgggaacccccagctttcaatggtggtggggaaattgttggctattttgttgataagcagttggttggcacaaatgaatggtc
acgctgcacagagaagatgatcaaggtccgtcagtacaccgtcaaagaaatccgagagggtgctgattacaaacttcgggtgagtgct
gtcaatgccgcaggggaaggaccgcctggagaaacacaacctgttactgtggctgaaccacaagagcctccagctgtggaactggat
gtttctgtcaagggtggaatacaaataatggctgggaagactcttagaattccagctgtggtgactggtcgccctgtacctacaaaagtatg
gaccaaagaagaaggggagctggataaagaccgtgttgtaatagacaacgttggaaccaaatctgaactaattatcaaggatgcactg
cgaaaagaccatggcagatatgtgattacagctacaaatagctgtggttccaaatttgcagcagccagggtagaagtttttgatgtccctgg
tccagttcttgacttaaaacctgttgtaacaaacagaaaaatgtgtctacttaactggtctgatccagaagatgatggaggaagtgaaataa
caggctttatcattgaaagaaaagatgccaagatgcatacttggagacaaccaatagagactgagagatctaaatgtgacatcacaggt
ctgcttgagggacaagaatataagttccgtgttattgccaagaacaagtttggctgtggccctcctgttgaaataggaccaattcttgcagttg
atccactaggtcctccaacatctccagagaggctcacatacactgaaaggacaaagtccactatcacacttgactggaaagagccccg
cagtaatggtggcagtcccatccaaggatatatcattgaaaaacggcgtcatgacaaacctgactttgaaagagttaacaagcgactctg
cccaaccacatcttttctggttgaaaatcttgatgaacaccaaatgtatgagttccgtgtcaaagctgtcaatgaaattggtgaaagtgaacc
atccctacctcttaatgtagtcatacaagatgatgaagtgcctccaactattaagttgcgtctgagtgttcgaggagacactatcaaagttaa
ggcaggagagcctgtccacatccctgcagatgtgacaggccttccaatgcctaagattgaatggtccaaaaatgaaactgtaattgaaaa
acccactgatgcacttcagataaccaaggaagaggtatcccgaagtgaggcaaaaactgagcttagcattcccaaagcggtccggga
ggacaaaggcacttacacagttactgcttccaatcgccttggctcagtgttccgaaatgttcacgttgaagtatatgaccgcccatccccac
caagaaatcttgctgttactgacattaaagctgaatcttgctacttgacatggatgcccctcttgataatggtggcagtgaaatcacccattat
gttattgacaaacgtgatgcaagtaggaagaaagcagaatggaggaagtcaccaacactgctgtagagaaaagatatggatctgg
aaacttatccccaatggtcagtatgagttccgagtcagggcagtgaataaatatggaatcagtgatgagtgcaaatcagataaagtagtc
attcaagatccttatcgccttcctggacctccaggaaaaccaaaagttttggcacgcaccaaaggatcaatgctagtgagctggactcctc
ctttggacaatggtggctctccaattactggctactggctggagaaaagagaagagggaagtccttattggtcacgtgttagccgagcacc
aataaccaaagtgggattgaaaggcgtggaatttaatgttcctcgtttgcttgaaggcgttaaataccagttcagagccatggcaataaatg
ctgcaggaattggtcctcccagtgaaccatcagatccagaggttgcaggagatcccatatttccaccggggccaccttcttgcccagaagt
taaagataaaacgaagtcaagcatctcactaggatggaaacctccagccaaagatggtggcagcccaatcaaaggatacattgtaga
aatgcaagaagaaggtactactgactggaaaagagtaaatgaaccagacaaacttataactacctgtgaatgtgtggtgcctaatctga
aagagctcaggaagtacagattcagagtgaaagctgtcaatgaagctggtgaatctgaaccaagtgatacaactggggagatccctgc
cactgatattcaagaggaaccagaagttttcattgacattggagcacaggactgtctggtttgtaaagctggctcacagattaggattcctgc
tgtcatcaagggacgcccaacaccaaaatcatcttgggaatttgatggaaaggcaaagaaagcaatgaaggatggagttcatgacata
cccgaagatgcacagctggagactgctgaaaactcctcagtaattattattccggagtgtaaacgatctcatacaggcaaatacagcatc
acagccaagaataaagcaggacaaaagactgcaaattgcagagttaaagtcatggatgtaccaggcccacccaaagatctgaaagt
cagtgatatcacaaggggtagttgcagactttcatggaagatgccagacgacgatggaggagacaggatcaaaggctatgttattgaga
agaggactattgatggaaaagcctggaccaaagtcaatccagactgtggaagcaccacatttgtagtgcctgatctcctctctgaacagc
aatatttcttccgtgtgcgagcagaaaaccgttttggtattggcccacctgtggaaaccattcagaggaccactgccagagatccgatatat
cctcctgatcctcctattaaactcaagattggcctcatcacaaagaacacagtgcatctgtcatggaaacccccgaagaatgatgggggct
cccctgttacccactatattgttgagtgccttgcatgggaccctactgggacaaagaaagaagcctggaggcagtgcaataagcgtgatgt
ggaagaactgcaatttactgttgaagacctagtagaaggtggggaatatgaattccgagtcaaagctgtcaatgctgcaggagtcagca

agccttcagccactgttgggccctgtgactgtcaaagaccagacatgccaccatcaattgatctaaaagaattcatggaggttgaagaag
gaaccaatgttaacattgtggccaaaattaaaggtgtgccattcccgacactaacctggtttaaagctcctccaaagaagcctgataacaa
agaacctgttctctatgacacccatgtcaacaaactggtggtagatgatacttgcactttagttattccgcagtctcgcaggagtgacactgg
cttatataccatcacagctgtaaataatctgggaacagcatcaaaggagatgagactgaatgtcctgggtcgtcctggccctccagtggga
cccataaaatttgaatctgtttcagcagatcaaatgacactatcttggtttccacctaaagatgatggtgggtctaagattacaaactatgtaat
tgagaaaagagaagctaacaggaagacatgggtccatgtctccagtgaacctaaggagtgcacgtacacgattcccaaattgctagaa
ggccatgaatatgtattccgaatcatggcccagaataaatatggcattggagaacctcttgacagtgaacctgaaacagcaagaaacctc
ttctctgtccctggagcaccagataaaccaacagttagcagcgtgactcgtaactccatgactgtcaactgggaagagccagaatatgat
ggaggctctcctgtgacagggtactggctggaaatgaaagacaccacttcaaagagatggaagagagttaaccgagatcctatcaaag
ccatgactttgggtgtttcttataaagtgactggtcttattgaaggttccgactatcaattccgggtatatgcaatcaatgctgctggcgtgggtc
cagcaagtctgccatcagacccagcgactgctagagatccaattgcccctcctggtcctccatttcccaaagtgacagattggactaaatc
atctgcagatctggagtggtctcccccactaaaagatggtggatccaaagtaactggatacatcgttgaatataaagaagaaggaaaag
aagaatgggaaaagggtaaagataaagaagtgagaggaacaaagctcgttgtgacaggattaaaggaaggagcattctacaaattta
gagttagtgcagtcaacattgctggcattggagaacctggagaggtcacagatgtcattgaaatgaaggacagacttgtttcacctgacctt
cagctagatgccagtgtcagagatagaattgttgtccatgctggaggggtgatccgaatcattgcctatgtgtctggaaagcctcctccaac
cgtcacctggaacatgaatgaaagaaccttacctcaagaagccaccattgagaccacagccattagctcatccatggtcatcaagaact
gccagaggagccatcaaggcgtctattctcttcttgccaaaaatgaagccggagaaagaaagaagacaattattgttgatgtattagatgtt
ccaggtcccgttggaacaccattcctagctcacaacctaaccaatgagtcctgcaaactgacatggttttctccagaagatgatggaggct
ctccaatcaccaattatgtcattgaaaagcgtgaatctgaccgcagagcatggaccccagtgacatatacagttacccgacaaaatgcta
ctgtccagggtctcattcaaggaaaagcctactttttccgaattgcggctgaaaatagtattggcatgggtccatttgttgagacatcagagg
cacttgttatcagagagccaataactgtaccagagcgtcctgaagacctggaagtcaaagaagttactaaaaatactgtaactttgacttg
gaatcctcctaagtatgatggtgggtcagaaattattaactatgtcctagaaagtcggctcattgggactgagaagttccacaaagttacaa
atgacaacttgcttagcagaaaatacactgttaaaggcttaaaagaaggtgatacctatgagtaccgtgtcagtgctgtcaacattgttgga
caaggcaaaccatcattttgcaccaaaccaattacttgcaaggatgagctggcacccccaacgcttcacctcgacttcagagataagctc
acgattcgagttggtgaagcttttgccctcactggccgttactcaggcaaaccaaagcctaaggtttcctggttcaaagatgaagctgatgtg
ctggaagatgatcgcactcatataaagactacaccagcaacacttgctttagagaagatcaaggccaaacgttcagattccggcaaata
ctgtgtggttgtggagaacagtacaggctctaggaaaggtttctgtcaagttaatgttgttgaccgtcctggaccaccagtaggaccagttag
ttttgatgaggtgaccaaagattacatggttatctcttggaagcctcctttagatgatggaggcagtaaaatcaccaattatattattgagaag
aaggaagtgggtaaagacgtctggatgccagtgacatctgcaagtgctaaaacaacatgcaaagtttctaaactacttgaaggaaaag
attatattttccggatacatgctgaaaatctgtatggaataagtgatcctctggtgtctgattcaatgaaagccaaagatcgtttcagggttcctg
atgcacctgatcagccaattgttacagaagttaccaaagactctgcattagtaacctggaataagccacatgatggaggaaaacccatca
caaactacatcctggaaaagagagaaactatgtctaaacgatgggctagagttaccaaagatcctattcatccatacactaaatttagggt
tcctgatcttctagaaggatgtcagtatgaattccgggtttctgcagaaaatgaaattggtattggagatccaagcccaccatccaaaccagt
ctttgctaaagatccaattgctaaaccaagtccacctgttaatcctgaagcaatagatacaacatgcaattcagtcgatctaacttggcagc
caccacgtcatgatggtgggagcaagattctgggttatattgttgagtaccagaaagttggagatgaagagtggagaagagccaatcac
acccctgagtcatgtcctgaaactaaatataaagtcaccggtcttcgggacggtcaaacctataagtttagagtgttagcagtcaatgcagc
tggtgaatcagatccagctcatgttccggagccagtcctagtaaaagacaggcttgaacccccctgagttgattcttgatgccaacatggca
agagaacaacacattaaagttggtgatactctaagacttagtgccatcatcaaaggagtgccattcccaaaagtaacttggaaaaaaga
agacagagatgctccaactaaagcaagaattgatgtgactccagttggtagcaagcttgaaattcgtaatgctgcccatgaagatggtgg
aatttattctttaacagtggagaatccagctggttcaaaaactgtctcagtaaaagtacttgtattagataaacctggcccacctagagatctg
gaagtcagtgaaattaggaaagattcatgttaccttacttggaaagaaccactggatgatggtggttctgttattaccaattatgtggttgaga
ggagagatgttgccagcgcccagtggtcacctctctcagctacatcaaagaaaaagagtcacttcgctaagcatctgaatgaaggcaac
cagtacctcttccgagtagctgcggagaaccagtatggacgtggtcctttgttgaaacaccaaaaccaatcaaggctttggatcctctccat
cccccagggccacccaaggacctgcaccatgtagatgttgacaagactgaagtctccctagtctggaataagccggatcgtgatggtggt
tctccaatcactggatatttggtagaatatcaagaagaaggcacccaggactggattaaatttaagactgtgacaaacttagagtgtgtggt
tactggactacaacaaggaaagacctatagattccgtgtaaaagctgaaaacattgtgggtcttggtctccctgacacaactatcccgata
gaatgtcaagaaaaactagtgcctccatccgtggagctagatgtgaaattaattgaaggtcttgtggtaaaggctggaaccacagtcagat
tccctgctattataagaggtgtgcctgttcctactgcaaagtggacaaccgatgggagtgagattaaaaccgatgagcactacacagttga
aacagacaacttctcatcagtacttaccattaagaactgcttaaggagagacactggggaatatcaaatcacagtttccaatgcagccggt
agcaaaacagtagccgtacatcttactgttcttgatgttcctggcccaccaacaggtcctattaatattctggatgttactcctgaacacatga
ctatctcatggcagccacctaaggatgatggaggaagccctgtgataaattatattgttgagaaacaagatacaaggaaagacacgtgg
ggtgttgtctcttccggaagcagtaagacaaagctgaaaatcccacatctgcagaagggctgtgaatatgttttccgagttagagcagaga
ataagataggtgttggtcctccccttgactccacacctactgttgctaagcataaatttagtcctccgtctcctcctggtaaaccagtggttactg
acattactgaaaatgcagcaacagtgtcttggaccctgccaaaatctgatggtggcagtccaataactggctactatatggaacgtcgaga
agtaactggcaaatgggtgagggtcaacaaaacacctatcgctgacctgaagttcagagtgactggactctatgaaggaaatacatatg
agtttagagtttttgctgaaaatcttgcaggactaagcaaaccatccccaagttctgatccaataaaagcttgccggcccatcaaaccacct
ggaccacctattaatcctaaactgaaagacaagagcagagaaacagctgatttggtgtggacaaagcctctcagtgatggtggtagccc

cattctaggatatgtagtggaatgtcagaaacctggcacggcacaatggaacaggattaataaagatgaactcattaggcaatgtgccttt
agggtacctggactaattgaaggaaatgagtacagattccgtataaaggcagctaatattgtaggagagggtgagccaagagaactag
cagaatctgtgattgcaaaagatatccttcatcctccagaagtagaacttgatgttacttgtcgtgatgttattaccgtgagagtaggccaaac
tatccgcattctagctcgagtcaaaggcagacctgaaccagacataacttggactaaggaaggcaaagtattggtccgagaaaagagg
gtggaccttattcaggatctacctcgtgttgagttacaaattaaagaagctgttagagctgatcatggcaagtatatcatctcagctaagaac
agcagtggacatgcccaaggttcagccatcgttaacgtccttgacagacctgggccttgccagaatttgaaggttaccaatgtaaccaaa
gagaactgtacaatttcttgggaaaacccactagataatggtggctcagaaataacaaacttcatagtagaatatcgcaaaccaaacca
gaaaggctggtcaattgttgcatcagatgtcactaaacgattaatcaaggccaaccttttagccaacaatgaatactatttccgagtttgtgc
agagaataaagtaggtgttgggccaaccatcgaaacaaaaactcccattctggctattaaccctattgacagaccaggtgagcctgaaa
accttcacattgcagataaaggaaagacatttgtctatctaaagtggcggaggcctgactatgatggtggcagtccaaatctgtcatatcat
gttgagagaaggcttaagggctccgatgactgggaaagagtgcataaaggaagcattaaagaaactcactacatggttgacagatgtgt
tgaaaaccagatttatgagttcagagtgcaaacaaagaatgaaggtggggaaagtgactgggtgaagacagaggaagttgttgtgaaa
gaagacttacaaaaaccagtacttgatctgaaattaagtggggtcctaactgtcaaagcaggggacaccattaggcttgaggcaggggtt
agaggcaaaccattcccagaagttgcatggaccaaggacaaagacgctacagacttaacaagatcaccaagggtcaagattgatacc
cgtgctgattcatctaaattttctcttactaaagcaaagcgaagtgatgggggtaaatatgtagttacggcaactaacacggctggcagttttg
tggcctatgccactgtcaatgttttagataagcctggtcctgtgagaaatctgaaaattgttgatgtgtccagtgataggtgtactgtttgctggg
atccaccagaagatgatggtggctgtgaaatccaaaattatattctagaaaaatgtgagacaaagcgaatggtttggtctacctattctgcta
ctgtcttgacacctggtactacagtaacacgtctcatagaaggaaatgaatatatttcagagtccgtgcagaaaataaaataggcacagg
gcctccaacagaaagtaaaccagtcatagccaaaaccaagtatgataaacctggtcgccctgatcccccagaagtcactaaagtaagc
aaagaagagatgactgtggtttggaatccacctgaatatgatggtggaaagtctataactggatactttttggagaaaaaggaaaagcatt
caacacgatgggtccctgtcaacaagagtgcaatccctgagagacgtatgaaagtacagaatctcctcccagaccatgaatatcagttcc
gtgtcaaggcagaaaatgaaattggaattggagaaccaagcttgccttcaagaccggtggtggcaaaagaccccatagagccacctg
gtccaccaaccaatttcagagtggttgatacaaccaaacattccataactcttgggtggggaaaaccagtctatgatggtggtgcaccgat
cattggatatgttgtggaaatgagaccaaaaatagcagatgcgtctcctgatgaaggctggaaacggtgtaatgctgcagcacagcttgta
cgcaaggaattcactgttaccagcttggatgaaaaccaggaatatgagttcagggtgtgtgcccaaaaccaagttggtattgggcgccct
gcagagctaaaggaagctatcaaacctaaagaaatactagaacctccggagattgatttggatgccagcatgaggaaactggtcatagt
gagagcaggatgccctattcgtctctttgctatagtgagaggacgaccagcccctaaagtcacttggcgaaaagttggcattgataatgtg
gtcagaaaaggacaagttgatctggttgacactatggccttccttgtcatccccaattctacccgtgatgactcaggaaaatattccttaaca
cttgtgaacccagcaggagaaaaggctgtattcgtaaatgtcagagtattagacactcctgggcctgtgtctgatttaaaagtttcagatgtc
actaaaacatcatgccatgtgtcctgggcccctcctgaaaacgacggtgggagccaagtgacacattatatcgtggagaaacgtgaggc
agacagaaagacatggtcgaccgttaccccagaagttaagaaaacaagcttccatgtaaccaatcttgtccctgggaatgagtattacttc
agagtaactgctgtcaacgaatatggccctggcgtcccaacagatgtcccaaaaccagtgcttgcatcagatcctctaagtgagccggat
cccccaaggaaattagaagtgactgaaatgaccaagaacagtgccaccttagcctggttacctccccctacgtgatggaggtgctaaaat
cgatggctacatcactagttacagagagaagaagagcagcctgcagatcgctggacagagtactcagtggtaaaagatctgagccttgttgt
cactggcctaaaggaaggaaagaaatacaaatttagagtagcggccagaaatgctgttggagtcagtttgccaagagaagctgaagg
agtgtatgaagccaaagaacaactgttgccaccaaagatccttatgccagagcaaataactatcaaagctgggaaaaaactccgaatt
gaagcccatgtgtatggaaagcctcatcccacctgtaaatggaaaaaaggagaagatgaagttgtcacatccagccacctggcagtgc
ataaagcagacagctcttcaattctgatcataaaagatgtgactaggaaagacagtggttactacagcctcacagcagagaacagttctg
ggacagacactcagaaaatcaaagttgtagtcatggatgcccccggcccccctcagcctccattgacatttctgatatagacgctgatgct
tgctccctgtcatggcacatccctctggaggacggaggcagtaacatcaccaattatatagtggagaagtgtgatgtaagccgaggtgact
gggtcacggctctagcttcagtcacaaaaacttcctgcagggttggaaagctgatcccaggccaggagtacatcttccgggtccgtgctga
aaaccgatttggcatttcagagcctctcacatctccaaagatggttgcgcagttcccatttggtgttcctagtgaaccaaagaatgcacgagt
caccaaagtcaacaaggactgtatttttgttgcttgggacagaccagatagtgatggagggagccccattattggttatctgattgaacgca
aggaaagaaacagtttgctgtgggtgaaagccaatgatactcttgtccggtcaactgaatatccttgtgctggccttgtagaaggtcttgagt
attcattcagaatctatgccctaaacaaagctggatccagcccacccagcaaacccacagaatatgtaactgcaagaatgccagttgatc
ctcctgggaaacctgaggttattgatgtcaccaagagtactgtatctctgatctgggctcgtccaaagcatgatggaggcagtaaaattattg
gctatttcgtagaagcttgcaaacttcctggtgataaatgggtacggtgcaatactgcacctcaccagattccccaggaagagtacacagc
tactggcctagaagagaaagctcagtatcaatttagagctattgccaggaccgcggtaaacattagcccaccttctgaaccttctgatcca
gtgactatcctcgcagaaaatgtccctcccaggatagacctgagtgtggctatgaaatctttgcttactgtgaaagctggaactaatgtctgct
tggatgctactgtttttggtaaaccgatgccaacagtttcttggaaaaaagatggcacactgctaaaaccagcagaaggcataaagatgg
ccatgcagcggaatctgtgcaccttggagctattcagcgtgaaccggaaggactcaggagactataccattactgctgaaaattcaagtg
gttctaaatcagccaccattaagcttaaagtgttagataaaccgggtcctccagcatctgttaaaatcaacaaaatgtattcagatcgtgctat
gctttcttgggaaccgcctcttgaagatggaggctcagaaatcaccaactatattgttgacaaacgtgaaacaagcaggcccaactgggc
tcaagtctctgcaactgtgcctatcaccagctgcagcgtggagaaacttatagagggccatgagtatcagttccgtatttgtgctgaaaataa
atatggagtaggcgatccagtcttcactgaaccagcaattgccaaaaacccatatgacccaccaggacgctgtgatcctcctgttattagc
aacataaccaaagatcacatgacagtcagctggaagccaccagcagatgatgggggctcacccatcactggctatttgcttgaaaagc
gggaaacccaggctgttaactggactaaggtcaacagaaaacctattatagaaagaacattaaaagcaacaggtcttcaagaaggtac

cgaatatgagttccgtgttacagctataaataaagctggaccaggcaaacccagtgacgcatccaaggccgcttatgctcgggaccctca
gtatcctcctgcgccaccggctttccctaaagtatatgatacaactcgcagctctgtgagtctatcttggggcaagccagcctatgacggcg
gcagccctatcattggttatctcgttgaagtaaaacgggctgactccgataactgggtgaggtgcaacttaccacagaatctacagaaaac
ccgctttgaggttactggcctgatggaagacacacaatatcaattccgtgtgtatgccgttaataagattggatacagtgaccccagtgatgt
gccagataaacactatcccaaggacatcttaattccacctgagggagaacttgatgcggacttaaggaagacactcatattacgtgctgg
agttactatgagactatatgtaccagtaaaaggacgcccacctccaaagattacttggtctaaaccaaatgtcaatctaagagacaggatt
ggactggacataaagtcaactgactttgacactttcttgcgctgtgaaaatgtgaacaaatatgatgcaggaaaatatatcttaaccctgga
gaacagctgtggtaaaaaggaatataccattgttgtgaaagtgcttgatactcctgggccacctgtcaatgtgactgttaaggaaatatcca
aagactctgcttatgttacctgggagcctcccattattgatggcggaagccccatcataaactatgtggtacaaaaacgtgatgcagagag
gaaatcctggtctacagtgacaactgagtgctccaaaacaagcttcagagtagctaatttggaggagggaaaatcctacttcttccgagtg
tttgctgaaaatgagtatggcattggtgatcccggtgaaactcgtgatgctgtcaaagcttcccaaactcctggaccagttgtggacctgaaa
gtgaggtctgtatctaagtcatcctgtagcattggctggaaaaagcctcacagtgatggtggaagtcggattattggatatgtagttgatttcct
gactgaagaaaataagtggcaacgagttatgaaatccttaagcctacagtactctgcaaaagatttgactgaagggaaggaatatacctt
cagagtgagtgctgagaatgaaaatggagaaggaaccccaagcgaaatcactgttgtggcaagggatgatgttgtggctcctgatcttg
acttaaagggtctacctgatttgtgctacttggctaaagaaaacagcaacttccggcttaagatccccataaaaggcaagccagctccatc
agtctcctggaagaaaggggaagatcctctagcaactgacactagagtcagtgttgagtcatctgcggttaacacaactcttatagtgtac
gattgccaaaaatctgatgctggaaaatacacaatcacacttaagaatgttgctggcaccaaggaaggaactatctccataaaggttgttg
gcaagcctggcatccccactggaccaatcaaatttgatgaagtcacagcagaagccatgaccttaaagtgggctcctccaaaggatgat
ggaggttctgaaatcaccaactatatcctagagaagagggattctgtgaacaacaagtgggtgacgtgcgcctcagctgtccagaaaac
cacctttagagtaaccagacttcatgagggcatggaatataccttcagggtcagtgccgaaaataaatatggtgtaggggaaggcctgaa
atcggagccaattgttgcgagacatccatttgatgtgcctgatgctcccccacctcccaatattgtggatgtcagacacgattcagtatctcta
acttggactgaccccaagaaaactggtggttctccaattacagggtatcatctcgagttcaaggaaagaaacagccttttgtggaagaga
gctaacaagactccgataaggatgagagactttaaagtgacaggattaactgaaggtcttgaatatgaattccgagttatggcaatcaattt
agcaggtgtgggcaagccaagcctaccatcagagcctgttgtggcactggacccaattgatcctcctggaaaacctgaggttattaacat
aacaaggaattcagtgactctcatttggactgaacctaaatatgacggtggtcataagttaactggatatatagtggagaagcgagatcta
ccttcgaagtcttggatgaaagccaaccatgttaatgtcccagaatgtgcctttactgtaactgaccttgttgagggtggaaaatatgaattca
gaattagagcaaagaatacagcaggtgctatcagtgctccatcagaaagtacagaaaccattatttgcaaggatgaatacgaggcacc
aacaattgtccttgatcccacaataaaagatgggctaacaattaaagcaggggataccattgttttgaatgccattagcattcttggcaaac
cccttccaaaatcaagttggtccaaggcaggaaaagacattagaccatcagatatcactcagataacttcaaccccaacatcttccatgct
tactatcaagtatgccactagaaaagatgcgggtgaatataccatcactgctaccaatccttttggcacgaaggtggaacatgtgaaggta
acagtccttgatgtacctggtcccccaggtcctgttgaaatcagtaatgtttctgctgaaaaagcaacacttacatggacacctcccttggaa
gatggcggctcaccaattaagtcctatatacttgaaaagagagaaaccagccgacttttgtggacagtggtttctgaagatattcagtcttgc
aggcatgtggcaaccaaacttatccaaggaaatgagtacatcttccgggtctcagctgtaaaccactatggcaaaggagaacctgtaca
gtctgaacctgtcaaaatggtagacagatttggtccccctggccctcctgaaaaaccagaggtatcaaatgtcactaagaacactgccact
gtcagctggaaaaggccagtggatgatggtggcagcgaaattacaggatatcatgtagaaaggagagagaaaagaaaagcctgcgatg
ggtgagagcaataaaaacaccagtttccgatctcaggtgcaaagtaacaggactgcaagaaggaagcacctacgaattccgtgtcagt
gcagaaaacagagcaggaattggtccacccagtgaggcttcagattctgttctgatgaaagatgcagcatatcctccaggaccaccttca
aatccgcatgtcactgatactaccaagaaatctgcttctttggcatggggcaagcctcattatgatggtggacttgaaatcactggctatgtcg
tggagcatcaaaaagtaggagacgaggcctggataaaagataccacaggaaccgccctcagaatcactcagttcgttgttcctgatcttc
agactaaagaaaaatacaacttcagaatcagtgccatcaacgatgcaggtgttggggagccagcggtgattccagatgttgaaatcgta
gaacgggagatggctcctgattttgaactagatgccgagcttcgaagaacacttgttgttagagcaggactcagtattaggatatttgtgcca
attaaaggtcgtcctgctcctgaagtgacatggaccaaagataacatcaacctgaaaaaccgagccaacattgaaaatacggaatcattt
actcttctgattatcccagaatgtaacagatatgataccggtaaatttgtcatgaccattgaaaacccggctgggaagaaaagtggctttgtg
aacgtcagagtcttggacacgccaggcccagtcctcaacctgcggcctacagacatcacaaaggacagtgtcaccctgcactgggacc
tccctctgatagatggaggctcacgtataacaaactacattgtagagaaacgtgaagcaacacggaaatcttattccacagccaccacta
agtgccataaatgcacatataaagttaccggcttgtctgaagggtgtgaatatttcttcagagtgatggcagagaatgaatatggaattggtg
agccaacagaaactacagagcccgtaaaagcctctgaagcaccatctccaccagacagccttaacatcatggacataactaagagca
ccgtcagcctggcatggcctaagcccaaacacgatggtggcagcaagatcactggctatgtgattgaagcccaaagaaaaggctctga
ccagtggacccacatcacaaccgtgaaagggttagaatgtgttgtgaggaatctaactgaaggagaggaatataccttccaagtgatgg
cagtgaacagcgcggggagaagtgcccctagagaaagcagacccgtcattgtcaaggagcagacaatgcttccagagctggatctcc
gtggcatctatcagaaactggtcattgccaaagctggtgacaacatcaaagttgaaattccagtgctcggtcgaccgaagcccacagtga
catggaaaaaaggagaccaaattcttaaacagacacagagagttaatttgaaaccacagcgacttcaaccattttaaatatcaatgagt
gtgtcagaagtgatagtgggccctatccattaacagcaaggaacattgtaggagaggttggtgatgtcatcaccattcaagtccatgatatc
ccagggccacctactggaccaatcaaatttgatgaagtttcatctgattttgtaaccttctcttgggacccacctgagaacgatggtggtgtac
caataagcaactatgtagtggaaatgcggcagactgacagtactacctgggttgagttagcaaccaccgttatacgtactacctataaagc
cacccgccttactactggattagagtatcagttccgtgtaaaagctcagaatagatatggagttggaccaggcatcacatcagcatgcata
gttgccaactatccatttaaggttcctggacctcctggtacccctcaggtaactgcagttaccaaggattcaatgacaattagctggcatgag

ccactttctgatggtggaagccccattttaggatatcatgttgaaagaaaagaacgaaatggtattctctggcagactgtgagcaaagcttta
gtaccaggcaacattttcaaatcaagtggacttacagatggtattgcttatgagttccgggtgattgcagaaaacatggcaggcaaaagta
agccaagcaagccatcagaacctatgttggctctggatcccattgacccacctggaaaaccagtacctctaaatattacaagacacaca
gtaacacttaaatgggctaagcctgaatatactgggggctttaaaattaccagttatatcgttgaaaagagagaccttcctaatggacggtg
gctgaaggccaacttcagcaacattttggagaatgaatttacagtcagtggcctaacagaagatgctgcatatgaattccgtgtgatcgcc
aaaaatgctgcaggtgccatcagtccaccatctgagccatctgatgctatcacttgcagggatgatgttgaggcaccaaagataaaggtg
gatgttaaatttaaggacacggttatattaaaagcaggtgaagcattcagactggaagctgatgtttcaggccgcccacctccaacaatgg
aatggagcaaagatggaaaagagctggaaggcacagcaaagttagaaataaaaattgcagatttctctactaatctggtaaacaaaga
ttcaacaagaagggatagtggtgcctatacccttacagcgactaatcctggtggctttgctaaacacattttcaatgtcaaagttcttgacaga
ccaggcccacctgaaggacctttggctgtaactgaagtgacatcagaaaagtgtgtactatcatggttccctccactggatgatggaggtg
ccaaaattgatcattacatagtacagaaacgtgaaaccagcagattggcatggacaaatgtagcctcagaagtccaagtaacaaagct
aaaggtcactaaactcttgaaaggcaatgaatacatattccgtgtcatggctgtaaataaatatggagtgggagagccactggaatcaga
gcctgtgcttgcagtgaatccttatggacccccctgatccgcccaaaaaccctgaagtgacaactattactaaagattcgatggttgtctgctg
gggacatcctgattctgatggtggaagtgaaatcatcaattatattgtggaacggcgtgataaagctggccaacgctggattaaatgcaac
aaaaaaactcttactgatttaagatataaagtgtctggactgacagaaggacatgaatatgagttcaggattatggctgaaaatgctgctgg
aattagtgcaccaagtcctaccagtccattttacaaggcttgtgacactgtgtttaaacctggaccaccaggtaacccacgtgttctggatac
aagcagatcatccatttcaatcgcttggaataaacctatctatgatggtggttcagaaatcactgggtatatggttgagattgccctgccaga
ggaagatgaatggcagattgtcactccaccagcaggactcaaggcaacttcgtatactatcactggcctcacagagaatcaggaatata
agatccgcatctatgccatgaattccgaaggacttggggaacctgcccttgttcctggaactccaaaggctgaagacagaatgctgcctcc
agaaattgaactggatgctgacctgcgcaaagttgttactataagggcctgctgcaccctgagactttttgttcccatcaaaggaaggcctg
cacctgaggtgaagtgggcccgggaccatggagaatctttagataaagctagcatcgaatccacaagctcttacaccctgcttattgttgg
aaatgtaaacagatttgacagtggcaaatatatactaactgtagaaaatagttcaggcagcaagtctgcatttgtcaatgttagagttctcga
tacaccaggcccccccacaggatctgaaggtaaaagaggtcactaagacatctgtcacactcacatgggacccacctctccttgatggag
gttcaaaaatcaagaactatattgttgaaaagcgggaatcaacaagaaaagcatattcaactgttgcaacaaactgccacaagacttcct
ggaaggtagaccagcttcaagaaggctgtagctactatttcagggttctcgcagaaaatgaatatggcattgggctgcctgctgaaaccg
cagaatctgtgaaagcatcagaacgacctcttcctccaggaaaaataactttgatggatgtcacaagaaatagtgtgtcactctcttggga
gaaaccagagcatgatggaggcagccgaattctaggctacattgtggagatgcagaccaaaggcagtgacaaatgggccacgtgtgc
cacagtcaaggtcactgaagccactatcactggattaattcagggtgaagaatactctttccgtgtttcagctcagaatgaaaagggcatca
gtgatcctagacaactgagtgtgccagtgatcgccaaagatcttgtcattccaccagccttcaaactcctgttcaatactttcactgtactggc
aggtgaagacctaaaagttgatgttccattcattggccgccctaccccagctgtaacctggcataaagataatgtaccactgaagcagac
aactagagtaaatgcagagagcacagaaaataattcactactgacaataaaggacgcctgccgagaagatgttggccattatgtggtta
aactgactaactcagctggtgaagctattgaaacccttaatgttatcgttcttgacaaaccagggcctccaactggaccagttaaaatggat
gaagtgacagctgatagtattactctttcctggggcccacccaagtatgatggtggaagttctatcaataattacattgttgagaaacgggac
acttccacaaccacctggcaaattgtatcagctacagttgcaaggacaacaataaaggcttgcagactgaagactggatgtgaatatcag
tttagaattgcagctgaaaacagatatggaagagtacctacctcaattcagagcctactgtagcccaatatccattcaaagttcctggtcct
cctggcactccagttgtcacactgtcctccagggacagcatggaagtacaatggaatgagccaatcagtgatggaggaagtagagtcatt
ggctatcatctagaacgcaaggaaagaaatagcatcctctgggttaagttgaataaaacacctattcctcaaaccaagtttaagacaact
ggccttgaagaaggtgttgaatatgaatttagagtctctgcagagaacatcgtgggcattggcaagccgagtaaagtatcagaatgttatgt
ggctcgtgacccatgtgatccaccaggacggccagaggcaatcattgtcacaaggaattctgtgactcttcagtggaagaaacccaccta
tgacggtggaagcaagatcactggttatattgttgagaagaaagaattacctgagggccgttggatgaaagccagttttacaaatattattg
acactcattttgaagtaactggcctagttgaagatcacagatatgagttccgggttatagcccgaaatgccgcaggagtgtttagtgagcctt
cagaaagcacaggagcaataacagctagagatgaggtagatccaccacgaataagtatggatccaaaatacaaagacacaatcgtg
gttcatgctggtgaatcattcaaggttgatgcagatatttatggcaaaccaataccaaccattcagtggataaaaggtgatcaggagctttca
aacacagctcgattagaaataaagagcaccgactttgccaccagtctcagtgtaaaagatgcagtacgtgtcgacagtggaaattacat
actgaaggccaaaaatgttgcaggagaaagatcagttactgtgaatgtcaaggttcttgacagaccagggccacctgaaggacctgttgt
tatctcaggagttacagcagaaaaatgcacactagcttggaaacccccacttcaggatggtgggagtgacatcataaattatattgtggaa
aggagagaaaccagccgcttagtttggactgtggttgatgccaatgtgcagactctcagctgcaaggttactaagcttcttgaaggcaatg
aatatactttccgtataatggcagtaaacaaatatggtgttggtgaacctcttgaatctgagccagtagttgccaagaatccatttgtagtacc
agatgcaccaaaagctccagaagtcacaacagtgaccaaggactcaatgattgttgtatgggaaagaccagcatctgatggtggtagtg
aaattcttggatatgttcttgagaaacgggataaagaaggcattagatggacaagatgccataagcgtctgattggagagttgcgcctgag
agtaactggactcatagaaaatcacgattatgagttcagagtttctgctgagaatgctgctggacttagtgaaccaagccctccttctgcttac
caaaaggcttgtgatcctatttataaaccaggacccccaaacaaccccaaagtcatagacataaccagatcttcagtattcctttcttggag
caaaccaatatatgatggtggctgtgaaattcaaggatacattgttgaaaaatgtgatgtgagtgttggtgaatggacaatgtgcactccac
caacaggaattaataaaacaaacatagaagtagagaagctgttggaaaagcatgaatacaacttccgtatctgtgctattaataaagctg
gagttggagaacatgctgacgtccctggacctattatagttgaagaaaaattagaagcaccagacattgatcttgacctagaactaagga
aaatcataaatataagggcaggtggctccttaaggttatttgttcctataaaaggtcgtcctacaccagaagttaaatggggaaaggtggat
ggtgaaatccgagatgcagctataattgatgtcactagcagtttcacctctcttgttcttgacaatgtcaaccgatatgatagtggaaaatata

cgcttacattagaaaacagcagtggaacaaagtctgcctttgttactgtgagagttctggacacgccaagtccacctgttaacctgaaagtc
acagaaatcaccaaagactcagtatcaattacatgggaacctcctttgttggatgggggatccaaaataaaaaattacattgttgagaaac
gtgaagccacaagaaaatcatatgctgctgttgtaactaactgccataagaattcttggaaaatcgatcagctccaagaaggttgcagttat
tactttagagtcacagctgagaatgagtatggtattggccttcctgcccagactgctgatccaattaaggttgcagaagtgccacaacctcct
ggaaaaataactgtggatgatgtcaccagaaacagtgtctctctgagttggacaaaacctgaacatgatggtggcagtaaaatcattcagt
atattgtggaaatgcaagctaaacacagtgagaaatggtcagagtgtgctcgagtaaagtctcttcaggcagtaattaccaacctaactca
aggggaagaatatctttttagagttgttgctgtaaatgaaaaggggagaagtgatcctcggtcccttgcagttccaatagttgccaaagatct
ggtaattgagccagatgtaaaacctgcattcagtagttacagtgtacaggttggccaagatttgaaaatagaagtgccaatttctggacgtc
ctaagccaaccattacctggactaaagatggtctcccactgaagcagaccacaagaatcaatgttaccgattcactggatctcaccacac
tcagtattaaagaaactcataaggatgatggtggacaatatggaatcacagttgccaatgttgttggtcagaagacagcatccatcgaaat
tgtaactctagataaacctgatcctccaaaaggacctgttaaatttgatgacgtcagtgctgaaagtattacattatcttggaaccctccattat
atacagggggctgccaaatcaccaactacattgttcagaaaagagatacaaccaccacagtatgggatgttgtttctgctactgttgctaga
actacactcaaagtgaccaaactgaaaactggtacagaataccaatttagaatatttgccgaaaacagatatggacaaagctttgcctta
gagtctgatccaattgtagctcaatatccctacaaagaaccaggccctccaggtacaccatttgccacagccatttccaaagactccatgg
tcatacagtggcatgaaccagtcaacaatggtggaagccccgtcataggttaccacctggagagaaaagaaagaaacagtattttgtgg
acaaaggtcaacaaaactattattcatgacacccaattcaaagcacagaatcttgaagaaggcattgaatatgaattcagagtgtatgctg
aaaatattgttggtgtaggcaaagcaagcaagaattctgaatgctatgtagccagagatccctgtgacccaccaggaaccccagaacca
ataatggttaaaagaaatgaaatcactttacagtggaccaaacctgtgtatgatggtggaagtatgattacaggctacattgtagagaaac
gtgatttgcctgatggtcgttggatgaaagctagctttacaaatgtcattgaaactcaatttactgtgtcaggtcttactgaagatcaaagatat
gaattcagagtcattgcaaagaatgcagctggtgcaataagtaaaccctctgacagtactggaccaataactgccaaggatgaggttga
actcccaagaatttcaatggatccaaaattcagagacacaattgtggtaaatgctggagaaacattcagacttgaggctgatgtccatgga
aagcccctacctaccattgagtggttaagaggagataaggaaattgaagaatctgctagatgtgaaataaagaacacagatttcaaggc
tttacttattgtaaaagatgcaattagaattgatggtgggcagtatattttaagagcttccaatgttgcaggttctaagtcattcccagtaaatgta
aaagtattagatagaccaggacctccagaagggccagtccaggttactggagtcacttctgaaaaatgctctttaacatggtctccaccac
ttcaagatggtggcagtgacatttctcactatgttgttgaaaagcgagaaaccagtcgacttgcctggactgttgttgcttcagaagttgtgac
caattctctgaaagttaccaaactcttagaaggtaatgaatatgttttccgtataatggctgtcaacaaatatggtgttggagagcctttggaat
ctgcaccagtactaatgaaaaatccatttgtgcttcctggaccaccaaaaagcttggaagtcacaaatattgccaaagactccatgaccgt
ctgttggaaccgtccagatagtgatggtggaagtgagattattggttacattgtagagaaaagagacagaagtggcattcgatggataaa
atgtaataaacgccgcattacagatttgcgtctaagagtgacaggattaacagaagatcatgagtatgaattcagggtctctgcagaaaat
gctgctggagttggggaaccaagtccagctacagtttattataaagcctgtgatcctgtgttcaaacctggcccacctaccaatgcacacatt
gtagacaccactaaaaattcaatcacacttgcctggggtaaacccatctatgatggcggcagtgagatcttgggatatgtagtagaaatct
gtaaagcagatgaagaagaatggcaaatagttactccacagactggcctgagagtcactcgatttgaaatttcaaaactcactgaacac
caagagtataaaatacgagtctgtgccctcaacaaagttggtttaggtgaggctacatcagttcctggtactgtgaaaccagaagataaac
ttgaagcacctgaacttgaccttgactccgaattaagaaaaggaattgttgtaagagctggtggatctgccagaattcacattccattcaaa
ggtcgtccaacgcctgagatcacttggtctcgagaggaaggtgaattcacagataaggtccaaattgaaaagggagtaaactatacccca
actatcaatagataactgtgatagaaatgatgctggaaaatacattcttaagttggaaaacagcagtggatcaaagtctgcttttgtaactgt
gaaagttcttgacactccaggaccaccacagaatttggcagtcaaagaagtgagaaaagattctgccttcctggtatgggagccacccat
cattgatggaggggcaaaggtcaagaactatgtgattgacaaacgtgagtcaaccagaaaagcgtatgctaatgtgagtagtaaatgca
gcaaaacaagttttaaagtggaaaaccttacagaaggagccatttattacttcagagtcatggctgaaaatgaatttggagttggtgttcca
gtggaaactgttgatgccgtgaaagctgctgaacctccttccccaccaggaaaggttacactcactgatgtgtcccagaccagtgcatcac
ttatgtgggagaaacctgaacatgatggcggtagcagagtcctggggtacgttgttgaaatgcagcccaaaggaactgaaaaatggag
cattgtggctgaatccaaagtctgtaatgcagttgttactggtttgagttctggacaagaatatcagttccgtgtcaaggcttataatgagaaa
ggaaaaagcgatccaagagtgttgggtgttcctgtcatagccaaggacttgactatacagcctagtttaaagttaccatttaacacatatagt
atccaagctggagaagatcttaaaatagaaattccagttataggccgaccaagacctaacatttcttgggtcaaagatggtgagcctctta
aacagacaacaagagtaaacgttgaagaaacagctacctcaactgttttgcacattaaagaaggtaacaaagatgactttggaaaatac
accgtaacggcaacaaatagtgcaggcacagcaacagaaaatctcagtgttatcgttttagaaaagcctggacctccagttggcccagtt
cggtttgatgaagttagtgcagactttgtagtcatatcttgggaacctccagcctatactggtggctgccaaataagcaactacattgtagag
aagcgagatacaaccaccaccacttggcacatggtatcagcaacagttgcaagaacaacaattaaaataaccaaactgaaaacagg
cacggagtaccagtttagaatttttgctgaaaacaggtatggaaaaagtgccccactggattctaaggcagttattgtacaatatccatttaa
agaacctggaccacctggaactccttttgtgacatcaatctcaaaagatcagatgcttgtgcaatggcatgagccagtgaatgatggaggc
accaaaattattggctaccatcttgaacagaaagaaaagaacagtattttatgggtcaagttaaataagacccccattcaggacaccaaa
ttcaaaacaactgggcttgatgagggccttgagtatgagttcaaagtttctgctgaaaatattgttggcattggcaagcctagcaaagtgtca
gaatgctttgttgctcgtgatccatgtgacccacctggtcgccctgaagccattgttattacaagaaacaatgtcacactgaaatggaagaa
acctgcctatgatggtggtagcaaaataacaggttatattgtagaaaagaaagatctacctgatggccgctggatgaaagccagctttacc
aacgtattagaaactgaatttacagtgagtggacttgtagaagaccaaagatatgaatttagagtaattgcaagaaatgcagctggaaact
ttagtgaaccatctgatagtagtggtgccattactgcaagagatgaaattgatgcaccaaatgcctctctggatccaaaatataaagatgtc
atcgttgttcatgcaggagagacttttgttcttgaagccgacatccgtggcaaacctatacctgatgttgtttggtcaaaagatggaaaagaa

cttgaagaaacagctgctagaatggaaattaaatctactattcagaaaacaactcttgttgtcaaagactgtatacggactgatggaggac
aatatattctgaaactcagcaatgttggtggtacaaagtctatacccatcactgtaaaggtacttgacaggccagggcctcctgaagggcct
ctgaaagttactggagttactgcggaaaaatgttacctggcatggaacccacctttgcaagatggtggtgctaatatttcacattacatcattg
aaaagagggagacaagccgactctcttggacccaggtttcaactgaggtacaggcccttaactacaaagttactaaacttcttcctggtaa
tgagtacattttccgtgtcatggctgtgaataaatatggaattggagagcccttggaatctgggcctgttacggcctgtaatccttataagcca
ccaggtcctccctcaacacctgaagtctcagcaatcaccaaagattctatggtagtaacatgggcacgcccagtagacgacggaggtac
cgaaattgagggctacattcttgaaaaacgagataaggaaggcgttagatggaccaagtgcaacaagaaaacattaacggatctgcg
gctcagggtaactggtcttaccgaaggccattcctatgaattcagagttgctgctgaaaatgcagctggtgtgggagaacctagtgagcca
tctgttttctaccgtgcgtgtgatgccttgtatccaccaggtcccccaagcaatccaaaagtgacggacacttccagatcttctgtctccctggc
atggagtaagccaatttatgatggtggcgcacctgttaaaggctatgttgtagaggtcaaagaagctgctgcggatgaatggacaacctgc
actccaccaacaggattacaaggaaagcagttcacagtgaccaagcttaaagaaaacactgaatataacttccgtatttgtgccatcaatt
ctgaaggtgtaggtgaacctgcaactctacctggctcagtggttgctcaggagaggatagagccaccagaaatagaactcgatgctgatc
tcagaaaggtggtcgttctgcgtgcaagtgctactttacgcttatttgtcactatcaaaggtcgaccagaacccgaagttaaatggaaaag
gcagaaggcattctcactgacagggctcagatagaggtgaccagctcatttacaatgttggtgattgataatgttaccagatttgacagtggt
cggtataatctgacattagaaaataatagtggctccaaaacagcttttgttaacgtcagagttcttgactcaccaagtgcccctgtgaatttga
ccataagagaagtgaagaaagactcagtgacgttgtcctgggaaccaccacttattgatggtggagctaagattacaaactacattgtcg
aaaaacgagaaactacaagaaaagcctatgctaccattacaaataattgcactaaaactactttcagaattgaaaatctacaagaagga
tgttcttactacttccgagtcttggcttccaatgaatatgggattggtttgccagctgaaacaacagaacccgttaaagtgtctgaaccacccc
tcccacctggaagagtaactcttgttgatgtgacccgtaatacagctacaattaagtgggagaaaccagaaagtgatggtggcagcaaa
attactggttatgtggttgaaatgcagactaaagggagtgaaaagtggagcacctgcacacaagttaagactctagaagcaactatatct
ggcttaactgcaggagaagagtatgtcttcagggtagctgcagttaacgaaaagggaagaagtgatccaagacaacttggagtgccag
taattgcaagggatattgaaataaagccttcagttgagcttcctttccatactttcaatgtaaaggctagagaacaacttaagattgatgtgcc
attcaaaggaagacctcaagctactgtgaactggagaaaagatggtcagactcttaaagagacaactagagtcaatgtttcttcttcaaag
actgtaacatcactatctattaaggaagcttcaaaggaagatgttggaacttatgaattatgtgtttcaaacagtgctggatccataacagttc
ctattactataattgtccttgacagaccaggacctccaggtcctatacgtattgatgaggttagttgtgacagcataaccatttcttggaatcctc
cagaatatgatggtggctgccaaattagcaattacattgttgaaaagaaagaaaccacctctacaacatggcacatagtttcacaagcag
ttgcaagaacatccattaaaatagttcgcctgacaacaggaagtgagtatcagttccgtgtttgtgcagaaaaccgctatggaaagagctc
ctacagtgaatcttcagctgttgttgcagagtatccattcagtcccccaggtcctcctggtactcctaaagttgtgcatgccacaaaatctacc
atgcttgtaacctggcaagtgccagttaatgatggaggaagtcgagtaattggctatcatcttgagtataaagaaagaagcagcattctttg
gtcaaaagcaaataaaatcctcattgctgatactcaaatgaaagtctccggccttgatgaaggactgatgtatgagtatcgtgtatatgctga
aaatattgctggaattggtaaatgcagtaaatcttgtgaaccagtccctgcaagagatccttgtgaccctcctggacaacctgaagtcacaa
atatcacaagaaaatcagtgtcacttaaatggtctaaaccacattatgatggtggagctaagatcacaggatacattgttgaacgcagaga
actaccagatggccggtggctgaagtgcaattatactaatatacaagaaacatactttgaagtaactgaacttactgaagatcagcgttatg
aattccgggtttttgcaaggaatgctgctgactcagttagtgagccatctgaatccactgggcctattatagttaaagatgatgttgagcctcc
aagagttatgatggatgtcaagttccgagacgttattgttgtcaaagctggagaggtccttaagataaatgcagacattgcagggcgacctc
tgccagtaatttcctgggccaaggatggtatagaaattgaagaaagagcaagaacagaaatcatctcaacagacaatcatactttgttaa
cagttaaagactgtataagacgagacactgggcaatatgtactaacactgaagaatgttgccggcactcggtctgtggccgttaattgcaa
agtacttgataagcctggtccaccagcaggaccacttgaaataaatggcctcactgctgagaaatgctctctttcctggggacgtccccaa
gaagatggtggtgcagatatcgactattacatcgtagaaaaacgtgaaacaagccaccttgcatggacaatatgtgaaggagagttaca
gatgacatcctgtaaagtaaccaagttactcaaaggcaatgaatatatatttagagtaactggtgttaataaatatggtgttggtgagcccct
agagagtgtagctataaaggcactagatccatttacagttccaagtccacccacgtctttggaaattacttctgtgaccaaagaatctatgac
actttgctggtcaagacccgagagtgatggaggtagtgaaatatctggatatataattgaaaggcgagagaaaaatagcctaagatgggt
gcgtgtaaacaaaaaaccagtttatgatctaagagtgaaatcaacaggacttcgggaaggatgtgaatatgaatatcgtgtttatgcagaa
aatgctgctggcctaagtcttccaagtgaaacctctcccttaattagggcagaagatccagtgttcctaccatctcctccatccaaacccaaa
attgtggactcaggcaagacaactataactattgcctgggttaagccgctgtttgatggtggggccccgataactggatatactgtagaata
caaaaaatctgatgacactgactggaaaacttccattcagagcttacgagggacagaatatacaataagcggactaacaacaggagct
gaatatgttttcagagtaaaatctgtcaataaggttggtgctagtgaccccagtgatagctctgaccctcagatagcaaaggaaagagaag
aagaacctttatttgatattgacagtgaaatgaggaagaccttgattgtcaaggctggtgcctcatttaccatgactgtgcctttccgaggaag
accagtacccaatgtcttgtggagtaagccagacactgacctccgtactagagcttatgttgataccacagactcccgtacatcactgacc
attgaaaatgccaacagaaatgactctggaaagtacacattaacaattcagaatgttttgagtgctgcttcactgaccttagttgtcaaagtttt
agataccccaggtcctccaaccaacattactgtgcaagatgtaaccaaagagtctgcagtgttatcctgggatgttcctgaaaacgatggt
ggagcaccagtgaagaattaccacatagaaaaacgtgaggccagcaagaaagcatgggtctctgtgaccaacaactgtaaccgcctc
tcctacaaagttaccaatttacaagaaggagctatctattacttcagagtctctggagaaaatgagtttggtgttggtataccagctgaaaca
aaggaaggagtaaaaataacagaaaaaccaagcccacctgaaaaacttggagtaacaagtatatccaaagacagtgtttccctgacc
tggctgaagcctgaacatgatggcggaagcagaattgtacactatgtcgttgaagcactagaaaaaggacagaaaaactgggttaaat
gtgcagtggcaaagtcaacccatcacgttgtttccggtctgagagagaattctgaatactttttccgagtgtttgctgaaaatcaagctggcct
gagtgacccgagagagcttctgcttcctgttcttattaaggagcaactagaaccacctgaaattgatatgaagaatttcccaagtcacactgt

atatgttagagctggttcaaaccttaaagttgacattccaatctctggaaaaccacttcccaaagtgaccttatcaagagatggtgtccccctt
aaggcaaccatgagatttaataccgaaattactgctgagaacctgaccatcaatctcaaagaaagtgttacagctgacgctgggagatat
gaaatcactgctgccaactccagtggtacaaccaaagctttcattaacattgttgtgctagacaggcctggtcctccaactggccctgttgtta
ttagtgatataactgaagaaagtgtgactctcaaatgggagccacctaagtatgacggtggaagtcaagttaccaactacattctactcaa
aagagaaacaagtactgcagtgtggactgaagtgtctgcaacagttgcaagaaccatgatgaaagtcatgaaactgaccacaggaga
agaataccaattccgcatcaaggcagaaaaccgctttggcatcagtgatcatatagattcagcttgtgtgactgtcaaactaccatacaca
acacctggaccaccatctacaccatgggtcactaatgttactcgagaaagcatcactgtgggctggcatgaaccagtgtcaaatggagg
cagtgcagtcgtaggctatcacctggaaatgaaagacagaaacagtattttatggcaaaaagccaacaaactggtcatccgcacaactc
acttcaaagtcacaacaatcagtgctggacttatttatgaattcagggtgtatgcagaaaatgctgctggagttggaaaacctagccatcctt
ctgaaccagtcttggcaattgatgcttgtgaacccccaagaaatgttcgtatcactgatatttcaaagaactctgtcagcctttcatggcaaca
accagctttcgatggaggtagcaagattacaggctacattgttgagagacgtgaccttccagatggcagatggaccaaggccagcttcac
caatgttactgaaactcaattcatcatctctggcttgactcagaattcccagtatgaattccgtgtctttgctaggaatgctgttggttccattagc
aatccatctgaggttgtagggcccattacttgcatcgattcttatggtggtcctgtaattgatttgcctctagaatatacagaagttgtcaaatac
agagcaggtacatctgtgaagctcagagctggcatttctggcaaacctgcgcctactattgagtggtataaagatgataaagaattacaaa
ccaatgcactggtgtgtgttgaaaataccacggacctcgcatctatactcatcaaagatgccgatcgccttaatagtggatgctatgaattaa
aactaaggaatgccatgggctcagcctcagccaccatcagagtacagatccttgacaaaccaggcccacctggtggaccaattgaattt
aagactgtaactgctgagaagatcacccttctctggcggcctccagctgatgatggtggtgcaaaaatcactcactacattgtggaaaagc
gtgagacaagccgcgttgtgtggtctatggtgtctgaacatttggaagagtgcatcattacaaccaccaaaattatcaaaggaaatgaata
catcttccgggtccgagccgtgaacaaatatggaattggcgagccactggaatctgattccgttgtagccaagaacgcatttgttacacctg
ggccaccaggcataccagaagtgacaaagattaccaagaattcgatgactgttgtatggagcaggccaattgcagatggcggtagtgat
ataagtggctatttccttgaaaaacgagacaagaagagcctaggatggtttaaagtactaaaagagactatccgtgacaccagacaaaa
agtaacaggactcacagaaaacagtgactatcaatacagagtttgtgctgtaaacgctgctggacagggtccattttctgaaccatctgaa
ttctacaaagctgctgatcctattgatcctccaggtccacctgctaagataagaatcgcagattcaaccaagtcatccatcacccttggctgg
agtaagcctgtctatgatgggggcagtgctgttactgggtatgttgtcgagataagacaaggagaggaagaggaatggactactgtctcta
ccaaaggagaggtcagaactacagaatatgtggtatccaacctgaaacctggagtcaattactacttccgggtatctgctgtaaactgtgct
ggacaaggagaacctatagaaatgaatgaacctgtacaagctaaagatatacttgaggcaccagagattgacctggatgtggctctcag
aacttctgttattgccaaagctggtgaagatgtacaagtgttgattccctttaaaggcagacctccacctactgtcacatggagaaaagatg
agaagaatcttggcagtgatgccagatacagcattgaaaacactgattcatcctcattactcaccattcctcaagttactcgcaatgataca
ggaaaatatattctcacaatagaaaatggagttggtgaacctaagtcttcaactgtgagtgttaaagtgcttgacacaccagctgcctgcca
gaaactacaggttaaacatgtttctcgaggcacagtcactttgctctgggatcctcctctcattgatggaggatctccaataattaattatgtcat
tgaaaagagagatgccaccaagagaacatggtctgtcgtgtcacacaaatgttctagcacatccttcaagctaatagatttgtcggagaa
gactccattcttcttcagagttcttgcagaaaatgaaattggaattggggaaccctgtgaaactacagagccagtgaaggctgctgaagta
ccagctcctatacgtgatctctcaatgaaagactcaacaaagacatctgtcatcctcagctggaccaaacctgactttgatggtggtagcgt
catcacagaatatgttgtagaaaggaaaggtaaaggtgaacagacgtggtcccacgctggcataagtaagacatgtgaaattgaggtta
gccaacttaaggagcagtcagtcctggagttcagagtgtttgccaaaaatgagaaaggactgagtgatcctgtcactattggccaattac
agtgaaagaacttattattacacctgaagttgacctgtcagatatccctggggcacaagtcactgtgagaattgggcacaatgtgcaccttg
aattaccttataagggaaaacccaaaccatccatcagttggctgaaagatggcttgccactgaaagaaagtgaatttgttcgcttcagtaa
aactgaaaacaaaattactttgagtattaagaatgccaagaaggagcatggaggaaaatacactgttattcttgataatgcagtgtgtaga
attgcagtccccattacagtcatcacccttggcccaccatcaaagcccaaaggacccattcgatttgatgaaatcaaggctgatagtgtcat
cctgtcatgggatgtacctgaagataatggaggaggagaaattacttgttacagcatcgagaagcgggaaacttcacaaactaactgga
agatggtgtgttcaagtgttgccagaacgactttcaaagttcctaatctagtcaaagatgctgagtaccagtttagagtgagagcagaaaac
agatacggagtcagccaaccacttgtctcaagcattattgtggcaaaacaccagttcaggattcctggtcccccaggaaagccagttatat
acaatgtgacttctgatggcatgtcactaacttgggatgctccagtttatgatggtggttcagaagttactggattccatgttgaaaagaaaga
aagaaatagcatcctctggcaaaaagttaatacatcaccaatctctggaagagaatatagagccactggactggtagaaggtctggatta
ccaattccgtgtatatgctgaaaattctgctggcctaagctcacctagtgacccaagcaaatttaccttagctgtttctccagtagacccacct
ggcactcctgactacattgatgtcacccgggaaaccatcacacttaaatggaacccaccattgcgtgatggaggcagtaagattgtgggc
tatagcattgagaaacggcaaggaaatgaacgctgggtgagatgcaactttactgacgtcagtgaatgtcagtacacagttacaggactc
agtcctggggatcgctatgagttcagaataattgcaagaaatgctgttggaactataagcccgccctcacagtcttctggcattattatgaca
agagatgaaaatgttccaccaatagtagagtttggccctgaatactttgatggtctcattattaagtccggagagagccttagaattaaagctt
tggtacaaggaagaccagtgcctcgagtaacttggttcaaagatggagtggaaatcgaaaagaggatgaatatggaaataaccgacgt
acttggatccaccagcctatttgttagagatgctactcgggaccatcgtggtgtatacacagtggaagccaaaaatgcatctggttctgcaa
aagcagaaattaaagtgaaagtacaagatacaccaggaaaagtagttgggccaataagattcaccaatattactggggagaagatga
ctctgtggtgggatgccccactcaatgacggttgtgctcccataacccactacatcattgaaaaacgggaaaccagcagacttgcctggg
cactaattgaggataaatgtgaagcccaaagttacactgccattaaactaataaacggcaatgaataccaattccgtgtttctgcagttaac
aagtttggtgttggcaggccacttgattctgatccagtggttgctcaaatacaatatactgttcctgatgcccctggcattccagaacctagca
acataacaggcaacagcattaccctgacatgggcaaggccagaatcagatggtggcagtgaaattcaacagtatatccttgaaagaag
agaaaagaaaagcacaagatgggtaaaagtgatcagcaaacgaccaatctctgaaacaagattcaaagtcactggtctgacagaag

gcaatgagtatgaattccatgtcatggctgaaaatgctgcaggagttggacctgcaagtggcatctcaagactcattaaatgtagagagcc
cgtcaacccaccaggtcctcccacagtggtcaaagtaacagacacatcaaagacaactgtgagcttagaatggtccaaaccagtgtttg
atggtggcatggaaataattgggtatattattgaaatgtgtaaggccgacttaggagactggcacaaggtgaatgcagaggcatgtgtga
aaacaagatatacagtcactgatctacaagcaggtgaagaatacaaattccgagttagtgctatcaatggtgctggaaaaggcgacagc
tgtgaagtgactggcacaattaaagcagttgaccggttaacagctcctgagttagacatagatgcaaacttcaaacagactcatgttgttag
agctggggccagtattcgcctcttcattgcctaccaaggtagacctactcctacagctgtgtggagcaaaccagactctaaccttagccttc
gggctgatatccatacaacagattccttcagcaccctcactgtggaaaactgcaacagaaatgatgcagggaaatatacccttactgtgg
aaaacaacagtggtagtaagtcaatcacattcaccgtgaaagtgctagacactccaggcccacctggcccaattaccttcaaagatgtg
acccggggatctgctacattgatgtgggatgcccctcttcttgacggtggtgcccgaatccatcattatgtggtagagaaacgagaggcaa
gtcgccgtagttggcaggttatcagtgaaaaatgcactcgtcagatcttcaaggtcaatgacctggccgaaggtgttccgtactatttccgtgt
ttctgcagtaaatgagtatggtgttggtgagccctatgaaatgccagaaccaattgtagccacagaacagcctgctccacctaggagactt
gatgttgttgatactagcaaatcctccgcagtcttagcttggcttaaacctgaccacgatggaggcagccggatcactggctacctgcttga
aatgagacaaaagggatctgacttctgggttgaagctggtcacaccaaacagctaactttcacagtagagcgtcttgttgagaaaactga
atatgaattccgtgtgaaggccaagaatgatgctggctatagtgaacccagagaagccttctcttctgtcatcattaaggagcctcaaatcg
agcccactgctgacctcactggaattaccaatcagcttataacttgcaaagcaggaagcccatttaccattgacgtaccaatcagtggtcgt
cctgcccccaaagtaacatggaaactggaagaaatgagacttaaagagacagatcgagtgagcattacaacaacaaaagacagaa
ccacactgactgtaaaggacagcatgagaggtgactctggaagatacttcttgaccctggaaaatacagctggtgttaaaacatttagcgt
cacagttgtggtcattggaaggccaggtccagtaaccggccccattgaggtctcatctgtctcagctgaatcgtgtgtcctgtcatggggag
aacctaaagatggaggaggcactgaaattactaattacatagttgaaaagcgtgaatcgggtacaacagcttggcagcttgtcaattcca
gtgtcaagcgcactcaaattaaagtcactcatctcacaaaatacatggaatattctttccgtgtcagttcagagaacagatttggtgtcagca
aacctctagaatcagcaccaataattgctgaacatccatttgtcccaccaagcgctcctaccagacctgaggtctaccatgtgtctgccaat
gccatgtctattcgttgggaagaaccctaccacgatggtggcagtaaaatcattggctactgggttgagaagaaagaacgtaatacaattc
tttgggtgaaagaaaacaaagtgccatgcttagagtgcaactacaaagtaactggtttagtagaaggactggaatatcagttcagaactta
tgcactcaatgctgcaggtgttagcaaggccagcgaagcttcaagacctataatggctcaaaatccagttgatgcaccaggcagaccag
aggtgacagatgtcacaagatcaacagtatcactgatttggtctgccccagcgtatgatggaggcagcaaggttgtgggctacatcatag
agcgtaagccagtcagtgaggtaggagatggtcgctggctgaagtgcaactacaccattgtatctgacaatttcttcaccgtgactgctctc
agtgaaggagacacttatgagttccgtgtgttagccaagaatgcagcaggcgtaattagcaaagggtctgaatctacaggccctgtcactt
gccgagatgaatacgctccacccaaagccgaactggatgcccgattacacggtgatctggttaccatcagagcaggttctgatcttgttctg
gatgctgcagttggtggcaaacctgaacccaaaattatctggaccaaaggagacaaggagctagatctctgtgaaaaagtctctttgcag
tatactggcaaacgagcaactgctgtgatcaagttctgtgacagaagtgacagtggaaaatacactttaacagtgaaaaatgccagcgg
gaccaaggccgtgtctgtcatggtcaaagtgcttgattcccctggcccatgtggaaagctcaccgtcagcagagtaacacaggagaagt
gcactttagcctggagccttccgcaggaagacggaggagcagaaatcactcactacatcgtggaaagacgcgagactagcaggctca
actgggtgattgttgaaggcgaatgcccaaccctatcctatgtcgttaccaggctcatcaagaacaatgagtacatattccgagtgagggc
agtaaacaaatatggccctggtgtgcctgttgaatcagagccaattgtagccagaaactcattcactattccatcacccggcatacctg
aagaagttgggactggcaaagagcatatcatcattcagtggacaaaacctgaatctgatggtggcaatgaaatcagcaactacctagta
gacaaacgtgagaagaagagcctgcgctggacacgtgtcaacaaagactatgtggtgtatgataccaggctgaaggtgaccagcctg
atggagggttgtgattaccagttccgggtgaccgcagtgaatgcagctggtaacagtgagcccagcgaagcttccaacttcatctcatgca
gagaaccatcatataccccctggaccaccttctgctccaagagttgtggataccaccaaacacagcattagtttggcatggaccaaaccca
tgtacgatggtggtactgacattgtaggatatgttctggaaatgcaagagaaggacactgatcagtggtaccgagtgcataccaatgccac
aataagaaatactgaattcactgtgccagaccttaaaatgggccagaaatattccttcagagttgctgccgtgaacgtgaagggtatgagc
gaatacagcgaatcaattgctgaaattgagcccgtggaaagaatagaaataccagatcttgagcttgcagatgatctaaagaagactgt
gaccatcagggctggggcctccttgcgcttgatggtgtctgtatctggaagaccacctcctgtcataacgtggagcaagcagggcattgac
cttgcaagccgggcaattattgacaccactgagagctactcattgctaatagtggacaaagttaatcggtacgatgctggaaaatacaca
attgaagctgaaaaccaatctggcaagaaatcagcaacagtccttgttaaagtctatgatactcctggtccctgtccttcagtgaaagttaag
gaagtatcaagagattctgtgactataacttgggaaattcccacgattgatggtggagctccagtcaacaattacatcgttgagaagcgtga
agctgctatgagagcattcaaaacagtaactaccaaatgcagcaagacactttacagaatttctggacttgtagaaggaaccatgtactat
ttcagagtgctgccagaaaatatttatggcattggagaaccttgtgaaacatctgatgcagtactggtctcagaagtgcctttggtgcctgca
aagctagaagtggtcgatgtcaccaaatccactgttacccttgcctgggaaaaaccactctacgatggtggtagccgactcactggatatg
ttctcgaggcctgcaaagctggcacagagagatggatgaaggttgtcaccttaaaacccacagtcctagagcacactgttacttccttaaa
tgaaggtgaacaatacttatttagaataagggcacaaaatgagaaaggtgtgtcagaaccaagagagactgtcacagccgtgactgta
caagacctcagagtgttgccaacaatcgatctttctacaatgcctcagaagaccatccatgtcccagctggcagaccagtagagctggtg
atacctattgctggccgtccacctcctgctgcttcctggttctttgctggttctaaactgagagaatcagagcgtgtcacagttgaaactcacac
taaagtagctaaattaaccatccgtgaaaccactatcagagatactggagaatacacacttgaattgaagaatgttaccggaactacttca
gaaaccattaaagttatcattcttgacaagcctggtccaccaacaggacctattaagattgatgaaattgatgctacatcaattaccatttcct
gggaaccacctgaattggacggtggtgctccactgagtggttatgtggtagaacaacgtgacgctcatcgtccaggatggctgcccgtttct
gaatcagtgactaggtccacgtttaagtttaccagactcaccgaaggaaatgagtatgtgttccgtgtggctgcaacaaaccgcttcgggat
tggctcttacttgcagtctgaggtcatagagtgtcgcagcagcatccgtattcctggacccccagaaacattacagatatttgatgtttcccgt

gatggcatgacacttacttggtacccaccagaggatgacggtggctcccaagtgactggatatattgtggagcgcaaagaagtgagagc
agatcgatgggtccgtgtaaataaagtacctgtgacaatgacacggtaccgctccactggccttactgaaggcttagaatatgaacaccgt
gtcacagccattaatgcaagagggtctgggaaaccaagtcgtccttccaaacccatcgttgccatggatccaattgctcctccaggaaag
ccacaaaacccaagagttactgatacaacaaggacatcagtctccctggcctggagtgttccagaagatgaaggaggatctaaagtca
caggctacttgattgaaatgcaaaaagtagatcaacatgaatggaccaagtgtaacaccactccaaccaagattcgagagtatactcta
acacacctacctcagggtgcagaatacaggttccgcgtcctagcttgtaatgctggtggacctggtgagcctgctgaggtaccaggaaca
gtcaaagtcactgaaatgcttgaatatcctgattatgaacttgatgaaagataccaagaaggtatctttgtaaggcaaggtggcgtcatcag
acttaccataccaatcaaaggaaaaccattcccaatatgtaaatggaccaaggaaggccaggatattagtaagcgtgccatgattgcaa
catctgaaacacacactgagcttgtgatcaaagaagcagacaggggtgattctggcacttatgacctggttctggaaaataaatgtggca
agaaggctgtctacatcaaggtcagggtgataggaagtcccaacagtccagaagggccactggaatatgatgacatccaagtccgctct
gtgagggtcagctggagacctcctgctgatgatggtggtgctgacatcttaggctacatcctcgagagacgagaagtgcctaaagccgcc
tggtataccattgattccagagtccgaggtacatctctggtggtaaaaggcctcaaagagaatgtagaataccatttccgtgtttcagcaga
aaaccagtttggcataagcaaacccttgaaatctgaggaaccagtcacaccaaaaacaccattgaatcctccagaacctccaagcaat
cctccagaagtactcgatgtaaccaagagttctgttagcttgtcctggtcccggcccaaagatgatggtggttctagagtcacaggctacta
catcgaacgcaaagagacatccactgacaagtgggtcagacacaacaagactcagatcaccaccacaatgtacactgtcacagggct
tgttcccgatgctgagtatcagttccgcatcatcgcacagaatgatgttggcctgagtgagaccagccctgcttctgaaccagttgtttgcaa
agatccatttgataaaccaagccaaccaggagaacttgagattctttcaatatccaaagatagtgtcactctacagtgggagaaacctga
atgtgatggtggtaaagaaattcttggatactgggttgaatatagacagtctggagacagtgcctggaagaagagcaataaggaacgtat
taaggacaagcaattcacaataggaggtttgctggaagctactgagtatgaattcagggtttttgctgagaatgagactgggctgagcaga
cctcgcagaactgctatgtctataaagactaaactcacatctggagaggccccaggaatacgcaaagaaatgaaggatgttaccacaa
aattgggtgaagctgctcaactctcatgccagattgttggaaggcctcttcctgacattaaatggtacagatttggtaaagagctcatacaaa
gccggaaatacaaaatgtcttcagatggacgcacacacactcttacagtaatgacagaggaacaggaagatgaaggtgtttatacctgc
atagccaccaatgaggttggagaagtagaaaccagtagtaagcttctcctgcaagcaacaccgcagttccatcctggttacccactgaa
agagaaatattatggagctgtgggttccacacttcggcttcatgttatgtacattggtcgtccagtacctgccatgacttggttccatggtcaga
aacttttgcaaaactcagaaaacattactattgaaaacactgagcactatactcatcttgtcatgaagaatgtccaacgtaagactcatgctg
ggaaatacaaagtccagctcagcaatgtttttggaacagttgatgccatccttgatgtggaaatacaagataaaccagacaaacctacag
gaccaattgtgatcgaagctctattgaagaactccgcagtgatcagctggaaaccacccgcagatgacggaggctcctggatcaccaac
tatgtggtggaaaaaatgtgaggccaaggagggggctgaatggcaattggtgtcttcagccatctcagtgacaacctgtagaattgtgaacc
tcacagaaaatgctggctattacttccgggtttcagctcagaacactttcggcatcagtgaccctctagaagtgtcctcagttgtgatcattaa
gagtccatttgaaaagccaggtgctcctggcaaaccaactattactgctgtcacaaaagattcttgtgttgtggcctggaagccacctgcca
gtgatggaggtgcaaagattagaaattactaccttgagaagcgtgagaagaagcagaataaatggatttctgtgacaacagaagaaatt
cgagaaactgtcttttcagtgaaaaacccttattgaaggtcttgaatacgagtttcgtgtgaaatgtgaaaatctaggtggggaaagtgaatgg
agtgaaatatcagaacccatcactcccaaatctgatgtcccaattcaggcaccacactttaaagaggaactgagaaatctaaatgtcaga
tatcagagcaatgctaccttggtctgcaaagtgactggtcatccaaaacctatcgtcaaatggtacagacaaggcaaagaaatcattgca
gatggattaaaatataggattcaagaatttaagggtggctaccaccagctcatcattgcaagtgtcacagatgatgatgccacagtttacca
agtcagagctaccaaccaaggggggatctgtgtctggcactgcctccttggaagtggaagttccagctaagatacacttacctaaaactctt
gaaggcatgggagcagttcatgctctccgaggtgaagtggtcagcatcaagattcctttcagtggcaaaccagatcctgtgatcacctggc
agaaaggacaagatctcattgacaataatggccactaccaagttattgtcacaagatccttcacatcacttgttttccccaatggggtagag
agaaaagatgctggtttctatgtggtctgtgctaaaaacagatttggaattgatcagaagacagttgaactggatgtggctgatgttcctgac
ccacccagaggagtcaaagttagtgatgtctcacgagattctgtcaacttaacatggactgagccagcctctgatggtggcagcaaaatc
accaactacattgttgaaaaatgtgcaactactgcagaaagatggctccgtgtaggacaggcccgagaaacacgttataccgtgatcaa
cttatttggaaaaacaagttaccagttccgggtaatagctgaaaataaatttggtctgagcaagccttcagagccttcagaaccaaccata
accaaagaagataagaccagagctatgaactatgatgaagaggtagatgaaaccagggaagtctccatgactaaagcatctcactctt
caaccaaggaactctatgagaaatatatgattgctgaagatcttgggcgtggtgagtttggaattgtccatcgttgtgttgaaacatcctcaaa
gaagacatacatggccaaatttgttaaagtcaaagggactgatcaggttttggtaaagaaggaaatttccattctgaatattgctaggcata
gaaacatcttacacctccatgaatcatttgaaagcatggaagaattagttatgatctttgagtttatatcaggacttgacatatttgagcgcatta
acacaagtgcttttgaacttaatgaaagagaaattgtaagttatgttcaccaggtctgtgaagcacttcagtttttacacagtcataatattgga
cactttgacattagaccagaaaatatcatttaccaaaccagaagaagctctaccattaaaatcatagaatttggtcaagcccgtcagctga
aaccaggggacaacttcaggcttctattcactgccccagaatactatgcacctgaagtccaccagcatgatgttgtcagcacagccacag
acatgtggtcacttggaacactggtatatgtgctattgagtggtatcaacccattcctggctgaaactaaccaacagatcattgagaatatca
tgaatgctgaatatactttcgatgaggaagcattcaaagagattagcattgaagccatggatttgttgaccggttgttagtgaaagagagga
aatctcgcatgacagcatcggaggctctccagcacccatggttgaagcagaagatagaaagagtcagtactaaagttatcagaacatta
aaacaccggcgttattaccacaccctgatcaagaaagacctcaacatggttgtgtcagcagcccggatctcctgtggtggtgcaattcgat
ctcagaagggagtgagtgttgctaaagttaaagtggcatccattgaaattggcccagtttctgggcagataatgcatgcagttggtgaaga
aggaggacatgtcaaatatgtatgcaaaattgaaaattatgatcagtctacccaagtgacttggtactttggcgtccgacagctggagaac
agtgagaaatacgaaatcacctacgaagatggagtggccatcctctatgtcaaagacattaccaaattagatgatggtacctacagatgc
aaagtagtcaatgactatggtgaagacagttcttatgcagagctatttgttaaaggtgtgagagaagtctatgactattactgccgtagaacc

atgaagaaaattaagcgcagaacagacacaatgagactcctggaaaggccaccagaatttaccctgcctctctataataagacagctta
tgtaggtgaaaatgtccggtttggagtaactataactgtccacccagagcctcatgtaacatggtataaatcaggtcagaaaatcaaacca
ggtgacaatgacaagaagtacacatttgagtcagacaagggtctttaccaattaacaatcaacagtgtcactacagatgatgacgctgaa
tatactgttgtggcaaggaacaaatatggtgaagacagctgtaaagcaaagctgacagtaaccctacacccacctccaacagatagtac
cttaagacccatgttcaaaaggttactggcaaatgcagaatgccaagaaggccaaagtgtctgctttgagatcagagtgtctggcatcccc
ccaccaacattaaaatgggagaaagatggtcagccactgtccctcgggcctaacattgaaattatccatgaaggcttggattattatgctct
gcacatcagggacactttgcctgaagacacgggttattatagagtcacagccactaacacagctgggtccaccagctgccaggctcacc
tacaagtggaacgcctgaggtacaagaaacaggaattcaagagtaaggaggagcatgagcgacacgtacaaaaacaaattgacaa
aaccctcagaatggctgaaattctttctggaactgaaagtgtaccactgacacaggtagctaaagaggctctgagagaagctgctgtccttt
ataaaccggctgtaagcaccaagactgtaaaaggggaattcagacttgagatagaagaaaagaaggaggagagaaaactccggat
gccttatgatgtaccagagccacgcaagtataagcagactaccatagaagaagaccaacgcatcaagcagttcgtgcccatgtctgac
atgaagtggtataaaaagatacgtgatcagtatgaaatgcctgggaaacttgacagagttgtacagaaacgacccaagcgcatccgcct
ttcaagatgggaacagttctatgtgatgcctcttccacgcattacagatcaatacagacctaaatggcgtattcctaaactgtcccaagatga
tcttgagatagtgagaccagcccgccggcgtacaccttctcctgattatgacttttactaccgacctagaagacgttctcttggggacatctct
gatgaagaattactcctccccattgatgactacttagcaatgaaaagaacagaggaagagaggctgcgtcttgaagaagagcttgagtta
ggtttttcagcttcacccccaagtcgaagccctccacactttgagctttctagcctacgttactcttcaccacaagctcatgtcaaggtggagg
aaacaagaaaagacttcaggtattcaacctatcacatcccaacgaaggctgaagctagtacaagttatgcagaactgagggaacggc
atgcccaggctgcgtacagacagccaaagcaacggcaaagaatcatggctgagagggaggatgaagagttgcttcgcccagttacga
ccacccagcatctctcagaatacaaaagcgaacttgacttcatgtcaaaggaggaaaagtctagaaagaaatcaaggcgacaaaga
gaagtgacagaaataacagaaattgaggaagaatacgaaatctcaaaacatgctcaaagagaatcatcctcatctgcgtctagactact
gagacgacggcgctccctgtctccaacttatattgagttaatgaggccagtgtctgagctgatccggtcacgtccacaaccggctgaggaa
tacgaagatgacacagaaagaaggtcacctactccagagagaactcgcccacgatcccccagccctgtgtctagtgagagatcactct
cgagatttgagaggtctgcaagatttgatatcttttccaggtatgagtccatgaaagctgctttaaaaactcagaagacatcagaaaggaa
gtatgaagttttgagtcagcagcctttcacactggaccatgcccctcgaatcacactgagaatgcgctcgcacagggtaccatgtggccaa
aatacacgttttattttaaatgttcagtctaagccaactgccgaggttaaatggtaccacaatggtgtggaactccaagaaagcagtaagatt
cattacaccaacacgagtggagtcctcaccctggaaattctggactgtcatactgatgacagtggaacctaccgtgctgtgtgcaccaact
acaagggcgaagcttctgactatgcaacgttggacgtgacaggaggggattataccacctatgcttcccaacgcagagatgaagaggtc
cccagatctgttttccctgagctgacaagaacagaggcgtatgctgtttcatcatttaagaaaacatctgagatggaagcttcgtcttctgtca
gggaagtgaaatcacagatgacggagacaagggaaagtctctcctcatatgaacactctgcatctgcagaaatgaaaagtgctgcatta
gaagaaaagtcactggaagaaaaatccacaaccagaaagatcaagacgactttggcagcaagaattctaacaaagccacggtccat
gaccgtctacgagggcgagtctgcaaggttttcttgtgacaccgatggtgagccggtaccaactgtgacctggctgcgtaaaggacaagt
gctaagtacttctgcccgccaccaagtgaccaccacaaagtacaaatcaacctttgagatctcttcagtccaggcttccgatgagggcaat
tacagcgtggtggtagaaaacagtgaagggaaacaagaagcagagttcactctgactattcaaaaggccagggtaactgaaaaggct
gtgacatcaccaccaagagtcaaatccccagagcctcgggtgaaatccccagaagcagttaagtctccaaaacgagtgaaatctccag
aaccttctcacccgaaagccgtatcacccacagagacaaaaccaacaccaacagagaaagttcagcacctcccagtctctgccccac
caaagattactcagttcctgaaagcagaagcttctaaagagattgcaaaactgacctgtgtggttgaaagcagtgtattaagggcaaaag
aggtcacctggtataaagatggcaagaaactgaaggaaaatgggcatttccagtttcattattcagcagatggtacctatgagctcaaaat
caataacctcactgaatctgatcaaggagaatatgtttgtgagatttctggtgaaggtggaacgtctaaaaccaacttacaatttatggggca
agcctttaagagtatccatgagaaggtatcaaaaatatcagaaactaagaaatcagatcagaaaaccactgagtcaacagtaaccaga
aaaactgaaccaaaagctcctgaaccaatttcctcaaaaccagtaattgttactgggttgcaggatacaactgtttcttcagacagtgttgct
aaatttgcagttaaggctactggagaaccccggccaactgccatctggacaaaagatggaaaggccattacacaaggaggtaaatata
aactctctgaagacaagggagggttcttcttagaaattcataagactgatacttctgacagtggactttatacttgtacagtaaaaaattcagc
tggatctgtgtcctctagctgcaaattaacaataaaagctataaaagatactgaggcacagaaagtctctacacaaaagacttctgaaatt
acacctcagaagaaagctgttgtccaagaggaaatttcccaaaaagccctaaggtctgaagaaattaagatgtcagaggcaaaatctc
aagaaaagttagccctcaaagaggaagcttcaaaggttctgatttctgaagaagtcaagaaatcagcagcaacctccctggaaaaatc
cattgtccatgaggaaatcactaaaacatcacaggcatcagaagaagtcagaactcatgctgagattaaagcattttctactcagatgag
cataaacgaaggtcaaagactggttttaaaagccaacattgctggtgccactgatgtgaaatgggtactgaatggcgtagagcttaccaa
ctctgaggagtaccgatatggtgtctcaggcagcgatcagaccctaaccatcaagcaagccagtcacagagatgaaggaatcctcacct
gcataagcaaaaccaaggaaggaatcgtcaagtgtcagtatgatttgacactgagcaaagaactctcagatgctccagccttcatctcac
agcctagatctcaaaatattaatgaaggacaaaatgttctctttacttgtgaaatcagtggcgagccatcccctgaaatcgaatggtttaaaa
acaacctgccaatttctatttcttcaaatgtcagcataagccgctccagaaatgtatactcccttgaaatccgaaatgcatcagtcagcgaca
gtggaaagtacacaattaaggccaaaaatttccgtggccagtgttcagctacagcttccttaatggtccttcctctagttgaagaaccttcca
gagaggtagtattgagaacaagtggtgacacaagcttgcaaggaagcttctcgtctcagtcagtccaaatgtctgcctccaagcaggag
gcctccttcagcagtttcagcagcagcagtgctagcagcatgactgagatgaaatttgcaagcatgtctgcccaaagcatgtcctccatgc
aagagtcctttgtagaaatgagttccagcagctttatgggaatatctaatatgacacaactggaaagctcaactagtaaaatgcttaaagca
ggcataagaggaattccgcctaaaattgaagctcttccatctgatatcagcattgatgaaggcaaagttctaacagtagcctgtgctttcacg
ggtgagcctaccccagaagtaacatggtcctgtggtggaagaaaaatccacagtcaagaacaggggaggttccacattgaaacaca

gatgacctgacaaccctgatcatcatggacgtacagaaacaagatggtggactttataccctgagtttagggaatgaatttggatctgactc tgccactgtgaatatacatattcgatccatttaa (SEQ ID NO:393)

**Q8WZ42**

MTTQAPTFTQPLQSVVVLEGSTATFEAHISGFPVPEVSWFRDGQVISTSTLPGVQISFSDGRAKLT
IPAVTKANSGRYSLKATNGSGQATSTAELLVKAETAPPNFVQRLQSMTVRQGSQVRLQVRVTGIP
TPVVKFYRDGAEIQSSLDFQISQEGDLYSLLIAEAYPEDSGTYSVNATNSVGRATSTAELLVQGEE
EVPAKKTKTIVSTAQISESRQTRIEKKIEAHFDARSIATVEMVIDGAAGQQLPHKTPHRIPPKPKSRS
PTPPSIAAKAQLARQQSPSPIRHSPSPVRHVRAPTPSPVRSVSPAARISTSPIRSVRSPLLMRKTQ
ASTVATGPEVPPPWKQEGYVASSSEAEMRETTLTTSTQIRTEERWEGRYGVQEQVTISGAAGAA
ASVSASASYAAEAVATGAKEVKQDADKSAAVATVVAAVDMARVREPVISAVEQTAQRTTTTAVHI
QPAQEQVRKEAEKTAVTKVVVAADKAKEQELKSRTKEVITTKQEQMHVTHEQIRKETEKTFVPKV
VISAAKAKEQETRISEEITKKQKQVTQEAIRQETEITAASMVVVATAKSTKLETVPGAQEETTTQQD
QMHLSYEKIMKETRKTVVPKVIVATPKVKEQDLVSRGREGITTKREQVQITQEKMRKEAEKTALSTI
AVATAKAKEQETILRTRETMATRQEQIQVTHGKVDVGKKAEAVATVVAAVDQARVREPREPGHLE
ESYAQQTTLEYGYKERISAAKVAEPPQRPASEPHVVPKAVKPRVIQAPSETHIKTTDQKGMHISSQ
IKKTTDLTTERLVHVDKRPRTASPHFTVSKISVPKTEHGYEASIAGSAIATLQKELSATSSAQKITKS
VKAPTVKPSETRVRAEPTPLPQFPFADTPDTYKSEAGVEVKKEVGVSITGTTVREERFEVLHGRE
AKVTETARVPAPVEIPVTPPTLVSGLKNVTVIEGESVTLECHISGYPSPTVTWYREDYQIESSIDFQI
TFQSGIARLMIREAFAEDSGRFTCSAVNEAGTVSTSCYLAVQVSEEFEKETTAVTEKFTTEEKRFV
ESRDVVMTDTSLTEEQAGPGEPAAPYFITKPVVQKLVEGGSVVFGCQVGGNPKPHVYWKKSGV
PLTTGYRYKVSYNKQTGECKLVISMTFADDAGEYTIVVRNKHGETSASASLLEEADYELLMKSQQ
EMLYQTQVTAFVQEPKVGETAPGFVYSEYEKEYEKEQALIRKKMAKDTVVVRTYVEDQEFHISSF
EERLIKEIEYRIIKTTLEELLEEDGEEKMAVDISESEAVESGFDLRIKNYRILEGMGVTFHCKMSGYP
LPKIAWYKDGKRIKHGERYQMDFLQDGRASLRIPVVLPEDEGIYTAFASNIKGNAICSGKLYVEPAA
PLGAPTYIPTLEPVSRIRSLSPRSVSRSPIRMSPARMSPARMSPARMSPARMSPGRRLEETDESQ
LERLYKPVFVLKPVSFKCLEGQTARFDLKVVGRPMPETFWFHDGQQIVNDYTHKVVIKEDGTQSLI
IVPATPSDSGEWTVVAQNRAGRSSISVILTVEAVEHQVKPMFVEKLKNVNIKEGSQLEMKVRATG
NPNPDIVWLKNSDIIVPHKYPKIRIEGTKGEAALKIDSTVSQDSAWYTATAINKAGRDTTRCKVNVE
VEFAEPEPERKLIIPRGTYRAKEIAAPELEPLHLRYGQEQWEEGDLYDKEKQQKPFFKKKLTSLRL
KRFGPAHFECRLTPIGDPTMVVEWLHDGKPLEAANRLRMINEFGYCSLDYGVAYSRDSGIITCRA
TNKYGTDHTSATLIVKDEKSLVEESQLPEGRKGLQRIEELERMAHEGALTGVTTDQKEKQKPDIVL
YPEPVRVLEGETARFRCRVTGYPQPKVNWYLNGQLIRKSKRFRVRYDGIHYLDIVDCKSYDTGEV
KVTAENPEGVIEHKVKLEIQQREDFRSVLRRAPEPRPEFHVHEPGKLQFEVQKVDRPVDTTETKE
VVKLKRAERITHEKVPEESEELRSKFKRRTEEGYYEAITAVELKSRKKDESYEELLRKTKDELLHW
TKELTEEEKKALAEEGKITIPTFKPDKIELSPSMEAPKIFERIQSQTVGQGSDAHFRVRVVGKPDPE
CEWYKNGVKIERSDRIYWYWPEDNVCELVIRDVTAEDSASIMVKAINIAGETSSHAFLLVQAKQLIT
FTQELQDVVAKEKDTMATFECETSEPFVKVKWYKDGMEVHEGDKYRMHSDRKVHFLSILTIDTSD
AEDYSCVLVEDENVKTTAKLIVEGAVVEFVKELQDIEVPESYSGELECIVSPENIEGKWYHNDVELK
SNGKYTITSRRGRQNLTVKDVTKEDQGEYSFVIDGKKTTCKLKMKPRPIAILQGLSDQKVCEGDIV
QLEVKVSLESVEGVWMKDGQEVQPSDRVHIVIDKQSHMLLIEDMTKEDAGNYSFTIPALGLSTSG
RVSVYSVDVITPLKDVNVIEGTKAVLECKVSVPDVTSVKWYLNDEQIKPDDRVQAIVKGTKQRLVIN
RTHASDEGPYKLIVGRVETNCNLSVEKIKIIRGLRDLTCTETQNVVFEVELSHSGIDVLWNFKDKEIK
PSSKYKIEAHGKIYKLTVLNMMKDDEGKYTFYAGENITSGKLTVAGGAISKPLTDQTVAESQEAVF
ECEVANPDSKGEWLRDGKHLPLTNNIRSESDGHKRRLIIAATKLDDIGEYTYKVATSKTSAKLKVE
AVKIKKTLKNLTVTETQDAVFTVELTHPNVKGVQWIKNGVVLESNEKYAISVKGTIYSLRIKNCAIVD
ESVYGFRLGRLGASARLHVETVKIIKKPKDVTALENATVAFEVSVSHDTVPVKWFHKNVEIKPSDK
HRLVSERKVHKLMLQNISPSDAGEYTAVVGQLECKAKLFVETLHITKTMKNIEVPETKTASFECEV
SHFNVPSMWLKNGVEIEMSEKFKIVVQGKLHQLIIMNTSTEDSAEYTFVCGNDQVSATLTVTPIMIT
SMLKDINAEEKDTITFEVTVNYEGISYKWLKNGVEIKSTDKCQMRTKKLTHSLNIRNVHFGDAADYT
FVAGKATSTATLYVEARHIEFRKHIKDIKVLEKKRAMFECEVSEPDITVQWMKDDQELQITDRIKIQK
EKYVHRLLIPSTRMSDAGKYTVVAGGNVSTAKLFVEGRDVRIRSIKKEVQVIEKQRAVVEFEVNED
DVDAHWYKDGIEINFQVQERHKYVVERRIHRMFISETRQSDAGEYTFVAGRNRSSVTLYVNAPEP
PQVLQELQPVTVQSGKPARFCAVISGRPQPKISWYKEEQLLSTGFKCKFLHDGQEYTLLLIEAFPE
DAAVYTCEAKNDYGVATTSASLSVEVPEVVSPDQEMPVYPPAIITPLQDTVTSEGQPARFQCRVS
GTDLKVSWYSKDKKIKPSRFFRMTQFEDTYQLEIAEAYPEDEGTYTFVASNAVGQVSSTANLSLE
APESILHERIEQEIEMEMKEFSSSFLSAEEEGLHSAELQLSKINETLELLSESPVYSTKFDSEKEGT

GPIFIKEVSNADISMGDVATLSVTVIGIPKPKIQWFFNGVLLTPSADYKFVFDGDDHSLIILFTKLEDE
GEYTCMASNDYGKTICSAYLKINSKGEGHKDTETESAVAKSLEKLGGPCPPHFLKELKPIRCAQGL
PAIFEYTVVGEPAPTVTWFKENKQLCTSVYYTIIHNPNGSGTFIVNDPQREDSGLYICKAENMLGE
STCAAELLVLLEDTDMTDTPCKAKSTPEAPEDFPQTPLKGPAVEALDSEQEIATFVKDTILKAALITE
ENQQLSYEHIAKANELSSQLPLGAQELQSILEQDKLTPESTREFLCINGSIHFQPLKEPSPNLQLQIV
QSQKTFSKEGILMPEEPETQAVLSDTEKIFPSAMSIEQINSLTVEPLKTLLAEPEGNYPQSSIEPPM
HSYLTSVAEEVLSPKEKTVSDTNREQRVTLQKQEAQSALILSQSLAEGHVESLQSPDVMISQVNY
EPLVPSEHSCTEGGKILIESANPLENAGQDSAVRIEEGKSLRFPLALEEKQVLLKEEHSDNVVMPP
DQIIESKREPVAIKKVQEVQGRDLLSKESLLSGIPEEQRLNLKIQICRALQAAVASEQPGLFSEWLR
NIEKVEVEAVNITQEPRHIMCMYLVTSAKSVTEEVTIIIEDVDPQMANLKMELRDALCAIIYEEIDILTA
EGPRIQQGAKTSLQEEMDSFSGSQKVEPITEPEVESKYLISPEEVSYFNVQSRVKYLDATPVTKGV
ASAVVSDEKQDESLKPSEEKEESSSESGTEEVATVKIQEAEGGFIKEDGPMIHTPLVDTVSEEGDI
VHLTTSITNAKEVNWYFENKLVPSDEKFKCLQDQNTYTLVIDKVNTEDHQGEYVCEALNDSGKTA
TSAKLTVVKRAAPVIKRKIEPLEVALGHLAKFTCEIQSAPNVRFQWFKAGREIYESDKCSIRSSKYIS
SLEILRTQVVDCGEYTCKASNEYGSVSCTATLTVTEAYPPTFLSRPKSLTTFVGKAAKFICTVTGTP
VIETIWQKDGAALSPSPNWKISDAENKHILELSNLTIQDRGVYSCKASNKFGADICQAELIIIDKPHFI
KELEPVQSAINKKVHLECQVDEDRKVTVTWSKDGQKLPPGKDYKICFEDKIATLEIPLAKLKDSGT
YVCTASNEAGSSSCSATVTVREPPSFVKKVDPSYLMLPGESARLHCKLKGSPVIQVTWFKNNKEL
SESNTVRMYFVNSEAILDITDVKVEDSGSYSCEAVNDVGSDSCSTEIVIKEPPSFIKTLEPADIVRGT
NALLQCEVSGTGPFEISWFKDKKQIRSSKKYRLFSQKSLVCLEIFSFNSADVGEYECVVANEVGKC
GCMATHLLKEPPTFVKKVDDLIALGGQTVTLQAAVRGSEPISVTWMKGQEVIREDGKIKMSFSNG
VAVLIIPDVQISFGGKYTCLAENEAGSQTSVGELIVKEPAKIIERAELIQVTAGDPATLEYTVAGTPEL
KPKWYKDGRPLVASKKYRISFKNNVAQLKFYSAELHDSGQYTFEISNEVGSSSCETTFTVLDRDIA
PFFTKPLRNVDSVVNGTCRLDCKIAGSLPMRVSWFKDGKEIAASDRYRIAFVEGTASLEIIRVDMN
DAGNFTCRATNSVGSKDSSGALIVQEPPSFVTKPGSKDVLPGSAVCLKSTFQGSTPLTIRWFKGN
KELVSGGSCYITKEALESSLELYLVKTSDSGTYTCKVSNVAGGVECSANLFVKEPATFVEKLEPSQ
LLKKGDATQLACKVTGTPPIKITWFANDREIKESSKHRMSFVESTAVLRLTDVGIEDSGEYMCEAQ
NEAGSDHCSSIVIVKESPYFTKEFKPIEVLKEYDVMLLAEVAGTPPFEITWFKDNTILRSGRKYKTFI
QDHLVSLQILKFVAADAGEYQCRVTNEVGSSICSARVTLREPPSFIKKIESTSSLRGGTAAFQATLK
GSLPITVTWLKDSDEITEDDNIRMTFENNVASLYLSGIEVKHDGKYVCQAKNDAGIQRCSALLSVK
EPATITEEAVSIDVTQGDPATLQVKFSGTKEITAKWFKDGQELTLGSKYKISVTDTVSILKIISTEKKD
SGEYTFEVQNDVGRSSCKARINVLDLIIPPSFTKKLKKMDSIKGSFIDLECIVAGSHPISIQWFKDDQ
EISASEKYKFSFHDNTAFLEISQLEGTDSGTYTCSATNKAGHNQCSGHLTVKEPPYFVEKPQSQD
VNPNTRVQLKALVGGTAPMTIKWFKDNKELHSGAARSVWKDDTSTSLELFAAKATDSGTYICQLS
NDVGTATSKATLFVKEPPQFIKKPSPVLVLRNGQSTTFECQITGTPKIRVSWYLDGNEITAIQKHGI
SFIDGLATFQISGARVENSGTYVCEARNDAGTASCSIELKVKEPPTFIRELKPVEVVKYSDVELECE
VTGTPPFEVTWLKNNREIRSSKKYTLTDRVSVFNLHITKCDPSDTGEYQCIVSNEGGSCSCSTRVA
LKEPPSFIKKIENTTTVLKSSATFQSTVAGSPPISITWLKDDQILDEDDNVYISFVDSVATLQIRSVDN
GHSGRYTCQAKNESGVERCYAFLLVQEPAQIVEKAKSVDVTEKDPMTLECVVAGTPELKVKWLK
DGKQIVPSRYFSMSFENNVASFRIQSVMKQDSGQYTFKVENDFGSSSCDAYLRVLDQNIPPSFTK
KLTKMDKVLGSSIHMECKVSGSLPISAQWFKDGKEISTSAKYRLVCHERSVSLEVNNLELEDTANY
TCKVSNVAGDDACSGILTVKEPPSFLVKPGRQQAIPDSTVEFKAILKGTPPFKIKWFKDDVELVSG
PKCFIGLEGSTSFLNLYSVDASKTGQYTCHVTNDVGSDSCTTMLLVTEPPKFVKKLEASKIVKAGD
SSRLECKIAGSPEIRVVWFRNEHELPASDKYRMTFIDSVAVIQMNNLSTEDSGDFICEAQNPAGST
SCSTKVIVKEPPVFSSFPPIVETLKNAEVSLECELSGTPPFEVVWYKDKRQLRSSKKYKIASKNFHT
SIHILNVDTSDIGEYHCKAQNEVGSDTCVCTVKLKEPPRFVSKLNSLTVVAGEPAELQASIEGAQPI
FVQWLKEKEEVIRESENIRITFVENVATLQFAKAEPANAGKYICQIKNDGGMEENMATLMVLEPAVI
VEKAGPMTVTVGETCTLECKVAGTPELSVEWYKDGKLLTSSQKHKFSFYNKISSLRILSVERQDA
GTYTFQVQNNVGKSSCTAVVDVSDRAVPPSFTRRLKNTGGVLGASCILECKVAGSSPISVAWFHE
KTKIVSGAKYQTTFSDNVCTLQLNSLDSSDMGNYTCVAANVAGSDECRAVLTVQEPPSFVKEPEP
LEVLPGKNVTFTSVIRGTPPFKVNWFRGARELVKGDRCNIYFEDTVAELELFNIDISQSGEYTCVVS
NNAGQASCTTRLFVKEPAAFLKRLSDHSVEPGKSIILESTYTGTLPISVTWKKDGFNITTSEKCNIVT
TEKTCILEILNSTKRDAGQYSCEIENEAGRDVCGALVSTLEPPYFVTELEPLEAAVGDSVSLQCQV
AGTPEITVSWYKGDTKLRPTPEYRTYFTNNVATLVFNKVNINDSGEYTCKAENSIGTASSKTVFRIQ
ERQLPPSFARQLKDIEQTVGLPVTLTCRLNGSAPIQVCWYRDGVLLRDDENLQTSFVDNVATLKIL
QTDLSHSGQYSCSASNPLGTASSSARLTAREPKKSPFFDIKPVSIDVIAGESADFECHVTGAQPM
RITWSKDNKEIRPGGNYTITCVGNTPHLRILKVGKGDSGQYTCQATNDVGKDMCSAQLSVKEPPK

FVKKLEASKVAKQGESIQLECKISGSPEIKVSWFRNDSELHESWKYNMSFINSVALLTINEASAEDS
GDYICEAHNGVGDASCSTALTVKAPPVFTQKPSPVGALKGSDVILQCEISGTPPFEVVWVKDRKQ
VRNSKKFKITSKHFDTSLHILNLEASDVGEYHCKATNEVGSDTCSCSVKFKEPPRFVKKLSDTSTLI
GDAVELRAIVEGFQPISVVWLKDRGEVIRESENTRISFIDNIATLQLGSPEASNSGKYICQIKNDAG
MRECSAVLTVLEPARIIEKPEPMTVTTGNPFALECVVTGTPELSAKWFKDGRELSADSKHHITFINK
VASLKIPCAEMSDKGLYSFEVKNSVGKSNCTVSVHVSDRIVPPSFIRKLKDVNAILGASVVLECRVS
GSAPISVGWFQDGNEIVSGPKCQSSFSENVCTLNLSLLEPSDTGIYTCVAANVAGSDECSAVLTV
QEPPSFEQTPDSVEVLPGMSLTFTSVIRGTPPFKVKWFKGSRELVPGESCNISLEDFVTELELFEV
QPLESGDYSCLVTNDAGSASCTTHLFVKEPATFVKRLADFSVETGSPIVLEATYTGTPPISVSWIKD
EYLISQSERCSITMTEKSTILEILESTIEDYAQYSCLIENEAGQDICEALVSVLEPPYFIEPLEHVEAVI
GEPATLQCKVDGTPEIRISWYKEHTKLRSAPAYKMQFKNNVASLVINKVDHSDVGEYSCKADNSV
GAVASSAVLVIKERKLPPFFARKLKDVHETLGFPVAFECRINGSEPLQVSWYKDGVLLKDDANLQT
SFVHNVATLQILQTDQSHIGQYNCSASNPLGTASSSAKLILSEHEVPPFFDLKPVSVDLALGESGTF
KCHVTGTAPIKITWAKDNREIRPGGNYKMTLVENTATLTVLKVGKGDAGQYTCYASNIAGKDSCS
AHLGVQEPPRFIKKLEPSRIVKQDEFTRYECKIGGSPEIKVLWYKDETEIQESSKFRMSFVDSVAVL
EMHNLSVEDSGDYTCEAHNAAGSASSSTSLKVKEPPIFRKKPHPIETLKGADVHLECELQGTPPF
HVSWYKDKRELRSGKKYKIMSENFLTSIHILNVDAADIGEYQCKATNDVGSDTCVGSIALKAPPRF
VKKLSDISTVVGKEVQLQTTIEGAEPISVVWFKDKGEIVRESDNIWISYSENIATLQFSRVEPANAG
KYTCQIKNDAGMQECFATLSVLEPATIVEKPESIKVTTGDTCTLECTVAGTPELSTKWFKDGKELT
SDNKYKISFFNKVSGLKIINVAPSDSGVYSFEVQNPVGKDSCTASLQVSDRTVPPSFTRKLKETNG
LSGSSVVMECKVYGSPPISVSWFHEGNEISSGRKYQTTLTDNTCALTVNMLEESDSGDYTCIATN
MAGSDECSAPLTVREPPSFVQKPDPMDVLTGTNVTFTSIVKGTPPFSVSWFKGSSELVPGDRCN
VSLEDSVAELELFDVDTSQSGEYTCIVSNEAGKASCTTHLYIKAPAKFVKRLNDYSIEKGKPLILEG
TFTGTPPISVTWKKNGINVTPSQRCNITTTEKSAILEIPSSTVEDAGQYNCYIENASGKDSCSAQILIL
EPPYFVKQLEPVKVSVGDSASLQCQLAGTPEIGVSWYKGDTKLRPTTTYKMHFRNNVATLVFNQ
VDINDSGEYICKAENSVGEVSASTFLTVQEQKLPPSFSRQLRDVQETVGLPVVFDCAISGSEPISV
SWYKDGKPLKDSPNVQTSFLDNTATLNIFKTDRSLAGQYSCTATNPIGSASSSARLILTEGKNPPF
FDIRLAPVDAVVGESADFECHVTGTQPIKVSWAKDSREIRSGGKYQISYLENSAHLTVLKVDKGDS
GQYTCYAVNEVGKDSCTAQLNIKERLIPPSFTKRLSETVEETEGNSFKLEGRVAGSQPITVAWYKN
NIEIQPTSNCEITFKNNTLVLQVRKAGMNDAGLYTCKVSNDAGSALCTSSIVIKEPKKPPVFDQHLT
PVTVSEGEYVQLSCHVQGSEPIRIQWLKAGREIKPSDRCSFSFASGTAVLELRDVAKADSGDYVC
KASNVAGSDTTKSKVTIKDKPAVAPATKKAAVDGRLFFVSEPQSIRVVEKTTATFIAKVGGDPIPNV
KWTKGKWRQLNQGGRVFIHQKGDEAKLEIRDTTKTDSGLYRCVAFNEHGEIESNVNLQVDERKK
QEKIEGDLRAMLKKTPILKKGAGEEEEIDIMELLKNVDPKEYEKYARMYGITDFRGLLQAFELLKQS
QEEETHRLEIEEIERSERDEKEFEELVSFIQQRLSQTEPVTLIKDIENQTVLKDNDAVFEIDIKINYPEI
KLSWYKGTEKLEPSDKFEISIDGDRHTLRVKNCQLKDQGNYRLVCGPHIASAKLTVIEPAWERHLQ
DVTLKEGQTCTMTCQFSVPNVKSEWFRNGRILKPQGRHKTEVEHKVHKLTIADVRAEDQGQYTC
KYEDLETSAELRIEAEPIQFTKRIQNIVVSEHQSATFECEVSFDDAIVTWYKGPTELTESQKYNFRN
DGRCHYMTIHNVTPDDEGVYSVIARLEPRGEARSTAELYLTTKEIKLELKPPDIPDSRVPIPTMPIRA
VPPEEIPPVVAPPIPLLLPTPEEKKPPPKRIEVTKKAVKKDAKKVVAKPKEMTPREEIVKKPPPPTTLI
PAKAPEIIDVSSKAEEVKIMTITRKKEVQKEKEAVYEKKQAVHKEKRVFIESFEEPYDELEVEPYTEP
FEQPYYEEPDEDYEEIKVEAKKEVHEEWEEDFEEGQEYYEREEGYDEGEEEWEEAYQEREVIQV
QKEVYEESHERKVPAKVPEKKAPPPPKVIKKPVIEKIEKTSRRMEEEKVQVTKVPEVSKKIVPQKP
SRTPVQEEVIEVKVPAVHTKKMVISEEKMFFASHTEEEVSVTVPEVQKEIVTEEKIHVAVSKRVEPP
PKVPELPEKPAPEEVAPVPIPKKVEPPAPKVPEVPKKPVPEEKKPVPVPKKEPAAPPKVPEVPKKP
VPEEKIPVPVAKKKEAPPAKVPEVQKRVVTEEKITIVTQREESPPPAVPEIPKKKVPEERKPVPRKE
EEVPPPPKVPALPKKPVPEEKVAVPVPVAKKAPPPRAEVSKKTVVEEKRFVAEEKLSFAVPQRVE
VTRHEVSAEEEWSYSEEEEGVSISVYREEEREEEEEAEVTEYEVMEEPEEYVVEEKLHIISKRVEA
EPAEVTERQEKKIVLKPKIPAKIEEPPPAKVPEAPKKIVPEKKVPAPVPKKEKVPPPKVPEEPKKPV
PEKKVPPKVIKMEEPLPAKVTERHMQITQEEKVLVAVTKKEAPPKARVPEEPKRAVPEEKVLKLKP
KREEEPPAKVTEFRKRVVKEEKVSIEAPKREPQPIKEVTIMEEKERAYTLEEEAVSVQREEEYEEY
EEYDYKEFEEYEPTEEYDQYEEYEEREYERYEEHEEYITEPEKPIPVKPVPEEPVPTKPKAPPAKV
LKKAVPEEKVPVPIPKKLKPPPPKVPEEPKKVFEEKIRISITKREKEQVTEPAAKVPMKPKRVVAEE
KVPVPRKEVAPPVRVPEVPKELEPEEVAFEEEVVTHVEEYLVEEEEEYIHEEEEFITEEEVVPVIPV
KVPEVPRKPVPEEKKPVPVPKKKEAPPAKVPEVPKKPEEKVPVLIPKKEKPPPAKVPEVPKKPVPE
EKVPVPVPKKVEAPPAKVPEVPKKPVPEKKVPVPAPKKVEAPPAKVPEVPKKLIPEEKKPTPVPKK
VEAPPPKVPKKREPVPVPVALPQEEEVLFEEEIVPEEEVLPEEEEVLPEEEEVLPEEEEVLPEEEEI

PPEEEEVPPEEEYVPEEEEFVPEEEVLPEVKPKVPVPAPVPEIKKKVTEKKVVIPKKEEAPPAKVPE
VPKKVEEKRIILPKEEEVLPVEVTEEPEEEPISEEEIPEEPPSIEEVEEVAPPRVPEVIKKAVPEAPTP
VPKKVEAPPAKVSKKIPEEKVPVPVQKKEAPPAKVPEVPKKVPEKKVLVPKKEAVPPAKGRTVLEE
KVSVAFRQEVVVKERLELEVVEAEVEEIPEEEEFHEVEEYFEEGEFHEVEEFIKLEQHRVEEEHRV
EKVHRVIEVFEAEEVEVFEKPKAPPKGPEISEKIIPPKKPPTKVVPRKEPPAKVPEVPKKIVVEEKVR
VPEEPRVPPTKVPDVLPPKEVVPEKKVPVPPAKKPEAPPPKVPEAPKEVVPEKKVPVPPPKKPEV
PPTKVPEVPKAAVPEKKVPEAIPPKPESPPPEVPEAPKEVVPEKKVPAAPPKKPEVTPVKVPEAPK
EVVPEKKVPVPPPKKPEVPPTKVPEVPKVAVPEKKVPEAIPPKPESPPPEVFEEPEEVALEEPPAE
VVEEPEPAAPPQVTVPPKKPVPEKKAPAVVAKKPELPPVKVPEVPKEVVPEKKVPLVVPKKPEAP
PAKVPEVPKEVVPEKKVAVPKKPEVPPAKVPEVPKKPVLEEKPAVPVPERAESPPPEVYEEPEEIA
PEEEIAPEEEKPVPVAEEEEPEVPPPAVPEEPKKIIPEKKVPVIKKPEAPPPKEPEPEKVIEKPKLKP
RPPPPPPAPPKEDVKEKIFQLKAIPKKKVPEKPQVPEKVELTPLKVPGGEKKVRKLLPERKPEPKE
EVVLKSVLRKRPEEEEPKVEPKKLEKVKKPAVPEPPPPKPVEEVEVPTVTKRERKIPEPTKVPEIKP
AIPLPAPEPKPKPEAEVKTIKPPPVEPEPTPIAAPVTVPVVGKKAEAKAPKEEAAKPKGPIKGVPKK
TPSPIEAERRKLRPGSGGEKPPDEAPFTYQLKAVPLKFVKEIKDIILTESEFVGSSAIFECLVSPSTAI
TTWMKDGSNIRESPKHRFIADGKDRKLHIIDVQLSDAGEYTCVLRLGNKEKTSTAKLVVEELPVRF
VKTLEEEVTVVKGQPLYLSCELNKERDVVWRKDGKIVVEKPGRIVPGVIGLMRALTINDADDTDAG
TYTVTVENANNLECSSCVKVVEVIRDWLVKPIRDQHVKPKGTAIFACDIAKDTPNIKWFKGYDEIPA
EPNDKTEILRDGNHLYLKIKNAMPEDIAEYAVEIEGKRYPAKLTLGEREVELLKPIEDVTIYEKESAS
FDAEISEADIPGQWKLKGELLRPSPTCEIKAEGGKRFLTLRKVKLDQAGEVLYQALNAITTAILTVKE
IELDFAVPLKDVTVPERRQARFECVLTREANVIWSKGPDIIKSSDKFDIIADGKKHILVINDSQFDDE
GVYTAEVEGKKTSARLFVTGIRLKFMSPLEDQTVKEGETATFVCELSHEKMHVVWFKNDAKLHTS
RTVLISSEGKTHKLEMKEVTLDDISQIKAQVKELSSTAQLKVLEADPYFTVKLHDKTAVEKDEITLKC
EVSKDVPVKWFKDGEEIVPSPKYSIKADGLRRILKIKKADLKDKGEYVCDCGTDKTKANVTVEARLI
KVEKPLYGVEVFVGETAHFEIELSEPDVHGQWKLKGQPLTASPDCEIIEDGKKHILILHNCQLGMT
GEVSFQAANAKSAANLKVKELPLIFITPLSDVKVFEKDEAKFECEVSREPKTFRWLKGTQEITGDD
RFELIKDGTKHSMVIKSAAFEDEAKYMFEAEDKHTSGKLIIEGIRLKFLTPLKDVTAKEKESAVFTVE
LSHDNIRVKWFKNDQRLHTTRSVSMQDEGKTHSITFKDLSIDDTSQIRVEAMGMSSEAKLTVLEG
DPYFTGKLQDYTGVEKDEVILQCEISKADAPVKWFKDGKEIKPSKNAVIKADGKKRMLILKKALKSD
IGQYTCDCGTDKTSGKLDIEDREIKLVRPLHSVEVMETETARFETEISEDDIHANWKLKGEALLQTP
DCEIKEEGKIHSLVLHNCRLDQTGGVDFQAANVKSSAHLRVKPRVIGLLRPLKDVTVTAGETATFD
CELSYEDIPVEWYLKGKKLEPSDKVVPRSEGKVHTLTLRDVKLEDAGEVQLTAKDFKTHANLFVK
EPPVEFTKPLEDQTVEEGATAVLECEVSRENAKVKWFKNGTEILKSKKYEIVADGRVRKLVIHDCT
PEDIKTYTCDAKDFKTSCNLNVVPPHVEFLRPLTDLQVREKEMARFECELSRENAKVKWFKDGAE
IKKGKKYDIISKGAVRILVINKCLLDDEAEYSCEVRTARTSGMLTVLEEEAVFTKNLANIEVSETDTIK
LVCEVSKPGAEVIWYKGDEEIIETGRYEILTEGRKRILVIQNAHLEDAGNYNCRLPSSRTDGKVKVH
ELAAEFISKPQNLEILEGEKAEFVCSISKESFPVQWKRDDKTLESGDKYDVIADGKKRVLVVKDATL
QDMGTYVVMVGAARAAAHLTVIEKLRIVVPLKDTRVKEQQEVVFNCEVNTEGAKAKWFRNEEAIF
DSSKYIILQKDLVYTLRIRDAHLDDQANYNVSLTNHRGENVKSAANLIVEEEDLRIVEPLKDIETMEK
KSVTFWCKVNRLNVTLKWTKNGEEVPFDNRVSYRVDKYKHMLTIKDCGFPDEGEYIVTAGQDKS
VAELLIIEAPTEFVEHLEDQTVTEFDDAVFSCQLSREKANVKWYRNGREIKEGKKYKFEKDGSIHR
LIIKDCRLDDECEYACGVEDRKSRARLFVEEIPVEIIRPPQDILEAPGADVVFLAELNKDKVEVQWL
RNNMVVVQGDKHQMMSEGKIHRLQICDIKPRDQGEYRFIAKDKEARAKLELAAAPKIKTADQDLV
VDVGKPLTMVVPYDAYPKAEAEWFKENEPLSTKTIDTTAEQTSFRILEAKKGDKGRYKIVLQNKHG
KAEGFINLKVIDVPGPVRNLEVTETFDGEVSLAWEEPLTDGGSKIIGYVVERRDIKRKTWVLATDRA
ESCEFTVTGLQKGGVEYLFRVSARNRVGTGEPVETDNPVEARSKYDVPGPPLNVTITDVNRFGV
SLTWEPPEYDGGAEITNYVIELRDKTSIRWDTAMTVRAEDLSATVTDVVEGQEYSFRVRAQNRIG
VGKPSAATPFVKVADPIERPSPPVNLTSSDQTQSSVQLKWEPPLKDGGSPILGYIIERCEEGKDN
WIRCNMKLVPELTYKVTGLEKGNKYLYRVSAENKAGVSDPSEILGPLTADDAFVEPTMDLSAFKD
GLEVIVPNPITILVPSTGYPRPTATWCFGDKVLETGDRVKMKTLSAYAELVISPSERSDKGIYTLKLE
NRVKTISGEIDVNVIARPSAPKELKFGDITKDSVHLTWEPPDDDGGSPLTGYVVEKREVSRKTWTK
VMDFVTDLEFTVPDLVQGKEYLFKVCARNKCGPGEPAYVDEPVNMSTPATVPDPPENVKWRDR
TANSIFLTWDPPKNDGGSRIKGYIVERCPRGSDKWVACGEPVAETKMEVTGLEEGKWYAYRVKA
LNRQGASKPSRPTEEIQAVDTQEAPEIFLDVKLLAGLTVKAGTKIELPATVTGKPEPKITWTKADMIL
KQDKRITIENVPKKSTVTIVDSKRSDTGTYIIEAVNVCGRATAVVEVNVLDKPGPPAAFDITDVTNES
CLLTWNPPRDDGGSKITNYVVERRATDSEVWHKLSSTVKDTNFKATKLIPNKEYIFRVAAENMYG
VGEPVQASPITAKYQFDPPGPPTRLEPSDITKDAVTLTWCEPDDDGGSPITGYWVERLDPDTDKW

VRCNKMPVKDTTYRVKGLTNKKKYRFRVLAENLAGPGKPSKSTEPILIKDPIDPPWPPGKPTVKDV
GKTSVRLNWTKPEHDGGAKIESYVIEMLKTGTDEWVRVAEGVPTTQHLLPGLMEGQEYSFRVRA
VNKAGESEPSEPSDPVLCREKLYPPSPPRWLEVINITKNTADLKWTVPEKDGGSPITNYIVEKRDV
RRKGWQTVDTTVKDTKCTVTPLTEGSLYVFRVAAENAIGQSDYTEIEDSVLAKDTFTTPGPPYALA
VVDVTKRHVDLKWEPPKNDGGRPIQRYVIEKKERLGTRWVKAGKTAGPDCNFRVTDVIEGTEVQ
FQVRAENEAGVGHPSEPTEILSIEDPTSPPSPPLDLHVTDAGRKHIAIAWKPPEKNGGSPIIGYHVE
MCPVGTEKWMRVNSRPIKDLKFKVEEGVVPDKEYVLRVRAVNAIGVSEPSEISENVVAKDPDCKP
TIDLETHDIIVIEGEKLSIPVPFRAVPVPTVSWHKDGKEVKASDRLTMKNDHISAHLEVPKSVRADA
GIYTITLENKLGSATASINVKVIGLPGPCKDIKASDITKSSCKLTWEPPEFDGGTPILHYVLERREAG
RRTYIPVMSGENKLSWTVKDLIPNGEYFFRVKAVNKVGGGEYIELKNPVIAQDPKQPPDPPVDVE
VHNPTAEAMTITWKPPLYDGGSKIMGYIIEKIAKGEERWKRCNEHLVPILTYTAKGLEEGKEYQFR
VRAENAAGISEPSRATPPTKAVDPIDAPKVILRTSLEVKRGDEIALDASISGSPYPTITWIKDENVIVP
EEIKKRAAPLVRRRKGEVQEEEPFVLPLTQRLSIDNSKKGESQLRVRDSLRPDHGLYMIKVENDH
GIAKAPCTVSVLDTPGPPINFVFEDIRKTSVLCKWEPPLDDGGSEIINYTLEKKDKTKPDSEWIVVT
STLRHCKYSVTKLIEGKEYLFRVRAENRFGPGPPCVSKPLVAKDPFGPPDAPDKPIVEDVTSNSML
VKWNEPKDNGSPILGYWLEKREVNSTHWSRVNKSLLNALKANVDGLLEGLTYVFRVCAENAAGP
GKFSPPSDPKTAHDPISPPGPPIPRVTDTSSTTIELEWEPPAFNGGGEIVGYFVDKQLVGTNEWSR
CTEKMIKVRQYTVKEIREGADYKLRVSAVNAAGEGPPGETQPVTVAEPQEPPAVELDVSVKGGIQI
MAGKTLRIPAVVTGRPVPTKVWTKEEGELDKDRVVIDNVGTKSELIIKDALRKDHGRYVITATNSC
GSKFAAARVEVFDVPGPVLDLKPVVTNRKMCLLNWSDPEDDGGSEITGFIIERKDAKMHTWRQPI
ETERSKCDITGLLEGQEYKFRVIAKNKFGCGPPVEIGPILAVDPLGPPTSPERLTYTERTKSTITLD
WKEPRSNGGSPIQGYIIEKRRHDKPDFERVNKRLCPTTSFLVENLDEHQMYEFRVKAVNEIGESE
PSLPLNVVIQDDEVPPTIKLRLSVRGDTIKVKAGEPVHIPADVTGLPMPKIEWSKNETVIEKPTDALQ
ITKEEVSRSEAKTELSIPKAVREDKGTYTVTASNRLGSVFRNVHVEVYDRPSPPRNLAVTDIKAES
CYLTWDAPLDNGGSEITHYVIDKRDASRKKAEWEEVTNTAVEKRYGIWKLIPNGQYEFRVRAVNK
YGISDECKSDKVVIQDPYRLPGPPGKPKVLARTKGSMLVSWTPPLDNGGSPITGYWLEKREEGSP
YWSRVSRAPITKVGLKGVEFNVPRLLEGVKYQFRAMAINAAGIGPPSEPSDPEVAGDPIFPPGPPS
CPEVKDKTKSSISLGWKPPAKDGGSPIKGYIVEMQEEGTTDWKRVNEPDKLITTCECVVPNLKEL
RKYRFRVKAVNEAGESEPSDTTGEIPATDIQEEPEVFIDIGAQDCLVCKAGSQIRIPAVIKGRPTPK
SSWEFDGKAKKAMKDGVHDIPEDAQLETAENSSVIIIPECKRSHTGKYSITAKNKAGQKTANCRVK
VMDVPGPPKDLKVSDITRGSCRLSWKMPDDDGGDRIKGYVIEKRTIDGKAWTKVNPDCGSTTFV
VPDLLSEQQYFFRVRAENRFGIGPPVETIQRTTARDPIYPPDPPIKLKIGLITKNTVHLSWKPPKND
GGSPVTHYIVECLAWDPTGTKKEAWRQCNKRDVEELQFTVEDLVEGGEYEFRVKAVNAAGVSKP
SATVGPCDCQRPDMPPSIDLKEFMEVEEGTNVNIVAKIKGVPFPTLTWFKAPPKKPDNKEPVLYD
THVNKLVVDDTCTLVIPQSRRSDTGLYTITAVNNLGTASKEMRLNVLGRPGPPVGPIKFESVSADQ
MTLSWFPPKDDGGSKITNYVIEKREANRKTWVHVSSEPKECTYTIPKLLEGHEYVFRIMAQNKYGI
GEPLDSEPETARNLFSVPGAPDKPTVSSVTRNSMTVNWEEPEYDGGSPVTGYWLEMKDTTSKR
WKRVNRDPIKAMTLGVSYKVTGLIEGSDYQFRVYAINAAGVGPASLPSDPATARDPIAPPGPPFPK
VTDWTKSSADLEWSPPLKDGGSKVTGYIVEYKEEGKEEWEKGKDKEVRGTKLVVTGLKEGAFYK
FRVSAVNIAGIGEPGEVTDVIEMKDRLVSPDLQLDASVRDRIVVHAGGVIRIIAYVSGKPPPTVTWN
MNERTLPQEATIETTAISSSMVIKNCQRSHQGVYSLLAKNEAGERKKTIIVDVLDVPGPVGTPFLAH
NLTNESCKLTWFSPEDDGGSPITNYVIEKRESDRRAWTPVTYTVTRQNATVQGLIQGKAYFFRIAA
ENSIGMGPFVETSEALVIREPITVPERPEDLEVKEVTKNTVTLTWNPPKYDGGSEIINYVLESRLIGT
EKFHKVTNDNLLSRKYTVKGLKEGDTYEYRVSAVNIVGQGKPSFCTKPITCKDELAPPTLHLDFRD
KLTIRVGEAFALTGRYSGKPKPKVSWFKDEADVLEDDRTHIKTTPATLALEKIKAKRSDSGKYCVV
VENSTGSRKGFCQVNVVDRPGPPVGPVSFDEVTKDYMVISWKPPLDDGGSKITNYIIEKKEVGKD
VWMPVTSASAKTTCKVSKLLEGKDYIFRIHAENLYGISDPLVSDSMKAKDRFRVPDAPDQPIVTEV
TKDSALVTWNKPHDGGKPITNYILEKRETMSKRWARVTKDPIHPYTKFRVPDLLEGCQYEFRVSA
ENEIGIGDPSPPSKPVFAKDPIAKPSPPVNPEAIDTTCNSVDLTWQPPRHDGGSKILGYIVEYQKVG
DEEWRRANHTPESCPETKYKVTGLRDGQTYKFRVLAVNAAGESDPAHVPEPVLVKDRLEPPELIL
DANMAREQHIKVGDTLRLSAIIKGVPFPKVTWKKEDRDAPTKARIDVTPVGSKLEIRNAAHEDGGIY
SLTVENPAGSKTVSVKVLVLDKPGPPRDLEVSEIRKDSCYLTWKEPLDDGGSVITNYVVERRDVA
SAQWSPLSATSKKKSHFAKHLNEGNQYLFRVAAENQYGRGPFVETPKPIKALDPLHPPGPPKDLH
HVDVDKTEVSLVWNKPDRDGGSPITGYLVEYQEEGTQDWIKFKTVTNLECVVTGLQQGKTYRFR
VKAENIVGLGLPDTTIPIECQEKLVPPSVELDVKLIEGLVVKAGTTVRFPAIIRGVPVPTAKWTTDGS
EIKTDEHYTVETDNFSSVLTIKNCLRRDTGEYQITVSNAAGSKTVAVHLTVLDVPGPPTGPINILDVT
PEHMTISWQPPKDDGGSPVINYIVEKQDTRKDTWGVVSSGSSKTKLKIPHLQKGCEYVFRVRAEN

KIGVGPPLDSTPTVAKHKFSPPSPPGKPVVTDITENAATVSWTLPKSDGGSPITGYYMERREVTG
KWVRVNKTPIADLKFRVTGLYEGNTYEFRVFAENLAGLSKPSPSSDPIKACRPIKPPGPPINPKLKD
KSRETADLVWTKPLSDGGSPILGYVVECQKPGTAQWNRINKDELIRQCAFRVPGLIEGNEYRFRIK
AANIVGEGEPRELAESVIAKDILHPPEVELDVTCRDVITVRVGQTIRILARVKGRPEPDITWTKEGKV
LVREKRVDLIQDLPRVELQIKEAVRADHGKYIISAKNSSGHAQGSAIVNVLDRPGPCQNLKVTNVT
KENCTISWENPLDNGGSEITNFIVEYRKPNQKGWSIVASDVTKRLIKANLLANNEYYFRVCAENKV
GVGPTIETKTPILAINPIDRPGEPENLHIADKGKTFVYLKWRRPDYDGGSPNLSYHVERRLKGSDD
WERVHKGSIKETHYMVDRCVENQIYEFRVQTKNEGGESDWVKTEEVVVKEDLQKPVLDLKLSGV
LTVKAGDTIRLEAGVRGKPFPEVAWTKDKDATDLTRSPRVKIDTRADSSKFSLTKAKRSDGGKYV
VTATNTAGSFVAYATVNVLDKPGPVRNLKIVDVSSDRCTVCWDPPEDDGGCEIQNYILEKCETKR
MVWSTYSATVLTPGTTVTRLIEGNEYIFRVRAENKIGTGPPTESKPVIAKTKYDKPGRPDPPEVTK
VSKEEMTVVWNPPEYDGGKSITGYFLEKKEKHSTRWVPVNKSAIPERRMKVQNLLPDHEYQFRV
KAENEIGIGEPSLPSRPVVAKDPIEPPGPPTNFRVVDTTKHSITLGWGKPVYDGGAPIIGYVVEMRP
KIADASPDEGWKRCNAAAQLVRKEFTVTSLDENQEYEFRVCAQNQVGIGRPAELKEAIKPKEILEP
PEIDLDASMRKLVIVRAGCPIRLFAIVRGRPAPKVTWRKVGIDNVVRKGQVDLVDTMAFLVIPNSTR
DDSGKYSLTLVNPAGEKAVFVNVRVLDTPGPVSDLKVSDVTKTSCHVSWAPPENDGGSQVTHYI
VEKREADRKTWSTVTPEVKKTSFHVTNLVPGNEYYFRVTAVNEYGPGVPTDVPKPVLASDPLSEP
DPPRKLEVTEMTKNSATLAWLPPLRDGGAKIDGYITSYREEEQPADRWTEYSVVKDLSLVVTGLK
EGKKYKFRVAARNAVGVSLPREAEGVYEAKEQLLPPKILMPEQITIKAGKKLRIEAHVYGKPHPTC
KWKKGEDEVVTSSHLAVHKADSSSILIIKDVTRKDSGYYSLTAENSSGTDTQKIKVVVMDAPGPPQ
PPFDISDIDADACSLSWHIPLEDGGSNITNYIVEKCDVSRGDWVTALASVTKTSCRVGKLIPGQEYI
FRVRAENRFGISEPLTSPKMVAQFPFGVPSEPKNARVTKVNKDCIFVAWDRPDSDGGSPIIGYLIE
RKERNSLLWVKANDTLVRSTEYPCAGLVEGLEYSFRIYALNKAGSSPPSKPTEYVTARMPVDPPG
KPEVIDVTKSTVSLIWARPKHDGGSKIIGYFVEACKLPGDKWVRCNTAPHQIPQEEYTATGLEEKA
QYQFRAIARTAVNISPPSEPSDPVTILAENVPPRIDLSVAMKSLLTVKAGTNVCLDATVFGKPMPTV
SWKKDGTLLKPAEGIKMAMQRNLCTLELFSVNRKDSGDYTITAENSSGSKSATIKLKVLDKPGPPA
SVKINKMYSDRAMLSWEPPLEDGGSEITNYIVDKRETSRPNWAQVSATVPITSCSVEKLIEGHEYQ
FRICAENKYGVGDPVFTEPAIAKNPYDPPGRCDPPVISNITKDHMTVSWKPPADDGGSPITGYLLE
KRETQAVNWTKVNRKPIIERTLKATGLQEGTEYEFRVTAINKAGPGKPSDASKAAYARDPQYPPA
PPAFPKVYDTTRSSVSLSWGKPAYDGGSPIIGYLVEVKRADSDNWVRCNLPQNLQKTRFEVTGL
MEDTQYQFRVYAVNKIGYSDPSDVPDKHYPKDILIPPEGELDADLRKTLILRAGVTMRLYVPVKGR
PPPKITWSKPNVNLRDRIGLDIKSTDFDTFLRCENVNKYDAGKYILTLENSCGKKEYTIVVKVLDTP
GPPVNVTVKEISKDSAYVTWEPPIIDGGSPIINYVVQKRDAERKSWSTVTTECSKTSFRVANLEEG
KSYFFRVFAENEYGIGDPGETRDAVKASQTPGPVVDLKVRSVSKSSCSIGWKKPHSDGGSRIIGY
VVDFLTEENKWQRVMKSLSLQYSAKDLTEGKEYTFRVSAENENGEGTPSEITVVARDDVVAPDLD
LKGLPDLCYLAKENSNFRLKIPIKGKPAPSVSWKKGEDPLATDTRVSVESSAVNTTLIVYDCQKSD
AGKYTITLKNVAGTKEGTISIKVVGKPGIPTGPIKFDEVTAEAMTLKWAPPKDDGGSEITNYILEKRD
SVNNKWVTCASAVQKTTFRVTRLHEGMEYTFRVSAENKYGVGEGLKSEPIVARHPFDVPDAPPP
PNIVDVRHDSVSLTWTDPKKTGGSPITGYHLEFKERNSLLWKRANKTPIRMRDFKVTGLTEGLEY
EFRVMAINLAGVGKPSLPSEPVVALDPIDPPGKPEVINITRNSVTLIWTEPKYDGGHKLTGYIVEKR
DLPSKSWMKANHVNVPECAFTVTDLVEGGKYEFRIRAKNTAGAISAPSESTETIICKDEYEAPTIVL
DPTIKDGLTIKAGDTIVLNAISILGKPLPKSSWSKAGKDIRPSDITQITSTPTSSMLTIKYATRKDAGE
YTITATNPFGTKVEHVKVTVLDVPGPPGPVEISNVSAEKATLTWTPPLEDGGSPIKSYILEKRETSR
LLWTVVSEDIQSCRHVATKLIQGNEYIFRVSAVNHYGKGEPVQSEPVKMVDRFGPPGPPEKPEVS
NVTKNTATVSWKRPVDDGGSEITGYHVERREKKSLRWVRAIKTPVSDLRCKVTGLQEGSTYEFR
VSAENRAGIGPPSEASDSVLMKDAAYPPGPPSNPHVTDTTKKSASLAWGKPHYDGGLEITGYVV
EHQKVGDEAWIKDTTGTALRITQFVVPDLQTKEKYNFRISAINDAGVGEPAVIPDVEIVEREMAPDF
ELDAELRRTLVVRAGLSIRIFVPIKGRPAPEVTWTKDNINLKNRANIENTESFTLLIIPECNRYDTGKF
VMTIENPAGKKSGFVNVRVLDTPGPVLNLRPTDITKDSVTLHWDLPLIDGGSRITNYIVEKREATRK
SYSTATTKCHKCTYKVTGLSEGCEYFFRVMAENEYGIGEPTETTEPVKASEAPSPPDSLNIMDITK
STVSLAWPKPKHDGGSKITGYVIEAQRKGSDQWTHITTVKGLECVVRNLTEGEEYTFQVMAVNSA
GRSAPRESRPVIVKEQTMLPELDLRGIYQKLVIAKAGDNIKVEIPVLGRPKPTVTWKKGDQILKQTQ
RVNFETTATSTILNINECVRSDSGPYPLTARNIVGEVGDVITIQVHDIPGPPTGPIKFDEVSSDFVTF
SWDPPENDGGVPISNYVVEMRQTDSTTWVELATTVIRTTYKATRLTTGLEYQFRVKAQNRYGVG
PGITSACIVANYPFKVPGPPGTPQVTAVTKDSMTISWHEPLSDGGSPILGYHVERKERNGILWQTV
SKALVPGNIFKSSGLTDGIAYEFRVIAENMAGKSKPSKPSEPMLALDPIDPPGKPVPLNITRHTVTL
KWAKPEYTGGFKITSYIVEKRDLPNGRWLKANFSNILENEFTVSGLTEDAAYEFRVIAKNAAGAISP

PSEPSDAITCRDDVEAPKIKVDVKFKDTVILKAGEAFRLEADVSGRPPPTMEWSKDGKELEGTAKL
EIKIADFSTNLVNKDSTRRDSGAYTLTATNPGGFAKHIFNVKVLDRPGPPEGPLAVTEVTSEKCVLS
WFPPLDDGGAKIDHYIVQKRETSRLAWTNVASEVQVTKLKVTKLLKGNEYIFRVMAVNKYGVGEP
LESEPVLAVNPYGPPDPPKNPEVTTITKDSMVVCWGHPDSDGGSEIINYIVERRDKAGQRWIKCN
KKTLTDLRYKVSGLTEGHEYEFRIMAENAAGISAPSPTSPFYKACDTVFKPGPPGNPRVLDTSRSS
ISIAWNKPIYDGGSEITGYMVEIALPEEDEWQIVTPPAGLKATSYTITGLTENQEYKIRIYAMNSEGL
GEPALVPGTPKAEDRMLPPEIELDADLRKVVTIRACCTLRLFVPIKGRPAPEVKWARDHGESLDKA
SIESTSSYTLLIVGNVNRFDSGKYILTVENSSGSKSAFVNVRVLDTPGPPQDLKVKEVTKTSVTLTW
DPPLLDGGSKIKNYIVEKRESTRKAYSTVATNCHKTSWKVDQLQEGCSYYFRVLAENEYGIGLPA
ETAESVKASERPLPPGKITLMDVTRNSVSLSWEKPEHDGGSRILGYIVEMQTKGSDKWATCATVK
VTEATITGLIQGEEYSFRVSAQNEKGISDPRQLSVPVIAKDLVIPPAFKLLFNTFTVLAGEDLKVDVP
FIGRPTPAVTWHKDNVPLKQTTRVNAESTENNSLLTIKDACREDVGHYVVKLTNSAGEAIETLNVIV
LDKPGPPTGPVKMDEVTADSITLSWGPPKYDGGSSINNYIVEKRDTSTTTWQIVSATVARTTIKAC
RLKTGCEYQFRIAAENRYGKSTYLNSEPTVAQYPFKVPGPPGTPVVTLSSRDSMEVQWNEPISD
GGSRVIGYHLERKERNSILWVKLNKTPIPQTKFKTTGLEEGVEYEFRVSAENIVGIGKPSKVSECYV
ARDPCDPPGRPEAIIVTRNSVTLQWKKPTYDGGSKITGYIVEKKELPEGRWMKASFTNIIDTHFEVT
GLVEDHRYEFRVIARNAAGVFSEPSESTGAITARDEVDPPRISMDPKYKDTIVVHAGESFKVDADI
YGKPIPTIQWIKGDQELSNTARLEIKSTDFATSLSVKDAVRVDSGNYILKAKNVAGERSVTVNVKVL
DRPGPPEGPVVISGVTAEKCTLAWKPPLQDGGSDIINYIVERRETSRLVWTVVDANVQTLSCKVTK
LLEGNEYTFRIMAVNKYGVGEPLESEPVVAKNPFVVPDAPKAPEVTTVTKDSMIVVWERPASDGG
SEILGYVLEKRDKEGIRWTRCHKRLIGELRLRVTGLIENHDYEFRVSAENAAGLSEPSPPSAYQKA
CDPIYKPGPPNNPKVIDITRSSVFLSWSKPIYDGGCEIQGYIVEKCDVSVGEWTMCTPPTGINKTNI
EVEKLLEKHEYNFRICAINKAGVGEHADVPGPIIVEEKLEAPDIDLDLELRKIINIRAGGSLRLFVPIKG
RPTPEVKWGKVDGEIRDAAIIDVTSSFTSLVLDNVNRYDSGKYTLTLENSSGTKSAFVTVRVLDTP
SPPVNLKVTEITKDSVSITWEPPLLDGGSKIKNYIVEKREATRKSYAAVVTNCHKNSWKIDQLQEG
CSYYFRVTAENEYGIGLPAQTADPIKVAEVPQPPGKITVDDVTRNSVSLSWTKPEHDGGSKIIQYIV
EMQAKHSEKWSECARVKSLQAVITNLTQGEEYLFRVVAVNEKGRSDPRSLAVPIVAKDLVIEPDV
KPAFSSYSVQVGQDLKIEVPISGRPKPTITWTKDGLPLKQTTRINVTDSLDLTTLSIKETHKDDGGQ
YGITVANVVGQKTASIEIVTLDKPDPPKGPVKFDDVSAESITLSWNPPLYTGGCQITNYIVQKRDTT
TTVWDVVSATVARTTLKVTKLKTGTEYQFRIFAENRYGQSFALESDPIVAQYPYKEPGPPGTPFAT
AISKDSMVIQWHEPVNNGGSPVIGYHLERKERNSILWTKVNKTIIHDTQFKAQNLEEGIEYEFRVYA
ENIVGVGKASKNSECYVARDPCDPPGTPEPIMVKRNEITLQWTKPVYDGGSMITGYIVEKRDLPD
GRWMKASFTNVIETQFTVSGLTEDQRYEFRVIAKNAAGAISKPSDSTGPITAKDEVELPRISMDPK
FRDTIVVNAGETFRLEADVHGKPLPTIEWLRGDKEIEESARCEIKNTDFKALLIVKDAIRIDGGQYILR
ASNVAGSKSFPVNVKVLDRPGPPEGPVQVTGVTSEKCSLTWSPPLQDGGSDISHYVVEKRETSR
LAWTVVASEVVTNSLKVTKLLEGNEYVFRIMAVNKYGVGEPLESAPVLMKNPFVLPGPPKSLEVT
NIAKDSMTVCWNRPDSDGGSEIIGYIVEKRDRSGIRWIKCNKRRITDLRLRVTGLTEDHEYEFRVS
AENAAGVGEPSPATVYYKACDPVFKPGPPTNAHIVDTTKNSITLAWGKPIYDGGSEILGYVVEICK
ADEEEWQIVTPQTGLRVTRFEISKLTEHQEYKIRVCALNKVGLGEATSVPGTVKPEDKLEAPELDL
DSELRKGIVVRAGGSARIHIPFKGRPTPEITWSREEGEFTDKVQIEKGVNYTQLSIDNCDRNDAGK
YILKLENSSGSKSAFVTVKVLDTPGPPQNLAVKEVRKDSAFLVWEPPIIDGGAKVKNYVIDKRESTR
KAYANVSSKCSKTSFKVENLTEGAIYYFRVMAENEFGVGVPVETVDAVKAAEPPSPPGKVTLTDV
SQTSASLMWEKPEHDGGSRVLGYVVEMQPKGTEKWSIVAESKVCNAVVTGLSSGQEYQFRVKA
YNEKGKSDPRVLGVPVIAKDLTIQPSLKLPFNTYSIQAGEDLKIEIPVIGRPRPNISWVKDGEPLKQT
TRVNVEETATSTVLHIKEGNKDDFGKYTVTATNSAGTATENLSVIVLEKPGPPVGPVRFDEVSADF
VVISWEPPAYTGGCQISNYIVEKRDTTTTTWHMVSATVARTTIKITKLKTGTEYQFRIFAENRYGKS
APLDSKAVIVQYPFKEPGPPGTPFVTSISKDQMLVQWHEPVNDGGTKIIGYHLEQKEKNSILWVKL
NKTPIQDTKFKTTGLDEGLEYEFKVSAENIVGIGKPSKVSECFVARDPCDPPGRPEAIVITRNNVTL
KWKKPAYDGGSKITGYIVEKKDLPDGRWMKASFTNVLETEFTVSGLVEDQRYEFRVIARNAAGNF
SEPSDSSGAITARDEIDAPNASLDPKYKDVIVVHAGETFVLEADIRGKPIPDVVWSKDGKELEETAA
RMEIKSTIQKTTLVVKDCIRTDGGQYILKLSNVGGTKSIPITVKVLDRPGPPEGPLKVTGVTAEKCYL
AWNPPLQDGGANISHYIIEKRETSRLSWTQVSTEVQALNYKVTKLLPGNEYIFRVMAVNKYGIGEP
LESGPVTACNPYKPPGPPSTPEVSAITKDSMVVTWARPVDDGGTEIEGYILEKRDKEGVRWTKCN
KKTLTDLRLRVTGLTEGHSYEFRVAAENAAGVGEPSEPSVFYRACDALYPPGPPSNPKVTDTSRS
SVSLAWSKPIYDGGAPVKGYVVEVKEAAADEWTTCTPPTGLQGKQFTVTKLKENTEYNFRICAIN
SEGVGEPATLPGSVVAQERIEPPEIELDADLRKVVVLRASATLRLFVTIKGRPEPEVKWEKAEGILT
DRAQIEVTSSFTMLVIDNVTRFDSGRYNLTLENNSGSKTAFVNVRVLDSPSAPVNLTIREVKKDSV

TLSWEPPLIDGGAKITNYIVEKRETTRKAYATITNNCTKTTFRIENLQEGCSYYFRVLASNEYGIGLP
AETTEPVKVSEPPLPPGRVTLVDVTRNTATIKWEKPESDGGSKITGYVVEMQTKGSEKWSTCTQV
KTLEATISGLTAGEEYVFRVAAVNEKGRSDPRQLGVPVIARDIEIKPSVELPFHTFNVKAREQLKID
VPFKGRPQATVNWRKDGQTLKETTRVNVSSSKTVTSLSIKEASKEDVGTYELCVSNSAGSITVPITI
IVLDRPGPPGPIRIDEVSCDSITISWNPPEYDGGCQISNYIVEKKETTSTTWHIVSQAVARTSIKIVRL
TTGSEYQFRVCAENRYGKSSYSESSAVVAEYPFSPPGPPGTPKVVHATKSTMLVTWQVPVNDG
GSRVIGYHLEYKERSSILWSKANKILIADTQMKVSGLDEGLMYEYRVYAENIAGIGKCSKSCEPVPA
RDPCDPPGQPEVTNITRKSVSLKWSKPHYDGGAKITGYIVERRELPDGRWLKCNYTNIQETYFEV
TELTEDQRYEFRVFARNAADSVSEPSESTGPIIVKDDVEPPRVMMDVKFRDVIVVKAGEVLKINADI
AGRPLPVISWAKDGIEIEERARTEIISTDNHTLLTVKDCIRRDTGQYVLTLKNVAGTRSVAVNCKVL
DKPGPPAGPLEINGLTAEKCSLSWGRPQEDGGADIDYYIVEKRETSHLAWTICEGELQMTSCKVT
KLLKGNEYIFRVTGVNKYGVGEPLESVAIKALDPFTVPSPPTSLEITSVTKESMTLCWSRPESDGG
SEISGYIIERREKNSLRWVRVNKKPVYDLRVKSTGLREGCEYEYRVYAENAAGLSLPSETSPLIRA
EDPVFLPSPPSKPKIVDSGKTTITIAWVKPLFDGGAPITGYTVEYKKSDDTDWKTSIQSLRGTEYTIS
GLTTGAEYVFRVKSVNKVGASDPSDSSDPQIAKEREEEPLFDIDSEMRKTLIVKAGASFTMTVPFR
GRPVPNVLWSKPDTDLRTRAYVDTTDSRTSLTIENANRNDSGKYTLTIQNVLSAASLTLVVKVLDT
PGPPTNITVQDVTKESAVLSWDVPENDGGAPVKNYHIEKREASKKAWVSVTNNCNRLSYKVTNL
QEGAIYYFRVSGENEFGVGIPAETKEGVKITEKPSPPEKLGVTSISKDSVSLTWLKPEHDGGSRIV
HYVVEALEKGQKNWVKCAVAKSTHHVVSGLRENSEYFFRVFAENQAGLSDPRELLLPVLIKEQLE
PPEIDMKNFPSHTVYVRAGSNLKVDIPISGKPLPKVTLSRDGVPLKATMRFNTEITAENLTINLKESV
TADAGRYEITAANSSGTTKAFINIVVLDRPGPPTGPVVISDITEESVTLKWEPPKYDGGSQVTNYILL
KRETSTAVWTEVSATVARTMMKVMKLTTGEEYQFRIKAENRFGISDHIDSACVTVKLPYTTPGPPS
TPWVTNVTRESITVGWHEPVSNGGSAVVGYHLEMKDRNSILWQKANKLVIRTTHFKVTTISAGLIY
EFRVYAENAAGVGKPSHPSEPVLAIDACEPPRNVRITDISKNSVSLSWQQPAFDGGSKITGYIVER
RDLPDGRWTKASFTNVTETQFIISGLTQNSQYEFRVFARNAVGSISNPSEVVGPITCIDSYGGPVID
LPLEYTEVVKYRAGTSVKLRAGISGKPAPTIEWYKDDKELQTNALVCVENTTDLASILIKDADRLNS
GCYELKLRNAMGSASATIRVQILDKPGPPGGPIEFKTVTAEKITLLWRPPADDGGAKITHYIVEKRE
TSRVVWSMVSEHLEECIITTTKIIKGNEYIFRVRAVNKYGIGEPLESDSVVAKNAFVTPGPPGIPEVT
KITKNSMTVVWSRPIADGGSDISGYFLEKRDKKSLGWFKVLKETIRDTRQKVTGLTENSDYQYRV
CAVNAAGQGPFSEPSEFYKAADPIDPPGPPAKIRIADSTKSSITLGWSKPVYDGGSAVTGYVVEIR
QGEEEEWTTVSTKGEVRTTEYVVSNLKPGVNYYFRVSAVNCAGQGEPIEMNEPVQAKDILEAPEI
DLDVALRTSVIAKAGEDVQVLIPFKGRPPPTVTWRKDEKNLGSDARYSIENTDSSSLLTIPQVTRN
DTGKYILTIENGVGEPKSSTVSVKVLDTPAACQKLQVKHVSRGTVTLLWDPPLIDGGSPIINYVIEK
RDATKRTWSVVSHKCSSTSFKLIDLSEKTPFFFRVLAENEIGIGEPCETTEPVKAAEVPAPIRDLSM
KDSTKTSVILSWTKPDFDGGSVITEYVVERKGKGEQTWSHAGISKTCEIEVSQLKEQSVLEFRVFA
KNEKGLSDPVTIGPITVKELIITPEVDLSDIPGAQVTVRIGHNVHLELPYKGKPKPSISWLKDGLPLK
ESEFVRFSKTENKITLSIKNAKKEHGGKYTVILDNAVCRIAVPITVITLGPPSKPKGPIRFDEIKADSVI
LSWDVPEDNGGGEITCYSIEKRETSQTNWKMVCSSVARTTFKVPNLVKDAEYQFRVRAENRYGV
SQPLVSSIIVAKHQFRIPGPPGKPVIYNVTSDGMSLTWDAPVYDGGSEVTGFHVEKKERNSILWQK
VNTSPISGREYRATGLVEGLDYQFRVYAENSAGLSSPSDPSKFTLAVSPVDPPGTPDYIDVTRETI
TLKWNPPLRDGGSKIVGYSIEKRQGNERWVRCNFTDVSECQYTVTGLSPGDRYEFRIIARNAVGT
ISPPSQSSGIIMTRDENVPPIVEFGPEYFDGLIIKSGESLRIKALVQGRPVPRVTWFKDGVEIEKRMN
MEITDVLGSTSLFVRDATRDHRGVYTVEAKNASGSAKAEIKVKVQDTPGKVVGPIRFTNITGEKMT
LWWDAPLNDGCAPITHYIIEKRETSRLAWALIEDKCEAQSYTAIKLINGNEYQFRVSAVNKFGVGR
PLDSDPVVAQIQYTVPDAPGIPEPSNITGNSITLTWARPESDGGSEIQQYILERREKKSTRWVKVIS
KRPISETRFKVTGLTEGNEYEFHVMAENAAGVGPASGISRLIKCREPVNPPGPPTVVKVTDTSKTT
VSLEWSKPVFDGGMEIIGYIIEMCKADLGDWHKVNAEACVKTRYTVTDLQAGEEYKFRVSAINGA
GKGDSCEVTGTIKAVDRLTAPELDIDANFKQTHVVRAGASIRLFIAYQGRPTPTAVWSKPDSNLSL
RADIHTTDSFSTLTVENCNRNDAGKYTLTVENNSGSKSITFTVKVLDTPGPPGPITFKDVTRGSATL
MWDAPLLDGGARIHHYVVEKREASRRSWQVISEKCTRQIFKVNDLAEGVPYYFRVSAVNEYGVG
EPYEMPEPIVATEQPAPPRRLDVVDTSKSSAVLAWLKPDHDGGSRITGYLLEMRQKGSDFWVEA
GHTKQLTFTVERLVEKTEYEFRVKAKNDAGYSEPREAFSSVIIKEPQIEPTADLTGITNQLITCKAGS
PFTIDVPISGRPAPKVTWKLEEMRLKETDRVSITTTKDRTTLTVKDSMRGDSGRYFLTLENTAGVK
TFSVTVVVIGRPGPVTGPIEVSSVSAESCVLSWGEPKDGGGTEITNYIVEKRESGTTAWQLVNSSV
KRTQIKVTHLTKYMEYSFRVSSENRFGVSKPLESAPIIAEHPFVPPSAPTRPEVYHVSANAMSIRW
EEPYHDGGSKIIGYWVEKKERNTILWVKENKVPCLECNYKVTGLVEGLEYQFRTYALNAAGVSKA
SEASRPIMAQNPVDAPGRPEVTDVTRSTVSLIWSAPAYDGGSKVVGYIIERKPVSEVGDGRWLKC

NYTIVSDNFFTVTALSEGDTYEFRVLAKNAAGVISKGSESTGPVTCRDEYAPPKAELDARLHGDLV
TIRAGSDLVLDAAVGGKPEPKIIWTKGDKELDLCEKVSLQYTGKRATAVIKFCDRSDSGKYTLTVK
NASGTKAVSVMVKVLDSPGPCGKLTVSRVTQEKCTLAWSLPQEDGGAEITHYIVERRETSRLNW
VIVEGECPTLSYVVTRLIKNNEYIFRVRAVNKYGPGVPVESEPIVARNSFTIPSPPGIPEEVGTGKEH
IIIQWTKPESDGGNEISNYLVDKREKKSLRWTRVNKDYVVYDTRLKVTSLMEGCDYQFRVTAVNA
AGNSEPSEASNFISCREPSYTPGPPSAPRVVDTTKHSISLAWTKPMYDGGTDIVGYVLEMQEKDT
DQWYRVHTNATIRNTEFTVPDLKMGQKYSFRVAAVNVKGMSEYSESIAEIEPVERIEIPDLELADD
LKKTVTIRAGASLRLMVSVSGRPPPVITWSKQGIDLASRAIIDTTESYSLLIVDKVNRYDAGKYTIEA
ENQSGKKSATVLVKVYDTPGPCPSVKVKEVSRDSVTITWEIPTIDGGAPVNNYIVEKREAAMRAFK
TVTTKCSKTLYRISGLVEGTMYYFRVLPENIYGIGEPCETSDAVLVSEVPLVPAKLEVVDVTKSTVT
LAWEKPLYDGGSRLTGYVLEACKAGTERWMKVVTLKPTVLEHTVTSLNEGEQYLFRIRAQNEKG
VSEPRETVTAVTVQDLRVLPTIDLSTMPQKTIHVPAGRPVELVIPIAGRPPPAASWFFAGSKLRESE
RVTVETHTKVAKLTIRETTIRDTGEYTLELKNVTGTTSETIKVIILDKPGPPTGPIKIDEIDATSITISWE
PPELDGGAPLSGYVVEQRDAHRPGWLPVSESVTRSTFKFTRLTEGNEYVFRVAATNRFGIGSYL
QSEVIECRSSIRIPGPPETLQIFDVSRDGMTLTWYPPEDDGGSQVTGYIVERKEVRADRWVRVNK
VPVTMTRYRSTGLTEGLEYEHRVTAINARGSGKPSRPSKPIVAMDPIAPPGKPQNPRVTDTTRTS
VSLAWSVPEDEGGSKVTGYLIEMQKVDQHEWTKCNTTPTKIREYTLTHLPQGAEYRFRVLACNA
GGPGEPAEVPGTVKVTEMLEYPDYELDERYQEGIFVRQGGVIRLTIPIKGKPFPICKWTKEGQDIS
KRAMIATSETHTELVIKEADRGDSGTYDLVLENKCGKKAVYIKVRVIGSPNSPEGPLEYDDIQVRSV
RVSWRPPADDGGADILGYILERREVPKAAWYTIDSRVRGTSLVVKGLKENVEYHFRVSAENQFGI
SKPLKSEEPVTPKTPLNPPEPPSNPPEVLDVTKSSVSLSWSRPKDDGGSRVTGYYIERKETSTDK
WVRHNKTQITTTMYTVTGLVPDAEYQFRIIAQNDVGLSETSPASEPVVCKDPFDKPSQPGELEILSI
SKDSVTLQWEKPECDGGKEILGYWVEYRQSGDSAWKKSNKERIKDKQFTIGGLLEATEYEFRVF
AENETGLSRPRRTAMSIKTKLTSGEAPGIRKEMKDVTTKLGEAAQLSCQIVGRPLPDIKWYRFGKE
LIQSRKYKMSSDGRTHTLTVMTEEQEDEGVYTCIATNEVGEVETSSKLLLQATPQFHPGYPLKEK
YYGAVGSTLRLHVMYIGRPVPAMTWFHGQKLLQNSENITIENTEHYTHLVMKNVQRKTHAGKYKV
QLSNVFGTVDAILDVEIQDKPDKPTGPIVIEALLKNSAVISWKPPADDGGSWITNYVVEKCEAKEGA
EWQLVSSAISVTTCRIVNLTENAGYYFRVSAQNTFGISDPLEVSSVVIIKSPFEKPGAPGKPTITAVT
KDSCVVAWKPPASDGGAKIRNYYLEKREKKQNKWISVTTEEIRETVFSVKNLIEGLEYEFRVKCEN
LGGESEWSEISEPITPKSDVPIQAPHFKEELRNLNVRYQSNATLVCKVTGHPKPIVKWYRQGKEIIA
DGLKYRIQEFKGGYHQLIIASVTDDDATVYQVRATNQGGSVSGTASLEVEVPAKIHLPKTLEGMGA
VHALRGEVVSIKIPFSGKPDPVITWQKGQDLIDNNGHYQVIVTRSFTSLVFPNGVERKDAGFYVVC
AKNRFGIDQKTVELDVADVPDPPRGVKVSDVSRDSVNLTWTEPASDGGSKITNYIVEKCATTAER
WLRVGQARETRYTVINLFGKTSYQFRVIAENKFGLSKPSEPSEPTITKEDKTRAMNYDEEVDETRE
VSMTKASHSSTKELYEKYMIAEDLGRGEFGIVHRCVETSSKKTYMAKFVKVKGTDQVLVKKEISIL
NIARHRNILHLHESFESMEELVMIFEFISGLDIFERINTSAFELNEREIVSYVHQVCEALQFLHSHNIG
HFDIRPENIIYQTRRSSTIKIIEFGQARQLKPGDNFRLLFTAPEYYAPEVHQHDVVSTATDMWSLGT
LVYVLLSGINPFLAETNQQIIENIMNAEYTFDEEAFKEISIEAMDFVDRLLVKERKSRMTASEALQHP
WLKQKIERVSTKVIRTLKHRRYYHTLIKKDLNMVVSAARISCGGAIRSQKGVSVAKVKVASIEIGPV
SGQIMHAVGEEGGHVKYVCKIENYDQSTQVTWYFGVRQLENSEKYEITYEDGVAILYVKDITKLDD
GTYRCKVVNDYGEDSSYAELFVKGVREVYDYYCRRTMKKIKRRTDTMRLLERPPEFTLPLYNKTA
YVGENVRFGVTITVHPEPHVTWYKSGQKIKPGDNDKKYTFESDKGLYQLTINSVTTDDDAEYTVV
ARNKYGEDSCKAKLTVTLHPPPTDSTLRPMFKRLLANAECQEGQSVCFEIRVSGIPPPTLKWEKD
GQPLSLGPNIEIIHEGLDYYALHIRDTLPEDTGYYRVTATNTAGSTSCQAHLQVERLRYKKQEFKSK
EEHERHVQKQIDKTLRMAEILSGTESVPLTQVAKEALREAAVLYKPAVSTKTVKGEFRLEIEEKKEE
RKLRMPYDVPEPRKYKQTTIEEDQRIKQFVPMSDMKWYKKIRDQYEMPGKLDRVVQKRPKRIRLS
RWEQFYVMPLPRITDQYRPKWRIPKLSQDDLEIVRPARRRTPSPDYDFYYRPRRRSLGDISDEEL
LLPIDDYLAMKRTEEERLRLEEELELGFSASPPSRSPPHFELSSLRYSSPQAHVKVEETRKDFRYS
TYHIPTKAEASTSYAELRERHAQAAYRQPKQRQRIMAEREDEELLRPVTTTQHLSEYKSELDFMS
KEEKSRKKSRRQREVTEITEIEEEYEISKHAQRESSSSASRLLRRRRSLSPTYIELMRPVSELIRSR
PQPAEEYEDDTERRSPTPERTRPRSPSPVSSERSLSRFERSARFDIFSRYESMKAALKTQKTSER
KYEVLSQQPFTLDHAPRITLRMRSHRVPCGQNTRFILNVQSKPTAEVKWYHNGVELQESSKIHYT
NTSGVLTLEILDCHTDDSGTYRAVCTNYKGEASDYATLDVTGGDYTTYASQRRDEEVPRSVFPEL
TRTEAYAVSSFKKTSEMEASSSVREVKSQMTETRESLSSYEHSASAEMKSAALEEKSLEEKSTTR
KIKTTLAARILTKPRSMTVYEGESARFSCDTDGEPVPTVTWLRKGQVLSTSARHQVTTTKYKSTFE
ISSVQASDEGNYSVVVENSEGKQEAEFTLTIQKARVTEKAVTSPPRVKSPEPRVKSPEAVKSPKR
VKSPEPSHPKAVSPTETKPTPTEKVQHLPVSAPPKITQFLKAEASKEIAKLTCVVESSVLRAKEVT

WYKDGKKLKENGHFQFHYSADGTYELKINNLTESDQGEYVCEISGEGGTSKTNLQFMGQAFKSIH
EKVSKISETKKSDQKTTESTVTRKTEPKAPEPISSKPVIVTGLQDTTVSSDSVAKFAVKATGEPRPT
AIWTKDGKAITQGGKYKLSEDKGGFFLEIHKTDTSDSGLYTCTVKNSAGSVSSSCKLTIKAIKDTEA
QKVSTQKTSEITPQKKAVVQEEISQKALRSEEIKMSEAKSQEKLALKEEASKVLISEEVKKSAATSL
EKSIVHEEITKTSQASEEVRTHAEIKAFSTQMSINEGQRLVLKANIAGATDVKWVLNGVELTNSEEY
RYGVSGSDQTLTIKQASHRDEGILTCISKTKEGIVKCQYDLTLSKELSDAPAFISQPRSQNINEGQN
VLFTCEISGEPSPEIEWFKNNLPISISSNVSISRSRNVYSLEIRNASVSDSGKYTIKAKNFRGQCSAT
ASLMVLPLVEEPSREVVLRTSGDTSLQGSFSSQSVQMSASKQEASFSSFSSSSASSMTEMKFAS
MSAQSMSSMQESFVEMSSSSFMGISNMTQLESSTSKMLKAGIRGIPPKIEALPSDISIDEGKVLTV
ACAFTGEPTPEVTWSCGGRKIHSQEQGRFHIENTDDLTTLIIMDVQKQDGGLYTLSLGNEFGSDS
ATVNIHIRSI (SEQ ID NO:394)

## Figure 174

**Nucleotide:** AF272363
**Sequence:**
atgtcagggatggaaaaacttcagaatgcttcctggatctaccagcagaaactagaagatccattccagaaacacctgaacagcaccg
aggagtatctggccttcctctgcggacctcggcgcagccacttcttcctccccgtgtctgtggtgtatgtgccaatttttgtggtgggggtcattg
gcaatgtcctggtgtgcctggtgattctgcagcaccaggctatgaagacgcccaccaactactacctcttcagcctggcggtctctgacctc
ctggtcctgctccttggaatgcccctggaggtctatgagatgtggcgcaactaccctttcttgttcgggcccgtgggctgctacttcaagacgg
ccctctttgagaccgtgtgcttcgcctccatcctcagcatcaccaccgtcagcgtggagcgctacgtggccatcctacacccgttccgcgcc
aaactgcagagcacccggcgccgggccctcaggatcctcggcatcgtctggggcttctccgtgctcttctccctgcccaacaccagcatc
catggcatcaagttccactacttccccaatgggtccctggtcccaggttcggccacctgtacggtcatcaagcccatgtggatctacaatttc
atcatccaggtcacctccttcctattctacctcctccccatgactgtcatcagtgtcctctactacctcatggcactcagactaaagaaagaca
aatctcttgaggcagatgaagggaatgcaaatattcaaagaccctgcagaaaatcagtcaacaagatgctgtttgtcttggtcttagtgtttg
ctatctgttgggccccgttccacattgaccgactcttcttcagctttgtggaggagtggagtgaatccctggctgctgtgttcaacctcgtccatg
tggtgtcaggtgtcttcttctacctgagctcagctgtcaaccccattatctataacctactgtctcgccgcttccaggcagcattccagaatgtg
atctcttctttccacaaacagtggcactcccagcatgacccacagttgccacctgcccagcggaacatcttcctgacagaatgccactttgt
ggagctgaccgaagatataggtccccaattcccatgtcagtcatccatgcacaactctcacctcccaacagccctctctagtgaacagatg
tcaagaacaaactatcaaagcttccactttaacaaaacctga (SEQ ID NO:395)

**Q9GZQ4**
MSGMEKLQNASWIYQQKLEDPFQKHLNSTEEYLAFLCGPRRSHFFLPVSVVYVPIFVVGVIGNVL
VCLVILQHQAMKTPTNYYLFSLAVSDLLVLLLGMPLEVYEMWRNYPFLFGPVGCYFKTALFETVCF
ASILSITTVSVERYVAILHPFRAKLQSTRRRALRILGIVWGFSVLFSLPNTSIHGIKFHYFPNGSLVPG
SATCTVIKPMWIYNFIIQVTSFLFYLLPMTVISVLYYLMALRLKKDKSLEADEGNANIQRPCRKSVNK
MLFVLVLVFAICWAPFHIDRLFFSFVEEWSESLAAVFNLVHVVSGVFFYLSSAVNPIIYNLLSRRFQ
AAFQNVISSFHKQWHSQHDPQLPPAQRNIFLTECHFVELTEDIGPQFPCQSSMHNSHLPTALSSE
QMSRTNYQSFHFNKT (SEQ ID NO:396)

# Figure 175

**Nucleotide:** AF045458
**Sequence:**
atggagcccggccgcggcggcacagagaccgtgggcaagttcgagttctcccgcaaggacctgatcggccacggcgccttcgcggtg
gtcttcaagggccgccaccgcgagaagcacgatttggaggtcgccgtcaagtgcattaacaagaagaacctcgccaagtctcagacgc
tgctggggaaggaaatcaaaatcctgaaggaactgaaacatgaaaacatcgtggccctgtacgacttccaggaaatggctaattctgtct
acctggttatggagtactgcaacggtggggacctggccgactacctgcacgccatgcgcacgctgagcgaggacaccatcaggctcttc
ctgcagcagatcgcgggcgccatgcggcttctgcacagcaaaggcatcatccaccgcgacctgaaaccgcagaacatcctgctgtcca
accccgccggccgccgcgccaaccccaacagcatccgcgtcaagatcgctgacttcggcttcgcgcggtacctccagagcaacatgat
ggcggccacactctgcggctcccccatgtacatggcccccgaggtcatcatgtcccagcactacgacgggaaggcggacctgtggagc
atcggcaccatcgtctaccagtgcctgacggggaaggcgcccttccaggccagcagcccccaggacctgcgcctgttctacgagaaga
acaagacgttggtccccaccatcccccgggagacctcggccccgctgcggcagctgctcctggccctactgcaacgcaaccacaagg
accgcatggacttcgatgagtttttcatcaccctttcctcgatgccagcccctcggtcaggaaatccccacccgtgcctgtgccctcgtaccc
aagctcggggtccggcagcagctccagcagcagctccacctcccacctggcctccccgccgtccctgggcgagatgcagcagctgca
gaagaccctggcctccccggctgacaccgctggcttcctgcacagctcccgggactctggtggcagcaaggactcttcctgtgacacag
acgacttcgtcatggtccccgcgcagtttccaggtgacctggtggctgaggcgcccagtgccaaacccccgccagacagcctgatgtgc
agtgggagctcactggtggcctctgcgggcttggagagccacggccggaccccatctccatccccaccctgcagcagctcccccagtcc
ctcaggccgggctggcccgttctccagcagcaggtgcggcgcctctgtccccatcccagtccccacgcaggtgcagaactaccagcgc
attgagcgaaacctgcagtcacccacccagttccaaacacctcggtcctctgccatccgcaggtcaggcagcaccagcccccctgggcttt
gcaagggccagcccctcgccccctgcccacgctgagcatggaggcgtcctggccaggaagatgtctctgggtggaggccggccctac
acgccatctcctcaagttggaaccatccctgagcggccaggctggagcgggacgccctccccacagggagctgagatgcggggtggc
aggtcccctcgtccaggctcctctgcacccgagcactctccccgcacttccgggctgggctgccgcctgcacagcgcccccaacctgtct
gacttgcacgtcgtccgccccaagctgcccaaacccccccacggacccctgggagctgtgttcagcccaccacaggccagccctcccc
agccgtcccacggcctgcagtcctgccggaacctgcggggctcacccaagctgcccgacttcctgcagcgaaacccccctgccccccat
cctgggctcccccaccaaggctgtgccctcctttgacttcccgaagacccccagctcccagaacctgctggccctcctagcccggcaggg
cgtggtgatgacgccccctcgaaaccggacgctgcccgacctctcggaggtgggacccttccatggtcagccgttgggccctggcctgc
ggccaggcgaggaccccaagggcccctttggccggtctttcagcaccagccgcctcactgacctgctccttaaggcggcgtttgggaca
caagccccggacccgggcagcacggagagcctgcaggagaagcccatggagatcgcaccctcagctggctttggagggagcctgc
acccaggagcccgtgctgggggcaccagcagcccctccccggtggtcttcaccgtgggctctcccccgagcgggagcacgcccccc
agggcccccgcaccaggatgttctcagcgggccccactggctctgccagctcttctgcccgccacctggtgcctgggccctgcagcgag
gccccagcccctgagctccctgctccaggacacggctgcagctttgccgaccccattgctgcgaacctggagggggctgtgaccttcga
ggcccccgacctccctgaggagaccctcatggagcaagagcacacggagatcctgcgtggcctgcgcttcacgctgctgttcgtgcagc
acgtcctggagatcgcagccctgaagggcagcgccagtgaggcggcgggggccctgagtaccagctgcaggagagtgtggtggcc
gaccagatcagcctgctgagccgagaatggggcttcgcggaacagctggtgctgtacctgaaggtggccgagctactgtcctccggcct
gcaaagtgccatcgaccagatccgggccggcaagctctgcctgtcgtccactgtgaagcaggtggtgcgcaggctgaatgagctgtaca
aggccagcgtggtgtcctgccagggcctgagcctgcggctgcagcgcttcttcctggacaagcagcggctcctggaccgcattcacagc
atcactgccgagaggctcatcttcagccacgctgtgcagatggtgcagtcggctgccctggacgagatgttccagcaccgtgagggctgc
gtcccacgctaccacaaggccctgctgctcctggagggctgcagcacatgctctcggaccaggccgacatcgagaacgtcaccaagt
gcaagctgtgcattgagcggagactctcggcgctgctgactggcatctgtgcctga (SEQ ID NO:397)

**O75385**
MEPGRGGTETVGKFEFSRKDLIGHGAFAVVFKGRHREKHDLEVAVKCINKKNLAKSQTLLGKEIKI
LKELKHENIVALYDFQEMANSVYLVMEYCNGGDLADYLHAMRTLSEDTIRLFLQQIAGAMRLLHSK
GIIHRDLKPQNILLSNPAGRRANPNSIRVKIADFGFARYLQSNMMAATLCGSPMYMAPEVIMSQHY
DGKADLWSIGTIVYQCLTGKAPFQASSPQDLRLFYEKNKTLVPTIPRETSAPLRQLLLALLQRNHK
DRMDFDEFFHHPFLDASPSVRKSPPVPVPSYPSSGSGSSSSSSSTSHLASPPSLGEMQQLQKTL
ASPADTAGFLHSSRDSGGSKDSSCDTDDFVMVPAQFPGDLVAEAPSAKPPPDSLMCSGSSLVAS
AGLESHGRTPSPSPPCSSSPSPSGRAGPFSSSRCGASVPIPVPTQVQNYQRIERNLQSPTQFQT
PRSSAIRRSGSTSPLGFARASPSPPAHAEHGGVLARKMSLGGGRPYTPSPQVGTIPERPGWSGT
PSPQGAEMRGGRSPRPGSSAPEHSPRTSGLGCRLHSAPNLSDLHVVRPKLPKPPTDPLGAVFSP
PQASPPQPSHGLQSCRNLRGSPKLPDFLQRNPLPPILGSPTKAVPSFDFPKTPSSQNLLALLARQ
GVVMTPPRNRTLPDLSEVGPFHGQPLGPGLRPGEDPKGPFGRSFSTSRLTDLLLKAAFGTQAPD
PGSTESLQEKPMEIAPSAGFGGSLHPGARAGGTSSPSPVVFTVGSPPSGSTPPQGPRTRMFSAG
PTGSASSSARHLVPGPCSEAPAPELPAPGHGCSFADPIAANLEGAVTFEAPDLPEETLMEQEHTEI
LRGLRFTLLFVQHVLEIAALKGSASEAAGGPEYQLQESVVADQISLLSREWGFAEQLVLYLKVAEL

LSSGLQSAIDQIRAGKLCLSSTVKQVVRRLNELYKASVVSCQGLSLRLQRFFLDKQRLLDRIHSITA
ERLIFSHAVQMVQSAALDEMFQHREGCVPRYHKALLLLEGLQHMLSDQADIENVTKCKLCIERRL
SALLTGICA (SEQ ID NO:398)

# Figure 176

**Nucleotide:** U90338
**Sequence:**
atggcagctgctgaggaggagccgaagcccaaaaagctgaaggtggaggcgccgcaagcgctgagagaaaatattctctttggaatg
ggaaatcctctgcttgacatctctgctgtagtggacaaagatttccttgataagtattctctgaaaccaaatgaccaaatcttggctgaagaca
aacacaaggaactgtttgatgaacttgtgaaaaaattcaaagtcgaatatcatgctggtggctctacccagaattcaattaaagtggctcag
tggatgattcaacagccacacaaagcagcaacatttttggatgcattgggatagataaatttggggagatcctgaagagaaaagctgct
gaagcccatgtggatgctcattactacgagcagaatgagcagccaacaggaacttgtgctgcatgcatcactggtgacaacaggtccct
catagctaatcttgctgctgccaattgttataaaaaggaaaaacatcttgatctggagaaaaactggatgttggtagaaaaagcaagagttt
gttatatagcaggctttttcttacagtttccccagagtcagtattaaaggtggctcaccatgcttctgaaaacaacaggattttcactttgaatct
atctgcaccgtttattagccagttctacaaggaatcattgatgaaagttatgccttatgttgatatactttttggaaatgagacagaagctgcca
cttttgctagagagcaaggctttgagactaaagacattaaagagatagccaaaaagacacaagccctgccaaagatgaactcaaaga
ggcagcgaatcgtgatcttcacccaagggagagatgacactataatggctacagaaagtgaagtcactgcttttgctgtcttggatcaaga
ccagaaagaaattattgataccaatggagctggagatgcatttgttggaggttttctgtctcaactggtctctgacaagcctctgactgaatgt
atccgtgctggccactatgcagcaagcatcataattagacggactggctgcacctttcctgagaagccagacttccactga (SEQ ID NO:399)

**P55263**
MAAAEEEPKPKKLKVEAPQALRENILFGMGNPLLDISAVVDKDFLDKYSLKPNDQILAEDKHKELF
DELVKKFKVEYHAGGSTQNSIKVAQWMIQQPHKAATFFGCIGIDKFGEILKRKAAEAHVDAHYYEQ
NEQPTGTCAACITGDNRSLIANLAAANCYKKEKHLDLEKNWMLVEKARVCYIAGFFLTVSPESVLK
VAHHASENNRIFTLNLSAPFISQFYKESLMKVMPYVDILFGNETEAATFAREQGFETKDIKEIAKKT
QALPKMNSKRQRIVIFTQGRDDTIMATESEVTAFAVLDQDQKEIIDTNGAGDAFVGGFLSQLVSDK
PLTECIRAGHYAASIIIRRTGCTFPEKPDFH (SEQ ID NO:400)

# Figure 177

**Nucieotide:** L13738
**Sequence:**
atgcagccagaggagggcacaggctggctgctggagctgctgtccgaggtgcagctgcaacagtacttcctgcggctccgagatgacct
caacgtcacccgcctgtcccactttgagtacgtcaagaatgaggacctggagaagatcggcatgggtcggcctggccagcggcggctgt
gggaggctgtgaagaggaggaaggccttgtgcaaacgcaagtcgtggatgagtaaggtgttcagtggaaagcgactggaggctgagtt
cccacctcatcactctcagagcaccttccggaagacctcgcccgcccctggggcccagcaggggaggggcccctgcagagcctcac
ctgcctcattggggagaaggacctgcgcctcctggagaagctgggtgatggttcctttggcgtggtgcgcaggggcgagtgggacgcgc
cctcagggaagacggtgagtgtggctgtgaagtgcctgaagcccgatgtcctgagccagccagaagccatggacgacttcatccggga
ggtcaatgccatgcactcgctcgaccaccgaaacctcatccgcctctacggggtggtgctcacgccgcccatgaagatggtgacagagc
tggcacctctgggatcgttgttggaccggctacgtaagcaccagggccacttcctcctggggactctgagccgctacgctgtgcaggtggc
tgagggcatgggctacctggagtccaagcgctttattcaccgtgacctggctgcccgcaatctgctgttggctacccgcgacctggtcaag
atcggggactttggctgatgcgagcactacctcagaatgacgaccattacgtcatgcaggaacatcgcaaggtgcccttcgcctggtgt
gcccccgagagcctgaagacacgcaccttctcccatgccagcgacacctggatgttcggggtgacactgtgggaaatgttcacctacgg
ccaggagccctggatcggcctcaacggcagtcagatcctgcataagatcgacaaggagggggagcggctgccccggcccgaggact
gtccccaggacatctacaacgtcatggtccagtgctgggctcacaagccagaggacagacccacgtttgtggccctgcgggacttcctgc
tggaggcccagcccacagacatgcgggcccttcaggactttgaggaaccggacaagctgcacatccagatgaatgatgtcatcaccgt
catcgagggaagggccgagaactactggtggcgtggccagaacacacggacgctgtgtgtggggcccttccctcgcaacgtggtgacc
tccgtggccggcctgtcggcccaggacatcagccagcccctgcagaacagcttcatccacacagggcatggcgacagtgacccccgc
cactgctggggcttcccggacaggattgacgaactgtatctgggaaaccccatggacccccccgacctcctgagcgtggaactgagcac
ctcccggcccccccagcatctaggaggggtgaaaaaaccaacctatgaccctgtgagcgaggaccaagaccccttgtccagcgacttc
aagaggctgggcctgcggaagccaggcctgccccgagggctgtggctggcgaagccctcggcgcgggtgccgggcaccaaggcca
gccgaggcagcggggctgaggtcacgctcatcgacttcggtgaggagcccgtggtcccggccctacggccctgcccgccctccctggc
gcagctggccatggacgcctgctccctgctggacgagaccccgcctcagagccccacgcgggcactgccccggcccctgcaccccac
gcctgtggtggactgggacgcacgcccgctgcccccccccgcccgcctatgacgacgtggcccaggatgaggatgactttgagatctgct
ccatcaacagcaccctcgtgggcgcgggggtccctgccgggcccagccagggccagaccaactacgcctttgtgcctgagcaggcgc
ggccgcccccctcccctggaggacaacctgttcctcccgccccagggtgggggcaagccgcccagctccgcacagaccgcagagatct
tccaggcgctacagcaggagtgcatgaggcaactgcaggctccgggctccccggcccccctctcccagcccggggggtgacgacaag
ccccaggtgcctcctcgggtacccatccccctcggcccacgcgcccacacgtccagctgtctccagcccccccggcgaggaggag
accagccagtggcctggacctgcttcccctccccgggtgcctccgcgggagcccctgtcccctcaaggctcgaggacacccagcccccct
ggtaccacctggcagctccccgctgccaccccggctctcaagctcacctgggaagaccatgcccaccacccagagctttgcctcagacc
ccaagtacgccacccccccaggtgatccaggcccctggcgcgggtggtccctgcatcctgcccatcgtccgggatggcaagaaggtcag
cagcacccactattacttgctgcccgagcgaccatcctacctggagcgctaccagcgcttcctgcgtgaggcccagagccccgaggagc
ctaccccccctgcctgtgcctctgctgctgcccccacccagcaccccagcccccgccgcccccacggccaccgtgcggccgatgcccca
ggctgccttggaccccaaggccaacttctccaccaacaacagcaacccaggggcccggccaccacccccgagggccactgctcggc
tgccacagaggggctgccctggcgatgggccagaggcgggccggccagcagacaagatccagatggccatggtgcatggggtgac
cacagaggagtgccaggcggccctgcagtgccacggctggagcgtgcagagggctgcccagtatctgaaggtggagcagctcttcgg
gctgggtctgcggcccagaggggagtgccacaaagtgctggagatgttcgactggaacctggagcaggccggctgccaccttctgggc
tcctggggccctgcccaccacaagcgctga (SEQ ID NO:401)

**Q07912**
MQPEEGTGWLLELLSEVQLQQYFLRLRDDLNVTRLSHFEYVKNEDLEKIGMGRPGQRRLWEAVK
RRKALCKRKSWMSKVFSGKRLEAEFPPHHSQSTFRKTSPAPGGPAGEGPLQSLTCLIGEKDLRLL
EKLGDGSFGVVRRGEWDAPSGKTVSVAVKCLKPDVLSQPEAMDDFIREVNAMHSLDHRNLIRLY
GVVLTPPMKMVTELAPLGSLLDRLRKHQGHFLLGTLSRYAVQVAEGMGYLESKRFIHRDLAARNL
LLATRDLVKIGDFGLMRALPQNDDHYVMQEHRKVPFAWCAPESLKTRTFSHASDTWMFGVTLWE
MFTYGQEPWIGLNGSQILHKIDKEGERLPRPEDCPQDIYNVMVQCWAHKPEDRPTFVALRDFLLE
AQPTDMRALQDFEEPDKLHIQMNDVITVIEGRAENYWWRGQNTRTLCVGPFPRNVVTSVAGLSA
QDISQPLQNSFIHTGHGDSDPRHCWGFPDRIDELYLGNPMDPPDLLSVELSTSRPPQHLGGVKKP
TYDPVSEDQDPLSSDFKRLGLRKPGLPRGLWLAKPSARVPGTKASRGSGAEVTLIDFGEEPVVPA
LRPCPPSLAQLAMDACSLLDETPPQSPTRALPRPLHPTPVVDWDARPLPPPPAYDDVAQDEDDF
EICSINSTLVGAGVPAGPSQGQTNYAFVPEQARPPPPLEDNLFLPPQGGGKPPSSAQTAEIFQAL
QQECMRQLQAPGSPAPSPSPGGDDKPQVPPRVPIPPRPTRPHVQLSPAPPGEEETSQWPGPAS
PPRVPPREPLSPQGSRTPSPLVPPGSSPLPPRLSSSPGKTMPTTQSFASDPKYATPQVIQAPGAG
GPCILPIVRDGKKVSSTHYYLLPERPSYLERYQRFLREAQSPEEPTPLPVPLLLPPPSTPAPAAPTA

TVRPMPQAALDPKANFSTNNSNPGARPPPPRATARLPQRGCPGDGPEAGRPADKIQMAMVHGV
TTEECQAALQCHGWSVQRAAQYLKVEQLFGLGLRPRGECHKVLEMFDWNLEQAGCHLLGSWG
PAHHKR (SEQ ID NO:402)

# Figure 178

**Nucleotide:** X16416
**Sequence:**
atgttggagatctgcctgaagctggtgggctgcaaatccaagaaggggctgtcctcgtcctccagctgttatctggaagaagcccttcagc
ggccagtagcatctgactttgagcctcagggtctgagtgaagccgctcgttggaactccaaggaaaaccttctcgctggacccagtgaaa
atgaccccaaccttttcgttgcactgtatgattttgtggccagtggagataacactctaagcataactaaaggtgaaaagctccgggtcttag
gctataatcacaatggggaatggtgtgaagcccaaaccaaaaatggccaaggctgggtcccaagcaactacatcacgccagtcaaca
gtctggagaaacactcctggtaccatgggcctgtgtcccgcaatgccgctgagtatctgctgagcagcgggatcaatggcagcttcttggt
gcgtgagagtgagagcagtcctggccagaggtccatctcgctgagatacgaagggagggtgtaccattacaggatcaacactgcttctg
atggcaagctctacgtctcctccgagagccgcttcaacaccctggccgagttggttcatcatcattcaacggtggccgacgggctcatcac
cacgctccattatccagccccaaagcgcaacaagcccactgtctatggtgtgtcccccaactacgacaagtgggagatggaacgcacg
gacatcaccatgaagcacaagctgggcgggggccagtacggggaggtgtacgagggcgtgtggaagaaatacagcctgacggtgg
ccgtgaagaccttgaaggaggacaccatggaggtggaagagttcttgaaagaagctgcagtcatgaaagagatcaaacaccctaacc
tggtgcagctccttggggtctgcacccgggagcccccgttctatatcatcactgagttcatgacctacgggaacctcctggactacctgagg
gagtgcaaccggcaggaggtgaacgccgtggtgctgctgtacatggccactcagatctcgtcagccatggagtacctggagaagaaaa
acttcatccacagagatcttgctgcccgaaactgcctggtaggggagaaccacttggtgaaggtagctgattttggcctgagcaggttgatg
acaggggacacctacacagcccatgctggagccaagttccccatcaaatggactgcacccgagagcctggcctacaacaagttctcca
tcaagtccgacgtctgggcatttggagtattgctttgggaaattgctacctatggcatgtccccttacccgggaattgacctgtcccaggtgtat
gagctgctagagaaggactaccgcatggagcgcccagaaggctgcccagagaaggtctatgaactcatgcgagcatgttggcagtgg
aatccctctgaccggccctcctttgctgaaatccaccaagcctttgaaacaatgttccaggaatccagtatctcagacgaagtggaaaagg
agctggggaaacaaggcgtccgtggggctgtgagtaccttgctgcaggccccagagctgcccaccaagacgaggacctccaggaga
gctgcagagcacagagacaccactgacgtgcctgagatgcctcactccaagggccagggagagagcgatcctctggaccatgagcct
gccgtgtctccattgctccctcgaaaagagcgaggtcccccggagggcggcctgaatgaagatgagcgccttctccccaaagacaaaa
agaccaacttgttcagcgccttgatcaagaagaagaagaagacagccccaaccccctcccaaacgcagcagctccttccgggagatgg
acggccagccggagcgcagaggggccggcgaggaagagggccgagacatcagcaacggggcactggctttcaccccccttggaca
cagctgacccagccaagtccccaaagcccagcaatggggctggggtccccaatggagccctccgggagtccgggggctcaggcttcc
ggtctccccacctgtggaagaagtccagcacgctgaccagcagccgcctagccaccggcgaggaggagggcggtggcagctccagc
aagcgcttcctgcgctcttgctccgcctcctgcgttccccatggggccaaggacacggagtggaggtcagtcacgctgcctcgggacttgc
agtccacgggaagacagtttgactcgtccacatttggagggcacaaaagtgagaagccggctctgcctcggaagagggcaggggag
aacaggtctgaccaggtgacccgaggcacagtaacgcctcccccccaggctggtgaaaaagaatgaggaagctgctgatgaggtcttc
aaagacatcatggagtccagcccgggctccagcccgcccaacctgactccaaaacccctccggcggcaggtcaccgtggcccctgcc
tcgggcctcccccacaaggaagaagctgaaaagggcagtgccttagggacccctgctgcagctgagccagtgacccccaccagcaa
agcaggctcaggtgcaccagggggcaccagcaagggccccgccgaggagtccagagtgaggaggcacaagcactcctctgagtcg
ccagggagggacaaggggaaattgtccaggctcaaacctgccccgccgcccccaccagcagcctctgcagggaaggctggaggaa
agccctcgcagagcccgagccaggaggcggccggggaggcagtcctgggcgcaaagacaaaagccacgagtctggttgatgctgt
gaacagtgacgctgccaagcccagccagccgggagagggcctcaaaaagcccgtgctcccggccactccaaagccacagtccgcc
aagccgtcggggaccccccatcagcccagcccccgttccctccacgttgccatcagcatcctcggccctggcaggggaccagccgtcttc
cactgccttcatccctctcatatcaacccgagtgtctcttcggaaaacccgccagcctccagagcggatcgccagcggcgccatcaccaa
gggcgtggtcctggacagcaccgaggcgctgtgcctcgccatctctaggaactccgagcagatggccagccacagcgcagtgctgga
ggccggcaaaaacctctacacgttctgcgtgagctatgtggattccatccagcaaatgaggaacaagtttgccttccgagaggccatcaa
caaactggagaataatctccgggagcttcagatctgcccggcgacagcaggcagtggtccggcggccactcaggacttcagcaagctc
ctcagttcggtgaaggaaatcagtgacatagtgcagaggtag (SEQ ID NO:403)

**P00519**
MLEICLKLVGCKSKKGLSSSSSCYLEEALQRPVASDFEPQGLSEAARWNSKENLLAGPSENDPNL
FVALYDFVASGDNTLSITKGEKLRVLGYNHNGEWCEAQTKNGQGWVPSNYITPVNSLEKHSWYH
GPVSRNAAEYLLSSGINGSFLVRESESSPGQRSISLRYEGRVYHYRINTASDGKLYVSSESRFNTL
AELVHHHSTVADGLITTLHYPAPKRNKPTVYGVSPNYDKWEMERTDITMKHKLGGGQYGEVYEG
VWKKYSLTVAVKTLKEDTMEVEEFLKEAAVMKEIKHPNLVQLLGVCTREPPFYIITEFMTYGNLLDY
LRECNRQEVNAVVLLYMATQISSAMEYLEKKNFIHRDLAARNCLVGENHLVKVADFGLSRLMTGD
TYTAHAGAKFPIKWTAPESLAYNKFSIKSDVWAFGVLLWEIATYGMSPYPGIDLSQVYELLEKDYR
MERPEGCPEKVYELMRACWQWNPSDRPSFAEIHQAFETMFQESSISDEVEKELGKQGVRGAVS
TLLQAPELPTKTRTSRRAAEHRDTTDVPEMPHSKGQGESDPLDHEPAVSPLLPRKERGPPEGGL
NEDERLLPKDKKTNLFSALIKKKKKTAPTPPKRSSSFREMDGQPERRGAGEEEGRDISNGALAFT
PLDTADPAKSPKPSNGAGVPNGALRESGGSGFRSPHLWKKSSTLTSSRLATGEEEGGGSSSKRF

LRSCSASCVPHGAKDTEWRSVTLPRDLQSTGRQFDSSTFGGHKSEKPALPRKRAGENRSDQVT
RGTVTPPPRLVKKNEEAADEVFKDIMESSPGSSPPNLTPKPLRRQVTVAPASGLPHKEEAEKGSA
LGTPAAAEPVTPTSKAGSGAPGGTSKGPAEESRVRRHKHSSESPGRDKGKLSRLKPAPPPPPAA
SAGKAGGKPSQSPSQEAAGEAVLGAKTKATSLVDAVNSDAAKPSQPGEGLKKPVLPATPKPQSA
KPSGTPISPAPVPSTLPSASSALAGDQPSSTAFIPLISTRVSLRKTRQPPERIASGAITKGVVLDSTE
ALCLAISRNSEQMASHSAVLEAGKNLYTFCVSYVDSIQQMRNKFAFREAINKLENNLRELQICPAT
AGSGPAATQDFSKLLSSVKEISDIVQR (SEQ ID NO:404)

# Figure 179

**Nucleotide:** M23725
**Sequence:**
atgtcgaagccccatagtgaagccgggactgccttcattcagacccagcagctgcacgcagccatggctgacacattcctggagcacat
gtgccgcctggacattgattcaccacccatcacagcccggaacactggcatcatctgtaccattggcccagcttcccgatcagtggagac
gttgaaggagatgattaagtctggaatgaatgtggctcgtctgaacttctctcatggaactcatgagtaccatgcggagaccatcaagaatg
tgcgcacagccacggaaagctttgcttctgaccccatcctctaccggcccgttgctgtggctctagacactaaaggacctgagatccgaac
tgggctcatcaagggcagcggcactgcagaggtggagctgaagaagggagccactctcaaaatcacgctggataacgcctacatgga
aaagtgtgacgagaacatcctgtggctggactacaagaacatctgcaaggtggtggaagtgggcagcaagatctacgtggatgatggg
cttatttctctccaggtgaagcagaaaggtgccgacttcctggtgacggaggtggaaaatggtggctccttgggcagcaagaagggtgtg
aaccttcctggggctgctgtggacttgcctgctgtgtcggagaaggacatccaggatctgaagtttggggtcgagcaggatgttgatatggt
gtttgcgtcattcatccgcaaggcatctgatgtccatgaagttaggaaggtcctgggagagaagggaaagaacatcaagattatcagcaa
aatcgagaatcatgaggggggttcggaggtttgatgaaatcctggaggccagtgatgggatcatggtggctcgtggtgatctaggcattgag
attcctgcagagaaggtcttccttgctcagaagatgatgattggacggtgcaaccgagctgggaagcctgtcatctgtgctactcagatgct
ggagagcatgatcaagaagccccgccccactcgggctgaaggcagtgatgtggccaatgcagtcctggatggagccgactgcatcat
gctgtctggagaaacagccaaaggggactatcctctggaggctgtgcgcatgcagaacctgattgcccgtgaggcagaggctgccatct
accacttgcaattatttgaggaactccgccgcctggcgcccattaccagcgaccccacagaagccaccgccgtgggtgccgtggaggc
ctccttcaagtgctgcagtggggccataatcgtcctcaccaagtctggcaggtctgctcaccaggtggccagataccgcccacgtgcccc
catcattgctgtgacccggaatccccagacagctcgtcaggcccacctgtaccgtggcatcttccctgtgctgtgcaaggacccagtccag
gaggcctgggctgaggacgtggacctccgggtgaactttgccatgaatgttggcaaggcccgaggcttcttcaagaagggagatgtggt
cattgtgctgaccggatggcgccctggctccggcttcaccaacaccatgcgtgttgttcctgtgccgtga (SEQ ID NO:405)

**P14786**
MSKPHSEAGTAFIQTQQLHAAMADTFLEHMCRLDIDSPPITARNTGIICTIGPASRSVETLKEMIKS
GMNVARLNFSHGTHEYHAETIKNVRTATESFASDPILYRPVAVALDTKGPEIRTGLIKGSGTAEVEL
KKGATLKITLDNAYMEKCDENILWLDYKNICKVVEVGSKIYVDDGLISLQVKQKGADFLVTEVENG
GSLGSKKGVNLPGAAVDLPAVSEKDIQDLKFGVEQDVDMVFASFIRKASDVHEVRKVLGEKGKNI
KIISKIENHEGVRRFDEILEASDGIMVARGDLGIEIPAEKVFLAQKMMIGRCNRAGKPVICATQMLES
MIKKPRPTRAEGSDVANAVLDGADCIMLSGETAKGDYPLEAVRMQNLIAREAEAAIYHLQLFEELR
RLAPITSDPTEATAVGAVEASFKCCSGAIIVLTKSGRSAHQVARYRPRAPIIAVTRNPQTARQAHLY
RGIFPVLCKDPVQEAWAEDVDLRVNFAMNVGKARGFFKKGDVVIVLTGWRPGSGFTNTMRVVPV
P (SEQ ID NO:406)

# Figure 180

**Nucleotide:** M18907
**Sequence:**
atggctctcatcccagacttggccatggaaacctggcttctcctggctgtcagcctggtgctcctctatctatatggaacccattcacatggact
ttttaagaagcttggaattccagggcccacacctctgcctttttgggaaatattttgtcctaccataagggcttttgtatgtttgacatggaatgtc
ataaaaagtatggaaaagtgtggggcttttatgatggtcaacagcctgtgctggctatcacagatcctgacatgatcaaaacagtgctagtg
aaagaatgttattctgtcttcacaaaccggaggccttttggtccagtgggatttatgaaaagtgccatctctatagctgaggatgaagaatgg
aagagattacgatcattgctgtctccaaccttcaccagtggaaaactcaaggagatggtccctatcattgcccagtatggagatgtgttggtg
agaaatctgaggcgggaagcagagacaggcaagcctgtcaccttgaaagacgtctttggggcctacagcatggatgtgatcactagca
catcatttggagtgaacatcgactctctcaacaatccacaagaccccttgtggaaaacaccaagaagcttttaagatttgatttttggatcc
attctttctctcaataacagtctttccattcctcatcccaattcttgaagtattaaatatctgtgtgtttccaagagaagttacaaatttttaagaaa
atctgtaaaaaggatgaaagaaagtcgcctcgaagatacacaaaagcaccgagtggatttccttcagctgatgattgactctcagaattc
aaaagaaactgagtcccacaaagctctgtccgatctggagctcgtggcccaatcaattatctttatttttgctggctatgaaaccacgagca
gtgttctctccttcattatgtatgaactggccactcaccctgatgtccagcagaaactgcaggaggaaattgatgcagttttacccaataagg
caccacccacctatgatactgtgctacagatggagtatcttgacatggtggtgaatgaaacgctcagattattcccaattgctatgagacttg
agagggtctgcaaaaaagatgttgagatcaatgggatgttcattcccaaaggggtggtggtgatgattccaagctatgctcttcaccgtgac
ccaaagtactggacagagcctgagaagttcctccctgaaagattcagcaagaagaacaaggacaacatagatccttacatatacacac
cctttggaagtggacccagaaactgcattggcatgaggtttgctctcatgaacatgaaacttgctctaatcagagtccttcagaacttctcctt
caaaccttgtaaagaaacacagatccccctgaaattaagcttaggaggacttcttcaaccagaaaaacccgttgttctaaaggttgagtca
agggatggcaccgtaagtggagcctga (SEQ ID NO:407)

**P08684**
MALIPDLAMETWLLLAVSLVLLYLYGTHSHGLFKKLGIPGPTPLPFLGNILSYHKGFCMFDMECHKK
YGKVWGFYDGQQPVLAITDPDMIKTVLVKECYSVFTNRRPFGPVGFMKSAISIAEDEEWKRLRSL
LSPTFTSGKLKEMVPIIAQYGDVLVRNLRREAETGKPVTLKDVFGAYSMDVITSTSFGVNIDSLNNP
QDPFVENTKKLLRFDFLDPFFLSITVFPFLIPILEVLNICVFPREVTNFLRKSVKRMKESRLEDTQKH
RVDFLQLMIDSQNSKETESHKALSDLELVAQSIIFIFAGYETTSSVLSFIMYELATHPDVQQKLQEEI
DAVLPNKAPPTYDTVLQMEYLDMVVNETLRLFPIAMRLERVCKKDVEINGMFIPKGWVVMIPSYAL
HRDPKYWTEPEKFLPERFSKKNKDNIDPYIYTPFGSGPRNCIGMRFALMNMKLALIRVLQNFSFKP
CKETQIPLKLSLGGLLQPEKPVVLKVESRDGTVSGA (SEQ ID NO:408)

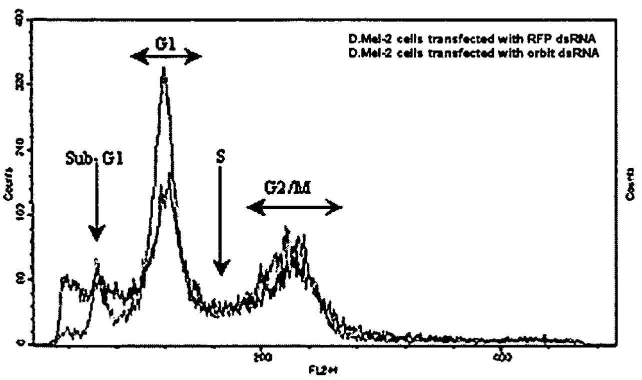
D.Mel-2 cells transfected with RFP dsRNA
D.Mel-2 cells transfected with orbit dsRNA
G1
Sub-G1
S
G2/M
Counts
200
400
FL2-H

Figure 181

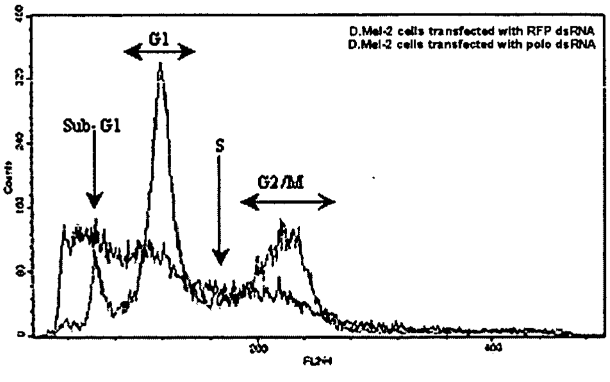


Figure 181 (cont'd)

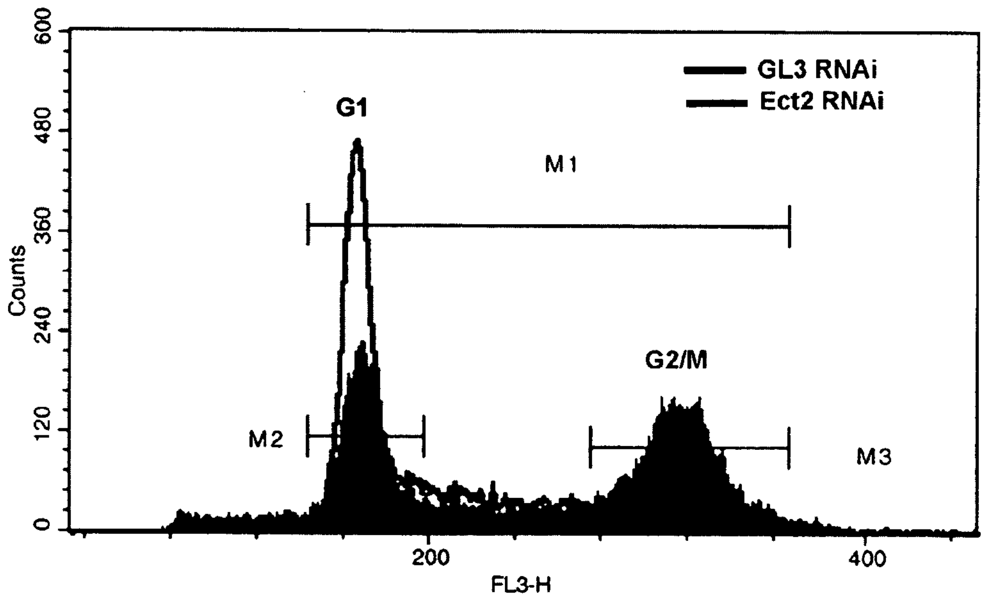
GL3 RNAi
Ect2 RNAi
G1
M1
G2/M
M2
M3
Counts
0
120
240
360
480
600
200
400
FL3-H

Figure 182

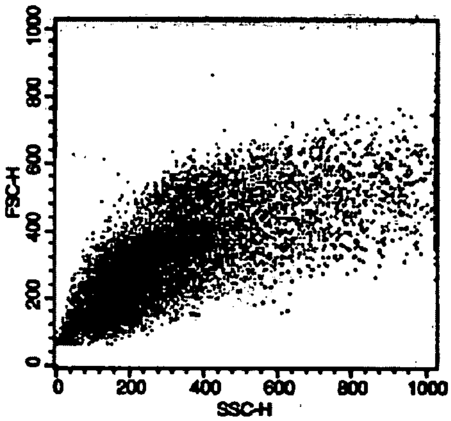


Figure 183

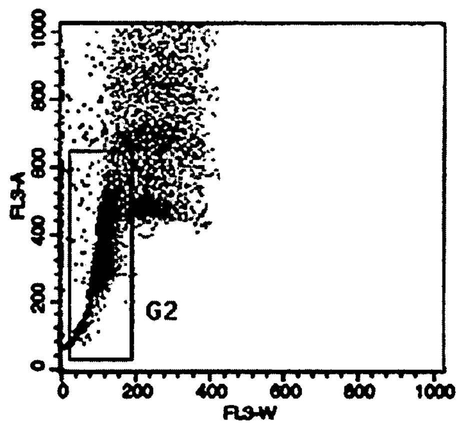


Figure 184

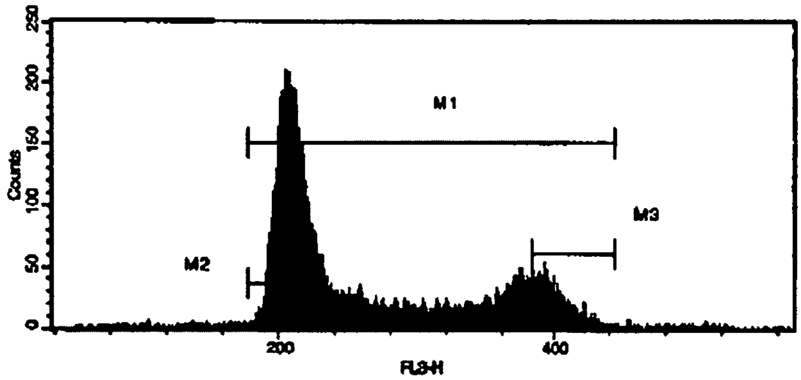


Histogram Statistics

File: CAM20AUG02.009
Acquisition Date: 20-Aug-02
Gated Events: 10151
X Parameter: FL3-H (Linear)

Log Data Units: Linear Values
Gate: G2
Total Events: 14850

| Marker | Left, Right | Events | % Gated | % Total | Mean | Geo Mean | | |
|---|---|---|---|---|---|---|---|---|
| All | 0, 1023 | 10151 | 100.00 | 68.36 | 269.57 | 257.81 | | |
| M1 | 178, 444 | 9730 | 95.85 | 65.52 | 268.08 | 258.78 | G1 events | - 48.6% |
| M2 | 178, 208 | 2466 | 24.29 | 16.61 | 201.04 | 200.98 | G2/M events | - 23.1% |
| M3 | 384, 444 | 1176 | 11.59 | 7.92 | 400.75 | 400.53 | S events | - 24.2% |

Figure 185

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5976838 A
- WO 8903891 A
- WO 9013667 A
- EP 239400 A
- US 4683195 A
- US 4683202 A
- US 5837832 A
- US 5143854 A
- WO 9015070 A
- WO 9210092 A
- US 6100090 A
- WO 0001417 A
- WO 002927 A


### Non-patent literature cited in the description


- **J. SAMBROOK ; E. F. FRITSCH ; T. MANIATIS.** Molecular Cloning: A Laboratory Manual. Cold Spring Harbor Laboratory Press, 1989, vol. 1-3
- **AUSUBEL, F. M. et al.** Current Protocols in Molecular Biology. John Wiley & Sons, 1995
- **B. ROE ; J. CRABTREE ; A. KAHN.** DNA Isolation and Sequencing: Essential Techniques. John Wiley & Sons, 1996
- **J. M. POLAK ; JAMES O'D. MCGEE.** In Situ Hybridization: Principles and Practice. Oxford University Press, 1990
- Oligonucleotide Synthesis: A Practical Approach. IRL Press, 1984
- **D. M. J. LILLEY ; J. E. DAHLBERG.** Methods of Enzymology: DNA Structure Part A: Synthesis and Physical Analysis of DNA Methods in Enzymology. Academic Press, 1992
- **DEVEREUX et al.** *Nucleic Acids Research,* 1984, vol. 12, 387
- **ALTSCHUL et al.** *J. Mol. Biol.,* 1990, vol. 215, 403-410
- Guide to Molecular Cloning Techniques. **BERGER ; KIMMEL.** Methods in Enzymology. Academic Press, 1987, vol. 152
- Current Protocols in Molecular Biology. John Wiley & Sons, Inc, 1995
- **KARLIN ; ALTSCHUL.** *Proc. Natl. Acad. Sci. USA,* 1990, vol. 87 (6), 2264-8
- **HENIKOFF ; HENIKOFF.** *Proc. Natl. Aacad Sci. USA,* 1992, vol. 89 (22), 10915-9
- **WOOTTON ; FEDERHEN.** *Computers and Chemistry,* 1993, vol. 17, 149-163
- **CLAVERIE ; STATES.** *Computers and Chemistry,* 1993, vol. 17, 191-201
- **LANDEGREN, U. et al.** *Science,* 1988, vol. 242, 229-237
- **LEWIS, R.** *Genetic Engineering News,* 1990, vol. 10 (1), 54-55
- **MOK et al.** *Gynaecologic Oncology,* 1994, vol. 52, 247-252
- **LIZARDI et al.** *Nat Genet,* 1998, vol. 19, 225
- **WALKER et al.** *Proc. Natl. Acad. Sci. USA,* 1992, vol. 80, 392
- Current Protocols in Molecular Biology. John Wiley & Sons, Inc, 1994, vol. 1
- **LEMIEUX et al.** *Molecular Breeding,* 1998, vol. 4, 277-289
- **SCHENA ; DAVIS.** Parallel Analysis with Biological Chips. in PCR Methods Manual
- **SCHENA ; DAVIS.** Genes, Genomes and Chips. In DNA Microarrays: A Practical Approach. Oxford University Press, 1999
- Microarray Biochip Technology. Eaton Publishing Company
- **CORTES.** *The Scientist,* 2000
- **GWYNNE ; PAGE.** Microarray analysis: the next revolution in molecular biology. *Science,* 06 August 1999
- **EAKINS ; CHU.** *Trends in Biotechnology,* 1999, vol. 17, 217-218
- **CELIS et al.** *FEBS Lett,* 2000, vol. 480 (1), 2-16
- **LOCKHART ; WINZELER.** *Nature,* 2000, vol. 405 (6788), 827-836
- **KHAN et al.** *NATURE,* 1999, vol. 20 (2), 223-9
- **MARX.** *Science,* 2000, vol. 289, 1670-1672
- **SCHERF et al.** *Nat Genet,* 2000, vol. 24 (3), 236-44
- **ROSS et al.** *Nat Genet,* 2000, vol. 24 (3), 227-35
- **WANG et al.** *Science,* 1998, vol. 280 (5366), 1077-82
- *Chemical & Engineering News,* 22 February 1999, vol. 77 (8), 27-36
- **ROCKETT ; DIX.** *Xenobiotica,* 2000, vol. 30 (2), 155-77

- **AFSHARI et al.** *Cancer Res,* 1999, vol. 59 (19), 4759-60
- **NUWAYSIR et al.** *Molecular Carcinogenesis,* 1999, vol. 24, 153-159
- **CORTESE.** *The Scientist,* 2000, vol. 14 (11), 26
- **FODOR et al.** *Science,* 1991, vol. 251, 767
- **DOWER ; FODOR.** *Ann. Rep. Med. Chem.,* 1991, vol. 26, 271
- **SHALON et al.** *Genome Res,* 1996, vol. 6 (7), 639-45
- **EKINS ; CHU.** *Trends in Biotechnology,* 1999, vol. 17, 217-218
- **MARSHALL ; HODGSON.** *Nature Biotechnology,* 1998, vol. 16 (1), 27-31
- **BRAZMA ; VILO J.** *FEBS Lett,* 2000, vol. 480 (1), 17-24
- **BORREBAECK CA.** *Immunol Today,* 2000, vol. 21 (8), 379-82
- **MACBEATH ; SCHREIBER.** *Science,* 2000, vol. 289 (5485), 1760-1763
- **NECKERS et al.** *Crit Rev Oncog,* 1992, vol. 3 (1-2), 175-231
- **FIRE et al.** *Nature,* 1998, vol. 391, 806-811
- **BLOW, J.J.** *J Cell Biol,* 1993, vol. 122, 993-1002
- **MIZUNO et al.** *Mol Cell Biol,* 1998, vol. 18, 3552-62
- **SMITH ; JOHNSON.** *Gene,* 1988, vol. 67 (10), 31-40
- **SAUNDERS et al.** *J. Cell Biol.,* 1997, vol. 137 (4), 881-90
- **ANDERSEN ; KARSENTI.** *J. Cell. Biol.,* 1997, vol. 139 (4), 975-83
- **MORITZ et al.** *J. Cell Biol.,* 1998, vol. 142 (3), 775-86
- **HOWARD ; HYMAN ; CHANDRA ; ENDOW.** Motility Assays for Motor Proteins. Academic Press, 1993
- **TOUMEBIZE ; HEALD.** *Nature,* 1996, vol. 382 (6590), 420-5
- **LI ; KELLY.** *Proc. Natl. Acad. Sci USA,* 1984, vol. 81, 6973-6977
- **WAGA ; STILLMAN.** *Nature,* 1994, vol. 369, 207-212
- **CREVEL ; COTTERIL.** *EMBO J.,* 1991, vol. 10, 4361-4369
- **BLOW ; LASKEY.** *Cell,* 1986, vol. 47, 577-587
- **SHEEHAN et al.** *J. Cell Biol.,* 1988, vol. 106, 1-12
- **KRUDE et al.** *Cell,* 1997, vol. 88, 109-19
- **KREK.** *Curr Opin Genet Dev,* 1998, vol. 8, 36-42
- **LISTOVSKY et al.** *Exp Cell Res,* 2000, vol. 255, 184-191
- **WIANNY ; ZERNICKA-GOETZ.** *Nat Cell Biol,* 2000, vol. 2, 70-75
- **DEROSSI et al.** *J. Biol. Chem.,* 1994, vol. 269, 10444-50
- **MATZ et al.** *Nat Biotechnol.,* 1999, vol. 17, 969-973
- **INOUE et al.** *J Cell Biol.,* 2000, vol. 149, 153-166
- **MAIATO et al.** *J Cell Biol.,* 2002, vol. 157, 749-760
- **SUNKEL ; GLOVER.** *J Cell Sci.,* 1988, vol. 89, 25-38
- **FENTON ; GLOVER.** *Nature,* 1993, vol. 363, 637-640
- **CARMENA et al.** *J Cell Biol.,* 1998, vol. 143, 659-671
- **HARBORTH et al.** *J Cell Sci.,* 2001, vol. 114, 4557-4565